(19) Europäisches Patentamt
European Patent Office
Office européen des brevets

(11) EP 2 199 395 A1

(12) EUROPEAN PATENT APPLICATION

(43) Date of publication:
**23.06.2010 Bulletin 2010/25**

(51) Int Cl.:
***C12N 15/82*** *(2006.01)* ***A01H 5/00*** *(2006.01)*

(21) Application number: **09178586.5**

(22) Date of filing: **02.08.2007**

(84) Designated Contracting States:
**AT BE BG CH CY CZ DE DK EE ES FI FR GB GR HU IE IS IT LI LT LU LV MC MT NL PL PT RO SE SI SK TR**

(30) Priority:
**12.10.2006 US 851258 P**
**01.12.2006 US 868095 P**
**12.10.2006 US 851250 P**
**12.10.2006 US 851265 P**
**17.11.2006 US 859717 P**
**10.08.2006 US 836804 P**
**06.10.2006 EP 06121928**
**27.10.2006 EP 06123066**
**31.10.2006 EP 06123237**
**05.10.2006 EP 06121856**
**02.08.2006 EP 06118347**
**20.09.2006 EP 06120994**

(62) Document number(s) of the earlier application(s) in accordance with Art. 76 EPC:
**07788181.1 / 2 054 516**

(27) Previously filed application:
**02.08.2007 EP 07788181**

(71) Applicant: **CropDesign N.V.**
**9052 Zwijnaarde (BE)**

(72) Inventors:
- **Sanz Molinero, Ana Isabel**
  **9050 Gentbrugge (BE)**
- **Reuzeau, Christophe**
  **24350 Tocan Saint Apre (FR)**
- **Frankard, Valerie**
  **1410 Waterloo (BE)**
- **Mironov, Vladimir**
  **9000 Gent (BE)**

(74) Representative: **Mistry, Meeta**
**BASF SE**
**Global Intellectual Property**
**GVX - C6**
**67056 Ludwigshafen (DE)**

Remarks:
This application was filed on 10-12-2009 as a divisional application to the application mentioned under INID code 62.

# (54) Plants transformed with SYT-polypeptide having increased yield under abiotic stress and a method for making the same

(57) The present invention relates generally to the field of molecular biology and concerns a method for enhancing various economically important characteristics in plants. More specifically, the present invention concerns a method for improving yield-related traits, such as enhanced yield and/or enhanced growth, or modified content of storage compoundsin plants by modulating expression in a plant of a nucleic acid encoding a GRP (Growht Related Protein) polypeptide. The present invention also concerns plants having modulated expression of a nucleic acid encoding a GRP polypeptide, which plants have improved characterisitics relative to control plants. The invention also provides hitherto unknown GRP-encoding nucleic acids, and constructs comprising the same, useful in performing the methods of the invention.

EP 2 199 395 A1

## Description

The present invention relates generally to the field of molecular biology and concerns a method for improving various plant characteristics by modulating expression in a plant of a nucleic acid encoding a GRP (Growth Related Protein). The present invention also concerns plants having modulated expression of a nucleic acid encoding a GRP polypeptide, which plants have improved characteristics relative to corresponding wild type plants or other control plants. The invention also provides constructs useful in the methods of the invention.

The ever-increasing world population and the dwindling supply of arable land available for agriculture fuels research towards increasing the efficiency of agriculture. Conventional means for crop and horticultural improvements utilise selective breeding techniques to identify plants having desirable characteristics. However, such selective breeding techniques have several drawbacks, namely that these techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

A trait of particular economic interest is increased yield. Yield is normally defined as the measurable produce of economic value from a crop. This may be defined in terms of quantity and/or quality. Yield is directly dependent on several factors, for example, the number and size of the organs, plant architecture (for example, the number of branches), seed production, leaf senescence and more. Root development, nutrient uptake, stress tolerance and early vigour may also be important factors in determining yield. Optimizing the abovementioned factors may therefore contribute to increasing crop yield.

Seed yield is a particularly important trait, since the seeds of many plants are important for human and animal nutrition. Crops such as corn, rice, wheat, canola and soybean account for over half the total human caloric intake, whether through direct consumption of the seeds themselves or through consumption of meat products raised on processed seeds. They are also a source of sugars, oils and many kinds of metabolites used in industrial processes. Seeds contain an embryo (the source of new shoots and roots) and an endosperm (the source of nutrients for embryo growth during germination and during early growth of seedlings). The development of a seed involves many genes, and requires the transfer of metabolites from the roots, leaves and stems into the growing seed. The endosperm, in particular, assimilates the metabolic precursors of carbohydrates, oils and proteins and synthesizes them into storage macromolecules to fill out the grain.

Another important trait for many crops is early vigour. Improving early vigour is an important objective of modern rice breeding programs in both temperate and tropical rice cultivars. Long roots are important for proper soil anchorage in water-seeded rice. Where rice is sown directly into flooded fields, and where plants must emerge rapidly through water, longer shoots are associated with vigour. Where drill-seeding is practiced, longer mesocotyls and coleoptiles are important for good seedling emergence. The ability to engineer early vigour into plants would be of great importance in agriculture. For example, poor early vigour has been a limitation to the introduction of maize (Zea mays L.) hybrids based on Corn Belt germplasm in the European Atlantic.

A further important trait is that of improved abiotic stress tolerance. Abiotic stress is a primary cause of crop loss worldwide, reducing average yields for most major crop plants by more than 50% (Wang et al., Planta (2003) 218: 1-14). Abiotic stresses may be caused by drought, salinity, extremes of temperature, chemical toxicity and oxidative stress. The ability to improve plant tolerance to abiotic stress would be of great economic advantage to farmers worldwide and would allow for the cultivation of crops during adverse conditions and in territories where cultivation of crops may not otherwise be possible.

Crop yield may therefore be increased by optimising one of the above-mentioned factors.

Depending on the end use, the modification of certain yield traits may be favoured over others. For example for applications such as forage or wood production, or bio-fuel resource, an increase in the vegetative parts of a plant may be desirable, and for applications such as flour, starch or oil production, an increase in seed parameters may be particularly desirable. Even amongst the seed parameters, some may be favoured over others, depending on the application. Various mechanisms may contribute to increasing seed yield, whether that is in the form of increased seed size or increased seed number.

One approach to increasing yield (seed yield and/or biomass) in plants may be through modification of the inherent growth mechanisms of a plant, such as the cell cycle or various signalling pathways involved in plant growth or in defense mechanisms.

It has now been found that various characteristics may be improved in plants by modulating expression in a plant of a nucleic acid encoding a GRP polypeptide in a plant. The GRP polypeptide may be one of the following: an Ankyrin - Zinc finger polypeptide (AZ), a SYT polypeptide; a chloroplastic fructose-1, 6-bisphosphatase (cpFBPase) polypeptide; a small inducible kinase (SIK); a Class II homeodomain-leucine zipper (HD Zip) transcription factor; and a SYB1 polypeptide. The improved characteristics comprise yield related traits, such as enhanced yield and/or enhanced growth, or modified content of storage compounds.

**Background**

Ankyrin - Zinc finger polypeptide

Plant biomass is yield for forage crops like alfalfa, silage corn and hay. Many proxies for yield have been used in grain crops. Chief amongst these are estimates of plant size. Plant size can be measured in many ways depending on species and developmental stage, but include total plant dry weight, above-ground dry weight, above-ground fresh weight, leaf area, stem volume, plant height, rosette diameter, leaf length, root length, root mass, tiller number and leaf number. Many species maintain a conservative ratio between the size of different parts of the plant at a given developmental stage. These allometric relationships are used to extrapolate from one of these measures of size to another (e.g. Tittonell et al 2005 Agric Ecosys & Environ 105: 213). Plant size at an early developmental stage will typically correlate with plant size later in development. A larger plant with a greater leaf area can typically absorb more light and carbon dioxide than a smaller plant and therefore will likely gain a greater weight during the same period (Fasoula & Tollenaar 2005 Maydica 50:39). This is in addition to the potential continuation of the micro-environmental or genetic advantage that the plant had to achieve the larger size initially. There is a strong genetic component to plant size and growth rate (e.g. ter Steege et al 2005 Plant Physiology 139:1078), and so for a range of diverse genotypes plant size under one environmental condition is likely to correlate with size under another (Hittalmani et al 2003 Theoretical Applied Genetics 107:679). In this way a standard environment is used as a proxy for the diverse and dynamic environments encountered at different locations and times by crops in the field.

Harvest index, the ratio of seed yield to aboveground dry weight, is relatively stable under many environmental conditions and so a robust correlation between plant size and grain yield can often be obtained (e.g. Rebetzke et al 2002 Crop Science 42:739). These processes are intrinsically linked because the majority of grain biomass is dependent on current or stored photosynthetic productivity by the leaves and stem of the plant (Gardener et al 1985 Physiology of Crop Plants. Iowa State University Press, pp68-73). Therefore, selecting for plant size, even at early stages of development, has been used as an indicator for future potential yield (e.g. Tittonell et al 2005 Agric Ecosys & Environ 105: 213). When testing for the impact of genetic differences on stress tolerance, the ability to standardize soil properties, temperature, water and nutrient availability and light intensity is an intrinsic advantage of greenhouse or plant growth chamber environments compared to the field. However, artificial limitations on yield due to poor pollination due to the absence of wind or insects, or insufficient space for mature root or canopy growth, can restrict the use of these controlled environments for testing yield differences. Therefore, measurements of plant size in early development, under standardized conditions in a growth chamber or greenhouse, are standard practices to provide indication of potential genetic yield advantages.

Transcription is performed by RNA polymerases. These polymerases are usually associated with other proteins (transcription factors) that determine the specificity of the transcription process. The transcription factors bind to cis-regulatory elements of the gene and may also mediate binding of other regulatory proteins. Stegmaier et al. proposed a classification of transcription factors based on their DNA-binding domains. 5 superclasses were discriminated, based on the presence of: 1) basic domains, 2) zinc-coordinating domains, 3) helix-turn-helix domains, 4) beta scaffold domains with minor groove contacts and 0) other domains (Stegmaier et al., Genome informatics 15, 276-286, 2004). The group of transcription factors comprising zinc-coordinating domains is quite divers and may be further classified according to their conserved cysteine and histidine residues, including the WRKY domains, C6 zinc clusters, DM and GCM domains.

Besides DNA binding motifs, transcription factors may also comprise protein-protein interaction motifs. One such motif is the ankyrin motif. It is present in very diverse families of proteins, usually as a repeat of 2 to over 20 units. Each unit contains two antiparallel helices and a beta-hairpin.

Although many plant proteins with zinc finger domains are well characterised, little is known about plant proteins comprising the C3H1 zinc finger motif. PEI1, a transcription factor that reportedly plays a role in embryo development, has a zinc finger motif that resembles the C3H1 motif but lacks an ankyrin motif (Li and Thomas, Plant Cell 10, 383-398, 1998). WO 02/44389 describes AtSIZ, a transcription factor isolated from *Arabidopsis.* Expression of *AtSIZ* under control of the CaMV35S promoter was found to promote transcription of stress-induced genes, and plants with increased expression of *AtSIZ* reportedly had a higher survival rate under salt stress than control plants, but no analysis was provided with respect to seed yield. It was postulated that AtSIZ could be used for increasing resistance in plants to osmotic stress.

SYT

Abiotic stresses such as drought stress, salinity stress, heat stress and cold stress, or a combination of one or more of these, are major limiting factors of plant growth and productivity (Boyer (1982) Science 218: 443-448). These stresses have as common theme important for plant growth water availability. Since high salt content in some soils results in less available water for cell intake, its effect is similar to those observed under drought conditions. Additionally, under freezing temperatures, plant cells loose water as a result of ice formation that starts from the apoplast and withdraws water from the symplast (McKersie and Leshem (1994) Stress and stress coping in cultivated plants, Kluwer Academic Publishers). During heat stress, stomata aperture is affected to adjust cooling by evapotranspiration, thereby affecting the water content of the plant. Commonly, a plant's molecular response mechanisms to each of these stress conditions is similar.

Plants are exposed during their entire life cycle to conditions of reduced environmental water content. Most plants have evolved strategies to protect themselves against these conditions. However, if the severity and duration of the stress conditions are too great, the effects on plant development, growth and yield of most crop plant are profound. Continuous exposure to reduced environmental water availability causes major alterations in plant metabolism. These great changes in metabolism ultimately lead to cell death and consequently to yield losses. Crop losses and crop yield losses of major crops such as rice, maize (corn), and wheat caused by these stresses represent a significant economic and political factor and contribute to food shortages in many parts of the world.

Another example of abiotic environmental stress is the reduced availability of one or more nutrients that need to be assimilated by the plants for growth and development. Because of the strong influence of nutrition utilization efficiency on plant yield and product quality, a huge amount of fertilizer is poured onto fields to optimize plant growth and quality. Productivity of plants is limited by three primary nutrients: phosphorous, potassium and nitrogen, which are usually the rate-limiting elements in plant growth. The major nutritional element required for plant growth is nitrogen (N). It is a constituent of numerous important compounds found in living cells, including amino acids, proteins (enzymes), nucleic acids, and chlorophyll. 1.5% to 2% of plant dry matter is nitrogen and approximately 16% of total plant protein. Thus, nitrogen availability is a major limiting factor in crop plant growth and production (Frink et al. (1999) Proc Natl Acad Sci USA 96(4): 1175-1180), and has a major impact on protein accumulation and amino acid composition. Therefore, of great interest are crop plants with an increased yield when grown under nitrogen-limiting conditions.

Plant biomass is yield for forage crops like alfalfa, silage corn and hay. Many proxies for yield have been used in grain crops. Chief amongst these are estimates of plant size. Plant size can be measured in many ways depending on species and developmental stage, but include total plant dry weight, above-ground dry weight, above-ground fresh weight, leaf area, stem volume, plant height, rosette diameter, leaf length, root length, root mass, tiller number and leaf number. Many species maintain a conservative ratio between the size of different parts of the plant at a given developmental stage. These allometric relationships are used to extrapolate from one of these measures of size to another (e.g. Tittonell et al. (2005) Agric Ecosys & Environ 105: 213). Plant size at an early developmental stage will typically correlate with plant size later in development. A larger plant with a greater leaf area can typically absorb more light and carbon dioxide than a smaller plant and therefore will likely gain a greater weight during the same period (Fasoula & Tollenaar (2005) Maydica 50:39). This is in addition to the potential continuation of the micro-environmental or genetic advantage that the plant had to achieve the larger size initially. There is a strong genetic component to plant size and growth rate (e.g. ter Steege et al. (2005) Plant Physiology 139:1078), and so for a range of diverse genotypes plant size under one environmental condition is likely to correlate with size under another (Hittalmani et al. (2003) Theoretical Applied Genetics 107:679). In this way a standard environment is used as a proxy for the diverse and dynamic environments encountered at different locations and times by crops in the field.

Developing stress tolerant plants is a strategy that has the potential to solve or mediate at least some aspects of yield loss (McKersie and Leshem (1994) Stress and stress coping in cultivated plants, Kluwer Academic Publishers). However, traditional breeding strategies to develop new lines of plants that exhibit resistance (tolerance) to these types of stresses are relatively slow and require specific resistant lines for crossing with the desired line. Limited germplasm resources for stress tolerance and incompatibility in crosses between distantly related plant species represent significant problems encountered in conventional breeding. Furthermore, such selective breeding techniques are typically labour intensive and result in plants that often contain heterogeneous genetic components that may not always result in the desirable trait being passed on from parent plants. Advances in molecular biology have allowed mankind to modify the germplasm of animals and plants. Genetic engineering of plants entails the isolation and manipulation of genetic material (typically in the form of DNA or RNA) and the subsequent introduction of that genetic material into a plant. Such technology has the capacity to deliver crops or plants having various improved economic, agronomic or horticultural traits.

SYT is a transcriptional co-activator that, in plants, forms a functional complex with transcription activators of the GRF (growth-regulating factor) family of proteins (Kim HJ, Kende H (2004) Proc Nat Acad Sc 101: 13374-9). SYT is called GIF for GRF-interacting factor in this paper, and AN3 for angustifolia3 in Horiguchi et al. (2005) Plant J 43: 68-78. The GRF transcription activators share structural domains (in the N-terminal region) with the SWI/SNF proteins of the chromatin-remodelling complexes in yeast (van der Knaap E et al., (2000) Plant Phys 122: 695-704). Transcriptional co-activators of these complexes are proposed to be involved in recruiting SWI/SNF complexes to enhancer and promoter regions to effect local chromatin remodelling (review Näär AM et al., (2001) Annu Rev Biochem 70: 475-501). The alteration in local chromatin structure modulates transcriptional activation. More precisely, SYT is proposed to interact with the plant SWI/SNF complex to affect transcriptional activation of GRF target gene(s) (Kim HJ, Kende H (2004) Proc Nat Acad Sc 101: 13374-9).

SYT belongs to a gene family of three members in *Arabidopsis.* The SYT polypeptide shares homology with the human SYT. The human SYT polypeptide was shown to be a transcriptional co-activator (Thaete et al. (1999) Hum Molec Genet 8: 585-591). Three domains characterize the mammalian SYT polypeptide:

(i) the N-terminal SNH (SYT N-terminal homology) domain, conserved in mammals, plants, nematodes and fish;
(ii) the C-terminal QPGY-rich domain, composed predominantly of glycine, proline, glutamine and tyrosine, occurring at variable intervals;
(iii) a methionine-rich (Met-rich) domain located between the two previous domains.

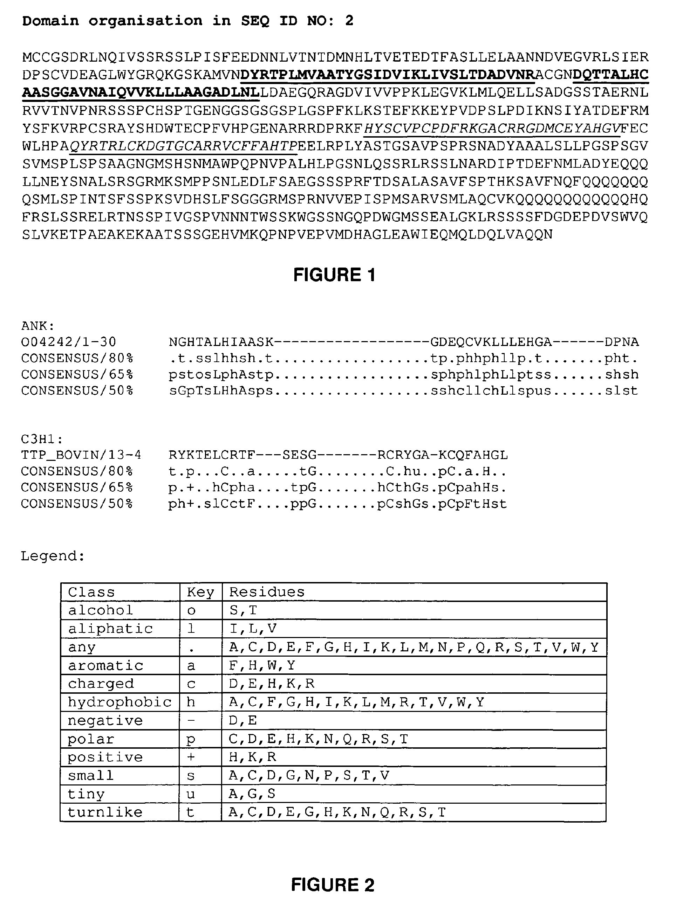

In plant SYT polypeptides, the SNH domain is well conserved. The C-terminal domain is rich in glycine and glutamine, but not in proline or tyrosine. It has therefore been named the QG-rich domain in contrast to the QPGY domain of mammals. As with mammalian SYT, a Met-rich domain may be identified N-terminally of the QG domain. The QG-rich domain may be taken to be substantially the C-terminal remainder of the polypeptide (minus the SHN domain); the Met-rich domain is typically comprised within the first half of the QG-rich (from the N-terminus to the C-terminus). A second Met-rich domain may precede the SNH domain in plant SYT polypeptides (see Fig 1).

A SYT loss-of function mutant and transgenic plants with reduced expression of SYT was reported to develop small and narrow leaves and petals, which have fewer cells (Kim HJ, Kende H (2004) Proc Nat Acad Sc 101: 13374-9).

Overexpression of AN3 in Arabidopsis thaliana resulted in plants with leaves that were 20-30% larger than those of the wild type (Horiguchi et al. (2005) Plant J 43: 68-78).

In Japanese patent application 2004-350553, a method for controlling the size of leaves in the horizontal direction is described, by controlling the expression of the AN3 gene.

cpFBPase

Harvest index, the ratio of seed yield to aboveground dry weight, is relatively stable under many environmental conditions and so a robust correlation between plant size and grain yield can often be obtained (e.g. Rebetzke et al 2002 Crop Science 42:739). These processes are intrinsically linked because the majority of grain biomass is dependent on current or stored photosynthetic productivity by the leaves and stem of the plant (Gardener et al 1985 Physiology of Crop Plants. Iowa State University Press, pp68-73). Therefore, selecting for plant size, even at early stages of development, has been used as an indicator for future potential yield (e.g. Tittonell et al 2005 Agric Ecosys & Environ 105: 213). When testing for the impact of genetic differences on stress tolerance, the ability to standardize soil properties, temperature, water and nutrient availability and light intensity is an intrinsic advantage of greenhouse or plant growth chamber environments compared to the field. However, artificial limitations on yield due to poor pollination due to the absence of wind or insects, or insufficient space for mature root or canopy growth, can restrict the use of these controlled environments for testing yield differences. Therefore, measurements of plant size in early development, under standardized conditions in a growth chamber or greenhouse, are standard practices to provide indication of potential genetic yield advantages.

Photosynthetic carbon metabolism in higher plants is an essential process for plant growth and crop yield. Carbohydrates are produced in higher plants by the fixation of atmospheric CO2 via the reductive pentose phosphate (Calvin) pathway. This process takes place in the chloroplast, and the newly synthesized triosephosphates can be kept in the stromal compartment for starch synthesis, or may be exported to the cytosol for sucrose formation. During photosynthesis, the newly synthesized carbohydrate is channelled to one or the other form depending on the needs of the plant and the environmental conditions.

The Calvin cycle is a complex pathway consisting of three phases of thirteen reactions catalyzed by eleven enzymes. One of the more important enzymes is chloroplastic fructose-1,6-bisphosphatase (cpFBPase), that catalyzes the irreversible conversion of fructose-1,6-bisphosphate to fructose-6-phosphate and Pi (inorganic P). The level of cpFBPase polypeptide in the chloroplast is very low compared to those of the other enzymes of the Calvin cycle.

The cpFBPase activity is regulated by the redox potential via the ferredoxin/thioredoxin system, which modulates the enzyme activity in response to light/dark conditions, and light-dependent changes in pH and $Mg^{2+}$ levels (Chiadmi et al. (1999) EMBO 18(23): 6809-6815). More specifically, the cpFBPase polypeptide is active in the light, and inactive in the dark, which takes place by a thioredoxin-mediated reduction-oxidation interplay between SH groups of the enzyme molecule and also via a light-induced rise in pH and Mg2+ concentration in the chloroplast stroma (Buchanan (1980) Annu Rev Plant Physiol 31: 341-374; Jacquot (1984) Bot Acta 103: 323-334).

Higher plant and algal cpFBPase polypeptides perform the same enzymatic step, but differ somewhat in the regulation of enzymatic activity. More specifically, algal cpFBPase polypeptides present a strict requirement for reduction to be active, but are less strict on reductant specificity, i.e., they can be activated by different plastidic thioredoxins (Huppe and Buchanan (1989) Z Naturforsch 44(5-6): 487-94).

In photosynthetic (autotrophic) cells in addition to the cpFBPase polypeptide, there is a second FBPase polypeptide (isoform) located in the cytosol (cyFBPase) and involved in sucrose synthesis and gluconeogenesis. The cyFBPase polypeptide presents very different regulatory properties as compared to the cpFBPase polypeptide: it is inhibited by excess substrate, displays an allosteric inhibition by AMP and fructose-2, 6-bisphophate, and presents a neutral pH optima. The cyFBPase polypeptide is found in heterotrophic systems (such as animal cells). An important difference between the cpFBPase polypeptides and the cyFBPase polypeptides is the presence in the former of an amino acid insertion that bears at least two conserved cysteine residues that are the targets of thioredoxin regulation.

Transgenic potato plants expressing a potato cpFBPase nucleic acid sequence under the control of a tuber-specific promoter were reported (Thorbjornsen et al. (2002) Planta 214: 616-624). The authors observed that the transgenic tubers did not differ from wild type tubers with respect to starch content, or the levels of neutral sugars and phosphorylated hexoses.

Two cyFBPase-encoding genes from cyanobacterium *Synechococcus* (FBPase/SBPase and FBPaseII) were operably linked to a tomato rbcS transit peptide-coding sequence for chloroplastic subcellular targeting of the chimeric proteins (Miyagawa et al. (2001) Nature Biotech 19: 965-969; Tamoi et al. (2006) Plant Cell Physiol 47(3): 380-390). The activities of FBPase/SBPase and FBPaseII polypeptides were not found to be regulated by redox conditions via the ferredoxin/thioredoxin system, since they lack the conserved cysteine residues. The tomato rbcS promoter was used to control the expression of both chimeric genes. Transgenic tobacco plants transformed with either chimeric construct were reported to grow significantly faster and larger than wild type plants under atmospheric conditions.

<u>SIK</u>

Plant breeders are often interested in improving specific aspects of yield depending on the crop or plant in question, and the part of that plant or crop which is of economic value. For example, for certain crops, or for certain end uses, a plant breeder may look specifically for improvements in plant biomass (weight) of one or more parts of a plant, which may include aboveground (harvestable) parts and/or (harvestable) parts below ground. This is particularly relevant where the aboveground parts or below ground parts of a plant are for consumption. For many crops, particularly cereals, it is an improvement in seed yield that is highly desirable. Increased seed yield may manifest itself in many ways, with each individual aspect of seed yield being of varying importance to a plant breeder depending on the crop or plant in question and its end use. For example, seed yield may be manifested as or result from a) an increase in seed biomass (total seed weight) which may be on an individual seed basis and/or per plant and/or per square meter; b) increased number of flowers per plant; c) increased number of (filled) seeds; d) increased seed filling rate (which is typically expressed as the ratio between the number of filled seeds divided by the total number of seeds; e) increased harvest index, which is typically expressed as a ratio of the yield of harvestable parts, such as seeds, divided by the total biomass; and f) increased thousand kernel weight (TKW), which is typically extrapolated from the number of filled seeds counted and their total weight.

An increase in seed yield may also be manifested as an increase in seed size and/or seed volume. Furthermore, an increase in seed yield may also manifest itself as an increase in seed area and/or seed length and/or seed width and/or seed perimeter. Increased yield may also result in modified architecture, or may occur because of modified architecture.

Taking corn as an example, a yield increase may be manifested as one or more of the following: increase in the number of plants per square meter, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per square meter, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

It would be of great advantage to a plant breeder to be able to be able to pick and choose the aspects of seed yield to be altered. It would be highly desirable to be able to pick off the shelf, so to speak, a gene suitable for altering a particular aspect, or component, of seed yield. For example an increase in the fill rate, combined with increased thousand kernel weight would be highly desirable for a crop such as corn. For rice and wheat a combination of increased fill rate, harvest index and increased thousand kernel weight would be highly desirable.

Published International patent Application, WO 02/074801, in the name of Genomine Inc., describes an AtSIK protein from *Arabidopsis thaliana* and a gene encoding said protein. It is mentioned that plants may be made resistant to osmotic stress by inhibiting expression of AtSIK, and that as a consequence the productivity of a plant may be increased. What is not mentioned however is which aspects of yield may be modified by inhibiting expression of AtSIK.

HD Zip

The study and genetic manipulation of plants has a long history that began even before the famed studies of Gregor Mendel. In perfecting this science, scientists have accomplished modification of particular traits in plants ranging from potato tubers having increased starch content to oilseed plants such as canola and sunflower having increased or altered fatty acid content. With the increased consumption and use of plant oils, the modification of seed oil content and seed oil levels has become increasingly widespread (e.g. Töpfer et al., 1995, Science 268: 681-686). Manipulation of biosynthetic pathways in transgenic plants provides a number of opportunities for molecular biologists and plant biochemists to affect plant metabolism giving rise to the production of specific higher-value products. The seed oil production or composition has been altered in numerous traditional oilseed plants such as soybean (U.S. Patent No. 5,955,650), canola (U.S. Patent No. 5,955,650), sunflower (U.S. Patent No. 6,084,164), rapeseed (Töpfer et al., 1995, Science 268: 68 1 -686), and non-traditional oil seed plants such as tobacco (Cahoon et al., 1992, Proc. Natl. Acad. Sci. USA89: 11184-11188).

Plant seed oils comprise both neutral and polar lipids (See Table 1). The neutral lipids contain primarily triacylglycerol, which is the main storage lipid that accumulates in oil bodies in seeds. The polar lipids are mainly found in the various membranes of the seed cells, e.g. the microsomal, plastidial, and mitochondrial membranes, and the cell membrane. The neutral and polar lipids contain several common fatty acids (See Table 2) and a range of less common fatty acids. The fatty acid composition of membrane lipids is highly regulated and only a select number of fatty acids are found in membrane lipids. On the other hand, a large number of unusual fatty acids can be incorporated into the neutral storage lipids in seeds of many plant species (Van de Loo F.J. et al., 1993, Unusual Fatty Acids in Lipid Metabolism in Plants pp. 91-126, editor TS Moore Jr. CRC Press; Millar et al., 2000, Trends Plant Sci. 5:95-101).

**Table 1:** plant lipid classes

| | |
|---|---|
| Neutral lipids | Triacylglycerol (TAG) |
| | Diacylglycerol (DAG) |
| | Monoacylglycerol (MAG) |
| Polar lipids | Monogalactosyldiacylglycerol (MGDG) |
| | Digalactosyldiacylglycerol (DGDG) |
| | Phosphatidylglycerol (PG) |
| | Phosphatidylcholine (PC) |
| | Phosphatidylethanolamine (PE) |
| | Phosphatidylinositol (PI) |
| | Phosphatidylserine (PS) |
| | Sulfoquinovosyldiacylglycerol |

**Table 2:** common plant fatty acids

| | |
|---|---|
| 16:0 | palmitic acid |
| 16:1 | palmitoleic acid |
| 16:3 | hiragonic acid |
| 18:0 | stearic acid |
| 18:1 | oleic acid |
| 18:2 | linoleic acid |
| 18:3 | linolenic acid |
| γ-18:3 | gamma-linoleic acid* |
| 20:0 | arachidic acid |

(continued)

| | |
|---|---|
| 20:1 | eicosenoic acid |
| 22:6 | docosahexanoic acid (DHA)* |
| 20:2 | eicosadienoic acid |
| 20:4 | arachidonic acid (AA)* |
| 20:5 | eicosapentaenoic acid (EPA)* |
| 22:1 | erucic acid |

In Table 2, the fatty acids denoted with an asterisk do not normally occur in plant seed oils, but their production in transgenic plant seed oil is of importance in plant biotechnology.

The primary sites of fatty acid biosynthesis in plants are the plastids. Fatty acid biosynthesis begins with the conversion of acetyl-CoA to malonyl-CoA by acetyl-CoA carboxylase (ACCase). The malonyl moiety is then transferred to an acyl carrier protein (ACP) by the malonyl-CoA:ACP transacylase. The enzyme beta-ketoacyl-ACP-synthase III (KAS III) catalyzes the initial condensation reaction of fatty acid biosynthesis, in which after decarboxylation of malonyl-ACP, the resulting carbanion is transferred to acetyl-CoA by a nucleophilic attack of the carbonyl-carbon, resulting in the formation of 3-ketobutyryl-ACP. The reaction cycle is completed by a reduction, a dehydration and again a reduction yielding butyric acid. This reaction cycle is repeated (with KAS I or KAS II catalyzing the condensation reaction) until the acyl-group reach a chain length of usually 16 to 18 carbon atoms. These acyl-ACPs can be desaturated by the stearoyl-ACP desaturase, used as substrates for plastidial acyltransferases in the formation of lipids through what has been referred to as the prokaryotic pathway, or exported to the cytosol after cleavage from ACP through the action of thioesterases. In the cytosol they enter the acyl-CoA pool and can be used for the synthesis of lipids through what has been referred to as the eukaryotic pathway in the endoplasmic reticulum.

Lipid synthesis through both the prokaryotic and eukaryotic pathways occurs through the successive acylation of glycerol-3-phosphate, catalyzed by glycerol-3-phosphate acyltransferases (GPAT) and lysophosphatidic acid acyltransferase (LPAAT) (Browse et al., 1986, Biochemical J. 235:25-31; Ohlrogge & Browse, 1995,5 Plant Ce11 7:957-970). The resulting phosphatidic acid (PA) is the precursor for other polar membrane lipids such as monogalactosyldiacylglycerol (MGD), digalactosyldiacylglycerol (DGD), phosphatidylglycerol (PG) and sulfoquinovosyldiacylglycerol (SQD) in the plastid and phosphatidylcholine (PC), phosphatidylethanolamine (PE), phosphatidylinositol (PI) and phosphatidylserine (PS) in the endoplasmic reticulum. The polar lipids are also the sites of further modification of the acyl-chain such as desaturation, acetylenation, and hydroxylation. In the endoplasmic reticulum, PA is also the intermediate in the biosynthesis of triacylglycerol (TAG), the major component of neutral lipids and hence of seed oil. Furthermore, alternative pathways for the biosynthesis of TAGS can exist (i.e. transacylation through the action of phosphatidylcholine:diacylglycerol acyltransferase) (Voelker, 1996, Genetic Engineering ed.:Setlow 1 8: 111-113; Shanklin & Cahoon, 1998, Annu. Rev. Plant Physiol. Plant Mol. Biol. 49:611-641; Frentzen, 1998, Lipids 100:161-166; Millar et al., 2000, Trends Plant Sci. 5:95-101). The reverse reaction, the breakdown of triacylglycerol to diacylglycerol and fatty acids is catalyzed by lipases. Such a breakdown can be seen towards the end of seed development resulting in a certain reduction in seed oil. (Buchanan et al., 2000).

Storage lipids in seeds are synthesized from carbohydrate-derived precursors. Plants have a complete glycolytic pathway in the cytosol (Plaxton, 1996, Annu. Rev. Plant Physiol. Plant Mol. Biol. 47: 185-214), and it has been shown that a complete pathway also exists in the plastids of rapeseed (Kang & Rawsthorne, 1994, Plant J. 6:795-805). Sucrose is the primary source of carbon and energy, transported from the leaves into the developing seeds. During the storage phase of seeds, sucrose is converted in the cytosol to provide the metabolic precursors glucose-6-phosphate and pyruvate. These are transported into the plastids and converted into acetyl-CoA that serves as the primary precursor for the synthesis of fatty acids. Acetyl-CoA in the plastids is the central precursor for lipid biosynthesis. Acetyl-CoA can be formed in the plastids by different reactions, and the exact contribution of each reaction is still being debated (Ohlrogge & Browse, 1995, Plant Ce11 7:957-970). It is accepted, however, that a large part of the acetyl-CoA is derived from glucose-6-phospate and pyruvate that are imported from the cytoplasm into the plastids. Sucrose is produced in the source organs (leaves, or anywhere that photosynthesis occurs) and is transported to the developing seeds, also termed sink organs. In the developing seeds, sucrose is the precursor for all the storage compounds, i.e. starch, lipids, and partly the seed storage proteins. Therefore, it is clear that carbohydrate metabolism in which sucrose plays a central role is very important to the accumulation of seed storage compounds.

Although lipid and fatty acid content of seed oil can be modified by the traditional methods of plant breeding, the advent of recombinant DNA technology has allowed for easier manipulation of the seed oil content of a plant, and in some cases, has allowed for the alteration of seed oils in ways that could not be accomplished by breeding alone (See, e.g., Töpfer et al., 1995, Science 268:681-686). For example, introduction of a $\Delta^{12}$-hydroxylase nucleic acid sequence into transgenic tobacco resulted in the formation of a novel fatty acid, ricinoleic acid, into the tobacco seed oil (Van de Loo et al., 1995, Proc. Natl. Acad. Sci USA 92:6743-6747). Tobacco plants have also been engineered to produce low levels of petroselinic acid by the introduction and expression of an acyl-ACP desaturase from coriander (Cahoon et al., 1992, Proc. Natl. Acad. Sci USA 89: 11 184-1 1 188).

The modification of seed oil content in plants has significant medical, nutritional, and economic ramifications. With regard to the medical ramifications, the long chain fatty acids (C18 and longer) found in many seed oils have been linked to reductions in hypercholesterolemia and other clinical disorders related to coronary heart disease (Brenner, 1976, Adv. Exp. Med. Biol. 83:85-101). Therefore, consumption of a plant having increased levels of these types of fatty acids may reduce the risk of heart disease. Enhanced levels of seed oil content are also useful in increasing the large-scale production of seed oils and thereby reducing the cost of these oils.

As mentioned earlier, several desaturase nucleic acids such as the $\Delta^6$-desaturase nucleic acid, $\Delta^{12}$-desaturase nucleic acid and acyl-ACP desaturase nucleic acid have been cloned and demonstrated to encode enzymes required for fatty acid synthesis in various plant species. Oleosin nucleic acid sequences from species such as Brassica, soybean, carrot, pine, and *Arabidopsis thaliana* have also been cloned and determined to encode proteins associated with the phospholipid monolayer membrane of oil bodies in those plants.

It has now been found that nucleic acid sequences encoding Class II homeodomain-leucine zipper (HD-Zip) transcription factors are useful in modifying the content of storage compounds in seeds.

Transcription factors are usually defined as proteins that show sequence-specific DNA binding and that are capable of activating and/or repressing transcription. The *Arabidopsis* genome codes for at least 1533 transcriptional regulators, which account for ~5.9% of its estimated total number of genes. About 45% of these transcription factors are reported to be from families specific to plants (Riechmann et al., 2000 (Science Vol. 290, 2105-2109)). One example of such a plant-specific family of transcription factors is the family of HD-Zip transcription factors.

Homeobox genes are transcription factors present in all eukaryotes and constitute a gene family of at least 89 members in *Arabidopsis thaliana.* They are characterized by the presence of a homeodomain, which usually consists of 60 conserved amino acid residues that form a helix-loop-helix-turn-helix structure that binds DNA. This DNA binding site is usually pseudopalindromic. Homeobox genes play crucial and diverse roles in many aspects of development, including the early development of animal embryos, the specification of cell types in yeast, and the initiation and maintenance of the shoot apical meristem in flowering plants (Sakakibara et al. Mol. Biol. Evol. 18(4): 491-502, 2001).

Numerous angiosperm homeobox genes have been isolated and sorted into seven distinct groups based on their amino acid similarities. These include the KNOX, BELL, HD-PHD-finger, HAT1, HAT 2, GL2, and ATHB8 groups. Genes in the latter four groups also encode a leucine zipper motif adjacent to the C-terminus of the homeodomain and collectively form the homeodomain-leucine zipper (HD-Zip) gene family. Aso et al., Mol. Biol. Evol. 16(4):544-552, 1999. Of the at least 89 members comprising the homeobox gene family in *Arabidopsis thaliana,* at least 47 comprise both a homeodomain and a leucine zipper. Although the combination of a homeodomain and a leucine zipper motif is unique to plants, it has also been encountered in moss in addition to vascular plants (Sakakibara et al. (2001) Mol Biol Evol 18 (4): 491-502).

Homeodomain leucine zipper (HD-Zip) proteins constitute a family of transcription factors characterized by the presence of a DNA-binding domain (HD) and an adjacent leucine zipper (Zip) motif. The leucine zipper, adjacent to the C-terminal end of the homeodomain, consists of several heptad repeats (at least four) in which a leucine (occasionally a valine or an isoleucine) typically appears every seventh amino acid. The leucine zipper is important for protein dimerisation. This dimerisation is a prerequisite for DNA binding (Sessa et al. (1993) EMBO J 12(9): 3507-3517), and may proceed between two identical HD-Zip proteins (homodimer) or between two different HD-Zip proteins (heterodimer).

The group of angiosperm homeobox genes HAT1, HAT2, GL2, and ATHB8 groups have been renamed the HD-Zip I-IV subfamilies. The combination of a homeodomain and a leucine zipper motif is unique to higher plants, suggesting that the HD-Zip genes may be involved in the regulation of developmental processes specific to plants. Aso et al., Mol. Biol. Evol. 16(4):544-552, 1999. The functions of the HD-Zip genes are diverse among the different subfamilies. HD-Zip I and II genes are likely involved in signal transduction networks of light, dehydration-induced ABA, or auxin. These signal transduction networks are related to the general growth regulation of plants. The overexpression of sense or antisense HD-Zip I or II mRNA usually alters growth rate and development. Most members of the HD-Zip III subfamily play roles in cell differentiation in the stele. HD-Zip IV genes are related to the differentiation of the outermost cell layer. Sakakibara et al. Mol. Biol. Evol. 18(4): 491-502, 2001.

Different HD-Zip proteins have been shown to either activate or repress transcription. In *Arabidopsis,* the class I HD-Zip ATHB1, -5, -6, and -16 were shown to act as transcriptional activators in transient expression assays on *Arabidopsis* leaves using a reporter gene, luciferase (Henriksson et al. (2005) Plant Phys 139: 509-518). Two rice class I HDZip proteins, Oshox4 and Oshox5, acted as activators in transient expression assays on rice cell suspension cultures using another reporter gene (glucuronidase; Meijer et al. (2000) Mol Gen Genet 263:12-21). In contrast, two rice class II HD-Zip proteins, Oshox1 and Oshox3, acted as transcriptional repressors in the same experiments (Meijer et al. (1997) Plant J 11: 263-276; Meijer *et al.* (2000) supra).

The Class II HD-Zip gene from *Arabidopsis thaliana,* HAT4, also known as ATHB-2, has been reported to act as a regulator of shade-avoidance responses (Morelli and Ruberti TIPS Vol. 7 No. 9, September 2002).

SYB1

Ran is a small signalling GTPase (GTP binding protein) in animal cells, which is involved in nucleocytoplasmic transport. Nucleocytoplasmic transport has been studied mainly in animal systems. Several Ran binding proteins are known, among them RanBP2, also known as Nup358. RanBP2 is postulated to associate on the cytoplasmic side with proteins of the Nuclear Pore Complex and putatively functions as a SUMO E3 ligase, although its structure is not of a typical E3 ligase. SUMOs (small ubiquitin-related modifiers) are eukaryotic proteins that are covalently ligated to other proteins, thereby regulating a number of cellular processes. In association with Ubc9 (which acts an E2 conjugating protein), RanBP2 labels substrates linked to nuclear import receptors with SUMO-1, in a similar way as ubiquitination of proteins. The SUMOylated substrate is then imported through the nuclear pore into the nucleus. Examples of imported proteins upon SUMOylation include NFX1-p15, a chaperone in mRNA export and the histone deacetylase HDAC-4. SUMO modification and hence RanBP2, is also implicated to play a role in gene expression, in cell cycle (during nuclear envelope breakdown), and in the formation of subcellular structures such as promyelocytic leukemia bodies.

Most of the studies were performed in model animal systems and little is known about the corresponding proteins and processes in plants. Proteins related to RanBP2, identified in *Arabidopsis,* share the zinc finger domains but are otherwise different in structure.

**Summary**

Surprisingly, it has now been found that modulating expression of a nucleic acid encoding a GRP polypeptide gives plants having improved characteristics relative to control plants.

In one aspect of the present invention, it has now been found that modulating expression in a plant of a nucleic acid encoding the AZ polypeptide from *Arabidopsis* (AtAZ) or a homologue thereof gives plants having increased yield relative to control plants. According to one embodiment of the present invention, there is provided a method for increasing plant yield, comprising modulating expression in a plant of a nucleic acid encoding the AZ polypeptide or a homologue thereof. Advantageously, performance of the methods according to the present invention results in plants having increased yield, particularly seed yield, relative to corresponding wild type plants. The present invention also provides nucleic acid sequences and constructs useful in performing such methods.

In another aspect of the present invention, it has now been found that modulating expression in a plant of a nucleic acid sequence encoding a SYT polypeptide gives plants having increased yield under abiotic stress relative to control plants. Therefore, the invention also provides a method for increasing plant yield under abiotic stress relative to control plants, comprising modulating expression in a plant of a nucleic acid sequence encoding a SYT polypeptide. The present invention also provides nucleic acid sequences and constructs useful in performing such methods.

In still another aspect of the present invention, it has now been found that increasing expression in aboveground parts of a plant of a nucleic acid sequence encoding a chloroplastic fructose-1,6-bisphosphatase (cpFBPase) polypeptide increases plant yield relative to control plants. Therefore, according to the present invention, there is provided a method for increasing plant yield relative to control plants, comprising increasing expression in aboveground parts of a plant of a nucleic acid sequence encoding a cpFBPase polypeptide. The present invention also provides nucleic acid sequences and constructs useful in performing such methods.

In yet another aspect of the present invention, it has now been found that modulating expression in a plant of a SIK nucleic acid and/or a SIK polypeptide gives plants having various improved yield-related traits relative to control plants, wherein overexpression of a SIK-encoding nucleic acid in a plant gives increased number of flowers per plant relative to control plants, and wherein the reduction or substantial elimination of a SIK nucleic acid gives increased thousand kernel weight, increased harvest index and increased fill rate relative to corresponding wild type plants. The present invention also provides nucleic acid sequences and constructs useful in performing such methods.

In a further aspect of the present invention, it has now been found that nucleic acids encoding Class II HD-Zip transcription factors are useful in modifying the content of storage compounds in seeds. The present invention therefore provides a method for modifying the content of storage compounds in seeds relative to control plants by modulating expression in a plant of a nucleic acid encoding a Class II HD-Zip transcription factor. The present invention also provides nucleic acid sequences and constructs useful in performing such methods. The invention further provides seeds having a modified content of storage compounds relative to control plants, which seeds have modulated expression of a nucleic acid encoding a Class II HD-Zip transcription factor. The present invention provides a method for modifying the content of storage compounds in seeds relative to control plants, comprising modulating expression in a plant of a nucleic acid encoding a Class II HD-Zip transcription factor.

In another aspect of the present invention, it has now been found that modulating expression in a plant of a nucleic acid encoding a SYB1 polypeptide gives plants having enhanced yield-related traits relative to control plants. This yield increase was surprisingly observed when the plants were cultivated under conditions without stress (non-stress conditions). The present invention therefore also provides a method for enhancing yield-related traits in plants relative to control plants, comprising modulating expression in a plant of a nucleic acid encoding a SYB1 polypeptide. The present invention also provides nucleic acid sequences and constructs useful in performing such methods.

**Definitions**

Polypeptide(s)/Protein(s)

The terms "polypeptide" and "protein" are used interchangeably herein and refer to amino acids in a polymeric form of any length, linked together by peptide bonds.

Polynucleotide(s)/Nucleic acid(s)/Nucleic acid seauence(s)/nucleotide sequence(s)

The terms "polynucleotide(s)", "nucleic acid sequence(s)", "nucleotide sequence(s)", "nucleic acid(s)", "nucleic acid molecule" are used interchangeably herein and refer to nucleotides, either ribonucleotides or deoxyribonucleotides or a combination of both, in a polymeric unbranched form of any length.

Control plant(s)

The choice of suitable control plants is a routine part of an experimental setup and may include corresponding wild type plants or corresponding plants without the gene of interest. The control plant is typically of the same plant species or even of the same variety as the plant to be assessed. The control plant may also be a nullizygote of the plant to be assessed. Nullizygotes are individuals missing the transgene by segregation. A "control plant" as used herein refers not only to whole plants, but also to plant parts, including seeds and seed parts.

Homoloaue(s)

"Homologues" of a protein encompass peptides, oligopeptides, polypeptides, proteins and enzymes having amino acid substitutions, deletions and/or insertions relative to the unmodified protein in question and having similar biological and functional activity as the unmodified protein from which they are derived.

A deletion refers to removal of one or more amino acids from a protein.

An insertion refers to one or more amino acid residues being introduced into a predetermined site in a protein. Insertions may comprise N-terminal and/or C-terminal fusions as well as intra-sequence insertions of single or multiple amino acids. Generally, insertions within the amino acid sequence will be smaller than N- or C-terminal fusions, of the order of about 1 to 10 residues. Examples of N- or C-terminal fusion proteins or peptides include the binding domain or activation domain of a transcriptional activator as used in the yeast two-hybrid system, phage coat proteins, (histidine)-6-tag, glutathione S-transferase-tag, protein A, maltose-binding protein, dihydrofolate reductase, Tag•100 epitope, c-myc epitope, FLAG®-epitope, lacZ, CMP (calmodulin-binding peptide), HA epitope, protein C epitope and VSV epitope.

A substitution refers to replacement of amino acids of the protein with other amino acids having similar properties (such as similar hydrophobicity, hydrophilicity, antigenicity, propensity to form or break $\alpha$-helical structures or $\beta$-sheet structures). Amino acid substitutions are typically of single residues, but may be clustered depending upon functional constraints placed upon the polypeptide; insertions will usually be of the order of about 1 to 10 amino acid residues. The amino acid substitutions are preferably conservative amino acid substitutions. Conservative substitution tables are well known in the art (see for example Creighton (1984) Proteins. W.H. Freeman and Company (Eds) and Table 3 below).

**Table 3:** Examples of conserved amino acid substitutions

| Residue | Conservative Substitutions | Residue | Conservative Substitutions |
|---|---|---|---|
| Ala | Ser | Leu | Ile; Val |
| Arg | Lys | Lys | Arg; Gln |
| Asn | Gln; His | Met | Leu; Ile |
| Asp | Glu | Phe | Met; Leu; Tyr |
| Gln | Asn | Ser | Thr; Gly |

(continued)

| Residue | Conservative Substitutions | Residue | Conservative Substitutions |
|---|---|---|---|
| Cys | Ser | Thr | Ser; Val |
| Glu | Asp | Trp | Tyr |
| Gly | Pro | Tyr | Trp; Phe |
| His | Asn; Gln | Val | Ile; Leu |
| Ile | Leu, Val | | |

Amino acid substitutions, deletions and/or insertions may readily be made using peptide synthetic techniques well known in the art, such as solid phase peptide synthesis and the like, or by recombinant DNA manipulation. Methods for the manipulation of DNA sequences to produce substitution, insertion or deletion variants of a protein are well known in the art. For example, techniques for making substitution mutations at predetermined sites in DNA are well known to those skilled in the art and include M13 mutagenesis, T7-Gen in vitro mutagenesis (USB, Cleveland, OH), QuickChange Site Directed mutagenesis (Stratagene, San Diego, CA), PCR-mediated site-directed mutagenesis or other site-directed mutagenesis protocols.

Derivatives

"Derivatives" include peptides, oligopeptides, polypeptides which may, compared to the amino acid sequence of the naturally-occurring form of the protein, such as the protein of interest, comprise substitutions of amino acids with non-naturally occurring amino acid residues, or additions of non-naturally occurring amino acid residues. "Derivatives" of a protein also encompass peptides, oligopeptides, polypeptides which comprise naturally occurring altered (glycosylated, acylated, prenylated, phosphorylated, myristoylated, sulphated etc.) or non-naturally altered amino acid residues compared to the amino acid sequence of a naturally-occurring form of the polypeptide. A derivative may also comprise one or more non-amino acid substituents or additions compared to the amino acid sequence from which it is derived, for example a reporter molecule or other ligand, covalently or non-covalently bound to the amino acid sequence, such as a reporter molecule which is bound to facilitate its detection, and non-naturally occurring amino acid residues relative to the amino acid sequence of a naturally-occurring protein. Furthermore, "derivatives" also include fusions of the naturally-occurring form of the protein with tagging peptides such as FLAG, HIS6 or thioredoxin (for a review of tagging peptides, see Terpe, Appl. Microbiol. Biotechnol. 60, 523-533, 2003).

Orthologue(s)/Paralohue(s)

Orthologues and paralogues encompass evolutionary concepts used to describe the ancestral relationships of genes. Paralogues are genes within the same species that have originated through duplication of an ancestral gene; orthologues are genes from different organisms that have originated through speciation, and are also derived from a common ancestral gene.

Domain

The term "domain" refers to a set of amino acids conserved at specific positions along an alignment of sequences of evolutionarily related proteins. While amino acids at other positions can vary between homologues, amino acids that are highly conserved at specific positions indicate amino acids that are likely essential in the structure, stability or function of a protein. Identified by their high degree of conservation in aligned sequences of a family of protein homologues, they can be used as identifiers to determine if any polypeptide in question belongs to a previously identified polypeptide family.

Motif/Consensus sequence/Signature

The term "motif' or "consensus sequence" or "signature" refers to a short conserved region in the sequence of evolutionarily related proteins. Motifs are frequently highly conserved parts of domains, but may also include only part of the domain, or be located outside of conserved domain (if all of the amino acids of the motif fall outside of a defined domain).

Hybridisation

The term "hybridisation" as defined herein is a process wherein substantially homologous complementary nucleotide sequences anneal to each other. The hybridisation process can occur entirely in solution, i.e. both complementary nucleic acids are in solution. The hybridisation process can also occur with one of the complementary nucleic acids immobilised to a matrix such as magnetic beads, Sepharose beads or any other resin. The hybridisation process can furthermore occur with one of the complementary nucleic acids immobilised to a solid support such as a nitrocellulose or nylon membrane or immobilised by e.g. photolithography to, for example, a siliceous glass support (the latter known as nucleic acid arrays or microarrays or as nucleic acid chips). In order to allow hybridisation to occur, the nucleic acid molecules are generally thermally or chemically denatured to melt a double strand into two single strands and/or to remove hairpins or other secondary structures from single stranded nucleic acids.

The term "stringency" refers to the conditions under which a hybridisation takes place. The stringency of hybridisation is influenced by conditions such as temperature, salt concentration, ionic strength and hybridisation buffer composition. Generally, low stringency conditions are selected to be about 30˚C lower than the thermal melting point ($T_m$) for the specific sequence at a defined ionic strength and pH. Medium stringency conditions are when the temperature is 20˚C below $T_m$, and high stringency conditions are when the temperature is 10˚C below $T_m$. High stringency hybridisation conditions are typically used for isolating hybridising sequences that have high sequence similarity to the target nucleic acid sequence. However, nucleic acids may deviate in sequence and still encode a substantially identical polypeptide, due to the degeneracy of the genetic code. Therefore medium stringency hybridisation conditions may sometimes be needed to identify such nucleic acid molecules.

The Tm is the temperature under defined ionic strength and pH, at which 50% of the target sequence hybridises to a perfectly matched probe. The $T_m$ is dependent upon the solution conditions and the base composition and length of the probe. For example, longer sequences hybridise specifically at higher temperatures. The maximum rate of hybridisation is obtained from about 16˚C up to 32˚C below $T_m$. The presence of monovalent cations in the hybridisation solution reduce the electrostatic repulsion between the two nucleic acid strands thereby promoting hybrid formation; this effect is visible for sodium concentrations of up to 0.4M (for higher concentrations, this effect may be ignored). Formamide reduces the melting temperature of DNA-DNA and DNA-RNA duplexes with 0.6 to 0.7˚C for each percent formamide, and addition of 50% formamide allows hybridisation to be performed at 30 to 45˚C, though the rate of hybridisation will be lowered. Base pair mismatches reduce the hybridisation rate and the thermal stability of the duplexes. On average and for large probes, the Tm decreases about 1˚C per % base mismatch. The Tm may be calculated using the following equations, depending on the types of hybrids:

1) DNA-DNA hybrids (Meinkoth and Wahl, Anal. Biochem., 138: 267-284, 1984):

$$T_m = 81.5°C + 16.6 x \log_{10}[Na^+]^a + 0.41 x \%[G/C^b] - 500 x [L^c]^{-1} - 0.61 x \% \text{ formamide}$$

2) DNA-RNA or RNA-RNA hybrids:

$$Tm = 79.8 + 18.5 (\log_{10}[Na^+]^a) + 0.58 (\%G/C^b) + 11.8 (\%G/C^b)^2 - 820/L^c$$

3) oligo-DNA or oligo-RNA[d] hybrids:

For <20 nucleotides: $T_m = 2 (I_n)$
For 20-35 nucleotides: $T_m = 22 + 1.46 (I_n)$

[a] or for other monovalent cation, but only accurate in the 0.01-0.4 M range.
[b] only accurate for %GC in the 30% to 75% range.
[c] L = length of duplex in base pairs.
[d] oligo, oligonucleotide; $I_n$, = effective length of primer = 2×(no. of G/C)+(no. of A/T).

Non-specific binding may be controlled using any one of a number of known techniques such as, for example, blocking the membrane with protein containing solutions, additions of heterologous RNA, DNA, and SDS to the hybridisation buffer, and treatment with Rnase. For non-homologous probes, a series of hybridizations may be performed by varying one of (i) progressively lowering the annealing temperature (for example from 68˚C to 42˚C) or (ii) progressively lowering the formamide concentration (for example from 50% to 0%). The skilled artisan is aware of various parameters which may be altered during hybridisation and which will either maintain or change the stringency conditions.

Besides the hybridisation conditions, specificity of hybridisation typically also depends on the function of post-hybridisation washes. To remove background resulting from non-specific hybridisation, samples are washed with dilute salt solutions. Critical factors of such washes include the ionic strength and temperature of the final wash solution: the lower the salt concentration and the higher the wash temperature, the higher the stringency of the wash. Wash conditions are typically performed at or below hybridisation stringency. A positive hybridisation gives a signal that is at least twice of that of the background. Generally, suitable stringent conditions for nucleic acid hybridisation assays or gene amplification detection procedures are as set forth above. More or less stringent conditions may also be selected. The skilled artisan is aware of various parameters which may be altered during washing and which will either maintain or change the stringency conditions.

For example, typical high stringency hybridisation conditions for DNA hybrids longer than 50 nucleotides encompass hybridisation at 65°C in 1x SSC or at 42°C in 1x SSC and 50% formamide, followed by washing at 65°C in 0.3x SSC. Examples of medium stringency hybridisation conditions for DNA hybrids longer than 50 nucleotides encompass hybridisation at 50°C in 4x SSC or at 40°C in 6x SSC and 50% formamide, followed by washing at 50°C in 2x SSC. The length of the hybrid is the anticipated length for the hybridising nucleic acid. When nucleic acids of known sequence are hybridised, the hybrid length may be determined by aligning the sequences and identifying the conserved regions described herein. 1×SSC is 0.15M NaCl and 15mM sodium citrate; the hybridisation solution and wash solutions may additionally include 5x Denhardt's reagent, 0.5-1.0% SDS, 100 μg/ml denatured, fragmented salmon sperm DNA, 0.5% sodium pyrophosphate.

For the purposes of defining the level of stringency, reference can be made to Sambrook et al. (2001) Molecular Cloning: a laboratory manual, 3rd Edition, Cold Spring Harbor Laboratory Press, CSH, New York or to Current Protocols in Molecular Biology, John Wiley & Sons, N.Y. (1989 and yearly updates).

<u>Splice variant</u>

The term "splice variant" as used herein encompasses variants of a nucleic acid sequence in which selected introns and/or exons have been excised, replaced, displaced or added, or in which introns have been shortened or lengthened. Such variants will be ones in which the biological activity of the protein is substantially retained; this may be achieved by selectively retaining functional segments of the protein. Such splice variants may be found in nature or may be manmade. Methods for predicting and isolating such splice variants are well known in the art (see for example Foissac and Schiex (2005) BMC Bioinformatics 6: 25).

<u>Allelic variant</u>

Alleles or allelic variants are alternative forms of a given gene, located at the same chromosomal position. Allelic variants encompass Single Nucleotide Polymorphisms (SNPs), as well as Small Insertion/Deletion Polymorphisms (INDELs). The size of INDELs is usually less than 100 bp. SNPs and INDELs form the largest set of sequence variants in naturally occurring polymorphic strains of most organisms.

<u>Gene shuffling/Directed evolution</u>

Gene shuffling or directed evolution consists of iterations of DNA shuffling followed by appropriate screening and/or selection to generate variants of nucleic acids or portions thereof encoding proteins having a modified biological activity (Castle et al., (2004) Science 304(5674): 1151-4; US patents 5,811,238 and 6,395,547).

<u>Regulatory element/Control sequence/Promoter</u>

The terms "regulatory element", "control sequence" and "promoter" are all used interchangeably herein and are to be taken in a broad context to refer to regulatory nucleic acid sequences capable of effecting expression of the sequences to which they are ligated. The term "promoter" typically refers to a nucleic acid control sequence located upstream from the transcriptional start of a gene and which is involved in recognising and binding of RNA polymerase and other proteins, thereby directing transcription of an operably linked nucleic acid. Encompassed by the aforementioned terms are transcriptional regulatory sequences derived from a classical eukaryotic genomic gene (including the TATA box which is required for accurate transcription initiation, with or without a CCAAT box sequence) and additional regulatory elements (i.e. upstream activating sequences, enhancers and silencers) which alter gene expression in response to developmental and/or external stimuli, or in a tissue-specific manner. Also included within the term is a transcriptional regulatory sequence of a classical prokaryotic gene, in which case it may include a -35 box sequence and/or -10 box transcriptional regulatory sequences. The term "regulatory element" also encompasses a synthetic fusion molecule or derivative that confers, activates or enhances expression of a nucleic acid molecule in a cell, tissue or organ.

A "plant promoter" comprises regulatory elements, which mediate the expression of a coding sequence segment in plant cells. Accordingly, a plant promoter need not be of plant origin, but may originate from viruses or micro-organisms, for example from viruses which attack plant cells. The "plant promoter" can also originate from a plant cell, e.g. from the plant which is transformed with the nucleic acid sequence to be expressed in the inventive process and described herein. This also applies to other "plant" regulatory signals, such as "plant" terminators. The promoters upstream of the nucleotide sequences useful in the methods of the present invention can be modified by one or more nucleotide substitution(s), insertion(s) and/or deletion(s) without interfering with the functionality or activity of either the promoters, the open reading frame (ORF) or the 3'-regulatory region such as terminators or other 3' regulatory regions which are located away from the ORF. It is furthermore possible that the activity of the promoters is increased by modification of their sequence, or that they are replaced completely by more active promoters, even promoters from heterologous organisms. For expression in plants, the nucleic acid molecule must, as described above, be linked operably to or comprise a suitable promoter which expresses the gene at the right point in time and with the required spatial expression pattern.

For the identification of functionally equivalent promoters, the promoter strength and/or expression pattern of a candidate promoter may be analysed for example by operably linking the promoter to a reporter gene and assaying the expression level and pattern of the reporter gene in various tissues of the plant. Suitable well-known reporter genes include for example beta-glucuronidase or beta-galactosidase. The promoter activity is assayed by measuring the enzymatic activity of the beta-glucuronidase or beta-galactosidase. The promoter strength and/or expression pattern may then be compared to that of a reference promoter (such as the one used in the methods of the present invention). Alternatively, promoter strength may be assayed by quantifying mRNA levels or by comparing mRNA levels of the nucleic acid used in the methods of the present invention, with mRNA levels of housekeeping genes such as 18S rRNA, using methods known in the art, such as Northern blotting with densitometric analysis of autoradiograms, quantitative real-time PCR or RT-PCR (Heid et al., 1996 Genome Methods 6: 986-994). Generally by "weak promoter" is intended a promoter that drives expression of a coding sequence at a low level. By "low level" is intended at levels of about 1/10,000 transcripts to about 1/100,000 transcripts, to about 1/500,0000 transcripts per cell. Conversely, a "strong promoter" drives expression of a coding sequence at high level, or at about 1/10 transcripts to about 1/100 transcripts to about 1/1000 transcripts per cell.

Operably linked

The term "operably linked" as used herein refers to a functional linkage between the promoter sequence and the gene of interest, such that the promoter sequence is able to initiate transcription of the gene of interest.

Constitutive promoter

A "constitutive promoter" refers to a promoter that is transcriptionally active during most, but not necessarily all, phases of growth and development and under most environmental conditions, in at least one cell, tissue or organ. Table 4a below gives examples of constitutive promoters.

**Table 4a:** Examples of constitutive promoters

| Gene Source | Reference |
|---|---|
| Actin | McElroy et al, Plant Cell, 2: 163-171, 1990 |
| HMGP | WO 2004/070039 |
| CAMV 35S | Odell et al, Nature, 313: 810-812, 1985 |
| CaMV 19S | Nilsson et al., Physiol. Plant. 100:456-462, 1997 |
| GOS2 | de Pater et al, Plant J Nov;2(6):837-44, 1992, WO 2004/065596 |
| Ubiquitin | Christensen et al, Plant Mol. Biol. 18: 675-689, 1992 |
| Rice cyclophilin | Buchholz et al, Plant Mol Biol. 25(5): 837-43, 1994 |
| Maize H3 histone | Lepetit et al, Mol. Gen. Genet. 231:276-285, 1992 |
| Alfalfa H3 histone | Wu et al. Plant Mol. Biol. 11:641-649, 1988 |
| Actin 2 | An et al, Plant J. 10(1); 107-121, 1996 |

(continued)

| Gene Source | Reference |
|---|---|
| 34S FMV | Sanger et al., Plant. Mol. Biol., 14, 1990: 433-443 |
| Rubisco small subunit | US 4,962,028 |
| OCS | Leisner (1988) Proc Natl Acad Sci USA 85(5): 2553 |
| SAD1 | Jain et al., Crop Science, 39 (6), 1999: 1696 |
| SAD2 | Jain et al., Crop Science, 39 (6), 1999: 1696 |
| nos | Shaw et al. (1984) Nucleic Acids Res. 12(20):7831-7846 |
| V-ATPase | WO 01/14572 |
| Super promoter | WO 95/14098 |
| G-box proteins | WO 94/12015 |

Ubiquitous promoter

A ubiquitous promoter is active in substantially all tissues or cells of an organism.

Developmentally-requlated promoter

A developmentally-regulated promoter is active during certain developmental stages or in parts of the plant that undergo developmental changes.

Inducible promoter

An inducible promoter has induced or increased transcription initiation in response to a chemical (for a review see Gatz 1997, Annu. Rev. Plant Physiol. Plant Mol. Biol., 48:89-108), environmental or physical stimulus, or may be "stress-inducible", i.e. activated when a plant is exposed to various stress conditions, or a "pathogen-inducible" i.e. activated when a plant is exposed to exposure to various pathogens.

Organ-specific/Tissue-specific promoter

An organ-specific or tissue-specific promoter is one that is capable of preferentially initiating transcription in certain organs or tissues, such as the leaves, roots, seed tissue etc. For example, a "root-specific promoter" is a promoter that is transcriptionally active predominantly in plant roots, substantially to the exclusion of any other parts of a plant, whilst still allowing for any leaky expression in these other plant parts. Promoters able to initiate transcription in certain cells only are referred to herein as "cell-specific".

A seed-specific promoter is transcriptionally active predominantly in seed tissue, but not necessarily exclusively in seed tissue (in cases of leaky expression). The seed-specific promoter may be active during seed development and/or during germination. The seed specific promoter may be endosperm/aleurone/embryo specific. Examples of seed-specific promoters are shown in Tables 4b to 4e below. Further examples of seed-specific promoters are given in Qing Qu and Takaiwa (Plant Biotechnol. J. 2, 113-125, 2004), which disclosure is incorporated by reference herein as if fully set forth.

**Table 4b:** Examples of seed-specific promoters

| Gene source | Reference |
|---|---|
| seed-specific genes | Simon et al., Plant Mol. Biol. 5: 191, 1985; |
| | Scofield et al., J. Biol. Chem. 262: 12202, 1987.; |
| | Baszczynski et al., Plant Mol. Biol. 14: 633, 1990. |
| Brazil Nut albumin | Pearson et al., Plant Mol. Biol. 18: 235-245, 1992. |
| legumin | Ellis et al., Plant Mol. Biol. 10: 203-214, 1988. |
| glutelin (rice) | Takaiwa et al., Mol. Gen. Genet. 208: 15-22, 1986; |

(continued)

| Gene source | Reference |
|---|---|
| | Takaiwa et al., FEBS Letts. 221: 43-47, 1987. |
| zein | Matzke et al Plant Mol Biol, 14(3):323-32 1990 |
| napA | Stalberg et al, Planta 199: 515-519, 1996. |
| wheat LMW and HMW glutenin-1 | Mol Gen Genet 216:81-90, 1989; NAR 17:461-2, 1989 |
| wheat SPA | Albani et al, Plant Cell, 9: 171-184, 1997 |
| wheat α, β, γ-gliadins | EMBO J. 3:1409-15, 1984 |
| barley Itr1 promoter | Diaz et al. (1995) Mol Gen Genet 248(5):592-8 |
| barley B1, C, D, hordein | Theor Appl Gen 98:1253-62, 1999; Plant J 4:343-55, 1993; Mol Gen Genet 250:750-60, 1996 |
| barley DOF | Mena et al, The Plant Journal, 116(1): 53-62, 1998 |
| blz2 | EP99106056.7 |
| synthetic promoter | Vicente-Carbajosa et al., Plant J. 13: 629-640, 1998. |
| rice prolamin NRP33 | Wu et al, Plant Cell Physiology 39(8) 885-889, 1998 |
| rice a-globulin Glb-1 | Wu et al, Plant Cell Physiology 39(8) 885-889, 1998 |
| rice OSH1 | Sato et al, Proc. Natl. Acad. Sci. USA, 93: 8117-8122, 1996 |
| rice α-globulin REB/OHP-1 | Nakase et al. Plant Mol. Biol. 33: 513-522, 1997 |
| rice ADP-glucose pyrophos-phorylase | Trans Res 6:157-68, 1997 |
| maize ESR gene family | Plant J 12:235-46, 1997 |
| sorghum α-kafirin | DeRose et al., Plant Mol. Biol 32:1029-35, 1996 |
| KNOX | Postma-Haarsma et al, Plant Mol. Biol. 39:257-71, 1999 |
| rice oleosin | Wu et al, J. Biochem. 123:386, 1998 |
| sunflower oleosin | Cummins et al., Plant Mol. Biol. 19: 873-876, 1992 |
| PRO0117, putative rice 40S ribosomal protein | WO 2004/070039 |
| PRO013 rice alanine aminotransferase | unpublished |
| PRO014 trypsin inhibitor ITR1 (barley) | unpublished |
| PRO015 rice WSI18 | WO 2004/070039 |
| PRO0175 rice RAB21 | WO 2004/070039 |
| PRO005 | WO 2004/070039 |
| PRO0095 | WO 2004/070039 |
| α-amylase (Amy32b) | Lanahan et al, Plant Cell 4:203-211, 1992; Skriver et al, Proc Natl Acad Sci USA 88:7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

**Table 4c:** examples of endosperm-specific promoters

| Gene source | Reference |
|---|---|
| glutelin (rice) | Takaiwa et al. (1986) Mol Gen Genet 208:15-22; Takaiwa et al. (1987) FEBS Letts. 221:43-47 |
| zein | Matzke et al., (1990) Plant Mol Biol 14(3): 323-32 |
| wheat LMW and HMW glutenin-1 | Colot et al. (1989) Mol Gen Genet 216:81-90, Anderson et al. (1989) NAR 17:461-2 |
| wheat SPA | Albani et al. (1997) Plant Cell 9:171-184 |
| wheat gliadins | Rafalski et al. (1984) EMBO 3:1409-15 |
| barley ltr1 promoter | Diaz et al. (1995) Mol Gen Genet 248(5):592-8 |
| barley B1, C, D, hordein | Cho et al. (1999) Theor Appl Genet 98:1253-62; Muller et al. (1993) Plant J 4:343-55; Sorenson et al. (1996) Mol Gen Genet 250:750-60 |
| barley DOF | Mena et al, (1998) Plant J 116(1): 53-62 |
| blz2 | Onate et al. (1999) J Biol Chem 274(14):9175-82 |
| synthetic promoter | Vicente-Carbajosa et al. (1998) Plant J 13:629-640 |
| rice prolamin NRP33 | Wu et al, (1998) Plant Cell Physiol 39(8) 885-889 |
| rice globulin Glb-1 | Wu et al. (1998) Plant Cell Physiol 39(8) 885-889 |
| rice globulin REB/OHP-1 | Nakase et al. (1997) Plant Molec Biol 33: 513-522 |
| rice ADP-glucose pyrophosphorylase | Russell et al. (1997) Trans Res 6:157-68 |
| maize ESR gene family | Opsahl-Ferstad et al. (1997) Plant J 12:235-46 |
| sorghum kafirin | DeRose et al. (1996) Plant Mol Biol 32:1029-35 |

**Table 4d:** Examples of embryo specific promoters:

| Gene source | Reference |
|---|---|
| rice OSH1 | Sato et al, Proc. Natl. Acad. Sci. USA, 93: 8117-8122, 1996 |
| KNOX | Postma-Haarsma et al, Plant Mol. Biol. 39:257-71, 1999 |
| PRO0151 | WO 2004/070039 |
| PRO0175 | WO 2004/070039 |
| PRO005 | WO 2004/070039 |
| PRO0095 | WO 2004/070039 |

**Table 4e:** Examples of aleurone-specific promoters:

| Gene source | Reference |
|---|---|
| α-amylase (Amy32b) | Lanahan et al, Plant Cell 4:203-211, 1992; Skriver et al, Proc Natl Acad Sci USA 88: 7266-7270, 1991 |
| cathepsin β-like gene | Cejudo et al, Plant Mol Biol 20:849-856, 1992 |
| Barley Ltp2 | Kalla et al., Plant J. 6:849-60, 1994 |
| Chi26 | Leah et al., Plant J. 4:579-89, 1994 |
| Maize B-Peru | Selinger et al., Genetics 149;1125-38,1998 |

A green tissue-specific promoter as defined herein is a promoter that is transcriptionally active predominantly in green tissue, substantially to the exclusion of any other parts of a plant, whilst still allowing for any leaky expression in these other plant parts.

Another example of a tissue-specific promoter is a meristem-specific promoter, which is transcriptionally active predominantly in meristematic tissue, substantially to the exclusion of any other parts of a plant, whilst still allowing for any leaky expression in these other plant parts.

Terminator

The term "terminator" encompasses a control sequence which is a DNA sequence at the end of a transcriptional unit which signals 3' processing and polyadenylation of a primary transcript and termination of transcription. The terminator can be derived from the natural gene, from a variety of other plant genes, or from T-DNA. The terminator to be added may be derived from, for example, the nopaline synthase or octopine synthase genes, or alternatively from another plant gene, or less preferably from any other eukaryotic gene.

Modulation

The term "modulation" means in relation to expression or gene expression, a process in which the expression level is changed by said gene expression in comparison to the control plant, the expression level may be increased or decreased. The original, unmodulated expression may be of any kind of expression of a structural RNA (rRNA, tRNA) or mRNA with subsequent translation. The term "modulating the activity" shall mean any change of the expression of the inventive nucleic acid sequences or encoded proteins, which leads to increased yield and/or increased growth of the plants.

Expression

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

Increased expression/overexpression

The term "increased expression" or "overexpression" as used herein means any form of expression that is additional to the original wild-type expression level.

Methods for increasing expression of genes or gene products are well documented in the art and include, for example, overexpression driven by appropriate promoters, the use of transcription enhancers or translation enhancers. Isolated nucleic acids which serve as promoter or enhancer elements may be introduced in an appropriate position (typically upstream) of a non-heterologous form of a polynucleotide so as to upregulate expression of a nucleic acid encoding the polypeptide of interest. For example, endogenous promoters may be altered in vivo by mutation, deletion, and/or substitution (see, Kmiec, US 5,565,350; Zarling et al., WO9322443), or isolated promoters may be introduced into a plant cell in the proper orientation and distance from a gene of the present invention so as to control the expression of the gene.

If polypeptide expression is desired, it is generally desirable to include a polyadenylation region at the 3'-end of a polynucleotide coding region. The polyadenylation region can be derived from the natural gene, from a variety of other plant genes, or from T-DNA. The 3' end sequence to be added may be derived from, for example, the nopaline synthase or octopine synthase genes, or alternatively from another plant gene, or less preferably from any other eukaryotic gene.

An intron sequence may also be added to the 5' untranslated region (UTR) or the coding sequence of the partial coding sequence to increase the amount of the mature message that accumulates in the cytosol. Inclusion of a spliceable intron in the transcription unit in both plant and animal expression constructs has been shown to increase gene expression at both the mRNA and protein levels up to 1000-fold (Buchman and Berg (1988) Mol. Cell biol. 8: 4395-4405; Callis et al. (1987) Genes Dev 1:1183-1200). Such intron enhancement of gene expression is typically greatest when placed near the 5' end of the transcription unit. Use of the maize introns Adh1-S intron 1, 2, and 6, the Bronze-1 intron are known in the art. For general information see: The Maize Handbook, Chapter 116, Freeling and Walbot, Eds., Springer, N.Y. (1994).

Endogenous gene

Reference herein to an "endogenous" gene not only refers to the gene in question as found in a plant in its natural form (i.e., without there being any human intervention), but also refers to that same gene (or a substantially homologous nucleic acid/gene) in an isolated form subsequently (re)introduced into a plant (a transgene). For example, a transgenic plant containing such a transgene may encounter a substantial reduction of the transgene expression and/or substantial reduction of expression of the endogenous gene. The isolated gene may be isolated from an organism or may be manmade, for example by chemical synthesis.

Decreased expression

Reference herein to "decreased epression" or "reduction or substantial elimination" of expression is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants.

For the reduction or substantial elimination of expression an endogenous gene in a plant, a sufficient length of substantially contiguous nucleotides of a nucleic acid sequence is required. In order to perform gene silencing, this may be as little as 20, 19, 18, 17, 16, 15, 14, 13, 12, 11, 10 or fewer nucleotides, alternatively this may be as much as the entire gene (including the 5' and/or 3' UTR, either in part or in whole). The stretch of substantially contiguous nucleotides may be derived from the nucleic acid encoding the protein of interest (target gene), or from any nucleic acid capable of encoding an orthologue, paralogue or homologue of the protein of interest. Preferably, the stretch of substantially contiguous nucleotides is capable of forming hydrogen bonds with the target gene (either sense or antisense strand), more preferably, the stretch of substantially contiguous nucleotides has, in increasing order of preference, 50%, 60%, 70%, 80%, 85%, 90%, 95%, 96%, 97%, 98%, 99%, 100% sequence identity to the target gene (either sense or antisense strand). A nucleic acid sequence encoding a (functional) polypeptide is not a requirement for the various methods discussed herein for the reduction or substantial elimination of expression of an endogenous gene.

This reduction or substantial elimination of expression may be achieved using routine tools and techniques. A preferred method for the reduction or substantial elimination of endogenous gene expression is by introducing and expressing in a plant a genetic construct into which the nucleic acid (in this case a stretch of substantially contiguous nucleotides derived from the gene of interest, or from any nucleic acid capable of encoding an orthologue, paralogue or homologue of any one of the protein of interest) is cloned as an inverted repeat (in part or completely), separated by a spacer (non-coding DNA).

In such a preferred method, expression of the endogenous gene is reduced or substantially eliminated through RNA-mediated silencing using an inverted repeat of a nucleic acid or a part thereof (in this case a stretch of substantially contiguous nucleotides derived from the gene of interest, or from any nucleic acid capable of encoding an orthologue, paralogue or homologue of the protein of interest), preferably capable of forming a hairpin structure. The inverted repeat is cloned in an expression vector comprising control sequences. A non-coding DNA nucleic acid sequence (a spacer, for example a matrix attachment region fragment (MAR), an intron, a polylinker, etc.) is located between the two inverted nucleic acids forming the inverted repeat. After transcription of the inverted repeat, a chimeric RNA with a self-complementary structure is formed (partial or complete). This double-stranded RNA structure is referred to as the hairpin RNA (hpRNA). The hpRNA is processed by the plant into siRNAs that are incorporated into an RNA-induced silencing complex (RISC). The RISC further cleaves the mRNA transcripts, thereby substantially reducing the number of mRNA transcripts to be translated into polypeptides. For further general details see for example, Grierson et al. (1998) WO 98/53083; Waterhouse et al. (1999) WO 99/53050).

Performance of the methods of the invention does not rely on introducing and expressing in a plant a genetic construct into which the nucleic acid is cloned as an inverted repeat, but any one or more of several well-known "gene silencing" methods may be used to achieve the same effects.

One such method for the reduction of endogenous gene expression is RNA-mediated silencing of gene expression (downregulation). Silencing in this case is triggered in a plant by a double stranded RNA sequence (dsRNA) that is substantially similar to the target endogenous gene. This dsRNA is further processed by the plant into about 20 to about 26 nucleotides called short interfering RNAs (siRNAs). The siRNAs are incorporated into an RNA-induced silencing complex (RISC) that cleaves the mRNA transcript of the endogenous target gene, thereby substantially reducing the number of mRNA transcripts to be translated into a polypeptide. Preferably, the double stranded RNA sequence corresponds to a target gene.

Another example of an RNA silencing method involves the introduction of nucleic acid sequences or parts thereof (in this case a stretch of substantially contiguous nucleotides derived from the gene of interest, or from any nucleic acid capable of encoding an orthologue, paralogue or homologue of the protein of interest) in a sense orientation into a plant. "Sense orientation" refers to a DNA sequence that is homologous to an mRNA transcript thereof. Introduced into a plant would therefore be at least one copy of the nucleic acid sequence. The additional nucleic acid sequence will reduce expression of the endogenous gene, giving rise to a phenomenon known as co-suppression. The reduction of gene expression will be more pronounced if several additional copies of a nucleic acid sequence are introduced into the plant, as there is a positive correlation between high transcript levels and the triggering of co-suppression.

Another example of an RNA silencing method involves the use of antisense nucleic acid sequences. An "antisense" nucleic acid sequence comprises a nucleotide sequence that is complementary to a "sense" nucleic acid sequence encoding a protein, i.e. complementary to the coding strand of a double-stranded cDNA molecule or complementary to an mRNA transcript sequence. The antisense nucleic acid sequence is preferably complementary to the endogenous gene to be silenced. The complementarity may be located in the "coding region" and/or in the "non-coding region" of a gene. The term "coding region" refers to a region of the nucleotide sequence comprising codons that are translated into amino acid residues. The term "non-coding region" refers to 5' and 3' sequences that flank the coding region that are transcribed but not translated into amino acids (also referred to as 5' and 3' untranslated regions).

Antisense nucleic acid sequences can be designed according to the rules of Watson and Crick base pairing. The antisense nucleic acid sequence may be complementary to the entire nucleic acid sequence (in this case a stretch of substantially contiguous nucleotides derived from the gene of interest, or from any nucleic acid capable of encoding an orthologue, paralogue or homologue of the protein of interest), but may also be an oligonucleotide that is antisense to only a part of the nucleic acid sequence (including the mRNA 5' and 3' UTR). For example, the antisense oligonucleotide sequence may be complementary to the region surrounding the translation start site of an mRNA transcript encoding a polypeptide. The length of a suitable antisense oligonucleotide sequence is known in the art and may start from about 50, 45, 40, 35, 30, 25, 20, 15 or 10 nucleotides in length or less. An antisense nucleic acid sequence according to the invention may be constructed using chemical synthesis and enzymatic ligation reactions using methods known in the art. For example, an antisense nucleic acid sequence (e.g., an antisense oligonucleotide sequence) may be chemically synthesized using naturally occurring nucleotides or variously modified nucleotides designed to increase the biological stability of the molecules or to increase the physical stability of the duplex formed between the antisense and sense nucleic acid sequences, e.g., phosphorothioate derivatives and acridine substituted nucleotides may be used. Examples of modified nucleotides that may be used to generate the antisense nucleic acid sequences are well known in the art. Known nucleotide modifications include methylation, cyclization and 'caps' and substitution of one or more of the naturally occurring nucleotides with an analogue such as inosine. Other modifications of nucleotides are well known in the art.

The antisense nucleic acid sequence can be produced biologically using an expression vector into which a nucleic acid sequence has been subcloned in an antisense orientation (i.e., RNA transcribed from the inserted nucleic acid will be of an antisense orientation to a target nucleic acid of interest). Preferably, production of antisense nucleic acid sequences in plants occurs by means of a stably integrated nucleic acid construct comprising a promoter, an operably linked antisense oligonucleotide, and a terminator.

The nucleic acid molecules used for silencing in the methods of the invention (whether introduced into a plant or generated in situ) hybridize with or bind to mRNA transcripts and/or genomic DNA encoding a polypeptide to thereby inhibit expression of the protein, e.g., by inhibiting transcription and/or translation. The hybridization can be by conventional nucleotide complementarity to form a stable duplex, or, for example, in the case of an antisense nucleic acid sequence which binds to DNA duplexes, through specific interactions in the major groove of the double helix. Antisense nucleic acid sequences may be introduced into a plant by transformation or direct injection at a specific tissue site. Alternatively, antisense nucleic acid sequences can be modified to target selected cells and then administered systemically. For example, for systemic administration, antisense nucleic acid sequences can be modified such that they specifically bind to receptors or antigens expressed on a selected cell surface, e.g., by linking the antisense nucleic acid sequence to peptides or antibodies which bind to cell surface receptors or antigens. The antisense nucleic acid sequences can also be delivered to cells using the vectors described herein.

According to a further aspect, the antisense nucleic acid sequence is an a-anomeric nucleic acid sequence. An a-anomeric nucleic acid sequence forms specific double-stranded hybrids with complementary RNA in which, contrary to the usual b-units, the strands run parallel to each other (Gaultier et al. (1987) Nucl Ac Res 15: 6625-6641). The antisense nucleic acid sequence may also comprise a 2'-o-methylribonucleotide (Inoue et al. (1987) Nucl Ac Res 15, 6131-6148) or a chimeric RNA-DNA analogue (Inoue et al. (1987) FEBS Lett. 215, 327-330).

The reduction or substantial elimination of endogenous gene expression may also be performed using ribozymes. Ribozymes are catalytic RNA molecules with ribonuclease activity that are capable of cleaving a single-stranded nucleic acid sequence, such as an mRNA, to which they have a complementary region. Thus, ribozymes (e.g., hammerhead ribozymes (described in Haselhoff and Gerlach (1988) Nature 334, 585-591) can be used to catalytically cleave mRNA transcripts encoding a polypeptide, thereby substantially reducing the number of mRNA transcripts to be translated into a polypeptide. A ribozyme having specificity for a nucleic acid sequence can be designed (see for example: Cech et al. U.S. Patent No. 4,987,071; and Cech et al. U.S. Patent No. 5,116,742). Alternatively, mRNA transcripts corresponding to a nucleic acid sequence can be used to select a catalytic RNA having a specific ribonuclease activity from a pool of RNA molecules (Bartel and Szostak (1993) Science 261, 1411-1418). The use of ribozymes for gene silencing in plants is known in the art (e.g., Atkins et al. (1994) WO 94/00012; Lenne et al. (1995) WO 95/03404; Lutziger et al. (2000) WO 00/00619; Prinsen et al. (1997) WO 97/13865 and Scott et al. (1997) WO 97/38116).

Gene silencing may also be achieved by insertion mutagenesis (for example, T-DNA insertion or transposon insertion) or by strategies as described by, among others, Angell and Baulcombe ((1999) Plant J 20(3): 357-62), (Amplicon VIGS WO 98/36083), or Baulcombe (WO 99/15682).

Gene silencing may also occur if there is a mutation on an endogenous gene and/or a mutation on an isolated gene/nucleic acid subsequently introduced into a plant. The reduction or substantial elimination may be caused by a non-functional polypeptide. For example, the polypeptide may bind to various interacting proteins; one or more mutation (s) and/or truncation(s) may therefore provide for a polypeptide that is still able to bind interacting proteins (such as receptor proteins) but that cannot exhibit its normal function (such as signalling ligand).

A further approach to gene silencing is by targeting nucleic acid sequences complementary to the regulatory region of the gene (e.g., the promoter and/or enhancers) to form triple helical structures that prevent transcription of the gene in target cells. See Helene, C., Anticancer Drug Res. 6, 569-84, 1991; Helene et al., Ann. N.Y. Acad. Sci. 660, 27-36 1992; and Maher, L.J. Bioassays 14, 807-15, 1992.

Other methods, such as the use of antibodies directed to an endogenous polypeptide for inhibiting its function in planta, or interference in the signalling pathway in which a polypeptide is involved, will be well known to the skilled man. In particular, it can be envisaged that manmade molecules may be useful for inhibiting the biological function of a target polypeptide, or for interfering with the signalling pathway in which the target polypeptide is involved.

Alternatively, a screening program may be set up to identify in a plant population natural variants of a gene, which variants encode polypeptides with reduced activity. Such natural variants may also be used for example, to perform homologous recombination.

Artificial and/or natural microRNAs (miRNAs) may be used to knock out gene expression and/or mRNA translation. Endogenous miRNAs are single stranded small RNAs of typically 19-24 nucleotides long. They function primarily to regulate gene expression and/ or mRNA translation. Most plant microRNAs (miRNAs) have perfect or near-perfect complementarity with their target sequences. However, there are natural targets with up to five mismatches. They are processed from longer non-coding RNAs with characteristic fold-back structures by double-strand specific RNases of the Dicer family. Upon processing, they are incorporated in the RNA-induced silencing complex (RISC) by binding to its main component, an Argonaute protein. MiRNAs serve as the specificity components of RISC, since they base-pair to target nucleic acids, mostly mRNAs, in the cytoplasm. Subsequent regulatory events include target mRNA cleavage and destruction and/or translational inhibition. Effects of miRNA overexpression are thus often reflected in decreased mRNA levels of target genes.

Artificial microRNAs (amiRNAs), which are typically 21 nucleotides in length, can be genetically engineered specifically to negatively regulate gene expression of single or multiple genes of interest. Determinants of plant microRNA target selection are well known in the art. Empirical parameters for target recognition have been defined and can be used to aid in the design of specific amiRNAs, (Schwab et al., Dev. Cell 8, 517-527, 2005). Convenient tools for design and generation of amiRNAs and their precursors are also available to the public (Schwab et al., Plant Cell 18, 1121-1133, 2006).

For optimal performance, the gene silencing techniques used for reducing expression in a plant of an endogenous gene requires the use of nucleic acid sequences from monocotyledonous plants for transformation of monocotyledonous plants, and from dicotyledonous plants for transformation of dicotyledonous plants. Preferably, a nucleic acid sequence from any given plant species is introduced into that same species. For example, a nucleic acid sequence from rice is transformed into a rice plant. However, it is not an absolute requirement that the nucleic acid sequence to be introduced originates from the same plant species as the plant in which it will be introduced. It is sufficient that there is substantial homology between the endogenous target gene and the nucleic acid to be introduced.

Described above are examples of various methods for the reduction or substantial elimination of expression in a plant of an endogenous gene. A person skilled in the art would readily be able to adapt the aforementioned methods for silencing so as to achieve reduction of expression of an endogenous gene in a whole plant or in parts thereof through the use of an appropriate promoter, for example.

<u>Selectable marker (gene)/Reporter gene</u>

"Selectable marker", "selectable marker gene" or "reporter gene" includes any gene that confers a phenotype on a cell in which it is expressed to facilitate the identification and/or selection of cells that are transfected or transformed with a nucleic acid construct of the invention. These marker genes enable the identification of a successful transfer of the nucleic acid molecules via a series of different principles. Suitable markers may be selected from markers that confer antibiotic or herbicide resistance, that introduce a new metabolic trait or that allow visual selection. Examples of selectable marker genes include genes conferring resistance to antibiotics (such as nptII that phosphorylates neomycin and kanamycin, or hpt, phosphorylating hygromycin, or genes conferring resistance to, for example, bleomycin, streptomycin, tetracyclin, chloramphenicol, ampicillin, gentamycin, geneticin (G418), spectinomycin or blasticidin), to herbicides (for example bar which provides resistance to Basta®; aroA or gox providing resistance against glyphosate, or the genes conferring resistance to, for example, imidazolinone, phosphinothricin or sulfonylurea), or genes that provide a metabolic trait (such as manA that allows plants to use mannose as sole carbon source or xylose isomerase for the utilisation of xylose, or antinutritive markers such as the resistance to 2-deoxyglucose). Expression of visual marker genes results in the formation of colour (for example β-glucuronidase, GUS or β-galactosidase with its coloured substrates, for example X-Gal), luminescence (such as the luciferin/luceferase system) or fluorescence (Green Fluorescent Protein, GFP, and derivatives thereof). This list represents only a small number of possible markers. The skilled worker is familiar with such markers. Different markers are preferred, depending on the organism and the selection method.

It is known that upon stable or transient integration of nucleic acids into plant cells, only a minority of the cells takes up the foreign DNA and, if desired, integrates it into its genome, depending on the expression vector used and the transfection technique used. To identify and select these integrants, a gene coding for a selectable marker (such as the ones described above) is usually introduced into the host cells together with the gene of interest. These markers can for example be used in mutants in which these genes are not functional by, for example, deletion by conventional methods. Furthermore, nucleic acid molecules encoding a selectable marker can be introduced into a host cell on the same vector that comprises the sequence encoding the polypeptides of the invention or used in the methods of the invention, or else in a separate vector. Cells which have been stably transfected with the introduced nucleic acid can be identified for example by selection (for example, cells which have integrated the selectable marker survive whereas the other cells die).

Since the marker genes, particularly genes for resistance to antibiotics and herbicides, are no longer required or are undesired in the transgenic host cell once the nucleic acids have been introduced successfully, the process according to the invention for introducing the nucleic acids advantageously employs techniques which enable the removal or excision of these marker genes. One such a method is what is known as co-transformation. The co-transformation method employs two vectors simultaneously for the transformation, one vector bearing the nucleic acid according to the invention and a second bearing the marker gene(s). A large proportion of transformants receives or, in the case of plants, comprises (up to 40% or more of the transformants), both vectors. In case of transformation with Agrobacteria, the transformants usually receive only a part of the vector, i.e. the sequence flanked by the T-DNA, which usually represents the expression cassette. The marker genes can subsequently be removed from the transformed plant by performing crosses. In another method, marker genes integrated into a transposon are used for the transformation together with desired nucleic acid (known as the Ac/Ds technology). The transformants can be crossed with a transposase source or the transformants are transformed with a nucleic acid construct conferring expression of a transposase, transiently or stable. In some cases (approx. 10%), the transposon jumps out of the genome of the host cell once transformation has taken place successfully and is lost. In a further number of cases, the transposon jumps to a different location. In these cases the marker gene must be eliminated by performing crosses. In microbiology, techniques were developed which make possible, or facilitate, the detection of such events. A further advantageous method relies on what is known as recombination systems; whose advantage is that elimination by crossing can be dispensed with. The best-known system of this type is what is known as the Cre/lox system. Cre1 is a recombinase that removes the sequences located between the loxP sequences. If the marker gene is integrated between the loxP sequences, it is removed once transformation has taken place successfully, by expression of the recombinase. Further recombination systems are the HIN/HIX, FLP/FRT and REP/STB system (Tribble et al., J. Biol. Chem., 275, 2000: 22255-22267; Velmurugan et al., J. Cell Biol., 149, 2000: 553-566). A site-specific integration into the plant genome of the nucleic acid sequences according to the invention is possible. Naturally, these methods can also be applied to microorganisms such as yeast, fungi or bacteria.

<u>Transgenic/Transgene/Recombinant</u>

For the purposes of the invention, "transgenic", "transgene" or "recombinant" means with regard to, for example, a nucleic acid sequence, an expression cassette, gene construct or a vector comprising the nucleic acid sequence or an organism transformed with the nucleic acid sequences, expression cassettes or vectors according to the invention, all those constructions brought about by recombinant methods in which either

(a) the nucleic acid sequences encoding proteins useful in the methods of the invention, or
(b) genetic control sequence(s) which is operably linked with the nucleic acid sequence according to the invention, for example a promoter, or
(c) a) and b)

are not located in their natural genetic environment or have been modified by recombinant methods, it being possible for the modification to take the form of, for example, a substitution, addition, deletion, inversion or insertion of one or more nucleotide residues. The natural genetic environment is understood as meaning the natural genomic or chromosomal locus in the original plant or the presence in a genomic library. In the case of a genomic library, the natural genetic environment of the nucleic acid sequence is preferably retained, at least in part. The environment flanks the nucleic acid sequence at least on one side and has a sequence length of at least 50 bp, preferably at least 500 bp, especially preferably at least 1000 bp, most preferably at least 5000 bp. A naturally occurring expression cassette - for example the naturally occurring combination of the natural promoter of the nucleic acid sequences with the corresponding nucleic acid sequence encoding a polypeptide useful in the methods of the present invention, as defined above - becomes a transgenic expression cassette when this expression cassette is modified by non-natural, synthetic ("artificial") methods such as, for example, mutagenic treatment. Suitable methods are described, for example, in US 5,565,350 or WO 00/15815.

A transgenic plant for the purposes of the invention is thus understood as meaning, as above, that the nucleic acids used in the method of the invention are not at their natural locus in the genome of said plant, it being possible for the nucleic acids to be expressed homologously or heterologously. However, as mentioned, transgenic also means that, while the nucleic acids according to the invention or used in the inventive method are at their natural position in the genome of a plant, the sequence has been modified with regard to the natural sequence, and/or that the regulatory sequences of the natural sequences have been modified. Transgenic is preferably understood as meaning the expression of the nucleic acids according to the invention at an unnatural locus in the genome, i.e. homologous or, preferably, heterologous expression of the nucleic acids takes place. Preferred transgenic plants are mentioned herein.

Transformation

The term "introduction" or "transformation" as referred to herein encompasses the transfer of an exogenous polynucleotide into a host cell, irrespective of the method used for transfer. Plant tissue capable of subsequent clonal propagation, whether by organogenesis or embryogenesis, may be transformed with a genetic construct of the present invention and a whole plant regenerated there from. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

The transfer of foreign genes into the genome of a plant is called transformation. Transformation of plant species is now a fairly routine technique. Advantageously, any of several transformation methods may be used to introduce the gene of interest into a suitable ancestor cell. The methods described for the transformation and regeneration of plants from plant tissues or plant cells may be utilized for transient or for stable transformation. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al., (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al., (1986) Mol. Gen Genet 202: 179-185); DNA or RNA-coated particle bombardment (Klein TM et al., (1987) Nature 327: 70) infection with (non-integrative) viruses and the like. Transgenic plants, including transgenic crop plants, are preferably produced via *Agrobacterium*-mediated transformation. An advantageous transformation method is the transformation *in planta.* To this end, it is possible, for example, to allow the agrobacteria to act on plant seeds or to inoculate the plant meristem with agrobacteria. It has proved particularly expedient in accordance with the invention to allow a suspension of transformed agrobacteria to act on the intact plant or at least on the flower primordia. The plant is subsequently grown on until the seeds of the treated plant are obtained (Clough and Bent, Plant J. (1998) 16, 735-743). Methods for *Agrobacterium*-mediated transformation of rice include well known methods for rice transformation, such as those described in any of the following: European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth. Said methods are further described by way of example in B. Jenes et al., Techniques for Gene Transfer, in: Transgenic Plants, Vol. 1, Engineering and Utilization, eds. S.D. Kung and R. Wu, Academic Press (1993) 128-143 and in Potrykus Annu. Rev. Plant Physiol. Plant Molec. Biol. 42 (1991) 205-225). The nucleic acids or the construct to be expressed is preferably cloned into a vector, which is suitable for transforming *Agrobacterium tumefaciens,* for example pBin19 (Bevan et al., Nucl. Acids Res. 12 (1984) 8711). Agrobacteria transformed by such a vector can then be used in known manner for the transformation of plants, such as plants used as a model, like *Arabidopsis* (*Arabidopsis thaliana* is within the scope of the present invention not considered as a crop plant), or crop plants such as, by way of example, tobacco plants, for example by immersing bruised leaves or chopped leaves in an agrobacterial solution and then culturing them in suitable media. The transformation of plants by means of *Agrobacterium tumefaciens* is described, for example, by Höfgen and Willmitzer in Nucl. Acid Res. (1988) 16, 9877 or is known inter alia from F.F. White, Vectors for Gene Transfer in Higher Plants; in Transgenic Plants, Vol. 1, Engineering and Utilization, eds. S.D. Kung and R. Wu, Academic Press, 1993, pp. 15-38.

In addition to the transformation of somatic cells, which then have to be regenerated into intact plants, it is also possible to transform the cells of plant meristems and in particular those cells which develop into gametes. In this case, the transformed gametes follow the natural plant development, giving rise to transgenic plants. Thus, for example, seeds of *Arabidopsis* are treated with agrobacteria and seeds are obtained from the developing plants of which a certain proportion is transformed and thus transgenic [Feldman, KA and Marks MD (1987). Mol Gen Genet 208:274-289; Feldmann K (1992). In: C Koncz, N-H Chua and J Shell, eds, Methods in Arabidopsis Research. Word Scientific, Singapore, pp. 274-289]. Alternative methods are based on the repeated removal of the inflorescences and incubation of the excision site in the center of the rosette with transformed agrobacteria, whereby transformed seeds can likewise be obtained at a later point in time (Chang (1994). Plant J. 5: 551-558; Katavic (1994). Mol Gen Genet, 245: 363-370). However, an especially effective method is the vacuum infiltration method with its modifications such as the "floral dip" method. In the case of vacuum infiltration of *Arabidopsis,* intact plants under reduced pressure are treated with an agrobacterial suspension [Bechthold, N (1993). C R Acad Sci Paris Life Sci, 316: 1194-1199], while in the case of the "floral dip" method the developing floral tissue is incubated briefly with a surfactant-treated agrobacterial suspension [Clough, SJ and Bent AF (1998) The Plant J. 16, 735-743]. A certain proportion of transgenic seeds are harvested in both cases, and these seeds can be distinguished from non-transgenic seeds by growing under the above-described selective conditions. In addition the stable transformation of plastids is of advantages because plastids are inherited maternally is most crops reducing or eliminating the risk of transgene flow through pollen. The transformation of the chloroplast genome is generally achieved by a process which has been schematically displayed in Klaus et al., 2004 [Nature Biotechnology 22 (2), 225-229]. Briefly the sequences to be transformed are cloned together with a selectable marker gene between flanking sequences homologous to the chloroplast genome. These homologous flanking sequences direct site specific integration into the plastome. Plastidal transformation has been described for many different plant species and an overview is given in Bock (2001) Transgenic plastids in basic research and plant biotechnology. J Mol Biol. 2001 Sep 21; 312 (3):425-38 or Maliga, P (2003) Progress towards commercialization of plastid transformation technology. Trends Biotechnol. 21, 20-28. Further biotechnological progress has recently been reported in form of marker free plastid transformants, which can be produced by a transient co-integrated maker gene (Klaus et al., 2004, Nature Biotechnology 22(2), 225-229).

<u>T-DNA activation tagging</u>

T-DNA activation tagging (Hayashi et al. Science (1992) 1350-1353), involves insertion of T-DNA, usually containing a promoter (may also be a translation enhancer or an intron), in the genomic region of the gene of interest or 10 kb up- or downstream of the coding region of a gene in a configuration such that the promoter directs expression of the targeted gene. Typically, regulation of expression of the targeted gene by its natural promoter is disrupted and the gene falls under the control of the newly introduced promoter. The promoter is typically embedded in a T-DNA. This T-DNA is randomly inserted into the plant genome, for example, through *Agrobacterium* infection and leads to modified expression of genes near the inserted T-DNA. The resulting transgenic plants show dominant phenotypes due to modified expression of genes close to the introduced promoter.

<u>TILLING</u>

The term "TILLING" is an abbreviation of "Targeted Induced Local Lesions In Genomes" and refers to a mutagenesis technology useful to generate and/or identify nucleic acids encoding proteins with modified expression and/or activity. TILLING also allows selection of plants carrying such mutant variants. These mutant variants may exhibit modified expression, either in strength or in location or in timing (if the mutations affect the promoter for example). These mutant variants may exhibit higher activity than that exhibited by the gene in its natural form. TILLING combines high-density mutagenesis with high-throughput screening methods. The steps typically followed in TILLING are: (a) EMS mutagenesis (Redei GP and Koncz C (1992) In Methods in Arabidopsis Research, Koncz C, Chua NH, Schell J, eds. Singapore, World Scientific Publishing Co, pp. 16-82; Feldmann et al., (1994) In Meyerowitz EM, Somerville CR, eds, Arabidopsis. Cold Spring Harbor Laboratory Press, Cold Spring Harbor, NY, pp 137-172; Lightner J and Caspar T (1998) In J Martinez-Zapater, J Salinas, eds, Methods on Molecular Biology, Vol. 82. Humana Press, Totowa, NJ, pp 91-104); (b) DNA preparation and pooling of individuals; (c) PCR amplification of a region of interest; (d) denaturation and annealing to allow formation of heteroduplexes; (e) DHPLC, where the presence of a heteroduplex in a pool is detected as an extra peak in the chromatogram; (f) identification of the mutant individual; and (g) sequencing of the mutant PCR product.

Methods for TILLING are well known in the art (McCallum et al., (2000) Nat Biotechnol 18: 455-457; reviewed by Stemple (2004) Nat Rev Genet 5(2): 145-50).

Homologous recombination

Homologous recombination allows introduction in a genome of a selected nucleic acid at a defined selected position. Homologous recombination is a standard technology used routinely in biological sciences for lower organisms such as yeast or the moss *Physcomitrella.* Methods for performing homologous recombination in plants have been described not only for model plants (Offringa et al. (1990) EMBO J 9(10): 3077-84) but also for crop plants, for example rice (Terada et al. (2002) Nat Biotech 20(10): 1030-4; Iida and Terada (2004) Curr Opin Biotech 15(2): 132-8).

Yield

The term "yield" in general means a measurable produce of economic value, typically related to a specified crop, to an area, and to a period of time. Individual plant parts directly contribute to yield based on their number, size and/or weight, or the actual yield is the yield per square meter for a crop and year, which is determined by dividing total production (includes both harvested and appraised production) by planted square meters. The term "yield" of a plant may relate to vegetative biomass (root and/or shoot biomass), to reproductive organs, and/or to propagules (such as seeds) of that plant.

Early vigour

"Early vigour" refers to active healthy well-balanced growth especially during early stages of plant growth, and may result from increased plant fitness due to, for example, the plants being better adapted to their environment (i.e. optimizing the use of energy resources and partitioning between shoot and root). Plants having early vigour also show increased seedling survival and a better establishment of the crop, which often results in highly uniform fields (with the crop growing in uniform manner, i.e. with the majority of plants reaching the various stages of development at substantially the same time), and often better and higher yield. Therefore, early vigour may be determined by measuring various factors, such as thousand kernel weight, percentage germination, percentage emergence, seedling growth, seedling height, root length, root and shoot biomass and many more.

Increase/Improve/Enhance

The terms "increase", "improve" or "enhance" are interchangeable and shall mean in the sense of the application at least a 3%, 4%, 5%, 6%, 7%, 8%, 9% or 10%, preferably at least 15% or 20%, more preferably 25%, 30%, 35% or 40% more yield and/or growth in comparison to control plants as defined herein.

Seed yield

Increased seed yield may manifest itself as one or more of the following: a) an increase in seed biomass (total seed weight) which may be on an individual seed basis and/or per plant and/or per square meter; b) increased number of flowers per plant; c) increased number of (filled) seeds; d) increased seed filling rate (which is expressed as the ratio between the number of filled seeds divided by the total number of seeds); e) increased harvest index, which is expressed as a ratio of the yield of harvestable parts, such as seeds, divided by the total biomass; and f) increased thousand kernel weight (TKW), which is extrapolated from the number of filled seeds counted and their total weight. An increased TKW may result from an increased seed size and/or seed weight, and may also result from an increase in embryo and/or endosperm size.

An increase in seed yield may also be manifested as an increase in seed size and/or seed volume. Furthermore, an increase in seed yield may also manifest itself as an increase in seed area and/or seed length and/or seed width and/or seed perimeter. Increased yield may also result in modified architecture, or may occur because of modified architecture.

Greenness Index

The "greenness index" as used herein is calculated from digital images of plants. For each pixel belonging to the plant object on the image, the ratio of the green value versus the red value (in the RGB model for encoding color) is calculated. The greenness index is expressed as the percentage of pixels for which the green-to-red ratio exceeds a given threshold. Under normal growth conditions, under salt stress growth conditions, and under reduced nutrient availability growth conditions, the greenness index of plants is measured in the last imaging before flowering. In contrast, under drought stress growth conditions, the greenness index of plants is measured in the first imaging after drought.

Plant

The term "plant" ,as used herein encompasses whole plants, ancestors and progeny of the plants and plant parts, including seeds, shoots, stems, leaves, roots (including tubers), flowers, and tissues and organs, wherein each of the aforementioned comprise the gene/nucleic acid of interest. The term "plant" also encompasses plant cells, suspension cultures, callus tissue, embryos, meristematic regions, gametophytes, sporophytes, pollen and microspores, again wherein each of the aforementioned comprises the gene/nucleic acid of interest.

Plants that are particularly useful in the methods of the invention include all plants which belong to the superfamily Viridiplantae, in particular monocotyledonous and dicotyledonous plants including fodder or forage legumes, ornamental plants, food crops, trees or shrubs selected from the list comprising *Acer* spp., *Actinidia* spp., *Abelmoschus* spp., *Agave sisalana, Agropyron* spp., *Agrostis stolonifera, Allium* spp., *Amaranthus* spp., *Ammophila arenaria, Ananas comosus, Annona* spp., *Apium graveolens, Arachis spp, Artocarpus* spp., *Asparagus officinalis, Avena* spp. *(e.g. Avena sativa, Avena fatua, Avena byzantina, Avena fatua var. sativa, Avena hybrida), Averrhoa carambola, Bambusa sp., Benincasa hispida, Bertholletia excelsea, Beta vulgaris, Brassica* spp. *(e.g. Brassica napus, Brassica rapa* ssp. [canola, oilseed rape, turnip rape]), *Cadaba farinosa, Camellia sinensis, Canna indica, Cannabis sativa, Capsicum* spp., *Carex elata, Carica papaya, Carissa macrocarpa, Carya* spp., *Carthamus tinctorius, Castanea* spp., *Ceiba pentandra, Cichorium endivia, Cinnamomum* spp., *Citrullus lanatus, Citrus* spp., Cocos spp., *Coffea* spp., *Colocasia esculenta, Cola* spp., *Corchorus sp., Coriandrum sativum, Corylus* spp., *Crataegus* spp., *Crocus sativus, Cucurbita* spp., *Cucumis* spp., *Cynara* spp., *Daucus carota, Desmodium* spp., *Dimocarpus longan, Dioscorea* spp., *Diospyros* spp., *Echinochloa* spp., *Elaeis (e.g. Elaeis guineensis, Elaeis oleifera), Eleusine coracana, Erianthus sp., Eriobotrya japonica, Eucalyptus sp., Eugenia uniflora, Fagopyrum* spp., *Fagus* spp., *Festuca arundinacea, Ficus carica, Fortunella* spp., *Fragaria* spp., *Ginkgo biloba, Glycine* spp. *(e.g. Glycine max, Soja hispida or Soja max), Gossypium hirsutum, Helianthus* spp. *(e.g. Helianthus annuus), Hemerocallis fulva, Hibiscus* spp., *Hordeum* spp. *(e.g. Hordeum vulgare), Ipomoea batatas, Juglans* spp., *Lactuca sativa, Lathyrus* spp., *Lens culinaris, Linum usitatissimum, Litchi chinensis, Lotus* spp., *Luffa acutangula, Lupinus* spp., *Luzula sylvatica, Lycopersicon* spp. *(e.g. Lycopersicon esculentum, Lycopersicon lycopersicum, Lycopersicon pyriforme), Macrotyloma* spp., *Malus* spp., *Malpighia emarginata, Mammea americana, Mangifera indica, Manihot* spp., *Manilkara zapota, Medicago sativa, Melilotus* spp., *Mentha* spp., *Miscanthus sinensis, Momordica* spp., *Morus nigra, Musa* spp., *Nicotiana* spp., *Olea* spp., *Opuntia* spp., *Ornithopus* spp., *Oryza* spp. *(e.g. Oryza sativa, Oryza latifolia), Panicum miliaceum, Panicum virgatum, Passiflora edulis, Pastinaca sativa, Pennisetum sp., Persea* spp., *Petroselinum crispum, Phalaris arundinacea, Phaseolus* spp., *Phleum pratense, Phoenix* spp., *Phragmites australis, Physalis* spp., *Pinus* spp., *Pistacia vera, Pisum* spp., *Poa* spp., *Populus* spp., *Prosopis* spp., *Prunus* spp., *Psidium* spp., *Punica granatum, Pyrus communis, Quercus* spp., *Raphanus sativus, Rheum rhabarbarum, Ribes* spp., *Ricinus communis, Rubus* spp., *Saccharum* spp., *Salix sp., Sambucus* spp., *Secale cereale, Sesamum* spp., *Sinapis sp., Solanum* spp. *(e.g. Solanum tuberosum, Solanum integrifolium or Solanum lycopersicum), Sorghum bicolor, Spinacia* spp., *Syzygium* spp., *Tagetes* spp., *Tamarindus indica, Theobroma cacao, Trifolium* spp., *Triticosecale rimpaui, Triticum* spp. *(e.g. Triticum aestivum, Triticum durum, Triticum turgidum, Triticum hybernum, Triticum macha, Triticum sativum or Triticum vulgare), Tropaeolum minus, Tropaeolum majus, Vaccinium* spp., *Vicia* spp., *Vigna* spp., *Viola odorata, Vitis* spp., *Zea mays, Zizania palustris, Ziziphus* spp., amongst others.

## Detailed description of the invention

## Detailed description for the Ankyrin - Zn finger polypeptide

Surprisingly, it has now been found that modulating expression in a plant of a nucleic acid encoding the AZ polypeptide from *Arabidopsis* (AtAZ) or a homologue thereof gives plants having increased yield relative to control plants. According to one embodiment of the present invention, there is provided a method for increasing plant yield, comprising modulating expression in a plant of a nucleic acid encoding the AZ polypeptide or a homologue thereof. Advantageously, performance of the methods according to the present invention results in plants having increased yield, particularly seed yield, relative to corresponding wild type plants.

A "reference", "reference plant", "control", "control plant", "wild type" or "wild type plant" is in particular a cell, a tissue, an organ, a plant, or a part thereof, which was not produced according to the method of the invention. Accordingly, the terms "wild type", "control" or "reference" are exchangeable and can be a cell or a part of the plant such as an organelle or tissue, or a plant, which was not modified or treated according to the herein described method according to the invention. Accordingly, the cell or a part of the plant such as an organelle or a plant used as wild type, control or reference corresponds to the cell, plant or part thereof as much as possible and is in any other property but in the result of the process of the invention as identical to the subject matter of the invention as possible. Thus, the wild type, control or reference is treated identically or as identical as possible, saying that only conditions or properties might be different which do not influence the quality of the tested property. That means in other words that the wild type denotes (1) a plant, which carries the unaltered or not modulated form of a gene or allele or (2) the starting material/plant from which the plants produced by the process or method of the invention are derived.

Preferably, any comparison between the wild type plants and the plants produced by the method of the invention is carried out under analogous conditions. The term "analogous conditions" means that all conditions such as, for example, culture or growing conditions, assay conditions (such as buffer composition, temperature, substrates, pathogen strain, concentrations and the like) are kept identical between the experiments to be compared.

The "reference", "control", or "wild type" is preferably a subject, e.g. an organelle, a cell, a tissue, a plant, which was not modulated, modified or treated according to the herein described process of the invention and is in any other property as similar to the subject matter of the invention as possible. The reference, control or wild type is in its genome, transcriptome, proteome or metabolome as similar as possible to the subject of the present invention. Preferably, the term "reference-" "control-" or "wild type-"-organelle, -cell, -tissue or plant, relates to an organelle, cell, tissue or plant, which is nearly genetically identical to the organelle, cell, tissue or plant, of the present invention or a part thereof preferably 95%, more preferred are 98%, even more preferred are 99,00%, in particular 99,10%, 99,30%, 99,50%, 99,70%, 99,90%, 99,99%, 99, 999% or more. Most preferably the "reference", "control", or "wild type" is a subject, e.g. an organelle, a cell, a tissue, a plant, which is genetically identical to the plant, cell organelle used according to the method of the invention except that nucleic acid molecules or the gene product encoded by them are changed, modulated or modified according to the inventive method.

The term "expression" or "gene expression" is as defined herein, preferably it results in the appearance of a phenotypic trait as a consequence of the transcription of a specific gene or specific genes.

The increase referring to the activity of the polypeptide amounts in a cell, a tissue, a organelle, an organ or an organism or a part thereof preferably to at least 5%, preferably to at least 10% or at to least 15%, especially preferably to at least 20%, 25%, 30% or more, very especially preferably are to at least 40%, 50% or 60%, most preferably are to at least 70% or more in comparison to the control, reference or wild type.

The term "increased yield" as defined herein is taken to mean an increase in biomass (weight) of one or more parts of a plant, which may include aboveground (harvestable) parts and/or (harvestable) parts below ground. In a preferred embodiment, the increased yield is increased seed yield.

Therefore, such harvestable parts are preferably seeds, and performance of the methods of the invention results in plants having increased seed yield relative to the seed yield of control plants.

An increase in seed yield may also be manifested as an increase in seed size and/or seed volume, which may also influence the composition of seeds (including oil, protein and carbohydrate total content and composition).

Taking corn as an example, a yield increase may be manifested as one or more of the following: increase in the number of plants per square meter, an increase in the number of ears per plant, an increase in the number of rows, number of kernels per row, kernel weight, thousand kernel weight, ear length/diameter, increase in seed filling rate (which is the number of filled seeds divided by the total number of seeds and multiplied by 100), among others. Taking rice as an example, a yield increase may be manifested by an increase in one or more of the following: number of plants per square meter, number of panicles per plant, number of spikelets per panicle, number of flowers (florets) per panicle (which is expressed as a ratio of the number of filled seeds over the number of primary panicles), increase in the seed filling rate, (which is the number of filled seeds divided by the total number of seeds and multiplied by 100), increase in thousand kernel weight, among others. An increase in yield may also result in modified architecture, or may occur as a result of modified architecture.

According to a preferred feature, performance of the methods of the invention result in plants having increased yield, particularly seed yield. Therefore, according to the present invention, there is provided a method for increasing plant yield, which method comprises modulating expression in a plant of a nucleic acid encoding the AZ polypeptide or a homologue thereof.

Since the transgenic plants according to the present invention have increased yield, it is likely that these plants exhibit an increased growth rate (during at least part of their life cycle), relative to the growth rate of control plants at a corresponding stage in their life cycle. The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as early vigour, growth rate, greenness index, flowering time and speed of seed maturation. The increase in growth rate may take place at one or more stages in the life cycle of a plant or during substantially the whole plant life cycle. Increased growth rate during the early stages in. the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the planting and harvesting of corn plants followed by, for example, the planting and optional harvesting of soy bean, potato or any other suitable plant). Harvesting additional times from the same rootstock in the case of some crop plants may also be possible. Altering the harvest cycle of a plant may lead to an increase in annual biomass production per square meter (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

According to a preferred feature of the present invention, performance of the methods of the invention gives plants having an increased growth rate or increased yield in comparison to control plants. Therefore, according to the present invention, there is provided a method for increasing yield and/or growth rate in plants, which method comprises modulating expression in a plant of a nucleic acid encoding the AZ polypeptide or a homologue thereof.

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11% or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi and insects.

In particular, the methods of the present invention may be performed under non-stress conditions or under conditions of mild drought to give plants having increased yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signaling pathways and cellular responses, such as the production of stress proteins, up-regulation of anti-oxidants, accumulation of compatible solutes and growth arrest. The term "non-stress" conditions as used herein are those environmental conditions that do not impose stress, such as the stresses described above, on plants. Non-stress conditions allow optimal growth of plants. Persons skilled in the art are aware of normal soil conditions and climatic conditions for a given location.

Performance of the methods of the invention gives plants grown under non-stress conditions or under mild drought conditions increased yield relative to control plants grown under comparable conditions. Therefore, according to the present invention, there is provided a method for increasing yield in plants grown under non-stress conditions or under mild drought conditions, which method comprises increasing expression in a plant of a nucleic acid encoding a AZ polypeptide.

In a preferred embodiment of the invention, the increase in yield and/or growth rate occurs according to the methods of the present invention under non-stress conditions.

Performance of the methods of the invention gives plants grown under conditions of nutrient deficiency, particularly under conditions of nitrogen deficiency, increased yield relative to control plants grown under comparable conditions. Therefore, according to the present invention, there is provided a method for increasing yield in plants grown under conditions of nutrient deficiency, which method comprises increasing expression in a plant of a nucleic acid encoding a AZ polypeptide. Nutrient deficiency may result from a lack of nutrients such as nitrogen, phosphates and other phosphorous-containing compounds, potassium, calcium, cadmium, magnesium, manganese, iron and boron, amongst others.

The methods of the invention are advantageously applicable to any plant. The abovementioned growth characteristics may advantageously be modified in any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum and oats.

Other advantageous plants are selected from the group consisting of Asteraceae such as the genera *Helianthus, Tagetes* e.g. the species *Helianthus annuus* [sunflower], *Tagetes lucida, Tagetes erecta* or *Tagetes tenuifolia* [Marigold]; Brassicaceae such as the genus *Brassica,* e.g. the species *Brassica napus, Brassica rapa* ssp. [canola, oilseed rape, turnip rape]; Fabaceae such as the genera *Glycine* e.g. the species *Glycine max, Soja hispida* or *Soja max* [soybean]; Linaceae such as the genera *Linum* e.g. the species *Linum usitatissimum,* [flax, linseed]; Poaceae such as the genera *Hordeum, Secale, Avena, Sorghum, Oryza, Zea, Triticum* e.g. the species *Hordeum vulgare* [barley], *Secale cereale* [rye], *Avena sativa, Avena fatua, Avena byzantina, Avena fatua* var. sativa, *Avena hybrida* [oat], *Sorghum bicolor* [sorghum, millet], *Oryza sativa, Oryza latifolia* [rice], *Zea mays* [corn, maize] *Triticum aestivum, Triticum durum, Triticum turgidum, Triticum hybernum, Triticum macha, Triticum sativum* or *Triticum vulgare* [wheat, bread wheat, common wheat]; Solanaceae such as the genera *Solanum, Lycopersicon* e.g. the species *Solanum tuberosum* [potato], *Lycopersicon esculentum, Lycopersicon lycopersicum, Lycopersicon pyriforme, Solanum integrifolium* or *Solanum lycopersicum* [tomato].

The term "AtAZ polypeptide or a homologue thereof' as defined herein refers to proteins that comprise at least one ankyrin repeat and at least one Zinc-finger C3H1 domain, which ankyrin repeat is located upstream of the C3H1 domain. Preferably, the AZ protein comprises two ankyrin repeats and two Zinc-finger C3H1 domains such as in the protein represented in SEQ ID NO: 2. Also preferably, the two ankyrin repeats are located close to each other. Further preferably, the Zinc-finger C3H1 domains are also located close to each other and C-terminally of the ankyrin repeats. In SEQ ID NO: 2, the ankyrin repeats are located on positions D90 to R120 and D125 to L157, the two Zn-finger domains are located on positions H301 to V327 and Q336 to P359 (Figure 1).

Also preferably, the AtAZ protein comprises at least one of the following consensus sequences:

(P/A)CSRAY(S/T)HDWTEC (motif 1, SEQ ID NO: 3)
HPGENARRRDPR (motif 2, SEQ ID NO: 4)
HG(V/I)FE(C/S)WLHP(A/S)QY(R/K)TRLCK (motif 3, SEQ ID NO: 5)
CFFAH (motif 4, SEQ ID NO: 6)

Preferably motif 1 is PCSRAYSHDWTEC, and motif 3 is preferably HGVFECWLHPAQYRTRLCK.
More preferably, the AtAZ protein comprises two of the above-mentioned motifs, especially preferably 3 of the above-mentioned motifs, most preferably all four motifs.

The ankyrin repeat (SMART SM00248, Interpro IPR002110), as described in the Interpro database, is one of the most common protein-protein interaction motifs in nature. Ankyrin repeats are (usually tandemly) repeated modules of usually about 33 amino acids. They occur in a large number of functionally diverse proteins mainly from eukaryotes. The few known examples from prokaryotes and viruses may be the result of horizontal gene transfers. The repeat has been found in proteins of diverse function such as transcriptional initiators, cell-cycle regulators, cytoskeletal, ion transporters and signal transducers. The ankyrin fold appears to be defined by its structure rather than its function since there is no specific sequence or structure which is universally recognised by it. The conserved fold of the ankyrin repeat unit is known from several crystal and solution structures. Each repeat folds into a helix-loop-helix structure with a beta-hairpin/loop region projecting out from the helices at a 90° angle. The repeats stack together to form an L-shaped structure.

The Zinc-finger domain ZnF_C3H1 (also known as Znf_CCCH, SMART SM00356; Interpro IPR000571), as described in the Interpro database is thought to be involved in DNA-binding. Zinc fingers exist as different types, depending on the positions of the cysteine residues. Proteins containing zinc finger domains of the C-x8-C-x5-C-x3-H type (wherein x represents any amino acid and the digits 8, 5 and 3 represent the number of amino acids between the conserved C or H residues) include zinc finger proteins from eukaryotes involved in cell cycle or growth phase-related regulation, e.g. human TIS11B (butyrate response factor 1), a probable regulatory protein involved in regulating the response to growth factors, and the mouse TTP growth factor-inducible nuclear protein, which has the same function. The mouse TTP protein is induced by growth factors. Another protein containing this domain is the human splicing factor U2AF 35 kD subunit, which plays a critical role in both constitutive and enhancer-dependent splicing by mediating essential protein-protein interactions and protein-RNA interactions required for 3' splice site selection. It has been shown that different CCCH zinc finger proteins interact with the 3' untranslated region of various mRNA. This type of zinc finger is very often present in two copies.

Figure 2 describes the consensus sequences as defined in the SMART database for the ankyrin domain and the zinc-finger domain; however it should be noted that these consensus sequences might be biased towards animal protein sequences.

The terms "domain" and "motif' are defined in the "definitions" section herein. Specialist databases exist for the identification of domains. The C3H1 or ankyrin domain in a AZ protein may be identified using, for example, SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAIPress, Menlo Park; Hulo et al., Nucl. Acids. Res. 32:D134-D137, (2004)) or Pfam (Bateman et al., Nucleic Acids Research 30(1): 276-280 (2002)). A set of tools for in silico analysis of protein sequences is available on the ExPASY proteomics server (hosted by the Swiss Institute of Bioinformatics (Gasteiger et al., ExPASy: the proteomics server for in-depth protein knowledge and analysis, Nucleic Acids Res. 31:3784-3788(2003)). The AZ protein sequence was analysed with the SMART tool (version 4.1; Schultz et al. (1998) Proc. Natl. Acad. Sci. USA 95, 5857-5864; Letunic et al. (2002) Nucleic Acids Res 30, 242-244) and was used to screen the Pfam (Version 17.0, March 2005; Bateman et al. (2004) Nucl. Acids Res. 32, D138-141) and InterPro database (Release 11.0, 26 July 2005; Mulder et al. (2005) Nucl. Acids. Res. 33, D201-205).

By aligning other protein sequences with SEQ ID NO: 2, the corresponding consensus sequences, the C3H1 domain, the ankyrin domain or other sequence motifs may easily be identified. In this way, AZ polypeptides or homologues thereof (encompassing orthologues and paralogues) may readily be identified, using routine techniques well known in the art, such as by sequence alignment. Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the alignment of two complete sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information. Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains (such as the C3H1 or ankyrin domain, or one of the motifs defined above) may be used as well. The sequence identity values, which are indicated below as a percentage were determined over the entire conserved domain or nucleic acid or amino acid sequence using the programs mentioned above using the default parameters.

Examples of AZ proteins or homologues thereof include the protein sequences listed in Table A of Example 1.

It is to be understood that sequences falling under the definition of "AZ polypeptide or homologue thereof' are not to be limited to the polypeptide sequences listed in Table A of Example 1, but that any polypeptide comprising at least one ankyrin repeat, and at least one C3H1 zinc finger domain and preferably also at least one of the consensus sequence of SEQ ID NO: 3, 4, 5 or 6 as defined above, may be suitable for use in the methods of the invention. Preferably, the polypeptide is a polypeptide from *Arabidopsis thaliana.*

Encompassed by the term "homologues" are orthologous sequences and paralogous sequences. Orthologues and paralogues may be found by performing a so-called reciprocal blast search. This may be done by a first BLAST involving BLASTing a query sequence (for example, SEQ ID NO: 1 or SEQ ID NO: 2) against any sequence database, such as the publicly available NCBI database. BLASTN or TBLASTX (using standard default values) may be used when starting from a nucleotide sequence and BLASTP or TBLASTN (using standard default values) may be used when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 1 or SEQ ID NO: 2, the second BLAST would therefore be against *Arabidopsis* sequences). The results of the first and second BLASTs are then compared. A paralogue is identified if a high-ranking hit from the second BLAST is from the same species as from which the query sequence is derived; an orthologue is identified if a high-ranking hit is not from the same species as from which the query sequence is derived. Preferred orthologues are orthologues of SEQ ID NO: 1 or SEQ ID NO: 2. High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. Preferably the score is greater than 50, more preferably greater than 100; and preferably the E-value is less than e-5, more preferably less than e-6. In the case of large families, ClustalW may be used, followed by the generation of a neighbour joining tree, to help visualize clustering of related genes and to identify orthologues and paralogues. Examples of sequences orthologous to SEQ ID NO: 2 include SEQ ID NO: 11, SEQ ID NO: 15 and SEQ ID NO: 17. SEQ ID NO: 19 is an example of a paralogue of SEQ ID NO: 2.

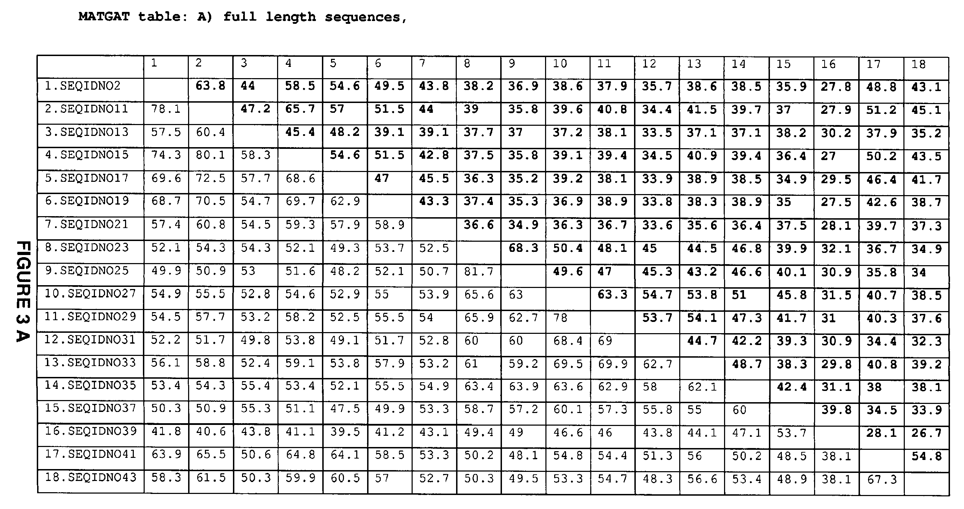

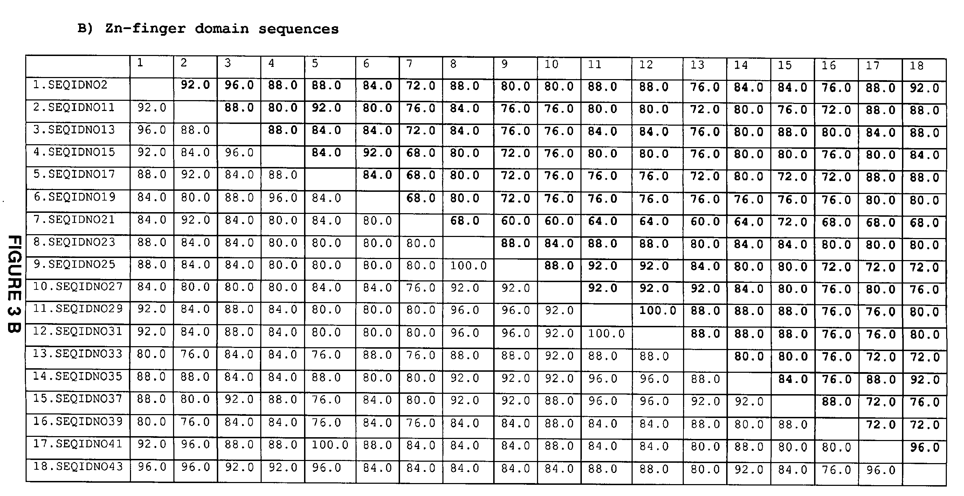

Preferably, the AZ proteins useful in the methods of the present invention have, besides at least one ankyrin repeat and at least one C3H1 domain, in increasing order of preference, at least 26%, 30%, 35%, 40%, 45%, 50%, 55%, 60%, 65%, 70%, 75%, 80%, 85%, 90%, 95%, 96%, 97%, 98% or 99% sequence identity to the protein of SEQ ID NO: 2. The matrix shown in Figure 3 (Matrix A) shows similarities and identities (in bold) over the full-length of various AZ proteins. In case only specific domains are compared, the identity or similarity may be higher among the different proteins (Matrix B: comparison of a Zn-finger domain sequence). An assay may be carried out to determine AZ activity. DNA-binding activity and protein-protein interactions may readily be determined *in vitro* or *in vivo* using techniques well known in the art. Examples of *in vitro* assays for DNA binding activity include: gel retardation analysis or yeast one-hybrid assays. An example of an *in vitro* assay for protein-protein interactions is the yeast two-hybrid analysis (Fields and Song (1989) Nature 340:245-6).

Furthermore, expression of the AZ protein or of a homologue thereof in plants, and in particular in rice, has the effect of increasing yield of the transgenic plant when compared to corresponding wild type plants, wherein increased yield comprises at least one of: total weight of seeds, total number of seeds and number of filled seeds.

An AZ polypeptide or homologue thereof is encoded by an *AZ* nucleic acid/gene. Therefore the term "*AZ* nucleic acid/gene" as defined herein is any nucleic acid/gene encoding an AZ polypeptide or a homologue thereof as defined above. Examples of *AZ* nucleic acids include but are not limited to those represented in Table A of Example 1. *AZ* nucleic acids/genes and variants thereof may be suitable in practising the methods of the invention. Preferably, the variants of an *AZ* gene originate from *Arabidopsis thaliana.* Variant *AZ* nucleic acid/genes include portions of an *AZ* nucleic acid/gene, splice variants, allelic variants and/or nucleic acids capable of hybridising with an *AZ* nucleic acid/gene.

Reference herein to a "nucleic acid sequence" is taken to mean a polymeric form of a deoxyribonucleotide or a ribonucleotide polymer of any length, either double- or single-stranded, or analogues thereof, that has the essential characteristic of a natural ribonucleotide in that it can hybridise to nucleic acid sequences in a manner similar to naturally occurring polynucleotides.

The term portion as defined herein refers to a piece of DNA encoding a polypeptide comprising at least one ankyrin repeat and at least one C3H1 Zn-finger domain. A portion may be prepared, for example, by making one or more deletions to an *AZ* nucleic acid. The portions may be used in isolated form or they may be fused to other coding (or non-coding) sequences in order to, for example, produce a protein that combines several activities. When fused to other coding sequences, the resulting polypeptide produced upon translation may be bigger than that predicted for the AZ fragment. The portion is typically at least 500, 700 or 900 nucleotides in length, preferably at least 1100, 1300 or 1500 nucleotides in length, more preferably at least 1700, 1900 or 2100 nucleotides in length and most preferably at least 2300 or 2400 nucleotides in length. Preferably, the portion is a portion of a nucleic acid as represented in Table A of Example 1. Most preferably the portion of an *AZ* nucleic acid is as represented by SEQ ID NO: 1 or SEQ ID NO: 53.

The terms "fragment", "fragment of a sequence" or "part of a sequence" "portion" or "portion thereof' mean a truncated sequence of the original sequence referred to. The truncated sequence (nucleic acid or protein sequence) can vary widely in length; the minimum size being a sequence of sufficient size to provide a sequence with at least a comparable function and/or activity of the original sequence referred to or hybridising with the nucleic acid molecule of the invention or used in the process of the invention under stringent conditions, while the maximum size is not critical. In some applications, the maximum size usually is not substantially greater than that required to provide the desired activity and/or function(s) of the original sequence. A comparable function means at least 40%, 45% or 50%, preferably at least 60%, 70%, 80% or 90% or more of the function of the original sequence.

Another variant of an *AZ* nucleic acid/gene is a nucleic acid capable of hybridising under reduced stringency conditions, preferably under stringent conditions, with an *AZ* nucleic acid/gene as hereinbefore defined or with a portion as hereinbefore defined. The hybridizing sequence is typically at least 300 nucleotides in length, preferably at least 400 nucleotides in length, more preferably at least 500 nucleotides in length and most preferably at least 600 nucleotides in length.

Preferably, the hybridising sequence is one that is capable of hybridising to a nucleic acid as represented by SEQ ID NO: 1, SEQ ID NO: 10, SEQ ID NO: 12, SEQ ID NO: 14, SEQ ID NO: 16, SEQ ID NO: 18, SEQ ID NO: 20, SEQ ID NO: 22, SEQ ID NO: 24, SEQ ID NO: 26, SEQ ID NO: 28, SEQ ID NO: 30, SEQ ID NO: 32, SEQ ID NO: 34, SEQ ID NO: 36, SEQ ID NO: 38, SEQ ID NO: 40, SEQ ID NO: 42 or SEQ ID NO: 53, or to a portion of any of the aforementioned sequences, a portion being defined as above. Most preferably the hybridising sequence is capable of hybridising to SEQ ID NO: 1, to SEQ ID NO: 53, or to portions (or probes) thereof. Methods for designing probes are well known in the art. Probes are generally less than 1000 bp, 900 bp, 800 bp, 700 bp, 600 bp in length, preferably less than 500 bp, 400 bp, 300 bp 200 bp or 100 bp in length. Commonly, probe lengths for DNA-DNA hybridizations such as Southern blotting, vary between 100 and 500 bp, whereas the hybridizing region in probes for DNA-DNA hybridizations such as in PCR amplification generally are shorter than 50 but longer than 10 nucleotides, preferably they are 15, 20, 25, 30, 35, 40, 45 or 50 bp in length.

Also useful in the methods of the invention are nucleic acids encoding homologues (including orthologues or paralogues) of the amino acid sequence represented by SEQ ID NO: 2, or derivatives thereof.

Another nucleic acid variant useful in the methods of the present invention is a splice variant encoding an AZ polypeptide as defined above. Preferred splice variants are splice variants of the nucleic acid encoding a polypeptide comprising at least on ankyrin repeat and at least one C3H1 domain. Preferably, the AZ polypeptide or the homologue thereof encoded by the splice variant has at least 26%, 30%, 35%, 40%, 45%, 50%, 55%, 60%, 65%, 70%, 75%, 80%, 85%, 90%, 95%, 96%, 97%, 98% or 99% sequence identity to SEQ ID NO: 2. Further preferred are splice variants represented by the nucleic acids represented in Table A of Example 1. For example, SEQ ID NO: 25 and SEQ ID NO: 47 are encoded by splice variants of the same gene. Most preferred is the splice variant represented by SEQ ID NO: 1 or SEQ ID NO: 53.

Another nucleic acid variant useful in the methods of the present invention is an allelic variant of a nucleic acid encoding an AZ polypeptide as defined above. Preferably, the polypeptide encoded by the allelic variant is represented by the polypeptide sequences listed in Table A of Example 1. Most preferably, the allelic variant encoding the AZ polypeptide is represented by SEQ ID NO: 1.

A further nucleic acid variant useful in the methods of the invention is a nucleic acid variant obtained by gene shuffling. Furthermore, site-directed mutagenesis may be used to generate variants of *AZ* nucleic acids. Several methods are available to achieve site-directed mutagenesis; the most common being PCR based methods (Current Protocols in Molecular Biology. Wiley Eds.).

Therefore, the invention provides a method for increasing yield and/or growth rate of a plant, comprising modulating expression in a plant of a variant of an *AZ* nucleic acid selected from:

(i) a portion of an *AZ* nucleic acid;
(ii) a nucleic acid hybridising to an *AZ* nucleic acid;
(iii) a splice variant of a nucleic acid encoding an AZ polypeptide;
(iv) an allelic variant of a nucleic acid encoding an AZ polypeptide; and
(v) a nucleic acid variant encoding an AZ polypeptide obtained by gene shuffling or site directed mutagenesis.

The *AZ* nucleic acid or variant thereof may be derived from any natural or artificial source. This nucleic acid may be modified from its native form in composition and/or genomic environment through deliberate human manipulation. The nucleic acid is preferably of plant origin, further preferably from a dicotyledonous species, further preferably from the family Brassicaceae, more preferably from *Arabidopsis thaliana.* Most preferably, the *AZ* nucleic acid is the *Arabidopsis thaliana* sequence represented by SEQ ID NO: 1, and the AZ amino acid sequence is as represented by SEQ ID NO: 2. Alternatively, the *AZ* nucleic acid represented by SEQ ID NO: 53 or the AZ amino acid sequence as represented by SEQ ID NO: 47 may also be useful in the methods of the present invention.

Any reference herein to an AZ polypeptide is therefore taken to mean an AZ protein as defined above. Any nucleic acid encoding such an AZ protein is suitable for use in the methods of the invention.

According to a preferred aspect of the present invention, modulated, preferably increased expression of the *AZ* nucleic acid or variant thereof is envisaged. Methods for increasing the expression of genes or gene products are well documented in the art. The expression of a nucleic acid sequence encoding an AZ polypeptide may be modulated by introducing a genetic modification, which may be introduced, for example, by any one (or more) of the following methods: T-DNA activation, TILLING, site-directed mutagenesis, directed evolution and homologous recombination or by introducing and expressing in a plant a nucleic acid encoding an AZ polypeptide or a homologue thereof. Following introduction of the genetic modification, there follows a step of selecting for modified expression of a nucleic acid encoding an AZ polypeptide or a homologue thereof, which modification in expression gives plants having increased yield. Also methods for decreasing the expression of genes or gene products are known in the art.

T-DNA activation is described above. A genetic modification may also be introduced in the locus of an *AZ* gene using the technique of TILLING (Targeted Induced Local Lesions In Genomes). The locus of a gene as defined herein is taken to mean a genomic region, which includes the gene of interest and 10 kb up- or down stream of the coding region. Site-directed mutagenesis may be used to generate variants of *AZ* nucleic acids. Several methods are available to achieve site-directed mutagenesis; the most common being PCR based methods (Current Protocols in Molecular Biology. Wiley Eds.). The effects of the invention may also be produced using homologous recombination.

A preferred method for introducing a genetic modification is to introduce and express in a plant a nucleic acid encoding an AZ polypeptide or a homologue thereof, as defined above. The nucleic acid to be introduced into a plant may be a full-length nucleic acid or may be a portion or a hybridising sequence as hereinbefore defined.

The invention furthermore provides genetic constructs and vectors to facilitate introduction and/or expression of the nucleotide sequences useful in the methods according to the invention.

Therefore, there is provided a gene construct comprising:

(i) an *AZ* nucleic acid or variant thereof, as defined hereinabove;
(ii) one or more control sequences operably linked the nucleic acid sequence of (i);

Constructs useful in the methods according to the present invention may be created using recombinant DNA technology well known to persons skilled in the art. The gene constructs may be inserted into vectors, which may be commercially available, suitable for transforming into plants and suitable for expression of the gene of interest in the transformed cells. The invention therefore provides use of a gene construct as defined hereinabove in the methods of the invention.

Plants are transformed with a vector comprising the sequence of interest (i.e., a nucleic acid encoding an AZ polypeptide or homologue thereof). The skilled artisan is well aware of the genetic elements that must be present on the vector in order to successfully transform, select and propagate host cells containing the sequence of interest. The sequence of interest is operably linked to one or more control sequences (at least to a promoter). The terms "regulatory element", "control sequence" and "promoter" are defined above. Advantageously, any type of promoter may be used to drive expression of the nucleic acid sequence.

Suitable promoters, which are functional in plants, are generally known. They may take the form of constitutive or inducible promoters. Suitable promoters can enable the developmental- and/or tissue-specific expression in multicellular eukaryotes; thus, leaf-, root-, flower-, seed-, stomata-, tuber- or fruit-specific promoters may advantageously be used in plants.

Different plant promoters usable in plants are promoters such as, for example, the USP, the LegB4-, the DC3 promoter or the ubiquitin promoter from parsley.

A "plant" promoter comprises regulatory elements, which mediate the expression of a coding sequence segment in plant cells. Accordingly, a plant promoter need not be of plant origin, but may originate from viruses or micro-organisms, in particular for example from viruses which attack plant cells.

The "plant" promoter can also originate from a plant cell, e.g. from the plant which is transformed with the nucleic acid sequence to be expressed in the inventive process and described herein. This also applies to other "plant" regulatory signals, for example in "plant" terminators.

For expression in plants, the nucleic acid molecule must, as described above, be linked operably to or comprise a suitable promoter which expresses the gene at the right point in time and in a cell- or tissue-specific manner. Useful promoters are constitutive promoters (Benfey et al., EMBO J. 8 (1989) 2195-2202), such as those which originate from plant viruses, such as 35S CAMV (Franck et al., Cell 21 (1980) 285-294), 19S CaMV (see also US 5352605 and WO 84/02913), 34S FMV (Sanger et al., Plant. Mol. Biol., 14, 1990: 433-443), the parsley ubiquitin promoter, or plant promoters such as the Rubisco small subunit promoter described in US 4,962,028 or the plant promoters PRP1 [Ward et al., Plant. Mol. Biol. 22 (1993)], SSU, PGEL1, OCS [Leisner (1988) Proc Natl Acad Sci USA 85(5): 2553-2557], lib4, usp, mas [Comai (1990) Plant Mol Biol 15 (3):373-381], STLS1, ScBV (Schenk (1999) Plant Mol Biol 39(6):1221-1230), B33, SAD1 or SAD2 (flax promoters, Jain et al., Crop Science, 39 (6), 1999: 1696-1701) or nos [Shaw et al. (1984) Nucleic Acids Res. 12(20):7831-7846]. Further examples of constitutive plant promoters are the sugar beet V-ATPase promoters (WO 01/14572). Examples of synthetic constitutive promoters are the Super promoter (WO 95/14098) and promoters derived from G-boxes (WO 94/12015). If appropriate, chemically inducible promoters may furthermore also be used, compare EP-A 388186, EP-A 335528, WO 97/06268. Stable, constitutive expression of the proteins according to the invention a plant can be advantageous. However, inducible expression of the polypeptide of the invention is advantageous, if, for example, late expression before the harvest is of advantage, as metabolic manipulation may lead to plant growth retardation.

The expression of plant genes can also be facilitated via a chemically inducible promoter. Chemically inducible promoters are particularly suitable when it is desired to express the gene in a time-specific manner. Examples of such promoters are a salicylic acid inducible promoter (WO 95/19443), and abscisic acid-inducible promoter (EP 335 528), a tetracyclin-inducible promoter (Gatz et al. (1992) Plant J. 2, 397-404), a cyclohexanol- or ethanol-inducible promoter (WO 93/21334) or others as described herein.

Other suitable promoters are those which react to biotic or abiotic stress conditions, for example the pathogen-induced PRP1 gene promoter (Ward et al., Plant. Mol. Biol. 22 (1993) 361-366), the tomato heat-inducible hsp80 promoter (US 5,187,267), the potato chill-inducible alpha-amylase promoter (WO 96/12814) or the wound-inducible pinII promoter (EP-A-0 375 091) or others as described herein.

Preferred promoters are in particular those which bring gene expression in tissues and organs, in seed cells, such as endosperm cells and cells of the developing embryo. Suitable promoters are the oilseed rape napin gene promoter (US 5,608,152), the *Vicia faba* USP promoter (Baeumlein et al., Mol Gen Genet, 1991, 225 (3): 459-67), the *Arabidopsis* oleosin promoter (WO 98/45461), the *Phaseolus vulgaris* phaseolin promoter (US 5,504,200), the *Brassica* Bce4 promoter (WO 91/13980), the bean arc5 promoter, the carrot DcG3 promoter, or the Legumin B4 promoter (LeB4; Baeumlein et al., 1992, Plant Journal, 2 (2): 233-9), and promoters which bring about the seed-specific expression in monocotyledonous plants such as maize, barley, wheat, rye, rice and the like. Advantageous seed-specific promoters are the sucrose binding protein promoter (WO 00/26388), the phaseolin promoter and the napin promoter. Suitable promoters which must be considered are the barley Ipt2 or Ipt1 gene promoter (WO 95/15389 and WO 95/23230), and the promoters described in WO 99/16890 (promoters from the barley hordein gene, the rice glutelin gene, the rice oryzin gene, the rice prolamin gene, the wheat gliadin gene, the wheat glutelin gene, the maize zein gene, the oat glutelin gene, the sorghum kasirin gene and the rye secalin gene). Further suitable promoters are Amy32b, Amy 6-6 and Aleurain [US 5,677,474], Bce4 (oilseed rape) [US 5,530,149], glycinin (soya) [EP 571 741], phosphoenolpyruvate carboxylase (soya) [JP 06/62870], ADR12-2 (soya) [WO 98/08962], isocitrate lyase (oilseed rape) [US 5,689,040] or $\alpha$-amylase (barley) [EP 781 849]. Other promoters which are available for the expression of genes in plants are leaf-specific promoters such as those described in DE-A 19644478 or light-regulated promoters such as, for example, the pea petE promoter.

Further suitable plant promoters are the cytosolic FBPase promoter or the potato ST-LSI promoter (Stockhaus et al., EMBO J. 8, 1989, 2445), the Glycine max phosphoribosylpyrophosphate amidotransferase promoter (GenBank Accession No. U87999) or the node-specific promoter described in EP-A-0 249 676.

According to one preferred feature of the invention, the *AZ* nucleic acid or variant thereof is operably linked to a seed-specific promoter. The seed-specific promoter may be active during seed development and/or during germination. Seed-specific promoters are well known in the art. Preferably, the seed-specific promoter is an embryo specific / endosperm specific /aleurone specific promoter. Further preferably, the seed-specific promoter drives expression in at least one of: the embryo, the endosperm, the aleurone. More preferably, the promoter is a WS118 or a functionally equivalent promoter. Most preferably, the promoter sequence is as represented by SEQ ID NO: 9 or SEQ ID NO: 55. It should be clear that the applicability of the present invention is not restricted to the *AZ* nucleic acid represented by SEQ ID NO: 1 or SEQ ID NO: 53, nor is the applicability of the invention restricted to expression of an *AZ* nucleic acid when driven by a seed-specific promoter. Examples of other seed-specific promoters (embryo specific / endosperm specific / aleurone specific promoters) which may also be used to drive expression of an *AZ* nucleic acid are provided above.

According to another preferred feature of the invention, the *AZ* nucleic acid or variant thereof is operably linked to a constitutive promoter. A preferred constitutive promoter is a constitutive promoter that is also substantially ubiquitously expressed. Further preferably the promoter is derived from a plant, more preferably a monocotyledonous plant. An example of such a promoter is the GOS2 promoter from rice (SEQ ID NO: 54 or SEQ ID NO: 56). It should be clear that the applicability of the present invention is not restricted to the *AZ* nucleic acid represented by SEQ ID NO: 1, nor is the applicability of the invention restricted to expression of a *AZ* nucleic acid when driven by a constitutive promoter, and in particular by a GOS2 promoter. Examples of other constitutive promoters which may also be used to drive expression of an *AZ* nucleic acid are shown above.

Optionally, one or more terminator sequences may be used in the construct introduced into a plant. Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

The present invention also encompasses plants, plant parts or plant cells obtainable by the methods according to the present invention. The present invention therefore provides plants obtainable by the method according to the present invention, which plants have introduced therein an AZ nucleic acid or variant thereof, as defined above.

The invention also provides a method for the production of transgenic plants having increased yield, comprising introduction and expression in a plant of an *AZ* nucleic acid or a variant thereof as defined above.

Host plants for the nucleic acids or the vector used in the method according to the invention, the expression cassette or construct or vector are, in principle, advantageously all plants, which are capable of synthesizing the polypeptides used in the inventive method.

More specifically, the present invention provides a method for the production of transgenic plants having increased yield, which method comprises:

(i) introducing and expressing in a plant or plant cell an *AZ* nucleic acid or variant thereof; and
(ii) cultivating the plant cell under conditions promoting plant growth and development.

The nucleic acid may be introduced directly into a plant cell or into the plant itself (including introduction into a tissue, organ or any other part of a plant). According to a preferred feature of the present invention, the nucleic acid is preferably introduced into a plant by transformation. The terms "introduction" or "transformation" are described in more detail in the definitions section.

Generally after transformation, plant cells or cell groupings are selected for the presence of one or more markers which are encoded by plant-expressible genes co-transferred with the gene of interest, following which the transformed material is regenerated into a whole plant. As mentioned, Agrobacteria transformed with an expression vector according to the invention may also be used in the manner known per se for the transformation of plants such as plants used as a model, like *Arabidopsis* (*Arabidopsis thaliana* is within the scope of the present invention not considered as a crop plant), or crop plants, such as cereals, maize, oats, rye, barley, wheat, soybean, rice, cotton, sugar beet, canola, sunflower, flax, hemp, potato, tobacco, tomato, carrot, bell peppers, oilseed rape, tapioca, cassava, arrow root, tagetes, alfalfa, lettuce and the various tree, nut, and grapevine species, in particular oil-containing crop plants such as soy, peanut, castor-oil plant, sunflower, maize, cotton, flax, oilseed rape, coconut, oil palm, safflower (*Carthamus tinctorius*) or cocoa beans, for example by bathing scarred leaves or leaf segments in an agrobacterial solution and subsequently culturing them on suitable media.

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention. The invention also includes host cells containing an isolated *AZ* nucleic acid or variant thereof. Preferred host cells according to the invention are plant cells. The invention also extends to harvestable parts of a plant such as, but not limited to seeds, leaves, fruits, flowers, stem, roots, rhizomes, tubers and bulbs. The invention furthermore relates to products directly derived from a harvestable part of such a plant, such as dry pellets or powders, oil, fat and fatty acids, starch or proteins.

The present invention also encompasses use of *AZ* nucleic acids or variants thereof and use of AZ polypeptides or homologues thereof.

One such use relates to improving the growth characteristics of plants, in particular in improving yield, especially seed yield. The increased seed yield preferably comprises increased thousand kernel weight.

Nucleic acids encoding AZ polypeptides may find use in breeding programmes in which a DNA marker is identified which may be genetically linked to an *AZ* gene. The nucleic acids/ genes may be used to define a molecular marker. This DNA or protein marker may then be used in breeding programmes to select plants having increased yield.

Allelic variants of an *AZ* nucleic acid/gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question, for example, different allelic variants of any one of the nucleic acids listed in Table A of Example 1. Growth performance may be monitored in a greenhouse or in the field. Further optional steps include crossing plants, in which the superior allelic variant was identified, with another plant. This could be used, for example, to make a combination of interesting phenotypic features.

An *AZ* nucleic acid may also be used as probes for genetically and physically mapping the genes that they are a part of, and as markers for traits linked to those genes. Such information may be useful in plant breeding in order to develop lines with desired phenotypes. Such use of *AZ* nucleic acids or variants thereof requires only a nucleic acid sequence of at least 15 nucleotides in length. The *AZ* nucleic acids or variants thereof may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the *AZ* nucleic acids. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the *AZ* nucleic acid in the genetic map previously obtained using this population (Botstein et al. (1980) Am. J. Hum. Genet. 32: 314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence *in situ* hybridization (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having increased yield, as described hereinbefore. These advantageous growth characteristics may also be combined with other economically advantageous traits, such as further yield-enhancing traits, tolerance to various stresses, traits modifying various architectural features and/or biochemical and/or physiological features.

**Detailed description for the SYT polypeptide**

Surprisingly, it has now been found that modulating expression of a nucleic acid sequence encoding a SYT polypeptide increases plant yield and/or early vigour under abiotic stress relative to control plants. Therefore, according to the present invention, there is provided a method for increasing plant yield and/or early vigour under abiotic stress relative to control plants, comprising modulating expression in a plant of a nucleic acid sequence encoding a SYT polypeptide.

Reference herein to "control plants" is taken to mean any suitable control plant or plants.

The "reference", "control", or "wild type" are used herein interchangeably and is preferably a subject, e.g. an organelle, a cell, a tissue, a plant, which is as similar to the subject matter of the invention as possible. The reference, control or wild type is in its genome, transcriptome, proteome or metabolome as similar as possible to the subject of the present invention. Preferably, the term "reference-" "control-" or "wild type-"-organelle, -cell, -tissue or plant, relates to an organelle, cell, tissue or plant, which is nearly genetically identical to the organelle, cell, tissue or plant, of the present invention or to a part thereof, having in increasing order of preference 95%, 98%, 99,00%, 99,10%, 99,30%, 99,50%, 99,70%, 99,90%, 99,99%, 99, 999% or more genetic identity to the organelle, cell, tissue or plant, of the present invention. Most preferable the "reference", "control", or "wild type" is preferably a subject, e.g. an organelle, a cell, a tissue, a plant, which is genetically identical to the plant, tissue, cell, organelle used according to the method of the invention except that the nucleic acid sequences or the gene product in question is changed, modulated or modified according to the inventive method.

Preferably, any comparison between the control plants and the plants produced by the method of the invention is carried out under analogous conditions. The term "analogous conditions" means that all conditions such as culture or growing conditions, assay conditions (such as buffer composition, temperature, substrates, pathogen strain, concentrations and the like) are kept identical between the experiments to be compared.

Any reference hereinafter to a "protein useful in the methods of the invention" is taken to mean a SYT polypeptide as defined herein. Any reference hereinafter to a "nucleic acid useful in the methods of the invention" is taken to mean a nucleic acid capable of encoding such a SYT polypeptide. The nucleic acid to be introduced into a plant (and therefore useful in performing the methods of the invention) is any nucleic acid encoding the type of protein which will now be described, hereafter also named "*SYT* nucleic acid" or "*SYT* gene".

The term "sequence" relates to polynucleotides, nucleic acids, nucleic acid molecules, peptides, polypeptides and proteins, depending on the context in which the term "sequence" is used. A "coding sequence" is a nucleic acid sequence, which is transcribed into mRNA and/or translated into a polypeptide when placed under the control of appropriate regulatory sequences. The boundaries of the coding sequence are determined by a translation start codon at the 5'-terminus and a translation stop codon at the 3'-terminus. A coding sequence can include, but is not limited to mRNA, cDNA, recombinant nucleic acid sequences or genomic DNA, while introns may be present as well under certain circumstances. The term "expression" or "gene expression" is as defined above. The term "modulation" is defined above and means in relation to expression or gene expression, an increase in expression.

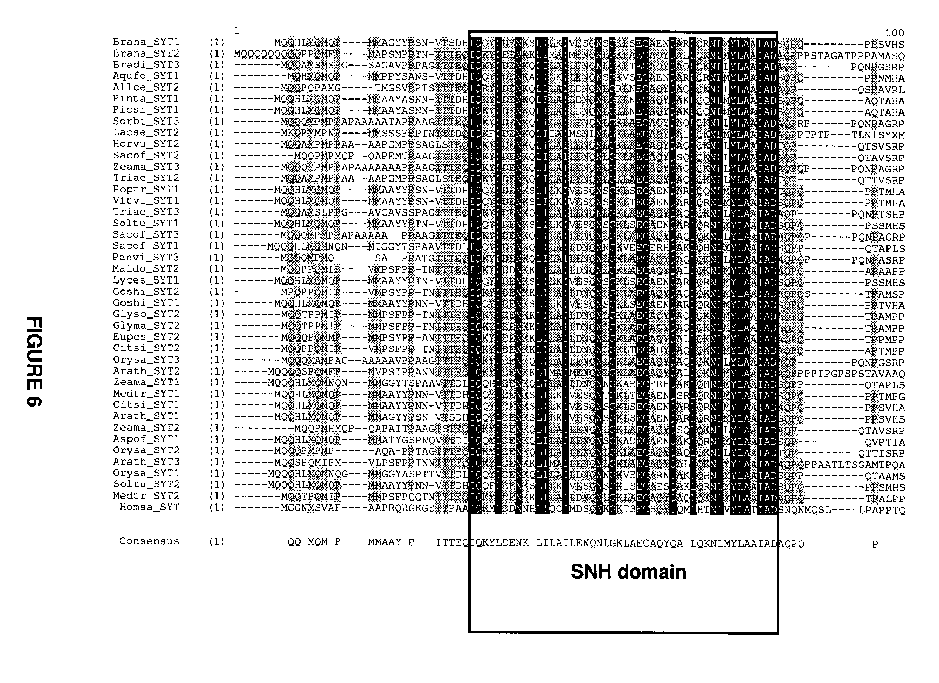

The term "SYT polypeptide" as defined herein refers to a polypeptide comprising from N-terminal to C-terminal: (i) an SNH domain having in increasing order of preference at least 20%, 25%, 30%, 35%, 40%, 45%, 50%, 55%, 60%, 65%, 70%, 75%, 80%, 85%, 90%, 91 %, 92%, 93%, 94%, 95%, 96%, 97%, 98%, 99% sequence identity to the SNH domain of SEQ ID NO: 58; and (ii) a Met-rich domain; and (iii) a QG-rich domain. Preferably, the SNH domain comprises the residues shown in black in Figure 6. Further preferably, the SNH domain is as represented by SEQ ID NO: 57.

Additionally, the SYT polypeptide may comprise one or more of the following: (a) SEQ ID NO: 146; (b) SEQ ID NO: 147; and (c) a Met-rich domain at the N-terminal preceding the SNH domain.

A SYT polypeptide is encoded by a SYT nucleic acid sequence. Therefore the term "SYT nucleic acid sequence" as defined herein is any nucleic acid sequence encoding a SYT polypeptide as defined hereinabove.

The terms "motif' and "domain" are defined in the definitions section. Specialist databases exist for the identification of domains, for example, SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAI Press, Menlo Park; Hulo et al., Nucl. Acids. Res. 32:D134-D137, (2004)), or Pfam (Bateman et al., Nucleic Acids Research 30(1): 276-280 (2002)). A set of tools for *in silico* analysis of protein sequences is available on the ExPASy proteomics server (Swiss Institute of Bioinformatics (Gasteiger et al., ExPASy: the proteomics server for in-depth protein knowledge and analysis, Nucleic Acids Res. 31: 3784-3788(2003)). Domains or motifs may also be identified using routine techniques, such as by sequence alignment.

SYT polypeptides may readily be identified using routine techniques well known in the art, such as by sequence alignment. Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the alignment of two complete sequences that maximizes the number of matches and minimizes the number of gaps. The BLAST algorithm (Basic Local Alignment Search Tool; Altschul et al. (1990) J Mol Biol 215: 403-10) calculates percent sequence identity (%) and performs a statistical analysis of the similarity between the two sequences (E-value). Percentage identity refers to the number of identical nucleotides (or amino acids) between the two compared nucleic acid (or polypeptide) sequences over a particular length. The higher the similarity between two sequences, the lower the E-value (or in other words the lower the chance that the hit was found by chance). Computation of the E-value is well known in the art. The software for performing BLAST analysis is publicly available through the National Centre for Biotechnology Information. SYT polypeptides comprising an SNH domain having in increasing order of preference at least 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% sequence identity to the SNH domain of SEQ ID NO: 58 may be identified this way. Alternatively, SYT polypeptides useful in the methods of the present invention have, in increasing order of preference, at least 30%, 35%, 40%, 45%, 50%, 55%, 60%, 65%, 70%, 75%, 80%, 85%, 90%, 95%, 96%, 97%, 98% or 99% sequence identity to the polypeptide of SEQ ID NO: 60. Minor manual editing may be performed to optimise alignment between conserved motifs, as would be apparent to a person skilled in the art. In some instances, default parameters may be adjusted to modify the stringency of the search. For example using BLAST, the statistical significance threshold (called "expect" value) for reporting matches against database sequences may be increased to show less stringent matches. In this way, short nearly exact matches may be identified. The presence of SEQ ID NO: 146 and of SEQ ID NO: 147 both comprised in the SYT polypeptides useful in the methods of the invention may be identified this way.

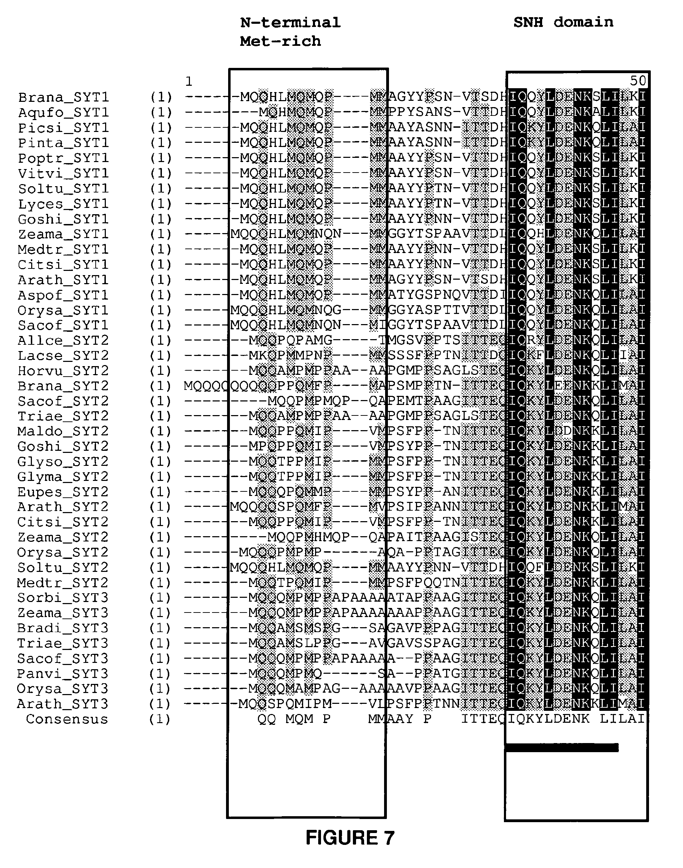

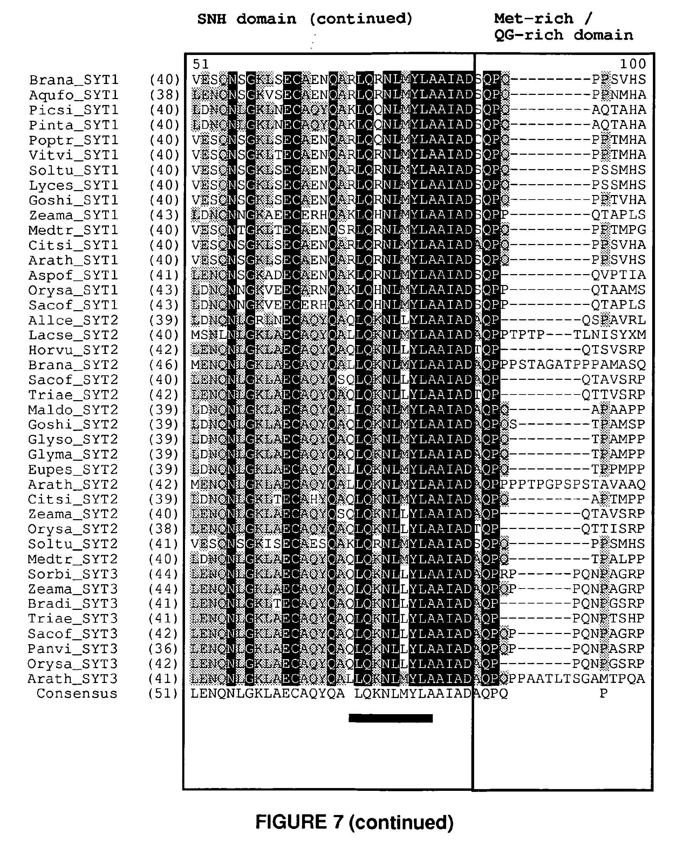

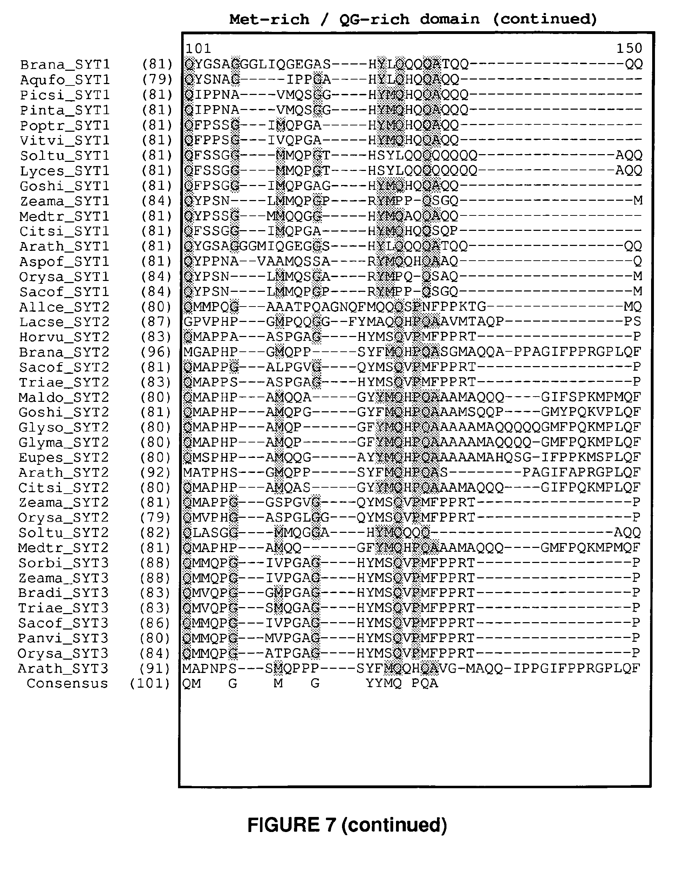

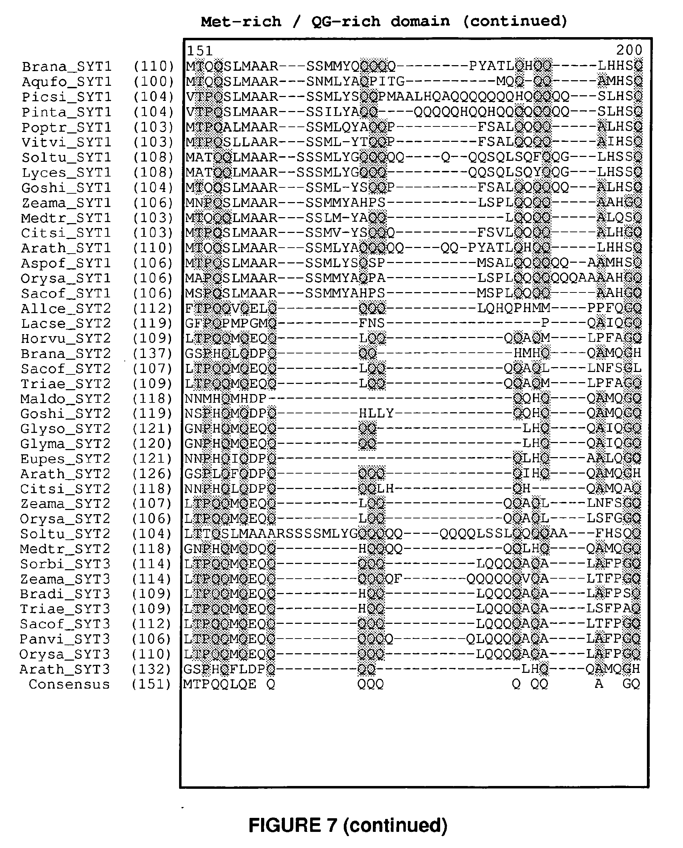

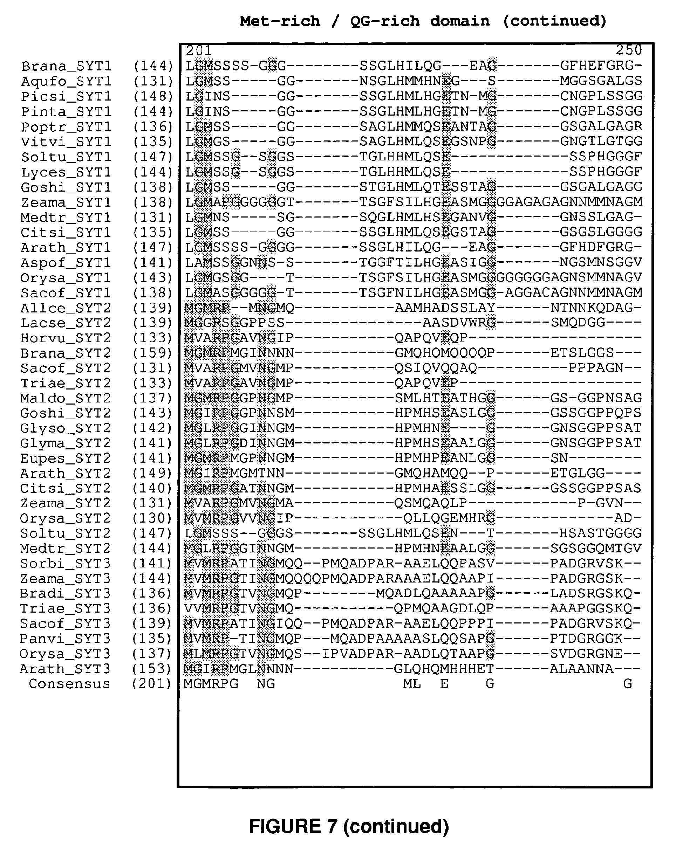

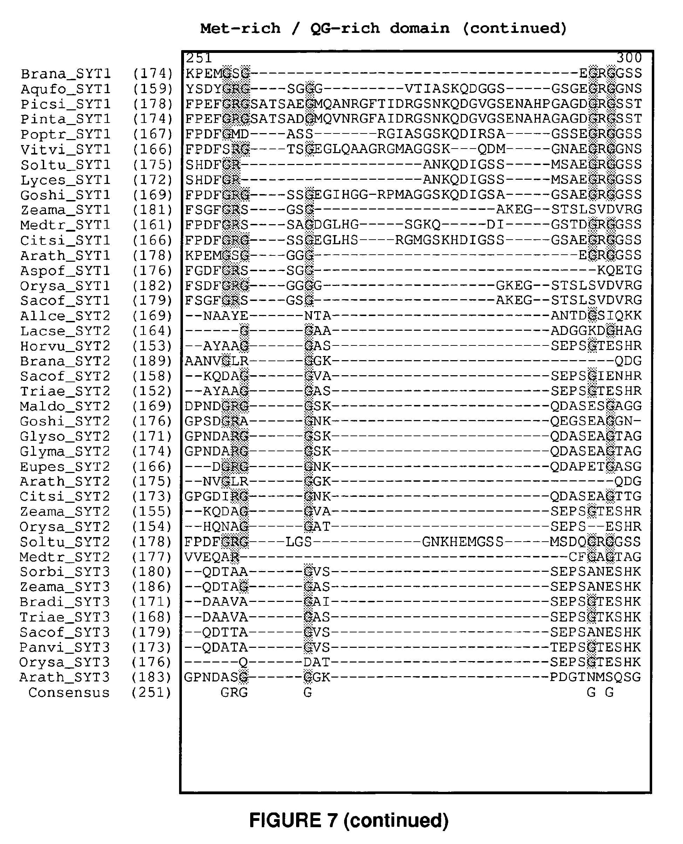

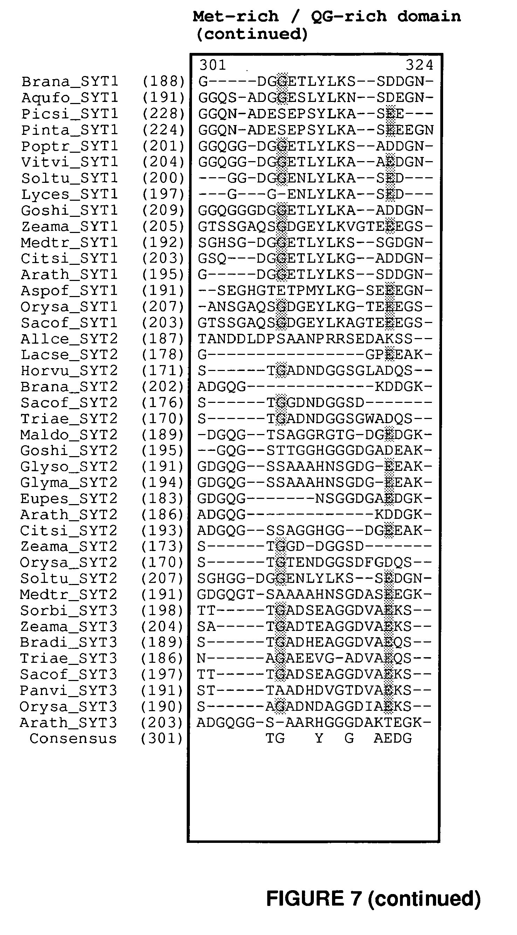

Furthermore, the presence of a Met-rich domain or a QG-rich domain may also readily be identified. As shown in Figure 7, the Met-rich domain and QG-rich domain follows the SNH domain. The QG-rich domain may be taken to be substantially the C-terminal remainder of the polypeptide (minus the SHN domain); the Met-rich domain is typically comprised within the first half of the QG-rich (from the N-term to the C-term). Primary amino acid composition (in %) to determine if a polypeptide domain is rich in specific amino acids may be calculated using software programs from the ExPASy server (Gasteiger E et al. (2003) ExPASy: the proteomics server for in-depth protein knowledge and analysis. Nucleic Acids Res 31:3784-3788), in particular the ProtParam tool. The composition of the polypeptide of interest may then be compared to the average amino acid composition (in %) in the Swiss-Prot Protein Sequence data bank (Table 5). Within this databank, the average Met (M) content is of 2.37%, the average Gin (Q) content is of 3.93% and the average Gly (G) content is of 6.93% (Table 5). As defined herein, a Met-rich domain or a QG-rich domain has Met content (in %) or a Gin and Gly content (in %) above the average amino acid composition (in %) in the Swiss-Prot Protein Sequence data bank. For example in SEQ ID NO: 60, the Met-rich domain at the N-terminal preceding the SNH domain (from amino acid positions 1 to 24) has Met content of 20.8 % and a QG-rich domain (from amino acid positions 71 to 200) has a Gin (Q) content of 18.6 % and a Gly (G) content of 21.4 %. Preferably, the Met domain as defined herein has a Met content (in %) that is at least 1.25, 1.5, 1.75, 2.0, 2.25, 2.5, 2.75, 3.0, 3.25, 3.5, 3.75, 4.0, 4.25, 4.5, 4.75, 5.0, 5.25, 5.0, 5.75, 6.0, 6.25, 6.5, 6.75, 7.0, 7.25, 7.5, 7.75, 8.0, 8.25, 8.5, 8.75, 9.0, 9.25, 9.5, 9.75, 10 or more as the average amino acid composition (in %) of said kind of protein sequences, which are included in the Swiss-Prot Protein Sequence data bank. Preferably, the QG-rich domain as defined herein has a Gln (Q) content and/or a Gly (G) content that is at least 1.25, 1.5, 1.75, 2.0, 2.25, 2.5, 2.75, 3.0, 3.25, 3.5, 3.75, 4.0, 4.25, 4.5, 4.75, 5.0, 5.25, 5.0, 5.75, 6.0, 6.25, 6.5, 6.75, 7.0, 7.25, 7.5, 7.75, 8.0, 8.25, 8.5, 8.75, 9.0, 9.25, 9.5, 9.75, 10 or more as much as the average amino acid composition (in %) of said kind of protein sequences, which are included in the Swiss-Prot Protein Sequence data bank.

**Table 5:** Mean amino acid composition (%) of proteins in SWISS PROT Protein Sequence data bank (July 2004):

| Residue | % | Residue | % |
|---|---|---|---|
| A = Ala | 7.80 | M = Met | 2.37 |
| C = Cys | 1.57 | N = Asn | 4.22 |
| D = Asp | 5.30 | P = Pro | 4.85 |
| E = Glu | 6.59 | Q = Gln | 3.93 |
| F = Phe | 4.02 | R = Arg | 5.29 |
| G = Gly | 6.93 | S = Ser | 6.89 |
| H = His | 2.27 | T = Thr | 5.46 |
| I = lie | 5.91 | V = Val | 6.69 |
| K = Lys | 5.93 | W = Trp | 1.16 |
| L = Leu | 9.62 | Y = Tyr | 3.09 |

Examples of SYT polypeptides include (encoded by polynucleotide sequence accession number in parenthesis; see also Table 6 and mentioned in the sequence protocol): *Arabidopsis thaliana* Arath_SYT1 (AY102639.1) SEQ ID NO: 60, *Arabidopsis thaliana* Arath_SYT2 (AY102640.1) SEQ ID NO: 62, *Arabidopsis thaliana* Arath_SYT3 (AY102641.1) SEQ ID NO: 64, *Aspergillus officinalis* Aspof_SYT (CV287542) SEQ ID NO: 66, *Brassica napus* Brana_SYT (CD823592) SEQ ID NO: 68, *Citrus sinensis* Citsi_SYT (CB290588) SEQ ID NO: 70, *Gossypium arboreum* Gosar_SYT (BM359324) SEQ ID NO: 72, *Medicago truncitlata* Medtr_SYT (CA858507.1) SEQ ID NO: 74, *Oryza sativa* Orysa_SYT1 (AK058575) SEQ ID NO: 76, *Oryza sativa* Orysa_SYT2 (AK105366) SEQ ID NO: 78, *Oryza sativa* Orysa_SYT3 (BP185008) SEQ ID NO: 80, *Solanum tuberosum* Soltu_SYT (BG590990) SEQ ID NO: 82, *Zea mays* Zeama_SYT1 (BG874129.1, CA409022.1) SEQ ID NO: 84, *Zea mays* Zeama_SYT2 (AY106697) SEQ ID NO: 86, *Homo sapiens* Homsa_SYT (CAG46900) SEQ ID NO: 88, *Allium cepa* Allce_SYT2 (CF437485) SEQ ID NO: 90, *Aquilegia formosa x Aquilegia pubescens* Aqufo_SYT1 (DT758802) SEQ ID NO: 92, *Brachypodium distachyon* Bradi_SYT3 (DV480064) SEQ ID NO: 94, *Brassica napus* Brana_SYT2 (CN732814) SEQ ID NO: 96, *Citrus sinensis* CitsLSYT2 (CV717501) SEQ ID NO: 98, *Euphorbia esula* Eupes_SYT2 (DV144834) SEQ ID NO: 100, *Glycine max* Glyma_SYT2 (BQ612648) SEQ ID NO: 102, *Glycine soya* Glyso_SYT2 (CA799921) SEQ ID NO: 104, *Gossypium hirsutum* Goshi_SYT1 (DT558852) SEQ ID NO: 106, *Gossypium hirsutum* Goshi_SYT2 (DT563805) SEQ ID NO: 108, *Hordeum vulgare* Horvu_SYT2 (CA032350) SEQ ID NO: 110, *Lactuca serriola* Lacse_SYT2 (DW110765) SEQ ID NO: 112, *Lycopersicon esculentum* Lyces_SYT1 (AW934450, BP893155) SEQ ID NO: 114, *Malus domestica* Maldo_SYT2 (CV084230, DR997566) SEQ ID NO: 116, *Medicago truncatula* Medtr_SYT2 (CA858743, BI310799, AL382135) SEQ ID NO: 118, *Panicum virgatum* Panvi_SYT3 (DN152517) SEQ ID NO: 120, *Picea sitchensis* PicsLSYT1 (DR484100, DR478464) SEQ ID NO: 122, *Pinus taeda* Pinta_SYT1 (DT625916) SEQ ID NO: 124, *Populus tremula* Poptr_SYT1 (DT476906) SEQ ID NO: 126, *Saccharum officinarum* Sacof_SYTI (CA078249, CA078630, CA082679, CA234526, CA239244, CA083312) SEQ ID NO: 128, *Saccharum officinarum.* Sacof_SYT2 (CA110367) SEQ ID NO: *130, Saccharum officinarum* Sacof_SYT3 (CA161933, CA265085) SEQ ID NO: 132, *Solanum tuberosum* Soltu_SYT1 (CK265597) SEQ ID NO: 134, *Sorghum bicolor* Sorbi_SYT3 (CX611128) SEQ ID NO: 136, *Triticum aestivum* Triae_SYT2 (CD901951) SEQ ID NO: 138, *Triticum aestivum* Triae_SYT3 (BJ246754, BJ252709) SEQ ID NO: 140, *Vitis vinifera* Vitvi_SYT1 (DV219834) SEQ ID NO: 142, *Zea mays* Zeama_SYT3 (CO468901) SEQ ID NO: 144, *Brassica napus* Brana_SYT SEQ ID NO: 151, *Glycine max* Glyma_SYT SEQ ID NO: 153.

**Table 6:** Examples of nucleic acid sequences encoding SYT polypeptides

| Name | NCBI nucleotide accession number | Nucleic acid sequence SEQ ID NO | Translated polypeptide SEQ ID NO | Source |
|---|---|---|---|---|
| Arath_SYT1 | AY102639.1 | 59 | 60 | *Arabidopsis thaliana* |
| Arath_SYT2 | AY102640.1 | 61 | 62 | *Arabidopsis thaliana* |
| Arath_SYT3 | AY102641.1 | 63 | 64 | *Arabidopsis thaliana* |
| Aspof_SYT1 | CV287542 | 65 | 66 | *Aspergillus officinalis* |
| Brana_SYT1 | CD823592 | 67 | 68 | *Brassica napus* |
| Citsi_SYT1 | CB290588 | 69 | 70 | *Citrus sinensis* |
| Gosar_SYT1 | BM359324 | 71 | 72 | *Gossypium arboreum* |
| Medtr_SYT1 | CA858507.1 | 73 | 74 | *Medicago truncatula* |
| Orysa_SYT1 | AK058575 | 75 | 76 | *Oryza sativa* |
| Orysa_SYT2 | AK105366 | 77 | 78 | *Oryza sativa* |
| Orysa_SYT3 | BP185008 | 79 | 80 | *Oryza sativa* |
| Soltu_SYT2 | BG590990 | 81 | 82 | *Solanum tuberosum* |
| Zeama_SYT1 | BG874129.1 CA409022.1* | 83 | 84 | *Zea mays* |
| Zeama_SYT2 | AY106697 | 85 | 86 | *Zea mays* |
| Homsa_SYT | CR542103 | 87 | 88 | *Homo sapiens* |
| Allce_SYT2 | CF437485 | 89 | 90 | *Allium cepa* |
| Aqufo_SYT1 | DT758802.1 | 91 | 92 | *Aquilegia formosa x Aquilegia pubescens* |

(continued)

| Name | NCBI nucleotide accession number | Nucleic acid sequence SEQ ID NO | Translated polypeptide SEQ ID NO | Source |
|---|---|---|---|---|
| Bradi_SYT3 | DV480064.1 | 93 | 94 | *Brachypodium distachyon* |
| Brana_SYT2 | CN732814 | 95 | 96 | *Brassica napa* |
| Citsi_SYT2 | CV717501 | 97 | 98 | *Citrus sinensis* |
| Eupes_SYT2 | DV144834 | 99 | 100 | *Euphorbia esula* |
| Glyma_SYT2 | BQ612648 | 101 | 102 | *Glycine max* |
| Glyso_SYT2 | CA799921 | 103 | 104 | *Glycine soya* |
| Goshi_SYT1 | DT558852 | 105 | 106 | *Gossypium hirsutum* |
| Goshi_SYT2 | DT563805 | 107 | 108 | *Gossypium hirsutum* |
| Horvu_SYT2 | CA032350 | 109 | 110 | *Hordeum vulgare* |
| Lacse_SYT2 | DW110765 | 111 | 112 | *Lactuca serriola* |
| Lyces_SYT1 | AW934450.1<br><br>BP893155.1* | 113 | 114 | *Lycopersicon esculentum* |
| Maldo_SYT2 | CV084230<br>DR997566* | 115 | 116 | *Malus domestica* |
| Medtr_SYT2 | CA858743<br>B1310799.1<br>AL382135.1* | 117 | 118 | *Medicago truncula ta* |
| Panvi_SYT3 | DN 152517 | 119 | 120 | *Panicum virgatum* |
| Picsi_SYTI | DR484100<br>DR478464.1 | 121 | 122 | *Picea sitchensis* |
| Pinta_SYT1 | DT625916 | 123 | 124 | *Pinus taeda* |
| Poptr_SYT1 | DT476906 | 125 | 126 | *Populus tremula* |
| Sacof_SYT1 | CA078249.1<br>CA078630<br>CA082679<br>CA234526<br>CA239244<br>CA083312* | 127 | 128 | *Saccharum officinarum* |
| Sacof_SYT2 | CA110367 | 129 | 130 | *Saccharum officinarum* |
| Sacof_SYT3 | CA161933.1<br>CA265085* | 131 | 132 | *Saccharum officinarum* |
| Soltu_SYT1 | CK265597 | 133 | 134 | *Solanum tuberosum* |
| Sorbi_SYT3 | CX611128 | 135 | 136 | *Sorghum bicolor* |
| Triae_SYT2 | CD901951 | 137 | 138 | *Triticum aestivum* |
| Triae_SYT3 | BJ246754<br>BJ252709* | 139 | 140 | *Triticum aestivum* |

(continued)

| Name | NCBI nucleotide accession number | Nucleic acid sequence SEQ ID NO | Translated polypeptide SEQ ID NO | Source |
|---|---|---|---|---|
| Vitvi_SYT1 | DV219834 | 141 | 142 | *Vitis vinifera* |
| Zeama_SYT3 | C0468901 | 143 | 144 | *Zea mays* |
| Brana_SYT | NA | 150 | 151 | *Brassica napus* |
| Glyma_SYT | NA | 152 | 153 | *Glycine max* |
| *Compiled from cited accessions<br>NA: not available (proprietary) | | | | |

Examples of nucleic acids encoding SYT polypeptides are given in Table 6 above. Such nucleic acids are useful in performing the methods of the invention. The amino acid sequences given in Table 6 above are example sequences of orthologues and paralogues of the SYT polypeptide represented by SEQ ID NO: 58, the terms "orthologues" and "paralogues" being as defined herein. Further orthologues and paralogues may readily be identified by performing a so-called reciprocal blast search. Typically, this involves a first BLAST involving BLASTing a query sequence (for example using any of the sequences listed in Table 6 above) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 59 or SEQ ID NO: 60, the second BLAST would therefore be against *Arabidopsis* sequences). The results of the first and second BLASTs are then compared. A paralogue is identified if a high-ranking hit from the first blast is from the same species as from which the query sequence is derived, a BLAST back then ideally results in the query sequence amongst the highest hits; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, Clustalw may be used, followed by a neighbour joining tree, to help visualize clustering of related genes and to identify orthologues and paralogues.

It is to be understood that sequences falling under the definition of a "SYT polypeptide" are not to be limited to the polypeptides given in Table 6 (and mentioned in the sequence protocol), but that any polypeptide comprising from N-terminal to C-terminal: (i) an SNH domain having at least 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% sequence identity to the SNH domain of SEQ ID NO: 58; and (ii) a Met-rich domain; and (iii) a QG-rich domain may be suitable in performing the methods of the invention. Preferably, the SNH domain comprises the residues shown in black in Figure 6. Additionally, the SYT polypeptide may comprise one or more of the following: (a) SEQ ID NO: 146; (b) SEQ ID NO: 147; and (c) a Met-rich domain at the N-terminal preceding the SNH domain. Most preferably, the SYT polypeptide is as represented by SEQ ID NO: 60, SEQ ID NO: 151 or SEQ ID NO: 153.

A SYT polypeptide typically interacts with GRF (growth-regulating factor) polypeptides in yeast two-hybrid systems. Yeast two-hybrid interaction assays are well known in the art (see Field et al. (1989) Nature 340(6230): 245-246). For example, the SYT polypeptide as represented by SEQ ID NO: 4 is capable of interacting with AtGRF5 and with AtGRF9.

in a further embodiment the invention provides an isolated nucleic acid sequence comprising a nucleic acid sequence selected from the group consisting of:

(a) an isolated nucleic acid sequence as depicted in SEQ ID NO: 150 and SEQ ID NO: 152;
(b) an isolated nucleic acid sequence encoding the polypeptide as depicted in SEQ ID NO: 151 and SEQ ID NO: 153;
(c) an isolated nucleic acid sequence whose sequence can be deduced from a polypeptide as depicted in SEQ ID NO: 151 and SEQ ID NO: 153 as a result of the degeneracy of the genetic code;
(d) an isolated nucleic acid sequence which encodes a polypeptide which has at least 70% identity with the polypeptide encoded by the nucleic acid sequence of (a) to (c);

(e) an isolated nucleic acid sequence encoding a homologue, derivative or active fragment of the polypeptide as depicted in SEQ ID NO: 151 and SEQ ID NO: 153, which homologue, derivative or fragment is of plant origin and comprises advantageously from N-terminal to C-terminal:

(i) an SNH domain having in increasing order of preference at least 20%, 25%, 30%, 35%, 40%, 45%, 50%, 55%, 60%, 65%, 70%, 75%, 80%, 85%, 90%, 91 %, 92%, 93%, 94%, 95%, 96%, 97%, 98%, 99% sequence identity to the SNH domain of SEQ ID NO: 58;
(ii) a Met-rich domain;
(iii) a QG-rich domain;

(f) an isolated nucleic acid sequence capable of hybridising with a nucleic acid of (a) to (c) above, or its complement, wherein the hybridising sequence or the complement thereof encodes the plant protein of (a) to (e);

whereby modified expression of the nucleic acid sequence increases yield and/or early vigour in plants under abiotic stress compared to control plants.

The nucleic acid sequences encoding SYT polypeptides as given in Table 6, or orthologues or paralogues of any of the aforementioned SEQ ID NOs need not be full-length nucleic acid sequences, since performance of the methods of the invention does not rely on the use of full-length nucleic acid sequences.

SYT nucleic acid variants may also be suitable in practising the methods of the invention. Variant SYT nucleic acid sequences typically are those having the same function as a naturally occurring SYT nucleic acid sequence, which can be the same biological function or the function of increasing yield and/or early vigour when expression of the nucleic acid sequence is modulated in a plant under abiotic stress relative to control plants. Such variants include portions of a SYT nucleic acid sequence, splice variants of a SYT nucleic acid sequence, allelic variants of a SYT nucleic acid sequence, variants of a SYT nucleic acid sequence obtained by gene shuffling and/or nucleic acid sequences capable of hybridising with a SYT nucleic acid sequence as defined below. Preferably, the nucleic acid variant is a variant of a nucleic acid sequence is as represented by SEQ ID NO: 59, SEQ ID NO: 150 or SEQ ID NO: 152. The terms hybridising sequence, splice variant, allelic variant and gene shuffling are as described herein.

Nucleic acids encoding SYT polypeptides need not be full-length nucleic acids, since performance of the methods of the invention does not rely on the use of full-length nucleic acid sequences. According to the present invention, there is provided a method for enhancing yield and/or early vigour in plants under abiotic stress, comprising introducing and expressing in a plant a portion of any one of the nucleic acid sequences given in Table 6, or a portion of a nucleic acid encoding an orthologue, paralogue or homologue of any of the amino acid sequences given in Table 6.

The term portion as used herein refers to a piece of DNA encoding a polypeptide comprising from N-terminal to C-terminal: (i) an SNH domain having in increasing order of preference at least 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% sequence identity to the SNH domain of SEQ ID NO: 58 and (ii) a Met-rich domain; and (iii) a QG-rich domain. A portion may be prepared, for example, by making one or more deletions to a SYT nucleic acid sequence. The portions may be used in isolated form or they may be fused to other coding (or non coding) sequences in order to, for example, produce a polypeptide that combines several activities. When fused to other coding sequences, the resulting polypeptide produced upon translation may be bigger than that predicted for the SYT fragment. Preferably, the portion is a portion of a nucleic acid sequence as represented by any one given in Table 6, or orthologues or paralogues of any of the aforementioned SEQ ID NOs. Most preferably the portion is a portion of a nucleic acid sequence is as represented by SEQ ID NO: 59, SEQ ID NO: 150 or SEQ ID NO: 152.

Another variant of a SYT nucleic acid sequence is a nucleic acid sequence capable of hybridising under reduced stringency conditions, preferably under stringent conditions, with a SYT nucleic acid sequence as hereinbefore defined, which hybridising sequence encodes a SYT polypeptide or a portion as defined hereinabove.

According to the present invention, there is provided a method for enhancing yield and/or early vigour in plants under abiotic stress, comprising introducing and expressing in a plant a nucleic acid capable of hybridizing to any one of the nucleic acids given in Table 6, or comprising introducing and expressing in a plant a nucleic acid capable of hybridising to a nucleic acid encoding an orthologue, paralogue or homologue of any of the nucleic acid sequences given in Table 6.

Hybridising sequences useful in the methods of the invention encode a SYT polypeptide comprising from N-terminal to C-terminal: (i) an SNH domain having in increasing order of preference at least 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% sequence identity to the SNH domain of SEQ ID NO: 58 and (ii) a Met-rich domain; and (iii) a QG-rich domain. Preferably, the hybridising sequence is one that is capable of hybridising to a nucleic acid sequence as given in Table 6, or orthologues or paralogues of any of the aforementioned SEQ ID NOs, or to a portion of any of the aforementioned sequences as defined hereinabove. Most preferably the hybridizing sequence is one that is capable of hybridising to a nucleic acid sequence is as represented by SEQ ID NO: 59, SEQ ID NO: 150, SEQ ID NO: 152, or to portions (or probes) thereof. Methods for designing probes are well known in the art. Probes are generally less than 1000 bp in length, preferably less than 500 bp in length. Commonly, probe lengths for DNA-DNA hybridisations such as Southern blotting, vary between 100 and 500 bp, whereas the hybridising region in probes for DNA-DNA hybridisations such as in PCR amplification generally are shorter than 50 but longer than 10 nucleotides. The hybridising sequence is typically at least 100, 125, 150, 175, 200 or 225 nucleotides in length, preferably at least 250, 275, 300, 325, 350, 375, 400, 425, 450 or 475 nucleotides in length, further preferably least 500, 525, 550, 575, 600, 625, 650, 675, 700 or 725 nucleotides in length, or as long as a full length SYT cDNA.

Another nucleic acid variant useful in the methods of the invention is a splice variant encoding a SYT polypeptide as defined hereinabove. Preferred splice variants are splice variants of the SYT nucleic acid sequences as given in Table 6, or orthologues or paralogues of any of the aforementioned SEQ ID NOs. Most preferred is a splice variant of a SYT nucleic acid sequence as represented by SEQ ID NO: 59, SEQ ID NO: 150 or SEQ ID NO: 152.

According to the present invention, there is provided a method for enhancing yield and/or early vigour in plants under abiotic stress, comprising introducing and expressing in a plant a splice variant of any one of the nucleic acid sequences given in Table 6, or a splice variant of a nucleic acid encoding an orthologue, paralogue or homologue of any of the amino acid sequences given in Table 6.

Another nucleic acid variant useful in performing the methods of the invention is an allelic variant of a nucleic acid encoding a SYT polypeptide as defined hereinabove. Allelic variants exist in nature, and encompassed within the methods of the present invention is the use of these natural alleles. Preferred allelic variants are allelic variants of the SYT nucleic acid sequences as given in Table 6, or orthologues or paralogues of any of the aforementioned SEQ ID NOs. Most preferred is a splice variant of a SYT nucleic acid sequence as represented by SEQ ID NO: 59.

According to the present invention, there is provided a method for enhancing yield and/or early vigour in plants under abiotic stress, comprising introducing and expressing in a plant an allelic variant of any one of the nucleic acids given in Table 6, or comprising introducing and expressing in a plant an allelic variant of a nucleic acid encoding an orthologue, paralogue or homologue of any of the amino acid sequences given in Table 6.

A further nucleic acid variant useful in the methods of the invention is a nucleic acid variant encoding a SYT polypeptide obtained by gene shuffling (or directed evolution).

Another nucleic acid variant useful in the methods of the invention is a nucleic acid variant encoding a SYT polypeptide obtained by site-directed mutagenesis. Site-directed mutagenesis may be used to generate variants of SYT nucleic acid sequences. Several methods are available to achieve site-directed mutagenesis, the most common being PCR based methods (Current Protocols in Molecular Biology. Wiley Eds).

According to the present invention, there is provided a method for enhancing yield and/or early vigour in plants under abiotic stress, comprising introducing and expressing in a plant a variant of any one of the nucleic acid sequences given in Table 6, or comprising introducing and expressing in a plant a variant of a nucleic acid encoding an orthologue, paralogue or homologue of any of the amino acid sequences given in Table 6, which variant nucleic acid is obtained by gene shuffling or site-directed mutagenesis.

The following SYT nucleic acid variants are examples of variants suitable in practising the methods of the invention:

(i) a portion of a SYT nucleic acid sequence;
(ii) a nucleic acid sequence capable of hybridising with a SYT nucleic acid sequence;
(iii) a splice variant of a SYT nucleic acid sequence;
(iv) an allelic variant of a SYT nucleic acid sequence;
(v) a SYT nucleic acid sequence obtained by gene shuffling;
(vi) a SYT nucleic acid sequence obtained by site-directed mutagenesis.

Also useful in the methods of the invention are nucleic acids encoding homologues of SYT polypeptides as given in Table 6, or orthologues or paralogues of any of the aforementioned SEQ ID NOs.

**[0271]** Also useful in the methods of the invention are nucleic acids encoding derivatives of any one of the SYT nucleic acid sequences as given in Table 6, or orthologues or paralogues of any of the aforementioned SEQ ID NOs. Derivatives of orthologues or paralogues of any of the aforementioned SEQ ID NOs are further examples that may be suitable for use in the methods of the invention.

SYT nucleic acid sequences may be derived from any artificial source or natural source, such as plants, algae, fungi or animals. The nucleic acid sequence may be modified from its native form in composition and/or genomic environment through deliberate human manipulation. Preferably the nucleic acid sequence encoding a SYT polypeptide is of plant origin. The nucleic acid sequence may be isolated from a dicotyledonous species, preferably from the family Brassicaceae, further preferably from *Arabidopsis thaliana* or *Brassica napus.* Alternatively, nucleic acid sequence may be isolated from the family Fabaceae, preferably from *Glycine max.* More preferably, the SYT nucleic acid sequence isolated from:

(a) *Arabidopsis thaliana* is as represented by SEQ ID NO: 59 and the SYT polypeptide as represented by SEQ ID NO: 60;
(b) *Brassica napus* is as represented by SEQ ID NO: 150 and the SYT polypeptide as represented by SEQ ID NO: 151;
(c) *Glycine max* is as represented by SEQ ID NO: 152 and the SYT polypeptide as represented by SEQ ID NO: 153.

The terms "yield", "seed yield" and "early vigour" are defined above. The terms "increased", "improved", "enhanced", "amplified", "extended", or "rised" are interchangeable and are defined hereinabove. Taking corn as an example, a yield increase may be manifested as one or more of the following: increase in the number of plants per square meter, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may be manifested by an increase in one or more of the following: number of plants per square meter, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.
An increase in yield may also result in modified architecture, or may occur as a result of modified architecture.
According to a preferred feature, performance of the methods of the invention result in plants having increased biomass, increased seed yield and/or early vigour under abiotic stress relative to control plants. Therefore, according to the present invention, there is provided a method for increasing biomass, seed yield and/or early vigour in a plant under abiotic stress relative to control plants, which method comprises modulating expression in a plant of a nucleic acid sequence encoding a SYT polypeptide. Preferably, by increased biomass is herein taken to mean the aboveground part (or leafy biomass) during plant development and at maturity. Preferably, by increased seed yield is herein taken to mean any one of the following: total seed yield, number of filled seeds, seed fill rate, TKW and harvest index.
Since the transgenic plants according to the present invention have increased yield under abiotic stress, it is likely that these plants exhibit an increased growth rate under abiotic stress (during at least part of their life cycle, for example at seedling stage for early vigour), relative to the growth rate of control plants at a corresponding stage in their life cycle. The increased growth rate may be specific to one or more parts of a plant (including seeds), or may be throughout substantially the whole plant. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible. If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the planting and harvesting of corn plants followed by, for example, the planting and optional harvesting of soybean, potato or any other suitable plant). Harvesting additional times from the same rootstock in the case of some crop plants may also be possible. Altering the harvest cycle of a plant may lead to an increase in annual biomass production per square meter (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others. The growth rate of plants is measured under abiotic stress such as salt stress; water stress (drought or excess water); reduced nutrient availability stress; temperature stresses caused by atypical hot or cold/freezing temperatures; oxidative stress; metal stress; chemical toxicity stress; or combinations thereof.
Performance of the methods of the invention gives plants having an increased growth rate under abiotic stress relative to control plants. Therefore, according to the present invention, there is provided a method for increasing growth rate in plants under abiotic stress relative to control plants, which method comprises modulating expression in a plant of a nucleic acid sequence encoding a SYT polypeptide.
Plants typically respond to exposure to stress by growing more slowly. In conditions of severe stress, the plant may even stop growing altogether. Mild stress in the sense of the invention leads to a reduction in the growth of the stressed plants of less than 40%, 35% or 30%, preferably less than 25%, 20% or 15%, more preferably less than 14%, 13%, 12%, 11% or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the typical stresses to which a plant may be exposed. These stresses may be the everyday biotic and/or abiotic (environmental) stresses to which a plant is exposed. Biotic stresses are typically those stresses caused by pathogens, such as bacteria, viruses, fungi, nematodes and insects. Typical abiotic or environmental stresses comprise any one or more of: salt stress, water stress (drought or excess water), reduced nutrient availability stress, temperature stresses caused by atypical hot or cold/freezing temperatures, oxidative stress, metal stress or chemical toxicity stress.

Performance of the methods according to the present invention results in plants having increased yield and/or early vigour under abiotic stress relative to control plants. As reported in Wang et al., (Planta (2003) 218: 1-14), abiotic stress leads to a series of morphological, physiological, biochemical and molecular changes that adversely affect plant growth and productivity. Drought, salinity, extreme temperatures and oxidative stress are often interconnected and may induce growth and cellular damage through similar mechanisms. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturation of functional and structural proteins. Reduced nutrient availability, in particular reduced nitrogen availability, is a major limiting factor for plant growth, for example through the reduced availability of amino acids for protein synthesis. As a consequence, these diverse environmental stresses often activate similar cell signaling pathways and cellular responses, such as the production of stress proteins, up-regulation of anti-oxidants, accumulation of compatible solutes and growth arrest.

Since diverse environmental stresses activate similar pathways, the exemplification of the present invention with salt stress should not be seen as a limitation to salt stress, but more as a screen to indicate the involvement of SYT polypeptides in abiotic stresses in general. A review in TRENDS in Plant Science (Jian-Kang Zhu, Vol. 6, No. 2, Feb. 2001) confirms that transgenic plants performing better under salt stress often also perform better under other stresses including chilling, freezing, heat and drought. A particularly high degree of "cross talk" is reported between drought stress and high-salinity stress (Rabbani et al., Plant Physiology, December 2003, Vol. 133, pp. 1755-1767). Therefore, it would be apparent that a SYT polypeptide (as defined herein) would, along with its usefulness in increasing yield and/or early vigour in plants under salt stress, also find use in increasing yield and/or early vigour of the plant under various other abiotic stresses.

The term "abiotic stress" as defined herein is taken to mean any one or more of: salt stress, water stress (drought or excess water), reduced nutrient availability stress, temperature stresses caused by atypical hot or cold/ freezing temperatures, oxidative stress, metal stress or chemical toxicity stress. The term salt stress is not restricted to common salt (NaCl), but may be any one or more of: NaCl, KCl, LiCl, $MgCl_2$, $CaCl_2$, amongst others.

Performance of the methods of the invention gives plants having increased yield and/or early vigour under abiotic stress relative to control plants. Therefore, according to the present invention, there is provided a method for increasing plant yield and/or early vigour under abiotic stress relative to control plants, which method comprises modulating expression in a plant of a nucleic acid sequence encoding a SYT polypeptide. By abiotic stress is taken to mean any one or more of: salt stress, water stress (drought or excess water), reduced nutrient availability stress, temperature stresses caused by atypical hot or cold/freezing temperatures, oxidative stress, metal stress or chemical toxicity stress.

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum and oats.

A preferred method for introducing a genetic modification is to introduce and express in a plant a nucleic acid sequence encoding a SYT polypeptide. A SYT polypeptide is defined as a polypeptide comprising from N-terminal to C-terminal: (i) an SNH domain having in increasing order of preference at least 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% sequence identity to the SNH domain of SEQ ID NO: 58; and (ii) a Met-rich domain; and (iii) a QG-rich domain. Preferably, the SNH domain comprises the residues shown in black in Figure 6. Further preferably, the SNH domain is represented by SEQ ID NO: 57.

According to a preferred aspect of the present invention, the modulated expression of a SYT nucleic acid sequence is increased expression. The increase in expression may lead to raised SYT mRNA or polypeptide levels, which could equate to raised activity of the SYT polypeptide; or the activity may also be raised when there is no change in polypeptide levels, or even when there is a reduction in polypeptide levels. This may occur when the intrinsic properties of the SYT polypeptide are altered, for example, by making mutant versions that are more active that the wild type polypeptide. Methods for increasing or reducing expression of genes or gene products are known in the art.

The invention also provides genetic constructs and vectors to facilitate introduction and/or expression of the nucleotide sequences useful in the methods according to the invention.

Therefore, there is provided a genetic construct comprising:

(i) A nucleic acid sequence encoding a SYT polypeptide, as defined hereinabove, or a nucleic acid sequence as represented by SEQ ID NO: 150 or SEQ ID NO: 152;
(ii) One or more control sequences capable of driving expression of the nucleic acid sequence of (i); and optionally
(iii) A transcription termination sequence.

A preferred construct is one where the control sequence is a promoter derived from a plant, preferably from a monocotyledonous plant if a monocotyledonous is to be transformed.

Constructs useful in the methods according to the present invention may be constructed using recombinant DNA technology well known to persons skilled in the art. The genetic constructs may be inserted into vectors, which may be commercially available, suitable for transforming into plants and suitable for expression of the gene of interest in the transformed cells. The invention therefore provides use of a genetic construct as defined hereinabove in the methods of the invention.

Plants are transformed with a vector comprising the sequence of interest (i.e., a nucleic acid sequence encoding a SYT polypeptide). The sequence of interest is operably linked to one or more control sequences (at least to a promoter).

Advantageously, any type of promoter may be used to drive expression of the nucleic acid sequence.

Useful promoters are constitutive promoters (Benfey et al., EMBO J. 8 (1989) 2195-2202), such as those which originate from plant viruses, such as 35S CAMV (Franck et al., Cell 21 (1980) 285-294), 19S CaMV (see also US 5352605 and WO 84/02913), 34S FMV (Sanger et al., Plant. Mol. Biol., 14, 1990: 433-443), the parsley ubiquitin promoter, or plant promoters such as the Rubisco small subunit promoter described in US 4,962,028 or the plant promoters PRP1 [Ward et al., Plant. Mol. Biol. 22 (1993)], SSU, PGEL1, OCS [Leisner (1988) Proc Natl Acad Sci USA 85(5): 2553-2557], lib4, usp, mas [Comai (1990) Plant Mol Biol 15 (3):373-381], STLS1, ScBV (Schenk (1999) Plant Mol Biol 39(6): 1221-1230), B33, SAD1 or SAD2 (flax promoters, Jain et al., Crop Science, 39 (6), 1999: 1696-1701) or nos [Shaw et al. (1984) Nucleic Acids Res. 12(20):7831-7846]. Further examples of constitutive plant promoters are the sugarbeet V-ATPase promoters (WO 01/14572). Examples of synthetic constitutive promoters are the Super promoter (WO 95/14098) and promoters derived from G-boxes (WO 94/12015). If appropriate, chemical inducible promoters may furthermore also be used, compare EP-A 388186, EP-A 335528, WO 97/06268. Stable, constitutive expression of the polypeptides according to the invention a plant can be advantageous. However, inducible expression of the polypeptide of the invention is advantageous, if a late expression before the harvest is of advantage, as metabolic manipulation may lead to plant growth retardation.

The expression of plant genes can also be facilitated via a chemical inducible promoter (for a review, see Gatz 1997, Annu. Rev. Plant Physiol. Plant Mol. Biol., 48:89-108). Chemically inducible promoters are particularly suitable when it is desired to express the gene in a time-specific manner. Examples of such promoters are a salicylic acid inducible promoter (WO 95/19443), and abscisic acid-inducible promoter (EP 335 528), a tetracyclin-inducible promoter (Gatz et al. (1992) Plant J. 2, 397-404), a cyclohexanol- or ethanol-inducible promoter (WO 93/21334) or others as described herein.

Other suitable promoters are those that react to biotic or abiotic stress, for example the pathogen-induced PRP1 gene promoter (Ward et al., Plant. Mol. Biol. 22 (1993) 361-366), the tomato heat-inducible hsp80 promoter (US 5,187,267), the potato chill-inducible alpha-amylase promoter (WO 96/12814) or the wound-inducible pinII promoter (EP-A-0 375 091) or others as described herein.

Preferred promoters are in particular those which bring gene expression in tissues and organs, in seed cells, such as endosperm cells and cells of the developing embryo. Suitable promoters are the oilseed rape napin gene promoter (US 5,608,152), the Vicia faba USP promoter (Baeumlein et al., Mol Gen Genet, 1991, 225 (3): 459-67), the Arabidopsis oleosin promoter (WO 98/45461), the Phaseolus vulgaris phaseolin promoter (US 5,504,200), the Brassica Bce4 promoter (WO 91/13980), the bean arc5 promoter, the carrot DcG3 promoter, or the Legumin B4 promoter (LeB4; Baeumlein et al., 1992, Plant Journal, 2 (2): 233-9), and promoters which bring about the seed-specific expression in monocotyledonous plants such as maize, barley, wheat, rye, rice and the like. Advantageous seed-specific promoters are the sucrose binding protein promoter (WO 00/26388), the phaseolin promoter and the napin promoter. Suitable promoters which must be considered are the barley Ipt2 or Ipt1 gene promoter (WO 95/15389 and WO 95/23230), and the promoters described in WO 99/16890 (promoters from the barley hordein gene, the rice glutelin gene, the rice oryzin gene, the rice prolamin gene, the wheat gliadin gene, the wheat glutelin gene, the maize zein gene, the oat glutelin gene, the sorghum kasirin gene and the rye secalin gene). Further suitable promoters are Amy32b, Amy 6-6 and Aleurain [US 5,677,474], Bce4 (oilseed rape) [US 5,530,149], glycinin (soya) [EP 571 741], phosphoenolpyruvate carboxylase (soya) [JP 06/62870], ADR12-2 (soya) [WO 98/08962], isocitrate lyase (oilseed rape) [US 5,689,040] or $\alpha$-amylase (barley) [EP 781 849]. Other promoters which are available for the expression of genes in plants are leaf-specific promoters such as those described in DE-A 19644478 or light-regulated promoters such as, for example, the pea petE promoter.

Further suitable plant promoters are the cytosolic FBPase promoter or the potato ST-LSI promoter (Stockhaus et al., EMBO J. 8, 1989, 2445), the Glycine max phosphoribosylpyrophosphate amidotransferase promoter (GenBank Accession No. U87999) or the node-specific promoter described in EP-A-O 249 676.

In one embodiment, the SYT nucleic acid sequence is operably linked to a constitutive promoter. A constitutive promoter is transcriptionally active during most, but not necessarily all, phases of its growth and development and is substantially ubiquitously expressed. Preferably the promoter is derived from a plant, more preferably the promoter is from a monocotyledonous plant if a monocotyledonous plant is to be transformed. Further preferably, the constitutive promoter is a GOS2 promoter that is represented by a nucleic acid sequence substantially similar to SEQ ID NO: 145 or SEQ ID NO: 56. Most preferably the GOS2 promoter is as represented by SEQ ID NO: 56 or SEQ ID NO: 145 . It should be clear that the applicability of the present invention is not restricted to the SYT nucleic acid sequence represented by SEQ ID NO: 59, nor is the applicability of the invention restricted to expression of a SYT nucleic acid sequence when driven by a GOS2 promoter. Examples of other constitutive promoters that may also be used to drive expression of a SYT nucleic acid sequence are shown in the definitions section.

Optionally, one or more terminator sequences may be used in the construct introduced into a plant. Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

The present invention also encompasses plants obtainable by the methods according to the present invention. The present invention therefore provides plants, plant parts and plant cells obtainable by the methods according to the present invention, which plants have introduced therein a SYT nucleic acid sequence and which plants, plant parts and plant cells are preferably from a crop plant, further preferably from a monocotyledonous plant.

The invention also provides a method for the production of transgenic plants having increased yield and/or early vigour under abiotic stress, comprising introduction and expression in a plant of a SYT nucleic acid sequence.

More specifically, the present invention provides a method for the production of transgenic plants, preferably monocotyledonous plants, having increased yield and/or early vigour under abiotic stress, which method comprises:

(i) introducing and expressing in a plant or plant cell a nucleic acid sequence encoding a SYT polypeptide; and
(ii) cultivating the plant cell under conditions promoting plant growth and development.

The nucleic acid of (i) may be any of the nucleic acids capable of encoding a SYT polypeptide, as defined hereinabove, or a nucleic acid sequence as represented by SEQ ID NO: 150 or SEQ ID NO: 152.

Subsequent generations of the plants obtained from cultivating step (ii) may be propagated by a variety of means, such as by clonal propagation or classical breeding techniques. For example, a first generation (or T1) transformed plant may be selfed to give homozygous second generation (or T2) transformants, and the T2 plants further propagated through classical breeding techniques.

The nucleic acid sequence may be introduced directly into a plant cell or into the plant itself (including introduction into a tissue, organ or any other part of a plant). According to a preferred feature of the present invention, the nucleic acid sequence is introduced into a plant by transformation.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants.

Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed to give homozygous second generation (or T2) transformants, and the T2 plants further propagated through classical breeding techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention. The invention also includes host cells containing an isolated SYT nucleic acid sequence. Preferred host cells according to the invention are plant cells. The invention also extends to harvestable parts of a plant such as, but not limited to seeds, leaves, fruits, flowers, stem cultures, roots, rhizomes, tubers and bulbs. The invention furthermore relates to products derived, preferably directly derived, from a harvestable part of such a plant, such as dry pellets or powders, meal, oil, fat and fatty acids, starch or proteins.

According to a preferred feature of the invention, the modulated expression is increased expression. Methods for increasing expression of nucleic acids or genes, or gene products, are well documented in the art and examples are provided in the definitions section.

Alternatively, the expression of a nucleic acid sequence encoding a SYT polypeptide may be modulated by introducing a genetic modification, for example, by any one (or more) of the following techniques: T-DNA activation, TILLING, homologous recombination, or by introducing and expressing in a plant a nucleic acid sequence encoding a SYT polypeptide. Following introduction of the genetic modification, there follows a step of selecting for modulated expression of a nucleic acid sequence encoding a SYT polypeptide, which modulated expression gives plants having increased yield and/or early vigour under abiotic stress.

One such technique is T-DNA activation tagging. The promoter to be introduced may be any promoter capable of driving expression of a gene in the desired organism, in this case a plant. For example, constitutive, tissue-preferred, cell type-preferred and inducible promoters are all suitable for use in T-DNA activation. The effects of the invention may also be reproduced using the technique of TILLING (Targeted Induced Local Lesions In Genomes). The effects of the invention may also be reproduced using homologous recombination.

The present invention also encompasses use of SYT nucleic acid sequences and use of SYT polypeptides, and use of a construct as defined hereinabove in increasing plant yield and/or early vigour under abiotic stress.

SYT nucleic acid sequences or SYT polypeptides may find use in breeding programmes in which a DNA marker is identified that may be genetically linked to a SYT. The SYT nucleic acid sequences or SYT polypeptides may be used to define a molecular marker. This DNA or polypeptide marker may then be used in breeding programmes to select plants having increased yield. The SYT gene may, for example, be a nucleic acid sequence as represented by any one of the SYT nucleic acid sequences as given in Table 6, or orthologues or paralogues of any of the aforementioned SEQ ID NOs.

**[0317]** Allelic variants of a SYT nucleic acid sequence may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question, for example, different allelic variants of any one of the SYT nucleic acid sequences as given in Table 6, or orthologues or paralogues of any of the aforementioned SEQ ID NOs. Growth performance may be monitored in a greenhouse or in the field. Further optional steps include crossing plants, in which the superior allelic variant was identified, with another plant. This could be used, for example, to make a combination of interesting phenotypic features.

SYT nucleic acid sequences may also be used as probes for genetically and physically mapping the genes that they are a part of, and as markers for traits linked to those genes. Such information may be useful in plant breeding in order to develop lines with desired phenotypes. Such use of SYT nucleic acid sequences requires only a nucleic acid sequence of at least 15 nucleotides in length. The SYT nucleic acid sequences may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the SYT nucleic acid sequences. The resulting banding patterns may then be subjected to genetic analyses using computer programs such as MapMaker (Lander et al. (1987) Genomics 1: 174-181) in order to construct a genetic map. In addition, the nucleic acid sequences may be used to probe Southern blots containing restriction endonuclease-treated genomic DNAs of a set of individuals representing parent and progeny of a defined genetic cross. Segregation of the DNA polymorphisms is noted and used to calculate the position of the SYT nucleic acid sequence in the genetic map previously obtained using this population (Botstein et al. (1980) Am. J. Hum. Genet. 32:314-331).

The production and use of plant gene-derived probes for use in genetic mapping is described in Bematzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence *in situ* hybridization (FISH) mapping (Trask (1991) Trends Genet. 7:149-154). Although current methods of FISH mapping favor use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acid sequences. Examples include allele-specific amplification (Kazazian (1989) J. Lab. Clin. Med 11: 95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the nucleic acid sequence is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having increased yield under abiotic stress, as described hereinbefore. These yield-enhancing traits may also be combined with other economically advantageous traits, such as further yield-enhancing traits, tolerance to various stresses, traits modifying various architectural features and/or biochemical and/or physiological features.

**Detailed description for the cpFBPase polypeptide**

Surprisingly, it has now been found that increasing expression in aboveground parts of a plant of a nucleic acid sequence encoding a chloroplastic fructose-1,6-bisphosphatase (cpFBPase) polypeptide increases plant yield relative to control plants. Therefore, according to the present invention, there is provided a method for increasing plant yield relative to control plants, comprising increasing expression in aboveground parts of a plant of a nucleic acid sequence encoding a cpFBPase polypeptide.

Reference herein to "control plants" is taken to mean any suitable control plant or plants. The "reference", "control", or "wild type" are used herein interchangeably and is preferably a subject, e.g. an organelle, a cell, a tissue, a plant, which is as similar to the subject matter of the invention as possible. The reference, control or wild type is in its genome, transcriptome, proteome or metabolome as similar as possible to the subject of the present invention. Preferably, the term "reference-" "control-" or "wild type-"-organelle, -cell, -tissue or plant, relates to an organelle, cell, tissue or plant, which is nearly genetically identical to the organelle, cell, tissue or plant, of the present invention or a part thereof, preferably 95%, 98%, 99,00%, 99,10%, 99,30%, 99,50%, 99,70%, 99,90%, 99,99%, 99, 999% or more identical. Most preferably the "reference", "control", or "wild type" is preferably a subject, e.g. an organelle, a cell, a tissue, a plant, which is genetically identical to the plant, tissue, cell, organelle used according to the method of the invention except that the nucleic acid sequences or the gene product encoded by them are changed, modulated or modified according to the inventive method.

Unless otherwise specified, the terms "polynucleotides", "nucleic acid" and "nucleic acid molecule", are interchangeably in the present context. Unless otherwise specified, the terms "peptide", "polypeptide" and "protein" are interchangeably in the present context. The term "sequence" may relate to polynucleotides, nucleic acids, nucleic acid molecules, peptides, polypeptides and proteins, depending on the context in which the term "sequence" is used. The terms "gene(s)", "polynucleotide", "nucleic acid sequence", "nucleotide sequence", or "nucleic acid molecule(s)" as used herein refers to a polymeric form of nucleotides of any length, either ribonucleotides or deoxyribonucleotides. The terms refer only to the primary structure of the molecule.

Thus, the terms "nucleic acid sequence", "gene(s)", "polynucleotide", "nucleotide sequence", or "nucleic acid molecule(s)" as used herein include double- and single-stranded DNA and RNA. They also include known types of modifications, for example, methylation, "caps", substitutions of one or more of the naturally occurring nucleotides with an analog. Preferably, the DNA or RNA sequence of the invention comprises a coding sequence encoding the herein defined polypeptide.

A "coding sequence" is a nucleic acid sequence, which is transcribed into mRNA and/or translated into a polypeptide when placed under the control of appropriate regulatory sequences. The boundaries of the coding sequence are determined by a translation start codon at the 5'-terminus and a translation stop codon at the 3'-terminus. A coding sequence can include, but is not limited to mRNA, cDNA, recombinant nucleic acid sequences or genomic DNA, while introns may be present as well under certain circumstances.

The term "chloroplastic fructose-1, 6-bisphosphatase (cpFBPase) polypeptide" as defined herein refers to a polypeptide functioning in the chloroplast and comprising: (i) at least one FBPase domain; and (ii) at least one redox regulatory insertion.

An example of a cpFBPase polypeptide as defined hereinabove as functioning in the chloroplast and comprising: (i) at least one FBPase domain; and (ii) at least one redox regulatory insertion is as represented in SEQ ID NO: 155. Further such examples are represented by any one of SEQ ID NO: 157, SEQ ID NO: 159, SEQ ID NO: 161, SEQ ID NO: 163, SEQ ID NO: 165, SEQ ID NO: 167, SEQ ID NO: 169, SEQ ID NO: 171, SEQ ID NO: 173, SEQ ID NO: 175, SEQ ID NO: 177, SEQ ID NO: 179, SEQ ID NO: 181, SEQ ID NO: 183, SEQ ID NO: 185, SEQ ID NO: 187, SEQ ID NO: 189, SEQ ID NO: 191, SEQ ID NO: 193, SEQ ID NO: 195 and SEQ ID NO: 197, or orthologues or paralogues of any of the aforementioned SEQ ID NOs. The invention is illustrated by transforming plants with the *Chlamydomonas reinhardthii* sequence represented by SEQ ID NO: 154, encoding the polypeptide of SEQ ID NO: 155. SEQ ID NO: 157 (encoded by SEQ ID NO: 156, from *Bigelowiella natans*), SEQ ID NO: 159 (encoded by SEQ ID NO: 158, from *Aquilegia formosa x Aquilegia pubescens*), SEQ ID NO: 161 (encoded by SEQ ID NO: 160, from *Arabidopsis thaliana*), SEQ ID NO: 163 (encoded by SEQ ID NO: 162, from *Brassica napus*), SEQ ID NO: 165 (encoded by SEQ ID NO: 164, from *Cyanidioschyzon merolae*) SEQ ID NO: 167 (encoded by SEQ ID NO: 166, from *Glycine max*), SEQ ID NO: 169 (encoded by SEQ ID NO: 168, from *Lycopersicon esculentum*), SEQ ID NO: 171 (encoded by SEQ ID NO: 170, from *Medicago truncatula*), SEQ ID NO: 173 (encoded by SEQ ID NO: 172, from *Nicotiana tabacum*), SEQ ID NO: 175 (encoded by SEQ ID NO: 174, from *Oryza sativa*), SEQ ID NO: 177 (encoded by SEQ ID NO: 176, from *Ostreococcus lucimarinus*), SEQ ID NO: 179 (encoded by SEQ ID NO: 178, from *Ostreococcus tauri*), SEQ ID NO: 181 (encoded by SEQ ID NO: 180, from *Phaeodactylum tricornutum*), SEQ ID NO: 183 (encoded by SEQ ID NO: 182, from *Pisum sativa*), SEQ ID NO: 185 (encoded by SEQ ID NO: 184, from *Poncirus trifoliata*), SEQ ID NO: 187 (encoded by SEQ ID NO: 186, from *Populus tremuloides*), SEQ ID NO: 189 (encoded by SEQ ID NO: 188, from *Solanum tuberosum*), SEQ ID NO: 191 (encoded by SEQ ID NO: 190, from *Spinacia oleracea*), SEQ ID NO: 193 (encoded by SEQ ID NO: 192, from *Triticum aestivum*), SEQ ID NO: 195 (encoded by SEQ ID NO: 194, from *Zea mays*) and SEQ ID NO: 197 (encoded by SEQ ID NO: 196, from *Physicomitrella patens*), are orthologues of the polypeptide of SEQ ID NO: 155.

It is to be understood that sequences falling under the definition of a "cpFBPase polypeptide" are not to be limited to the polypeptides given in Table 7 (and mentioned herein above) but that any polypeptide functioning in the chloroplast and comprising: (i) at least one FBPase domain; and (ii) at least one redox regulatory insertion, may be suitable in performing the methods of the invention. Preferably, the cpFBPase polypeptide is as represented by SEQ ID NO: 155.

However, performance of the invention is not restricted to these sequences; the methods of the invention may advantageously be performed using any cpFBPase-encoding nucleic acid or cpFBPase polypeptide as defined herein.

Examples of nucleic acids encoding cpFBPase polypeptides are given in Table C of Example 12 herein. Such nucleic acids are useful in performing the methods of the invention. The amino acid sequences given in Table C of Example 12 are example sequences of orthologues and paralogues of the cpFBPase polypeptide represented by SEQ ID NO: 155, the terms "orthologues" and "paralogues" being as defined herein. Orthologues and paralogues may easily be found by performing a so-called reciprocal blast search. This may be done by a first BLAST involving BLASTing a query sequence (for example, SEQ ID NO: 154 or SEQ ID NO: 155) against any sequence database, such as the publicly available NCBI database. BLASTN or TBLASTX (using standard default values) may be used when starting from a nucleotide sequence and BLASTP or TBLASTN (using standard default values) may be used when starting from a polypeptide sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 154 or SEQ ID NO: 155, the second BLAST would therefore be against *Chlamydomonas* sequences). The results of the first and second BLASTs are then compared. A paralogue is identified if a high-ranking hit from the first BLAST is from the same species as from which the query sequence is derived, a BLAST back then ideally results in the query sequence as highest hit (besides itself); an orthologue is identified if a high-ranking hit in the first BLAST is not from the same species as from which the query sequence is derived and preferably results upon BLAST back in the query sequence amongst the highest hits. High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. An example detailing the identification of orthologues and paralogues is given in Example 12. In the case of large families, ClustalW may be used, followed by a neighbour joining tree, to help visualize clustering of related genes and to identify orthologues and paralogues. Preferably, cpFBPase polypeptides useful in the methods of the invention are functioning in the chloroplast and comprise: (i) at least one FBPase domain; and (ii) at least one redox regulatory insertion; and (iii) in increasing order of preference, at least 40%, 45%, 50%, 55%, 65%, 70%, 75%, 80%, 85%, 90%, 95% or 98% sequence identity to SEQ ID NO: 155 (calculations shown in Example 14).

The alignment of multiple polypeptide sequences is used to find conserved domains and characteristic motifs in protein families, in the determination of evolutionary linkage and in the improved prediction of secondary and tertiary structure. Many programs are available to a person skilled in the art to perform such analysis, for example, the ones proposed by the Expasy proteomics toolbox hosted by the Swiss Institute for Bioinformatics.

Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains may also be used. The sequence identity values may be determined over the entire nucleic acid or amino acid sequence or over selected domains or conserved motif(s), using the programs mentioned above using the default parameters.

The terms "domain" and "motif' are described in the definitions section. Special databases exisit for the identification of domains. The FBPase domain in a cpFBPase polypeptide may be identified using, for example, SMART (Schultz et al. (1998) Proc. Natl. Acad. Sci. USA 95, 5857-5864; Letunic et al. (2002) Nucleic Acids Res 30, 242-244; hosted by the EMBL at Heidelberg, Germany), InterPro (Mulder et al., (2003) Nucl. Acids. Res. 31, 315-318; hosted by the European Bioinformatics Institute (EBI) in the United Kingdom), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAIPress, Menlo Park; Hulo et al., Nucl. Acids. Res. 32: D134-D137, (2004), The ExPASy proteomics server is provided as a service to the scientific community (hosted by the Swiss Institute of Bioinformatics (SIB) in Switzerland) or Pfam (Bateman et al., Nucleic Acids Research 30(1): 276-280 (2002), hosted by the Sanger Institute in the United Kingdom). For example, in the InterPro database, the FBPase domain comprised in the cpFBPase polypeptide is designated IPR000146. In Example 15 are listed the entries identified in a number of databases, related to a cpFBPase polypeptide as represented by SEQ ID NO : 155.

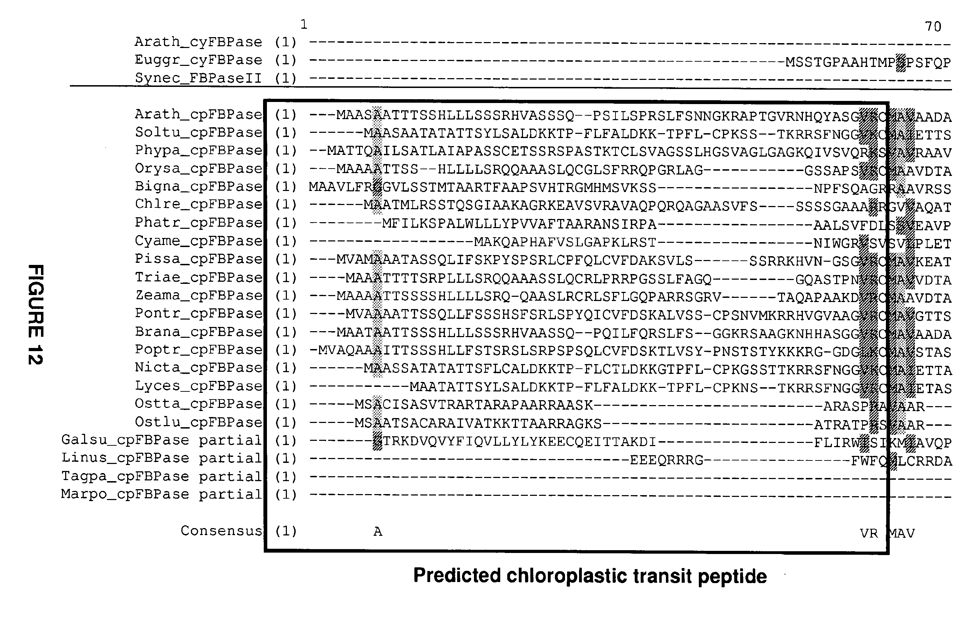

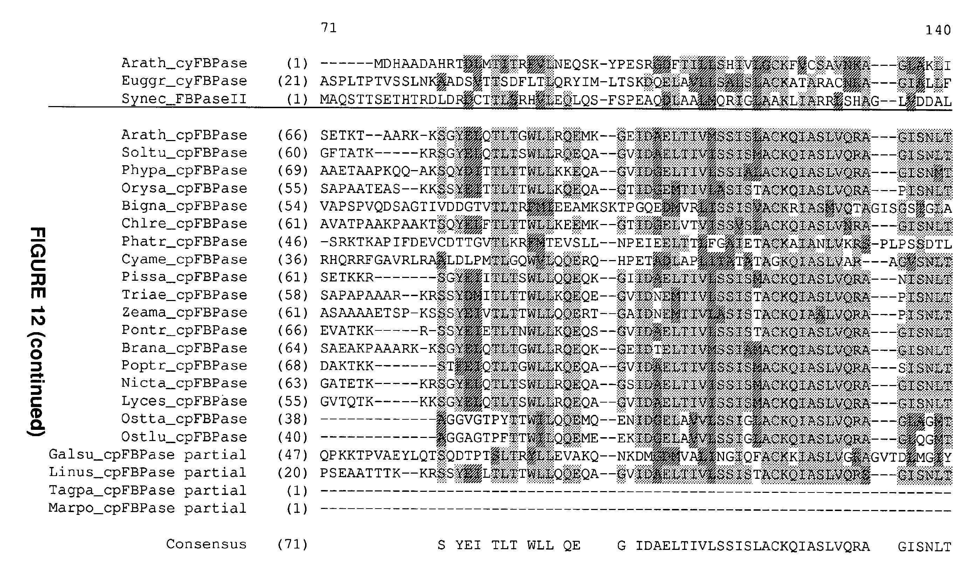

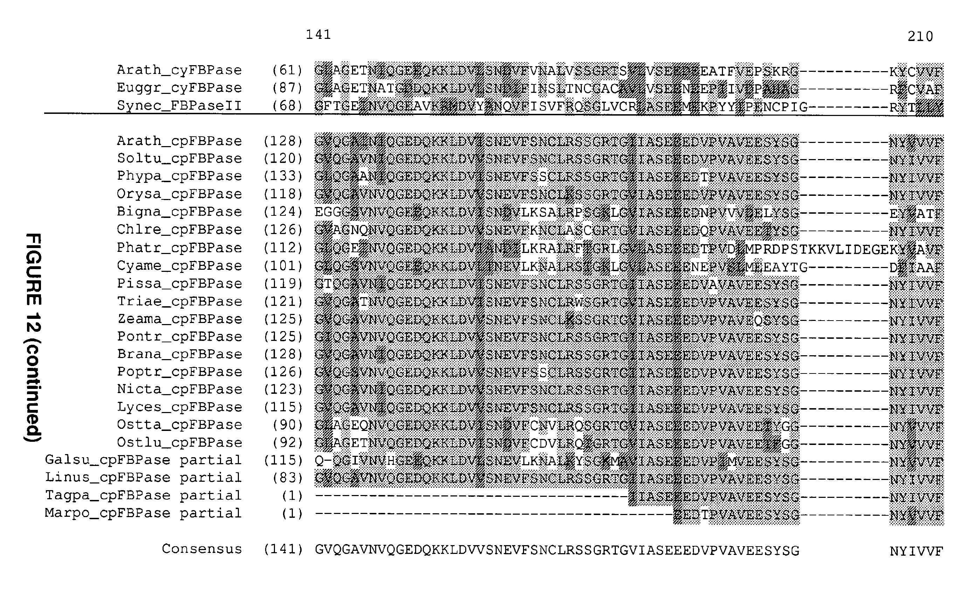

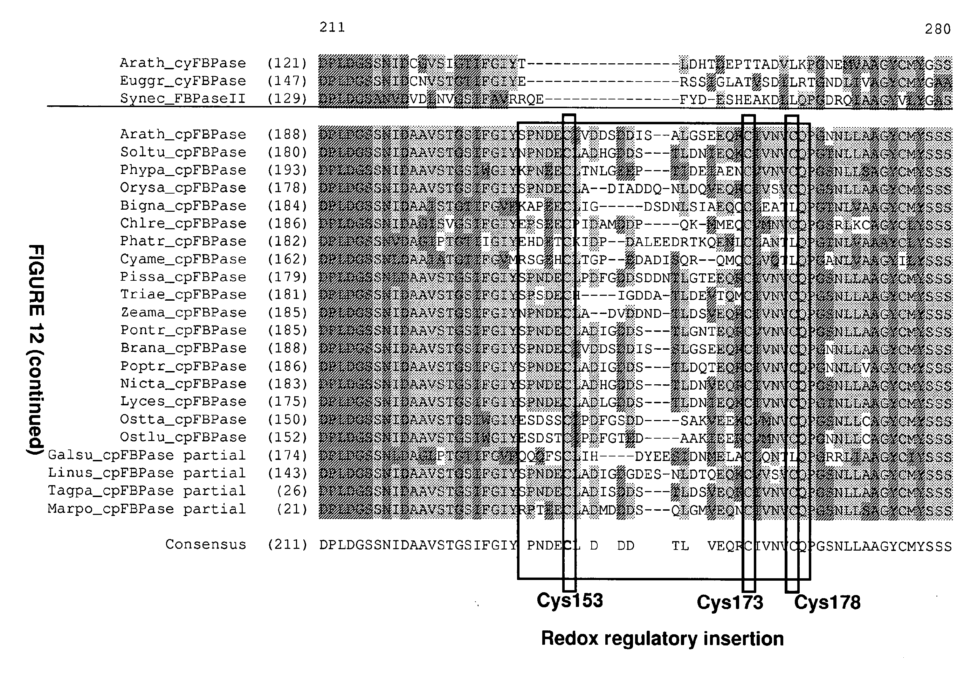

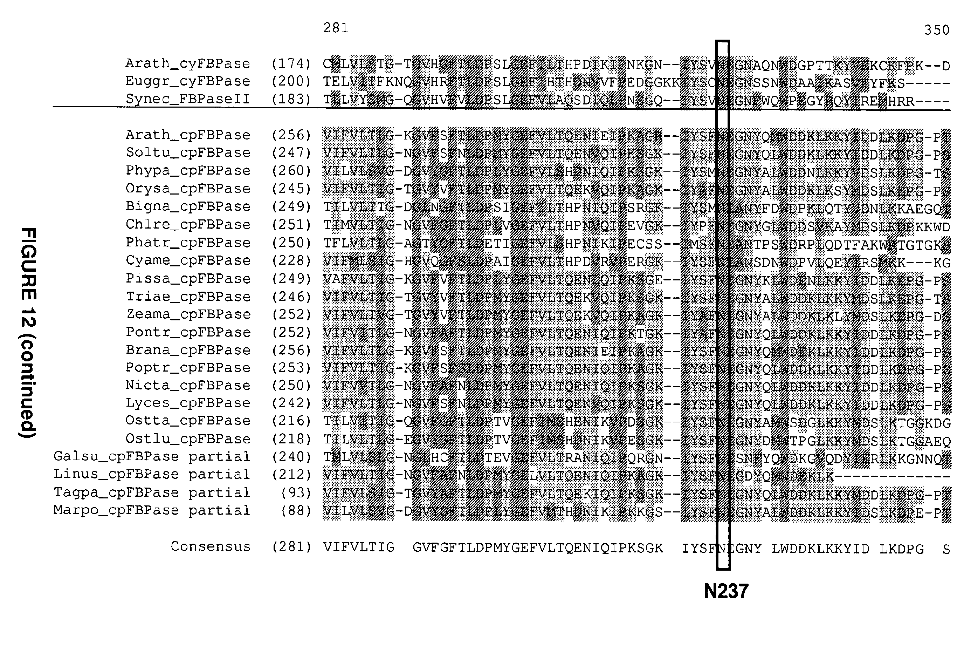

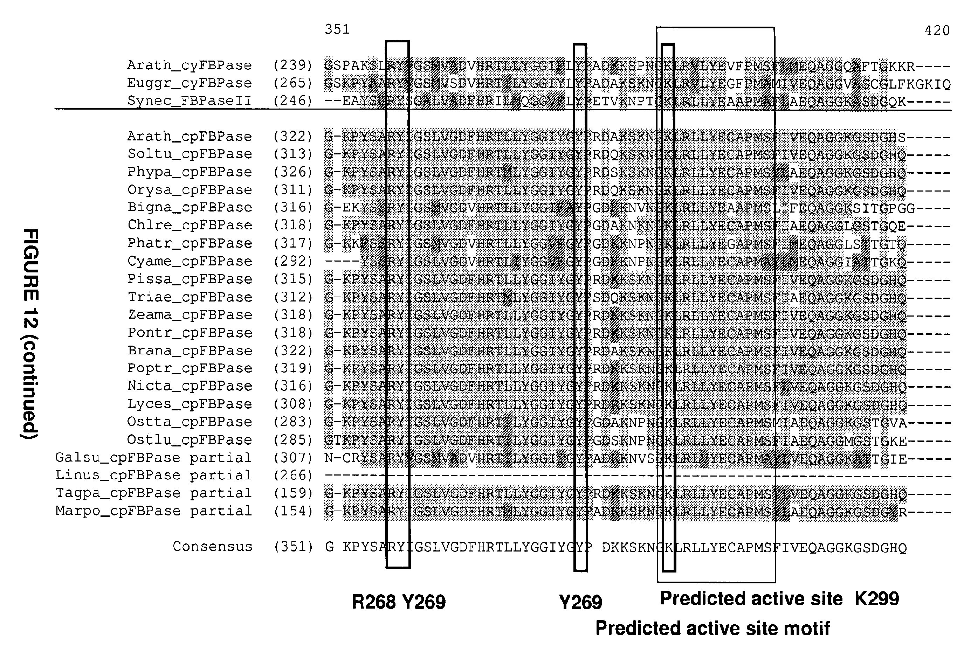

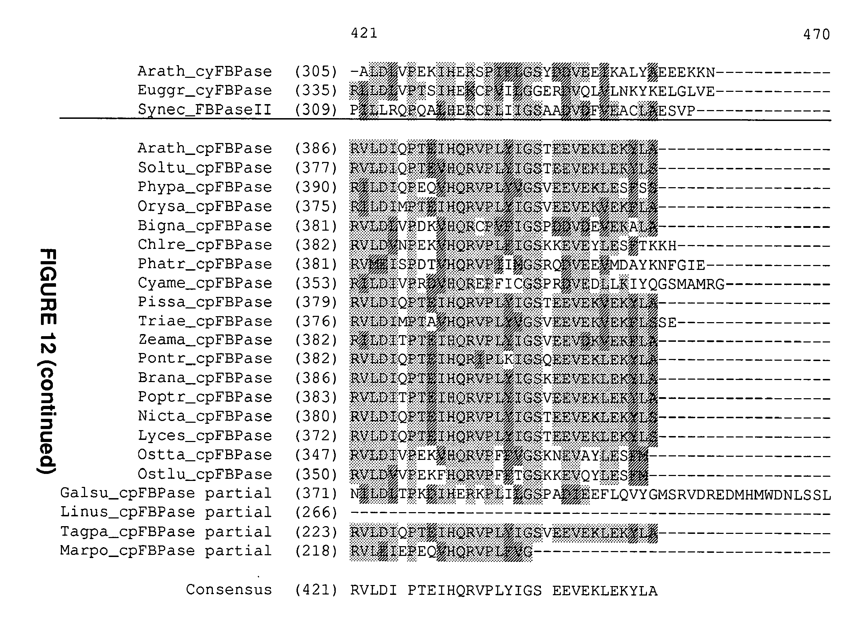

An important motif comprised within the FBPase domain is the redox regulatory insertion, which is present only the cpFBPase polypeptides and not in the cyFBPase polypeptides. By aligning all FBPases polypeptides using the methods described hereinabove and in Example 13 (and Figure 12), an insertion of amino acids comprising at least two cysteine residues necessary for disulphide bridge formation (i.e. redox regulation) is identified. The conserved cysteines are named after their position in the mature pea (*Pisum sativa*) polypeptide, i.e. Cys153, Cys173 and Cys178. Cys153 and Cys173 usually are the two partners involved in disulphide bridge formation (Chiadmi et al. (1999) EMBO J 18(23): 6809-6815). Cys153 and Cys173 are separated by a loop whose length is of 10, 11, 12, 13, 14, 15, 16, 17, 18, 19, 20, 21, 22, 23, 24, or 25 amino acid residues, preferably of 14, 15, 16, 17, 18, or 19 amino acid residues.

The task of protein subcellular localisation prediction is important and well studied. Knowing a protein's localisation helps elucidate its function. Experimental methods for protein localization range from immunolocalization to tagging of proteins using green fluorescent protein (GFP). Such methods are accurate although labor-intensive compared with computational methods. Recently much progress has been made in computational prediction of protein localisation from sequence data. Among algorithms well known to a person skilled in the art are available at the ExPASy Proteomics tools hosted by the Swiss Institute for Bioinformatics, for example, PSort, TargetP, ChloroP, Predotar, LipoP, MITOPROT, PATS, PTS1, SignalP and others. The identification of subcellular localisation of the polypeptide of the invention is shown in Example 16. In particular SEQ ID NO: 155 of the present invention is assigned to the plastidic (chloroplastic) compartment of photosynthetic (autotrophic) cells.

Methods for targeting to plastids are well known in the art and include the use of transit peptides. Table 7 below shows examples of transit peptides which can be used to target any FBPase polypeptide to a plastid, which FBPase polypeptide is not, in its natural form, normally targeted to a plastid, or which FBPase polypeptide in its natural form is targeted to a plastid by virtue of a different transit peptide (for example, its natural transit peptide). For example a nucleic acid sequence encoding a cyFBPase may also be suitable for use in the methods of the invention so long as the nucleic acid is targeted to a plastid, preferably to a chloroplast, and that is comprises at least one regulatory redox insertion.

**Table 7:** Examples of transit peptide sequences useful in targeting amino acids to plastids

| **NCBI Accession Number** | **Source Organism** | **Protein Function** | Transit Peptide Sequence |
|---|---|---|---|
| P07839 | *Chlamydomonas* | Ferredoxin | MAMAMRSTFAARVGAKPAVRGARPAS RMSCMA |
| AAR23425 | *Chlamydomonas* | Rubisco activase | MQVTMKSSAVSGQRVGGARVATRSVR RAQLQV |
| CAA56932 | *Arabidopsis thaliana* | Asp amino transferase | MASLMLSLGSTSLLPREINKDKLKLGTS ASNPFLKAKSFSRVTMTVAVKPSR |
| CAA31991 | *Arabidopsis thaliana* | Acyl carrier protein 1 | MATQFSASVSLQTSCLATTRISFQKPALI SNHGKTNLSFNLRRSIPSRRLSVSC |
| CAB63798 | *Arabidopsis thaliana* | Acyl carrier protein2 | MASIAASASISLQARPRQLAIAASQVKSF SNGRRSSLSFNLRQLPTRLTVSCAAKP ETVDKVCAVVRKQL |
| CAB63799 | *Arabidopsis thaliana* | Acyl carrier protein3 | MASIATSASTSLQARPRQLVIGAKQVKS FSYGSRSNLSFNLRQLPTRLTVYCAAK PETVDKVCAVVRKQLSLKE |

cpFBPase polypeptides as represented by SEQ ID NO: 155 are enzymes with as Enzyme Commission (EC; classification of enzymes by the reactions they catalyse) number EC 3.1.3.11 I for fructose-bisphosphatase (also called D-fructose-1,6-bisphosphate 1-phosphohydrolase). cpFBPase polypeptides catalyze the irreversible conversion of fructose-1,6-bisphophate to fructose-6-phosphate and Pi. The functional assay may be an assay for cpFBPase activity based on a colorimetric Pi assay, as described by Huppe and Buchanan (1989) in Naturforsch. 44c: 487-494. Other methods to assay the enzymatic activity are described by Alscher-Herman (1982) in Plant Physiol 70: 728-734.

By "functioning in the chloroplast" is taken to mean herein that the cpFBPase polypeptide is active in the chloroplast, i.e., the cpFBPase polypeptide is performing the enzymatic reaction consisting in hydrolysing fructose-1, 6-bisphosphate into fructose-6-phosphate and Pi, in the chloroplast.

The nucleic acid sequences encoding cpFBpase polypeptides as given in Table 7, or encoding orthologues or paralogues of any of the aforementioned SEQ ID NOs need not be full-length nucleic acid sequences, since performance of the methods of the invention does not rely on the use of full-length nucleic acid sequences.

cpFBPase nucleic acid variants may also be suitable in practising the methods of the invention. Variant cpFBPase nucleic acid sequences typically are those having the same function as a naturally occurring cpFBPase nucleic acid sequence, which can be the same biological function or the function of increasing yield when expression of the nucleic acid sequence is increased in aboveground parts of a plant relative to a control plant. Examples of such cpFBPase variants include portions of nucleic acid sequences, nucleic acid sequences capable of hybridising to cpFBPases, splice variants, allelic variants either naturally occurring or by DNA manipulation, a cpFBPase nucleic acid sequence obtained by gene shuffling, or a cpFBPase nucleic acid sequence obtained by site-directed mutagenesis.

The term "portion" as used herein refers to a piece of DNA encoding a polypeptide functioning in the chloroplast and comprising: (i) at least one FBPase domain; and (ii) at least one redox regulatory insertion.

A portion may be prepared, for example, by making one or more deletions to a nucleic acid encoding a cpFBPase polypeptide as defined hereinabove. The portions may be used in isolated form or they may be fused to other coding (or non coding) sequences in order to, for example, produce a polypeptide that combines several activities. In another example, the naturally occurring transit peptide coding sequence may be replaced by a transit peptide coding sequence from another photosynthetic organism, or by a synthetic one. If chloroplast transformation is considered, the transit peptide coding sequence may be removed altogether. When fused to other coding sequences, the resultant polypeptide produced upon translation may be bigger than that predicted for the cpFBPase portion. Portions useful in the methods of the invention are typically at least 900 nucleotides in length, preferably at least 1000 nucleotides in length, more preferably at least 1100 nucleotides in length and most preferably at least 1200 nucleotides in length. Preferably, the portion is a portion of a nucleic acid sequence as represented by any one of the nucleic acid sequences given in Table 7, or nucleic acid sequences encoding orthologues or paralogues of any of the aforementioned SEQ ID NOs. Most preferably the portion is a portion of a nucleic acid sequence as represented by SEQ ID NO: 154.

According to the present invention, there is provided a method for increasing yield in plants, comprising increasing expression in a plant of a portion of any one of the nucleic acid sequences given in Table C of Example 12, or of a portion of a nucleic acid encoding an orthologue, paralogue or homologue of any of the amino acid sequences given in Table C of Example 12.

Another nucleic acid variant useful in the methods of the invention, is a nucleic acid capable of hybridising under reduced stringency conditions, preferably under stringent conditions, with a nucleic acid sequence encoding a cpFBPase polypeptide as defined hereinabove, or a with a portion as defined hereinabove.

According to the present invention, there is provided a method for increasing yield in plants, comprising increasing expression in a plant of a nucleic acid capable of hybridizing to any one of the nucleic acids given in Table C of Example 12, or comprising increasing expression in a plant of a nucleic acid capable of hybridising to a nucleic acid encoding an orthologue, paralogue or homologue of any of the nucleic acid sequences given in Table C of Example 12.

Hybridising sequences useful in the methods of the invention, encode a cpFBPase polypeptide functioning in the chloroplast and comprising: (i) at least one FBPase domain; and (ii) at least one redox regulatory insertion, and having substantially the same biological activity as the cpFBPase polypeptide as represented by SEQ ID NO: 155. Methods for designing probes are well known in the art. The hybridising sequence is typically less than 1000 bp in length, preferably less than 900, 800, 700, 600 or 500 bp in length. Commonly, hybridising sequence lengths for DNA-DNA hybridisations such as Southern blotting vary between 100 and 500 bp, whereas for DNA-DNA hybridisations such as in PCR amplification generally shorter than 50 but longer than 10 nucleotides. Preferably, the hybridising sequence is one that is capable of hybridising to any of the nucleic acid sequences (or to probes derived from) as represented by the nucleic acid sequences given in Table 7, or nucleic acid sequences encoding orthologues or paralogues of any of the aforementioned SEQ ID NOs, or to a portion of any of the aforementioned sequences, a portion being as defined above. Most preferably the hybridising sequence is capable of hybridising to SEQ ID NO: 154, or to portions (or probes) thereof.

Another nucleic acid variant useful in the methods of the invention is a splice variant encoding a cpFBPase polypeptide as defined hereinabove. Such variants will be ones in which the biological activity of the protein is substantially retained; this may be achieved by selectively retaining functional segments of the protein. Preferred splice variants are splice variants of the cpFBPase nucleic acid sequences as given in Table 7, or nucleic acid sequences encoding orthologues or paralogues of any of the aforementioned SEQ ID NOs. Most preferred is a splice variant of a cpFBPase nucleic acid sequence as represented by SEQ ID NO: 154.

Another nucleic acid variant useful in performing the methods of the invention is an allelic variant of a nucleic acid sequence encoding a cpFBPase polypeptide as defined hereinabove. Allelic variants exist in nature, and encompassed within the methods of the present invention is the use of these natural alleles. Preferred allelic variants are allelic variants of the cpFBPase nucleic acid sequences as given in Table 7, or nucleic acid sequences encoding orthologues or paralogues of any of the aforementioned SEQ ID NOs. Most preferred is a splice variant of a cpFBPase nucleic acid sequence as represented by SEQ ID NO: 154.

According to the present invention, there is provided a method for increasing yield in plants, comprising increasing expression in a plant of a splice variant of any one of the nucleic acid sequences given in Table C of Example 12, or of a splice variant of a nucleic acid encoding an orthologue, paralogue or homologue of any of the amino acid sequences given in Table C of Example 12.

A further nucleic acid variant useful in the methods of the invention is a nucleic acid variant encoding a cpFBPase polypeptide obtained by gene shuffling (or directed evolution). Most preferred is a nucleic acid variant obtained by gene shuffling of a cpFBPase nucleic acid sequence as represented by SEQ ID NO: 155.

According to the present invention, there is provided a method for increasing yield in plants, comprising increasing expression in a plant of a variant of any one of the nucleic acid sequences given in Table C of Example 12, or comprising increasing expression in a plant of a variant of a nucleic acid encoding an orthologue, paralogue or homologue of any of the amino acid sequences given in Table C of Example 12, which variant nucleic acid is obtained by gene shuffling.

Another nucleic acid variant useful in the methods of the invention is a nucleic acid variant encoding a cpFBPase polypeptide obtained by site-directed mutagenesis. Site-directed mutagenesis may be used to generate variants of cpFBPase nucleic acid sequences. Several methods are available to achieve site-directed mutagenesis, the most common being PCR based methods (Current Protocols in Molecular Biology, Wiley Eds). For example, a mutation affecting the disulfide bridge formation is to change one of the conserved cysteines into serine, thereby making cpFBPase constitutively active (Chiadmi et al. (1999) EMBO J 18(23): 6809-6815). Most preferred is a nucleic acid variant obtained by site-directed mutagenesis of a cpFBPase nucleic acid sequence as represented by SEQ ID NO: 154.

The following cpFBPase nucleic acid variants are examples of variants suitable in practising the methods of the invention:

(i) a portion of a cpFBPase nucleic acid sequence;
(ii) a nucleic acid sequence capable of hybridising with a cpFBPase nucleic acid sequence;
(iii) a splice variant of a cpFBPase nucleic acid sequence;
(iv) an allelic variant of a cpFBPase nucleic acid sequence;
(v) a cpFBPase nucleic acid sequence obtained by gene shuffling;
(vi) a cpFBPase nucleic acid sequence obtained by site-directed mutagenesis.

Also useful in the methods of the invention are nucleic acid sequences encoding homologues of cpFBPase polypeptides as given in Table 7, or encoding orthologues or paralogues of any of the aforementioned SEQ ID NOs.

**[0358]** Also useful in the methods of the invention are nucleic acid sequences encoding derivatives of any one of the cpFBPase polypeptides as given in Table 7, or orthologues or paralogues of any of the aforementioned SEQ ID NOs. Derivatives of cpFBPase polypeptides as represented by any one given in Table 7, or orthologues or paralogues of any of the aforementioned SEQ ID NOs are further examples that may be suitable for use in the methods of the invention.

Nucleic acid sequences encoding cpFBPase polypeptides may be derived from any artificial source or natural source, such as plant, algae, diatom or animal. The nucleic acid sequence may be modified from its native form in composition and/or genomic environment through deliberate human manipulation. Preferably the nucleic acid sequence encoding a cpFBPase polypeptide originates from a photosynthetic cell (Plantae kingdom). Further preferably the nucleic acid sequence encoding a cpFBPase polypeptide originates from a plant cell. More preferably, the nucleic acid sequence encoding a cpFBPase polypeptide originates from a diatom cell. Most preferably the nucleic acid sequence encoding a cpFBPase polypeptide originates from an algal (red, brown or green) cell. The nucleic acid sequence may be isolated from green algae belonging Chlorophyta or Charophyta, or from land plants, non-vascular or vascular. For example, the nucleic acid sequence encoding the cpFBPase polypeptide is isolated from *Chlamydomonas sp., Chlorella sp., Bigelowiella natans, Cyanidioschyzon merolae, Ostreococcus lucimarinus, Ostreococcus tauri, Galderia sulphuraria Physicomitrella patens, Phaeodactylum tricornutum, Aquilegia formosa x Aquilegia pubescen,s Arabidopsis thaliana, Brassica napus, Glycine max, Lycopersicon esculentum, Medicago truncatula, Nicotiana tabacum, Oryza sativa, Pisum sativa, Poncirus trifoliate, Populus tremuloides, Solanum fuberosum, Spinacia oleracea, Triticum aestivum, Zea mays* and more. Most preferably the nucleic acid sequence encoding a cpFBPase polypeptide is from *Chalmydomonas reinhardtii.*

Performance of the methods of the invention gives plants having enhanced yield. The terms "yield", "increased", "improved", "enhanced", "amplified", "extended", "augmented" or "rised" are interchangeable and are defined above. Increased biomass may manifest itself as increased root biomass. Increased root biomass may be due to increased number of roots, increased root thickness and/or increased root length. Increased yield may manifest itself as one or more of the following:

(i) increased biomass (weight) of one or more parts of a plant, particularly aboveground (harvestable) parts, increased root biomass or increased biomass of any other harvestable part;
(ii) increased early vigour, defined herein as the seedling aboveground area three weeks post-germination;
(iii) increased total seed yield, which includes an increase in seed biomass (seed weight) and which may be an increase in the seed weight per plant or on an individual seed basis;
(iv) increased number of panicles per plant;
(v) increased number of flowers ("florets") per panicle;

(vi) increased seed fill rate;
(vii) increased number of (filled) seeds;
(viii) increased seed size (length, width area, perimeter), which may also influence the composition of seeds;
(ix) increased seed volume, which may also influence the composition of seeds;
(x) increased harvest index, which is expressed as a ratio of the yield of harvestable parts, such as seeds, over the total biomass; and
(xi) increased thousand kernel weight (TKW), which is extrapolated from the number of filled seeds counted and their total weight. An increased TKW may result from an increased seed size and/or seed weight. An increased TKW may result from an increase in embryo size and/or endosperm size.

An increase in seed size, seed volume, seed area, seed perimeter, seed width and seed length may be due to an increase in specific parts of a seed, for example due to an increase in the size of the embryo and/or endosperm and/or aleurone and/or scutellum, or other parts of a seed.

In particular, increased yield is increased seed yield, and is selected from one or more of the following: (i) increased seed weight; (ii) increased number of filled seeds; (iii) increased seed fill rate; and (iv) increased harvest index.

Taking corn as an example, a yield increase may be manifested as one or more of the following: increase in the number of plants per square meter, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others.

Taking rice as an example, a yield increase may be manifested by an increase in one or more of the following: number of plants per square meter, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

An increase in yield may also result in modified architecture, or may occur as a result of modified architecture.

According to a preferred feature, performance of the methods of the invention results in plants having increased seed yield relative to control plants. Therefore, according to the present invention, there is provided a method for increasing seed yield, which method comprises increasing expression in aboveground parts of a plant of a nucleic acid sequence encoding a cpFBPase polypeptide.

Since the transgenic plants according to the present invention have increased yield, it is likely that these plants exhibit an increased growth rate (during at least part of their life cycle), relative to the growth rate of control plants at a corresponding stage in their life cycle.

The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the sowing and harvesting of corn plants followed by, for example, the sowing and optional harvesting of soybean, potato or any other suitable plant). Harvesting additional times from the same rootstock in the case of some crop plants may also be possible. Altering the harvest cycle of a plant may lead to an increase in annual biomass production per square meter (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

According to a preferred feature of the present invention, performance of the methods of the invention gives plants having an increased growth rate relative to control plants. Therefore, according to the present invention, there is provided a method for increasing the growth rate of plants, which method comprises modulating expression, preferably increasing expression, in a plant of a nucleic acid encoding a cpFBPase polypeptide as defined herein.

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11% or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi and insects.

In particular, the methods of the present invention may be performed under non-stress conditions or under conditions of mild drought to give plants having increased yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location.

Performance of the methods of the invention gives plants grown under non-stress conditions or under mild drought conditions increased yield relative to control plants grown under comparable conditions. Therefore, according to the present invention, there is provided a method for increasing yield in plants grown under non-stress conditions or under mild drought conditions, which method comprises increasing expression in a plant of a nucleic acid encoding a cpFBPase polypeptide.

Performance of the methods of the invention gives plants grown under conditions of nutrient deficiency, particularly under conditions of nitrogen deficiency, increased yield relative to control plants grown under comparable conditions. Therefore, according to the present invention, there is provided a method for increasing yield in plants grown under conditions of nutrient deficiency, which method comprises increasing expression in a plant of a nucleic acid encoding a cpFBPase polypeptide. Nutrient deficiency may result from a lack of nutrients such as nitrogen, phosphates and other phosphorous-containing compounds, potassium, calcium, cadmium, magnesium, manganese, iron and boron, amongst others.

Preferably the increase in yield and/or growth rate occurs according to the method of invention under non-stress or mild abiotic or mild biotic stress conditions.

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

The term "increasing expression" is defined above. The increase in expression of the nucleic acid sequence encoding a cpFBPase polypeptide in aboveground parts leads to increased yield of the plants relative to control plants. Preferably, the increase in expression of the nucleic acid is 1.25, 1.5, 1.75, 2, 5, 7.5, 10, 15, 20, 25, 30, 35, 40, 45, 50, 55, 60, 65, 70, 75, 80, 85, 90, 95, 100 or more fold the expression of the endogenous plant cpFBPase polypeptide.

The term "aboveground parts of plant" is herein taken to mean plant parts excluding the roots, root hairs and any other plant part that is in the soil, i.e., that is not directly exposed to light.

By increasing the expression (in a plastid) of a nucleic acid sequence encoding a cpFBPase polypeptide, an increase in the amount of cpFBPase polypeptide is obtained. This increase in amount of cpFBPase polypeptide (in a plastid) leads to an increase in cpFBPase activity. Alternatively, activity may also be increased when there is no change in the amount of a cpFBPase polypeptide, or even when there is a reduction in the amount of a cpFBPase polypeptide. This may occur when the intrinsic properties of the polypeptide are altered, for example, by making mutant versions that are more active than the wild type polypeptide.

The expression of a nucleic acid sequence encoding a cpFBPase polypeptide is increased in a plastid using techniques well known in the art, such as by targeting a cpFBPase polypeptide to the plastid using transit peptide sequences or by direct transformation of a cpFBPase polypeptide without transit peptide sequences, into a plastid. Expression may be increased in any plastid, however, preferred is preferentially increasing expression in a chloroplast.

The expression of a nucleic acid sequence encoding a cpFBPase polypeptide may be modulated by introducing a genetic modification, for example, by any one (or more) of the following techniques: T-DNA activation, TILLING, homologous recombination, or by introducing and expressing in a plant a nucleic acid sequence encoding a cpFBPase polypeptide. Following introduction of the genetic modification, there follows a step of selecting for increased expression of a nucleic acid sequence encoding a cpFBPase polypeptide, which increased expression gives plants having increased yield relative to control plants.

One such technique is T-DNA activation tagging. The promoter to be introduced may be any promoter capable of driving expression of a gene in the desired organism, in this case a plant. For example, constitutive, aboveground parts, below ground parts, tissue-preferred, cell type-preferred and inducible promoters are all suitable for use in T-DNA activation. The effects of the invention may also be reproduced using the technique of TILLING or using homologous recombination.

A preferred method for introducing a genetic modification is to introduce and express in aboveground parts of a plant a nucleic acid sequence encoding a cpFBPase polypeptide. The cpFBPase as defined herein refers to a polypeptide functioning in the chloroplast and comprising: (i) at least one FBPase domain; and (ii) at least one redox regulatory insertion.

In one embodiment of the present invention, the expression of a nucleic acid sequence encoding a cpFBPase polypeptide is increased expression (in aboveground parts of plant). The increase in expression may lead to raised cpFBPase mRNA or polypeptide levels, which could equate to raised activity of the cpFBPase polypeptide; or the activity may also be raised when there is no change in polypeptide levels, or even when there is a reduction in polypeptide levels. This may occur when the intrinsic properties of the cpFBPase polypeptide are altered, for example, by making mutant versions that are more active that the wild type polypeptide. Methods for increasing expression of genes or gene products are well documented in the art.

The invention also provides genetic constructs and vectors to facilitate introduction and/or expression of the nucleotide sequences useful in the methods according to the invention.

Therefore, there is provided a genetic construct comprising:

(i) A nucleic acid sequence encoding a cpFBPase polypeptide, as defined hereinabove;
(ii) One or more control sequences capable of driving expression in aboveground parts of a plant, of the nucleic acid sequence of (i); and optionally
(iii) A transcription termination sequence;

A preferred construct is one whether the control sequence is a promoter derived from a plant, preferably from a monocotyledonous plant if a monocotyledonous is to be transformed.

Constructs useful in the methods according to the present invention may be constructed using recombinant DNA technology well known to persons skilled in the art. The genetic constructs may be inserted into vectors, which may be commercially available, suitable for transforming into plants and suitable for expression of the gene of interest in the transformed cells. The invention therefore provides use of a genetic construct as defined hereinabove in the methods of the invention.

Plants are transformed with a vector comprising the sequence of interest (i.e., a nucleic acid sequence encoding a cpFBPase polypeptide). The sequence of interest is operably linked to one or more control sequences (at least to a promoter). The terms "regulatory element", "control sequence" and "promoter" are all used interchangeably herein and are defined above.

Advantageously, the promoter used to drive expression of the nucleic acid sequence may be a tissue-preferred promoter, i.e. one that is capable of preferentially initiating transcription in certain tissues, such as the leaves, stems, seed tissue etc. Promoters able to initiate transcription in certain tissues only are referred to herein as "tissue-specific", similarly, promoters able to initiate transcription in certain cells only are referred to herein as "cell-specific". Additionally or alternatively, the promoter may occur natural or synthetic. Preferably, the promoter is capable of driving expression of a nucleic acid encoding a cpFBPase polypeptide in aboveground parts of a plant, i.e., in parts exposed to light for proper cpFBPase redox regulation.

Other suitable promoters are constitutive promoters (Benfey et al., EMBO J. 8 (1989) 2195-2202), such as those which originate from plant viruses, such as 35S CAMV (Franck et al., Cell 21 (1980) 285-294), 19S CaMV (see also US 5352605 and WO 84/02913), 34S FMV (Sanger et al., Plant. Mol. Biol., 14, 1990: 433-443), the parsley ubiquitin promoter, or plant promoters such as the Rubisco small subunit promoter described in US 4,962,028 or the plant promoters PRP1 [Ward et al., Plant. Mol. Biol. 22 (1993)], SSU, PGEL1, OCS [Leisner (1988) Proc Natl Acad Sci USA 85(5): 2553-2557], lib4, usp, mas [Comai (1990) Plant Mol Biol 15 (3):373-381], STLS1, ScBV (Schenk (1999) Plant Mol Biol 39(6):1221-1230), B33, SAD1 or SAD2 (flax promoters, Jain et al., Crop Science, 39 (6), 1999: 1696-1701) or nos [Shaw et al. (1984) Nucleic Acids Res. 12(20):7831-7846]. Further examples of constitutive plant promoters are the sugarbeet V-ATPase promoters (WO 01/14572). Examples of synthetic constitutive promoters are the Super promoter (WO 95/14098) and promoters derived from G-boxes (WO 94/12015). If appropriate, chemical inducible promoters may furthermore also be used, compare EP-A 388186, EP-A 335528, WO97/06268. Stable, constitutive expression of the polypeptides according to the invention a plant can be advantageous. However, inducible expression of the polypeptide of the invention is advantageous, if a late expression before the harvest is of advantage, as metabolic manipulation may lead to plant growth retardation.

The expression of plant genes can also be facilitated via a chemical inducible promoter. Examples of such promoters are a salicylic acid inducible promoter (WO 95/19443), and abscisic acid-inducible promoter (EP 335 528), a tetracyclin-inducible promoter (Gatz et al. (1992) Plant J. 2, 397-404), a cyclohexanol- or ethanol-inducible promoter (WO 93/21334) or others as described herein.

Other suitable promoters are those that react to biotic or abiotic stress, for example the pathogen-induced PRP1 gene promoter (Ward et al., Plant. Mol. Biol. 22 (1993) 361-366), the tomato heat-inducible hsp80 promoter (US 5,187,267), the potato chill-inducible alpha-amylase promoter (WO 96/12814) or the wound-inducible pinII promoter (EP-A-0 375 091) or others as described herein.

Preferred promoters are in particular those which effect expression in tissues and organs, in seed cells, such as endosperm cells and cells of the developing embryo. Suitable promoters are the oilseed rape napin gene promoter (US 5,608,152), the Vicia faba USP promoter (Baeumlein et al., Mol Gen Genet, 1991, 225 (3): 459-67), the Arabidopsis oleosin promoter (WO 98/45461), the Phaseolus vulgaris phaseolin promoter (US 5,504,200), the Brassica Bce4 promoter (WO 91/13980), the bean arc5 promoter, the carrot DcG3 promoter, or the Legumin B4 promoter (LeB4; Baeumlein et al., 1992, Plant Journal, 2 (2): 233-9), and promoters which bring about the seed-specific expression in monocotyledonous plants such as maize, barley, wheat, rye, rice and the like. Advantageous seed-specific promoters are the sucrose binding protein promoter (WO 00/26388), the phaseolin promoter and the napin promoter. Suitable promoters which must be considered are the barley lpt2 or lpt1 gene promoter (WO 95/15389 and WO 95/23230), and the promoters described in WO 99/16890 (promoters from the barley hordein gene, the rice glutelin gene, the rice oryzin gene, the rice prolamin gene, the wheat gliadin gene, the wheat glutelin gene, the maize zein gene, the oat glutelin gene, the sorghum kasirin gene and the rye secalin gene). Further suitable promoters are Amy32b, Amy 6-6 and Aleurain [US 5,677,474], Bce4 (oilseed rape) [US 5,530,149], glycinin (soya) [EP 571 741], phosphoenolpyruvate carboxylase (soya) [JP 06/62870], ADR12-2 (soya) [WO 98/08962], isocitrate lyase (oilseed rape) [US 5,689,040] or $\alpha$-amylase (barley) [EP 781 849].

Other promoters which are available for the expression of genes in plants are leaf-specific promoters such as those described in DE-A 19644478 or light-regulated promoters such as, for example, the pea petE promoter.

In one embodiment, the nucleic acid sequence is operably linked to a constitutive promoter. A constitutive promoter is transcriptionally active during most, but not necessarily all, phases of its growth and development and is substantially ubiquitously expressed (including in aboveground parts of a plant). Preferably the promoter is derived from a plant, more preferably the promoter is from a monocotyledonous plant if a monocotyledonous plant is to be transformed. Further preferably, the constitutive promoter is a GOS2 promoter that is represented by a nucleic acid sequence substantially similar to SEQ ID NO: 208 or SEQ ID NO: 56. Most preferably the GOS2 promoter is as represented by SEQ ID NO: 56 or SEQ ID NO: 208. It should be clear that the applicability of the present invention is not restricted to the cpFBPase nucleic acid sequence as represented by SEQ ID NO: 154, nor is the applicability of the invention restricted to expression of a cpFBPase nucleic acid sequence when driven by a GOS2 promoter. Examples of other constitutive promoters that may also be used to drive expression of a cpFBPase nucleic acid sequence are shown above.

Optionally, one or more terminator sequences may be used in the construct introduced into a plant. Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section. Different markers are preferred, depending on the organism and the selection method.

The present invention also encompasses plants (including seeds) obtainable by the methods according to the present invention. The present invention therefore provides plants, plant parts and plant cells obtainable by the methods according to the present invention, which plants have introduced therein a cpFBPase nucleic acid sequence and which plants, plant parts and plant cells are preferably from a crop plant, further preferably from a monocotyledonous plant.

The invention also provides a method for the production of transgenic plants having increased yield relative to control plants, comprising introduction and expression in a plant of a nucleic acid sequence encoding a cpFBPase polypeptide.

More specifically, the present invention provides a method for the production of transgenic plants, preferably monocotyledonous plants, having increased yield relative to control plants, which method comprises:

(i) introducing and expressing in aboveground parts of a plant or plant cell a nucleic acid sequence encoding a cpFBPase polypeptide; and
(ii) cultivating the plant cell under conditions promoting plant growth and development.

The nucleic acid of (i) may be any of the nucleic acids capable of encoding a cpFBPase polypeptide as defined herein.

The nucleic acid sequence may be introduced directly into a plant cell or into the plant itself (including introduction into a tissue, organ or any other part of a plant). According to a preferred feature of the present invention, the nucleic acid sequence is introduced into a plant by transformation.

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may be evaluated, for instance using Southern analysis, for the presence of the gene of interest, copy number and/or genomic organisation. Alternatively or additionally, expression levels of the newly introduced DNA may be monitored using Northern and/or Western analysis, quantitative PCR, such techniques being well known to persons having ordinary skill in the art.

The generated transformed plants may be propagated by a variety of means, such as by clonal propagation or classical breeding techniques. For example, a first generation (or T1) transformed plant may be selfed to give homozygous second generation (or T2) transformants, and the T2 plants further propagated through classical breeding techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum and oats.

Other advantageous plants are selected from the group consisting of Asteraceae such as the genera Helianthus, Tagetes e.g. the species Helianthus annus [sunflower], Tagetes lucida, Tagetes erecta or Tagetes tenuifolia [Marigold], Brassicaceae such as the genera Brassica, Arabadopsis e.g. the species Brassica napus, Brassica rapa ssp. [canola, oilseed rape, turnip rape] or Arabidopsis thaliana; Fabaceae such as the genera Glycine e.g. the species Glycine max, Soja hispida or Soja max [soybean]; Linaceae such as the genera Linum e.g. the species Linum usitatissimum, [flax, linseed]; Poaceae such as the genera Hordeum, Secale, Avena, Sorghum, Oryza, Zea, Triticum e.g. the species Hordeum vulgare [barley]; Secale cereale [rye], Avena sativa, Avena fatua, Avena byzantina, Avena fatua var. sativa, Avena hybrida [oat], Sorghum bicolor [Sorghum, millet], Oryza sativa, Oryza latifolia [rice], Zea mays [corn, maize] Triticum aestivum, Triticum durum, Triticum turgidum, Triticum hybernum, Triticum macha, Triticum sativum or Triticum vulgare [wheat, bread wheat, common wheat]; Solanaceae such as the genera Solanum, Lycopersicon e.g. the species Solanum tuberosum [potato], Lycopersicon esculentum, Lycopersicon lycopersicum, Lycopersicon pyriforme, Solanum integrifolium or Solanum lycopersicum [tomato].

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic (s) as those produced by the parent in the methods according to the invention. The invention also includes host cells containing an isolated cpFBPase nucleic acid sequence. Preferred host cells according to the invention are plant cells. The invention also extends to harvestable parts of a plant such as, but not limited to seeds, leaves, fruits, flowers, stem cultures, rhizomes, tubers and bulbs. The invention furthermore relates to products derived, preferably directly derived, from a harvestable part of such a plant, such as dry pellets or powders, meal, oil, fat and fatty acids, starch or proteins.

The present invention also encompasses use of cpFBPase nucleic acid sequences and use of cpFBPase polypeptides, and use of a construct as defined hereinabove in increasing plant yield relative to control plants. The increased plant yield is in particular increased seed yield. By increased seed yield is herein taken to mean any one of the following: (i) increased seed weight; (ii) increased number of filled seeds; (iii) increased seed fill rate; and (iv) increased harvest index.

cpFBPase nucleic acid sequences or cpFBPase polypeptides may find use in breeding programmes in which a DNA marker is identified that may be genetically linked to a cpFBPase locus. The cpFBPase nucleic acid sequences or cpFBPase polypeptides may be used to define a molecular marker. This DNA or polypeptide marker may then be used in breeding programmes to select plants having increased yield. The cpFBPase gene may, for example, be a nucleic acid sequence as represented by any one of the cpFBPase nucleic acid sequences as given in Table 7, or nucleic acid sequences encoding orthologues or paralogues of any of the aforementioned SEQ ID NOs.

**[0414]** Allelic variants of a cpFBPase nucleic acid sequence may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question, for example, different allelic variants of any one of the cpFBPase nucleic acid sequences as given in Table 7, or nucleic acid sequences encoding orthologues or paralogues of any of the aforementioned SEQ ID NOs. Growth performance may be monitored in a greenhouse or in the field. Further optional steps include crossing plants, in which the superior allelic variant was identified, with another plant. This could be used, for example, to make a combination of interesting phenotypic features.

cpFBPase nucleic acid sequences may also be used as probes for genetically and physically mapping the genes that they are a part of, and as markers for traits linked to those genes. Such information may be useful in plant breeding in order to develop lines with desired phenotypes. Such use of cpFBPase nucleic acid sequences requires only a nucleic acid sequence of at least 15 nucleotides in length. The cpFBPase nucleic acid sequences may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the cpFBPase nucleic acid sequences. The resulting banding patterns may then be subjected to genetic analyses using computer programs such as MapMaker (Lander et al. (1987) Genomics 1: 174-181) in order to construct a genetic map. In addition, the nucleic acid sequences may be used to probe Southern blots containing restriction endonuclease-treated genomic DNAs of a set of individuals representing parent and progeny of a defined genetic cross. Segregation of the DNA polymorphisms is noted and used to calculate the position of the cpFBPase nucleic acid sequence in the genetic map previously obtained using this population (Botstein et al. (1980) Am. J. Hum. Genet. 32:314-331).

The production and use of plant gene-derived probes for use in genetic mapping is described in Bematzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence *in situ* hybridization (FISH) mapping (Trask (1991) Trends Genet. 7: 149-154). Although current methods of FISH mapping favor use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acid sequences. Examples include allele-specific amplification (Kazazian (1989) J. Lab. Clin. Med 11: 95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the nucleic acid sequence is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having increased yield relative to control plants, as described hereinbefore. These yield-enhancing traits may also be combined with other economically advantageous traits, such as further yield-enhancing traits, tolerance to various stresses, traits modifying various architectural features and/or biochemical and/or physiological features.

**Detailed description for the SIK polypeptide**

It has now been found that modulating expression in a plant of a SIK nucleic acid and/or a SIK polypeptide gives plants having various improved yield-related traits relative to control plants, wherein overexpression of a SIK-encoding nucleic acid in a plant gives increased number of flowers per plant relative to control plants, and wherein the reduction or substantial elimination of a SIK nucleic acid gives increased thousand kernel weight, increased harvest index and increased fill rate relative to corresponding wild type plants.

Therefore, the invention provides a method for improving yield-related traits in plants relative to control plants, comprising modulating expression in a plant of a SIK nucleic acid and/or a SIK polypeptide, wherein the modulated expression is overexpression of a SIK-encoding nucleic acid in a plant resulting in an increased number of flowers per plant relative to control plants, and wherein the modulated expression is a reduction or substantial elimination of a SIK nucleic acid resulting in an increased thousand kernel weight (TKW), increased harvest index (HI) and increased fill rate relative to corresponding wild type plants.

The term "modulation" as defined herein is taken to mean a change in the level of gene expression in comparison to a control plant. In the case where a SIK-encoding nucleic acid is being used to increase the number of flowers per plant, the expression level is increased, and in the case where a SIK nucleic acid is being used to increase TKW, HI or fill rate, the expression level is decreased. The original, unmodulated expression may be of any kind of expression of a structural RNA (rRNA, tRNA) or mRNA with subsequent translation.

The term "expression" or "gene expression" means the transcription of a specific gene or specific genes or specific genetic construct. The term "expression" or "gene expression" in particular means the transcription of a gene or genes or genetic construct into structural RNA (rRNA, tRNA) or mRNA with or without subsequent translation of the latter into a protein. The process includes transcription of DNA and processing of the resulting mRNA product.

The various improved yield-related traits are improved by at least 5%, 6%, 7%, 8%, 9% or 10%, preferably at least 15% or 20%, more preferably 25%, 30%, 35%, 40%, 45%, 50%, 55%, 60%, 65% or 70% compared to control plants.

The improvement may be in one or more of the following: increased number of flowers per plant, increased seed filling rate (which as defined herein is the ratio between the number of filled seeds divided by the total number of seeds; increased harvest index, which as defined herein is the ratio of the yield of harvestable parts, such as seeds, divided by the total biomass; and increased thousand kernel weight (TKW), which as defined herein is derived by extrapolating the number of filled seeds counted and their total weight. Increased TKW may result from an increased seed size and/or seed weight, and may also result from an increase in embryo and/or endosperm size.

An increase in thousand kernel weight, increased harvest index and increased fill rate rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11% or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi and insects.

In particular, the methods of the present invention may be performed under non-stress conditions or under conditions of mild drought to give plants having increased thousand kernel weight, increased harvest index and increased fill rate relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signalling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location.

Performance of the methods of the invention gives plants grown under non-stress conditions or under mild drought conditions increased thousand kernel weight, increased harvest index and increased fill rate relative to control plants grown under comparable conditions. Therefore, according to the present invention, there is provided a method for increasing thousand kernel weight, increased harvest index and increased fill rate in plants grown under non-stress conditions or under mild drought conditions, which method comprises increasing expression in a plant of a nucleic acid encoding a SIK polypeptide.

Performance of the methods of the invention gives plants grown under conditions of nutrient deficiency, particularly under conditions of nitrogen deficiency, increased thousand kernel weight, increased harvest index and increased fill rate relative to control plants grown under comparable conditions. Therefore, according to the present invention, there is provided a method for increasing thousand kernel weight, increased harvest index and increased fill rate in plants grown under conditions of nutrient deficiency, which method comprises increasing expression in a plant of a nucleic acid encoding a SIK polypeptide. Nutrient deficiency may result from a lack of nutrients such as nitrogen, phosphates and other phosphorous-containing compounds, potassium, calcium, cadmium, magnesium, manganese, iron and boron, amongst others.

The present invention encompasses plants or parts thereof (including seeds) obtainable by the methods according to the present invention. The plants or parts thereof comprise a nucleic acid transgene encoding a SIK polypeptide as defined above.

According to one aspect of the present invention, a reduction or substantial elimination of a SIK nucleic acid results in one or more of an increased thousand kernel weight (TKW), increased harvest index (HI) and increased fill rate relative to control plants.

Reference herein to a "SIK nucleic acid" is taken to mean a polymeric form of a deoxyribonucleotide or a ribonucleotide polymer of any length, either double- or single-stranded, or analogues thereof, that has the essential characteristic of a natural ribonucleotide in that it can hybridise to SIK nucleic acid sequences in a manner similar to naturally occurring polynucleotides.

Reference herein to "a reduction or substantial elimination of a SIK nucleic acid or gene" and to an "endogenous" SIK gene is as described in the definitions section.

For the reduction or substantial elimination of expression an endogenous SIK gene in a plant, a sufficient length of substantially contiguous nucleotides of a SIK nucleic acid sequence is required. The stretch of substantially contiguous nucleotides may be derived from any SIK nucleic acid, preferably a SIK nucleic acid represented by any one of SEQ ID NO 213 to 225 or any one of the nucleic acid sequences given in Table 8 or Table 9 below. A SIK nucleic acid sequence encoding a (functional) polypeptide is not a requirement for the various methods discussed herein for the reduction or substantial elimination of expression of an endogenous SIK gene.

This reduction or substantial elimination may be achieved using routine tools and techniques, as described above. A preferred method for the reduction or substantial elimination of expression of a SIK nucleic acid is by introducing and expressing in a plant a genetic construct into which the SIK nucleic acid is cloned as an inverted repeat (in part or completely), separated by a spacer (non-coding DNA).

According to another aspect of the present invention, overexpression in a plant of a SIK-encoding nucleic acid results in an increased number of flowers per plant relative to control plants.

A preferred method for overexpression of a SIK-encoding nucleic acid is by introducing and expressing in a plant a nucleic acid encoding a SIK polypeptide as defined below.

A "SIK-encoding nucleic acid" encodes a "SIK polypeptide" or "SIK amino acid sequence" which as defined herein is taken to mean a polypeptide according to SEQ ID NO: 210 and orthologues and paralogues thereof as defined herein.

The SIK polypeptide represented by SEQ ID NO: 210 and orthologues and paralogues thereof may typically also comprise the following features.

### ATP-binding region

ATP-binding region VSESLTSTSYKASFRDDFTDPKTIEAIVSRL (Motif 1) or an ATP-binding region having in increasing order of preference at least 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% identity to Motif I.

### Serine threonine kinase active site signature

Serine threonine kinase active site signature AMYN*D*FSTSNIQI with conserved D residue (Motif 2) or a serine threonine kinase active site signature having in increasing order of preference at least 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% identity to Motif II.

### Serine-rich domain

An N-terminal serine-rich domain.

### Myristoylation sites

The orthologues and paralogues may further comprise one or more myristoylation sites that could serve to anchor the protein to the membrane.

### Kinase activity

Furthermore, the SIK orthologues and paralogues will exhibit kinase activity of which can readily be determined using routine tools and techniques. Several assays are available (for example Current Protocols in Molecular Biology, Volumes 1 and 2, Ausubel et al. (1994), Current Protocols; or online such as http://www.protocol-online.org).

In brief, the kinase assay generally involves (1) bringing the kinase protein into contact with a substrate polypeptide containing the target site to be phosphorylated; (2) allowing phosphorylation of the target site in an appropriate kinase buffer under appropriate conditions; (3) separating phosphorylated products from non-phosphorylated substrate after a suitable reaction period. The presence or absence of kinase activity is determined by the presence or absence of a phosphorylated target. In addition, quantitative measurements may be performed.

Purified SIK proteins, or cell extracts containing or enriched in the SIK protein could be used as source for the kinase protein. As a substrate, small peptides are particularly well suited. The peptide must comprise one or more serine, threonine, or tyrosine residues in a phosphorylation site motif. A compilation of phosphorylation sites may be found in Biochimica et Biophysica Acta 1314, 191-225, (1996). In addition, the peptide substrates may advantageously have a net positive charge to facilitate binding to phosphocellulose filters, (allowing to separate the phosphorylated from non-phosphorylated peptides and to detect the phosphorylated peptides). If a phosphorylation site motif is not known, a general tyrosine kinase substrate may be used. For example, "Src-related peptide" (RRLIEDAEYAARG) is a substrate for many receptor and non-receptor tyrosine kinases). To determine the kinetic parameters for phosphorylation of the synthetic peptide, a range of peptide concentrations is required. For initial reactions, a peptide concentration of 0.7-1.5 mM may be used.

For each kinase enzyme, it is important to determine the optimal buffer, ionic strength, and pH for activity. A standard 5x Kinase Buffer generally contains 5 mg/ml BSA (Bovine Serum Albumin preventing kinase adsorption to the assay tube), 150 mM Tris-Cl (pH 7.5), 100 mM $MgCl_2$. Divalent cations are required for most tyrosine kinases, although some tyrosine kinases (for example, insulin-, IGF-1-, and PDGF receptor kinases) require $MnCl_2$ instead of $MgCl_2$ (or in addition to $MgCl_2$). The optimal concentrations of divalent cations must be determined empirically for each protein kinase.

A commonly used donor of the phophoryl group is radio-labelled [gamma-$^{32}$P]ATP (normally at 0.2 mM final concentra-

tion). The amount of $^{32}P$ incorporated in the peptides may be determined by measuring activity on the nitrocellulose dry pads in a scintillation counter.

Alternatively or additionally, the activity of a SIK orthologue or paralogue may be assayed by overexpression in a plant of a nucleic acid encoding a SIK polypeptide under the control of a constitutive promoter to check for increase the number of flowers per plant relative to control plants, and also by the reduction or substantial elimination of a SIK nucleic acid under the control of a constitutive promoter to check for one or more of an increase in thousand kernel weight (TKW), harvest index (HI) and fill rate in plants relative to control plants.

The invention is illustrated by transforming plants with the *Oryza sativa* sequence represented by SEQ ID NO: 209, encoding the polypeptide sequence of SEQ ID NO: 210, however performance of the invention is not restricted to these sequences. The methods of the invention may advantageously be performed using any nucleic acid encoding a SIK polypeptide as defined herein, such as any of the nucleic acid sequences given in Table 8 and Table 9. The examples of orthologues and paralogues of SEQ ID NO: 210 given in Table 8 and Table 9 were obtained from The Institute of Genetic research (TIGR). The % identity given in Table 9 is on a nucleotide level. The accession number given starting with 'TC' identifies the sequence as found through TIGR. In case of partial nucleic acids, it would be well within the capabilities of a person skilled in the art to obtain the full length sequence (or at least a sufficient length of sequence for performing the methods of the invention).

**Table 8**: Examples of putative SIK orthologues and paralogues

| # | TC | Putative function |
|---|---|---|
| 1 | Arabidopsis\|TC272322(Q9C9Y3) | Hypothetical protein F17014.23 |
| 2 | Barley\|TC134680 | (Q8RUD7) Similar to protein kinase AtSIK (P0485B12.21 protein) |
| 3 | Cotton\|TC30779 | (Q9C9Y3) Hypothetical protein F17014.23 |
| 4 | Grape\|TC43027 | (Q94C15) Protein kinase AtSIK |
| 5 | L.japonicus\|TC17767 | (Q94C15) Protein kinase AtSIK |
| 6 | LettuceITC15801 | protein kinase AtSIK |
| 7 | Maize\|TC255367 | (Q8RUD7) Similar to protein kinase AtSIK (P0485B12.21 protein) |
| 8 | Maize\|TC272965 | (Q8RUD7) Similar to protein kinase AtSIK (P0485B12.21 protein) |
| 9 | Medicago\|TC96175 | (Q9C9Y3) Hypothetical protein F17014.23 |
| 10 | Pepper\|TC5595 | (Q94C15) Protein kinase AtSIK |
| 11 | Potato\|TC117743 | Protein kinase domain putative |
| 12 | Rice\|TC268277 | (Q8RUD7) Similar to protein kinase AtSIK (P0485B12.21 protein) |
| 13 | S.officinarum\|TC67974(Q8RUD7) | Similar to protein kinase AtSIK (P0485B12.21 protein) |
| 14 | Sorghum\|TC103780 | (Q8RUD7) Similar to protein kinase AtSIK (P0485B12.21 protein) |
| 15 | Soybean\|TC229774 | (Q94C15) Protein kinase AtSIK |
| 16 | Tomato\|TC158378 | 68416.m01018 protein kinase family protein contains protein kinase domain Pfam:PF00069 |
| 17 | Tomato\|TC166426 | 68416.m01018 protein kinase family protein contains protein kinase domain Pfam:PF00069 |
| 18 | Wheat\|TC257477 | (Q8RUD7) Similar to protein kinase AtSIK (P0485B12.21 protein) |

**Table 9:** Examples of putative SIK orthologues and paralogues.

| Sequence1 | Sequence2 | % Identity | Match length | p-value | Recip. best hits |
|---|---|---|---|---|---|
| Barley\|TC134680 | Arabidopsis\|TC272322 | 63 | 1030 | 6.80E-66 | * |
| Cotton\|TC30779 | Arabidopsis\|TC272322 | 71 | 1008 | 9.40E-103 | * |
| Cotton\|TC30779 | Barley\|TC134680 | 68 | 971 | 8.30E-80 | * |

(continued)

| Sequence1 | Sequence2 | % Identity | Match length | p-value | Recip. best hits |
|---|---|---|---|---|---|
| Grape\|TC43027 | Arabidopsis\|TC272322 | 60 | 619 | 4.30E-25 | * |
| Grape\|TC43027 | Cotton\|TC30779 | 80 | 245 | 5.60E-29 | * |
| Grape\|TC43027 | Maize\|TC272965 | 58 | 483 | 3.20E-13 | * |
| Grape\|TC43027 | Rice\|TC268277 | 66 | 318 | 3.50E-19 | * |
| Grape\|TC43027 | S.officinarum\|TC67974 | 63 | 372 | 2.40E-17 | * |
| Grape\|TC43027 | Soybean\|TC229774 | 77 | 276 | 4.60E-30 | * |
| Grape\|TC43027 | Tomato\|TC158378 | 69 | 420 | 9.20E-33 | * |
| Grape\|TC43027 | Tomato\|TC166426 | 75 | 241 | 1.80E-23 | |
| L.japonicus\|TC17767 | Arabidopsis\|TC272322 | 72 | 659 | 1.30E-64 | * |
| L.japonicus\|TC17767 | Barley\|TC134680 | 67 | 659 | 4.40E-53 | * |
| L.japonicus\|TC17767 | Cotton\|TC30779 | 80 | 654 | 9.20E-89 | * |
| Lettuce\|TC15801 | Arabidopsis\|TC272322 | 69 | 529 | 8.90E-44 | * |
| Lettuce\|TC15801 | Cotton\|TC30779 | 77 | 501 | 1.20E-58 | * |
| Leftuce\|TC15801 | L.japonicus\|TC17767 | 75 | 412 | 2.20E-45 | * |
| Maize\|TC255367 | Arabidopsis\|TC272322 | 62 | 528 | 5.50E-24 | * |
| Maize\|TC255367 | Barley\|TC134680 | 81 | 451 | 1.10E-60 | * |
| Maize\|TC255367 | Cotton\|TC30779 | 63 | 738 | 6.90E-44 | * |
| Maize\|TC255367 | L.japonicus\|TC17767 | 66 | 366 | 2.20E-24 | * |
| Maize\|TC255367 | Lettuce\|TC15801 | 65 | 455 | 4.10E-30 | * |
| Maize\|TC272965 | Barley\|TC134680 | 68 | 623 | 2.90E-43 | |
| Medicago\|TC96175 | Arabidopsis\|TC272322 | 67 | 1245 | 2.70E-102 | * |
| Medicago\|TC96175 | Barley\|TC134680 | 65 | 1069 | 2.50E-75 | * |
| Medicago\|TC96175 | Cotton\|TC30779 | 76 | 1041 | 2.20E-125 | * |
| Medicago\|TC96175 | L.japonicus\|TC17767 | 89 | 666 | 3.50E-114 | * |
| Medicago\|TC96175 | Lettuce\|TC15801 | 73 | 504 | 1.00E-51 | * |
| Medicago\|TC96175 | Maize\|TC255367 | 62 | 813 | 3.60E-41 | * |
| Pepper\|TC5595 | Arabidopsis\|TC272322 | 66 | 696 | 6.40E-49 | * |
| Pepper\|TC5595 | Barley\|TC134680 | 66 | 510 | 2.90E-38 | * |
| Pepper\|TC5595 | Cotton\|TC30779 | 76 | 553 | 1.60E-66 | * |
| Pepper\|TC5595 | L.japonicus\|TC17767 | 75 | 474 | 4.50E-53 | * |
| Pepper\|TC5595 | Lettuce\|TC15801 | 75 | 541 | 3.30E-60 | * |
| Pepper\|TC5595 | Maize\|TC255367 | 66 | 462 | 1.00E-29 | * |
| Pepper\|TC5595 | Medicago\|TC96175 | 67 | 800 | 1.30E-60 | * |
| Potato\|TC117743 | Arabidopsis\|TC272322 | 64 | 1082 | 8.80E-65 | * |
| Potato\|TC117743 | Barley\|TC134680 | 66 | 565 | 1.30E-39 | * |
| Potato\|TC117743 | Cotton\|TC30779 | 77 | 587 | 1.80E-70 | * |
| Potato\|TC117743 | L.japonicus\|TC17767 | 75 | 496 | 3.60E-54 | * |

(continued)

| Sequence1 | Sequence2 | % Identity | Match length | p-value | Recip. best hits |
|---|---|---|---|---|---|
| Potato\|TC117743 | Lettuce\|TC15801 | 77 | 543 | 2.30E-65 | * |
| Potato\|TC117743 | Maize\|TC255367 | 67 | 444 | 1.30E-33 | * |
| Potato\|TC117743 | Medicago\|TC96175 | 68 | 804 | 4.20E-64 | * |
| Potato\|TC117743 | Pepper\|TC5595 | 82 | 826 | 1.60E-121 | * |
| Rice\|TC268277 | Arabidopsis\|TC272322 | 63 | 1019 | 3.10E-66 | * |
| Rice\|TC268277 | Barley\|TC134680 | 78 | 1371 | 5.70E-179 | * |
| Rice\|TC268277 | Coffon\|TC30779 | 64 | 1436 | 3.40E-101 | * |
| Rice\|TC268277 | L.japonicus\|TC17767 | 70 | 656 | 1.90E-61 | * |
| Rice\|TC268277 | Maize\|TC255367 | 81 | 860 | 4.50E-121 | * |
| Rice\|TC268277 | Maize\|TC272965 | 74 | 646 | 4.00E-68 | |
| Rice\|TC268277 | Medicago\|TC96175 | 63 | 1684 | 3.00E-104 | * |
| Rice\|TC268277 | Pepper\|TC5595 | 65 | 600 | 5.50E-40 | * |
| Rice\|TC268277 | Potato\|TC117743 | 65 | 691 | 3.80E-45 | * |
| S.officinarumlTC67974 | ArabidopsislTC272322 | 63 | 938 | 1.50E-59 | * |
| S.officinarum\|TC67974 | Barley\|TC134680 | 76 | 1294 | 2.60E-151 | * |
| S.officinarum\|TC67974 | Cotton\|TC30779 | 66 | 876 | 5.10E-70 | * |
| S.officinarum\|TC67974 | L.japonicus\|TC17767 | 69 | 656 | 4.70E-57 | * |
| S.officinarum\|TC67974 | Lettuce\|TC15801 | 66 | 390 | 4.70E-28 | * |
| S.officinarum\|TC67974 | Maize\|TC255367 | 96 | 367 | 2.80E-72 | |
| S.officinarum\|TC67974 | Maize\|TC272965 | 90 | 649 | 2.20E-112 | * |
| S.officinarum\|TC67974 | Medicago\|TC96175 | 64 | 1140 | 7.80E-75 | * |
| S.officinarum\|TC67974 | Pepper\|TC5595 | 66 | 471 | 1.80E-33 | * |
| S.officinarum\|TC67974 | Potato\|TC117743 | 67 | 460 | 1.00E-35 | * |
| S.officinarum\|TC67974 | Rice\|TC268277 | 82 | 1338 | 1.20E-194 | * |
| S.officinarum\|TC67974 | Sorghum\|TC103780 | 95 | 1359 | 8.70E-277 | * |
| S.officinarum\|TC67974 | Soybean\|TC229774 | 70 | 750 | 1.70E-67 | * |
| Sorghum\|TC103780 | Arabidopsis\|TC27232 | 263 | 1057 | 1.40E-65 | * |
| Sorghum\|TC103780 | Barley\|TC134680 | 75 | 1421 | 5.10E-163 | * |
| Sorghum\|TC103780 | Cofton\|TC30779 | 64 | 1333 | 8.60E-91 | * |
| Sorghum\|TC103780 | L.japonicus\|TC17767 | 69 | 656 | 6.40E-57 | * |
| Sorghum\|TC103780 | Lettuce\|TC15801 | 64 | 493 | 3.70E-30 | * |
| Sorghum\|TC103780 | Maize\|TC255367 | 92 | 948 | 1.70E-180 | * |
| Sorghum\|TC103780 | Maize\|TC272965 | 89 | 651 | 2.40E-110 | |
| Sorghum\|TC103780 | Medicago\|TC96175 | 62 | 1653 | 5.10E-96 | * |
| Sorghum\|TC103780 | Pepper\|TC5595 | 64 | 580 | 2.60E-36 | * |
| Sorghum\|TC103780 | Potato\|TC117743 | 65 | 602 | 6.50E-39 | * |
| Sorghum\|TC103780 | Rice\|TC268277 | 80 | 1852 | 1.40E-260 | * |

(continued)

| Sequence1 | Sequence2 | % Identity | Match length | p-value | Recip. best hits |
|---|---|---|---|---|---|
| Soybean\|TC229774 | Arabidopsis\|TC27232 | 272 | 757 | 1.10E-77 | * |
| Soybean\|TC229774 | Barley\|TC134680 | 69 | 756 | 5.70E-67 | * |
| Soybean\|TC229774 | Cotton\|TC30779 | 78 | 737 | 3.40E-96 | * |
| Soybean\|TC229774 | L.japonicus\|TC17767 | 90 | 495 | 4.10E-87 | * |
| Soybean\|TC229774 | Lettuce\|TC15801 | 77 | 249 | 1.90E-26 | * |
| Soybean\|TC229774 | Maize\|TC255367 | 71 | 203 | 2.00E-15 | * |
| Soybean\|TC229774 | Medicago\|TC96175 | 87 | 717 | 2.10E-118 | * |
| Soybean\|TC229774 | Pepper\|TC5595 | 76 | 308 | 1.50E-32 | * |
| Soybean\|TC229774 | Potato\|TC117743 | 76 | 332 | 1.80E-35 | * |
| Soybean\|TC229774 | Rice\|TC268277 | 71 | 744 | 4.20E-74 | * |
| Soybean\|TC229774 | Sorghum\|TC103780 | 69 | 757 | 1.30E-65 | * |
| Tomato\|TC158378 | Arabidopsis\|TC272322 | 71 | 686 | 3.10E-64 | * |
| Tomato\|TC158378 | Barley\|TC134680 | 65 | 907 | 8.80E-63 | * |
| Tomato\|TC158378 | Cotton\|TC30779 | 78 | 669 | 2.90E-86 | * |
| Tomato\|TC158378 | L.japonicus\|TC17767 | 78 | 427 | 1.70E-52 | * |
| Tomato\|TC158378 | Lettuce\|TC15801 | 81 | 181 | 3.90E-21 | |
| Tomato\|TC158378 | Maize\|TC272965 | 60 | 428 | 4.80E-14 | * |
| Tomato\|TC158378 | Medicago\|TC96175 | 68 | 908 | 4.60E-76 | * |
| Tomato\|TC158378 | Pepper\|TC5595 | 84 | 243 | 6.40E-34 | |
| Tomato\|TC158378 | Potato\|TC117743 | 96 | 264 | 8.90E-50 | * |
| Tomato\|TC158378 | Rice\|TC268277 | 66 | 897 | 1.70E-64 | * |
| Tomato\|TC158378 | S.officinarum\|TC67974 | 65 | 906 | 2.40E-60 | * |
| Tomato\|TC158378 | Soybean\|TC229774 | 74 | 700 | 4.80E-78 | * |
| Tomato\|TC166426 | Arabidopsis\|TC272322 | 70 | 675 | 1.80E-62 | |
| Tomato\|TC166426 | Barley\|TC134680 | 67 | 680 | 1.40E-54 | |
| Tomato\|TC166426 | Cotton\|TC30779 | 77 | 679 | 3.80E-85 | |
| Tomato\|TC166426 | L.japonicus\|TC17767 | 77 | 440 | 1.30E-50 | |
| Tomato\|TC166426 | Lettuce\|TC15801 | 81 | 193 | 6.50E-23 | * |
| Tomato\|TC166426 | Medicago\|TC96175 | 73 | 675 | 1.30E-69 | |
| Tomato\|TC166426 | Pepper\|TC5595 | 95 | 255 | 1.10E-46 | * |
| Tomato\|TC166426 | Potato\|TC117743 | 85 | 276 | 2.40E-39 | |
| Tomato\|TC166426 | Rice\|TC268277 | 67 | 680 | 2.70E-55 | |
| Tomato\|TC166426 | S.officinarum\|TC67974 | 67 | 680 | 4.90E-53 | |
| Tomato\|TC166426 | Sorghum\|TC103780 | 67 | 680 | 1.60E-53 | * |
| Tomato\|TC166426 | Soybean\|TC229774 | 73 | 680 | 2.30E-72 | |
| Wheat\|TC257477 | Barley\|TC134680 | 91 | 672 | 7.OOE-119 | * |
| Wheat\|TC257477 | Maize\|TC272965 | 69 | 639 | 2.80E-49 | * |

(continued)

| Sequence1 | Sequence2 | % Identity | Match length | p-value | Recip. best hits |
|---|---|---|---|---|---|
| Wheat\|TC257477 | Rice\|TC268277 | 68 | 647 | 4.10E-47 | * |
| Wheat\|TC257477 | S.officinarum\|TC67974 | 67 | 648 | 4.10E-46 | * |
| Wheat\|TC257477 | Sorghum\|TC103780 | 69 | 648 | 4.20E-49 | * |
| Wheat\|TC257477 | Tomato\|TC158378 | 61 | 441 | 1.20E-18 | * |

Orthologous and paralogous SIK polypeptides may readily be found, and nucleic acids encoding such orthologues and paralogues would be useful in performing the methods of the invention.

Orthologues and paralogues may easily be found by performing a so-called reciprocal blast search. Typically this involves a first BLAST involving BLASTing a query sequence (for example, SEQ ID NO: 209 or SEQ ID NO: 210) against any sequence database, such as the publicly available NCBI database which may be found at: http://www.ncbi.nlm.nih.gov. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 209 or SEQ ID NO: 210, the second BLAST would therefore be against *Oryza* sequences). The results of the first and second BLASTs are then compared. A paralogue is identified if a high-ranking hit from the first blast is from the same species as from which the query sequence is derived, a BLAST back then ideally results in the query sequence being among the highest hits; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. Preferably the score is greater than 50, more preferably greater than 100; and preferably the E-value is less than e-5, more preferably less than e-6. In the case of large families, ClustalW may be used, followed by a neighbour joining tree, to help visualize clustering of related genes and to identify orthologues and paralogues.

Orthologues and paralogues may also be identified using the BLAST procedure described below. Homologues (or homologous proteins, encompassing orthologues and paralogues) may readily be identified using routine techniques well known in the art, such as by sequence alignment. Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the alignment of two complete sequences that maximizes the number of matches and minimizes the number of gaps. The BLAST algorithm (Altschul et al. (1990) J Mol Biol 215: 403-410) calculates percent sequence identity and performs a statistical analysis of the similarity between the two sequences. The software for performing BLAST analysis is publicly available through the National Centre for Biotechnology Information. Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 4, 29, 2003). Minor manual editing may be performed to optimise alignment between conserved motifs, as would be apparent to a person skilled in the art. Furthermore, instead of using full-length sequences for the identification of homologues, specific domains may be used as well. The sequence identity values, which are indicated below as a percentage were determined over the entire SIK nucleic acid or amino acid sequence using the programs mentioned above using the default parameters.

Preferably, the SIK polypeptides encoded by the SIK-encoding nucleic acids useful in the methods of the present invention have, in increasing order of preference, at least 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% identity to the polypeptide of SEQ ID NO: 210.

Alternatively, the sequence identity among homologues may be determined using a specific domain. Any given domain may be identified and delineated using the databases and tools for protein identification listed above, and/or methods for the alignment of sequences for comparison. In some instances, default parameters may be adjusted to modify the stringency of the search. For example using BLAST, the statistical significance threshold (called "expect" value) for reporting matches against database sequences may be increased to show less stringent matches. In this way, short nearly exact matches may be identified.

The SIK polypeptides may be identifiable by the presence of an ATP-binding region, a serine threonine kinase active site signature, an N-terminal serine-rich domain and one or more myristoylation sites. The terms "domain" and "motif" are defined above. Specialist databases exist for the identification of domains, such as SMART (Schultz et al. (1998) Proc. Natl. Acad. Sci. USA 95, 5857-5864; Letunic et al. (2002) Nucleic Acids Res 30, 242-244), InterPro (Mulder et al., (2003) Nucl. Acids. Res. 31, 315-318), Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp. 53-61, AAAI Press, Menlo Park; Hulo et al., Nucl. Acids. Res. 32:D134-D137, (2004)) or Pfam (Bateman et al., Nucleic Acids Research 30(1): 276-280 (2002)). A set of tools for *in silico* analysis of protein sequences is available on the ExPASY proteomics server (hosted by the Swiss Institute of Bioinformatics (Gasteiger et al., ExPASy: the proteomics server for in-depth protein knowledge and analysis, Nucleic Acids Res. 31:3784-3788(2003)). However, the various domains may also easily be identified upon sequence alignment of a putative SIK polypeptide with SIK polypeptides known in the art.

Also useful in the methods of the invention are nucleic acids encoding homologues of a SIK polypeptide represented by SEQ ID NO: 210 or orthologues or paralogues thereof as defined above.

Also useful in the methods of the invention are nucleic acids encoding derivatives of SEQ ID NO: 210 or nucleic acids encoding derivatives of orthologues, paralogues or homologues of SEQ ID NO: 210.

Nucleic acids encoding SIK polypeptides defined herein need not be full-length nucleic acids, since performance of the methods of the invention does not rely on the use of full length nucleic acid sequences. Examples of nucleic acids suitable for use in performing the methods of the invention include the nucleic acid sequences given in Table 8 and Table 9, but are not limited to those sequences. Nucleic acid variants may also be useful in practising the methods of the invention. Examples of such nucleic acid variants include portions of nucleic acids encoding a SIK polypeptide as defined herein, splice variants of nucleic acids encoding a SIK polypeptide as defined herein, allelic variants of nucleic acids encoding a SIK polypeptide as defined herein and variants of nucleic acids encoding a SIK polypeptide as defined herein that are obtained by gene shuffling. The terms portion, splice variant, allelic variant and gene shuffling are as described herein.

According to the present invention, there is provided a method for increasing the number of flowers per plant relative to control plants, comprising introducing and expressing in a plant a portion of any one of the nucleic acid sequences given in Table 8 or Table 9, or a portion of a nucleic acid encoding an orthologue, paralogue or homologue of any of the amino acid sequences given in Table 8 or Table 9.

Portions useful in the methods of the invention, encode a polypeptide having substantially the same biological activity as the SIK polypeptide represented by any of the amino acid sequences given in Table 8 or Table 9. Preferably, the portion is a portion of any one of the nucleic acids given in Table 8 or Table 9, or a portion of any one of the nucleic acids sequences represented by SEQ ID NO: 213 to 225. The portion is typically at least 300 consecutive nucleotides in length, preferably at least 400 consecutive nucleotides in length, more preferably at least 500 consecutive nucleotides in length, the consecutive nucleotides being of any one of the nucleic acid sequences given in Table 8 or Table 9 or any one of the nucleic acids sequences represented by SEQ ID NO: 213 to 225. Most preferably the portion is a portion of the nucleic acid of SEQ ID NO: 209. Preferably, the portion encodes an amino acid sequence comprising any one or more of an ATP-binding region, a serine threonine kinase active site signature, an N-terminal serine-rich domain, one or more myristoylation sites and kinase activity.

A portion of a nucleic acid encoding a SIK polypeptide as defined herein may be prepared, for example, by making one or more deletions to the nucleic acid. The portions may be used in isolated form or they may be fused to other coding (or non coding) sequences in order to, for example, produce a protein that combines several activities. When fused to other coding sequences, the resultant polypeptide produced upon translation may be bigger than that predicted for the portion.

Another nucleic acid variant useful in the methods of the invention is a nucleic acid capable of hybridising, under reduced stringency conditions, preferably under stringent conditions, with a nucleic acid encoding a SIK polypeptide as defined herein, or with a portion as defined herein.

Hybridising sequences useful in the methods of the invention, encode a polypeptide having substantially the same biological activity as the SIK polypeptide represented by any of the amino acid sequences given in Table 8 or Table 9. The hybridising sequence is typically at least 300 consecutive nucleotides in length, preferably at least 400 consecutive nucleotides in length, more preferably at least 500 consecutive nucleotides in length, the consecutive nucleotides being of any one of the nucleic acid sequences given in Table 8 or Table 9. Preferably, the hybridising sequence is one that is capable of hybridising to any of the nucleic acids given in Table 8 or Table 9, or to a portion of any of these sequences, a portion being as defined above. Most preferably, the hybridising sequence is capable of hybridising to a nucleic acid as represented by SEQ ID NO: 209 or to a portion thereof. Preferably, the hybridising sequence encodes an amino acid sequence comprising any one or more of an ATP-binding region, a serine threonine kinase active site signature, an N-terminal serine-rich domain, one or more myristoylation sites and kinase activity.

According to the present invention, there is provided a method for increasing the number of flowers per plant, comprising introducing and expressing in a plant a nucleic acid capable of hybridizing to any one of the nucleic acids given in Table 8 or Table 9 or any one of the nucleic acids sequences represented by SEQ ID NO: 213 to 225, or comprising introducing and expressing in a plant a nucleic acid capable of hybridising to a nucleic acid encoding an orthologue, paralogue or homologue of any of the nucleic acid sequences given in Table 1 or Table 2 or any one of the nucleic acids sequences represented by SEQ ID NO: 213 to 225.

Another nucleic acid variant useful in the methods of the invention is a splice variant encoding a SIK polypeptide as defined hereinabove, the term "splice variant" being as defined above.

According to the present invention, there is provided a method for increasing the number of flowers per plant, comprising introducing and expressing in a plant a splice variant of any one of the nucleic acid sequences given in Table 8 or Table 9 or a splice variant of any one of the nucleic acids represented by SEQ ID NO: 213 to 225, or a splice variant of a nucleic acid encoding an orthologue, paralogue or homologue of any of the amino acid sequences given in Table 8 or Table 9 or any one of the nucleic acids sequences represented by SEQ ID NO: 213 to 225.

Preferred splice variants are splice variants of a nucleic acid represented by SEQ ID NO: 209 or a splice variant encoding an orthologue or paralogue of SEQ ID NO: 210. Preferably, the amino acid encoded by the splice variant comprises any one or more of an ATP-binding region, a serine threonine kinase active site signature, an N-terminal serine-rich domain, one or more myristoylation sites and kinase activity.

Another nucleic acid variant useful in performing the methods of the invention is an allelic variant of a nucleic acid encoding a SIK polypeptide as defined hereinabove.

According to the present invention, there is provided a method for increasing the number of flowers per plant, comprising introducing and expressing in a plant an allelic variant of any one of the nucleic acids given in Table 8 or Table 9 or of any one of the nucleic acid sequences represented by SEQ ID NO: 213 to 225, or comprising introducing and expressing in a plant an allelic variant of a nucleic acid encoding an orthologue, paralogue or homologue of any of the amino acid sequences given in Table 8 or Table 9 or of any one of the nucleic acids sequences represented by SEQ ID NO: 213 to 225.

Preferably, the allelic variant is an allelic variant of SEQ ID NO: 209 or an allelic variant of a nucleic acid encoding an orthologue or paralogue or homologue of SEQ ID NO: 210. Preferably, the amino acid encoded by the allelic variant comprises any one or more of an ATP-binding region, a serine threonine kinase active site signature, an N-terminal serine-rich domain, one or more myristoylation sites and kinase activity.

A further nucleic acid variant useful in the methods of the invention is a nucleic acid variant obtained by gene shuffling. Gene shuffling or directed evolution may also be used to generate variants of nucleic acids encoding SIK polypeptides.

According to the present invention, there is provided a method for increasing the number of flowers per plant, comprising introducing and expressing in a plant a variant of any one of the nucleic acid sequences given in Table Table 8 or Table 9, or of any one of the nucleic acids sequences represented by SEQ ID NO: 213 to 225, or comprising introducing and expressing in a plant a variant of a nucleic acid encoding an orthologue, paralogue or homologue of any of the amino acid sequences given in Table 8 or Table 9 or of any one of the nucleic acids sequences represented by SEQ ID NO: 213 to 225, which variant nucleic acid is obtained by gene shuffling. Preferably, the variant nucleic acid obtained by gene shuffling encodes an amino acid comprising any one or more of an ATP-binding region, a serine threonine kinase active site signature, an N-terminal serine-rich domain, one or more myristoylation sites and kinase activity.

Furthermore, nucleic acid variants may also be obtained by site-directed mutagenesis. Several methods are available to achieve site-directed mutagenesis, the most common being PCR based methods (current protocols in molecular biology. Wiley Eds.

SIK nucleic acids encoding SIK-like polypeptides may be derived from any natural or artificial source. The SIK nucleic acid may be modified from its native form in composition and/or genomic environment through deliberate human manipulation. Preferably the SIK-encoding nucleic acid is from a plant, further preferably from a monocotyledonous plant, more preferably from the family of Poaceae, most preferably the nucleic acid is from *Oryza sativa.*

Any reference herein to a SIK polypeptide is therefore taken to mean a SIK polypeptide as defined above. Any SIK nucleic acid encoding such a SIK polypeptide is suitable for use in performing the methods of the invention to increase the number of flowers per plant.

The present invention also encompasses plants or parts thereof (including seeds) obtainable by the methods according to the present invention. The plants or parts thereof comprise a nucleic acid transgene encoding a SIK polypeptide as defined above.

The invention also provides genetic constructs and vectors to facilitate introduction and/or expression of the SIK nucleic acid sequences useful in the methods according to the invention, in a plant.

Therefore, there is provided a gene construct comprising:

(i) a SIK nucleic acid as defined hereinabove or a SIK-encoding nucleic acid;
(ii) one or more control sequences operably linked to the SIK nucleic acid of (i).

A preferred construct in the case of reduction or substantial elimination of a SIK nucleic acid to give increased thousand kernel weight, increased harvest index and increased fill rate is one comprising an inverted repeat of a SIK nucleic acid, preferably capable of forming a hairpin structure, which inverted repeat is under the control of a constitutive promoter.

Constructs useful in the methods according to the present invention may be constructed using recombinant DNA technology well known to persons skilled in the art. The gene constructs may be inserted into vectors, which may be commercially available, suitable for transforming into plants and suitable for transcribing of the gene of interest in the transformed cells. The invention therefore provides use of a gene construct as defined hereinabove in the methods of the invention.

Plants are transformed with a vector comprising the sequence of interest. The skilled artisan is well aware of the genetic elements that must be present on the vector in order to successfully transform, select and propagate host cells containing the sequence of interest. The sequence of interest is operably linked to one or more control sequences (at least to a promoter). The terms "regulatory element", "control sequence" and "promoter" are all used interchangeably herein and are defined above.

Advantageously, any type of promoter may be used in the methods o the present invention. The term "promoter" refers to a nucleic acid control sequence located upstream from the transcriptional start of a gene and which is involved in recognising and binding of RNA polymerase and other proteins, thereby directing transcription of an operably linked nucleic acid. The promoter may be a constitutive promoter. Alternatively, the promoter may be an inducible promoter. Additionally or alternatively, the promoter may be a tissue-specific promoter. Promoters able to initiate transcription in certain tissues only are referred to herein as "tissue-specific", similarly, promoters able to initiate transcription in certain cells only are referred to herein as "cell-specific".

Preferably, the nucleic acid sequence is operably linked to a constitutive promoter. Preferably the promoter is derived from a plant, more preferably the promoter is from a monocotyledonous plant if a monocotyledonous plant is to be transformed. Further preferably, the constitutive promoter is a GOS2 promoter that is represented by a nucleic acid sequence substantially similar to SEQ ID NO: 56 or 226. Most preferably the GOS2 promoter is as represented by SEQ ID NO: 56 or 226. It should be clear that the applicability of the present invention is not restricted to the SIK nucleic acid sequence as represented by SEQ ID NO: 209, nor is the applicability of the invention restricted to expression of a SIK nucleic acid sequence when driven by a GOS2 promoter. Examples of other constitutive promoters that may also be used to drive expression of a SIK nucleic acid sequence are shown above.

Optionally, one or more terminator sequences may be used in the construct introduced into a plant. Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

The expression of a SIK nucleic acid or SIK polypeptide may also be modulated by introducing a genetic modification, within the locus of a SIK gene. The locus of a gene as defined herein is taken to mean a genomic region, which includes the gene of interest and 10 kb up- or down stream of the coding region.

The genetic modification may be introduced, for example, by any one (or more) of the following methods: T-DNA tagging, TILLING and homologous recombination. Following introduction of the genetic modification, there follows a step of selecting for suitable modulated expression.

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum and oats.

Other advantageous plants are selected from the group consisting of Asteraceae such as the genera Helianthus, Tagetes e.g. the species Helianthus annus [sunflower], Tagetes lucida, Tagetes erecta or Tagetes tenuifolia [Marigold], Brassicaceae such as the genera Brassica, Arabadopsis e.g. the species Brassica napus, Brassica rapa ssp. [canola, oilseed rape, turnip rape] or Arabidopsis thaliana. Fabaceae such as the genera Glycine e.g. the species Glycine max, Soja hispida or Soja max [soybean]. Linaceae such as the genera Linum e.g. the species Linum usitatissimum, [flax, linseed]; Poaceae such as the genera Hordeum, Secale, Avena, Sorghum, Oryza, Zea, Triticum e.g. the species Hordeum vulgare [barley]; Secale cereale [rye], Avena sativa, Avena fatua, Avena byzantina, Avena fatua var. sativa, Avena hybrida [oat], Sorghum bicolor [Sorghum, millet], Oryza sativa, Oryza latifolia [rice], Zea mays [corn, maize] Triticum aestivum, Triticum durum, Triticum turgidum, Triticum hybernum, Triticum macha, Triticum sativum or Triticum vulgare [wheat, bread wheat, common wheat]; Solanaceae such as the genera Solanum, Lycopersicon e.g. the species Solanum tuberosum [potato], Lycopersicon esculentum, Lycopersicon lycopersicum., Lycopersicon pyriforme, Solanum integrifolium or Solanum lycopersicum [tomato].

The present invention also encompasses plants obtainable by the methods according to the present invention. The present invention therefore provides plants, plant parts or plant cells thereof obtainable by the method according to the present invention, which plants or parts or cells thereof comprise a SIK nucleic acid transgene (which may encode a SIK polypeptide as defined above).

The invention furthermore provides a method for the production of transgenic plants having the various improved yield-related traits mentioned above, comprising introduction and expression in a plant SIK nucleic acid as defined.

Host plants for the nucleic acids or the vector used in the method according to the invention, the expression cassette or construct or vector are, in principle, advantageously all plants, which are capable of synthesizing the polypeptides used in the inventive method.

More specifically, the present invention provides a method for the production of transgenic plants having various improved yield-related traits relative to control plants, which method comprises:

(i) introducing and expressing a SIK nucleic acid in a plant cell; and
(ii) cultivating the plant cell under conditions promoting plant growth and development;
(iii) obtaining plants having increased number of flowers per plant.

Also provided is a method for the production of transgenic plants having various improved yield-related traits relative to control plants, which method comprises:

(i) introducing into a plant cell a construct for downregulating SIK gene expression; and
(ii) cultivating the plant cell under conditions promoting plant growth and development;
(iii) obtaining plants having one or more of increased thousand kernel weight, incraesd harvest index and increased fill rate.

The nucleic acid may be introduced directly into a plant cell or into the plant itself (including introduction into a tissue, organ or any other part of a plant). According to a preferred feature of the present invention, the nucleic acid is preferably introduced into a plant by transformation.

Further preferably the construct for downregulating SIK gene expression and introduced into the plant cell or plant comprise an inverted repeat of the SIK nucleic acid or a part thereof.

Generally after transformation, plant cells or cell groupings are selected for the presence of one or more markers which are encoded by plant-expressible genes co-transferred with the gene of interest, following which the transformed material is regenerated into a whole plant.

As mentioned Agrobacteria transformed with an expression vector according to the invention may also be used in the manner known per se for the transformation of plants such as experimental plants like Arabidopsis or crop plants, such as, for example, cereals, maize, oats, rye, barley, wheat, soya, rice, cotton, sugarbeet, canola, sunflower, flax, hemp, potato, tobacco, tomato, carrot, bell peppers, oilseed rape, tapioca, cassava, arrow root, tagetes, alfalfa, lettuce and the various tree, nut, and grapevine species, in particular oil-containing crop plants such as soya, peanut, castor-oil plant, sunflower, maize, cotton, flax, oilseed rape, coconut, oil palm, safflower (Carthamus tinctorius) or cocoa beans, for example by bathing scarred leaves or leaf segments in an agrobacterial solution and subsequently growing them in suitable media.

The genetically modified plant cells can be regenerated via all methods with which the skilled worker is familiar. Suitable methods can be found in the abovementioned publications by S.D. Kung and R. Wu, Potrykus or Höfgen and Willmitzer.

To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may be evaluated, for instance using Southern analysis, for the presence of the gene of interest, copy number and/or genomic organisation. Alternatively or additionally, expression levels may be monitored using Northern and/or Western analysis, or quantitative PCR, all techniques being well known to persons having ordinary skill in the art.

The generated transformed plants may be propagated by a variety of means, such as by clonal propagation or classical breeding techniques. For example, a first generation (or T1) transformed plant may be selfed to give homozygous second generation (or T2) transformants, and the T2 plants further propagated through classical breeding techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic (s) as those produced by the parent in the methods according to the invention.

The invention also includes host cells containing an isolated SIK nucleic acid as defined hereinabove. Preferred host cells according to the invention are plant cells. Host plants for the nucleic acids or the vector used in the method according to the invention, the expression cassette or construct or vector are, in principle, advantageously all plants, which are capable of synthesizing the polypeptides used in the inventive method.

The invention also extends to harvestable parts of a plant such as, but not limited to seeds, leaves, fruits, flowers, stems, rhizomes, tubers and bulbs. The invention furthermore relates to products derived, preferably directly derived, from a harvestable part of such a plant, such as dry pellets or powders, oil, fat and fatty acids, starch or proteins.

The present invention also encompasses use of SIK nucleic acids and SIK polypeptides in improving various yield-related traits as mentioned above.

Nucleic acids encoding SIK polypeptides may find use in breeding programmes in which a DNA marker is identified which may be genetically linked to a SIK gene. The nucleic acids/genes may be used to define a molecular marker. This DNA marker may then be used in breeding programmes to select plants having increased yield as defined hereinabove in the methods of the invention.

Allelic variants of a SIK nucleic acid/gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

A SIK nucleic acid may also be used as probes for genetically and physically mapping the genes that they are a part of, and as markers for traits linked to those genes. Such information may be useful in plant breeding in order to develop lines with desired phenotypes. Such use of SIK nucleic acids requires only a nucleic acid sequence of at least 15 nucleotides in length. The SIK nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the SIK nucleic acids. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the SIK nucleic acid in the genetic map previously obtained using this population (Botstein et al. (1980) Am. J. Hum. Genet. 32:314-331).

The production and use of plant gene-derived probes for use in genetic mapping is described in Bernatzky and Tanksley (Plant Mol. Biol. Reporter 4: 37-41, 1986). Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence in situ hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having altered yield-related traits, as described hereinbefore. These traits may also be combined with other economically advantageous traits, such as further yield-enhancing traits, tolerance to other abiotic and biotic stresses, traits modifying various architectural features and/or biochemical and/or physiological features.

**Detailed description for the Class II HD-Zip transcription factors**

It has now been found that nucleic acids encoding Class I HD-Zip transcription factors are useful in modifying the content of storage compounds in seeds. The present invention therefore provides a method for modifying the content of storage compounds in seeds relative to control plants by modulating expression in a plant of a nucleic acid encoding a Class II HD-Zip transcription factor. The present invention also provides nucleic acid sequences and constructs useful in performing such methods. The invention further provides seeds having a modified content of storage compounds relative to control plants, which seeds have modulated expression of a nucleic acid encoding a Class II HD-Zip transcription factor.

The present invention provides a method for modifying the content of storage compounds in seeds relative to control plants, comprising modulating expression in a plant of a nucleic acid encoding a Class II HD-Zip transcription factor.

A preferred method for modulating (preferably, increasing) expression of a nucleic acid encoding a Class II HD-Zip transcription factor is by introducing and expressing in a plant a nucleic acid encoding a Class II HD-Zip transcription factor as will now be defined.

A "Class II HD-Zip transcription factor" is taken to mean a polypeptide comprising the following: (i) a homeodomain box; (ii) a leucine zipper; and (iii) Motifs I, II and III given below (in any order).

**Motif I (SEQ ID NO: 279)**

RKKLRL, or Motif I with one or more conservative amino acid substitution at any position, and/or Motif I with one or two non-conservative change(s) at any position; and

**Motif II (SEQ ID NO: 280)**

TKLKQTEVDCEFLRRCCENLTEEN, or Motif II with one or more conservative amino acid substitution at any position, and/or a motif having in increasing order of preference at least 50%, 55%, 60%, 65%, 70%, 75%, 80%, 85%, 90%, 95% or more sequence identity to Motif II; and

**Motif III (SEQ ID NO: 281)**

TLTMCPSCER, or Motif III with one or more conservative amino acid substitution at any position, and/or Motif III with one, two or three non-conservative change(s) at any position.

Any reference herein to a "nucleic acid encoding a Class II HD-Zip transcription factor" or to a Class II HD-Zip-encoding nucleic acid" is taken to mean a nucleic acid encoding a Class II HD-Zip transcription factor as defined hereinabove, such nucleic acids being useful in performing the methods of the invention.

As mentioned, a preferred method for modulating (preferably, increasing) expression of a nucleic acid encoding a Class II HD-Zip transcription factor is by introducing and expressing in a plant a nucleic acid encoding a Class II HD-Zip transcription factor.

The present invention is illustrated by transforming plants with the nucleic acid sequence represented by SEQ ID NO: 229, encoding the polypeptide sequence of SEQ ID NO: 230.

However, performance of the invention is not restricted to these sequences; the methods of the invention may advantageously be performed using any Class II HD-Zip transcription factor-encoding nucleic acid or Class II HD-Zip transcription factor as defined herein.

For example, nucleic acids encoding orthologues or paralogues of an amino acid sequence represented by SEQ ID NO: 230 may be useful in performing the methods of the invention. Examples of such orthologues and paralogues are provided in Table A of Example 1.

Orthologues and paralogues encompass evolutionary concepts used to describe the ancestral relationships of genes. Paralogues are genes within the same species that have originated through duplication of an ancestral gene and orthologues are genes from different organisms that have originated through speciation.

Orthologues and paralogues may easily be found by performing a so-called reciprocal blast search. Typically this involves a first BLAST involving BLASTing a query sequence (for example, SEQ ID NO: 229 or SEQ ID NO: 230) against any sequence database, such as the publicly available NCBI database which may be found at: http://www.ncbi.nlm.nih.gov. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 229 or SEQ ID NO: 230, the second BLAST would therefore be against *Oryza* sequences). The results of the first and second BLASTs are then compared. A paralogue is identified if a high-ranking hit from the first blast is from the same species as from which the query sequence is derived, a BLAST back then ideally results in the query sequence being among the highest hits; an orthologue is identified if a high-ranking hit in the first BLAST is not from the same species as from which the query sequence is derived, and preferably results upon BLAST back in the query sequence being among the highest hits. High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

Nucleic acids encoding homologues of an amino acid sequence represented by SEQ ID NO: 230, or nucleic acids encoding homologues of any of the amino acid sequences given in Table H, may also be useful in performing the methods of the invention.

Also useful in the methods of the invention are nucleic acids encoding derivatives of any one of the amino acids given in Table H or derivatives of orthologues or paralogues of any of the amino acid sequences given in Table H. "Derivatives" include peptides, oligopeptides, polypeptides which may, compared to the amino acid sequence of the naturally-occurring form of the protein, such as the one presented in SEQ ID NO: 230, comprise substitutions of amino acids with non-naturally occurring amino acid residues, or additions of non-naturally occurring amino acid residues.

Homologues (or homologous proteins, encompassing orthologues and paralogues) may readily be identified using routine techniques well known in the art, such as by sequence alignment. Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the alignment of two complete sequences that maximizes the number of matches and minimizes the number of gaps. The BLAST algorithm (Altschul et al. (1990) J Mol Biol 215: 403-410) calculates percent sequence identity and performs a statistical analysis of the similarity between the two sequences. The software for performing BLAST analysis is publicly available through the National Centre for Biotechnology Information. Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 4, 29, 2003). Minor manual editing may be performed to optimise alignment between conserved motifs, as would be apparent to a person skilled in the art. Furthermore, instead of using full-length sequences for the identification of homologues, specific domains may be used as well.

The sequence identity values, which are indicated below as a percentage were determined over the entire nucleic acid or amino acid sequence using ClustalW and default parameters.

Preferably, the polypeptides encoded by the nucleic acids useful in the methods of the present invention (such as any of the polypeptide sequences given in Table H) have, in increasing order of preference, at least 40%, 41%, 42%, 43%, 44%, 45%, 46%, 47%, 48%, 49%, 50%, 51%, 52%, 53%, 54%, 55%, 56%, 57%, 58%, 59%, 60%, 61%, 62%, 63%, 64%, 65%, 66%, 67%, 68%, 69%, 70%, 71%, 72%, 73%, 74%, 75%, 76%, 77%, 78%, 79%, 80%, 81%, 82%, 83%, 84%, 85%, 86%, 87%, 88%, 89%, 90%, 91%, 92%, 93%, 94%, 95%, 96%, 97%, 98%, 99% identity to the polypeptide of SEQ ID NO: 230.

According to the present invention, there is provided a method for modifying the content of storage compounds in seeds relative to control plants, comprising modulating expression in a plant of a nucleic acid encoding a Class II HD-Zip transcription factor represented by SEQ ID NO: 2 or an orthologue, paralogue or homologue thereof.

A preferred method for modulating (preferably, increasing) expression of a nucleic acid encoding a Class II HD-Zip transcription factor is by introducing and expressing in a plant a nucleic acid encoding a Class II HD-Zip transcription factor represented by SEQ ID NO: 230 or an orthologue, paralogue or homologue thereof.

The orthologues, paralogues and homologues described above fall under the definition of a Class II HD-Zip transcription factor, i.e. meaning that the orthologues, paralogues and homologues are polypeptides comprising the following: (i) a homeodomain box; (ii) a leucine zipper; and (iii) Motifs I, II and III described herein.

The various domains and motifs may be used to help identify sequences useful in the methods of the invention. Specialist databases also exist for the identification of domains, for example, SMART (Schultz et al. (1998) Proc. Natl. Acad. Sci. USA 95, 5857-5864; Letunic et al. (2002) Nucleic Acids Res 30, 242-244, InterPro (Mulder et al., (2003) Nucl. Acids. Res. 31, 315-318, Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAIPress, Menlo Park; Hulo et al., Nucl. Acids. Res. 32:D134-D137, (2004), or Pfam (Bateman et al., Nucleic Acids Research 30(1): 276-280 (2002). Domains and motifs may also be identified using routine techniques, such as by sequence alignment as described herein.

Nucleic acids useful in the methods of the invention need not be full-length, since performance of the methods of the invention does not rely on the use of full length nucleic acids. Examples include portions of the nucleic acid sequence represented by SEQ ID NO: 229 or portions of any one of the nucleic acid sequences given in Table H.

According to the present invention, there is provided a method for modifying the content of storage compounds in seeds relative to control plants, comprising modulating expression in a plant of a portion of a nucleic acid sequence represented by SEQ ID NO: 229, or comprising modulating expression in a plant of a portion of any one of the nucleic acid sequences given in Table H, or comprising modulating expression in a plant of a portion of a nucleic acid sequence encoding an orthologue, paralogue or homologue of any of the amino acid sequences given in Table H.

A preferred method for modulating (preferably, increasing) expression in a plant of such a portion is by introducing and expressing in a plant a portion of a nucleic acid sequence given in Table H, or comprising introducing and expressing in a plant a portion of a nucleic acid sequence encoding an orthologue, paralogue or homologue of any of the polypeptide sequences given in Table H.

Portions useful in the methods of the invention, include portions of sufficient length to encode a polypeptide falling under the definition of a Class II HD-Zip transcription factor, i.e. meaning that the polypeptide comprises the following: (i) a homeodomain box; (ii) a leucine zipper; and (iii) Motifs I, II and III described herein. Furthermore, such portions have substantially the same biological activity as Class II HD-Zip transcription factors.

Preferably, the portion is at least 500 consecutive nucleotides in length, preferably at least 750 consecutive nucleotides in length, more preferably at least 1,250 consecutive nucleotides in length, the consecutive nucleotides being of SEQ ID NO: 229, or of any one of the nucleic acid sequences given in Table H, or of any nucleic acid encoding an orthologue, paralogue or homologue of any one of the amino acid sequences given in Table H. Most preferably the portion is a portion of the nucleic acid of SEQ ID NO: 229.

A portion as defined herein may be prepared, for example, by making one or more deletions to the nucleic acid in question. The portions may be used in isolated form or they may be fused to other coding (or non coding) sequences in order to, for example, produce a protein that combines several activities. When fused to other coding sequences, the resultant polypeptide produced upon translation may be bigger than that predicted for the portion.

Another nucleic acid useful in the methods of the invention is a nucleic acid capable of hybridising, under reduced stringency conditions, preferably under medium stringency conditions, more preferably under stringent conditions, with a nucleic acid represented by SEQ ID NO: 229, or capable of hybridising, under reduced stringency conditions, preferably under medium stringency conditions, more preferably under stringent conditions, with a nucleic acid sequence given in Table H, or capable of hybridising under reduced stringency conditions, preferably under medium stringency conditions, more preferably under stringent conditions with a nucleic acid encoding an orthologue, paralogue or homologue of a polypeptide sequence given in Table H.

According to the present invention, there is provided a method for modifying the content of storage compounds in seeds relative to control plants, comprising modulating expression in a plant of a nucleic acid capable of hybridising to a nucleic acid represented by SEQ ID NO: 229, or comprising modulating expression in a plant of a nucleic acid capable of hybridising to a nucleic acid sequence given in Table H, or comprising modulating expression in a plant of a nucleic acid capable of hybridising to a nucleic acid sequence encoding an orthologue, paralogue or homologue of any of the polypeptide sequences given in Table H. Most preferably the hybridising sequence is capable of hybridising to a nucleic acid as represented by SEQ ID NO: 229. The hybridising sequence is preferably capable of hybridising under reduced stringency conditions, preferably under medium stringency conditions, more preferably under stringent conditions.

A preferred method for modulating (preferably, increasing) expression in a plant of such a hybridising sequence is by introducing and expressing in a plant a nucleic acid capable of hybridising to a nucleic acid sequence given in Table H, or comprising introducing and expressing in a plant of a nucleic acid sequence capable of hybridising to a nucleic acid sequence encoding an orthologue, paralogue or homologue of any one of the amino acid sequences given in Table H. Most preferably the hybridising sequence is capable of hybridising to a nucleic acid as represented by SEQ ID NO: 229. The hybridising sequence is preferably capable of hybridising under reduced stringency conditions, preferably under medium stringency conditions, more preferably under stringent conditions.

Hybridising sequences useful in the methods of the invention, include nucleic acids of sufficient length to encode a polypeptide falling under the definition of a Class II HD-Zip transcription factor, i.e. meaning that the polypeptide comprises the following: (i) a homeodomain box; (ii) a leucine zipper; and (iii) Motifs I, II and III described herein. Furthermore, such hybridising sequences have substantially the same biological activity as the Class II HD-Zip transcription factors.

The hybridising sequence is typically at least 500 consecutive nucleotides in length, preferably at least 750 consecutive nucleotides in length, more preferably at least 1,250 consecutive nucleotides in length, the consecutive nucleotides being of any nucleic acid capable of hybridising to a nucleic acid sequence given in Table H, or of any nucleic acid encoding an orthologue, paralogue or homologue of any one of the amino acid sequences given in Table H. Most preferably the consecutive nucleotides are of a nucleic acid capable of hybridising to a nucleic acid represented by SEQ ID NO: 229 or to a portion thereof.

Another nucleic acid useful in the methods of the invention is a splice variant of SEQ ID NO: 229, or a splice variant of any of the nucleic acid sequences given in Table H, or a splice variant of a nucleic acid encoding an orthologue, paralogue or homologue of any one of the amino acid sequences given in Table H.

According to the present invention, there is provided a method for modifying the content of storage compounds in seeds relative to control plants, comprising modulating expression in a plant of a splice variant of a nucleic acid represented by SEQ ID NO: 229, or comprising modulating expression in a plant of a splice variant of nucleic acid sequence given in Table H, or comprising modulating expression in a plant of a splice variant of a nucleic acid sequence encoding an orthologue, paralogue or homologue of any one of the amino acid sequences given in Table H.

A preferred method for modulating (preferably, increasing) expression in a plant of such a splice variant is by introducing and expressing in a plant a splice variant of a nucleic acid sequence given in Table H, or comprising introducing and expressing in a plant a splice variant of a nucleic acid sequence encoding an orthologue, paralogue or homologue of any one of the amino acid sequences given in Table H.

Splice variants useful in the methods of the invention, include nucleic acids of sufficient length to encode a polypeptide falling under the definition of a Class II HD-Zip transcription factor, i.e. meaning that the polypeptide comprises the following: (i) a homeodomain box; (ii) a leucine zipper; and (iii) Motifs I, II and III described herein. Furthermore, such splice variants have substantially the same biological activity as the Class II HD-Zip transcription factors.

Another nucleic acid useful in performing the methods of the invention is an allelic variant of SEQ ID NO: 229, or an allelic variant of any of the nucleic acid sequences given in Table H, or an allelic variant of a nucleic acid encoding an orthologue, paralogue or homologue of any one of the amino acid sequences given in Table H. Allelic variants exist in nature, and encompassed within the methods of the present invention is the use of these natural alleles. According to the present invention, there is provided a method for modifying the content of storage compounds in seeds relative to control plants, comprising modulating expression in a plant of an allelic variant of a nucleic acid represented by SEQ ID NO: 229, or comprising modulating expression in a plant of an allelic variant of nucleic acid sequence given in Table H, or comprising modulating expression in a plant of an allelic variant of a nucleic acid sequence encoding an orthologue, paralogue or homologue of any one of the amino acid sequences given in Table H.

A preferred method for modulating (preferably, increasing) expression in a plant of such an allelic variant is by introducing and expressing in a plant an allelic variant of a nucleic acid sequence given in Table H, or comprising introducing and expressing in a plant an allelic variant of a nucleic acid sequence encoding an orthologue, paralogue or homologue of any one of the amino acid sequences given in Table H.

Allelic variants useful in the methods of the invention, include nucleic acids of sufficient length to encode a polypeptide falling under the definition of a Class II HD-Zip transcription factor, i.e. meaning that the polypeptide comprises the following: (i) a homeodomain box; (ii) a leucine zipper; and (iii) Motifs I, II and III described herein. Furthermore, such allelic variants have substantially the same biological activity as the Class II HD-Zip transcription factors.

A further nucleic acid useful in the methods of the invention is a nucleic acid obtained by gene shuffling. Gene shuffling or directed evolution may also be used to generate variants of any one of the nucleic acids given in Table H, or variants of nucleic acids encoding orthologues, paralogues or homologues of any one of the amino acid sequences given in Table H.

According to the present invention, there is provided a method for modifying the content of storage compounds in seeds relative to control plants, comprising modulating expression in a plant of a variant of a nucleic acid represented by SEQ ID NO: 229, or comprising modulating expression in a plant of a variant of any one of the nucleic acid sequences given in Table H, or comprising modulating expression in a plant a variant of a nucleic acid encoding an orthologue, paralogue or homologue of any of the amino acid sequences given in Table H, which variant nucleic acid is obtained by gene shuffling.

A preferred method for modulating (preferably, increasing) expression in a plant of such a variant obtained by gene shuffling is by introducing and expressing in a plant a variant of any one of the nucleic acid sequences given in Table H, or comprising introducing and expressing in a plant a variant of a nucleic acid sequence encoding an orthologue, paralogue or homologue of any of the amino acid sequences given in Table H, which variant nucleic acid is obtained by gene shuffling.

Such variants obtained by gene shuffling useful in the methods of the invention, include nucleic acids of sufficient length to encode a polypeptide falling under the definition of a Class II HD-Zip transcription factor, i.e. meaning that the polypeptide comprising the following: (i) a homeodomain box; (ii) a leucine zipper; and (iii) Motifs I, it and III described herein.

Furthermore, nucleic acids useful in the methods of the invention may also be obtained by site-directed mutagenesis. Several methods are available to achieve site-directed mutagenesis, the most common being PCR-based methods (Current Protocols in Molecular Biology. Wiley Eds.).

Class II HD-Zip transcription factors exhibit the general biological activity of transcription factors (at least in their native form) and typically have DNA-binding activity and an activation domain. A person skilled in the art may easily determine the presence of an activation domain and DNA-binding activity using routine tools and techniques. Sessa et al., 1997 (J Mol Biol 274(3):303-309) studied the DNA-binding properties of the ATHB-1 and ATHB-2 (= HAT4) HD-Zip (HD-Zip-1 and -2) domains and found that they interact with DNA as homodimers and recognize two distinct 9 bp pseudopalindromic sequences, CAAT(A/T)ATTG (BS-1) and CAAT(G/C)ATTG (BS-2), respectively. From a mutational analysis of the HD-Zip-2 domain, they determined that conserved amino acid residues of helix 3, Val47 and Asn51, and Arg55 are essential for the DNA-binding activity of the HD-Zip-2 domain. They also report that the preferential recognition of a G/C base-pair at the central position by the HD-Zip-2 domain is abolished either by the replacement of Arg55 with lysine or by the substitution of Glu46 and Thr56 with the corresponding residues of the HD-Zip-1 domain (alanine and tryptophan, respectively).

According to a preferred feature of the invention, the modulated expression is increased expression. Methods for increasing expression of nucleic acids or genes, or gene products, are well documented in the art.

Another method for modulating expression in a plant of a nucleic acid encoding a Class II HD-Zip transcription factor comprises the reduction or substantial elimination of expression in a plant of an endogenous gene encoding a Class II HD-Zip transcription factor. Reference herein to "reduction or substantial elimination" is taken to mean a decrease in endogenous gene expression and/or polypeptide levels and/or polypeptide activity relative to control plants. The reduction or substantial elimination is in increasing order of preference at least 10%, 20%, 30%, 40% or 50%, 60%, 70%, 80%, 85%, 90%, or 95%, 96%, 97%, 98%, 99% or more reduced compared to that of control plants. Methods for decreasing expression are described above in the "definitions" sections.

For the reduction or substantial elimination of expression an endogenous gene in a plant, a sufficient length of substantially contiguous nucleotides of a nucleic acid sequence is required. In order to perform gene silencing, this may be as little as 20, 19, 18, 17, 16, 15, 14, 13, 12, 11, 10 or fewer nucleotides, alternatively this may be as much as the entire gene (including the 5' and/or 3' UTR, either in part or in whole). The stretch of substantially contiguous nucleotides may be derived from SEQ ID NO: 229, or from any of the nucleic acid sequences given in Table H, or from any nucleic acid capable of encoding an orthologue, paralogue or homologue of any one of the amino acid sequences given in Table H. A nucleic acid sequence encoding a (functional) polypeptide is not a requirement for the various methods discussed herein for the reduction or substantial elimination of expression of an endogenous gene.

Nucleic acids suitable for use in the methods of the invention may be derived from any natural or artificial source. The nucleic acid may be modified from its native form in composition and/or genomic environment through deliberate human manipulation. Preferably the nucleic acid is from a plant, further preferably from a dicotyledonous plant, further preferably from the family Brassicaceae, more preferably from *Arabidopsis thaliana.*

A preferred method for modulating (preferably, increasing) expression of a nucleic acid encoding a Class II HD-Zip transcription factor is by introducing and expressing in a plant a nucleic acid encoding a Class II HD-Zip transcription factor; however the effects of performing the method, i.e. the modified content of seed storage compounds, may also be achieved using other well known techniques. One such technique is T-DNA activation tagging. The effects of the invention may also be reproduced using the technique of TILLING. The effects of the invention may also be reproduced using homologous recombination, which allows introduction in a genome of a selected nucleic acid at a defined selected position.

Performance of the methods of the invention gives plants having seeds with a modified content of seed storage compounds relative to the seeds of control plants. The modified content of seed storage compounds refers to a modified content of one or more of lipids, oil, fatty acids, starch, sugar and proteins relative to that of control plants. Preferably, modulation of expression in a plant of a nucleic acid encoding a Class II HD-Zip transcription factor gives plants with seeds having increased oil content relative to the seeds of control plants. In such a case, the modulated expression is typically overexpression in a plant of a nucleic acid encoding a Class II HD-Zip transcription factor. Preferably, modulation of expression in a plant of a nucleic acid encoding a Class II HD-Zip transcription factor gives plants with seeds having increased protein content relative to the seeds of control plants. In such a case, the modulated expression is typically the reduction or substantial elimination of expression of an endogenous Class II HD-Zip transcription factor-encoding gene.

The present invention also encompasses plants or parts thereof (particularly seeds) obtainable by the methods according to the present invention. The plants or parts thereof comprise a nucleic acid transgene (comprising any one of the nucleic acid sequences described above as being useful in the methods of the invention).

The invention also provides genetic constructs and vectors to facilitate introduction and/or expression in a plant of the nucleic acid sequences described above as being useful in the methods of the invention.

More particularly, there is provided a gene construct comprising:

(i) A nucleic acid encoding a Class II HD-Zip transcription factor as defined hereinabove;
(ii) One or more control sequences operably linked to the nucleic acid of (i).

Constructs useful in the methods according to the present invention may be constructed using recombinant DNA technology well known to persons skilled in the art. The gene constructs may be inserted into vectors, which may be commercially available, suitable for transforming into plants and suitable for expression of the gene of interest in the transformed cells. The invention therefore provides use of a gene construct as defined hereinabove in the methods of the invention.

Plants are transformed with a vector comprising the sequence of interest. The skilled artisan is well aware of the genetic elements that must be present in the vector in order to successfully transform, select and propagate host cells containing the sequence of interest. The sequence of interest is operably linked to one or more control sequences (at least to a promoter). The terms "regulatory element", "control sequence" and "promoter" are all used interchangeably herein and are defined above.

Advantageously, any type of promoter, whether natural or synthetic, may be used to drive expression of the nucleic acid sequence. See the "Definitions" section herein for definitions of various promoter types.

According to a preferred feature of the invention, the nucleic acid of interest is operably linked to a constitutive promoter. A constitutive promoter is transcriptionally active during most but not necessarily all phases of growth and development and is substantially ubiquitously expressed. The constitutive promoter is preferably a GOS2 promoter, more preferably the constitutive promoter is a rice GOS2 promoter, further preferably the constitutive promoter is represented by a nucleic acid sequence substantially similar to SEQ ID NO: 56 or SEQ ID NO: 282, most preferably the constitutive promoter is as represented by SEQ ID NO: 56 or SEQ ID NO: 282. Examples of other constitutive promoters which may also be used to perform the methods of the invention are shown above.

Optionally, one or more terminator sequences may be used in the construct introduced into a plant. Additional regulatory elements may include transcriptional as well as translational enhancers. Those skilled in the art will be aware of terminator and enhancer sequences that may be suitable for use in performing the invention. An intron sequence may also be added to the 5' untranslated region (UTR) or in the coding sequence to increase the amount of the mature message that accumulates in the cytosol, as described in the definitions section. Other control sequences (besides promoter, enhancer, silencer, intron sequences, 3'UTR and/or 5'UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). Therefore, the genetic construct may optionally comprise a selectable marker gene. Selectable markers are described in more detail in the "definitions" section herein. The marker genes may be removed or excised from the transgenic cell once they are no longer needed. Techniques for marker removal are known in the art, useful techniques are described above in the definitions section.

The invention also provides a method for the production of transgenic plants having modified content of storage compounds in seeds relative to control plants, comprising introduction and expression in a plant of a nucleic acid sequence represented by SEQ ID NO: 229, or comprising introduction and expression in a plant of a nucleic acid sequence given in Table H, or comprising introduction and expression in a plant of a nucleic acid sequence encoding an orthologue, paralogue or homologue of any of the amino acid sequences given in Table H, or comprising introduction and expression in a plant of any of the nucleic acids defined herein as being useful in the methods of the invention.

More specifically, the present invention provides a method for the production of transgenic plants having modified content of storage compounds in seeds relative to control plants, which method comprises:

(i) introducing and expressing in a plant, plant part or plant cell a nucleic acid sequence represented by SEQ ID NO: 229, or comprising introducing and expressing in a plant a nucleic acid sequence given in Table H, or comprising introducing and expressing in a plant a nucleic acid sequence encoding an orthologue, paralogue or homologue of any of the amino acid sequences given in Table H; and
(ii) cultivating the plant cell under conditions promoting plant growth and development; and
(iii) harvest of seeds from the plant of (ii); and optionally
(iv) extraction of any one or more of lipids, oils, fatty acids, starch, sugar or protein from the seeds of (iii).

The harvested seeds may be processed to extract particular seed storage compounds, such as lipids, oils fatty acids, starch, sugar or protein. In particular, the seeds are used for oil extraction. The oils may be extracted from processed or unprocessed seeds.

The nucleic acid may be introduced directly into a plant cell or into the plant itself (including introduction into a tissue, organ or any other part of a plant). According to a preferred feature of the present invention, the nucleic acid is preferably introduced into a plant by transformation.

The term "transformation" as referred to herein is as defined above. Plant tissue capable of subsequent clonal propagation, whether by organogenesis or embryogenesis, may be transformed with a genetic construct of the present invention and a whole plant regenerated from there. The particular tissue chosen will vary depending on the clonal propagation systems available for, and best suited to, the particular species being transformed. Exemplary tissue targets include leaf disks, pollen, embryos, cotyledons, hypocotyls, megagametophytes, callus tissue, existing meristematic tissue (e.g., apical meristem, axillary buds, and root meristems), and induced meristem tissue (e.g., cotyledon meristem and hypocotyl meristem). The polynucleotide may be transiently or stably introduced into a host cell and may be maintained non-integrated, for example, as a plasmid. Alternatively, it may be integrated into the host genome. The resulting transformed plant cell may then be used to regenerate a transformed plant in a manner known to persons skilled in the art.

Transformation of plant species is now a fairly routine technique. Advantageously, any of several transformation methods may be used to introduce the gene of interest into a suitable ancestor cell. Transformation methods include the use of liposomes, electroporation, chemicals that increase free DNA uptake, injection of the DNA directly into the plant, particle gun bombardment, transformation using viruses or pollen and microprojection. Methods may be selected from the calcium/polyethylene glycol method for protoplasts (Krens, F.A. et al. (1982) Nature 296, 72-74; Negrutiu I et al. (1987) Plant Mol Biol 8: 363-373); electroporation of protoplasts (Shillito R.D. et al. (1985) Bio/Technol 3, 1099-1102); microinjection into plant material (Crossway A et al. (1986) Mol. Gen Genet 202: 179-185); DNA or RNA-coated particle bombardment (Klein TM et al. (1987) Nature 327: 70) infection with (non-integrative) viruses and the like. Transgenic rice plants are preferably produced via *Agrobacterium*-mediated transformation using any of the well known methods for rice transformation, such as described in any of the following: published European patent application EP 1198985 A1, Aldemita and Hodges (Planta 199: 612-617, 1996); Chan et al. (Plant Mol Biol 22 (3): 491-506, 1993), Hiei et al. (Plant J 6 (2): 271-282, 1994), which disclosures are incorporated by reference herein as if fully set forth. In the case of corn transformation, the preferred method is as described in either Ishida et al. (Nat. Biotechnol 14(6): 745-50, 1996) or Frame et al. (Plant Physiol 129(1): 13-22, 2002), which disclosures are incorporated by reference herein as if fully set forth.

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may be evaluated, for instance using Southern analysis, for the presence of the gene of interest, copy number and/or genomic organisation. Alternatively or additionally, expression levels of the newly introduced DNA may be monitored using Northern and/or Western analysis, or quantitative PCR, all techniques being well known to persons having ordinary skill in the art.

The generated transformed plants may be propagated by a variety of means, such as by clonal propagation or classical breeding techniques. For example, a first generation (or T1) transformed plant may be selfed to give homozygous second generation (or T2) transformants, and the T2 plants further propagated through classical breeding techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The invention also includes host cells containing any one of the nucleic acids described above as being useful in the methods of the invention. Preferred host cells according to the invention are plant cells.

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum and oats.

According to a further preferred embodiment of the present invention, the plant is a crop plant typically cultivated for oil production. Examples of such crop plants for oil production include rapeseed, canola, linseed, soybean, sunflower, maize, oat, rye, barley, wheat, pepper, tagetes, cotton, oil palm, coconut palm, flax, castor, peanut, olive, avocado, sesame, jatropha.

The invention also extends to harvestable parts of a plant, particularly seeds, but also leaves, fruits, flowers, stems, rhizomes, tubers, roots and bulbs. The invention furthermore relates to products derived, preferably directly derived, from a harvestable part of such a plant, such as dry pellets or powders, oil, fat and fatty acids, starch or proteins. A particular product of interest derived from a harvestable part of a plant is oil.

The present invention also encompasses use of any of the nucleic acids mentioned herein as being useful in the methods of the invention in modifying seed storage content relative to control plants. Particularly useful is the nucleic acid represented by SEQ ID NO: 229, and any of the nucleic acid sequences given in Table A, and any nucleic acid sequence encoding an orthologue, paralogue or homologue of any of the polypeptide sequences given in Table H. The present invention also encompasses use of a polypeptide sequence represented by SEQ ID NO: 230, and use of a polypeptide sequences given in Table H in modifying seed storage content relative to control plants.

These nucleic acids or the encoded polypeptides may find use in breeding programmes in which a DNA marker is identified which may be genetically linked to a Class II HD-Zip transcription factor-encoding gene. The nucleic acids/genes, or the polypeptides themselves may be used to define a molecular marker. This DNA or protein marker may then be used in breeding programmes to select plants having modified content of seed storage compounds.

Allelic variants may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

The nucleic acids may also be used as probes for genetically and physically mapping the genes that they are a part of, and as markers for traits linked to those genes. Such information may be useful in plant breeding in order to develop lines with desired phenotypes. Such use of requires only a nucleic acid sequence of at least 15 nucleotides in length. The nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the nucleic acids. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the nucleic acid in the genetic map previously obtained using this population (Botstein et al. (1980) Am. J. Hum. Genet. 32:314-331).

The production and use of plant gene-derived probes for use in genetic mapping is described in Bematzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence *in situ* hybridisation (FISH) mapping (Trask (1991) Trends Genet. 7:149-154). Although current methods of FISH mapping favor use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having modified content of seed storage compounds, as described hereinbefore. These traits may also be combined with other economically advantageous traits, such as yield-enhancing traits, tolerance to other abiotic and biotic stresses, traits modifying various architectural features and/or biochemical and/or physiological features.

**Detailed description for the SYB1 polypeptide**

Surprisingly, it has now been found that modulating expression in a plant of a nucleic acid encoding a SYB1 polypeptide gives plants having enhanced yield-related traits relative to control plants. This yield increase was surprisingly observed when the plants were cultivated under conditions without stress (non-stress conditions). The particular class of SYB1 polypeptides suitable for enhancing yield-related traits in plants is described in detail below.

The present invention provides a method for enhancing yield-related traits in plants relative to control plants, comprising modulating expression in a plant of a nucleic acid encoding a SYB1 polypeptide. The term "modulation" means in relation to expression or gene expression, a process in which the expression level is changed by said gene expression in comparison to the control plant, preferably the expression level is increased. The original, unmodulated expression may be of any kind of expression of a structural RNA (rRNA, tRNA) or mRNA with subsequent translation.

Any reference hereinafter to a "protein useful in the methods of the invention" is taken to mean a SYB1 polypeptide as defined herein. Any reference hereinafter to a "nucleic acid useful in the methods of the invention" is taken to mean a nucleic acid capable of encoding such a SYB1 polypeptide. The terms "polypeptide" and "protein" are used interchangeably herein and refer to amino acids in a polymeric form of any length. The terms "polynucleotide(s)", "nucleic acid sequence(s)", "nucleotide sequence(s)" are used interchangeably herein and refer to nucleotides, either ribonucleotides or deoxyribonucleotides or a combination of both, in a polymeric form of any length.

A preferred method for modulating (preferably, increasing) expression of a nucleic acid encoding a protein useful in the methods of the invention is by introducing and expressing in a plant a nucleic acid encoding a protein useful in the methods of the invention as defined below.

The nucleic acid to be introduced into a plant (and therefore useful in performing the methods of the invention) is any nucleic acid encoding the type of protein which will now be described, hereafter also named "*SYB1* nucleic acid" or "*SYB1* gene". A "SYB1" polypeptide as defined herein refers to any amino acid sequence comprising 3 Zinc finger domains of the RanBP type (SMART accession number: SM00547, Interpro accession number: IPR001876) and optionally one or more low complexity domain(s), but lacking, when analysed against the SMART database, any other domains that do not overlap with the Zinc finger domains or, if present, the low complexity domain. The ZnF_RBZ type Zinc finger domain is present in Ran-binding proteins (RanBPs), and other proteins. In RanBPs, this domain binds RanGDP. Low complexity domains may be identified using the SEG algorithm of Wootton and Federhen (Methods Enzymol. 266 (1996), pp. 554-571). Preferably SYB1 polypeptides in their natural form (that is, as they occur in nature) are in increasing order of preference, no longer then 350, 300, 290, 280, 270, 260, 250, 240, 230, 220, 210, 190 or 180 amino acids, more preferably the length of SYB1 polypeptide ranges between 180 and 130 amino acids.

Zinc finger domains are known to bind zinc ions and are generally involved in protein-DNA or protein-protein interactions. Preferably, the Zinc finger domains in the SYB1 protein start with one of the following motifs: (G/R/D/N) DW (motif 1, SEQ ID NO: 344), or GSW (motif 2, SEQ ID NO: 345), and has a first conserved cysteine residue on position 5 (wherein positions 1 to 3 are taken by motif 1 or motif 2), a second conserved cysteine residue between positions 7 and 10, a third conserved cysteine residue between positions 18 and 21 and a fourth conserved cysteine residue between positions 21 and 24. Furthermore, the Zinc finger domain in the SYB1 protein preferably comprises the conserved motifs NF(Q/C/S)(R/K)R (motif 3, SEQ ID NO: 346) or N(F/Y)(A/S/P)(N/S/F)R (motif 4, SEQ ID NO 347).

Optionally, the sequence located between the Zn-finger domains (say starting after the fourth conserved cysteine residue and ending before the start of motif 1 or 2) is enriched in glycine residues, the glycine content may be as high as 35%, or even higher (whereas the glycine content in an average protein is around 6.93% (SWISS PROT notes, release 44, July 2004)). Additionally or alternatively, the serine content in this sequence may also be increased compared to the serine content in an average protein (6.89%).

Preferably, the polypeptide sequence which when used in the construction of a phylogenetic tree, such as the one depicted in Figure 2b, clusters with the group of SYB1 polypeptides comprising the amino acid sequence represented by SEQ ID NO: 286 rather than with any other group.

Examples of polypeptides useful in the methods of the invention and nucleic acids encoding the same are as given below in the table of Example 40.

Also useful in the methods of the invention are homologues of any one of the amino acid sequences given in the table of Example 40, and derivatives of any one of the polypeptides given in the table of Example 40 or orthologues or paralogues of any of the aforementioned SEQ ID NOs.

**[0615]** The invention is illustrated by transforming plants with the *Arabidopsis thaliana* nucleic acid sequence represented by SEQ ID NO: 285, encoding the polypeptide sequence of SEQ ID NO: 286, however performance of the invention is not restricted to these sequences. The methods of the invention may advantageously be performed using any nucleic acid encoding a protein useful in the methods of the invention as defined herein, including orthologues and paralogues, such as any of the nucleic acid sequences given in the table of Example 40. The amino acid sequences given in the table of Example 40 may be considered to be orthologues and paralogues of the SYB1 polypeptide represented by SEQ ID NO: 286.

Orthologues and paralogues may easily be found by performing a so-called reciprocal blast search. Typically, this involves a first BLAST involving BLASTing a query sequence (for example using any of the sequences listed in the table of Example 40) against any sequence database, such as the publicly available NCBI database. BLASTN or TBLASTX (using standard default values) are generally used when starting from a nucleotide sequence, and BLASTP or TBLASTN (using standard default values) when starting from a protein sequence. The BLAST results may optionally be filtered. The full-length sequences of either the filtered results or non-filtered results are then BLASTed back (second BLAST) against sequences from the organism from which the query sequence is derived (where the query sequence is SEQ ID NO: 285 or SEQ ID NO: 286, the second BLAST would therefore be against *Arabidopsis thaliana* sequences). The results of the first and second BLASTs are then compared. A paralogue is identified if a high-ranking hit from the first blast is from the same species as from which the query sequence is derived, a BLAST back then ideally results in the query sequence as highest hit; an orthologue is identified if a high-ranking hit in the first BLAST is not from the same species as from which the query sequence is derived, and preferably results upon BLAST back in the query sequence being among the highest hits.

High-ranking hits are those having a low E-value. The lower the E-value, the more significant the score (or in other words the lower the chance that the hit was found by chance). Computation of the E-value is well known in the art. In addition to E-values, comparisons are also scored by percentage identity. Percentage identity refers to the number of identical nucleotides (or amino acids) between the two compared nucleic acid (or polypeptide) sequences over a particular length. In the case of large families, ClustalW may be used, followed by a neighbour joining tree, to help visualize clustering of related genes and to identify orthologues and paralogues.

The table of Example 40 gives examples of orthologues and paralogues of the SYB1 protein represented by SEQ ID NO 286. Further orthologues and paralogues may readily be identified using the BLAST procedure described above.

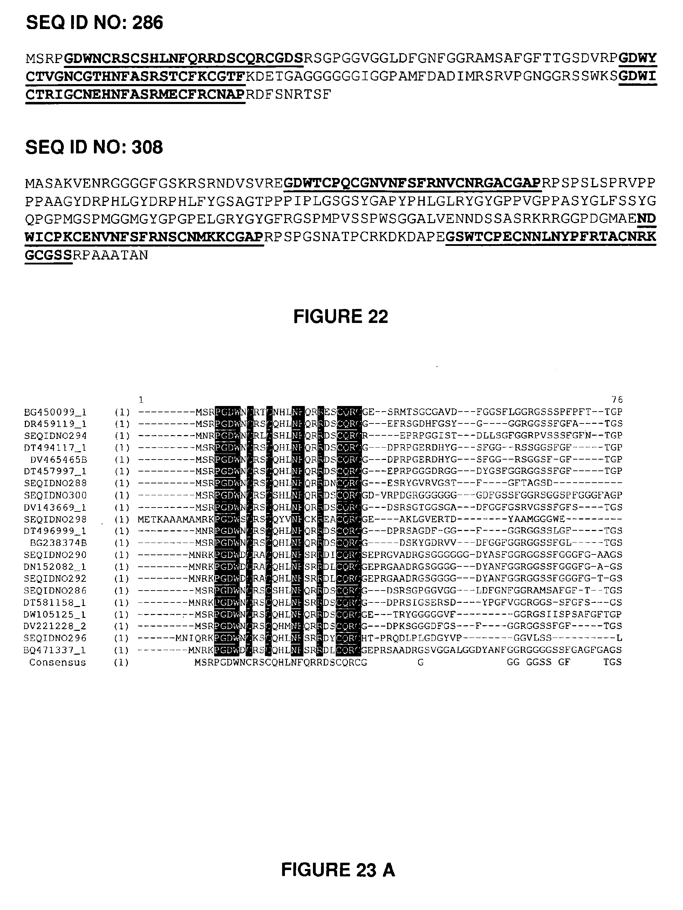

The proteins of the invention are identifiable by the presence of three conserved ZnF_RBZ type Zinc finger domain domains (shown in Figure 22). The terms "domain" and "motif" or "signature" are defined in the "Definitions" section. Specialist databases also exist for the identification of domains, for example, SMART (Schultz et al. (1998) Proc. Natl. Acad. Sci. USA 95, 5857-5864; Letunic et al. (2002) Nucleic Acids Res 30, 242-244, InterPro (Mulder et al., (2003) Nucl. Acids. Res. 31, 315-318, Prosite (Bucher and Bairoch (1994), A generalized profile syntax for biomolecular sequences motifs and its function in automatic sequence interpretation. (In) ISMB-94; Proceedings 2nd International Conference on Intelligent Systems for Molecular Biology. Altman R., Brutlag D., Karp P., Lathrop R., Searls D., Eds., pp53-61, AAAIPress, Menlo Park; Hulo et al., Nucl. Acids. Res. 32:D134-D137, (2004), or Pfam (Bateman et al., Nucleic Acids Research 30(1): 276-280 (2002). A set of tools for *in silico* analysis of protein sequences is available on the ExPASY proteomics server (hosted by the Swiss Institute of Bioinformatics (Gasteiger et al., ExPASy: the proteomics server for in-depth protein knowledge and analysis, Nucleic Acids Res. 31:3784-3788(2003)).

Domains may also be identified using routine techniques, such as by sequence alignment. Methods for the alignment of sequences for comparison are well known in the art, such methods include GAP, BESTFIT, BLAST, FASTA and TFASTA. GAP uses the algorithm of Needleman and Wunsch ((1970) J Mol Biol 48: 443-453) to find the global (i.e. spanning the complete sequences) alignment of two sequences that maximizes the number of matches and minimizes the number of gaps. The BLAST algorithm (Altschul et al. (1990) J Mol Biol 215: 403-10) calculates percent sequence identity and performs a statistical analysis of the similarity between the two sequences. The software for performing BLAST analysis is publicly available through the National Centre for Biotechnology Information (NCBI). Homologues may readily be identified using, for example, the ClustalW multiple sequence alignment algorithm (version 1.83), with the default pairwise alignment parameters, and a scoring method in percentage. Global percentages of similarity and identity may also be determined using one of the methods available in the MatGAT software package (Campanella et al., BMC Bioinformatics. 2003 Jul 10;4:29. MatGAT: an application that generates similarity/identity matrices using protein or DNA sequences.). Minor manual editing may be performed to optimise alignment between conserved motifs, as would be apparent to a person skilled in the art. Furthermore, instead of using full-length sequences for the identification of homologues, specific domains (such as the ZnF_RBZ type Zinc finger domain, or one of the motifs defined above) may be used as well. The sequence identity values, which are indicated below in Example 42 as a percentage were determined over the entire nucleic acid or amino acid sequence, and/or over selected domains or conserved motif(s), using the programs mentioned above using the default parameters.

Furthermore, SYB1 proteins (at least in their native form) may interact with proteins or nucleic acids and has the effect of increasing seed yield when expressed according to the methods of the present invention. Further details are provided in Example 45.

Nucleic acids encoding proteins useful in the methods of the invention need not be full-length nucleic acids, since performance of the methods of the invention does not rely on the use of full-length nucleic acid sequences. Examples of nucleic acids suitable for use in performing the methods of the invention include the nucleic acid sequences given in the table of Example 40, but are not limited to those sequences. Nucleic acid variants may also be useful in practising the methods of the invention. Examples of such nucleic acid variants include portions of nucleic acids encoding a protein useful in the methods of the invention, nucleic acids hybridising to nucleic acids encoding a protein useful in the methods of the invention, splice variants of nucleic acids encoding a protein useful in the methods of the invention, allelic variants of nucleic acids encoding a protein useful in the methods of the invention and variants of nucleic acids encoding a protein useful in the methods of the invention that are obtained by gene shuffling. The terms portion, hybridising sequence, splice variant, allelic variant and gene shuffling will now be described.

According to the present invention, there is provided a method for enhancing yield-related traits in plants, comprising introducing and expressing in a plant a portion of any one of the nucleic acid sequences given in the table of Example 40, or a portion of a nucleic acid encoding an orthologue, paralogue or homologue of any of the amino acid sequences given in the table of Example 40.

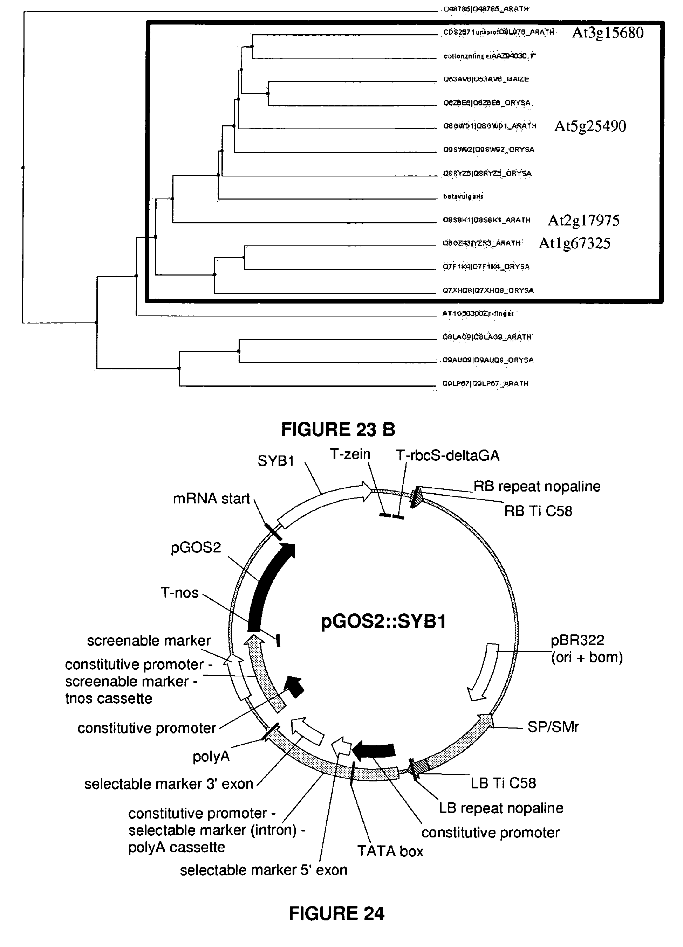

Portions useful in the methods of the invention, encode a polypeptide falling within the definition of a nucleic acid encoding a protein useful in the methods of the invention as defined herein and having substantially the same biological activity as the amino acid sequences given in the table of Example 40. Preferably, the portion is a portion of any one of the nucleic acids given in the table of Example 40. The portion is typically at least 200 consecutive nucleotides in length, preferably at least 300 consecutive nucleotides in length, more preferably at least 400 consecutive nucleotides in length and most preferably at least 500 consecutive nucleotides in length, the consecutive nucleotides being of any one of the nucleic acid sequences given in the table of Example 40. Most preferably the portion is a portion of the nucleic acid of SEQ ID NO: 285. Preferably, the portion encodes an amino acid sequence comprising three ZnF_RBZ type Zinc finger domains as defined herein. Preferably, the portion encodes an amino acid sequence which when used in the construction of a SYB1 phylogenetic tree, such as the one depicted in Figure 23b, tends to cluster with the group of SYB1 proteins comprising the amino acid sequence represented by SEQ ID NO: 286 rather than with any other group.

A portion of a nucleic acid encoding a SYB1 protein as defined herein may be prepared, for example, by making one or more deletions to the nucleic acid. The portions may be used in isolated form or they may be fused to other coding (or non coding) sequences in order to, for example, produce a protein that combines several activities. When fused to other coding sequences, the resultant polypeptide produced upon translation may be bigger than that predicted for the SYB1 protein portion.

Another nucleic acid variant useful in the methods of the invention is a nucleic acid capable of hybridising, under reduced stringency conditions, preferably under stringent conditions, with a nucleic acid encoding a SYB1 protein as defined herein, or with a portion as defined herein.

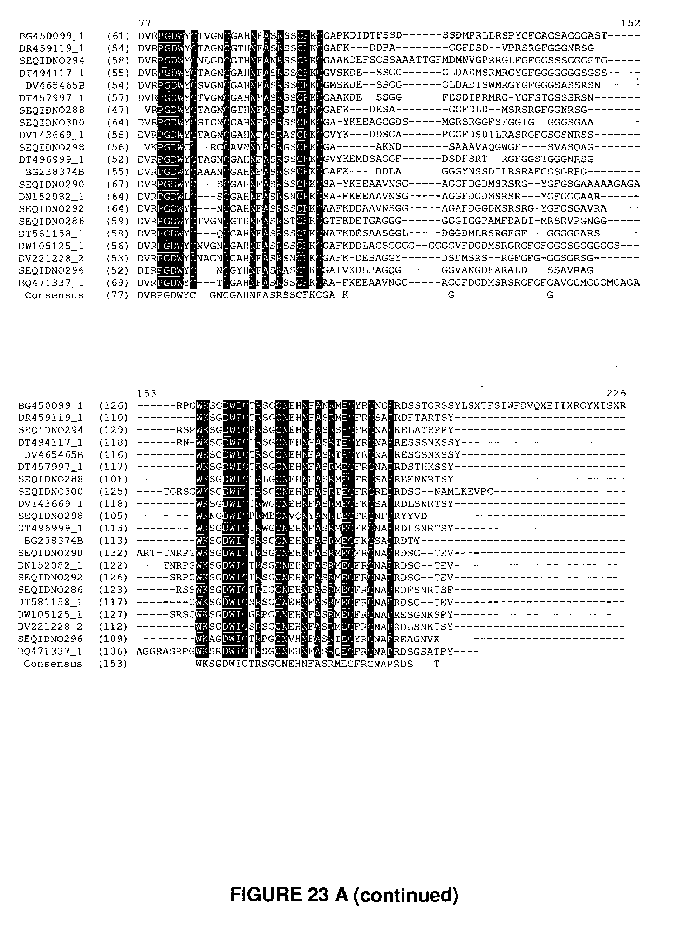

Hybridising sequences useful in the methods of the invention, encode a polypeptide having three ZnF_RBZ type Zinc finger domains (see the alignment of Figure 23a) and having substantially the same biological activity as the SYB1 protein represented by any of the amino acid sequences given in the table of Example 40. The hybridising sequence is typically at least 200 consecutive nucleotides in length, preferably at least 300 consecutive nucleotides in length, more preferably at least 400 consecutive nucleotides in length and most preferably at least 500 consecutive nucleotides in length, the consecutive nucleotides being of any one of the nucleic acid sequences given in the table of Example 40. Preferably, the hybridising sequence is one that is capable of hybridising to any of the nucleic acids given in the table of Example 40, or to a portion of any of these sequences, a portion being as defined above. Most preferably, the hybridising sequence is capable of hybridising to a nucleic acid as represented by SEQ ID NO: 285 or to a portion thereof. Preferably, the hybridising sequence encodes an amino acid sequence comprising any one or more of the motifs or domains as defined herein. Preferably, the hybridising sequence encodes an amino acid sequence which when used in the construction of a SYB1 phylogenetic tree, such as the one depicted in Figure 23b, tends to cluster with the group of SYB1 proteins comprising the amino acid sequence represented by SEQ ID NO: 286 rather than with any other group.

According to the present invention, there is provided a method for enhancing yield-related traits in plants, comprising introducing and expressing in a plant a nucleic acid capable of hybridizing to any one of the nucleic acids given in the table of Example 40, or comprising introducing and expressing in a plant a nucleic acid capable of hybridising to a nucleic acid encoding an orthologue, paralogue or homologue of any of the nucleic acid sequences given in the table of Example 40.

Another nucleic acid variant useful in the methods of the invention is a splice variant encoding a SYB1 protein as defined hereinabove, the term "splice variant" being as defined above.

According to the present invention, there is provided a method for enhancing yield-related traits in plants, comprising introducing and expressing in a plant a splice variant of any one of the nucleic acid sequences given in the table of Example 40, or a splice variant of a nucleic acid encoding an orthologue, paralogue or homologue of any of the amino acid sequences given in the table of Example 40.

Preferred splice variants are splice variants of a nucleic acid represented by SEQ ID NO: 285 or a splice variant of a nucleic acid encoding an orthologue or paralogue of SEQ ID NO: 286. Preferably, the amino acid sequence encoded by the splice variant comprises any one or more of the motifs or domains as defined herein. Preferably, the amino acid sequence encoded by the splice variant, when used in the construction of a SYB1 phylogenetic tree, such as the one depicted in Figure 23b, tends to cluster with the group of SYB1 proteins comprising the amino acid sequence represented by SEQ ID NO: 286 rather than with any other group.

Another nucleic acid variant useful in performing the methods of the invention is an allelic variant of a nucleic acid encoding a SYB1 protein as defined hereinabove. Allelic variants exist in nature, and encompassed within the methods of the present invention is the use of these natural alleles. The allelic variants useful in the methods of the present invention have substantially the same biological activity as the SYB1 protein of SEQ ID NO: 286.

According to the present invention, there is provided a method for enhancing yield-related traits in plants, comprising introducing and expressing in a plant an allelic variant of any one of the nucleic acids given in the table of Example 40, or comprising introducing and expressing in a plant an allelic variant of a nucleic acid encoding an orthologue, paralogue or homologue of any of the amino acid sequences given in the table of Example 40.

Preferably, the allelic variant is an allelic variant of SEQ ID NO: 285 or an allelic variant of a nucleic acid encoding an orthologue or paralogue of SEQ ID NO: 286. Preferably, the amino acid sequence encoded by the allelic variant comprises any one or more of the motifs or domains as defined herein. Preferably, the amino acid sequence encoded by the allelic variant, when used in the construction of a SYB1 phylogenetic tree, such as the one depicted in Figure 23b, tends to cluster with the group of SYB1 proteins comprising the amino acid sequence represented by SEQ ID NO: 286 rather than with any other group.

A further nucleic acid variant useful in the methods of the invention is a nucleic acid variant obtained by gene shuffling, the term "gene shuffling" as described above.

According to the present invention, there is provided a method for enhancing yield-related traits in plants, comprising introducing and expressing in a plant a variant of any one of the nucleic acid sequences given in the table of Example 40, or comprising introducing and expressing in a plant a variant of a nucleic acid encoding an orthologue, paralogue or homologue of any of the amino acid sequences given in the table of Example 40, which variant nucleic acid is obtained by gene shuffling.

Preferably, the variant nucleic acid obtained by gene shuffling encodes an amino acid sequence comprising any one or more of the motifs or domains as defined herein. Preferably, the amino acid sequence encoded by the variant nucleic acid obtained by gene shuffling, when used in the construction of a SYB1 phylogenetic tree such as the one depicted in Figure 23b, tends to cluster with the group of SYB1 proteins comprising the amino acid sequence represented by SEQ ID NO: 286 rather than with any other group.

Furthermore, nucleic acid variants may also be obtained by site-directed mutagenesis. Several methods are available to achieve site-directed mutagenesis, the most common being PCR based methods (Current Protocols in Molecular Biology. Wiley Eds.).

Nucleic acids encoding SYB1 proteins may be derived from any natural or artificial source. The nucleic acid may be modified from its native form in composition and/or genomic environment through deliberate human manipulation. Preferably the SYB1-encoding nucleic acid is from a plant, further preferably from a dicotyledonous plant, more preferably from the Brassicaceae family; most preferably the nucleic acid is from *Arabidopsis thaliana.*

Any reference herein to a SYB1 protein is therefore taken to mean a SYB1 protein as defined above. Any nucleic acid encoding such a SYB1 protein is suitable for use in performing the methods of the invention.

The present invention also encompasses plants or parts thereof (including seeds) obtainable by the methods according to the present invention. The plants or parts thereof comprise a nucleic acid transgene encoding a SYB1 protein as defined above.

The invention also provides genetic constructs and vectors to facilitate introduction and/or expression of the nucleic acid sequences useful in the methods according to the invention, in a plant. Constructs useful in the methods according to the present invention may be constructed using recombinant DNA technology well known to persons skilled in the art. The gene constructs may be inserted into vectors, which may be commercially available, suitable for transforming into plants and suitable for expression of the gene of interest in the transformed cells. The invention also provides use of a gene construct as defined herein in the methods of the invention.

More specifically, the present invention provides a construct comprising:

(i) a *SYB1* nucleic acid or variant thereof, as defined hereinabove;
(ii) one or more control sequences operably linked the nucleic acid sequence of (i); and optionally
(iii) a transcription termination sequence.

Preferably, the nucleic acid encoding a SYB1 polypeptide is as defined above. The term "control sequence" and "termination sequence" are as defined herein.

Plants are transformed with a vector comprising the sequence of interest (i.e., a nucleic acid encoding a SYB1 polypeptide as defined herein. The skilled artisan is well aware of the genetic elements that must be present on the vector in order to successfully transform, select and propagate host cells containing the sequence of interest. The sequence of interest is operably linked to one or more control sequences (at least to a promoter). The terms "regulatory element", "control sequence" and "promoter" are all used interchangeably herein and are defined above.

Advantageously, any type of promoter may be used to drive expression of the nucleic acid sequence. Preferably, the *SYB1* nucleic acid or variant thereof is operably linked to a constitutive promoter. A preferred constitutive promoter is one that is also substantially ubiquitously expressed. Further preferably the promoter is derived from a plant, more preferably a monocotyledonous plant. Most preferred is use of a GOS2 promoter (from rice) (SEQ ID NO: 56 or 343). It should be clear that the applicability of the present invention is not restricted to the *SYB1* nucleic acid represented by SEQ ID NO: 285, nor is the applicability of the invention restricted to expression of a *SYB1* nucleic acid when driven by a GOS2 promoter. Examples of other constitutive promoters which may also be used to drive expression of a *SYB1* nucleic acid are shown above.

Optionally, one or more terminator sequences may be used in the construct introduced into a plant. Additional regulatory elements may include transcriptional as well as translational enhancers. Those skilled in the art will be aware of terminator and enhancer sequences that may be suitable for use in performing the invention. An intron sequence may also be added to the 5’ untranslated region (UTR) or in the coding sequence to increase the amount of the mature message that accumulates in the cytosol, as described in the definitions section. Other control sequences (besides promoter, enhancer, silencer, intron sequences, 3’UTR and/or 5’UTR regions) may be protein and/or RNA stabilizing elements. Such sequences would be known or may readily be obtained by a person skilled in the art.

The genetic constructs of the invention may further include an origin of replication sequence that is required for maintenance and/or replication in a specific cell type. One example is when a genetic construct is required to be maintained in a bacterial cell as an episomal genetic element (e.g. plasmid or cosmid molecule). Preferred origins of replication include, but are not limited to, the f1-ori and colE1.

For the detection of the successful transfer of the nucleic acid sequences as used in the methods of the invention and/or selection of transgenic plants comprising these nucleic acids, it is advantageous to use marker genes (or reporter genes). "Selectable markers" are described in more detail in the definitions section. The marker genes may be removed or excised from the transgenic cell once they are no longer needed. Techniques for marker removal are known in the art, useful techniques are described above in the definitions section.

The invention also provides a method for the production of transgenic plants having enhanced yield-related traits relative to control plants, comprising introduction and expression in a plant of any nucleic acid encoding a SYB1 protein as defined hereinabove.

More specifically, the present invention provides a method for the production of transgenic plants having increased yield, which method comprises:

(i) introducing and expressing in a plant or plant cell a *SYB1* nucleic acid or variant thereof; and
(ii) cultivating the plant cell under conditions promoting plant growth and development.

The nucleic acid of (i) may be any of the nucleic acids capable of encoding a SYB1 polypeptide as defined herein.

The nucleic acid may be introduced directly into a plant cell or into the plant itself (including introduction into a tissue, organ or any other part of a plant). According to a preferred feature of the present invention, the nucleic acid is preferably introduced into a plant by transformation. The term "transformation" is described in more detail in the "definitions" section herein.

The genetically modified plant cells can be regenerated via all methods with which the skilled worker is familiar. Suitable methods can be found in the abovementioned publications by S.D. Kung and R. Wu, Potrykus or Höfgen and Willmitzer.

Generally after transformation, plant cells or cell groupings are selected for the presence of one or more markers which are encoded by plant-expressible genes co-transferred with the gene of interest, following which the transformed material is regenerated into a whole plant. To select transformed plants, the plant material obtained in the transformation is, as a rule, subjected to selective conditions so that transformed plants can be distinguished from untransformed plants. For example, the seeds obtained in the above-described manner can be planted and, after an initial growing period, subjected to a suitable selection by spraying. A further possibility consists in growing the seeds, if appropriate after sterilization, on agar plates using a suitable selection agent so that only the transformed seeds can grow into plants. Alternatively, the transformed plants are screened for the presence of a selectable marker such as the ones described above.

Following DNA transfer and regeneration, putatively transformed plants may also be evaluated, for instance using Southern analysis, for the presence of the gene of interest, copy number and/or genomic organisation. Alternatively or additionally, expression levels of the newly introduced DNA may be monitored using Northern and/or Western analysis, both techniques being well known to persons having ordinary skill in the art.

The generated transformed plants may be propagated by a variety of means, such as by clonal propagation or classical breeding techniques. For example, a first generation (or T1) transformed plant may be selfed and homozygous second-generation (or T2) transformants selected, and the T2 plants may then further be propagated through classical breeding techniques.

The generated transformed organisms may take a variety of forms. For example, they may be chimeras of transformed cells and non-transformed cells; clonal transformants (e.g., all cells transformed to contain the expression cassette); grafts of transformed and untransformed tissues (e.g., in plants, a transformed rootstock grafted to an untransformed scion).

The present invention clearly extends to any plant cell or plant produced by any of the methods described herein, and to all plant parts and propagules thereof. The present invention extends further to encompass the progeny of a primary transformed or transfected cell, tissue, organ or whole plant that has been produced by any of the aforementioned methods, the only requirement being that progeny exhibit the same genotypic and/or phenotypic characteristic(s) as those produced by the parent in the methods according to the invention.

The invention also includes host cells containing an isolated nucleic acid encoding a SYB1 protein as defined hereinabove. Preferred host cells according to the invention are plant cells.

Host plants for the nucleic acids or the vector used in the method according to the invention, the expression cassette or construct or vector are, in principle, advantageously all plants, which are capable of synthesizing the polypeptides used in the inventive method.

The methods of the invention are advantageously applicable to any plant. Plants that are particularly useful in the methods of the invention include all plants which belong to the superfamily Viridiplantae, in particular monocotyledonous and dicotyledonous plants including fodder or forage legumes, ornamental plants, food crops, trees or shrubs. According to a preferred embodiment of the present invention, the plant is a crop plant. Examples of crop plants include soybean, sunflower, canola, alfalfa, rapeseed, cotton, tomato, potato and tobacco. Further preferably, the plant is a monocotyledonous plant. Examples of monocotyledonous plants include sugarcane. More preferably the plant is a cereal. Examples of cereals include rice, maize, wheat, barley, millet, rye, triticale, sorghum and oats.

The invention also extends to harvestable parts of a plant such as, but not limited to seeds, leaves, fruits, flowers, stems, rhizomes, tubers and bulbs. The invention furthermore relates to products derived, preferably directly derived, from a harvestable part of such a plant, such as dry pellets or powders, oil, fat and fatty acids, starch or proteins.

According to a preferred feature of the invention, the modulated expression is increased expression. As mentioned above, a preferred method for modulating (preferably, increasing) expression of a nucleic acid encoding a SYB1 protein is by introducing and expressing in a plant a nucleic acid encoding a SYB1 protein; however the effects of performing the method, i.e. enhancing yield-related traits may also be achieved using other well known techniques. One such technique is T-DNA activation tagging. The effects of the invention may also be reproduced using the technique of TILLING, or with homologous recombination, which allows introduction in a genome of a selected nucleic acid at a defined selected position..

Performance of the methods of the invention gives plants having enhanced yield-related traits. In particular performance of the methods of the invention gives plants having increased yield, especially increased seed yield relative to control plants. The terms "yield" and "seed yield" are described in more detail in the "definitions" section herein.

Reference herein to enhanced yield-related traits is taken to mean an increase in biomass (weight) of one or more parts of a plant, which may include aboveground (harvestable) parts and/or (harvestable) parts below ground.

In particular, such harvestable parts are seeds, and performance of the methods of the invention results in plants having increased seed yield relative to the seed yield of suitable control plants.

Taking corn as an example, a yield increase may be manifested as one or more of the following: increase in the number of plants established per square meter, an increase in the number of ears per plant, an increase in the number of rows, number of kernels per row, kernel weight, thousand kernel weight, ear length/diameter, increase in the seed filling rate (which is the number of filled seeds divided by the total number of seeds and multiplied by 100), among others. Taking rice as an example, a yield increase may manifest itself as an increase in one or more of the following: number of plants per square meter, number of panicles per plant, number of spikelets per panicle, number of flowers (florets) per panicle (which is expressed as a ratio of the number of filled seeds over the number of primary panicles), increase in the seed filling rate (which is the number of filled seeds divided by the total number of seeds and multiplied by 100), increase in thousand kernel weight, among others.

Since the transgenic plants according to the present invention have increased yield, it is likely that these plants exhibit an increased growth rate (during at least part of their life cycle), relative to the growth rate of control plants at a corresponding stage in their life cycle. The increased growth rate may be specific to one or more parts of a plant (including seeds), or may be throughout substantially the whole plant. Plants having an increased growth rate may have a shorter life cycle. The life cycle of a plant may be taken to mean the time needed to grow from a dry mature seed up to the stage where the plant has produced dry mature seeds, similar to the starting material. This life cycle may be influenced by factors such as early vigour, growth rate, greenness index, flowering time and speed of seed maturation. The increase in growth rate may take place at one or more stages in the life cycle of a plant or during substantially the whole plant life cycle. Increased growth rate during the early stages in the life cycle of a plant may reflect enhanced vigour. The increase in growth rate may alter the harvest cycle of a plant allowing plants to be sown later and/or harvested sooner than would otherwise be possible (a similar effect may be obtained with earlier flowering time). If the growth rate is sufficiently increased, it may allow for the further sowing of seeds of the same plant species (for example sowing and harvesting of rice plants followed by sowing and harvesting of further rice plants all within one conventional growing period). Similarly, if the growth rate is sufficiently increased, it may allow for the further sowing of seeds of different plants species (for example the planting and harvesting of corn plants followed by, for example, the planting and optional harvesting of soy bean, potato or any other suitable plant). Harvesting additional times from the same rootstock in the case of some crop plants may also be possible. Altering the harvest cycle of a plant may lead to an increase in annual biomass production per square meter (due to an increase in the number of times (say in a year) that any particular plant may be grown and harvested). An increase in growth rate may also allow for the cultivation of transgenic plants in a wider geographical area than their wild-type counterparts, since the territorial limitations for growing a crop are often determined by adverse environmental conditions either at the time of planting (early season) or at the time of harvesting (late season). Such adverse conditions may be avoided if the harvest cycle is shortened. The growth rate may be determined by deriving various parameters from growth curves, such parameters may be: T-Mid (the time taken for plants to reach 50% of their maximal size) and T-90 (time taken for plants to reach 90% of their maximal size), amongst others.

According to a preferred feature of the present invention, performance of the methods of the invention gives plants having an increased growth rate relative to control plants. Therefore, according to the present invention, there is provided a method for increasing the growth rate of plants, which method comprises modulating expression, preferably increasing expression, in a plant of a nucleic acid encoding a SYB1 protein as defined herein.

An increase in yield and/or growth rate occurs whether the plant is under non-stress conditions or whether the plant is exposed to various stresses compared to control plants. Plants typically respond to exposure to stress by growing more slowly. In conditions of severe stress, the plant may even stop growing altogether. Mild stress on the other hand is defined herein as being any stress to which a plant is exposed which does not result in the plant ceasing to grow altogether without the capacity to resume growth. Mild stress in the sense of the invention leads to a reduction in the growth of the stressed plants of less than 40%, 35% or 30%, preferably less than 25%, 20% or 15%, more preferably less than 14%, 13%, 12%, 11% or 10% or less in comparison to the control plant under non-stress conditions. Due to advances in agricultural practices (irrigation, fertilization, pesticide treatments) severe stresses are not often encountered in cultivated crop plants. As a consequence, the compromised growth induced by mild stress is often an undesirable feature for agriculture. Mild stresses are the everyday biotic and/or abiotic (environmental) stresses to which a plant is exposed. Abiotic stresses may be due to drought or excess water, anaerobic stress, salt stress, chemical toxicity, oxidative stress and hot, cold or freezing temperatures. The abiotic stress may be an osmotic stress caused by a water stress (particularly due to drought), salt stress, oxidative stress or an ionic stress. Biotic stresses are typically those stresses caused by pathogens, such as bacteria, viruses, fungi and insects.

In particular, the methods of the present invention may be performed under non-stress conditions or under conditions of mild drought to give plants having increased yield relative to control plants. As reported in Wang et al. (Planta (2003) 218: 1-14), abiotic stress leads to a series of morphological, physiological, biochemical and molecular changes that adversely affect plant growth and productivity. Drought, salinity, extreme temperatures and oxidative stress are known to be interconnected and may induce growth and cellular damage through similar mechanisms. Rabbani et al. (Plant Physiol (2003) 133: 1755-1767) describes a particularly high degree of "cross talk" between drought stress and high-salinity stress. For example, drought and/or salinisation are manifested primarily as osmotic stress, resulting in the disruption of homeostasis and ion distribution in the cell. Oxidative stress, which frequently accompanies high or low temperature, salinity or drought stress, may cause denaturing of functional and structural proteins. As a consequence, these diverse environmental stresses often activate similar cell signaling pathways and cellular responses, such as the production of stress proteins, up-regulation of anti-oxidants, accumulation of compatible solutes and growth arrest. The term "non-stress" conditions as used herein are those environmental conditions that allow optimal growth of plants. Persons skilled in the art are aware of normal soil conditions and climatic conditions for a given location.

Performance of the methods of the invention gives plants grown under non-stress conditions or under mild drought conditions increased yield relative to suitable control plants grown under comparable conditions. Therefore, according to the present invention, there is provided a method for increasing yield in plants grown under non-stress conditions or under mild drought conditions, which method comprises increasing expression in a plant of a nucleic acid encoding a SYB1 polypeptide.

Performance of the methods of the invention gives plants grown under conditions of nutrient deficiency, particularly under conditions of nitrogen deficiency, increased yield relative to control plants grown under comparable conditions. Therefore, according to the present invention, there is provided a method for increasing yield in plants grown under conditions of nutrient deficiency, which method comprises increasing expression in a plant of a nucleic acid encoding a SYB1 polypeptide. Nutrient deficiency may result from a lack of nutrients such as nitrogen, phosphates and other phosphorous-containing compounds, potassium, calcium, cadmium, magnesium, manganese, iron and boron, amongst others.

In a preferred embodiment of the invention, the increase in yield and/or growth rate occurs according to the methods of the present invention under non-stress conditions.

The present invention also encompasses use of nucleic acids encoding the SYB1 protein described herein and use of these SYB1 proteins in enhancing yield-related traits in plants. The present invention furthermore encompasses use of plants

Nucleic acids encoding the SYB1 protein described herein, or the SYB1 proteins themselves, may find use in breeding programmes in which a DNA marker is identified which may be genetically linked to a SYB1-encoding gene. The nucleic acids/genes, or the SYB1 proteins themselves may be used to define a molecular marker. This DNA or protein marker may then be used in breeding programmes to select plants having enhanced yield-related traits as defined hereinabove in the methods of the invention.

Allelic variants of a SYB1 protein-encoding nucleic acid/gene may also find use in marker-assisted breeding programmes. Such breeding programmes sometimes require introduction of allelic variation by mutagenic treatment of the plants, using for example EMS mutagenesis; alternatively, the programme may start with a collection of allelic variants of so called "natural" origin caused unintentionally. Identification of allelic variants then takes place, for example, by PCR. This is followed by a step for selection of superior allelic variants of the sequence in question and which give increased yield. Selection is typically carried out by monitoring growth performance of plants containing different allelic variants of the sequence in question. Growth performance may be monitored in a greenhouse or in the field. Further optional steps include crossing plants in which the superior allelic variant was identified with another plant. This could be used, for example, to make a combination of interesting phenotypic features.

Nucleic acids encoding SYB1 proteins may also be used as probes for genetically and physically mapping the genes that they are a part of, and as markers for traits linked to those genes. Such information may be useful in plant breeding in order to develop lines with desired phenotypes. Such use of SYB1 protein-encoding nucleic acids requires only a nucleic acid sequence of at least 15 nucleotides in length. The SYB1 protein-encoding nucleic acids may be used as restriction fragment length polymorphism (RFLP) markers. Southern blots (Sambrook J, Fritsch EF and Maniatis T (1989) Molecular Cloning, A Laboratory Manual) of restriction-digested plant genomic DNA may be probed with the SYB1 protein-encoding nucleic acids. The resulting banding patterns may then be subjected to genetic analyses using computer programs such as MapMaker (Lander et al. (1987) Genomics 1: 174-181) in order to construct a genetic map. In addition, the nucleic acids may be used to probe Southern blots containing restriction endonuclease-treated genomic DNAs of a set of individuals representing parent and progeny of a defined genetic cross. Segregation of the DNA polymorphisms is noted and used to calculate the position of the SYB1 protein-encoding nucleic acid in the genetic map previously obtained using this population (Botstein et al. (1980) Am. J. Hum. Genet. 32:314-331).

The production and use of plant gene-derived probes for use in genetic mapping is described in Bernatzky and Tanksley (1986) Plant Mol. Biol. Reporter 4: 37-41. Numerous publications describe genetic mapping of specific cDNA clones using the methodology outlined above or variations thereof. For example, F2 intercross populations, backcross populations, randomly mated populations, near isogenic lines, and other sets of individuals may be used for mapping. Such methodologies are well known to those skilled in the art.

The nucleic acid probes may also be used for physical mapping (i.e., placement of sequences on physical maps; see Hoheisel et al. In: Non-mammalian Genomic Analysis: A Practical Guide, Academic press 1996, pp. 319-346, and references cited therein).

In another embodiment, the nucleic acid probes may be used in direct fluorescence *in situ* hybridisation (FISH) mapping (Trask (1991) Trends Genet. 7:149-154). Although current methods of FISH mapping favour use of large clones (several kb to several hundred kb; see Laan et al. (1995) Genome Res. 5:13-20), improvements in sensitivity may allow performance of FISH mapping using shorter probes.

A variety of nucleic acid amplification-based methods for genetic and physical mapping may be carried out using the nucleic acids. Examples include allele-specific amplification (Kazazian (1989) J. Lab. Clin. Med 11:95-96), polymorphism of PCR-amplified fragments (CAPS; Sheffield et al. (1993) Genomics 16:325-332), allele-specific ligation (Landegren et al. (1988) Science 241:1077-1080), nucleotide extension reactions (Sokolov (1990) Nucleic Acid Res. 18:3671), Radiation Hybrid Mapping (Walter et al. (1997) Nat. Genet. 7:22-28) and Happy Mapping (Dear and Cook (1989) Nucleic Acid Res. 17:6795-6807). For these methods, the sequence of a nucleic acid is used to design and produce primer pairs for use in the amplification reaction or in primer extension reactions. The design of such primers is well known to those skilled in the art. In methods employing PCR-based genetic mapping, it may be necessary to identify DNA sequence differences between the parents of the mapping cross in the region corresponding to the instant nucleic acid sequence. This, however, is generally not necessary for mapping methods.

The methods according to the present invention result in plants having enhanced yield-related traits, as described hereinbefore. These traits may also be combined with other economically advantageous traits, such as further yield-enhancing traits, tolerance to other abiotic and biotic stresses, traits modifying various architectural features and/or biochemical and/or physiological features.

## Description of figures

The present invention will now be described with reference to the following figures in which:

**Fig. 1** shows an example of the domain structure of an AZ polypeptide. The protein encoded by SEQ ID NO: 2 comprises two ankyrin repeats (bold, underlined) and two C3H1 domains (italics, underlined).

**Fig. 2** shows the Ankyrin (ANK) and C3H1 Zinc finger (C3H1) consensus sequences according to the SMART database, the symbols for the various amino acid groups are given in the legend.

**Fig. 3** represents a sequence identity/similarity matrix prepared with MATGAT (BLOSUM62, gap opening penalty 11, gap extension penalty 1). Above the diagonal, in bold, the sequence identities are displayed, below the diagonal, sequence similarities are given for: A) full length protein sequences, B) partial protein sequences comprising the most C-terminal putative Zn-finger domain.

**Fig. 4** shows the binary vector p056, for expression in *Oryza sativa* of an *Arabidopsis thaliana* AZ coding sequence under the control of a WS118 promoter (internal reference PRO0151).

**Fig. 5** shows the typical domain structure of SYT polypeptides from plants and mammals. The conserved SNH domain is located at the N-terminal end of the polypeptide. The C-terminal remainder of the polypeptide consists of a QG-rich domain in plant SYT polypeptides, and of a QPGY-rich domain in mammalian SYT polypeptides. A Met-rich domain is typically comprised within the first half of the QG-rich (from the N-term to the C-term) in plants or QPGY-rich in mammals. A second Met-rich domain may precede the SNH domain in plant SYT polypeptides

**Fig. 6** shows a multiple alignment of the N-terminal end of several SYT polypeptides, using VNTI AlignX multiple alignment program, based on a modified ClustalW algorithm (InforMax, Bethesda, MD, http://www.informaxinc.com), with default settings for gap opening penalty of 10 and a gap extension of 0.05). The SNH domain is boxed across the plant and human SYT polypeptides. The last line in the alignment consists of a consensus sequence derived from the aligned sequences.

**Fig. 7** shows a multiple alignment of several plant SYT polypeptides, using VNTI AlignX multiple alignment program, based on a modified ClustalW algorithm (InforMax, Bethesda, MD, http://www.informaxinc.com), with default settings for gap opening penalty of 10 and a gap extension of 0.05). The two main domains, from N-terminal to C-terminal, are boxed and identified as SNH domain and the Met-rich/QG-rich domain. Additionally, the N-terminal Met-rich domain is also boxed, and the positions of SEQ ID NO: 90 and SEQ ID NO 91 are underlined in bold.

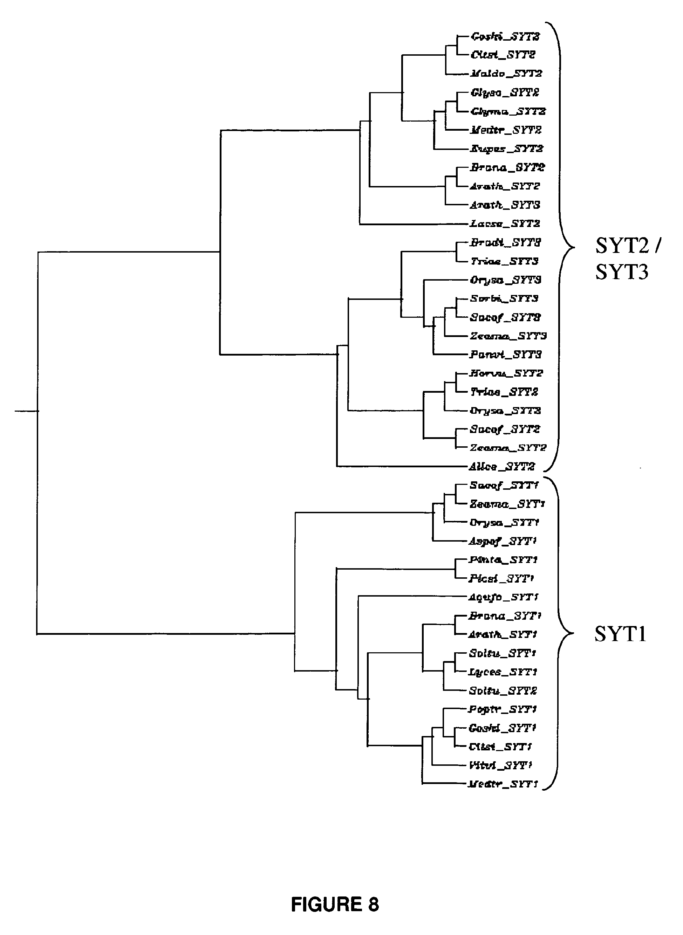

**Fig. 8** shows a Neighbour joining tree resulting from the alignment of multiple SYT polypeptides using CLUSTALW 1.83 (http://align.genome.jp/sit-bin/clustalw). The SYT1 and SYT2/SYT3 clades are identified with brackets.

**Fig. 9** shows a binary vector p0523, for expression in *Oryza sativa* of an *Arabidopsis thaliana* AtSYT1 under the control of a GOS2 promoter (internal reference PRO0129)

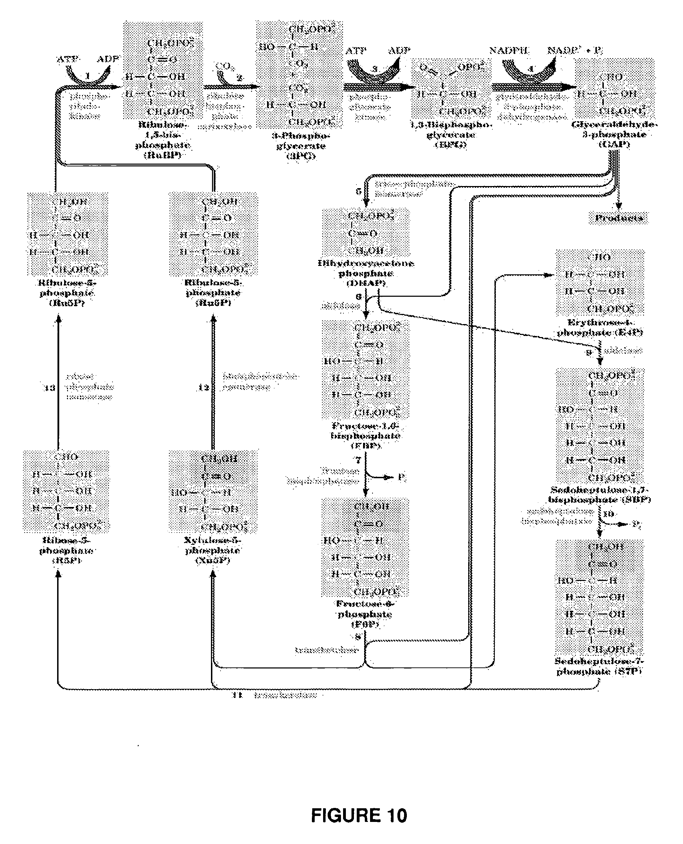

**Figure 10** is an overview of the Calvin cycle. The thirteen enzymatic reactions are shown, as well as the enzyme names that perform these reactions. The black arrow shows the position of cpFBPase in the cycle.

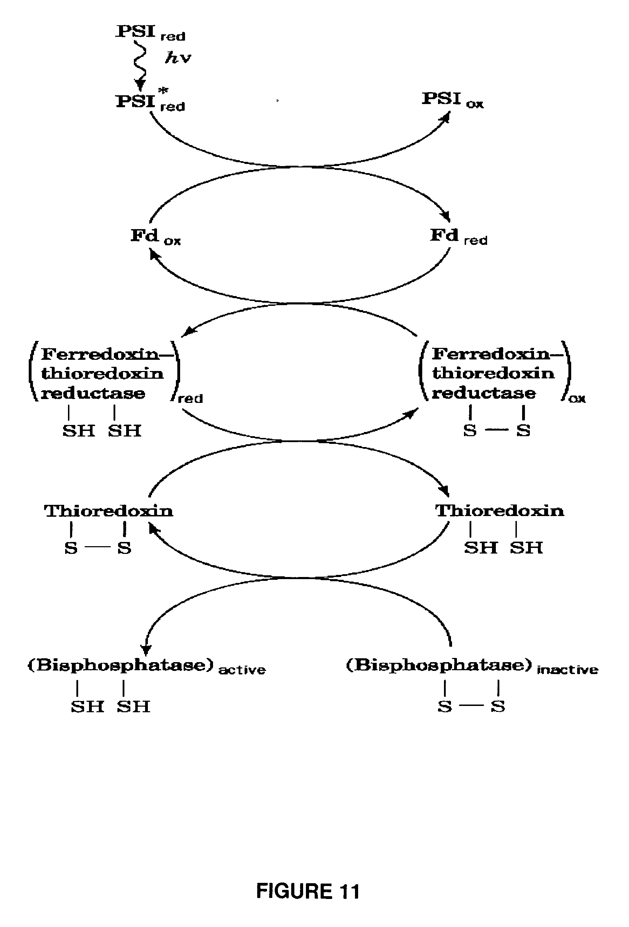

**Figure 11** is a scheme showing the light-activation of cpFBPase via the ferredoxin-thioredoxin system.

**Figure 12** is an alignment of cyFBPase polypeptides and cpFBPase polypeptides. The polypeptide sequences were aligned using AlignX program from Vector NTI suite (InforMax, Bethesda, MD). Multiple alignment was done with a gap opening penalty of 10 and a gap extension of 0.01. The predicted chloroplastic transit peptide of the cpFBPase polypeptides is boxed. The redox regulatory insertion and the cysteines involved in disulfide bridge formation are indicated. Finally, the active site region is also boxed, and the amino acid residues Asn237, Tyr269, Tyr289 and Arg268 which bind the 6-phosphate of fructose-1,6-bisphosphate, and Lys299 which binds the fructose are shown.

**Figure 13** shows a binary vector p1597, for increased expression in *Oryza sativa* of a *Chlamydomonas reindhardtii* nucleic acid encoding a cpFBPase polypeptide under the control of a GOS2 promoter (internal reference PRO0129).

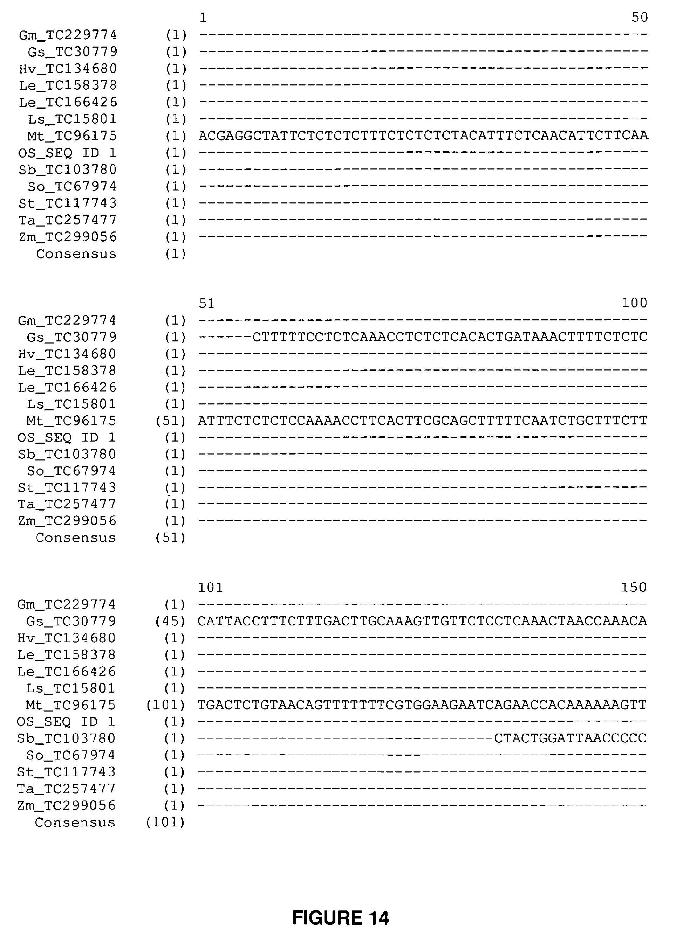

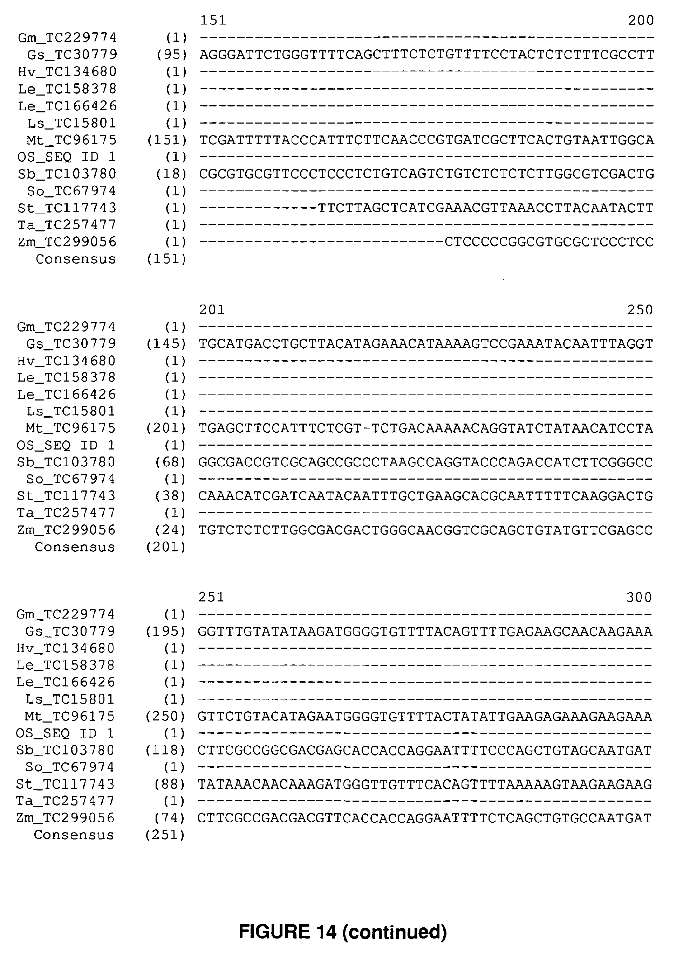

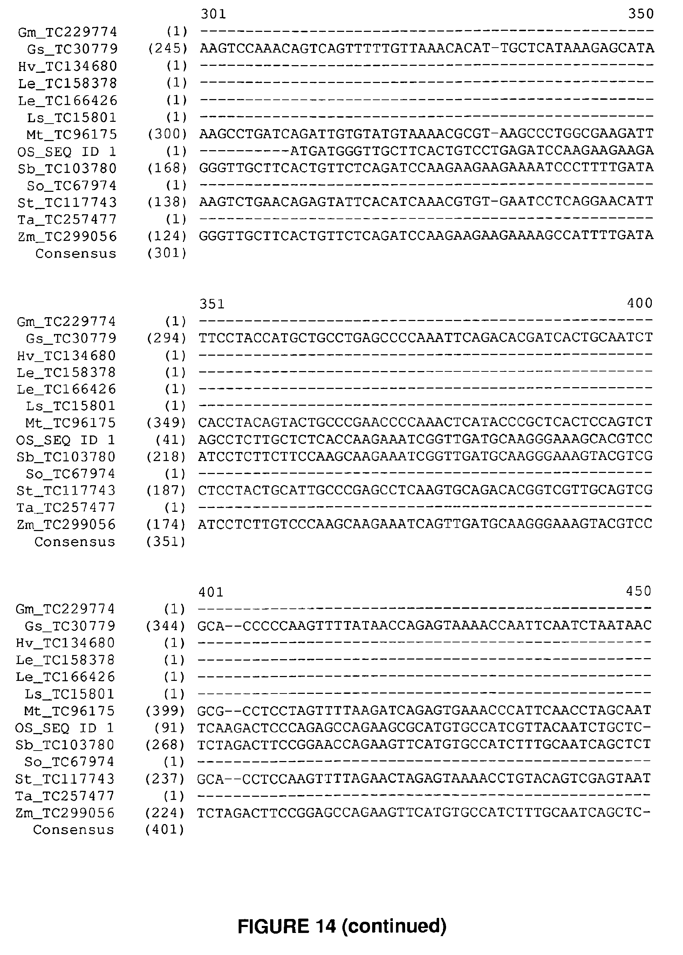

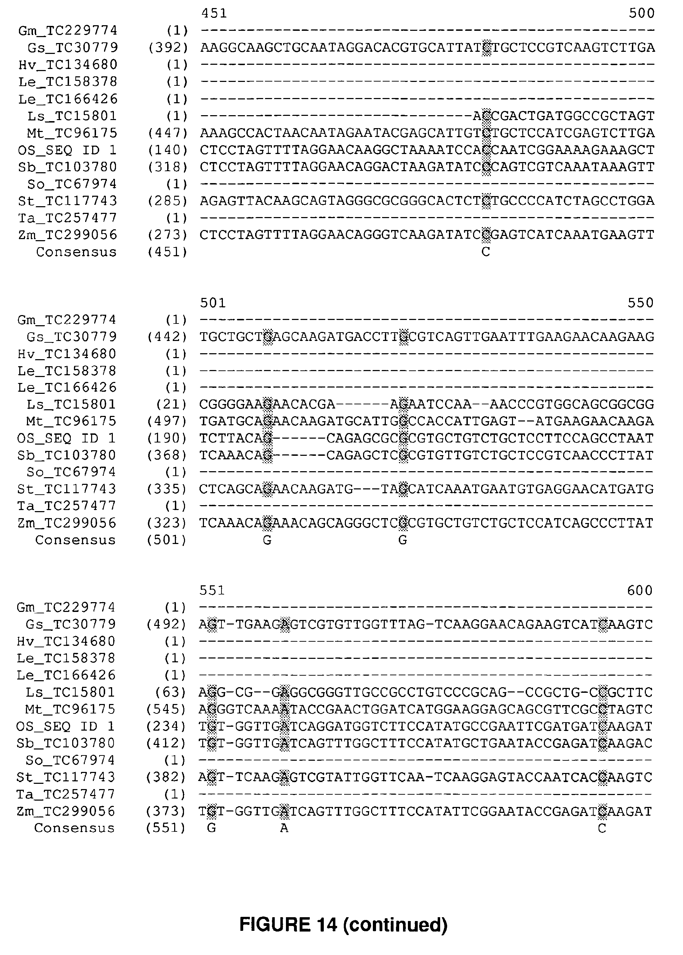

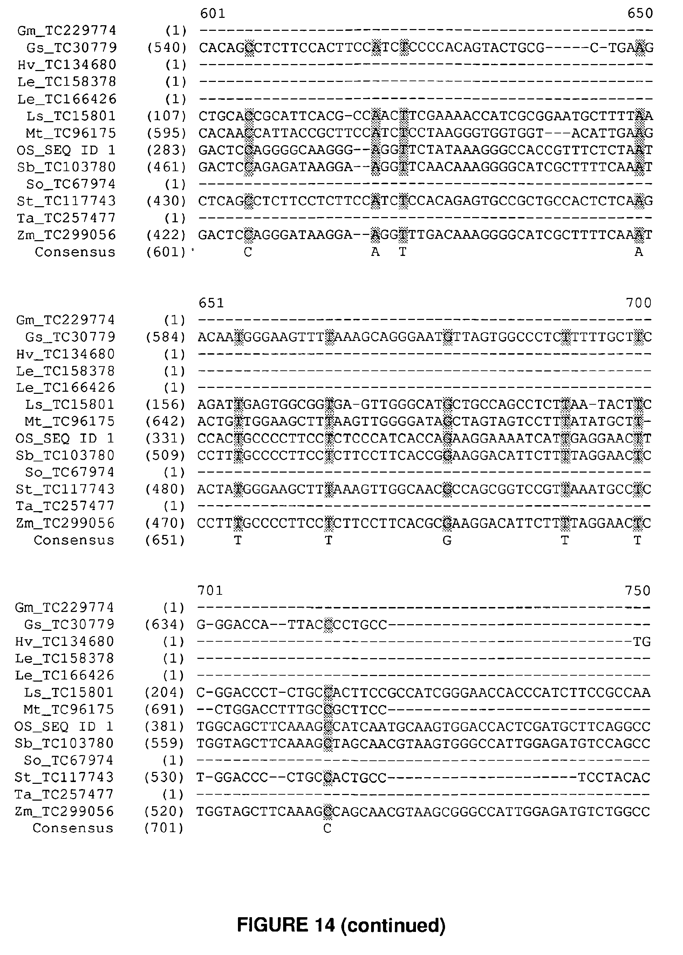

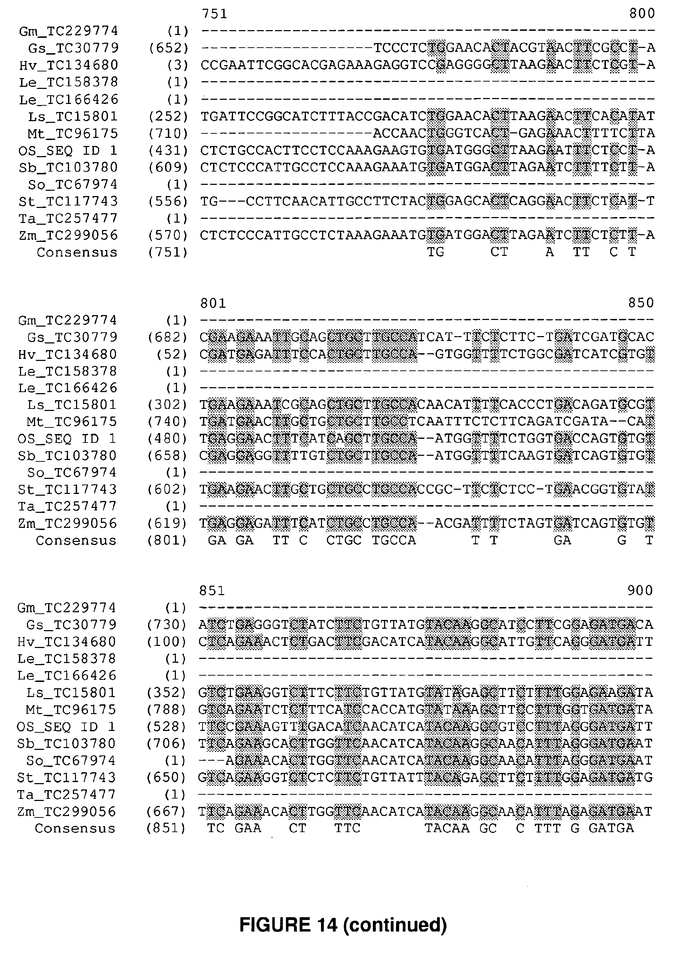

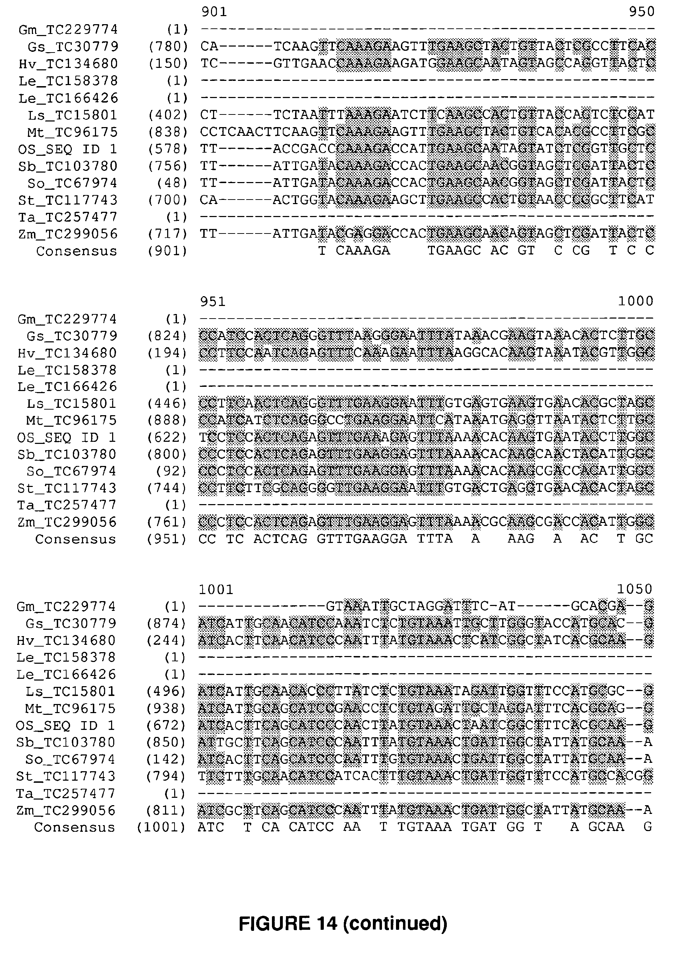

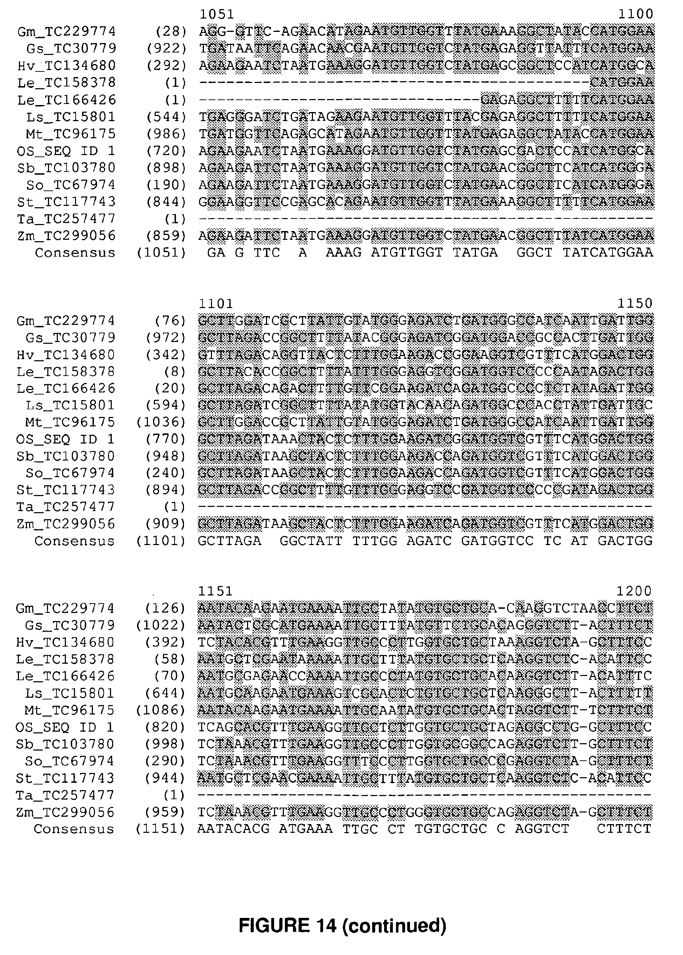

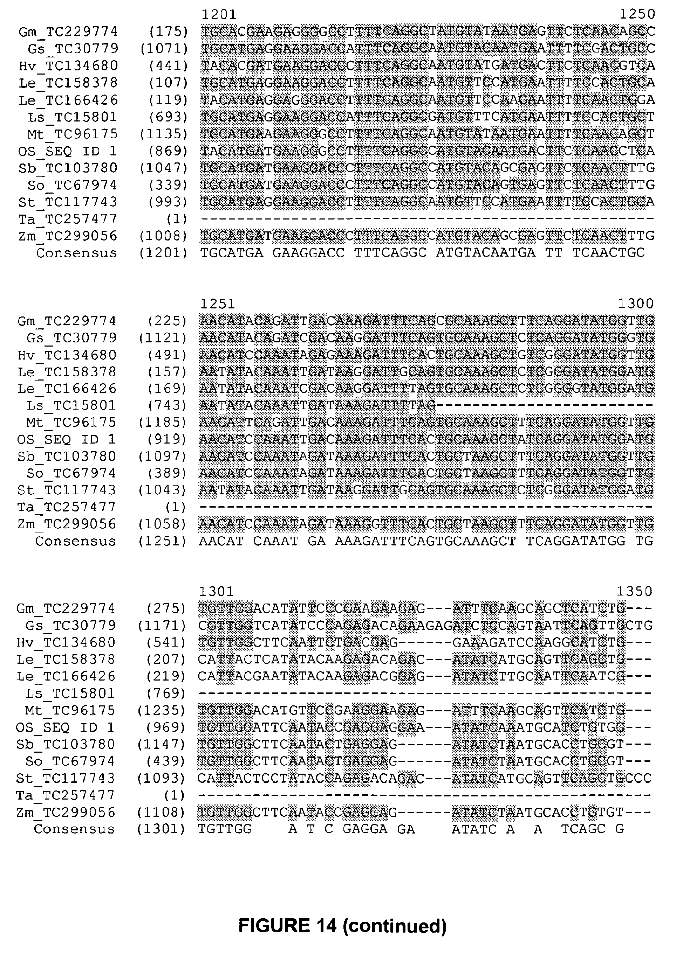

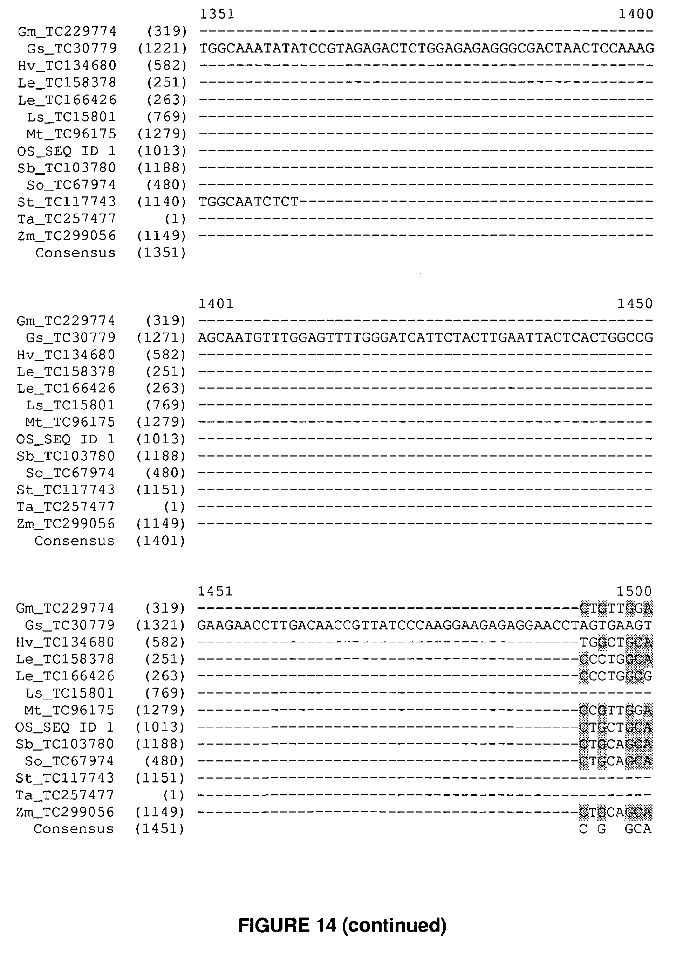

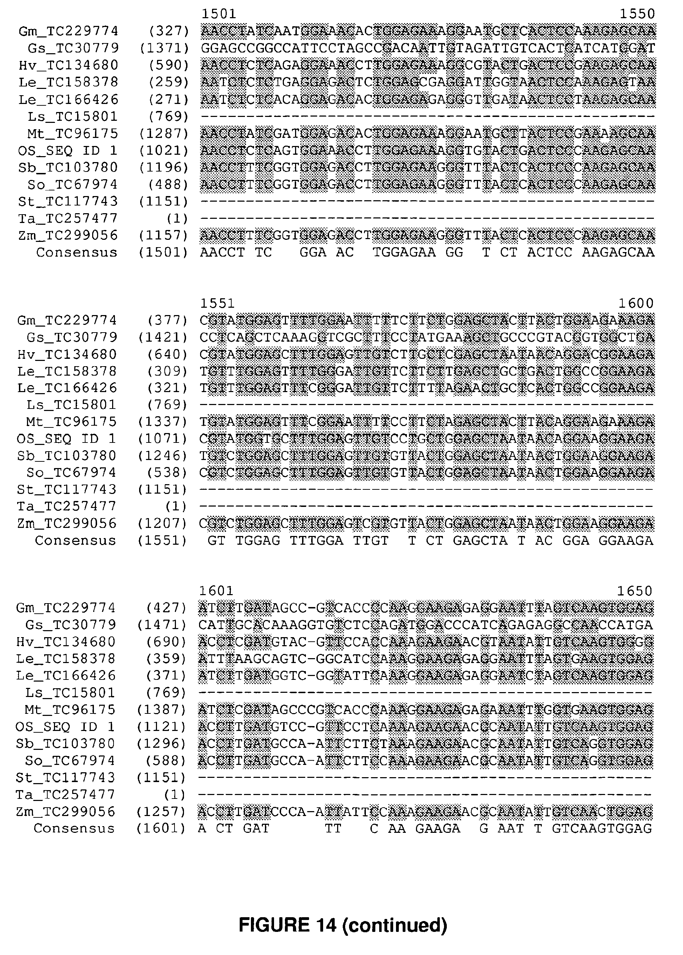

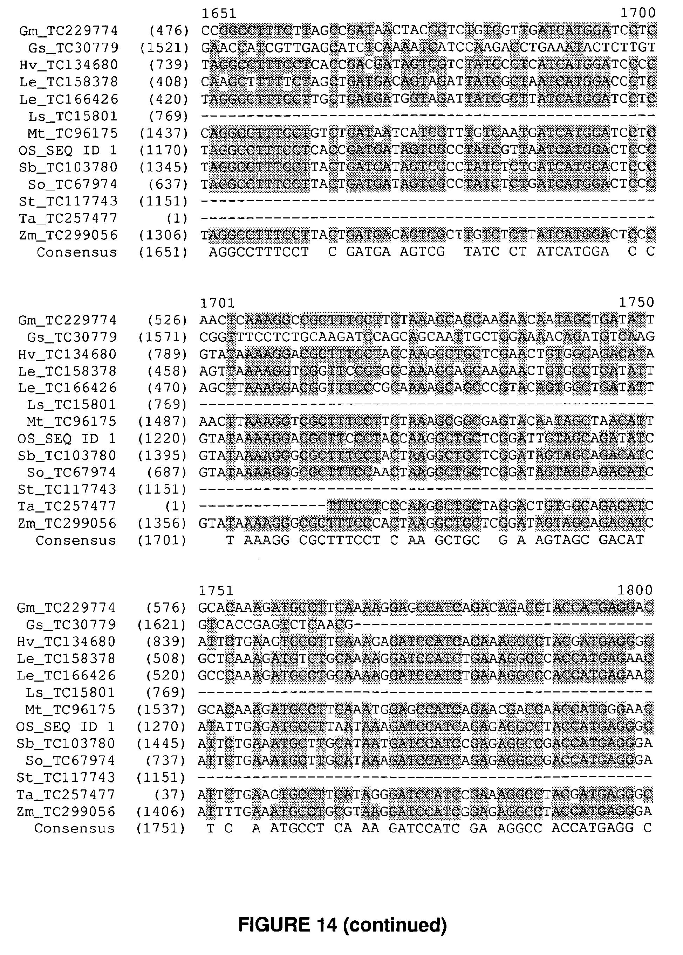

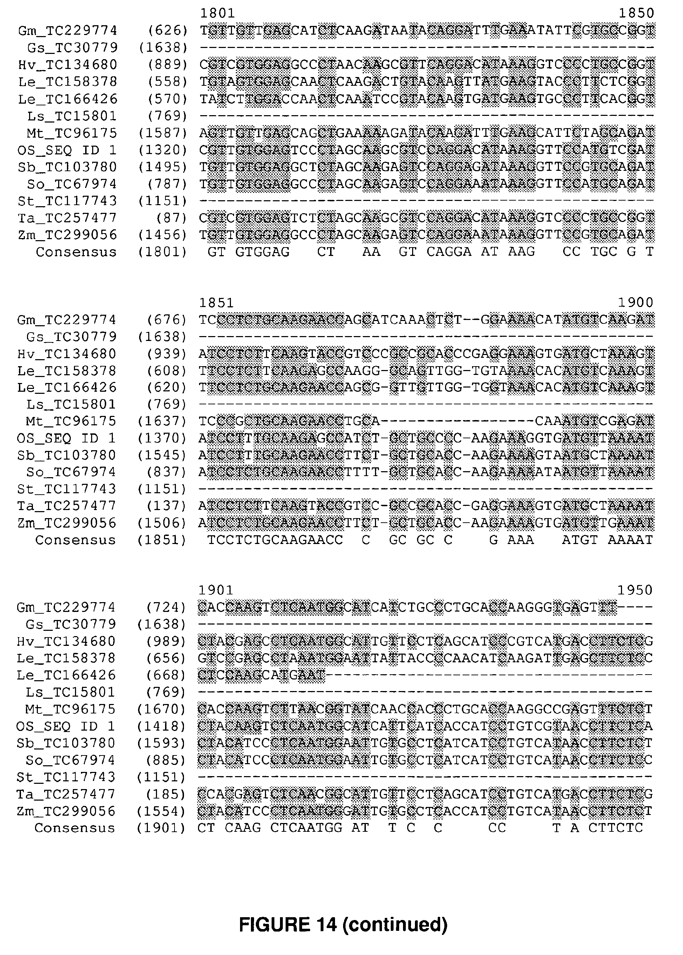

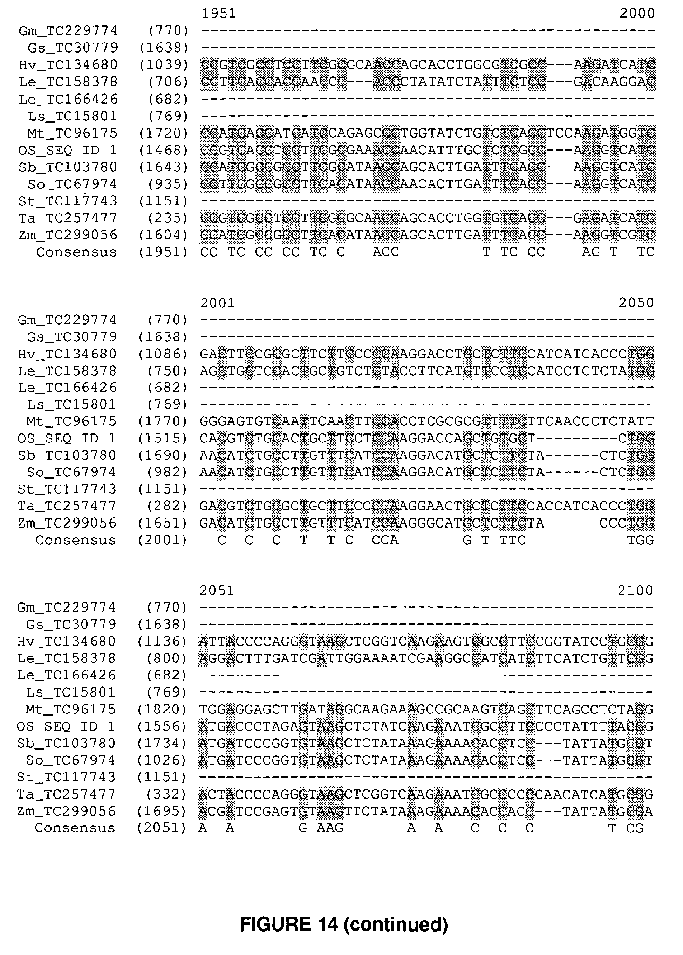

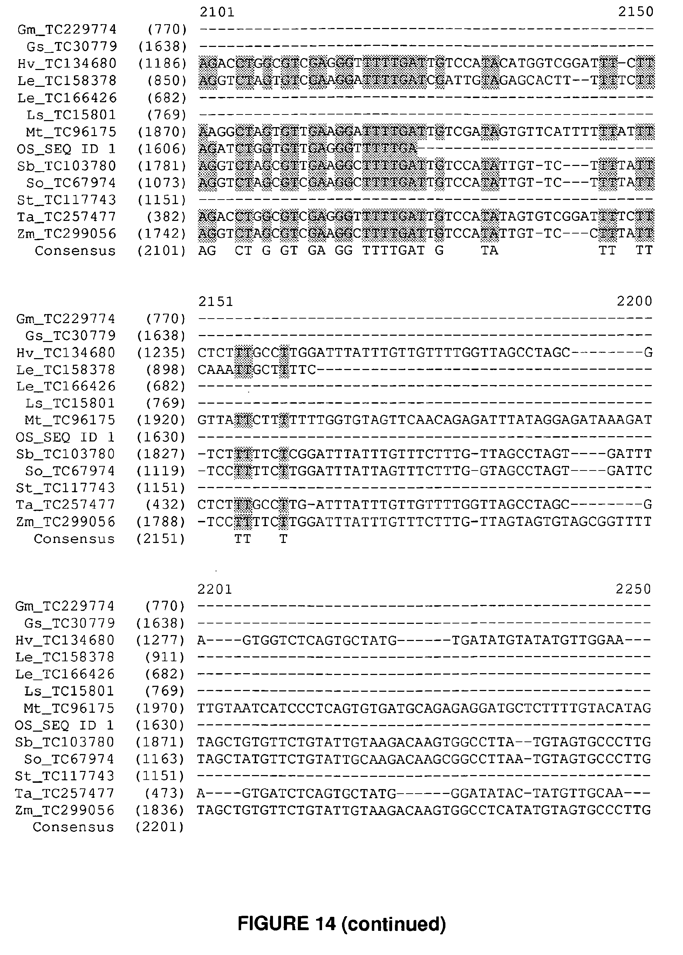

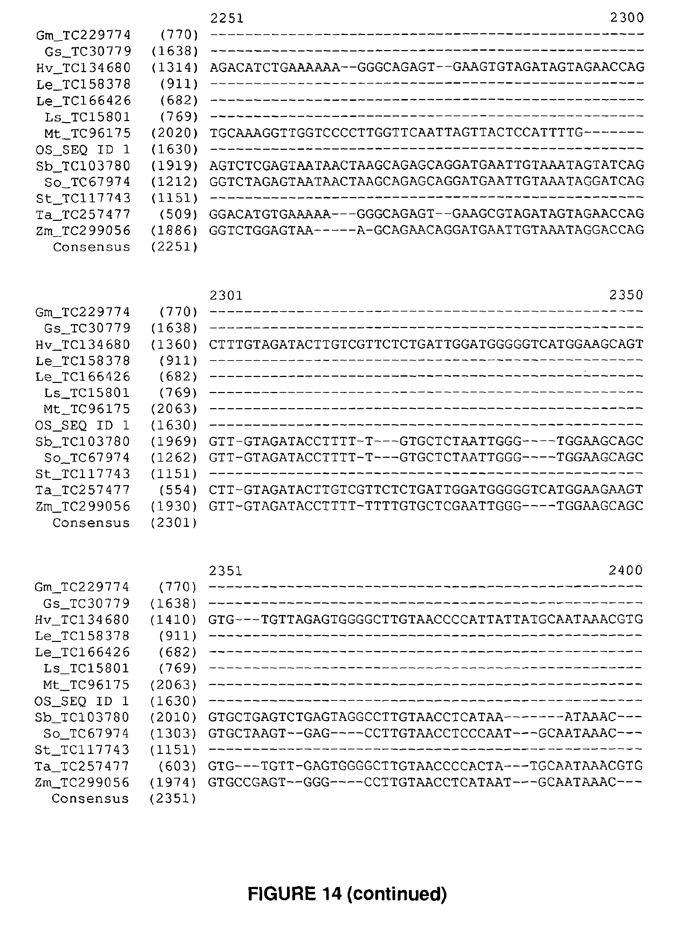

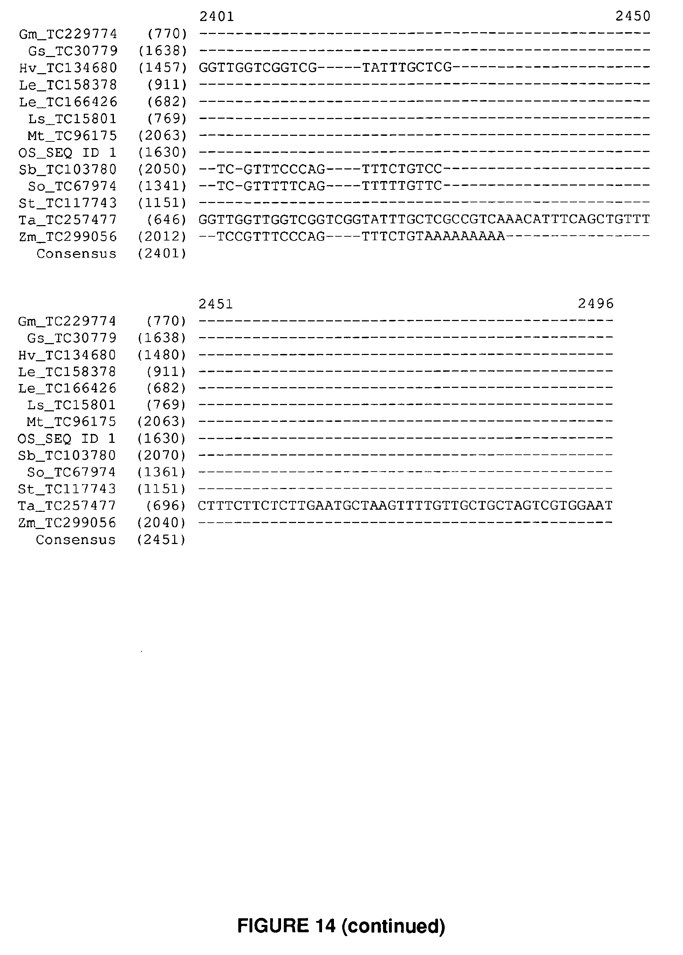

**Fig. 14** is an alignment of SIK nucleic acids encoding putative SIK orthologues described in Table 2.

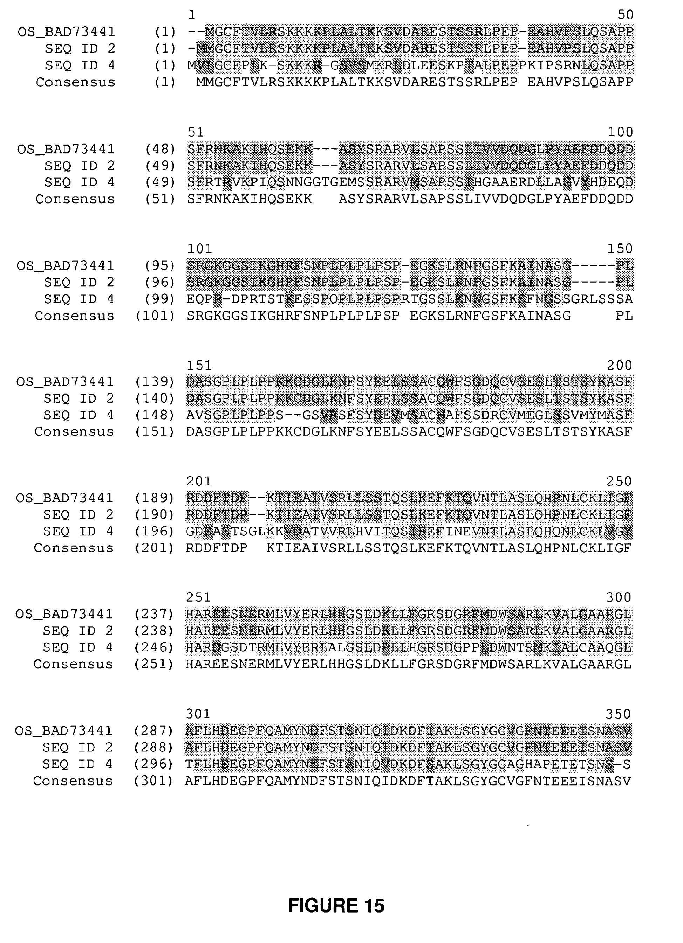

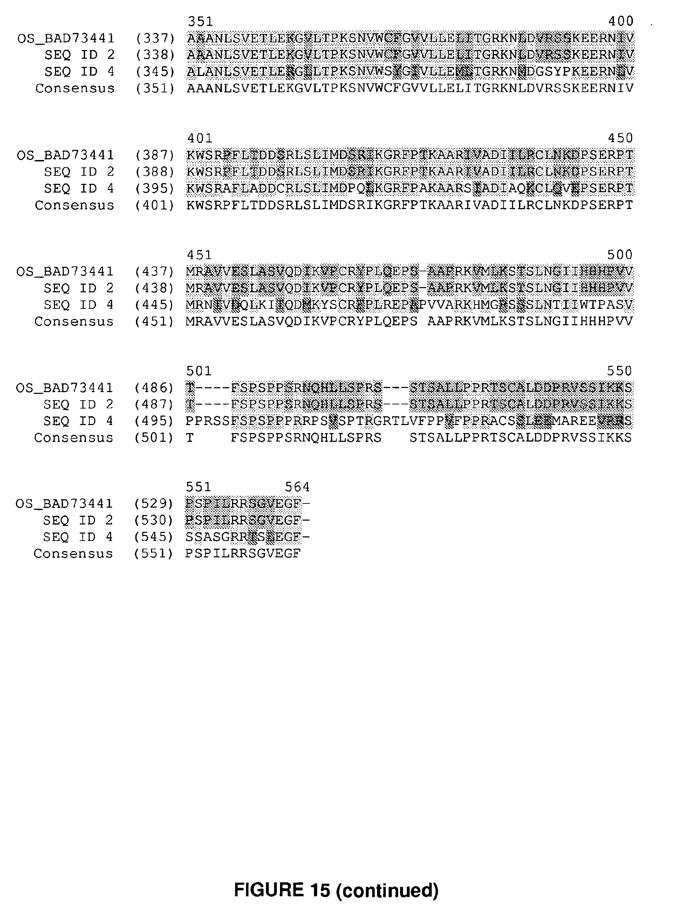

**Fig. 15** is an alignment of rice SIK polypeptide (SEQ ID NO: 2) and *Arabidopsis* SIK polypeptide (SEQ ID NO: 4) and OSSIK polypeptide having NCBI accession number OS_BAD73441.

**Fig. 16** represents the binary for vector endogenous gene silencing in *Oryza sativa* using a SIK nucleic acid represented by SEQ ID NO: 1 using a hairpin construct under the control of a constitutive promoter, GOS2.

**Fig. 17** shows a binary vector for expression in *Oryza sativa* of a SIK-encoding nucleic acid from *Oryza sativa* under the control of a GOS2 promoter.

**Fig. 18** shows a section of a HD-Zip phylogenetic tree showing Class II HD-Zip members.

**Fig. 19** shows an alignment of several Class II HD-Zip polypeptide sequences.

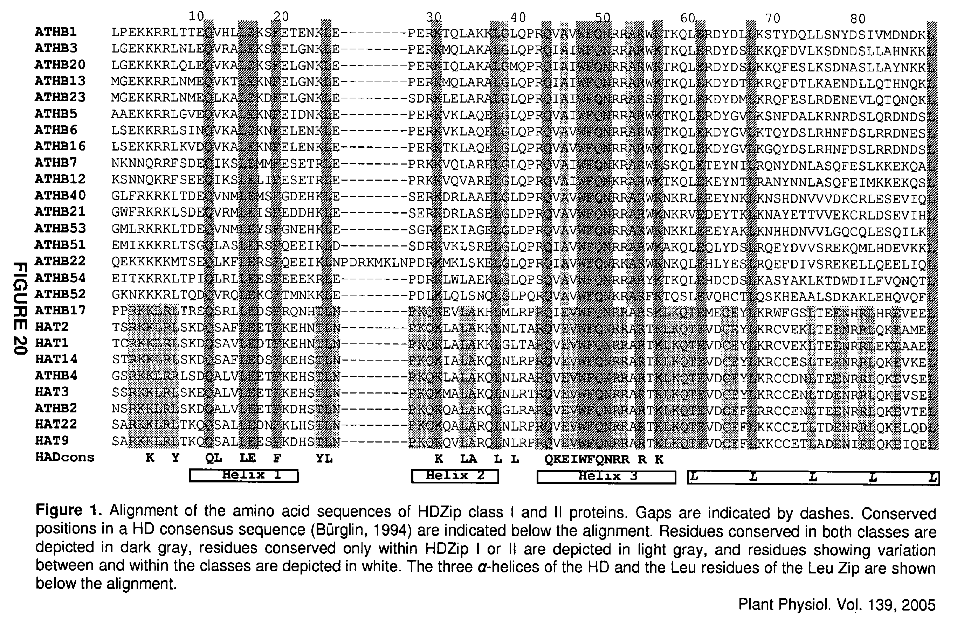

**Fig. 20** is an alignment taken from Henrikson et al., 2005 (Plant Physiol, Vol. 139, pp. 509-518). The Leu Zip domains in all HDZip I and II proteins are in identical positions, C terminal to the HD. The HDZip domains of HDZip I and II proteins are similar to each other in sequence, although a number of amino acid positions distinguish HDZip I from II. The amino acid at position 46 is invariant within the HDZip I and II, but distinct between the classes. Several other amino acids, e.g. the ones at positions 6, 25, 29, 30, 58, and 61, are invariable within the HDZip II and differ from HDZip I amino acids, which show variation at these positions.

**Fig. 21** shows a binary vector for increased expression in *Oryza sativa* of the *Arabidopsis thaliana* Class II HD-Zip transcription factor (HAT4)-encoding nucleic acid under the control of a GOS2 promoter.

**Fig. 22** shows the domain structure of two examples of SYB1 proteins. The Zinc finger domains are indicated in underlined bold.

**Fig. 23a** shows a multiple alignment of various SYB1 proteins (the sequences used are: CDS2671 (SEQ ID NO: 2), AAZ94630 (SEQ ID NO: 4), Q53AV6 (SEQ ID NO: 6), Q6Z6E6 (SEQ ID NO: 8), Q8GWD1 (SEQ ID NO: 10), Q9SW92 (SEQ ID NO: 12), Q8RYZ5 (SEQ ID NO: 14), beta vulgaris (SEQ ID NO: 16), Q8S8K1 (SEQ ID NO: 18), Q8GZ43 (SEQ ID NO: 20), Q7F1K4 (SEQ ID NO: 22), Q7XHQ8 (SEQ ID NO: 24)); Fig. 23b shows a phylogenetic tree in which the black box delineates the group of SYB1 proteins.

**Fig. 24** shows the binary vector for increased expression in Oryza *sativa* of an *Arabidopsis thaliana* SYB1 protein-encoding nucleic acid under the control of a GOS2 promoter (internal reference PRO0129).

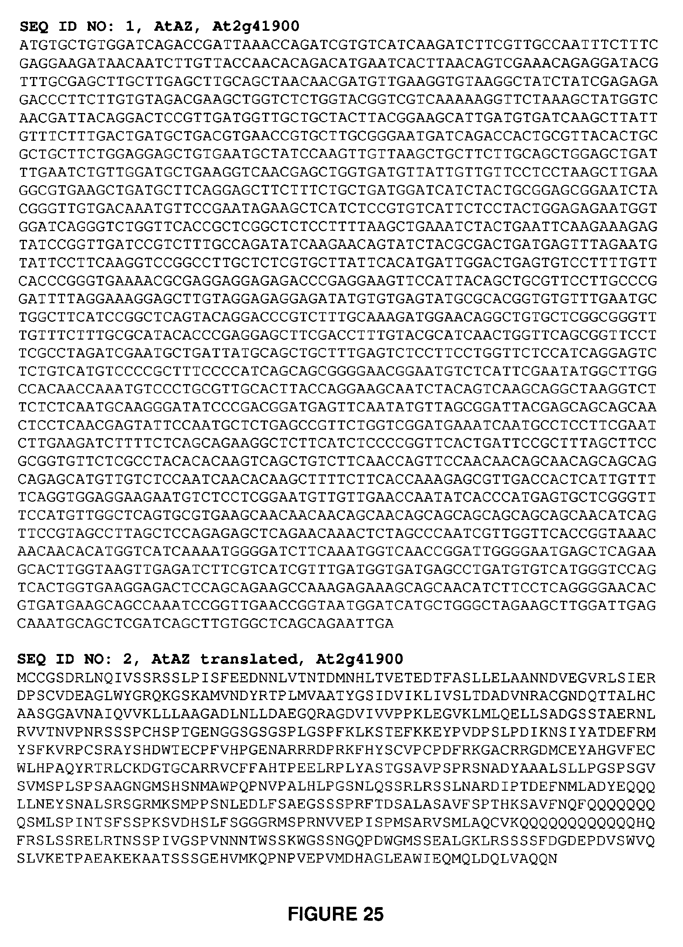

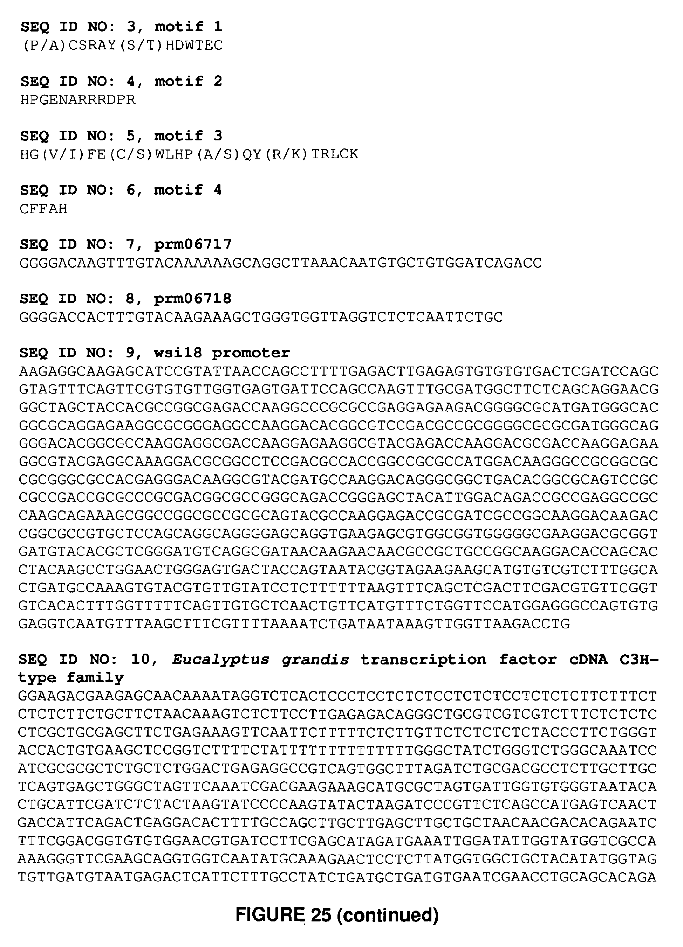

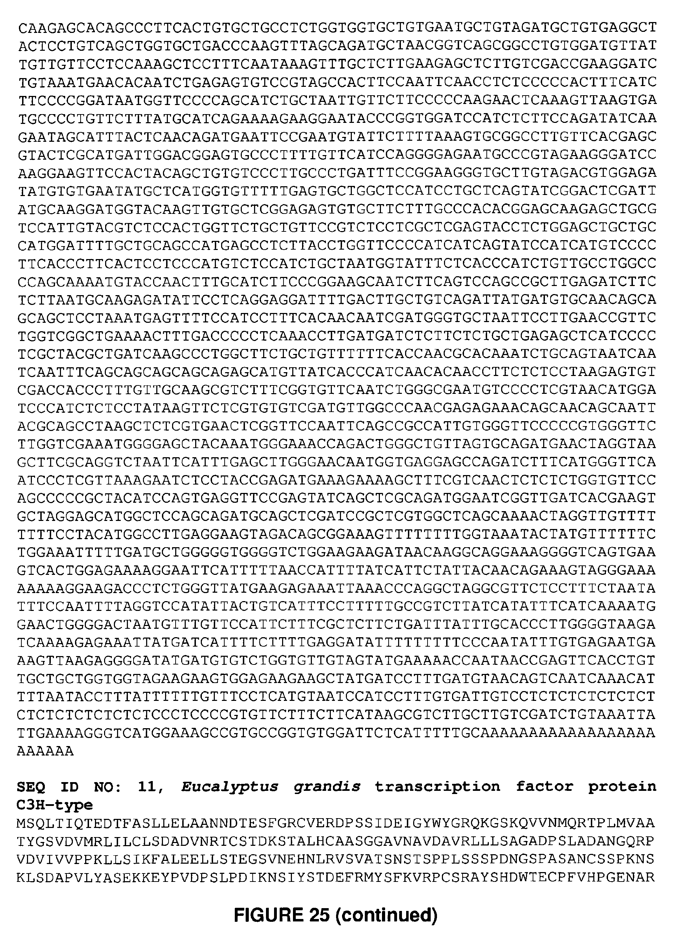

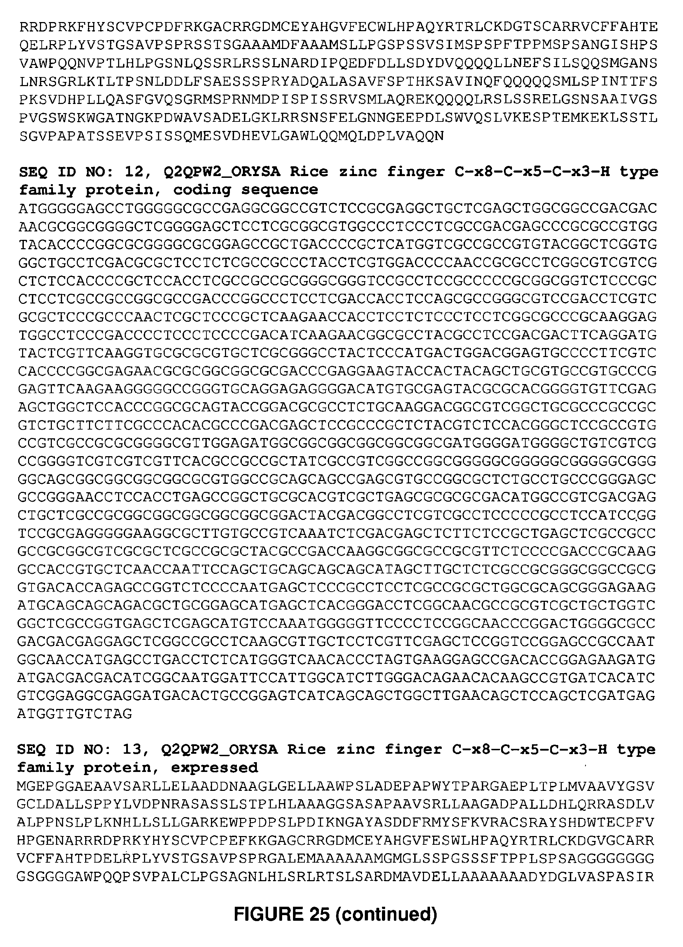

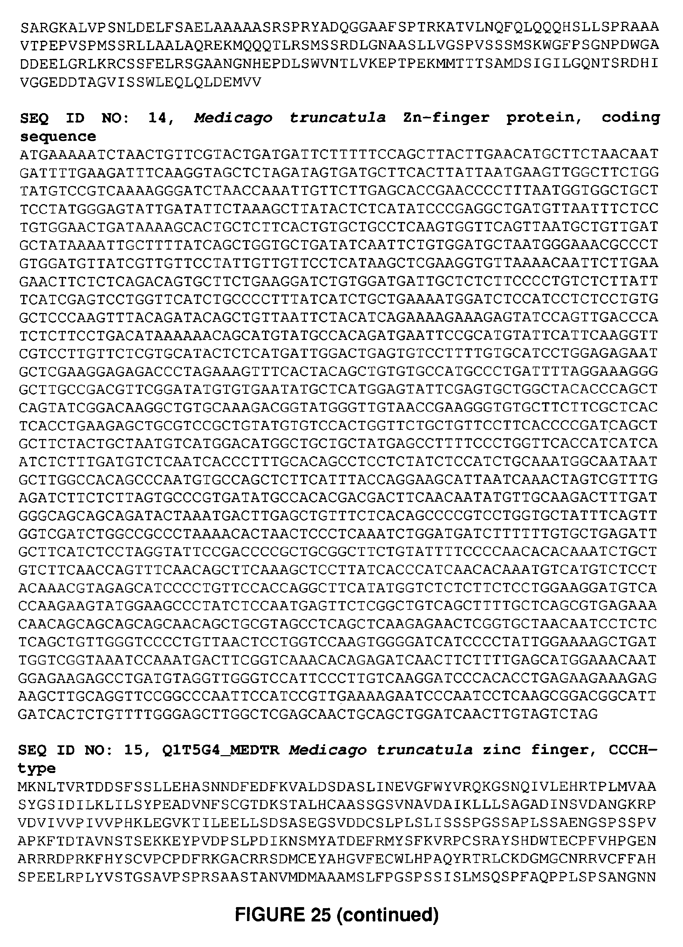

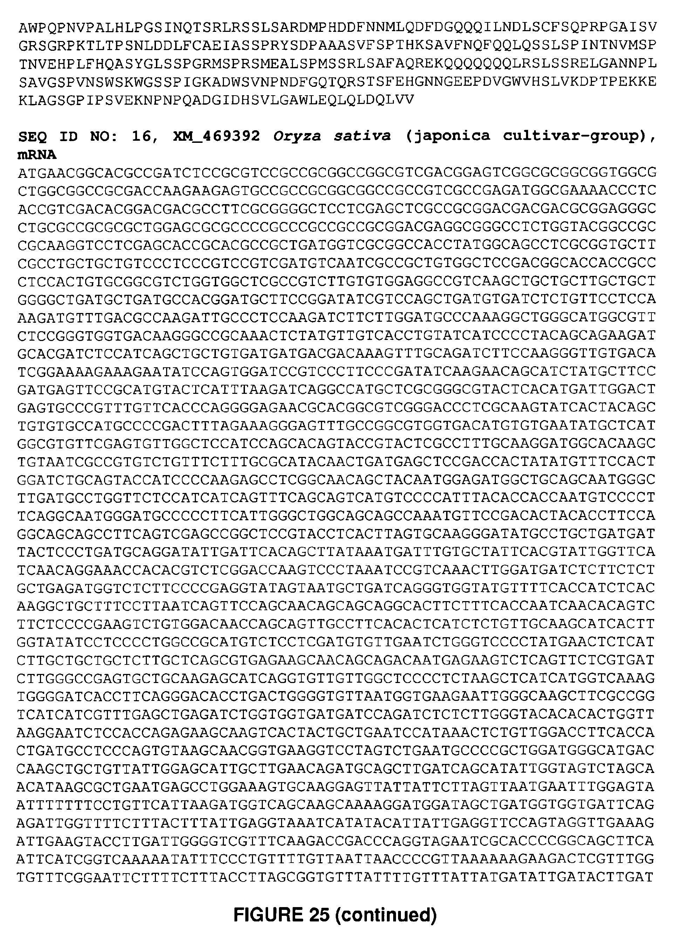

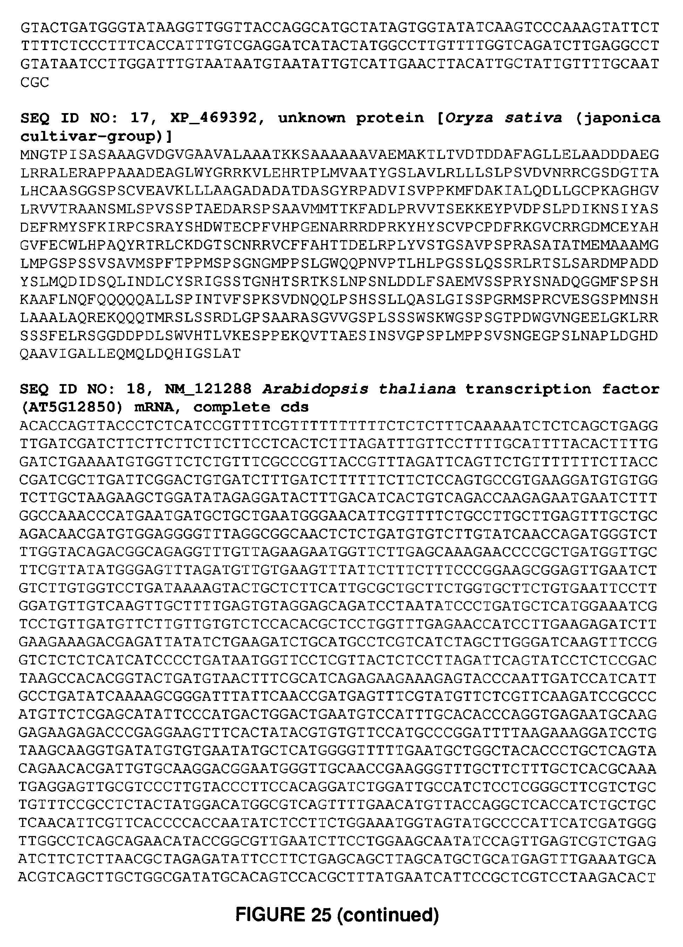

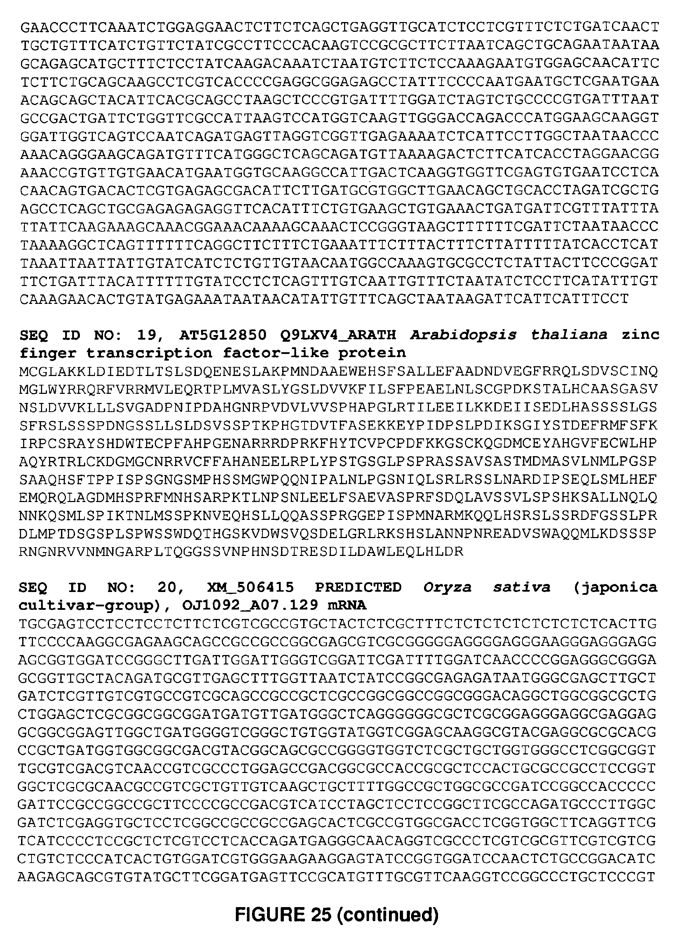

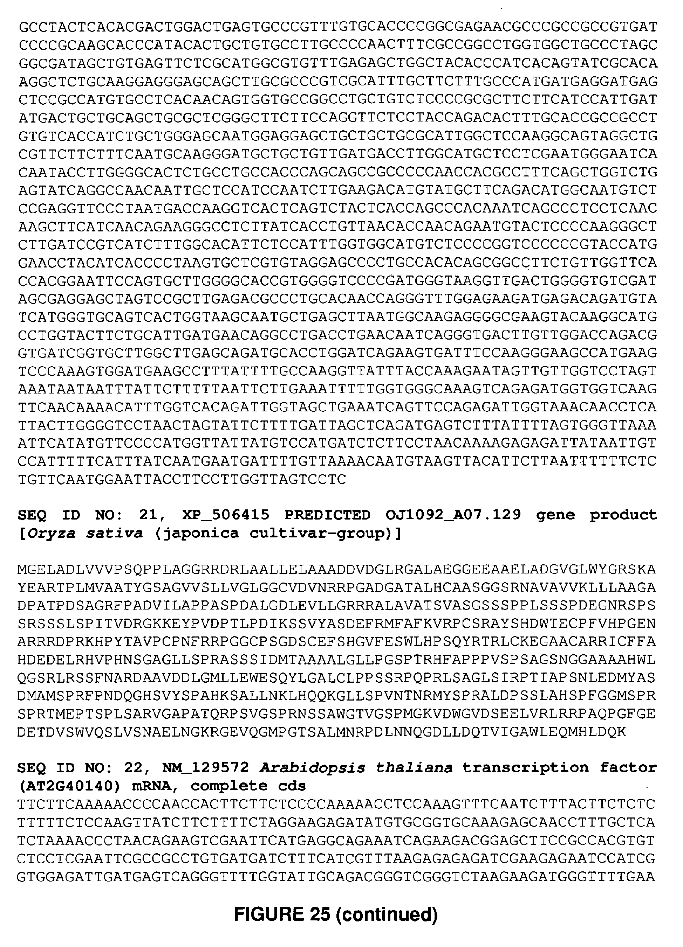

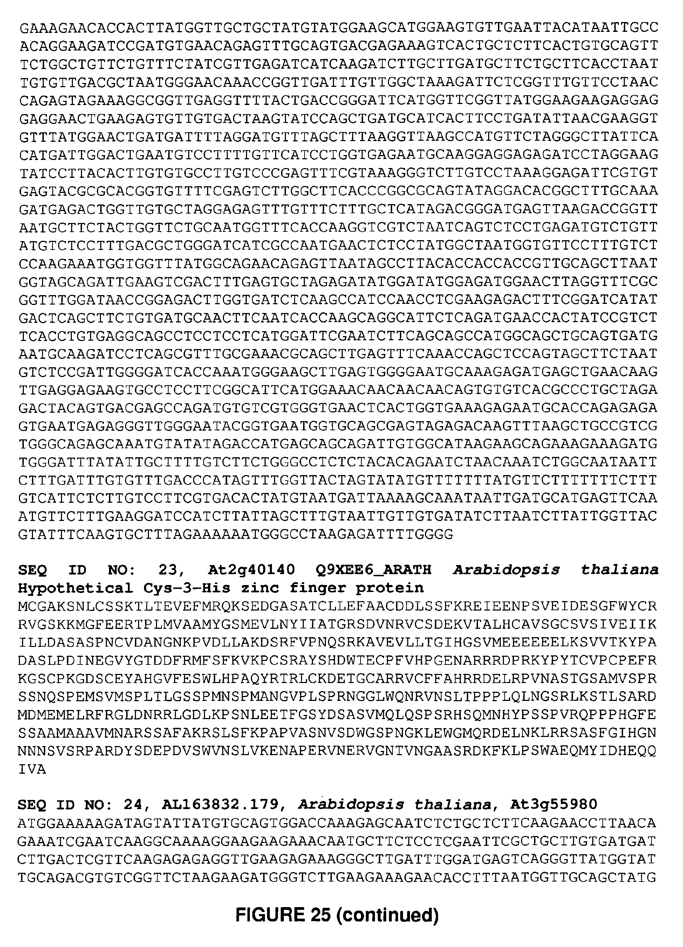

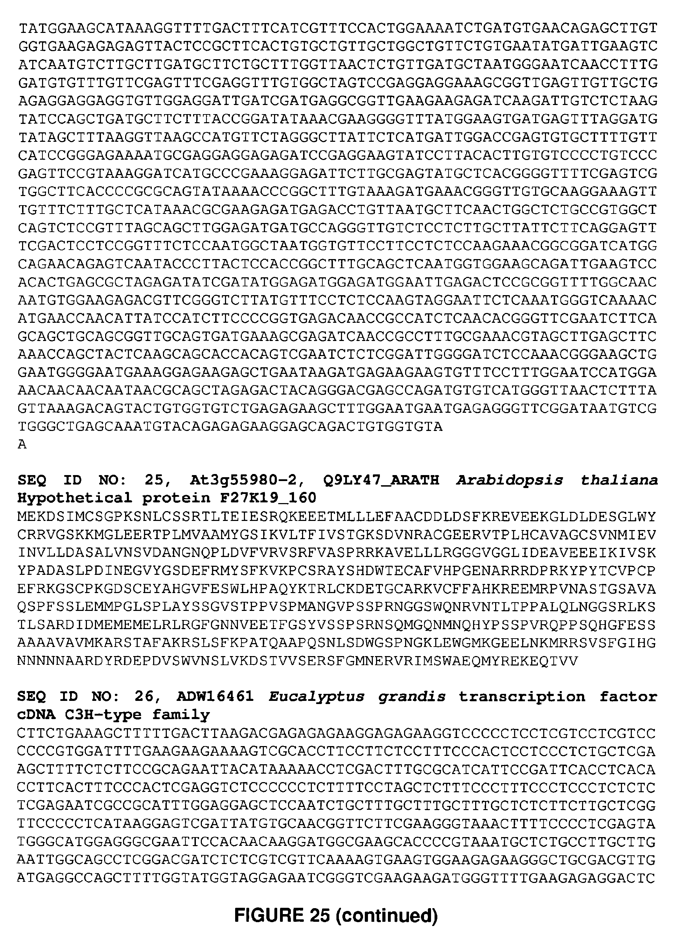

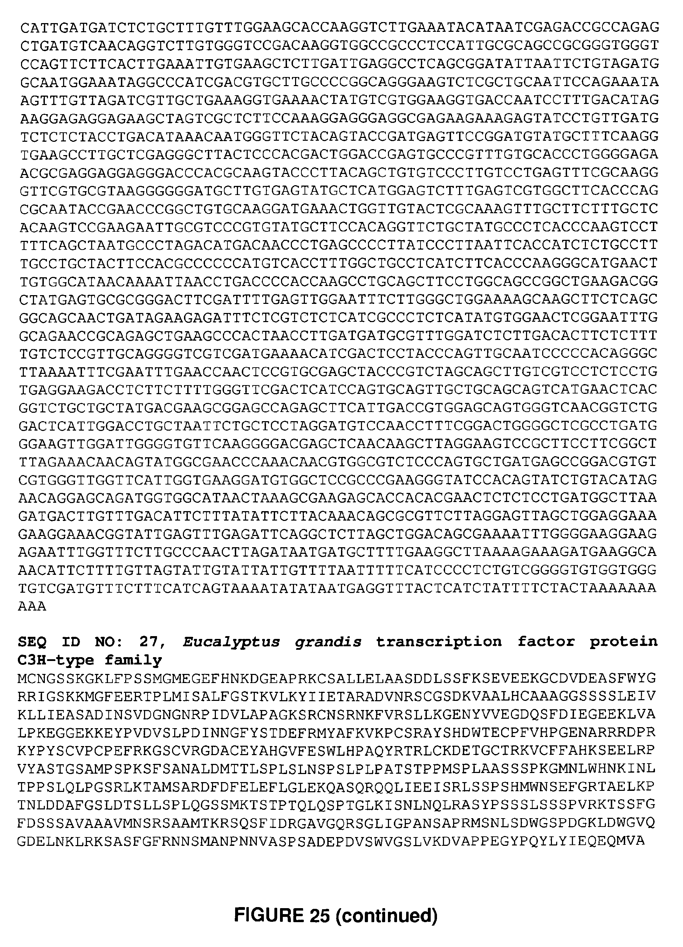

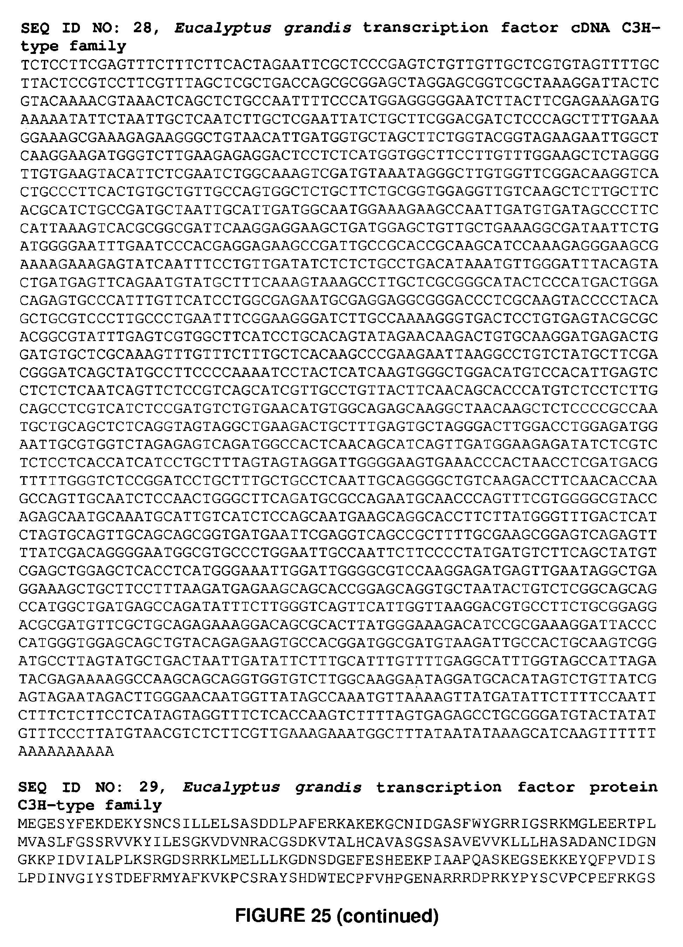

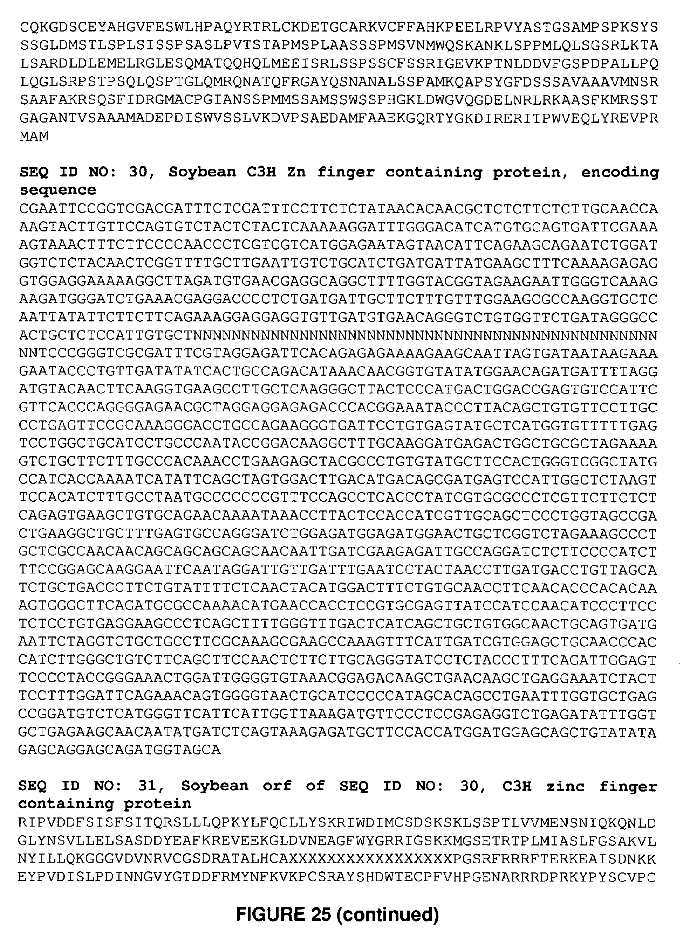

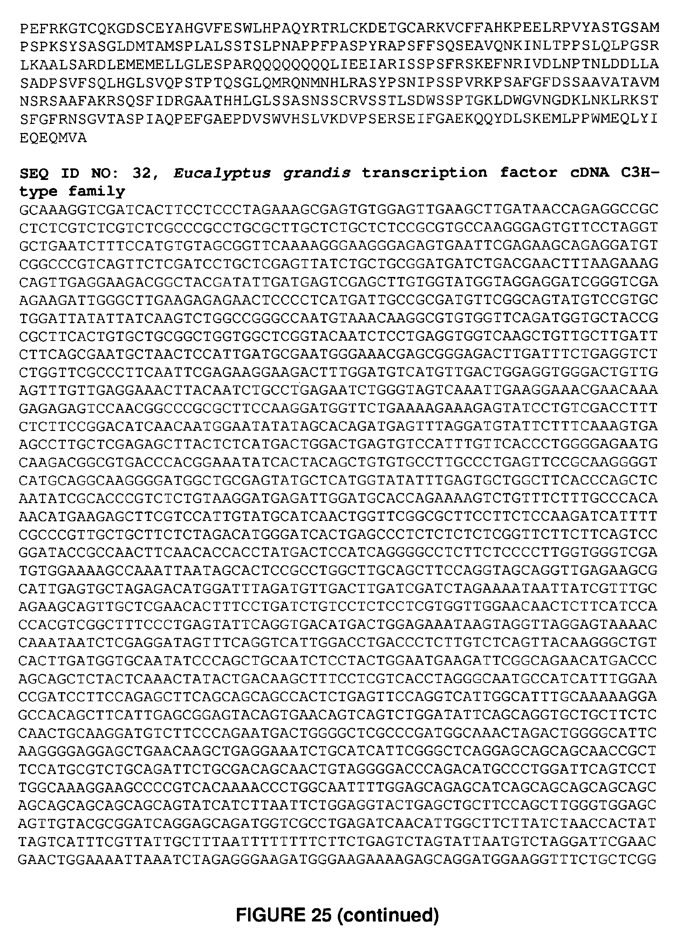

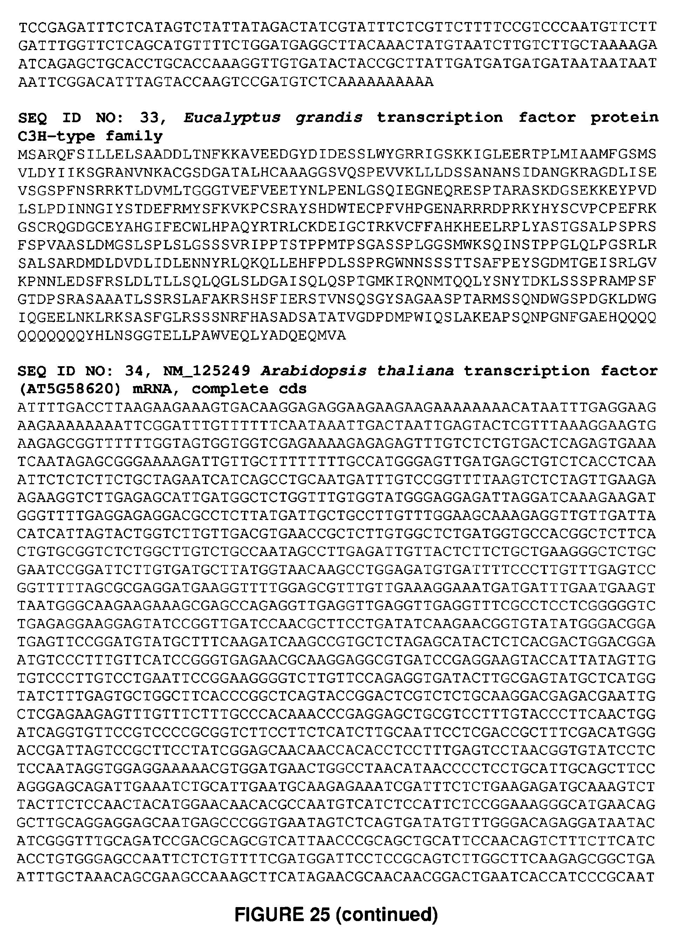

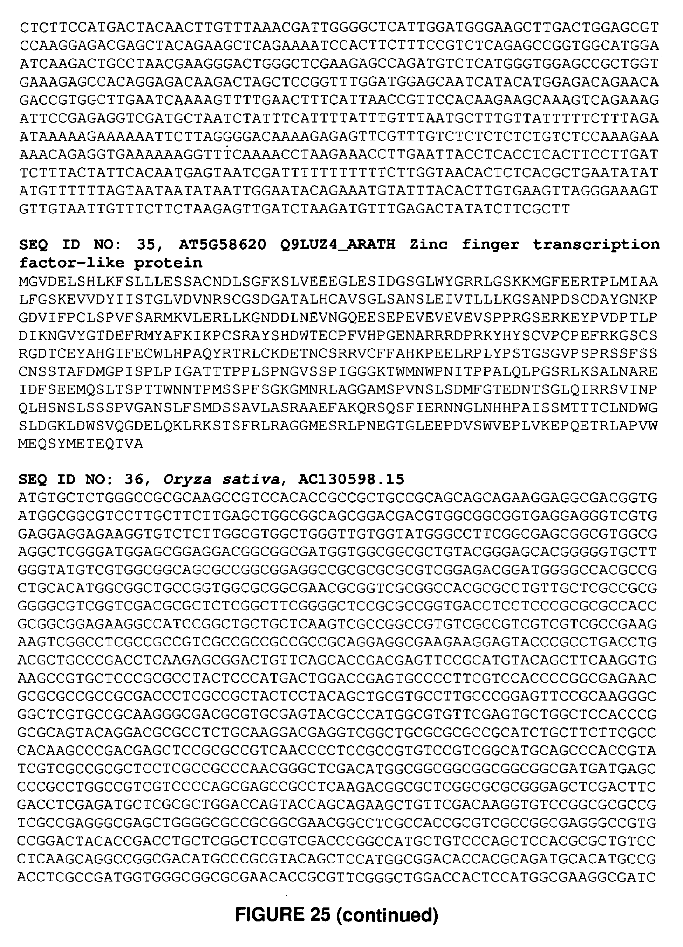

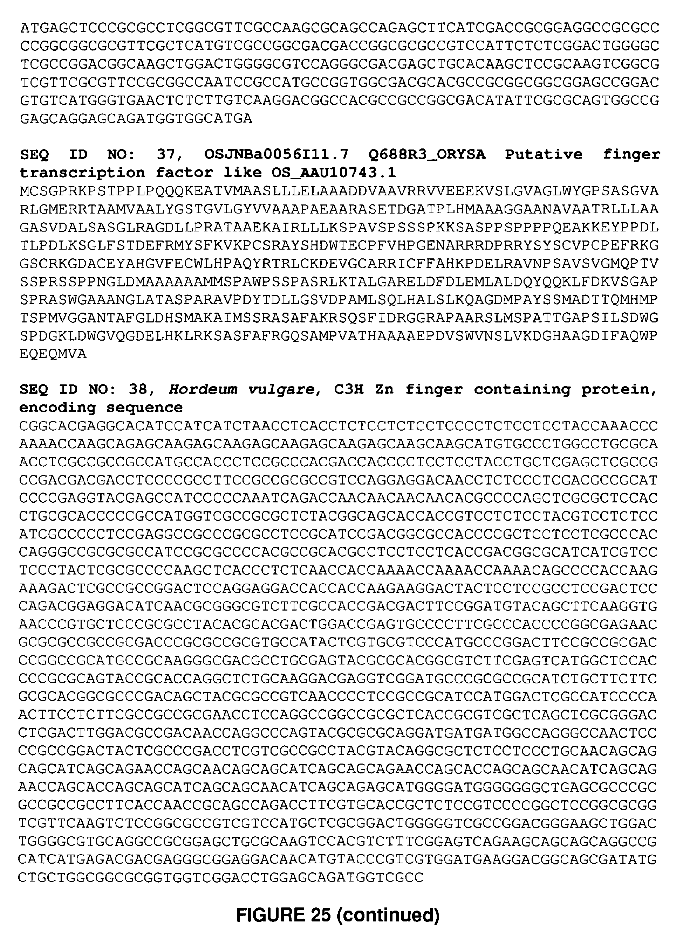

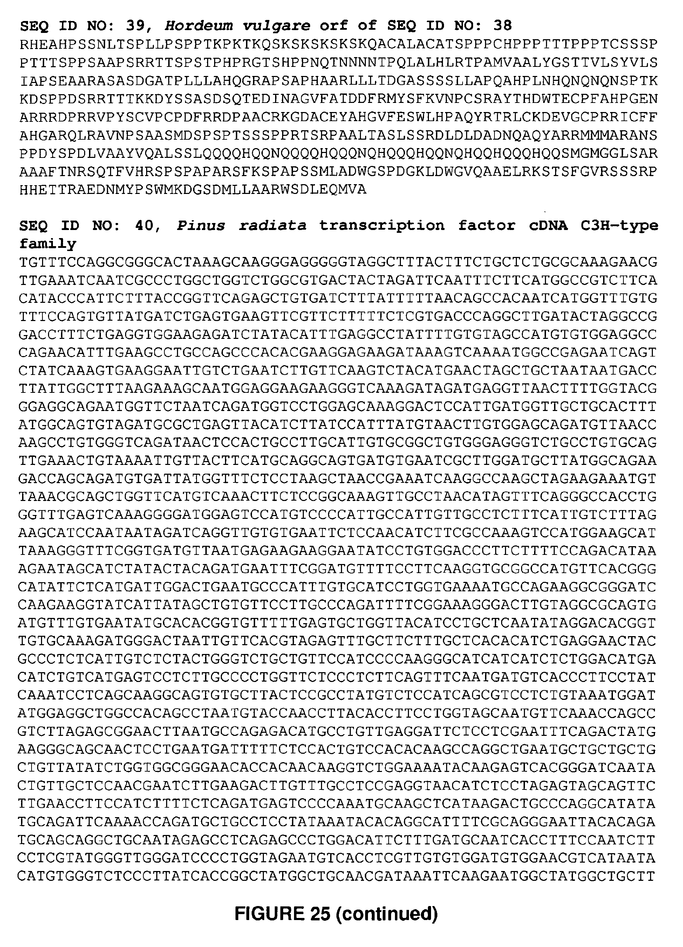

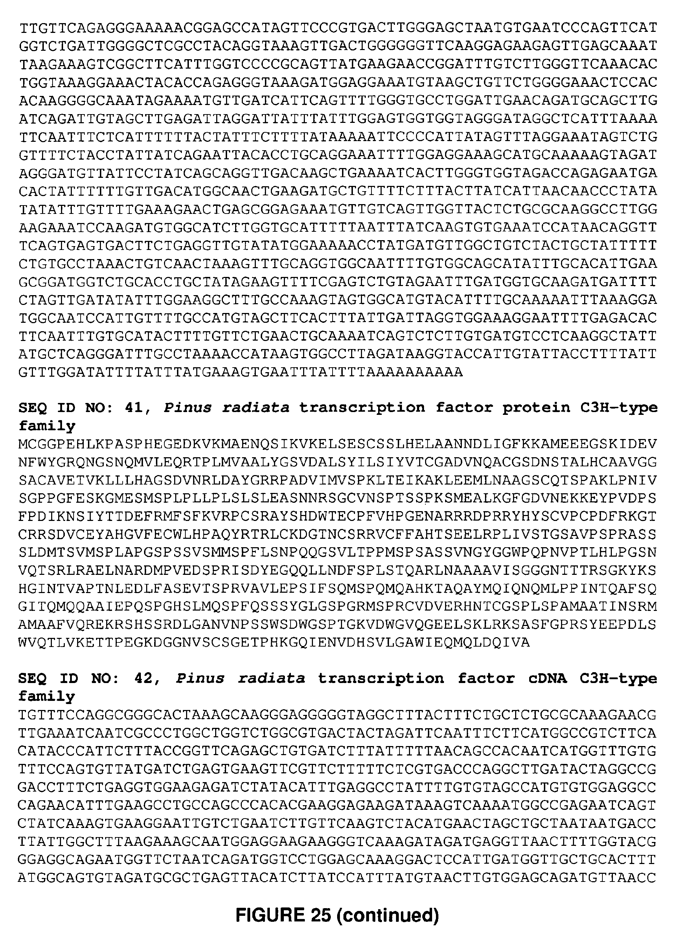

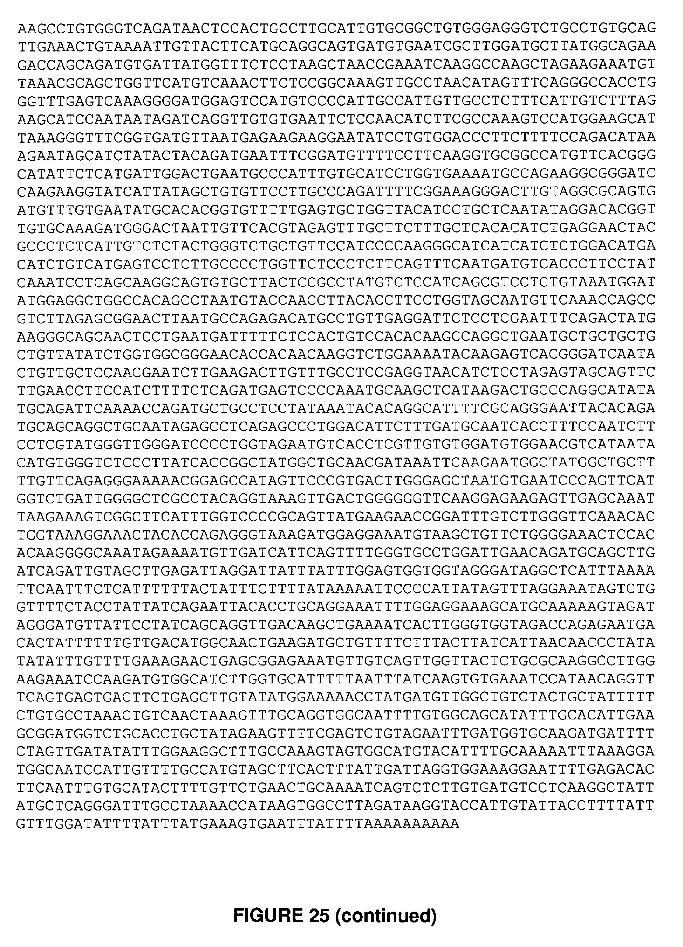

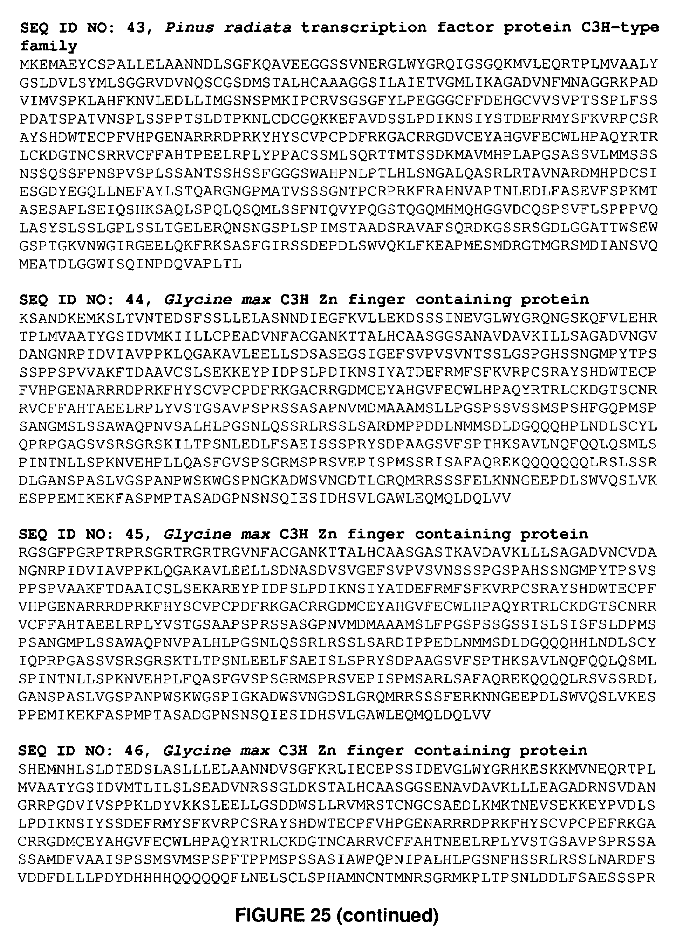

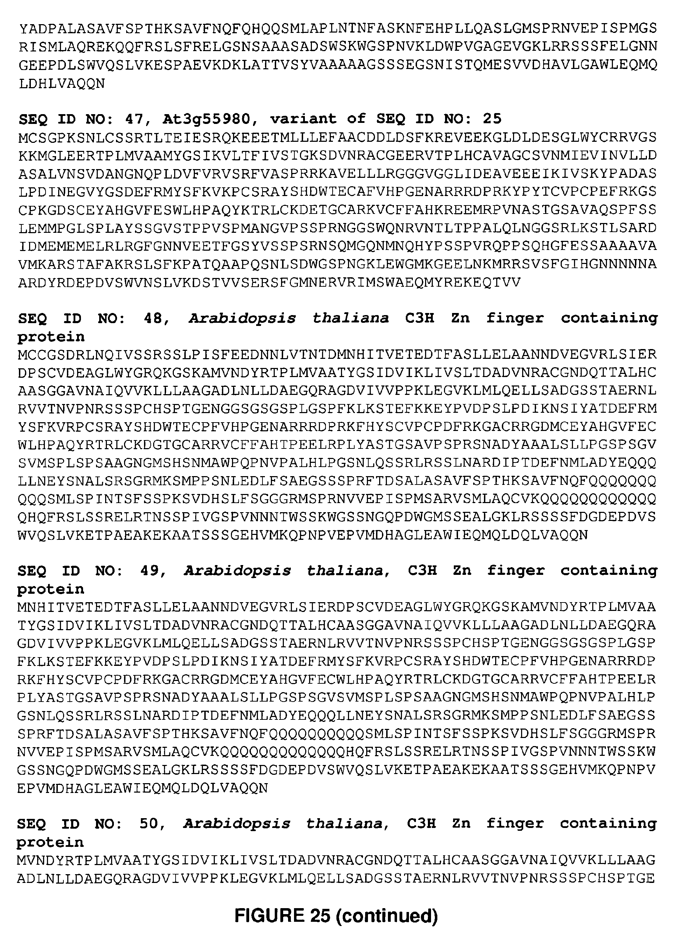

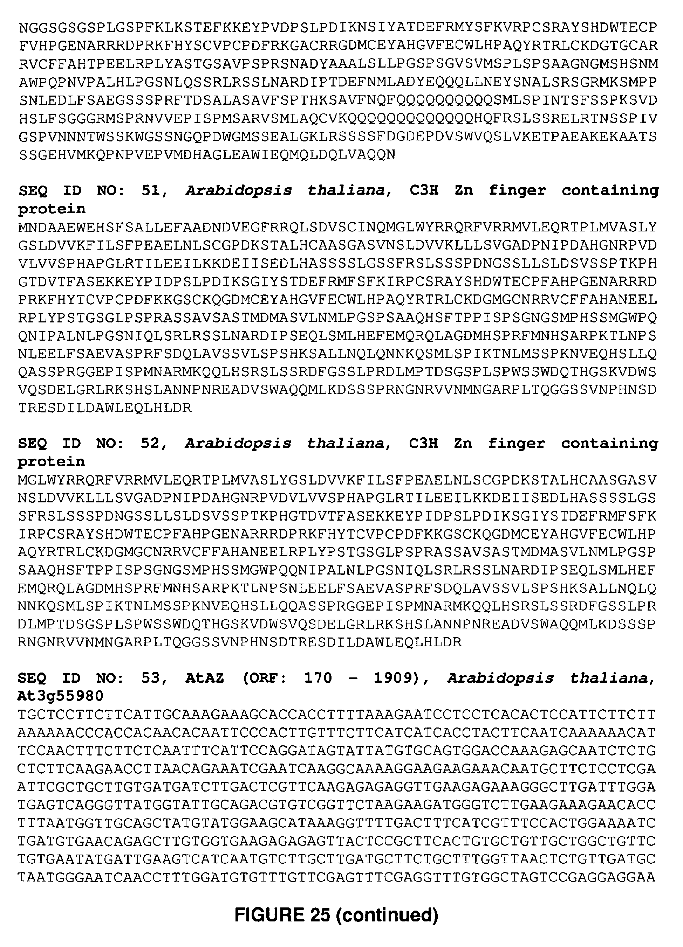

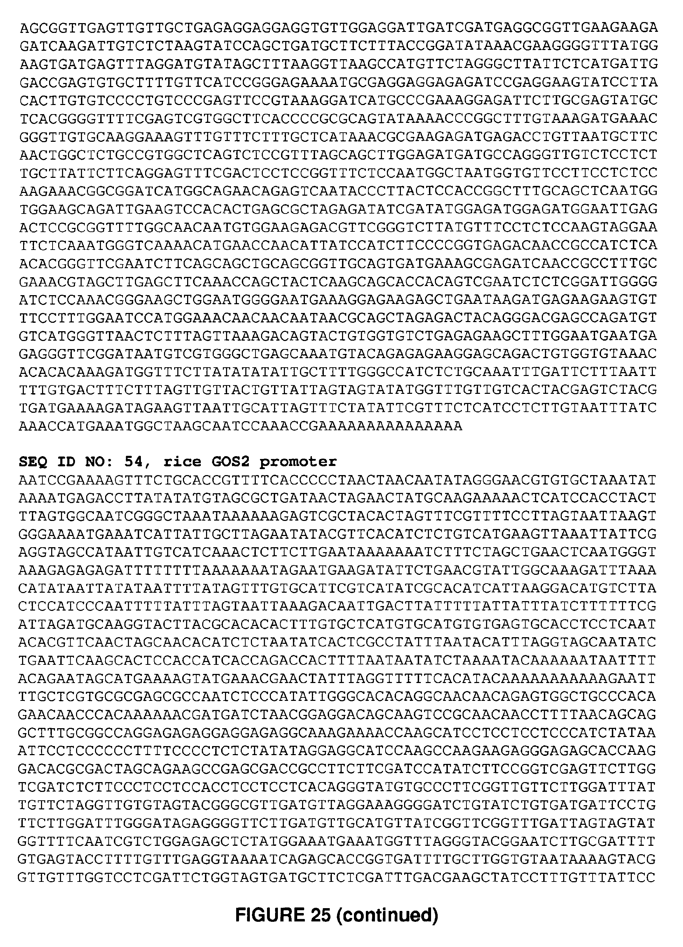

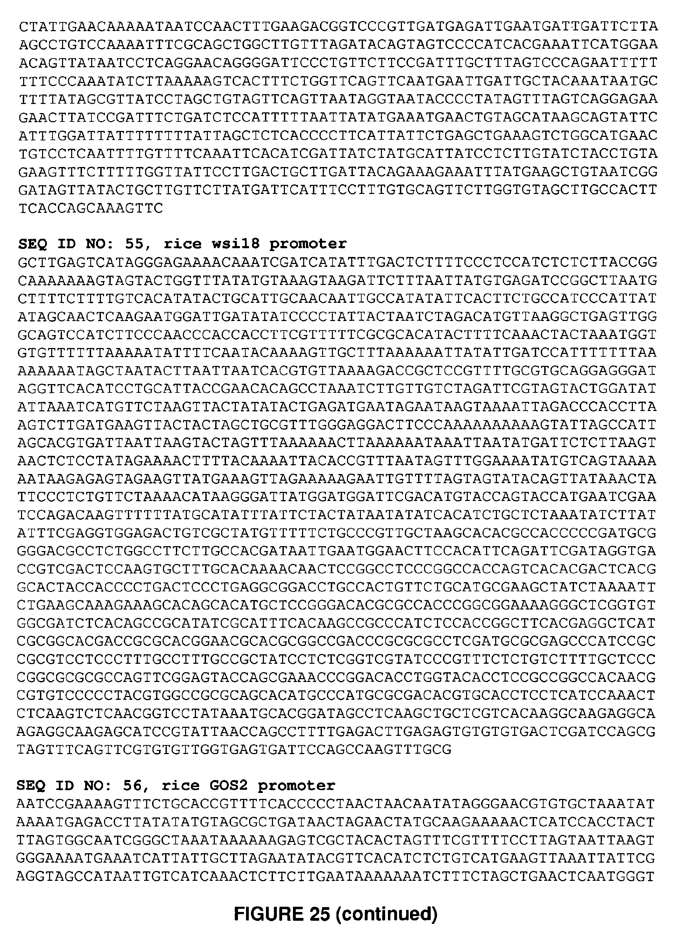

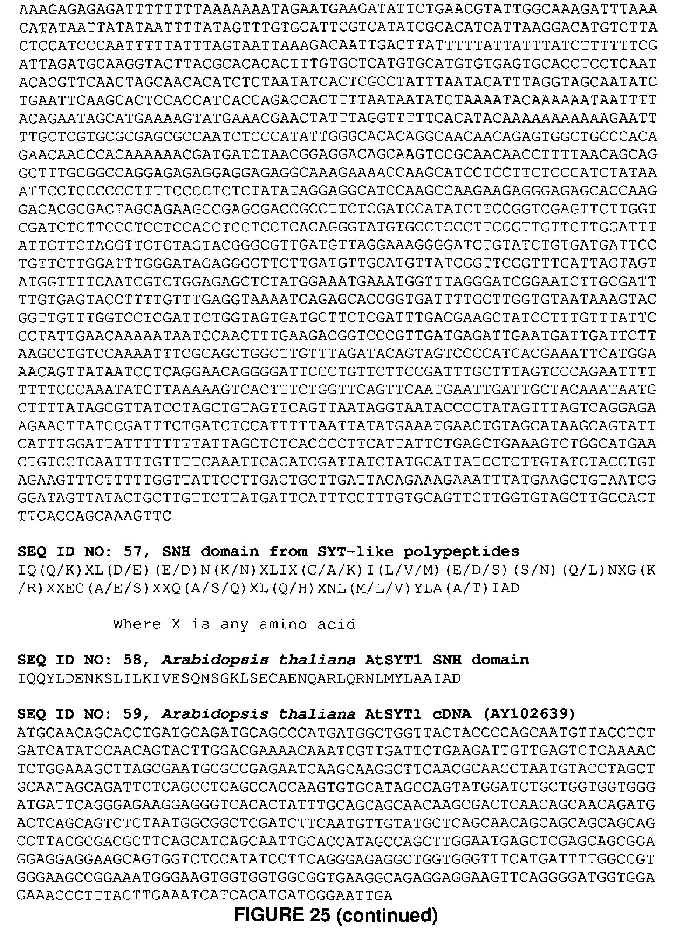

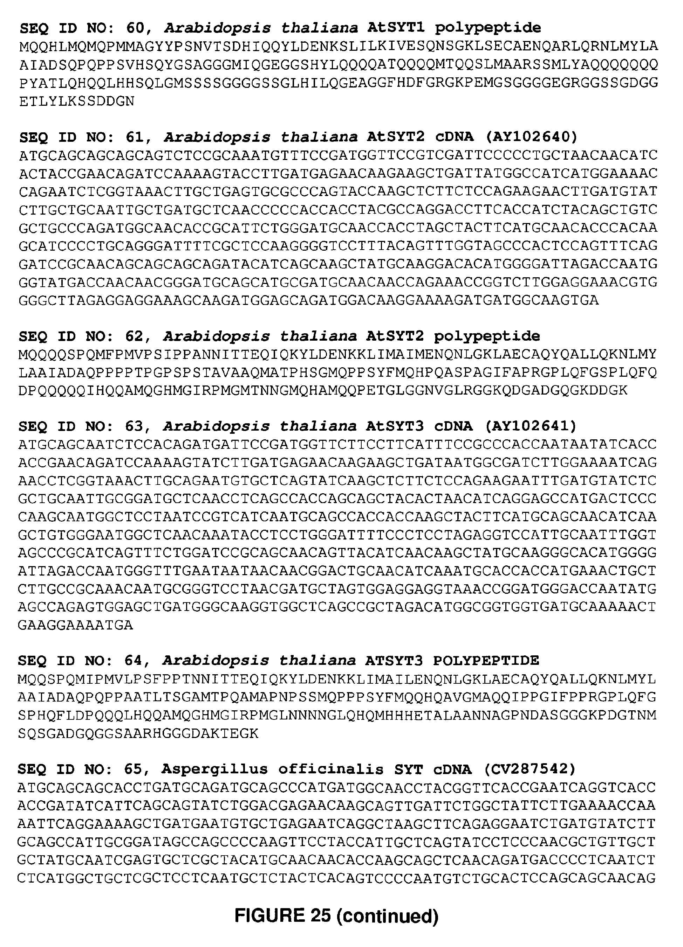

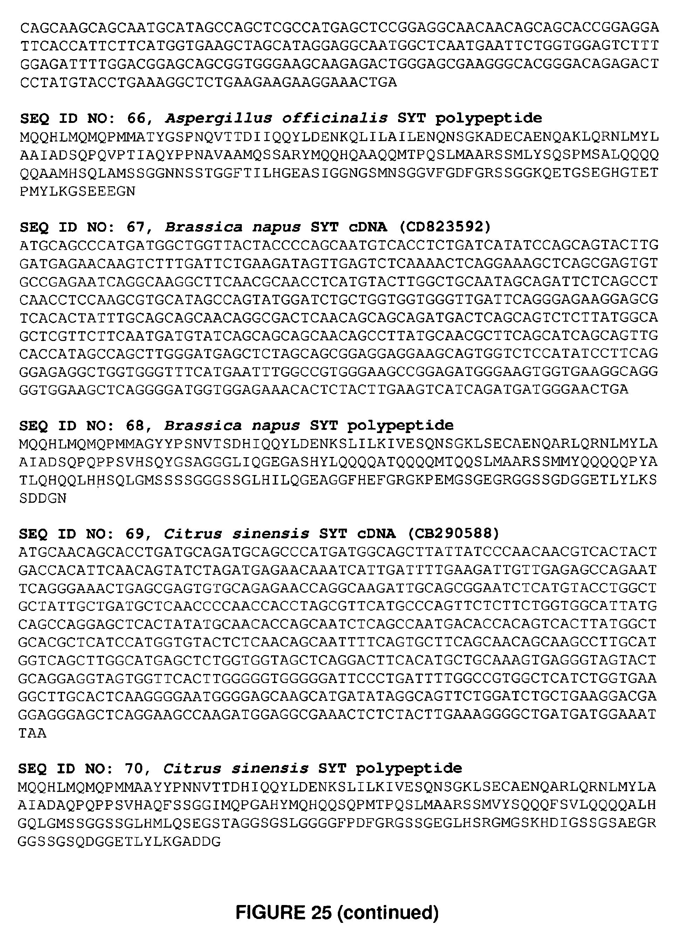

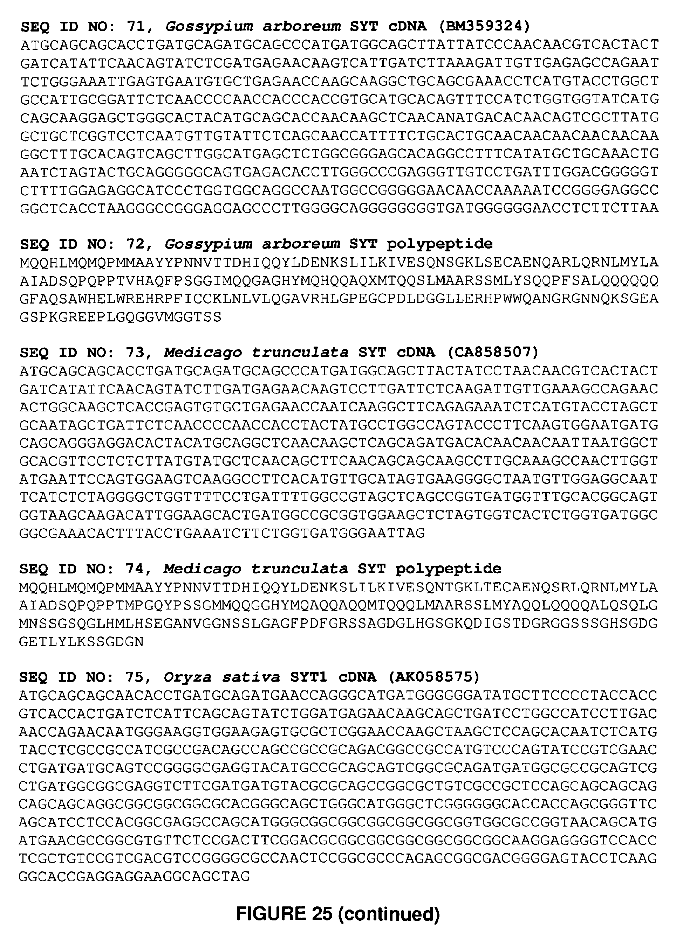

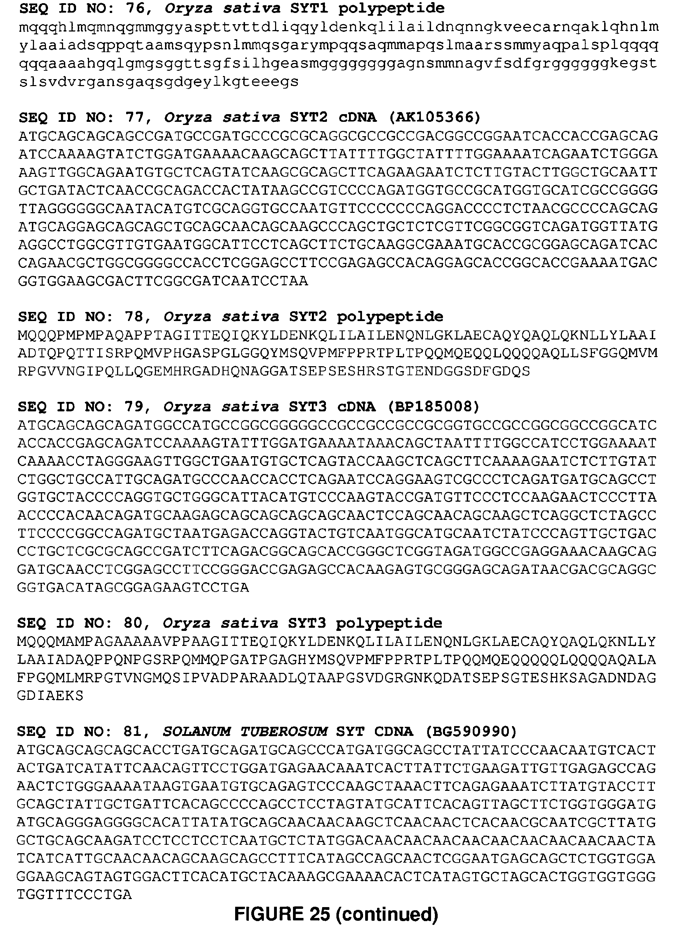

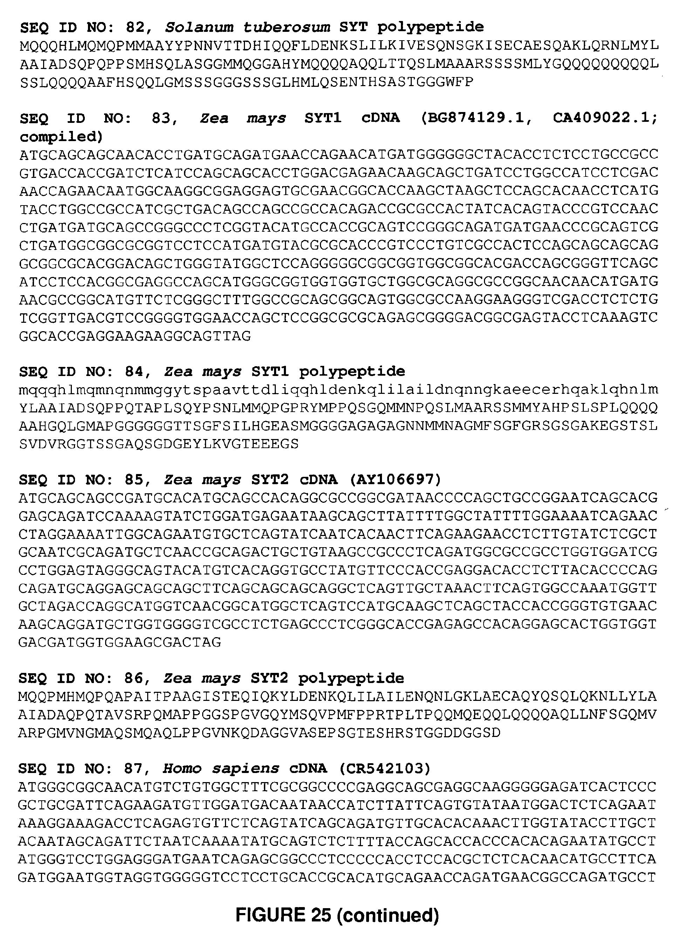

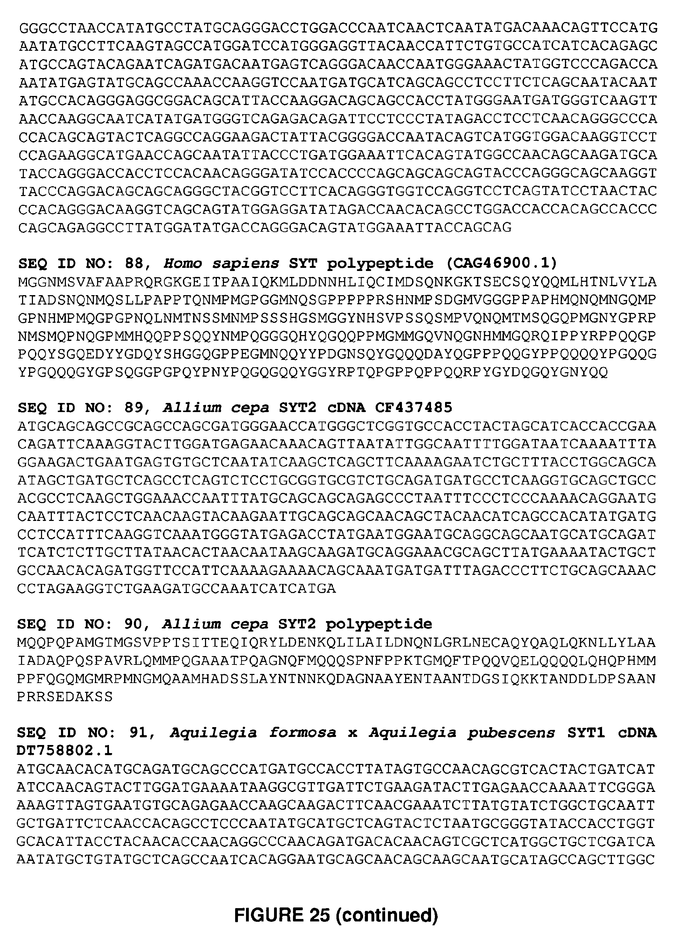

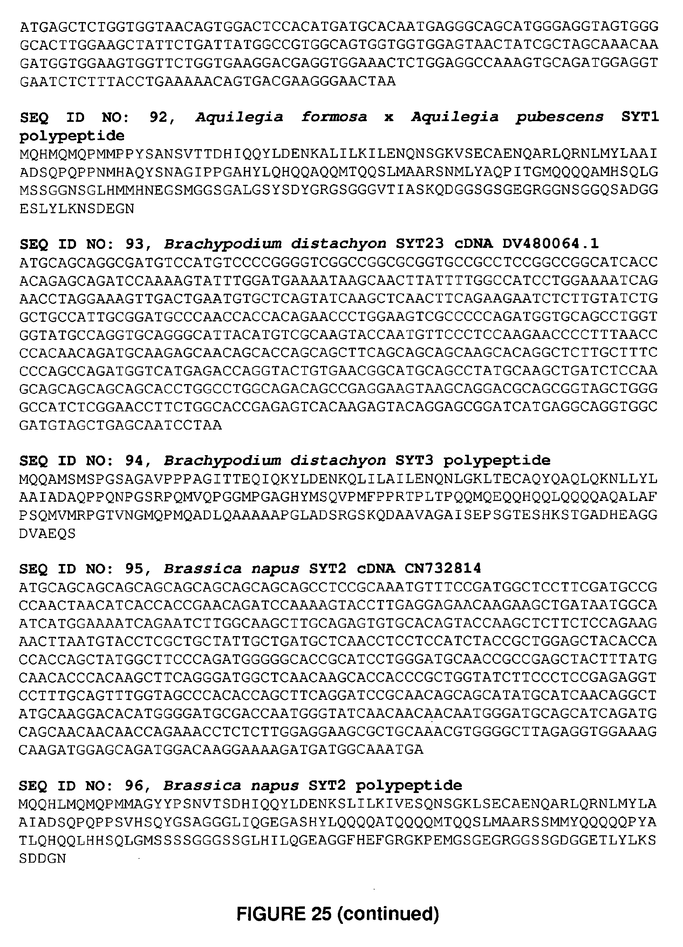

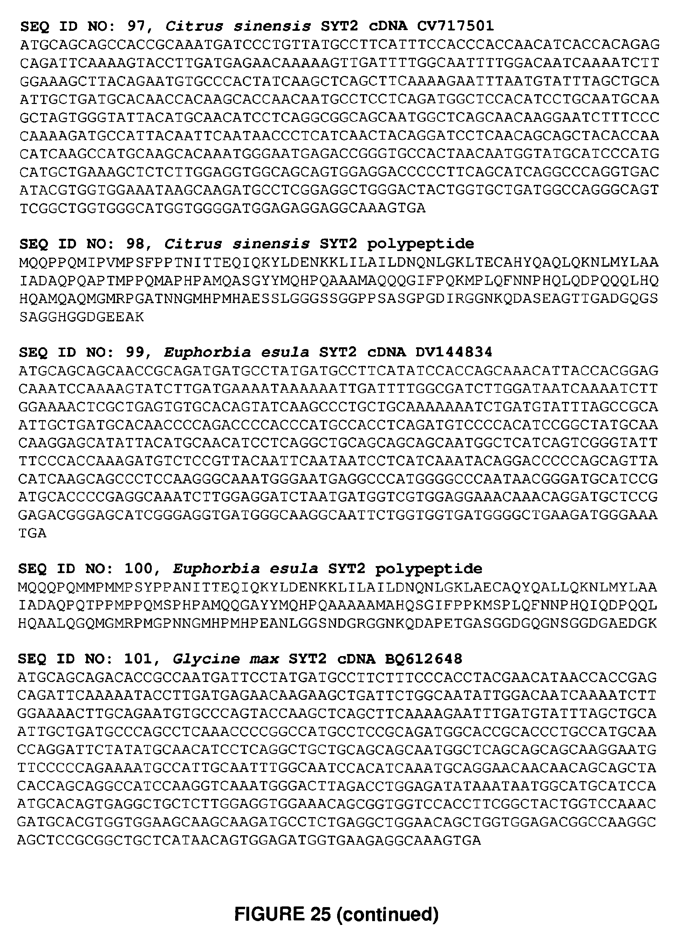

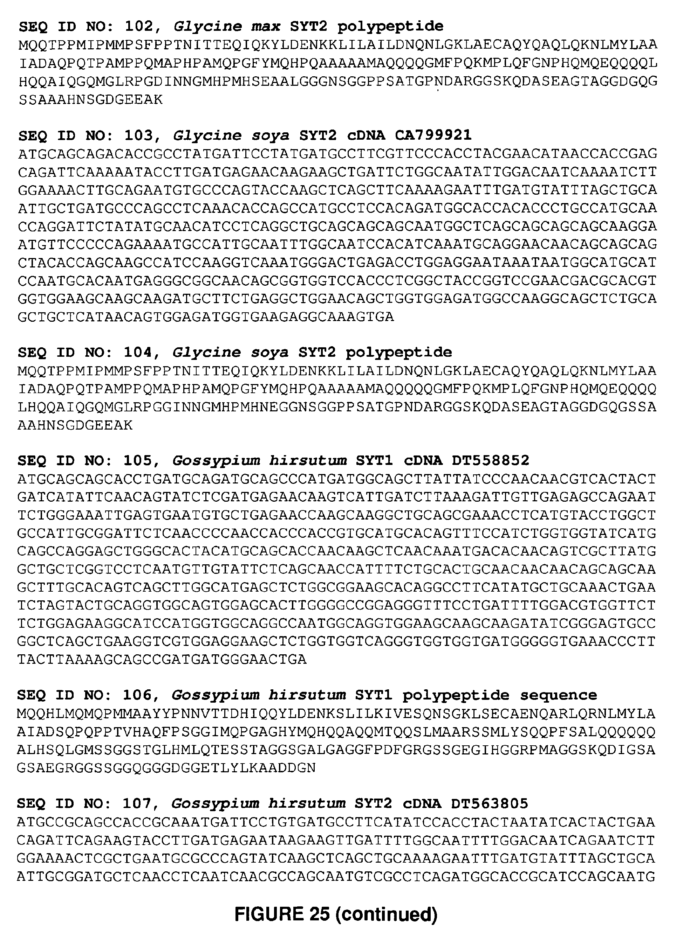

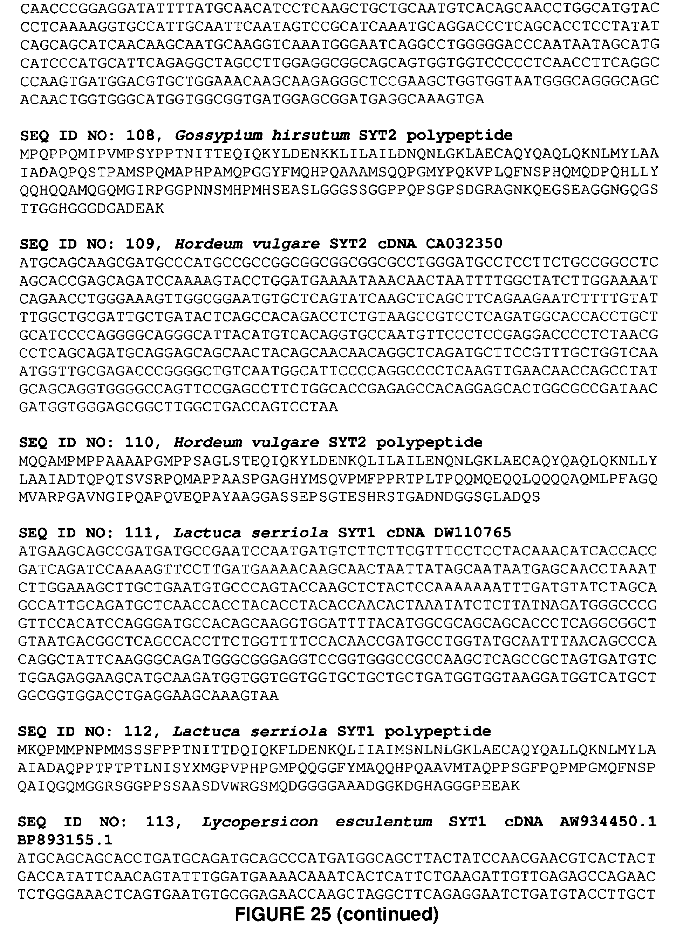

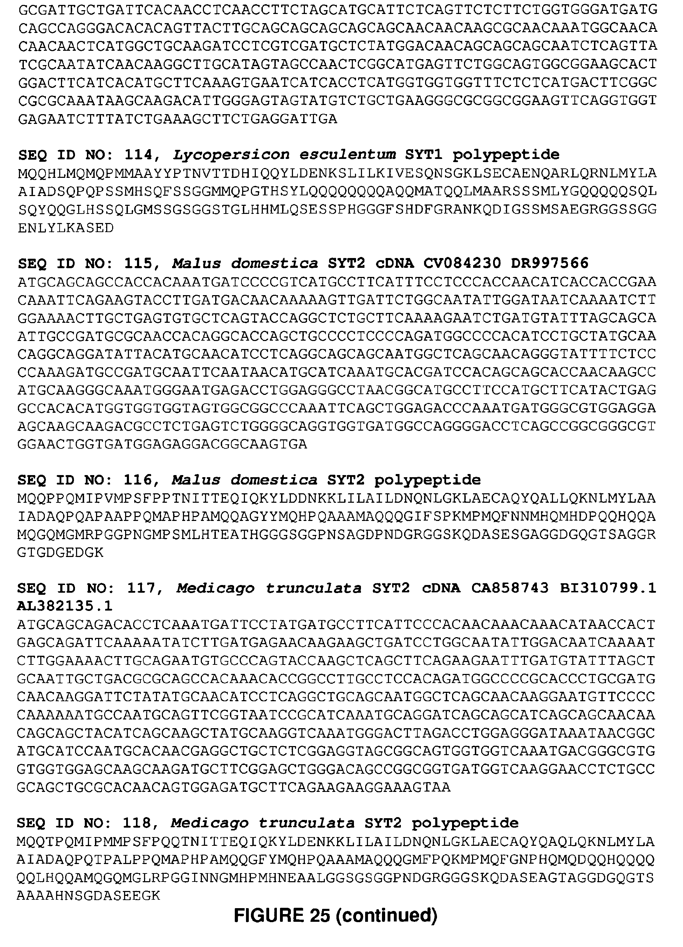

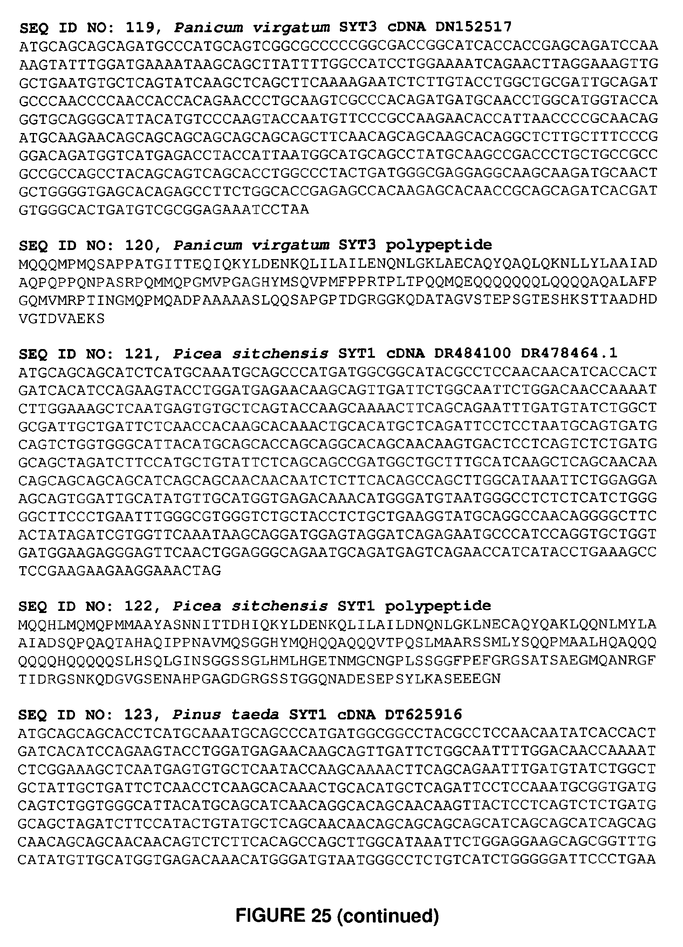

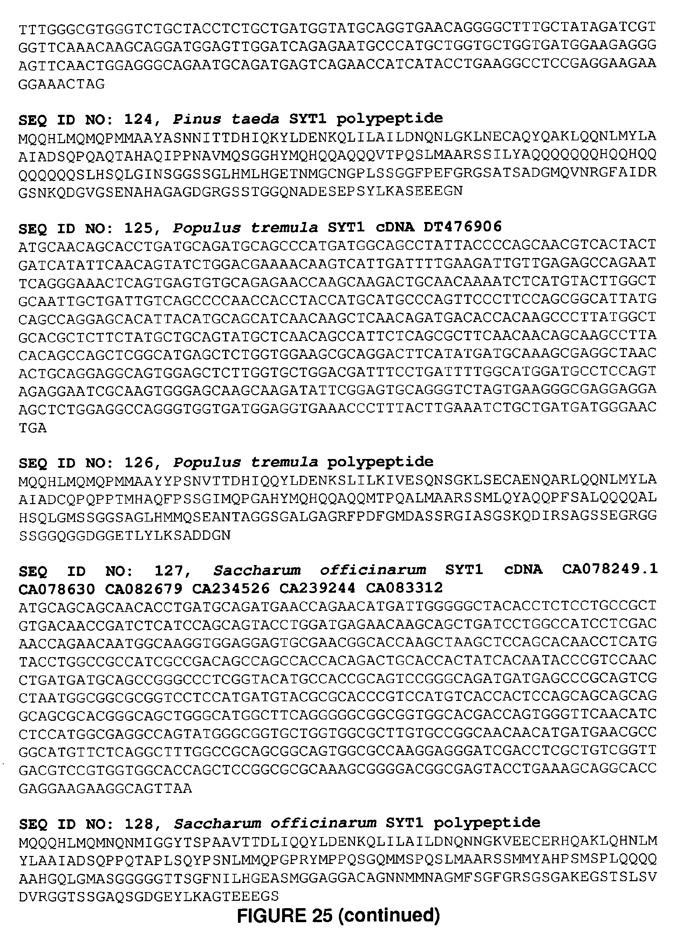

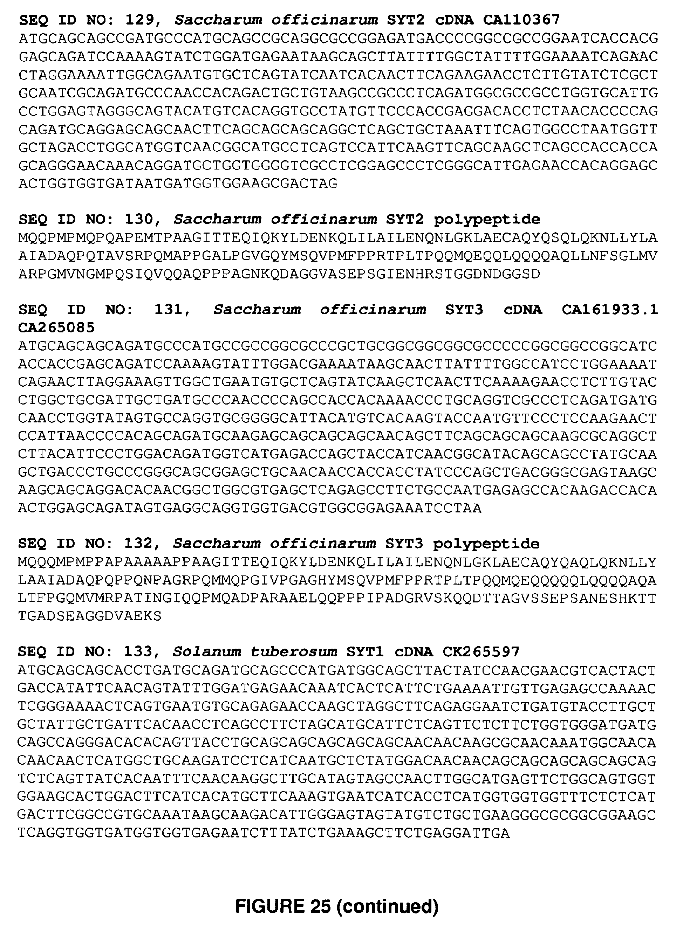

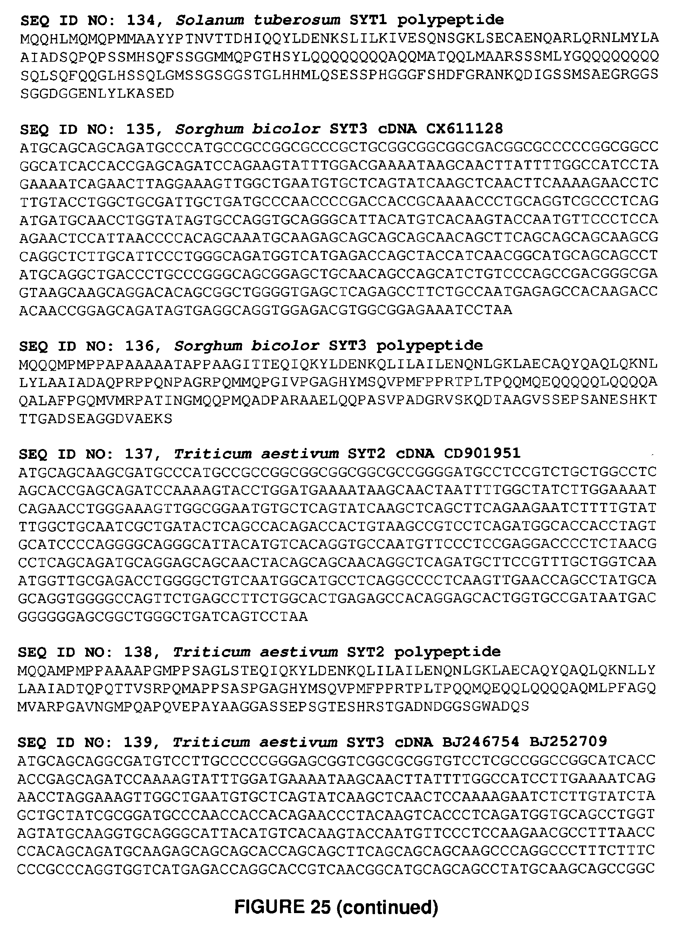

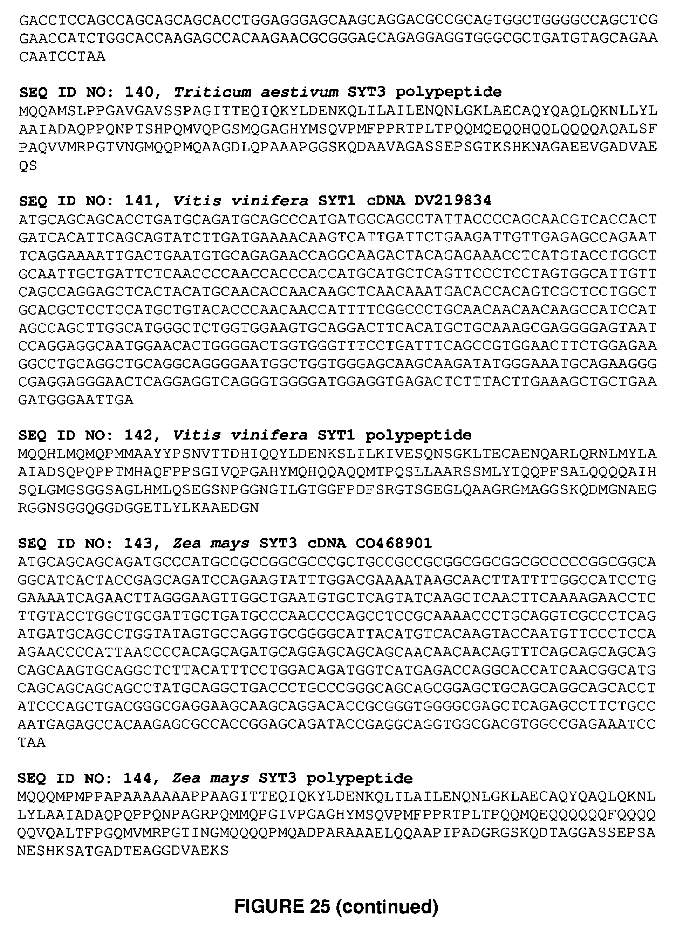

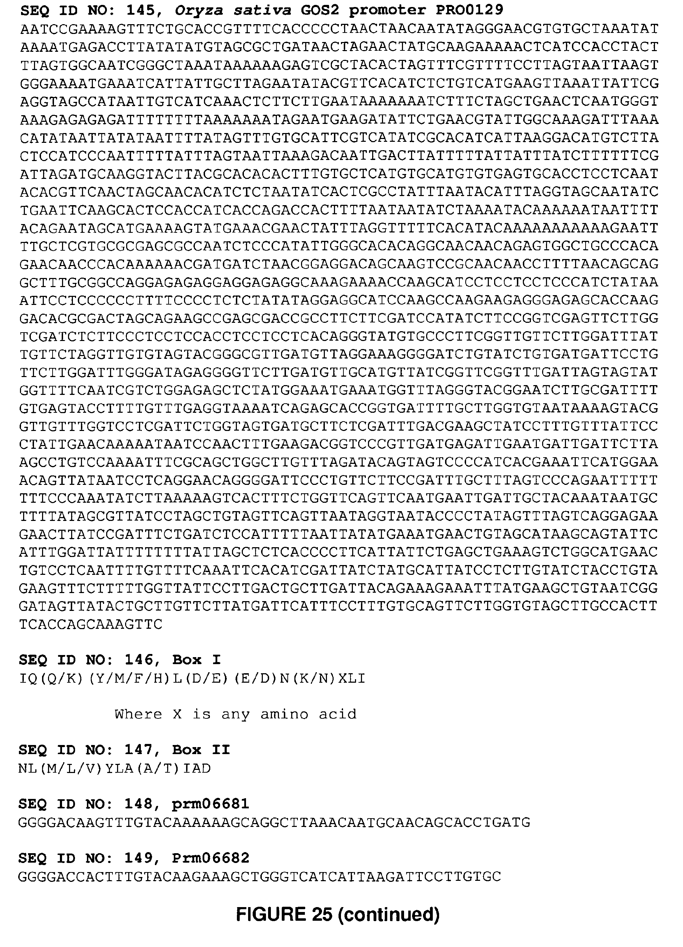

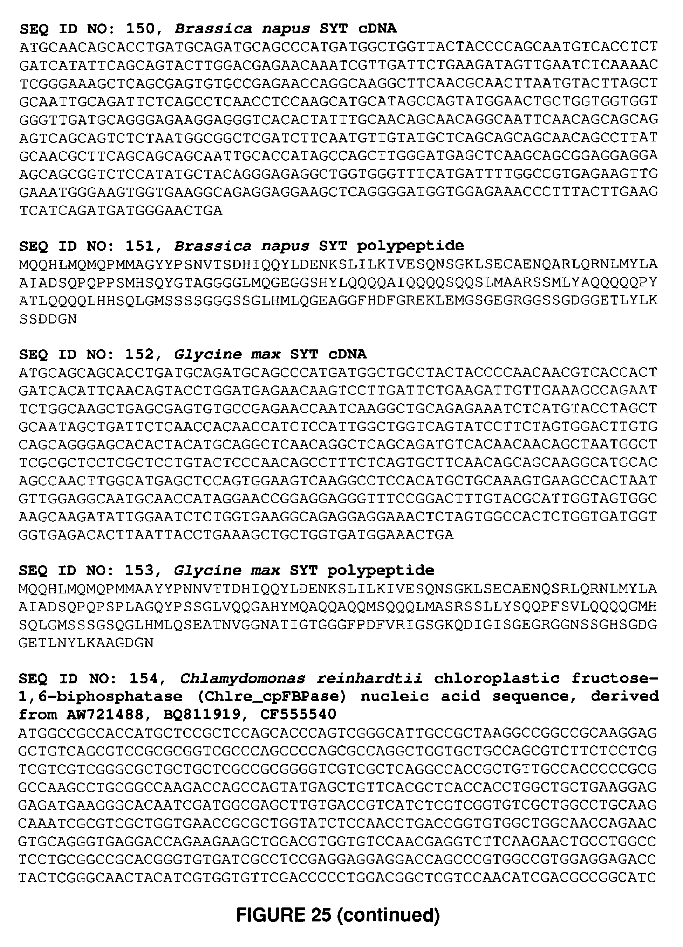

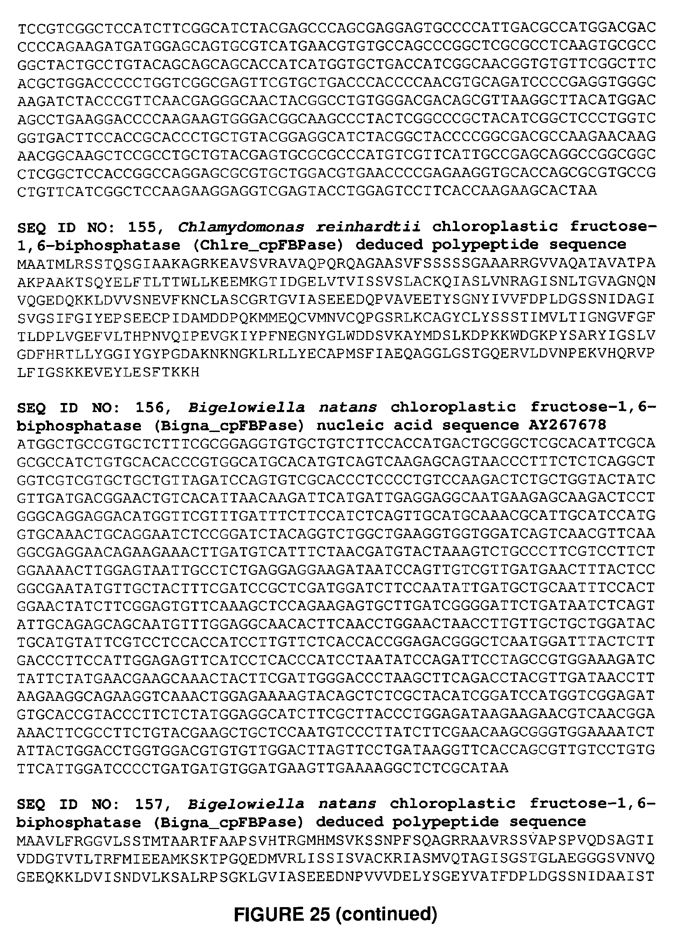

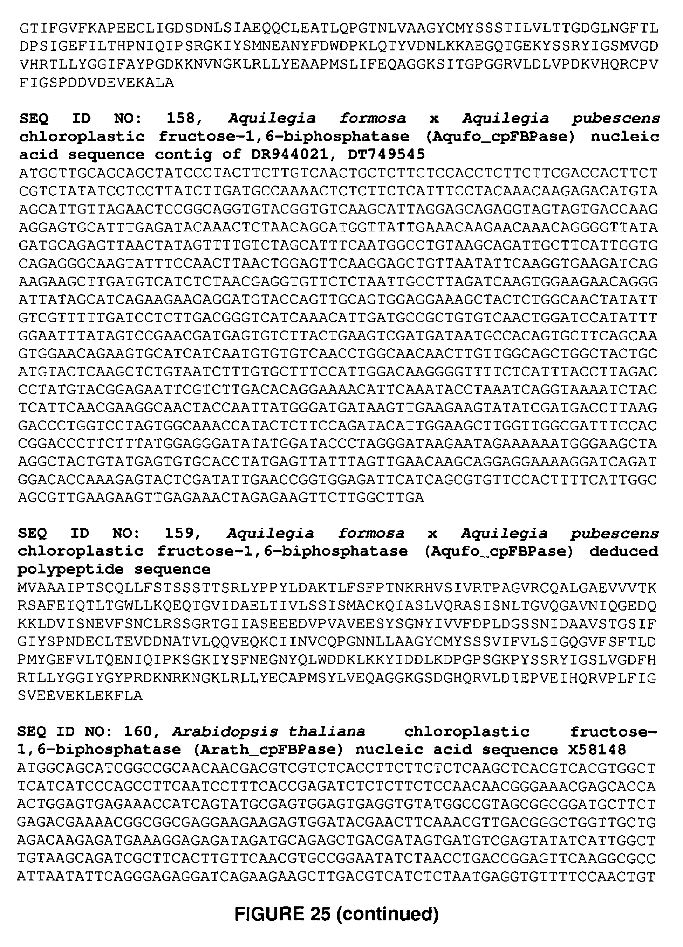

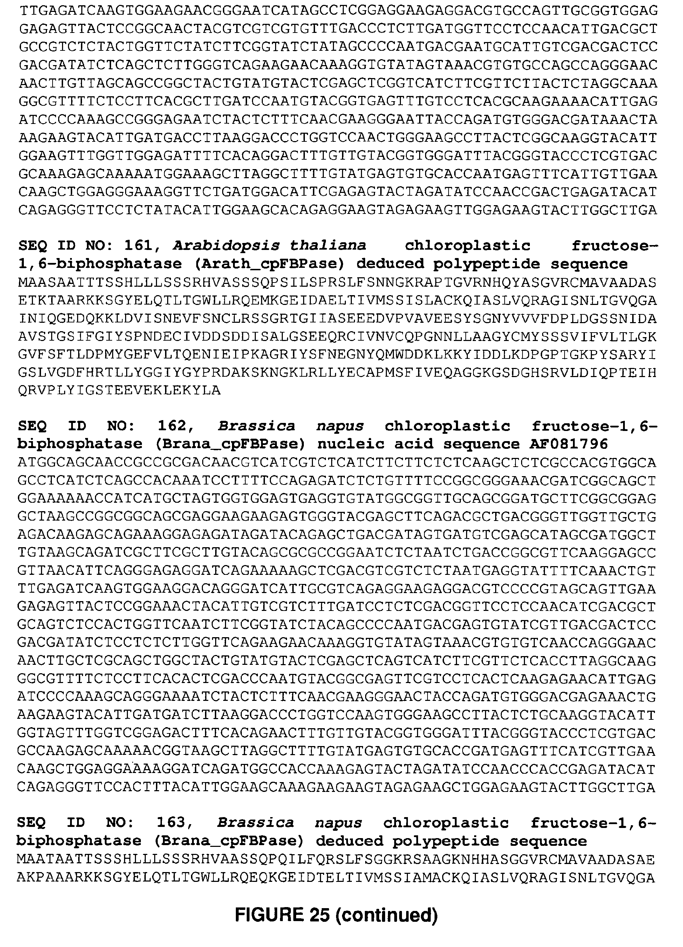

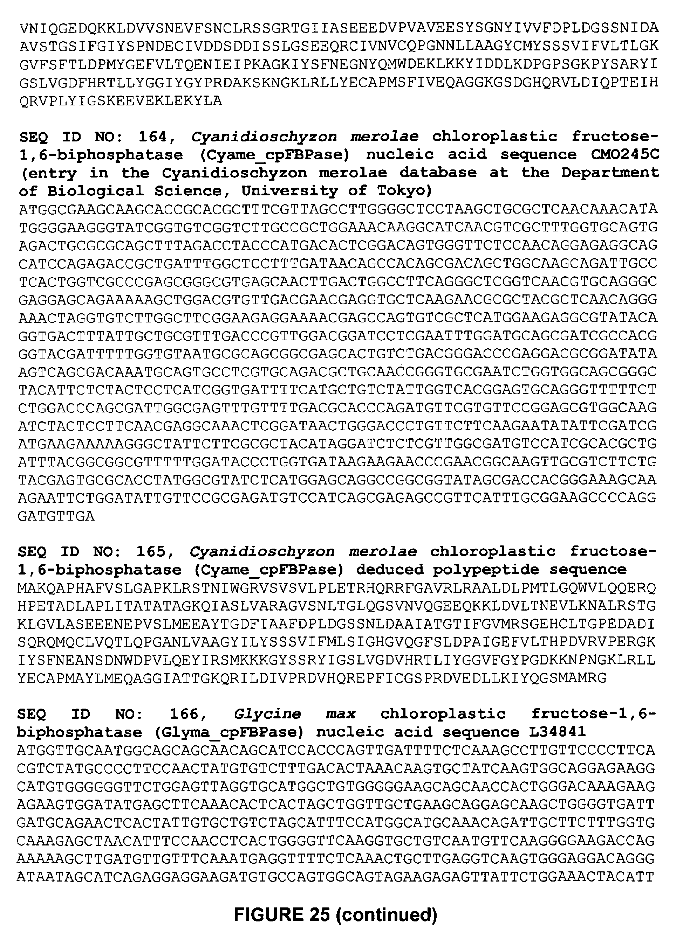

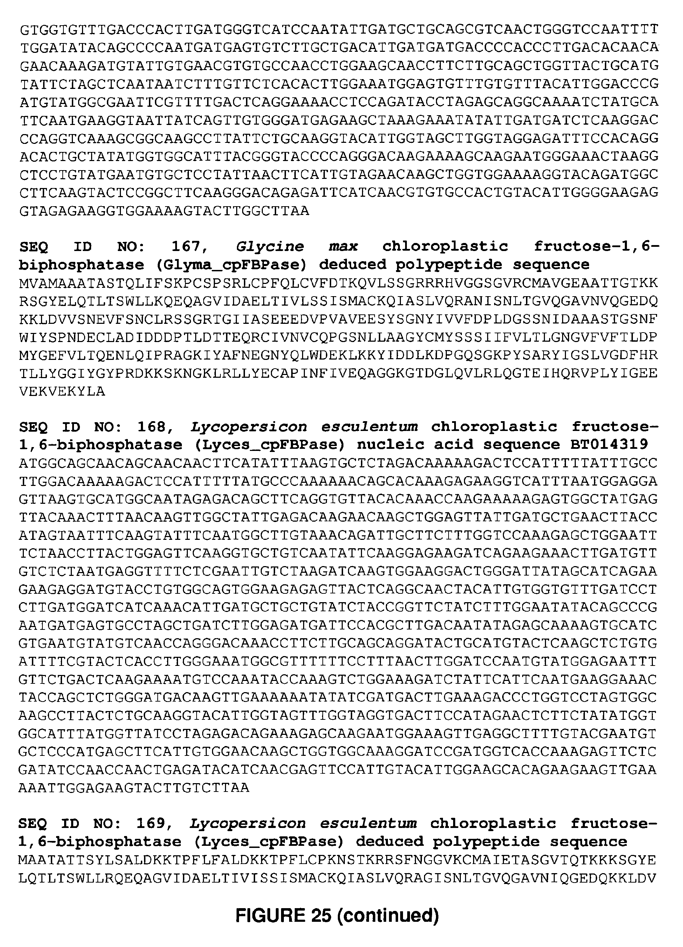

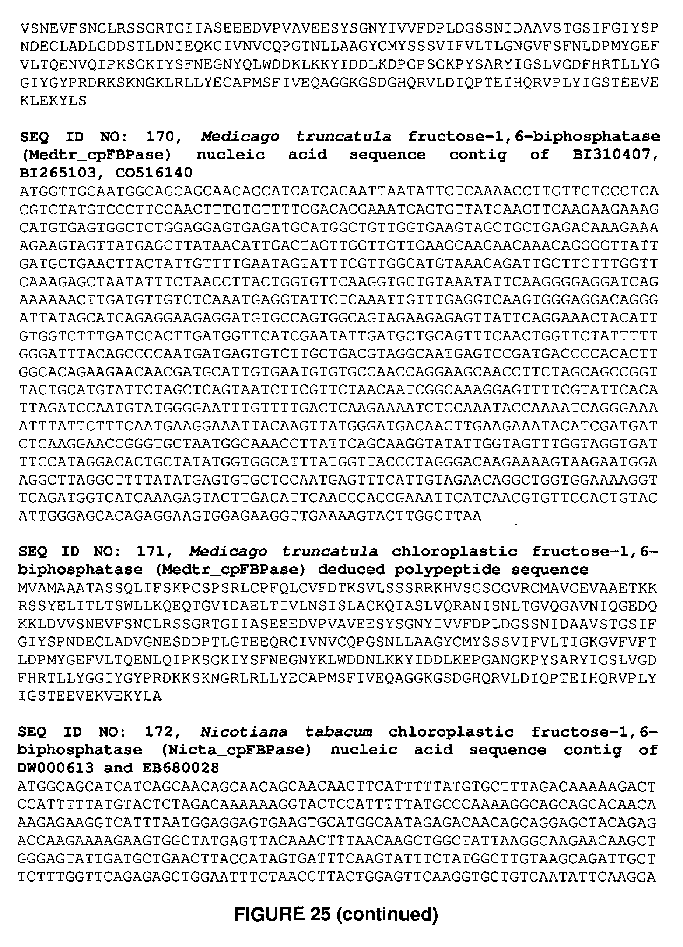

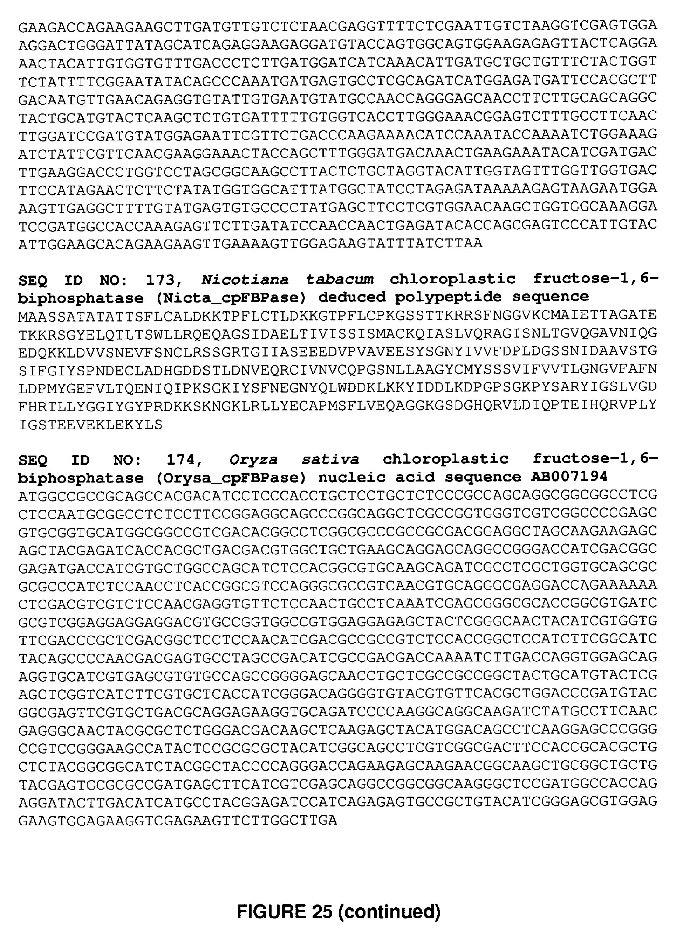

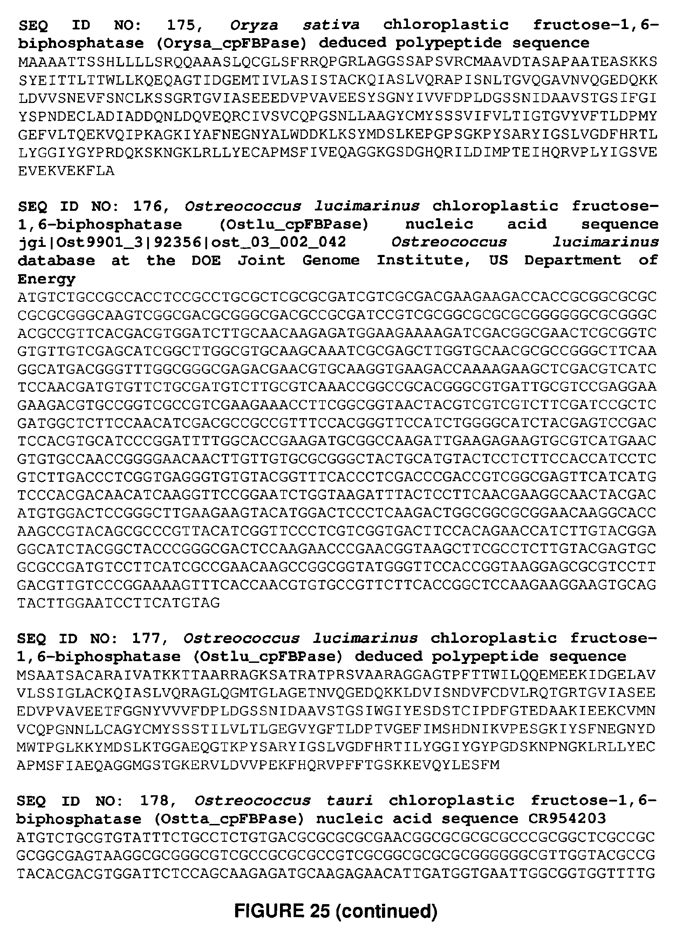

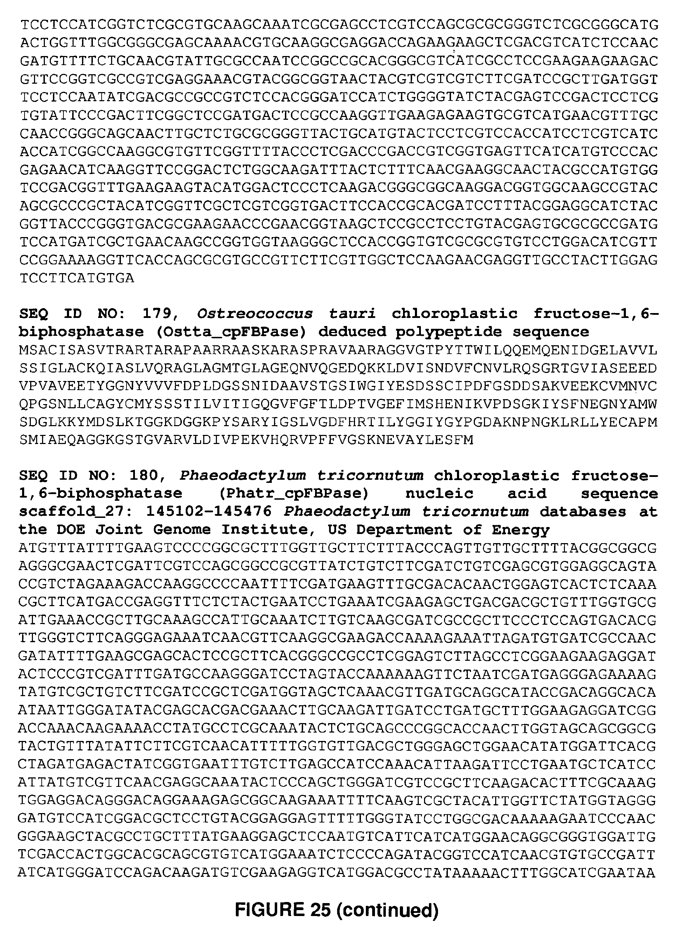

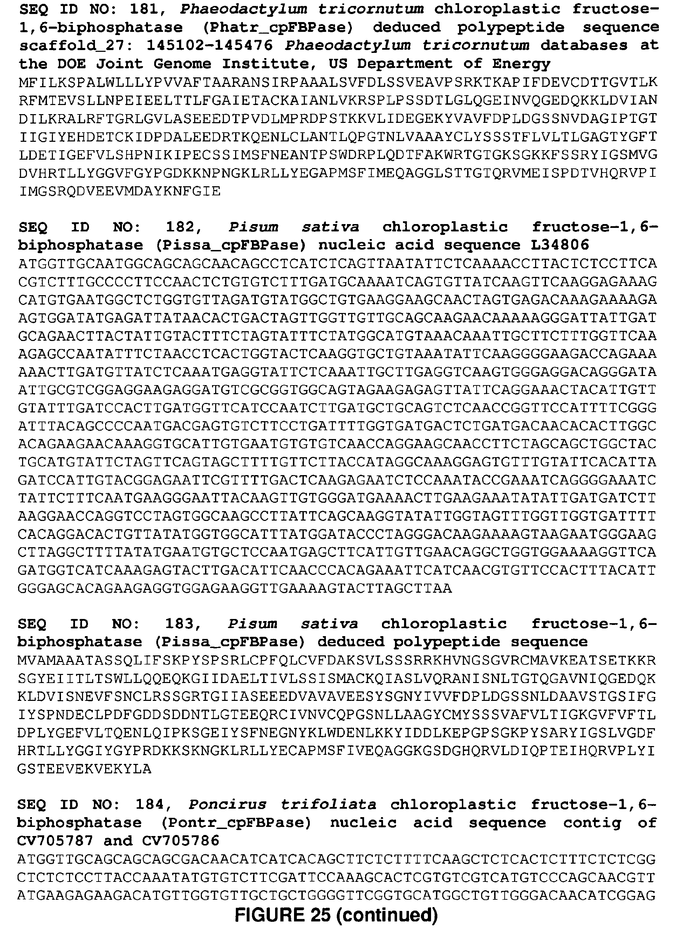

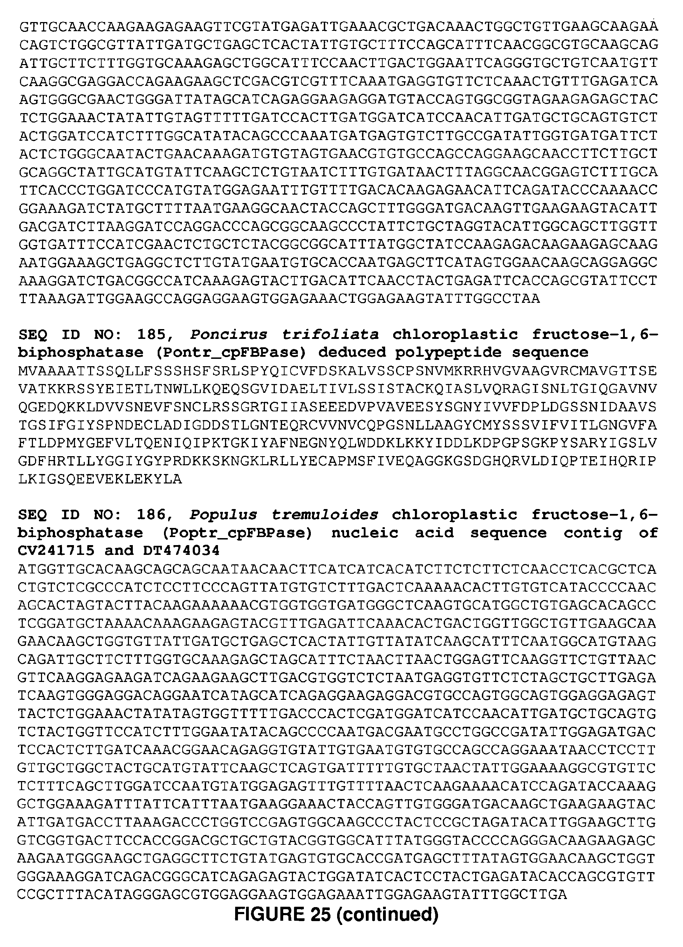

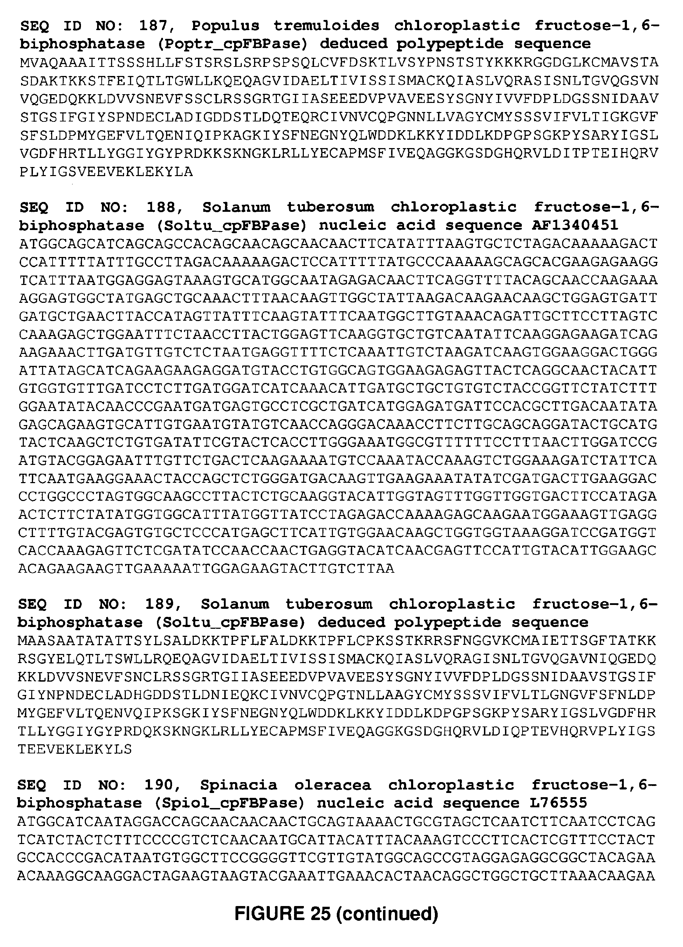

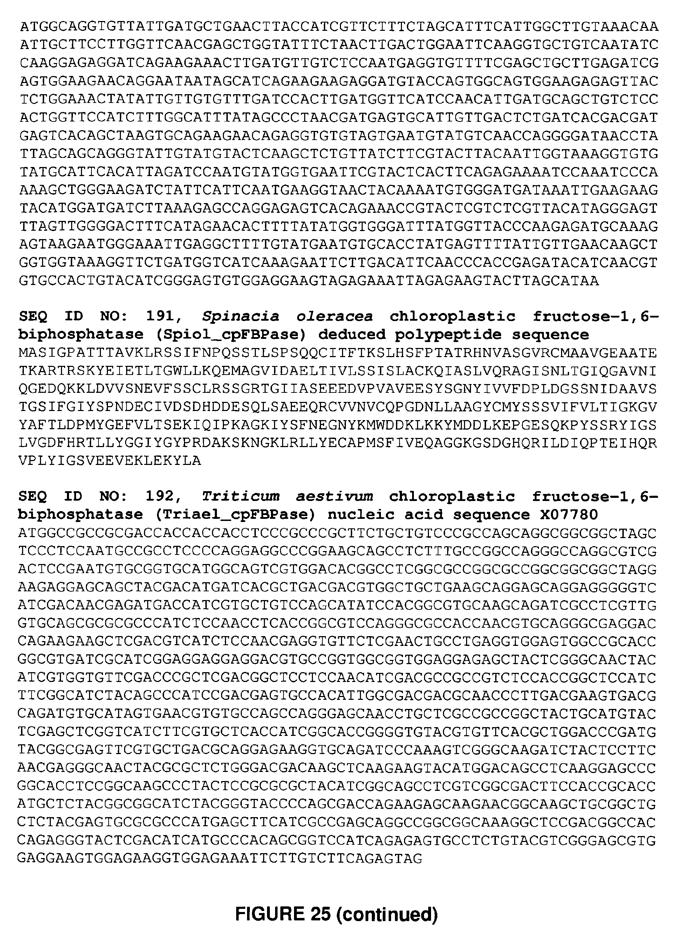

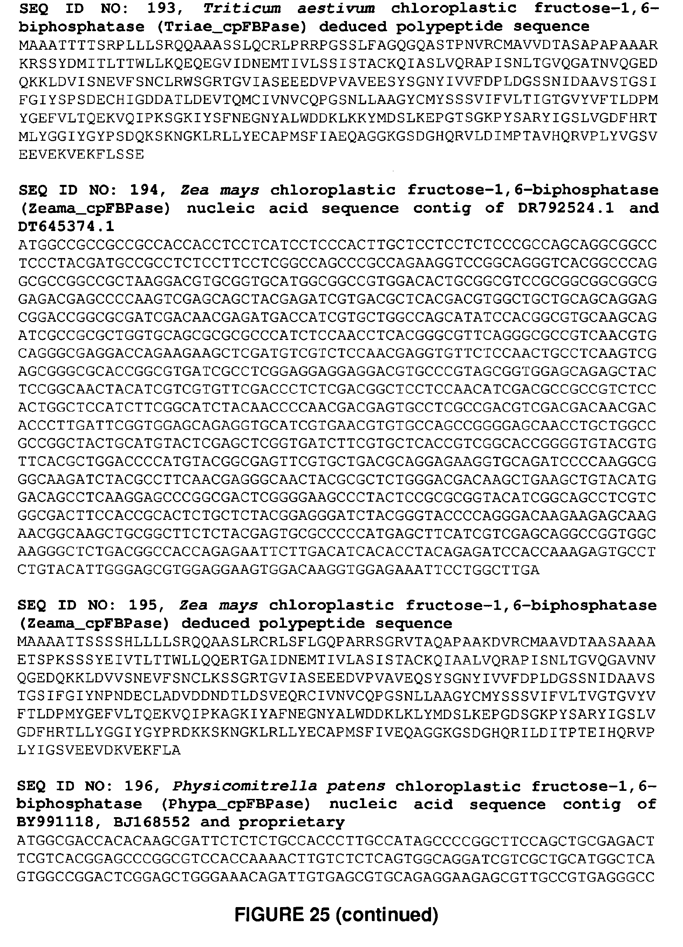

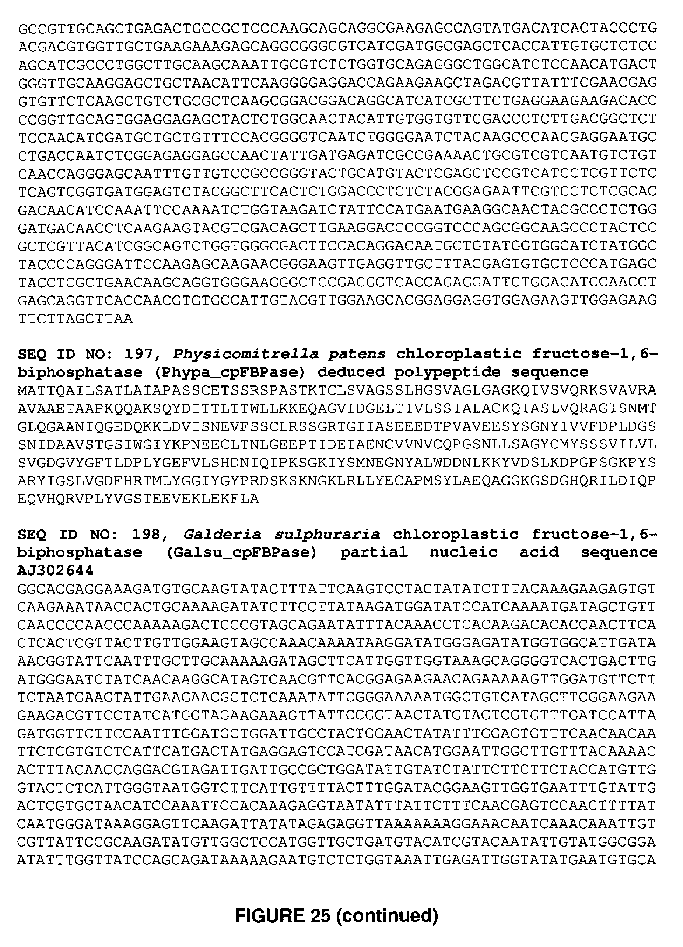

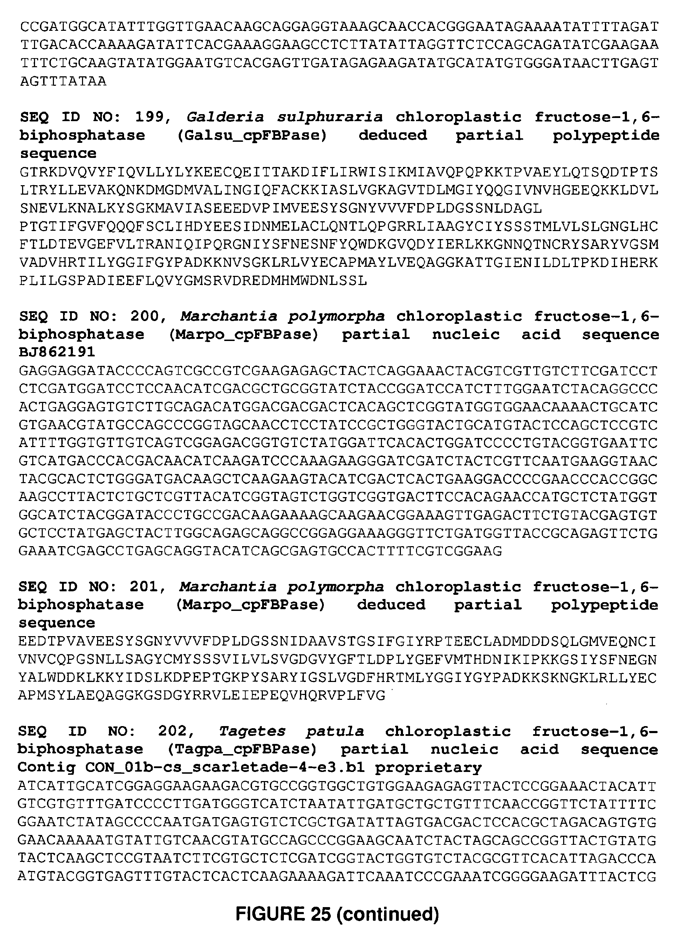

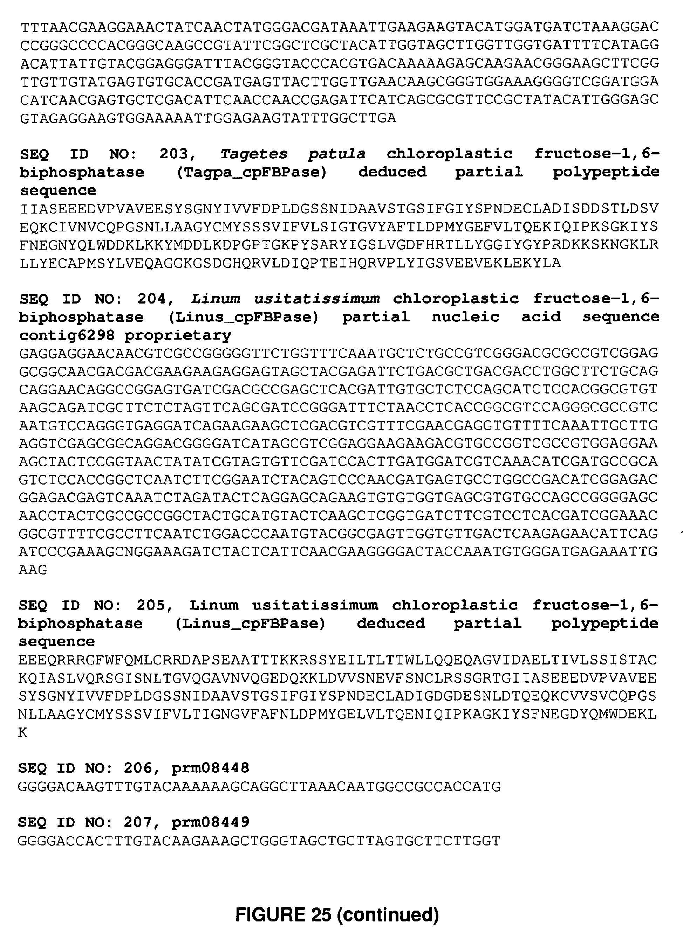

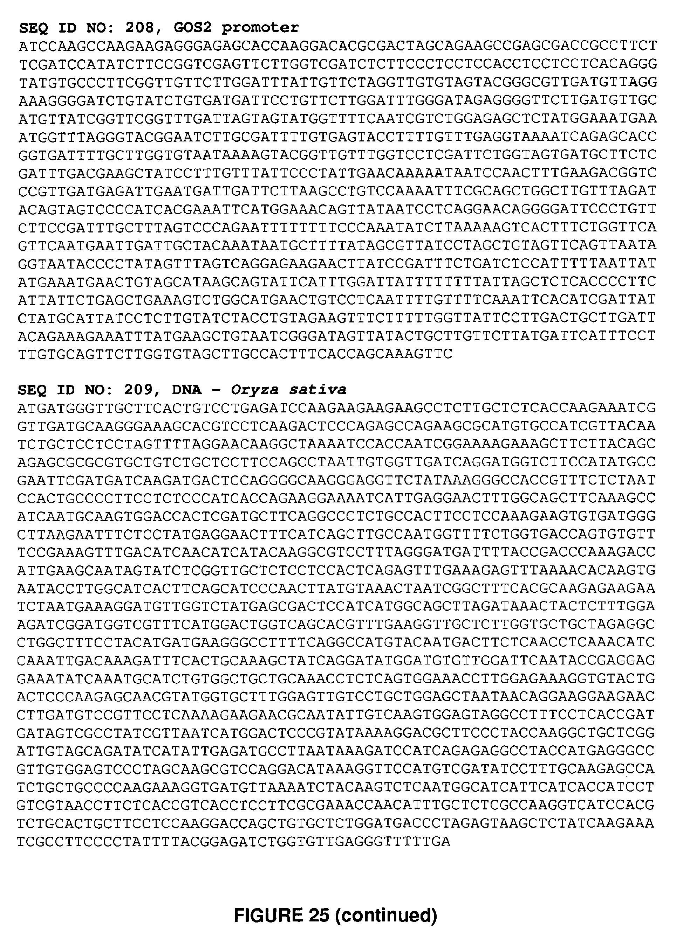

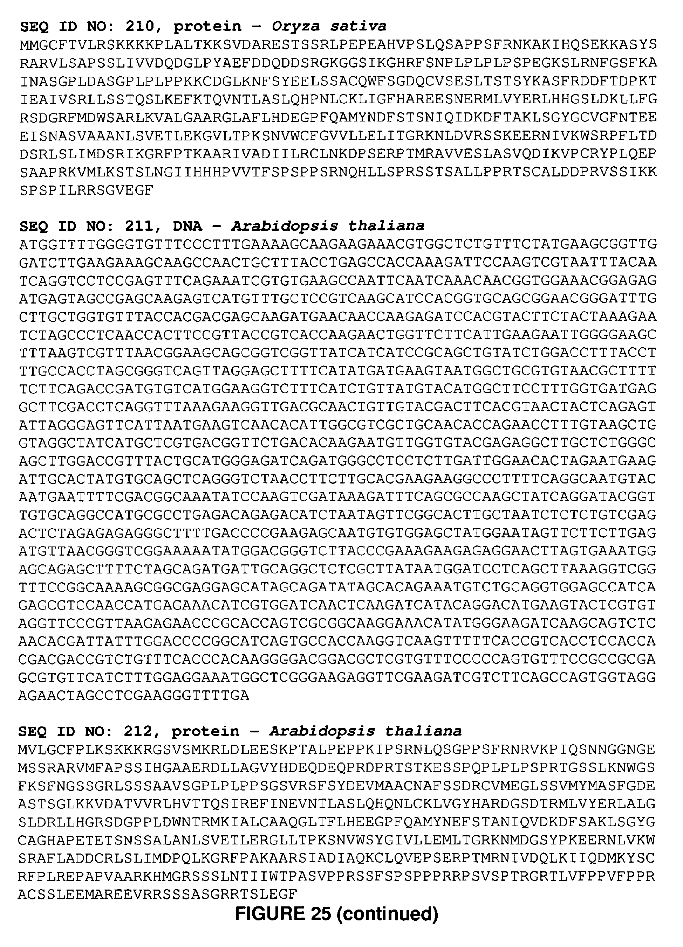

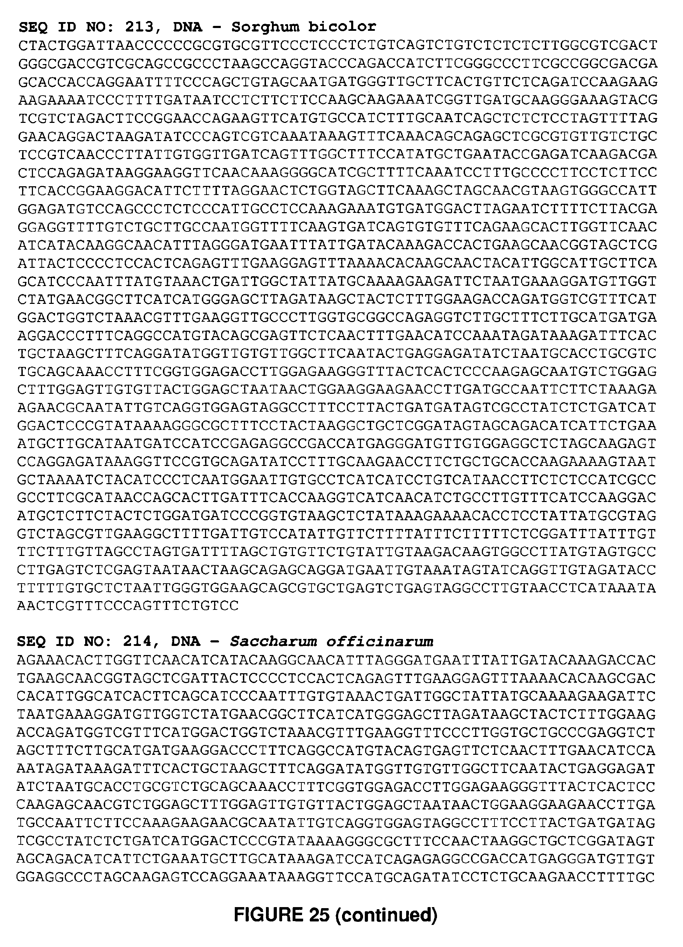

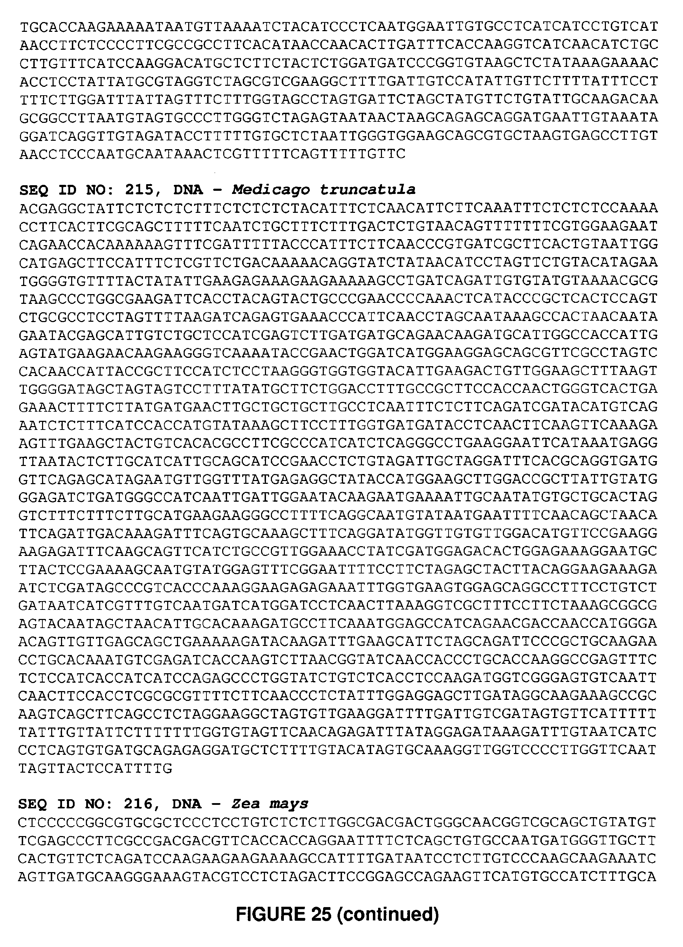

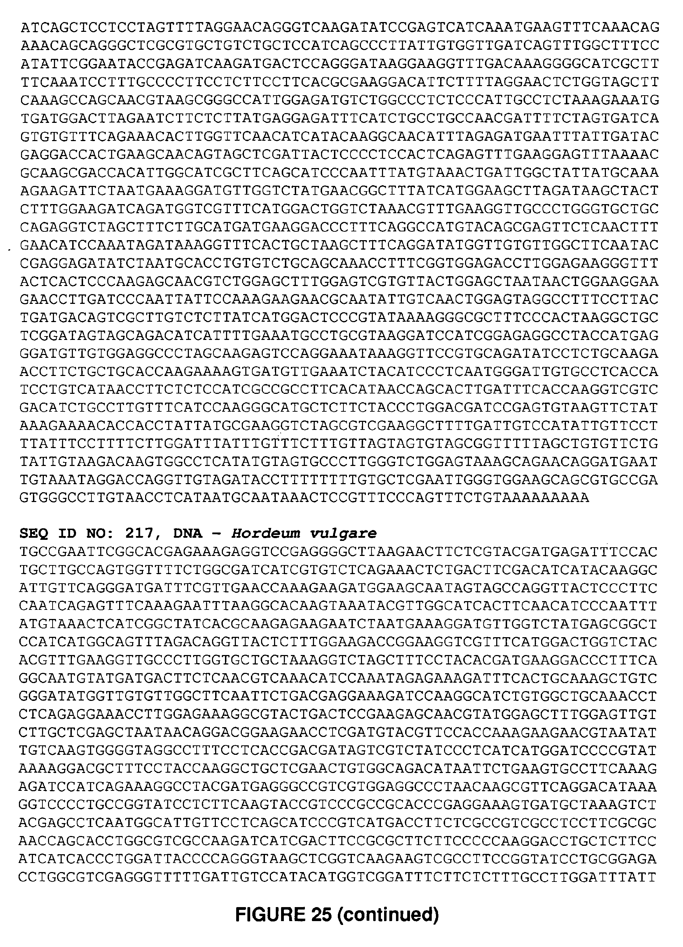

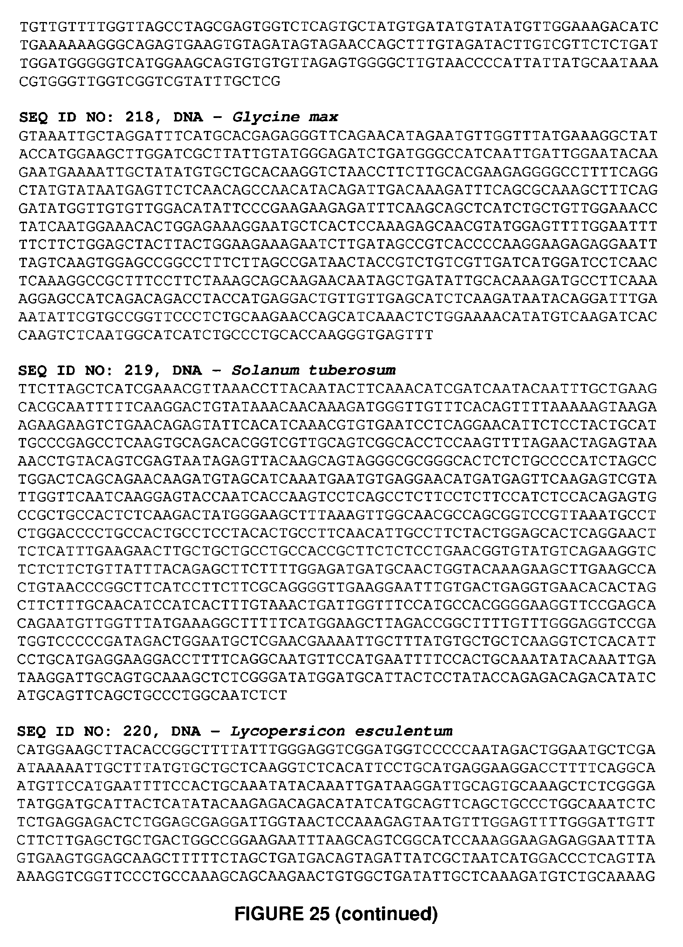

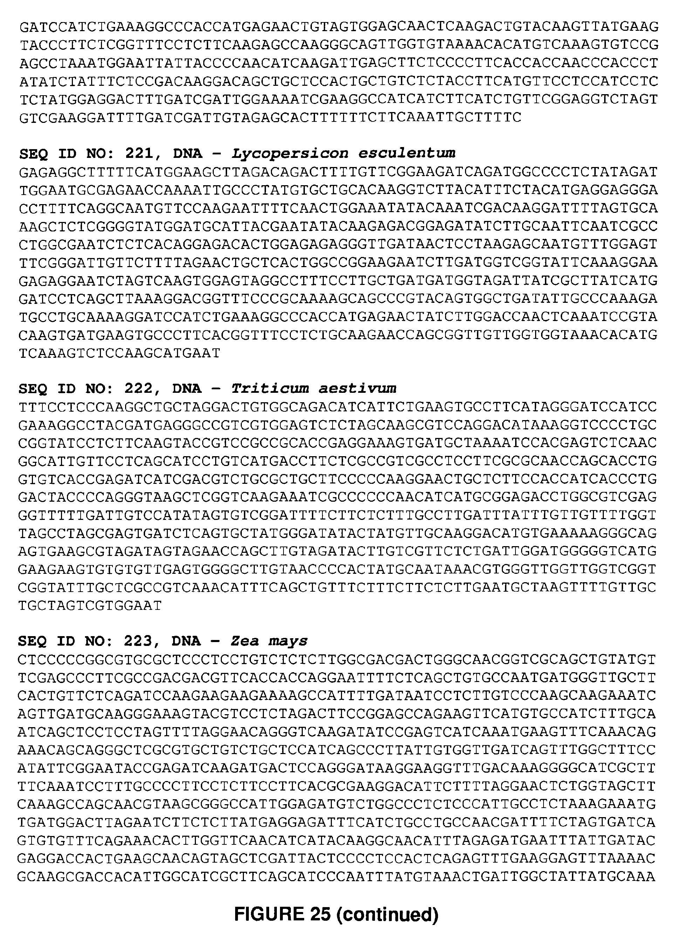

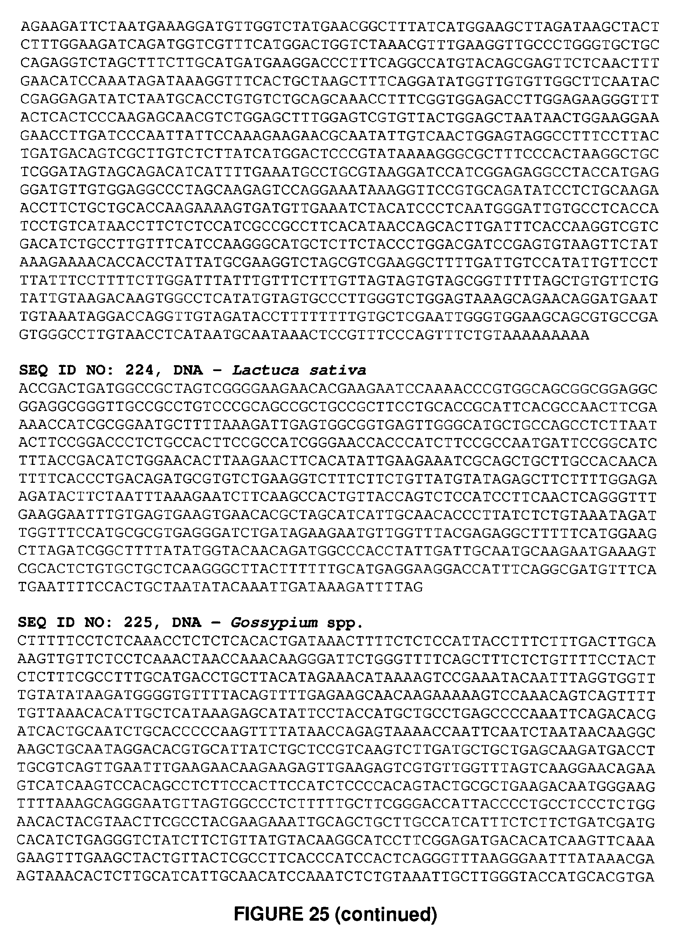

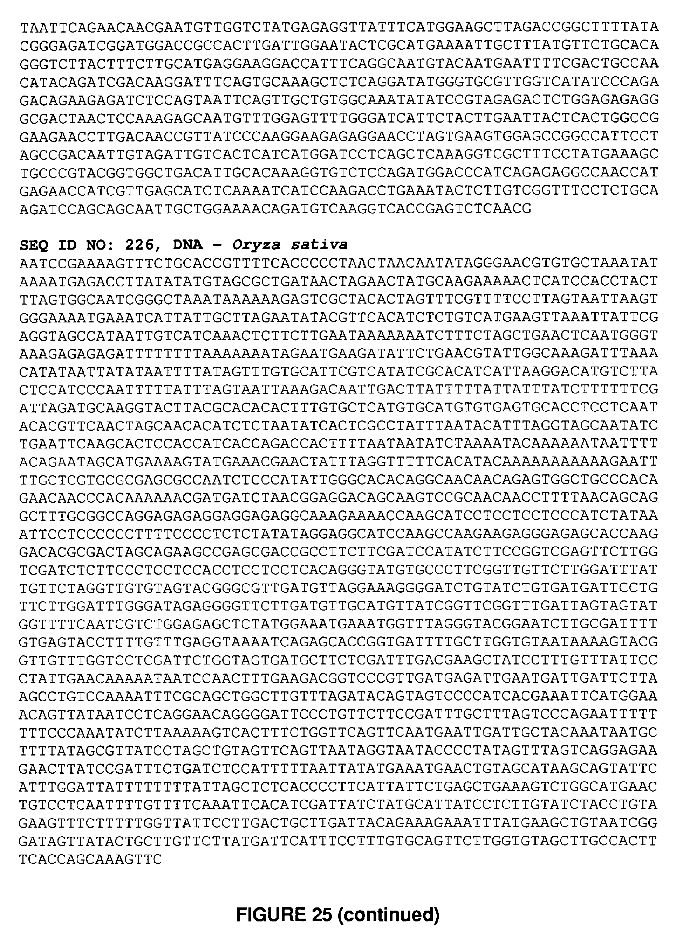

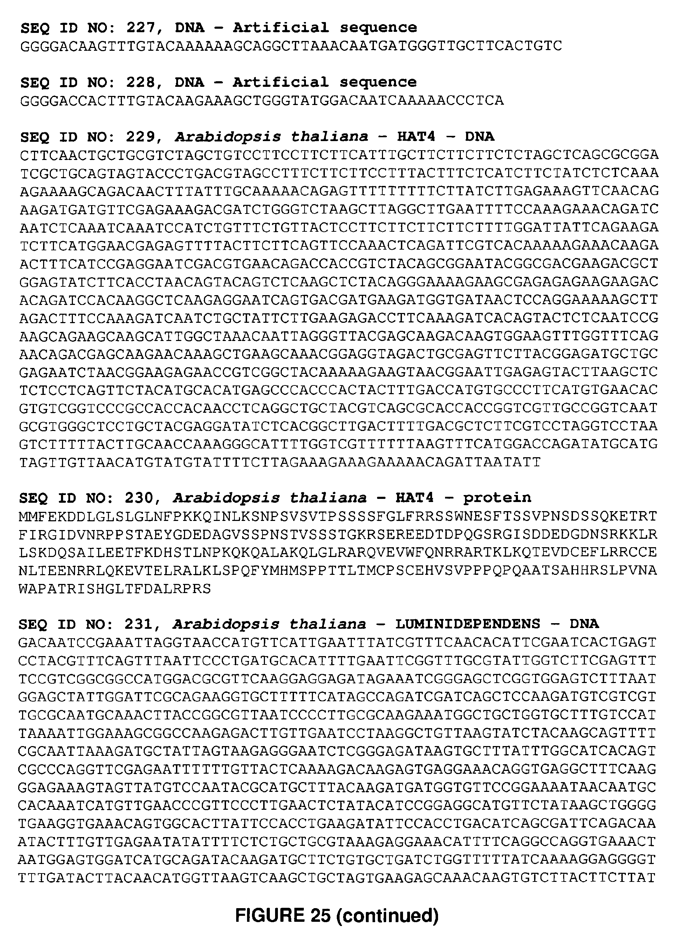

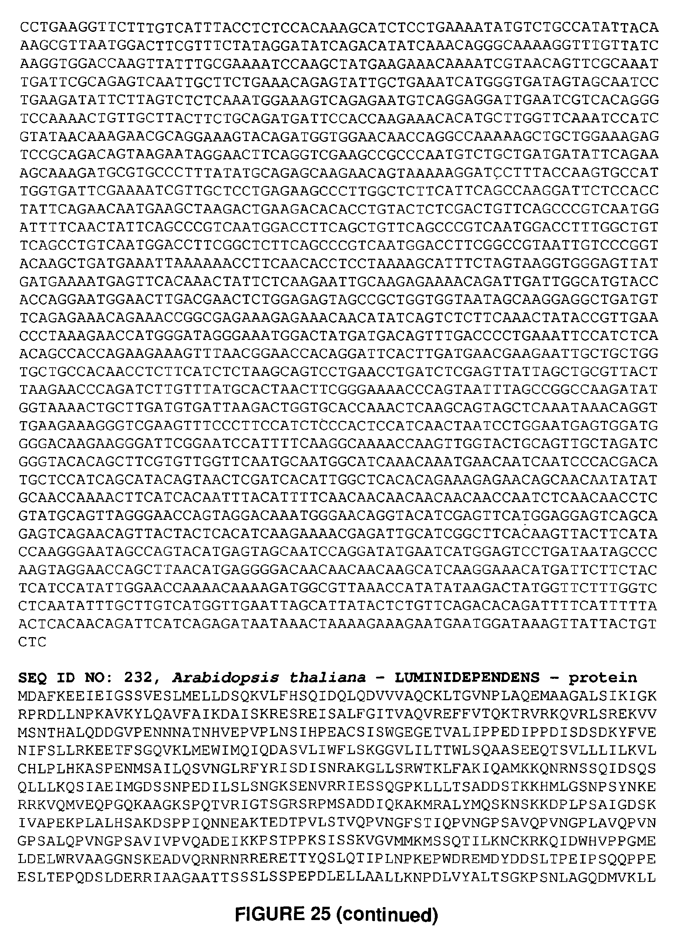

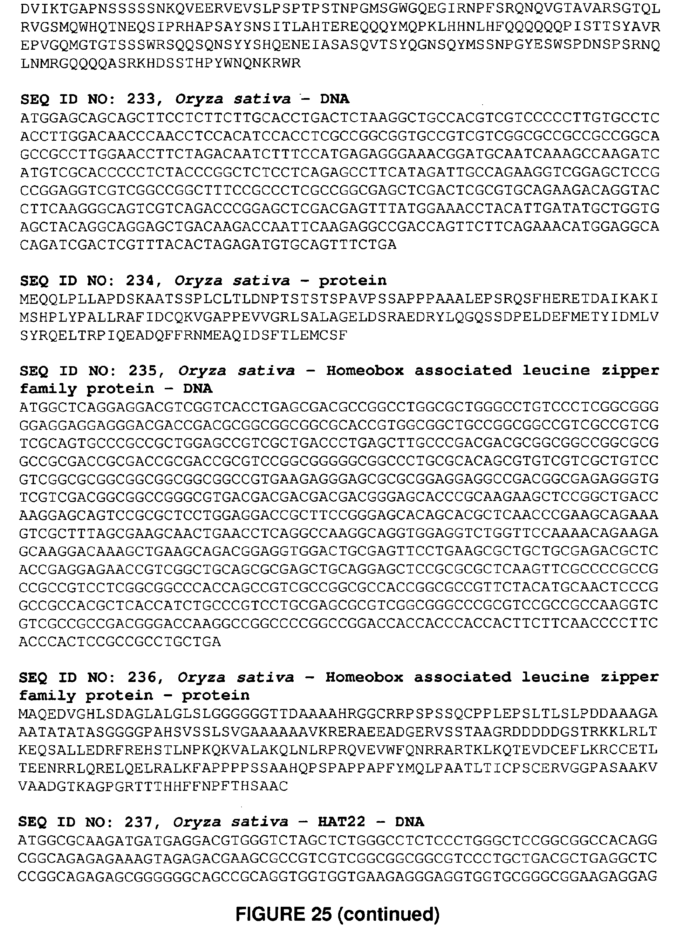

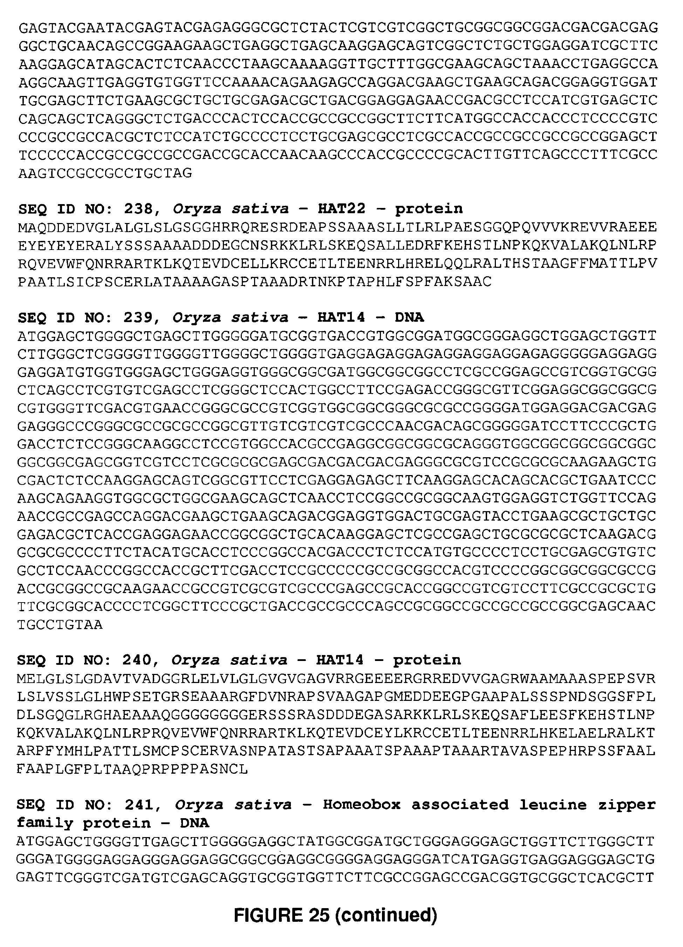

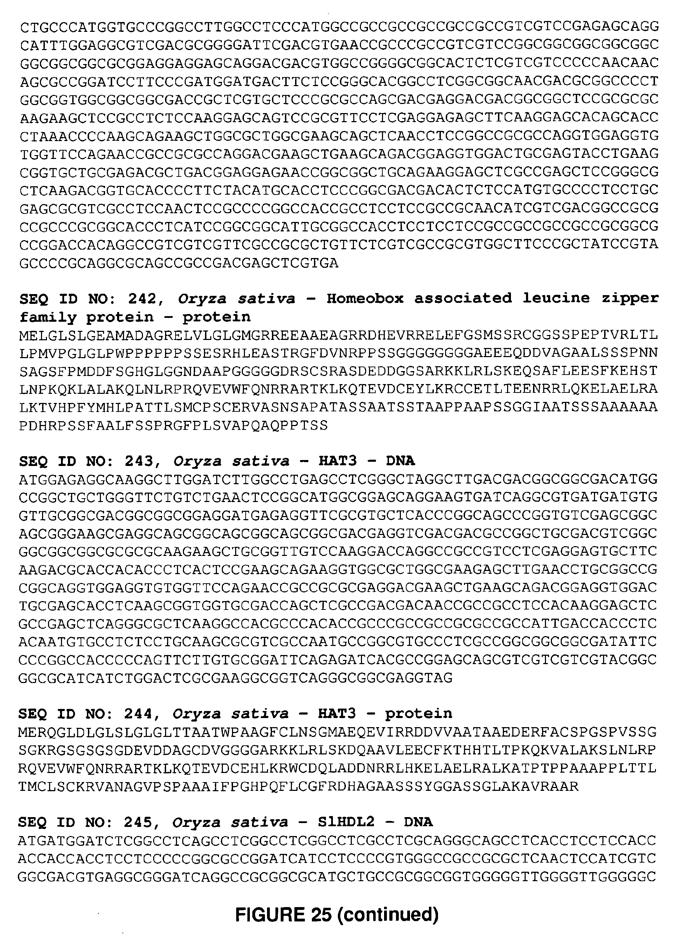

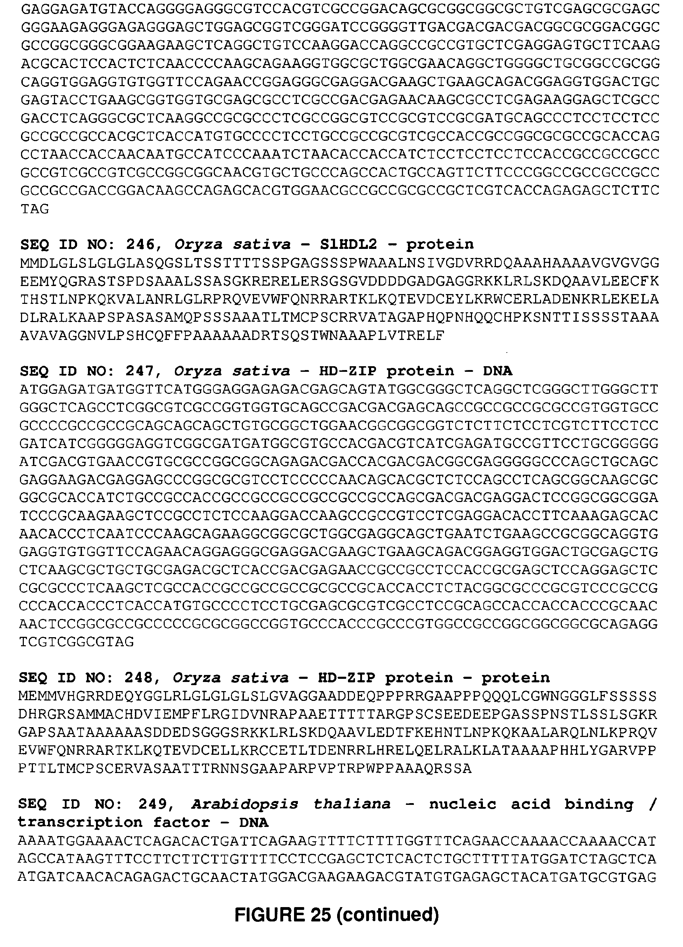

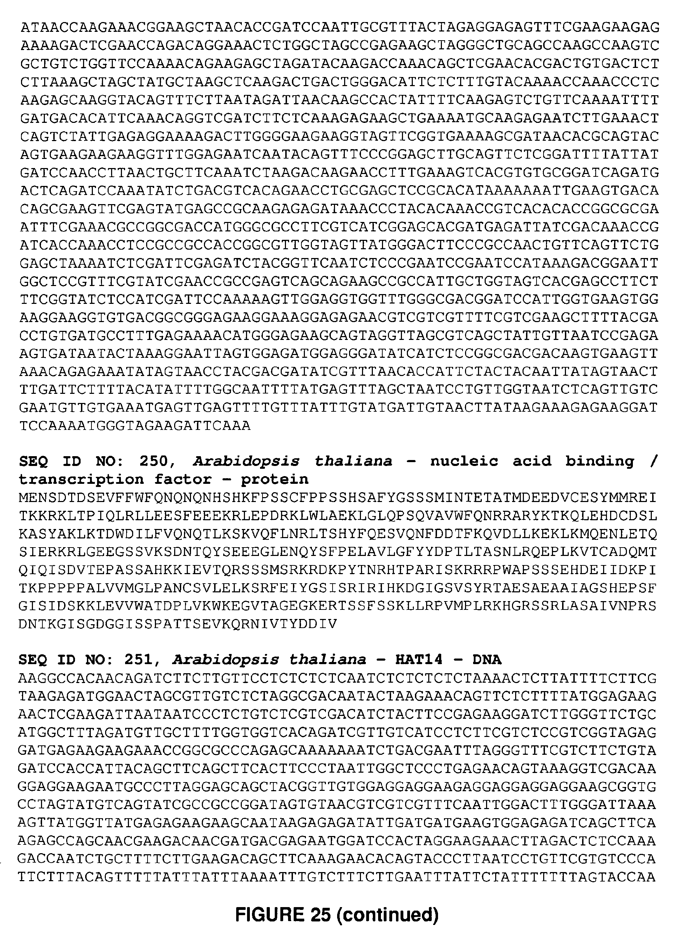

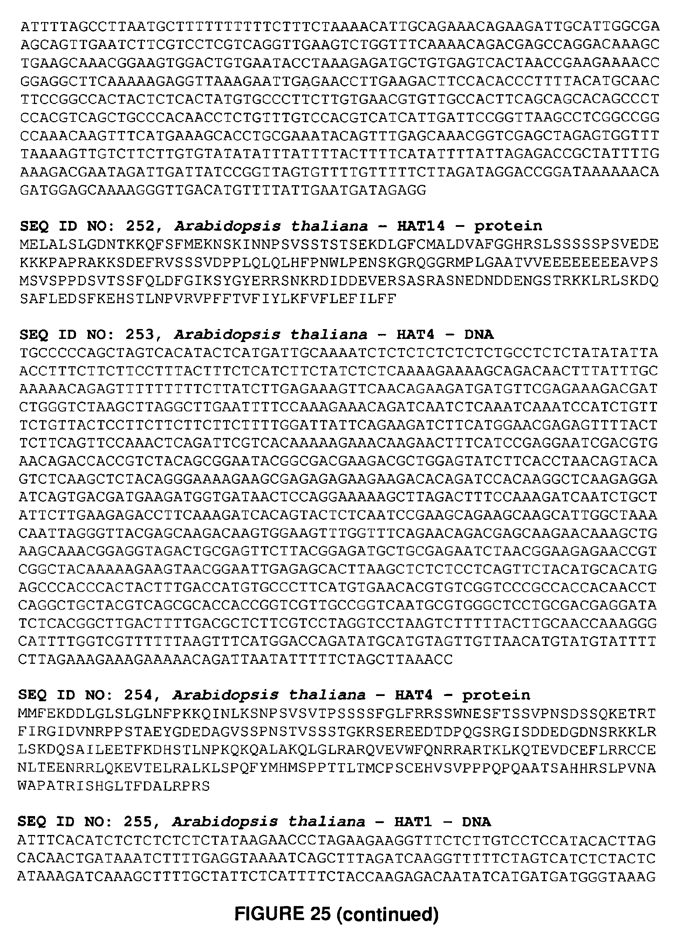

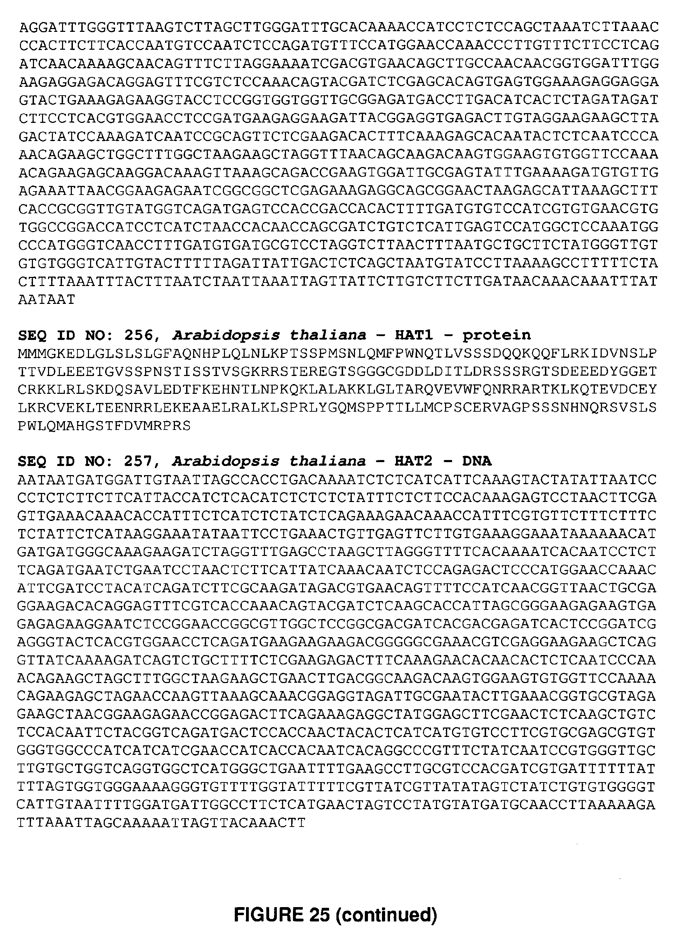

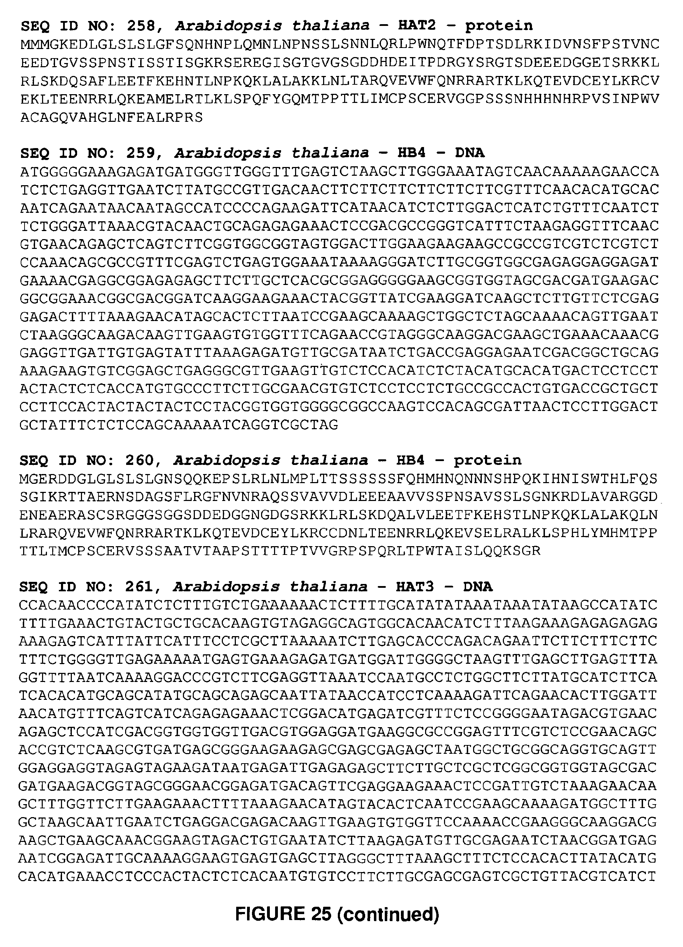

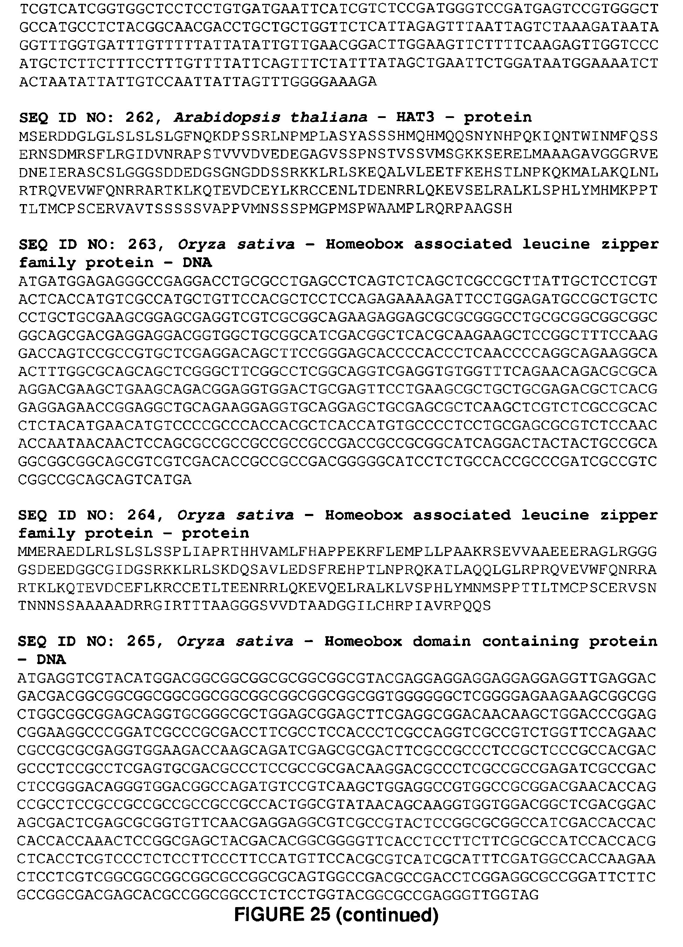

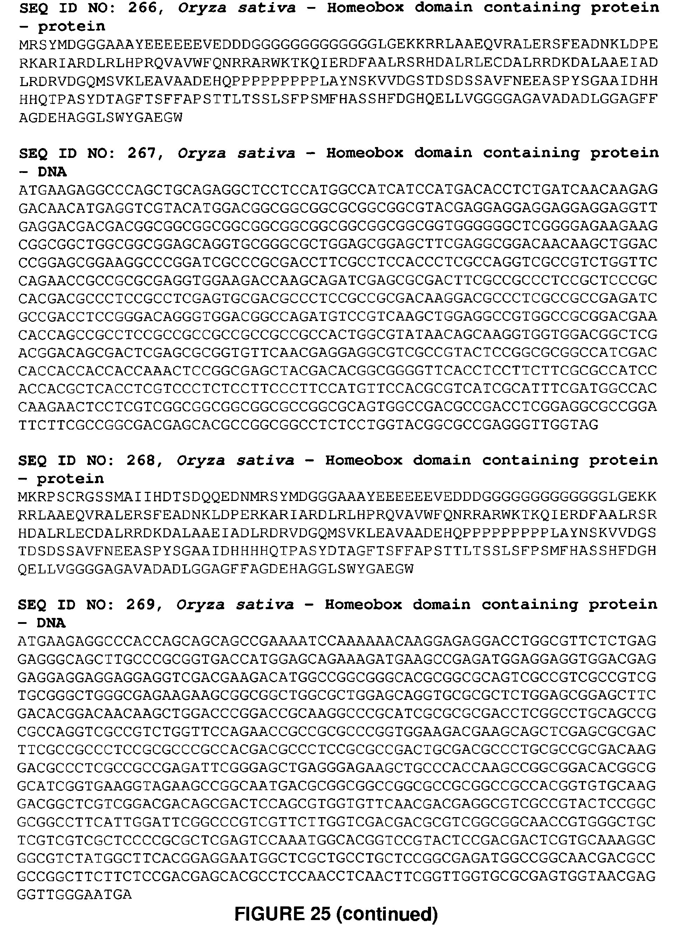

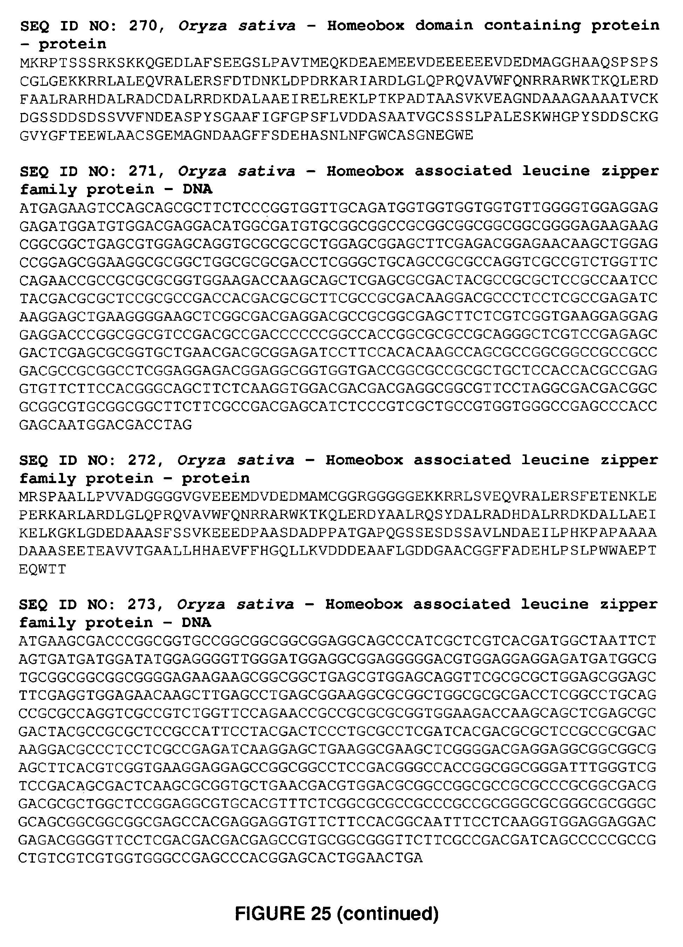

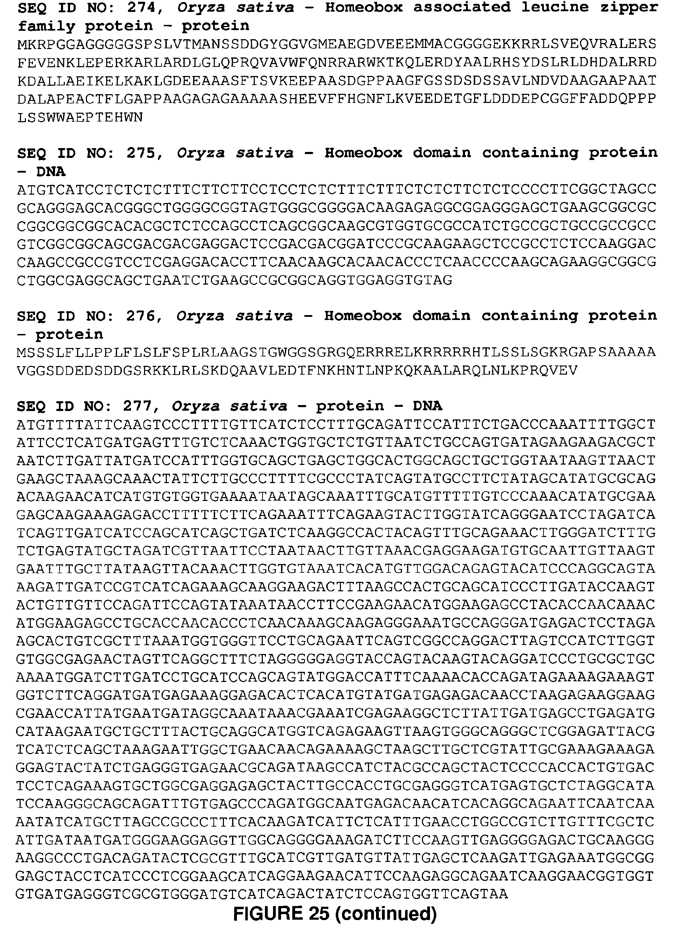

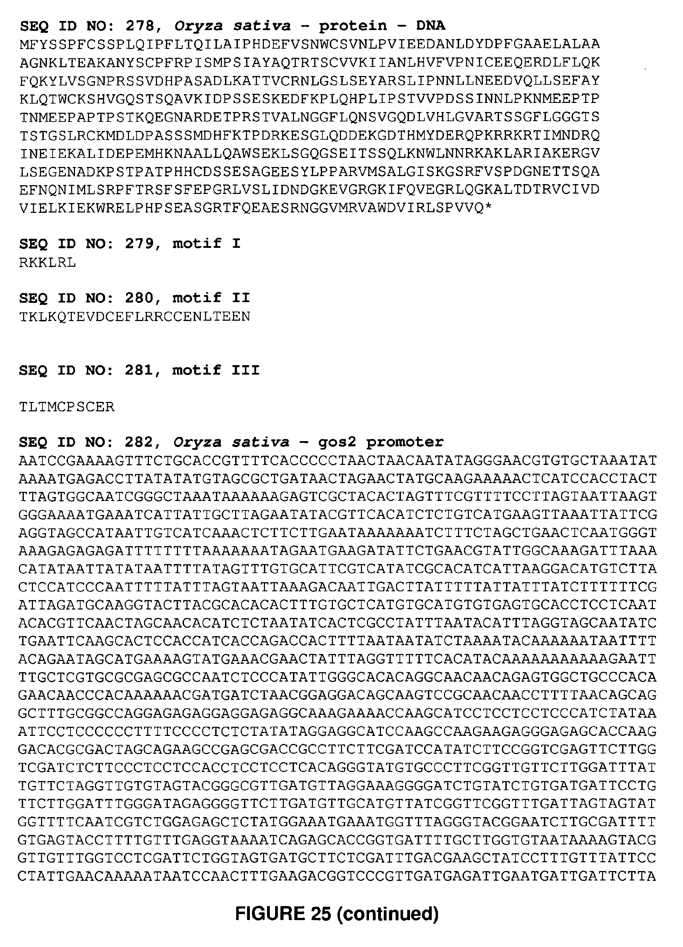

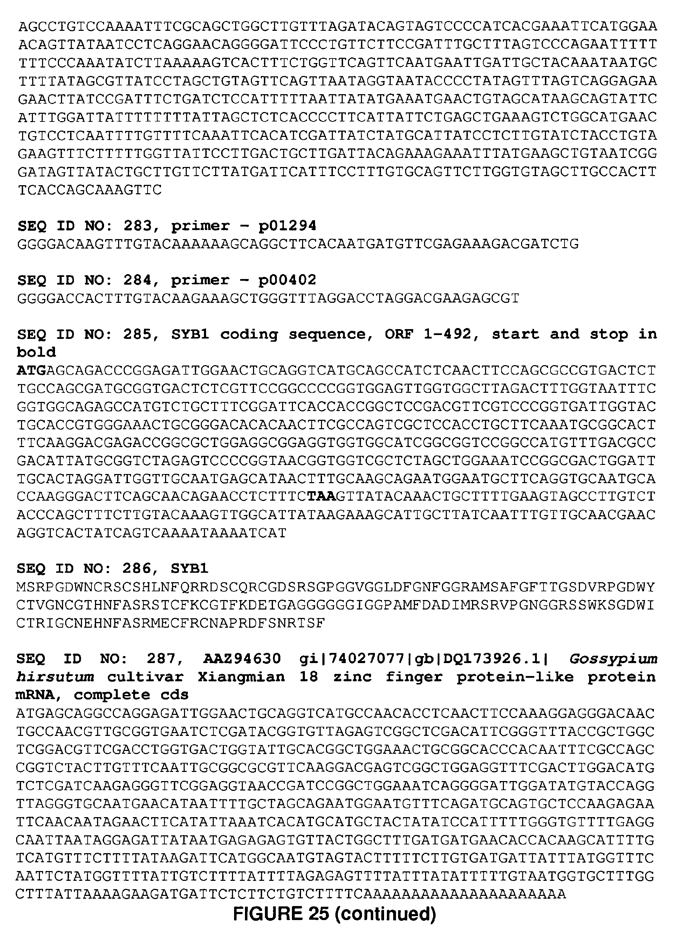

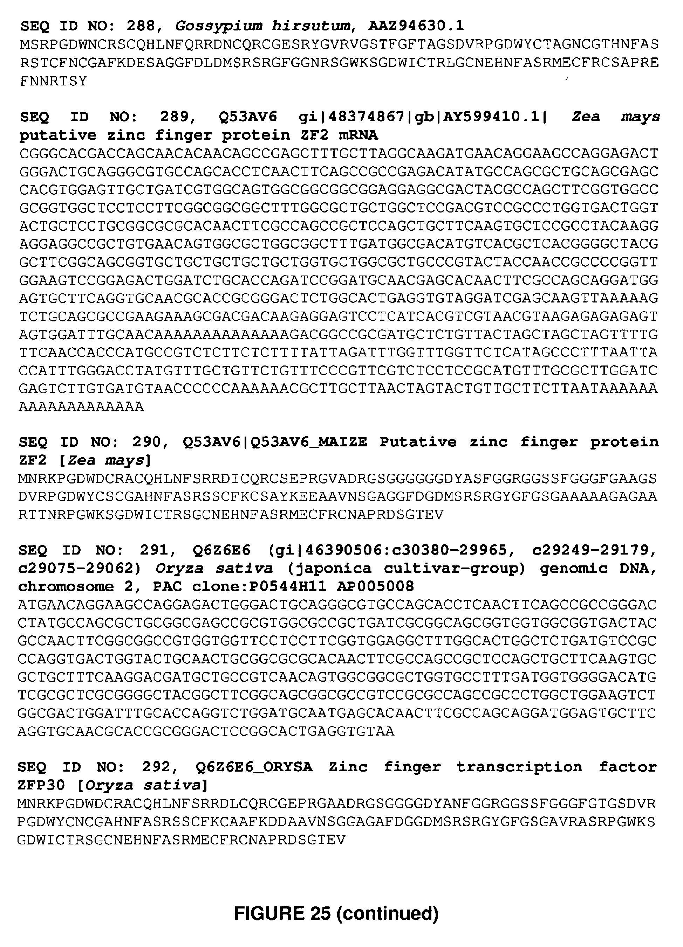

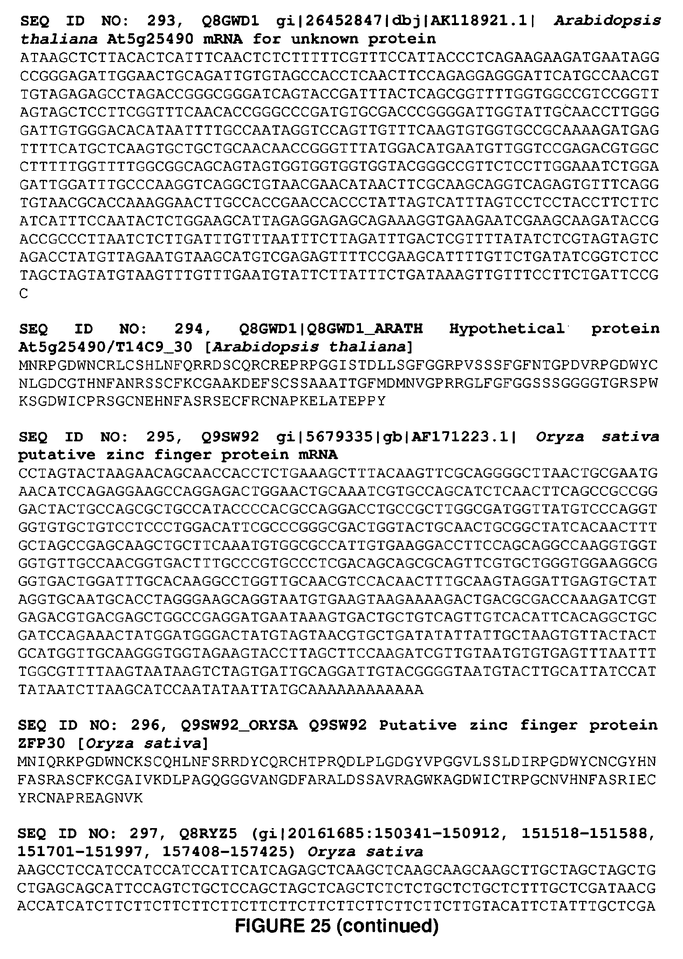

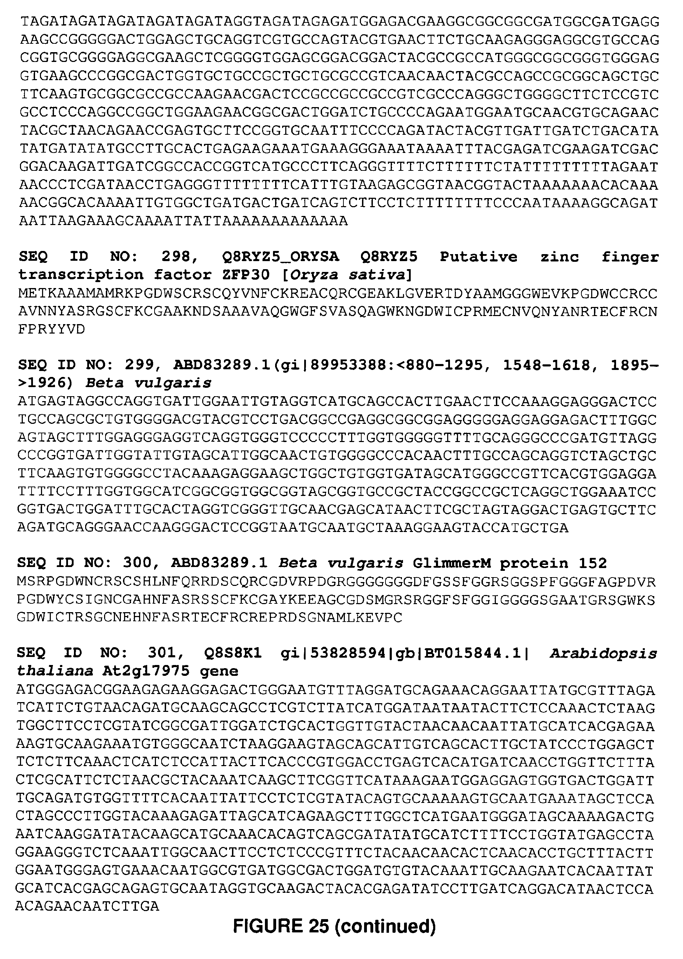

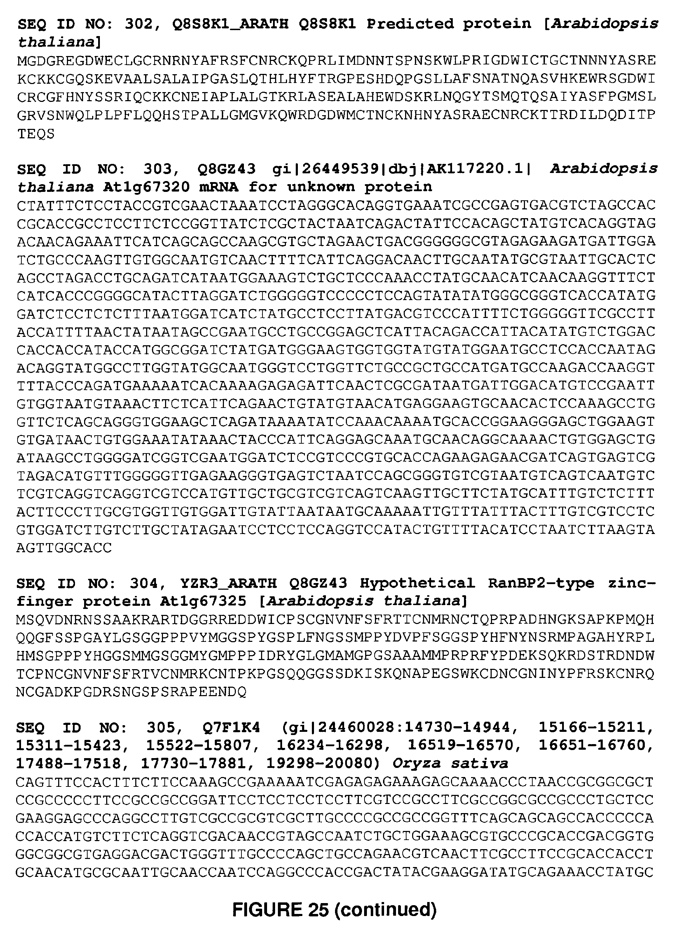

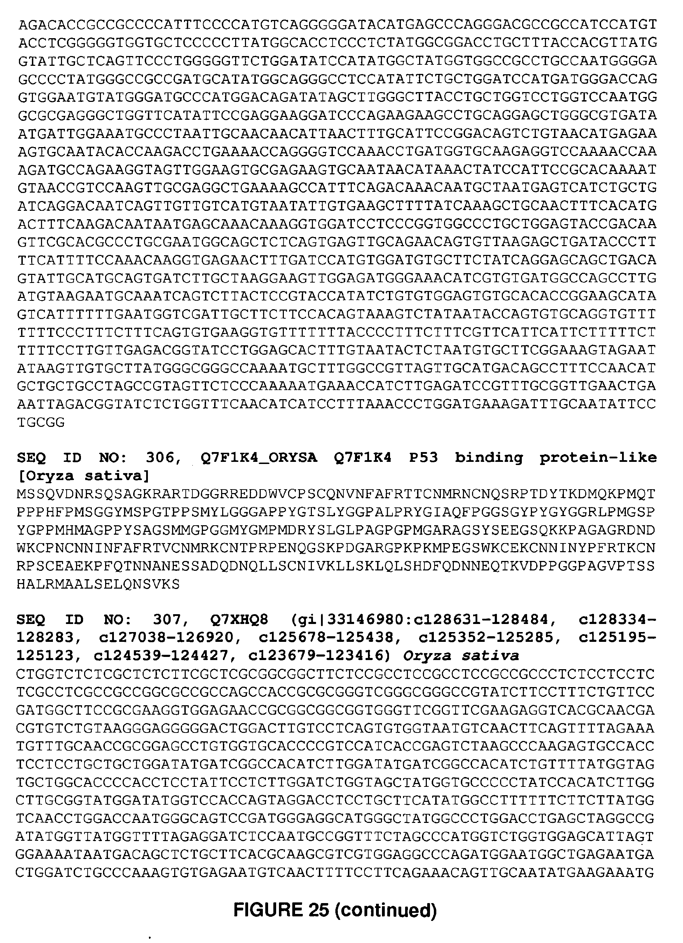

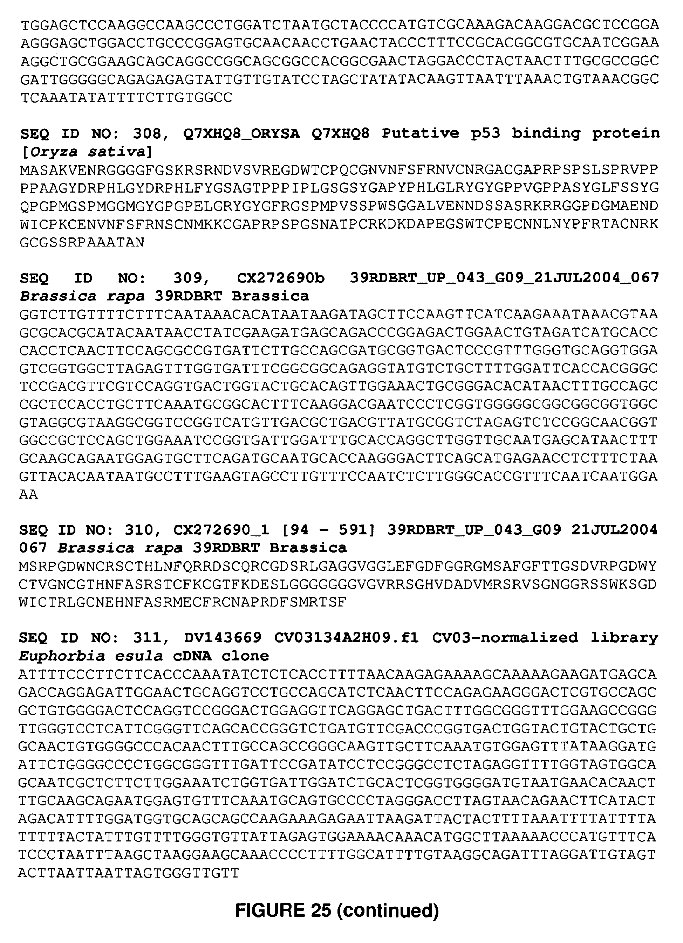

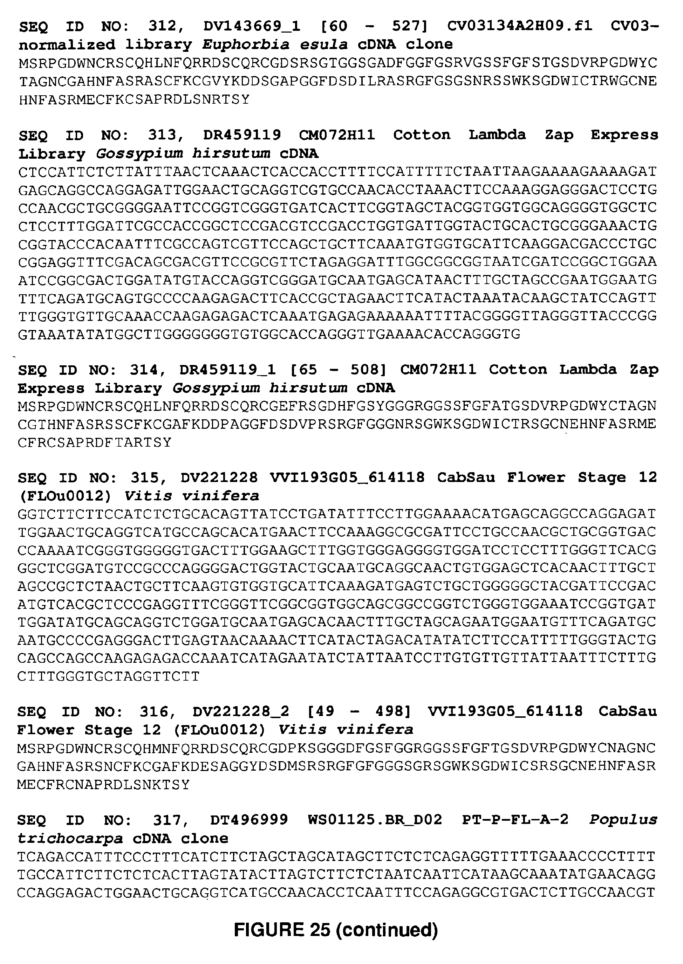

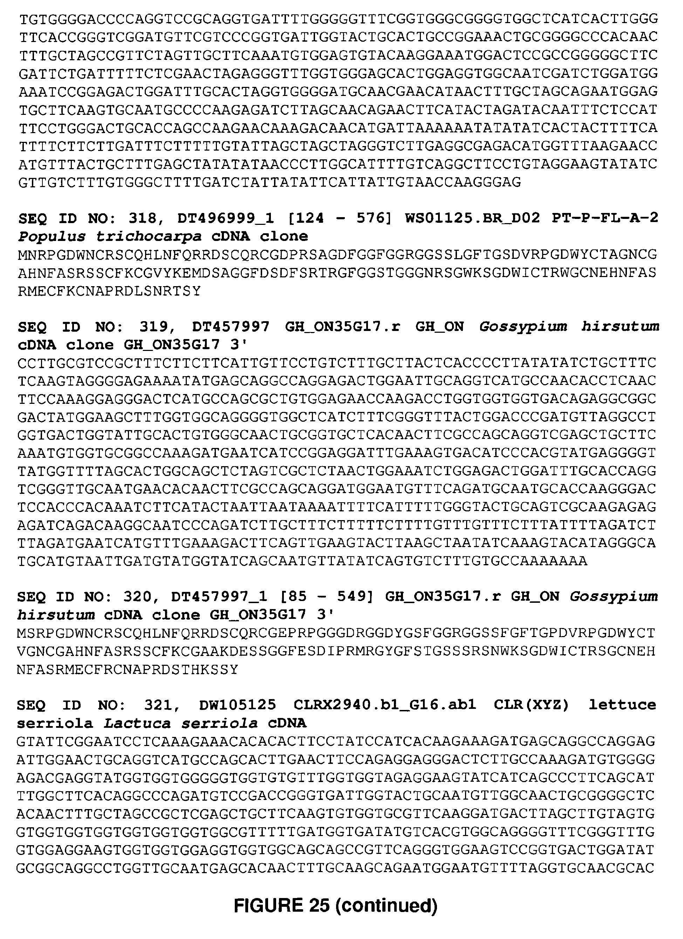

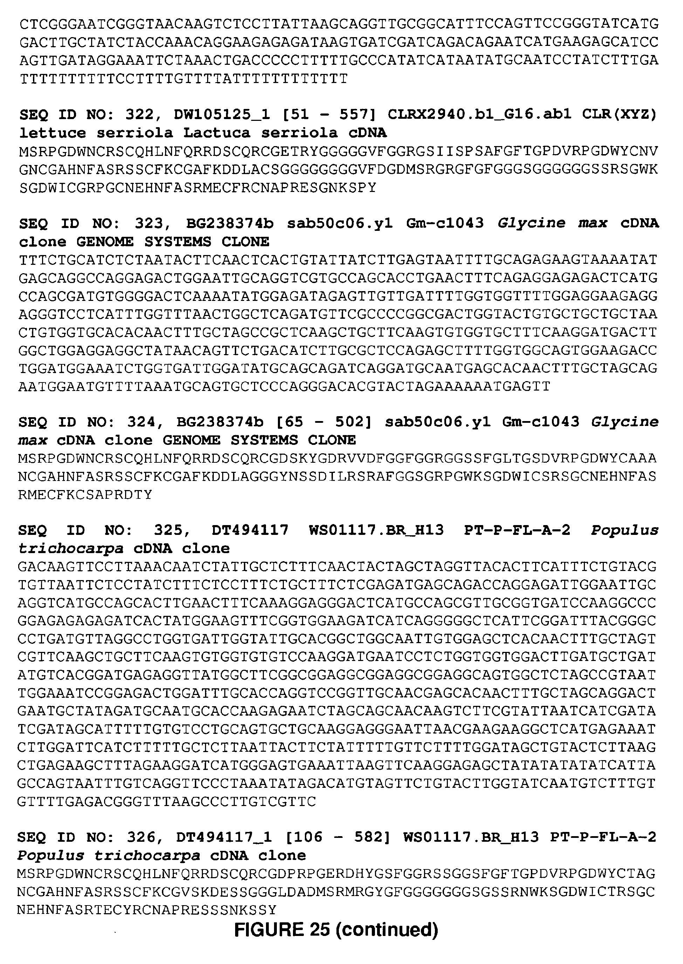

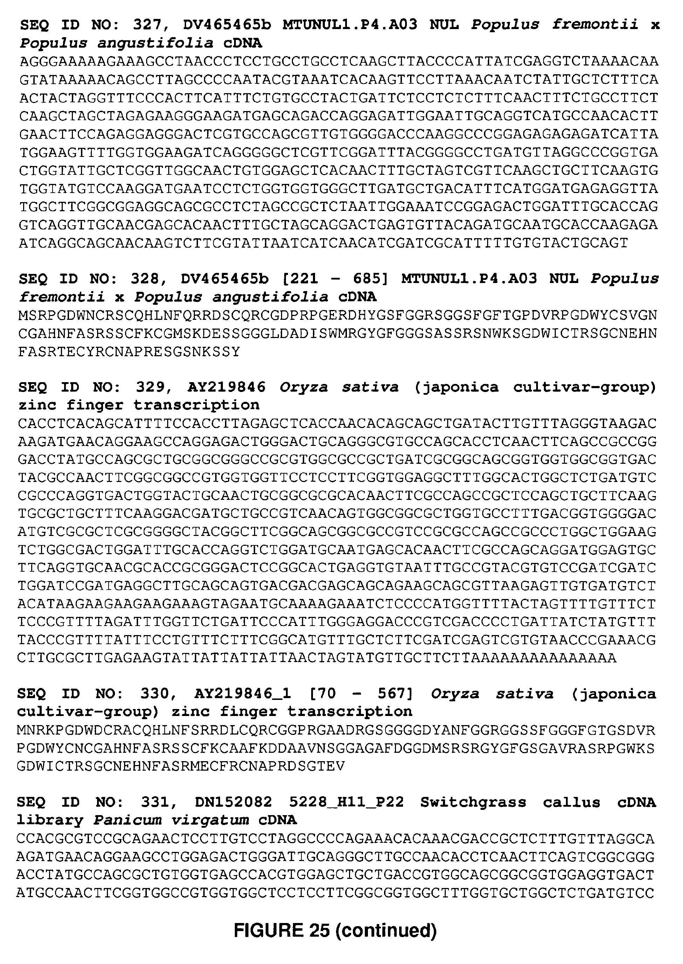

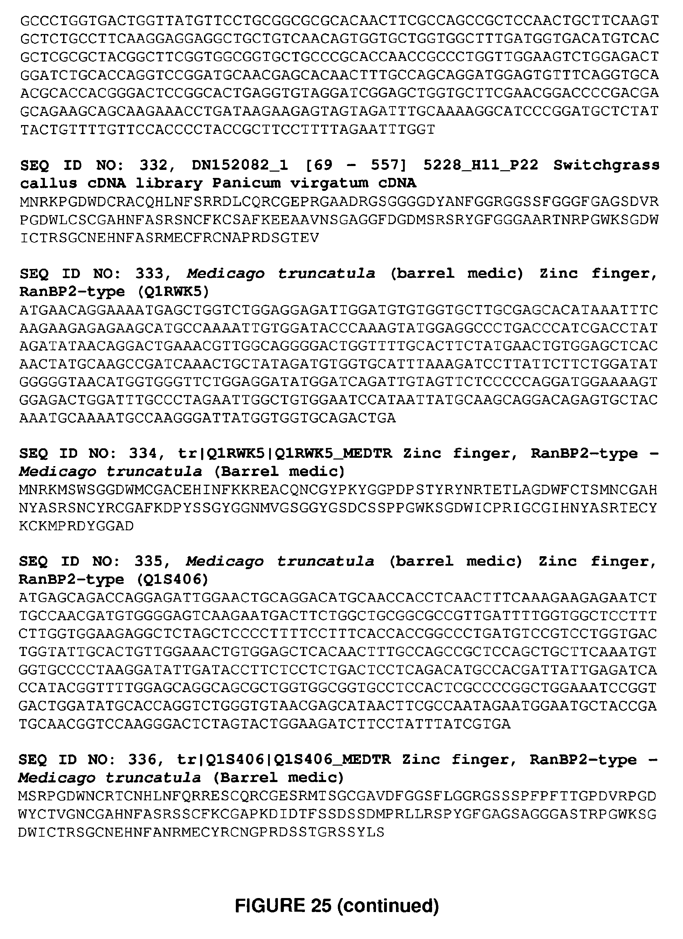

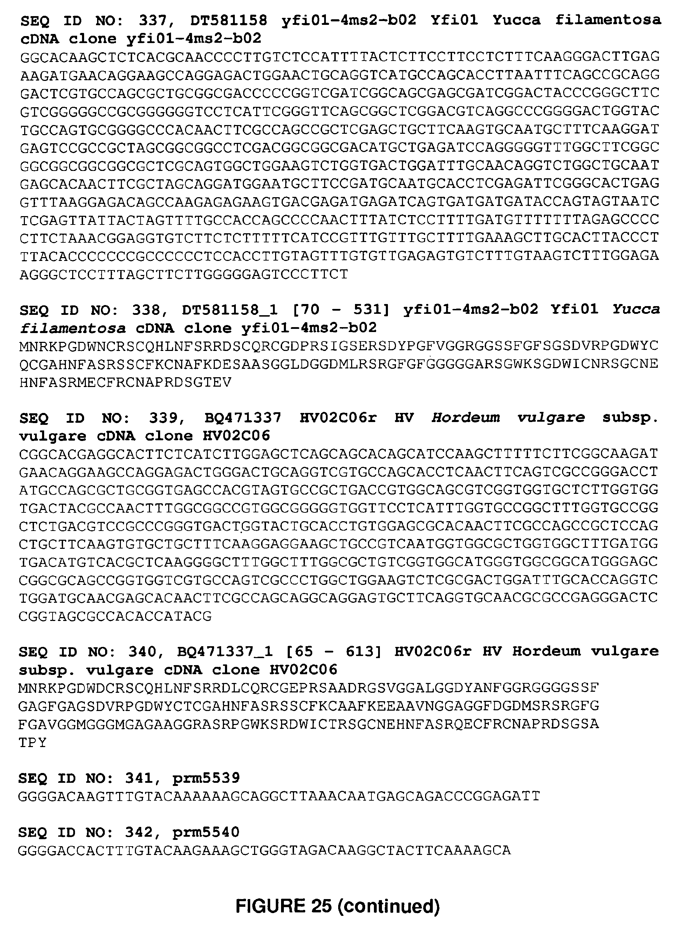

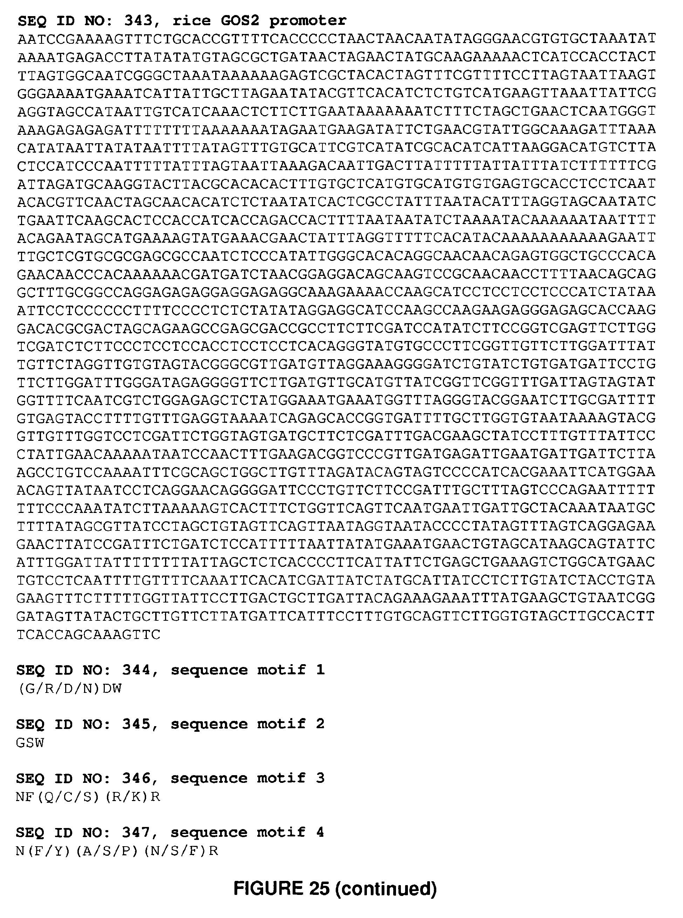

**Fig. 25** details examples of sequences useful in performing the methods according to the present invention. SEQ ID NO: 1 to SEQ ID NO: 56 relate to AZ sequences. SEQ ID NO: 1 and 2 represent the AZ coding sequence CDS3104 and the deduced protein sequence. SEQ ID NO: 53 and 47 represent the AZ coding sequence CDS3108 and the deduced protein sequence. SEQ ID NO: 3 to 6 represent conserved sequences that may be present in an AZ protein, SEQ ID NO: 9 and 55 represent sequences of the WS118 promoter used in the examples section. SEQ ID NO: 10 to 52 are examples of sequences of other AZ proteins and nucleic acids encoding these proteins, SEQ ID NO: 54 and 56 are the sequences of the rice GOS2 promoter.
SEQ ID NO: 56 to 153 relate to SYT sequences. SYT nucleic acid sequences are presented from start to stop. The majority of these sequences are derived from EST sequencing, which is of lower quality. Therefore, nucleic acid substitutions may be encountered.
SEQ ID NO: 56, and SEQ ID NO: 154 to 208 represent examples of sequences relating to cpFBPase, useful in performing the methods according to the present invention.
SEQ ID NO: 56 and 209 to 228 are examples of sequences useful in performing the methods according to the present invention relating to SIK proteins/nudeic acids, or useful in isolating such sequences. Sequences may result from public EST assemblies, with lesser quality sequencing. As a consequence, a few nucleic acid substitutions may be expected. The 5' and 3' UTR of the naturally transcribed sequences may also be used for the performing the methods of the invention for the reduction or substantial elimination of endogenous SIK gene expression.
SEQ ID NO: 229 to 284 are examples of sequences useful in performing the methods according to the present invention relating to Class II HD-Zip transcription factor (HAT4).
SEQ ID NO: 56, and SEQ ID NO: 285 to 347 are examples of sequences relating to SYB1 proteins and nucleic acids and are useful in performing the methods according to the present invention.

## Examples

The present invention will now be described with reference to the following examples, which are by way of illustration alone. The following examples are not intended to completely define or otherwise limit the scope of the invention.

DNA manipulation: unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

### *Example 1: Identification of sequences related to the nucleic acid sequence used in the methods of the invention*

Sequences (full length cDNA, ESTs or genomic) related to the nucleic acid sequence used in the methods of the present invention were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. For example, the polypeptide encoded by the nucleic acid used in the present invention was used for the TBLASTN algorithm, with default settings and the filter to ignore low complexity sequences set off. The output of the analysis was viewed by pairwise comparison, and ranked according to the probability score (E-value), where the score reflect the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search. For example the E-value may be increased to show less stringent matches. This way, short nearly exact matches may be identified.

Table A provides a list of nucleic acid sequences related to the nucleic acid sequence used in the methods of the present invention.

**Table A:** Examples of AZ polypeptides:

| Plant Source | Nucleic acid SEQ ID NO: | Protein SEQ ID NO: |
|---|---|---|
| *Arabidopsis thaliana* | 1 | 2 |
| *Eucalyptus grandis* | 10 | 11 |
| *Oryza sativa* | 12 | 13 |
| *Medicago truncatula* | 14 | 15 |
| *Oryza sativa* | 16 | 17 |
| *Arabidopsis thaliana* | 18 | 19 |
| *Oryza sativa* | 20 | 21 |
| *Arabidopsis thaliana* | 22 | 23 |
| *Arabidopsis thaliana* | 24 | 25 |
| *Eucalyptus grandis* | 26 | 27 |
| *Eucalyptus grandis* | 28 | 29 |
| *Glycine max* | 30 | 31 |
| *Eucalyptus grandis* | 32 | 33 |
| *Arabidopsis thaliana* | 34 | 35 |
| *Oryza sativa* | 36 | 37 |
| *Hordeum_vulgare* | 38 | 39 |
| *Pinus radiata* | 40 | 41 |
| *Pinus radiata* | 42 | 43 |
| *Glycine max* | | 44 |
| *Glycine max* | | 45 |
| *Glycine max* | | 46 |
| *Arabidopsis thaliana* | | 47 |
| *Arabidopsis thaliana* | | 48 |
| *Arabidopsis thaliana* | | 49 |
| *Arabidopsis thaliana* | | 50 |
| *Arabidopsis thaliana* | | 51 |
| *Arabidopsis thaliana* | | 52 |
| *Arabidopsis thaliana* | 53 | |

In some instances, related sequences have tentatively been assembled and publicly disclosed by research institutions, such as The Institute for Genomic Research (TIGR). The Eukaryotic Gene Orthologs (EGO) database may be used to identify such related sequences, either by keyword search or by using the BLAST algorithm with the nucleic acid or polypeptide sequence of interest.

***Example 2: Gene Cloning* of *AZ***

The *Arabidopsis AZ* encoding gene (CDS3104) was amplified by PCR using as template an *Arabidopsis thaliana* seedling cDNA library (Invitrogen, Paisley, UK). After reverse transcription of RNA extracted from seedlings, the cDNAs were cloned into pCMV Sport 6.0. Average insert size of the bank was 1.5 kb, and the original number of clones was of $1.59x10^7$ cfu. Original titer was determined to be $9.6x10^5$ cfu/ml, after a first amplification of $6x10^{11}$ cfu/ml. After plasmid extraction, 200 ng of template was used in a 50 μl PCR mix. Primers prm06717 (sense, AttB1 site in italic, start codon in bold: 5'-*ggggacaagtttgtacaaaaaagca*ggcttaaacaatgtgctgtggatcagacc-3') (SEQ ID NO 7) and prm06718 (reverse, complementary, AttB2 site in italic: 5'-*ggggaccactttgtacaagaaagctgggt*ggttaggtctctcaattctgc-3') (SEQ ID NO 8), which include the AttB sites for Gateway recombination, were used for PCR amplification. PCR was performed using Hifi Taq DNA polymerase in standard conditions. A PCR fragment of the expected size was amplified and purified also using standard methods. The first step of the Gateway procedure, the BP reaction, was then performed, during which the PCR fragment recombines *in vivo* with the pDONR201 plasmid to produce, according to the Gateway terminology, an "entry clone", p07. Plasmid pDONR201 was purchased from Invitrogen, as part of the Gateway® technology.

***Example 3: Vector Construction***

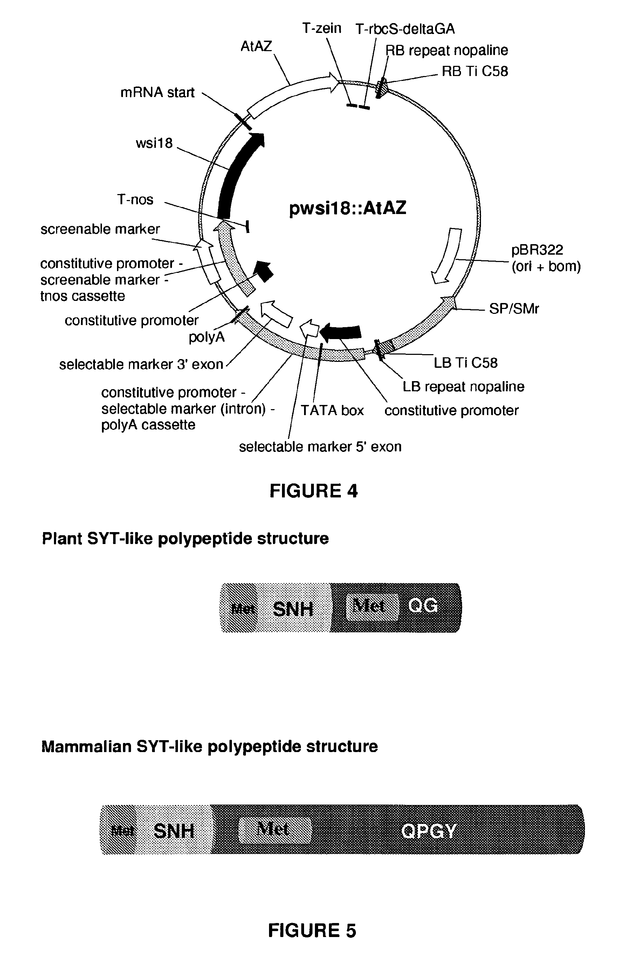

The entry clone p07 were subsequently used in an LR reaction with p02417, a destination vector used for plant (*Oryza sativa*) transformation. This vector contains as functional elements within the T-DNA borders: a plant selectable marker; a screenable marker expression cassette; and a Gateway cassette intended for LR *in vivo* recombination with the sequence of interest already cloned in the entry clone. A rice WSI18 promoter (SEQ ID NO: 9) for seed specific expression (PRO0151) was located upstream of this Gateway cassette (p056, Figure 4).

Many different binary (and super binary) vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey). Many are based on the vector pBIN19 described by Bevan (Nucleic Acid Research. 1984. 12:8711-8721) that includes a plant gene expression cassette flanked by the left and right border sequences from the Ti plasmid of *Agrobacterium tumefaciens.* A plant gene expression cassette consists of at least two genes - a selection marker gene and a plant promoter regulating the transcription of the cDNA or genomic DNA of the trait gene. Various selection marker genes can be used including the *Arabidopsis* gene encoding a mutated acetohydroxy acid synthase (AHAS) enzyme (US patents 57673666 and 6225105). Similarly, various promoters can be used to regulate the trait gene to provide constitutive, developmental, tissue or environmental regulation of gene transcription.

After the LR recombination step, the resulting expression vector, p056 (Figure 4), was transformed into *Agrobacterium* strain LBA4044 using heat shock or electroporation protocols. Transformed colonies were grown on YEP media and selected by respective antibiotics for two days at 28°C. These *Agrobacterium* cultures were used for the plant transformation.

Other *Agrobacterium tumefaciens* strains can be used for plant transformation and are well known in the art. Examples of such strains are C58C1 or EHA105.

***Example 4: Plant transformation***

*Rice transformation*

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 minutes in 0.2%$HgCl_2$, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were subcultured on fresh medium 3 days before co-cultivation (to boost cell division activity).

*Agrobacterium* strain LBA4404 containing the expression vector was used for cocultivation. *Agrobacterium* was inoculated on AB medium with the appropriate antibiotics and cultured for 3 days at 28°C. The bacteria were then collected and suspended in liquid co-cultivation medium to a density (OD600) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25˚C. Co-cultivated calli were grown on 2,4-D-containing medium for 4 weeks in the dark at 28˚C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan et *al.* 1993, Hiei *et al.* 1994).

*Corn transformation*

Transformation of maize (*Zea mays*) is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for tansformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25˚C for 2-3 weeks, or until shoots develop. The green shoots are transferred from each embryo to maize rooting medium and incubated at 25 ˚C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis.

After incubation with Agrobacterium, the embryos are grown *in vitro* on callus induction medium, then regeneration medium, containing the selection agent (for example imidazolinone but various selection markers can be used). The Petri plates are incubated in the light at 25 ˚C for 2-3 weeks, or until shoots develop. The green shoots are transferred from each embryo to rooting medium and incubated at 25 ˚C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseedlcanola transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for *in vitro* sowing. The cotyledon petiole explants with the cotyledon attached are excised from the *in vitro* seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 ˚C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

*Alfalfa transformation*

A regenerating clone of alfalfa (*Medicago sativa*) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58Cl pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2S04, and 100 μm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

*Example 5: Evaluation setup of AZ expression in rice under the control of the rice* ***WSI18*** *promoter*

Approximately 15 to 20 independent T0 rice transformants were generated. The primary transformants were transferred from a tissue culture chamber to a greenhouse for growing and harvest of T1 seed. Seven events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The selected T1 plants were transferred to a greenhouse. Each plant received a unique barcode label to link unambiguously the phenotyping data to the corresponding plant. The selected T1 plants were grown on soil in 10 cm diameter, specially designed pots with transparent bottoms to allow visualization of the roots, under the following environmental settings: photoperiod= 11.5 h, daylight intensity= 30,000 lux or more, daytime temperature= 28˚C, night time temperature= 22˚C, relative humidity= 60-70%. Transgenic plants and the corresponding nullizygotes were grown side-by-side at random positions. Care was taken that the plants were not subjected to any stress. From the stage of sowing until the stage of maturity the plants were passed several times through a digital imaging cabinet. At each time point digital images (2048x1536 pixels, 16 million colours) were taken of each plant from at least 6 different angles. A digital camera also recorded images through the bottom of the pot during plant growth.

*Drought screen*

Plants from T2 seeds are grown in potting soil under normal conditions until they approached the heading stage. They are then transferred to a "dry" section where irrigation is withheld. Humidity probes are inserted in randomly chosen pots to monitor the soil water content (SWC). When SWC goes below certain thresholds, the plants are automatically re-watered continuously until a normal level was reached again. The plants are then re-transferred again to normal conditions. The rest of the cultivation (plant maturation, seed harvest) is the same as for plants not grown under abiotic stress conditions. Growth and yield parameters are recorded as detailed for growth under normal conditions.

*Nitrogen use efficiency screen*

Rice plants from T2 seeds are grown in potting soil under normal conditions except for the nutrient solution.

The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Growth and yield parameters are recorded as detailed for growth under normal conditions.

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the time point at which the plant had reached its maximal leafy biomass. Root features such as total projected area (which can be correlated to total root volume), average diameter and length of roots above a certain thickness threshold (length of thick roots, or length of thin roots) were deduced from the generated image using appropriate software.

The mature primary panicles were harvested, bagged, barcode-labelled and then dried for three days in the oven at 37˚C. The panicles were then threshed and all the seeds collected. The filled husks were separated from the empty ones using an air-blowing device. After separation, both seed lots were then counted using a commercially available counting machine. The empty husks were discarded. The filled husks were weighed on an analytical balance and the cross-sectional area of the seeds was measured using digital imaging. This procedure resulted in the set of the following seed-related parameters:

The number of filled seeds was determined by counting the number of filled husks that remained after the separation step. The total seed yield (total seed weight) was measured by weighing all filled husks harvested from a plant. Total seed number per plant was measured by counting the number of husks harvested from a plant. The harvest index (HI) in the present invention is defined as the ratio between the total seed yield and the above ground area ($mm^2$), multiplied by a factor $10^6$. Thousand Kernel Weight (TKW) is extrapolated from the number of filled seeds counted and their total weight. The seed fill rate as defined in the present invention is the proportion (expressed as a %) of the number of filled seeds over the total number of seeds (or florets). These parameters were derived in an automated way from the digital images using image analysis software and were analysed statistically. Individual seed parameters (including width, length, area, weight) were measured using a custom-made device consisting of two main components, a weighing and imaging device, coupled to software for image analysis.

A two factor ANOVA (analyses of variance) corrected for the unbalanced design was used as statistical model for the overall evaluation of plant phenotypic characteristics. An F-test was carried out on all the parameters measured of all the plants of all the events transformed with that gene. The F-test was carried out to check for an effect of the gene over all the transformation events and to verify for an overall effect of the gene, also named herein "global gene effect". If the value of the F test shows that the data are significant, than it is concluded that there is a "gene" effect, meaning that not only presence or the position of the gene is causing the effect. The threshold for significance for a true global gene effect is set at 5% probability level for the F test.

To check for an effect of the genes within an event, i.e., for a line-specific effect, a t-test was performed within each event using data sets from the transgenic plants and the corresponding null plants. "Null plants" or "null segregants" or "nullizygotes" are the plants treated in the same way as the transgenic plant, but from which the transgene has segregated. Null plants can also be described as the homozygous negative transformed plants. The threshold for significance for the t-test is set at 10% probability level. The results for some events can be above or below this threshold. This is based on the hypothesis that a gene might only have an effect in certain positions in the genome, and that the occurrence of this position-dependent effect is not uncommon. This kind of gene effect is also named herein a "line effect of the gene". The p-value is obtained by comparing the t-value to the t-distribution or alternatively, by comparing the F-value to the F-distribution. The p-value then gives the probability that the null hypothesis (i.e., that there is no effect of the transgene) is correct.

The data obtained for AZ in the first experiment were confirmed in a second experiment with T2 plants. Four lines that had the correct expression pattern were selected for further analysis. Seed batches from the positive plants (both hetero- and homozygotes) in T1, were screened by monitoring marker expression. For each chosen event, the heterozygote seed batches were then retained for T2 evaluation. Within each seed batch an equal number of positive and negative plants were grown in the greenhouse for evaluation.

A total number of 120 AZ transformed plants were evaluated in the T2 generation, that is 30 plants per event of which 15 positives for the transgene, and 15 negatives.

Because two experiments with overlapping events had been carried out, a combined analysis was performed. This is useful to check consistency of the effects over the two experiments, and if this is the case, to accumulate evidence from both experiments in order to increase confidence in the conclusion. The method used was a mixed-model approach that takes into account the multilevel structure of the data (i.e. experiment - event - segregants). P-values are obtained by comparing likelihood ratio test to chi square distributions.

***Example 6: Evaluation of AZ transformants: measurement of yield-related parameters***

Upon analysis of the seeds as described above, the inventors found that plants transformed with the AZ gene construct had a higher seed yield, expressed as thousand kernel weight, compared to plants lacking the AZ transgene. Furthermore, increased emergence vigour and increased greenness index was observed in plants carrying the transgene compared to the control plants.

For one of the constructs, thousand-kernel weight was increased with 2.7% in the T1 generation. These positive results were again obtained in the T2 generation (increase of 2.1%). The T2 data were re-evaluated in a combined analysis with the results for the T1 generation, and the obtained p-values showed that the observed effects were highly significant.

***Example 7: Gene Cloning of AtSYT1***

The *Arabidopsis thaliana* AtSYT1 gene was amplified by PCR using as template an *Arabidopsis thaliana* seedling cDNA library (Invitrogen, Paisley, UK). After reverse transcription of RNA extracted from seedlings, the cDNAs were cloned into pCMV Sport 6.0. Average insert size of the bank was 1.5 kb and the original number of clones was of the order of 1.59 x $10^7$ cfu. Original titer was determined to be 9.6 x $10^5$ cfu/ml after first amplification of 6x$10^{11}$ cfu/ml. After plasmid extraction, 200 ng of template was used in a 50 μl PCR mix. Primers prm06681 (SEQ ID NO: 148; sense, start codon in bold, AttB1 site in italic: 5'-GGGGACAA*GTTTGTACAAAAAAGCAGG*CTTAAACA**ATG**CAACAGCACCTGATG -3') and prm06682 (SEQ ID NO: 149; reverse, complementary, AttB2 site in italic: 5'-GGGGACCA*CTTTGTACAAGAAAGCTGGG*TCATCATTAAGATTCCTTGTGC-3'), which include the AttB sites for Gateway recombination, were used for PCR amplification. PCR was performed using Hifi Taq DNA polymerase in standard conditions. A PCR fragment of 727 bp (including attB sites) was amplified and purified also using standard methods. The first step of the Gateway procedure, the BP reaction, was then performed, during which the PCR fragment recombines *in vivo* with the pDONR201 plasmid to produce, according to the Gateway terminology, an "entry clone", p07466. Plasmid pDONR201 was purchased from Invitrogen, as part of the Gateway® technology.

***Example 8: Vector Construction***

The entry clone p07466 was subsequently used in an LR reaction with p00640, a destination vector used for plant (*Oryza sativa*) transformation. This vector contains as functional elements within the T-DNA borders: a plant selectable marker; a screenable marker expression cassette; and a Gateway cassette intended for LR *in vivo* recombination with the sequence of interest already cloned in the entry clone. A rice GOS2 promoter (SEQ ID NO: 145) for constitutive expression (PRO0129) was located upstream of this Gateway cassette.

Many different binary (and super binary) vector systems have been described for plant transformation (e.g. An, G. in Agrobacterium Protocols. Methods in Molecular Biology vol 44, pp 47-62, Gartland KMA and MR Davey eds. Humana Press, Totowa, New Jersey). Many are based on the vector pBIN19 described by Bevan (Nucleic Acid Research. 1984. 12:8711-8721) that includes a plant gene expression cassette flanked by the left and right border sequences from the Ti plasmid of Agrobacterium tumefaciens. A plant gene expression cassette consists of at least two genes - a selection marker gene and a plant promoter regulating the transcription of the cDNA or genomic DNA of the trait gene. Various selection marker genes can be used including the Arabidopsis gene encoding a mutated acetohydroxy acid synthase (AHAS) enzyme (US patents 57673666 and 6225105). Similarly, various promoters can be used to regulate the trait gene to provide constitutive, developmental, tissue or environmental regulation of gene transcription.

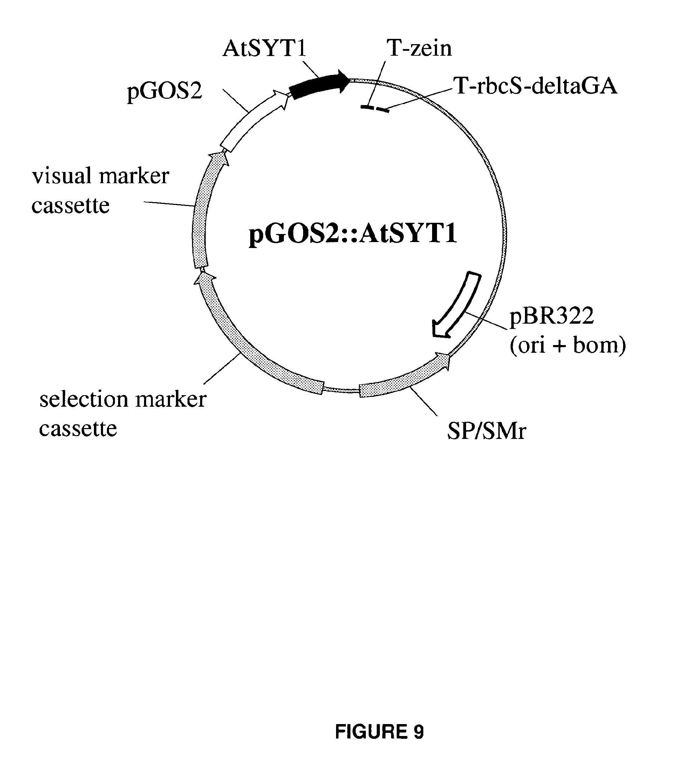

After the LR recombination step, the resulting expression vector pGOS2::AtSYT1 (Figure 9) was transformed into *Agrobacterium* strain LBA4044 using heat shock or electroporation protocols. Transformed colonies were grown on YEP media and selected by respective antibiotics for two days at 28°C. These *Agrobacterium* cultures were used for the plant transformation.

Other *Agrobacterium tumefaciens* strains can be used for plant transformation and are well known in the art. Examples of such strains are C58C1 or EHA105.

***Example 9: Plant transformation***

*Rice transformation*

The *Agrobacterium* containing the expression vector was used to transform *Oryza sativa* plants. Mature dry seeds of the rice japonica cultivar Nipponbare were dehusked. Sterilization was carried out by incubating for one minute in 70% ethanol, followed by 30 minutes in 0.2%$HgCl_2$, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were subcultured on fresh medium 3 days before co-cultivation (to boost cell division activity).

*Agrobacterium* strain LBA4404 containing the expression vector was used for cocultivation. *Agrobacterium* was inoculated on AB medium with the appropriate antibiotics and cultured for 3 days at 28˚C. The bacteria were then collected and suspended in liquid co-cultivation medium to a density (OD600) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25˚C. Co-cultivated calli were grown on 2,4-D-containing medium for 4 weeks in the dark at 28˚C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan *et al.* 1993, Hiei *et al.* 1994).

*Corn transformation*

Transformation of maize (Zea *mays*) is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for tansformation, but other genotypes can be used successfully as well. Ears are harvested from corn plant approximately 11 days after pollination (DAP) when the length of the immature embryo is about 1 to 1.2 mm. Immature embryos are cocultivated with *Agrobacterium tumefaciens* containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 ˚C for 2-3 weeks, or until shoots develop. The green shoots are transferred from each embryo to maize rooting medium and incubated at 25 ˚C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with *Agrobacterium tumefaciens* containing the expression vector, and transgenic plants are recovered through organogenesis. After incubation with Agrobacterium, the embryos are grown *in vitro* on callus induction medium, then regeneration medium, containing the selection agent (for example imidazolinone but various selection markers can be used). The Petri plates are incubated in the light at 25 ˚C for 2-3 weeks, or until shoots develop. The green shoots are transferred from each embryo to rooting medium and incubated at 25 ˚C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for *in vitro* sowing. The hypocotyl, the radicle and one cotyledon are excised from seven-day old young seedlings. The epicotyl and the remaining cotyledon are further grown to develop axillary nodes. These axillary nodes are excised and incubated with *Agrobacterium tumefaciens* containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseedlcanola transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for *in vitro* sowing. The cotyledon petiole explants with the cotyledon attached are excised from the *in vitro* seedlings, and inoculated with *Agrobacterium* (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 ˚C, 16 hr light. After two days of co-cultivation with *Agrobacterium,* the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

*Alfalfa transformation*

A regenerating clone of alfalfa (*Medicago sativa*) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of *Agrobacterium tumefaciens* C58Cl pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 $\mu$m acetosyringinone. The explants are washed in half-strength Murashige-Skoog medium (Murashige and Skoog, 1962) and plated on the same SH induction medium without acetosyringinone but with a suitable selection agent and suitable antibiotic to inhibit *Agrobacterium* growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

***Example 10: Evaluation setup of AtSYT1 transgenic rice plants under abiotic stress***

Four events, of which the T1 progeny segregated 3:1 for presence/absence of the transgene, were retained. For each of these events, approximately 15 T1 seedlings containing the transgene (hetero- and homo-zygotes) and approximately 15 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), with temperatures on average 28˚C in the light and 22˚C in the dark, and a relative humidity on average of 70%.

*Salt stress screen*

Plants were grown on a substrate made of coco fibers and argex (3 to 1 ratio). A normal nutrient solution was used during the first two weeks after transplanting the plantlets in the greenhouse. After the first two weeks, 25 mM of salt (NaCl) was added to the nutrient solution, until the plants were harvested. Seed-related parameters were then measured.

*Drought screen*

Plants are grown in potting soil under normal conditions until they approach the heading stage. They are then transferred to a "dry" section where irrigation is withheld. Humidity probes are inserted in randomly chosen pots to monitor the soil water content (SWC). When SWC goes below certain thresholds, the plants are automatically re-watered continuously until a normal level is reached again. The plants are then re-transferred again to normal conditions. The rest of the cultivation (plant maturation, seed harvest) is the same as for plants not grown under abiotic stress conditions. Seed-related parameters are then measured

An alternative method to impose water stress on the transgenic plants is by treatment with water containing an osmolyte such as polyethylene glycol (PEG) at specific water potential. Since PEG may be toxic, the plants are given only a short-term exposure and then normal watering is resumed.

*Reduced nutrient (nitrogen) availability screen*

The rice plants are grown in potting soil under normal conditions except for the nutrient solution. The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Seed-related parameters are then measured

**Statistical analysis: F-test**

A two factor ANOVA (analysis of variants) was used as a statistical model for the overall evaluation of plant phenotypic characteristics. An F-test was carried out on all the parameters measured of all the plants of all the events transformed with the gene of the present invention. The F-test was carried out to check for an effect of the gene over all the transformation events and for an overall effect of the gene, also known as a global gene effect. The threshold for significance for a true global gene effect was set at a 5% probability level for the F-test. A significant F-test value points to a gene effect, meaning that it is not only the presence or position of the gene that is causing the differences in phenotype.

**Biomass-related parameter measurement**

From the stage of sowing until the stage of maturity the plants were passed several times through a digital imaging cabinet. At each time point digital images (2048x1536 pixels, 16 million colours) were taken of each plant from at least 6 different angles.

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the time point at which the plant had reached its maximal leafy biomass. The early vigour is the plant (seedling) aboveground area three weeks post-germination.

**Seed-related parameter measurements**

The mature primary panicles were harvested, counted, bagged, barcode-labeled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand kernel weight (TKW) is extrapolated from the number of filled seeds counted and their total weight. The harvest index (HI) in the present invention is defined as the ratio between the total seed yield and the above ground area ($mm^2$), multiplied by a factor $10^6$. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

***Example 11: Results of the evaluation of AtSYT1 transgenic rice plants under abiotic stress (salt stress)***

The results of the evaluation of AtSYT1 transgenic rice plants submitted to salt stress are presented in Table B. The percentage difference between the transgenics and the corresponding nullizygotes is also shown, with a P value from the F test below 0.05. Aboveground biomass, early vigour, total seed yield, number of filled seeds, seed fill rate, TKW and harvest index are significantly increased in the AtSYT1 transgenic plants compared to the control plants (in this case, the nullizygotes), under abiotic stress.

**Table B:** Results of the evaluation of AtSYT1 transgenic rice plants under abiotic stress.

| Trait | % Difference |
|---|---|
| Aboveground biomass | 19 |
| Early vigour | 18 |
| Total seed yield | 30 |
| Nbr of filled seeds | 18 |
| Fill rate | 15 |
| TKW | 10 |
| Harvest index | 16 |

***Example 12: Identification of sequences related to SEQ ID NO: 154 and SEQ ID NO: 155***

Sequences (full length cDNA, ESTs or genomic) related to SEQ ID NO: 154 and SEQ ID NO: 155 were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. The polypeptide encoded by SEQ ID NO: 154 was used for the TBLASTN algorithm, with default settings and the filter to ignore low complexity sequences set off. The output of the analysis was viewed by pairwise comparison, and ranked according to the probability score (E-value), where the score reflects the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search.

In addition to the publicly available nucleic acid sequences available at NCBI, proprietary sequence databases are also searched following the same procedure as described herein above.

Table C provides a list of nucleic acid sequences related to the nucleic acid sequence as represented by SEQ ID NO: 154.

**Table C:** Nucleic acid sequences related to the nucleic acid sequence (SEQ ID NO: 154) useful in the methods of the present invention, and the corresponding deduced polypeptides.

| Name | Source organism | Nucleic acid SEQ ID NO: | Polypeptide SEQ ID NO: | Database accession number | Status |
|---|---|---|---|---|---|
| Chlre_ cpFBPase | *Chlamydomonas reinhardtii* | 154 | 155 | Derived from<br>AW721488<br>BQ811919<br>CF555540 | Full length |
| Bigna_ cpFBPase | *Bigelowiella natans* | 156 | 157 | AY267678 | Full length |
| Aqufo_ cpFBPase | *Aquilegia formosa x Aquilegia pubescens* | 158 | 159 | DR944021<br>DT749545 | Full length |
| Arath_ cpFBPase | *Arabidopsis thaliana* | 160 | 161 | X58148 | Full length |
| Brana_ cpFBPase | *Brassica napus* | 162 | 163 | AF081796 | Full length |

(continued)

| Name | Source organism | Nucleic acid SEQ ID NO: | Polypeptide SEQ ID NO: | Database accession number | Status |
|---|---|---|---|---|---|
| Cyame_ cpFBPase | *Cyanidioschyzon merolae* | 164 | 165 | CMO245C* | Full length |
| Glyma_ cpFBPase | *Glycine max* | 166 | 167 | L34841 | Full length |
| Lyces_ cpfBPase | *Lycopersicon esculentum* | 168 | 169 | BT014319 | Full length |
| Medtr_ cpFBPase | *Medicago truncatula* | 170 | 171 | B1310407<br>B1265103<br>CO516140 | Full length |
| Nicta_ cpFBPase | *Nicotiana tabacum* | 172 | 173 | DW000613<br>EB680028 | Full length |
| Orysa_ cpFBPase | *Oryza sativa* | 174 | 175 | AB007194 | Full length |
| Ostlu_ cpFBPase | *Ostreococcus lucimarinus* | 176 | 177 | jgilOst9901_319 2356joust_03_00 2_042** | Full length |
| Ostta_ cpFBPase | *Ostreococcus tauri* | 178 | 179 | CR954203 | Full length |
| Phatr_ cpFBPase | *Phaeodactylum tricornutum* | 180 | 181 | scaffold_27: 145102-145476** | Full length |
| Pissa_ cpFBPase | *Pisum sativa* | 182 | 183 | L34806 | Full length |
| Pontr_ cpFBPase | *Poncirus trifoliata* | 184 | 185 | CV705787<br>CV705786 | Full length |
| Poptr_ cpFBPase | *Populus tremuloides* | 186 | 187 | CV241715<br>DT474034 | Full length |
| Soltu_ cpFBPase | *Solanum tuberosum* | 188 | 189 | AF1340451 | Full length |
| Spiol_ cpFBPase | *Spinacia oleracea* | 190 | 191 | L76555 | Full length |
| Triae_ cpFBPase | *Triticum aestivum* | 192 | 193 | X07780 | Full length |
| Zeama_ cpFBPase | *Zea mays* | 194 | 195 | DR792524.1<br>DT645374.1 | Full length |
| Phypa_ cpFBPase | *Physicomitrella patens* | 196 | 197 | BY991118.1<br>BJ168552.1 + proprietary | Full length |
| Gaisu_ cpFBPase | *Galderia sulphuraria* | 198 | 199 | AJ302644 | Partial |
| Marpo_ cpFBPase | *Marchantia polymorpha* | 200 | 201 | BJ862191 | Partial |
| Tagpa_ cpFBPase | *Tagetes patula* | 202 | 203 | Contig CON_01b-cs_scarletade-4-e3.b1 proprietary | Partial |

(continued)

| Name | Source organism | Nucleic acid SEQ ID NO: | Polypeptide SEQ ID NO: | Database accession number | Status |
|---|---|---|---|---|---|
| Linus_ cpFBPase | 204 | 204 | 205 | contig6298 proprietary | Partial |
| * *Cyanidioschyzon merolae* database at the Department of Biological Science, University of Tokyo<br>** *Ostreococcus lucimarinus* and *Phaeodactylum tricornutum* databases at the DOE Joint Genome Institute, US Department of Energy | | | | | |

***Example 13: Alignment of relevant polypeptide sequences***

AlignX from the Vector NTI (Invitrogen) is based on the popular Clustal algorithm of progressive alignment (Thompson et al. (1997) Nucleic Acids Res 25:4876-4882; Chenna et al. (2003). Nucleic Acids Res 31:3497-3500). A phylogenetic tree can be constructed using a neighbour-joining clustering algorithm. Default values are for the gap open penalty of 10, for the gap extension penalty of 0,1 and the selected weight matrix is Blosum 62 (if polypeptides are aligned).

The result of the multiple sequence alignment using polypeptides relevant in identifying the ones useful in performing the methods of the invention is shown in Figure 12 of the present application. Chloroplastic FBPase polypeptides (cpFBPase) differ from cytoplasmic FBPase (cyFBPase) polypeptides by the presence of a transit peptide at their N-terminus for plastidic (chloroplastic) subcellular targeting (boxed in the figure). In addition, the former contain an insertion of amino acids (also boxed, and named redox regulatory insertion) comprising at least two cysteine residues necessary for disulphide bridge formation (i.e. redox regulation). The conserved cysteines are named after their position in the mature pea (*Pisum sativa*) cpFBPase polypeptide, i.e. Cys153, Cys173 and Cys178. Cys153 and Cys173 usually are the two partners involved in disulphide bridge formation (Chiadmi et al. (1999) EMBO J 18(23): 6809-6815). The active site motif of cpFBPase as defined in the Prosite database is PS00124 and corresponds to the following amino acid sequence: [AG]-[RK]-[L1]-X(1,2)-[LIV]-[FY]-E-X(2)-P-[LIVM]-[GSA], wherein [RK] is the active site that binds and X any amino acid. The predicted active site motif and predicted active site itself (comprised within the motif) have also been boxed in Figure 12. Also identified in Figure 12 are amino acid residues Asn237, Tyr269, Tyr289 and Arg268 which bind the 6-phosphate of fructose-1,6-bisphosphate, and Lys299 which binds the fructose (Chiadmi et al. (1999) EMBO J 18(23): 6809-6815). These latter are also numbered after their position in the mature pea (*Pisum sativa*) cpFBPase polypeptide.

***Example 14: Calculation of global percentage identity between polypeptide sequences useful in performing the methods of the invention***

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.

Parameters used in the comparison were:

Scoring matrix: Blosum62

First Gap: 12

Extending gap: 2

Results of the software analysis are shown in Table D for the global similarity and identity over the full length of the polypeptide sequences (excluding the partial polypeptide sequences). Percentage identity is given above the diagonal and percentage similarity is given below the diagonal.

The percentage identity between the polypeptide sequences useful in performing the methods of the invention can be as low as 40 % amino acid identity compared to SEQ ID NO: 155.

**Table D:** MatGAT results for global similarity and identity over the full length of the polypeptide sequences.

| | **1** | **2** | **3** | **4** | **5** | **6** | **7** | **8** | **9** | **10** | **11** | **12** | **13** | **14** | **15** | **16** | **17** | **18** | **19** | **20** | **21** | **22** |
|---|---|---|---|---|---|---|---|---|---|---|---|---|---|---|---|---|---|---|---|---|---|---|
| **1.Arath_ cpfBPase** | | 79 | 67 | 76 | 51 | 62 | 42 | 40 | 79 | 74 | 74 | 79 | 92 | 60 | 80 | 79 | 80 | 58 | 42 | 39 | 16 | 35 |
| **2. Soltu_ cpfBPase** | 87 | | 69 | 77 | 51 | 62 | 43 | 41 | 79 | 76 | 74 | 82 | 80 | 58 | 82 | 94 | 97 | 58 | 42 | 40 | 15 | 36 |
| **3. Phymi_ cpfBPase** | 81 | 81 | | 69 | 50 | 62 | 42 | 41 | 67 | 70 | 68 | 69 | 68 | 60 | 68 | 68 | 69 | 60 | 41 | 38 | 13 | 34 |
| **4.Orysa_ cpfBPase** | 85 | 86 | 81 | | 52 | 65 | 43 | 41 | 76 | 85 | 88 | 78 | 78 | 59 | 77 | 77 | 78 | 57 | 41 | 39 | 16 | 35 |
| **5. Bigna_ cpfBPase** | 68 | 68 | 67 | 69 | | 52 | 49 | 42 | 51 | 51 | 51 | 51 | 51 | 52 | 52 | 52 | 50 | 51 | 43 | 40 | 16 | 36 |
| **6. Chlre_ cpfBPase** | 75 | 73 | 76 | 76 | 66 | | 45 | 41 | 61 | 63 | 63 | 63 | 64 | 63 | 62 | 62 | 63 | 61 | 42 | 40 | 17 | 33 |
| **7. Phatr_ cpfBPase** | 61 | 60 | 61 | 61 | 64 | 61 | | 42 | 44 | 45 | 44 | 44 | 42 | 47 | 43 | 42 | 43 | 48 | 40 | 35 | 15 | 32 |
| **8. Cyame_ cpFBPase** | 59 | 61 | 59 | 60 | 61 | 60 | 59 | | 41 | 41 | 41 | 41 | 40 | 41 | 42 | 41 | 42 | 41 | 36 | 32 | 15 | 34 |
| **9. Pissa_ cpFBPase** | 86 | 89 | 80 | 84 | 68 | 72 | 60 | 59 | | 73 | 75 | 83 | 79 | 59 | 81 | 80 | 80 | 56 | 41 | 39 | 15 | 35 |
| **10. Triae_ cpFBPase** | 83 | 85 | 81 | 90 | 67 | 74 | 60 | 61 | 83 | | 81 | 75 | 75 | 61 | 74 | 74 | 75 | 60 | 41 | 39 | 15 | 35 |
| **11. Zeama- cpFBPase** | 84 | 84 | 81 | 92 | 68 | 76 | 61 | 60 | 84 | 89 | | 75 | 76 | 59 | 75 | 75 | 76 | 57 | 40 | 38 | 15 | 33 |
| **12. Pontr_ cpFBPase** | 88 | 90 | 81 | 84 | 67 | 74 | 62 | 61 | 92 | 84 | 84 | | 80 | 59 | 85 | 82 | 82 | 56 | 41 | 40 | 16 | 35 |
| **13.Brana_ cpFBPase** | 96 | 87 | 81 | 86 | 67 | 76 | 60 | 60 | 87 | 85 | 85 | 87 | | 59 | 81 | 80 | 80 | 57 | 42 | 39 | 15 | 36 |
| **14. Ostta_ cpFBPase** | 72 | 71 | 72 | 73 | 69 | 73 | 60 | 57 | 72 | 71 | 72 | 71 | 71 | | 58 | 58 | 59 | 85 | 45 | 44 | 15 | 38 |

(continued)

| | 1 | 2 | 3 | 4 | 5 | 6 | 7 | 8 | 9 | 10 | 11 | 12 | 13 | 14 | 15 | 16 | 17 | 18 | 19 | 20 | 21 | 22 |
|---|---|---|---|---|---|---|---|---|---|---|---|---|---|---|---|---|---|---|---|---|---|---|
| **15. Poptr_ cpFBPase** | 89 | 89 | 82 | 85 | 67 | 73 | 63 | 60 | 89 | 84 | 84 | 91 | 88 | 71 | | 82 | 82 | 56 | 41 | 39 | 13 | 35 |
| **16. Nicta_ cpFBPase** | 87 | 98 | 81 | 85 | 68 | 73 | 59 | 61 | 88 | 84 | 86 | 89 | 87 | 70 | 89 | | 93 | 57 | 41 | 40 | 15 | 36 |
| **17. Lyces_ cpFBPase** | 86 | 98 | 80 | 86 | 67 | 73 | 61 | 60 | 89 | 85 | 85 | 89 | 86 | 72 | 89 | 96 | | 58 | 42 | 40 | 16 | 36 |
| **18. Ostlu_ cpFBPase** | 72 | 70 | 72 | 73 | 66 | 72 | 60 | 58 | 72 | 72 | 71 | 70 | 73 | 91 | 71 | 70 | 72 | | 44 | 43 | 16 | 37 |
| ***19. Arath_ cyFBPase*** | 56 | 57 | 56 | 56 | 58 | 57 | 53 | 50 | 56 | 56 | 56 | 55 | 57 | 60 | 55 | 56 | 57 | 61 | | 48 | 16 | 42 |
| ***20. Euggr_ cyFBPase*** | 57 | 57 | 57 | 59 | 57 | 58 | 57 | 51 | 57 | 58 | 57 | 56 | 58 | 63 | 58 | 56 | 58 | 61 | 63 | | 15 | 35 |
| ***21. Synec_ FBPase SBPase*** | 31 | 30 | 30 | 31 | 31 | 34 | 31 | 30 | 31 | 28 | 30 | 30 | 31 | 29 | 29 | 29 | 31 | 32 | 32 | 32 | | 16 |
| **22. Synec_ *FBPaseII*** | 52 | 53 | 53 | 53 | 52 | 51 | 47 | 45 | 52 | 52 | 51 | 52 | 52 | 57 | 52 | 53 | 54 | 57 | 61 | 53 | 33 | |

***Example 15: Identification of domains comprised in polypeptide sequences useful in performing the methods of the invention***

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Interpro is hosted at the European Bioinformatics Institute in the United Kingdom.

The results of the InterPro scan of the polypeptide sequence as represented by SEQ ID NO: 155 are presented in Table E.

**Table E:** InterPro scan results of the polypeptide sequence as represented by SEQ ID NO: 155

| Database | Accession number | Accession name |
|---|---|---|
| InterPro | IPR000146 | Inositol phosphatase/fructose-1,6-bisphosphatase |
| ProDom | PD001491 | Inositol phosphatase/fructose-1,6-bisphosphatase |
| PRINTS | PR00115 | FBPHPHTASE |
| PIR superfamily | PIRSF000904 | Fructose-1,6-bisphosphatase/sedoheptulose-1,7-bisphosphatase |
| PANTHER | PTHR11556 | FRUCTOSE-1,6-BISPHOSPHATASE-RELATED |
| Pfam | PF00316 | FBPase |
| PROFILE | PS00124 | FBPASE active site |
| PROSITE | PS00124 | FBPASE active site |

***Example 16: Topology prediction of the polypeptide sequences useful in performing the methods of the invention (subcellular localization, transmembrane...)***

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark.

For the sequences predicted to contain an N-terminal presequence a potential cleavage site can also be predicted.

A number of parameters were selected, such as organism group (non-plant or plant), cutoff sets (none, predefined set of cutoffs, or user-specified set of cutoffs), and the calculation of prediction of cleavage sites (yes or no).

The results of TargetP 1.1 analysis of the polypeptide sequence as represented by SEQ ID NO: 155 are presented Table F. The "plant" organism group has been selected, no cutoffs defined, and the predicted length of the transit peptide requested. The subcellular localization of the polypeptide sequence as represented by SEQ ID NO: 155 is the chloroplast, and the predicted length of the transit peptide is of 56 amino acids starting from the N-terminus (not as reliable as the prediction of the subcellular localization itself, may vary in length of a few amino acids). The mature pea cpFBPase has a transit peptide of 53 amino acids in length. When aligning the pea cpFBPase and the cpFBPase of SEQ ID NO: 155, it is possible to deduce the length of the transit peptide in the latter, also of 53 amino acids.

**Table F:** TargetP 1.1 analysis of the polypeptide sequence as represented by SEQ ID NO: 155

| | |
|---|---|
| Length (AA) | 415 |
| Chloroplastic transit peptide | 0.832 |
| Mitochondrial transit peptide | 0.106 |
| Secretory pathway signal peptide | 0.003 |

(continued)

| | |
|---|---|
| Other subcellular targeting | 0.065 |
| Predicted Location | Chloroplastic |
| Reliability class | 2 |
| Predicted transit peptide length | 56 |

Many other algorithms can be used to perform such analyses, including:

- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;

***Example 17: Assay related to the polypeptide sequences useful in performing the methods of the invention***

Polypeptide sequence as represented by SEQ ID NO: 155 is an enzyme with as Enzyme Commission (EC; classification of enzymes by the reactions they catalyse) number EC 3.1.3.11 for fructose-bisphosphatase (also called D-fructose-1,6-bisphosphate 1-phosphohydrolase). The functional assay maybe an assay for FBPase activity based on a colorimetric Pi assay, as described by Huppe and Buchanan (1989) in Naturforsch. 44c: 487-494. Other methods to assay the enzymatic activity are described by Alscher-Herman (1982) in Plant Physiol 70: 728-734.

***Example 18: Cloning of nucleic acid sequence as represented by SEQ ID NO: 154***

Unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

The nucleic acid sequence used in the methods of the invention was amplified by PCR using as template a Chlamydomonas reinhardtii CC-1690 cDNA library ("Core Library") (in Lambda ZAP II vector from Stratagene) purchased at the Chlamy Center (formerly the Chlamydomonas Genetics Center) at Duke University, North Carolina, USA. PCR was performed using Hifi Taq DNA polymerase in standard conditions, using 200 ng of template in a 50 μl PCR mix. The primers used were

- prm08448 SEQ ID NO: 206; sense, AttB1 site in lower case:

  5'-ggggacaagtttgtacaaaaaagcaggcttaaacaatggccgccaccatg-3'; and

- prm08449 (SEQ ID NO: 207; reverse, complementary, AttB2 site in lower case:

  5'-ggggaccactttgtacaagaaagctgggtagctgcttagtgcttcttggt-3',

which include the AttB sites for Gateway recombination. The amplified PCR fragment was purified also using standard methods. The first step of the Gateway procedure, the BP reaction, was then performed, during which the PCR fragment recombines in vivo with the pDONR201 plasmid to produce, according to the Gateway terminology, an "entry clone", p15972. Plasmid pDONR201 was purchased from Invitrogen, as part of the Gateway® technology.

***Example 19: Expression vector construction using the nucleic acid sequence* as *represented by SEQ ID NO: 154***

The entry clone p15972 was subsequently used in an LR reaction with p05050, a destination vector used for *Oryza sativa* transformation. This vector contains as functional elements within the T-DNA borders: a plant selectable marker; a screenable marker expression cassette; and a Gateway cassette intended for LR in vivo recombination with the nucleic acid sequence of interest already cloned in the entry clone. A rice GOS2 promoter (SEQ ID NO: 208) for constitutive expression (PRO0129) was located upstream of this Gateway cassette.

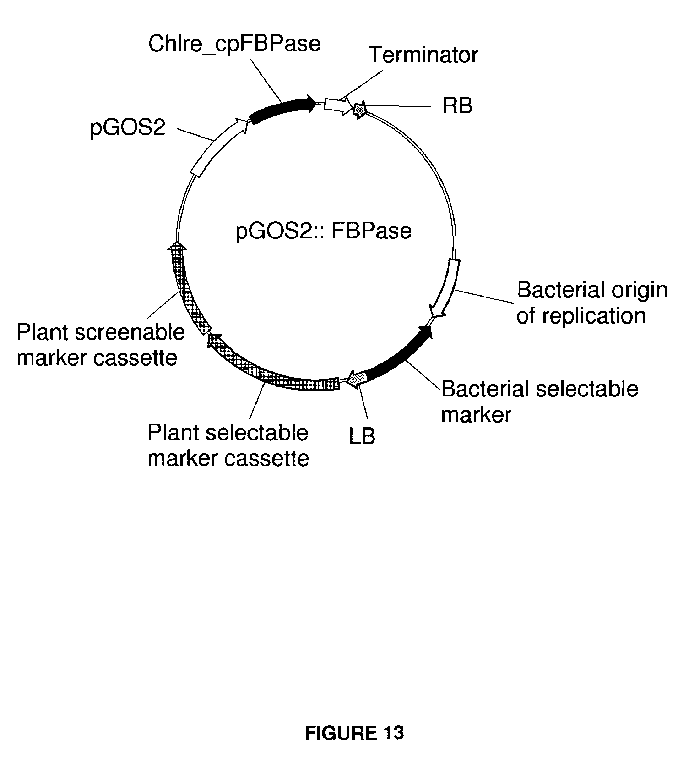

After the LR recombination step, the resulting expression vector pGOS2::FBPase (Figure 13) was transformed into *Agrobacterium* strain LBA4044 according to methods well known in the art.

*Example 20: Plant transformation*

*Rice transformation*

The Agrobacterium containing the expression vector was used to transform Oryza sativa plants. Mature dry seeds of the rice japonica cultivar Nipponbare were dehusked. Sterilization was carried out by incubating for one minute in 70% ethanol, followed by 30 minutes in 0.2%HgCl2, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were subcultured on fresh medium 3 days before co-cultivation (to boost cell division activity).

Agrobacterium strain LBA4404 containing the expression vector was used for cocultivation. Agrobacterium was inoculated on AB medium with the appropriate antibiotics and cultured for 3 days at 28˚C. The bacteria were then collected and suspended in liquid co-cultivation medium to a density (OD600) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25˚C. Co-cultivated calli were grown on 2,4-D-containing medium for 4 weeks in the dark at 28˚C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan et al. 1993, Hiei et al. 1994).

*Corn transformation*

Transformation of maize (Zea mays) is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for tansformation, but other genotypes can be used successfully as well. Ears are harvested from corn plant approximately 11 days after pollination (DAP) when the length of the immature embryo is about 1 to 1.2 mm. Immature embryos are cocultivated with Agrobacterium tumefaciens containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 ˚C for 2-3 weeks, or until shoots develop. The green shoots are transferred from each embryo to maize rooting medium and incubated at 25 ˚C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with Agrobacterium tumefaciens containing the expression vector, and transgenic plants are recovered through organogenesis. After incubation with Agrobacterium, the embryos are grown in vitro on callus induction medium, then regeneration medium, containing the selection agent (for example imidazolinone but various selection markers can be used). The Petri plates are incubated in the light at 25 ˚C for 2-3 weeks, or until shoots develop. The green shoots are transferred from each embryo to rooting medium and incubated at 25 ˚C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for in vitro sowing. The hypocotyl, the radicle and one cotyledon are excised from seven-day old young seedlings. The epicotyl and the remaining cotyledon are further grown to develop axillary nodes. These axillary nodes are excised and incubated with Agrobacterium tumefaciens containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseed/canola transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with Agrobacterium (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with Agrobacterium, the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

*Alfalfa transformation*

A regenerating clone of alfalfa (Medicago sativa) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of Agrobacterium tumefaciens C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 μm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit Agrobacterium growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

***Example 21: Phenotypic evaluation procedure***

<u>21.1 Evaluation setup</u>

Approximately 35 independent T0 rice transformants were generated. The primary transformants were transferred from a tissue culture chamber to a greenhouse for growing and harvest of T1 seed. Eight events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28°C in the light and 22°C in the dark, and a relative humidity of 70%.

*Drought screen*

Plants from T2 seeds are grown in potting soil under normal conditions until they approached the heading stage. They are then transferred to a "dry" section where irrigation is withheld. Humidity probes are inserted in randomly chosen pots to monitor the soil water content (SWC). When SWC goes below certain thresholds, the plants are automatically re-watered continuously until a normal level was reached again. The plants are then re-transferred again to normal conditions. The rest of the cultivation (plant maturation, seed harvest) is the same as for plants not grown under abiotic stress conditions. Growth and yield parameters are recorded as detailed for growth under normal conditions.

*Nitrogen use efficiency screen*

Rice plants from T2 seeds are grown in potting soil under normal conditions except for the nutrient solution. The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Growth and yield parameters are recorded as detailed for growth under normal conditions.

Four T1 events were further evaluated in the T2 generation following the same evaluation procedure as for the T1 generation but with more individuals per event. From the stage of sowing until the stage of maturity the plants were passed several times through a digital imaging cabinet. At each time point digital images (2048x1536 pixels, 16 million colours) were taken of each plant from at least 6 different angles.

<u>21.2 Statistical analysis: F-test</u>

A two factor ANOVA (analysis of variants) was used as a statistical model for the overall evaluation of plant phenotypic characteristics. An F-test was carried out on all the parameters measured of all the plants of all the events transformed with the gene of the present invention. The F-test was carried out to check for an effect of the gene over all the transformation events and to verify for an overall effect of the gene, also known as a global gene effect. The threshold for significance for a true global gene effect was set at a 5% probability level for the F-test. A significant F-test value points to a gene effect, meaning that it is not only the mere presence or position of the gene that is causing the differences in phenotype.

<u>21.3 Parameters measured</u>

**Biomass-related parameter measurement**

From the stage of sowing until the stage of maturity the plants were passed several times through a digital imaging cabinet. At each time point digital images (2048x1536 pixels, 16 million colours) were taken of each plant from at least 6 different angles.

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the time point at which the plant had reached its maximal leafy biomass. The early vigour is the plant (seedling) aboveground area three weeks post-germination.

**Seed-related parameter measurements**

The mature primary panicles were harvested, counted, bagged, barcode-labeled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand kernel weight (TKW) is extrapolated from the number of filled seeds counted and their total weight. The harvest index (HI) in the present invention is defined as the ratio between the total seed yield and the above ground area ($mm^2$), multiplied by a factor $10^6$. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

### *Example 22: Results of the phenotypic evaluation of the transgenic plants*

The results of the evaluation of transgenic rice plants expressing the nucleic acid sequence useful in performing the methods of the invention are presented in Table G. The percentage difference between the transgenics and the corresponding nullizygotes is also shown, with a P value from the F test below 0.05.

Total seed yield, number of filled seeds, seed fill rate and harvest index are significantly increased in the transgenic plants expressing the nucleic acid sequence useful in performing the methods of the invention, compared to the control plants (in this case, the nullizygotes).

**Table G:** Results of the evaluation of transgenic rice plants expressing the nucleic acid sequence useful in performing the methods of the invention.

| Trait | % Increase in T1 generation | % Increase in T2 generation |
|---|---|---|
| Total seed yield | 7 | 28 |
| Number of filled seeds | 5 | 28 |
| Fill rate | 6 | 19 |
| Harvest index | 4 | 21 |

### *Example 23: Gene Cloning*

The rice SIK gene was amplified by PCR with primers (SEQ ID NO: 227; sense, start codon in bold, AttB1 site in italic: 5'-ggggacaagtttgtacaaaaaagcaggcttaaacaatgatgggttgcttcactgtc-3') and (SEQ ID NO: 228; reverse, complementary, AttB2 site in italic: 5'-ggggaccactttgtacaagaaagctgggtatggacaatcaaaaaccctca-3'), which include the AttB sites for Gateway recombination, were used for PCR amplification. PCR was performed using Hifi Taq DNA polymerase under standard conditions. The PCR fragment was purified using standard methods. The first step of the Gateway procedure, the BP reaction, was then performed, during which the PCR fragment recombines *in vivo* with the pDONR201 plasmid to produce, according to the Gateway terminology, an "entry clone". Plasmid pDONR201 was purchased from Invitrogen, as part of the Gateway® technology.

### *Example 24: Vector Construction Downregulation*

The entry clone was subsequently used in an LR reaction with a destination vector used for Oryza sativa transformation for the downregulation construct. This vectors contained within the T-DNA borders: a plant selectable marker; a screenable marker expression cassette; and a Gateway cassette intended for LR in vivo recombination so as to integrate the sequence of interest from the entry clone in sense or anti sense orientation. A rice GOS2 promoter (SEQ ID NO: 226) for constitutive expression was located upstream of this Gateway cassette.

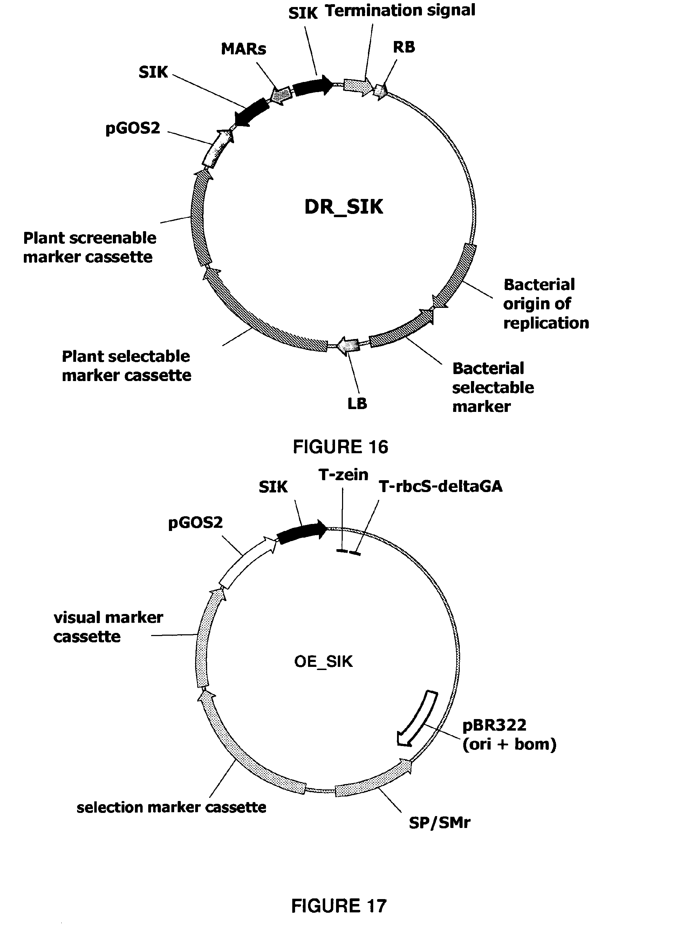

After the LR recombination step, the resulting expression vector (Figure 16) was transformed into Agrobacterium strain LBA4044 and subsequently to Oryza sativa plants. Transformed rice plants were allowed to grow and were then examined for the parameters described in Example 26.

### *Example 25: Vector Construction Overexpression*

The entry clone was subsequently used in an LR reaction with a destination vector used for plant (Oryza sativa) transformation. This vector contained within the T-DNA borders: a plant selectable marker; a screenable marker expression cassette; and a Gateway cassette intended for LR in vivo recombination with the sequence of interest already cloned in the entry clone. A rice GOS2 promoter for constitutive expression was located upstream of this Gateway cassette.

After the LR recombination step, the resulting expression vector (Figure 17) was transformed into Agrobacterium strain LBA4044. Transformed colonies were grown on YEP media and selected by respective antibiotics for two days at 28°C. These Agrobacterium cultures were used for the plant transformation. Transformed rice plants were allowed to grow and were then examined for the parameters described in Example 26.

***Example 26: Evaluation of plants transformed with SIK in anti sense orientation***

Approximately 15 to 20 independent T0 rice transformants were generated. The primary transformants were transferred from a tissue culture chamber to a greenhouse for growing and harvest of T1 seed. Six events for which the T1 progeny segregated 3:1 for presence/absence of the transgene, were retained. For each of these events, approximately 10 T1 seedlings containing the transgene (hetero- and homozygotes) and approximately 10 T1 seedlings lacking the transgene (nullizygotes) were selected by monitoring visual marker expression.

The selected T1 plants were transferred to a greenhouse. Each plant received a unique barcode label to link unambiguously the phenotyping data to the corresponding plant. The selected T1 plants were grown on soil in 10 cm diameter pots under the following environmental settings: photoperiod= 11.5 h, daylight intensity= 30,000 lux or more, daytime temperature= 28˚C, night time temperature= 22˚C, relative humidity= 60-70%. Plants were grown under optimal watering conditions until they approached the heading stage when irrigation was withheld. Humidity probes were inserted in randomly chosen pots to monitor the soil water content (SWC). When SWC dropped below 20%, the plants were automatically re-watered to achieve optimal watering conditions again. Transgenic plants and the corresponding nullizygotes were grown side-by-side at random positions. From the stage of sowing until the stage of maturity the plants were passed several times through a digital imaging cabinet. At each time point digital images (2048x1536 pixels, 16 million colours) were taken of each plant from at least 6 different angles.

*Drought screen*

Plants from T2 seeds are grown in potting soil under normal conditions until they approached the heading stage. They are then transferred to a "dry" section where irrigation is withheld. Humidity probes are inserted in randomly chosen pots to monitor the soil water content (SWC). When SWC goes below certain thresholds, the plants are automatically re-watered continuously until a normal level was reached again. The plants are then re-transferred again to normal conditions. The rest of the cultivation (plant maturation, seed harvest) is the same as for plants not grown under abiotic stress conditions. Growth and yield parameters are recorded as detailed for growth under normal conditions.

*Nitrogen use efficiency screen*

Rice plants from T2 seeds are grown in potting soil under normal conditions except for the nutrient solution. The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Growth and yield parameters are recorded as detailed for growth under normal conditions.

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts aboveground. The Areamax is the aboveground area at the time point at which the plant had reached its maximal leafy biomass.

The mature primary panicles were harvested, bagged, barcode-labelled and then dried for three days in the oven at 37˚C. The panicles were then threshed and all the seeds collected. The filled husks were separated from the empty ones using an air-blowing device. After separation, both seed lots were then counted using a commercially available counting machine. The empty husks were discarded. The filled husks were weighed on an analytical balance and the cross-sectional area of the seeds was measured using digital imaging. This procedure resulted in the set of the following seed-related parameters:

The flowers-per-panicle is a parameter estimating the average number of florets per panicle on a plant, derived from the number of total seeds divided by the number of first panicles. The tallest panicle and all the panicles that overlapped with the tallest panicle when aligned vertically, were considered as first panicles and were counted manually. The number of filled seeds was determined by counting the number of filled husks that remained after the separation step. The total seed yield (total seed weight) was measured by weighing all filled husks harvested from a plant. Total seed number per plant was measured by counting the number of husks harvested from a plant and corresponds to the number of florets per plant. These parameters were derived in an automated way from the digital images using image analysis software and were analysed statistically. Individual seed parameters (including width, length, area, weight) were measured using a custom-made device consisting of two main components, a weighing and imaging device, coupled to software for image analysis. The harvest index in the present invention is defined as the ratio between the total seed yield (g) and the above ground area ($mm^2$), multiplied by a factor 106.

A two factor ANOVA (analyses of variance) corrected for the unbalanced design was used as statistical model for the overall evaluation of plant phenotypic characteristics. An F-test was carried out on all the parameters measured of all the plants of all the events transformed with that gene. The F-test was carried out to check for an effect of the gene over all the transformation events and to verify for an overall effect of the gene, also named herein "global gene effect". If the value of the F test shows that the data are significant, than it is concluded that there is a "gene" effect, meaning that not only presence or the position of the gene is causing the effect. The threshold for significance for a true global gene effect is set at 5% probability level for the F test.

To check for an effect of the genes within an event, i.e., for a line-specific effect, a t-test was performed within each event using data sets from the transgenic plants and the corresponding null plants. "Null plants" or "null segregants" or "nullizygotes" are the plants treated in the same way as the transgenic plant, but from which the transgene has segregated. Null plants can also be described as the homozygous negative transformed plants. The threshold for significance for the t-test is set at 10% probability level. The results for some events can be above or below this threshold. This is based on the hypothesis that a gene might only have an effect in certain positions in the genome, and that the occurrence of this position-dependent effect is not uncommon. This kind of gene effect is also named herein a "line effect of the gene". The p-value was obtained by comparing the t-value to the t-distribution or alternatively, by comparing the F-value to the F-distribution. The p-value then gives the probability that the null hypothesis (i.e., that there is no effect of the transgene) is correct.

***Example 27: Results***

1. Thousand Kernel Weight

The transgenic lines transformed with the downregulation construct gave an overall percentage increase of 3% compared to controls with the best line giving a 10% increase compared to controls.

2. Harvest Index

The transgenic lines transformed with the downregulation construct gave an overall percentage increase of 16% compared to controls with the best line giving a 61% increase compared to controls.

3. Fill rate

The transgenic lines transformed with the downregulation construct gave an overall percentage increase of 14% compared to controls with the best line giving a 41% increase compared to controls.

4. Flowers per panicle (number of flowers per plant)

The transgenic lines transformed with the downregulation construct gave an overall percentage decrease of -3% compared to controls with the best line giving a -11 % decrease compared to controls. A decrease in the number of flowers may be important for grasses (when used for lawns for example).

The transgenic lines transformed with the overexpression construct gave an overall percentage increase of 6% compared to controls with the best line giving a 14% increase compared to controls.

***Example 28: Identification of sequences related to SEQ ID NO: 229 and SEQ ID NO: 230***

Sequences (full length cDNA, ESTs or genomic) related to SEQ ID NO: 229 and SEQ ID NO: 230 were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). This program was used to find regions of local similarity between sequences by comparing nucleic acid or polypeptide sequences to sequence databases and by calculating the statistical significance of matches. The polypeptide encoded by SEQ ID NO: 229 was used for the TBLASTN algorithm, with default settings and with the filter to ignore low complexity sequences set off. The output of the analysis was viewed by pairwise comparison, and ranked according to the probability score (E-value), where the score reflects the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length.

Table H provides a list of nucleic acid sequences related to the nucleic acid sequence as represented by SEQ I D NO: 229.

**Table H:** Nucleic acid and polypeptide sequences related to the nucleic acid sequence of SEQ ID NO: 229 and SEQ ID NO: 230 which are useful in performing the methods of the present invention.

| Name | Source organism | Nucleic acid SEQ ID NO: | Poly-peptide SEQ ID NO: | Database accession number | Status |
|---|---|---|---|---|---|
| HAT4 | *Arabidopsis thaliana* | 229 | 230 | AT4G16780 | Full length |
| LD | *Arabidopsis thaliana* | 231 | 232 | NP_192165.1 | Full length |
| | *Oryza sativa* | 233 | 234 | Os03g47022 | Full length |
| | *Oryza sativa* | 235 | 236 | Os03g12860 | Full length |
| HAT22 | *Oryza sativa* | 237 | 238 | Os10g01470 | Full length |
| HAT14 | Oryza *sativa* | 239 | 240 | Os08g36220 | Full length |
| | *Oryza sativa* | 241 | 242 | Os09g27450 | Full length |
| HAT3 | *Oryza sativa* | 243 | 244 | Os06g04850 | Full length |
| S1HDL2 | *Oryza sativa* | 245 | 246 | Os06g04870 | Full length |
| HD-ZIP | *Oryza sativa* | 247 | 248 | Os10g41230 | Full length |
| | *Arabidopsis thaliana* | 249 | 250 | NP_174025.3 | Full length |
| HAT14 | *Arabidopsis thaliana* | 251 | 252 | NP_ 974743.1 | Full length |
| HAT4 | *Arabidopsis thaliana* | 253 | 254 | NP_193411.1 | Full length |
| HAT1 | *Arabidopsis thaliana* | 255 | 256 | NP_193476.1 | Full length |
| HAT2 | *Arabidopsis thaliana* | 257 | 258 | NP_199548.1 | Full length |
| HB-4 | *Arabidopsis thaliana* | 259 | 260 | NP_182018.1 | Full length |
| HAT3 | *Arabidopsis thaliana* | 261 | 262 | NP_191598.1 | Full length |
| | *Oryza sativa* | 263 | 264 | Os04g46350 | Full length |
| | *Oryza sativa* | 265 | 266 | Os10g23090 | Full length |
| | *Oryza sativa* | 267 | 268 | Os10g23090 | Full length |
| | *Oryza sativa* | 269 | 270 | Os03g08960 | Full length |
| | *Oryza sativa* | 271 | 272 | Os08g37580 | Full length |
| | *Oryza sativa* | 273 | 274 | Os09g29460 | Full length |
| | *Oryza sativa* | 275 | 276 | Os10g39720 | Full length |
| | *Oryza sativa* | 277 | 278 | Os05g09630 | Full length |

***Example 29: Alignment of relevant polypeptide sequences***

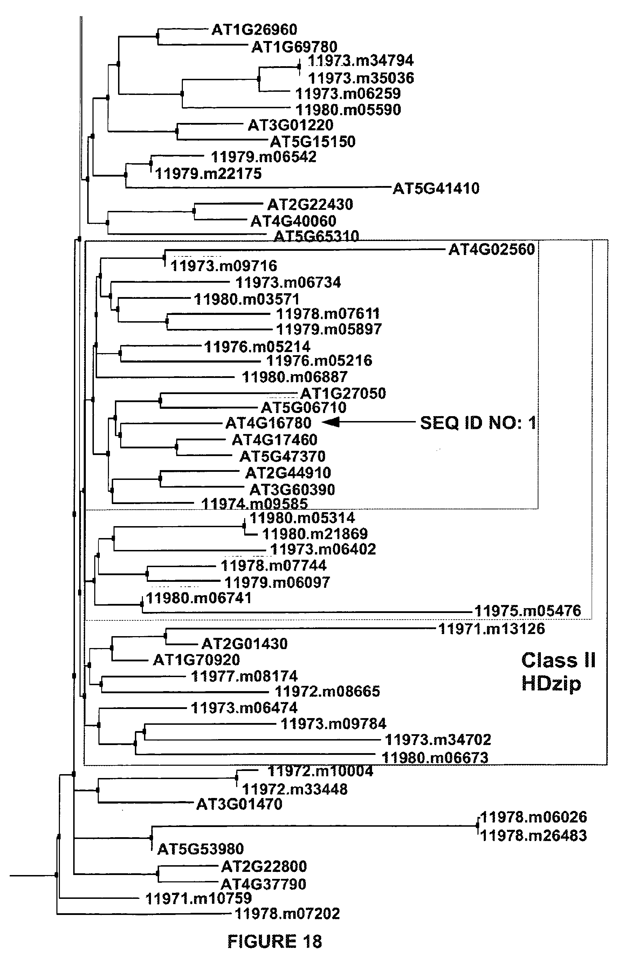

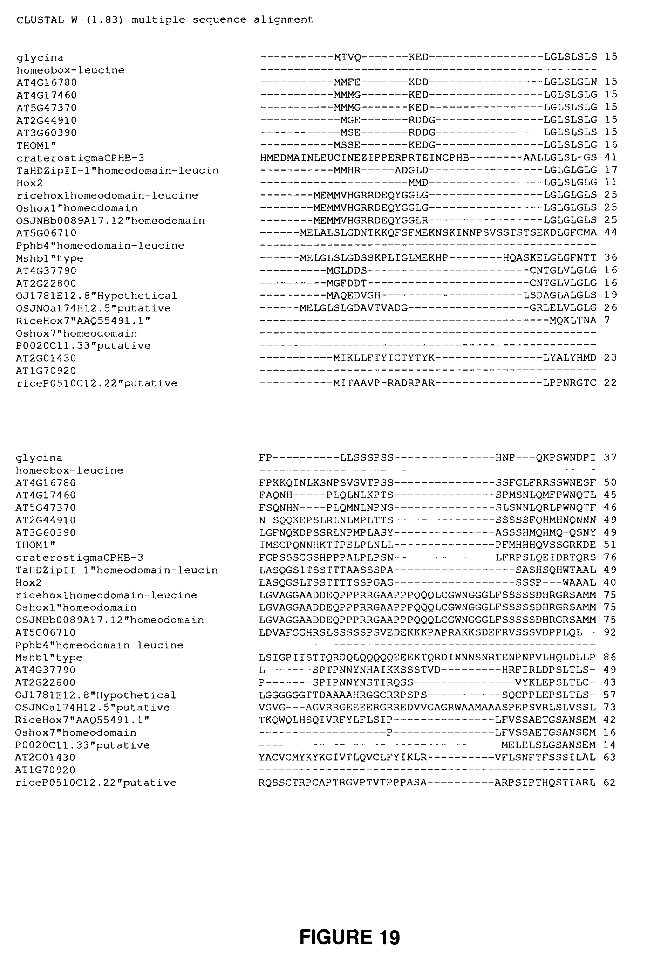

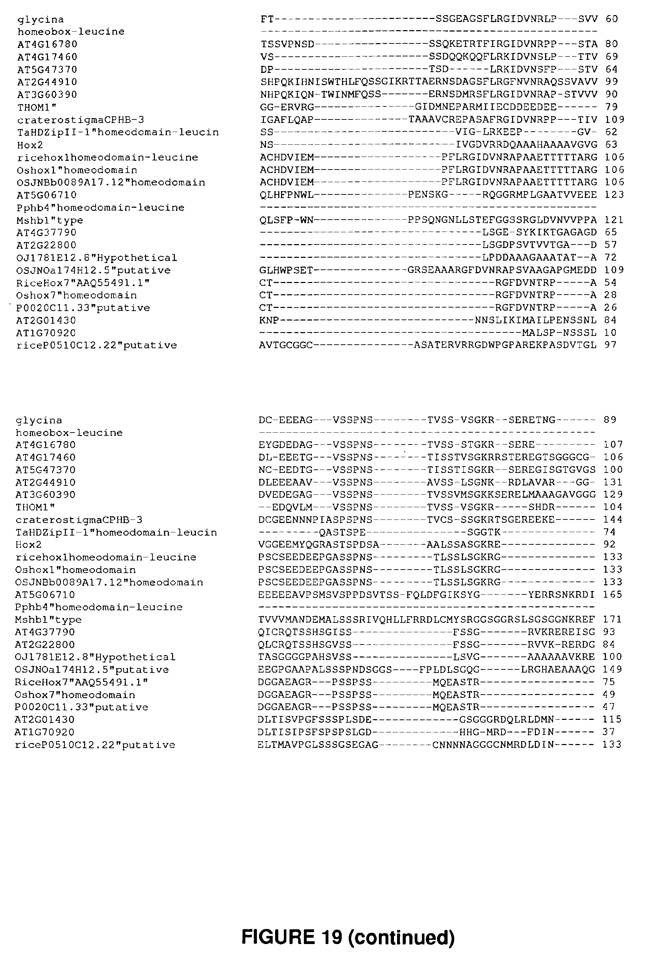

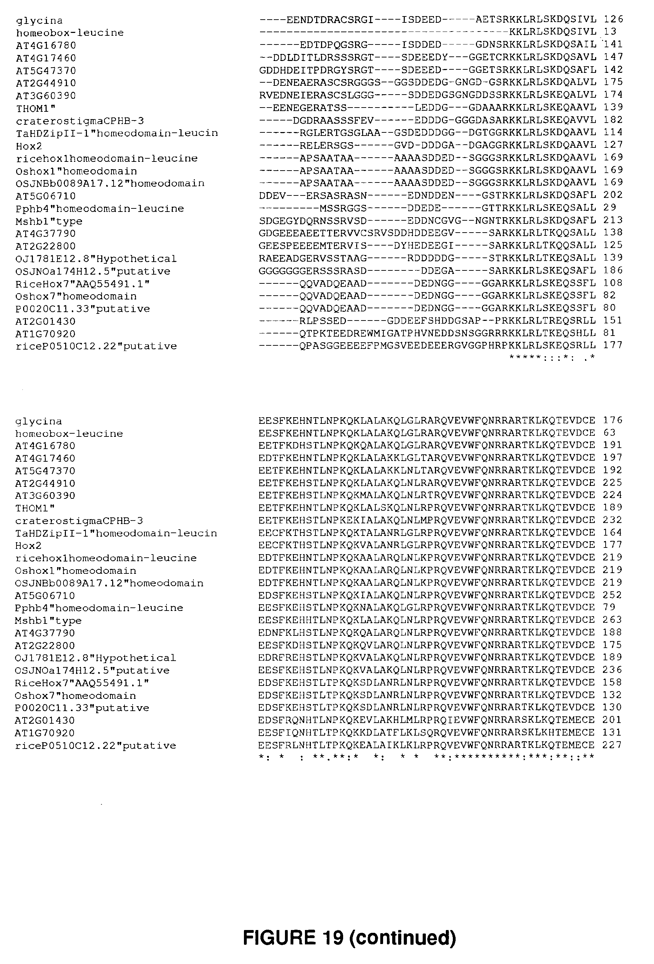

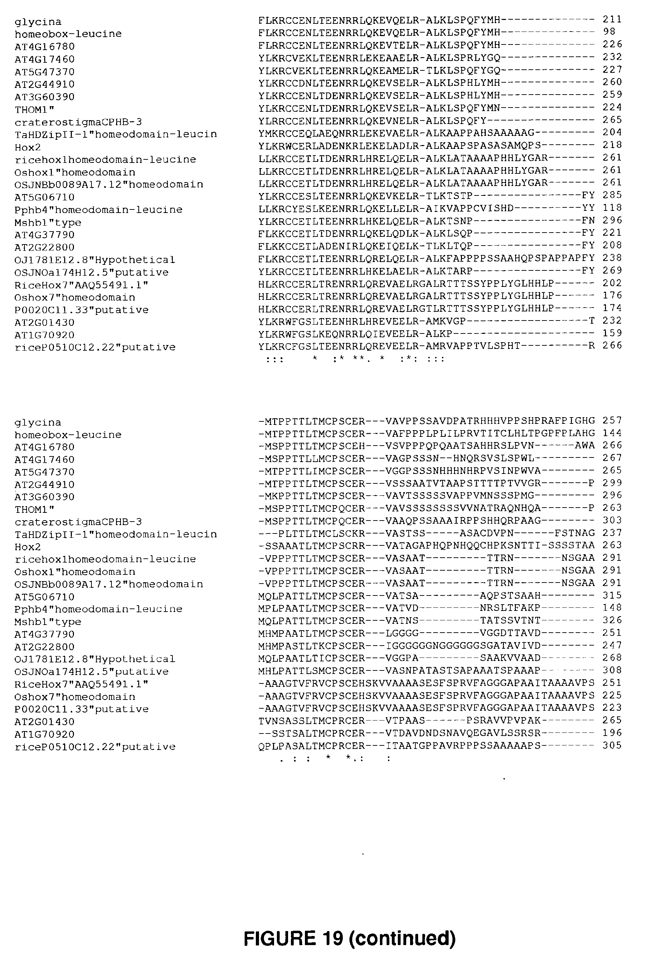

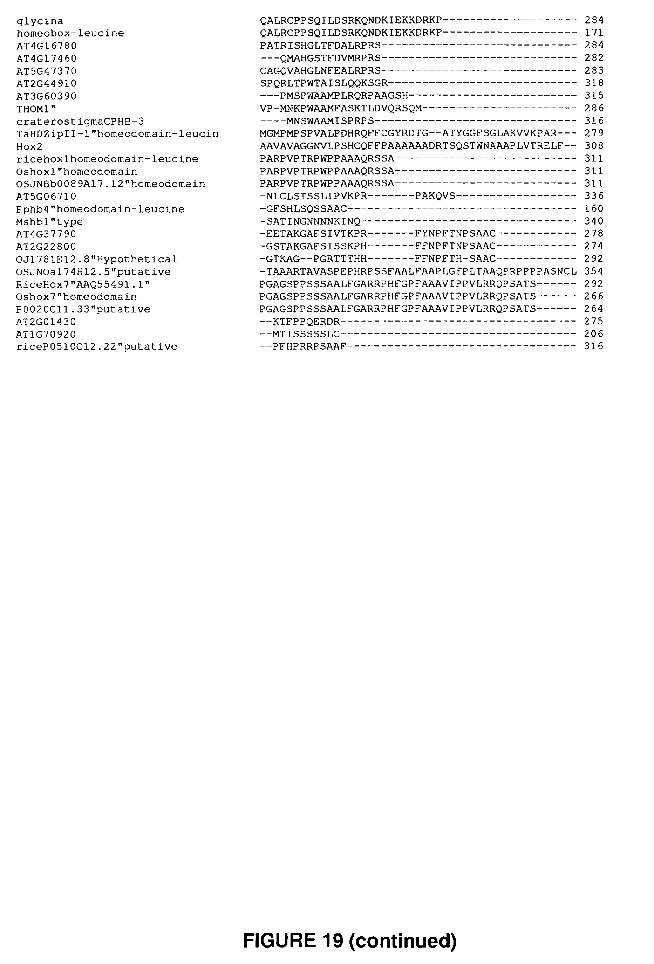

AlignX from the Vector NTI (Invitrogen) is based on the popular Clustal algorithm of progressive alignment (Thompson et al. (1997) Nucleic Acids Res 25:4876-4882; Chenna et al. (2003). Nucleic Acids Res 31:3497-3500). A phylogenetic tree was constructed using a neighbour-joining clustering algorithm (see Figure 18). Default values are for the gap open penalty of 10, for the gap extension penalty of 0,1 and the selected weight matrix is Blosum 62 (if polypeptides are aligned). The result of the multiple sequence alignment using is shown in Figure 19.

### *Example 30: Identification of domains comprised in polypeptide sequences useful in performing the methods of the invention*

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Interpro is hosted at the European Bioinformatics Institute in the United Kingdom.

The results of the InterPro scan of the polypeptide sequence as represented by SEQ ID NO: 230 are presented in Table I.

**Table I:** InterPro scan results of the polypeptide sequence as represented by SEQ ID NO: 230

| **Database** | **Accession number** | **Accession name** |
|---|---|---|
| InterPro | IPRO06712 | HDZIP |
| I nterPro | IPRO01356 | homeobox |
| InterPro | IPR003106 | HALZ |
| ProDom | PDAOK5J1 | homeobox PPHB4 DNA binding |
| ProDom | PD728753 | DNA binding |
| ProDom | PD064887 | DNA binding |
| PIR superfamily | PIR PS00001 | asn glycosylation motif |
| PIR superfamily | PS00004 | cAMP phosphorylation site |
| PIR superfamily | PS00005 | PKC phosphorylation site |
| PIR superfamily | PS00006 | CK2 phsophorylation site |
| PIR superfamily | PS00008 | myristoylation |
| PIR superfamily | PS00009 | amidation |
| PIR superfamily | PS00027 | homeobox |
| PIR superfamily | PS00029 | leucine zipper |
| Pfam | PF04618 | HDZIP |
| Pfam | PF00046 | homeobox |
| Pfam | PF02183 | HALZ |

### *Example 31: Cloning of nucleic acid sequence represented by SEQ ID NO: 229*

The Arabidopsis thaliana HAT4-encoding gene was amplified by PCR using as a template an Arabidopsis thaliana cDNA library (Invitrogen, Paisley, UK). After reverse transcription of RNA extracted from seedlings, the cDNAs were cloned into pCMV Sport 6.0. Average insert size of the bank was 1.6 kb and the original number of clones was of the order of 1.67x107 cfu. Original titer was determined to be 3.34 x106 cfu/ml after first amplification of 6x1010 cfu/ml. After plasmid extraction, 200 ng of template was used in a 50 μl PCR mix. The primers used were:

Forward (SEQ ID NO: 283)
ggggacaagtttgtacaaaaaagcaggcttcacaatgatgttcgagaaagacgatctg

Reverse (SEQ ID NO: 284)
ggggaccactttgtacaagaaagctgggtttaggacctaggacgaagagcgt

which include the AttB sites for Gateway recombination. The amplified PCR fragment was purified also using standard methods. The first step of the Gateway procedure, the BP reaction, was then performed, during which the PCR fragment recombined in vivo with the pDONR201 plasmid to produce, according to the Gateway terminology, an "entry clone". Plasmid pDONR201 was purchased from Invitrogen, as part of the Gateway® technology.

### *Example 32: Expression vector construction using the nucleic acid sequence as represented by SEQ ID NO: 229*

The entry clone was subsequently used in an LR reaction with a destination vector used for *Oryza sativa* transformation. This vector contains as functional elements within the T-DNA borders: a plant selectable marker; a screenable marker expression cassette; and a Gateway cassette intended for LR *in vivo* recombination with the nucleic acid sequence of interest already cloned in the entry clone. A rice GOS2 promoter (SEQ ID NO: 282) for constitutive expression was located upstream of this Gateway cassette.

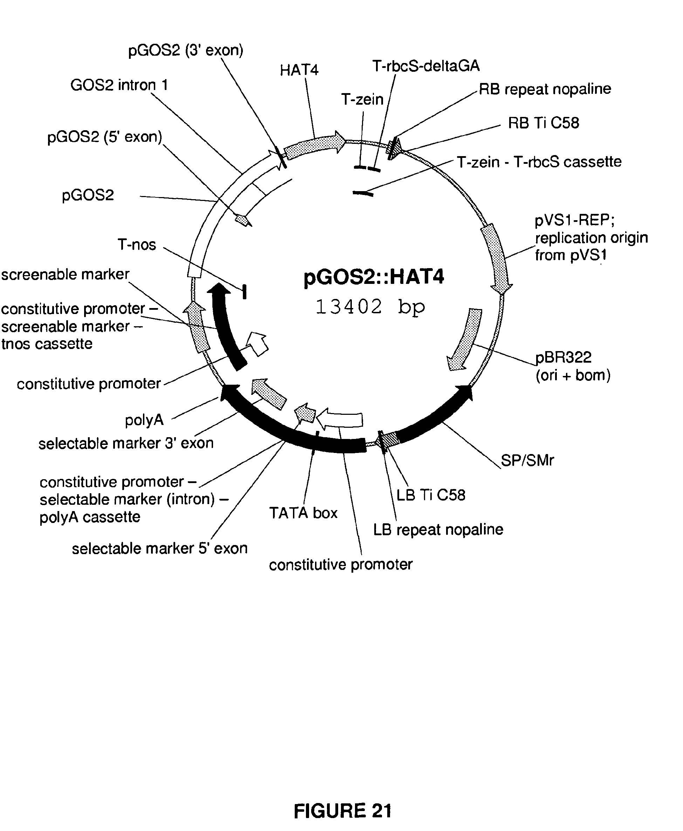

After the LR recombination step, the resulting expression vector (Figure 21) was transformed into *Agrobacterium* strain LBA4044 according to methods well known in the art.

### *Example 33: Plant transformation*

*Rice transformation*

The Agrobacterium containing the expression vector was used to transform Oryza sativa plants. Mature dry seeds of the rice japonica cultivar Nipponbare were dehusked. Sterilization was carried out by incubating for one minute in 70% ethanol, followed by 30 minutes in 0.2%$HgCl2$, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were subcultured on fresh medium 3 days before co-cultivation (to boost cell division activity).

Agrobacterium strain LBA4404 containing the expression vector was used for cocultivation. Agrobacterium was inoculated on AB medium with the appropriate antibiotics and cultured for 3 days at 28˚C. The bacteria were then collected and suspended in liquid co-cultivation medium to a density (OD600) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25˚C. Co-cultivated calli were grown on 2,4-D-containing medium for 4 weeks in the dark at 28˚C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were transferred to soil. Hardened shoots were grown under high humidity and short days in a greenhouse.

Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of overt 50 % (Aldemita and Hodges 1996, Chan et al. 1993, Hiei et al. 1994).

### *Example 34: Phenotypic evaluation procedure*

<u>34.1 Evaluation setup</u>

Approximately 35 independent T0 rice transformants were generated. The primary transformants were transferred from a tissue culture chamber to a greenhouse for growing and harvest of T1 seed. Eight events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28˚C in the light and 22˚C in the dark, and a relative humidity of 70%.

Four T1 events were further evaluated in the T2 generation following the same evaluation procedure as for the T1 generation but with more individuals per event.

*Drought screen*

Plants from T2 seeds are grown in potting soil under normal conditions until they approached the heading stage. They are then transferred to a "dry" section where irrigation is withheld. Humidity probes are inserted in randomly chosen pots to monitor the soil water content (SWC). When SWC goes below certain thresholds, the plants are automatically re-watered continuously until a normal level was reached again. The plants are then re-transferred again to normal conditions. The rest of the cultivation (plant maturation, seed harvest) is the same as for plants not grown under abiotic stress conditions. Growth and yield parameters are recorded as detailed for growth under normal conditions.

*Nitrogen use efficiency screen*

Rice plants from T2 seeds are grown in potting soil under normal conditions except for the nutrient solution. The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Growth and yield parameters are recorded as detailed for growth under normal conditions.

Non-destructive oil measurements from rice seeds was measured using the Oxford QP20+ pulsed NMR. Whole seeds were used without dehusking.

<u>34.2 Statistical analysis: F-test</u>

A two factor ANOVA (analysis of variants) was used as a statistical model for the overall evaluation of plant phenotypic characteristics. An F-test was carried out on all the parameters measured of all the plants of all the events transformed with the gene of the present invention. The F-test was carried out to check for an effect of the gene over all the transformation events and to verify an overall effect of the gene, also known as a global gene effect. The threshold for significance for a true global gene effect was set at a 5% probability level for the F-test. A significant F-test value points to a gene effect, meaning that it is not only the mere presence or position of the gene that is causing the differences in phenotype.

34.3 Results

The best line showed an 11% increase in oil content compared to control plants, with an average increase in oil content of 6% over all lines and a p value from the F-test of <0.0001

***Example 35: Transformation of Corn***

Transformation of maize (Zea mays) is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for tansformation, but other genotypes can be used successfully as well. Ears are harvested from corn plant approximately 11 days after pollination (DAP) when the length of the immature embryo is about 1 to 1.2 mm. Immature embryos are cocultivated with Agrobacterium tumefaciens containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 ˚C for 2-3 weeks, or until shoots develop. The green shoots are transferred from each embryo to maize rooting medium and incubated at 25 ˚C for 2-3 weeks, until roots develop. The rooted shoots are transplanted to soil in the greenhouse. T1 seeds are produced from plants that exhibit tolerance to the selection agent and that contain a single copy of the T-DNA insert.

***Example 36: Transformation of Wheat***

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with Agrobacterium tumefaciens containing the expression vector, and transgenic plants are recovered through organogenesis. After incubation with Agrobacterium, the embryos are grown in vitro on callus induction medium, then regeneration medium, containing the selection agent (for example imidazolinone but various selection markers can be used). The Petri plates are incubated in the light at 25 ˚C for 2-3 weeks, or until shoots develop. The green shoots are transferred from each embryo to rooting medium and incubated at 25 ˚C for 2-3 weeks, until roots develop. The rooted shoots are transplanted to soil in the greenhouse. T1 seeds are produced from plants that exhibit tolerance to the selection agent and that contain a single copy of the T-DNA insert.

***Example 37: Transformation of Soybean***

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for in vitro sowing. The hypocotyl, the radicle and one cotyledon are excised from seven-day old young seedlings. The epicotyl and the remaining cotyledon are further grown to develop axillary nodes. These axillary nodes are excised and incubated with Agrobacterium tumefaciens containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

***Example 38: Transformation of Rapeseed/Canola***

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with Agrobacterium (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 ˚C, 16 hr light. After two days of co-cultivation with Agrobacterium, the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

***Example 39: Transformation of Alfalfa***

A regenerating clone of alfalfa (Medicago sativa) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112).

Alternatively, the RA3 variety (University of Wisconsin) has been selected for use in tissue culture (Walker et al., 1978 Am J Bot 65:654-659). Petiole explants are cocultivated with an overnight culture of Agrobacterium tumefaciens C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2S04, and 100 $\mu$m acetosyringinone. The explants are washed in half-strength Murashige-Skoog medium (Murashige and Skoog, 1962) and plated on the same SH induction medium without acetosyringinone but with a suitable selection agent and suitable antibiotic to inhibit Agrobacterium growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

***Example 40: Identification of sequences related to SEQ ID NO: 285 and SEQ ID NO: 286***

Sequences (full length cDNA, ESTs or genomic) related to SEQ ID NO: 285 and/or protein sequences related to SEQ ID NO: 286 were identified amongst those maintained in the Entrez Nucleotides database at the National Center for Biotechnology Information (NCBI) using database sequence search tools, such as the Basic Local Alignment Tool (BLAST) (Altschul et al. (1990) J. Mol. Biol. 215:403-410; and Altschul et al. (1997) Nucleic Acids Res. 25:3389-3402). The program is used to find regions of local similarity between sequences by comparing nucleic acid or polypeptide sequences to sequence databases and by calculating the statistical significance of matches. The polypeptide encoded by SEQ ID NO: 285 was used for the TBLASTN algorithm, with default settings and the filter to ignore low complexity sequences set off. The output of the analysis was viewed by pairwise comparison, and ranked according to the probability score (E-value), where the score reflects the probability that a particular alignment occurs by chance (the lower the E-value, the more significant the hit). In addition to E-values, comparisons were also scored by percentage identity. Percentage identity refers to the number of identical nucleotides (or amino acids) between the two compared nucleic acid (or polypeptide) sequences over a particular length. In some instances, the default parameters may be adjusted to modify the stringency of the search.

In addition to the publicly available nucleic acid sequences available at NCBI, proprietary sequence databases are also searched following the same procedure as described herein above.

Table J provides a list of nucleic acid and protein sequences related to the nucleic acid sequence as represented by SEQ ID NO: 285 and the protein sequence represented by SEQ ID NO: 286.

Table J: Nucleic acid sequences related to the nucleic acid sequence (SEQ ID NO: 1) useful in the methods of the present invention, and the corresponding deduced polypeptides.

| **Name** | **Source organism** | **Nucleic acid SEQ ID NO:** | **Polypeptide SEQ ID NO:** | **Database accession number** | **Status** |
|---|---|---|---|---|---|
| SYB1 | *Arabidopsis thaliana* | 285 | 286 | NM112438 | Full length |
| zinc finger protein-like protein | *Gossypium hirsutum* | 287 | 288 | AAZ94630 | Full length |
| putative zinc finger protein ZF2 | *Zea mays* | 289 | 290 | Q53AV6 | Full length |
| Zinc finger transcription factor ZFP30 | *Oryza sativa* | 291 | 292 | Q6Z6E6 | Full length |
| Hypothetical protein At5g25490 | *Arabidopsis thaliana* | 293 | 294 | Q8GWD1 | Full length |
| Putative zinc finger protein | *Oryza sativa* | 295 | 296 | Q9SW92 | Full length |
| Putative zinc finger transcription factor | *Oryza sativa* | 297 | 298 | Q8RYZ5 | Full length |
| GlimmerM protein 152 | *Beta vulgaris* | 299 | 300 | ABD83289 | Full length |
| Predicted protein At2g17975 | *Arabidopsis thaliana* | 301 | 302 | Q8S8K1 | Full length |
| Hypothetical RanBP2-type zinc-finger protein At1 g67325 | *Arabidopsis thaliana* | 303 | 304 | Q8GZ43 | Full length |
| P53 binding protein-like | *Oryza sativa* | 305 | 306 | Q7F1K4 | Full length |
| Putative p53 binding protein | *Oryza sativa* | 307 | 308 | Q7XHQ8 | Full length |

(continued)

| Name | Source organism | Nucleic acid SEQ ID NO: | Polypeptide SEQ ID NO: | Database accession number | Status |
|---|---|---|---|---|---|
| Hypothetical protein | *Brassica rapa* | 309 | 310 | CX272690 | Full length |
| Hypothetical protein | *Euphorbia esula* | 311 | 312 | DV143669 | Full length |
| Hypothetical protein | *Gossypium hirsutum* | 313 | 314 | DR459119 | Full length |
| Hypothetical protein | *Vitis vinifera* | 315 | 316 | DV221228 | Full length |
| Hypothetical protein | *Populus trichocarpa* | 317 | 318 | DT496999 | Full length |
| Hypothetical protein | *Gossypium hirsutum* | 319 | 320 | DT457997 | Full length |
| Hypothetical protein | *Lactuca serriola* | 321 | 322 | DW105125 | Full length |
| Hypothetical protein | *Glycine max* | 323 | 324 | BG238374 | Full length |
| Hypothetical protein | *Populus trichocarpa* | 325 | 226 | DT494117 | Full length |
| Hypothetical protein | *Populus* sp. | 327 | 328 | DV465465 | Full length |
| zinc finger transcription factor | *Oryza sativa* | 329 | 330 | AY219846 | Full length |
| Hypothetical protein | *Panicum virgatum* | 331 | 332 | DN152082 | Full length |
| Zinc finger, RanBP2-type | *Medicago truncatula* | 333 | 334 | Q1 RWK5 | Full length |
| Zinc finger, RanBP2-type | *Medicago truncatula* | 335 | 336 | Q1S406 | Full length |
| Hypothetical protein | *Yucca filamentosa* | 337 | 338 | DT581158 | Full length |
| Hypothetical protein | *Hordeum vulgare* | 339 | 340 | BQ471337 | Full length |

***Example 41: Alignment of relevant polypeptide sequences***

AlignX from the Vector NTI (Invitrogen) is based on the popular Clustal algorithm of progressive alignment (Thompson et al. (1997) Nucleic Acids Res 25:4876-4882; Chenna et al. (2003). Nucleic Acids Res 31:3497-3500). A phylogenetic tree can be constructed using a neighbour-joining clustering algorithm. Default values are for the gap open penalty of 10, for the gap extension penalty of 0,1 and the selected weight matrix is Blosum 62 (if polypeptides are aligned).

The result of a multiple sequence alignment using polypeptides relevant in identifying the ones useful in performing the methods of the invention is shown in Figure 23a of the present application. The three Zinc-finger domains can be easily identified.

***Example 42: Calculation of global percentage identity between polypeptide sequences useful in performing the methods of the invention***

Global percentages of similarity and identity between full length polypeptide sequences useful in performing the methods of the invention were determined using one of the methods available in the art, the MatGAT (Matrix Global Alignment Tool) software (BMC Bioinformatics. 2003 4:29. MatGAT: an application that generates similarity/identity matrices using protein or DNA sequences. Campanella JJ, Bitincka L, Smalley J; software hosted by Ledion Bitincka). MatGAT software generates similarity/identity matrices for DNA or protein sequences without needing pre-alignment of the data. The program performs a series of pair-wise alignments using the Myers and Miller global alignment algorithm (with a gap opening penalty of 12, and a gap extension penalty of 2), calculates similarity and identity using for example Blosum 62 (for polypeptides), and then places the results in a distance matrix. Sequence similarity is shown in the bottom half of the dividing line and sequence identity is shown in the top half of the diagonal dividing line.
Parameters used in the comparison were:

Scoring matrix: Blosum62
First Gap: 12
Extending gap: 2

Results of the software analysis are shown in Table K for the global similarity and identity over the full length of the polypeptide sequences (excluding the partial polypeptide sequences). Percentage identity is given above the diagonal and percentage similarity is given below the diagonal.
The percentage identity between the polypeptide sequences useful in performing the methods of the invention can be as low as 16.9 % amino acid identity compared to SEQ ID NO: 286.

**Table K:** MatGAT results for global similarity and identity over the full length of the polypeptide sequences.

| | 1 | 2 | 3 | 4 | 5 | 6 | 7 | 8 | 9 | 10 | 11 | 12 | 13 | 14 | 15 | 16 | 17 | 18 | 19 | 20 | 21 | 22 | 23 | 24 | 25 | 26 | 27 | 28 |
|---|---|---|---|---|---|---|---|---|---|---|---|---|---|---|---|---|---|---|---|---|---|---|---|---|---|---|---|---|
| 1.SEQID286 | | 69.5 | 56.1 | 61.4 | 58.7 | 50.6 | 37.9 | 63.2 | 24.2 | 20.9 | 16.9 | 20.7 | 59.1 | 74.7 | 71.5 | 71.3 | 69 | 67.9 | 66.3 | 67.1 | 63.2 | 65.3 | 61.4 | 60.3 | 40.8 | 60.1 | 64.1 | 57.8 |
| 2.SEQID288 | 75.6 | | 57.5 | 60.2 | 58.2 | 54.7 | 43 | 59.2 | 20.1 | 17.1 | 15.7 | 18.3 | 50 | 73.1 | 81.1 | 75.5 | 74.3 | 69.2 | 65.9 | 70.4 | 68.1 | 67.9 | 59.6 | 63.1 | 46.3 | 58.2 | 66.3 | 57 |
| 3.SEQID290 | 67 | 67 | | 84.3 | 57.5 | 49.4 | 38.9 | 66.8 | 26.3 | 20.4 | 17.3 | 21.5 | 39.7 | 59.9 | 61.3 | 63 | 63 | 63.5 | 61.6 | 58.7 | 60.4 | 59.3 | 83.7 | 87.5 | 38.8 | 56.9 | 69.5 | 79.9 |
| 4.SEQID292 | 71.1 | 69.9 | 89.8 | | 58.2 | 55.4 | 42.9 | 62.5 | 23.7 | 20.3 | 17 | 22.5 | 46.2 | 64.5 | 66.7 | 67.8 | 65.5 | 69.8 | 65.9 | 65.1 | 61.7 | 64 | 99.4 | 91 | 44.5 | 59.1 | 75.4 | 80.5 |
| 5.SEQID294 | 68.2 | 66.5 | 68.8 | 67.1 | | 48.6 | 39.1 | 59.9 | 22.3 | 19.8 | 17.2 | 20.4 | 43 | 59.6 | 60.6 | 64.1 | 62.9 | 63.7 | 65.1 | 57.6 | 62.6 | 62 | 57.6 | 57.3 | 38.3 | 59.6 | 62.8 | 55.3 |
| 6.SEQID296 | 63.4 | 69 | 60.8 | 64.5 | 56.5 | | 45.8 | 48.6 | 23.6 | 19.1 | 16.7 | 18.3 | 38 | 54 | 55.1 | 52.3 | 53.2 | 51.8 | 53.1 | 53.8 | 52.4 | 55.6 | 55.4 | 51.5 | 41.1 | 46.4 | 57.1 | 48.6 |
| 7.SEQID298 | 56.1 | 66.9 | 54 | 58.4 | 55.9 | 61.4 | | 37 | 20.5 | 17 | 14.5 | 18.9 | 29.9 | 39.2 | 43.9 | 43.5 | 41.3 | 44.5 | 38.8 | 42.3 | 40.8 | 43 | 42.3 | 41.3 | 42 | 39.1 | 46.6 | 38.5 |
| 8.SEQID300 | 68 | 64.5 | 75 | 73.3 | 71.5 | 58.7 | 54.7 | | 24.5 | 21.3 | 19.8 | 22.6 | 47.1 | 61.8 | 64.6 | 69.5 | 65.7 | 67.4 | 65 | 61.4 | 65.7 | 66.1 | 62.5 | 64.9 | 41.2 | 62.7 | 64 | 65.4 |
| 9.SEQID302 | 32.5 | 29.1 | 36.9 | 34 | 32.5 | 32.1 | 31.3 | 32.5 | | 22.3 | 17.9 | 21.4 | 16.6 | 21.6 | 21.2 | 23.1 | 23.4 | 24.5 | 20.5 | 23.4 | 23.4 | 23.1 | 23.7 | 24.8 | 23.8 | 23.4 | 24.7 | 24.8 |
| 10.SEQID304 | 27.1 | 22.6 | 29.9 | 29.9 | 27.4 | 27.4 | 20.8 | 28.1 | 39.9 | | 47 | 36.1 | 17.2 | 18.8 | 18.8 | 20 | 19.8 | 20.7 | 19.8 | 19.5 | 20.3 | 20.3 | 20.3 | 20.8 | 21.5 | 20.3 | 20.8 | 21.9 |
| 11.SEQID306 | 23.9 | 21.3 | 25.4 | 24.2 | 24.5 | 22.2 | 19.9 | 28 | 33.7 | 57.9 | | 31.2 | 15.1 | 18.1 | 16.3 | 17.5 | 16.9 | 18.9 | 17.1 | 16.3 | 17.2 | 17.5 | 17.3 | 17.6 | 17.5 | 18.1 | 16.8 | 18.1 |
| 12.SEQID308 | 28.5 | 24.9 | 28.2 | 29.2 | 29.6 | 24.9 | 24.2 | 29.2 | 35.7 | 49.7 | 40.3 | | 16.8 | 20.4 | 19.9 | 20 | 21.5 | 23.1 | 21.1 | 18.5 | 23 | 22.3 | 22.5 | 23.2 | 19.4 | 18.6 | 20.4 | 23.2 |
| 13.SEQID310 | 63.4 | 57.6 | 48.3 | 53.6 | 50.6 | 48.3 | 50.4 | 52.3 | 23.1 | 21.9 | 19 | 21.7 | | 51.6 | 52.6 | 53.2 | 50.6 | 52.9 | 48.9 | 54 | 49.4 | 50 | 46.2 | 43.4 | 29.1 | 46.8 | 48.4 | 41.9 |
| 14.SEQID312 | 84.1 | 82.1 | 71.6 | 75.3 | 70 | 67.3 | 61.5 | 68 | 32.5 | 25.3 | 23.3 | 29.6 | 56.4 | | 78.3 | 77.8 | 81.1 | 74.7 | 67.6 | 76.4 | 69.7 | 70.2 | 64.5 | 62.4 | 41.4 | 63.5 | 69.8 | 57.8 |
| 15.SEQID314 | 78.7 | 85.1 | 69.3 | 73.5 | 67.1 | 68.2 | 62.2 | 69.2 | 30.2 | 25 | 22.5 | 26.4 | 58.8 | 84.6 | | 80.9 | 80.4 | 78.2 | 67.8 | 77 | 71.3 | 72 | 66.1 | 66.7 | 43.9 | 67.1 | 71.9 | 61.8 |
| 16.SEQID316 | 79.9 | 84.7 | 74.4 | 79.5 | 73.5 | 66 | 64.7 | 75 | 31.7 | 26.4 | 23.3 | 28.9 | 58 | 86.5 | 90.7 | | 82.9 | 80 | 73.5 | 79.2 | 78 | 78.1 | 67.8 | 68 | 47.1 | 67.1 | 77.4 | 63.4 |
| 17.SEQID318 | 77.4 | 85.4 | 70.5 | 76.5 | 70 | 68.2 | 60.9 | 70.3 | 31.3 | 26 | 22.2 | 28.5 | 58.9 | 86.5 | 88.7 | 92.1 | | 76.3 | 68.6 | 72.9 | 73.6 | 71.6 | 65.5 | 66.7 | 42.9 | 65.9 | 73 | 62.9 |
| 18.SEQID320 | 78.7 | 78.7 | 73.3 | 79.5 | 73.5 | 66.5 | 63.9 | 74.4 | 31.3 | 26.7 | 25.1 | 31.4 | 56.8 | 84.6 | 84.5 | 89 | 86.5 | | 71.8 | 72 | 79 | 82.9 | 69.2 | 66.5 | 43.8 | 70.6 | 72.5 | 62.4 |
| 19.SEQID322 | 75.7 | 71 | 73.3 | 75.1 | 74.7 | 63.3 | 56.2 | 74.4 | 29.9 | 27.4 | 23.6 | 28.2 | 53.3 | 76.9 | 73.4 | 79.3 | 76.3 | 78.1 | | 65.9 | 73.8 | 72.1 | 65.4 | 62.4 | 43.4 | 64.1 | 66.9 | 64.7 |
| 20.SEQID324 | 74.4 | 83.6 | 68.8 | 74.7 | 67.1 | 69.9 | 62.3 | 69.8 | 30.2 | 25 | 22.5 | 24.2 | 60.3 | 82.1 | 84.5 | 87.3 | 83.4 | 80.6 | 73.4 | | 67.1 | 68.2 | 65.1 | 62.7 | 45.5 | 63.2 | 70.7 | 57.5 |
| 21.SEQID326 | 75 | 76.1 | 69.9 | 72.9 | 74.1 | 64.2 | 59.7 | 72.7 | 31 | 28.8 | 24.5 | 30.7 | 53.5 | 81.1 | 81.8 | 84.9 | 81.1 | 86.8 | 79.3 | 77.4 | | 91.9 | 61.7 | 59.8 | 43 | 67.6 | 69.5 | 62 |
| 22.SEQID328 | 76.2 | 78.1 | 68.8 | 74.7 | 73.5 | 69 | 65.2 | 73.3 | 30.2 | 28.5 | 23.6 | 29.2 | 54.2 | 82.7 | 83.2 | 86.5 | 81.3 | 91 | 78.1 | 79.4 | 94.3 | | 64.2 | 63.2 | 44.1 | 65.9 | 70.2 | 58.3 |
| 23.SEQID330 | 70.5 | 69.3 | 89.2 | 99.4 | 66.5 | 64.5 | 57.8 | 72.7 | 33.6 | 29.5 | 24.8 | 29.2 | 53 | 74.7 | 72.9 | 78.9 | 75.9 | 78.9 | 74.6 | 74.1 | 72.3 | 74.7 | | 90.4 | 44.5 | 58.6 | 75.4 | 80 |
| 24.SEQID332 | 70.1 | 71.8 | 89.8 | 94.6 | 67.1 | 63.8 | 57.1 | 70.9 | 35.4 | 29.2 | 23.9 | 32.1 | 50.9 | 76.1 | 74.8 | 81 | 76.7 | 78.5 | 74 | 73.6 | 74.2 | 75.5 | 94 | | 42.7 | 57.4 | 73.3 | 80.3 |
| 25.SEQID334 | 57.3 | 66.7 | 55.7 | 59 | 55.9 | 58.6 | 60.4 | 57 | 30.2 | 28.5 | 22.5 | 26 | 44.4 | 64.1 | 66.2 | 64.7 | 63.6 | 62.6 | 58.6 | 65.8 | 59.1 | 61.3 | 59 | 60.1 | | 40 | 46 | 38.4 |
| 26.SEQID336 | 70.6 | 64.7 | 68.8 | 71.2 | 73.5 | 58.8 | 56.5 | 72.1 | 34.3 | 28.8 | 26.2 | 27.4 | 53.5 | 73.5 | 72.4 | 73.5 | 74.7 | 77.1 | 73.5 | 72.4 | 74.1 | 73.5 | 70.6 | 69.4 | 54.1 | | 63.2 | 54.5 |
| 27.SEQID338 | 72 | 75.3 | 76.7 | 81.3 | 69.4 | 68.2 | 66.2 | 70.3 | 35.4 | 28.5 | 23.1 | 27.1 | 52.6 | 79.5 | 79.9 | 85.1 | 81.8 | 81.3 | 74 | 79.9 | 76.1 | 78.7 | 80.7 | 80.4 | 64.3 | 71.8 | | 69 |
| 28.SEQID340 | 66.1 | 63.9 | 85.2 | 84.2 | 63.4 | 56.8 | 51.9 | 75.4 | 35.4 | 30.9 | 25.4 | 32.1 | 47 | 67.8 | 67.2 | 71.6 | 69.4 | 69.4 | 72.1 | 65.6 | 71 | 68.3 | 83.6 | 83.6 | 51.9 | 67.2 | 73.2 | |

***Example 43: Identification of domains comprised in polypeptide sequences useful in performing the methods of the invention***

The Integrated Resource of Protein Families, Domains and Sites (InterPro) database is an integrated interface for the commonly used signature databases for text- and sequence-based searches. The InterPro database combines these databases, which use different methodologies and varying degrees of biological information about well-characterized proteins to derive protein signatures. Collaborating databases include SWISS-PROT, PROSITE, TrEMBL, PRINTS, ProDom and Pfam, Smart and TIGRFAMs. Interpro is hosted at the European Bioinformatics Institute in the United Kingdom.

The results of the InterPro scan of the polypeptide sequence as represented by SEQ ID NO: 286 are presented in Table L.

**Table L:** InterPro scan results of the polypeptide sequence as represented by SEQ ID NO: 286

| Database | Accession number | Accession name |
|---|---|---|
| InterPro | IPR001878 | Zinc finger, RanBP2-type |
| PROFILE | PS50199 | ZF_RANBP2_2 |
| PROSITE | PS01358 | ZF_RANBP2_1 |
| PFAM | PF00641 | zf-RanBP |
| SMART | SM00547 | ZnF_RBZ |

***Example 44: Topology prediction of the polypeptide sequences useful in performing the methods of the invention (subcellular localization, transmembrane...)***

TargetP 1.1 predicts the subcellular location of eukaryotic proteins. The location assignment is based on the predicted presence of any of the N-terminal pre-sequences: chloroplast transit peptide (cTP), mitochondrial targeting peptide (mTP) or secretory pathway signal peptide (SP). Scores on which the final prediction is based are not really probabilities, and they do not necessarily add to one. However, the location with the highest score is the most likely according to TargetP, and the relationship between the scores (the reliability class) may be an indication of how certain the prediction is. The reliability class (RC) ranges from 1 to 5, where 1 indicates the strongest prediction. TargetP is maintained at the server of the Technical University of Denmark.

For the sequences predicted to contain an N-terminal presequence a potential cleavage site can also be predicted.

A number of parameters were selected, such as organism group (non-plant or plant), cutoff sets (none, predefined set of cutoffs, or user-specified set of cutoffs), and the calculation of prediction of cleavage sites (yes or no).

The results of TargetP 1.1 analysis of the polypeptide sequence as represented by SEQ ID NO: 286 are presented Table M. The "plant" organism group has been selected, no cutoffs defined, and the predicted length of the transit peptide requested. There is no clear prediction of the subcellular localisation, only a weak prediction for a mitochondrial localisation (reliability class 5, which is the lowest reliability). SYB1 proteins therefore may also be located in the cytoplasm.

**Table M:** TargetP 1.1 analysis of the polypeptide sequence as represented by SEQ ID NO: 286

| | |
|---|---|
| Length (AA) | 164 |
| Chloroplastic transit peptide | 0.417 |
| Mitochondrial transit peptide | 0.505 |
| Secretory pathway signal peptide | 0.014 |
| Other subcellular targeting | 0.184 |
| Predicted Location | Mitochondrial |
| Reliability class | 5 |
| Predicted transit peptide length | 12 |

Many other algorithms can be used to perform such analyses, including:

- ChloroP 1.1 hosted on the server of the Technical University of Denmark;
- Protein Prowler Subcellular Localisation Predictor version 1.2 hosted on the server of the Institute for Molecular Bioscience, University of Queensland, Brisbane, Australia;
- PENCE Proteome Analyst PA-GOSUB 2.5 hosted on the server of the University of Alberta, Edmonton, Alberta, Canada;

***Example 45: Assay related to the polypeptide sequences useful in performing the methods of the invention***

The polypeptide sequence as represented by SEQ ID NO: 286 may interact with nucleic acids as well as with proteins, by virtue of the presence of the Zinc finger domains. DNA binding assays are well known in the art, including PCR-assisted DNA binding site selection and a DNA binding gel-shift assay; for a general reference, see Current Protocols in Molecular Biology, Volumes 1 and 2, Ausubel et al. (1994), Current Protocols.

The protein represented by SEQ ID NO: 286 is predicted to interact with several proteins (results from analysis with the SMART database), as shown in Table N. The functionality of a SYB1 protein may thus be tested in a yeast two-hybrid screen with candidate ligand proteins.

**Table N:** Putative interactors of SEQ ID NO: 286

| **Description** | **Identifier** |
|---|---|
| putative cytochrome P450 | At2g46960 |
| Unknown | At5g03670 |
| weak similarity to cytochrome b558/566, subunit B | F18B3.90 |
| hypersensitive-induced response protein | MQB2.6" |
| glycosyl hydrolase family 9 | T21 L14.7 |
| Unknown | F26O13.50 |
| brix domain-containing protein | At5g61770 |
| peterpan | At5g61770 |
| Unknown/Predicted GTPase | Q9SJF1/At1g08410 |
| Nucleotide binding protein | Q9SHS8/At2g27200 |

Furthermore, expression of a SYB1 protein according to the methods of the present invention results in increased seed yield as described below.

***Example 46: Cloning of nucleic acid sequence*** **as** ***represented by SEQ ID NO: 285***

Unless otherwise stated, recombinant DNA techniques are performed according to standard protocols described in (Sambrook (2001) Molecular Cloning: a laboratory manual, 3rd Edition Cold Spring Harbor Laboratory Press, CSH, New York) or in Volumes 1 and 2 of Ausubel et al. (1994), Current Protocols in Molecular Biology, Current Protocols. Standard materials and methods for plant molecular work are described in Plant Molecular Biology Labfax (1993) by R.D.D. Croy, published by BIOS Scientific Publications Ltd (UK) and Blackwell Scientific Publications (UK).

The Arabidopsis thaliana SYB1 gene was amplified by PCR using as template an Arabidopsis thaliana seedling cDNA library (Invitrogen, Paisley, UK). After reverse transcription of RNA extracted from seedlings, the cDNAs were cloned into pCMV Sport 6.0. Average insert size of the bank was 1.5 kb and the original number of clones was of the order of 1.59 x 107 cfu. Original titer was determined to be 9.6 x 105 cfu/ml after first amplification of 6 x 1011 cfu/ml. After plasmid extraction, 200 ng of template was used in a 50 μl PCR mix. Primers prm5539 (SEQ ID NO: 341; sense, start codon in bold, AttB1 site in italic: 5'-ggggacaagtttgtacaaaaaagcaggcttaaacaatgagcagacccggagatt -3') and prm5540 (SEQ ID NO: 342; reverse, complementary, AttB2 site in italic: 5'-ggggaccactttgtacaagaaagctgggtagacaaggctacttcaaaagca -3'), which include the AttB sites for Gateway recombination, were used for PCR amplification. PCR was performed using Hifi Taq DNA polymerase in standard conditions. A PCR fragment of 559 bp (including attB sites) was amplified and purified also using standard methods. The first step of the Gateway procedure, the BP reaction, was then performed, during which the PCR fragment recombines in vivo with the pDONR201 plasmid to produce, according to the Gateway terminology, an "entry clone", p58a. Plasmid pDONR201 was purchased from Invitrogen, as part of the Gateway® technology.

***Example 47: Expression vector construction using the nucleic acid sequence as represented by SEQ ID NO: 285***

The entry clone p58a was subsequently used in an LR reaction with p0640, a destination vector used for Oryza sativa transformation. This vector contains as functional elements within the T-DNA borders: a plant selectable marker; a screenable marker expression cassette; and a Gateway cassette intended for LR in vivo recombination with the nucleic acid sequence of interest already cloned in the entry clone. A rice GOS2 promoter (SEQ ID NO: 343) for constitutive expression (pGOS2) was located upstream of this Gateway cassette.
After the LR recombination step, the resulting expression vector pGOS2::SYB1 (Figure 24) was transformed into Agrobacterium strain LBA4044 according to methods well known in the art.

***Example 48: Plant transformation***

*Rice transformation*

The Agrobacterium containing the expression vector was used to transform Oryza sativa plants. Mature dry seeds of the rice japonica cultivar Nipponbare were dehusked. Sterilization was carried out by incubating for one minute in 70% ethanol, followed by 30 minutes in 0.2%$HgCl_2$, followed by a 6 times 15 minutes wash with sterile distilled water. The sterile seeds were then germinated on a medium containing 2,4-D (callus induction medium). After incubation in the dark for four weeks, embryogenic, scutellum-derived calli were excised and propagated on the same medium. After two weeks, the calli were multiplied or propagated by subculture on the same medium for another 2 weeks. Embryogenic callus pieces were subcultured on fresh medium 3 days before co-cultivation (to boost cell division activity).
Agrobacterium strain LBA4404 containing the expression vector was used for cocultivation. Agrobacterium was inoculated on AB medium with the appropriate antibiotics and cultured for 3 days at 28˚C. The bacteria were then collected and suspended in liquid co-cultivation medium to a density (OD600) of about 1. The suspension was then transferred to a Petri dish and the calli immersed in the suspension for 15 minutes. The callus tissues were then blotted dry on a filter paper and transferred to solidified, co-cultivation medium and incubated for 3 days in the dark at 25˚C. Co-cultivated calli were grown on 2,4-D-containing medium for 4 weeks in the dark at 28˚C in the presence of a selection agent. During this period, rapidly growing resistant callus islands developed. After transfer of this material to a regeneration medium and incubation in the light, the embryogenic potential was released and shoots developed in the next four to five weeks. Shoots were excised from the calli and incubated for 2 to 3 weeks on an auxin-containing medium from which they were, transferred to soil. Hardened shoots were grown under high humidity and short days in a greenhouse.
Approximately 35 independent T0 rice transformants were generated for one construct. The primary transformants were transferred from a tissue culture chamber to a greenhouse. After a quantitative PCR analysis to verify copy number of the T-DNA insert, only single copy transgenic plants that exhibit tolerance to the selection agent were kept for harvest of T1 seed. Seeds were then harvested three to five months after transplanting. The method yielded single locus transformants at a rate of over 50 % (Aldemita and Hodges1996, Chan et al. 1993, Hiei et al. 1994).

*Corn transformation*

Transformation of maize (Zea mays) is performed with a modification of the method described by Ishida et al. (1996) Nature Biotech 14(6): 745-50. Transformation is genotype-dependent in corn and only specific genotypes are amenable to transformation and regeneration. The inbred line A188 (University of Minnesota) or hybrids with A188 as a parent are good sources of donor material for transformation, but other genotypes can be used successfully as well. Ears are harvested from corn plant approximately 11 days after pollination (DAP) when the length of the immature embryo is about 1 to 1.2 mm. Immature embryos are cocultivated with Agrobacterium tumefaciens containing the expression vector, and transgenic plants are recovered through organogenesis. Excised embryos are grown on callus induction medium, then maize regeneration medium, containing the selection agent (for example imidazolinone but various selection markers can be used). The Petri plates are incubated in the light at 25 ˚C for 2-3 weeks, or until shoots develop. The green shoots are transferred from each embryo to maize rooting medium and incubated at 25 ˚C for 2-3 weeks, until roots develop. The rooted shoots are transplanted to soil in the greenhouse. T1 seeds are produced from plants that exhibit tolerance to the selection agent and that contain a single copy of the T-DNA insert.

*Wheat transformation*

Transformation of wheat is performed with the method described by Ishida et al. (1996) Nature Biotech 14(6): 745-50. The cultivar Bobwhite (available from CIMMYT, Mexico) is commonly used in transformation. Immature embryos are co-cultivated with Agrobacterium tumefaciens containing the expression vector, and transgenic plants are recovered through organogenesis. After incubation with Agrobacterium, the embryos are grown in vitro on callus induction medium, then regeneration medium, containing the selection agent (for example imidazolinone but various selection markers can be used). The Petri plates are incubated in the light at 25 °C for 2-3 weeks, or until shoots develop. The green shoots are transferred from each embryo to rooting medium and incubated at 25 °C for 2-3 weeks, until roots develop. The rooted shoots are transplanted to soil in the greenhouse. T1 seeds are produced from plants that exhibit tolerance to the selection agent and that contain a single copy of the T-DNA insert.

*Soybean transformation*

Soybean is transformed according to a modification of the method described in the Texas A&M patent US 5,164,310. Several commercial soybean varieties are amenable to transformation by this method. The cultivar Jack (available from the Illinois Seed foundation) is commonly used for transformation. Soybean seeds are sterilised for in vitro sowing. The hypocotyl, the radicle and one cotyledon are excised from seven-day old young seedlings. The epicotyl and the remaining cotyledon are further grown to develop axillary nodes. These axillary nodes are excised and incubated with Agrobacterium tumefaciens containing the expression vector. After the cocultivation treatment, the explants are washed and transferred to selection media. Regenerated shoots are excised and placed on a shoot elongation medium. Shoots no longer than 1 cm are placed on rooting medium until roots develop. The rooted shoots are transplanted to soil in the greenhouse. T1 seeds are produced from plants that exhibit tolerance to the selection agent and that contain a single copy of the T-DNA insert.

*Rapeseedlcanola transformation*

Cotyledonary petioles and hypocotyls of 5-6 day old young seedling are used as explants for tissue culture and transformed according to Babic et al. (1998, Plant Cell Rep 17: 183-188). The commercial cultivar Westar (Agriculture Canada) is the standard variety used for transformation, but other varieties can also be used. Canola seeds are surface-sterilized for in vitro sowing. The cotyledon petiole explants with the cotyledon attached are excised from the in vitro seedlings, and inoculated with Agrobacterium (containing the expression vector) by dipping the cut end of the petiole explant into the bacterial suspension. The explants are then cultured for 2 days on MSBAP-3 medium containing 3 mg/l BAP, 3 % sucrose, 0.7 % Phytagar at 23 °C, 16 hr light. After two days of co-cultivation with Agrobacterium, the petiole explants are transferred to MSBAP-3 medium containing 3 mg/l BAP, cefotaxime, carbenicillin, or timentin (300 mg/l) for 7 days, and then cultured on MSBAP-3 medium with cefotaxime, carbenicillin, or timentin and selection agent until shoot regeneration. When the shoots are 5 - 10 mm in length, they are cut and transferred to shoot elongation medium (MSBAP-0.5, containing 0.5 mg/l BAP). Shoots of about 2 cm in length are transferred to the rooting medium (MS0) for root induction. The rooted shoots are transplanted to soil in the greenhouse. T1 seeds are produced from plants that exhibit tolerance to the selection agent and that contain a single copy of the T-DNA insert.

Alfalfa transformation

A regenerating clone of alfalfa (Medicago sativa) is transformed using the method of (McKersie et al., 1999 Plant Physiol 119: 839-847). Regeneration and transformation of alfalfa is genotype dependent and therefore a regenerating plant is required. Methods to obtain regenerating plants have been described. For example, these can be selected from the cultivar Rangelander (Agriculture Canada) or any other commercial alfalfa variety as described by Brown DCW and A Atanassov (1985. Plant Cell Tissue Organ Culture 4: 111-112). Alternatively, the RA3 variety (University of Wisconsin) has been selected for use in tissue culture (Walker et al., 1978 Am J Bot 65:654-659). Petiole explants are cocultivated with an overnight culture of Agrobacterium tumefaciens C58C1 pMP90 (McKersie et al., 1999 Plant Physiol 119: 839-847) or LBA4404 containing the expression vector. The explants are cocultivated for 3 d in the dark on SH induction medium containing 288 mg/ L Pro, 53 mg/ L thioproline, 4.35 g/ L K2SO4, and 100 μm acetosyringinone. The explants are washed in half-strength Murashige-Skoog medium (Murashige and Skoog, 1962) and plated on the same SH induction medium without acetosyringinone but with a suitable selection agent and suitable antibiotic to inhibit Agrobacterium growth. After several weeks, somatic embryos are transferred to BOi2Y development medium containing no growth regulators, no antibiotics, and 50 g/ L sucrose. Somatic embryos are subsequently germinated on half-strength Murashige-Skoog medium. Rooted seedlings were transplanted into pots and grown in a greenhouse. T1 seeds are produced from plants that exhibit tolerance to the selection agent and that contain a single copy of the T-DNA insert.

***Example 49: Phenotypic evaluation procedure***

49.1 Evaluation setup

Approximately 35 independent T0 rice transformants were generated. The primary transformants were transferred from a tissue culture chamber to a greenhouse for growing and harvest of T1 seed. Six events, of which the T1 progeny segregated 3:1 for presence/absence of the transgene, were retained. For each of these events, approximately 10 T1 seedlings containing the transgene (hetero- and homo-zygotes) and approximately 10 T1 seedlings lacking the transgene (nullizygotes) were selected by monitoring visual marker expression. The transgenic plants and the corresponding nullizygotes were grown side-by-side at random positions. Greenhouse conditions were of shorts days (12 hours light), 28˚C in the light and 22˚C in the dark, and a relative humidity of 70%.

*Drought screen*

Plants from T2 seeds are grown in potting soil under normal conditions until they approached the heading stage. They are then transferred to a "dry" section where irrigation is withheld. Humidity probes are inserted in randomly chosen pots to monitor the soil water content (SWC). When SWC goes below certain thresholds, the plants are automatically re-watered continuously until a normal level was reached again. The plants are then re-transferred again to normal conditions. The rest of the cultivation (plant maturation, seed harvest) is the same as for plants not grown under abiotic stress conditions. Growth and yield parameters are recorded as detailed for growth under normal conditions.

*Nitrogen use efficiency screen*

Rice plants from T2 seeds are grown in potting soil under normal conditions except for the nutrient solution. The pots are watered from transplantation to maturation with a specific nutrient solution containing reduced N nitrogen (N) content, usually between 7 to 8 times less. The rest of the cultivation (plant maturation, seed harvest) is the same as for plants not grown under abiotic stress. Growth and yield parameters are recorded as detailed for growth under normal conditions.

Four T1 events were further evaluated in the T2 generation following the same evaluation procedure as for the T1 generation but with more individuals per event. From the stage of sowing until the stage of maturity the plants were passed several times through a digital imaging cabinet. At each time point digital images (2048x1536 pixels, 16 million colours) were taken of each plant from at least 6 different angles.

49.2 Statistical analysis: F-test

A two factor ANOVA (analysis of variants) was used as a statistical model for the overall evaluation of plant phenotypic characteristics. An F-test was carried out on all the parameters measured of all the plants of all the events transformed with the gene of the present invention. The F-test was carried out to check for an effect of the gene over all the transformation events and to verify for an overall effect of the gene, also known as a global gene effect. The threshold for significance for a true global gene effect was set at a 5% probability level for the F-test. A significant F-test value points to a gene effect, meaning that it is not only the mere presence or position of the gene that is causing the differences in phenotype.

49.3 Parameters measured

**Biomass-related parameter measurement**

From the stage of sowing until the stage of maturity the plants were passed several times through a digital imaging cabinet. At each time point digital images (2048x1536 pixels, 16 million colours) were taken of each plant from at least 6 different angles.

The plant aboveground area (or leafy biomass) was determined by counting the total number of pixels on the digital images from aboveground plant parts discriminated from the background. This value was averaged for the pictures taken on the same time point from the different angles and was converted to a physical surface value expressed in square mm by calibration. Experiments show that the aboveground plant area measured this way correlates with the biomass of plant parts above ground. The above ground area is the time point at which the plant had reached its maximal leafy biomass. The early vigour is the plant (seedling) aboveground area three weeks post-germination. Increase in root biomass is expressed as an increase in total root biomass (measured as maximum biomass of roots observed during the lifespan of a plant); or as an increase in the root/shoot index (measured as the ratio between root mass and shoot mass in the period of active growth of root and shoot).

**Seed-related parameter measurements**

The mature primary panicles were harvested, counted, bagged, barcode-labelled and then dried for three days in an oven at 37°C. The panicles were then threshed and all the seeds were collected and counted. The filled husks were separated from the empty ones using an air-blowing device. The empty husks were discarded and the remaining fraction was counted again. The filled husks were weighed on an analytical balance. The number of filled seeds was determined by counting the number of filled husks that remained after the separation step. The total seed yield was measured by weighing all filled husks harvested from a plant. Total seed number per plant was measured by counting the number of husks harvested from a plant. Thousand Kernel Weight (TKW) is extrapolated from the number of filled seeds counted and their total weight. The Harvest Index (HI) in the present invention is defined as the ratio between the total seed yield and the above ground area ($mm^2$), multiplied by a factor 106. The total number of flowers per panicle as defined in the present invention is the ratio between the total number of seeds and the number of mature primary panicles. The seed fill rate as defined in the present invention is the proportion (expressed as a %) of the number of filled seeds over the total number of seeds (or florets).

***Example 50: Results of the phenotypic evaluation of the transgenic plants***

The results of the evaluation of transgenic rice plants expressing the nucleic acid sequence useful in performing the methods of the invention are presented in Table O. The percentage difference between the transgenics and the corresponding nullizygotes is also shown, with a P value from the F test below 0.05.

Total seed yield, number of filled seeds, seed fill rate, harvest index and thousand kernel weight are significantly increased in the transgenic plants expressing the nucleic acid sequence useful in performing the methods of the invention, compared to the control plants (in this case, the nullizygotes).

**Table O:** Results of the evaluation of transgenic rice plants expressing the nucleic acid sequence useful in performing the methods of the invention.

| Trait | % Increase (T1 generation) | % Increase (T2 generation) |
|---|---|---|
| Total seed yield | 20 | 23 |
| Number of filled seeds | 17 | 19 |
| Fill rate | 12 | 20 |
| Harvest index | 15 | 20 |
| Thousand Kernel Weight | 2 | 4 |

**Claims**

1. Method for increasing plant yield under abiotic stress relative to control plants, comprising modulating expression in a plant of a nucleic acid sequence encoding a synovial sarcoma translocation (SYT) polypeptide, and optionally selecting for plants having increased yield, wherein said SYT polypeptide comprises from N-terminal to C-terminal: (i) an SNH domain having in increasing order of preference at least 20%, 25%, 30%, 35%, 40%, 45%, 50%, 55%, 60%, 65%, 70%, 75%, 80%, 85%, 90%, 91%, 92%, 93%, 94%, 95%, 96%, 97%, 98%, 99% sequence identity to the SNH domain of SEQ ID NO: 58; and (ii) a Met-rich domain; and (iii) a QG-rich domain.

2. Method according to claim 1, wherein said SNH domain comprises the residues shown in black in Figure 6.

3. Method according to claim 1 or 2, wherein said SYT polypeptide further comprises one or more of the following: (i) SEQ ID NO: 146; (ii) SEQ ID NO: 147; (iii) a Met-rich domain at the N-terminus preceding the SNH domain.

4. Method according to any one of claims 1 to 3, wherein said SYT polypeptide comprises the sequence as represented by SEQ ID NO: 62.

5. Method according to any one of claims 1 to 4, wherein said abiotic stress is one of the two: salt stress or drought stress.

6. Method according to any one of claims 1 to 4, wherein said abiotic stress is reduced nutrient availability stress, preferably reduced nitrogen availability.

7. Method according to any one of claims 1 to 6, wherein said modulated expression is effected by any one or more of the following: T-DNA activation, TILLING, or homologous recombination.

**8.** Method according to any one of claims 1 to 6, wherein said modulated expression is effected by introducing and expressing in a plant, plant part or plant cell a nucleic acid sequence encoding a SYT polypeptide.

**9.** Method according to any one of claims 1 to 8, wherein said increased yield is one or more of the following: total seed yield, number of filled seeds, seed fill rate, TKW, harvest index, biomass and early vigour.

**10.** Method according to claim 8, wherein said nucleic acid sequence is one or more of the following SYT nucleic acid variants:

(i) a portion of a SYT nucleic acid sequence;
(ii) a nucleic acid sequence capable of hybridising with a SYT nucleic acid sequence;
(iii) a splice variant of a SYT nucleic acid sequence;
(iv) an allelic variant of a SYT nucleic acid sequence;
(v) a SYT nucleic acid sequence obtained by gene shuffling;
(vi) a SYT nucleic acid sequence obtained by site-directed mutagenesis.

**11.** Method according to claim 8 or 10, wherein said nucleic acid sequence encodes any one of the SYT polypeptides given in Table 6, or orthologues or paralogues of SEQ ID NO: 62.

**12.** Method according to claim 8 or 10, wherein said nucleic acid sequence encodes the SYT polypeptides as represented by SEQ ID NO: 62.

**13.** Method according to any one of claims 1 to 12, wherein said nucleic acid sequence encoding a SYT polypeptide is overexpressed in a plant.

**14.** Method according to any one of claims 8, 10, or 11, wherein said nucleic acid sequence encoding a SYT polypeptide is of plant origin.

**15.** Method according to any one of claims 8, 10 to 14, wherein said nucleic acid sequence encoding a SYT polypeptide is operably linked to a constitutive promoter.

**16.** Method according to claim 15, wherein said constitutive promoter is plant-derived, preferably from a monocotyledonous plant.

**17.** Method according to claim 15 or 16, wherein said constitutive promoter is a GOS2 promoter.

**18.** Plant, plant part or plant cell obtainable by a method according to any one of claims 1 to 17.

**19.** Use of a SYT nucleic acid sequence or use of a SYT polypeptide for improving yield under abiotic stress relative to control plants.

**20.** Use according to claim 19, wherein said increased yield is one or more of the following: total seed yield, number of filled seeds, seed fill rate, TKW, harvest index, biomass and early vigour.

**Domain organisation in SEQ ID NO: 2**

MCCGSDRLNQIVSSRSSLPISFEEDNNLVTNTDMNHLTVETEDTFASLLELAANNDVEGVRLSIER
DPSCVDEAGLWYGRQKGSKAMVN**DYRTPLMVAATYGSIDVIKLIVSLTDADVNR**ACGN**DQTTALHC**
**AASGGAVNAIQVVKLLLAAGADLNL**LDAEGQRAGDVIVVPPKLEGVKLMLQELLSADGSSTAERNL
RVVTNVPNRSSSPCHSPTGENGGSGSGSPLGSPFKLKSTEFKKEYPVDPSLPDIKNSIYATDEFRM
YSFKVRPCSRAYSHDWTECPFVHPGENARRRDPRKF*HYSCVPCPDFRKGACRRGDMCEYAHGV*FEC
WLHPA*QYRTRLCKDGTGCARRVCFFAHTP*EELRPLYASTGSAVPSPRSNADYAAALSLLPGSPSGV
SVMSPLSPSAAGNGMSHSNMAWPQPNVPALHLPGSNLQSSRLRSSLNARDIPTDEFNMLADYEQQQ
LLNEYSNALSRSGRMKSMPPSNLEDLFSAEGSSSPRFTDSALASAVFSPTHKSAVFNQFQQQQQQ
QSMLSPINTSFSSPKSVDHSLFSGGGRMSPRNVVEPISPMSARVSMLAQCVKQQQQQQQQQQQHQ
FRSLSSRELRTNSSPIVGSPVNNNTWSSKWGSSNGQPDWGMSSEALGKLRSSSSFDGDEPDVSWVQ
SLVKETPAEAKEKAATSSSGEHVMKQPNPVEPVMDHAGLEAWIEQMQLDQLVAQQN

## FIGURE 1

```
ANK:
O04242/1-30      NGHTALHIAASK------------------GDEQCVKLLLEHGA------DPNA
CONSENSUS/80%    .t.sslhhsh.t..................tp.phhphllp.t.......pht.
CONSENSUS/65%    pstosLphAstp..................sphphlphLlptss......shsh
CONSENSUS/50%    sGpTsLHhAsps..................sshcllchLlspus......slst
```

```
C3H1:
TTP_BOVIN/13-4   RYKTELCRTF---SESG-------RCRYGA-KCQFAHGL
CONSENSUS/80%    t.p...C..a.....tG........C.hu..pC.a.H..
CONSENSUS/65%    p.+..hCpha....tpG.......hCthGs.pCpahHs.
CONSENSUS/50%    ph+.slCctF....ppG.......pCshGs.pCpFtHst
```

Legend:

| Class | Key | Residues |
|---|---|---|
| alcohol | o | S,T |
| aliphatic | l | I,L,V |
| any | . | A,C,D,E,F,G,H,I,K,L,M,N,P,Q,R,S,T,V,W,Y |
| aromatic | a | F,H,W,Y |
| charged | c | D,E,H,K,R |
| hydrophobic | h | A,C,F,G,H,I,K,L,M,R,T,V,W,Y |
| negative | - | D,E |
| polar | p | C,D,E,H,K,N,Q,R,S,T |
| positive | + | H,K,R |
| small | s | A,C,D,G,N,P,S,T,V |
| tiny | u | A,G,S |
| turnlike | t | A,C,D,E,G,H,K,N,Q,R,S,T |

## FIGURE 2

**MATGAT table: A) full length sequences,**

| | 1 | 2 | 3 | 4 | 5 | 6 | 7 | 8 | 9 | 10 | 11 | 12 | 13 | 14 | 15 | 16 | 17 | 18 |
|---|---|---|---|---|---|---|---|---|---|---|---|---|---|---|---|---|---|---|
| 1.SEQIDNO2 | | **63.8** | **44** | **58.5** | **54.6** | **49.5** | **43.8** | **38.2** | **36.9** | **38.6** | **37.9** | **35.7** | **38.6** | **38.5** | **35.9** | **27.8** | **48.8** | **43.1** |
| 2.SEQIDNO11 | 78.1 | | **47.2** | **65.7** | **57** | **51.5** | **44** | **39** | **35.8** | **39.6** | **40.8** | **34.4** | **41.5** | **39.7** | **37** | **27.9** | **51.2** | **45.1** |
| 3.SEQIDNO13 | 57.5 | 60.4 | | **45.4** | **48.2** | **39.1** | **39.1** | **37.7** | **37** | **37.2** | **38.1** | **33.5** | **37.1** | **37.1** | **38.2** | **30.2** | **37.9** | **35.2** |
| 4.SEQIDNO15 | 74.3 | 80.1 | 58.3 | | **54.6** | **51.5** | **42.8** | **37.5** | **35.8** | **39.1** | **39.4** | **34.5** | **40.9** | **39.4** | **36.4** | **27** | **50.2** | **43.5** |
| 5.SEQIDNO17 | 69.6 | 72.5 | 57.7 | 68.6 | | **47** | **45.5** | **36.3** | **35.2** | **39.2** | **38.1** | **33.9** | **38.9** | **38.5** | **34.9** | **29.5** | **46.4** | **41.7** |
| 6.SEQIDNO19 | 68.7 | 70.5 | 54.7 | 69.7 | 62.9 | | **43.3** | **37.4** | **35.3** | **36.9** | **38.9** | **33.8** | **38.3** | **38.9** | **35** | **27.5** | **42.6** | **38.7** |
| 7.SEQIDNO21 | 57.4 | 60.8 | 54.5 | 59.3 | 57.9 | 58.9 | | **36.6** | **34.9** | **36.3** | **36.7** | **33.6** | **35.6** | **36.4** | **37.5** | **28.1** | **39.7** | **37.3** |
| 8.SEQIDNO23 | 52.1 | 54.3 | 54.3 | 52.1 | 49.3 | 53.7 | 52.5 | | **68.3** | **50.4** | **48.1** | **45** | **44.5** | **46.8** | **39.9** | **32.1** | **36.7** | **34.9** |
| 9.SEQIDNO25 | 49.9 | 50.9 | 53 | 51.6 | 48.2 | 52.1 | 50.7 | 81.7 | | **49.6** | **47** | **45.3** | **43.2** | **46.6** | **40.1** | **30.9** | **35.8** | **34** |
| 10.SEQIDNO27 | 54.9 | 55.5 | 52.8 | 54.6 | 52.9 | 55 | 53.9 | 65.6 | 63 | | **63.3** | **54.7** | **53.8** | **51** | **45.8** | **31.5** | **40.7** | **38.5** |
| 11.SEQIDNO29 | 54.5 | 57.7 | 53.2 | 58.2 | 52.5 | 55.5 | 54 | 65.9 | 62.7 | 78 | | **53.7** | **54.1** | **47.3** | **41.7** | **31** | **40.3** | **37.6** |
| 12.SEQIDNO31 | 52.2 | 51.7 | 49.8 | 53.8 | 49.1 | 51.7 | 52.8 | 60 | 60 | 68.4 | 69 | | **44.7** | **42.2** | **39.3** | **30.9** | **34.4** | **32.3** |
| 13.SEQIDNO33 | 56.1 | 58.8 | 52.4 | 59.1 | 53.8 | 57.9 | 53.2 | 61 | 59.2 | 69.5 | 69.9 | 62.7 | | **48.7** | **38.3** | **29.8** | **40.8** | **39.2** |
| 14.SEQIDNO35 | 53.4 | 54.3 | 55.4 | 53.4 | 52.1 | 55.5 | 54.9 | 63.4 | 63.9 | 63.6 | 62.9 | 58 | 62.1 | | **42.4** | **31.1** | **38** | **38.1** |
| 15.SEQIDNO37 | 50.3 | 50.9 | 55.3 | 51.1 | 47.5 | 49.9 | 53.3 | 58.7 | 57.2 | 60.1 | 57.3 | 55.8 | 55 | 60 | | **39.8** | **34.5** | **33.9** |
| 16.SEQIDNO39 | 41.8 | 40.6 | 43.8 | 41.1 | 39.5 | 41.2 | 43.1 | 49.4 | 49 | 46.6 | 46 | 43.8 | 44.1 | 47.1 | 53.7 | | **28.1** | **26.7** |
| 17.SEQIDNO41 | 63.9 | 65.5 | 50.6 | 64.8 | 64.1 | 58.5 | 53.3 | 50.2 | 48.1 | 54.8 | 54.4 | 51.3 | 56 | 50.2 | 48.5 | 38.1 | | **54.8** |
| 18.SEQIDNO43 | 58.3 | 61.5 | 50.3 | 59.9 | 60.5 | 57 | 52.7 | 50.3 | 49.5 | 53.3 | 54.7 | 48.3 | 56.6 | 53.4 | 48.9 | 38.1 | 67.3 | |

**FIGURE 3 A**

B) Zn-finger domain sequences

| | 1 | 2 | 3 | 4 | 5 | 6 | 7 | 8 | 9 | 10 | 11 | 12 | 13 | 14 | 15 | 16 | 17 | 18 |
|---|---|---|---|---|---|---|---|---|---|---|---|---|---|---|---|---|---|---|
| 1.SEQIDNO2 | | 92.0 | 96.0 | 88.0 | 88.0 | 84.0 | 72.0 | 88.0 | 80.0 | 80.0 | 88.0 | 88.0 | 76.0 | 84.0 | 84.0 | 76.0 | 88.0 | 92.0 |
| 2.SEQIDNO11 | 92.0 | | 88.0 | 80.0 | 92.0 | 80.0 | 76.0 | 84.0 | 76.0 | 76.0 | 80.0 | 80.0 | 72.0 | 80.0 | 76.0 | 72.0 | 88.0 | 88.0 |
| 3.SEQIDNO13 | 96.0 | 88.0 | | 88.0 | 84.0 | 84.0 | 72.0 | 84.0 | 76.0 | 76.0 | 84.0 | 84.0 | 76.0 | 80.0 | 88.0 | 80.0 | 84.0 | 88.0 |
| 4.SEQIDNO15 | 92.0 | 84.0 | 96.0 | | 84.0 | 92.0 | 68.0 | 80.0 | 72.0 | 76.0 | 80.0 | 80.0 | 76.0 | 80.0 | 80.0 | 76.0 | 80.0 | 84.0 |
| 5.SEQIDNO17 | 88.0 | 92.0 | 84.0 | 88.0 | | 84.0 | 68.0 | 80.0 | 72.0 | 76.0 | 76.0 | 76.0 | 72.0 | 80.0 | 72.0 | 72.0 | 88.0 | 88.0 |
| 6.SEQIDNO19 | 84.0 | 80.0 | 88.0 | 96.0 | 84.0 | | 68.0 | 80.0 | 72.0 | 76.0 | 76.0 | 76.0 | 76.0 | 76.0 | 76.0 | 76.0 | 80.0 | 80.0 |
| 7.SEQIDNO21 | 84.0 | 92.0 | 84.0 | 80.0 | 84.0 | 80.0 | | 68.0 | 60.0 | 60.0 | 64.0 | 64.0 | 60.0 | 64.0 | 72.0 | 68.0 | 68.0 | 68.0 |
| 8.SEQIDNO23 | 88.0 | 84.0 | 84.0 | 80.0 | 80.0 | 80.0 | 80.0 | | 88.0 | 84.0 | 88.0 | 88.0 | 80.0 | 84.0 | 84.0 | 80.0 | 80.0 | 80.0 |
| 9.SEQIDNO25 | 88.0 | 84.0 | 84.0 | 80.0 | 80.0 | 80.0 | 80.0 | 100.0 | | 88.0 | 92.0 | 92.0 | 84.0 | 80.0 | 80.0 | 72.0 | 72.0 | 72.0 |
| 10.SEQIDNO27 | 84.0 | 80.0 | 80.0 | 80.0 | 84.0 | 84.0 | 76.0 | 92.0 | 92.0 | | 92.0 | 92.0 | 92.0 | 84.0 | 80.0 | 76.0 | 80.0 | 76.0 |
| 11.SEQIDNO29 | 92.0 | 84.0 | 88.0 | 84.0 | 80.0 | 80.0 | 80.0 | 96.0 | 96.0 | 92.0 | | 100.0 | 88.0 | 88.0 | 88.0 | 76.0 | 76.0 | 80.0 |
| 12.SEQIDNO31 | 92.0 | 84.0 | 88.0 | 84.0 | 80.0 | 80.0 | 80.0 | 96.0 | 96.0 | 92.0 | 100.0 | | 88.0 | 88.0 | 88.0 | 76.0 | 76.0 | 80.0 |
| 13.SEQIDNO33 | 80.0 | 76.0 | 84.0 | 84.0 | 76.0 | 88.0 | 76.0 | 88.0 | 88.0 | 92.0 | 88.0 | 88.0 | | 80.0 | 80.0 | 76.0 | 72.0 | 72.0 |
| 14.SEQIDNO35 | 88.0 | 88.0 | 84.0 | 84.0 | 88.0 | 80.0 | 80.0 | 92.0 | 92.0 | 92.0 | 96.0 | 96.0 | 88.0 | | 84.0 | 76.0 | 88.0 | 92.0 |
| 15.SEQIDNO37 | 88.0 | 80.0 | 92.0 | 88.0 | 76.0 | 84.0 | 80.0 | 92.0 | 92.0 | 88.0 | 96.0 | 96.0 | 92.0 | 92.0 | | 88.0 | 72.0 | 76.0 |
| 16.SEQIDNO39 | 80.0 | 76.0 | 84.0 | 84.0 | 76.0 | 84.0 | 76.0 | 84.0 | 84.0 | 88.0 | 84.0 | 84.0 | 88.0 | 80.0 | 88.0 | | 72.0 | 72.0 |
| 17.SEQIDNO41 | 92.0 | 96.0 | 88.0 | 88.0 | 100.0 | 88.0 | 84.0 | 84.0 | 84.0 | 88.0 | 84.0 | 84.0 | 80.0 | 88.0 | 80.0 | 80.0 | | 96.0 |
| 18.SEQIDNO43 | 96.0 | 96.0 | 92.0 | 92.0 | 96.0 | 84.0 | 84.0 | 84.0 | 84.0 | 84.0 | 88.0 | 88.0 | 80.0 | 92.0 | 84.0 | 76.0 | 96.0 | |

FIGURE 3 B

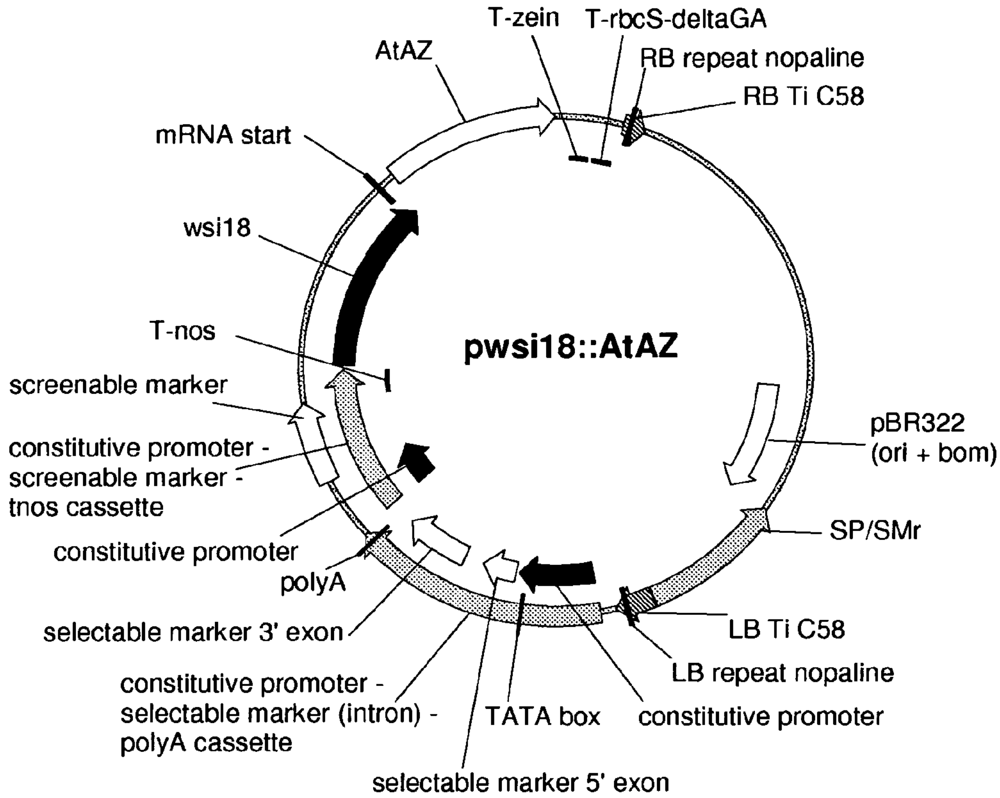


**FIGURE 4**

**Plant SYT-like polypeptide structure**

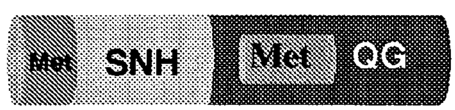


**Mammalian SYT-like polypeptide structure**

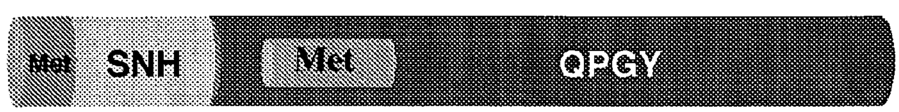


**FIGURE 5**

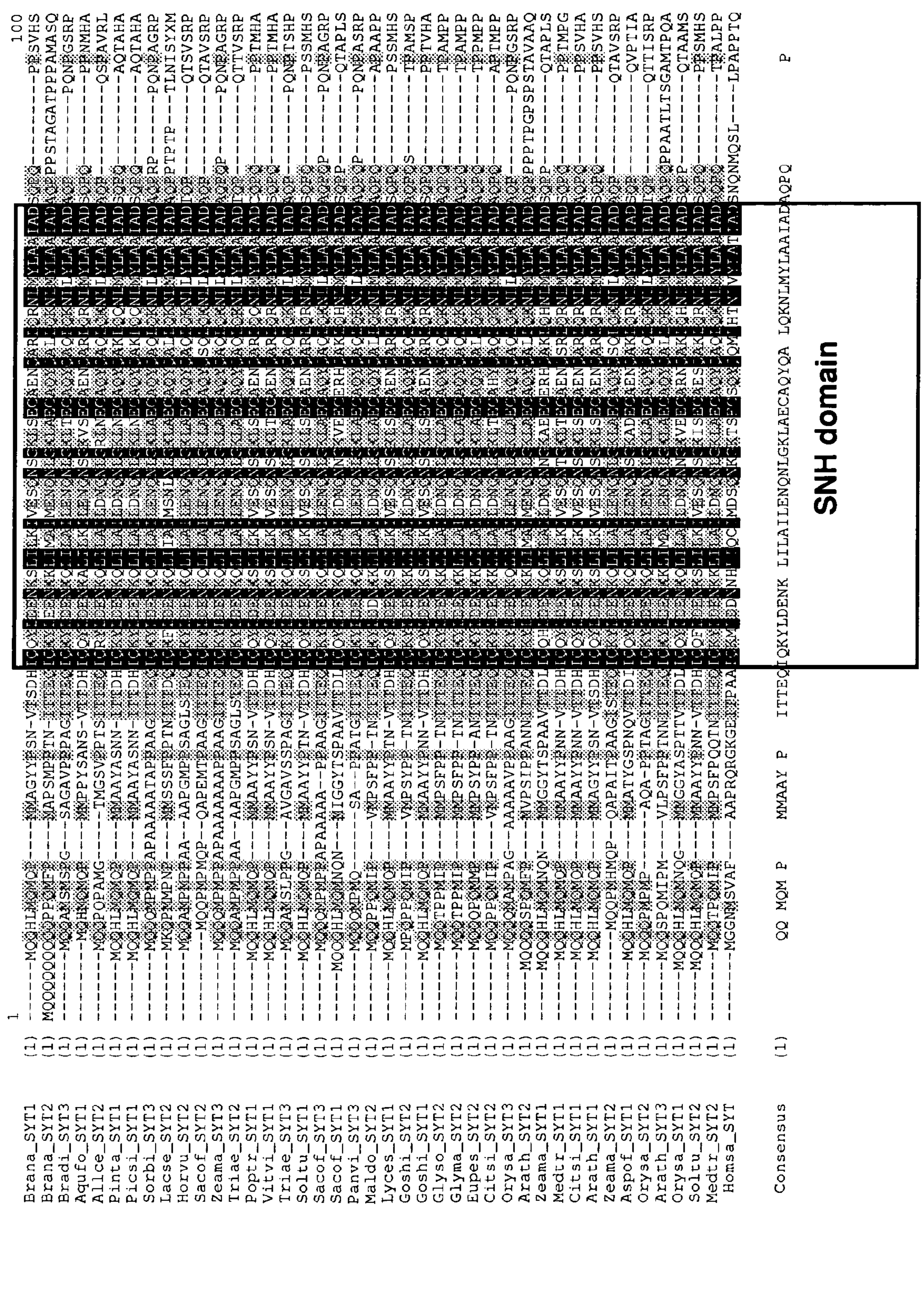


FIGURE 6

```
                                    N-terminal                  SNH domain
                                    Met-rich

                     1                                                50
Brana_SYT1     (1)   ------MQQHLMQMQP----MMAGYYPSN-VTSDHIQQYLDENKSLILKI
Aqufo_SYT1     (1)   --------MQHMQMQP----MMPPYSANS-VTTDHIQQYLDENKALILKI
Picsi_SYT1     (1)   ------MQQHLMQMQP----MMAAYASNN-ITTDHIQKYLDENKQLILAI
Pinta_SYT1     (1)   ------MQQHLMQMQP----MMAAYASNN-ITTDHIQKYLDENKQLILAI
Poptr_SYT1     (1)   ------MQQHLMQMQP----MMAAYYPSN-VTTDHIQQYLDENKSLILKI
Vitvi_SYT1     (1)   ------MQQHLMQMQP----MMAAYYPSN-VTTDHIQQYLDENKSLILKI
Soltu_SYT1     (1)   ------MQQHLMQMQP----MMAAYYPTN-VTTDHIQQYLDENKSLILKI
Lyces_SYT1     (1)   ------MQQHLMQMQP----MMAAYYPTN-VTTDHIQQYLDENKSLILKI
Goshi_SYT1     (1)   ------MQQHLMQMQP----MMAAYYPNN-VTTDHIQQYLDENKSLILKI
Zeama_SYT1     (1)   -----MQQQHLMQMNQN---MMGGYTSPAAVTTDIIQQHLDENKQLILAI
Medtr_SYT1     (1)   ------MQQHLMQMQP----MMAAYYPNN-VTTDHIQQYLDENKSLILKI
Citsi_SYT1     (1)   ------MQQHLMQMQP----MMAAYYPNN-VTTDHIQQYLDENKSLILKI
Arath_SYT1     (1)   ------MQQHLMQMQP----MMAGYYPSN-VTSDHIQQYLDENKSLILKI
Aspof_SYT1     (1)   ------MQQHLMQMQP----MMATYGSPNQVTTDIIQQYLDENKQLILAI
Orysa_SYT1     (1)   -----MQQQHLMQMNQG---MMGGYASPTTVTTDIIQQYLDENKQLILAI
Sacof_SYT1     (1)   -----MQQQHLMQMNQN---MIGGYTSPAAVTTDIIQQYLDENKQLILAI
Allce_SYT2     (1)   -------MQQPQPAMG-----TMGSVPPTSITTEQIQRYLDENKQLILAI
Lacse_SYT2     (1)   -------MKQPMMPNP----MMSSSFPPTNITTDQIQKFLDENKQLIIAI
Horvu_SYT2     (1)   -------MQQAMPMPPAA--AAPGMPPSAGLSTEQIQKYLDENKQLILAI
Brana_SYT2     (1)   MQQQQQQQQQPPQMFP----MAPSMPPTN-ITTEQIQKYLEENKKLIMAI
Sacof_SYT2     (1)   ---------MQQPMPMQP--QAPEMTPAAGITTEQIQKYLDENKQLILAI
Triae_SYT2     (1)   -------MQQAMPMPPAA--AAPGMPPSAGLSTEQIQKYLDENKQLILAI
Maldo_SYT2     (1)   -------MQQPPQMIP----VMPSFPP-TNITTEQIQKYLDDNKKLILAI
Goshi_SYT2     (1)   -------MPQPPQMIP----VMPSYPP-TNITTEQIQKYLDENKKLILAI
Glyso_SYT2     (1)   -------MQQTPPMIP----MMPSFPP-TNITTEQIQKYLDENKKLILAI
Glyma_SYT2     (1)   -------MQQTPPMIP----MMPSFPP-TNITTEQIQKYLDENKKLILAI
Eupes_SYT2     (1)   -------MQQQPQMMP----MMPSYPP-ANITTEQIQKYLDENKKLILAI
Arath_SYT2     (1)   -----MQQQQSPQMFP----MVPSIPPANNITTEQIQKYLDENKKLIMAI
Citsi_SYT2     (1)   -------MQQPPQMIP----VMPSFPP-TNITTEQIQKYLDENKKLILAI
Zeama_SYT2     (1)   ---------MQQPMHMQP--QAPAITPAAGISTEQIQKYLDENKQLILAI
Orysa_SYT2     (1)   ------MQQQPMPMP------AQA-PPTAGITTEQIQKYLDENKQLILAI
Soltu_SYT2     (1)   -----MQQQHLMQMQP----MMAAYYPNN-VTTDHIQQFLDENKSLILKI
Medtr_SYT2     (1)   -------MQQTPQMIP----MMPSFPQQTNITTEQIQKYLDENKKLILAI
Sorbi_SYT3     (1)   -------MQQQMPMPPAPAAAAATAPPAAGITTEQIQKYLDENKQLILAI
Zeama_SYT3     (1)   -------MQQQMPMPPAPAAAAAAAPPAAGITTEQIQKYLDENKQLILAI
Bradi_SYT3     (1)   -------MQQAMSMSPG---SAGAVPPPAGITTEQIQKYLDENKQLILAI
Triae_SYT3     (1)   -------MQQAMSLPPG---AVGAVSSPAGITTEQIQKYLDENKQLILAI
Sacof_SYT3     (1)   -------MQQQMPMPPAPAAAAA--PPAAGITTEQIQKYLDENKQLILAI
Panvi_SYT3     (1)   -------MQQQMPMQ------SA--PPATGITTEQIQKYLDENKQLILAI
Orysa_SYT3     (1)   -------MQQQMAMPAG--AAAAAVPPAAGITTEQIQKYLDENKQLILAI
Arath_SYT3     (1)   ------MQQSPQMIPM----VLPSFPPTNNITTEQIQKYLDENKKLIMAI
 Consensus     (1)           QQ MQM P    MMAAY P   ITTEQIQKYLDENK LILAI
```

**FIGURE 7**

```
                          SNH domain (continued)          Met-rich /
                                                          QG-rich domain
                   51                                                100
Brana_SYT1  (40)   VESQNSGKLSECAENQARLQRNLMYLAAIADSQPQ---------PPSVHS
Aqufo_SYT1  (38)   LENQNSGKVSECAENQARLQRNLMYLAAIADSQPQ---------PPNMHA
Picsi_SYT1  (40)   LDNQNLGKLNECAQYQAKLQQNLMYLAAIADSQPQ---------AQTAHA
Pinta_SYT1  (40)   LDNQNLGKLNECAQYQAKLQQNLMYLAAIADSQPQ---------AQTAHA
Poptr_SYT1  (40)   VESQNSGKLSECAENQARLQRNLMYLAAIADCQPQ---------PPTMHA
Vitvi_SYT1  (40)   VESQNSGKLTECAENQARLQRNLMYLAAIADSQPQ---------PPTMHA
Soltu_SYT1  (40)   VESQNSGKLSECAENQARLQRNLMYLAAIADSQPQ---------PSSMHS
Lyces_SYT1  (40)   VESQNSGKLSECAENQARLQRNLMYLAAIADSQPQ---------PSSMHS
Goshi_SYT1  (40)   VESQNSGKLSECAENQARLQRNLMYLAAIADSQPQ---------PPTVHA
Zeama_SYT1  (43)   LDNQNNGKAEECERHQAKLQHNLMYLAAIADSQPP---------QTAPLS
Medtr_SYT1  (40)   VESQNTGKLTECAENQSRLQRNLMYLAAIADSQPQ---------PPTMPG
Citsi_SYT1  (40)   VESQNSGKLSECAENQARLQRNLMYLAAIADAQPQ---------PPSVHA
Arath_SYT1  (40)   VESQNSGKLSECAENQARLQRNLMYLAAIADSQPQ---------PPSVHS
Aspof_SYT1  (41)   LENQNSGKADECAENQAKLQRNLMYLAAIADSQP----------QVPTIA
Orysa_SYT1  (43)   LDNQNNGKVEECARNQAKLQHNLMYLAAIADSQPP---------QTAAMS
Sacof_SYT1  (43)   LDNQNNGKVEECERHQAKLQHNLMYLAAIADSQPP---------QTAPLS
Allce_SYT2  (39)   LDNQNLGRLNECAQYQAQLQKNLLYLAAIADAQP---------QSPAVRL
Lacse_SYT2  (40)   MSNLNLGKLAECAQYQALLQKNLMYLAAIADAQPPTPTP---TLNISYXM
Horvu_SYT2  (42)   LENQNLGKLAECAQYQAQLQKNLLYLAAIADTQP---------QTSVSRP
Brana_SYT2  (46)   MENQNLGKLAECAQYQALLQKNLMYLAAIADAQPPPSTAGATPPPAMASQ
Sacof_SYT2  (40)   LENQNLGKLAECAQYQSQLQKNLLYLAAIADAQP---------QTAVSRP
Triae_SYT2  (42)   LENQNLGKLAECAQYQAQLQKNLLYLAAIADTQP---------QTTVSRP
Maldo_SYT2  (39)   LDNQNLGKLAECAQYQALLQKNLMYLAAIADAQPQ---------APAAPP
Goshi_SYT2  (39)   LDNQNLGKLAECAQYQAQLQKNLMYLAAIADAQPQS--------TPAMSP
Glyso_SYT2  (39)   LDNQNLGKLAECAQYQAQLQKNLMYLAAIADAQPQ---------TPAMPP
Glyma_SYT2  (39)   LDNQNLGKLAECAQYQAQLQKNLMYLAAIADAQPQ---------TPAMPP
Eupes_SYT2  (39)   LDNQNLGKLAECAQYQALLQKNLMYLAAIADAQPQ---------TPPMPP
Arath_SYT2  (42)   MENQNLGKLAECAQYQALLQKNLMYLAAIADAQPPPPTPGPSPSTAVAAQ
Citsi_SYT2  (39)   LDNQNLGKLTECAHYQAQLQKNLMYLAAIADAQPQ---------APTMPP
Zeama_SYT2  (40)   LENQNLGKLAECAQYQSQLQKNLLYLAAIADAQP---------QTAVSRP
Orysa_SYT2  (38)   LENQNLGKLAECAQYQAQLQKNLLYLAAIADTQP---------QTTISRP
Soltu_SYT2  (41)   VESQNSGKISECAESQAKLQRNLMYLAAIADSQPQ---------PPSMHS
Medtr_SYT2  (40)   LDNQNLGKLAECAQYQAQLQKNLMYLAAIADAQPQ---------TPALPP
Sorbi_SYT3  (44)   LENQNLGKLAECAQYQAQLQKNLLYLAAIADAQPRP------PQNPAGRP
Zeama_SYT3  (44)   LENQNLGKLAECAQYQAQLQKNLLYLAAIADAQPQP------PQNPAGRP
Bradi_SYT3  (41)   LENQNLGKLTECAQYQAQLQKNLLYLAAIADAQP--------PQNPGSRP
Triae_SYT3  (41)   LENQNLGKLAECAQYQAQLQKNLLYLAAIADAQP--------PQNPTSHP
Sacof_SYT3  (42)   LENQNLGKLAECAQYQAQLQKNLLYLAAIADAQPQP------PQNPAGRP
Panvi_SYT3  (36)   LENQNLGKLAECAQYQAQLQKNLLYLAAIADAQPQP------PQNPASRP
Orysa_SYT3  (42)   LENQNLGKLAECAQYQAQLQKNLLYLAAIADAQP--------PQNPGSRP
Arath_SYT3  (41)   LENQNLGKLAECAQYQALLQKNLMYLAAIADAQPQPPAATLTSGAMTPQA
 Consensus  (51)   LENQNLGKLAECAQYQA LQKNLMYLAAIADAQPQ          P
```

**FIGURE 7 (continued)**

**Met-rich / QG-rich domain (continued)**

```
                        101                                              150
Brana_SYT1      (81)   QYGSAGGGLIQGEGAS----HYLQQQQATQQ-----------------QQ
Aqufo_SYT1      (79)   QYSNAG-----IPPGA----HYLQHQQAQQ-------------------
Picsi_SYT1      (81)   QIPPNA----VMQSGG----HYMQHQQAQQQ------------------
Pinta_SYT1      (81)   QIPPNA----VMQSGG----HYMQHQQAQQQ------------------
Poptr_SYT1      (81)   QFPSSG---IMQPGA-----HYMQHQQAQQ-------------------
Vitvi_SYT1      (81)   QFPPSG---IVQPGA-----HYMQHQQAQQ-------------------
Soltu_SYT1      (81)   QFSSGG----MMQPGT----HSYLQQQQQQQ---------------AQQ
Lyces_SYT1      (81)   QFSSGG----MMQPGT----HSYLQQQQQQQ---------------AQQ
Goshi_SYT1      (81)   QFPSGG---IMQPGAG----HYMQHQQAQQ-------------------
Zeama_SYT1      (84)   QYPSN----LMMQPGP----RYMPP-QSGQ------------------M
Medtr_SYT1      (81)   QYPSSG---MMQQGG-----HYMQAQQAQQ-------------------
Citsi_SYT1      (81)   QFSSGG---IMQPGA-----HYMQHQQSQP-------------------
Arath_SYT1      (81)   QYGSAGGGMIQGEGGS----HYLQQQQATQQ-----------------QQ
Aspof_SYT1      (81)   QYPPNA--VAAMQSSA----RYMQQHQAAQ------------------Q
Orysa_SYT1      (84)   QYPSN----LMMQSGA----RYMPQ-QSAQ------------------M
Sacof_SYT1      (84)   QYPSN----LMMQPGP----RYMPP-QSGQ------------------M
Allce_SYT2      (80)   QMMPQG---AAATPQAGNQFMQQQSPNFPPKTG---------------MQ
Lacse_SYT2      (87)   GPVPHP---GMPQQGG--FYMAQQHPQAAVMTAQP-------------PS
Horvu_SYT2      (83)   QMAPPA---ASPGAG----HYMSQVPMFPPRT-----------------P
Brana_SYT2      (96)   MGAPHP---GMQPP-----SYFMQHPQASGMAQQA-PPAGIFPPRGPLQF
Sacof_SYT2      (81)   QMAPPG---ALPGVG----QYMSQVPMFPPRT-----------------P
Triae_SYT2      (83)   QMAPPS---ASPGAG----HYMSQVPMFPPRT-----------------P
Maldo_SYT2      (80)   QMAPHP---AMQQA-----GYYMQHPQAAAMAQQQ----GIFSPKMPMQF
Goshi_SYT2      (81)   QMAPHP---AMQPG-----GYFMQHPQAAAMSQQP----GMYPQKVPLQF
Glyso_SYT2      (80)   QMAPHP---AMQP------GFYMQHPQAAAAAMAQQQQQGMFPQKMPLQF
Glyma_SYT2      (80)   QMAPHP---AMQP------GFYMQHPQAAAAAMAQQQQ-GMFPQKMPLQF
Eupes_SYT2      (80)   QMSPHP---AMQQG-----AYYMQHPQAAAAAMAHQSG-IFPPKMSPLQF
Arath_SYT2      (92)   MATPHS---GMQPP-----SYFMQHPQAS--------PAGIFAPRGPLQF
Citsi_SYT2      (80)   QMAPHP---AMQAS-----GYYMQHPQAAAMAQQQ----GIFPQKMPLQF
Zeama_SYT2      (81)   QMAPPG---GSPGVG----QYMSQVPMFPPRT-----------------P
Orysa_SYT2      (79)   QMVPHG---ASPGLGG---QYMSQVPMFPPRT-----------------P
Soltu_SYT2      (82)   QLASGG----MMQGGA----HYMQQQQ--------------------AQQ
Medtr_SYT2      (81)   QMAPHP---AMQQ------GFYMQHPQAAAMAQQQ----GMFPQKMPMQF
Sorbi_SYT3      (88)   QMMQPG---IVPGAG----HYMSQVPMFPPRT-----------------P
Zeama_SYT3      (88)   QMMQPG---IVPGAG----HYMSQVPMFPPRT-----------------P
Bradi_SYT3      (83)   QMVQPG---GMPGAG----HYMSQVPMFPPRT-----------------P
Triae_SYT3      (83)   QMVQPG---SMQGAG----HYMSQVPMFPPRT-----------------P
Sacof_SYT3      (86)   QMMQPG---IVPGAG----HYMSQVPMFPPRT-----------------P
Panvi_SYT3      (80)   QMMQPG---MVPGAG----HYMSQVPMFPPRT-----------------P
Orysa_SYT3      (84)   QMMQPG---ATPGAG----HYMSQVPMFPPRT-----------------P
Arath_SYT3      (91)   MAPNPS---SMQPPP----SYFMQQHQAVG-MAQQ-IPPGIFPPRGPLQF
Consensus      (101)   QM    G    M   G      YYMQ PQA
```

**FIGURE 7 (continued)**

**Met-rich / QG-rich domain (continued)**

```
                      151                                              200
Brana_SYT1    (110)   MTQQSLMAAR---SSMMYQQQQQ--------PYATLQHQQ-----LHHSQ
Aqufo_SYT1    (100)   MTQQSLMAAR---SNMLYAQPITG----------MQQ-QQ-----AMHSQ
Picsi_SYT1    (104)   VTPQSLMAAR---SSMLYSQQPMAALHQAQQQQQQHQQQQQ---SLHSQ
Pinta_SYT1    (104)   VTPQSLMAAR---SSILYAQQ----QQQQHQQHQQQQQQQQQ---SLHSQ
Poptr_SYT1    (103)   MTPQALMAAR---SSMLQYAQQP---------FSALQQQQ-----ALHSQ
Vitvi_SYT1    (103)   MTPQSLLAAR---SSML-YTQQP---------FSALQQQQ-----AIHSQ
Soltu_SYT1    (108)   MATQQLMAAR--SSSMLYGQQQQQ----Q--QQSQLSQFQQG---LHSSQ
Lyces_SYT1    (108)   MATQQLMAAR--SSSMLYGQQQ---------QQSQLSQYQQG---LHSSQ
Goshi_SYT1    (104)   MTQQSLMAAR---SSML-YSQQP---------FSALQQQQQQ---ALHSQ
Zeama_SYT1    (106)   MNPQSLMAAR---SSMMYAHPS----------LSPLQQQQ-----AAHGQ
Medtr_SYT1    (103)   MTQQQLMAAR---SSLM-YAQQ-------------LQQQQ-----ALQSQ
Citsi_SYT1    (103)   MTPQSLMAAR---SSMV-YSQQQ---------FSVLQQQQ-----ALHGQ
Arath_SYT1    (110)   MTQQSLMAAR---SSMLYAQQQQQ----QQ-PYATLQHQQ-----LHHSQ
Aspof_SYT1    (106)   MTPQSLMAAR---SSMLYSQSP----------MSALQQQQQQ--AAMHSQ
Orysa_SYT1    (106)   MAPQSLMAAR---SSMMYAQPA----------LSPLQQQQQQQAAAAHGQ
Sacof_SYT1    (106)   MSPQSLMAAR---SSMMYAHPS----------MSPLQQQQ-----AAHGQ
Allce_SYT2    (112)   FTPQQVQELQ---------QQQ----------LQHQPHMM----PPFQGQ
Lacse_SYT2    (119)   GFPQPMPGMQ---------FNS-----------------P----QAIQGQ
Horvu_SYT2    (109)   LTPQQMQEQQ---------LQQ-------------QQAQM----LPFAGQ
Brana_SYT2    (137)   GSPHQLQDPQ---------QQ---------------HMHQ----QAMQGH
Sacof_SYT2    (107)   LTPQQMQEQQ---------LQQ-------------QQAQL----LNFSGL
Triae_SYT2    (109)   LTPQQMQEQQ---------LQQ-------------QQAQM----LPFAGQ
Maldo_SYT2    (118)   NNMHQMHDP---------------------------QQHQ----QAMQGQ
Goshi_SYT2    (119)   NSPHQMQDPQ---------HLLY-------------QQHQ----QAMQGQ
Glyso_SYT2    (121)   GNPHQMQEQQ---------QQ----------------LHQ----QAIQGQ
Glyma_SYT2    (120)   GNPHQMQEQQ---------QQ----------------LHQ----QAIQGQ
Eupes_SYT2    (121)   NNPHQIQDPQ--------------------------QLHQ----AALQGQ
Arath_SYT2    (126)   GSPLQFQDPQ---------QQQ--------------QIHQ----QAMQGH
Citsi_SYT2    (118)   NNPHQLQDPQ---------QQLH-------------QH------QAMQAQ
Zeama_SYT2    (107)   LTPQQMQEQQ---------LQQ-------------QQAQL----LNFSGQ
Orysa_SYT2    (106)   LTPQQMQEQQ---------LQQ-------------QQAQL----LSFGGQ
Soltu_SYT2    (104)   LTTQSLMAAARSSSSMLYGQQQQQ----QQQQLSSLQQQQAA---FHSQQ
Medtr_SYT2    (118)   GNPHQMQDQQ---------HQQQQ-----------QQLHQ----QAMQGQ
Sorbi_SYT3    (114)   LTPQQMQEQQ---------QQQ----------LQQQQAQA----LAFPGQ
Zeama_SYT3    (114)   LTPQQMQEQQ---------QQQQF-------QQQQQQVQA----LTFPGQ
Bradi_SYT3    (109)   LTPQQMQEQQ---------HQQ----------LQQQQAQA----LAFPSQ
Triae_SYT3    (109)   LTPQQMQEQQ---------HQQ----------LQQQQAQA----LSFPAQ
Sacof_SYT3    (112)   LTPQQMQEQQ---------QQQ----------LQQQQAQA----LTFPGQ
Panvi_SYT3    (106)   LTPQQMQEQQ---------QQQQ--------QLQQQQAQA----LAFPGQ
Orysa_SYT3    (110)   LTPQQMQEQQ---------QQQ----------LQQQQAQA----LAFPGQ
Arath_SYT3    (132)   GSPHQFLDPQ---------QQ----------------LHQ----QAMQGH
 Consensus    (151)   MTPQQLQE Q         QQQ              Q QQ     A  GQ
```

**FIGURE 7 (continued)**

**Met-rich / QG-rich domain (continued)**

```
                          201                                              250
Brana_SYT1      (144)     LGMSSSS-GGG---------SSGLHILQG---EAG-------GFHEFGRG-
Aqufo_SYT1      (131)     LGMSS-----GG-------NSGLHMMHNEG---S-------MGGSGALGS
Picsi_SYT1      (148)     LGINS-----GG-------SSGLHMLHGETN-MG-------CNGPLSSGG
Pinta_SYT1      (144)     LGINS-----GG-------SSGLHMLHGETN-MG-------CNGPLSSGG
Poptr_SYT1      (136)     LGMSS-----GG-------SAGLHMMQSEANTAG-------GSGALGAGR
Vitvi_SYT1      (135)     LGMGS-----GG-------SAGLHMLQSEGSNPG-------GNGTLGTGG
Soltu_SYT1      (147)     LGMSSG--SGGS-------TGLHHMLQSE-------------SSPHGGGF
Lyces_SYT1      (144)     LGMSSG--SGGS-------TGLHHMLQSE-------------SSPHGGGF
Goshi_SYT1      (138)     LGMSS-----GG-------STGLHMLQTESSTAG-------GSGALGAGG
Zeama_SYT1      (138)     LGMAPGGGGGGT-------TSGFSILHGEASMGGGAGAGAGNNMMNAGM
Medtr_SYT1      (131)     LGMNS-----SG-------SQGLHMLHSEGANVG-------GNSSLGAG-
Citsi_SYT1      (135)     LGMSS-----GG-------SSGLHMLQSEGSTAG-------GSGSLGGGG
Arath_SYT1      (147)     LGMSSSS-GGGG-------SSGLHILQG---EAG-------GFHDFGRG-
Aspof_SYT1      (141)     LAMSSGGNNS-S-------TGGFTILHGEASIGG-------NGSMNSGGV
Orysa_SYT1      (143)     LGMGSGG----T-------TSGFSILHGEASMGGGGGGGAGNSMMNAGV
Sacof_SYT1      (138)     LGMASGGGGG-T-------TSGFNILHGEASMGG-AGGACAGNNMMNAGM
Allce_SYT2      (139)     MGMRP--MNGMQ------------AAMHADSSLAY------NTNNKQDAG-
Lacse_SYT2      (139)     MGGRSGGPPSS---------------AASDVWRG------SMQDGG----
Horvu_SYT2      (133)     MVARPGAVNGIP------------QAPQVEQP------------------
Brana_SYT2      (159)     MGMRPMGINNNN------------GMQHQMQQQP------ETSLGGS---
Sacof_SYT2      (131)     MVARPGMVNGMP------------QSIQVQQAQ----------PPPAGN--
Triae_SYT2      (133)     MVARPGAVNGMP------------QAPQVEP-------------------
Maldo_SYT2      (137)     MGMRPGGPNGMP------------SMLHTEATHGG------GS-GGPNSAG
Goshi_SYT2      (143)     MGIRPGGPNNSM------------HPMHSEASLGG------GSSGGPPQPS
Glyso_SYT2      (142)     MGLRPGGINNGM------------HPMHNE----G------GNSGGPPSAT
Glyma_SYT2      (141)     MGLRPGDINNGM------------HPMHSEAALGG------GNSGGPPSAT
Eupes_SYT2      (141)     MGMRPMGPNNGM------------HPMHPEANLGG------SN--------
Arath_SYT2      (149)     MGIRPMGMTNN-------------GMQHAMQQ--P------ETGLGG----
Citsi_SYT2      (140)     MGMRPGATNNGM------------HPMHAESSLGG------GSSGGPPSAS
Zeama_SYT2      (131)     MVARPGMVNGMA------------QSMQAQLP------------P-GVN--
Orysa_SYT2      (130)     MVMRPGVVNGIP-------------QLLQGEMHRG-------------AD-
Soltu_SYT2      (147)     LGMSSS--GGGS-------SSGLHMLQSEN---T-------HSASTGGGG
Medtr_SYT2      (144)     MGLRPGGINNGM------------HPMHNEAALGG------SGSGGQMTGV
Sorbi_SYT3      (141)     MVMRPATINGMQQ--PMQADPAR-AAELQQPASV------PADGRVSK--
Zeama_SYT3      (144)     MVMRPGTINGMQQQQPMQADPARAAAELQQAAPI------PADGRGSK--
Bradi_SYT3      (136)     MVMRPGTVNGMQP--------MQADLQAAAAAPG------LADSRGSKQ-
Triae_SYT3      (136)     VVMRPGTVNGMQ------------QPMQAAGDLQP------AAAPGGSKQ-
Sacof_SYT3      (139)     MVMRPATINGIQQ--PMQADPAR-AAELQQPPPI------PADGRVSKQ-
Panvi_SYT3      (135)     MVMRP-TINGMQP---MQADPAAAAASLQQSAPG------PTDGRGGK--
Orysa_SYT3      (137)     MLMRPGTVNGMQS--IPVADPAR-AADLQTAAPG------SVDGRGNE--
Arath_SYT3      (153)     MGIRPMGLNNNN------------GLQHQMHHHET------ALAANNA---
Consensus       (201)     MGMRPG  NG               ML  E    G              G
```

**FIGURE 7 (continued)**

**Met-rich / QG-rich domain (continued)**

```
                      251                                              300
Brana_SYT1   (174)  KPEMGSG------------------------------------EGRGGSS
Aqufo_SYT1   (159)  YSDYGRG----SGGG---------VTIASKQDGGS-----GSGEGRGGNS
Picsi_SYT1   (178)  FPEFGRGSATSAEGMQANRGFTIDRGSNKQDGVGSENAHPGAGDGRGSST
Pinta_SYT1   (174)  FPEFGRGSATSADGMQVNRGFAIDRGSNKQDGVGSENAHAGAGDGRGSST
Poptr_SYT1   (167)  FPDFGMD----ASS-------RGIASGSKQDIRSA-----GSSEGRGGSS
Vitvi_SYT1   (166)  FPDFSRG----TSGEGLQAAGRGMAGGSK---QDM-----GNAEGRGGNS
Soltu_SYT1   (175)  SHDFGR--------------------ANKQDIGSS-----MSAEGRGGSS
Lyces_SYT1   (172)  SHDFGR--------------------ANKQDIGSS-----MSAEGRGGSS
Goshi_SYT1   (169)  FPDFGRG----SSGEGIHGG-RPMAGGSKQDIGSA-----GSAEGRGGSS
Zeama_SYT1   (181)  FSGFGRS----GSG--------------------AKEG--STSLSVDVRG
Medtr_SYT1   (161)  FPDFGRS----SAGDGLHG-----SGKQ-----DI-----GSTDGRGGSS
Citsi_SYT1   (166)  FPDFGRG----SSGEGLHS----RGMGSKHDIGSS-----GSAEGRGGSS
Arath_SYT1   (178)  KPEMGSG----GGG-----------------------------EGRGGSS
Aspof_SYT1   (176)  FGDFGRS----SGG-------------------------------KQETG
Orysa_SYT1   (182)  FSDFGRG---GGGG--------------------GKEG--STSLSVDVRG
Sacof_SYT1   (179)  FSGFGRS----GSG--------------------AKEG--STSLSVDVRG
Allce_SYT2   (169)  --NAAYE------NTA------------------------ANTDGSIQKK
Lacse_SYT2   (164)  ------G------GAA------------------------ADGGKDGHAG
Horvu_SYT2   (153)  --AYAAG------GAS------------------------SEPSGTESHR
Brana_SYT2   (189)  AANVGLR------GGK-------------------------------QDG
Sacof_SYT2   (158)  --KQDAG------GVA------------------------SEPSGIENHR
Triae_SYT2   (152)  --AYAAG------GAS------------------------SEPSGTESHR
Maldo_SYT2   (169)  DPNDGRG------GSK------------------------QDASESGAGG
Goshi_SYT2   (176)  GPSDGRA------GNK------------------------QEGSEAGGN-
Glyso_SYT2   (171)  GPNDARG------GSK------------------------QDASEAGTAG
Glyma_SYT2   (174)  GPNDARG------GSK------------------------QDASEAGTAG
Eupes_SYT2   (166)  ---DGRG------GNK------------------------QDAPETGASG
Arath_SYT2   (175)  --NVGLR------GGK-------------------------------QDG
Citsi_SYT2   (173)  GPGDIRG------GNK------------------------QDASEAGTTG
Zeama_SYT2   (155)  --KQDAG------GVA------------------------SEPSGTESHR
Orysa_SYT2   (154)  --HQNAG------GAT------------------------SEPS--ESHR
Soltu_SYT2   (178)  FPDFGRG----LGS------------GNKHEMGSS-----MSDQGRGGSS
Medtr_SYT2   (177)  VVEQAR------------------------------------CFGAGTAG
Sorbi_SYT3   (180)  --QDTAA------GVS------------------------SEPSANESHK
Zeama_SYT3   (186)  --QDTAG------GAS------------------------SEPSANESHK
Bradi_SYT3   (171)  --DAAVA------GAI------------------------SEPSGTESHK
Triae_SYT3   (168)  --DAAVA------GAS------------------------SEPSGTKSHK
Sacof_SYT3   (179)  --QDTTA------GVS------------------------SEPSANESHK
Panvi_SYT3   (173)  --QDATA------GVS------------------------TEPSGTESHK
Orysa_SYT3   (176)  ------Q------DAT------------------------SEPSGTESHK
Arath_SYT3   (183)  GPNDASG------GGK------------------------PDGTNMSQSG
 Consensus   (251)      GRG      G                              G G
```

**FIGURE 7 (continued)**

**Met-rich / QG-rich domain (continued)**

```
                      301                    324
Brana_SYT1    (188)   G-----DGGETLYLKS--SDDGN-
Aqufo_SYT1    (191)   GGQS-ADGGESLYLKN--SDEGN-
Picsi_SYT1    (228)   GGQN-ADESEPSYLKA--SEE---
Pinta_SYT1    (224)   GGQN-ADESEPSYLKA--SEEEGN
Poptr_SYT1    (201)   GGQGG-DGGETLYLKS--ADDGN-
Vitvi_SYT1    (204)   GGQGG-DGGETLYLKA--AEDGN-
Soltu_SYT1    (200)   ---GG-DGGENLYLKA--SED---
Lyces_SYT1    (197)   ---G---G-ENLYLKA--SED---
Goshi_SYT1    (209)   GGQGGGDGGETLYLKA--ADDGN-
Zeama_SYT1    (205)   GTSSGAQSGDGEYLKVGTEEEGS-
Medtr_SYT1    (192)   SGHSG-DGGETLYLKS--SGDGN-
Citsi_SYT1    (203)   GSQ---DGGETLYLKG--ADDGN-
Arath_SYT1    (195)   G-----DGGETLYLKS--SDDGN-
Aspof_SYT1    (191)   --SEGHGTETPMYLKG-SEEEGN-
Orysa_SYT1    (207)   -ANSGAQSGDGEYLKG-TEEEGS-
Sacof_SYT1    (203)   GTSSGAQSGDGEYLKAGTEEEGS-
Allce_SYT2    (187)   TANDDLDPSAANPRRSEDAKSS--
Lacse_SYT2    (178)   G----------------GPEEAK-
Horvu_SYT2    (171)   S------TGADNDGGSGLADQS--
Brana_SYT2    (202)   ADGQG-------------KDDGK-
Sacof_SYT2    (176)   S------TGGDNDGGSD-------
Triae_SYT2    (170)   S------TGADNDGGSGWADQS--
Maldo_SYT2    (189)   -DGQG--TSAGGRGTG-DGEDGK-
Goshi_SYT2    (195)   --GQG--STTGGHGGGDGADEAK-
Glyso_SYT2    (191)   GDGQG--SSAAAHNSGDG-EEAK-
Glyma_SYT2    (194)   GDGQG--SSAAAHNSGDG-EEAK-
Eupes_SYT2    (183)   GDGQG-------NSGGDGAEDGK-
Arath_SYT2    (186)   ADGQG-------------KDDGK-
Citsi_SYT2    (193)   ADGQG--SSAGGHGG--DGEEAK-
Zeama_SYT2    (173)   S------TGGD-DGGSD-------
Orysa_SYT2    (170)   S------TGTENDGGSDFGDQS--
Soltu_SYT2    (207)   SGHGG-DGGENLYLKS--SEDGN-
Medtr_SYT2    (191)   GDGQGT-SAAAAHNSGDASEEGK-
Sorbi_SYT3    (198)   TT-----TGADSEAGGDVAEKS--
Zeama_SYT3    (204)   SA-----TGADTEAGGDVAEKS--
Bradi_SYT3    (189)   S------TGADHEAGGDVAEQS--
Triae_SYT3    (186)   N------AGAEEVG-ADVAEQS--
Sacof_SYT3    (197)   TT-----TGADSEAGGDVAEKS--
Panvi_SYT3    (191)   ST-----TAADHDVGTDVAEKS--
Orysa_SYT3    (190)   S------AGADNDAGGDIAEKS--
Arath_SYT3    (203)   ADGQGG-S-AARHGGGDAKTEGK-
 Consensus    (301)          TG    Y  G  AEDG
```

**FIGURE 7 (continued)**

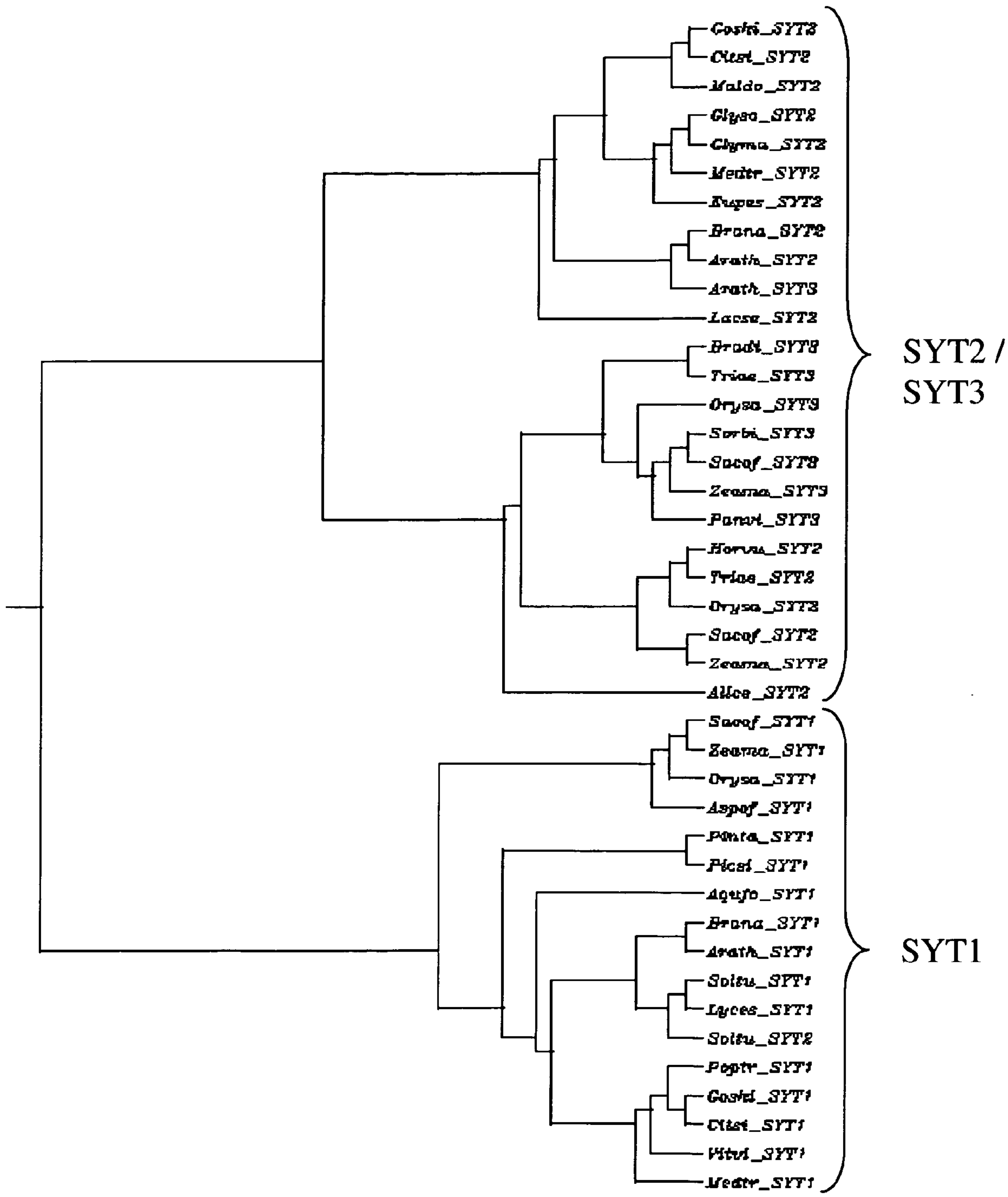

Goshi_SYT2
Citsi_SYT2
Maldo_SYT2
Glyso_SYT2
Glyma_SYT2
Medtr_SYT2
Eupes_SYT2
Brana_SYT2
Arath_SYT2
Arath_SYT3
Lacsa_SYT2
Bradi_SYT3
Triae_SYT3
Orysa_SYT3
Sorbi_SYT3
Sacof_SYT3
Zeama_SYT3
Panvi_SYT3
Horvu_SYT2
Triae_SYT2
Orysa_SYT2
Sacof_SYT2
Zeama_SYT2
Allce_SYT2
Sacof_SYT1
Zeama_SYT1
Orysa_SYT1
Aspof_SYT1
Pinta_SYT1
Picsi_SYT1
Aqufo_SYT1
Brana_SYT1
Arath_SYT1
Soltu_SYT1
Lyces_SYT1
Soltu_SYT2
Poptr_SYT1
Goshi_SYT1
Citsi_SYT1
Vitvi_SYT1
Medtr_SYT1
SYT2 / SYT3
SYT1


FIGURE 8

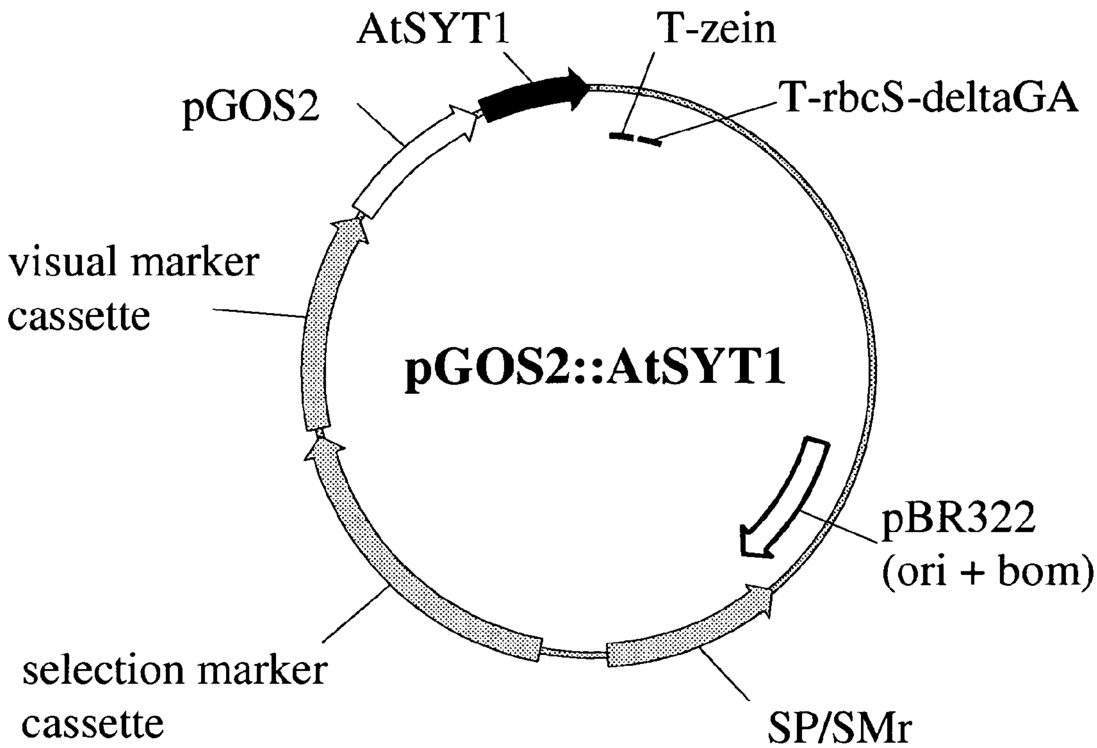
AtSYT1
T-zein
T-rbcS-deltaGA
pGOS2
visual marker cassette
pGOS2::AtSYT1
pBR322 (ori + bom)
selection marker cassette
SP/SMr

FIGURE 9

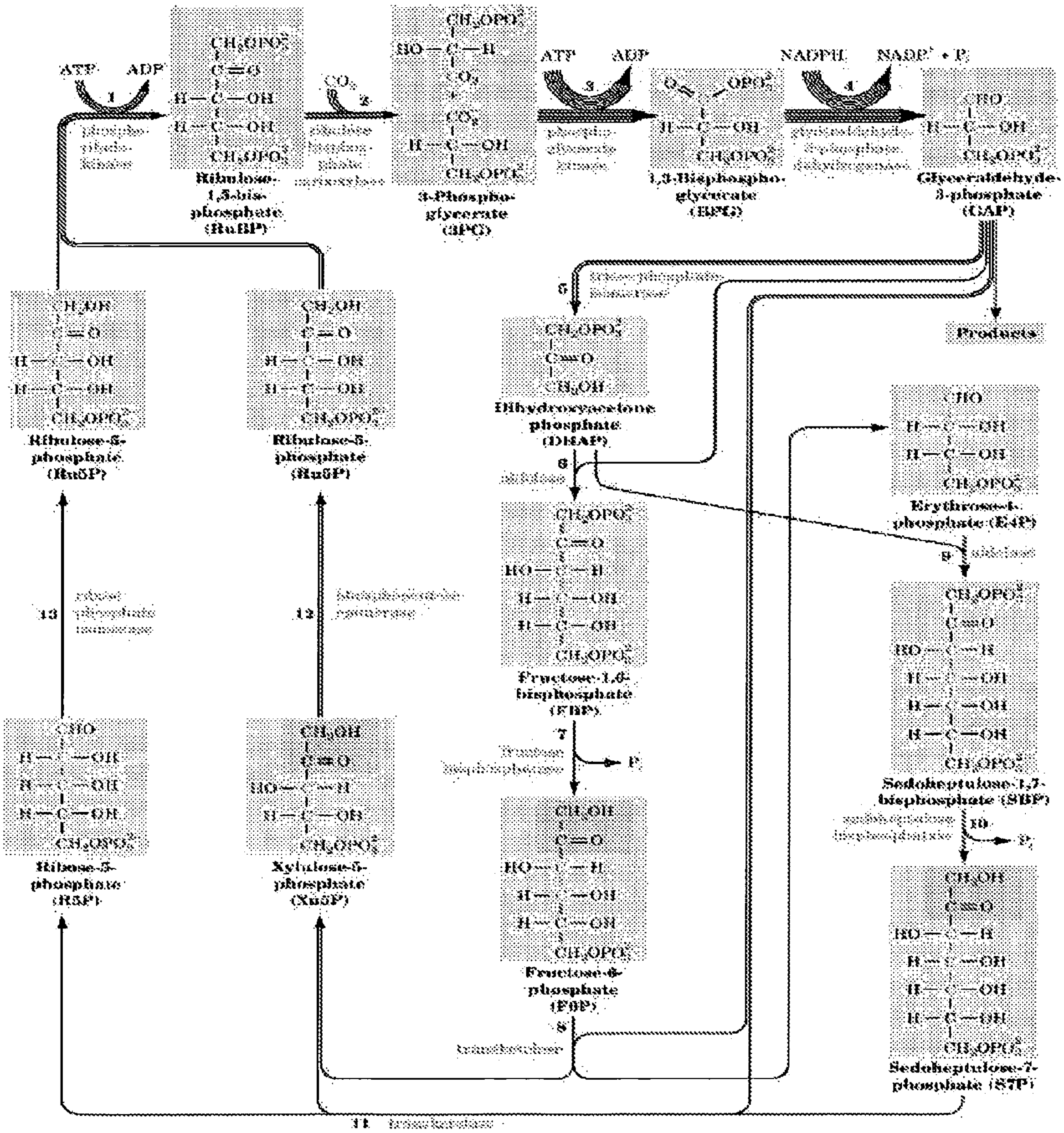
ATP
ADP
1
2
ATP
ADP
3
NADPH
NADP+ + Pi
4
Ribulose-1,5-bisphosphate (RuBP)
3-Phosphoglycerate (3PG)
1,3-Bisphosphoglycerate (BPG)
Glyceraldehyde-3-phosphate (GAP)
5
Products
Ribulose-5-phosphate (Ru5P)
Ribulose-5-phosphate (Ru5P)
Dihydroxyacetone phosphate (DHAP)
6
Erythrose-4-phosphate (E4P)
9
13
12
Fructose-1,6-bisphosphate (FBP)
7
Pi
Sedoheptulose-1,7-bisphosphate (SBP)
10
Pi
Ribose-5-phosphate (R5P)
Xylulose-5-phosphate (Xu5P)
Fructose-6-phosphate (F6P)
8
Sedoheptulose-7-phosphate (S7P)
11

# FIGURE 10

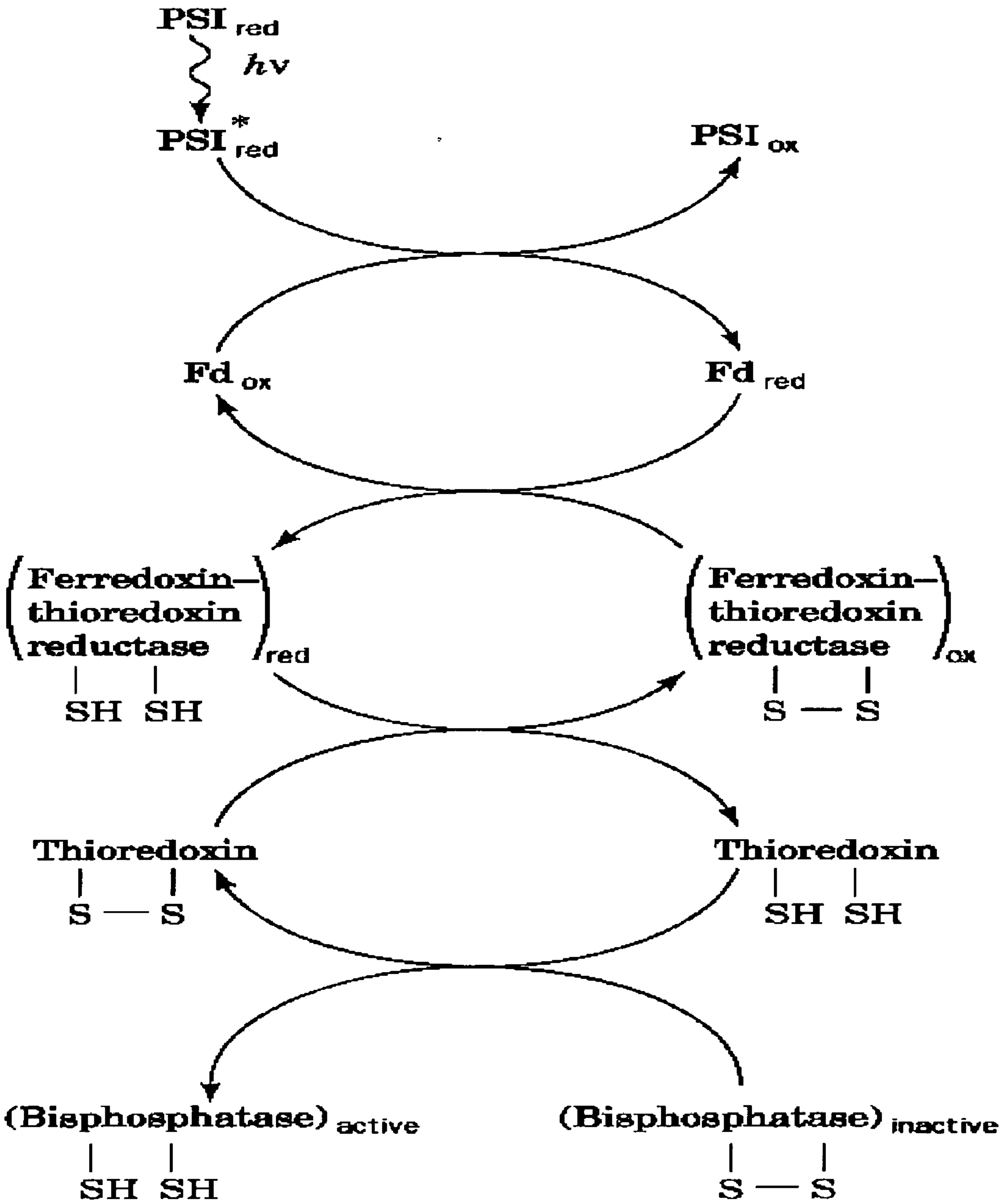

PSI red
hv
PSI* red
PSI ox
Fd ox
Fd red
(Ferredoxin–thioredoxin reductase) red
SH SH
(Ferredoxin–thioredoxin reductase) ox
S — S
Thioredoxin
S — S
Thioredoxin
SH SH
(Bisphosphatase) active
SH SH
(Bisphosphatase) inactive
S — S


FIGURE 11

```
                           1                                                                    70
Arath_cyFBPase         (1) ----------------------------------------------------------------------
Euggr_cyFBPase         (1) ----------------------------------------------------MSSTGPAAHTMPSPSFQP
Synec_FBPaseII         (1) ----------------------------------------------------------------------

Arath_cpFBPase         (1) ---MAASAATTTSSHLLLSSSRHVASSSQ--PSILSPRSLFSNNGKRAPTGVRNHQYASGVRCMAVAADA
Soltu_cpFBPase         (1) ------MAASAATATATTSYLSALDKKTP-FLFALDKK-TPFL-CPKSS--TKRRSFNGGVKCMAIETTS
Phypa_cpFBPase         (1) --MATTQAILSATLAIAPASSCETSSRSPASTKTCLSVAGSSLHGSVAGLGAGKQIVSVQRKSMAVRAAV
Orysa_cpFBPase         (1) ---MAAAATTSS--HLLLLSRQQAAASLQCGLSFRRQPGRLAG-----------GSSAPSVRCMAAVDTA
Bigna_cpFBPase         (1) MAAVLFRGGVLSSTMTAARTFAAPSVHTRGMHMSVKSS-----------------NPFSQAGRRAAVRSS
Chlre_cpFBPase         (1) ------MAATMLRSSTQSGIAAKAGRKEAVSVRAVAQPQRQAGAASVFS----SSSSGAAARRGVVAQAT
Phatr_cpFBPase         (1) --------MFILKSPALWLLLYPVVAFTAARANSIRPA-----------------AALSVFDLSSVEAVP
Cyame_cpFBPase         (1) ------------------MAKQAPHAFVSLGAPKLRST-----------------NIWGRVSVSVLPLET
Pissa_cpFBPase         (1) ---MVAMAAATASSQLIFSKPYSPSRLCPFQLCVFDAKSVLS------SSRRKHVN-GSGVRCMAVKEAT
Triae_cpFBPase         (1) ----MAAATTTTSRPLLLSRQQAAASSLQCRLPRRPGSSLFAGQ---------GQASTPNVRCMAVVDTA
Zeama_cpFBPase         (1) ---MAAAATTSSSSHLLLLSRQ-QAASLRCRLSFLGQPARRSGRV------TAQAPAAKDVRCMAAVDTA
Pontr_cpFBPase         (1) ----MVAAAATTSSQLLFSSSHSFSRLSPYQICVFDSKALVSS-CPSNVMKRRHVGVAAGVRCMAVGTTS
Brana_cpFBPase         (1) ---MAATAATTSSSHLLLSSSRHVAASSQ--PQILFQRSLFS--GGKRSAAGKNHHASGGVRCMAVAADA
Poptr_cpFBPase         (1) -MVAQAAAITTSSSHLLFSTSRSLSRPSPSQLCVFDSKTLVSY-PNSTSTYKKKRG-GDGLKCMAVSTAS
Nicta_cpFBPase         (1) ------MAASSATATATTSFLCALDKKTP-FLCTLDKKGTPFL-CPKGSSTTKRRSFNGGVKCMAIETTA
Lyces_cpFBPase         (1) -----------MAATATTSYLSALDKKTP-FLFALDKK-TPFL-CPKNS--TKRRSFNGGVKCMAIETAS
Ostta_cpFBPase         (1) -----MSACISASVTRARTARAPAARRAASK-------------------------ARASPRAVAAR---
Ostlu_cpFBPase         (1) -----MSAATSACARAIVATKKTTAARRAGKS------------------------ATRATPRSVAAR---
Galsu_cpFBPase partial (1) -------STRKDVQVYFIQVLLYLYKEECQEITTAKDI-----------------FLIRWISIKMIAVQP
Linus_cpFBPase partial (1) ------------------------------------EEEQRRRG-----------------FWFQMLCRRDA
Tagpa_cpFBPase partial (1) ----------------------------------------------------------------------
Marpo_cpFBPase partial (1) ----------------------------------------------------------------------

Consensus              (1)         A                                                   VR MAV
```

Predicted chloroplastic transit peptide

FIGURE 12

```
                                71                                                                      140
Arath_cyFBPase            (1)  ------MDHAADAHRTDLMTITRFVLNEQSK-YPESRGDFTILLSHIVLGCKFVCSAVNKA---GLAKLI
Euggr_cyFBPase           (21)  ASPLTPTVSSLNKAADSVTTSDFLTLQRYIM-LTSKDQELAVLLSALSLACKATARACNKA---GIALLF
Synec_FBPaseII            (1)  MAQSTTSETHTRDLDRDCTTLSRHVLEQLQS-FSPEAQDLAALMQRIGLAAKLIARRLSHAG--LVDDAL

Arath_cpFBPase           (66)  SETKT--AARK-KSGYELQTLTGWLLRQEMK--GEIDAELTIVMSSISLACKQIASLVQRA---GISNLT
Soltu_cpFBPase           (60)  GFTATK-----KRSGYELQTLISWLLRQEQA--GVIDAELTIVISSISMACKQIASLVQRA---GISNLT
Phypa_cpFBPase           (69)  AAETAAPKQQ-AKSQYDITTLTTWLLKKEQA--GVIDGELTIVLSSIALACKQIASLVQRA---GISNMT
Orysa_cpFBPase           (55)  SAPAATEAS--KKSSYEITTLTTWLLKQEQA--GTIDGEMTIVLASISTACKQIASLVQRA---PISNLT
Bigna_cpFBPase           (54)  VAPSPVQDSAGTIVDDGTVTLTRFMIEEAMKSKTPGQEDMVRLISSISVACKRIASMVQTAGISGSTGLA
Chlre_cpFBPase           (61)  AVATPAAKPAAKTSQYELFTLTTWLLKEEMK--GTIDGELVTVISSVSLACKQIASLVNRA---GISNLT
Phatr_cpFBPase           (46)  -SRKTKAPIFDEVCDTTGVTLKRFMTEVSLL--NPEIEELTTLFGAIETACKAIANLVKRS-PLPSSDTL
Cyame_cpFBPase           (36)  RHQRRFGAVRLRAALDLPMTLGQWVLQQERQ--HPETADLAPLIIATATAGKQIASLVAR---ACVSNLT
Pissa_cpFBPase           (61)  SETKKR-------SGYEIITLTSWLLQQEQK--GIIDAELTIVLSSISMACKQIASLVQRA---NISNLT
Triae_cpFBPase           (58)  SAPAPAAAR--KRSSYDMITLTTWLLKQEQE--GVIDNEMTIVLSSISTACKQIASLVQRA---PISNLT
Zeama_cpFBPase           (61)  ASAAAAETSP-KSSSYEIVTLTTWLLQQERT--GAIDNEMTIVLASISTACKQIAALVQRA---PISNLT
Pontr_cpFBPase           (66)  EVATKK-----R-SSYEIETLTNWLLKQEQS--GVIDAELTIVLSSISTACKQIASLVQRA---GISNLT
Brana_cpFBPase           (64)  SAEAKPAAARK-KSGYELQTLTGWLLRQEQK--GEIDTELTIVMSSIAMACKQIASLVQRA---GISNLT
Poptr_cpFBPase           (68)  DAKTKK-------STFEIQTLTGWLLKQEQA--GVIDAELTIVISSISMACKQIASLVQRA---SISNLT
Nicta_cpFBPase           (63)  GATETK-----KRSGYELQTLTSWLLRQEQA--GSIDAELTIVISSISMACKQIASLVQRA---GISNLT
Lyces_cpFBPase           (55)  GVTQTK-----KKSGYELQTLTSWLLRQEQA--GVIDAELTIVISSISMACKQIASLVQRA---GISNLT
Ostta_cpFBPase           (38)  -------------AGGVGTPYTTWILQQEMQ--ENIDGELAVVLSSIGLACKQIASLVQRA---GLAGMT
Ostlu_cpFBPase           (40)  -------------AGGAGTPFTTWILQQEME--EKIDGELAVVLSSIGLACKQIASLVQRA---GLQGMT
Galsu_cpFBPase partial   (47)  QPKKTPVAEYLQTSQDTPTSLTRYLLEVAKQ--NKDMSDMVALINGIQFACKKIASLVGKAGVTDLMGLY
Linus_cpFBPase partial   (20)  PSEAATTTK--KRSSYELLTLTTWLLQQEQA--GVIDAELTIVLSSISTACKQIASLVQRS---GISNLT
Tagpa_cpFBPase partial    (1)  ----------------------------------------------------------------------
Marpo_cpFBPase partial    (1)  ----------------------------------------------------------------------

Consensus                (71)               S YEI TLT WLL QE    G IDAELTIVLSSISLACKQIASLVQRA   GISNLT
```

**FIGURE 12 (continued)**

```
                                141                                                                    210
Arath_cyFBPase          (61)   GLAGETNIQGEEQKKLDVLSNDVFVNALVSSGRTSVLVSEEDEEATFVEPSKRG----------KYCVVF
Euggr_cyFBPase          (87)   GLAGETNATGEDQKKLDVLSNDLFINSLTNCGACAVLVSEENEEPIIVEPAHRG----------RFCVAF
Synec_FBPaseII          (68)   GFTGEINVQGEAVKKMDVYANQVFISVFRQSGLVCRLASEEMEKPYYIPENCPIG---------RYTLLY

Arath_cpFBPase          (128)  GVQGAINIQGEDQKKLDVISNEVFSNCLRSSGRTGIIASEEEDVPVAVEESYSG----------NYVVVF
Soltu_cpFBPase          (120)  GVQGAVNIQGEDQKKLDVVSNEVFSNCLRSSGRTGIIASEEEDVPVAVEESYSG----------NYIVVF
Phypa_cpFBPase          (133)  GLQGAANIQGEDQKKLDVISNEVFSSCLRSSGRTGIIASEEEDTPVAVEESYSG----------NYIVVF
Orysa_cpFBPase          (118)  GVQGAVNVQGEDQKKLDVVSNEVFSNCLKSSGRTGVIASEEEDVPVAVEESYSG----------NYIVVF
Bigna_cpFBPase          (124)  EGGGSVNVQGEEQKKLDVISNDVLKSALRPSGKLGVIASEEEDNPVVVDELYSG----------EYVATF
Chlre_cpFBPase          (126)  GVAGNQNVQGEDQKKLDVVSNEVFKNCLASCGRTGVIASEEEDQPVAVEEIYSG----------NYIVVF
Phatr_cpFBPase          (112)  GLQGEINVQGEDQKKLDVIANDLLKRALRFTGRLGVLASEEEDTPVDLMPRDPSTKKVLIDEGEKYVAVF
Cyame_cpFBPase          (101)  GLQGSVNVQGEEQKKLDVLTNEVLKNALRSTGKLGVLASEEENEPVSLMEEAYTG---------DEIAAF
Pissa_cpFBPase          (119)  GTQGAVNIQGEDQKKLDVISNEVFSNCLRSSGRTGIIASEEEDVAVAVEESYSG----------NYIVVF
Triae_cpFBPase          (121)  GVQGATNVQGEDQKKLDVISNEVFSNCLRWSGRTGVIASEEEDVPVAVEESYSG----------NYIVVF
Zeama_cpFBPase          (125)  GVQGAVNVQGEDQKKLDVVSNEVFSNCLKSSGRTGVIASEEEDVPVAVEQSYSG----------NYIVVF
Pontr_cpFBPase          (125)  GIQGAVNVQGEDQKKLDVVSNEVFSNCLRSSGRTGIIASEEEDVPVAVEESYSG----------NYIVVF
Brana_cpFBPase          (128)  GVQGAVNIQGEDQKKLDVVSNEVFSNCLRSSGRTGIIASEEEDVPVAVEESYSG----------NYIVVF
Poptr_cpFBPase          (126)  GVQGSVNVQGEDQKKLDVVSNEVFSSCLRSSGRTGIIASEEEDVPVAVEESYSG----------NYIVVF
Nicta_cpFBPase          (123)  GVQGAVNIQGEDQKKLDVVSNEVFSNCLRSSGRTGIIASEEEDVPVAVEESYSG----------NYIVVF
Lyces_cpFBPase          (115)  GVQGAVNIQGEDQKKLDVVSNEVFSNCLRSSGRTGIIASEEEDVPVAVEESYSG----------NYIVVF
Ostta_cpFBPase          (90)   GLAGEQNVQGEDQKKLDVISNDVFCNVLRQSGRTGVIASEEEDVPVAVEEIYGG----------NYVVVF
Ostlu_cpFBPase          (92)   GLAGETNVQGEDQKKLDVISNDVFCDVLRQTGRTGVIASEEEDVPVAVEETFGG----------NYVVVF
Galsu_cpFBPase partial  (115)  Q-QGIVNVHGEEQKKLDVLSNEVLKNALKYSGKMAVIASEEEDVPIMVEESYSG----------NYVVVF
Linus_cpFBPase partial  (83)   GVQGAVNVQGEDQKKLDVVSNEVFSNCLRSSGRTGIIASEEEDVPVAVEESYSG----------NYIVVF
Tagpa_cpFBPase partial  (1)    ------------------------------------IIASEEEDVPVAVEESYSG----------NYIVVF
Marpo_cpFBPase partial  (1)    ----------------------------------------EEDTPVAVEESYSG----------NYVVVF

Consensus               (141)  GVQGAVNVQGEDQKKLDVVSNEVFSNCLRSSGRTGVIASEEEDVPVAVEESYSG          NYIVVF
```

FIGURE 12 (continued)

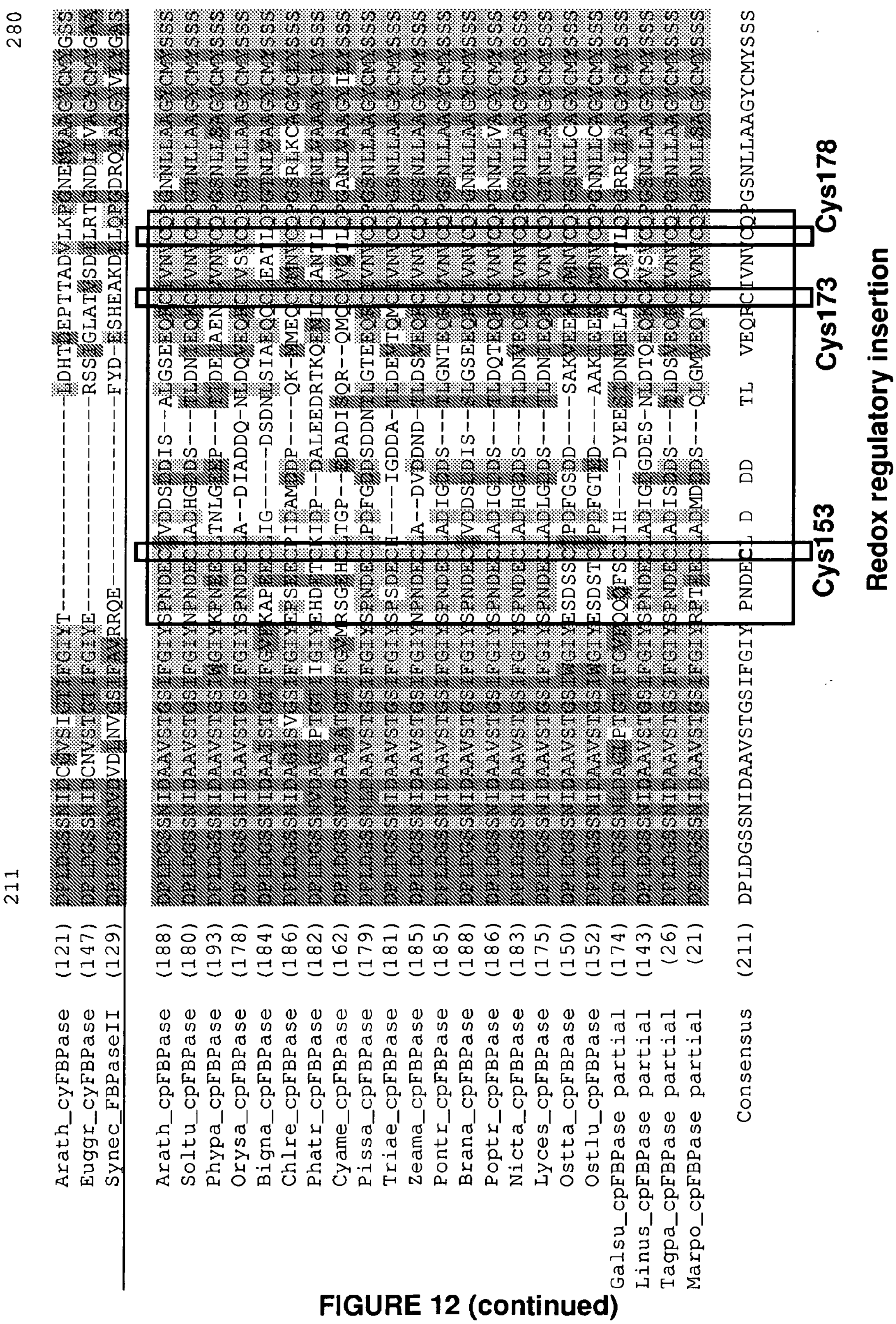


FIGURE 12 (continued)

```
                                   281                                                                  350
Arath_cyFBPase          (174) CMLVLSTG-TGVHGFTLDPSLGEFILTHPDIKIPNKGN--IYSVNEGNAQNWDGPTTKYVEKCKFPK--D
Euggr_cyFBPase          (200) TELVITFKNQGVHRFTLDPSLGEFIHTHPNVVFPEDGGKKIYSCNEGNSSNWDAAIKASVEYFKS-----
Synec_FBPaseII          (183) TILVYSMG-QGVHVFVLDPSLGEFVLAQSDIQLPNSGQ--IYSVNEGNEWQWPEGYRQYIREMHRR----

Arath_cpFBPase          (256) VIFVLTLG-KGVFSFTLDPMYGEFVLTQENIEIPKAGR--IYSFNEGNYQMWDDKLKKYIDDLKDPG-PT
Soltu_cpFBPase          (247) VIFVLTLG-NGVFSFNLDPMYGEFVLTQENVQIPKSGK--IYSFNEGNYQLWDDKLKKYIDDLKDPG-PS
Phypa_cpFBPase          (260) VILVLSVG-DGVYGFTLDPLYGEFVLSHENIQIPKSGK--IYSMNEGNYALWDDNLKKYVDSLKDPG-TS
Orysa_cpFBPase          (245) VIFVLTIG-TGVYVFTLDPMYGEFVLTQEKVQIPKAGK--IYAFNEGNYALWDDKLKSYMDSLKEPG-PS
Bigna_cpFBPase          (249) TILVLTTG-DGLNGFTLDPSIGEFILTHPNIQIPSRGK--IYSMNEANYFDWDPKLQTYVDNLKKAEGQT
Chlre_cpFBPase          (251) TIMVLTIG-NGVFGFTLDPLVGEFVLTHPNVQIPEVGK--IYPFNEGNYGLWDDSVKAYMDSLKDPKKWD
Phatr_cpFBPase          (250) TFLVLTLG-AGTVGFTLDETIGEFVLSHPNIKIPECSS--IMSFNEANTPSWDRPLQDTFAKWSTGTGKS
Cyame_cpFBPase          (228) VIFMLSIG-HGVQGFSLDPAIGEFVLTHPDVRVPERGK--IYSFNEANSDNWDPVLQEYIRSMKK---KG
Pissa_cpFBPase          (249) VAFVLTIG-KGVFVFTLDPLYGEFVLTQENLQIPKSGE--IYSFNEGNYKLWDENLKKYIDDLKEPG-PS
Triae_cpFBPase          (246) VIFVLTIG-TGVYVFTLDPMYGEFVLTQEKVQIPKSGK--IYSFNEGNYALWDDKLKKYMDSLKEPG-TS
Zeama_cpFBPase          (252) VIFVLTVG-TGVYVFTLDPMYGEFVLTQEKVQIPKAGK--IYAFNEGNYALWDDKLKLYMDSLKEPG-DS
Pontr_cpFBPase          (252) VIFVITLG-NGVFAFTLDPMYGEFVLTQENIQIPKTGK--IYAFNEGNYQLWDDKLKKYIDDLKDPG-PS
Brana_cpFBPase          (256) VIFVLTLG-KGVFSFTLDPMYGEFVLTQENIEIPKAGK--IYSFNEGNYQMWDEKLKKYIDDLKDPG-PS
Poptr_cpFBPase          (253) VIFVLTIG-KGVFSFSLDPMYGEFVLTQENIQIPKAGK--IYSFNEGNYQLWDDKLKKYIDDLKDPG-PS
Nicta_cpFBPase          (250) VIFVVTLG-NGVFAFNLDPMYGEFVLTQENIQIPKSGK--IYSFNEGNYQLWDDKLKKYIDDLKDPG-PS
Lyces_cpFBPase          (242) VIFVLTLG-NGVFSFNLDPMYGEFVLTQENVQIPKSGK--IYSFNEGNYQLWDDKLKKYIDDLKDPG-PS
Ostta_cpFBPase          (216) TILVITIG-QGVFGFTLDPTVGEFIMSHENIKVPDSGK--IYSFNEGNYAMWSDGLKKYMDSLKTGGKDG
Ostlu_cpFBPase          (218) TILVLTLG-EGVYGFTLDPTVGEFIMSHPNIKVPESGK--IYSFNEGNYDMWTPGLKKYMDSLKTGGAEQ
Galsu_cpFBPase partial  (240) TMLVLSLG-NGLHCFTLDTEVGEFVLTRANIQIPQRGN--IYSFNESNFYQWDKGVQDYIERLKKGNNQT
Linus_cpFBPase partial  (212) VIFVLTIG-NGVFAFNLDPMYGELVLTQENIQIPKAGK--IYSFNEGDYQMWDEKLK------------
Tagpa_cpFBPase partial   (93) VIFVLSIG-TGVYAFTLDPMYGEFVLTQEKIQIPKSGK--IYSFNEGNYQLWDDKLKKYMDDLKDPG-PT
Marpo_cpFBPase partial   (88) VILVLSVG-DGVYGFTLDPLYGEFVMTHENIKIPKKGS--IYSFNEGNYALWDDKLKKYIDSLKDPE-PT

             Consensus  (281) VIFVLTIG GVFGFTLDPMYGEFVLTQENIQIPKSGK  IYSFNEGNY LWDDKLKKYID LKDPG  S
```

N237

FIGURE 12 (continued)

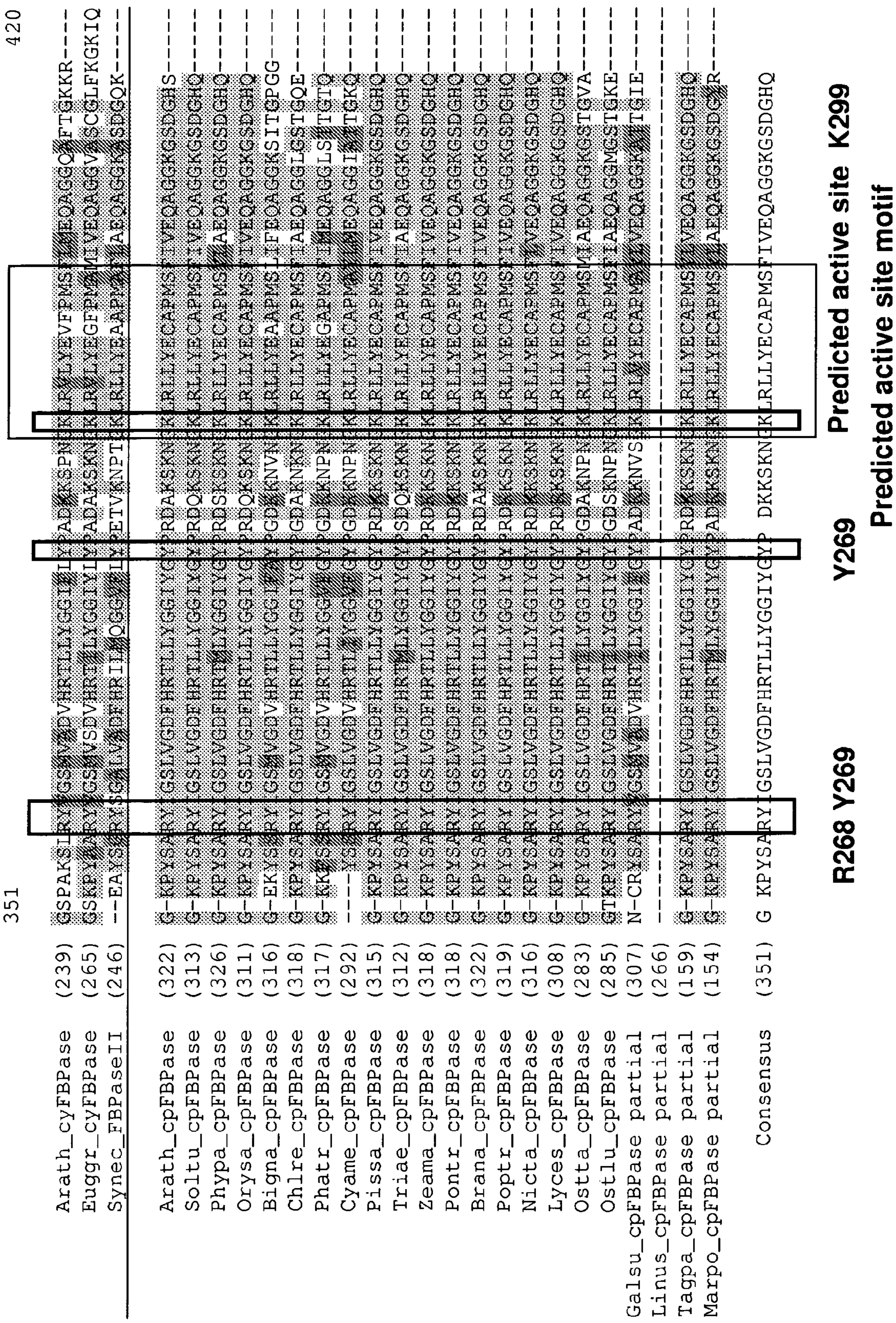


**FIGURE 12 (continued)**

```
                                     421                                               470
       Arath_cyFBPase       (305)   -ALDLVPEKIHERSPIFLGSYDDVEEIKALYAEEEKKN------------
       Euggr_cyFBPase       (335)   RILDLVPTSIHEKCPVILGGERDVQLVLNKYKELGLVE------------
       Synec_FBPaseII       (309)   PILLRQPQALHERCPLIIGSAADVDFVEACLAESVP--------------
_________________________________________________________________________________________

       Arath_cpFBPase       (386)   RVLDIQPTEIHQRVPLYIGSTEEVEKLEKYLA------------------
       Soltu_cpFBPase       (377)   RVLDIQPTEVHQRVPLYIGSTEEVEKLEKYLS------------------
       Phypa_cpFBPase       (390)   RILDIQPEQVHQRVPLYVGSVEEVEKLESFSS------------------
       Orysa_cpFBPase       (375)   RILDIMPTEIHQRVPLYIGSVEEVEKVEKFLA------------------
       Bigna_cpFBPase       (381)   RVLDLVPDKVHQRCPVFIGSPDDVDEVEKALA------------------
       Chlre_cpFBPase       (382)   RVLDVNPEKVHQRVPLFIGSKKEVEYLESFTKKH----------------
       Phatr_cpFBPase       (381)   RVMEISPDTVHQRVPIIMGSRQDVEEVMDAYKNFGIE-------------
       Cyame_cpFBPase       (353)   RILDIVPRDVHQREPFICGSPRDVEDLLKIYQGSMAMRG-----------
       Pissa_cpFBPase       (379)   RVLDIQPTEIHQRVPLYIGSTEEVEKVEKYLA------------------
       Triae_cpFBPase       (376)   RVLDIMPTAVHQRVPLYVGSVEEVEKVEKFLSSE----------------
       Zeama_cpFBPase       (382)   RILDITPTEIHQRVPLYIGSVEEVDKVEKFLA------------------
       Pontr_cpFBPase       (382)   RVLDIQPTEIHQRIPLKIGSQEEVEKLEKYLA------------------
       Brana_cpFBPase       (386)   RVLDIQPTEIHQRVPLYIGSKEEVEKLEKYLA------------------
       Poptr_cpFBPase       (383)   RVLDITPTEIHQRVPLYIGSVEEVEKLEKYLA------------------
       Nicta_cpFBPase       (380)   RVLDIQPTEIHQRVPLYIGSTEEVEKLEKYLS------------------
       Lyces_cpFBPase       (372)   RVLDIQPTEIHQRVPLYIGSTEEVEKLEKYLS------------------
       Ostta_cpFBPase       (347)   RVLDIVPEKVHQRVPFFVGSKNEVAYLESFM-------------------
       Ostlu_cpFBPase       (350)   RVLDVVPEKFHQRVPFFTGSKKEVQYLESFM-------------------
Galsu_cpFBPase partial      (371)   NILDLTPKDIHERKPLIIGSPADIEEFLQVYGMSRVDREDMHMWDNLSSL
Linus_cpFBPase partial      (266)   --------------------------------------------------
Tagpa_cpFBPase partial      (223)   RVLDIQPTEIHQRVPLYIGSVEEVEKLEKYLA------------------
Marpo_cpFBPase partial      (218)   RVLEIEPEQVHQRVPLFVG-------------------------------

            Consensus       (421)   RVLDI PTEIHQRVPLYIGS EEVEKLEKYLA
```

FIGURE 12 (continued)

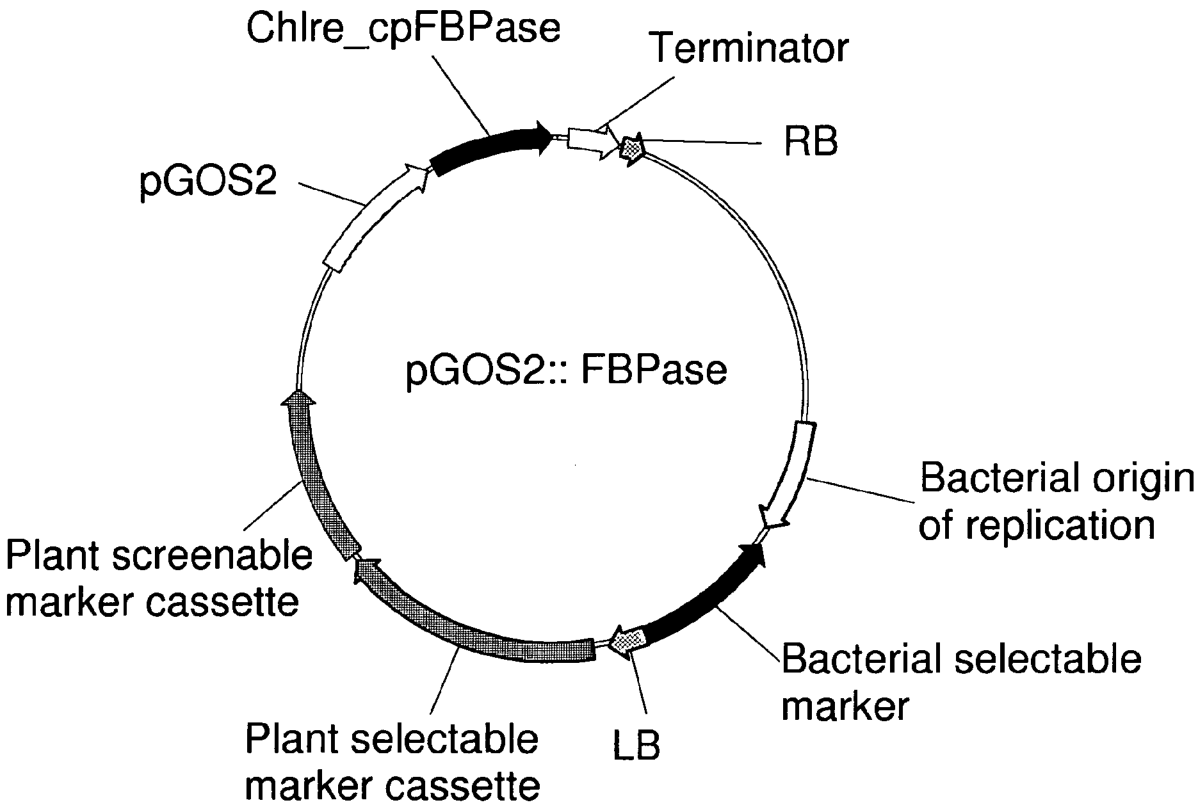
Chlre_cpFBPase
Terminator
RB
pGOS2
pGOS2:: FBPase
Bacterial origin
of replication
Plant screenable
marker cassette
Bacterial selectable
marker
Plant selectable
marker cassette
LB

FIGURE 13

```
                        1                                                50
Gm_TC229774     (1)     --------------------------------------------------
 Gs_TC30779     (1)     --------------------------------------------------
Hv_TC134680     (1)     --------------------------------------------------
Le_TC158378     (1)     --------------------------------------------------
Le_TC166426     (1)     --------------------------------------------------
 Ls_TC15801     (1)     --------------------------------------------------
 Mt_TC96175     (1)     ACGAGGCTATTCTCTCTCTTTCTCTCTCTACATTTCTCAACATTCTTCAA
OS_SEQ ID 1     (1)     --------------------------------------------------
Sb_TC103780     (1)     --------------------------------------------------
 So_TC67974     (1)     --------------------------------------------------
St_TC117743     (1)     --------------------------------------------------
Ta_TC257477     (1)     --------------------------------------------------
Zm_TC299056     (1)     --------------------------------------------------
  Consensus     (1)

                        51                                              100
Gm_TC229774     (1)     --------------------------------------------------
 Gs_TC30779     (1)     ------CTTTTTCCTCTCAAACCTCTCTCACACTGATAAACTTTTCTCTC
Hv_TC134680     (1)     --------------------------------------------------
Le_TC158378     (1)     --------------------------------------------------
Le_TC166426     (1)     --------------------------------------------------
 Ls_TC15801     (1)     --------------------------------------------------
 Mt_TC96175    (51)     ATTTCTCTCTCCAAAACCTTCACTTCGCAGCTTTTTCAATCTGCTTTCTT
OS_SEQ ID 1     (1)     --------------------------------------------------
Sb_TC103780     (1)     --------------------------------------------------
 So_TC67974     (1)     --------------------------------------------------
St_TC117743     (1)     --------------------------------------------------
Ta_TC257477     (1)     --------------------------------------------------
Zm_TC299056     (1)     --------------------------------------------------
  Consensus    (51)

                        101                                             150
Gm_TC229774     (1)     --------------------------------------------------
 Gs_TC30779    (45)     CATTACCTTTCTTTGACTTGCAAAGTTGTTCTCCTCAAACTAACCAAACA
Hv_TC134680     (1)     --------------------------------------------------
Le_TC158378     (1)     --------------------------------------------------
Le_TC166426     (1)     --------------------------------------------------
 Ls_TC15801     (1)     --------------------------------------------------
 Mt_TC96175   (101)     TGACTCTGTAACAGTTTTTTTCGTGGAAGAATCAGAACCACAAAAAAGTT
OS_SEQ ID 1     (1)     --------------------------------------------------
Sb_TC103780     (1)     ---------------------------------CTACTGGATTAACCCCC
 So_TC67974     (1)     --------------------------------------------------
St_TC117743     (1)     --------------------------------------------------
Ta_TC257477     (1)     --------------------------------------------------
Zm_TC299056     (1)     --------------------------------------------------
  Consensus   (101)
```

**FIGURE 14**

```
                      151                                               200
Gm_TC229774     (1)   --------------------------------------------------
  Gs_TC30779   (95)   AGGGATTCTGGGTTTTCAGCTTTCTCTGTTTTCCTACTCTCTTTCGCCTT
Hv_TC134680     (1)   --------------------------------------------------
Le_TC158378     (1)   --------------------------------------------------
Le_TC166426     (1)   --------------------------------------------------
  Ls_TC15801    (1)   --------------------------------------------------
  Mt_TC96175  (151)   TCGATTTTTACCCATTTCTTCAACCCGTGATCGCTTCACTGTAATTGGCA
OS_SEQ ID 1     (1)   --------------------------------------------------
Sb_TC103780    (18)   CGCGTGCGTTCCCTCCCTCTGTCAGTCTGTCTCTCTCTTGGCGTCGACTG
  So_TC67974    (1)   --------------------------------------------------
St_TC117743     (1)   -------------TTCTTAGCTCATCGAAACGTTAAACCTTACAATACTT
Ta_TC257477     (1)   --------------------------------------------------
Zm_TC299056     (1)   ---------------------------CTCCCCCGGCGTGCGCTCCCTCC
   Consensus  (151)

                      201                                               250
Gm_TC229774     (1)   --------------------------------------------------
  Gs_TC30779  (145)   TGCATGACCTGCTTACATAGAAACATAAAAGTCCGAAATACAATTTAGGT
Hv_TC134680     (1)   --------------------------------------------------
Le_TC158378     (1)   --------------------------------------------------
Le_TC166426     (1)   --------------------------------------------------
  Ls_TC15801    (1)   --------------------------------------------------
  Mt_TC96175  (201)   TGAGCTTCCATTTCTCGT-TCTGACAAAAACAGGTATCTATAACATCCTA
OS_SEQ ID 1     (1)   --------------------------------------------------
Sb_TC103780    (68)   GGCGACCGTCGCAGCCGCCCTAAGCCAGGTACCCAGACCATCTTCGGGCC
  So_TC67974    (1)   --------------------------------------------------
St_TC117743    (38)   CAAACATCGATCAATACAATTTGCTGAAGCACGCAATTTTTCAAGGACTG
Ta_TC257477     (1)   --------------------------------------------------
Zm_TC299056    (24)   TGTCTCTCTTGGCGACGACTGGGCAACGGTCGCAGCTGTATGTTCGAGCC
   Consensus  (201)

                      251                                               300
Gm_TC229774     (1)   --------------------------------------------------
  Gs_TC30779  (195)   GGTTTGTATATAAGATGGGGTGTTTTACAGTTTTGAGAAGCAACAAGAAA
Hv_TC134680     (1)   --------------------------------------------------
Le_TC158378     (1)   --------------------------------------------------
Le_TC166426     (1)   --------------------------------------------------
  Ls_TC15801    (1)   --------------------------------------------------
  Mt_TC96175  (250)   GTTCTGTACATAGAATGGGGTGTTTTACTATATTGAAGAGAAAGAAGAAA
OS_SEQ ID 1     (1)   --------------------------------------------------
Sb_TC103780   (118)   CTTCGCCGGCGACGAGCACCACCAGGAATTTTCCCAGCTGTAGCAATGAT
  So_TC67974    (1)   --------------------------------------------------
St_TC117743    (88)   TATAAACAACAAAGATGGGTTGTTTCACAGTTTTAAAAAGTAAGAAGAAG
Ta_TC257477     (1)   --------------------------------------------------
Zm_TC299056    (74)   CTTCGCCGACGACGTTCACCACCAGGAATTTTCTCAGCTGTGCCAATGAT
   Consensus  (251)
```

**FIGURE 14 (continued)**

```
                        301                                              350
Gm_TC229774      (1)    --------------------------------------------------
  Gs_TC30779   (245)    AAGTCCAAACAGTCAGTTTTGTTAAACACAT-TGCTCATAAAGAGCATA
Hv_TC134680      (1)    --------------------------------------------------
Le_TC158378      (1)    --------------------------------------------------
Le_TC166426      (1)    --------------------------------------------------
  Ls_TC15801     (1)    --------------------------------------------------
  Mt_TC96175   (300)    AAGCCTGATCAGATTGTGTATGTAAAACGCGT-AAGCCCTGGCGAAGATT
OS_SEQ ID 1      (1)    ----------ATGATGGGTTGCTTCACTGTCCTGAGATCCAAGAAGAAGA
Sb_TC103780    (168)    GGGTTGCTTCACTGTTCTCAGATCCAAGAAGAAGAAAATCCCTTTTGATA
  So_TC67974     (1)    --------------------------------------------------
St_TC117743    (138)    AAGTCTGAACAGAGTATTCACATCAAACGTGT-GAATCCTCAGGAACATT
Ta_TC257477      (1)    --------------------------------------------------
Zm_TC299056    (124)    GGGTTGCTTCACTGTTCTCAGATCCAAGAAGAAGAAAAGCCATTTTGATA
   Consensus   (301)

                        351                                              400
Gm_TC229774      (1)    --------------------------------------------------
  Gs_TC30779   (294)    TTCCTACCATGCTGCCTGAGCCCCAAATTCAGACACGATCACTGCAATCT
Hv_TC134680      (1)    --------------------------------------------------
Le_TC158378      (1)    --------------------------------------------------
Le_TC166426      (1)    --------------------------------------------------
  Ls_TC15801     (1)    --------------------------------------------------
  Mt_TC96175   (349)    CACCTACAGTACTGCCCGAACCCCAAACTCATACCCGCTCACTCCAGTCT
OS_SEQ ID 1     (41)    AGCCTCTTGCTCTCACCAAGAAATCGGTTGATGCAAGGGAAAGCACGTCC
Sb_TC103780    (218)    ATCCTCTTCTTCCAAGCAAGAAATCGGTTGATGCAAGGGAAAGTACGTCG
  So_TC67974     (1)    --------------------------------------------------
St_TC117743    (187)    CTCCTACTGCATTGCCCGAGCCTCAAGTGCAGACACGGTCGTTGCAGTCG
Ta_TC257477      (1)    --------------------------------------------------
Zm_TC299056    (174)    ATCCTCTTGTCCCAAGCAAGAAATCAGTTGATGCAAGGGAAAGTACGTCC
   Consensus   (351)

                        401                                              450
Gm_TC229774      (1)    --------------------------------------------------
  Gs_TC30779   (344)    GCA--CCCCCAAGTTTTATAACCAGAGTAAAACCAATTCAATCTAATAAC
Hv_TC134680      (1)    --------------------------------------------------
Le_TC158378      (1)    --------------------------------------------------
Le_TC166426      (1)    --------------------------------------------------
  Ls_TC15801     (1)    --------------------------------------------------
  Mt_TC96175   (399)    GCG--CCTCCTAGTTTTAAGATCAGAGTGAAACCCATTCAACCTAGCAAT
OS_SEQ ID 1     (91)    TCAAGACTCCCAGAGCCAGAAGCGCATGTGCCATCGTTACAATCTGCTC-
Sb_TC103780    (268)    TCTAGACTTCCGGAACCAGAAGTTCATGTGCCATCTTTGCAATCAGCTCT
  So_TC67974     (1)    --------------------------------------------------
St_TC117743    (237)    GCA--CCTCCAAGTTTTAGAACTAGAGTAAAACCTGTACAGTCGAGTAAT
Ta_TC257477      (1)    --------------------------------------------------
Zm_TC299056    (224)    TCTAGACTTCCGGAGCCAGAAGTTCATGTGCCATCTTTGCAATCAGCTC-
   Consensus   (401)
```

**FIGURE 14 (continued)**

```
                          451                                              500
   Gm_TC229774      (1)   --------------------------------------------------
    Gs_TC30779    (392)   AAGGCAAGCTGCAATAGGACACGTGCATTATCTGCTCCGTCAAGTCTTGA
   Hv_TC134680      (1)   --------------------------------------------------
   Le_TC158378      (1)   --------------------------------------------------
   Le_TC166426      (1)   --------------------------------------------------
    Ls_TC15801      (1)   ------------------------------ACCGACTGATGGCCGCTAGT
    Mt_TC96175    (447)   AAAGCCACTAACAATAGAATACGAGCATTGTCTGCTCCATCGAGTCTTGA
   OS_SEQ ID 1    (140)   CTCCTAGTTTTAGGAACAAGGCTAAAATCCACCAATCGGAAAAGAAAGCT
   Sb_TC103780    (318)   CTCCTAGTTTTAGGAACAGGACTAAGATATCCCAGTCGTCAAATAAAGTT
    So_TC67974      (1)   --------------------------------------------------
   St_TC117743    (285)   AGAGTTACAAGCAGTAGGGCGCGGGCACTCTCTGCCCCATCTAGCCTGGA
   Ta_TC257477      (1)   --------------------------------------------------
   Zm_TC299056    (273)   CTCCTAGTTTTAGGAACAGGGTCAAGATATCCGAGTCATCAAATGAAGTT
     Consensus    (451)                                 C

                          501                                              550
   Gm_TC229774      (1)   --------------------------------------------------
    Gs_TC30779    (442)   TGCTGCTGAGCAAGATGACCTTGCGTCAGTTGAATTTGAAGAACAAGAAG
   Hv_TC134680      (1)   --------------------------------------------------
   Le_TC158378      (1)   --------------------------------------------------
   Le_TC166426      (1)   --------------------------------------------------
    Ls_TC15801     (21)   CGGGGAAGAACACGA------AGAATCCAA--AACCCGTGGCAGCGGCGG
    Mt_TC96175    (497)   TGATGCAGAACAAGATGCATTGGCCACCATTGAGT--ATGAAGAACAAGA
   OS_SEQ ID 1    (190)   TCTTACAG------CAGAGCGCGCGTGCTGTCTGCTCCTTCCAGCCTAAT
   Sb_TC103780    (368)   TCAAACAG------CAGAGCTCGCGTGTTGTCTGCTCCGTCAACCCTTAT
    So_TC67974      (1)   --------------------------------------------------
   St_TC117743    (335)   CTCAGCAGAACAAGATG---TAGCATCAAATGAATGTGAGGAACATGATG
   Ta_TC257477      (1)   --------------------------------------------------
   Zm_TC299056    (323)   TCAAACAGAAACAGCAGGGCTCGCGTGCTGTCTGCTCCATCAGCCCTTAT
     Consensus    (501)          G              G

                          551                                              600
   Gm_TC229774      (1)   --------------------------------------------------
    Gs_TC30779    (492)   AGT-TGAAGAGTCGTGTTGGTTTAG-TCAAGGAACAGAAGTCATCAAGTC
   Hv_TC134680      (1)   --------------------------------------------------
   Le_TC158378      (1)   --------------------------------------------------
   Le_TC166426      (1)   --------------------------------------------------
    Ls_TC15801     (63)   AGG-CG--GAGGCGGGTTGCCGCCTGTCCCGCAG--CCGCTG-CCGCTTC
    Mt_TC96175    (545)   AGGGTCAAAATACCGAACTGGATCATGGAAGGAGCAGCGTTCGCCTAGTC
   OS_SEQ ID 1    (234)   TGT-GGTTGATCAGGATGGTCTTCCATATGCCGAATTCGATGATCAAGAT
   Sb_TC103780    (412)   TGT-GGTTGATCAGTTTGGCTTTCCATATGCTGAATACCGAGATCAAGAC
    So_TC67974      (1)   --------------------------------------------------
   St_TC117743    (382)   AGT-TCAAGAGTCGTATTGGTTCAA-TCAAGGAGTACCAATCACCAAGTC
   Ta_TC257477      (1)   --------------------------------------------------
   Zm_TC299056    (373)   TGT-GGTTGATCAGTTTGGCTTTCCATATTCGGAATACCGAGATCAAGAT
     Consensus    (551)    G       A                                  C
```

**FIGURE 14 (continued)**

```
                             601                                              650
  Gm_TC229774      (1)   --------------------------------------------------
   Gs_TC30779    (540)   CACAGCCTCTTCCACTTCCATCTCCCCACAGTACTGCG-----C-TGAAG
  Hv_TC134680      (1)   --------------------------------------------------
  Le_TC158378      (1)   --------------------------------------------------
  Le_TC166426      (1)   --------------------------------------------------
   Ls_TC15801    (107)   CTGCAGCGCATTCACG-CCAACTTCGAAAACCATCGCGGAATGCTTTTAA
   Mt_TC96175    (595)   CACAACCATTACCGCTTCCATCTCCTAAGGGTGGTGGT---ACATTGAAG
  OS_SEQ ID 1    (283)   GACTCCAGGGGCAAGGG--AGGTTCTATAAAGGGCCACCGTTCTCTAAT
  Sb_TC103780    (461)   GACTCCAGAGATAAGGA--AGGTTCAACAAAGGGGCATCGCTTTTCAAAT
   So_TC67974      (1)   --------------------------------------------------
  St_TC117743    (430)   CTCAGCCTCTTCCTCTTCCATCTCCACAGAGTGCCGCTGCCACTCTCAAG
  Ta_TC257477      (1)   --------------------------------------------------
  Zm_TC299056    (422)   GACTCCAGGGATAAGGA--AGGTTTGACAAAGGGGCATCGCTTTTCAAAT
    Consensus    (601)        C                 A  T                           A

                             651                                              700
  Gm_TC229774      (1)   --------------------------------------------------
   Gs_TC30779    (584)   ACAATGGGAAGTTTTAAAGCAGGGAATGTTAGTGGCCCTCTTTTTGCTTC
  Hv_TC134680      (1)   --------------------------------------------------
  Le_TC158378      (1)   --------------------------------------------------
  Le_TC166426      (1)   --------------------------------------------------
   Ls_TC15801    (156)   AGATTGAGTGGCGGTGA-GTTGGGCATGCTGCCAGCCTCTTAA-TACTTC
   Mt_TC96175    (642)   ACTGTTGGAAGCTTTAAGTTGGGGATAGCTAGTAGTCCTTTATATGCTT-
  OS_SEQ ID 1    (331)   CCACTGCCCCTTCCTCTCCCATCACCAGAAGGAAAATCATTGAGGAACTT
  Sb_TC103780    (509)   CCTTTGCCCCTTCCTCTTCCTTCACCGGAAGGACATTCTTTTAGGAACTC
   So_TC67974      (1)   --------------------------------------------------
  St_TC117743    (480)   ACTATGGGAAGCTTTAAAGTTGGCAACGCCAGCGGTCCGTTAAATGCCTC
  Ta_TC257477      (1)   --------------------------------------------------
  Zm_TC299056    (470)   CCTTTGCCCCTTCCTCTTCCTTCACGCGAAGGACATTCTTTTAGGAACTC
    Consensus    (651)       T         T              G             T        T

                             701                                              750
  Gm_TC229774      (1)   --------------------------------------------------
   Gs_TC30779    (634)   G-GGACCA--TTACCCCTGCC-----------------------------
  Hv_TC134680      (1)   ------------------------------------------------TG
  Le_TC158378      (1)   --------------------------------------------------
  Le_TC166426      (1)   --------------------------------------------------
   Ls_TC15801    (204)   C-GGACCCT-CTGCCACTTCCGCCATCGGGAACCACCCATCTTCCGCCAA
   Mt_TC96175    (691)   --CTGGACCTTTGCCGCTTCC-----------------------------
  OS_SEQ ID 1    (381)   TGGCAGCTTCAAAGCCATCAATGCAAGTGGACCACTCGATGCTTCAGGCC
  Sb_TC103780    (559)   TGGTAGCTTCAAAGCTAGCAACGTAAGTGGGCCATTGGAGATGTCCAGCC
   So_TC67974      (1)   --------------------------------------------------
  St_TC117743    (530)   T-GGACCC--CTGCCACTGCC---------------------TCCTACAC
  Ta_TC257477      (1)   --------------------------------------------------
  Zm_TC299056    (520)   TGGTAGCTTCAAAGCCAGCAACGTAAGCGGGCCATTGGAGATGTCTGGCC
    Consensus    (701)                  C
```

**FIGURE 14 (continued)**

```
                   751                                              800
Gm_TC229774    (1) --------------------------------------------------
 Gs_TC30779  (652) -------------------TCCCTCTGGAACACTACGTAACTTCGCCT-A
Hv_TC134680    (3) CCGAATTCGGCACGAGAAAGAGGTCCGAGGGGCTTAAGAACTTCTCGT-A
Le_TC158378    (1) --------------------------------------------------
Le_TC166426    (1) --------------------------------------------------
 Ls_TC15801  (252) TGATTCCGGCATCTTTACCGACATCTGGAACACTTAAGAACTTCACATAT
 Mt_TC96175  (710) -------------------ACCAACTGGGTCACT-GAGAAACTTTTCTTA
OS_SEQ ID 1  (431) CTCTGCCACTTCCTCCAAAGAAGTGTGATGGGCTTAAGAATTTCTCCT-A
Sb_TC103780  (609) CTCTCCCATTGCCTCCAAAGAAATGTGATGGACTTAGAATCTTTTCTT-A
 So_TC67974    (1) --------------------------------------------------
St_TC117743  (556) TG---CCTTCAACATTGCCTTCTACTGGAGCACTCAGGAACTTCTCAT-T
Ta_TC257477    (1) --------------------------------------------------
Zm_TC299056  (570) CTCTCCCATTGCCTCTAAAGAAATGTGATGGACTTAGAATCTTCTCTT-A
  Consensus  (751)                          TG     CT    A  TT  C T

                   801                                              850
Gm_TC229774    (1) --------------------------------------------------
 Gs_TC30779  (682) CGAAGAAATTGCAGCTGCTTGCCATCAT-TTCTCTTC-TGATCGATGCAC
Hv_TC134680   (52) CGATGAGATTTCCACTGCTTGCCA--GTGGTTTTCTGGCGATCATCGTGT
Le_TC158378    (1) --------------------------------------------------
Le_TC166426    (1) --------------------------------------------------
 Ls_TC15801  (302) TGAAGAAATCGCAGCTGCTTGCCACAACATTTTCACCCTGACAGATGCGT
 Mt_TC96175  (740) TGATGAACTTGCTGCTGCTTGCCTCAATTTCTCTTCAGATCGATA--CAT
OS_SEQ ID 1  (480) TGAGGAACTTTCATCAGCTTGCCA--ATGGTTTTCTGGTGACCAGTGTGT
Sb_TC103780  (658) CGAGGAGGTTTTGTCTGCTTGCCA--ATGGTTTTCAAGTGATCAGTGTGT
 So_TC67974    (1) --------------------------------------------------
St_TC117743  (602) TGAAGAACTTGCTGCTGCCTGCCACCGC-TTCTCTCC-TGAACGGTGTAT
Ta_TC257477    (1) --------------------------------------------------
Zm_TC299056  (619) TGAGGAGATTTCATCTGCCTGCCA--ACGATTTTCTAGTGATCAGTGTGT
  Consensus  (801)  GA GA  TT C  CTGC TGCCA     T T       GA     G  T

                   851                                              900
Gm_TC229774    (1) --------------------------------------------------
 Gs_TC30779  (730) ATCTGAGGGTCTATCTTCTGTTATGTACAAGGCATCCTTCGGAGATGACA
Hv_TC134680  (100) CTCAGAAACTCTGACTTCGACATCATACAAGGCATTGTTCAGGGATGATT
Le_TC158378    (1) --------------------------------------------------
Le_TC166426    (1) --------------------------------------------------
 Ls_TC15801  (352) GTCTGAAGGTCTTTCTTCTGTTATGTATAGAGCTTCTTTTGGAGAAGATA
 Mt_TC96175  (788) GTCAGAATCTCTTTCATCCACCATGTATAAAGCTTCCTTTGGTGATGATA
OS_SEQ ID 1  (528) TTCCGAAAGTTTGACATCAACATCATACAAGGCGTCCTTTAGGGATGATT
Sb_TC103780  (706) TTCAGAAGCACTTGGTTCAACATCATACAAGGCAACATTTAGGGATGAAT
 So_TC67974    (1) ---AGAAACACTTGGTTCAACATCATACAAGGCAACATTTAGGGATGAAT
St_TC117743  (650) GTCAGAAGGTCTCTCTTCTGTTATTTACAGAGCTTCTTTTGGAGATGATG
Ta_TC257477    (1) --------------------------------------------------
Zm_TC299056  (667) TTCAGAAACACTTGGTTCAACATCATACAAGGCAACATTTAGAGATGAAT
  Consensus  (851)  TC GAA   CT   TTC        TACAA GC  C TTT G GATGA
```

FIGURE 14 (continued)

```
                         901                                            950
Gm_TC229774      (1)   --------------------------------------------------
  Gs_TC30779   (780)   CA------TCAAGTTCAAAGAAGTTTGAAGCTACTGTTACTCGCCTTCAC
 Hv_TC134680   (150)   TC------GTTGAACCAAAGAAGATGGAAGCAATAGTAGCCAGGTTACTC
 Le_TC158378     (1)   --------------------------------------------------
 Le_TC166426     (1)   --------------------------------------------------
  Ls_TC15801   (402)   CT------TCTAATTTAAAGAATCTTCAAGCCACTGTTACCAGTCTCCAT
  Mt_TC96175   (838)   CCTCAACTTCAAGTTCAAAGAAGTTTGAAGCTACTGTCACACGCCTTCGC
 OS_SEQ ID 1   (578)   TT------ACCGACCCAAAGACCATTGAAGCAATAGTATCTCGGTTGCTC
 Sb_TC103780   (756)   TT------ATTGATACAAAGACCACTGAAGCAACGGTAGCTCGATTACTC
  So_TC67974    (48)   TT------ATTGATACAAAGACCACTGAAGCAACGGTAGCTCGATTACTC
 St_TC117743   (700)   CA------ACTGGTACAAAGAAGCTTGAAGCCACTGTAACCCGGCTTCAT
 Ta_TC257477     (1)   --------------------------------------------------
 Zm_TC299056   (717)   TT------ATTGATACGAGGACCACTGAAGCAACAGTAGCTCGATTACTC
   Consensus   (901)                T CAAAGA    TGAAGC AC GT  C CG  T C C

                         951                                           1000
Gm_TC229774      (1)   --------------------------------------------------
  Gs_TC30779   (824)   CCATCCACTCAGGGTTTAAGGGAATTTATAAACGAAGTAAACACTCTTGC
 Hv_TC134680   (194)   CCTTCCAATCAGAGTTTCAAAGAATTTAAGGCACAAGTAAATACGTTGGC
 Le_TC158378     (1)   --------------------------------------------------
 Le_TC166426     (1)   --------------------------------------------------
  Ls_TC15801   (446)   CCTTCAACTCAGGGTTTGAAGGAATTTGTGAGTGAAGTGAACACGCTAGC
  Mt_TC96175   (888)   CCATCATCTCAGGGCCTGAAGGAATTCATAAATGAGGTTAATACTCTTGC
 OS_SEQ ID 1   (622)   TCCTCCACTCAGAGTTTGAAAGAGTTTAAAACACAAGTGAATACCTTGGC
 Sb_TC103780   (800)   CCCTCCACTCAGAGTTTGAAGGAGTTTAAAACACAAGCAACTACATTGGC
  So_TC67974    (92)   CCCTCCACTCAGAGTTTGAAGGAGTTTAAAACACAAGCGACCACATTGGC
 St_TC117743   (744)   CCTTCTTCGCAGGGGTTGAAGGAATTTGTGACTGAGGTGAACACACTAGC
 Ta_TC257477     (1)   --------------------------------------------------
 Zm_TC299056   (761)   CCCTCCACTCAGAGTTTGAAGGAGTTTAAAACGCAAGCGACCACATTGGC
   Consensus   (951)   CC TC ACTCAG GTTTGAAGGA TTTA  A   AAG  A  AC  T GC

                         1001                                          1050
Gm_TC229774      (1)   --------------GTAAATTGCTAGGATTTC-AT------GCACGA--G
  Gs_TC30779   (874)   ATCATTGCAACATCCAAATCTCTGTAAATTGCTTGGGTACCATGCAC--G
 Hv_TC134680   (244)   ATCACTTCAACATCCCAATTTATGTAAACTCATCGGCTATCACGCAA--G
 Le_TC158378     (1)   --------------------------------------------------
 Le_TC166426     (1)   --------------------------------------------------
  Ls_TC15801   (496)   ATCATTGCAACACCCTTATCTCTGTAAATAGATTGGTTTCCATGCGC--G
  Mt_TC96175   (938)   ATCATTGCAGCATCCGAACCTCTGTAGATTGCTAGGATTTCACGCAG--G
 OS_SEQ ID 1   (672)   ATCACTTCAGCATCCCAACTTATGTAAACTAATCGGCTTTCACGCAA--G
 Sb_TC103780   (850)   ATTGCTTCAGCATCCCAATTTATGTAAACTGATTGGCTATTATGCAA--A
  So_TC67974   (142)   ATCACTTCAGCATCCCAATTTGTGTAAACTGATTGGCTATTATGCAA--A
 St_TC117743   (794)   TTCTTTGCAACATCCATCACTTTGTAAACTGATTGGTTTCCATGCCACGG
 Ta_TC257477     (1)   --------------------------------------------------
 Zm_TC299056   (811)   ATCGCTTCAGCATCCCAATTTATGTAAACTGATTGGCTATTATGCAA--A
   Consensus  (1001)   ATC  T CA CATCC AA  T TGTAAA TGAT GG T   A GCAA  G
```

## FIGURE 14 (continued)

```
                           1051                                              1100
Gm_TC229774       (28)  AGG-GTTC-AGAACATAGAATGTTGGTTTATGAAAGGCTATACCATGGAA
 Gs_TC30779      (922)  TGATAATTCAGAACAACGAATGTTGGTCTATGAGAGGTTATTTCATGGAA
Hv_TC134680      (292)  AGAAGAATCTAATGAAAGGATGTTGGTCTATGAGCGGCTCCATCATGGCA
Le_TC158378        (1)  -------------------------------------------CATGGAA
Le_TC166426        (1)  --------------------------------GAGAGGCTTTTTCATGGAA
  Ls_TC15801     (544)  TGAGGGATCTGATAGAAGAATGTTGGTTTACGAGAGGCTTTTTCATGGAA
  Mt_TC96175     (986)  TGATGGTTCAGAGCATAGAATGTTGGTTTATGAGAGGCTATACCATGGAA
OS_SEQ ID 1      (720)  AGAAGAATCTAATGAAAGGATGTTGGTCTATGAGCGACTCCATCATGGCA
Sb_TC103780      (898)  AGAAGATTCTAATGAAAGGATGTTGGTCTATGAACGGCTTCATCATGGGA
  So_TC67974     (190)  AGAAGATTCTAATGAAAGGATGTTGGTCTATGAACGGCTTCATCATGGGA
St_TC117743      (844)  GGAAGGTTCCGAGCACAGAATGTTGGTTTATGAAAGGCTTTTTCATGGAA
Ta_TC257477        (1)  --------------------------------------------------
Zm_TC299056      (859)  AGAAGATTCTAATGAAAGGATGTTGGTCTATGAACGGCTTTATCATGGAA
  Consensus     (1051)   GA G TTC  A  AAAG ATGTTGGT TATGA  GGCT TATCATGGAA

                           1101                                              1150
Gm_TC229774       (76)  GCTTGGATCGCTTATTGTATGGGAGATCTGATGGGCCATCAATTGATTGG
 Gs_TC30779      (972)  GCTTAGACCGGCTTTTATACGGGAGATCGGATGGACCGCCACTTGATTGG
Hv_TC134680      (342)  GTTTAGACAGGTTACTCTTTGGAAGACCGGAAGGTCGTTTCATGGACTGG
Le_TC158378        (8)  GCTTACACCGGCTTTTATTTGGGAGGTCGGATGGTCCCCCAATAGACTGG
Le_TC166426       (20)  GCTTAGACAGACTTTTGTTCGGAAGATCAGATGGCCCCTCTATAGATTGG
  Ls_TC15801     (594)  GCTTAGATCGGCTTTTATATGGTACAACAGATGGCCCACCTATTGATTGC
  Mt_TC96175    (1036)  GCTTGGACCGCTTATTGTATGGGAGATCTGATGGGCCATCAATTGATTGG
OS_SEQ ID 1      (770)  GCTTAGATAAACTACTCTTTGGAAGATCGGATGGTCGTTTCATGGACTGG
Sb_TC103780      (948)  GCTTAGATAAGCTACTCTTTGGAAGACCAGATGGTCGTTTCATGGACTGG
  So_TC67974     (240)  GCTTAGATAAGCTACTCTTTGGAAGACCAGATGGTCGTTTCATGGACTGG
St_TC117743      (894)  GCTTAGACCGGCTTTTGTTTGGGAGGTCCGATGGTCCCCCGATAGACTGG
Ta_TC257477        (1)  --------------------------------------------------
Zm_TC299056      (909)  GCTTAGATAAGCTACTCTTTGGAAGATCAGATGGTCGTTTCATGGACTGG
  Consensus     (1101)  GCTTAGA  GGCTATT TTTGG AGATC GATGGTCC TC AT GACTGG

                           1151                                              1200
Gm_TC229774      (126)  AATACAAGAATGAAAATTGCTATATGTGCTGCA-CAAGGTCTAAGCTTCT
 Gs_TC30779     (1022)  AATACTCGCATGAAAATTGCTTTATGTTCTGCACAGGGTCTT-ACTTTCT
Hv_TC134680      (392)  TCTACACGTTTGAAGGTTGCCCTTGGTGCTGCTAAAGGTCTA-GCTTTCC
Le_TC158378       (58)  AATGCTCGAATAAAAATTGCTTTATGTGCTGCTCAAGGTCTC-ACATTCC
Le_TC166426       (70)  AATGCGAGAACCAAAATTGCCCTATGTGCTGCACAAGGTCTT-ACATTTC
  Ls_TC15801     (644)  AATGCAAGAATGAAAGTCGCACTCTGTGCTGCTCAAGGGCTT-ACTTTTT
  Mt_TC96175    (1086)  AATACAAGAATGAAAATTGCAATATGTGCTGCACTAGGTCTT-TCTTTCT
OS_SEQ ID 1      (820)  TCAGCACGTTTGAAGGTTGCTCTTGGTGCTGCTAGAGGCCTG-GCTTTCC
Sb_TC103780      (998)  TCTAAACGTTTGAAGGTTGCCCTTGGTGCGGCCAGAGGTCTT-GCTTTCT
  So_TC67974     (290)  TCTAAACGTTTGAAGGTTTCCCTTGGTGCTGCCCGAGGTCTA-GCTTTCT
St_TC117743      (944)  AATGCTCGAACGAAAATTGCTTTATGTGCTGCTCAAGGTCTC-ACATTCC
Ta_TC257477        (1)  --------------------------------------------------
Zm_TC299056      (959)  TCTAAACGTTTGAAGGTTGCCCTGGGTGCTGCCAGAGGTCTA-GCTTTCT
  Consensus     (1151)  AATACACG ATGAAA TTGC CT TGTGCTGC C AGGTCT   CTTTCT
```

**FIGURE 14 (continued)**

```
                       1201                                              1250
Gm_TC229774     (175)  TGCACGAAGAGGGGCCTTTTCAGGCTATGTATAATGAGTTCTCAACAGCC
 Gs_TC30779    (1071)  TGCATGAGGAAGGACCATTTCAGGCAATGTACAATGAATTTTCGACTGCC
Hv_TC134680     (441)  TACACGATGAAGGACCCTTTCAGGCAATGTATGATGACTTCTCAACGTCA
Le_TC158378     (107)  TGCATGAGGAAGGACCTTTTCAGGCAATGTTCCATGAATTTTCCACTGCA
Le_TC166426     (119)  TACATGAGGAGGGACCTTTTCAGGCAATGTTCCAAGAATTTTCAACTGGA
 Ls_TC15801     (693)  TGCATGAGGAAGGACCATTTCAGGCGATGTTTCATGAATTTTCCACTGCT
 Mt_TC96175    (1135)  TGCATGAAGAAGGGCCTTTTCAGGCAATGTATAATGAATTTTCAACAGCT
OS_SEQ ID 1     (869)  TACATGATGAAGGGCCTTTTCAGGCCATGTACAATGACTTCTCAACCTCA
Sb_TC103780    (1047)  TGCATGATGAAGGACCCTTTCAGGCCATGTACAGCGAGTTCTCAACTTTG
 So_TC67974     (339)  TGCATGATGAAGGACCCTTTCAGGCCATGTACAGTGAGTTCTCAACTTTG
St_TC117743     (993)  TGCATGAGGAAGGACCTTTTCAGGCAATGTTCCATGAATTTTCCACTGCA
Ta_TC257477       (1)  --------------------------------------------------
Zm_TC299056    (1008)  TGCATGATGAAGGACCCTTTCAGGCCATGTACAGCGAGTTCTCAACTTTG
  Consensus    (1201)  TGCATGA GAAGGACC TTTCAGGC ATGTACAATGA TT TCAACTGC

                       1251                                              1300
Gm_TC229774     (225)  AACATACAGATTGACAAAGATTTCAGCGCAAAGCTTTCAGGATATGGTTG
 Gs_TC30779    (1121)  AACATACAGATCGACAAGGATTTCAGTGCAAAGCTCTCAGGATATGGGTG
Hv_TC134680     (491)  AACATCCAAATAGAGAAAGATTTCACTGCAAAGCTGTCGGGATATGGTTG
Le_TC158378     (157)  AATATACAAATTGATAAGGATTGCAGTGCAAAGCTCTCGGGATATGGATG
Le_TC166426     (169)  AATATACAAATCGACAAGGATTTTAGTGCAAAGCTCTCGGGGTATGGATG
 Ls_TC15801     (743)  AATATACAAATTGATAAAGATTTTAG------------------------
 Mt_TC96175    (1185)  AACATTCAGATTGACAAAGATTTCAGTGCAAAGCTTTCAGGATATGGTTG
OS_SEQ ID 1     (919)  AACATCCAAATTGACAAAGATTTCACTGCAAAGCTATCAGGATATGGATG
Sb_TC103780    (1097)  AACATCCAAATAGATAAAGATTTCACTGCTAAGCTTTCAGGATATGGTTG
 So_TC67974     (389)  AACATCCAAATAGATAAAGATTTCACTGCTAAGCTTTCAGGATATGGTTG
St_TC117743    (1043)  AATATACAAATTGATAAGGATTGCAGTGCAAAGCTCTCGGGATATGGATG
Ta_TC257477       (1)  --------------------------------------------------
Zm_TC299056    (1058)  AACATCCAAATAGATAAAGGTTTCACTGCTAAGCTTTCAGGATATGGTTG
  Consensus    (1251)  AACAT CAAAT GA AAAGATTTCAGTGCAAAGCT TCAGGATATGG TG

                       1301                                              1350
Gm_TC229774     (275)  TGTTGGACATATTCCCGAAGAAGAG---ATTTCAAGCAGCTCATCTG---
 Gs_TC30779    (1171)  CGTTGGTCATATCCCAGAGACAGAAGAGATCTCCAGTAATTCAGTTGCTG
Hv_TC134680     (541)  TGTTGGCTTCAATTCTGACGAG------GAAAGATCCAAGGCATCTG---
Le_TC158378     (207)  CATTACTCATATACAAGAGACAGAC---ATATCATGCAGTTCAGCTG---
Le_TC166426     (219)  CATTACGAATATACAAGAGACGGAG---ATATCTTGCAATTCAATCG---
 Ls_TC15801     (769)  --------------------------------------------------
 Mt_TC96175    (1235)  TGTTGGACATGTTCCGAAGGAAGAG---ATTTCAAGCAGTTCATCTG---
OS_SEQ ID 1     (969)  TGTTGGATTCAATACCGAGGAGGAA---ATATCAAATGCATCTGTGG---
Sb_TC103780    (1147)  TGTTGGCTTCAATACTGAGGAG------ATATCTAATGCACCTGCGT---
 So_TC67974     (439)  TGTTGGCTTCAATACTGAGGAG------ATATCTAATGCACCTGCGT---
St_TC117743    (1093)  CATTACTCCTATACCAGAGACAGAC---ATATCATGCAGTTCAGCTGCCC
Ta_TC257477       (1)  --------------------------------------------------
Zm_TC299056    (1108)  TGTTGGCTTCAATACCGAGGAG------ATATCTAATGCACCTGTGT---
  Consensus    (1301)  TGTTGG    A T C GAGGA GA    ATATC A  A  TCAGC G
```

# FIGURE 14 (continued)

```
                        1351                                              1400
  Gm_TC229774    (319)  --------------------------------------------------
   Gs_TC30779   (1221)  TGGCAAATATATCCGTAGAGACTCTGGAGAGAGGGCGACTAACTCCAAAG
  Hv_TC134680    (582)  --------------------------------------------------
  Le_TC158378    (251)  --------------------------------------------------
  Le_TC166426    (263)  --------------------------------------------------
   Ls_TC15801    (769)  --------------------------------------------------
   Mt_TC96175   (1279)  --------------------------------------------------
  OS_SEQ ID 1   (1013)  --------------------------------------------------
  Sb_TC103780   (1188)  --------------------------------------------------
   So_TC67974    (480)  --------------------------------------------------
  St_TC117743   (1140)  TGGCAATCTCT---------------------------------------
  Ta_TC257477      (1)  --------------------------------------------------
  Zm_TC299056   (1149)  --------------------------------------------------
    Consensus   (1351)

                        1401                                              1450
  Gm_TC229774    (319)  --------------------------------------------------
   Gs_TC30779   (1271)  AGCAATGTTTGGAGTTTTGGGATCATTCTACTTGAATTACTCACTGGCCG
  Hv_TC134680    (582)  --------------------------------------------------
  Le_TC158378    (251)  --------------------------------------------------
  Le_TC166426    (263)  --------------------------------------------------
   Ls_TC15801    (769)  --------------------------------------------------
   Mt_TC96175   (1279)  --------------------------------------------------
  OS_SEQ ID 1   (1013)  --------------------------------------------------
  Sb_TC103780   (1188)  --------------------------------------------------
   So_TC67974    (480)  --------------------------------------------------
  St_TC117743   (1151)  --------------------------------------------------
  Ta_TC257477      (1)  --------------------------------------------------
  Zm_TC299056   (1149)  --------------------------------------------------
    Consensus   (1401)

                        1451                                              1500
  Gm_TC229774    (319)  -----------------------------------------CTGTTGGA
   Gs_TC30779   (1321)  GAAGAACCTTGACAACCGTTATCCCAAGGAAGAGAGGAACCTAGTGAAGT
  Hv_TC134680    (582)  -----------------------------------------TGGCTGCA
  Le_TC158378    (251)  -----------------------------------------CCCTGGCA
  Le_TC166426    (263)  -----------------------------------------CCCTGGCG
   Ls_TC15801    (769)  --------------------------------------------------
   Mt_TC96175   (1279)  -----------------------------------------CCGTTGGA
  OS_SEQ ID 1   (1013)  -----------------------------------------CTGCTGCA
  Sb_TC103780   (1188)  -----------------------------------------CTGCAGCA
   So_TC67974    (480)  -----------------------------------------CTGCAGCA
  St_TC117743   (1151)  --------------------------------------------------
  Ta_TC257477      (1)  --------------------------------------------------
  Zm_TC299056   (1149)  -----------------------------------------CTGCAGCA
    Consensus   (1451)                                           C G  GCA
```

**FIGURE 14 (continued)**

```
                      1501                                              1550
Gm_TC229774     (327) AACCTATCAATGGAAACACTGGAGAAAGGAATGCTCACTCCAAAGAGCAA
 Gs_TC30779    (1371) GGAGCCGGCCATTCCTAGCCGACAATTGTAGATTGTCACTCATCATGGAT
Hv_TC134680     (590) AACCTCTCAGAGGAAACCTTGGAGAAAGGCGTACTGACTCCGAAGAGCAA
Le_TC158378     (259) AATCTCTCTGAGGAGACTCTGGAGCGAGGATTGGTAACTCCAAAGAGTAA
Le_TC166426     (271) AATCTCTCACAGGAGACACTGGAGAGAGGGTTGATAACTCCTAACAGCAA
  Ls_TC15801    (769) --------------------------------------------------
 Mt_TC96175    (1287) AACCTATCGATGGAGACACTGGAGAAAGGAATGCTTACTCCGAAAAGCAA
OS_SEQ ID 1    (1021) AACCTCTCAGTGGAAACCTTGGAGAAAGGTGTACTGACTCCCAAGAGCAA
Sb_TC103780    (1196) AACCTTTCGGTGGAGACCTTGGAGAAGGGTTTACTCACTCCCAAGAGCAA
 So_TC67974     (488) AACCTTTCGGTGGAGACCTTGGAGAAGGGTTTACTCACTCCCAAGAGCAA
St_TC117743    (1151) --------------------------------------------------
Ta_TC257477       (1) --------------------------------------------------
Zm_TC299056    (1157) AACCTTTCGGTGGAGACCTTGGAGAAGGGTTTACTCACTCCCAAGAGCAA
  Consensus    (1501) AACCT TC   GGA AC  TGGAGAA GG  T CT ACTCC AAGAGCAA

                      1551                                              1600
Gm_TC229774     (377) CGTATGGAGTTTTGGAATTTTTCTTCTGGAGCTACTTACTGGAAGAAAGA
 Gs_TC30779    (1421) CCTCAGCTCAAAGGTCGCTTTCCTATGAAAGCTGCCCGTACGGTGGCTGA
Hv_TC134680     (640) CGTATGGAGCTTTGGAGTTGTCTTGCTCGAGCTAATAACAGGACGGAAGA
Le_TC158378     (309) TGTTTGGAGTTTTGGGATTGTTCTTCTTGAGCTGCTGACTGGCCGGAAGA
Le_TC166426     (321) TGTTTGGAGTTTCGGGATTGTTCTTTTAGAACTGCTCACTGGCCGGAAGA
  Ls_TC15801    (769) --------------------------------------------------
 Mt_TC96175    (1337) TGTATGGAGTTTCGGAATTTTCCTTCTAGAGCTACTTACAGGAAGAAAGA
OS_SEQ ID 1    (1071) CGTATGGTGCTTTGGAGTTGTCCTGCTGGAGCTAATAACAGGAAGGAAGA
Sb_TC103780    (1246) TGTCTGGAGCTTTGGAGTTGTGTTACTGGAGCTAATAACTGGAAGGAAGA
 So_TC67974     (538) CGTCTGGAGCTTTGGAGTTGTGTTACTGGAGCTAATAACTGGAAGGAAGA
St_TC117743    (1151) --------------------------------------------------
Ta_TC257477       (1) --------------------------------------------------
Zm_TC299056    (1207) CGTCTGGAGCTTTGGAGTCGTGTTACTGGAGCTAATAACTGGAAGGAAGA
  Consensus    (1551)  GT TGGAG TTTGGA TTGT  T CT GAGCTA T AC GGA GGAAGA

                      1601                                              1650
Gm_TC229774     (427) ATCTTGATAGCC-GTCACCCCAAGGAAGAGAGGAATTTAGTCAAGTGGAG
 Gs_TC30779    (1471) CATTGCACAAAGGTGTCTCCAGATGGACCCATCAGAGAGGCCAACCATGA
Hv_TC134680     (690) ACCTCGATGTAC-GTTCCACCAAAGAAGAACGTAATATTGTCAAGTGGGG
Le_TC158378     (359) ATTTAAGCAGTC-GGCATCCAAAGGAAGAGAGGAATTTAGTGAAGTGGAG
Le_TC166426     (371) ATCTTGATGGTC-GGTATTCAAAGGAAGAGAGGAATCTAGTCAAGTGGAG
  Ls_TC15801    (769) --------------------------------------------------
 Mt_TC96175    (1387) ATCTCGATAGCCCGTCACCCAAAGGAAGAGAGAAATTTGGTGAAGTGGAG
OS_SEQ ID 1    (1121) ACCTTGATGTCC-GTTCCTCAAAAGAAGAACGCAATATTGTCAAGTGGAG
Sb_TC103780    (1296) ACCTTGATGCCA-ATTCTTCTAAAGAAGAACGCAATATTGTCAGGTGGAG
 So_TC67974     (588) ACCTTGATGCCA-ATTCTTCCAAAGAAGAACGCAATATTGTCAGGTGGAG
St_TC117743    (1151) --------------------------------------------------
Ta_TC257477       (1) --------------------------------------------------
Zm_TC299056    (1257) ACCTTGATCCCA-ATTATTCCAAAGAAGAACGCAATATTGTCAACTGGAG
  Consensus    (1601) A CT GAT       TT   C AA GAAGA   G AAT T GTCAAGTGGAG
```

# FIGURE 14 (continued)

```
                       1651                                              1700
Gm_TC229774    (476)   CCGGCCTTTCTTAGCCGATAACTACCGTCTGTCGTTGATCATGGATCCTC
 Gs_TC30779   (1521)   GAACCATCGTTGAGCATCTCAAAATCATCCAAGACCTGAAATACTCTTGT
Hv_TC134680    (739)   TAGGCCTTTCCTCACCGACGATAGTCGTCTATCCCTCATCATGGATCCCC
Le_TC158378    (408)   CAAGCTTTTTCTAGCTGATGACAGTAGATTATCGCTAATCATGGACCCTC
Le_TC166426    (420)   TAGGCCTTTCCTTGCTGATGATGGTAGATTATCGCTTATCATGGATCCTC
  Ls_TC15801   (769)   --------------------------------------------------
  Mt_TC96175  (1437)   CAGGCCTTTCCTGTCTGATAATCATCGTTTGTCAATGATCATGGATCCTC
OS_SEQ ID 1   (1170)   TAGGCCTTTCCTCACCGATGATAGTCGCCTATCGTTAATCATGGACTCCC
Sb_TC103780   (1345)   TAGGCCTTTCCTTACTGATGATAGTCGCCTATCTCTGATCATGGACTCCC
  So_TC67974   (637)   TAGGCCTTTCCTTACTGATGATAGTCGCCTATCTCTGATCATGGACTCCC
St_TC117743   (1151)   --------------------------------------------------
Ta_TC257477      (1)   --------------------------------------------------
Zm_TC299056   (1306)   TAGGCCTTTCCTTACTGATGACAGTCGCTTGTCTCTTATCATGGACTCCC
  Consensus   (1651)    AGGCCTTTCCT  C GATGA AGTCG  TATC CT ATCATGGA  C C

                       1701                                              1750
Gm_TC229774    (526)   AACTCAAAGGCCGCTTTCCTTCTAAAGCAGCAAGAACAATAGCTGATATT
 Gs_TC30779   (1571)   CGGTTTCCTCTGCAAGATCCAGCAGCAATTGCTGGAAAACAGATGTCAAG
Hv_TC134680    (789)   GTATAAAAGGACGCTTTCCTACCAAGGCTGCTCGAACTGTGGCAGACATA
Le_TC158378    (458)   AGTTAAAAGGTCGGTTCCCTGCCAAAGCAGCAAGAACTGTGGCTGATATT
Le_TC166426    (470)   AGCTTAAAGGACGGTTTCCCGCAAAAGCAGCCCGTACAGTGGCTGATATT
  Ls_TC15801   (769)   --------------------------------------------------
  Mt_TC96175  (1487)   AACTTAAAGGTCGCTTTCCTTCTAAAGCGGCGAGTACAATAGCTAACATT
OS_SEQ ID 1   (1220)   GTATAAAAGGACGCTTCCCTACCAAGGCTGCTCGGATTGTAGCAGATATC
Sb_TC103780   (1395)   GTATAAAAGGGCGCTTTCCTACTAAGGCTGCTCGGATAGTAGCAGACATC
  So_TC67974   (687)   GTATAAAAGGGCGCTTTCCAACTAAGGCTGCTCGGATAGTAGCAGACATC
St_TC117743   (1151)   --------------------------------------------------
Ta_TC257477      (1)   --------------TTTCCTCCCAAGGCTGCTAGGACTGTGGCAGACATC
Zm_TC299056   (1356)   GTATAAAAGGGCGCTTTCCCACTAAGGCTGCTCGGATAGTAGCAGACATC
  Consensus   (1701)      T AAAGG CGCTTTCCT C AA GCTGC  G A AGTAGC GACAT

                       1751                                              1800
Gm_TC229774    (576)   GCACAAAGATGCCTTCAAAAGGAGCCATCAGACAGACCTACCATGAGGAC
 Gs_TC30779   (1621)   GTCACCGAGTCTCAACG---------------------------------
Hv_TC134680    (839)   ATTCTGAAGTGCCTTCAAAGAGATCCATCAGAAAGGCCTACGATGAGGGC
Le_TC158378    (508)   GCTCAAAGATGTCTGCAAAAGGATCCATCTGAAAGGCCCACCATGAGAAC
Le_TC166426    (520)   GCCCAAAGATGCCTGCAAAAGGATCCATCTGAAAGGCCCACCATGAGAAC
  Ls_TC15801   (769)   --------------------------------------------------
  Mt_TC96175  (1537)   GCACAAAGATGCCTTCAAATGGAGCCATCAGAACGACCAACCATGGGAAC
OS_SEQ ID 1   (1270)   ATATTGAGATGCCTTAATAAAGATCCATCAGAGAGGCCTACCATGAGGGC
Sb_TC103780   (1445)   ATTCTGAAATGCTTGCATAATGATCCATCCGAGAGGCCGACCATGAGGGA
  So_TC67974   (737)   ATTCTGAAATGCTTGCATAAAGATCCATCAGAGAGGCCGACCATGAGGGA
St_TC117743   (1151)   --------------------------------------------------
Ta_TC257477     (37)   ATTCTGAAGTGCCTTCATAGGGATCCATCCGAAAGGCCTACGATGAGGGC
Zm_TC299056   (1406)   ATTTTGAAATGCCTGCGTAAGGATCCATCGGAGAGGCCTACCATGAGGGA
  Consensus   (1751)    T C  A ATGCCT CA AA GATCCATC GA AGGCC ACCATGAGG C
```

# FIGURE 14 (continued)

```
                        1801                                              1850
   Gm_TC229774   (626)  TGTTGTTGAGCATCTCAAGATAATACAGGATTTGAAATATTCGTGCCGGT
    Gs_TC30779  (1638)  --------------------------------------------------
   Hv_TC134680   (889)  CGTCGTGGAGGCCCTAACAAGCGTTCAGGACATAAAGGTCCCCTGCCGGT
   Le_TC158378   (558)  TGTAGTGGAGCAACTCAAGACTGTACAAGTTATGAAGTACCCTTCTCGGT
   Le_TC166426   (570)  TATCTTGGACCAACTCAAATCCGTACAAGTGATGAAGTGCCCTTCACGGT
    Ls_TC15801   (769)  --------------------------------------------------
    Mt_TC96175  (1587)  AGTTGTTGAGCAGCTGAAAAAGATACAAGATTTGAAGCATTCTAGCAGAT
  OS_SEQ ID 1  (1320)  CGTTGTGGAGTCCCTAGCAAGCGTCCAGGACATAAAGGTTCCATGTCGAT
   Sb_TC103780  (1495)  TGTTGTGGAGGCTCTAGCAAGAGTCCAGGAGATAAAGGTTCCGTGCAGAT
    So_TC67974   (787)  TGTTGTGGAGGCCCTAGCAAGAGTCCAGGAAATAAAGGTTCCATGCAGAT
   St_TC117743  (1151)  --------------------------------------------------
   Ta_TC257477    (87)  CGTCGTGGAGTCTCTAGCAAGCGTCCAGGACATAAAGGTCCCCTGCCGGT
   Zm_TC299056  (1456)  TGTTGTGGAGGCCCTAGCAAGAGTCCAGGAAATAAAGGTTCCGTGCAGAT
     Consensus  (1801)   GT GTGGAG   CT   AA  GT CAGGA AT AAG   CC TGC G T

                        1851                                              1900
   Gm_TC229774   (676)  TCCCTCTGCAAGAACCAGCATCAAACTCT--GGAAAACATATGTCAAGAT
    Gs_TC30779  (1638)  --------------------------------------------------
   Hv_TC134680   (939)  ATCCTCTTCAAGTACCGTCCCGCCGCACCCGAGGAAAGTGATGCTAAAGT
   Le_TC158378   (608)  TTCCTCTTCAAGAGCCAAGG-GCAGTTGG-TGTAAAACACATGTCAAAGT
   Le_TC166426   (620)  TTCCTCTGCAAGAACCAGCG-GTTGTTGG-TGGTAAACACATGTCAAAGT
    Ls_TC15801   (769)  --------------------------------------------------
    Mt_TC96175  (1637)  TCCCGCTGCAAGAACCTGCA-----------------CAAATGTCGAGAT
  OS_SEQ ID 1  (1370)  ATCCTTTGCAAGAGCCATCT-GCTGCCCC-AAGAAAGGTGATGTTAAAAT
   Sb_TC103780  (1545)  ATCCTTTGCAAGAACCTTCT-GCTGCACC-AAGAAAAGTAATGCTAAAAT
    So_TC67974   (837)  ATCCTCTGCAAGAACCTTTT-GCTGCACC-AAGAAAAATAATGTTAAAAT
   St_TC117743  (1151)  --------------------------------------------------
   Ta_TC257477   (137)  ATCCTCTTCAAGTACCGTCC-GCCGCACC-GAGGAAAGTGATGCTAAAAT
   Zm_TC299056  (1506)  ATCCTCTGCAAGAACCTTCT-GCTGCACC-AAGAAAAGTGATGTTGAAAT
     Consensus  (1851)   TCCTCTGCAAGAACC  C  GC GC C    G AAA   ATGT AAAAT

                        1901                                              1950
   Gm_TC229774   (724)  CACCAAGTCTCAATGGCATCATCTGCCCTGCACCAAGGGTGAGTTT----
    Gs_TC30779  (1638)  --------------------------------------------------
   Hv_TC134680   (989)  CTACGAGCCTCAATGGCATTGTTCCTCAGCATCCCGTCATGACCTTCTCG
   Le_TC158378   (656)  GTCCGAGCCTAAATGGAATTATTACCCCAACATCAAGATTGAGCTTCTCC
   Le_TC166426   (668)  CTCCAAGCATGAAT------------------------------------
    Ls_TC15801   (769)  --------------------------------------------------
    Mt_TC96175  (1670)  CACCAAGTCTTAACGGTATCAACCACCCTGCACCAAGGCCGAGTTTCTCT
  OS_SEQ ID 1  (1418)  CTACAAGTCTCAATGGCATCATTCATCACCATCCTGTCGTAACCTTCTCA
   Sb_TC103780  (1593)  CTACATCCCTCAATGGAATTGTGCCTCATCATCCTGTCATAACCTTCTCT
    So_TC67974   (885)  CTACATCCCTCAATGGAATTGTGCCTCATCATCCTGTCATAACCTTCTCC
   St_TC117743  (1151)  --------------------------------------------------
   Ta_TC257477   (185)  CCACGAGTCTCAACGGCATTGTTCCTCAGCATCCTGTCATGACCTTCTCG
   Zm_TC299056  (1554)  CTACATCCCTCAATGGGATTGTGCCTCACCATCCTGTCATAACCTTCTCT
     Consensus  (1901)  CT CAAG CTCAATGG AT  T C  C     CC     T A CTTCTC
```

## FIGURE 14 (continued)

```
                       1951                                              2000
Gm_TC229774     (770)  --------------------------------------------------
 Gs_TC30779    (1638)  --------------------------------------------------
Hv_TC134680    (1039)  CCGTCGCCTCCTTCGCGCAACCAGCACCTGGCGTCGCC---AAGATCATC
Le_TC158378     (706)  CCTTCACCACCAACCC---ACCCTATATCTATTTCTCC---GACAAGGAC
Le_TC166426     (682)  --------------------------------------------------
 Ls_TC15801     (769)  --------------------------------------------------
 Mt_TC96175    (1720)  CCATCACCATCATCCAGAGCCCTGGTATCTGTCTCACCTCCAAGATGGTC
OS_SEQ ID 1    (1468)  CCGTCACCTCCTTCGCGAAACCAACATTTGCTCTCGCC---AAGGTCATC
Sb_TC103780    (1643)  CCATCGCCGCCTTCGCATAACCAGCACTTGATTTCACC---AAGGTCATC
 So_TC67974     (935)  CCTTCGCCGCCTTCACATAACCAACACTTGATTTCACC---AAGGTCATC
St_TC117743    (1151)  --------------------------------------------------
Ta_TC257477     (235)  CCGTCGCCTCCTTCGCGCAACCAGCACCTGGTGTCACC---GAGATCATC
Zm_TC299056    (1604)  CCATCGCCGCCTTCACATAACCAGCACTTGATTTCACC---AAGGTCGTC
  Consensus    (1951)  CC TC CC CC TC C   ACC         T TC CC    AG T  TC

                       2001                                              2050
Gm_TC229774     (770)  --------------------------------------------------
 Gs_TC30779    (1638)  --------------------------------------------------
Hv_TC134680    (1086)  GACTTCCGCGCTTCTTCCCCCAAGGACCTGCTCTTCCATCATCACCCTGG
Le_TC158378     (750)  AGCTGCTCCACTGCTGTCTCTACCTTCATGTTCCTCCATCCTCTCTATGG
Le_TC166426     (682)  --------------------------------------------------
 Ls_TC15801     (769)  --------------------------------------------------
 Mt_TC96175    (1770)  GGGAGTGTCAATTCAACTTCCACCTCGCGCGTTTTCTTCAACCCTCTATT
OS_SEQ ID 1    (1515)  CACGTCTGCACTGCTTCCTCCAAGGACCAGCTGTGCT---------CTGG
Sb_TC103780    (1690)  AACATCTGCCTTGTTTCATCCAAGGACATGCTCTTCTA------CTCTGG
 So_TC67974     (982)  AACATCTGCCTTGTTTCATCCAAGGACATGCTCTTCTA------CTCTGG
St_TC117743    (1151)  --------------------------------------------------
Ta_TC257477     (282)  GACGTCTGCGCTGCTTCCCCCAAGGAACTGCTCTTCCACCATCACCCTGG
Zm_TC299056    (1651)  GACATCTGCCTTGTTTCATCCAAGGGCATGCTCTTCTA------CCCTGG
  Consensus    (2001)   C  C  C  T  T C  CCA        G T TTC             TGG

                       2051                                              2100
Gm_TC229774     (770)  --------------------------------------------------
 Gs_TC30779    (1638)  --------------------------------------------------
Hv_TC134680    (1136)  ATTACCCCAGGGTAAGCTCGGTCAAGAAGTCGCCTTCCGGTATCCTGCGG
Le_TC158378     (800)  AGGACTTTGATCGATTGGAAAATCGAAGGCCATCATCTTCATCTGTTCGG
Le_TC166426     (682)  --------------------------------------------------
 Ls_TC15801     (769)  --------------------------------------------------
 Mt_TC96175    (1820)  TGGAGGAGCTTGATAGGCAAGAAAGCCGCAAGTCAGCTTCAGCCTCTAGG
OS_SEQ ID 1    (1556)  ATGACCCTAGAGTAAGCTCTATCAAGAAATCGCCTTCCCCTATTTTACGG
Sb_TC103780    (1734)  ATGATCCCGGTGTAAGCTCTATAAAGAAAACACCTCC---TATTATGCGT
 So_TC67974    (1026)  ATGATCCCGGTGTAAGCTCTATAAAGAAAACACCTCC---TATTATGCGT
St_TC117743    (1151)  --------------------------------------------------
Ta_TC257477     (332)  ACTACCCCAGGGTAAGCTCGGTCAAGAAATCGCCCCCAACATCATGCGG
Zm_TC299056    (1695)  ACGATCCGAGTGTAAGTTCTATAAAGAAAACACCACC---TATTATGCGA
  Consensus    (2051)  A  A        G AAG      A  A    C  C  C        T CG
```

## FIGURE 14 (continued)

```
                            2101                                             2150
 Gm_TC229774     (770)  --------------------------------------------------
  Gs_TC30779    (1638)  --------------------------------------------------
 Hv_TC134680    (1186)  AGACCTGGCGTCGAGGGTTTTTGATTGTCCATACATGGTCGGATTT-CTT
 Le_TC158378     (850)  AGGTCTAGTGTCGAAGGATTTTGATCGATTGTAGAGCACTT--TTTTCTT
 Le_TC166426     (682)  --------------------------------------------------
  Ls_TC15801     (769)  --------------------------------------------------
   Mt_TC96175   (1870)  AAGGCTAGTGTTGAAGGATTTTGATTGTCGATAGTGTTCATTTTTTATTT
 OS_SEQ ID 1    (1606)  AGATCTGGTGTTGAGGGTTTTTGA--------------------------
 Sb_TC103780    (1781)  AGGTCTAGCGTTGAAGGCTTTTGATTGTCCATATTGT-TC---TTTTATT
  So_TC67974    (1073)  AGGTCTAGCGTCGAAGGCTTTTGATTGTCCATATTGT-TC---TTTTATT
 St_TC117743    (1151)  --------------------------------------------------
 Ta_TC257477     (382)  AGACCTGGCGTCGAGGGTTTTTGATTGTCCATATAGTGTCGGATTTTCTT
 Zm_TC299056    (1742)  AGGTCTAGCGTCGAAGGCTTTTGATTGTCCATATTGT-TC---CTTTATT
   Consensus    (2101)  AG  CT G GT GA GG TTTTGAT G    TA           TT  TT

                            2151                                             2200
 Gm_TC229774     (770)  --------------------------------------------------
  Gs_TC30779    (1638)  --------------------------------------------------
 Hv_TC134680    (1235)  CTCTTTGCCTTGGATTTATTTGTTGTTTTGGTTAGCCTAGC--------G
 Le_TC158378     (898)  CAAATTGCTTTTC-------------------------------------
 Le_TC166426     (682)  --------------------------------------------------
  Ls_TC15801     (769)  --------------------------------------------------
   Mt_TC96175   (1920)  GTTATTCTTTTTTTGGTGTAGTTCAACAGAGATTTATAGGAGATAAAGAT
 OS_SEQ ID 1    (1630)  --------------------------------------------------
 Sb_TC103780    (1827)  -TCTTTTTCTCGGATTTATTTGTTTCTTTG-TTAGCCTAGT----GATTT
  So_TC67974    (1119)  -TCCTTTTCTTGGATTTATTAGTTTCTTTG-GTAGCCTAGT----GATTC
 St_TC117743    (1151)  --------------------------------------------------
 Ta_TC257477     (432)  CTCTTTGCCTTG-ATTTATTTGTTGTTTTGGTTAGCCTAGC--------G
 Zm_TC299056    (1788)  -TCCTTTTCTTGGATTTATTTGTTTCTTTG-TTAGTAGTGTAGCGGTTTT
   Consensus    (2151)      TT   T

                            2201                                             2250
 Gm_TC229774     (770)  --------------------------------------------------
  Gs_TC30779    (1638)  --------------------------------------------------
 Hv_TC134680    (1277)  A----GTGGTCTCAGTGCTATG------TGATATGTATATGTTGGAA---
 Le_TC158378     (911)  --------------------------------------------------
 Le_TC166426     (682)  --------------------------------------------------
  Ls_TC15801     (769)  --------------------------------------------------
   Mt_TC96175   (1970)  TTGTAATCATCCCTCAGTGTGATGCAGAGAGGATGCTCTTTTGTACATAG
 OS_SEQ ID 1    (1630)  --------------------------------------------------
 Sb_TC103780    (1871)  TAGCTGTGTTCTGTATTGTAAGACAAGTGGCCTTA--TGTAGTGCCCTTG
  So_TC67974    (1163)  TAGCTATGTTCTGTATTGCAAGACAAGCGGCCTTAA-TGTAGTGCCCTTG
 St_TC117743    (1151)  --------------------------------------------------
 Ta_TC257477     (473)  A----GTGATCTCAGTGCTATG------GGATATAC-TATGTTGCAA---
 Zm_TC299056    (1836)  TAGCTGTGTTCTGTATTGTAAGACAAGTGGCCTCATATGTAGTGCCCTTG
   Consensus    (2201)
```

## FIGURE 14 (continued)

```
                          2251                                             2300
Gm_TC229774      (770)  --------------------------------------------------
 Gs_TC30779     (1638)  --------------------------------------------------
Hv_TC134680     (1314)  AGACATCTGAAAAAA--GGGCAGAGT--GAAGTGTAGATAGTAGAACCAG
Le_TC158378      (911)  --------------------------------------------------
Le_TC166426      (682)  --------------------------------------------------
 Ls_TC15801      (769)  --------------------------------------------------
 Mt_TC96175     (2020)  TGCAAAGGTTGGTCCCCTTGGTTCAATTAGTTACTCCATTTTG-------
OS_SEQ ID 1     (1630)  --------------------------------------------------
Sb_TC103780     (1919)  AGTCTCGAGTAATAACTAAGCAGAGCAGGATGAATTGTAAATAGTATCAG
 So_TC67974     (1212)  GGTCTAGAGTAATAACTAAGCAGAGCAGGATGAATTGTAAATAGGATCAG
St_TC117743     (1151)  --------------------------------------------------
Ta_TC257477      (509)  GGACATGTGAAAAA---GGGCAGAGT--GAAGCGTAGATAGTAGAACCAG
Zm_TC299056     (1886)  GGTCTGGAGTAA-----A-GCAGAACAGGATGAATTGTAAATAGGACCAG
  Consensus     (2251)

                          2301                                             2350
Gm_TC229774      (770)  --------------------------------------------------
 Gs_TC30779     (1638)  --------------------------------------------------
Hv_TC134680     (1360)  CTTTGTAGATACTTGTCGTTCTCTGATTGGATGGGGGTCATGGAAGCAGT
Le_TC158378      (911)  --------------------------------------------------
Le_TC166426      (682)  --------------------------------------------------
 Ls_TC15801      (769)  --------------------------------------------------
 Mt_TC96175     (2063)  --------------------------------------------------
OS_SEQ ID 1     (1630)  --------------------------------------------------
Sb_TC103780     (1969)  GTT-GTAGATACCTTTT-T---GTGCTCTAATTGGG----TGGAAGCAGC
 So_TC67974     (1262)  GTT-GTAGATACCTTTT-T---GTGCTCTAATTGGG----TGGAAGCAGC
St_TC117743     (1151)  --------------------------------------------------
Ta_TC257477      (554)  CTT-GTAGATACTTGTCGTTCTCTGATTGGATGGGGGTCATGGAAGAAGT
Zm_TC299056     (1930)  GTT-GTAGATACCTTTT-TTTGTGCTCGAATTGGG----TGGAAGCAGC
  Consensus     (2301)

                          2351                                             2400
Gm_TC229774      (770)  --------------------------------------------------
 Gs_TC30779     (1638)  --------------------------------------------------
Hv_TC134680     (1410)  GTG---TGTTAGAGTGGGGCTTGTAACCCCATTATTATGCAATAAACGTG
Le_TC158378      (911)  --------------------------------------------------
Le_TC166426      (682)  --------------------------------------------------
 Ls_TC15801      (769)  --------------------------------------------------
 Mt_TC96175     (2063)  --------------------------------------------------
OS_SEQ ID 1     (1630)  --------------------------------------------------
Sb_TC103780     (2010)  GTGCTGAGTCTGAGTAGGCCTTGTAACCTCATAA-------ATAAAC---
 So_TC67974     (1303)  GTGCTAAGT--GAG----CCTTGTAACCTCCCAAT---GCAATAAAC---
St_TC117743     (1151)  --------------------------------------------------
Ta_TC257477      (603)  GTG---TGTT-GAGTGGGGCTTGTAACCCCACTA---TGCAATAAACGTG
Zm_TC299056     (1974)  GTGCCGAGT--GGG----CCTTGTAACCTCATAAT---GCAATAAAC---
  Consensus     (2351)
```

**FIGURE 14 (continued)**

```
                        2401                                              2450
Gm_TC229774    (770) --------------------------------------------------
  Gs_TC30779  (1638) --------------------------------------------------
 Hv_TC134680  (1457) GGTTGGTCGGTCG-----TATTTGCTCG----------------------
 Le_TC158378   (911) --------------------------------------------------
 Le_TC166426   (682) --------------------------------------------------
  Ls_TC15801   (769) --------------------------------------------------
  Mt_TC96175  (2063) --------------------------------------------------
 OS_SEQ ID 1  (1630) --------------------------------------------------
 Sb_TC103780  (2050) --TC-GTTTCCCAG----TTTCTGTCC-----------------------
  So_TC67974  (1341) --TC-GTTTTTCAG----TTTTTGTTC-----------------------
 St_TC117743  (1151) --------------------------------------------------
 Ta_TC257477   (646) GGTTGGTTGGTCGGTCGGTATTTGCTCGCCGTCAAACATTTCAGCTGTTT
 Zm_TC299056  (2012) --TCCGTTTCCCAG----TTTCTGTAAAAAAAAA----------------
   Consensus  (2401)

                        2451                                          2496
Gm_TC229774    (770) ----------------------------------------------
  Gs_TC30779  (1638) ----------------------------------------------
 Hv_TC134680  (1480) ----------------------------------------------
 Le_TC158378   (911) ----------------------------------------------
 Le_TC166426   (682) ----------------------------------------------
  Ls_TC15801   (769) ----------------------------------------------
  Mt_TC96175  (2063) ----------------------------------------------
 OS_SEQ ID 1  (1630) ----------------------------------------------
 Sb_TC103780  (2070) ----------------------------------------------
  So_TC67974  (1361) ----------------------------------------------
 St_TC117743  (1151) ----------------------------------------------
 Ta_TC257477   (696) CTTTCTTCTCTTGAATGCTAAGTTTTGTTGCTGCTAGTCGTGGAAT
 Zm_TC299056  (2040) ----------------------------------------------
   Consensus  (2451)
```

**FIGURE 14 (continued)**

```
                      1                                                50
OS_BAD73441    (1)  --MGCFTVLRSKKKKPLALTKKSVDARESTSSRLPEP-EAHVPSLQSAPP
   SEQ ID 2    (1)  -MMGCFTVLRSKKKKPLALTKKSVDARESTSSRLPEP-EAHVPSLQSAPP
   SEQ ID 4    (1)  MVLGCFPLK-SKKKR-GSVSMKRKDLEESKPTALPEPPKIPSRNLQSAPP
  Consensus    (1)   MMGCFTVLRSKKKKPLALTKKSVDARESTSSRLPEP EAHVPSLQSAPP

                    51                                                100
OS_BAD73441   (48)  SFRNKAKIHQSEKK---ASYSRARVLSAPSSLIVVDQDGLPYAEFDDQDD
   SEQ ID 2   (49)  SFRNKAKIHQSEKK---ASYSRARVLSAPSSLIVVDQDGLPYAEFDDQDD
   SEQ ID 4   (49)  SFRTRVKPIQSNNGGTGEMSSRARVMSAPSSTHGAAERDLLASVLHDEQD
  Consensus   (51)  SFRNKAKIHQSEKK   ASYSRARVLSAPSSLIVVDQDGLPYAEFDDQDD

                    101                                               150
OS_BAD73441   (95)  SRGKGGSIKGHRFSNPLPLPLPSP-EGKSLRNFGSFKAINASG-----PL
   SEQ ID 2   (96)  SRGKGGSIKGHRFSNPLPLPLPSP-EGKSLRNFGSFKAINASG-----PL
   SEQ ID 4   (99)  EQPR-DPRTSTKESSPQPLPLPSPRTGSSLKNWGSFKSFNGSSGRLSSSA
  Consensus  (101)  SRGKGGSIKGHRFSNPLPLPLPSP EGKSLRNFGSFKAINASG     PL

                    151                                               200
OS_BAD73441  (139)  DASGPLPLPPKKCDGLKNFSYEELSSACQWFSGDQCVSESLTSTSYKASF
   SEQ ID 2  (140)  DASGPLPLPPKKCDGLKNFSYEELSSACQWFSGDQCVSESLTSTSYKASF
   SEQ ID 4  (148)  AVSGPLPLPPS--GSWRSFSYDEVMRACNAFSSDRCVMEGLTSVMYMASF
  Consensus  (151)  DASGPLPLPPKKCDGLKNFSYEELSSACQWFSGDQCVSESLTSTSYKASF

                    201                                               250
OS_BAD73441  (189)  RDDFTDP--KTIEAIVSRLLSSTQSLKEFKTQVNTLASLQHPNLCKLIGF
   SEQ ID 2  (190)  RDDFTDP--KTIEAIVSRLLSSTQSLKEFKTQVNTLASLQHPNLCKLIGF
   SEQ ID 4  (196)  GDEAGTSGLKKVDATVVRLHVITQSIREFINEVNTLASLQHQNLCKLVGY
  Consensus  (201)  RDDFTDP  KTIEAIVSRLLSSTQSLKEFKTQVNTLASLQHPNLCKLIGF

                    251                                               300
OS_BAD73441  (237)  HAREESNERMLVYERLHHGSLDKLLFGRSDGRFMDWSARLKVALGAARGL
   SEQ ID 2  (238)  HAREESNERMLVYERLHHGSLDKLLFGRSDGRFMDWSARLKVALGAARGL
   SEQ ID 4  (246)  HARDGSDTRMLVYERLALGSLDRLLHGRSDGPPLDWNTRMKIALCAAQGL
  Consensus  (251)  HAREESNERMLVYERLHHGSLDKLLFGRSDGRFMDWSARLKVALGAARGL

                    301                                               350
OS_BAD73441  (287)  AFLHDEGPFQAMYNDFSTSNIQIDKDFTAKLSGYGCVGFNTEEEISNASV
   SEQ ID 2  (288)  AFLHDEGPFQAMYNDFSTSNIQIDKDFTAKLSGYGCVGFNTEEEISNASV
   SEQ ID 4  (296)  TFLHEEGPFQAMYNEFSTANIQVDKDFSAKLSGYGCAGHAPETETSNS-S
  Consensus  (301)  AFLHDEGPFQAMYNDFSTSNIQIDKDFTAKLSGYGCVGFNTEEEISNASV
```

**FIGURE 15**

```
                         351                                              400
OS_BAD73441     (337) AAANLSVETLEKGVLTPKSNVWCFGVVLLELITGRKNLDVRSSKEERNIV
    SEQ ID 2    (338) AAANLSVETLEKGVLTPKSNVWCFGVVLLELITGRKNLDVRSSKEERNIV
    SEQ ID 4    (345) ALANLSVETLERGILTPKSNVWSYGIVLLEMSTGRKNMDGSYPKEERNLV
   Consensus    (351) AAANLSVETLEKGVLTPKSNVWCFGVVLLELITGRKNLDVRSSKEERNIV

                         401                                              450
OS_BAD73441     (387) KWSRPFLTDDSRLSLIMDSRIKGRFPTKAARIVADIILRCLNKDPSERPT
    SEQ ID 2    (388) KWSRPFLTDDSRLSLIMDSRIKGRFPTKAARIVADIILRCLNKDPSERPT
    SEQ ID 4    (395) KWSRAFLADDCRLSLIMDPQLKGRFPAKAARSIADIAQKCLQVEPSERPT
   Consensus    (401) KWSRPFLTDDSRLSLIMDSRIKGRFPTKAARIVADIILRCLNKDPSERPT

                         451                                              500
OS_BAD73441     (437) MRAVVESLASVQDIKVPCRYPLQEPS-AAPRKVMLKSTSLNGIIHHHPVV
    SEQ ID 2    (438) MRAVVESLASVQDIKVPCRYPLQEPS-AAPRKVMLKSTSLNGIIHHHPVV
    SEQ ID 4    (445) MRNIVDQLKIIQDMKYSCRFPLREPAPVVARKHMGRSSSLNTIIWTPASV
   Consensus    (451) MRAVVESLASVQDIKVPCRYPLQEPS AAPRKVMLKSTSLNGIIHHHPVV

                         501                                              550
OS_BAD73441     (486) T----FSPSPPSRNQHLLSPRS---STSALLPPRTSCALDDPRVSSIKKS
    SEQ ID 2    (487) T----FSPSPPSRNQHLLSPRS---STSALLPPRTSCALDDPRVSSIKKS
    SEQ ID 4    (495) PPRSSFSPSPPPRRPSISPTRGRTLVFPPVFPPRACSSLEEMAREEVRKS
   Consensus    (501) T    FSPSPPSRNQHLLSPRS   STSALLPPRTSCALDDPRVSSIKKS

                         551          564
OS_BAD73441     (529) PSPILRRSGVEGF-
    SEQ ID 2    (530) PSPILRRSGVEGF-
    SEQ ID 4    (545) SSASGRRTSIEGF-
   Consensus    (551) PSPILRRSGVEGF
```

FIGURE 15 (continued)

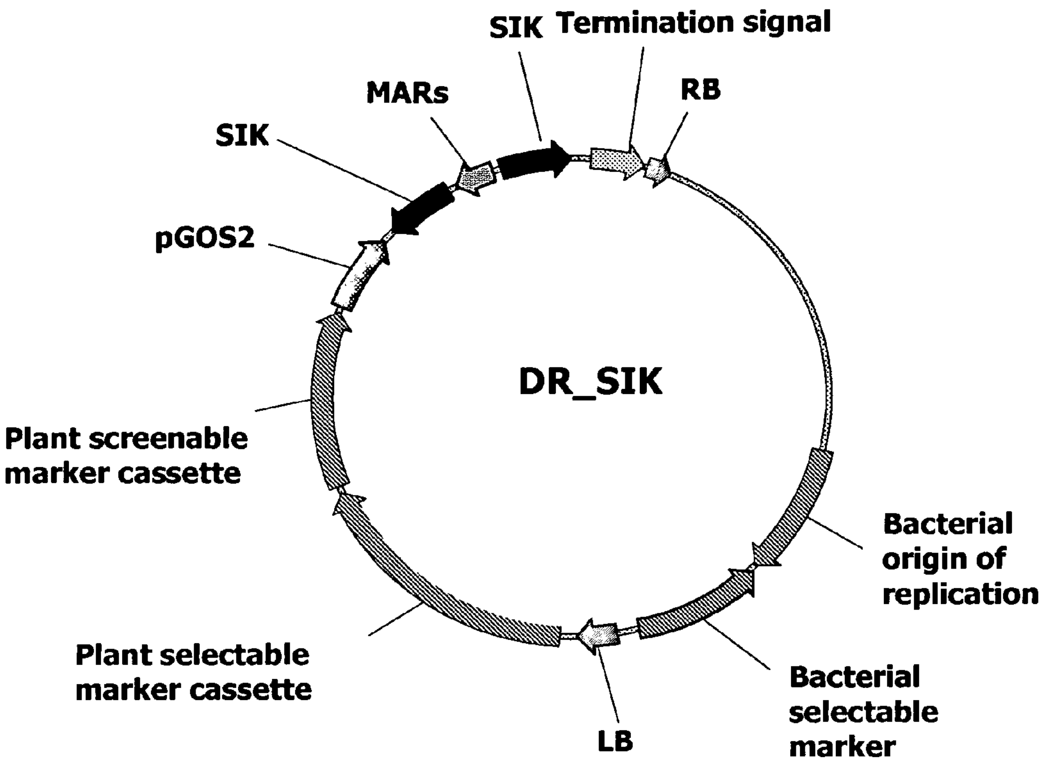


FIGURE 16

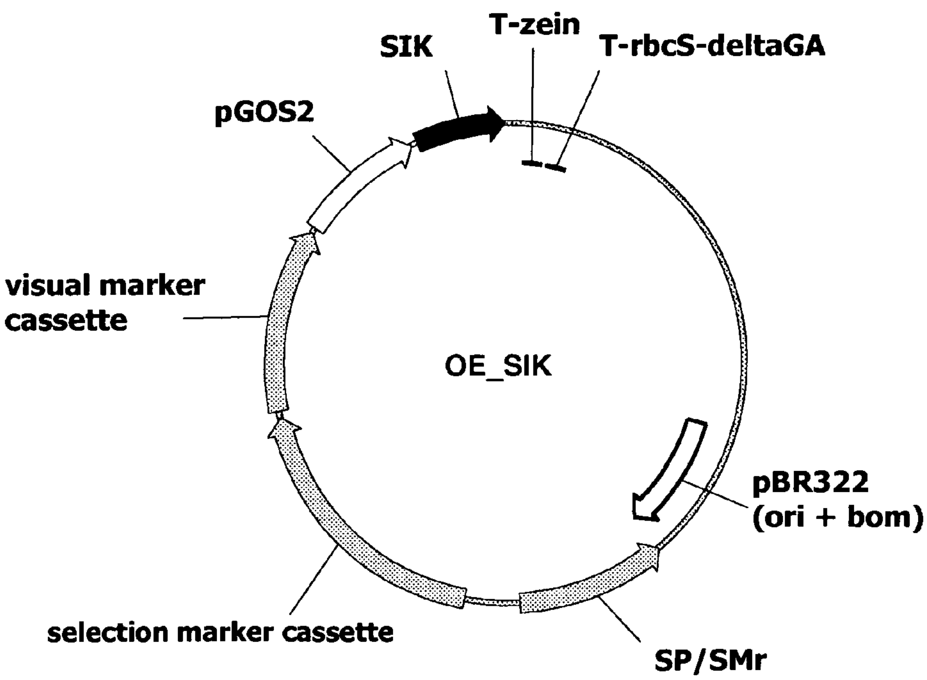


FIGURE 17

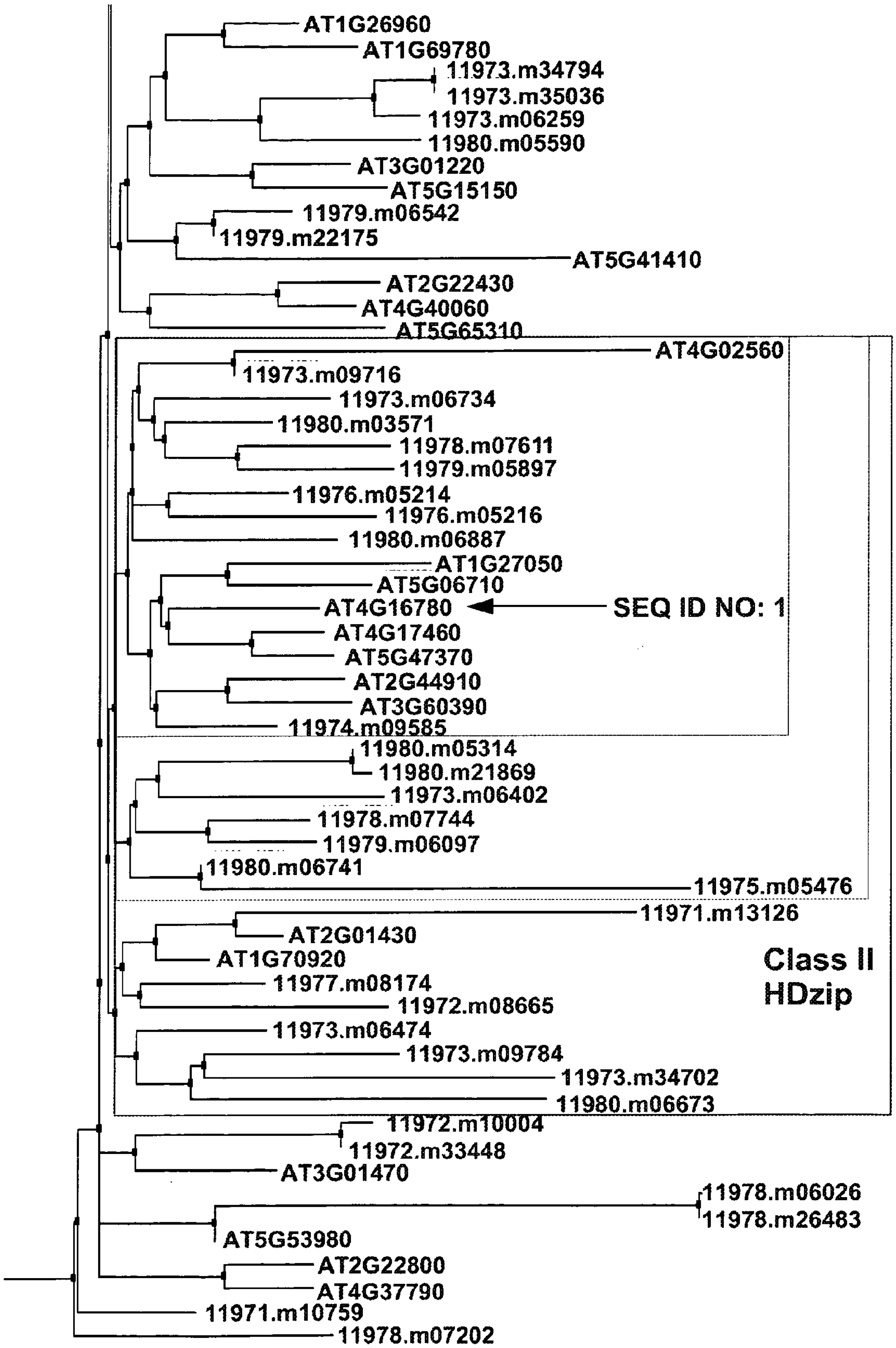
AT1G26960
AT1G69780
11973.m34794
11973.m35036
11973.m06259
11980.m05590
AT3G01220
AT5G15150
11979.m06542
11979.m22175
AT5G41410
AT2G22430
AT4G40060
AT5G65310
AT4G02560
11973.m09716
11973.m06734
11980.m03571
11978.m07611
11979.m05897
11976.m05214
11976.m05216
11980.m06887
AT1G27050
AT5G06710
AT4G16780
SEQ ID NO: 1
AT4G17460
AT5G47370
AT2G44910
AT3G60390
11974.m09585
11980.m05314
11980.m21869
11973.m06402
11978.m07744
11979.m06097
11980.m06741
11975.m05476
11971.m13126
AT2G01430
AT1G70920
Class II HDzip
11977.m08174
11972.m08665
11973.m06474
11973.m09784
11973.m34702
11980.m06673
11972.m10004
11972.m33448
AT3G01470
11978.m06026
11978.m26483
AT5G53980
AT2G22800
AT4G37790
11971.m10759
11978.m07202

FIGURE 18

```
CLUSTAL W (1.83) multiple sequence alignment


glycina                              -----------MTVQ-------KED-----------------LGLSLSLS 15
homeobox-leucine                     --------------------------------------------------
AT4G16780                            -----------MMFE-------KDD-----------------LGLSLGLN 15
AT4G17460                            -----------MMMG-------KED-----------------LGLSLSLG 15
AT5G47370                            -----------MMMG-------KED-----------------LGLSLSLG 15
AT2G44910                            ------------MGE-------RDDG----------------LGLSLSLG 15
AT3G60390                            ------------MSE-------RDDG----------------LGLSLSLS 15
THOM1"                               -----------MSSE-------KEDG----------------LGLSLSLG 16
craterostigmaCPHB-3                  HMEDMAINLEUCINEZIPPERPRTEINCPHB--------AALLGLSL-GS 41
TaHDZipII-1"homeodomain-leucin       -----------MMHR-----ADGLD-----------------LGLGLGLG 17
Hox2                                 ----------------------MMD-----------------LGLSLGLG 11
ricehox1homeodomain-leucine          --------MEMMVHGRRDEQYGGLG-----------------LGLGLGLS 25
Oshox1"homeodomain                   --------MEMMVHGRRDEQYGGLG-----------------LGLGLGLS 25
OSJNBb0089A17.12"homeodomain         --------MEMMVHGRRDEQYGGLR-----------------LGLGLGLS 25
AT5G06710                            ------MELALSLGDNTKKQFSFMEKNSKINNPSVSSTSTSEKDLGFCMA 44
Pphb4"homeodomain-leucine            --------------------------------------------------
Mshb1"type                           ------MELGLSLGDSSKPLIGLMEKHP--------HQASKELGLGFNTT 36
AT4G37790                            ----------MGLDDS------------------------CNTGLVLGLG 16
AT2G22800                            ----------MGFDDT------------------------CNTGLVLGLG 16
OJ1781E12.8"Hypothetical             ----------MAQEDVGH---------------------LSDAGLALGLS 19
OSJNOa174H12.5"putative              ------MELGLSLGDAVTVADG------------------GRLELVLGLG 26
RiceHox7"AAQ55491.1"                 -------------------------------------------MQKLTNA 7
Oshox7"homeodomain                   --------------------------------------------------
P0020C11.33"putative                 --------------------------------------------------
AT2G01430                            -----------MIKLLFTYICTYTYK----------------LYALYHMD 23
AT1G70920                            --------------------------------------------------
riceP0510C12.22"putative             -----------MITAAVP-RADRPAR----------------LPPNRGTC 22


glycina                              FP----------LLSSSPSS---------------HNP---QKPSWNDPI 37
homeobox-leucine                     --------------------------------------------------
AT4G16780                            FPKKQINLKSNPSVSVTPSS---------------SSFGLFRRSSWNESF 50
AT4G17460                            FAQNH-----PLQLNLKPTS---------------SPMSNLQMFPWNQTL 45
AT5G47370                            FSQNHN----PLQMNLNPNS---------------SLSNNLQRLPWNQTF 46
AT2G44910                            N-SQQKEPSLRLNLMPLTTS---------------SSSSSFQHMHNQNNN 49
AT3G60390                            LGFNQKDPSSRLNPMPLASY---------------ASSSHMQHMQ-QSNY 49
THOM1"                               IMSCPQNNHKTTPSLPLNLL---------------PFMHHHQVSSGRKDE 51
craterostigmaCPHB-3                  FGPSSSGGSHPPPALPLPSN---------------LFRPSLQEIDRTQRS 76
TaHDZipII-1"homeodomain-leucin       LASQGSITSSTTTAASSSPA------------------SASHSQHWTAAL 49
Hox2                                 LASQGSLTSSTTTTSSPGAG------------------SSSP---WAAAL 40
ricehox1homeodomain-leucine          LGVAGGAADDEQPPPRRGAAPPPQQQLCGWNGGGLFSSSSSDHRGRSAMM 75
Oshox1"homeodomain                   LGVAGGAADDEQPPPRRGAAPPPQQQLCGWNGGGLFSSSSSDHRGRSAMM 75
OSJNBb0089A17.12"homeodomain         LGVAGGAADDEQPPPRRGAAPPPQQQLCGWNGGGLFSSSSSDHRGRSAMM 75
AT5G06710                            LDVAFGGHRSLSSSSSPSVEDEKKKPAPRAKKSDEFRVSSSVDPPLQL-- 92
Pphb4"homeodomain-leucine            --------------------------------------------------
Mshb1"type                           LSIGPIISTTQRDQLQQQQQEEEKTQRDINNNSNRTENPNPVLHQLDLLP 86
AT4G37790                            L-------SPTPNNYNHAIKKSSSTVD---------HRFIRLDPSLTLS- 49
AT2G22800                            P-------SPIPNNYNSTIRQSS---------------VYKLEPSLTLC- 43
OJ1781E12.8"Hypothetical             LGGGGGGTTDAAAAHRGGCRRPSPS-----------SQCPPLEPSLTLS- 57
OSJNOa174H12.5"putative              VGVG---AGVRRGEEEERGRREDVVCAGRWAAMAAASPEPSVRLSLVSSL 73
RiceHox7"AAQ55491.1"                 TKQWQLHSQIVRFYLFLSIP---------------LFVSSAETGSANSEM 42
Oshox7"homeodomain                   -------------------P---------------LFVSSAETGSANSEM 16
P0020C11.33"putative                 ------------------------------------MELELSLGSANSEM 14
AT2G01430                            YACVCMYKYKGIVTLQVCLFYIKLR----------VFLSNFTFSSSILAL 63
AT1G70920                            --------------------------------------------------
riceP0510C12.22"putative             RQSSCTRPCAPTRGVPTVTPPPASA----------ARPSIPTHQSTIARL 62
```

# FIGURE 19

```
glycina                              FT------------------------SSGEAGSFLRGIDVNRLP---SVV 60
homeobox-leucine                     --------------------------------------------------
AT4G16780                            TSSVPNSD-----------------SSQKETRTFIRGIDVNRPP---STA 80
AT4G17460                            VS-----------------------SSDQQKQQFLRKIDVNSLP---TTV 69
AT5G47370                            DP-----------------------TSD------LRKIDVNSFP---STV 64
AT2G44910                            SHPQKIHNISWTHLFQSSGIKRTTAERNSDAGSFLRGFNVNRAQSSVAVV 99
AT3G60390                            NHPQKIQN-TWINMFQSS-------ERNSDMRSFLRGIDVNRAP-STVVV 90
THOM1"                               GG-ERVRG---------------GIDMNEPARMIIECDDEEDEE------ 79
craterostigmaCPHB-3                  IGAFLQAP--------------TAAAVCREPASAFRGIDVNRPP---TIV 109
TaHDZipII-1"homeodomain-leucin       SS---------------------------VIG-LRKEEP--------GV- 62
Hox2                                 NS---------------------------IVGDVRRDQAAAHAAAAVGVG 63
ricehox1homeodomain-leucine          ACHDVIEM-------------------PFLRGIDVNRAPAAETTTTTARG 106
Oshox1"homeodomain                   ACHDVIEM-------------------PFLRGIDVNRAPAAETTTTTARG 106
OSJNBb0089A17.12"homeodomain         ACHDVIEM-------------------PFLRGIDVNRAPAAETTTTTARG 106
AT5G06710                            QLHFPNWL--------------PENSKG-----RQGGRMPLGAATVVEEE 123
Pphb4"homeodomain-leucine            --------------------------------------------------
Mshb1"type                           QLSFP-WN--------------PPSQNGNLLSTEFGGSSRGLDVNVVPPA 121
AT4G37790                            ---------------------------------LSGE-SYKIKTGAGAGD 65
AT2G22800                            ---------------------------------LSGDPSVTVVTGA---D 57
OJ1781E12.8"Hypothetical             ---------------------------------LPDDAAAGAAATAT--A 72
OSJNOa174H12.5"putative              GLHWPSET--------------GRSEAAARGFDVNRAPSVAAGAPGMEDD 109
RiceHox7"AAQ55491.1"                 CT---------------------------------RGFDVNTRP-----A 54
Oshox7"homeodomain                   CT---------------------------------RGFDVNTRP-----A 28
P0020C11.33"putative                 CT---------------------------------RGFDVNTRP-----A 26
AT2G01430                            KNP-----------------------------NNSLIKIMAILPENSSNL 84
AT1G70920                            ---------------------------------------MALSP-NSSSL 10
riceP0510C12.22"putative             AVTGCGGC---------------ASATERVRRGDWPGPAREKPASDVTGL 97
```

```
glycina                              DC-EEEAG---VSSPNS--------TVSS-VSGKR--SERETNG------ 89
homeobox-leucine                     --------------------------------------------------
AT4G16780                            EYGDEDAG---VSSPNS--------TVSS-STGKR--SERE--------- 107
AT4G17460                            DL-EEETG---VSSPNS--------TISSTVSGKRRSTEREGTSGGGCG- 106
AT5G47370                            NC-EEDTG---VSSPNS--------TISSTISGKR--SEREGISGTGVGS 100
AT2G44910                            DLEEEAAV---VSSPNS--------AVSS-LSGNK--RDLAVAR---GG- 131
AT3G60390                            DVEDEGAG---VSSPNS--------TVSSVMSGKKSERELMAAAGAVGGG 129
THOM1"                               --EDQVLM---VSSPNS--------TVSS-VSGKR-----SHDR------ 104
craterostigmaCPHB-3                  DCGEENNNPIASPSPNS--------TVCS-SSGKRTSGEREEKE------ 144
TaHDZipII-1"homeodomain-leucin       ---------QASTSPE---------------SGGTK-------------- 74
Hox2                                 VGGEEMYQGRASTSPDSA-------AALSSASGKRE-------------- 92
ricehox1homeodomain-leucine          PSCSEEDEEPGASSPNS---------TLSSLSGKRG-------------- 133
Oshox1"homeodomain                   PSCSEEDEEPGASSPNS---------TLSSLSGKRG-------------- 133
OSJNBb0089A17.12"homeodomain         PSCSEEDEEPGASSPNS---------TLSSLSGKRG-------------- 133
AT5G06710                            EEEEEAVPSMSVSPPDSVTSS-FQLDFGIKSYG-------YERRSNKRDI 165
Pphb4"homeodomain-leucine            --------------------------------------------------
Mshb1"type                           TVVVMANDEMALSSSRIVQHLLFRRDLCMYSRGGSGGRSLSGSGGNKREF 171
AT4G37790                            QICRQTSSHSGISS---------------FSSG-------RVKREREISG 93
AT2G22800                            QLCRQTSSHSGVSS---------------FSSG-------RVVK-RERDG 84
OJ1781E12.8"Hypothetical             TASGGGGPAHSVSS---------------LSVG-------AAAAAAVKRE 100
OSJNOa174H12.5"putative              EEGPGAAPALSSSPNDSGGS----FPLDLSGQG------LRGHAEAAAQG 149
RiceHox7"AAQ55491.1"                 DGGAEAGR---PSSPSS---------MQEASTR----------------- 75
Oshox7"homeodomain                   DGGAEAGR---PSSPSS---------MQEASTR----------------- 49
P0020C11.33"putative                 DGGAEAGR---PSSPSS---------MQEASTR----------------- 47
AT2G01430                            DLTISVPGFSSSPLSDE-------------GSGGGRDQLRLDMN------ 115
AT1G70920                            DLTISIPSFSPSPSLGD-------------HHG-MRD---FDIN------ 37
riceP0510C12.22"putative             ELTMAVPGLSSSGSEGAG--------CNNNNAGGGCNMRDLDIN------ 133
```

# FIGURE 19 (continued)

```
glycina                               ----EENDTDRACSRGI----ISDEED-----AETSRKKLRLSKDQSIVL 126
homeobox-leucine                      -------------------------------------KKLRLSKDQSIVL 13
AT4G16780                             ------EDTDPQGSRG-----ISDDED-----GDNSRKKLRLSKDQSAIL 141
AT4G17460                             --DDLDITLDRSSSRGT----SDEEEDY---GGETCRKKLRLSKDQSAVL 147
AT5G47370                             GDDHDEITPDRGYSRGT----SDEEED----GGETSRKKLRLSKDQSAFL 142
AT2G44910                             --DENEAERASCSRGGGS--GGSDDEDG-GNGD-GSRKKLRLSKDQALVL 175
AT3G60390                             RVEDNEIERASCSLGGG-----SDDEDGSGNGDDSSRKKLRLSKEQALVL 174
THOM1"                                --EENEGERATSS----------LEDDG---GDAAARKKLRLSKEQAAVL 139
craterostigmaCPHB-3                   -----DGDRAASSSFEV------EDDDG-GGGDASARKKLRLSKEQAVVL 182
TaHDZipII-1"homeodomain-leucin        ------RGLERTGSGLAA--GSDEDDDGG--DGTGGRKKLRLSKDQAAVL 114
Hox2                                  ------RELERSGS------GVD-DDDGA--DGAGGRKKLRLSKDQAAVL 127
ricehox1homeodomain-leucine           ------APSAATAA------AAAASDDED--SGGGSRKKLRLSKDQAAVL 169
Oshox1"homeodomain                    ------APSAATAA------AAAASDDED--SGGGSRKKLRLSKDQAAVL 169
OSJNBb0089A17.12"homeodomain          ------APSAATAA------AAAASDDED--SGGGSRKKLRLSKDQAAVL 169
AT5G06710                             DDEV---ERSASRASN------EDNDDEN----GSTRKKLRLSKDQSAFL 202
Pphb4"homeodomain-leucine             ---------MSSRGGS------DDEDE------GTTRKKLRLSKEQSALL 29
Mshb1"type                            SDGEGYDQRNSSRVSD------EDDNCGVG--NGNTRKKLRLSKDQSAFL 213
AT4G37790                             GDGEEEAEETTERVVCSRVSDDHDDEEGV-----SARKKLRLTKQQSALL 138
AT2G22800                             GEESPEEEEMTERVIS----DYHEDEEGI-----SARKKLRLTKQQSALL 125
OJ1781E12.8"Hypothetical              RAEEADGERVSSTAAG------RDDDDDG-----STRKKLRLTKEQSALL 139
OSJNOa174H12.5"putative               GGGGGGGERSSSRASD--------DDEGA-----SARKKLRLSKEQSAFL 186
RiceHox7"AAQ55491.1"                  ------QQVADQEAAD-------DEDNGG----GGARKKLRLSKEQSSFL 108
Oshox7"homeodomain                    ------QQVADQEAAD-------DEDNGG----GGARKKLRLSKEQSSFL 82
P0020C11.33"putative                  ------QQVADQEAAD-------DEDNGG----GGARKKLRLSKEQSSFL 80
AT2G01430                             ------RLPSSED------GDDEEFSHDDGSAP--PRKKLRLTREQSRLL 151
AT1G70920                             ------QTPKTEEDREWMIGATPHVNEDDSNSGGRRRKKLRLTKEQSHLL 81
riceP0510C12.22"putative              ------QPASGGEEEEFPMGSVEEDEEERGVGGPHRPKKLRLSKEQSRLL 177
                                                                           *****:::*: .*


glycina                               EESFKEHNTLNPKQKLALAKQLGLRARQVEVWFQNRRARTKLKQTEVDCE 176
homeobox-leucine                      EESFKEHNTLNPKQKLALAKQLGLRARQVEVWFQNRRARTKLKQTEVDCE 63
AT4G16780                             EETFKDHSTLNPKQKQALAKQLGLRARQVEVWFQNRRARTKLKQTEVDCE 191
AT4G17460                             EDTFKEHNTLNPKQKLALAKKLGLTARQVEVWFQNRRARTKLKQTEVDCE 197
AT5G47370                             EETFKEHNTLNPKQKLALAKKLNLTARQVEVWFQNRRARTKLKQTEVDCE 192
AT2G44910                             EETFKEHSTLNPKQKLALAKQLNLRARQVEVWFQNRRARTKLKQTEVDCE 225
AT3G60390                             EETFKEHSTLNPKQKMALAKQLNLRTRQVEVWFQNRRARTKLKQTEVDCE 224
THOM1"                                EETFKEHNTLNPKQKLALSKQLNLRPRQVEVWFQNRRARTKLKQTEVDCE 189
craterostigmaCPHB-3                   EETFKEHSTLNPKEKIALAKQLNLMPRQVEVWFQNRRARTKLKQTEVDCE 232
TaHDZipII-1"homeodomain-leucin        EECFKTHSTLNPKQKTALANRLGLRPRQVEVWFQNRRARTKLKQTEVDCE 164
Hox2                                  EECFKTHSTLNPKQKVALANRLGLRPRQVEVWFQNRRARTKLKQTEVDCE 177
ricehox1homeodomain-leucine           EDTFKEHNTLNPKQKAALARQLNLKPRQVEVWFQNRRARTKLKQTEVDCE 219
Oshox1"homeodomain                    EDTFKEHNTLNPKQKAALARQLNLKPRQVEVWFQNRRARTKLKQTEVDCE 219
OSJNBb0089A17.12"homeodomain          EDTFKEHNTLNPKQKAALARQLNLKPRQVEVWFQNRRARTKLKQTEVDCE 219
AT5G06710                             EDSFKEHSTLNPKQKIALAKQLNLRPRQVEVWFQNRRARTKLKQTEVDCE 252
Pphb4"homeodomain-leucine             EESFKEHSTLNPKQKNALAKQLGLRPRQVEVWFQNRRARTKLKQTEVDCE 79
Mshb1"type                            EESFKEHHTLNPKQKLALAKQLNLRPRQVEVWFQNRRARTKLKQTEVDCE 263
AT4G37790                             EDNFKLHSTLNPKQKQALARQLNLRPRQVEVWFQNRRARTKLKQTEVDCE 188
AT2G22800                             EESFKDHSTLNPKQKQVLARQLNLRPRQVEVWFQNRRARTKLKQTEVDCE 175
OJ1781E12.8"Hypothetical              EDRFREHSTLNPKQKVALAKQLNLRPRQVEVWFQNRRARTKLKQTEVDCE 189
OSJNOa174H12.5"putative               EESFKEHSTLNPKQKVALAKQLNLRPRQVEVWFQNRRARTKLKQTEVDCE 236
RiceHox7"AAQ55491.1"                  EDSFKEHSTLTPKQKSDLANRLNLRPRQVEVWFQNRRARTKLKQTEVDCE 158
Oshox7"homeodomain                    EDSFKEHSTLTPKQKSDLANRLNLRPRQVEVWFQNRRARTKLKQTEVDCE 132
P0020C11.33"putative                  EDSFKEHSTLTPKQKSDLANRLNLRPRQVEVWFQNRRARTKLKQTEVDCE 130
AT2G01430                             EDSFRQNHTLNPKQKEVLAKHLMLRPRQIEVWFQNRRARSKLKQTEMECE 201
AT1G70920                             EESFIQNHTLTPKQKKDLATFLKLSQRQVEVWFQNRRARSKLKHTEMECE 131
riceP0510C12.22"putative              EESFRLNHTLTPKQKEALAIKLKLRPRQVEVWFQNRRARTKLKQTEMECE 227
                                      *: *  : **.**:*  *:  * *  **:**********:***:**::**
```

FIGURE 19 (continued)

```
glycina                            FLKRCCENLTEENRRLQKEVQELR-ALKLSPQFYMH-------------- 211
homeobox-leucine                   FLKRCCENLTEENRRLQKEVQELR-ALKLSPQFYMH-------------- 98
AT4G16780                          FLRRCCENLTEENRRLQKEVTELR-ALKLSPQFYMH-------------- 226
AT4G17460                          YLKRCVEKLTEENRRLEKEAAELR-ALKLSPRLYGQ-------------- 232
AT5G47370                          YLKRCVEKLTEENRRLQKEAMELR-TLKLSPQFYGQ-------------- 227
AT2G44910                          YLKRCCDNLTEENRRLQKEVSELR-ALKLSPHLYMH-------------- 260
AT3G60390                          YLKRCCENLTDENRRLQKEVSELR-ALKLSPHLYMH-------------- 259
THOM1"                             YLKRCCENLTDENRRLQKEVSELR-ALKLSPQFYMN-------------- 224
craterostigmaCPHB-3                YLRRCCENLTEENRRLQKEVNELR-ALKLSPQFY---------------- 265
TaHDZipII-1"homeodomain-leucin     YMKRCCEQLAEQNRRLEKEVAELR-ALKAAPPAHSAAAAAG--------- 204
Hox2                               YLKRWCERLADENKRLEKELADLR-ALKAAPSPASASAMQPS-------- 218
ricehox1homeodomain-leucine        LLKRCCETLTDENRRLHRELQELR-ALKLATAAAAPHHLYGAR------- 261
Oshox1"homeodomain                 LLKRCCETLTDENRRLHRELQELR-ALKLATAAAAPHHLYGAR------- 261
OSJNBb0089A17.12"homeodomain       LLKRCCETLTDENRRLHRELQELR-ALKLATAAAAPHHLYGAR------- 261
AT5G06710                          YLKRCCESLTEENRRLQKEVKELR-TLKTSTP----------------FY 285
Pphb4"homeodomain-leucine          LLKRCYESLKEENRRLQKELLELR-AIKVAPPCVISHD----------YY 118
Mshb1"type                         YLKRCCETLTEENRRLHKELQELR-ALKTSNP----------------FN 296
AT4G37790                          FLKKCCETLTDENRRLQKELQDLK-ALKLSQP----------------FY 221
AT2G22800                          FLKKCCETLADENIRLQKEIQELK-TLKLTQP----------------FY 208
OJ1781E12.8"Hypothetical           FLKRCCETLTEENRRLQRELQELR-ALKFAPPPPSSAAHQPSPAPPAPFY 238
OSJNOa174H12.5"putative            YLKRCCETLTEENRRLHKELAELR-ALKTARP----------------FY 269
RiceHox7"AAQ55491.1"               HLKRCCERLTRENRRLQREVAELRGALRTTTSSYPPLYGLHHLP------ 202
Oshox7"homeodomain                 HLKRCCERLTRENRRLQREVAELRGALRTTTSSYPPLYGLHHLP------ 176
P0020C11.33"putative               HLKRCCERLTRENRRLQREVAELRGTLRTTTSSYPPLYGLHHLP------ 174
AT2G01430                          YLKRWFGSLTEENHRLHREVEELR-AMKVGP------------------T 232
AT1G70920                          YLKRWFGSLKEQNRRLQIEVEELR-ALKP--------------------- 159
riceP0510C12.22"putative           YLKRCFGSLTEENRRLQREVEELR-AMRVAPPTVLSPHT----------R 266
                                    :::    *  :* **. *  :*: :::

glycina                            -MTPPTTLTMCPSCER---VAVPPSSAVDPATRHHHVPPSHPRAFPIGHG 257
homeobox-leucine                   -MTPPTTLTMCPSCER---VAFPPPLPLILPRVTITCLHLTPGPFPLAHG 144
AT4G16780                          -MSPPTTLTMCPSCEH---VSVPPPQPQAATSAHHRSLPVN------AWA 266
AT4G17460                          -MSPPTTLLMCPSCER---VAGPSSSN--HNQRSVSLSPWL--------- 267
AT5G47370                          -MTPPTTLIMCPSCER---VGGPSSSNHHHNHRPVSINPWVA-------- 265
AT2G44910                          -MTPPTTLTMCPSCER---VSSSAATVTAAPSTTTTPTVVGR-------P 299
AT3G60390                          -MKPPTTLTMCPSCER---VAVTSSSSSVAPPVMNSSSPMG--------- 296
THOM1"                             -MSPPTTLTMCPQCER---VAVSSSSSSSSVVNATRAQNHQA-------P 263
craterostigmaCPHB-3                -MSPPTTLTMCPQCER---VAAQPSSAAAIRPPSHHQRPAAG-------- 303
TaHDZipII-1"homeodomain-leucin     ---PLTTLTMCLSCKR---VASTSS-----ASACDVPN------FSTNAG 237
Hox2                               -SSAAATLTMCPSCRR---VATAGAPHQPNHQQCHPKSNTTI-SSSSTAA 263
ricehox1homeodomain-leucine        -VPPPTTLTMCPSCER---VASAAT---------TTRN-------NSGAA 291
Oshox1"homeodomain                 -VPPPTTLTMCPSCER---VASAAT---------TTRN-------NSGAA 291
OSJNBb0089A17.12"homeodomain       -VPPPTTLTMCPSCER---VASAAT---------TTRN-------NSGAA 291
AT5G06710                          MQLPATTLTMCPSCER---VATSA---------AQPSTSAAH-------- 315
Pphb4"homeodomain-leucine          MPLPAATLTMCPSCER---VATVD---------NRSLTFAKP-------- 148
Mshb1"type                         MQLPATTLTMCPSCER---VATNS---------TATSSVTNT-------- 326
AT4G37790                          MHMPAATLTMCPSCER---LGGGG---------VGGDTTAVD-------- 251
AT2G22800                          MHMPASTLTKCPSCER---IGGGGGGNGGGGGGSGATAVIVD-------- 247
OJ1781E12.8"Hypothetical           MQLPAATLTICPSCER---VGGPA---------SAAKVVAAD-------- 268
OSJNOa174H12.5"putative            MHLPATTLSMCPSCER---VASNPATASTSAPAAATSPAAAP-------- 308
RiceHox7"AAQ55491.1"               -AAAGTVFRVCPSCEHSKVVAAAASESFSPRVFAGGGAPAAITAAAAVPS 251
Oshox7"homeodomain                 -AAAGTVFRVCPSCEHSKVVAAAASESFSPRVFAGGGAPAAITAAAAVPS 225
P0020C11.33"putative               -AAAGTVFRVCPSCEHSKVVAAAASESFSPRVFAGGGAPAAITAAAAVPS 223
AT2G01430                          TVNSASSLTMCPRCER---VTPAAS------PSRAVVPVPAK-------- 265
AT1G70920                          --SSTSALTMCPRCER---VTDAVDNDSNAVQEGAVLSSRSR-------- 196
riceP0510C12.22"putative           QPLPASALTMCPRCER---ITAATGPPAVRPPPSSAAAAAPS-------- 305
                                      . : :  *  *.:   :
```

# FIGURE 19 (continued)

```
glycina                            QALRCPPSQILDSRKQNDKIEKKDRKP-------------------- 284
homeobox-leucine                   QALRCPPSQILDSRKQNDKIEKKDRKP-------------------- 171
AT4G16780                          PATRISHGLTFDALRPRS----------------------------- 284
AT4G17460                          ---QMAHGSTFDVMRPRS----------------------------- 282
AT5G47370                          CAGQVAHGLNFEALRPRS----------------------------- 283
AT2G44910                          SPQRLTPWTAISLQQKSGR---------------------------- 318
AT3G60390                          ---PMSPWAAMPLRQRPAAGSH------------------------- 315
THOM1"                             VP-MNKPWAAMFASKTLDVQRSQM----------------------- 286
craterostigmaCPHB-3                ----MNSWAAMISPRPS------------------------------ 316
TaHDZipII-1"homeodomain-leucin     MGMPMPSPVALPDHRQFFCGYRDTG--ATYGGFSGLAKVVKPAR--- 279
Hox2                               AAVAVAGGNVLPSHCQFFPAAAAAADRTSQSTWNAAAPLVTRELF-- 308
ricehox1homeodomain-leucine        PARPVPTRPWPPAAAQRSSA--------------------------- 311
Oshox1"homeodomain                 PARPVPTRPWPPAAAQRSSA--------------------------- 311
OSJNBb0089A17.12"homeodomain       PARPVPTRPWPPAAAQRSSA--------------------------- 311
AT5G06710                          -NLCLSTSSLIPVKPR-------PAKQVS------------------ 336
Pphb4"homeodomain-leucine          -GFSHLSQSSAAC---------------------------------- 160
Mshb1"type                         -SATINGNNNNKINQ-------------------------------- 340
AT4G37790                          -EETAKGAFSIVTKPR-------FYNPFTNPSAAC------------ 278
AT2G22800                          -GSTAKGAFSISSKPH-------FFNPFTNPSAAC------------ 274
OJ1781E12.8"Hypothetical           -GTKAG--PGRTTTHH-------FFNPFTH-SAAC------------ 292
OSJNOa174H12.5"putative            -TAAARTAVASPEPHRPSSFAALFAAPLGFPLTAAQPRPPPPASNCL 354
RiceHox7"AAQ55491.1"               PGAGSPPSSSAALFGARRPHFGPFAAAVIPPVLRRQPSATS------ 292
Oshox7"homeodomain                 PGAGSPPSSSAALFGARRPHFGPFAAAVIPPVLRRQPSATS------ 266
P0020C11.33"putative               PGAGSPPSSSAALFGARRPHFGPFAAAVIPPVLRRQPSATS------ 264
AT2G01430                          --KTFPPQERDR----------------------------------- 275
AT1G70920                          --MTISSSSSLC----------------------------------- 206
riceP0510C12.22"putative           --PFHPRRPSAAF---------------------------------- 316
```

FIGURE 19 (continued)

# FIGURE 20

```
                   10        20                30        40        50        60        70        80
ATHB1     LPEKKRRLTTEQVHLLEKSFETENKLE--------PERKTQLAKKLGLQPRQVAVWFQNRRARWKTKQLERDYDLLKSTYDQLLSNYDSIVMDNDKL
ATHB3     LGEKKKRLNLEQVRALEKSFELGNKLE--------PERKMQLAKALGLQPRQIAIWFQNRRARWKTKQLERDYDSLKKQFDVLKSDNDSLLAHNKKL
ATHB20    LGEKKKRLQLEQVKALEKSFELGNKLE--------PERKIQLAKALGMQPRQIAIWFQNRRARWKTRQLERDYDSLKKQFESLKSDNASLLAYNKKL
ATHB13    MGEKKRRLNMEQVKTLEKNFELGNKLE--------PERKMQLARALGLQPRQIAIWFQNRRARWKTKQLEKDYDTLKRQFDTLKAENDLLQTHNQKL
ATHB23    MGEKKRRLNMEQLKALEKDFELGNKLE--------SDRKLELARALGLQPRQIAIWFQNRRARSKTKQLEKDYDMLKRQFESLRDENEVLQTQNQKL
ATHB5     AAEKKRRLGVEQVKALEKNFEIDNKLE--------PERKVKLAQELGLQPRQVAIWFQNRRARWKTKQLERDYGVLKSNFDALKRNRDSLQRDNDSL
ATHB6     LSEKKRRLSINQVKALEKNFELENKLE--------PERKVKLAQELGLQPRQVAVWFQNRRARWKTKQLEKDYGVLKTQYDSLRHNFDSLRRDNESL
ATHB16    LSEKKRRLKVDQVKALEKNFELENKLE--------PERKTKLAQELGLQPRQVAVWFQNRRARWKTKQLEKDYGVLKGQYDSLRHNFDSLRRDNDSL
ATHB7     NKNNQRRFSDEQIKSLEMMFESETRLE--------PRKKVQLARELGLQPRQVAIWFQNKRARWKSKQLETEYNILRQNYDNLASQFESLKKEKQAL
ATHB12    KSNNQKRFSEEQIKSLELIFESETRLE--------PRKKVQVARELGLQPRQVAIWFQNKRARWKTKQLEKEYNTLRANYNNLASQFEIMKKEKQSL
ATHB40    GLFRKRKLTDEQVNMLEMSFGDEHKLE--------SERKDRLAAELGLDPRQVAVWFQNRRARWKNKRLEEEYNKLKNSHDNVVVDKCRLESEVIQL
ATHB21    GWFRKRKLSDEQVRMLEISFEDDHKLE--------SERKDRLASELGLDPRQVAVWFQNRRARWKNKRVEDEYTKLKNAYETTVVEKCRLDSEVIHL
ATHB53    GMLRKRKLTDEQVNMLEYSFGNEHKLE--------SGRKEKIAGELGLDPRQVAVWFQNRRARWKNKKLEEEYAKLKNHHDNVVLGQCQLESQILKL
ATHB51    EMIKKKRLTSGQLASLERSFQEEIKLD--------SDRKVKLSRELGLQPRQIAVWFQNRRARWKAKQLEQLYDSLRQEYDVVSREKQMLHDEVKKL
ATHB22    QEKKKKKMTSEQLKFLERSFQEEIKLNPDRKMKLNPDRKMKLSKELGLQPRQIAVWFQNRKARWKNKQLEHLYESLRQEFDIVSREKELLQEELIQL
ATHB54    EITKKRKLTPIQLRLLEESFEEEKRLE--------PDRKLWLAEKLGLQPSQVAVWFQNRRARYKTKQLEHDCDSLKASYAKLKTDWDILFVQNQTL
ATHB52    GKNKKKRLTQDQVRQLEKCFTMNKKLE--------PDLKLQLSNQLGLPQRQVAVWFQNKRARFKTQSLEVQHCTLQSKHEAALSDKAKLEHQVQFL
ATHB17    PPRKKLRLTREQSRLLEDSFRQNHTLN--------PKQKEVLAKHLMLRPRQIEVWFQNRRARSKLKQTEMECEYLKRWFGSLTEENHRLHREVEEL
HAT2      TSRKKLRLSKDQSAFLEETFKEHNTLN--------PKQKLALAKKLNLTARQVEVWFQNRRARTKLKQTEVDCEYLKRCVEKLTEENRRLQKEAMEL
HAT1      TCRKKLRLSKDQSAVLEDTFKEHNTLN--------PKQKLALAKKLGLTARQVEVWFQNRRARTKLKQTEVDCEYLKRCVEKLTEENRRLEKEAAEL
HAT14     STRKKLRLSKDQSAFLEDSFKEHSTLN--------PKQKIALAKQLNLRPRQVEVWFQNRRARTKLKQTEVDCEYLKRCCESLTEENRRLQKEVKEL
ATHB4     GSRKKLRRLSDQALVLEETFKEHSTLN--------PKQKLALAKQLNLRARQVEVWFQNRRARTKLKQTEVDCEYLKRCCDNLTEENRRLQKEVSEL
HAT3      SSRKKLRLSKEQALVLEETFKEHSTLN--------PKQKMALAKQLNLRTRQVEVWFQNRRARTKLKQTEVDCEYLKRCCENLTDENRRLQKEVSEL
ATHB2     NSRKKLRLSKDQALVLEETFKDHSTLN--------PKQKQALAKQLGLRARQVEVWFQNRRARTKLKQTEVDCEFLRRCCENLTEENRRLQKEVTEL
HAT22     SARKKLRLTKQQSALLEDNFKLHSTLN--------PKQKQALARQLNLRPRQVEVWFQNRRARTKLKQTEVDCEFLKKCCETLTDENRRLQKELQDL
HAT9      SARKKLRLTKQQSALLEESFKDHSTLN--------PKQKQVLARQLNLRPRQVEVWFQNRRARTKLKQTEVDCEFLKKCCETLADENIRLQKEIQEL
HADcons       K  Y   QL  LE  F    YL            K  LA  L L   QKEIWFQNRR R K
                      Helix 1                  Helix 2          Helix 3       L      L      L      L      L
```

**Figure 1.** Alignment of the amino acid sequences of HDZip class I and II proteins. Gaps are indicated by dashes. Conserved positions in a HD consensus sequence (Bürglin, 1994) are indicated below the alignment. Residues conserved in both classes are depicted in dark gray, residues conserved only within HDZip I or II are depicted in light gray, and residues showing variation between and within the classes are depicted in white. The three α-helices of the HD and the Leu residues of the Leu Zip are shown below the alignment.

Plant Physiol. Vol. 139, 2005

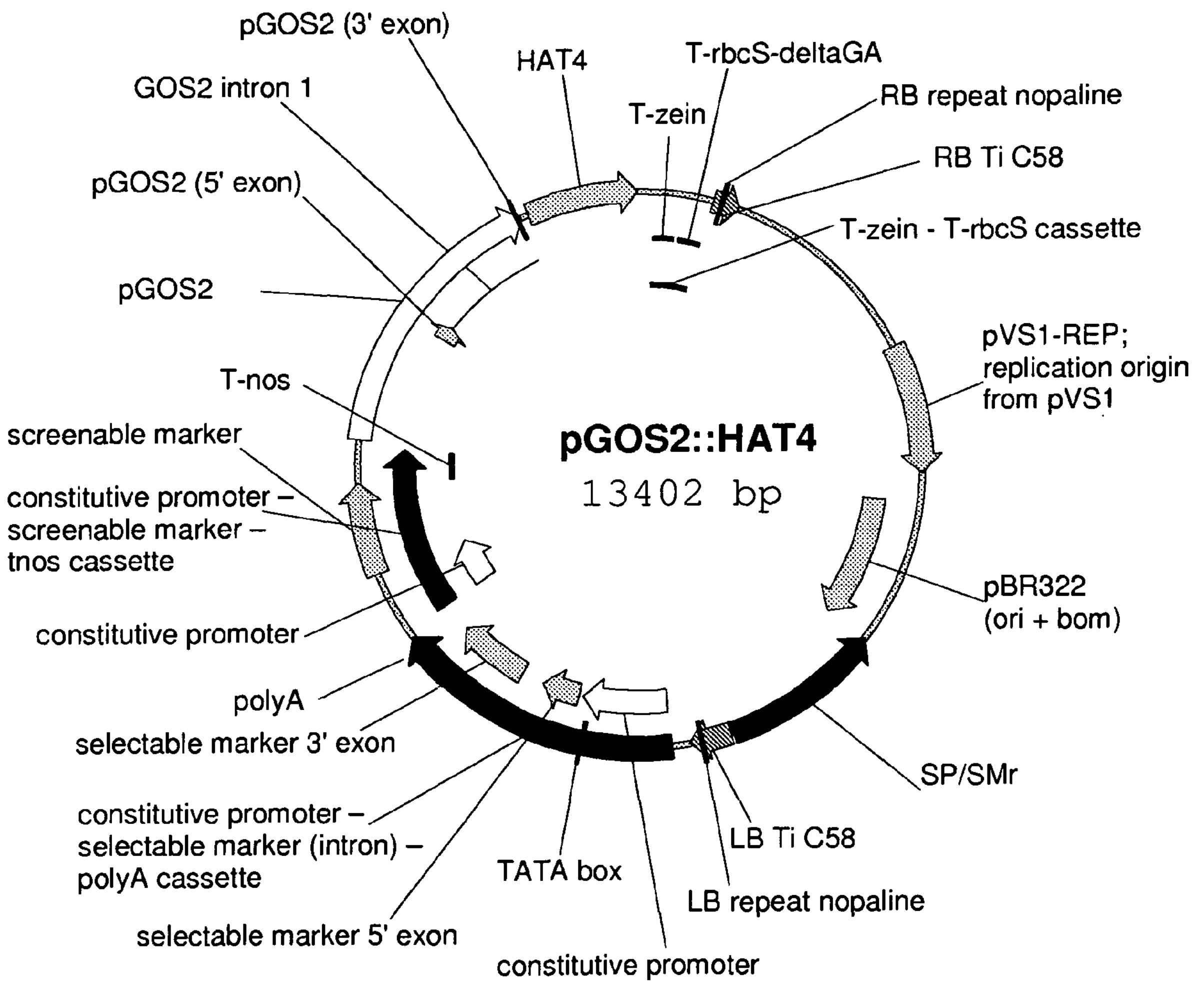
pGOS2 (3' exon)
HAT4
T-rbcS-deltaGA
GOS2 intron 1
T-zein
RB repeat nopaline
RB Ti C58
pGOS2 (5' exon)
T-zein - T-rbcS cassette
pGOS2
pVS1-REP;
replication origin
from pVS1
T-nos
screenable marker
pGOS2::HAT4
13402 bp
constitutive promoter –
screenable marker –
tnos cassette
pBR322
(ori + bom)
constitutive promoter
polyA
selectable marker 3' exon
SP/SMr
constitutive promoter –
selectable marker (intron) –
polyA cassette
LB Ti C58
TATA box
LB repeat nopaline
selectable marker 5' exon
constitutive promoter

FIGURE 21

## SEQ ID NO: 286

MSRP**GDWNCRSCSHLNFQRRDSCQRCGDS**RSGPGGVGGLDFGNFGGRAMSAFGFTTGSDVRP**GDWY CTVGNCGTHNFASRSTCFKCGTF**KDETGAGGGGGGIGGPAMFDADIMRSRVPGNGGRSSWKS**GDWI CTRIGCNEHNFASRMECFRCNAP**RDFSNRTSF

## SEQ ID NO: 308

MASAKVENRGGGGFGSKRSRNDVSVRE**GDWTCPQCGNVNFSFRNVCNRGACGAP**RPSPSLSPRVPP PPAAGYDRPHLGYDRPHLFYGSAGTPPPIPLGSGSYGAPYPHLGLRYGYGPPVGPPASYGLFSSYG QPGPMGSPMGGMGYGPGPELGRYGYGFRGSPMPVSSPWSGGALVENNDSSASRKRRGGPDGMAE**ND WICPKCENVNFSFRNSCNMKKCGAP**RPSPGSNATPCRKDKDAPE**GSWTCPECNNLNYPFRTACNRK GCGSS**RPAAATAN

FIGURE 22

```
                  1                                                                           76
BG450099_1  (1)  ---------MSRPGDWNCRTCNHLNFQRRESCQRCGE--SRMTSGCGAVD---FGGSFLGGRGSSSPFPFT--TGP
DR459119_1  (1)  ---------MSRPGDWNCRSCQHLNFQRRDSCQRCG---EFRSGDHFGSY---G----GGRGGSSFGFA----TGS
SEQIDNO294  (1)  ---------MNRPGDWNCRLCSHLNFQRRDSCQRCR-----EPRPGGIST---DLLSGFGGRPVSSSFGFN--TGP
DT494117_1  (1)  ---------MSRPGDWNCRSCQHLNFQRRDSCQRCG---DPRPGERDHYG---SFGG--RSSGGSFGF-----TGP
DV465465B   (1)  ---------MSRPGDWNCRSCQHLNFQRRDSCQRCG---DPRPGERDHYG---SFGG--RSGGSF-GF-----TGP
DT457997_1  (1)  ---------MSRPGDWNCRSCQHLNFQRRDSCQRCG---EPRPGGGDRGG---DYGSFGGRGGSSFGF-----TGP
SEQIDNO288  (1)  ---------MSRPGDWNCRSCQHLNFQRRDNCQRCG---ESRYGVRVGST---F----GFTAGSD-----------
SEQIDNO300  (1)  ---------MSRPGDWNCRSCSHLNFQRRDSCQRCGD-VRPDGRGGGGGG---GDFGSSFGGRSGGSPFGGGFAGP
DV143669_1  (1)  ---------MSRPGDWNCRSCQHLNFQRRDSCQRCG---DSRSGTGGSGA---DFGGFGSRVGSSFGFS----TGS
SEQIDNO298  (1)  METKAAAMAMRKPGDWSCRSCQYVNFCKREACQRCGE----AKLGVERTD--------YAAMGGGWE---------
DT496999_1  (1)  ---------MNRPGDWNCRSCQHLNFQRRDSCQRCG---DPRSAGDF-GG---F----GGRGGSSLGF-----TGS
BG238374B   (1)  ---------MSRPGDWNCRSCQHLNFQRRDSCQRCG-----DSKYGDRVV---DFGGFGGRGGSSFGL-----TGS
SEQIDNO290  (1)  --------MNRKPGDWDCRACQHLNFSRRDICQRCSEPRGVADRGSGGGGGG-DYASFGGRGGSSFGGGFG-AAGS
DN152082_1  (1)  --------MNRKPGDWDCRACQHLNFSRRDLCQRCGEPRGAADRGSGGGG---DYANFGGRGGSSFGGGFG-A-GS
SEQIDNO292  (1)  --------MNRKPGDWDCRACQHLNFSRRDLCQRCGEPRGAADRGSGGGG---DYANFGGRGGSSFGGGFG-T-GS
SEQIDNO286  (1)  ---------MSRPGDWNCRSCSHLNFQRRDSCQRCG---DSRSGPGGVGG---LDFGNFGGRAMSAFGF-T--TGS
DT581158_1  (1)  --------MNRKPGDWNCRSCQHLNFSRRDSCQRCG---DPRSIGSERSD----YPGFVGGRGGS-SFGFS---GS
DW105125_1  (1)  ---------MSRPGDWNCRSCQHLNFQRRDSCQRCGE---TRYGGGGGVF---------GGRGSIISPSAFGFTGP
DV221228_2  (1)  ---------MSRPGDWNCRSCQHMNFQRRDSCQRCG---DPKSGGGDFGS---F----GGRGGSSFGF-----TGS
SEQIDNO296  (1)  ------MNIQRKPGDWNCKSCQHLNFSRRDYCQRCHT-PRQDLPLGDGYVP--------GGVLSS----------L
BQ471337_1  (1)  --------MNRKPGDWDCRSCQHLNFSRRDLCQRCGEPRSAADRGSVGGALGGDYANFGGRGGGGSSFGAGFGAGS
Consensus   (1)           MSRPGDWNCRSCQHLNFQRRDSCQRCG         G                GG GGSS GF     TGS
```

FIGURE 23 A

```
                     77                                                                          152
BG450099_1    (61)  DVRPGDWYCTVGNCGAHNFASRSSCFKCGAPKDIDTFSSD------SSDMPRLLRSPYGFGAGSAGGGAST-----
DR459119_1    (54)  DVRPGDWYCTAGNCGTHNFASRSSCFKCGAFK---DDPA--------GGFDSD--VPRSRGFGGGNRSG-------
SEQIDNO294    (58)  DVRPGDWYCNLGDCGTHNFANRSSCFKCGAAKDEFSCSSAAATTGFMDMNVGPRRGLFGFGGSSSGGGGTG-----
DT494117_1    (55)  DVRPGDWYCTAGNCGAHNFASRSSCFKCGVSKDE--SSGG------GLDADMSRMRGYGFGGGGGGGSGSS-----
DV465465B     (54)  DVRPGDWYCSVGNCGAHNFASRSSCFKCGMSKDE--SSGG------GLDADISWMRGYGFGGGSASSRSN------
DT457997_1    (57)  DVRPGDWYCTVGNCGAHNFASRSSCFKCGAAKDE--SSGG------FESDIPRMRG-YGFSTGSSSRSN-------
SEQIDNO288    (47)  -VRPGDWYCTAGNCGTHNFASRSTCFNCGAFK---DESA--------GGFDLD--MSRSRGFGGNRSG--------
SEQIDNO300    (64)  DVRPGDWYCSIGNCGAHNFASRSSCFKCGA-YKEEAGCGDS-----MGRSRGGFSFGGIG--GGGSGAA-------
DV143669_1    (58)  DVRPGDWYCTAGNCGAHNFASRASCFKCGVYK---DDSGA------PGGFDSDILRASRGFGSGSNRSS-------
SEQIDNO298    (56)  -VKPGDWCC--RCCAVNNYASRGSCFKCGA------AKND-------SAAAVAQGWGF----SVASQAG-------
DT496999_1    (52)  DVRPGDWYCTAGNCGAHNFASRSSCFKCGVYKEMDSAGGF------DSDFSRT--RGFGGSTGGGNRSG-------
BG238374B     (55)  DVRPGDWYCAAANCGAHNFASRSSCFKCGAFK----DDLA------GGGYNSSDILRSRAFGGSGRPG--------
SEQIDNO290    (67)  DVRPGDWYC---SCGAHNFASRSSCFKCSA-YKEEAAVNSG-----AGGFDGDMSRSRG--YGFGSGAAAAAGAGA
DN152082_1    (64)  DVRPGDWLC---SCGAHNFASRSNCFKCSA-FKEEAAVNSG-----AGGFDGDMSRSR---YGFGGGAAR------
SEQIDNO292    (64)  DVRPGDWYC---NCGAHNFASRSSCFKCAAFKDDAAVNSGG-----AGAFDGGDMSRSRG-YGFGSGAVRA-----
SEQIDNO286    (59)  DVRPGDWYCTVGNCGTHNFASRSTCFKCGTFKDETGAGGG------GGGIGGPAMFDADI-MRSRVPGNGG-----
DT581158_1    (58)  DVRPGDWYC---QCGAHNFASRSSCFKCNAFKDESAASGGL-----DGGDMLRSRGFGF---GGGGGARS------
DW105125_1    (56)  DVRPGDWYCNVGNCGAHNFASRSSCFKCGAFKDDLACSGGG--GGGGVFDGDMSRGRGFGFGGGSGGGGGS---
DV221228_2    (53)  DVRPGDWYCNAGNCGAHNFASRSNCFKCGAFK-DESAGGY------DSDMSRS--RGFGFG-GGSGRSG-------
SEQIDNO296    (52)  DIRPGDWYC---NCGYHNFASRASCFKCGAIVKDLPAGQG------GGVANGDFARALD---SSAVRAG-------
BQ471337_1    (69)  DVRPGDWYC---TCGAHNFASRSSCFKCAA-FKEEAAVNGG-----AGGFDGDMSRSRGFGFGAVGGMGGGMGAGA
Consensus     (77)  DVRPGDWYC   GNCGAHNFASRSSCFKCGA K                G               G
```

```
                     153                                                                    226
BG450099_1    (126) ------RPGWKSGDWICTRSGCNEHNFANRMECYRCNGPRDSSTGRSSYLSXTFSIWFDVQXEIIXRGYXISXR
DR459119_1    (110) ---------WKSGDWICTRSGCNEHNFASRMECFRCSAPRDFTARTSY--------------------------
SEQIDNO294    (129) ------RSPWKSGDWICPRSGCNEHNFASRSECFRCNAPKELATEPPY--------------------------
DT494117_1    (118) ------RN-WKSGDWICTRSGCNEHNFASRTECYRCNAPRESSSNKSSY-------------------------
DV465465B     (116) ---------WKSGDWICTRSGCNEHNFASRTECYRCNAPRESGSNKSSY-------------------------
DT457997_1    (117) ---------WKSGDWICTRSGCNEHNFASRMECFRCNAPRDSTHKSSY--------------------------
SEQIDNO288    (101) ---------WKSGDWICTRLGCNEHNFASRMECFRCSAPREFNNRTSY--------------------------
SEQIDNO300    (125) ----TGRSGWKSGDWICTRSGCNEHNFASRTECFRCREPRDSG--NAMLKEVPC--------------------
DV143669_1    (118) ---------WKSGDWICTRWGCNEHNFASRMECFKCSAPRDLSNRTSY--------------------------
SEQIDNO298    (105) ---------WKNGDWICPRMECNVQNYANRTECFRCNFPRYYVD------------------------------
DT496999_1    (113) ---------WKSGDWICTRWGCNEHNFASRMECFKCNAPRDLSNRTSY--------------------------
BG238374B     (113) ---------WKSGDWICSRSGCNEHNFASRMECFKCSAPRDTY-------------------------------
SEQIDNO290    (132) ART-TNRPGWKSGDWICTRSGCNEHNFASRMECFRCNAPRDSG--TEV--------------------------
DN152082_1    (122) ----TNRPGWKSGDWICTRSGCNEHNFASRMECFRCNAPRDSG--TEV--------------------------
SEQIDNO292    (126) -----SRPGWKSGDWICTRSGCNEHNFASRMECFRCNAPRDSG--TEV--------------------------
SEQIDNO286    (123) ------RSSWKSGDWICTRIGCNEHNFASRMECFRCNAPRDFSNRTSF--------------------------
DT581158_1    (117) --------CWKSGDWICNRSGCNEHNFASRMECFRCNAPRDSG--TEV--------------------------
DW105125_1    (127) -----SRSGWKSGDWICGRPGCNEHNFASRMECFRCNAPRESGNKSPY--------------------------
DV221228_2    (112) ---------WKSGDWICSRSGCNEHNFASRMECFRCNAPRDLSNKTSY--------------------------
SEQIDNO296    (109) ---------WKAGDWICTRPGCNVHNFASRIECYRCNAPREAGNVK----------------------------
BQ471337_1    (136) AGGRASRPGWKSRDWICTRSGCNEHNFASRQECFRCNAPRDSGSATPY--------------------------
Consensus     (153)          WKSGDWICTRSGCNEHNFASRMECFRCNAPRDS    T
```

FIGURE 23 A (continued)

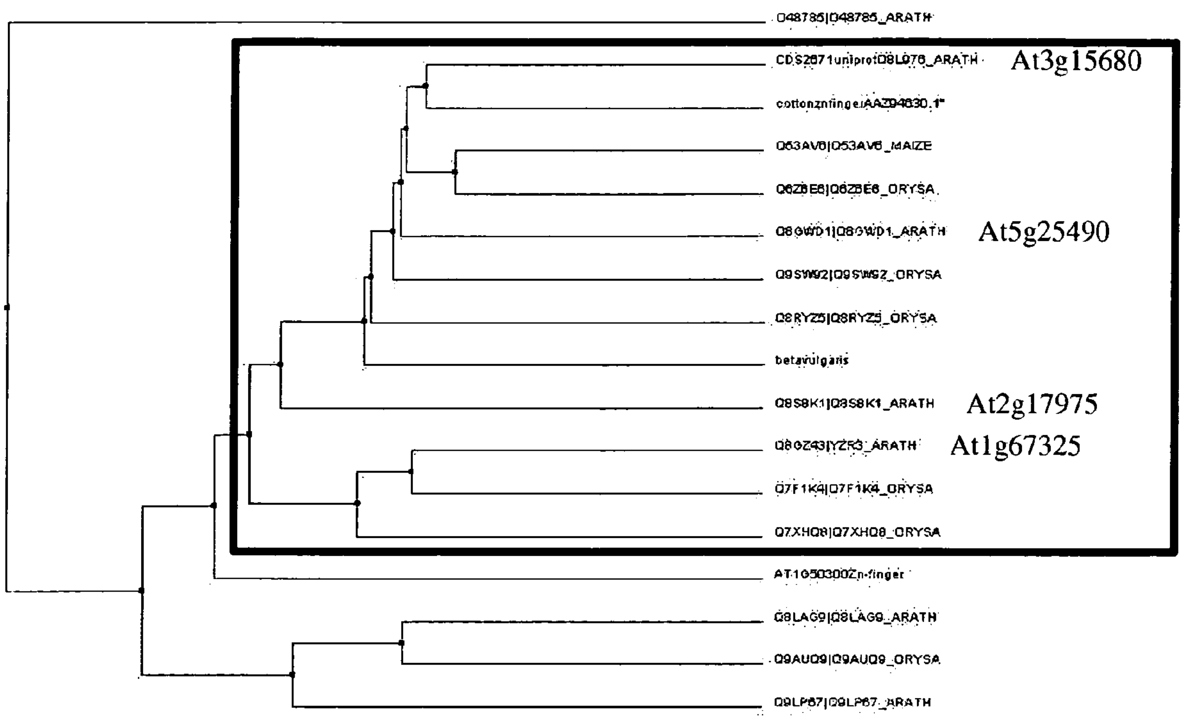


**FIGURE 23 B**

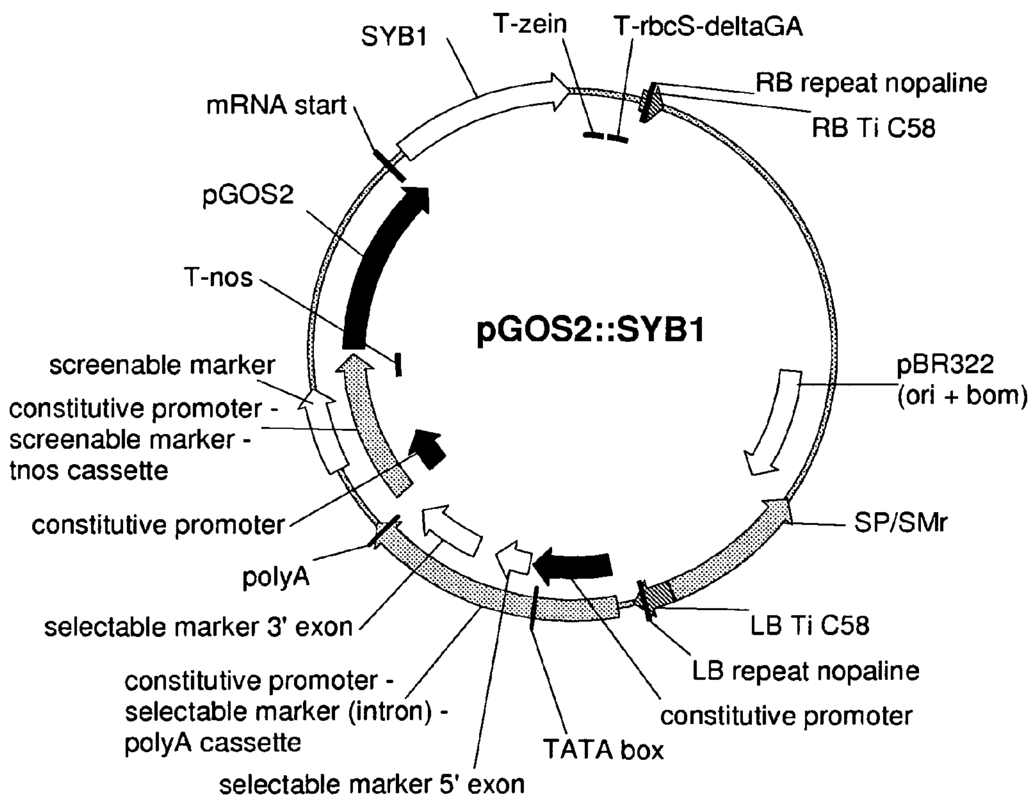


**FIGURE 24**

**SEQ ID NO: 1, AtAZ, At2g41900**

ATGTGCTGTGGATCAGACCGATTAAACCAGATCGTGTCATCAAGATCTTCGTTGCCAATTTCTTTC
GAGGAAGATAACAATCTTGTTACCAACACAGACATGAATCACTTAACAGTCGAAACAGAGGATACG
TTTGCGAGCTTGCTTGAGCTTGCAGCTAACAACGATGTTGAAGGTGTAAGGCTATCTATCGAGAGA
GACCCTTCTTGTGTAGACGAAGCTGGTCTCTGGTACGGTCGTCAAAAAGGTTCTAAAGCTATGGTC
AACGATTACAGGACTCCGTTGATGGTTGCTGCTACTTACGGAAGCATTGATGTGATCAAGCTTATT
GTTTCTTTGACTGATGCTGACGTGAACCGTGCTTGCGGGAATGATCAGACCACTGCGTTACACTGC
GCTGCTTCTGGAGGAGCTGTGAATGCTATCCAAGTTGTTAAGCTGCTTCTTGCAGCTGGAGCTGAT
TTGAATCTGTTGGATGCTGAAGGTCAACGAGCTGGTGATGTTATTGTTGTTCCTCCTAAGCTTGAA
GGCGTGAAGCTGATGCTTCAGGAGCTTCTTTCTGCTGATGGATCATCTACTGCGGAGCGGAATCTA
CGGGTTGTGACAAATGTTCCGAATAGAAGCTCATCTCCGTGTCATTCTCCTACTGGAGAGAATGGT
GGATCAGGGTCTGGTTCACCGCTCGGCTCTCCTTTTAAGCTGAAATCTACTGAATTCAAGAAAGAG
TATCCGGTTGATCCGTCTTTGCCAGATATCAAGAACAGTATCTACGCGACTGATGAGTTTAGAATG
TATTCCTTCAAGGTCCGGCCTTGCTCTCGTGCTTATTCACATGATTGGACTGAGTGTCCTTTTGTT
CACCCGGGTGAAAACGCGAGGAGGAGAGACCCGAGGAAGTTCCATTACAGCTGCGTTCCTTGCCCG
GATTTTAGGAAAGGAGCTTGTAGGAGAGGAGATATGTGTGAGTATGCGCACGGTGTGTTTGAATGC
TGGCTTCATCCGGCTCAGTACAGGACCCGTCTTTGCAAAGATGGAACAGGCTGTGCTCGGCGGGTT
TGTTTCTTTGCGCATACACCCGAGGAGCTTCGACCTTTGTACGCATCAACTGGTTCAGCGGTTCCT
TCGCCTAGATCGAATGCTGATTATGCAGCTGCTTTGAGTCTCCTTCCTGGTTCTCCATCAGGAGTC
TCTGTCATGTCCCCGCTTTCCCCATCAGCAGCGGGGAACGGAATGTCTCATTCGAATATGGCTTGG
CCACAACCAAATGTCCCTGCGTTGCACTTACCAGGAAGCAATCTACAGTCAAGCAGGCTAAGGTCT
TCTCTCAATGCAAGGGATATCCCGACGGATGAGTTCAATATGTTAGCGGATTACGAGCAGCAGCAA
CTCCTCAACGAGTATTCCAATGCTCTGAGCCGTTCTGGTCGGATGAAATCAATGCCTCCTTCGAAT
CTTGAAGATCTTTTCTCAGCAGAAGGCTCTTCATCTCCCCGGTTCACTGATTCCGCTTTAGCTTCC
GCGGTGTTCTCGCCTACACACAAGTCAGCTGTCTTCAACCAGTTCCAACAACAGCAACAGCAGCAG
CAGAGCATGTTGTCTCCAATCAACACAAGCTTTTCTTCACCAAAGAGCGTTGACCACTCATTGTTT
TCAGGTGGAGGAAGAATGTCTCCTCGGAATGTTGTTGAACCAATATCACCCATGAGTGCTCGGGTT
TCCATGTTGGCTCAGTGCGTGAAGCAACAACAACAGCAACAGCAGCAGCAGCAGCAGCAACATCAG
TTCCGTAGCCTTAGCTCCAGAGAGCTCAGAACAAACTCTAGCCCAATCGTTGGTTCACCGGTAAAC
AACAACACATGGTCATCAAAATGGGGATCTTCAAATGGTCAACCGGATTGGGGAATGAGCTCAGAA
GCACTTGGTAAGTTGAGATCTTCGTCATCGTTTGATGGTGATGAGCCTGATGTGTCATGGGTCCAG
TCACTGGTGAAGGAGACTCCAGCAGAAGCCAAAGAGAAAGCAGCAACATCTTCCTCAGGGGAACAC
GTGATGAAGCAGCCAAATCCGGTTGAACCGGTAATGGATCATGCTGGGCTAGAAGCTTGGATTGAG
CAAATGCAGCTCGATCAGCTTGTGGCTCAGCAGAATTGA

**SEQ ID NO: 2, AtAZ translated, At2g41900**

MCCGSDRLNQIVSSRSSLPISFEEDNNLVTNTDMNHLTVETEDTFASLLELAANNDVEGVRLSIER
DPSCVDEAGLWYGRQKGSKAMVNDYRTPLMVAATYGSIDVIKLIVSLTDADVNRACGNDQTTALHC
AASGGAVNAIQVVKLLLAAGADLNLLDAEGQRAGDVIVVPPKLEGVKLMLQELLSADGSSTAERNL
RVVTNVPNRSSSPCHSPTGENGGSGSGSPLGSPFKLKSTEFKKEYPVDPSLPDIKNSIYATDEFRM
YSFKVRPCSRAYSHDWTECPFVHPGENARRRDPRKFHYSCVPCPDFRKGACRRGDMCEYAHGVFEC
WLHPAQYRTRLCKDGTGCARRVCFFAHTPEELRPLYASTGSAVPSPRSNADYAAALSLLPGSPSGV
SVMSPLSPSAAGNGMSHSNMAWPQPNVPALHLPGSNLQSSRLRSSLNARDIPTDEFNMLADYEQQQ
LLNEYSNALSRSGRMKSMPPSNLEDLFSAEGSSSPRFTDSALASAVFSPTHKSAVFNQFQQQQQQ
QSMLSPINTSFSSPKSVDHSLFSGGGRMSPRNVVEPISPMSARVSMLAQCVKQQQQQQQQQQQHQ
FRSLSSRELRTNSSPIVGSPVNNNTWSSKWGSSNGQPDWGMSSEALGKLRSSSSFDGDEPDVSWVQ
SLVKETPAEAKEKAATSSSGEHVMKQPNPVEPVMDHAGLEAWIEQMQLDQLVAQQN

**FIGURE 25**

**SEQ ID NO: 3, motif 1**
(P/A)CSRAY(S/T)HDWTEC

**SEQ ID NO: 4, motif 2**
HPGENARRRDPR

**SEQ ID NO: 5, motif 3**
HG(V/I)FE(C/S)WLHP(A/S)QY(R/K)TRLCK

**SEQ ID NO: 6, motif 4**
CFFAH

**SEQ ID NO: 7, prm06717**
GGGGACAAGTTTGTACAAAAAAGCAGGCTTAAACAATGTGCTGTGGATCAGACC

**SEQ ID NO: 8, prm06718**
GGGGACCACTTTGTACAAGAAAGCTGGGTGGTTAGGTCTCTCAATTCTGC

**SEQ ID NO: 9, wsi18 promoter**
AAGAGGCAAGAGCATCCGTATTAACCAGCCTTTTGAGACTTGAGAGTGTGTGTGACTCGATCCAGC
GTAGTTTCAGTTCGTGTGTTGGTGAGTGATTCCAGCCAAGTTTGCGATGGCTTCTCAGCAGGAACG
GGCTAGCTACCACGCCGGCGAGACCAAGGCCCGCGCCGAGGAGAAGACGGGGCGCATGATGGGCAC
GGCGCAGGAGAAGGCGCGGGAGGCCAAGGACACGGCGTCCGACGCCGCGGGGCGCGCGATGGGCAG
GGGACACGGCGCCAAGGAGGCGACCAAGGAGAAGGCGTACGAGACCAAGGACGCGACCAAGGAGAA
GGCGTACGAGGCAAAGGACGCGGCCTCCGACGCCACCGGCCGCGCCATGGACAAGGGCCGCGGCGC
CGCGGGCGCCACGAGGGACAAGGCGTACGATGCCAAGGACAGGGCGGCTGACACGGCGCAGTCCGC
CGCCGACCGCGCCCGCGACGGCGCCGGGCAGACCGGGAGCTACATTGGACAGACCGCCGAGGCCGC
CAAGCAGAAAGCGGCCGGCGCCGCGCAGTACGCCAAGGAGACCGCGATCGCCGGCAAGGACAAGAC
CGGCGCCGTGCTCCAGCAGGCAGGGGAGCAGGTGAAGAGCGTGGCGGTGGGGGCGAAGGACGCGGT
GATGTACACGCTCGGGATGTCAGGCGATAACAAGAACAACGCCGCTGCCGGCAAGGACACCAGCAC
CTACAAGCCTGGAACTGGGAGTGACTACCAGTAATACGGTAGAAGAAGCATGTGTCGTCTTTGGCA
CTGATGCCAAAGTGTACGTGTTGTATCCTCTTTTTTAAGTTTCAGCTCGACTTCGACGTGTTCGGT
GTCACACTTTGGTTTTTCAGTTGTGCTCAACTGTTCATGTTTCTGGTTCCATGGAGGGCCAGTGTG
GAGGTCAATGTTTAAGCTTTCGTTTTAAAATCTGATAATAAAGTTGGTTAAGACCTG

**SEQ ID NO: 10, *Eucalyptus grandis* transcription factor cDNA C3H-type family**
GGAAGACGAAGAGCAACAAAATAGGTCTCACTCCCTCCTCTCTCCTCTCTCCTCTCTCTTCTTTCT
CTCTCTTCTGCTTCTAACAAAGTCTCTTCCTTGAGAGACAGGGCTGCGTCGTCGTCTTTCTCTCTC
CTCGCTGCGAGCTTCTGAGAAAGTTCAATTCTTTTTCTCTTGTTCTCTCTCTCTACCCTTCTGGGT
ACCACTGTGAAGCTCCGGTCTTTTCTATTTTTTTTTTTTTTTGGGCTATCTGGGTCTGGGCAAATCC
ATCGCGCGCTCTGCTCTGGACTGAGAGGCCGTCAGTGGCTTTAGATCTGCGACGCCTCTTGCTTGC
TCAGTGAGCTGGGCTAGTTCAAATCGACGAAGAAAGCATGCGCTAGTGATTGGTGTGGGTAATACA
CTGCATTCGATCTCTACTAAGTATCCCCAAGTATACTAAGATCCCGTTCTCAGCCATGAGTCAACT
GACCATTCAGACTGAGGACACTTTTGCCAGCTTGCTTGAGCTTGCTGCTAACAACGACACAGAATC
TTTCGGACGGTGTGTGGAACGTGATCCTTCGAGCATAGATGAAATTGGATATTGGTATGGTCGCCA
AAAGGGTTCGAAGCAGGTGGTCAATATGCAAAGAACTCCTCTTATGGTGGCTGCTACATATGGTAG
TGTTGATGTAATGAGACTCATTCTTTGCCTATCTGATGCTGATGTGAATCGAACCTGCAGCACAGA

**FIGURE 25 (continued)**

CAAGAGCACAGCCCTTCACTGTGCTGCCTCTGGTGGTGCTGTGAATGCTGTAGATGCTGTGAGGCT
ACTCCTGTCAGCTGGTGCTGACCCAAGTTTAGCAGATGCTAACGGTCAGCGGCCTGTGGATGTTAT
TGTTGTTCCTCCAAAGCTCCTTTCAATAAAGTTTGCTCTTGAAGAGCTCTTGTCGACCGAAGGATC
TGTAAATGAACACAATCTGAGAGTGTCCGTAGCCACTTCCAATTCAACCTCTCCCCCACTTTCATC
TTCCCCGGATAATGGTTCCCCAGCATCTGCTAATTGTTCTTCCCCCAAGAACTCAAAGTTAAGTGA
TGCCCCTGTTCTTTATGCATCAGAAAAGAAGGAATACCCGGTGGATCCATCTCTTCCAGATATCAA
GAATAGCATTTACTCAACAGATGAATTCCGAATGTATTCTTTTAAAGTGCGGCCTTGTTCACGAGC
GTACTCGCATGATTGGACGGAGTGCCCTTTTGTTCATCCAGGGGAGAATGCCCGTAGAAGGGATCC
AAGGAAGTTCCACTACAGCTGTGTCCCTTGCCCTGATTTCCGGAAGGGTGCTTGTAGACGTGGAGA
TATGTGTGAATATGCTCATGGTGTTTTTGAGTGCTGGCTCCATCCTGCTCAGTATCGGACTCGATT
ATGCAAGGATGGTACAAGTTGTGCTCGGAGAGTGTGCTTCTTTGCCCACACGGAGCAAGAGCTGCG
TCCATTGTACGTCTCCACTGGTTCTGCTGTTCCGTCTCCTCGCTCGAGTACCTCTGGAGCTGCTGC
CATGGATTTTGCTGCAGCCATGAGCCTCTTACCTGGTTCCCCATCATCAGTATCCATCATGTCCCC
TTCACCCTTCACTCCTCCCATGTCTCCATCTGCTAATGGTATTTCTCACCCATCTGTTGCCTGGCC
CCAGCAAAATGTACCAACTTTGCATCTTCCCGGAAGCAATCTTCAGTCCAGCCGCTTGAGATCTTC
TCTTAATGCAAGAGATATTCCTCAGGAGGATTTTGACTTGCTGTCAGATTATGATGTGCAACAGCA
GCAGCTCCTAAATGAGTTTTCCATCCTTTCACAACAATCGATGGGTGCTAATTCCTTGAACCGTTC
TGGTCGGCTGAAAACTTTGACCCCCTCAAACCTTGATGATCTCTTCTCTGCTGAGAGCTCATCCCC
TCGCTACGCTGATCAAGCCCTGGCTTCTGCTGTTTTTCACCAACGCACAAATCTGCAGTAATCAA
TCAATTTCAGCAGCAGCAGCAGAGCATGTTATCACCCATCAACACAACCTTCTCTCCTAAGAGTGT
CGACCACCCTTTGTTGCAAGCGTCTTTCGGTGTTCAATCTGGGCGAATGTCCCCTCGTAACATGGA
TCCCATCTCTCCTATAAGTTCTCGTGTGTCGATGTTGGCCCAACGAGAGAAACAGCAACAGCAATT
ACGCAGCCTAAGCTCTCGTGAACTCGGTTCCAATTCAGCCGCCATTGTGGGTTCCCCCGTGGGTTC
TTGGTCGAAATGGGGAGCTACAAATGGGAAACCAGACTGGGCTGTTAGTGCAGATGAACTAGGTAA
GCTTCGCAGGTCTAATTCATTTGAGCTTGGGAACAATGGTGAGGAGCCAGATCTTTCATGGGTTCA
ATCCCTCGTTAAAGAATCTCCTACCGAGATGAAAGAAAAGCTTTCGTCAACTCTCTCTGGTGTTCC
AGCCCCCGCTACATCCAGTGAGGTTCCGAGTATCAGCTCGCAGATGGAATCGGTTGATCACGAAGT
GCTAGGAGCATGGCTCCAGCAGATGCAGCTCGATCCGCTCGTGGCTCAGCAAAACTAGGTTGTTTT
TTTTCCTACATGGCCTTGAGGAAGTAGACAGCGGAAAGTTTTTTTTGGTAAATACTATGTTTTTTC
TGGAAATTTTTGATGCTGGGGGTGGGGTCTGGAAGAAGATAACAAGGCAGGAAAGGGGTCAGTGAA
GTCACTGGAGAAAAGGAATTCATTTTTAACCATTTTATCATTCTATTACAACAGAAAGTAGGGAAA
AAAAAGGAAGACCCTCTGGGTTATGAAGAGAAATTAAACCCAGGCTAGGCGTTCTCCTTTCTAATA
TTTCCAATTTTAGGTCCATATTACTGTCATTTCCTTTTTGCCGTCTTATCATATTTCATCAAAATG
GAACTGGGGACTAATGTTTGTTCCATTCTTTCGCTCTTCTGATTTATTTGCACCCTTGGGGTAAGA
TCAAAAGAGAAATTATGATCATTTTCTTTTGAGGATATTTTTTTTTCCCAATATTTGTGAGAATGA
AAGTTAAGAGGGGATATGATGTGTCTGGTGTTGTAGTATGAAAAACCAATAACCGAGTTCACCTGT
TGCTGCTGGTGGTAGAAGAAGTGGAGAAGAAGCTATGATCCTTTGATGTAACAGTCAATCAAACAT
TTTAATACCTTTATTTTTTGTTTCCTCATGTAATCCATCCTTTGTGATTGTCCTCTCTCTCTCTCT
CTCTCTCTCTCTCTCCCTCCCCGTGTTCTTTCTTCATAAGCGTCTTGCTTGTCGATCTGTAAATTA
TTGAAAAGGGTCATGGAAAGCCGTGCCGGTGTGGATTCTCATTTTTGCAAAAAAAAAAAAAAAAAA
AAAAAA

**SEQ ID NO: 11, *Eucalyptus grandis* transcription factor protein C3H-type**

MSQLTIQTEDTFASLLELAANNDTESFGRCVERDPSSIDEIGYWYGRQKGSKQVVNMQRTPLMVAA
TYGSVDVMRLILCLSDADVNRTCSTDKSTALHCAASGGAVNAVDAVRLLLSAGADPSLADANGQRP
VDVIVVPPKLLSIKFALEELLSTEGSVNEHNLRVSVATSNSTSPPLSSSPDNGSPASANCSSPKNS
KLSDAPVLYASEKKEYPVDPSLPDIKNSIYSTDEFRMYSFKVRPCSRAYSHDWTECPFVHPGENAR

## FIGURE 25 (continued)

RRDPRKFHYSCVPCPDFRKGACRRGDMCEYAHGVFECWLHPAQYRTRLCKDGTSCARRVCFFAHTE
QELRPLYVSTGSAVPSPRSSTSGAAAMDFAAAMSLLPGSPSSVSIMSPSPFTPPMSPSANGISHPS
VAWPQQNVPTLHLPGSNLQSSRLRSSLNARDIPQEDFDLLSDYDVQQQQLLNEFSILSQQSMGANS
LNRSGRLKTLTPSNLDDLFSAESSSPRYADQALASAVFSPTHKSAVINQFQQQQSMLSPINTTFS
PKSVDHPLLQASFGVQSGRMSPRNMDPISPISSRVSMLAQREKQQQQLRSLSSRELGSNSAAIVGS
PVGSWSKWGATNGKPDWAVSADELGKLRRSNSFELGNNGEEPDLSWVQSLVKESPTEMKEKLSSTL
SGVPAPATSSEVPSISSQMESVDHEVLGAWLQQMQLDPLVAQQN

**SEQ ID NO: 12, Q2QPW2_ORYSA Rice zinc finger C-x8-C-x5-C-x3-H type family protein, coding sequence**
ATGGGGGAGCCTGGGGGCGCCGAGGCGGCCGTCTCCGCGAGGCTGCTCGAGCTGGCGGCCGACGAC
AACGCGGCGGGGCTCGGGGAGCTCCTCGCGGCGTGGCCCTCCCTCGCCGACGAGCCCGCGCCGTGG
TACACCCCGGCGCGGGGCGCGGAGCCGCTGACCCCGCTCATGGTCGCCGCCGTGTACGGCTCGGTG
GGCTGCCTCGACGCGCTCCTCTCGCCGCCCTACCTCGTGGACCCCAACCGCGCCTCGGCGTCGTCG
CTCTCCACCCCGCTCCACCTCGCCGCCGCGGGCGGGTCCGCCTCCGCCCCCGCGGCGGTCTCCCGC
CTCCTCGCCGCCGGCGCCGACCCGGCCCTCCTCGACCACCTCCAGCGCCGGGCGTCCGACCTCGTC
GCGCTCCCGCCCAACTCGCTCCCGCTCAAGAACCACCTCCTCTCCCTCCTCGGCGCCCGCAAGGAG
TGGCCTCCCGACCCCTCCCTCCCCGACATCAAGAACGGCGCCTACGCCTCCGACGACTTCAGGATG
TACTCGTTCAAGGTGCGCGCGTGCTCGCGGGCCTACTCCCATGACTGGACGGAGTGCCCCTTCGTC
CACCCCGGCGAGAACGCGCGGCGGCGCGACCCGAGGAAGTACCACTACAGCTGCGTGCCGTGCCCG
GAGTTCAAGAAGGGGGCCGGGTGCAGGAGAGGGGACATGTGCGAGTACGCGCACGGGGTGTTCGAG
AGCTGGCTCCACCCGGCGCAGTACCGGACGCGCCTCTGCAAGGACGGCGTCGGCTGCGCCCGCCGC
GTCTGCTTCTTCGCCCACACGCCCGACGAGCTCCGCCCGCTCTACGTCTCCACGGGCTCCGCCGTG
CCGTCGCCGCGCGGGGCGTTGGAGATGGCGGCGGCGGCGGCGGCGATGGGGATGGGGCTGTCGTCG
CCGGGGTCGTCGTCGTTCACGCCGCCGCTATCGCCGTCGGCCGGCGGGGGCGGGGGCGGGGGCGGG
GGCAGCGGCGGCGGCGGCGCGTGGCCGCAGCAGCCGAGCGTGCCGGCGCTCTGCCTGCCCGGGAGC
GCCGGGAACCTCCACCTGAGCCGGCTGCGCACGTCGCTGAGCGCGCGCGACATGGCCGTCGACGAG
CTGCTCGCCGCGGCGGCGGCGGCGGCGGACTACGACGGCCTCGTCGCCTCCCCCGCCTCCATCCGG
TCCGCGAGGGGGAAGGCGCTTGTGCCGTCAAATCTCGACGAGCTCTTCTCCGCTGAGCTCGCCGCC
GCCGCGGCGTCGCGCTCGCCGCGCTACGCCGACCAAGGCGGCGCCGCGTTCTCCCCGACCCGCAAG
GCCACCGTGCTCAACCAATTCCAGCTGCAGCAGCAGCATAGCTTGCTCTCGCCGCGGGCGGCCGCG
GTGACACCAGAGCCGGTCTCCCCAATGAGCTCCCGCCTCCTCGCCGCGCTGGCGCAGCGGGAGAAG
ATGCAGCAGCAGACGCTGCGGAGCATGAGCTCACGGGACCTCGGCAACGCCGCGTCGCTGCTGGTC
GGCTCGCCGGTGAGCTCGAGCATGTCCAAATGGGGGTTCCCCTCCGGCAACCCGGACTGGGGCGCC
GACGACGAGGAGCTCGGCCGCCTCAAGCGTTGCTCCTCGTTCGAGCTCCGGTCCGGAGCCGCCAAT
GGCAACCATGAGCCTGACCTCTCATGGGTCAACACCCTAGTGAAGGAGCCGACACCGGAGAAGATG
ATGACGACGACATCGGCAATGGATTCCATTGGCATCTTGGGACAGAACACAAGCCGTGATCACATC
GTCGGAGGCGAGGATGACACTGCCGGAGTCATCAGCAGCTGGCTTGAACAGCTCCAGCTCGATGAG
ATGGTTGTCTAG

**SEQ ID NO: 13, Q2QPW2_ORYSA Rice zinc finger C-x8-C-x5-C-x3-H type family protein, expressed**
MGEPGGAEAAVSARLLELAADDNAAGLGELLAAWPSLADEPAPWYTPARGAEPLTPLMVAAVYGSV
GCLDALLSPPYLVDPNRASASSLSTPLHLAAAGGSASAPAAVSRLLAAGADPALLDHLQRRASDLV
ALPPNSLPLKNHLLSLLGARKEWPPDPSLPDIKNGAYASDDFRMYSFKVRACSRAYSHDWTECPFV
HPGENARRRDPRKYHYSCVPCPEFKKGAGCRRGDMCEYAHGVFESWLHPAQYRTRLCKDGVGCARR
VCFFAHTPDELRPLYVSTGSAVPSPRGALEMAAAAAAMGMGLSSPGSSSFTPPLSPSAGGGGGGGG
GSGGGGAWPQQPSVPALCLPGSAGNLHLSRLRTSLSARDMAVDELLAAAAAAADYDGLVASPASIR

**FIGURE 25 (continued)**

SARGKALVPSNLDELFSAELAAAAASRSPRYADQGGAAFSPTRKATVLNQFQLQQQHSLLSPRAAA
VTPEPVSPMSSRLLAALAQREKMQQQTLRSMSSRDLGNAASLLVGSPVSSSMSKWGFPSGNPDWGA
DDEELGRLKRCSSFELRSGAANGNHEPDLSWVNTLVKEPTPEKMMTTTSAMDSIGILGQNTSRDHI
VGGEDDTAGVISSWLEQLQLDEMVV

**SEQ ID NO: 14, *Medicago truncatula* Zn-finger protein, coding sequence**

ATGAAAAATCTAACTGTTCGTACTGATGATTCTTTTTCCAGCTTACTTGAACATGCTTCTAACAAT
GATTTTGAAGATTTCAAGGTAGCTCTAGATAGTGATGCTTCACTTATTAATGAAGTTGGCTTCTGG
TATGTCCGTCAAAAGGGATCTAACCAAATTGTTCTTGAGCACCGAACCCCTTTAATGGTGGCTGCT
TCCTATGGGAGTATTGATATTCTAAAGCTTATACTCTCATATCCCGAGGCTGATGTTAATTTCTCC
TGTGGAACTGATAAAAGCACTGCTCTTCACTGTGCTGCCTCAAGTGGTTCAGTTAATGCTGTTGAT
GCTATAAAATTGCTTTTATCAGCTGGTGCTGATATCAATTCTGTGGATGCTAATGGGAAACGCCCT
GTGGATGTTATCGTTGTTCCTATTGTTGTTCCTCATAAGCTCGAAGGTGTTAAAACAATTCTTGAA
GAACTTCTCTCAGACAGTGCTTCTGAAGGATCTGTGGATGATTGCTCTCTTCCCCTGTCTCTTATT
TCATCGAGTCCTGGTTCATCTGCCCCTTTATCATCTGCTGAAAATGGATCTCCATCCTCTCCTGTG
GCTCCCAAGTTTACAGATACAGCTGTTAATTCTACATCAGAAAAGAAAGAGTATCCAGTTGACCCA
TCTCTTCCTGACATAAAAAACAGCATGTATGCCACAGATGAATTCCGCATGTATTCATTCAAGGTT
CGTCCTTGTTCTCGTGCATACTCTCATGATTGGACTGAGTGTCCTTTTGTGCATCCTGGAGAGAAT
GCTCGAAGGAGAGACCCTAGAAAGTTTCACTACAGCTGTGTGCCATGCCCTGATTTTAGGAAAGGG
GCTTGCCGACGTTCGGATATGTGTGAATATGCTCATGGAGTATTCGAGTGCTGGCTACACCCAGCT
CAGTATCGGACAAGGCTGTGCAAAGACGGTATGGGTTGTAACCGAAGGGTGTGCTTCTTCGCTCAC
TCACCTGAAGAGCTGCGTCCGCTGTATGTGTCCACTGGTTCTGCTGTTCCTTCACCCCGATCAGCT
GCTTCTACTGCTAATGTCATGGACATGGCTGCTGCTATGAGCCTTTTCCCTGGTTCACCATCATCA
ATCTCTTTGATGTCTCAATCACCCTTTGCACAGCCTCCTCTATCTCCATCTGCAAATGGCAATAAT
GCTTGGCCACAGCCCAATGTGCCAGCTCTTCATTTACCAGGAAGCATTAATCAAACTAGTCGTTTG
AGATCTTCTCTTAGTGCCCGTGATATGCCACACGACGACTTCAACAATATGTTGCAAGACTTTGAT
GGGCAGCAGCAGATACTAAATGACTTGAGCTGTTTCTCACAGCCCCGTCCTGGTGCTATTTCAGTT
GGTCGATCTGGCCGCCCTAAAACACTAACTCCCTCAAATCTGGATGATCTTTTTTGTGCTGAGATT
GCTTCATCTCCTAGGTATTCCGACCCCGCTGCGGCTTCTGTATTTTCCCCAACACACAAATCTGCT
GTCTTCAACCAGTTTCAACAGCTTCAAAGCTCCTTATCACCCATCAACACAAATGTCATGTCTCCT
ACAAACGTAGAGCATCCCCTGTTCCACCAGGCTTCATATGGTCTCTCTTCTCCTGGAAGGATGTCA
CCAAGAAGTATGGAAGCCCTATCTCCAATGAGTTCTCGGCTGTCAGCTTTTGCTCAGCGTGAGAAA
CAACAGCAGCAGCAGCAACAGCTGCGTAGCCTCAGCTCAAGAGAACTCGGTGCTAACAATCCTCTC
TCAGCTGTTGGGTCCCCTGTTAACTCCTGGTCCAAGTGGGGATCATCCCCTATTGGAAAAGCTGAT
TGGTCGGTAAATCCAAATGACTTCGGTCAAACACAGAGATCAACTTCTTTTGAGCATGGAAACAAT
GGAGAAGAGCCTGATGTAGGTTGGGTCCATTCCCTTGTCAAGGATCCCACACCTGAGAAGAAAGAG
AAGCTTGCAGGTTCCGGCCCAATTCCATCCGTTGAAAAGAATCCCAATCCTCAAGCGGACGGCATT
GATCACTCTGTTTTGGGAGCTTGGCTCGAGCAACTGCAGCTGGATCAACTTGTAGTCTAG

**SEQ ID NO: 15, Q1T5G4_MEDTR *Medicago truncatula* zinc finger, CCCH-type**

MKNLTVRTDDSFSSLLEHASNNDFEDFKVALDSDASLINEVGFWYVRQKGSNQIVLEHRTPLMVAA
SYGSIDILKLILSYPEADVNFSCGTDKSTALHCAASSGSVNAVDAIKLLLSAGADINSVDANGKRP
VDVIVVPIVVPHKLEGVKTILEELLSDSASEGSVDDCSLPLSLISSSPGSSAPLSSAENGSPSSPV
APKFTDTAVNSTSEKKEYPVDPSLPDIKNSMYATDEFRMYSFKVRPCSRAYSHDWTECPFVHPGEN
ARRRDPRKFHYSCVPCPDFRKGACRRSDMCEYAHGVFECWLHPAQYRTRLCKDGMGCNRRVCFFAH
SPEELRPLYVSTGSAVPSPRSAASTANVMDMAAAMSLFPGSPSSISLMSQSPFAQPPLSPSANGNN

## FIGURE 25 (continued)

AWPQPNVPALHLPGSINQTSRLRSSLSARDMPHDDFNNMLQDFDGQQQILNDLSCFSQPRPGAISV
GRSGRPKTLTPSNLDDLFCAEIASSPRYSDPAAASVFSPTHKSAVFNQFQQLQSSLSPINTNVMSP
TNVEHPLFHQASYGLSSPGRMSPRSMEALSPMSSRLSAFAQREKQQQQQQQLRSLSSRELGANNPL
SAVGSPVNSWSKWGSSPIGKADWSVNPNDFGQTQRSTSFEHGNNGEEPDVGWVHSLVKDPTPEKKE
KLAGSGPIPSVEKNPNPQADGIDHSVLGAWLEQLQLDQLVV

**SEQ ID NO: 16, XM_469392 *Oryza sativa* (japonica cultivar-group), mRNA**

ATGAACGGCACGCCGATCTCCGCGTCCGCCGCGGCCGGCGTCGACGGAGTCGGCGCGGCGGTGGCG
CTGGCGGCCGCGACCAAGAAGAGTGCCGCCGCGGCGGCCGCCGTCGCCGAGATGGCGAAAACCCTC
ACCGTCGACACGGACGACGCCTTCGCGGGGCTCCTCGAGCTCGCCGCGGACGACGACGCGGAGGGC
CTGCGCCGCGCGCTGGAGCGCGCCCCGCCCCGCCGCCGCGGACGAGGCGGGCCTCTGGTACGGCCGC
CGCAAGGTCCTCGAGCACCGCACGCCGCTGATGGTCGCGGCCACCTATGGCAGCCTCGCGGTGCTT
CGCCTGCTGCTGTCCCTCCCGTCCGTCGATGTCAATCGCCGCTGTGGCTCCGACGGCACCACCGCC
CTCCACTGTGCGGCGTCTGGTGGCTCGCCGTCTTGTGTGGAGGCCGTCAAGCTGCTGCTTGCTGCT
GGGGCTGATGCTGATGCCACGGATGCTTCCGGATATCGTCCAGCTGATGTGATCTCTGTTCCTCCA
AAGATGTTTGACGCCAAGATTGCCCTCCAAGATCTTCTTGGATGCCCAAAGGCTGGGCATGGCGTT
CTCCGGGTGGTGACAAGGGCCGCAAACTCTATGTTGTCACCTGTATCATCCCCTACAGCAGAAGAT
GCACGATCTCCATCAGCTGCTGTGATGATGACGACAAAGTTTGCAGATCTTCCAAGGGTTGTGACA
TCGGAAAAGAAAGAATATCCAGTGGATCCGTCCCTTCCCGATATCAAGAACAGCATCTATGCTTCC
GATGAGTTCCGCATGTACTCATTTAAGATCAGGCCATGCTCGCGGGCGTACTCACATGATTGGACT
GAGTGCCCGTTTGTTCACCCAGGGGAGAACGCACGGCGTCGGGACCCTCGCAAGTATCACTACAGC
TGTGTGCCATGCCCCGACTTTAGAAAGGGAGTTTGCCGGCGTGGTGACATGTGTGAATATGCTCAT
GGCGTGTTCGAGTGTTGGCTCCATCCAGCACAGTACCGTACTCGCCTTTGCAAGGATGGCACAAGC
TGTAATCGCCGTGTCTGTTTCTTTGCGCATACAACTGATGAGCTCCGACCACTATATGTTTCCACT
GGATCTGCAGTACCATCCCCAAGAGCCTCGGCAACAGCTACAATGGAGATGGCTGCAGCAATGGGC
TTGATGCCTGGTTCTCCATCATCAGTTTCAGCAGTCATGTCCCCATTTACACCACCAATGTCCCCT
TCAGGCAATGGGATGCCCCCTTCATTGGGCTGGCAGCAGCCAAATGTTCCGACACTACACCTTCCA
GGCAGCAGCCTTCAGTCGAGCCGGCTCCGTACCTCACTTAGTGCAAGGGATATGCCTGCTGATGAT
TACTCCCTGATGCAGGATATTGATTCACAGCTTATAAATGATTTGTGCTATTCACGTATTGGTTCA
TCAACAGGAAACCACACGTCTCGGACCAAGTCCCTAAATCCGTCAAACTTGGATGATCTCTTCTCT
GCTGAGATGGTCTCTTCCCCGAGGTATAGTAATGCTGATCAGGGTGGTATGTTTTCACCATCTCAC
AAGGCTGCTTTCCTTAATCAGTTCCAGCAACAGCAGCAGGCACTTCTTTCACCAATCAACACAGTC
TTCTCCCCGAAGTCTGTGGACAACCAGCAGTTGCCTTCACACTCATCTCTGTTGCAAGCATCACTT
GGTATATCCTCCCCTGGCCGCATGTCTCCTCGATGTGTTGAATCTGGGTCCCCTATGAACTCTCAT
CTTGCTGCTGCTCTTGCTCAGCGTGAGAAGCAACAGCAGACAATGAGAAGTCTCAGTTCTCGTGAT
CTTGGGCCGAGTGCTGCAAGAGCATCAGGTGTTGTTGGCTCCCCTCTAAGCTCATCATGGTCAAAG
TGGGGATCACCTTCAGGGACACCTGACTGGGGTGTTAATGGTGAAGAATTGGGCAAGCTTCGCCGG
TCATCATCGTTTGAGCTGAGATCTGGTGGTGATGATCCAGATCTCTCTTGGGTACACACACTGGTT
AAGGAATCTCCACCAGAGAAGCAAGTCACTACTGCTGAATCCATAAACTCTGTTGGACCTTCACCA
CTGATGCCTCCCAGTGTAAGCAACGGTGAAGGTCCTAGTCTGAATGCCCCGCTGGATGGGCATGAC
CAAGCTGCTGTTATTGGAGCATTGCTTGAACAGATGCAGCTTGATCAGCATATTGGTAGTCTAGCA
ACATAAGCGCTGAATGAGCCTGGAAAGTGCAAGGAGTTATTATTCTTAGTTAATGAATTTGGAGTA
ATTTTTTTCCTGTTCATTAAGATGGTCAGCAAGCAAAAGGATGGATAGCTGATGGTGGTGATTCAG
AGATTGGTTTTCTTTACTTTATTGAGGTAAATCATATACATTATTGAGGTTCCAGTAGGTTGAAAG
ATTGAAGTACCTTGATTGGGGTCGTTTCAAGACCGACCCAGGTAGAATCGCACCCCGGCAGCTTCA
ATTCATCGGTCAAAAATATTTCCCTGTTTTGTTAATTAACCCCGTTAAAAAAGAAGACTCGTTTGG
TGTTTCGGAATTCTTTTCTTTACCTTAGCGGTGTTTATTTTGTTTATTATGATATTGATACTTGAT

**FIGURE 25 (continued)**

GTACTGATGGGTATAAGGTTGGTTACCAGGCATGCTATAGTGGTATATCAAGTCCCAAAGTATTCT
TTTTCTCCCTTTCACCATTTGTCGAGGATCATACTATGGCCTTGTTTTGGTCAGATCTTGAGGCCT
GTATAATCCTTGGATTTGTAATAATGTAATATTGTCATTGAACTTACATTGCTATTGTTTTGCAAT
CGC

**SEQ ID NO: 17, XP_469392, unknown protein [*Oryza sativa* (japonica cultivar-group)]**
MNGTPISASAAAGVDGVGAAVALAAATKKSAAAAAAVAEMAKTLTVDTDDAFAGLLELAADDDAEG
LRRALERAPPAAADEAGLWYGRRKVLEHRTPLMVAATYGSLAVLRLLLSLPSVDVNRRCGSDGTTA
LHCAASGGSPSCVEAVKLLLAAGADADATDASGYRPADVISVPPKMFDAKIALQDLLGCPKAGHGV
LRVVTRAANSMLSPVSSPTAEDARSPSAAVMMTTKFADLPRVVTSEKKEYPVDPSLPDIKNSIYAS
DEFRMYSFKIRPCSRAYSHDWTECPFVHPGENARRRDPRKYHYSCVPCPDFRKGVCRRGDMCEYAH
GVFECWLHPAQYRTRLCKDGTSCNRRVCFFAHTTDELRPLYVSTGSAVPSPRASATATMEMAAAMG
LMPGSPSSVSAVMSPFTPPMSPSGNGMPPSLGWQQPNVPTLHLPGSSLQSSRLRTSLSARDMPADD
YSLMQDIDSQLINDLCYSRIGSSTGNHTSRTKSLNPSNLDDLFSAEMVSSPRYSNADQGGMFSPSH
KAAFLNQFQQQQQALLSPINTVFSPKSVDNQQLPSHSSLLQASLGISSPGRMSPRCVESGSPMNSH
LAAALAQREKQQQTMRSLSSRDLGPSAARASGVVGSPLSSSWSKWGSPSGTPDWGVNGEELGKLRR
SSSFELRSGGDDPDLSWVHTLVKESPPEKQVTTAESINSVGPSPLMPPSVSNGEGPSLNAPLDGHD
QAAVIGALLEQMQLDQHIGSLAT

**SEQ ID NO: 18, NM_121288 *Arabidopsis thaliana* transcription factor (AT5G12850) mRNA, complete cds**
ACACCAGTTACCCTCTCATCCGTTTTCGTTTTTTTTTTCTCTCTTTCAAAAATCTCTCAGCTGAGG
TTGATCGATCTTCTTCTTCTTCTTCCTCACTCTTTAGATTTGTTCCTTTTGCATTTTACACTTTTG
GATCTGAAAATGTGGTTCTCTGTTTCGCCCGTTACCGTTTAGATTCAGTTCTGTTTTTTTCTTACC
CGATCGCTTGATTCGGACTGTGATCTTTGATCTTTTTTCTTCTCCAGTGCCGTGAAGGATGTGTGG
TCTTGCTAAGAAGCTGGATATAGAGGATACTTTGACATCACTGTCAGACCAAGAGAATGAATCTTT
GGCCAAACCCATGAATGATGCTGCTGAATGGGAACATTCGTTTTCTGCCTTGCTTGAGTTTGCTGC
AGACAACGATGTGGAGGGGTTTAGGCGGCAACTCTCTGATGTGTCTTGTATCAACCAGATGGGTCT
TTGGTACAGACGGCAGAGGTTTGTTAGAAGAATGGTTCTTGAGCAAAGAACCCCGCTGATGGTTGC
TTCGTTATATGGGAGTTTAGATGTTGTGAAGTTTATTCTTTCTTTCCCGGAAGCGGAGTTGAATCT
GTCTTGTGGTCCTGATAAAAGTACTGCTCTTCATTGCGCTGCTTCTGGTGCTTCTGTGAATTCCTT
GGATGTTGTCAAGTTGCTTTTGAGTGTAGGAGCAGATCCTAATATCCCTGATGCTCATGGAAATCG
TCCTGTTGATGTTCTTGTTGTGTCTCCACACGCTCCTGGTTTGAGAACCATCCTTGAAGAGATCTT
GAAGAAAGACGAGATTATATCTGAAGATCTGCATGCCTCGTCATCTAGCTTGGGATCAAGTTTCCG
GTCTCTCTCATCATCCCCTGATAATGGTTCCTCGTTACTCTCCTTAGATTCAGTATCCTCTCCGAC
TAAGCCACACGGTACTGATGTAACTTTCGCATCAGAGAAGAAAGAGTACCCAATTGATCCATCATT
GCCTGATATCAAAAGCGGGATTTATTCAACCGATGAGTTTCGTATGTTCTCGTTCAAGATCCGCCC
ATGTTCTCGAGCATATTCCCATGACTGGACTGAATGTCCATTTGCACACCCAGGTGAGAATGCAAG
GAGAAGAGACCCGAGGAAGTTTCACTATACGTGTGTTCCATGCCCGGATTTTAAGAAAGGATCCTG
TAAGCAAGGTGATATGTGTGAATATGCTCATGGGGTTTTTGAATGCTGGCTACACCCTGCTCAGTA
CAGAACACGATTGTGCAAGGACGGAATGGGTTGCAACCGAAGGGTTTGCTTCTTTGCTCACGCAAA
TGAGGAGTTGCGTCCCTTGTACCCTTCCACAGGATCTGGATTGCCATCTCCTCGGGCTTCGTCTGC
TGTTTCCGCCTCTACTATGGACATGGCGTCAGTTTTGAACATGTTACCAGGCTCACCATCTGCTGC
TCAACATTCGTTCACCCCACCAATATCTCCTTCTGGAAATGGTAGTATGCCCCATTCATCGATGGG
TTGGCCTCAGCAGAACATACCGGCGTTGAATCTTCCTGGAAGCAATATCCAGTTGAGTCGTCTGAG
ATCTTCTCTTAACGCTAGAGATATTCCTTCTGAGCAGCTTAGCATGCTGCATGAGTTTGAAATGCA
ACGTCAGCTTGCTGGCGATATGCACAGTCCACGCTTTATGAATCATTCCGCTCGTCCTAAGACACT

FIGURE 25 (continued)

GAACCCTTCAAATCTGGAGGAACTCTTCTCAGCTGAGGTTGCATCTCCTCGTTTCTCTGATCAACT
TGCTGTTTCATCTGTTCTATCGCCTTCCCACAAGTCCGCGCTTCTTAATCAGCTGCAGAATAATAA
GCAGAGCATGCTTTCTCCTATCAAGACAAATCTAATGTCTTCTCCAAAGAATGTGGAGCAACATTC
TCTTCTGCAGCAAGCCTCGTCACCCCGAGGCGGAGAGCCTATTTCCCCAATGAATGCTCGAATGAA
ACAGCAGCTACATTCACGCAGCCTAAGCTCCCGTGATTTTGGATCTAGTCTGCCCCGTGATTTAAT
GCCGACTGATTCTGGTTCGCCATTAAGTCCATGGTCAAGTTGGGACCAGACCCATGGAAGCAAGGT
GGATTGGTCAGTCCAATCAGATGAGTTAGGTCGGTTGAGAAAATCTCATTCCTTGGCTAATAACCC
AAACAGGGAAGCAGATGTTTCATGGGCTCAGCAGATGTTAAAAGACTCTTCATCACCTAGGAACGG
AAACCGTGTTGTGAACATGAATGGTGCAAGGCCATTGACTCAAGGTGGTTCGAGTGTGAATCCTCA
CAACAGTGACACTCGTGAGAGCGACATTCTTGATGCGTGGCTTGAACAGCTGCACCTAGATCGCTG
AGCCTCAGCTGCGAGAGAGAGGTTCACATTTCTGTGAAGCTGTGAAACTGATGATTCGTTTATTTA
TTATTCAAGAAAGCAAACGGAAACAAAAGCAAACTCCGGGTAAGCTTTTTTCGATTCTAATAACCC
TAAAAGGCTCAGTTTTTTCAGGCTTCTTTCTGAAATTTCTTTACTTTCTTATTTTTATCACCTCAT
TAAATTAATTATTGTATCATCTCTGTTGTAACAATGGCCAAAGTGCGCCTCTATTACTTCCCGGAT
TTCTGATTTACATTTTTTGTATCCTCTCAGTTTGTCAATTGTTTCTAATATCTCCTTCATATTTGT
CAAAGAACACTGTATGAGAAATAATAACATATTGTTTCAGCTAATAAGATTCATTCATTTCCT

**SEQ ID NO: 19, AT5G12850 Q9LXV4_ARATH *Arabidopsis thaliana* zinc finger transcription factor-like protein**

MCGLAKKLDIEDTLTSLSDQENESLAKPMNDAAEWEHSFSALLEFAADNDVEGFRRQLSDVSCINQ
MGLWYRRQRFVRRMVLEQRTPLMVASLYGSLDVVKFILSFPEAELNLSCGPDKSTALHCAASGASV
NSLDVVKLLLSVGADPNIPDAHGNRPVDVLVVSPHAPGLRTILEEILKKDEIISEDLHASSSSLGS
SFRSLSSSPDNGSSLLSLDSVSSPTKPHGTDVTFASEKKEYPIDPSLPDIKSGIYSTDEFRMFSFK
IRPCSRAYSHDWTECPFAHPGENARRRDPRKFHYTCVPCPDFKKGSCKQGDMCEYAHGVFECWLHP
AQYRTRLCKDGMGCNRRVCFFAHANEELRPLYPSTGSGLPSPRASSAVSASTMDMASVLNMLPGSP
SAAQHSFTPPISPSGNGSMPHSSMGWPQQNIPALNLPGSNIQLSRLRSSLNARDIPSEQLSMLHEF
EMQRQLAGDMHSPRFMNHSARPKTLNPSNLEELFSAEVASPRFSDQLAVSSVLSPSHKSALLNQLQ
NNKQSMLSPIKTNLMSSPKNVEQHSLLQQASSPRGGEPISPMNARMKQQLHSRSLSSRDFGSSLPR
DLMPTDSGSPLSPWSSWDQTHGSKVDWSVQSDELGRLRKSHSLANNPNREADVSWAQQMLKDSSSP
RNGNRVVNMNGARPLTQGGSSVNPHNSDTRESDILDAWLEQLHLDR

**SEQ ID NO: 20, XM_506415 PREDICTED *Oryza sativa* (japonica cultivar-group), OJ1092_A07.129 mRNA**

TGCGAGTCCTCCTCCTCTTCTCGTCGCCGTGCTACTCTCGCTTTCTCTCTCTCTCTCTCTCACTTG
TTCCCCAAGGCGAGAAGCAGCCGCCGCCGGCGAGCGTCGCGGGGGAGGGGAGGGAAGGGAGGGAGG
AGCGGTGGATCCGGGCTTGATTGGATTGGGTCGGATTCGATTTTGGATCAACCCCGGAGGGCGGGA
GCGGTTGCTACAGATGCGTTGAGCTTTGGTTAATCTATCCGGCGAGAGATAATGGGCGAGCTTGCT
GATCTCGTTGTCGTGCCGTCGCAGCCGCCGCTCGCCGGCGGCCGGCGGGACAGGCTGGCGGCGCTG
CTGGAGCTCGCGGCGGCGGATGATGTTGATGGGCTCAGGGGGGCGCTCGCGGAGGGAGGCGAGGAG
GCGGCGGAGTTGGCTGATGGGGTCGGGCTGTGGTATGGTCGGAGCAAGGCGTACGAGGCGCGCACG
CCGCTGATGGTGGCGGCGACGTACGGCAGCGCCGGGGTGGTCTCGCTGCTGGTGGGCCTCGGCGGT
TGCGTCGACGTCAACCGTCGCCCTGGAGCCGACGGCGCCACCGCGCTCCACTGCGCCGCCTCCGGT
GGCTCGCGCAACGCCGTCGCTGTTGTCAAGCTGCTTTTGGCCGCTGGCGCCGATCCGGCCACCCCC
GATTCCGCCGGCCGCTTCCCCGCCGACGTCATCCTAGCTCCTCCGGCTTCGCCAGATGCCCTTGGC
GATCTCGAGGTGCTCCTCGGCCGCCGCCGAGCACTCGCCGTGGCGACCTCGGTGGCTTCAGGTTCG
TCATCCCCTCCGCTCTCGTCCTCACCAGATGAGGGCAACAGGTCGCCCTCGTCGCGTTCGTCGTCG
CTGTCTCCCATCACTGTGGATCGTGGGAAGAAGGAGTATCCGGTGGATCCAACTCTGCCGGACATC
AAGAGCAGCGTGTATGCTTCGGATGAGTTCCGCATGTTTGCGTTCAAGGTCCGGCCCTGCTCCCGT

**FIGURE 25 (continued)**

GCCTACTCACACGACTGGACTGAGTGCCCGTTTGTGCACCCCGGCGAGAACGCCCGCCGCCGTGAT
CCCCGCAAGCACCCATACACTGCTGTGCCTTGCCCCAACTTTCGCCGGCCTGGTGGCTGCCCTAGC
GGCGATAGCTGTGAGTTCTCGCATGGCGTGTTTGAGAGCTGGCTACACCCATCACAGTATCGCACA
AGGCTCTGCAAGGAGGGAGCAGCTTGCGCCCGTCGCATTTGCTTCTTTGCCCATGATGAGGATGAG
CTCCGCCATGTGCCTCACAACAGTGGTGCCGGCCTGCTGTCTCCCCGCGCTTCTTCATCCATTGAT
ATGACTGCTGCAGCTGCGCTCGGGCTTCTTCCAGGTTCTCCTACCAGACACTTTGCACCGCCGCCT
GTGTCACCATCTGCTGGGAGCAATGGAGGAGCTGCTGCTGCGCATTGGCTCCAAGGCAGTAGGCTG
CGTTCTTCTTTCAATGCAAGGGATGCTGCTGTTGATGACCTTGGCATGCTCCTCGAATGGGAATCA
CAATACCTTGGGGCACTCTGCCTGCCACCCAGCAGCCGCCCCCAACCACGCCTTTCAGCTGGTCTG
AGTATCAGGCCAACAATTGCTCCATCCAATCTTGAAGACATGTATGCTTCAGACATGGCAATGTCT
CCGAGGTTCCCTAATGACCAAGGTCACTCAGTCTACTCACCAGCCCACAAATCAGCCCTCCTCAAC
AAGCTTCATCAACAGAAGGGCCTCTTATCACCTGTTAACACCAACAGAATGTACTCCCCAAGGGCT
CTTGATCCGTCATCTTTGGCACATTCTCCATTTGGTGGCATGTCTCCCCGGTCCCCCCGTACCATG
GAACCTACATCACCCCTAAGTGCTCGTGTAGGAGCCCCTGCCACACAGCGGCCTTCTGTTGGTTCA
CCACGGAATTCCAGTGCTTGGGGCACCGTGGGGTCCCCGATGGGTAAGGTTGACTGGGGTGTCGAT
AGCGAGGAGCTAGTCCGCTTGAGACGCCCTGCACAACCAGGGTTTGGAGAAGATGAGACAGATGTA
TCATGGGTGCAGTCACTGGTAAGCAATGCTGAGCTTAATGGCAAGAGGGGCGAAGTACAAGGCATG
CCTGGTACTTCTGCATTGATGAACAGGCCTGACCTGAACAATCAGGGTGACTTGTTGGACCAGACG
GTGATCGGTGCTTGGCTTGAGCAGATGCACCTGGATCAGAAGTGATTTCCAAGGGAAGCCATGAAG
TCCCAAAGTGGATGAAGCCTTTATTTGCCAAGGTTATTTACCAAAGAATAGTTGTTGGTCCTAGT
AAATAATAATTTATTCTTTTTAATTCTTGAAATTTTTGGTGGGCAAAGTCAGAGATGGTGGTCAAG
TTCAACAAAACATTTGGTCACAGATTGGTAGCTGAAATCAGTTCCAGAGATTGGTAAACAACCTCA
TTACTTGGGGTCCTAACTAGTATTCTTTTGATTAGCTCAGATGAGTCTTTATTTTAGTGGGTTAAA
ATTCATATGTTCCCCATGGTTATTATGTCCATGATCTCTTCCTAACAAAAGAGAGATTATAATTGT
CCATTTTTCATTTATCAATGAATGATTTTGTTAAAACAATGTAAGTTACATTCTTAATTTTTTCTC
TGTTCAATGGAATTACCTTCCTTGGTTAGTCCTC

**SEQ ID NO: 21, XP_506415 PREDICTED OJ1092_A07.129 gene product [*Oryza sativa* (japonica cultivar-group)]**

MGELADLVVVPSQPPLAGGRRDRLAALLELAAADDVDGLRGALAEGGEEAAELADGVGLWYGRSKA
YEARTPLMVAATYGSAGVVSLLVGLGGCVDVNRRPGADGATALHCAASGGSRNAVAVVKLLLAAGA
DPATPDSAGRFPADVILAPPASPDALGDLEVLLGRRRALAVATSVASGSSSPPLSSSPDEGNRSPS
SRSSSLSPITVDRGKKEYPVDPTLPDIKSSVYASDEFRMFAFKVRPCSRAYSHDWTECPFVHPGEN
ARRRDPRKHPYTAVPCPNFRRPGGCPSGDSCEFSHGVFESWLHPSQYRTRLCKEGAACARRICFFA
HDEDELRHVPHNSGAGLLSPRASSSIDMTAAAALGLLPGSPTRHFAPPPVSPSAGSNGGAAAAHWL
QGSRLRSSFNARDAAVDDLGMLLEWESQYLGALCLPPSSRPQPRLSAGLSIRPTIAPSNLEDMYAS
DMAMSPRFPNDQGHSVYSPAHKSALLNKLHQQKGLLSPVNTNRMYSPRALDPSSLAHSPFGGMSPR
SPRTMEPTSPLSARVGAPATQRPSVGSPRNSSAWGTVGSPMGKVDWGVDSEELVRLRRPAQPGFGE
DETDVSWVQSLVSNAELNGKRGEVQGMPGTSALMNRPDLNNQGDLLDQTVIGAWLEQMHLDQK

**SEQ ID NO: 22, NM_129572 *Arabidopsis thaliana* transcription factor (AT2G40140) mRNA, complete cds**

TTCTTCAAAAACCCCAACCACTTCTTCTCCCCAAAAACCTCCAAAGTTTCAATCTTTACTTCTCTC
TTTTTCTCCAAGTTATCTTCTTTTCTAGGAAGAGATATGTGCGGTGCAAAGAGCAACCTTTGCTCA
TCTAAAACCCTAACAGAAGTCGAATTCATGAGGCAGAAATCAGAAGACGGAGCTTCCGCCACGTGT
CTCCTCGAATTCGCCGCCTGTGATGATCTTTCATCGTTTAAGAGAGAGATCGAAGAGAATCCATCG
GTGGAGATTGATGAGTCAGGGTTTTGGTATTGCAGACGGGTCGGGTCTAAGAAGATGGGTTTTGAA

## FIGURE 25 (continued)

GAAAGAACACCACTTATGGTTGCTGCTATGTATGGAAGCATGGAAGTGTTGAATTACATAATTGCC
ACAGGAAGATCCGATGTGAACAGAGTTTGCAGTGACGAGAAAGTCACTGCTCTTCACTGTGCAGTT
TCTGGCTGTTCTGTTTCTATCGTTGAGATCATCAAGATCTTGCTTGATGCTTCTGCTTCACCTAAT
TGTGTTGACGCTAATGGGAACAAACCGGTTGATTTGTTGGCTAAAGATTCTCGGTTTGTTCCTAAC
CAGAGTAGAAAGGCGGTTGAGGTTTTACTGACCGGGATTCATGGTTCGGTTATGGAAGAAGAGGAG
GAGGAACTGAAGAGTGTTGTGACTAAGTATCCAGCTGATGCATCACTTCCTGATATTAACGAAGGT
GTTTATGGAACTGATGATTTTAGGATGTTTAGCTTTAAGGTTAAGCCATGTTCTAGGGCTTATTCA
CATGATTGGACTGAATGTCCTTTTGTTCATCCTGGTGAGAATGCAAGGAGGAGAGATCCTAGGAAG
TATCCTTACACTTGTGTGCCTTGTCCCGAGTTTCGTAAAGGGTCTTGTCCTAAAGGAGATTCGTGT
GAGTACGCGCACGGTGTTTTCGAGTCTTGGCTTCACCCGGCGCAGTATAGGACACGGCTTTGCAAA
GATGAGACTGGTTGTGCTAGGAGAGTTTGTTTCTTTGCTCATAGACGGGATGAGTTAAGACCGGTT
AATGCTTCTACTGGTTCTGCAATGGTTTCACCAAGGTCGTCTAATCAGTCTCCTGAGATGTCTGTT
ATGTCTCCTTTGACGCTGGGATCATCGCCAATGAACTCTCCTATGGCTAATGGTGTTCCTTTGTCT
CCAAGAAATGGTGGTTTATGGCAGAACAGAGTTAATAGCCTTACACCACCACCGTTGCAGCTTAAT
GGTAGCAGATTGAAGTCGACTTTGAGTGCTAGAGATATGGATATGGAGATGGAACTTAGGTTTCGC
GGTTTGGATAACCGGAGACTTGGTGATCTCAAGCCATCCAACCTCGAAGAGACTTTCGGATCATAT
GACTCAGCTTCTGTGATGCAACTTCAATCACCAAGCAGGCATTCTCAGATGAACCACTATCCGTCT
TCACCTGTGAGGCAGCCTCCTCCTCATGGATTCGAATCTTCAGCAGCCATGGCAGCTGCAGTGATG
AATGCAAGATCCTCAGCGTTTGCGAAACGCAGCTTGAGTTTCAAACCAGCTCCAGTAGCTTCTAAT
GTCTCCGATTGGGGATCACCAAATGGGAAGCTTGAGTGGGGAATGCAAAGAGATGAGCTGAACAAG
TTGAGGAGAAGTGCCTCCTTCGGCATTCATGGAAACAACAACAACAGTGTGTCACGCCCTGCTAGA
GACTACAGTGACGAGCCAGATGTGTCGTGGGTGAACTCACTGGTGAAAGAGAATGCACCAGAGAGA
GTGAATGAGAGGGTTGGGAATACGGTGAATGGTGCAGCGAGTAGAGACAAGTTTAAGCTGCCGTCG
TGGGCAGAGCAAATGTATATAGACCATGAGCAGCAGATTGTGGCATAAGAAGCAGAAAGAAAGATG
TGGGATTTATATTGCTTTTGTCTTCTGGGCCTCTCTACACAGAATCTAACAAATCTGGCAATAATT
CTTTGATTTGTGTTTGACCCATAGTTTGGTTACTAGTATATGTTTTTTTATGTTCTTTTTTTCTTT
GTCATTCTCTTGTCCTTCGTGACACTATGTAATGATTAAAAGCAAATAATTGATGCATGAGTTCAA
ATGTTCTTTGAAGGATCCATCTTATTAGCTTTGTAATTGTTGTGATATCTTAATCTTATTGGTTAC
GTATTTCAAGTGCTTTAGAAAAAATGGGCCTAAGAGATTTTGGGG

**SEQ ID NO: 23, At2g40140 Q9XEE6_ARATH *Arabidopsis thaliana* Hypothetical Cys-3-His zinc finger protein**

MCGAKSNLCSSKTLTEVEFMRQKSEDGASATCLLEFAACDDLSSFKREIEENPSVEIDESGFWYCR
RVGSKKMGFEERTPLMVAAMYGSMEVLNYIIATGRSDVNRVCSDEKVTALHCAVSGCSVSIVEIIK
ILLDASASPNCVDANGNKPVDLLAKDSRFVPNQSRKAVEVLLTGIHGSVMEEEEEELKSVVTKYPA
DASLPDINEGVYGTDDFRMFSFKVKPCSRAYSHDWTECPFVHPGENARRRDPRKYPYTCVPCPEFR
KGSCPKGDSCEYAHGVFESWLHPAQYRTRLCKDETGCARRVCFFAHRRDELRPVNASTGSAMVSPR
SSNQSPEMSVMSPLTLGSSPMNSPMANGVPLSPRNGGLWQNRVNSLTPPPLQLNGSRLKSTLSARD
MDMEMELRFRGLDNRRLGDLKPSNLEETFGSYDSASVMQLQSPSRHSQMNHYPSSPVRQPPPHGFE
SSAAMAAAVMNARSSAFAKRSLSFKPAPVASNVSDWGSPNGKLEWGMQRDELNKLRRSASFGIHGN
NNNSVSRPARDYSDEPDVSWVNSLVKENAPERVNERVGNTVNGAASRDKFKLPSWAEQMYIDHEQQ
IVA

**SEQ ID NO: 24, AL163832.179, *Arabidopsis thaliana*, At3g55980**

ATGGAAAAAGATAGTATTATGTGCAGTGGACCAAAGAGCAATCTCTGCTCTTCAAGAACCTTAACA
GAAATCGAATCAAGGCAAAAGGAAGAAGAAACAATGCTTCTCCTCGAATTCGCTGCTTGTGATGAT
CTTGACTCGTTCAAGAGAGAGGTTGAAGAGAAAGGGCTTGATTTGGATGAGTCAGGGTTATGGTAT
TGCAGACGTGTCGGTTCTAAGAAGATGGGTCTTGAAGAAAGAACACCTTTAATGGTTGCAGCTATG

## FIGURE 25 (continued)

TATGGAAGCATAAAGGTTTTGACTTTCATCGTTTCCACTGGAAAATCTGATGTGAACAGAGCTTGT
GGTGAAGAGAGAGTTACTCCGCTTCACTGTGCTGTTGCTGGCTGTTCTGTGAATATGATTGAAGTC
ATCAATGTCTTGCTTGATGCTTCTGCTTTGGTTAACTCTGTTGATGCTAATGGGAATCAACCTTTG
GATGTGTTTGTTCGAGTTTCGAGGTTTGTGGCTAGTCCGAGGAGGAAAGCGGTTGAGTTGTTGCTG
AGAGGAGGAGGTGTTGGAGGATTGATCGATGAGGCGGTTGAAGAAGAGATCAAGATTGTCTCTAAG
TATCCAGCTGATGCTTCTTTACCGGATATAAACGAAGGGGTTTATGGAAGTGATGAGTTTAGGATG
TATAGCTTTAAGGTTAAGCCATGTTCTAGGGCTTATTCTCATGATTGGACCGAGTGTGCTTTTGTT
CATCCGGGAGAAAATGCGAGGAGGAGAGATCCGAGGAAGTATCCTTACACTTGTGTCCCCTGTCCC
GAGTTCCGTAAAGGATCATGCCCGAAAGGAGATTCTTGCGAGTATGCTCACGGGGTTTTCGAGTCG
TGGCTTCACCCCGCGCAGTATAAAACCCGGCTTTGTAAAGATGAAACGGGTTGTGCAAGGAAAGTT
TGTTTCTTTGCTCATAAACGCGAAGAGATGAGACCTGTTAATGCTTCAACTGGCTCTGCCGTGGCT
CAGTCTCCGTTTAGCAGCTTGGAGATGATGCCAGGGTTGTCTCCTCTTGCTTATTCTTCAGGAGTT
TCGACTCCTCCGGTTTCTCCAATGGCTAATGGTGTTCCTTCCTCTCCAAGAAACGGCGGATCATGG
CAGAACAGAGTCAATACCCTTACTCCACCGGCTTTGCAGCTCAATGGTGGAAGCAGATTGAAGTCC
ACACTGAGCGCTAGAGATATCGATATGGAGATGGAGATGGAATTGAGACTCCGCGGTTTTGGCAAC
AATGTGGAAGAGACGTTCGGGTCTTATGTTTCCTCTCCAAGTAGGAATTCTCAAATGGGTCAAAAC
ATGAACCAACATTATCCATCTTCCCCGGTGAGACAACCGCCATCTCAACACGGGTTCGAATCTTCA
GCAGCTGCAGCGGTTGCAGTGATGAAAGCGAGATCAACCGCCTTTGCGAAACGTAGCTTGAGCTTC
AAACCAGCTACTCAAGCAGCACCACAGTCGAATCTCTCGGATTGGGGATCTCCAAACGGGAAGCTG
GAATGGGGAATGAAAGGAGAAGAGCTGAATAAGATGAGAAGAAGTGTTTCCTTTGGAATCCATGGA
AACAACAACAATAACGCAGCTAGAGACTACAGGGACGAGCCAGATGTGTCATGGGTTAACTCTTTA
GTTAAAGACAGTACTGTGGTGTCTGAGAGAAGCTTTGGAATGAATGAGAGGGTTCGGATAATGTCG
TGGGCTGAGCAAATGTACAGAGAGAAGGAGCAGACTGTGGTGTA
A

**SEQ ID NO: 25, At3g55980-2, Q9LY47_ARATH *Arabidopsis thaliana* Hypothetical protein F27K19_160**

MEKDSIMCSGPKSNLCSSRTLTEIESRQKEEETMLLLEFAACDDLDSFKREVEEKGLDLDESGLWY
CRRVGSKKMGLEERTPLMVAAMYGSIKVLTFIVSTGKSDVNRACGEERVTPLHCAVAGCSVNMIEV
INVLLDASALVNSVDANGNQPLDVFVRVSRFVASPRRKAVELLLRGGGVGGLIDEAVEEEIKIVSK
YPADASLPDINEGVYGSDEFRMYSFKVKPCSRAYSHDWTECAFVHPGENARRRDPRKYPYTCVPCP
EFRKGSCPKGDSCEYAHGVFESWLHPAQYKTRLCKDETGCARKVCFFAHKREEMRPVNASTGSAVA
QSPFSSLEMMPGLSPLAYSSGVSTPPVSPMANGVPSSPRNGGSWQNRVNTLTPPALQLNGGSRLKS
TLSARDIDMEMEMELRLRGFGNNVEETFGSYVSSPSRNSQMGQNMNQHYPSSPVRQPPSQHGFESS
AAAAVAVMKARSTAFAKRSLSFKPATQAAPQSNLSDWGSPNGKLEWGMKGEELNKMRRSVSFGIHG
NNNNNAARDYRDEPDVSWVNSLVKDSTVVSERSFGMNERVRIMSWAEQMYREKEQTVV

**SEQ ID NO: 26, ADW16461 *Eucalyptus grandis* transcription factor cDNA C3H-type family**

CTTCTGAAAGCTTTTTGACTTAAGACGAGAGAGAAGGAGAGAAGGTCCCCCTCCTCGTCCTCGTCC
CCCCGTGGATTTTGAAGAAGAAAAGTCGCACCTTCCTTCTCCTTTCCCACTCCTCCCTCTGCTCGA
AGCTTTTCTCTTCCGCAGAATTACATAAAAACCTCGACTTTGCGCATCATTCCGATTCACCTCACA
CCTTCACTTTCCCACTCGAGGTCTCCCCCCTCTTTTCCTAGCTCTTTCCCTTTCCCTCCCTCTCTC
TCGAGAATCGCCGCATTTGGAGGAGCTCCAATCTGCTTTGCTTTGCTTTGCTCTCTTCTTGCTCGG
TTCCCCCTCATAAGGAGTCGATTATGTGCAACGGTTCTTCGAAGGGTAAACTTTTCCCCTCGAGTA
TGGGCATGGAGGGCGAATTCCACAACAAGGATGGCGAAGCACCCCGTAAATGCTCTGCCTTGCTTG
AATTGGCAGCCTCGGACGATCTCTCGTCGTTCAAAAGTGAAGTGGAAGAGAAGGGCTGCGACGTTG
ATGAGGCCAGCTTTTGGTATGGTAGGAGAATCGGGTCGAAGAAGATGGGTTTTGAAGAGAGGACTC

**FIGURE 25 (continued)**

CATTGATGATCTCTGCTTTGTTTGGAAGCACCAAGGTCTTGAAATACATAATCGAGACCGCCAGAG
CTGATGTCAACAGGTCTTGTGGGTCCGACAAGGTGGCCGCCCTCCATTGCGCAGCCGCGGGTGGGT
CCAGTTCTTCACTTGAAATTGTGAAGCTCTTGATTGAGGCCTCAGCGGATATTAATTCTGTAGATG
GCAATGGAAATAGGCCCATCGACGTGCTTGCCCCGGCAGGGAAGTCTCGCTGCAATTCCAGAAATA
AGTTTGTTAGATCGTTGCTGAAAGGTGAAAACTATGTCGTGGAAGGTGACCAATCCTTTGACATAG
AAGGAGAGGAGAAGCTAGTCGCTCTTCCAAAGGAGGGAGGCGAGAAGAAAGAGTATCCTGTTGATG
TCTCTCTACCTGACATAAACAATGGGTTCTACAGTACCGATGAGTTCCGGATGTATGCTTTCAAGG
TGAAGCCTTGCTCGAGGGCTTACTCCCACGACTGGACCGAGTGCCCGTTTGTGCACCCTGGGGAGA
ACGCGAGGAGGAGGGACCCACGCAAGTACCCTTACAGCTGTGTCCCTTGTCCTGAGTTTCGCAAGG
GTTCGTGCGTAAGGGGGGATGCTTGTGAGTATGCTCATGGAGTCTTTGAGTCGTGGCTTCACCCAG
CGCAATACCGAACCCGGCTGTGCAAGGATGAAACTGGTTGTACTCGCAAAGTTTGCTTCTTTGCTC
ACAAGTCCGAAGAATTGCGTCCCGTGTATGCTTCCACAGGTTCTGCTATGCCCTCACCCAAGTCCT
TTTCAGCTAATGCCCTAGACATGACAACCCTGAGCCCCTTATCCCTTAATTCACCATCTCTGCCTT
TGCCTGCTACTTCCACGCCCCCCATGTCACCTTTGGCTGCCTCATCTTCACCCAAGGGCATGAACT
TGTGGCATAACAAAATTAACCTGACCCCACCAAGCCTGCAGCTTCCTGGCAGCCGGCTGAAGACGG
CTATGAGTGCGCGGGACTTCGATTTTGAGTTGGAATTTCTTGGGCTGGAAAAGCAAGCTTCTCAGC
GGCAGCAACTGATAGAAGAGATTTCTCGTCTCTCATCGCCCTCTCATATGTGGAACTCGGAATTTG
GCAGAACCGCAGAGCTGAAGCCCACTAACCTTGATGATGCGTTTGGATCTCTTGACACTTCTCTTT
TGTCTCCGTTGCAGGGGTCGTCGATGAAAACATCGACTCCTACCCAGTTGCAATCCCCCACAGGGC
TTAAAATTTCGAATTTGAACCAACTCCGTGCGAGCTACCCGTCTAGCAGCTTGTCGTCCTCTCCTG
TGAGGAAGACCTCTTCTTTTGGGTTCGACTCATCCAGTGCAGTTGCTGCAGCAGTCATGAACTCAC
GGTCTGCTGCTATGACGAAGCGGAGCCAGAGCTTCATTGACCGTGGAGCAGTGGGTCAACGGTCTG
GACTCATTGGACCTGCTAATTCTGCTCCTAGGATGTCCAACCTTTCGGACTGGGGCTCGCCTGATG
GGAAGTTGGATTGGGGTGTTCAAGGGGACGAGCTCAACAAGCTTAGGAAGTCCGCTTCCTTCGGCT
TTAGAAACAACAGTATGGCGAACCCAAACAACGTGGCGTCTCCCAGTGCTGATGAGCCGGACGTGT
CGTGGGTTGGTTCATTGGTGAAGGATGTGGCTCCGCCCGAAGGGTATCCACAGTATCTGTACATAG
AACAGGAGCAGATGGTGGCATAACTAAAGCGAAGAGCACCACACGAACTCTCTCCTGATGGCTTAA
GATGACTTGTTTGACATTCTTTATATTCTTACAAACAGCGCGTTCTTAGGAGTTAGCTGGAGGAAA
GAAGGAAACGGTATTGAGTTTGAGATTCAGGCTCTTAGCTGGACAGCGAAAATTTGGGGAAGGAAG
AGAATTTGGTTTCTTGCCCAACTTAGATAATGATGCTTTTGAAGGCTTAAAAGAAAGATGAAGGCA
AACATTCTTTTGTTAGTATTGTATTATTGTTTTAATTTTTCATCCCCTCTGTCGGGGTGTGGTGGG
TGTCGATGTTTCTTTCATCAGTAAAATATATAATGAGGTTTACTCATCTATTTTCTACTAAAAAAA
AAA

**SEQ ID NO: 27, *Eucalyptus grandis* transcription factor protein C3H-type family**

MCNGSSKGKLFPSSMGMEGEFHNKDGEAPRKCSALLELAASDDLSSFKSEVEEKGCDVDEASFWYG
RRIGSKKMGFEERTPLMISALFGSTKVLKYIIETARADVNRSCGSDKVAALHCAAAGGSSSSLEIV
KLLIEASADINSVDGNGNRPIDVLAPAGKSRCNSRNKFVRSLLKGENYVVEGDQSFDIEGEEKLVA
LPKEGGEKKEYPVDVSLPDINNGFYSTDEFRMYAFKVKPCSRAYSHDWTECPFVHPGENARRRDPR
KYPYSCVPCPEFRKGSCVRGDACEYAHGVFESWLHPAQYRTRLCKDETGCTRKVCFFAHKSEELRP
VYASTGSAMPSPKSFSANALDMTTLSPLSLNSPSLPLPATSTPPMSPLAASSSPKGMNLWHNKINL
TPPSLQLPGSRLKTAMSARDFDFELEFLGLEKQASQRQQLIEEISRLSSPSHMWNSEFGRTAELKP
TNLDDAFGSLDTSLLSPLQGSSMKTSTPTQLQSPTGLKISNLNQLRASYPSSSLSSSPVRKTSSFG
FDSSSAVAAAVMNSRSAAMTKRSQSFIDRGAVGQRSGLIGPANSAPRMSNLSDWGSPDGKLDWGVQ
GDELNKLRKSASFGFRNNSMANPNNVASPSADEPDVSWVGSLVKDVAPPEGYPQYLYIEQEQMVA

## FIGURE 25 (continued)

**SEQ ID NO: 28, *Eucalyptus grandis* transcription factor cDNA C3H-type family**

TCTCCTTCGAGTTTCTTTCTTCACTAGAATTCGCTCCCGAGTCTGTTGTTGCTCGTGTAGTTTTGC
TTACTCCGTCCTTCGTTTAGCTCGCTGACCAGCGCGGAGCTAGGAGCGGTCGCTAAAGGATTACTC
GTACAAAACGTAAACTCAGCTCTGCCAATTTTCCCATGGAGGGGGAATCTTACTTCGAGAAAGATG
AAAAATATTCTAATTGCTCAATCTTGCTCGAATTATCTGCTTCGGACGATCTCCCAGCTTTTGAAA
GGAAAGCGAAAGAGAAGGGCTGTAACATTGATGGTGCTAGCTTCTGGTACGGTAGAAGAATTGGCT
CAAGGAAGATGGGTCTTGAAGAGAGGACTCCTCTCATGGTGGCTTCCTTGTTTGGAAGCTCTAGGG
TTGTGAAGTACATTCTCGAATCTGGCAAAGTCGATGTAAATAGGGCTTGTGGTTCGGACAAGGTCA
CTGCCCTTCACTGTGCTGTTGCCAGTGGCTCTGCTTCTGCGGTGGAGGTTGTCAAGCTCTTGCTTC
ACGCATCTGCCGATGCTAATTGCATTGATGGCAATGGAAAGAAGCCAATTGATGTGATAGCCCTTC
CATTAAAGTCACGCGGCGATTCAAGGAGGAAGCTGATGGAGCTGTTGCTGAAAGGCGATAATTCTG
ATGGGGAATTTGAATCCCACGAGGAGAAGCCGATTGCCGCACCGCAAGCATCCAAAGAGGGAAGCG
AAAAGAAAGAGTATCAATTTCCTGTTGATATCTCTCTGCCTGACATAAATGTTGGGATTTACAGTA
CTGATGAGTTCAGAATGTATGCTTTCAAAGTAAAGCCTTGCTCGCGGGCATACTCCCATGACTGGA
CAGAGTGCCCATTTGTTCATCCTGGCGAGAATGCGAGGAGGCGGGACCCTCGCAAGTACCCCTACA
GCTGCGTCCCTTGCCCTGAATTTCGGAAGGGATCTTGCCAAAAGGGTGACTCCTGTGAGTACGCGC
ACGGCGTATTTGAGTCGTGGCTTCATCCTGCACAGTATAGAACAAGACTGTGCAAGGATGAGACTG
GATGTGCTCGCAAAGTTTGTTTCTTTGCTCACAAGCCCGAAGAATTAAGGCCTGTCTATGCTTCGA
CGGGATCAGCTATGCCTTCCCCAAAATCCTACTCATCAAGTGGGCTGGACATGTCCACATTGAGTC
CTCTCTCAATCAGTTCTCCGTCAGCATCGTTGCCTGTTACTTCAACAGCACCCATGTCTCCTCTTG
CAGCCTCGTCATCTCCGATGTCTGTGAACATGTGGCAGAGCAAGGCTAACAAGCTCTCCCCGCCAA
TGCTGCAGCTCTCAGGTAGTAGGCTGAAGACTGCTTTGAGTGCTAGGGACTTGGACCTGGAGATGG
AATTGCGTGGTCTAGAGAGTCAGATGGCCACTCAACAGCATCAGTTGATGGAAGAGATATCTCGTC
TCTCCTCACCATCATCCTGCTTTAGTAGTAGGATTGGGGAAGTGAAACCCACTAACCTCGATGACG
TTTTTGGGTCTCCGGATCCTGCTTTGCTGCCTCAATTGCAGGGGCTGTCAAGACCTTCAACACCAA
GCCAGTTGCAATCTCCAACTGGGCTTCAGATGCGCCAGAATGCAACCCAGTTTCGTGGGGCGTACC
AGAGCAATGCAAATGCATTGTCATCTCCAGCAATGAAGCAGGCACCTTCTTATGGGTTTGACTCAT
CTAGTGCAGTTGCAGCAGCGGTGATGAATTCGAGGTCAGCCGCTTTTGCGAAGCGGAGTCAGAGTT
TTATCGACAGGGGAATGGCGTGCCCTGGAATTGCCAATTCTTCCCCTATGATGTCTTCAGCTATGT
CGAGCTGGAGCTCACCTCATGGGAAATTGGATTGGGGCGTCCAAGGAGATGAGTTGAATAGGCTGA
GGAAAGCTGCTTCCTTTAAGATGAGAAGCAGCACCGGAGCAGGTGCTAATACTGTCTCGGCAGCAG
CCATGGCTGATGAGCCAGATATTTCTTGGGTCAGTTCATTGGTTAAGGACGTGCCTTCTGCGGAGG
ACGCGATGTTCGCTGCAGAGAAAGGACAGCGCACTTATGGGAAAGACATCCGCGAAAGGATTACCC
CATGGGTGGAGCAGCTGTACAGAGAAGTGCCACGGATGGCGATGTAAGATTGCCACTGCAAGTCGG
ATGCCTTAGTATGCTGACTAATTGATATTCTTTGCATTTGTTTTGAGGCATTTGGTAGCCATTAGA
TACGAGAAAAGGCCAAGCAGCAGGTGGTGTCTTGGCAAGGAATAGGATGCACATAGTCTGTTATCG
AGTAGAATAGACTTGGGAACAATGGTTATAGCCAAATGTTAAAAGTTATGATATTCTTTTCCAATT
CTTTCTCTTCCTCATAGTAGGTTTCTCACCAAGTCTTTTAGTGAGAGCCTGCGGGATGTACTATAT
GTTTCCCTTATGTAACGTCTCTTCGTTGAAAGAAATGGCTTTATAATATAAAGCATCAAGTTTTTT
AAAAAAAAAA

**SEQ ID NO: 29, *Eucalyptus grandis* transcription factor protein C3H-type family**

MEGESYFEKDEKYSNCSILLELSASDDLPAFERKAKEKGCNIDGASFWYGRRIGSRKMGLEERTPL
MVASLFGSSRVVKYILESGKVDVNRACGSDKVTALHCAVASGSASAVEVVKLLLHASADANCIDGN
GKKPIDVIALPLKSRGDSRRKLMELLLKGDNSDGEFESHEEKPIAAPQASKEGSEKKEYQFPVDIS
LPDINVGIYSTDEFRMYAFKVKPCSRAYSHDWTECPFVHPGENARRRDPRKYPYSCVPCPEFRKGS

## FIGURE 25 (continued)

CQKGDSCEYAHGVFESWLHPAQYRTRLCKDETGCARKVCFFAHKPEELRPVYASTGSAMPSPKSYS
SSGLDMSTLSPLSISSPSASLPVTSTAPMSPLAASSSPMSVNMWQSKANKLSPPMLQLSGSRLKTA
LSARDLDLEMELRGLESQMATQQHQLMEEISRLSSPSSCFSSRIGEVKPTNLDDVFGSPDPALLPQ
LQGLSRPSTPSQLQSPTGLQMRQNATQFRGAYQSNANALSSPAMKQAPSYGFDSSSAVAAAVMNSR
SAAFAKRSQSFIDRGMACPGIANSSPMMSSAMSSWSSPHGKLDWGVQGDELNRLRKAASFKMRSST
GAGANTVSAAAMADEPDISWVSSLVKDVPSAEDAMFAAEKGQRTYGKDIRERITPWVEQLYREVPR
MAM

**SEQ ID NO: 30, Soybean C3H Zn finger containing protein, encoding sequence**

CGAATTCCGGTCGACGATTTCTCGATTTCCTTCTCTATAACACAACGCTCTCTTCTCTTGCAACCA
AAGTACTTGTTCCAGTGTCTACTCTACTCAAAAAGGATTTGGGACATCATGTGCAGTGATTCGAAA
AGTAAACTTTCTTCCCCAACCCTCGTCGTCATGGAGAATAGTAACATTCAGAAGCAGAATCTGGAT
GGTCTCTACAACTCGGTTTTGCTTGAATTGTCTGCATCTGATGATTATGAAGCTTTCAAAAGAGAG
GTGGAGGAAAAAGGCTTAGATGTGAACGAGGCAGGCTTTTGGTACGGTAGAAGAATTGGGTCAAAG
AAGATGGGATCTGAAACGAGGACCCCTCTGATGATTGCTTCTTTGTTTGGAAGCGCCAAGGTGCTC
AATTATATTCTTCTTCAGAAAGGAGGAGGTGTTGATGTGAACAGGGTCTGTGGTTCTGATAGGGCC
ACTGCTCTCCATTGTGCTNNNNNNNNNNNNNNNNNNNNNNNNNNNNNNNNNNNNNNNNNNNNNNNN
NNTCCCGGGTCGCGATTTCGTAGGAGATTCACAGAGAGAAAAGAAGCAATTAGTGATAATAAGAAA
GAATACCCTGTTGATATATCACTGCCAGACATAAACAACGGTGTATATGGAACAGATGATTTTAGG
ATGTACAACTTCAAGGTGAAGCCTTGCTCAAGGGCTTACTCCCATGACTGGACCGAGTGTCCATTC
GTTCACCCAGGGGAGAACGCTAGGAGGAGAGACCCACGGAAATACCCTTACAGCTGTGTTCCTTGC
CCTGAGTTCCGCAAAGGGACCTGCCAGAAGGGTGATTCCTGTGAGTATGCTCATGGTGTTTTTGAG
TCCTGGCTGCATCCTGCCCAATACCGGACAAGGCTTTGCAAGGATGAGACTGGCTGCGCTAGAAAA
GTCTGCTTCTTTGCCCACAAACCTGAAGAGCTACGCCCTGTGTATGCTTCCACTGGGTCGGCTATG
CCATCACCAAAATCATATTCAGCTAGTGGACTTGACATGACAGCGATGAGTCCATTGGCTCTAAGT
TCCACATCTTTGCCTAATGCCCCCCCGTTTCCAGCCTCACCCTATCGTGCGCCCTCGTTCTTCTCT
CAGAGTGAAGCTGTGCAGAACAAAATAAACCTTACTCCACCATCGTTGCAGCTCCCTGGTAGCCGA
CTGAAGGCTGCTTTGAGTGCCAGGGATCTGGAGATGGAGATGGAACTGCTCGGTCTAGAAAGCCCT
GCTCGCCAACAACAGCAGCAGCAGCAACAATTGATCGAAGAGATTGCCAGGATCTCTTCCCCATCT
TTCCGGAGCAAGGAATTCAATAGGATTGTTGATTTGAATCCTACTAACCTTGATGACCTGTTAGCA
TCTGCTGACCCTTCTGTATTTTCTCAACTACATGGACTTTCTGTGCAACCTTCAACACCCACACAA
AGTGGGCTTCAGATGCGCCAAAACATGAACCACCTCCGTGCGAGTTATCCATCCAACATCCCTTCC
TCTCCTGTGAGGAAGCCCTCAGCTTTGGGTTTGACTCATCAGCTGCTGTGGCAACTGCAGTGATG
AATTCTAGGTCTGCTGCCTTCGCAAAGCGAAGCCAAAGTTTCATTGATCGTGGAGCTGCAACCCAC
CATCTTGGGCTGTCTTCAGCTTCCAACTCTTCTTGCAGGGTATCCTCTACCCTTTCAGATTGGAGT
TCCCCTACCGGGAAACTGGATTGGGGTGTAAACGGAGACAAGCTGAACAAGCTGAGGAAATCTACT
TCCTTTGGATTCAGAAACAGTGGGGTAACTGCATCCCCCATAGCACAGCCTGAATTTGGTGCTGAG
CCGGATGTCTCATGGGTTCATTCATTGGTTAAAGATGTTCCCTCCGAGAGGTCTGAGATATTTGGT
GCTGAGAAGCAACAATATGATCTCAGTAAAGAGATGCTTCCACCATGGATGGAGCAGCTGTATATA
GAGCAGGAGCAGATGGTAGCA

**SEQ ID NO: 31, Soybean orf of SEQ ID NO: 30, C3H zinc finger containing protein**

RIPVDDFSISFSITQRSLLLQPKYLFQCLLYSKRIWDIMCSDSKSKLSSPTLVVMENSNIQKQNLD
GLYNSVLLELSASDDYEAFKREVEEKGLDVNEAGFWYGRRIGSKKMGSETRTPLMIASLFGSAKVL
NYILLQKGGGVDVNRVCGSDRATALHCAXXXXXXXXXXXXXXXXXPGSRFRRRFTERKEAISDNKK
EYPVDISLPDINNGVYGTDDFRMYNFKVKPCSRAYSHDWTECPFVHPGENARRRDPRKYPYSCVPC

**FIGURE 25 (continued)**

PEFRKGTCQKGDSCEYAHGVFESWLHPAQYRTRLCKDETGCARKVCFFAHKPEELRPVYASTGSAM
PSPKSYSASGLDMTAMSPLALSSTSLPNAPPFPASPYRAPSFFSQSEAVQNKINLTPPSLQLPGSR
LKAALSARDLEMEMELLGLESPARQQQQQQQLIEEIARISSPSFRSKEFNRIVDLNPTNLDDLLA
SADPSVFSQLHGLSVQPSTPTQSGLQMRQNMNHLRASYPSNIPSSPVRKPSAFGFDSSAAVATAVM
NSRSAAFAKRSQSFIDRGAATHHLGLSSASNSSCRVSSTLSDWSSPTGKLDWGVNGDKLNKLRKST
SFGFRNSGVTASPIAQPEFGAEPDVSWVHSLVKDVPSERSEIFGAEKQQYDLSKEMLPPWMEQLYI
EQEQMVA

**SEQ ID NO: 32, *Eucalyptus grandis* transcription factor cDNA C3H-type family**

GCAAAGGTCGATCACTTCCTCCCTAGAAAGCGAGTGTGGAGTTGAAGCTTGATAACCAGAGGCCGC
CTCTCGTCTCGTCTCGCCCGCCTGCGCTTGCTCTGCTCTCCGCGTGCCAAGGGAGTGTTCCTAGGT
GCTGAATCTTTCCATGTGTAGCGGTTCAAAAGGGAAGGGAGAGTGAATTCGAGAAGCAGAGGATGT
CGGCCCGTCAGTTCTCGATCCTGCTCGAGTTATCTGCTGCGGATGATCTGACGAACTTTAAGAAAG
CAGTTGAGGAAGACGGCTACGATATTGATGAGTCGAGCTTGTGGTATGGTAGGAGGATCGGGTCGA
AGAAGATTGGGCTTGAAGAGAGAACTCCCCTCATGATTGCCGCGATGTTCGGCAGTATGTCCGTGC
TGGATTATATTATCAAGTCTGGCCGGGCCAATGTAAACAAGGCGTGTGGTTCAGATGGTGCTACCG
CGCTTCACTGTGCTGCGGCTGGTGGCTCGGTACAATCTCCTGAGGTGGTCAAGCTGTTGCTTGATT
CTTCAGCGAATGCTAACTCCATTGATGCGAATGGGAAACGAGCGGGAGACTTGATTTCTGAGGTCT
CTGGTTCGCCCTTCAATTCGAGAAGGAAGACTTTGGATGTCATGTTGACTGGAGGTGGGACTGTTG
AGTTTGTTGAGGAAACTTACAATCTGCCTGAGAATCTGGGTAGTCAAATTGAAGGAAACGAACAAA
GAGAGAGTCCAACGGCCCGCGCTTCCAAGGATGGTTCTGAAAAGAAAGAGTATCCTGTCGACCTTT
CTCTTCCGGACATCAACAATGGAATATATAGCACAGATGAGTTTAGGATGTATTCTTTCAAAGTGA
AGCCTTGCTCGAGAGCTTACTCTCATGACTGGACTGAGTGTCCATTTGTTCACCCTGGGGAGAATG
CAAGACGGCGTGACCCACGGAAATATCACTACAGCTGTGTGCCTTGCCCTGAGTTCCGCAAGGGGT
CATGCAGGCAAGGGGATGGCTGCGAGTATGCTCATGGTATATTTGAGTGCTGGCTTCACCCAGCTC
AATATCGCACCCGTCTCTGTAAGGATGAGATTGGATGCACCAGAAAAGTCTGTTTCTTTGCCCACA
AACATGAAGAGCTTCGTCCATTGTATGCATCAACTGGTTCGGCGCTTCCTTCTCCAAGATCATTTT
CGCCCGTTGCTGCTTCTCTAGACATGGGATCACTGAGCCCTCTCTCTCTCGGTTCTTCTTCAGTCC
GGATACCGCCAACTTCAACACCACCTATGACTCCATCAGGGGCCTCTTCTCCCCTTGGTGGGTCGA
TGTGGAAAAGCCAAATTAATAGCACTCCGCCTGGCTTGCAGCTTCCAGGTAGCAGGTTGAGAAGCG
CATTGAGTGCTAGAGACATGGATTTAGATGTTGACTTGATCGATCTAGAAAATAATTATCGTTTGC
AGAAGCAGTTGCTCGAACACTTTCCTGATCTGTCCTCTCCTCGTGGTTGGAACAACTCTTCATCCA
CCACGTCGGCTTTCCCTGAGTATTCAGGTGACATGACTGGAGAAATAAGTAGGTTAGGAGTAAAAC
CAAATAATCTCGAGGATAGTTTCAGGTCATTGGACCTGACCCTCTTGTCTCAGTTACAAGGGCTGT
CACTTGATGGTGCAATATCCCAGCTGCAATCTCCTACTGGAATGAAGATTCGGCAGAACATGACCC
AGCAGCTCTACTCAAACTATACTGACAAGCTTTCCTCGTCACCTAGGGCAATGCCATCATTTGGAA
CCGATCCTTCCAGAGCTTCAGCAGCAGCCACTCTGAGTTCCAGGTCATTGGCATTTGCAAAAAGGA
GCCACAGCTTCATTGAGCGGAGTACAGTGAACAGTCAGTCTGGATATTCAGCAGGTGCTGCTTCTC
CAACTGCAAGGATGTCTTCCCAGAATGACTGGGGCTCGCCCGATGGCAAACTAGACTGGGGCATTC
AAGGGGAGGAGCTGAACAAGCTGAGGAAATCTGCATCATTCGGGCTCAGGAGCAGCAGCAACCGCT
TCCATGCGTCTGCAGATTCTGCGACAGCAACTGTAGGGGACCCAGACATGCCCTGGATTCAGTCCT
TGGCAAAGGAAGCCCCGTCACAAAACCCTGGCAATTTTGGAGCAGAGCATCAGCAGCAGCAGCAGC
AGCAGCAGCAGCAGCAGTATCATCTTAATTCTGGAGGTACTGAGCTGCTTCCAGCTTGGGTGGAGC
AGTTGTACGCGGATCAGGAGCAGATGGTCGCCTGAGATCAACATTGGCTTCTTATCTAACCACTAT
TAGTCATTTCGTTATTGCTTTAATTTTTTTTCTTCTGAGTCTAGTATTAATGTCTAGGATTCGAAC
GAACTGGAAAATTAAATCTAGAGGGAAGATGGGAAGAAAAGAGCAGGATGGAAGGTTTCTGCTCGG

**FIGURE 25 (continued)**

TCCGAGATTTCTCATAGTCTATTATAGACTATCGTATTTCTCGTTCTTTTCCGTCCCAATGTTCTT
GATTTGGTTCTCAGCATGTTTTCTGGATGAGGCTTACAAACTATGTAATCTTGTCTTGCTAAAAGA
ATCAGAGCTGCACCTGCACCAAAGGTTGTGATACTACCGCTTATTGATGATGATGATAATAATAAT
AATTCGGACATTTAGTACCAAGTCCGATGTCTCAAAAAAAAAA

**SEQ ID NO: 33, *Eucalyptus grandis* transcription factor protein C3H-type family**

MSARQFSILLELSAADDLTNFKKAVEEDGYDIDESSLWYGRRIGSKKIGLEERTPLMIAAMFGSMS
VLDYIIKSGRANVNKACGSDGATALHCAAAGGSVQSPEVVKLLLDSSANANSIDANGKRAGDLISE
VSGSPFNSRRKTLDVMLTGGGTVEFVEETYNLPENLGSQIEGNEQRESPTARASKDGSEKKEYPVD
LSLPDINNGIYSTDEFRMYSFKVKPCSRAYSHDWTECPFVHPGENARRRDPRKYHYSCVPCPEFRK
GSCRQGDGCEYAHGIFECWLHPAQYRTRLCKDEIGCTRKVCFFAHKHEELRPLYASTGSALPSPRS
FSPVAASLDMGSLSPLSLGSSSVRIPPTSTPPMTPSGASSPLGGSMWKSQINSTPPGLQLPGSRLR
SALSARDMDLDVDLIDLENNYRLQKQLLEHFPDLSSPRGWNNSSSTTSAFPEYSGDMTGEISRLGV
KPNNLEDSFRSLDLTLLSQLQGLSLDGAISQLQSPTGMKIRQNMTQQLYSNYTDKLSSSPRAMPSF
GTDPSRASAAATLSSRSLAFAKRSHSFIERSTVNSQSGYSAGAASPTARMSSQNDWGSPDGKLDWG
IQGEELNKLRKSASFGLRSSSNRFHASADSATATVGDPDMPWIQSLAKEAPSQNPGNFGAEHQQQQ
QQQQQQQYHLNSGGTELLPAWVEQLYADQEQMVA

**SEQ ID NO: 34, NM_125249 *Arabidopsis thaliana* transcription factor (AT5G58620) mRNA, complete cds**

ATTTTGACCTTAAGAAGAAAGTGACAAGGAGAGGAAGAAGAAGAAAAAAAAACATAATTTGAGGAAG
AAGAAAAAAAAATTCGGATTTGTTTTTTCAATAAATTGACTAATTGAGTACTCGTTTAAAGGAAGTG
AAGAGCGGTTTTTTGGTAGTGGTGGTCGAGAAAAGAGAGAGTTTGTCTCTGTGACTCAGAGTGAAA
TCAATAGAGCGGGAAAAGATTGTTGCTTTTTTTTGCCATGGGAGTTGATGAGCTGTCTCACCTCAA
ATTCTCTCTTCTGCTAGAATCATCAGCCTGCAATGATTTGTCCGGTTTTAAGTCTCTAGTTGAAGA
AGAAGGTCTTGAGAGCATTGATGGCTCTGGTTTGTGGTATGGGAGGAGATTAGGATCAAAGAAGAT
GGGTTTTGAGGAGAGGACGCCTCTTATGATTGCTGCCTTGTTTGGAAGCAAAGAGGTTGTTGATTA
CATCATTAGTACTGGTCTTGTTGACGTGAACCGCTCTTGTGGCTCTGATGGTGCCACGGCTCTTCA
CTGTGCGGTCTCTGGCTTGTCTGCCAATAGCCTTGAGATTGTTACTCTTCTGCTGAAGGGCTCTGC
GAATCCGGATTCTTGTGATGCTTATGGTAACAAGCCTGGAGATGTGATTTTCCCTTGTTTGAGTCC
GGTTTTTAGCGCGAGGATGAAGGTTTTGGAGCGTTTGTTGAAAGGAAATGATGATTTGAATGAAGT
TAATGGGCAAGAAGAAAGCGAGCCAGAGGTTGAGGTTGAGGTTGAGGTTTCGCCTCCTCGGGGGTC
TGAGAGGAAGGAGTATCCGGTTGATCCAACGCTTCCTGATATCAAGAACGGTGTATATGGGACGGA
TGAGTTCCGGATGTATGCTTTCAAGATCAAGCCGTGCTCTAGAGCATACTCTCACGACTGGACGGA
ATGTCCCTTTGTTCATCCGGGTGAGAACGCAAGGAGGCGTGATCCGAGGAAGTACCATTATAGTTG
TGTCCCTTGTCCTGAATTCCGGAAGGGGTCTTGTTCCAGAGGTGATACTTGCGAGTATGCTCATGG
TATCTTTGAGTGCTGGCTTCACCCGGCTCAGTACCGGACTCGTCTCTGCAAGGACGAGACGAATTG
CTCGAGAAGAGTTTGTTTCTTTGCCCACAAACCCGAGGAGCTGCGTCCTTTGTACCCTTCAACTGG
ATCAGGTGTTCCGTCCCCGCGGTCTTCCTTCTCATCTTGCAATTCCTCGACCGCTTTCGACATGGG
ACCGATTAGTCCGCTTCCTATCGGAGCAACAACCACACCTCCTTTGAGTCCTAACGGTGTATCCTC
TCCAATAGGTGGAGGAAAAACGTGGATGAACTGGCCTAACATAACCCCTCCTGCATTGCAGCTTCC
AGGGAGCAGATTGAAATCTGCATTGAATGCAAGAGAAATCGATTTCTCTGAAGAGATGCAAAGTCT
TACTTCTCCAACTACATGGAACAACACGCCAATGTCATCTCCATTCTCCGGAAAGGGCATGAACAG
GCTTGCAGGAGGAGCAATGAGCCCGGTGAATAGTCTCAGTGATATGTTTGGGACAGAGGATAATAC
ATCGGGTTTGCAGATCCGACGCAGCGTCATTAACCCGCAGCTGCATTCCAACAGTCTTTCTTCATC
ACCTGTGGGAGCCAATTCTCTGTTTTCGATGGATTCCTCCGCAGTCTTGGCTTCAAGAGCGGCTGA
ATTTGCTAAACAGCGAAGCCAAAGCTTCATAGAACGCAACAACGGACTGAATCACCATCCCGCAAT

## FIGURE 25 (continued)

CTCTTCCATGACTACAACTTGTTTAAACGATTGGGGCTCATTGGATGGGAAGCTTGACTGGAGCGT
CCAAGGAGACGAGCTACAGAAGCTCAGAAAATCCACTTCTTTCCGTCTCAGAGCCGGTGGCATGGA
ATCAAGACTGCCTAACGAAGGGACTGGGCTCGAAGAGCCAGATGTCTCATGGGTGGAGCCGCTGGT
GAAAGAGCCACAGGAGACAAGACTAGCTCCGGTTTGGATGGAGCAATCATACATGGAGACAGAACA
GACCGTGGCTTGAATCAAAAGTTTTGAACTTTCATTAACCGTTCCACAAGAAGCAAAGTCAGAAAG
ATTCCGAGAGGTCGATGCTAATCTATTTCATTTTATTTGTTTAATGCTTTGTTATTTTTCTTTAGA
ATAAAAAGAAAAAATTCTTAGGGGACAAAAGAGAGTTCGTTTGTCTCTCTCTCTGTCTCCAAAGAA
AAACAGAGGTGAAAAAAGGTTTCAAAACCTAAGAAACCTTGAATTACCTCACCTCACTTCCTTGAT
TCTTTACTATTCACAATGAGTAATCGATTTTTTTTTTTCTTGGTAACACTCTCACGCTGAATATAT
ATGTTTTTTAGTAATAATATAATTGGAATACAGAAATGTATTTACACTTGTGAAGTTAGGGAAAGT
GTTGTAATTGTTTCTTCTAAGAGTTGATCTAAGATGTTTGAGACTATATCTTCGCTT

**SEQ ID NO: 35, AT5G58620 Q9LUZ4_ARATH Zinc finger transcription factor-like protein**

MGVDELSHLKFSLLLESSACNDLSGFKSLVEEEGLESIDGSGLWYGRRLGSKKMGFEERTPLMIAA
LFGSKEVVDYIISTGLVDVNRSCGSDGATALHCAVSGLSANSLEIVTLLLKGSANPDSCDAYGNKP
GDVIFPCLSPVFSARMKVLERLLKGNDDLNEVNGQEESEPEVEVEVEVSPPRGSERKEYPVDPTLP
DIKNGVYGTDEFRMYAFKIKPCSRAYSHDWTECPFVHPGENARRRDPRKYHYSCVPCPEFRKGSCS
RGDTCEYAHGIFECWLHPAQYRTRLCKDETNCSRRVCFFAHKPEELRPLYPSTGSGVPSPRSSFSS
CNSSTAFDMGPISPLPIGATTTPPLSPNGVSSPIGGGKTWMNWPNITPPALQLPGSRLKSALNARE
IDFSEEMQSLTSPTTWNNTPMSSPFSGKGMNRLAGGAMSPVNSLSDMFGTEDNTSGLQIRRSVINP
QLHSNSLSSSPVGANSLFSMDSSAVLASRAAEFAKQRSQSFIERNNGLNHHPAISSMTTTCLNDWG
SLDGKLDWSVQGDELQKLRKSTSFRLRAGGMESRLPNEGTGLEEPDVSWVEPLVKEPQETRLAPVW
MEQSYMETEQTVA

**SEQ ID NO: 36, *Oryza sativa*, AC130598.15**

ATGTGCTCTGGGCCGCGCAAGCCGTCCACACCGCCGCTGCCGCAGCAGCAGAAGGAGGCGACGGTG
ATGGCGGCGTCCTTGCTTCTTGAGCTGGCGGCAGCGGACGACGTGGCGGCGGTGAGGAGGGTCGTG
GAGGAGGAGAAGGTGTCTCTTGGCGTGGCTGGGTTGTGGTATGGGCCTTCGGCGAGCGGCGTGGCG
AGGCTCGGGATGGAGCGGAGGACGGCGGCGATGGTGGCGGCGCTGTACGGGAGCACGGGGGTGCTT
GGGTATGTCGTGGCGGCAGCGCCGGCGGAGGCCGCGCGCGCGTCGGAGACGGATGGGGCCACGCCG
CTGCACATGGCGGCTGCCGGTGGCGCGGCGAACGCGGTCGCGGCCACGCGCCTGTTGCTCGCCGCG
GGGGCGTCGGTCGACGCGCTCTCGGCTTCGGGGCTCCGCGCCGGTGACCTCCTCCCGCGCGCCACC
GCGGCGGAGAAGGCCATCCGGCTGCTGCTCAAGTCGCCGGCCGTGTCGCCGTCGTCGTCGCCGAAG
AAGTCGGCCTCGCCGCCGTCGCCGCCGCCGCCGCAGGAGGCGAAGAAGGAGTACCCGCCTGACCTG
ACGCTGCCCGACCTCAAGAGCGGACTGTTCAGCACCGACGAGTTCCGCATGTACAGCTTCAAGGTG
AAGCCGTGCTCCCGCGCCTACTCCCATGACTGGACCGAGTGCCCCTTCGTCCACCCCGGCGAGAAC
GCGCGCCGCCGCGACCCTCGCCGCTACTCCTACAGCTGCGTGCCTTGCCCGGAGTTCCGCAAGGGC
GGCTCGTGCCGCAAGGGCGACGCGTGCGAGTACGCCCATGGCGTGTTCGAGTGCTGGCTCCACCCG
GCGCAGTACAGGACGCGCCTCTGCAAGGACGAGGTCGGCTGCGCGCGCCGCATCTGCTTCTTCGCC
CACAAGCCCGACGAGCTCCGCGCCGTCAACCCCTCCGCCGTGTCCGTCGGCATGCAGCCCACCGTA
TCGTCGCCGCGCTCCTCGCCGCCCAACGGGCTCGACATGGCGGCGGCGGCGGCGGCGATGATGAGC
CCCGCCTGGCCGTCGTCCCCAGCGAGCCGCCTCAAGACGGCGCTCGGCGCGCGGGAGCTCGACTTC
GACCTCGAGATGCTCGCGCTGGACCAGTACCAGCAGAAGCTGTTCGACAAGGTGTCCGGCGCGCCG
TCGCCGAGGGCGAGCTGGGGCGCCGCGGCGAACGGCCTCGCCACCGCGTCGCCGGCGAGGGCCGTG
CCGGACTACACCGACCTGCTCGGCTCCGTCGACCCGGCCATGCTGTCCCAGCTCCACGCGCTGTCC
CTCAAGCAGGCCGGCGACATGCCCGCGTACAGCTCCATGGCGGACACCACGCAGATGCACATGCCG
ACCTCGCCGATGGTGGGCGGCGCGAACACCGCGTTCGGGCTGGACCACTCCATGGCGAAGGCGATC

## FIGURE 25 (continued)

ATGAGCTCCCGCGCCTCGGCGTTCGCCAAGCGCAGCCAGAGCTTCATCGACCGCGGAGGCCGCGCC
CCGGCGGCGCGTTCGCTCATGTCGCCGGCGACGACCGGCGCGCCGTCCATTCTCTCGGACTGGGGC
TCGCCGGACGGCAAGCTGGACTGGGGCGTCCAGGGCGACGAGCTGCACAAGCTCCGCAAGTCGGCG
TCGTTCGCGTTCCGCGGCCAATCCGCCATGCCGGTGGCGACGCACGCCGCGGCGGCGGAGCCGGAC
GTGTCATGGGTGAACTCTCTTGTCAAGGACGGCCACGCCGCCGGCGACATATTCGCGCAGTGGCCG
GAGCAGGAGCAGATGGTGGCATGA

**SEQ ID NO: 37, OSJNBa0056I11.7 Q688R3_ORYSA Putative finger transcription factor like OS_AAU10743.1**

MCSGPRKPSTPPLPQQQKEATVMAASLLLELAAADDVAAVRRVVEEEKVSLGVAGLWYGPSASGVA
RLGMERRTAAMVAALYGSTGVLGYVVAAAPAEAARASETDGATPLHMAAAGGAANAVAATRLLLAA
GASVDALSASGLRAGDLLPRATAAEKAIRLLLKSPAVSPSSSPKKSASPPSPPPPQEAKKEYPPDL
TLPDLKSGLFSTDEFRMYSFKVKPCSRAYSHDWTECPFVHPGENARRRDPRRYSYSCVPCPEFRKG
GSCRKGDACEYAHGVFECWLHPAQYRTRLCKDEVGCARRICFFAHKPDELRAVNPSAVSVGMQPTV
SSPRSSPPNGLDMAAAAAAMMSPAWPSSPASRLKTALGARELDFDLEMLALDQYQQKLFDKVSGAP
SPRASWGAAANGLATASPARAVPDYTDLLGSVDPAMLSQLHALSLKQAGDMPAYSSMADTTQMHMP
TSPMVGGANTAFGLDHSMAKAIMSSRASAFAKRSQSFIDRGGRAPAARSLMSPATTGAPSILSDWG
SPDGKLDWGVQGDELHKLRKSASFAFRGQSAMPVATHAAAAEPDVSWVNSLVKDGHAAGDIFAQWP
EQEQMVA

**SEQ ID NO: 38, *Hordeum vulgare*, C3H Zn finger containing protein, encoding sequence**

CGGCACGAGGCACATCCATCATCTAACCTCACCTCTCCTCTCCTCCCCTCTCCTCCTACCAAACCC
AAAACCAAGCAGAGCAAGAGCAAGAGCAAGAGCAAGAGCAAGCAAGCATGTGCCCTGGCCTGCGCA
ACCTCGCCGCCGCCATGCCACCCTCCGCCCACGACCACCCCTCCTCCTACCTGCTCGAGCTCGCCG
CCGACGACGACCTCCCCGCCTTCCGCCGCGCCGTCCAGGAGGACAACCTCTCCCTCGACGCCGCAT
CCCCGAGGTACGAGCCATCCCCCAAATCAGACCAACAACAACAACACGCCCCAGCTCGCGCTCCAC
CTGCGCACCCCCGCCATGGTCGCCGCGCTCTACGGCAGCACCACCGTCCTCTCCTACGTCCTCTCC
ATCGCCCCCTCCGAGGCCGCCCGCGCCTCCGCATCCGACGGCGCCACCCCGCTCCTCCTCGCCCAC
CAGGGCCGCGCGCCATCCGCGCCCCACGCCGCACGCCTCCTCCTCACCGACGGCGCATCATCGTCC
TCCCTACTCGCGCCCCAAGCTCACCCTCTCAACCACCAAAACCAAAACCAAAACAGCCCCACCAAG
AAAGACTCGCCGCCGGACTCCAGGAGGACCACCACCAAGAAGGACTACTCCTCCGCCTCCGACTCC
CAGACGGAGGACATCAACGCGGGCGTCTTCGCCACCGACGACTTCCGGATGTACAGCTTCAAGGTG
AACCCGTGCTCCCGCGCCTACACGCACGACTGGACCGAGTGCCCCTTCGCCCACCCCGGCGAGAAC
GCGCGCCGCCGCGACCCGCGCCGCGTGCCATACTCGTGCGTCCCATGCCCGGACTTCCGCCGCGAC
CCGGCCGCATGCCGCAAGGGCGACGCCTGCGAGTACGCGCACGGCGTCTTCGAGTCATGGCTCCAC
CCCGCGCAGTACCGCACCAGGCTCTGCAAGGACGAGGTCGGATGCCCGCGCCGCATCTGCTTCTTC
GCGCACGGCGCCCGACAGCTACGCGCCGTCAACCCCTCCGCCGCATCCATGGACTCGCCATCCCCA
ACTTCCTCTTCGCCGCCGCGAACCTCCAGGCCGGCCGCGCTCACCGCGTCGCTCAGCTCGCGGGAC
CTCGACTTGGACGCCGACAACCAGGCCCAGTACGCGCGCAGGATGATGATGGCCAGGGCCAACTCC
CCGCCGGACTACTCGCCCGACCTCGTCGCCGCCTACGTACAGGCGCTCTCCTCCCTGCAACAGCAG
CAGCATCAGCAGAACCAGCAACAGCAGCATCAGCAGCAGAACCAGCACCAGCAGCAACATCAGCAG
AACCAGCACCAGCAGCATCAGCAGCAACATCAGCAGAGCATGGGGATGGGGGGGCTGAGCGCCCGC
GCCGCCGCCTTCACCAACCGCAGCCAGACCTTCGTGCACCGCTCTCCGTCCCCGGCTCCGGCGCGG
TCGTTCAAGTCTCCGGCGCCGTCGTCCATGCTCGCGGACTGGGGGTCGCCGGACGGGAAGCTGGAC
TGGGGCGTGCAGGCCGCGGAGCTGCGCAAGTCCACGTCTTTCGGAGTCAGAAGCAGCAGCAGGCCG
CATCATGAGACGACGAGGGCGGAGGACAACATGTACCCGTCGTGGATGAAGGACGGCAGCGATATG
CTGCTGGCGGCGCGGTGGTCGGACCTGGAGCAGATGGTCGCC

## FIGURE 25 (continued)

**SEQ ID NO: 39, *Hordeum vulgare* orf of SEQ ID NO: 38**
RHEAHPSSNLTSPLLPSPPTKPKTKQSKSKSKSKSKQACALACATSPPPCHPPPTTTPPPTCSSSP
PTTTSPPSAAPSRRTTSPSTPHPRGTSHPPNQTNNNNTPQLALHLRTPAMVAALYGSTTVLSYVLS
IAPSEAARASASDGATPLLLAHQGRAPSAPHAARLLLTDGASSSSLLAPQAHPLNHQNQNQNSPTK
KDSPPDSRRTTTKKDYSSASDSQTEDINAGVFATDDFRMYSFKVNPCSRAYTHDWTECPFAHPGEN
ARRRDPRRVPYSCVPCPDFRRDPAACRKGDACEYAHGVFESWLHPAQYRTRLCKDEVGCPRRICFF
AHGARQLRAVNPSAASMDSPSPTSSSPPRTSRPAALTASLSSRDLDLDADNQAQYARRMMMARANS
PPDYSPDLVAAYVQALSSLQQQHQQNQQQQHQQQNQHQQQHQQNQHQQHQQQHQQSMGMGGLSAR
AAAFTNRSQTFVHRSPSPAPARSFKSPAPSSMLADWGSPDGKLDWGVQAAELRKSTSFGVRSSSRP
HHETTRAEDNMYPSWMKDGSDMLLAARWSDLEQMVA

**SEQ ID NO: 40, *Pinus radiata* transcription factor cDNA C3H-type family**
TGTTTCCAGGCGGGCACTAAAGCAAGGGAGGGGGTAGGCTTTACTTTCTGCTCTGCGCAAAGAACG
TTGAAATCAATCGCCCTGGCTGGTCTGGCGTGACTACTAGATTCAATTTCTTCATGGCCGTCTTCA
CATACCCATTCTTTACCGGTTCAGAGCTGTGATCTTTATTTTTAACAGCCACAATCATGGTTTGTG
TTTCCAGTGTTATGATCTGAGTGAAGTTCGTTCTTTTTCTCGTGACCCAGGCTTGATACTAGGCCG
GACCTTTCTGAGGTGGAAGAGATCTATACATTTGAGGCCTATTTTGTGTAGCCATGTGTGGAGGCC
CAGAACATTTGAAGCCTGCCAGCCCACACGAAGGAGAAGATAAAGTCAAAATGGCCGAGAATCAGT
CTATCAAAGTGAAGGAATTGTCTGAATCTTGTTCAAGTCTACATGAACTAGCTGCTAATAATGACC
TTATTGGCTTTAAGAAAGCAATGGAGGAAGAAGGGTCAAAGATAGATGAGGTTAACTTTTGGTACG
GGAGGCAGAATGGTTCTAATCAGATGGTCCTGGAGCAAAGGACTCCATTGATGGTTGCTGCACTTT
ATGGCAGTGTAGATGCGCTGAGTTACATCTTATCCATTTATGTAACTTGTGGAGCAGATGTTAACC
AAGCCTGTGGGTCAGATAACTCCACTGCCTTGCATTGTGCGGCTGTGGGAGGGTCTGCCTGTGCAG
TTGAAACTGTAAAATTGTTACTTCATGCAGGCAGTGATGTGAATCGCTTGGATGCTTATGGCAGAA
GACCAGCAGATGTGATTATGGTTTCTCCTAAGCTAACCGAAATCAAGGCCAAGCTAGAAGAAATGT
TAAACGCAGCTGGTTCATGTCAAACTTCTCCGGCAAAGTTGCCTAACATAGTTTCAGGGCCACCTG
GGTTTGAGTCAAAGGGGATGGAGTCCATGTCCCCATTGCCATTGTTGCCTCTTTCATTGTCTTTAG
AAGCATCCAATAATAGATCAGGTTGTGTGAATTCTCCAACATCTTCGCCAAAGTCCATGGAAGCAT
TAAAGGGTTTCGGTGATGTTAATGAGAAGAAGGAATATCCTGTGGACCCTTCTTTTCCAGACATAA
AGAATAGCATCTATACTACAGATGAATTTCGGATGTTTTCCTTCAAGGTGCGGCCATGTTCACGGG
CATATTCTCATGATTGGACTGAATGCCCATTTGTGCATCCTGGTGAAAATGCCAGAAGGCGGGATC
CAAGAAGGTATCATTATAGCTGTGTTCCTTGCCCAGATTTTCGGAAAGGGACTTGTAGGCGCAGTG
ATGTTTGTGAATATGCACACGGTGTTTTTGAGTGCTGGTTACATCCTGCTCAATATAGGACACGGT
TGTGCAAAGATGGGACTAATTGTTCACGTAGAGTTTGCTTCTTTGCTCACACATCTGAGGAACTAC
GCCCTCTCATTGTCTCTACTGGGTCTGCTGTTCCATCCCCAAGGGCATCATCATCTCTGGACATGA
CATCTGTCATGAGTCCTCTTGCCCCTGGTTCTCCCTCTTCAGTTTCAATGATGTCACCCTTCCTAT
CAAATCCTCAGCAAGGCAGTGTGCTTACTCCGCCTATGTCTCCATCAGCGTCCTCTGTAAATGGAT
ATGGAGGCTGGCCACAGCCTAATGTACCAACCTTACACCTTCCTGGTAGCAATGTTCAAACCAGCC
GTCTTAGAGCGGAACTTAATGCCAGAGACATGCCTGTTGAGGATTCTCCTCGAATTTCAGACTATG
AAGGGCAGCAACTCCTGAATGATTTTTCTCCACTGTCCACACAAGCCAGGCTGAATGCTGCTGCTG
CTGTTATATCTGGTGGCGGGAACACCACAACAAGGTCTGGAAAATACAAGAGTCACGGGATCAATA
CTGTTGCTCCAACGAATCTTGAAGACTTGTTTGCCTCCGAGGTAACATCTCCTAGAGTAGCAGTTC
TTGAACCTTCCATCTTTTCTCAGATGAGTCCCCAAATGCAAGCTCATAAGACTGCCCAGGCATATA
TGCAGATTCAAAACCAGATGCTGCCTCCTATAAATACACAGGCATTTTCGCAGGGAATTACACAGA
TGCAGCAGGCTGCAATAGAGCCTCAGAGCCCTGGACATTCTTTGATGCAATCACCTTTCCAATCTT
CCTCGTATGGGTTGGGATCCCCTGGTAGAATGTCACCTCGTTGTGTGGATGTGGAACGTCATAATA
CATGTGGGTCTCCCTTATCACCGGCTATGGCTGCAACGATAAATTCAAGAATGGCTATGGCTGCTT

FIGURE 25 (continued)

TTGTTCAGAGGGAAAAACGGAGCCATAGTTCCCGTGACTTGGGAGCTAATGTGAATCCCAGTTCAT
GGTCTGATTGGGGCTCGCCTACAGGTAAAGTTGACTGGGGGGTTCAAGGAGAAGAGTTGAGCAAAT
TAAGAAAGTCGGCTTCATTTGGTCCCCGCAGTTATGAAGAACCGGATTTGTCTTGGGTTCAAACAC
TGGTAAAGGAAACTACACCAGAGGGTAAAGATGGAGGAAATGTAAGCTGTTCTGGGGAAACTCCAC
ACAAGGGGCAAATAGAAAATGTTGATCATTCAGTTTTGGGTGCCTGGATTGAACAGATGCAGCTTG
ATCAGATTGTAGCTTGAGATTAGGATTATTTATTTGGAGTGGTGGTAGGGATAGGCTCATTTAAAA
TTCAATTTCTCATTTTTTACTATTTCTTTTATAAAAATTCCCCATTATAGTTTAGGAAATAGTCTG
GTTTTCTACCTATTATCAGAATTACACCTGCAGGAAATTTTGGAGGAAAGCATGCAAAAAGTAGAT
AGGGATGTTATTCCTATCAGCAGGTTGACAAGCTGAAAATCACTTGGGTGGTAGACCAGAGAATGA
CACTATTTTTTGTTGACATGGCAACTGAAGATGCTGTTTTCTTTACTTATCATTAACAACCCTATA
TATATTTGTTTTGAAAGAACTGAGCGGAGAAATGTTGTCAGTTGGTTACTCTGCGCAAGGCCTTGG
AAGAAATCCAAGATGTGGCATCTTGGTGCATTTTTAATTTATCAAGTGTGAAATCCATAACAGGTT
TCAGTGAGTGACTTCTGAGGTTGTATATGGAAAAACCTATGATGTTGGCTGTCTACTGCTATTTTT
CTGTGCCTAAACTGTCAACTAAAGTTTGCAGGTGGCAATTTTGTGGCAGCATATTTGCACATTGAA
GCGGATGGTCTGCACCTGCTATAGAAGTTTTCGAGTCTGTAGAATTTGATGGTGCAAGATGATTTT
CTAGTTGATATATTTGGAAGGCTTTGCCAAAGTAGTGGCATGTACATTTTGCAAAAATTTAAAGGA
TGGCAATCCATTGTTTTGCCATGTAGCTTCACTTTATTGATTAGGTGGAAAGGAATTTTGAGACAC
TTCAATTTGTGCATACTTTTGTTCTGAACTGCAAAATCAGTCTCTTGTGATGTCCTCAAGGCTATT
ATGCTCAGGGATTTGCCTAAAACCATAAGTGGCCTTAGATAAGGTACCATTGTATTACCTTTTATT
GTTTGGATATTTTATTTATGAAAGTGAATTTATTTTAAAAAAAAAA

**SEQ ID NO: 41, *Pinus radiata* transcription factor protein C3H-type family**

MCGGPEHLKPASPHEGEDKVKMAENQSIKVKELSESCSSLHELAANNDLIGFKKAMEEEGSKIDEV
NFWYGRQNGSNQMVLEQRTPLMVAALYGSVDALSYILSIYVTCGADVNQACGSDNSTALHCAAVGG
SACAVETVKLLLHAGSDVNRLDAYGRRPADVIMVSPKLTEIKAKLEEMLNAAGSCQTSPAKLPNIV
SGPPGFESKGMESMSPLPLLPLSLSLEASNNRSGCVNSPTSSPKSMEALKGFGDVNEKKEYPVDPS
FPDIKNSIYTTDEFRMFSFKVRPCSRAYSHDWTECPFVHPGENARRRDPRRYHYSCVPCPDFRKGT
CRRSDVCEYAHGVFECWLHPAQYRTRLCKDGTNCSRRVCFFAHTSEELRPLIVSTGSAVPSPRASS
SLDMTSVMSPLAPGSPSSVSMMSPFLSNPQQGSVLTPPMSPSASSVNGYGGWPQPNVPTLHLPGSN
VQTSRLRAELNARDMPVEDSPRISDYEGQQLLNDFSPLSTQARLNAAAAVISGGGNTTTRSGKYKS
HGINTVAPTNLEDLFASEVTSPRVAVLEPSIFSQMSPQMQAHKTAQAYMQIQNQMLPPINTQAFSQ
GITQMQQAAIEPQSPGHSLMQSPFQSSSYGLGSPGRMSPRCVDVERHNTCGSPLSPAMAATINSRM
AMAAFVQREKRSHSSRDLGANVNPSSWSDWGSPTGKVDWGVQGEELSKLRKSASFGPRSYEEPDLS
WVQTLVKETTPEGKDGGNVSCSGETPHKGQIENVDHSVLGAWIEQMQLDQIVA

**SEQ ID NO: 42, *Pinus radiata* transcription factor cDNA C3H-type family**

TGTTTCCAGGCGGGCACTAAAGCAAGGGAGGGGGTAGGCTTTACTTTCTGCTCTGCGCAAAGAACG
TTGAAATCAATCGCCCTGGCTGGTCTGGCGTGACTACTAGATTCAATTTCTTCATGGCCGTCTTCA
CATACCCATTCTTTACCGGTTCAGAGCTGTGATCTTTATTTTTAACAGCCACAATCATGGTTTGTG
TTTCCAGTGTTATGATCTGAGTGAAGTTCGTTCTTTTTCTCGTGACCCAGGCTTGATACTAGGCCG
GACCTTTCTGAGGTGGAAGAGATCTATACATTTGAGGCCTATTTTGTGTAGCCATGTGTGGAGGCC
CAGAACATTTGAAGCCTGCCAGCCCACACGAAGGAGAAGATAAAGTCAAAATGGCCGAGAATCAGT
CTATCAAAGTGAAGGAATTGTCTGAATCTTGTTCAAGTCTACATGAACTAGCTGCTAATAATGACC
TTATTGGCTTTAAGAAAGCAATGGAGGAAGAAGGGTCAAAGATAGATGAGGTTAACTTTTGGTACG
GGAGGCAGAATGGTTCTAATCAGATGGTCCTGGAGCAAAGGACTCCATTGATGGTTGCTGCACTTT
ATGGCAGTGTAGATGCGCTGAGTTACATCTTATCCATTTATGTAACTTGTGGAGCAGATGTTAACC

**FIGURE 25 (continued)**

AAGCCTGTGGGTCAGATAACTCCACTGCCTTGCATTGTGCGGCTGTGGGAGGGTCTGCCTGTGCAG
TTGAAACTGTAAAATTGTTACTTCATGCAGGCAGTGATGTGAATCGCTTGGATGCTTATGGCAGAA
GACCAGCAGATGTGATTATGGTTTCTCCTAAGCTAACCGAAATCAAGGCCAAGCTAGAAGAAATGT
TAAACGCAGCTGGTTCATGTCAAACTTCTCCGGCAAAGTTGCCTAACATAGTTTCAGGGCCACCTG
GGTTTGAGTCAAAGGGGATGGAGTCCATGTCCCCATTGCCATTGTTGCCTCTTTCATTGTCTTTAG
AAGCATCCAATAATAGATCAGGTTGTGTGAATTCTCCAACATCTTCGCCAAAGTCCATGGAAGCAT
TAAAGGGTTTCGGTGATGTTAATGAGAAGAAGGAATATCCTGTGGACCCTTCTTTTCCAGACATAA
AGAATAGCATCTATACTACAGATGAATTTCGGATGTTTTCCTTCAAGGTGCGGCCATGTTCACGGG
CATATTCTCATGATTGGACTGAATGCCCATTTGTGCATCCTGGTGAAAATGCCAGAAGGCGGGATC
CAAGAAGGTATCATTATAGCTGTGTTCCTTGCCCAGATTTTCGGAAAGGGACTTGTAGGCGCAGTG
ATGTTTGTGAATATGCACACGGTGTTTTTGAGTGCTGGTTACATCCTGCTCAATATAGGACACGGT
TGTGCAAAGATGGGACTAATTGTTCACGTAGAGTTTGCTTCTTTGCTCACACATCTGAGGAACTAC
GCCCTCTCATTGTCTCTACTGGGTCTGCTGTTCCATCCCCAAGGGCATCATCATCTCTGGACATGA
CATCTGTCATGAGTCCTCTTGCCCCTGGTTCTCCCTCTTCAGTTTCAATGATGTCACCCTTCCTAT
CAAATCCTCAGCAAGGCAGTGTGCTTACTCCGCCTATGTCTCCATCAGCGTCCTCTGTAAATGGAT
ATGGAGGCTGGCCACAGCCTAATGTACCAACCTTACACCTTCCTGGTAGCAATGTTCAAACCAGCC
GTCTTAGAGCGGAACTTAATGCCAGAGACATGCCTGTTGAGGATTCTCCTCGAATTTCAGACTATG
AAGGGCAGCAACTCCTGAATGATTTTTCTCCACTGTCCACACAAGCCAGGCTGAATGCTGCTGCTG
CTGTTATATCTGGTGGCGGGAACACCACAACAAGGTCTGGAAAATACAAGAGTCACGGGATCAATA
CTGTTGCTCCAACGAATCTTGAAGACTTGTTTGCCTCCGAGGTAACATCTCCTAGAGTAGCAGTTC
TTGAACCTTCCATCTTTTCTCAGATGAGTCCCCAAATGCAAGCTCATAAGACTGCCCAGGCATATA
TGCAGATTCAAAACCAGATGCTGCCTCCTATAAATACACAGGCATTTTCGCAGGGAATTACACAGA
TGCAGCAGGCTGCAATAGAGCCTCAGAGCCCTGGACATTCTTTGATGCAATCACCTTTCCAATCTT
CCTCGTATGGGTTGGGATCCCCTGGTAGAATGTCACCTCGTTGTGTGGATGTGGAACGTCATAATA
CATGTGGGTCTCCCTTATCACCGGCTATGGCTGCAACGATAAATTCAAGAATGGCTATGGCTGCTT
TTGTTCAGAGGGAAAAACGGAGCCATAGTTCCCGTGACTTGGGAGCTAATGTGAATCCCAGTTCAT
GGTCTGATTGGGGCTCGCCTACAGGTAAAGTTGACTGGGGGGTTCAAGGAGAAGAGTTGAGCAAAT
TAAGAAAGTCGGCTTCATTTGGTCCCCGCAGTTATGAAGAACCGGATTTGTCTTGGGTTCAAACAC
TGGTAAAGGAAACTACACCAGAGGGTAAAGATGGAGGAAATGTAAGCTGTTCTGGGGAAACTCCAC
ACAAGGGGCAAATAGAAAATGTTGATCATTCAGTTTTGGGTGCCTGGATTGAACAGATGCAGCTTG
ATCAGATTGTAGCTTGAGATTAGGATTATTTATTTGGAGTGGTGGTAGGGATAGGCTCATTTAAAA
TTCAATTTCTCATTTTTTACTATTTCTTTTATAAAAATTCCCCATTATAGTTTAGGAAATAGTCTG
GTTTTCTACCTATTATCAGAATTACACCTGCAGGAAATTTTGGAGGAAAGCATGCAAAAAGTAGAT
AGGGATGTTATTCCTATCAGCAGGTTGACAAGCTGAAAATCACTTGGGTGGTAGACCAGAGAATGA
CACTATTTTTTGTTGACATGGCAACTGAAGATGCTGTTTTCTTTACTTATCATTAACAACCCTATA
TATATTTGTTTTGAAAGAACTGAGCGGAGAAATGTTGTCAGTTGGTTACTCTGCGCAAGGCCTTGG
AAGAAATCCAAGATGTGGCATCTTGGTGCATTTTTAATTTATCAAGTGTGAAATCCATAACAGGTT
TCAGTGAGTGACTTCTGAGGTTGTATATGGAAAAACCTATGATGTTGGCTGTCTACTGCTATTTTT
CTGTGCCTAAACTGTCAACTAAAGTTTGCAGGTGGCAATTTTGTGGCAGCATATTTGCACATTGAA
GCGGATGGTCTGCACCTGCTATAGAAGTTTTCGAGTCTGTAGAATTTGATGGTGCAAGATGATTTT
CTAGTTGATATATTTGGAAGGCTTTGCCAAAGTAGTGGCATGTACATTTTGCAAAAATTTAAAGGA
TGGCAATCCATTGTTTTGCCATGTAGCTTCACTTTATTGATTAGGTGGAAAGGAATTTTGAGACAC
TTCAATTTGTGCATACTTTTGTTCTGAACTGCAAAATCAGTCTCTTGTGATGTCCTCAAGGCTATT
ATGCTCAGGGATTTGCCTAAAACCATAAGTGGCCTTAGATAAGGTACCATTGTATTACCTTTTATT
GTTTGGATATTTTATTTATGAAAGTGAATTTATTTTAAAAAAAAAA

**FIGURE 25 (continued)**

**SEQ ID NO: 43, *Pinus radiata* transcription factor protein C3H-type family**
MKEMAEYCSPALLELAANNDLSGFKQAVEEGGSSVNERGLWYGRQIGSGQKMVLEQRTPLMVAALY
GSLDVLSYMLSGGRVDVNQSCGSDMSTALHCAAAGGSILAIETVGMLIKAGADVNFMNAGGRKPAD
VIMVSPKLAHFKNVLEDLLIMGSNSPMKIPCRVSGSGFYLPEGGGCFFDEHGCVVSVPTSSPLFSS
PDATSPATVNSPLSSPPTSLDTPKNLCDCGQKKEFAVDSSLPDIKNSIYSTDEFRMYSFKVRPCSR
AYSHDWTECPFVHPGENARRRDPRKYHYSCVPCPDFRKGACRRGDVCEYAHGVFECWLHPAQYRTR
LCKDGTNCSRRVCFFAHTPEELRPLYPPACSSMLSQRTTMTSSDKMAVMHPLAPGSASSVLMMSSS
NSSQSSFPNSPVSPLSSANTSSHSSFGGGSWAHPNLPTLHLSNGALQASRLRTAVNARDMHPDCSI
ESGDYEGQLLNEFAYLSTQARGNGPMATVSSSGNTPCRPRKFRAHNVAPTNLEDLFASEVFSPKMT
ASESAFLSEIQSHKSAQLSPQLQSQMLSSFNTQVYPQGSTQGQMHMQHGGVDCQSPSVFLSPPPVQ
LASYSLSSLGPLSSLTGELERQNSNGSPLSPIMSTAADSRAVAFSQRDKGSSRSGDLGGATTWSEW
GSPTGKVNWGIRGEELQKFRKSASFGIRSSDEPDLSWVQKLFKEAPMESMDRGTMGRSMDIANSVQ
MEATDLGGWISQINPDQVAPLTL

**SEQ ID NO: 44, *Glycine max* C3H Zn finger containing protein**
KSANDKEMKSLTVNTEDSFSSLLELASNNDIEGFKVLLEKDSSSINEVGLWYGRQNGSKQFVLEHR
TPLMVAATYGSIDVMKIILLCPEADVNFACGANKTTALHCAASGGSANAVDAVKILLSAGADVNGV
DANGNRPIDVIAVPPKLQGAKAVLEELLSDSASEGSIGEFSVPVSVNTSSLGSPGHSSNGMPYTPS
SSPPSPVVAKFTDAAVCSLSEKKEYPIDPSLPDIKNSIYATDEFRMFSFKVRPCSRAYSHDWTECP
FVHPGENARRRDPRKFHYSCVPCPDFRKGACRRGDMCEYAHGVFECWLHPAQYRTRLCKDGTSCNR
RVCFFAHTAEELRPLYVSTGSAVPSPRSSASAPNVMDMAAAMSLLPGSPSSVSSMSPSHFGQPMSP
SANGMSLSSAWAQPNVSALHLPGSNLQSSRLRSSLSARDMPPDDLNMMSDLDGQQQHPLNDLSCYL
QPRPGAGSVSRSGRSKILTPSNLEDLFSAEISSSPRYSDPAAGSVFSPTHKSAVLNQFQQLQSMLS
PINTNLLSPKNVEHPLLQASFGVSPSGRMSPRSVEPISPMSSRISAFAQREKQQQQQQLRSLSSR
DLGANSPASLVGSPANPWSKWGSPNGKADWSVNGDTLGRQMRRSSSFELKNNGEEPDLSWVQSLVK
ESPPEMIKEKFASPMPTASADGPNSNSQIESIDHSVLGAWLEQMQLDQLVV

**SEQ ID NO: 45, *Glycine max* C3H Zn finger containing protein**
RGSGFPGRPTRPRSGRTRGRTRGVNFACGANKTTALHCAASGASTKAVDAVKLLLSAGADVNCVDA
NGNRPIDVIAVPPKLQGAKAVLEELLSDNASDVSVGEFSVPVSVNSSSPGSPAHSSNGMPYTPSVS
PPSPVAAKFTDAAICSLSEKAREYPIDPSLPDIKNSIYATDEFRMFSFKVRPCSRAYSHDWTECPF
VHPGENARRRDPRKFHYSCVPCPDFRKGACRRGDMCEYAHGVFECWLHPAQYRTRLCKDGTSCNRR
VCFFAHTAEELRPLYVSTGSAAPSPRSSASGPNVMDMAAAMSLFPGSPSSGSSISLSISFSLDPMS
PSANGMPLSSAWAQPNVPALHLPGSNLQSSRLRSSLSARDIPPEDLNMMSDLDGQQQHHLNDLSCY
IQPRPGASSVSRSGRSKTLTPSNLEELFSAEISLSPRYSDPAAGSVFSPTHKSAVLNQFQQLQSML
SPINTNLLSPKNVEHPLFQASFGVSPSGRMSPRSVEPISPMSARLSAFAQREKQQQQLRSVSSRDL
GANSPASLVGSPANPWSKWGSPIGKADWSVNGDSLGRQMRRSSSFERKNNGEEPDLSWVQSLVKES
PPEMIKEKFASPMPTASADGPNSNSQIESIDHSVLGAWLEQMQLDQLVV

**SEQ ID NO: 46, *Glycine max* C3H Zn finger containing protein**
SHEMNHLSLDTEDSLASLLLELAANNDVSGFKRLIECEPSSIDEVGLWYGRHKESKKMVNEQRTPL
MVAATYGSIDVMTLILSLSEADVNRSSGLDKSTALHCAASGGSENAVDAVKLLLEAGADRNSVDAN
GRRPGDVIVSPPKLDYVKKSLEELLGSDDWSLLRVMRSTCNGCSAEDLKMKTNEVSEKKEYPVDLS
LPDIKNSIYSSDEFRMYSFKVRPCSRAYSHDWTECPFVHPGENARRRDPRKFHYSCVPCPEFRKGA
CRRGDMCEYAHGVFECWLHPAQYRTRLCKDGTNCARRVCFFAHTNEELRPLYVSTGSAVPSPRSSA
SSAMDFVAAISPSSMSVMSPSPFTPPMSPSSASIAWPQPNIPALHLPGSNFHSSRLRSSLNARDFS
VDDFDLLLPDYDHHHHQQQQQQFLNELSCLSPHAMNCNTMNRSGRMKPLTPSNLDDLFSAESSSPR

**FIGURE 25 (continued)**

YADPALASAVFSPTHKSAVFNQFQHQQSMLAPLNTNFASKNFEHPLLQASLGMSPRNVEPISPMGS
RISMLAQREKQQFRSLSFRELGSNSAAASADSWSKWGSPNVKLDWPVGAGEVGKLRRSSSFELGNN
GEEPDLSWVQSLVKESPAEVKDKLATTVSYVAAAAAGSSSEGSNISTQMESVVDHAVLGAWLEQMQ
LDHLVAQQN

**SEQ ID NO: 47, At3g55980, variant of SEQ ID NO: 25**
MCSGPKSNLCSSRTLTEIESRQKEEETMLLLEFAACDDLDSFKREVEEKGLDLDESGLWYCRRVGS
KKMGLEERTPLMVAAMYGSIKVLTFIVSTGKSDVNRACGEERVTPLHCAVAGCSVNMIEVINVLLD
ASALVNSVDANGNQPLDVFVRVSRFVASPRRKAVELLLRGGGVGGLIDEAVEEEIKIVSKYPADAS
LPDINEGVYGSDEFRMYSFKVKPCSRAYSHDWTECAFVHPGENARRRDPRKYPYTCVPCPEFRKGS
CPKGDSCEYAHGVFESWLHPAQYKTRLCKDETGCARKVCFFAHKREEMRPVNASTGSAVAQSPFSS
LEMMPGLSPLAYSSGVSTPPVSPMANGVPSSPRNGGSWQNRVNTLTPPALQLNGGSRLKSTLSARD
IDMEMEMELRLRGFGNNVEETFGSYVSSPSRNSQMGQNMNQHYPSSPVRQPPSQHGFESSAAAAVA
VMKARSTAFAKRSLSFKPATQAAPQSNLSDWGSPNGKLEWGMKGEELNKMRRSVSFGIHGNNNNNA
ARDYRDEPDVSWVNSLVKDSTVVSERSFGMNERVRIMSWAEQMYREKEQTVV

**SEQ ID NO: 48, *Arabidopsis thaliana* C3H Zn finger containing protein**
MCCGSDRLNQIVSSRSSLPISFEEDNNLVTNTDMNHITVETEDTFASLLELAANNDVEGVRLSIER
DPSCVDEAGLWYGRQKGSKAMVNDYRTPLMVAATYGSIDVIKLIVSLTDADVNRACGNDQTTALHC
AASGGAVNAIQVVKLLLAAGADLNLLDAEGQRAGDVIVVPPKLEGVKLMLQELLSADGSSTAERNL
RVVTNVPNRSSSPCHSPTGENGGSGSGSPLGSPFKLKSTEFKKEYPVDPSLPDIKNSIYATDEFRM
YSFKVRPCSRAYSHDWTECPFVHPGENARRRDPRKFHYSCVPCPDFRKGACRRGDMCEYAHGVFEC
WLHPAQYRTRLCKDGTGCARRVCFFAHTPEELRPLYASTGSAVPSPRSNADYAAALSLLPGSPSGV
SVMSPLSPSAAGNGMSHSNMAWPQPNVPALHLPGSNLQSSRLRSSLNARDIPTDEFNMLADYEQQQ
LLNEYSNALSRSGRMKSMPPSNLEDLFSAEGSSSPRFTDSALASAVFSPTHKSAVFNQFQQQQQQQ
QQQSMLSPINTSFSSPKSVDHSLFSGGGRMSPRNVVEPISPMSARVSMLAQCVKQQQQQQQQQQQQ
QHQFRSLSSRELRTNSSPIVGSPVNNNTWSSKWGSSNGQPDWGMSSEALGKLRSSSSFDGDEPDVS
WVQSLVKETPAEAKEKAATSSSGEHVMKQPNPVEPVMDHAGLEAWIEQMQLDQLVAQQN

**SEQ ID NO: 49, *Arabidopsis thaliana*, C3H Zn finger containing protein**
MNHITVETEDTFASLLELAANNDVEGVRLSIERDPSCVDEAGLWYGRQKGSKAMVNDYRTPLMVAA
TYGSIDVIKLIVSLTDADVNRACGNDQTTALHCAASGGAVNAIQVVKLLLAAGADLNLLDAEGQRA
GDVIVVPPKLEGVKLMLQELLSADGSSTAERNLRVVTNVPNRSSSPCHSPTGENGGSGSGSPLGSP
FKLKSTEFKKEYPVDPSLPDIKNSIYATDEFRMYSFKVRPCSRAYSHDWTECPFVHPGENARRRDP
RKFHYSCVPCPDFRKGACRRGDMCEYAHGVFECWLHPAQYRTRLCKDGTGCARRVCFFAHTPEELR
PLYASTGSAVPSPRSNADYAAALSLLPGSPSGVSVMSPLSPSAAGNGMSHSNMAWPQPNVPALHLP
GSNLQSSRLRSSLNARDIPTDEFNMLADYEQQQLLNEYSNALSRSGRMKSMPPSNLEDLFSAEGSS
SPRFTDSALASAVFSPTHKSAVFNQFQQQQQQQQQQSMLSPINTSFSSPKSVDHSLFSGGGRMSPR
NVVEPISPMSARVSMLAQCVKQQQQQQQQQQQQQQHQFRSLSSRELRTNSSPIVGSPVNNNTWSSKW
GSSNGQPDWGMSSEALGKLRSSSSFDGDEPDVSWVQSLVKETPAEAKEKAATSSSGEHVMKQPNPV
EPVMDHAGLEAWIEQMQLDQLVAQQN

**SEQ ID NO: 50, *Arabidopsis thaliana*, C3H Zn finger containing protein**
MVNDYRTPLMVAATYGSIDVIKLIVSLTDADVNRACGNDQTTALHCAASGGAVNAIQVVKLLLAAG
ADLNLLDAEGQRAGDVIVVPPKLEGVKLMLQELLSADGSSTAERNLRVVTNVPNRSSSPCHSPTGE

**FIGURE 25 (continued)**

NGGSGSGSPLGSPFKLKSTEFKKEYPVDPSLPDIKNSIYATDEFRMYSFKVRPCSRAYSHDWTECP
FVHPGENARRRDPRKFHYSCVPCPDFRKGACRRGDMCEYAHGVFECWLHPAQYRTRLCKDGTGCAR
RVCFFAHTPEELRPLYASTGSAVPSPRSNADYAAALSLLPGSPSGVSVMSPLSPSAAGNGMSHSNM
AWPQPNVPALHLPGSNLQSSRLRSSLNARDIPTDEFNMLADYEQQQLLNEYSNALSRSGRMKSMPP
SNLEDLFSAEGSSSPRFTDSALASAVFSPTHKSAVFNQFQQQQQQQQQSMLSPINTSFSSPKSVD
HSLFSGGGRMSPRNVVEPISPMSARVSMLAQCVKQQQQQQQQQQQQHQFRSLSSRELRTNSSPIV
GSPVNNNTWSSKWGSSNGQPDWGMSSEALGKLRSSSSFDGDEPDVSWVQSLVKETPAEAKEKAATS
SSGEHVMKQPNPVEPVMDHAGLEAWIEQMQLDQLVAQQN

**SEQ ID NO: 51, *Arabidopsis thaliana*, C3H Zn finger containing protein**

MNDAAEWEHSFSALLEFAADNDVEGFRRQLSDVSCINQMGLWYRRQRFVRRMVLEQRTPLMVASLY
GSLDVVKFILSFPEAELNLSCGPDKSTALHCAASGASVNSLDVVKLLLSVGADPNIPDAHGNRPVD
VLVVSPHAPGLRTILEEILKKDEIISEDLHASSSSLGSSFRSLSSSPDNGSSLLSLDSVSSPTKPH
GTDVTFASEKKEYPIDPSLPDIKSGIYSTDEFRMFSFKIRPCSRAYSHDWTECPFAHPGENARRRD
PRKFHYTCVPCPDFKKGSCKQGDMCEYAHGVFECWLHPAQYRTRLCKDGMGCNRRVCFFAHANEEL
RPLYPSTGSGLPSPRASSAVSASTMDMASVLNMLPGSPSAAQHSFTPPISPSGNGSMPHSSMGWPQ
QNIPALNLPGSNIQLSRLRSSLNARDIPSEQLSMLHEFEMQRQLAGDMHSPRFMNHSARPKTLNPS
NLEELFSAEVASPRFSDQLAVSSVLSPSHKSALLNQLQNNKQSMLSPIKTNLMSSPKNVEQHSLLQ
QASSPRGGEPISPMNARMKQQLHSRSLSSRDFGSSLPRDLMPTDSGSPLSPWSSWDQTHGSKVDWS
VQSDELGRLRKSHSLANNPNREADVSWAQQMLKDSSSPRNGNRVVNMNGARPLTQGGSSVNPHNSD
TRESDILDAWLEQLHLDR

**SEQ ID NO: 52, *Arabidopsis thaliana*, C3H Zn finger containing protein**

MGLWYRRQRFVRRMVLEQRTPLMVASLYGSLDVVKFILSFPEAELNLSCGPDKSTALHCAASGASV
NSLDVVKLLLSVGADPNIPDAHGNRPVDVLVVSPHAPGLRTILEEILKKDEIISEDLHASSSSLGS
SFRSLSSSPDNGSSLLSLDSVSSPTKPHGTDVTFASEKKEYPIDPSLPDIKSGIYSTDEFRMFSFK
IRPCSRAYSHDWTECPFAHPGENARRRDPRKFHYTCVPCPDFKKGSCKQGDMCEYAHGVFECWLHP
AQYRTRLCKDGMGCNRRVCFFAHANEELRPLYPSTGSGLPSPRASSAVSASTMDMASVLNMLPGSP
SAAQHSFTPPISPSGNGSMPHSSMGWPQQNIPALNLPGSNIQLSRLRSSLNARDIPSEQLSMLHEF
EMQRQLAGDMHSPRFMNHSARPKTLNPSNLEELFSAEVASPRFSDQLAVSSVLSPSHKSALLNQLQ
NNKQSMLSPIKTNLMSSPKNVEQHSLLQQASSPRGGEPISPMNARMKQQLHSRSLSSRDFGSSLPR
DLMPTDSGSPLSPWSSWDQTHGSKVDWSVQSDELGRLRKSHSLANNPNREADVSWAQQMLKDSSSP
RNGNRVVNMNGARPLTQGGSSVNPHNSDTRESDILDAWLEQLHLDR

**SEQ ID NO: 53, AtAZ (ORF: 170 - 1909), *Arabidopsis thaliana*, At3g55980**

TGCTCCTTCTTCATTGCAAAGAAAGCACCACCTTTTAAAGAATCCTCCTCACACTCCATTCTTCTT
AAAAAACCCACCACAACACAATTCCCACTTGTTTCTTCATCATCACCTACTTCAATCAAAAAACAT
TCCAACTTTCTTCTCAATTTCATTCCAGGATAGTATTATGTGCAGTGGACCAAAGAGCAATCTCTG
CTCTTCAAGAACCTTAACAGAAATCGAATCAAGGCAAAAGGAAGAAGAAACAATGCTTCTCCTCGA
ATTCGCTGCTTGTGATGATCTTGACTCGTTCAAGAGAGAGGTTGAAGAGAAAGGGCTTGATTTGGA
TGAGTCAGGGTTATGGTATTGCAGACGTGTCGGTTCTAAGAAGATGGGTCTTGAAGAAAGAACACC
TTTAATGGTTGCAGCTATGTATGGAAGCATAAAGGTTTTGACTTTCATCGTTTCCACTGGAAAATC
TGATGTGAACAGAGCTTGTGGTGAAGAGAGAGTTACTCCGCTTCACTGTGCTGTTGCTGGCTGTTC
TGTGAATATGATTGAAGTCATCAATGTCTTGCTTGATGCTTCTGCTTTGGTTAACTCTGTTGATGC
TAATGGGAATCAACCTTTGGATGTGTTTGTTCGAGTTTCGAGGTTTGTGGCTAGTCCGAGGAGGAA

## FIGURE 25 (continued)

AGCGGTTGAGTTGTTGCTGAGAGGAGGAGGTGTTGGAGGATTGATCGATGAGGCGGTTGAAGAAGA
GATCAAGATTGTCTCTAAGTATCCAGCTGATGCTTCTTTACCGGATATAAACGAAGGGGTTTATGG
AAGTGATGAGTTTAGGATGTATAGCTTTAAGGTTAAGCCATGTTCTAGGGCTTATTCTCATGATTG
GACCGAGTGTGCTTTTGTTCATCCGGGAGAAAATGCGAGGAGGAGAGATCCGAGGAAGTATCCTTA
CACTTGTGTCCCCTGTCCCGAGTTCCGTAAAGGATCATGCCCGAAAGGAGATTCTTGCGAGTATGC
TCACGGGGTTTTCGAGTCGTGGCTTCACCCCGCGCAGTATAAAACCCGGCTTTGTAAAGATGAAAC
GGGTTGTGCAAGGAAAGTTTGTTTCTTTGCTCATAAACGCGAAGAGATGAGACCTGTTAATGCTTC
AACTGGCTCTGCCGTGGCTCAGTCTCCGTTTAGCAGCTTGGAGATGATGCCAGGGTTGTCTCCTCT
TGCTTATTCTTCAGGAGTTTCGACTCCTCCGGTTTCTCCAATGGCTAATGGTGTTCCTTCCTCTCC
AAGAAACGGCGGATCATGGCAGAACAGAGTCAATACCCTTACTCCACCGGCTTTGCAGCTCAATGG
TGGAAGCAGATTGAAGTCCACACTGAGCGCTAGAGATATCGATATGGAGATGGAGATGGAATTGAG
ACTCCGCGGTTTTGGCAACAATGTGGAAGAGACGTTCGGGTCTTATGTTTCCTCTCCAAGTAGGAA
TTCTCAAATGGGTCAAAACATGAACCAACATTATCCATCTTCCCCGGTGAGACAACCGCCATCTCA
ACACGGGTTCGAATCTTCAGCAGCTGCAGCGGTTGCAGTGATGAAAGCGAGATCAACCGCCTTTGC
GAAACGTAGCTTGAGCTTCAAACCAGCTACTCAAGCAGCACCACAGTCGAATCTCTCGGATTGGGG
ATCTCCAAACGGGAAGCTGGAATGGGGAATGAAAGGAGAAGAGCTGAATAAGATGAGAAGAAGTGT
TTCCTTTGGAATCCATGGAAACAACAACAATAACGCAGCTAGAGACTACAGGGACGAGCCAGATGT
GTCATGGGTTAACTCTTTAGTTAAAGACAGTACTGTGGTGTCTGAGAGAAGCTTTGGAATGAATGA
GAGGGTTCGGATAATGTCGTGGGCTGAGCAAATGTACAGAGAGAAGGAGCAGACTGTGGTGTAAAC
ACACACAAAGATGGTTTCTTATATATATTGCTTTTGGGCCATCTCTGCAAATTTGATTCTTTAATT
TTTGTGACTTTCTTTAGTTGTTACTGTTATTAGTAGTATATGGTTTGTTGTCACTACGAGTCTACG
TGATGAAAAGATAGAAGTTAATTGCATTAGTTTCTATATTCGTTTCTCATCCTCTTGTAATTTATC
AAACCATGAAATGGCTAAGCAATCCAAACCGAAAAAAAAAAAAAAA

**SEQ ID NO: 54, rice GOS2 promoter**
AATCCGAAAAGTTTCTGCACCGTTTTCACCCCCTAACTAACAATATAGGGAACGTGTGCTAAATAT
AAAATGAGACCTTATATATGTAGCGCTGATAACTAGAACTATGCAAGAAAAACTCATCCACCTACT
TTAGTGGCAATCGGGCTAAATAAAAAAGAGTCGCTACACTAGTTTCGTTTTCCTTAGTAATTAAGT
GGGAAAATGAAATCATTATTGCTTAGAATATACGTTCACATCTCTGTCATGAAGTTAAATTATTCG
AGGTAGCCATAATTGTCATCAAACTCTTCTTGAATAAAAAAATCTTTCTAGCTGAACTCAATGGGT
AAAGAGAGAGATTTTTTTTAAAAAAATAGAATGAAGATATTCTGAACGTATTGGCAAAGATTTAAA
CATATAATTATATAATTTTATAGTTTGTGCATTCGTCATATCGCACATCATTAAGGACATGTCTTA
CTCCATCCCAATTTTTATTTAGTAATTAAAGACAATTGACTTATTTTTATTATTTATCTTTTTTCG
ATTAGATGCAAGGTACTTACGCACACACTTTGTGCTCATGTGCATGTGTGAGTGCACCTCCTCAAT
ACACGTTCAACTAGCAACACATCTCTAATATCACTCGCCTATTTAATACATTTAGGTAGCAATATC
TGAATTCAAGCACTCCACCATCACCAGACCACTTTTAATAATATCTAAAATACAAAAAAATAATTTT
ACAGAATAGCATGAAAAGTATGAAACGAACTATTTAGGTTTTTCACATACAAAAAAAAAAAGAATT
TTGCTCGTGCGCGAGCGCCAATCTCCCATATTGGGCACACAGGCAACAACAGAGTGGCTGCCCACA
GAACAACCCACAAAAAACGATGATCTAACGGAGGACAGCAAGTCCGCAACAACCTTTTAACAGCAG
GCTTTGCGGCCAGGAGAGAGGAGGAGAGGCAAAGAAAACCAAGCATCCTCCTCCTCCCATCTATAA
ATTCCTCCCCCCTTTTCCCCTCTCTATATAGGAGGCATCCAAGCCAAGAAGAGGGAGAGCACCAAG
GACACGCGACTAGCAGAAGCCGAGCGACCGCCTTCTTCGATCCATATCTTCCGGTCGAGTTCTTGG
TCGATCTCTTCCCTCCTCCACCTCCTCCTCACAGGGTATGTGCCCTTCGGTTGTTCTTGGATTTAT
TGTTCTAGGTTGTGTAGTACGGGCGTTGATGTTAGGAAAGGGGATCTGTATCTGTGATGATTCCTG
TTCTTGGATTTGGGATAGAGGGGTTCTTGATGTTGCATGTTATCGGTTCGGTTTGATTAGTAGTAT
GGTTTTCAATCGTCTGGAGAGCTCTATGGAAATGAAATGGTTTAGGGTACGGAATCTTGCGATTTT
GTGAGTACCTTTTGTTTGAGGTAAAATCAGAGCACCGGTGATTTTGCTTGGTGTAATAAAAGTACG
GTTGTTTGGTCCTCGATTCTGGTAGTGATGCTTCTCGATTTGACGAAGCTATCCTTTGTTTATTCC

## FIGURE 25 (continued)

CTATTGAACAAAAATAATCCAACTTTGAAGACGGTCCCGTTGATGAGATTGAATGATTGATTCTTA
AGCCTGTCCAAAATTTCGCAGCTGGCTTGTTTAGATACAGTAGTCCCCATCACGAAATTCATGGAA
ACAGTTATAATCCTCAGGAACAGGGGATTCCCTGTTCTTCCGATTTGCTTTAGTCCCAGAATTTTT
TTTCCCAAATATCTTAAAAAGTCACTTTCTGGTTCAGTTCAATGAATTGATTGCTACAAATAATGC
TTTTATAGCGTTATCCTAGCTGTAGTTCAGTTAATAGGTAATACCCCTATAGTTTAGTCAGGAGAA
GAACTTATCCGATTTCTGATCTCCATTTTTAATTATATGAAATGAACTGTAGCATAAGCAGTATTC
ATTTGGATTATTTTTTTTATTAGCTCTCACCCCTTCATTATTCTGAGCTGAAAGTCTGGCATGAAC
TGTCCTCAATTTTGTTTTCAAATTCACATCGATTATCTATGCATTATCCTCTTGTATCTACCTGTA
GAAGTTTCTTTTTGGTTATTCCTTGACTGCTTGATTACAGAAAGAAATTTATGAAGCTGTAATCGG
GATAGTTATACTGCTTGTTCTTATGATTCATTTCCTTTGTGCAGTTCTTGGTGTAGCTTGCCACTT
TCACCAGCAAAGTTC

**SEQ ID NO: 55, rice wsi18 promoter**
GCTTGAGTCATAGGGAGAAAACAAATCGATCATATTTGACTCTTTTCCCTCCATCTCTCTTACCGG
CAAAAAAAGTAGTACTGGTTTATATGTAAAGTAAGATTCTTTAATTATGTGAGATCCGGCTTAATG
CTTTTCTTTTGTCACATATACTGCATTGCAACAATTGCCATATATTCACTTCTGCCATCCCATTAT
ATAGCAACTCAAGAATGGATTGATATATCCCCTATTACTAATCTAGACATGTTAAGGCTGAGTTGG
GCAGTCCATCTTCCCAACCCACCACCTTCGTTTTTCGCGCACATACTTTTCAAACTACTAAATGGT
GTGTTTTTTAAAAATATTTTCAATACAAAAGTTGCTTTAAAAAATTATATTGATCCATTTTTTTAA
AAAAAATAGCTAATACTTAATTAATCACGTGTTAAAAGACCGCTCCGTTTTGCGTGCAGGAGGGAT
AGGTTCACATCCTGCATTACCGAACACAGCCTAAATCTTGTTGTCTAGATTCGTAGTACTGGATAT
ATTAAATCATGTTCTAAGTTACTATATACTGAGATGAATAGAATAAGTAAAATTAGACCCACCTTA
AGTCTTGATGAAGTTACTACTAGCTGCGTTTGGGAGGACTTCCCAAAAAAAAAAGTATTAGCCATT
AGCACGTGATTAATTAAGTACTAGTTTAAAAAACTTAAAAAATAAATTAATATGATTCTCTTAAGT
AACTCTCCTATAGAAAACTTTTACAAAATTACACCGTTTAATAGTTTGGAAAATATGTCAGTAAAA
AATAAGAGAGTAGAAGTTATGAAAGTTAGAAAAAGAATTGTTTTAGTAGTATACAGTTATAAACTA
TTCCCTCTGTTCTAAAACATAAGGGATTATGGATGGATTCGACATGTACCAGTACCATGAATCGAA
TCCAGACAAGTTTTTTATGCATATTTATTCTACTATAATATATCACATCTGCTCTAAATATCTTAT
ATTTCGAGGTGGAGACTGTCGCTATGTTTTTCTGCCCGTTGCTAAGCACACGCCACCCCCGATGCG
GGGACGCCTCTGGCCTTCTTGCCACGATAATTGAATGGAACTTCCACATTCAGATTCGATAGGTGA
CCGTCGACTCCAAGTGCTTTGCACAAAACAACTCCGGCCTCCCGGCCACCAGTCACACGACTCACG
GCACTACCACCCCTGACTCCCTGAGGCGGACCTGCCACTGTTCTGCATGCGAAGCTATCTAAAATT
CTGAAGCAAAGAAAGCACAGCACATGCTCCGGGACACGCGCCACCCGGCGGAAAAGGGCTCGGTGT
GGCGATCTCACAGCCGCATATCGCATTTCACAAGCCGCCCATCTCCACCGGCTTCACGAGGCTCAT
CGCGGCACGACCGCGCACGGAACGCACGCGGCCGACCCGCGCGCCTCGATGCGCGAGCCCATCCGC
CGCGTCCTCCCTTTGCCTTTGCCGCTATCCTCTCGGTCGTATCCCGTTTCTCTGTCTTTTGCTCCC
CGGCGCGCGCCAGTTCGGAGTACCAGCGAAACCCGGACACCTGGTACACCTCCGCCGGCCACAACG
CGTGTCCCCCTACGTGGCCGCGCAGCACATGCCCATGCGCGACACGTGCACCTCCTCATCCAAACT
CTCAAGTCTCAACGGTCCTATAAATGCACGGATAGCCTCAAGCTGCTCGTCACAAGGCAAGAGGCA
AGAGGCAAGAGCATCCGTATTAACCAGCCTTTTGAGACTTGAGAGTGTGTGTGACTCGATCCAGCG
TAGTTTCAGTTCGTGTGTTGGTGAGTGATTCCAGCCAAGTTTGCG

**SEQ ID NO: 56, rice GOS2 promoter**
AATCCGAAAAGTTTCTGCACCGTTTTCACCCCCTAACTAACAATATAGGGAACGTGTGCTAAATAT
AAAATGAGACCTTATATATGTAGCGCTGATAACTAGAACTATGCAAGAAAAACTCATCCACCTACT
TTAGTGGCAATCGGGCTAAATAAAAAAGAGTCGCTACACTAGTTTCGTTTTCCTTAGTAATTAAGT
GGGAAAATGAAATCATTATTGCTTAGAATATACGTTCACATCTCTGTCATGAAGTTAAATTATTCG
AGGTAGCCATAATTGTCATCAAACTCTTCTTGAATAAAAAAATCTTTCTAGCTGAACTCAATGGGT

## FIGURE 25 (continued)

AAAGAGAGAGATTTTTTTAAAAAAATAGAATGAAGATATTCTGAACGTATTGGCAAAGATTTAAA
CATATAATTATATAATTTTATAGTTTGTGCATTCGTCATATCGCACATCATTAAGGACATGTCTTA
CTCCATCCCAATTTTTATTTAGTAATTAAAGACAATTGACTTATTTTTATTATTTATCTTTTTTCG
ATTAGATGCAAGGTACTTACGCACACACTTTGTGCTCATGTGCATGTGTGAGTGCACCTCCTCAAT
ACACGTTCAACTAGCAACACATCTCTAATATCACTCGCCTATTTAATACATTTAGGTAGCAATATC
TGAATTCAAGCACTCCACCATCACCAGACCACTTTTAATAATATCTAAAATACAAAAAATAATTTT
ACAGAATAGCATGAAAAGTATGAAACGAACTATTTAGGTTTTTCACATACAAAAAAAAAAAGAATT
TTGCTCGTGCGCGAGCGCCAATCTCCCATATTGGGCACACAGGCAACAACAGAGTGGCTGCCCACA
GAACAACCCACAAAAAACGATGATCTAACGGAGGACAGCAAGTCCGCAACAACCTTTTAACAGCAG
GCTTTGCGGCCAGGAGAGAGGAGGAGAGGCAAAGAAAACCAAGCATCCTCCTTCTCCCATCTATAA
ATTCCTCCCCCCTTTTCCCCTCTCTATATAGGAGGCATCCAAGCCAAGAAGAGGGAGAGCACCAAG
GACACGCGACTAGCAGAAGCCGAGCGACCGCCTTCTCGATCCATATCTTCCGGTCGAGTTCTTGGT
CGATCTCTTCCCTCCTCCACCTCCTCCTCACAGGGTATGTGCCTCCCTTCGGTTGTTCTTGGATTT
ATTGTTCTAGGTTGTGTAGTACGGGCGTTGATGTTAGGAAAGGGGATCTGTATCTGTGATGATTCC
TGTTCTTGGATTTGGGATAGAGGGGTTCTTGATGTTGCATGTTATCGGTTCGGTTTGATTAGTAGT
ATGGTTTTCAATCGTCTGGAGAGCTCTATGGAAATGAAATGGTTTAGGGATCGGAATCTTGCGATT
TTGTGAGTACCTTTTGTTTGAGGTAAAATCAGAGCACCGGTGATTTTGCTTGGTGTAATAAAGTAC
GGTTGTTTGGTCCTCGATTCTGGTAGTGATGCTTCTCGATTTGACGAAGCTATCCTTTGTTTATTC
CCTATTGAACAAAAATAATCCAACTTTGAAGACGGTCCCGTTGATGAGATTGAATGATTGATTCTT
AAGCCTGTCCAAAATTTCGCAGCTGGCTTGTTTAGATACAGTAGTCCCCATCACGAAATTCATGGA
AACAGTTATAATCCTCAGGAACAGGGGATTCCCTGTTCTTCCGATTTGCTTTAGTCCCAGAATTTT
TTTTCCCAAATATCTTAAAAAGTCACTTTCTGGTTCAGTTCAATGAATTGATTGCTACAAATAATG
CTTTTATAGCGTTATCCTAGCTGTAGTTCAGTTAATAGGTAATACCCCTATAGTTTAGTCAGGAGA
AGAACTTATCCGATTTCTGATCTCCATTTTTAATTATATGAAATGAACTGTAGCATAAGCAGTATT
CATTTGGATTATTTTTTTTATTAGCTCTCACCCCTTCATTATTCTGAGCTGAAAGTCTGGCATGAA
CTGTCCTCAATTTTGTTTTCAAATTCACATCGATTATCTATGCATTATCCTCTTGTATCTACCTGT
AGAAGTTTCTTTTTGGTTATTCCTTGACTGCTTGATTACAGAAAGAAATTTATGAAGCTGTAATCG
GGATAGTTATACTGCTTGTTCTTATGATTCATTTCCTTTGTGCAGTTCTTGGTGTAGCTTGCCACT
TTCACCAGCAAAGTTC

**SEQ ID NO: 57, SNH domain from SYT-like polypeptides**
IQ(Q/K)XL(D/E)(E/D)N(K/N)XLIX(C/A/K)I(L/V/M)(E/D/S)(S/N)(Q/L)NXG(K/R)XXEC(A/E/S)XXQ(A/S/Q)XL(Q/H)XNL(M/L/V)YLA(A/T)IAD

Where X is any amino acid

**SEQ ID NO: 58, *Arabidopsis thaliana* AtSYT1 SNH domain**
IQQYLDENKSLILKIVESQNSGKLSECAENQARLQRNLMYLAAIAD

**SEQ ID NO: 59, *Arabidopsis thaliana* AtSYT1 cDNA (AY102639)**
ATGCAACAGCACCTGATGCAGATGCAGCCCATGATGGCTGGTTACTACCCCAGCAATGTTACCTCT
GATCATATCCAACAGTACTTGGACGAAAACAAATCGTTGATTCTGAAGATTGTTGAGTCTCAAAAC
TCTGGAAAGCTTAGCGAATGCGCCGAGAATCAAGCAAGGCTTCAACGCAACCTAATGTACCTAGCT
GCAATAGCAGATTCTCAGCCTCAGCCACCAAGTGTGCATAGCCAGTATGGATCTGCTGGTGGTGGG
ATGATTCAGGGAGAAGGAGGGTCACACTATTTGCAGCAGCAACAAGCGACTCAACAGCAACAGATG
ACTCAGCAGTCTCTAATGGCGGCTCGATCTTCAATGTTGTATGCTCAGCAACAGCAGCAGCAGCAG
CCTTACGCGACGCTTCAGCATCAGCAATTGCACCATAGCCAGCTTGGAATGAGCTCGAGCAGCGGA
GGAGGAGGAAGCAGTGGTCTCCATATCCTTCAGGGAGAGGCTGGTGGGTTTCATGATTTTGGCCGT
GGGAAGCCGGAAATGGGAAGTGGTGGTGGCGGTGAAGGCAGAGGAGGAAGTTCAGGGGATGGTGGA
GAAACCCTTTACTTGAAATCATCAGATGATGGGAATTGA

**FIGURE 25 (continued)**

**SEQ ID NO: 60, *Arabidopsis thaliana* AtSYT1 polypeptide**
MQQHLMQMQPMMAGYYPSNVTSDHIQQYLDENKSLILKIVESQNSGKLSECAENQARLQRNLMYLA
AIADSQPQPPSVHSQYGSAGGGMIQGEGGSHYLQQQQATQQQQMTQQSLMAARSSMLYAQQQQQQQ
PYATLQHQQLHHSQLGMSSSSGGGGSSGLHILQGEAGGFHDFGRGKPEMGSGGGGEGRGGSSGDGG
ETLYLKSSDDGN

**SEQ ID NO: 61, *Arabidopsis thaliana* AtSYT2 cDNA (AY102640)**
ATGCAGCAGCAGCAGTCTCCGCAAATGTTTCCGATGGTTCCGTCGATTCCCCCTGCTAACAACATC
ACTACCGAACAGATCCAAAAGTACCTTGATGAGAACAAGAAGCTGATTATGGCCATCATGGAAAAC
CAGAATCTCGGTAAACTTGCTGAGTGCGCCCAGTACCAAGCTCTTCTCCAGAAGAACTTGATGTAT
CTTGCTGCAATTGCTGATGCTCAACCCCCACCACCTACGCCAGGACCTTCACCATCTACAGCTGTC
GCTGCCCAGATGGCAACACCGCATTCTGGGATGCAACCACCTAGCTACTTCATGCAACACCCACAA
GCATCCCCTGCAGGGATTTTCGCTCCAAGGGGTCCTTTACAGTTTGGTAGCCCACTCCAGTTTCAG
GATCCGCAACAGCAGCAGCAGATACATCAGCAAGCTATGCAAGGACACATGGGGATTAGACCAATG
GGTATGACCAACAACGGGATGCAGCATGCGATGCAACAACCAGAAACCGGTCTTGGAGGAAACGTG
GGGCTTAGAGGAGGAAAGCAAGATGGAGCAGATGGACAAGGAAAAGATGATGGCAAGTGA

**SEQ ID NO: 62, *Arabidopsis thaliana* AtSYT2 polypeptide**
MQQQQSPQMFPMVPSIPPANNITTEQIQKYLDENKKLIMAIMENQNLGKLAECAQYQALLQKNLMY
LAAIADAQPPPPTPGPSPSTAVAAQMATPHSGMQPPSYFMQHPQASPAGIFAPRGPLQFGSPLQFQ
DPQQQQIHQQAMQGHMGIRPMGMTNNGMQHAMQQPETGLGGNVGLRGGKQDGADGQGKDDGK

**SEQ ID NO: 63, *Arabidopsis thaliana* AtSYT3 cDNA (AY102641)**
ATGCAGCAATCTCCACAGATGATTCCGATGGTTCTTCCTTCATTTCCGCCCACCAATAATATCACC
ACCGAACAGATCCAAAAGTATCTTGATGAGAACAAGAAGCTGATAATGGCGATCTTGGAAAATCAG
AACCTCGGTAAACTTGCAGAATGTGCTCAGTATCAAGCTCTTCTCCAGAAGAATTTGATGTATCTC
GCTGCAATTGCGGATGCTCAACCTCAGCCACCAGCAGCTACACTAACATCAGGAGCCATGACTCCC
CAAGCAATGGCTCCTAATCCGTCATCAATGCAGCCACCACCAAGCTACTTCATGCAGCAACATCAA
GCTGTGGGAATGGCTCAACAAATACCTCCTGGGATTTTCCCTCCTAGAGGTCCATTGCAATTTGGT
AGCCCGCATCAGTTTCTGGATCCGCAGCAACAGTTACATCAACAAGCTATGCAAGGGCACATGGGG
ATTAGACCAATGGGTTTGAATAATAACAACGGACTGCAACATCAAATGCACCACCATGAAACTGCT
CTTGCCGCAAACAATGCGGGTCCTAACGATGCTAGTGGAGGAGGTAAACCGGATGGGACCAATATG
AGCCAGAGTGGAGCTGATGGGCAAGGTGGCTCAGCCGCTAGACATGGCGGTGGTGATGCAAAAACT
GAAGGAAAATGA

**SEQ ID NO: 64, *Arabidopsis thaliana* ATSYT3 POLYPEPTIDE**
MQQSPQMIPMVLPSFPPTNNITTEQIQKYLDENKKLIMAILENQNLGKLAECAQYQALLQKNLMYL
AAIADAQPQPPAATLTSGAMTPQAMAPNPSSMQPPPSYFMQQHQAVGMAQQIPPGIFPPRGPLQFG
SPHQFLDPQQQLHQQAMQGHMGIRPMGLNNNNGLQHQMHHHETALAANNAGPNDASGGGKPDGTNM
SQSGADGQGGSAARHGGGDAKTEGK

**SEQ ID NO: 65, Aspergillus officinalis SYT cDNA (CV287542)**
ATGCAGCAGCACCTGATGCAGATGCAGCCCATGATGGCAACCTACGGTTCACCGAATCAGGTCACC
ACCGATATCATTCAGCAGTATCTGGACGAGAACAAGCAGTTGATTCTGGCTATTCTTGAAAACCAA
AATTCAGGAAAAGCTGATGAATGTGCTGAGAATCAGGCTAAGCTTCAGAGGAATCTGATGTATCTT
GCAGCCATTGCGGATAGCCAGCCCCAAGTTCCTACCATTGCTCAGTATCCTCCCAACGCTGTTGCT
GCTATGCAATCGAGTGCTCGCTACATGCAACAACACCAAGCAGCTCAACAGATGACCCCTCAATCT
CTCATGGCTGCTCGCTCCTCAATGCTCTACTCACAGTCCCCAATGTCTGCACTCCAGCAGCAACAG

## FIGURE 25 (continued)

CAGCAAGCAGCAATGCATAGCCAGCTCGCCATGAGCTCCGGAGGCAACAACAGCAGCACCGGAGGA
TTCACCATTCTTCATGGTGAAGCTAGCATAGGAGGCAATGGCTCAATGAATTCTGGTGGAGTCTTT
GGAGATTTTGGACGGAGCAGCGGTGGGAAGCAAGAGACTGGGAGCGAAGGGCACGGGACAGAGACT
CCTATGTACCTGAAAGGCTCTGAAGAAGAAGGAAACTGA

**SEQ ID NO: 66, *Aspergillus officinalis* SYT polypeptide**
MQQHLMQMQPMMATYGSPNQVTTDIIQQYLDENKQLILAILENQNSGKADECAENQAKLQRNLMYL
AAIADSQPQVPTIAQYPPNAVAAMQSSARYMQQHQAAQQMTPQSLMAARSSMLYSQSPMSALQQQQ
QQAAMHSQLAMSSGGNNSSTGGFTILHGEASIGGNGSMNSGGVFGDFGRSSGGKQETGSEGHGTET
PMYLKGSEEEGN

**SEQ ID NO: 67, *Brassica napus* SYT cDNA (CD823592)**
ATGCAGCCCATGATGGCTGGTTACTACCCCAGCAATGTCACCTCTGATCATATCCAGCAGTACTTG
GATGAGAACAAGTCTTTGATTCTGAAGATAGTTGAGTCTCAAAACTCAGGAAAGCTCAGCGAGTGT
GCCGAGAATCAGGCAAGGCTTCAACGCAACCTCATGTACTTGGCTGCAATAGCAGATTCTCAGCCT
CAACCTCCAAGCGTGCATAGCCAGTATGGATCTGCTGGTGGTGGGTTGATTCAGGGAGAAGGAGCG
TCACACTATTTGCAGCAGCAACAGGCGACTCAACAGCAGCAGATGACTCAGCAGTCTCTTATGGCA
GCTCGTTCTTCAATGATGTATCAGCAGCAGCAACAGCCTTATGCAACGCTTCAGCATCAGCAGTTG
CACCATAGCCAGCTTGGGATGAGCTCTAGCAGCGGAGGAGGAAGCAGTGGTCTCCATATCCTTCAG
GGAGAGGCTGGTGGGTTTCATGAATTTGGCCGTGGGAAGCCGGAGATGGGAAGTGGTGAAGGCAGG
GGTGGAAGCTCAGGGGATGGTGGAGAAACACTCTACTTGAAGTCATCAGATGATGGGAACTGA

**SEQ ID NO: 68, *Brassica napus* SYT polypeptide**
MQQHLMQMQPMMAGYYPSNVTSDHIQQYLDENKSLILKIVESQNSGKLSECAENQARLQRNLMYLA
AIADSQPQPPSVHSQYGSAGGGLIQGEGASHYLQQQQATQQQQMTQQSLMAARSSMMYQQQQQPYA
TLQHQQLHHSQLGMSSSSGGGSSGLHILQGEAGGFHEFGRGKPEMGSGEGRGGSSGDGGETLYLKS
SDDGN

**SEQ ID NO: 69, *Citrus sinensis* SYT cDNA (CB290588)**
ATGCAACAGCACCTGATGCAGATGCAGCCCATGATGGCAGCTTATTATCCCAACAACGTCACTACT
GACCACATTCAACAGTATCTAGATGAGAACAAATCATTGATTTTGAAGATTGTTGAGAGCCAGAAT
TCAGGGAAACTGAGCGAGTGTGCAGAGAACCAGGCAAGATTGCAGCGGAATCTCATGTACCTGGCT
GCTATTGCTGATGCTCAACCCCAACCACCTAGCGTTCATGCCCAGTTCTCTTCTGGTGGCATTATG
CAGCCAGGAGCTCACTATATGCAACACCAGCAATCTCAGCCAATGACACCACAGTCACTTATGGCT
GCACGCTCATCCATGGTGTACTCTCAACAGCAATTTTCAGTGCTTCAGCAACAGCAAGCCTTGCAT
GGTCAGCTTGGCATGAGCTCTGGTGGTAGCTCAGGACTTCACATGCTGCAAAGTGAGGGTAGTACT
GCAGGAGGTAGTGGTTCACTTGGGGGTGGGGGATTCCCTGATTTTGGCCGTGGCTCATCTGGTGAA
GGCTTGCACTCAAGGGGAATGGGGAGCAAGCATGATATAGGCAGTTCTGGATCTGCTGAAGGACGA
GGAGGGAGCTCAGGAAGCCAAGATGGAGGCGAAACTCTCTACTTGAAAGGGGCTGATGATGGAAAT
TAA

**SEQ ID NO: 70, *Citrus sinensis* SYT polypeptide**
MQQHLMQMQPMMAAYYPNNVTTDHIQQYLDENKSLILKIVESQNSGKLSECAENQARLQRNLMYLA
AIADAQPQPPSVHAQFSSGGIMQPGAHYMQHQQSQPMTPQSLMAARSSMVYSQQQFSVLQQQQALH
GQLGMSSGGSSGLHMLQSEGSTAGGSGSLGGGGFPDFGRGSSGEGLHSRGMGSKHDIGSSGSAEGR
GGSSGSQDGGETLYLKGADDG

## FIGURE 25 (continued)

**SEQ ID NO: 71, *Gossypium arboreum* SYT cDNA (BM359324)**
ATGCAGCAGCACCTGATGCAGATGCAGCCCATGATGGCAGCTTATTATCCCAACAACGTCACTACT
GATCATATTCAACAGTATCTCGATGAGAACAAGTCATTGATCTTAAAGATTGTTGAGAGCCAGAAT
TCTGGGAAATTGAGTGAATGTGCTGAGAACCAAGCAAGGCTGCAGCGAAACCTCATGTACCTGGCT
GCCATTGCGGATTCTCAACCCCAACCACCCACCGTGCATGCACAGTTTCCATCTGGTGGTATCATG
CAGCAAGGAGCTGGGCACTACATGCAGCACCAACAAGCTCAACANATGACACAACAGTCGCTTATG
GCTGCTCGGTCCTCAATGTTGTATTCTCAGCAACCATTTTCTGCACTGCAACAACAACAACAACAA
GGCTTTGCACAGTCAGCTTGGCATGAGCTCTGGCGGGAGCACAGGCCTTTCATATGCTGCAAACTG
AATCTAGTACTGCAGGGGGCAGTGAGACACCTTGGGCCCGAGGGTTGTCCTGATTTGGACGGGGGT
CTTTTGGAGAGGCATCCCTGGTGGCAGGCCAATGGCCGGGGGAACAACCAAAAATCCGGGGAGGCC
GGCTCACCTAAGGGCCGGGAGGAGCCCTTGGGGCAGGGGGGGGTGATGGGGGGAACCTCTTCTTAA

**SEQ ID NO: 72, *Gossypium arboreum* SYT polypeptide**
MQQHLMQMQPMMAAYYPNNVTTDHIQQYLDENKSLILKIVESQNSGKLSECAENQARLQRNLMYLA
AIADSQPQPPTVHAQFPSGGIMQQGAGHYMQHQQAQXMTQQSLMAARSSMLYSQQPFSALQQQQQQ
GFAQSAWHELWREHRPFICCKLNLVLQGAVRHLGPEGCPDLDGGLLERHPWWQANGRGNNQKSGEA
GSPKGREEPLGQGGVMGGTSS

**SEQ ID NO: 73, *Medicago truncula*ta SYT cDNA (CA858507)**
ATGCAGCAGCACCTGATGCAGATGCAGCCCATGATGGCAGCTTACTATCCTAACAACGTCACTACT
GATCATATTCAACAGTATCTTGATGAGAACAAGTCCTTGATTCTCAAGATTGTTGAAAGCCAGAAC
ACTGGCAAGCTCACCGAGTGTGCTGAGAACCAATCAAGGCTTCAGAGAAATCTCATGTACCTAGCT
GCAATAGCTGATTCTCAACCCCAACCACCTACTATGCCTGGCCAGTACCCTTCAAGTGGAATGATG
CAGCAGGGAGGACACTACATGCAGGCTCAACAAGCTCAGCAGATGACACAACAACAATTAATGGCT
GCACGTTCCTCTCTTATGTATGCTCAACAGCTTCAACAGCAGCAAGCCTTGCAAAGCCAACTTGGT
ATGAATTCCAGTGGAAGTCAAGGCCTTCACATGTTGCATAGTGAAGGGGCTAATGTTGGAGGCAAT
TCATCTCTAGGGGCTGGTTTTCCTGATTTTGGCCGTAGCTCAGCCGGTGATGGTTTGCACGGCAGT
GGTAAGCAAGACATTGGAAGCACTGATGGCCGCGGTGGAAGCTCTAGTGGTCACTCTGGTGATGGC
GGCGAAACACTTTACCTGAAATCTTCTGGTGATGGGAATTAG

**SEQ ID NO: 74, *Medicago truncula*ta SYT polypeptide**
MQQHLMQMQPMMAAYYPNNVTTDHIQQYLDENKSLILKIVESQNTGKLTECAENQSRLQRNLMYLA
AIADSQPQPPTMPGQYPSSGMMQQGGHYMQAQQAQQMTQQQLMAARSSLMYAQQLQQQQALQSQLG
MNSSGSQGLHMLHSEGANVGGNSSLGAGFPDFGRSSAGDGLHGSGKQDIGSTDGRGGSSSGHSGDG
GETLYLKSSGDGN

**SEQ ID NO: 75, *Oryza sativa* SYT1 cDNA (AK058575)**
ATGCAGCAGCAACACCTGATGCAGATGAACCAGGGCATGATGGGGGGATATGCTTCCCCTACCACC
GTCACCACTGATCTCATTCAGCAGTATCTGGATGAGAACAAGCAGCTGATCCTGGCCATCCTTGAC
AACCAGAACAATGGGAAGGTGGAAGAGTGCGCTCGGAACCAAGCTAAGCTCCAGCACAATCTCATG
TACCTCGCCGCCATCGCCGACAGCCAGCCGCCGCAGACGGCCGCCATGTCCCAGTATCCGTCGAAC
CTGATGATGCAGTCCGGGGCGAGGTACATGCCGCAGCAGTCGGCGCAGATGATGGCGCCGCAGTCG
CTGATGGCGGCGAGGTCTTCGATGATGTACGCGCAGCCGGCGCTGTCGCCGCTCCAGCAGCAGCAG
CAGCAGCAGGCGGCGGCGGCGCACGGGCAGCTGGGCATGGGCTCGGGGGGCACCACCAGCGGGTTC
AGCATCCTCCACGGCGAGGCCAGCATGGGCGGCGGCGGCGGCGGCGGTGGCGCCGGTAACAGCATG
ATGAACGCCGGCGTGTTCTCCGACTTCGGACGCGGCGGCGGCGGCGGCGGCAAGGAGGGGTCCACC
TCGCTGTCCGTCGACGTCCGGGGCGCCAACTCCGGCGCCCAGAGCGGCGACGGGGAGTACCTCAAG
GGCACCGAGGAGGAAGGCAGCTAG

## FIGURE 25 (continued)

**SEQ ID NO: 76, *Oryza sativa* SYT1 polypeptide**
mqqqhlmqmnqgmmggyasptttvttdliqqyldenkqlilaildnqnngkveecarnqaklqhnlm
ylaaiadsqppqtaamsqypsnlmmqsgarympqqsaqmmapqslmaarssmmyaqpalsplqqqq
qqqaaaahgqlgmgsggttsgfsilhgeasmgggggggagnsmmnagvfsdfgrgggggkegst
slsvdvrgansgaqsgdgeylkgteeegs

**SEQ ID NO: 77, *Oryza sativa* SYT2 cDNA (AK105366)**
ATGCAGCAGCAGCCGATGCCGATGCCCGCGCAGGCGCCGCCGACGGCCGGAATCACCACCGAGCAG
ATCCAAAAGTATCTGGATGAAAACAAGCAGCTTATTTTGGCTATTTTGGAAAATCAGAATCTGGGA
AAGTTGGCAGAATGTGCTCAGTATCAAGCGCAGCTTCAGAAGAATCTCTTGTACTTGGCTGCAATT
GCTGATACTCAACCGCAGACCACTATAAGCCGTCCCCAGATGGTGCCGCATGGTGCATCGCCGGGG
TTAGGGGGGCAATACATGTCGCAGGTGCCAATGTTCCCCCCCAGGACCCCTCTAACGCCCCAGCAG
ATGCAGGAGCAGCAGCTGCAGCAACAGCAAGCCCAGCTGCTCTCGTTCGGCGGTCAGATGGTTATG
AGGCCTGGCGTTGTGAATGGCATTCCTCAGCTTCTGCAAGGCGAAATGCACCGCGGAGCAGATCAC
CAGAACGCTGGCGGGGCCACCTCGGAGCCTTCCGAGAGCCACAGGAGCACCGGCACCGAAAATGAC
GGTGGAAGCGACTTCGGCGATCAATCCTAA

**SEQ ID NO: 78, *Oryza sativa* SYT2 polypeptide**
MQQQPMPMPAQAPPTAGITTEQIQKYLDENKQLILAILENQNLGKLAECAQYQAQLQKNLLYLAAI
ADTQPQTTISRPQMVPHGASPGLGGQYMSQVPMFPPRTPLTPQQMQEQQLQQQQAQLLSFGGQMVM
RPGVVNGIPQLLQGEMHRGADHQNAGGATSEPSESHRSTGTENDGGSDFGDQS

**SEQ ID NO: 79, *Oryza sativa* SYT3 cDNA (BP185008)**
ATGCAGCAGCAGATGGCCATGCCGGCGGGGGCCGCCGCCGCCGCGGTGCCGCCGGCGGCCGGCATC
ACCACCGAGCAGATCCAAAAGTATTTGGATGAAAATAAACAGCTAATTTTGGCCATCCTGGAAAAT
CAAAACCTAGGGAAGTTGGCTGAATGTGCTCAGTACCAAGCTCAGCTTCAAAAGAATCTCTTGTAT
CTGGCTGCCATTGCAGATGCCCAACCACCTCAGAATCCAGGAAGTCGCCCTCAGATGATGCAGCCT
GGTGCTACCCCAGGTGCTGGGCATTACATGTCCCAAGTACCGATGTTCCCTCCAAGAACTCCCTTA
ACCCCACAACAGATGCAAGAGCAGCAGCAGCAGCAACTCCAGCAACAGCAAGCTCAGGCTCTAGCC
TTCCCCGGCCAGATGCTAATGAGACCAGGTACTGTCAATGGCATGCAATCTATCCCAGTTGCTGAC
CCTGCTCGCGCAGCCGATCTTCAGACGGCAGCACCGGGCTCGGTAGATGGCCGAGGAAACAAGCAG
GATGCAACCTCGGAGCCTTCCGGGACCGAGAGCCACAAGAGTGCGGGAGCAGATAACGACGCAGGC
GGTGACATAGCGGAGAAGTCCTGA

**SEQ ID NO: 80, *Oryza sativa* SYT3 polypeptide**
MQQQMAMPAGAAAAAVPPAAGITTEQIQKYLDENKQLILAILENQNLGKLAECAQYQAQLQKNLLY
LAAIADAQPPQNPGSRPQMMQPGATPGAGHYMSQVPMFPPRTPLTPQQMQEQQQQLQQQQAQALA
FPGQMLMRPGTVNGMQSIPVADPARAADLQTAAPGSVDGRGNKQDATSEPSGTESHKSAGADNDAG
GDIAEKS

**SEQ ID NO: 81, *SOLANUM TUBEROSUM* SYT CDNA (BG590990)**
ATGCAGCAGCAGCACCTGATGCAGATGCAGCCCATGATGGCAGCCTATTATCCCAACAATGTCACT
ACTGATCATATTCAACAGTTCCTGGATGAGAACAAATCACTTATTCTGAAGATTGTTGAGAGCCAG
AACTCTGGGAAAATAAGTGAATGTGCAGAGTCCCAAGCTAAACTTCAGAGAAATCTTATGTACCTT
GCAGCTATTGCTGATTCACAGCCCCAGCCTCCTAGTATGCATTCACAGTTAGCTTCTGGTGGGATG
ATGCAGGGAGGGGCACATTATATGCAGCAACAACAAGCTCAACAACTCACAACGCAATCGCTTATG
GCTGCAGCAAGATCCTCCTCCTCAATGCTCTATGGACAACAACAACAACAACAACAACAACAACTA
TCATCATTGCAACAACAGCAAGCAGCCTTTCATAGCCAGCAACTCGGAATGAGCAGCTCTGGTGGA
GGAAGCAGTAGTGGACTTCACATGCTACAAAGCGAAAACACTCATAGTGCTAGCACTGGTGGTGGG
TGGTTTCCCTGA

**FIGURE 25 (continued)**

**SEQ ID NO: 82, *Solanum tuberosum* SYT polypeptide**
MQQQHLMQMQPMMAAYYPNNVTTDHIQQFLDENKSLILKIVESQNSGKISECAESQAKLQRNLMYL
AAIADSQPQPPSMHSQLASGGMMQGGAHYMQQQQAQQLTTQSLMAAARSSSSMLYGQQQQQQQQL
SSLQQQQAAFHSQQLGMSSSGGGSSSGLHMLQSENTHSASTGGGWFP

**SEQ ID NO: 83, *Zea mays* SYT1 cDNA (BG874129.1, CA409022.1; compiled)**
ATGCAGCAGCAACACCTGATGCAGATGAACCAGAACATGATGGGGGGCTACACCTCTCCTGCCGCC
GTGACCACCGATCTCATCCAGCAGCACCTGGACGAGAACAAGCAGCTGATCCTGGCCATCCTCGAC
AACCAGAACAATGGCAAGGCGGAGGAGTGCGAACGGCACCAAGCTAAGCTCCAGCACAACCTCATG
TACCTGGCCGCCATCGCTGACAGCCAGCCGCCACAGACCGCGCCACTATCACAGTACCCGTCCAAC
CTGATGATGCAGCCGGGCCCTCGGTACATGCCACCGCAGTCCGGGCAGATGATGAACCCGCAGTCG
CTGATGGCGGCGCGGTCCTCCATGATGTACGCGCACCCGTCCCTGTCGCCACTCCAGCAGCAGCAG
GCGGCGCACGGACAGCTGGGTATGGCTCCAGGGGGCGGCGGTGGCGGCACGACCAGCGGGTTCAGC
ATCCTCCACGGCGAGGCCAGCATGGGCGGTGGTGGTGCTGGCGCAGGCGCCGGCAACAACATGATG
AACGCCGGCATGTTCTCGGGCTTTGGCCGCAGCGGCAGTGGCGCCAAGGAAGGGTCGACCTCTCTG
TCGGTTGACGTCCGGGGTGGAACCAGCTCCGGCGCGCAGAGCGGGGACGGCGAGTACCTCAAAGTC
GGCACCGAGGAAGAAGGCAGTTAG

**SEQ ID NO: 84, *Zea mays* SYT1 polypeptide**
mqqqhlmqmnqnmmggytspaavttdliqqhldenkqlilaildnqnngkaeecerhqaklqhnlm
YLAAIADSQPPQTAPLSQYPSNLMMQPGPRYMPPQSGQMMNPQSLMAARSSMMYAHPSLSPLQQQQ
AAHGQLGMAPGGGGGGTTSGFSILHGEASMGGGGAGAGAGNNMMNAGMFSGFGRSGSGAKEGSTSL
SVDVRGGTSSGAQSGDGEYLKVGTEEEGS

**SEQ ID NO: 85, *Zea mays* SYT2 cDNA (AY106697)**
ATGCAGCAGCCGATGCACATGCAGCCACAGGCGCCGGCGATAACCCCAGCTGCCGGAATCAGCACG
GAGCAGATCCAAAAGTATCTGGATGAGAATAAGCAGCTTATTTTGGCTATTTTGGAAAATCAGAAC
CTAGGAAAATTGGCAGAATGTGCTCAGTATCAATCACAACTTCAGAAGAACCTCTTGTATCTCGCT
GCAATCGCAGATGCTCAACCGCAGACTGCTGTAAGCCGCCCTCAGATGGCGCCGCCTGGTGGATCG
CCTGGAGTAGGGCAGTACATGTCACAGGTGCCTATGTTCCCACCGAGGACACCTCTTACACCCCAG
CAGATGCAGGAGCAGCAGCTTCAGCAGCAGCAGGCTCAGTTGCTAAACTTCAGTGGCCAAATGGTT
GCTAGACCAGGCATGGTCAACGGCATGGCTCAGTCCATGCAAGCTCAGCTACCACCGGGTGTGAAC
AAGCAGGATGCTGGTGGGGTCGCCTCTGAGCCCTCGGGCACCGAGAGCCACAGGAGCACTGGTGGT
GACGATGGTGGAAGCGACTAG

**SEQ ID NO: 86, *Zea mays* SYT2 polypeptide**
MQQPMHMQPQAPAITPAAGISTEQIQKYLDENKQLILAILENQNLGKLAECAQYQSQLQKNLLYLA
AIADAQPQTAVSRPQMAPPGGSPGVGQYMSQVPMFPPRTPLTPQQMQEQQLQQQQAQLLNFSGQMV
ARPGMVNGMAQSMQAQLPPGVNKQDAGGVASEPSGTESHRSTGGDDGGSD

**SEQ ID NO: 87, *Homo sapiens* cDNA (CR542103)**
ATGGGCGGCAACATGTCTGTGGCTTTCGCGGCCCCGAGGCAGCGAGGCAAGGGGGAGATCACTCCC
GCTGCGATTCAGAAGATGTTGGATGACAATAACCATCTTATTCAGTGTATAATGGACTCTCAGAAT
AAAGGAAAGACCTCAGAGTGTTCTCAGTATCAGCAGATGTTGCACACAAACTTGGTATACCTTGCT
ACAATAGCAGATTCTAATCAAAATATGCAGTCTCTTTTACCAGCACCACCCACACAGAATATGCCT
ATGGGTCCTGGAGGGATGAATCAGAGCGGCCCTCCCCCACCTCCACGCTCTCACAACATGCCTTCA
GATGGAATGGTAGGTGGGGGTCCTCCTGCACCGCACATGCAGAACCAGATGAACGGCCAGATGCCT

**FIGURE 25 (continued)**

GGGCCTAACCATATGCCTATGCAGGGACCTGGACCCAATCAACTCAATATGACAAACAGTTCCATG
AATATGCCTTCAAGTAGCCATGGATCCATGGGAGGTTACAACCATTCTGTGCCATCATCACAGAGC
ATGCCAGTACAGAATCAGATGACAATGAGTCAGGGACAACCAATGGGAAACTATGGTCCCAGACCA
AATATGAGTATGCAGCCAAACCAAGGTCCAATGATGCATCAGCAGCCTCCTTCTCAGCAATACAAT
ATGCCACAGGGAGGCGGACAGCATTACCAAGGACAGCAGCCACCTATGGGAATGATGGGTCAAGTT
AACCAAGGCAATCATATGATGGGTCAGAGACAGATTCCTCCCTATAGACCTCCTCAACAGGGCCCA
CCACAGCAGTACTCAGGCCAGGAAGACTATTACGGGGACCAATACAGTCATGGTGGACAAGGTCCT
CCAGAAGGCATGAACCAGCAATATTACCCTGATGGAAATTCACAGTATGGCCAACAGCAAGATGCA
TACCAGGGACCACCTCCACAACAGGGATATCCACCCCAGCAGCAGCAGTACCCAGGGCAGCAAGGT
TACCCAGGACAGCAGCAGGGCTACGGTCCTTCACAGGGTGGTCCAGGTCCTCAGTATCCTAACTAC
CCACAGGGACAAGGTCAGCAGTATGGAGGATATAGACCAACACAGCCTGGACCACCACAGCCACCC
CAGCAGAGGCCTTATGGATATGACCAGGGACAGTATGGAAATTACCAGCAG

**SEQ ID NO: 88, *Homo sapiens* SYT polypeptide (CAG46900.1)**
MGGNMSVAFAAPRQRGKGEITPAAIQKMLDDNNHLIQCIMDSQNKGKTSECSQYQQMLHTNLVYLA
TIADSNQNMQSLLPAPPTQNMPMGPGGMNQSGPPPPPRSHNMPSDGMVGGGPPAPHMQNQMNGQMP
GPNHMPMQGPGPNQLNMTNSSMNMPSSSHGSMGGYNHSVPSSQSMPVQNQMTMSQGQPMGNYGPRP
NMSMQPNQGPMMHQQPPSQQYNMPQGGGQHYQGQQPPMGMMGQVNQGNHMMGQRQIPPYRPPQQGP
PQQYSGQEDYYGDQYSHGGQGPPEGMNQQYYPDGNSQYGQQQDAYQGPPPQQGYPPQQQQYPGQQG
YPGQQQGYGPSQGGPGPQYPNYPQGQGQQYGGYRPTQPGPPQPPQQRPYGYDQGQYGNYQQ

**SEQ ID NO: 89, *Allium cepa* SYT2 cDNA CF437485**
ATGCAGCAGCCGCAGCCAGCGATGGGAACCATGGGCTCGGTGCCACCTACTAGCATCACCACCGAA
CAGATTCAAAGGTACTTGGATGAGAACAAACAGTTAATATTGGCAATTTTGGATAATCAAAATTTA
GGAAGACTGAATGAGTGTGCTCAATATCAAGCTCAGCTTCAAAAGAATCTGCTTTACCTGGCAGCA
ATAGCTGATGCTCAGCCTCAGTCTCCTGCGGTGCGTCTGCAGATGATGCCTCAAGGTGCAGCTGCC
ACGCCTCAAGCTGGAAACCAATTTATGCAGCAGCAGAGCCCTAATTTCCCTCCCAAAACAGGAATG
CAATTTACTCCTCAACAAGTACAAGAATTGCAGCAGCAACAGCTACAACATCAGCCACATATGATG
CCTCCATTTCAAGGTCAAATGGGTATGAGACCTATGAATGGAATGCAGGCAGCAATGCATGCAGAT
TCATCTCTTGCTTATAACACTAACAATAAGCAAGATGCAGGAAACGCAGCTTATGAAAATACTGCT
GCCAACACAGATGGTTCCATTCAAAAGAAAACAGCAAATGATGATTTAGACCCTTCTGCAGCAAAC
CCTAGAAGGTCTGAAGATGCCAAATCATCATGA

**SEQ ID NO: 90, *Allium cepa* SYT2 polypeptide**
MQQPQPAMGTMGSVPPTSITTEQIQRYLDENKQLILAILDNQNLGRLNECAQYQAQLQKNLLYLAA
IADAQPQSPAVRLQMMPQGAAATPQAGNQFMQQQSPNFPPKTGMQFTPQQVQELQQQQLQHQPHMM
PPFQGQMGMRPMNGMQAAMHADSSLAYNTNNKQDAGNAAYENTAANTDGSIQKKTANDDLDPSAAN
PRRSEDAKSS

**SEQ ID NO: 91, *Aquilegia formosa* x *Aquilegia pubescens* SYT1 cDNA DT758802.1**
ATGCAACACATGCAGATGCAGCCCATGATGCCACCTTATAGTGCCAACAGCGTCACTACTGATCAT
ATCCAACAGTACTTGGATGAAAATAAGGCGTTGATTCTGAAGATACTTGAGAACCAAAATTCGGGA
AAAGTTAGTGAATGTGCAGAGAACCAAGCAAGACTTCAACGAAATCTTATGTATCTGGCTGCAATT
GCTGATTCTCAACCACAGCCTCCCAATATGCATGCTCAGTACTCTAATGCGGGTATACCACCTGGT
GCACATTACCTACAACACCAACAGGCCCAACAGATGACACAACAGTCGCTCATGGCTGCTCGATCA
AATATGCTGTATGCTCAGCCAATCACAGGAATGCAGCAACAGCAAGCAATGCATAGCCAGCTTGGC

## FIGURE 25 (continued)

ATGAGCTCTGGTGGTAACAGTGGACTCCACATGATGCACAATGAGGGCAGCATGGGAGGTAGTGGG
GCACTTGGAAGCTATTCTGATTATGGCCGTGGCAGTGGTGGTGGAGTAACTATCGCTAGCAAACAA
GATGGTGGAAGTGGTTCTGGTGAAGGACGAGGTGGAAACTCTGGAGGCCAAAGTGCAGATGGAGGT
GAATCTCTTTACCTGAAAAACAGTGACGAAGGGAACTAA

**SEQ ID NO: 92, *Aquilegia formosa* x *Aquilegia pubescens* SYT1 polypeptide**
MQHMQMQPMMPPYSANSVTTDHIQQYLDENKALILKILENQNSGKVSECAENQARLQRNLMYLAAI
ADSQPQPPNMHAQYSNAGIPPGAHYLQHQQAQQMTQQSLMAARSNMLYAQPITGMQQQQAMHSQLG
MSSGGNSGLHMMHNEGSMGGSGALGSYSDYGRGSGGGVTIASKQDGGSGSGEGRGGNSGGQSADGG
ESLYLKNSDEGN

**SEQ ID NO: 93, *Brachypodium distachyon* SYT23 cDNA DV480064.1**
ATGCAGCAGGCGATGTCCATGTCCCCGGGGTCGGCCGGCGCGGTGCCGCCTCCGGCCGGCATCACC
ACAGAGCAGATCCAAAAGTATTTGGATGAAAATAAGCAACTTATTTTGGCCATCCTGGAAAATCAG
AACCTAGGAAAGTTGACTGAATGTGCTCAGTATCAAGCTCAACTTCAGAAGAATCTCTTGTATCTG
GCTGCCATTGCGGATGCCCAACCACCACAGAACCCTGGAAGTCGCCCCCAGATGGTGCAGCCTGGT
GGTATGCCAGGTGCAGGGCATTACATGTCGCAAGTACCAATGTTCCCTCCAAGAACCCCTTTAACC
CCACAACAGATGCAAGAGCAACAGCACCAGCAGCTTCAGCAGCAGCAAGCACAGGCTCTTGCTTTC
CCCAGCCAGATGGTCATGAGACCAGGTACTGTGAACGGCATGCAGCCTATGCAAGCTGATCTCCAA
GCAGCAGCAGCAGCACCTGGCCTGGCAGACAGCCGAGGAAGTAAGCAGGACGCAGCGGTAGCTGGG
GCCATCTCGGAACCTTCTGGCACCGAGAGTCACAAGAGTACAGGAGCGGATCATGAGGCAGGTGGC
GATGTAGCTGAGCAATCCTAA

**SEQ ID NO: 94, *Brachypodium distachyon* SYT3 polypeptide**
MQQAMSMSPGSAGAVPPPAGITTEQIQKYLDENKQLILAILENQNLGKLTECAQYQAQLQKNLLYL
AAIADAQPPQNPGSRPQMVQPGGMPGAGHYMSQVPMFPPRTPLTPQQMQEQQHQQLQQQQAQALAF
PSQMVMRPGTVNGMQPMQADLQAAAAAPGLADSRGSKQDAAVAGAISEPSGTESHKSTGADHEAGG
DVAEQS

**SEQ ID NO: 95, *Brassica napus* SYT2 cDNA CN732814**
ATGCAGCAGCAGCAGCAGCAGCAGCAGCAGCCTCCGCAAATGTTTCCGATGGCTCCTTCGATGCCG
CCAACTAACATCACCACCGAACAGATCCAAAAGTACCTTGAGGAGAACAAGAAGCTGATAATGGCA
ATCATGGAAAATCAGAATCTTGGCAAGCTTGCAGAGTGTGCACAGTACCAAGCTCTTCTCCAGAAG
AACTTAATGTACCTCGCTGCTATTGCTGATGCTCAACCTCCTCCATCTACCGCTGGAGCTACACCA
CCACCAGCTATGGCTTCCCAGATGGGGGCACCGCATCCTGGGATGCAACCGCCGAGCTACTTTATG
CAACACCCACAAGCTTCAGGGATGGCTCAACAAGCACCACCCGCTGGTATCTTCCCTCCGAGAGGT
CCTTTGCAGTTTGGTAGCCCACACCAGCTTCAGGATCCGCAACAGCAGCATATGCATCAACAGGCT
ATGCAAGGACACATGGGGATGCGACCAATGGGTATCAACAACAACAATGGGATGCAGCATCAGATG
CAGCAACAACAACCAGAAACCTCTCTTGGAGGAAGCGCTGCAAACGTGGGGCTTAGAGGTGGAAAG
CAAGATGGAGCAGATGGACAAGGAAAAGATGATGGCAAATGA

**SEQ ID NO: 96, *Brassica napus* SYT2 polypeptide**
MQQHLMQMQPMMAGYYPSNVTSDHIQQYLDENKSLILKIVESQNSGKLSECAENQARLQRNLMYLA
AIADSQPQPPSVHSQYGSAGGGLIQGEGASHYLQQQQATQQQQMTQQSLMAARSSMMYQQQQQPYA
TLQHQQLHHSQLGMSSSSGGGSSGLHILQGEAGGFHEFGRGKPEMGSGEGRGGSSGDGGETLYLKS
SDDGN

FIGURE 25 (continued)

**SEQ ID NO: 97, *Citrus sinensis* SYT2 cDNA CV717501**
ATGCAGCAGCCACCGCAAATGATCCCTGTTATGCCTTCATTTCCACCCACCAACATCACCACAGAG
CAGATTCAAAAGTACCTTGATGAGAACAAAAAGTTGATTTTGGCAATTTTGGACAATCAAAATCTT
GGAAAGCTTACAGAATGTGCCCACTATCAAGCTCAGCTTCAAAAGAATTTAATGTATTTAGCTGCA
ATTGCTGATGCACAACCACAAGCACCAACAATGCCTCCTCAGATGGCTCCACATCCTGCAATGCAA
GCTAGTGGGTATTACATGCAACATCCTCAGGCGGCAGCAATGGCTCAGCAACAAGGAATCTTTCCC
CAAAAGATGCCATTACAATTCAATAACCCTCATCAACTACAGGATCCTCAACAGCAGCTACACCAA
CATCAAGCCATGCAAGCACAAATGGGAATGAGACCGGGTGCCACTAACAATGGTATGCATCCCATG
CATGCTGAAAGCTCTCTTGGAGGTGGCAGCAGTGGAGGACCCCCTTCAGCATCAGGCCCAGGTGAC
ATACGTGGTGGAAATAAGCAAGATGCCTCGGAGGCTGGGACTACTGGTGCTGATGGCCAGGGCAGT
TCGGCTGGTGGGCATGGTGGGGATGGAGAGGAGGCAAAGTGA

**SEQ ID NO: 98, *Citrus sinensis* SYT2 polypeptide**
MQQPPQMIPVMPSFPPTNITTEQIQKYLDENKKLILAILDNQNLGKLTECAHYQAQLQKNLMYLAA
IADAQPQAPTMPPQMAPHPAMQASGYYMQHPQAAAMAQQQGIFPQKMPLQFNNPHQLQDPQQQLHQ
HQAMQAQMGMRPGATNNGMHPMHAESSLGGGSSGGPPSASGPGDIRGGNKQDASEAGTTGADGQGS
SAGGHGGDGEEAK

**SEQ ID NO: 99, *Euphorbia esula* SYT2 cDNA DV144834**
ATGCAGCAGCAACCGCAGATGATGCCTATGATGCCTTCATATCCACCAGCAAACATTACCACGGAG
CAAATCCAAAAGTATCTTGATGAAAATAAAAAATTGATTTTGGCGATCTTGGATAATCAAAATCTT
GGAAAACTCGCTGAGTGTGCACAGTATCAAGCCCTGCTGCAAAAAAATCTGATGTATTTAGCCGCA
ATTGCTGATGCACAACCCCAGACCCCACCCATGCCACCTCAGATGTCCCCACATCCGGCTATGCAA
CAAGGAGCATATTACATGCAACATCCTCAGGCTGCAGCAGCAGCAATGGCTCATCAGTCGGGTATT
TTCCCACCAAAGATGTCTCCGTTACAATTCAATAATCCTCATCAAATACAGGACCCCCAGCAGTTA
CATCAAGCAGCCCTCCAAGGGCAAATGGGAATGAGGCCCATGGGGCCCAATAACGGGATGCATCCG
ATGCACCCCGAGGCAAATCTTGGAGGATCTAATGATGGTCGTGGAGGAAACAAACAGGATGCTCCG
GAGACGGGAGCATCGGGAGGTGATGGGCAAGGCAATTCTGGTGGTGATGGGGCTGAAGATGGGAAA
TGA

**SEQ ID NO: 100, *Euphorbia esula* SYT2 polypeptide**
MQQQPQMMPMMPSYPPANITTEQIQKYLDENKKLILAILDNQNLGKLAECAQYQALLQKNLMYLAA
IADAQPQTPPMPPQMSPHPAMQQGAYYMQHPQAAAAAMAHQSGIFPPKMSPLQFNNPHQIQDPQQL
HQAALQGQMGMRPMGPNNGMHPMHPEANLGGSNDGRGGNKQDAPETGASGGDGQGNSGGDGAEDGK

**SEQ ID NO: 101, *Glycine max* SYT2 cDNA BQ612648**
ATGCAGCAGACACCGCCAATGATTCCTATGATGCCTTCTTTCCCACCTACGAACATAACCACCGAG
CAGATTCAAAAATACCTTGATGAGAACAAGAAGCTGATTCTGGCAATATTGGACAATCAAAATCTT
GGAAAACTTGCAGAATGTGCCCAGTACCAAGCTCAGCTTCAAAAGAATTTGATGTATTTAGCTGCA
ATTGCTGATGCCCAGCCTCAAACCCCGGCCATGCCTCCGCAGATGGCACCGCACCCTGCCATGCAA
CCAGGATTCTATATGCAACATCCTCAGGCTGCTGCAGCAGCAATGGCTCAGCAGCAGCAAGGAATG
TTCCCCCAGAAAATGCCATTGCAATTTGGCAATCCACATCAAATGCAGGAACAACAACAGCAGCTA
CACCAGCAGGCCATCCAAGGTCAAATGGGACTTAGACCTGGAGATATAAATAATGGCATGCATCCA
ATGCACAGTGAGGCTGCTCTTGGAGGTGGAAACAGCGGTGGTCCACCTTCGGCTACTGGTCCAAAC
GATGCACGTGGTGGAAGCAAGCAAGATGCCTCTGAGGCTGGAACAGCTGGTGGAGACGGCCAAGGC
AGCTCCGCGGCTGCTCATAACAGTGGAGATGGTGAAGAGGCAAAGTGA

FIGURE 25 (continued)

**SEQ ID NO: 102, *Glycine max* SYT2 polypeptide**
MQQTPPMIPMMPSFPPTNITTEQIQKYLDENKKLILAILDNQNLGKLAECAQYQAQLQKNLMYLAA
IADAQPQTPAMPPQMAPHPAMQPGFYMQHPQAAAAAMAQQQQGMFPQKMPLQFGNPHQMQEQQQQL
HQQAIQGQMGLRPGDINNGMHPMHSEAALGGGNSGGPPSATGPNDARGGSKQDASEAGTAGGDGQG
SSAAAHNSGDGEEAK

**SEQ ID NO: 103, *Glycine soya* SYT2 cDNA CA799921**
ATGCAGCAGACACCGCCTATGATTCCTATGATGCCTTCGTTCCCACCTACGAACATAACCACCGAG
CAGATTCAAAAATACCTTGATGAGAACAAGAAGCTGATTCTGGCAATATTGGACAATCAAAATCTT
GGAAAACTTGCAGAATGTGCCCAGTACCAAGCTCAGCTTCAAAAGAATTTGATGTATTTAGCTGCA
ATTGCTGATGCCCAGCCTCAAACACCAGCCATGCCTCCACAGATGGCACCACACCCTGCCATGCAA
CCAGGATTCTATATGCAACATCCTCAGGCTGCAGCAGCAGCAATGGCTCAGCAGCAGCAGCAAGGA
ATGTTCCCCCAGAAAATGCCATTGCAATTTGGCAATCCACATCAAATGCAGGAACAACAGCAGCAG
CTACACCAGCAAGCCATCCAAGGTCAAATGGGACTGAGACCTGGAGGAATAAATAATGGCATGCAT
CCAATGCACAATGAGGGCGGCAACAGCGGTGGTCCACCCTCGGCTACCGGTCCGAACGACGCACGT
GGTGGAAGCAAGCAAGATGCTTCTGAGGCTGGAACAGCTGGTGGAGATGGCCAAGGCAGCTCTGCA
GCTGCTCATAACAGTGGAGATGGTGAAGAGGCAAAGTGA

**SEQ ID NO: 104, *Glycine soya* SYT2 polypeptide**
MQQTPPMIPMMPSFPPTNITTEQIQKYLDENKKLILAILDNQNLGKLAECAQYQAQLQKNLMYLAA
IADAQPQTPAMPPQMAPHPAMQPGFYMQHPQAAAAAMAQQQQQGMFPQKMPLQFGNPHQMQEQQQQ
LHQQAIQGQMGLRPGGINNGMHPMHNEGGNSGGPPSATGPNDARGGSKQDASEAGTAGGDGQGSSA
AAHNSGDGEEAK

**SEQ ID NO: 105, *Gossypium hirsutum* SYT1 cDNA DT558852**
ATGCAGCAGCACCTGATGCAGATGCAGCCCATGATGGCAGCTTATTATCCCAACAACGTCACTACT
GATCATATTCAACAGTATCTCGATGAGAACAAGTCATTGATCTTAAAGATTGTTGAGAGCCAGAAT
TCTGGGAAATTGAGTGAATGTGCTGAGAACCAAGCAAGGCTGCAGCGAAACCTCATGTACCTGGCT
GCCATTGCGGATTCTCAACCCCAACCACCCACCGTGCATGCACAGTTTCCATCTGGTGGTATCATG
CAGCCAGGAGCTGGGCACTACATGCAGCACCAACAAGCTCAACAAATGACACAACAGTCGCTTATG
GCTGCTCGGTCCTCAATGTTGTATTCTCAGCAACCATTTTCTGCACTGCAACAACAACAGCAGCAA
GCTTTGCACAGTCAGCTTGGCATGAGCTCTGGCGGAAGCACAGGCCTTCATATGCTGCAAACTGAA
TCTAGTACTGCAGGTGGCAGTGGAGCACTTGGGGCCGGAGGGTTTCCTGATTTTGGACGTGGTTCT
TCTGGAGAAGGCATCCATGGTGGCAGGCCAATGGCAGGTGGAAGCAAGCAAGATATCGGGAGTGCC
GGCTCAGCTGAAGGTCGTGGAGGAAGCTCTGGTGGTCAGGGTGGTGGTGATGGGGGTGAAACCCTT
TACTTAAAAGCAGCCGATGATGGGAACTGA

**SEQ ID NO: 106, *Gossypium hirsutum* SYT1 polypeptide sequence**
MQQHLMQMQPMMAAYYPNNVTTDHIQQYLDENKSLILKIVESQNSGKLSECAENQARLQRNLMYLA
AIADSQPQPPTVHAQFPSGGIMQPGAGHYMQHQQAQQMTQQSLMAARSSMLYSQQPFSALQQQQQQ
ALHSQLGMSSGGSTGLHMLQTESSTAGGSGALGAGGFPDFGRGSSGEGIHGGRPMAGGSKQDIGSA
GSAEGRGGSSGGQGGGDGGETLYLKAADDGN

**SEQ ID NO: 107, *Gossypium hirsutum* SYT2 cDNA DT563805**
ATGCCGCAGCCACCGCAAATGATTCCTGTGATGCCTTCATATCCACCTACTAATATCACTACTGAA
CAGATTCAGAAGTACCTTGATGAGAATAAGAAGTTGATTTTGGCAATTTTGGACAATCAGAATCTT
GGAAAACTCGCTGAATGCGCCCAGTATCAAGCTCAGCTGCAAAAGAATTTGATGTATTTAGCTGCA
ATTGCGGATGCTCAACCTCAATCAACGCCAGCAATGTCGCCTCAGATGGCACCGCATCCAGCAATG

FIGURE 25 (continued)

CAACCCGGAGGATATTTTATGCAACATCCTCAAGCTGCTGCAATGTCACAGCAACCTGGCATGTAC
CCTCAAAAGGTGCCATTGCAATTCAATAGTCCGCATCAAATGCAGGACCCTCAGCACCTCCTATAT
CAGCAGCATCAACAAGCAATGCAAGGTCAAATGGGAATCAGGCCTGGGGGACCCAATAATAGCATG
CATCCCATGCATTCAGAGGCTAGCCTTGGAGGCGGCAGCAGTGGTGGTCCCCCTCAACCTTCAGGC
CCAAGTGATGGACGTGCTGGAAACAAGCAAGAGGGCTCCGAAGCTGGTGGTAATGGGCAGGGCAGC
ACAACTGGTGGGCATGGTGGCGGTGATGGAGCGGATGAGGCAAAGTGA

**SEQ ID NO: 108, *Gossypium hirsutum* SYT2 polypeptide**
MPQPPQMIPVMPSYPPTNITTEQIQKYLDENKKLILAILDNQNLGKLAECAQYQAQLQKNLMYLAA
IADAQPQSTPAMSPQMAPHPAMQPGGYFMQHPQAAAMSQQPGMYPQKVPLQFNSPHQMQDPQHLLY
QQHQQAMQGQMGIRPGGPNNSMHPMHSEASLGGGSSGGPPQPSGPSDGRAGNKQEGSEAGGNGQGS
TTGGHGGGDGADEAK

**SEQ ID NO: 109, *Hordeum vulgare* SYT2 cDNA CA032350**
ATGCAGCAAGCGATGCCCATGCCGCCGGCGGCGGCGGCGCCTGGGATGCCTCCTTCTGCCGGCCTC
AGCACCGAGCAGATCCAAAAGTACCTGGATGAAAATAAACAACTAATTTTGGCTATCTTGGAAAAT
CAGAACCTGGGAAAGTTGGCGGAATGTGCTCAGTATCAAGCTCAGCTTCAGAAGAATCTTTTGTAT
TTGGCTGCGATTGCTGATACTCAGCCACAGACCTCTGTAAGCCGTCCTCAGATGGCACCACCTGCT
GCATCCCCAGGGGCAGGGCATTACATGTCACAGGTGCCAATGTTCCCTCCGAGGACCCCTCTAACG
CCTCAGCAGATGCAGGAGCAGCAACTACAGCAACAACAGGCTCAGATGCTTCCGTTTGCTGGTCAA
ATGGTTGCGAGACCCGGGGCTGTCAATGGCATTCCCCAGGCCCCTCAAGTTGAACAACCAGCCTAT
GCAGCAGGTGGGGCCAGTTCCGAGCCTTCTGGCACCGAGAGCCACAGGAGCACTGGCGCCGATAAC
GATGGTGGGAGCGGCTTGGCTGACCAGTCCTAA

**SEQ ID NO: 110, *Hordeum vulgare* SYT2 polypeptide**
MQQAMPMPPAAAAPGMPPSAGLSTEQIQKYLDENKQLILAILENQNLGKLAECAQYQAQLQKNLLY
LAAIADTQPQTSVSRPQMAPPAASPGAGHYMSQVPMFPPRTPLTPQQMQEQQLQQQQAQMLPFAGQ
MVARPGAVNGIPQAPQVEQPAYAAGGASSEPSGTESHRSTGADNDGGSGLADQS

**SEQ ID NO: 111, *Lactuca serriola* SYT1 cDNA DW110765**
ATGAAGCAGCCGATGATGCCGAATCCAATGATGTCTTCTTCGTTTCCTCCTACAAACATCACCACC
GATCAGATCCAAAAGTTCCTTGATGAAAACAAGCAACTAATTATAGCAATAATGAGCAACCTAAAT
CTTGGAAAGCTTGCTGAATGTGCCCAGTACCAAGCTCTACTCCAAAAAAATTTGATGTATCTAGCA
GCCATTGCAGATGCTCAACCACCTACACCTACACCAACACTAAATATCTCTTATNAGATGGGCCCG
GTTCCACATCCAGGGATGCCACAGCAAGGTGGATTTTACATGGCGCAGCAGCACCCTCAGGCGGCT
GTAATGACGGCTCAGCCACCTTCTGGTTTTCCACAACCGATGCCTGGTATGCAATTTAACAGCCCA
CAGGCTATTCAAGGGCAGATGGGCGGGAGGTCCGGTGGGCCGCCAAGCTCAGCCGCTAGTGATGTC
TGGAGAGGAAGCATGCAAGATGGTGGTGGTGGTGCTGCTGCTGATGGTGGTAAGGATGGTCATGCT
GGCGGTGGACCTGAGGAAGCAAAGTAA

**SEQ ID NO: 112, *Lactuca serriola* SYT1 polypeptide**
MKQPMMPNPMMSSSFPPTNITTDQIQKFLDENKQLIIAIMSNLNLGKLAECAQYQALLQKNLMYLA
AIADAQPPTPTPTLNISYXMGPVPHPGMPQQGGFYMAQQHPQAAVMTAQPPSGFPQPMPGMQFNSP
QAIQGQMGGRSGGPPSSAASDVWRGSMQDGGGGAAADGGKDGHAGGGPEEAK

**SEQ ID NO: 113, *Lycopersicon esculentum* SYT1 cDNA AW934450.1 BP893155.1**
ATGCAGCAGCACCTGATGCAGATGCAGCCCATGATGGCAGCTTACTATCCAACGAACGTCACTACT
GACCATATTCAACAGTATTTGGATGAAAACAAATCACTCATTCTGAAGATTGTTGAGAGCCAGAAC
TCTGGGAAACTCAGTGAATGTGCGGAGAACCAAGCTAGGCTTCAGAGGAATCTGATGTACCTTGCT

**FIGURE 25 (continued)**

GCGATTGCTGATTCACAACCTCAACCTTCTAGCATGCATTCTCAGTTCTCTTCTGGTGGGATGATG
CAGCCAGGGACACACAGTTACTTGCAGCAGCAGCAGCAGCAACAACAAGCGCAACAAATGGCAACA
CAACAACTCATGGCTGCAAGATCCTCGTCGATGCTCTATGGACAACAGCAGCAGCAATCTCAGTTA
TCGCAATATCAACAAGGCTTGCATAGTAGCCAACTCGGCATGAGTTCTGGCAGTGGCGGAAGCACT
GGACTTCATCACATGCTTCAAAGTGAATCATCACCTCATGGTGGTGGTTTCTCTCATGACTTCGGC
CGCGCAAATAAGCAAGACATTGGGAGTAGTATGTCTGCTGAAGGGCGCGGCGGAAGTTCAGGTGGT
GAGAATCTTTATCTGAAAGCTTCTGAGGATTGA

**SEQ ID NO: 114, *Lycopersicon esculentum* SYT1 polypeptide**
MQQHLMQMQPMMAAYYPTNVTTDHIQQYLDENKSLILKIVESQNSGKLSECAENQARLQRNLMYLA
AIADSQPQPSSMHSQFSSGGMMQPGTHSYLQQQQQQQAQQMATQQLMAARSSSMLYGQQQQQSQL
SQYQQGLHSSQLGMSSGSGGSTGLHHMLQSESSPHGGGFSHDFGRANKQDIGSSMSAEGRGGSSGG
ENLYLKASED

**SEQ ID NO: 115, *Malus domestica* SYT2 cDNA CV084230 DR997566**
ATGCAGCAGCCACCACAAATGATCCCCGTCATGCCTTCATTTCCTCCCACCAACATCACCACCGAA
CAAATTCAGAAGTACCTTGATGACAACAAAAAGTTGATTCTGGCAATATTGGATAATCAAAATCTT
GGAAAACTTGCTGAGTGTGCTCAGTACCAGGCTCTGCTTCAAAAGAATCTGATGTATTTAGCAGCA
ATTGCCGATGCGCAACCACAGGCACCAGCTGCCCCTCCCCAGATGGCCCCACATCCTGCTATGCAA
CAGGCAGGATATTACATGCAACATCCTCAGGCAGCAGCAATGGCTCAGCAACAGGGTATTTTCTCC
CCAAAGATGCCGATGCAATTCAATAACATGCATCAAATGCACGATCCACAGCAGCACCAACAAGCC
ATGCAAGGGCAAATGGGAATGAGACCTGGAGGGCCTAACGGCATGCCTTCCATGCTTCATACTGAG
GCCACACATGGTGGTGGTAGTGGCGGCCCAAATTCAGCTGGAGACCCAAATGATGGGCGTGGAGGA
AGCAAGCAAGACGCCTCTGAGTCTGGGGCAGGTGGTGATGGCCAGGGGACCTCAGCCGGCGGGCGT
GGAACTGGTGATGGAGAGGACGGCAAGTGA

**SEQ ID NO: 116, *Malus domestica* SYT2 polypeptide**
MQQPPQMIPVMPSFPPTNITTEQIQKYLDDNKKLILAILDNQNLGKLAECAQYQALLQKNLMYLAA
IADAQPQAPAAPPQMAPHPAMQQAGYYMQHPQAAAMAQQQGIFSPKMPMQFNNMHQMHDPQQHQQA
MQGQMGMRPGGPNGMPSMLHTEATHGGGSGGPNSAGDPNDGRGGSKQDASESGAGGDGQGTSAGGR
GTGDGEDGK

**SEQ ID NO: 117, *Medicago trunculata* SYT2 cDNA CA858743 BI310799.1 AL382135.1**
ATGCAGCAGACACCTCAAATGATTCCTATGATGCCTTCATTCCCACAACAAACAAACATAACCACT
GAGCAGATTCAAAAATATCTTGATGAGAACAAGAAGCTGATCCTGGCAATATTGGACAATCAAAAT
CTTGGAAAACTTGCAGAATGTGCCCAGTACCAAGCTCAGCTTCAGAAGAATTTGATGTATTTAGCT
GCAATTGCTGACGCGCAGCCACAAACACCGGCCTTGCCTCCACAGATGGCCCCGCACCCTGCGATG
CAACAAGGATTCTATATGCAACATCCTCAGGCTGCAGCAATGGCTCAGCAACAAGGAATGTTCCCC
CAAAAAATGCCAATGCAGTTCGGTAATCCGCATCAAATGCAGGATCAGCAGCATCAGCAGCAACAA
CAGCAGCTACATCAGCAAGCTATGCAAGGTCAAATGGGACTTAGACCTGGAGGGATAAATAACGGC
ATGCATCCAATGCACAACGAGGCTGCTCTCGGAGGTAGCGGCAGTGGTGGTCAAATGACGGGCGTG
GTGGTGGAGCAAGCAAGATGCTTCGGAGCTGGGACAGCCGGCGGTGATGGTCAAGGAACCTCTGCC
GCAGCTGCGCACAACAGTGGAGATGCTTCAGAAGAAGGAAAGTAA

**SEQ ID NO: 118, *Medicago truncatula* SYT2 polypeptide**
MQQTPQMIPMMPSFPQQTNITTEQIQKYLDENKKLILAILDNQNLGKLAECAQYQAQLQKNLMYLA
AIADAQPQTPALPPQMAPHPAMQQGFYMQHPQAAAMAQQQGMFPQKMPMQFGNPHQMQDQQHQQQQ
QQLHQQAMQGQMGLRPGGINNGMHPMHNEAALGGSGSGGPNDGRGGGSKQDASEAGTAGGDGQGTS
AAAAHNSGDASEEGK

FIGURE 25 (continued)

**SEQ ID NO: 119, *Panicum virgatum* SYT3 cDNA DN152517**
ATGCAGCAGCAGATGCCCATGCAGTCGGCGCCCCCGGCGACCGGCATCACCACCGAGCAGATCCAA
AAGTATTTGGATGAAAATAAGCAGCTTATTTTGGCCATCCTGGAAAATCAGAACTTAGGAAAGTTG
GCTGAATGTGCTCAGTATCAAGCTCAGCTTCAAAAGAATCTCTTGTACCTGGCTGCGATTGCAGAT
GCCCAACCCCAACCACCACAGAACCCTGCAAGTCGCCCACAGATGATGCAACCTGGCATGGTACCA
GGTGCAGGGCATTACATGTCCCAAGTACCAATGTTCCCGCCAAGAACACCATTAACCCCGCAACAG
ATGCAAGAACAGCAGCAGCAGCAGCAGCAGCTTCAACAGCAGCAAGCACAGGCTCTTGCTTTCCCG
GGACAGATGGTCATGAGACCTACCATTAATGGCATGCAGCCTATGCAAGCCGACCCTGCTGCCGCC
GCCGCCAGCCTACAGCAGTCAGCACCTGGCCCTACTGATGGGCGAGGAGGCAAGCAAGATGCAACT
GCTGGGGTGAGCACAGAGCCTTCTGGCACCGAGAGCCACAAGAGCACAACCGCAGCAGATCACGAT
GTGGGCACTGATGTCGCGGAGAAATCCTAA

**SEQ ID NO: 120, *Panicum virgatum* SYT3 polypeptide**
MQQQMPMQSAPPATGITTEQIQKYLDENKQLILAILENQNLGKLAECAQYQAQLQKNLLYLAAIAD
AQPQPPQNPASRPQMMQPGMVPGAGHYMSQVPMFPPRTPLTPQQMQEQQQQQQLQQQQAQALAFP
GQMVMRPTINGMQPMQADPAAAAASLQQSAPGPTDGRGGKQDATAGVSTEPSGTESHKSTTAADHD
VGTDVAEKS

**SEQ ID NO: 121, *Picea sitchensis* SYT1 cDNA DR484100 DR478464.1**
ATGCAGCAGCATCTCATGCAAATGCAGCCCATGATGGCGGCATACGCCTCCAACAACATCACCACT
GATCACATCCAGAAGTACCTGGATGAGAACAAGCAGTTGATTCTGGCAATTCTGGACAACCAAAAT
CTTGGAAAGCTCAATGAGTGTGCTCAGTACCAAGCAAAACTTCAGCAGAATTTGATGTATCTGGCT
GCGATTGCTGATTCTCAACCACAAGCACAAACTGCACATGCTCAGATTCCTCCTAATGCAGTGATG
CAGTCTGGTGGGCATTACATGCAGCACCAGCAGGCACAGCAACAAGTGACTCCTCAGTCTCTGATG
GCAGCTAGATCTTCCATGCTGTATTCTCAGCAGCCGATGGCTGCTTTGCATCAAGCTCAGCAACAA
CAGCAGCAGCAGCATCAGCAGCAACAACAATCTCTTCACAGCCAGCTTGGCATAAATTCTGGAGGA
AGCAGTGGATTGCATATGTTGCATGGTGAGACAAACATGGGATGTAATGGGCCTCTCTCATCTGGG
GGCTTCCCTGAATTTGGGCGTGGGTCTGCTACCTCTGCTGAAGGTATGCAGGCCAACAGGGGCTTC
ACTATAGATCGTGGTTCAAATAAGCAGGATGGAGTAGGATCAGAGAATGCCCATCCAGGTGCTGGT
GATGGAAGAGGGAGTTCAACTGGAGGGCAGAATGCAGATGAGTCAGAACCATCATACCTGAAAGCC
TCCGAAGAAGAAGGAAACTAG

**SEQ ID NO: 122, *Picea sitchensis* SYT1 polypeptide**
MQQHLMQMQPMMAAYASNNITTDHIQKYLDENKQLILAILDNQNLGKLNECAQYQAKLQQNLMYLA
AIADSQPQAQTAHAQIPPNAVMQSGGHYMQHQQAQQQVTPQSLMAARSSMLYSQQPMAALHQAQQQ
QQQQHQQQQQSLHSQLGINSGGSSGLHMLHGETNMGCNGPLSSGGFPEFGRGSATSAEGMQANRGF
TIDRGSNKQDGVGSENAHPGAGDGRGSSTGGQNADESEPSYLKASEEEGN

**SEQ ID NO: 123, *Pinus taeda* SYT1 cDNA DT625916**
ATGCAGCAGCACCTCATGCAAATGCAGCCCATGATGGCGGCCTACGCCTCCAACAATATCACCACT
GATCACATCCAGAAGTACCTGGATGAGAACAAGCAGTTGATTCTGGCAATTTTGGACAACCAAAAT
CTCGGAAAGCTCAATGAGTGTGCTCAATACCAAGCAAAACTTCAGCAGAATTTGATGTATCTGGCT
GCTATTGCTGATTCTCAACCTCAAGCACAAACTGCACATGCTCAGATTCCTCCAAATGCGGTGATG
CAGTCTGGTGGGCATTACATGCAGCATCAACAGGCACAGCAACAAGTTACTCCTCAGTCTCTGATG
GCAGCTAGATCTTCCATACTGTATGCTCAGCAACAACAGCAGCAGCAGCATCAGCAGCATCAGCAG
CAACAGCAGCAACAACAGTCTCTTCACAGCCAGCTTGGCATAAATTCTGGAGGAAGCAGCGGTTTG
CATATGTTGCATGGTGAGACAAACATGGGATGTAATGGGCCTCTGTCATCTGGGGGATTCCCTGAA

## FIGURE 25 (continued)

TTTGGGCGTGGGTCTGCTACCTCTGCTGATGGTATGCAGGTGAACAGGGGCTTTGCTATAGATCGT
GGTTCAAACAAGCAGGATGGAGTTGGATCAGAGAATGCCCATGCTGGTGCTGGTGATGGAAGAGGG
AGTTCAACTGGAGGGCAGAATGCAGATGAGTCAGAACCATCATACCTGAAGGCCTCCGAGGAAGAA
GGAAACTAG

**SEQ ID NO: 124, *Pinus taeda* SYT1 polypeptide**
MQQHLMQMQPMMAAYASNNITTDHIQKYLDENKQLILAILDNQNLGKLNECAQYQAKLQQNLMYLA
AIADSQPQAQTAHAQIPPNAVMQSGGHYMQHQQAQQQVTPQSLMAARSSILYAQQQQQQHQQHQQ
QQQQQQSLHSQLGINSGGSSGLHMLHGETNMGCNGPLSSGGFPEFGRGSATSADGMQVNRGFAIDR
GSNKQDGVGSENAHAGAGDGRGSSTGGQNADESEPSYLKASEEEGN

**SEQ ID NO: 125, *Populus tremula* SYT1 cDNA DT476906**
ATGCAACAGCACCTGATGCAGATGCAGCCCATGATGGCAGCCTATTACCCCAGCAACGTCACTACT
GATCATATTCAACAGTATCTGGACGAAAACAAGTCATTGATTTTGAAGATTGTTGAGAGCCAGAAT
TCAGGGAAACTCAGTGAGTGTGCAGAGAACCAAGCAAGACTGCAACAAAATCTCATGTACTTGGCT
GCAATTGCTGATTGTCAGCCCCAACCACCTACCATGCATGCCCAGTTCCCTTCCAGCGGCATTATG
CAGCCAGGAGCACATTACATGCAGCATCAACAAGCTCAACAGATGACACCACAAGCCCTTATGGCT
GCACGCTCTTCTATGCTGCAGTATGCTCAACAGCCATTCTCAGCGCTTCAACAACAGCAAGCCTTA
CACAGCCAGCTCGGCATGAGCTCTGGTGGAAGCGCAGGACTTCATATGATGCAAAGCGAGGCTAAC
ACTGCAGGAGGCAGTGGAGCTCTTGGTGCTGGACGATTTCCTGATTTTGGCATGGATGCCTCCAGT
AGAGGAATCGCAAGTGGGAGCAAGCAAGATATTCGGAGTGCAGGGTCTAGTGAAGGGCGAGGAGGA
AGCTCTGGAGGCCAGGGTGGTGATGGAGGTGAAACCCTTTACTTGAAATCTGCTGATGATGGGAAC
TGA

**SEQ ID NO: 126, *Populus tremula* polypeptide**
MQQHLMQMQPMMAAYYPSNVTTDHIQQYLDENKSLILKIVESQNSGKLSECAENQARLQQNLMYLA
AIADCQPQPPTMHAQFPSSGIMQPGAHYMQHQQAQQMTPQALMAARSSMLQYAQQPFSALQQQQAL
HSQLGMSSGGSAGLHMMQSEANTAGGSGALGAGRFPDFGMDASSRGIASGSKQDIRSAGSSEGRGG
SSGGQGGDGGETLYLKSADDGN

**SEQ ID NO: 127, *Saccharum officinarum* SYT1 cDNA CA078249.1 CA078630 CA082679 CA234526 CA239244 CA083312**
ATGCAGCAGCAACACCTGATGCAGATGAACCAGAACATGATTGGGGGCTACACCTCTCCTGCCGCT
GTGACAACCGATCTCATCCAGCAGTACCTGGATGAGAACAAGCAGCTGATCCTGGCCATCCTCGAC
AACCAGAACAATGGCAAGGTGGAGGAGTGCGAACGGCACCAAGCTAAGCTCCAGCACAACCTCATG
TACCTGGCCGCCATCGCCGACAGCCAGCCACCACAGACTGCACCACTATCACAATACCCGTCCAAC
CTGATGATGCAGCCGGGCCCTCGGTACATGCCACCGCAGTCCGGGCAGATGATGAGCCCGCAGTCG
CTAATGGCGGCGCGGTCCTCCATGATGTACGCGCACCCGTCCATGTCACCACTCCAGCAGCAGCAG
GCAGCGCACGGGCAGCTGGGCATGGCTTCAGGGGGCGGCGGTGGCACGACCAGTGGGTTCAACATC
CTCCATGGCGAGGCCAGTATGGGCGGTGCTGGTGGCGCTTGTGCCGGCAACAACATGATGAACGCC
GGCATGTTCTCAGGCTTTGGCCGCAGCGGCAGTGGCGCCAAGGAGGGATCGACCTCGCTGTCGGTT
GACGTCCGTGGTGGCACCAGCTCCGGCGCGCAAAGCGGGGACGGCGAGTACCTGAAAGCAGGCACC
GAGGAAGAAGGCAGTTAA

**SEQ ID NO: 128, *Saccharum officinarum* SYT1 polypeptide**
MQQQHLMQMNQNMIGGYTSPAAVTTDLIQQYLDENKQLILAILDNQNNGKVEECERHQAKLQHNLM
YLAAIADSQPPQTAPLSQYPSNLMMQPGPRYMPPQSGQMMSPQSLMAARSSMMYAHPSMSPLQQQQ
AAHGQLGMASGGGGGTTSGFNILHGEASMGGAGGACAGNNMMNAGMFSGFGRSGSGAKEGSTSLSV
DVRGGTSSGAQSGDGEYLKAGTEEEGS

**FIGURE 25 (continued)**

**SEQ ID NO: 129, *Saccharum officinarum* SYT2 cDNA CA110367**
ATGCAGCAGCCGATGCCCATGCAGCCGCAGGCGCCGGAGATGACCCCGGCCGCCGGAATCACCACG
GAGCAGATCCAAAAGTATCTGGATGAGAATAAGCAGCTTATTTTGGCTATTTTGGAAAATCAGAAC
CTAGGAAAATTGGCAGAATGTGCTCAGTATCAATCACAACTTCAGAAGAACCTCTTGTATCTCGCT
GCAATCGCAGATGCCCAACCACAGACTGCTGTAAGCCGCCCTCAGATGGCGCCGCCTGGTGCATTG
CCTGGAGTAGGGCAGTACATGTCACAGGTGCCTATGTTCCCACCGAGGACACCTCTAACACCCCAG
CAGATGCAGGAGCAGCAACTTCAGCAGCAGCAGGCTCAGCTGCTAAATTTCAGTGGCCTAATGGTT
GCTAGACCTGGCATGGTCAACGGCATGCCTCAGTCCATTCAAGTTCAGCAAGCTCAGCCACCACCA
GCAGGGAACAAACAGGATGCTGGTGGGGTCGCCTCGGAGCCCTCGGGCATTGAGAACCACAGGAGC
ACTGGTGGTGATAATGATGGTGGAAGCGACTAG

**SEQ ID NO: 130, *Saccharum officinarum* SYT2 polypeptide**
MQQPMPMQPQAPEMTPAAGITTEQIQKYLDENKQLILAILENQNLGKLAECAQYQSQLQKNLLYLA
AIADAQPQTAVSRPQMAPPGALPGVGQYMSQVPMFPPRTPLTPQQMQEQQLQQQQAQLLNFSGLMV
ARPGMVNGMPQSIQVQQAQPPPAGNKQDAGGVASEPSGIENHRSTGGDNDGGSD

**SEQ ID NO: 131, *Saccharum officinarum* SYT3 cDNA CA161933.1 CA265085**
ATGCAGCAGCAGATGCCCATGCCGCCGGCGCCCGCTGCGGCGGCGGCGCCCCCGGCGGCCGGCATC
ACCACCGAGCAGATCCAAAAGTATTTGGACGAAAATAAGCAACTTATTTTGGCCATCCTGGAAAAT
CAGAACTTAGGAAAGTTGGCTGAATGTGCTCAGTATCAAGCTCAACTTCAAAAGAACCTCTTGTAC
CTGGCTGCGATTGCTGATGCCCAACCCCAGCCACCACAAAACCCTGCAGGTCGCCCTCAGATGATG
CAACCTGGTATAGTGCCAGGTGCGGGGCATTACATGTCACAAGTACCAATGTTCCCTCCAAGAACT
CCATTAACCCCACAGCAGATGCAAGAGCAGCAGCAGCAACAGCTTCAGCAGCAGCAAGCGCAGGCT
CTTACATTCCCTGGACAGATGGTCATGAGACCAGCTACCATCAACGGCATACAGCAGCCTATGCAA
GCTGACCCTGCCCGGGCAGCGGAGCTGCAACAACCACCACCTATCCCAGCTGACGGGCGAGTAAGC
AAGCAGCAGGACACAACGGCTGGCGTGAGCTCAGAGCCTTCTGCCAATGAGAGCCACAAGACCACA
ACTGGAGCAGATAGTGAGGCAGGTGGTGACGTGGCGGAGAAATCCTAA

**SEQ ID NO: 132, *Saccharum officinarum* SYT3 polypeptide**
MQQQMPMPPAPAAAAAPPAAGITTEQIQKYLDENKQLILAILENQNLGKLAECAQYQAQLQKNLLY
LAAIADAQPQPPQNPAGRPQMMQPGIVPGAGHYMSQVPMFPPRTPLTPQQMQEQQQQQLQQQQAQA
LTFPGQMVMRPATINGIQQPMQADPARAAELQQPPPIPADGRVSKQQDTTAGVSSEPSANESHKTT
TGADSEAGGDVAEKS

**SEQ ID NO: 133, *Solanum tuberosum* SYT1 cDNA CK265597**
ATGCAGCAGCACCTGATGCAGATGCAGCCCATGATGGCAGCTTACTATCCAACGAACGTCACTACT
GACCATATTCAACAGTATTTGGATGAGAACAAATCACTCATTCTGAAAATTGTTGAGAGCCAAAAC
TCGGGAAAACTCAGTGAATGTGCAGAGAACCAAGCTAGGCTTCAGAGGAATCTGATGTACCTTGCT
GCTATTGCTGATTCACAACCTCAGCCTTCTAGCATGCATTCTCAGTTCTCTTCTGGTGGGATGATG
CAGCCAGGGACACACAGTTACCTGCAGCAGCAGCAGCAGCAACAACAAGCGCAACAAATGGCAACA
CAACAACTCATGGCTGCAAGATCCTCATCAATGCTCTATGGACAACAACAGCAGCAGCAGCAGCAG
TCTCAGTTATCACAATTTCAACAAGGCTTGCATAGTAGCCAACTTGGCATGAGTTCTGGCAGTGGT
GGAAGCACTGGACTTCATCACATGCTTCAAAGTGAATCATCACCTCATGGTGGTGGTTTCTCTCAT
GACTTCGGCCGTGCAAATAAGCAAGACATTGGGAGTAGTATGTCTGCTGAAGGGCGCGGCGGAAGC
TCAGGTGGTGATGGTGGTGAGAATCTTTATCTGAAAGCTTCTGAGGATTGA

## FIGURE 25 (continued)

**SEQ ID NO: 134, *Solanum tuberosum* SYT1 polypeptide**
MQQHLMQMQPMMAAYYPTNVTTDHIQQYLDENKSLILKIVESQNSGKLSECAENQARLQRNLMYLA
AIADSQPQPSSMHSQFSSGGMMQPGTHSYLQQQQQQQAQQMATQQLMAARSSSMLYGQQQQQQQ
SQLSQFQQGLHSSQLGMSSGSGGSTGLHHMLQSESSPHGGGFSHDFGRANKQDIGSSMSAEGRGGS
SGGDGGENLYLKASED

**SEQ ID NO: 135, *Sorghum bicolor* SYT3 cDNA CX611128**
ATGCAGCAGCAGATGCCCATGCCGCCGGCGCCCGCTGCGGCGGCGGCGACGGCGCCCCCGGCGGCC
GGCATCACCACCGAGCAGATCCAGAAGTATTTGGACGAAAATAAGCAACTTATTTGGCCATCCTA
GAAAATCAGAACTTAGGAAAGTTGGCTGAATGTGCTCAGTATCAAGCTCAACTTCAAAAGAACCTC
TTGTACCTGGCTGCGATTGCTGATGCCCAACCCCGACCACCGCAAAACCCTGCAGGTCGCCCTCAG
ATGATGCAACCTGGTATAGTGCCAGGTGCAGGGCATTACATGTCACAAGTACCAATGTTCCCTCCA
AGAACTCCATTAACCCCACAGCAAATGCAAGAGCAGCAGCAGCAACAGCTTCAGCAGCAGCAAGCG
CAGGCTCTTGCATTCCCTGGGCAGATGGTCATGAGACCAGCTACCATCAACGGCATGCAGCAGCCT
ATGCAGGCTGACCCTGCCCGGGCAGCGGAGCTGCAACAGCCAGCATCTGTCCCAGCCGACGGGCGA
GTAAGCAAGCAGGACACAGCGGCTGGGGTGAGCTCAGAGCCTTCTGCCAATGAGAGCCACAAGACC
ACAACCGGAGCAGATAGTGAGGCAGGTGGAGACGTGGCGGAGAAATCCTAA

**SEQ ID NO: 136, *Sorghum bicolor* SYT3 polypeptide**
MQQQMPMPPAPAAAAATAPPAAGITTEQIQKYLDENKQLILAILENQNLGKLAECAQYQAQLQKNL
LYLAAIADAQPRPPQNPAGRPQMMQPGIVPGAGHYMSQVPMFPPRTPLTPQQMQEQQQQLQQQA
QALAFPGQMVMRPATINGMQQPMQADPARAAELQQPASVPADGRVSKQDTAAGVSSEPSANESHKT
TTGADSEAGGDVAEKS

**SEQ ID NO: 137, *Triticum aestivum* SYT2 cDNA CD901951**
ATGCAGCAAGCGATGCCCATGCCGCCGGCGGCGGCGGCGCCGGGGATGCCTCCGTCTGCTGGCCTC
AGCACCGAGCAGATCCAAAAGTACCTGGATGAAAATAAGCAACTAATTTTGGCTATCTTGGAAAAT
CAGAACCTGGGAAAGTTGGCGGAATGTGCTCAGTATCAAGCTCAGCTTCAGAAGAATCTTTTGTAT
TTGGCTGCAATCGCTGATACTCAGCCACAGACCACTGTAAGCCGTCCTCAGATGGCACCACCTAGT
GCATCCCCAGGGGCAGGGCATTACATGTCACAGGTGCCAATGTTCCCTCCGAGGACCCCTCTAACG
CCTCAGCAGATGCAGGAGCAGCAACTACAGCAGCAACAGGCTCAGATGCTTCCGTTTGCTGGTCAA
ATGGTTGCGAGACCTGGGGCTGTCAATGGCATGCCTCAGGCCCCTCAAGTTGAACCAGCCTATGCA
GCAGGTGGGGCCAGTTCTGAGCCTTCTGGCACTGAGAGCCACAGGAGCACTGGTGCCGATAATGAC
GGGGGGAGCGGCTGGGCTGATCAGTCCTAA

**SEQ ID NO: 138, *Triticum aestivum* SYT2 polypeptide**
MQQAMPMPPAAAAPGMPPSAGLSTEQIQKYLDENKQLILAILENQNLGKLAECAQYQAQLQKNLLY
LAAIADTQPQTTVSRPQMAPPSASPGAGHYMSQVPMFPPRTPLTPQQMQEQQLQQQQAQMLPFAGQ
MVARPGAVNGMPQAPQVEPAYAAGGASSEPSGTESHRSTGADNDGGSGWADQS

**SEQ ID NO: 139, *Triticum aestivum* SYT3 cDNA BJ246754 BJ252709**
ATGCAGCAGGCGATGTCCTTGCCCCCGGGAGCGGTCGGCGCGGTGTCCTCGCCGGCCGGCATCACC
ACCGAGCAGATCCAAAAGTATTTGGATGAAAATAAGCAACTTATTTTGGCCATCCTTGAAAATCAG
AACCTAGGAAAGTTGGCTGAATGTGCTCAGTATCAAGCTCAACTCCAAAAGAATCTCTTGTATCTA
GCTGCTATCGCGGATGCCCAACCACCACAGAACCCTACAAGTCACCCTCAGATGGTGCAGCCTGGT
AGTATGCAAGGTGCAGGGCATTACATGTCACAAGTACCAATGTTCCCTCCAAGAACGCCTTTAACC
CCACAGCAGATGCAAGAGCAGCAGCACCAGCAGCTTCAGCAGCAGCAAGCCCAGGCCCTTTCTTTC
CCCGCCCAGGTGGTCATGAGACCAGGCACCGTCAACGGCATGCAGCAGCCTATGCAAGCAGCCGGC

**FIGURE 25 (continued)**

GACCTCCAGCCAGCAGCAGCACCTGGAGGGAGCAAGCAGGACGCCGCAGTGGCTGGGGCCAGCTCG
GAACCATCTGGCACCAAGAGCCACAAGAACGCGGGAGCAGAGGAGGTGGGCGCTGATGTAGCAGAA
CAATCCTAA

**SEQ ID NO: 140, *Triticum aestivum* SYT3 polypeptide**
MQQAMSLPPGAVGAVSSPAGITTEQIQKYLDENKQLILAILENQNLGKLAECAQYQAQLQKNLLYL
AAIADAQPPQNPTSHPQMVQPGSMQGAGHYMSQVPMFPPRTPLTPQQMQEQQHQQLQQQQAQALSF
PAQVVMRPGTVNGMQQPMQAAGDLQPAAAPGGSKQDAAVAGASSEPSGTKSHKNAGAEEVGADVAE
QS

**SEQ ID NO: 141, *Vitis vinifera* SYT1 cDNA DV219834**
ATGCAGCAGCACCTGATGCAGATGCAGCCCATGATGGCAGCCTATTACCCCAGCAACGTCACCACT
GATCACATTCAGCAGTATCTTGATGAAAACAAGTCATTGATTCTGAAGATTGTTGAGAGCCAGAAT
TCAGGAAAATTGACTGAATGTGCAGAGAACCAGGCAAGACTACAGAGAAACCTCATGTACCTGGCT
GCAATTGCTGATTCTCAACCCCAACCACCCACCATGCATGCTCAGTTCCCTCCTAGTGGCATTGTT
CAGCCAGGAGCTCACTACATGCAACACCAACAAGCTCAACAAATGACACCACAGTCGCTCCTGGCT
GCACGCTCCTCCATGCTGTACACCCAACAACCATTTTCGGCCCTGCAACAACAACAAGCCATCCAT
AGCCAGCTTGGCATGGGCTCTGGTGGAAGTGCAGGACTTCACATGCTGCAAAGCGAGGGGAGTAAT
CCAGGAGGCAATGGAACACTGGGGACTGGTGGGTTTCCTGATTTCAGCCGTGGAACTTCTGGAGAA
GGCCTGCAGGCTGCAGGCAGGGGAATGGCTGGTGGGAGCAAGCAAGATATGGGAAATGCAGAAGGG
CGAGGAGGGAACTCAGGAGGTCAGGGTGGGGATGGAGGTGAGACTCTTTACTTGAAAGCTGCTGAA
GATGGGAATTGA

**SEQ ID NO: 142, *Vitis vinifera* SYT1 polypeptide**
MQQHLMQMQPMMAAYYPSNVTTDHIQQYLDENKSLILKIVESQNSGKLTECAENQARLQRNLMYLA
AIADSQPQPPTMHAQFPPSGIVQPGAHYMQHQQAQQMTPQSLLAARSSMLYTQQPFSALQQQQAIH
SQLGMGSGGSAGLHMLQSEGSNPGGNGTLGTGGFPDFSRGTSGEGLQAAGRGMAGGSKQDMGNAEG
RGGNSGGQGGDGGETLYLKAAEDGN

**SEQ ID NO: 143, *Zea mays* SYT3 cDNA CO468901**
ATGCAGCAGCAGATGCCCATGCCGCCGGCGCCCGCTGCCGCCGCGGCGGCGGCGCCCCCGGCGGCA
GGCATCACTACCGAGCAGATCCAGAAGTATTTGGACGAAAATAAGCAACTTATTTTGGCCATCCTG
GAAAATCAGAACTTAGGGAAGTTGGCTGAATGTGCTCAGTATCAAGCTCAACTTCAAAAGAACCTC
TTGTACCTGGCTGCGATTGCTGATGCCCAACCCCAGCCTCCGCAAAACCCTGCAGGTCGCCCTCAG
ATGATGCAGCCTGGTATAGTGCCAGGTGCGGGGCATTACATGTCACAAGTACCAATGTTCCCTCCA
AGAACCCCATTAACCCCACAGCAGATGCAGGAGCAGCAGCAACAACAACAGTTTCAGCAGCAGCAG
CAGCAAGTGCAGGCTCTTACATTTCCTGGACAGATGGTCATGAGACCAGGCACCATCAACGGCATG
CAGCAGCAGCAGCCTATGCAGGCTGACCCTGCCCGGGCAGCAGCGGAGCTGCAGCAGGCAGCACCT
ATCCCAGCTGACGGGCGAGGAAGCAAGCAGGACACCGCGGGTGGGGCGAGCTCAGAGCCTTCTGCC
AATGAGAGCCACAAGAGCGCCACCGGAGCAGATACCGAGGCAGGTGGCGACGTGGCCGAGAAATCC
TAA

**SEQ ID NO: 144, *Zea mays* SYT3 polypeptide**
MQQQMPMPPAPAAAAAAAPPAAGITTEQIQKYLDENKQLILAILENQNLGKLAECAQYQAQLQKNL
LYLAAIADAQPQPPQNPAGRPQMMQPGIVPGAGHYMSQVPMFPPRTPLTPQQMQEQQQQQFQQQQ
QQVQALTFPGQMVMRPGTINGMQQQQPMQADPARAAAELQQAAPIPADGRGSKQDTAGGASSEPSA
NESHKSATGADTEAGGDVAEKS

## FIGURE 25 (continued)

**SEQ ID NO: 145, *Oryza sativa* GOS2 promoter PRO0129**
AATCCGAAAAGTTTCTGCACCGTTTTCACCCCCTAACTAACAATATAGGGAACGTGTGCTAAATAT
AAAATGAGACCTTATATATGTAGCGCTGATAACTAGAACTATGCAAGAAAAACTCATCCACCTACT
TTAGTGGCAATCGGGCTAAATAAAAAAGAGTCGCTACACTAGTTTCGTTTTCCTTAGTAATTAAGT
GGGAAAATGAAATCATTATTGCTTAGAATATACGTTCACATCTCTGTCATGAAGTTAAATTATTCG
AGGTAGCCATAATTGTCATCAAACTCTTCTTGAATAAAAAAATCTTTCTAGCTGAACTCAATGGGT
AAAGAGAGAGATTTTTTTTAAAAAAATAGAATGAAGATATTCTGAACGTATTGGCAAAGATTTAAA
CATATAATTATATAATTTTATAGTTTGTGCATTCGTCATATCGCACATCATTAAGGACATGTCTTA
CTCCATCCCAATTTTTATTTAGTAATTAAAGACAATTGACTTATTTTTATTATTTATCTTTTTTCG
ATTAGATGCAAGGTACTTACGCACACACTTTGTGCTCATGTGCATGTGTGAGTGCACCTCCTCAAT
ACACGTTCAACTAGCAACACATCTCTAATATCACTCGCCTATTTAATACATTTAGGTAGCAATATC
TGAATTCAAGCACTCCACCATCACCAGACCACTTTTAATAATATCTAAAATACAAAAAATAATTTT
ACAGAATAGCATGAAAAGTATGAAACGAACTATTTAGGTTTTTCACATACAAAAAAAAAAAGAATT
TTGCTCGTGCGCGAGCGCCAATCTCCCATATTGGGCACACAGGCAACAACAGAGTGGCTGCCCACA
GAACAACCCACAAAAAACGATGATCTAACGGAGGACAGCAAGTCCGCAACAACCTTTTAACAGCAG
GCTTTGCGGCCAGGAGAGAGGAGGAGAGGCAAAGAAAACCAAGCATCCTCCTCCTCCCATCTATAA
ATTCCTCCCCCCTTTTCCCCTCTCTATATAGGAGGCATCCAAGCCAAGAAGAGGGAGAGCACCAAG
GACACGCGACTAGCAGAAGCCGAGCGACCGCCTTCTTCGATCCATATCTTCCGGTCGAGTTCTTGG
TCGATCTCTTCCCTCCTCCACCTCCTCCTCACAGGGTATGTGCCCTTCGGTTGTTCTTGGATTTAT
TGTTCTAGGTTGTGTAGTACGGGCGTTGATGTTAGGAAAGGGGATCTGTATCTGTGATGATTCCTG
TTCTTGGATTTGGGATAGAGGGGTTCTTGATGTTGCATGTTATCGGTTCGGTTTGATTAGTAGTAT
GGTTTTCAATCGTCTGGAGAGCTCTATGGAAATGAAATGGTTTAGGGTACGGAATCTTGCGATTTT
GTGAGTACCTTTTGTTTGAGGTAAAATCAGAGCACCGGTGATTTTGCTTGGTGTAATAAAAGTACG
GTTGTTTGGTCCTCGATTCTGGTAGTGATGCTTCTCGATTTGACGAAGCTATCCTTTGTTTATTCC
CTATTGAACAAAAATAATCCAACTTTGAAGACGGTCCCGTTGATGAGATTGAATGATTGATTCTTA
AGCCTGTCCAAAATTTCGCAGCTGGCTTGTTTAGATACAGTAGTCCCCATCACGAAATTCATGGAA
ACAGTTATAATCCTCAGGAACAGGGGATTCCCTGTTCTTCCGATTTGCTTTAGTCCCAGAATTTTT
TTTCCCAAATATCTTAAAAAGTCACTTTCTGGTTCAGTTCAATGAATTGATTGCTACAAATAATGC
TTTTATAGCGTTATCCTAGCTGTAGTTCAGTTAATAGGTAATACCCCTATAGTTTAGTCAGGAGAA
GAACTTATCCGATTTCTGATCTCCATTTTTAATTATATGAAATGAACTGTAGCATAAGCAGTATTC
ATTTGGATTATTTTTTTTATTAGCTCTCACCCCTTCATTATTCTGAGCTGAAAGTCTGGCATGAAC
TGTCCTCAATTTTGTTTTCAAATTCACATCGATTATCTATGCATTATCCTCTTGTATCTACCTGTA
GAAGTTTCTTTTTGGTTATTCCTTGACTGCTTGATTACAGAAAGAAATTTATGAAGCTGTAATCGG
GATAGTTATACTGCTTGTTCTTATGATTCATTTCCTTTGTGCAGTTCTTGGTGTAGCTTGCCACTT
TCACCAGCAAAGTTC

**SEQ ID NO: 146, Box I**
IQ(Q/K)(Y/M/F/H)L(D/E)(E/D)N(K/N)XLI

Where X is any amino acid

**SEQ ID NO: 147, Box II**
NL(M/L/V)YLA(A/T)IAD

**SEQ ID NO: 148, prm06681**
GGGGACAAGTTTGTACAAAAAAGCAGGCTTAAACAATGCAACAGCACCTGATG

**SEQ ID NO: 149, Prm06682**
GGGGACCACTTTGTACAAGAAAGCTGGGTCATCATTAAGATTCCTTGTGC

## FIGURE 25 (continued)

**SEQ ID NO: 150, *Brassica napus* SYT cDNA**

ATGCAACAGCACCTGATGCAGATGCAGCCCATGATGGCTGGTTACTACCCCAGCAATGTCACCTCT
GATCATATTCAGCAGTACTTGGACGAGAACAAATCGTTGATTCTGAAGATAGTTGAATCTCAAAAC
TCGGGAAAGCTCAGCGAGTGTGCCGAGAACCAGGCAAGGCTTCAACGCAACTTAATGTACTTAGCT
GCAATTGCAGATTCTCAGCCTCAACCTCCAAGCATGCATAGCCAGTATGGAACTGCTGGTGGTGGT
GGGTTGATGCAGGGAGAAGGAGGGTCACACTATTTGCAACAGCAACAGGCAATTCAACAGCAGCAG
AGTCAGCAGTCTCTAATGGCGGCTCGATCTTCAATGTTGTATGCTCAGCAGCAGCAACAGCCTTAT
GCAACGCTTCAGCAGCAGCAATTGCACCATAGCCAGCTTGGGATGAGCTCAAGCAGCGGAGGAGGA
AGCAGCGGTCTCCATATGCTACAGGGAGAGGCTGGTGGGTTTCATGATTTTGGCCGTGAGAAGTTG
GAAATGGGAAGTGGTGAAGGCAGAGGAGGAAGCTCAGGGGATGGTGGAGAAACCCTTTACTTGAAG
TCATCAGATGATGGGAACTGA

**SEQ ID NO: 151, *Brassica napus* SYT polypeptide**

MQQHLMQMQPMMAGYYPSNVTSDHIQQYLDENKSLILKIVESQNSGKLSECAENQARLQRNLMYLA
AIADSQPQPPSMHSQYGTAGGGGLMQGEGGSHYLQQQQAIQQQQSQQSLMAARSSMLYAQQQQQPY
ATLQQQQLHHSQLGMSSSSGGGSSGLHMLQGEAGGFHDFGREKLEMGSGEGRGGSSGDGGETLYLK
SSDDGN

**SEQ ID NO: 152, *Glycine max* SYT cDNA**

ATGCAGCAGCACCTGATGCAGATGCAGCCCATGATGGCTGCCTACTACCCCAACAACGTCACCACT
GATCACATTCAACAGTACCTGGATGAGAACAAGTCCTTGATTCTGAAGATTGTTGAAAGCCAGAAT
TCTGGCAAGCTGAGCGAGTGTGCCGAGAACCAATCAAGGCTGCAGAGAAATCTCATGTACCTAGCT
GCAATAGCTGATTCTCAACCACAACCATCTCCATTGGCTGGTCAGTATCCTTCTAGTGGACTTGTG
CAGCAGGGAGCACACTACATGCAGGCTCAACAGGCTCAGCAGATGTCACAACAACAGCTAATGGCT
TCGCGCTCCTCGCTCCTGTACTCCCAACAGCCTTTCTCAGTGCTTCAACAGCAGCAAGGCATGCAC
AGCCAACTTGGCATGAGCTCCAGTGGAAGTCAAGGCCTCCACATGCTGCAAAGTGAAGCCACTAAT
GTTGGAGGCAATGCAACCATAGGAACCGGAGGAGGGTTTCCGGACTTTGTACGCATTGGTAGTGGC
AAGCAAGATATTGGAATCTCTGGTGAAGGCAGAGGAGGAAACTCTAGTGGCCACTCTGGTGATGGT
GGTGAGACACTTAATTACCTGAAAGCTGCTGGTGATGGAAACTGA

**SEQ ID NO: 153, *Glycine max* SYT polypeptide**

MQQHLMQMQPMMAAYYPNNVTTDHIQQYLDENKSLILKIVESQNSGKLSECAENQSRLQRNLMYLA
AIADSQPQPSPLAGQYPSSGLVQQGAHYMQAQQAQQMSQQQLMASRSSLLYSQQPFSVLQQQQGMH
SQLGMSSSGSQGLHMLQSEATNVGGNATIGTGGGFPDFVRIGSGKQDIGISGEGRGGNSSGHSGDG
GETLNYLKAAGDGN

**SEQ ID NO: 154, *Chlamydomonas reinhardtii* chloroplastic fructose-1,6-biphosphatase (Chlre_cpFBPase) nucleic acid sequence, derived from AW721488, BQ811919, CF555540**

ATGGCCGCCACCATGCTCCGCTCCAGCACCCAGTCGGGCATTGCCGCTAAGGCCGGCCGCAAGGAG
GCTGTCAGCGTCCGCGCGGTCGCCCAGCCCCAGCGCCAGGCTGGTGCTGCCAGCGTCTTCTCCTCG
TCGTCGTCGGGCGCTGCTGCTCGCCGCGGGGTCGTCGCTCAGGCCACCGCTGTTGCCACCCCCGCG
GCCAAGCCTGCGGCCAAGACCAGCCAGTATGAGCTGTTCACGCTCACCACCTGGCTGCTGAAGGAG
GAGATGAAGGGCACAATCGATGGCGAGCTTGTGACCGTCATCTCGTCGGTGTCGCTGGCCTGCAAG
CAAATCGCGTCGCTGGTGAACCGCGCTGGTATCTCCAACCTGACCGGTGTGGCTGGCAACCAGAAC
GTGCAGGGTGAGGACCAGAAGAAGCTGGACGTGGTGTCCAACGAGGTCTTCAAGAACTGCCTGGCC
TCCTGCGGCCGCACGGGTGTGATCGCCTCCGAGGAGGAGGACCAGCCCGTGGCCGTGGAGGAGACC
TACTCGGGCAACTACATCGTGGTGTTCGACCCCCTGGACGGCTCGTCCAACATCGACGCCGGCATC

## FIGURE 25 (continued)

TCCGTCGGCTCCATCTTCGGCATCTACGAGCCCAGCGAGGAGTGCCCCATTGACGCCATGGACGAC
CCCCAGAAGATGATGGAGCAGTGCGTCATGAACGTGTGCCAGCCCGGCTCGCGCCTCAAGTGCGCC
GGCTACTGCCTGTACAGCAGCAGCACCATCATGGTGCTGACCATCGGCAACGGTGTGTTCGGCTTC
ACGCTGGACCCCCTGGTCGGCGAGTTCGTGCTGACCCACCCCAACGTGCAGATCCCCGAGGTGGGC
AAGATCTACCCGTTCAACGAGGGCAACTACGGCCTGTGGGACGACAGCGTTAAGGCTTACATGGAC
AGCCTGAAGGACCCCAAGAAGTGGGACGGCAAGCCCTACTCGGCCCGCTACATCGGCTCCCTGGTC
GGTGACTTCCACCGCACCCTGCTGTACGGAGGCATCTACGGCTACCCCGGCGACGCCAAGAACAAG
AACGGCAAGCTCCGCCTGCTGTACGAGTGCGCGCCCATGTCGTTCATTGCCGAGCAGGCCGGCGGC
CTCGGCTCCACCGGCCAGGAGCGCGTGCTGGACGTGAACCCCGAGAAGGTGCACCAGCGCGTGCCG
CTGTTCATCGGCTCCAAGAAGGAGGTCGAGTACCTGGAGTCCTTCACCAAGAAGCACTAA

**SEQ ID NO: 155, *Chlamydomonas reinhardtii* chloroplastic fructose-1,6-biphosphatase (Chlre_cpFBPase) deduced polypeptide sequence**
MAATMLRSSTQSGIAAKAGRKEAVSVRAVAQPQRQAGAASVFSSSSSGAAARRGVVAQATAVATPA
AKPAAKTSQYELFTLTTWLLKEEMKGTIDGELVTVISSVSLACKQIASLVNRAGISNLTGVAGNQN
VQGEDQKKLDVVSNEVFKNCLASCGRTGVIASEEEDQPVAVEETYSGNYIVVFDPLDGSSNIDAGI
SVGSIFGIYEPSEECPIDAMDDPQKMMEQCVMNVCQPGSRLKCAGYCLYSSSTIMVLTIGNGVFGF
TLDPLVGEFVLTHPNVQIPEVGKIYPFNEGNYGLWDDSVKAYMDSLKDPKKWDGKPYSARYIGSLV
GDFHRTLLYGGIYGYPGDAKNKNGKLRLLYECAPMSFIAEQAGGLGSTGQERVLDVNPEKVHQRVP
LFIGSKKEVEYLESFTKKH

**SEQ ID NO: 156, *Bigelowiella natans* chloroplastic fructose-1,6-biphosphatase (Bigna_cpFBPase) nucleic acid sequence AY267678**
ATGGCTGCCGTGCTCTTTCGCGGAGGTGTGCTGTCTTCCACCATGACTGCGGCTCGCACATTCGCA
GCGCCATCTGTGCACACCCGTGGCATGCACATGTCAGTCAAGAGCAGTAACCCTTTCTCTCAGGCT
GGTCGTCGTGCTGCTGTTAGATCCAGTGTCGCACCCTCCCCTGTCCAAGACTCTGCTGGTACTATC
GTTGATGACGGAACTGTCACATTAACAAGATTCATGATTGAGGAGGCAATGAAGAGCAAGACTCCT
GGGCAGGAGGACATGGTTCGTTTGATTTCTTCCATCTCAGTTGCATGCAAACGCATTGCATCCATG
GTGCAAACTGCAGGAATCTCCGGATCTACAGGTCTGGCTGAAGGTGGTGGATCAGTCAACGTTCAA
GGCGAGGAACAGAAGAAACTTGATGTCATTTCTAACGATGTACTAAAGTCTGCCCTTCGTCCTTCT
GGAAAACTTGGAGTAATTGCCTCTGAGGAGGAAGATAATCCAGTTGTCGTTGATGAACTTTACTCC
GGCGAATATGTTGCTACTTTCGATCCGCTCGATGGATCTTCCAATATTGATGCTGCAATTTCCACT
GGAACTATCTTCGGAGTGTTCAAAGCTCCAGAAGAGTGCTTGATCGGGGATTCTGATAATCTCAGT
ATTGCAGAGCAGCAATGTTTGGAGGCAACACTTCAACCTGGAACTAACCTTGTTGCTGCTGGATAC
TGCATGTATTCGTCCTCCACCATCCTTGTTCTCACCACCGGAGACGGGCTCAATGGATTTACTCTT
GACCCTTCCATTGGAGAGTTCATCCTCACCCATCCTAATATCCAGATTCCTAGCCGTGGAAAGATC
TATTCTATGAACGAAGCAAACTACTTCGATTGGGACCCTAAGCTTCAGACCTACGTTGATAACCTT
AAGAAGGCAGAAGGTCAAACTGGAGAAAAGTACAGCTCTCGCTACATCGGATCCATGGTCGGAGAT
GTGCACCGTACCCTTCTCTATGGAGGCATCTTCGCTTACCCTGGAGATAAGAAGAACGTCAACGGA
AAACTTCGCCTTCTGTACGAAGCTGCTCCAATGTCCCTTATCTTCGAACAAGCGGGTGGAAAATCT
ATTACTGGACCTGGTGGACGTGTGTTGGACTTAGTTCCTGATAAGGTTCACCAGCGTTGTCCTGTG
TTCATTGGATCCCCTGATGATGTGGATGAAGTTGAAAAGGCTCTCGCATAA

**SEQ ID NO: 157, *Bigelowiella natans* chloroplastic fructose-1,6-biphosphatase (Bigna_cpFBPase) deduced polypeptide sequence**
MAAVLFRGGVLSSTMTAARTFAAPSVHTRGMHMSVKSSNPFSQAGRRAAVRSSVAPSPVQDSAGTI
VDDGTVTLTRFMIEEAMKSKTPGQEDMVRLISSISVACKRIASMVQTAGISGSTGLAEGGGSVNVQ
GEEQKKLDVISNDVLKSALRPSGKLGVIASEEEDNPVVVDELYSGEYVATFDPLDGSSNIDAAIST

FIGURE 25 (continued)

GTIFGVFKAPEECLIGDSDNLSIAEQQCLEATLQPGTNLVAAGYCMYSSSTILVLTTGDGLNGFTL
DPSIGEFILTHPNIQIPSRGKIYSMNEANYFDWDPKLQTYVDNLKKAEGQTGEKYSSRYIGSMVGD
VHRTLLYGGIFAYPGDKKNVNGKLRLLYEAAPMSLIFEQAGGKSITGPGGRVLDLVPDKVHQRCPV
FIGSPDDVDEVEKALA

**SEQ ID NO: 158, *Aquilegia formosa* x *Aquilegia pubescens* chloroplastic fructose-1,6-biphosphatase (Aqufo_cpFBPase) nucleic acid sequence contig of DR944021, DT749545**

ATGGTTGCAGCAGCTATCCCTACTTCTTGTCAACTGCTCTTCTCCACCTCTTCTTCGACCACTTCT
CGTCTATATCCTCCTTATCTTGATGCCAAAACTCTCTTCTCATTTCCTACAAACAAGAGACATGTA
AGCATTGTTAGAACTCCGGCAGGTGTACGGTGTCAAGCATTAGGAGCAGAGGTAGTAGTGACCAAG
AGGAGTGCATTTGAGATACAAACTCTAACAGGATGGTTATTGAAACAAGAACAAACAGGGGTTATA
GATGCAGAGTTAACTATAGTTTTGTCTAGCATTTCAATGGCCTGTAAGCAGATTGCTTCATTGGTG
CAGAGGGCAAGTATTTCCAACTTAACTGGAGTTCAAGGAGCTGTTAATATTCAAGGTGAAGATCAG
AAGAAGCTTGATGTCATCTCTAACGAGGTGTTCTCTAATTGCCTTAGATCAAGTGGAAGAACAGGG
ATTATAGCATCAGAAGAAGAGGATGTACCAGTTGCAGTGGAGGAAAGCTACTCTGGCAACTATATT
GTCGTTTTTGATCCTCTTGACGGGTCATCAAACATTGATGCCGCTGTGTCAACTGGATCCATATTT
GGAATTTATAGTCCGAACGATGAGTGTCTTACTGAAGTCGATGATAATGCCACAGTGCTTCAGCAA
GTGGAACAGAAGTGCATCATCAATGTGTGTCAACCTGGCAACAACTTGTTGGCAGCTGGCTACTGC
ATGTACTCAAGCTCTGTAATCTTTGTGCTTTCCATTGGACAAGGGGTTTTCTCATTTACCTTAGAC
CCTATGTACGGAGAATTCGTCTTGACACAGGAAAACATTCAAATACCTAAATCAGGTAAAATCTAC
TCATTCAACGAAGGCAACTACCAATTATGGGATGATAAGTTGAAGAAGTATATCGATGACCTTAAG
GACCCTGGTCCTAGTGGCAAACCATACTCTTCCAGATACATTGGAAGCTTGGTTGGCGATTTCCAC
CGGACCCTTCTTTATGGAGGGATATATGGATACCCTAGGGATAAGAATAGAAAAAATGGGAAGCTA
AGGCTACTGTATGAGTGTGCACCTATGAGTTATTTAGTTGAACAAGCAGGAGGAAAAGGATCAGAT
GGACACCAAAGAGTACTCGATATTGAACCGGTGGAGATTCATCAGCGTGTTCCACTTTTCATTGGC
AGCGTTGAAGAAGTTGAGAAACTAGAGAAGTTCTTGGCTTGA

**SEQ ID NO: 159, *Aquilegia formosa* x *Aquilegia pubescens* chloroplastic fructose-1,6-biphosphatase (Aqufo_cpFBPase) deduced polypeptide sequence**

MVAAAIPTSCQLLFSTSSSTTSRLYPPYLDAKTLFSFPTNKRHVSIVRTPAGVRCQALGAEVVVTK
RSAFEIQTLTGWLLKQEQTGVIDAELTIVLSSISMACKQIASLVQRASISNLTGVQGAVNIQGEDQ
KKLDVISNEVFSNCLRSSGRTGIIASEEEDVPVAVEESYSGNYIVVFDPLDGSSNIDAAVSTGSIF
GIYSPNDECLTEVDDNATVLQQVEQKCIINVCQPGNNLLAAGYCMYSSSVIFVLSIGQGVFSFTLD
PMYGEFVLTQENIQIPKSGKIYSFNEGNYQLWDDKLKKYIDDLKDPGPSGKPYSSRYIGSLVGDFH
RTLLYGGIYGYPRDKNRKNGKLRLLYECAPMSYLVEQAGGKGSDGHQRVLDIEPVEIHQRVPLFIG
SVEEVEKLEKFLA

**SEQ ID NO: 160, *Arabidopsis thaliana* chloroplastic fructose-1,6-biphosphatase (Arath_cpFBPase) nucleic acid sequence X58148**

ATGGCAGCATCGGCCGCAACAACGACGTCGTCTCACCTTCTTCTCTCAAGCTCACGTCACGTGGCT
TCATCATCCCAGCCTTCAATCCTTTCACCGAGATCTCTCTTCTCCAACAACGGGAAACGAGCACCA
ACTGGAGTGAGAAACCATCAGTATGCGAGTGGAGTGAGGTGTATGGCCGTAGCGGCGGATGCTTCT
GAGACGAAAACGGCGGCGAGGAAGAAGAGTGGATACGAACTTCAAACGTTGACGGGCTGGTTGCTG
AGACAAGAGATGAAAGGAGAGATAGATGCAGAGCTGACGATAGTGATGTCGAGTATATCATTGGCT
TGTAAGCAGATCGCTTCACTTGTTCAACGTGCCGGAATATCTAACCTGACCGGAGTTCAAGGCGCC
ATTAATATTCAGGGAGAGGATCAGAAGAAGCTTGACGTCATCTCTAATGAGGTGTTTTCCAACTGT

FIGURE 25 (continued)

TTGAGATCAAGTGGAAGAACGGGAATCATAGCCTCGGAGGAAGAGGACGTGCCAGTTGCGGTGGAG
GAGAGTTACTCCGGCAACTACGTCGTCGTGTTTGACCCTCTTGATGGTTCCTCCAACATTGACGCT
GCCGTCTCTACTGGTTCTATCTTCGGTATCTATAGCCCCAATGACGAATGCATTGTCGACGACTCC
GACGATATCTCAGCTCTTGGGTCAGAAGAACAAAGGTGTATAGTAAACGTGTGCCAGCCAGGGAAC
AACTTGTTAGCAGCCGGCTACTGTATGTACTCGAGCTCGGTCATCTTCGTTCTTACTCTAGGCAAA
GGCGTTTTCTCCTTCACGCTTGATCCAATGTACGGTGAGTTTGTCCTCACGCAAGAAAACATTGAG
ATCCCCAAAGCCGGGAGAATCTACTCTTTCAACGAAGGGAATTACCAGATGTGGGACGATAAACTA
AAGAAGTACATTGATGACCTTAAGGACCCTGGTCCAACTGGGAAGCCTTACTCGGCAAGGTACATT
GGAAGTTTGGTTGGAGATTTTCACAGGACTTTGTTGTACGGTGGGATTTACGGGTACCCTCGTGAC
GCAAAGAGCAAAAAATGGAAAGCTTAGGCTTTTGTATGAGTGTGCACCAATGAGTTTCATTGTTGAA
CAAGCTGGAGGGAAAGGTTCTGATGGACATTCGAGAGTACTAGATATCCAACCGACTGAGATACAT
CAGAGGGTTCCTCTATACATTGGAAGCACAGAGGAAGTAGAGAAGTTGGAGAAGTACTTGGCTTGA

**SEQ ID NO: 161, *Arabidopsis thaliana* chloroplastic fructose-1,6-biphosphatase (Arath_cpFBPase) deduced polypeptide sequence**
MAASAATTTSSHLLLSSSRHVASSSQPSILSPRSLFSNNGKRAPTGVRNHQYASGVRCMAVAADAS
ETKTAARKKSGYELQTLTGWLLRQEMKGEIDAELTIVMSSISLACKQIASLVQRAGISNLTGVQGA
INIQGEDQKKLDVISNEVFSNCLRSSGRTGIIASEEEDVPVAVEESYSGNYVVVFDPLDGSSNIDA
AVSTGSIFGIYSPNDECIVDDSDDISALGSEEQRCIVNVCQPGNNLLAAGYCMYSSSVIFVLTLGK
GVFSFTLDPMYGEFVLTQENIEIPKAGRIYSFNEGNYQMWDDKLKKYIDDLKDPGPTGKPYSARYI
GSLVGDFHRTLLYGGIYGYPRDAKSKNGKLRLLYECAPMSFIVEQAGGKGSDGHSRVLDIQPTEIH
QRVPLYIGSTEEVEKLEKYLA

**SEQ ID NO: 162, *Brassica napus* chloroplastic fructose-1,6-biphosphatase (Brana_cpFBPase) nucleic acid sequence AF081796**
ATGGCAGCAACCGCCGCGACAACGTCATCGTCTCATCTTCTTCTCTCAAGCTCTCGCCACGTGGCA
GCCTCATCTCAGCCACAAATCCTTTTCCAGAGATCTCTGTTTTCCGGCGGGAAACGATCGGCAGCT
GGAAAAAACCATCATGCTAGTGGTGGAGTGAGGTGTATGGCGGTTGCAGCGGATGCTTCGGCGGAG
GCTAAGCCGGCGGCAGCGAGGAAGAAGAGTGGGTACGAGCTTCAGACGCTGACGGGTTGGTTGCTG
AGACAAGAGCAGAAAGGAGAGATAGATACAGAGCTGACGATAGTGATGTCGAGCATAGCGATGGCT
TGTAAGCAGATCGCTTCGCTTGTACAGCGCGCCGGAATCTCTAATCTGACCGGCGTTCAAGGAGCC
GTTAACATTCAGGGAGAGGATCAGAAAAAGCTCGACGTCGTCTCTAATGAGGTATTTTCAAACTGT
TTGAGATCAAGTGGAAGGACAGGGATCATTGCGTCAGAGGAAGAGGACGTCCCCGTAGCAGTTGAA
GAGAGTTACTCCGGAAACTACATTGTCGTCTTTGATCCTCTCGACGGTTCCTCCAACATCGACGCT
GCAGTCTCCACTGGTTCAATCTTCGGTATCTACAGCCCCAATGACGAGTGTATCGTTGACGACTCC
GACGATATCTCCTCTCTTGGTTCAGAAGAACAAAGGTGTATAGTAAACGTGTGTCAACCAGGGAAC
AACTTGCTCGCAGCTGGCTACTGTATGTACTCGAGCTCAGTCATCTTCGTTCTCACCTTAGGCAAG
GGCGTTTTCTCCTTCACACTCGACCCAATGTACGGCGAGTTCGTCCTCACTCAAGAGAACATTGAG
ATCCCCAAAGCAGGGAAAATCTACTCTTTCAACGAAGGGAACTACCAGATGTGGGACGAGAAACTG
AAGAAGTACATTGATGATCTTAAGGACCCTGGTCCAAGTGGGAAGCCTTACTCTGCAAGGTACATT
GGTAGTTTGGTCGGAGACTTTCACAGAACTTTGTTGTACGGTGGGATTTACGGGTACCCTCGTGAC
GCCAAGAGCAAAAACGGTAAGCTTAGGCTTTTGTATGAGTGTGCACCGATGAGTTTCATCGTTGAA
CAAGCTGGAGGAAAAGGATCAGATGGCCACCAAAGAGTACTAGATATCCAACCCACCGAGATACAT
CAGAGGGTTCCACTTTACATTGGAAGCAAAGAAGAAGTAGAGAAGCTGGAGAAGTACTTGGCTTGA

**SEQ ID NO: 163, *Brassica napus* chloroplastic fructose-1,6-biphosphatase (Brana_cpFBPase) deduced polypeptide sequence**
MAATAATTSSSHLLLSSSRHVAASSQPQILFQRSLFSGGKRSAAGKNHHASGGVRCMAVAADASAE
AKPAAARKKSGYELQTLTGWLLRQEQKGEIDTELTIVMSSIAMACKQIASLVQRAGISNLTGVQGA

## FIGURE 25 (continued)

VNIQGEDQKKLDVVSNEVFSNCLRSSGRTGIIASEEEDVPVAVEESYSGNYIVVFDPLDGSSNIDA
AVSTGSIFGIYSPNDECIVDDSDDISSLGSEEQRCIVNVCQPGNNLLAAGYCMYSSSVIFVLTLGK
GVFSFTLDPMYGEFVLTQENIEIPKAGKIYSFNEGNYQMWDEKLKKYIDDLKDPGPSGKPYSARYI
GSLVGDFHRTLLYGGIYGYPRDAKSKNGKLRLLYECAPMSFIVEQAGGKGSDGHQRVLDIQPTEIH
QRVPLYIGSKEEVEKLEKYLA

**SEQ ID NO: 164, *Cyanidioschyzon merolae* chloroplastic fructose-1,6-biphosphatase (Cyame_cpFBPase) nucleic acid sequence CMO245C (entry in the Cyanidioschyzon merolae database at the Department of Biological Science, University of Tokyo)**
ATGGCGAAGCAAGCACCGCACGCTTTCGTTAGCCTTGGGGCTCCTAAGCTGCGCTCAACAAACATA
TGGGGAAGGGTATCGGTGTCGGTCTTGCCGCTGGAAACAAGGCATCAACGTCGCTTTGGTGCAGTG
AGACTGCGCGCAGCTTTAGACCTACCCATGACACTCGGACAGTGGGTTCTCCAACAGGAGAGGCAG
CATCCAGAGACCGCTGATTTGGCTCCTTTGATAACAGCCACAGCGACAGCTGGCAAGCAGATTGCC
TCACTGGTCGCCCGAGCGGGCGTGAGCAACTTGACTGGCCTTCAGGGCTCGGTCAACGTGCAGGGC
GAGGAGCAGAAAAAGCTGGACGTGTTGACGAACGAGGTGCTCAAGAACGCGCTACGCTCAACAGGG
AAACTAGGTGTCTTGGCTTCGGAAGAGGAAAACGAGCCAGTGTCGCTCATGGAAGAGGCGTATACA
GGTGACTTTATTGCTGCGTTTGACCCGTTGGACGGATCCTCGAATTTGGATGCAGCGATCGCCACG
GGTACGATTTTTGGTGTAATGCGCAGCGGCGAGCACTGTCTGACGGGACCCGAGGACGCGGATATA
AGTCAGCGACAAATGCAGTGCCTCGTGCAGACGCTGCAACCGGGTGCGAATCTGGTGGCAGCGGGC
TACATTCTCTACTCCTCATCGGTGATTTTCATGCTGTCTATTGGTCACGGAGTGCAGGGTTTTTCT
CTGGACCCAGCGATTGGCGAGTTTGTTTTGACGCACCCAGATGTTCGTGTTCCGGAGCGTGGCAAG
ATCTACTCCTTCAACGAGGCAAACTCGGATAACTGGGACCCTGTTCTTCAAGAATATATTCGATCG
ATGAAGAAAAAGGGCTATTCTTCGCGCTACATAGGATCTCTCGTTGGCGATGTCCATCGCACGCTG
ATTTACGGCGGCGTTTTTGGATACCCTGGTGATAAGAAGAACCCGAACGGCAAGTTGCGTCTTCTG
TACGAGTGCGCACCTATGGCGTATCTCATGGAGCAGGCCGGCGGTATAGCGACCACGGGAAAGCAA
AGAATTCTGGATATTGTTCCGCGAGATGTCCATCAGCGAGAGCCGTTCATTTGCGGAAGCCCCAGG
GATGTTGA

**SEQ ID NO: 165, *Cyanidioschyzon merolae* chloroplastic fructose-1,6-biphosphatase (Cyame_cpFBPase) deduced polypeptide sequence**
MAKQAPHAFVSLGAPKLRSTNIWGRVSVSVLPLETRHQRRFGAVRLRAALDLPMTLGQWVLQQERQ
HPETADLAPLITATATAGKQIASLVARAGVSNLTGLQGSVNVQGEEQKKLDVLTNEVLKNALRSTG
KLGVLASEEENEPVSLMEEAYTGDFIAAFDPLDGSSNLDAAIATGTIFGVMRSGEHCLTGPEDADI
SQRQMQCLVQTLQPGANLVAAGYILYSSSVIFMLSIGHGVQGFSLDPAIGEFVLTHPDVRVPERGK
IYSFNEANSDNWDPVLQEYIRSMKKKGYSSRYIGSLVGDVHRTLIYGGVFGYPGDKKNPNGKLRLL
YECAPMAYLMEQAGGIATTGKQRILDIVPRDVHQREPFICGSPRDVEDLLKIYQGSMAMRG

**SEQ ID NO: 166, *Glycine max* chloroplastic fructose-1,6-biphosphatase (Glyma_cpFBPase) nucleic acid sequence L34841**
ATGGTTGCAATGGCAGCAGCAACAGCATCCACCCAGTTGATTTTCTCAAAGCCTTGTTCCCCTTCA
CGTCTATGCCCCTTCCAACTATGTGTCTTTGACACTAAACAAGTGCTATCAAGTGGCAGGAGAAGG
CATGTGGGGGGTTCTGGAGTTAGGTGCATGGCTGTGGGGGAAGCAGCAACCACTGGGACAAAGAAG
AGAAGTGGATATGAGCTTCAAACACTCACTAGCTGGTTGCTGAAGCAGGAGCAAGCTGGGGTGATT
GATGCAGAACTCACTATTGTGCTGTCTAGCATTTCCATGGCATGCAAACAGATTGCTTCTTTGGTG
CAAAGAGCTAACATTTCCAACCTCACTGGGGTTCAAGGTGCTGTCAATGTTCAAGGGGAAGACCAG
AAAAAGCTTGATGTTGTTTCAAATGAGGTTTTCTCAAACTGCTTGAGGTCAAGTGGGAGGACAGGG
ATAATAGCATCAGAGGAGGAAGATGTGCCAGTGGCAGTAGAAGAGAGTTATTCTGGAAACTACATT

FIGURE 25 (continued)

GTGGTGTTTGACCCACTTGATGGGTCATCCAATATTGATGCTGCAGCGTCAACTGGGTCCAATTTT
TGGATATACAGCCCCAATGATGAGTGTCTTGCTGACATTGATGATGACCCCACCCTTGACACAACA
GAACAAAGATGTATTGTGAACGTGTGCCAACCTGGAAGCAACCTTCTTGCAGCTGGTTACTGCATG
TATTCTAGCTCAATAATCTTTGTTCTCACACTTGGAAATGGAGTGTTTGTGTTTACATTGGACCCG
ATGTATGGCGAATTCGTTTTGACTCAGGAAAACCTCCAGATACCTAGAGCAGGCAAAATCTATGCA
TTCAATGAAGGTAATTATCAGTTGTGGGATGAGAAGCTAAAGAAATATATTGATGATCTCAAGGAC
CCAGGTCAAAGCGGCAAGCCTTATTCTGCAAGGTACATTGGTAGCTTGGTAGGAGATTTCCACAGG
ACACTGCTATATGGTGGCATTTACGGGTACCCCAGGGACAAGAAAAGCAAGAATGGGAAACTAAGG
CTCCTGTATGAATGTGCTCCTATTAACTTCATTGTAGAACAAGCTGGTGGAAAAGGTACAGATGGC
CTTCAAGTACTCCGGCTTCAAGGGACAGAGATTCATCAACGTGTGCCACTGTACATTGGGGAAGAG
GTAGAGAAGGTGGAAAAGTACTTGGCTTAA

**SEQ ID NO: 167, *Glycine max* chloroplastic fructose-1,6-biphosphatase (Glyma_cpFBPase) deduced polypeptide sequence**
MVAMAAATASTQLIFSKPCSPSRLCPFQLCVFDTKQVLSSGRRRHVGGSGVRCMAVGEAATTGTKK
RSGYELQTLTSWLLKQEQAGVIDAELTIVLSSISMACKQIASLVQRANISNLTGVQGAVNVQGEDQ
KKLDVVSNEVFSNCLRSSGRTGIIASEEEDVPVAVEESYSGNYIVVFDPLDGSSNIDAAASTGSNF
WIYSPNDECLADIDDDPTLDTTEQRCIVNVCQPGSNLLAAGYCMYSSSIIFVLTLGNGVFVFTLDP
MYGEFVLTQENLQIPRAGKIYAFNEGNYQLWDEKLKKYIDDLKDPGQSGKPYSARYIGSLVGDFHR
TLLYGGIYGYPRDKKSKNGKLRLLYECAPINFIVEQAGGKGTDGLQVLRLQGTEIHQRVPLYIGEE
VEKVEKYLA

**SEQ ID NO: 168, *Lycopersicon esculentum* chloroplastic fructose-1,6-biphosphatase (Lyces_cpFBPase) nucleic acid sequence BT014319**
ATGGCAGCAACAGCAACAACTTCATATTTAAGTGCTCTAGACAAAAAGACTCCATTTTTATTTGCC
TTGGACAAAAAGACTCCATTTTTATGCCCAAAAAACAGCACAAAGAGAAGGTCATTTAATGGAGGA
GTTAAGTGCATGGCAATAGAGACAGCTTCAGGTGTTACACAAACCAAGAAAAAGAGTGGCTATGAG
TTACAAACTTTAACAAGTTGGCTATTGAGACAAGAACAAGCTGGAGTTATTGATGCTGAACTTACC
ATAGTAATTTCAAGTATTTCAATGGCTTGTAAACAGATTGCTTCTTTGGTCCAAAGAGCTGGAATT
TCTAACCTTACTGGAGTTCAAGGTGCTGTCAATATTCAAGGAGAAGATCAGAAGAAACTTGATGTT
GTCTCTAATGAGGTTTTCTCGAATTGTCTAAGATCAAGTGGAAGGACTGGGATTATAGCATCAGAA
GAAGAGGATGTACCTGTGGCAGTGGAAGAGAGTTACTCAGGCAACTACATTGTGGTGTTTGATCCT
CTTGATGGATCATCAAACATTGATGCTGCTGTATCTACCGGTTCTATCTTTGGAATATACAGCCCG
AATGATGAGTGCCTAGCTGATCTTGGAGATGATTCCACGCTTGACAATATAGAGCAAAAGTGCATC
GTGAATGTATGTCAACCAGGGACAAACCTTCTTGCAGCAGGATACTGCATGTACTCAAGCTCTGTG
ATTTTCGTACTCACCTTGGGAAATGGCGTTTTTTCCTTTAACTTGGATCCAATGTATGGAGAATTT
GTTCTGACTCAAGAAAATGTCCAAATACCAAAGTCTGGAAAGATCTATTCATTCAATGAAGGAAAC
TACCAGCTCTGGGATGACAAGTTGAAAAAATATATCGATGACTTGAAAGACCCTGGTCCTAGTGGC
AAGCCTTACTCTGCAAGGTACATTGGTAGTTTGGTAGGTGACTTCCATAGAACTCTTCTATATGGT
GGCATTTATGGTTATCCTAGAGACAGAAAGAGCAAGAATGGAAAGTTGAGGCTTTTGTACGAATGT
GCTCCCATGAGCTTCATTGTGGAACAAGCTGGTGGCAAAGGATCCGATGGTCACCAAAGAGTTCTC
GATATCCAACCAACTGAGATACATCAACGAGTTCCATTGTACATTGGAAGCACAGAAGAAGTTGAA
AAATTGGAGAAGTACTTGTCTTAA

**SEQ ID NO: 169, *Lycopersicon esculentum* chloroplastic fructose-1,6-biphosphatase (Lyces_cpFBPase) deduced polypeptide sequence**
MAATATTSYLSALDKKTPFLFALDKKTPFLCPKNSTKRRSFNGGVKCMAIETASGVTQTKKKSGYE
LQTLTSWLLRQEQAGVIDAELTIVISSISMACKQIASLVQRAGISNLTGVQGAVNIQGEDQKKLDV

## FIGURE 25 (continued)

VSNEVFSNCLRSSGRTGIIASEEEDVPVAVEESYSGNYIVVFDPLDGSSNIDAAVSTGSIFGIYSP
NDECLADLGDDSTLDNIEQKCIVNVCQPGTNLLAAGYCMYSSSVIFVLTLGNGVFSFNLDPMYGEF
VLTQENVQIPKSGKIYSFNEGNYQLWDDKLKKYIDDLKDPGPSGKPYSARYIGSLVGDFHRTLLYG
GIYGYPRDRKSKNGKLRLLYECAPMSFIVEQAGGKGSDGHQRVLDIQPTEIHQRVPLYIGSTEEVE
KLEKYLS

**SEQ ID NO: 170, *Medicago truncatula* fructose-1,6-biphosphatase (Medtr_cpFBPase) nucleic acid sequence contig of BI310407, BI265103, CO516140**

ATGGTTGCAATGGCAGCAGCAACAGCATCATCACAATTAATATTCTCAAAACCTTGTTCTCCCTCA
CGTCTATGTCCCTTCCAACTTTGTGTTTTCGACACGAAATCAGTGTTATCAAGTTCAAGAAGAAAG
CATGTGAGTGGCTCTGGAGGAGTGAGATGCATGGCTGTTGGTGAAGTAGCTGCTGAGACAAAGAAA
AGAAGTAGTTATGAGCTTATAACATTGACTAGTTGGTTGTTGAAGCAAGAACAAACAGGGGTTATT
GATGCTGAACTTACTATTGTTTTGAATAGTATTTCGTTGGCATGTAAACAGATTGCTTCTTTGGTT
CAAAGAGCTAATATTTCTAACCTTACTGGTGTTCAAGGTGCTGTAAATATTCAAGGGGAGGATCAG
AAAAAACTTGATGTTGTCTCAAATGAGGTATTCTCAAATTGTTTGAGGTCAAGTGGGAGGACAGGG
ATTATAGCATCAGAGGAAGAGGATGTGCCAGTGGCAGTAGAAGAGAGTTATTCAGGAAACTACATT
GTGGTCTTTGATCCACTTGATGGTTCATCGAATATTGATGCTGCAGTTTCAACTGGTTCTATTTTT
GGGATTTACAGCCCCAATGATGAGTGTCTTGCTGACGTAGGCAATGAGTCCGATGACCCCACACTT
GGCACAGAAGAACAACGATGCATTGTGAATGTGTGCCAACCAGGAAGCAACCTTCTAGCAGCCGGT
TACTGCATGTATTCTAGCTCAGTAATCTTCGTTCTAACAATCGGCAAAGGAGTTTTCGTATTCACA
TTAGATCCAATGTATGGGGAATTTGTTTTGACTCAAGAAAATCTCCAAATACCAAAATCAGGGAAA
ATTTATTCTTTCAATGAAGGAAATTACAAGTTATGGGATGACAACTTGAAGAAATACATCGATGAT
CTCAAGGAACCGGGTGCTAATGGCAAACCTTATTCAGCAAGGTATATTGGTAGTTTGGTAGGTGAT
TTCCATAGGACACTGCTATATGGTGGCATTTATGGTTACCCTAGGGACAAGAAAAGTAAGAATGGA
AGGCTTAGGCTTTTATATGAGTGTGCTCCAATGAGTTTCATTGTAGAACAGGCTGGTGGAAAAGGT
TCAGATGGTCATCAAAGAGTACTTGACATTCAACCCACCGAAATTCATCAACGTGTTCCACTGTAC
ATTGGGAGCACAGAGGAAGTGGAGAAGGTTGAAAAGTACTTGGCTTAA

**SEQ ID NO: 171, *Medicago truncatula* chloroplastic fructose-1,6-biphosphatase (Medtr_cpFBPase) deduced polypeptide sequence**

MVAMAAATASSQLIFSKPCSPSRLCPFQLCVFDTKSVLSSSRRKHVSGSGGVRCMAVGEVAAETKK
RSSYELITLTSWLLKQEQTGVIDAELTIVLNSISLACKQIASLVQRANISNLTGVQGAVNIQGEDQ
KKLDVVSNEVFSNCLRSSGRTGIIASEEEDVPVAVEESYSGNYIVVFDPLDGSSNIDAAVSTGSIF
GIYSPNDECLADVGNESDDPTLGTEEQRCIVNVCQPGSNLLAAGYCMYSSSVIFVLTIGKGVFVFT
LDPMYGEFVLTQENLQIPKSGKIYSFNEGNYKLWDDNLKKYIDDLKEPGANGKPYSARYIGSLVGD
FHRTLLYGGIYGYPRDKKSKNGRLRLLYECAPMSFIVEQAGGKGSDGHQRVLDIQPTEIHQRVPLY
IGSTEEVEKVEKYLA

**SEQ ID NO: 172, *Nicotiana tabacum* chloroplastic fructose-1,6-biphosphatase (Nicta_cpFBPase) nucleic acid sequence contig of DW000613 and EB680028**

ATGGCAGCATCATCAGCAACAGCAACAGCAACAACTTCATTTTTATGTGCTTTAGACAAAAAGACT
CCATTTTTATGTACTCTAGACAAAAAAGGTACTCCATTTTTATGCCCAAAAGGCAGCAGCACAACA
AAGAGAAGGTCATTTAATGGAGGAGTGAAGTGCATGGCAATAGAGACAACAGCAGGAGCTACAGAG
ACCAAGAAAAGAAGTGGCTATGAGTTACAAACTTTAACAAGCTGGCTATTAAGGCAAGAACAAGCT
GGGAGTATTGATGCTGAACTTACCATAGTGATTTCAAGTATTTCTATGGCTTGTAAGCAGATTGCT
TCTTTGGTTCAGAGAGCTGGAATTTCTAACCTTACTGGAGTTCAAGGTGCTGTCAATATTCAAGGA

FIGURE 25 (continued)

GAAGACCAGAAGAAGCTTGATGTTGTCTCTAACGAGGTTTTCTCGAATTGTCTAAGGTCGAGTGGA
AGGACTGGGATTATAGCATCAGAGGAAGAGGATGTACCAGTGGCAGTGGAAGAGAGTTACTCAGGA
AACTACATTGTGGTGTTTGACCCTCTTGATGGATCATCAAACATTGATGCTGCTGTTTCTACTGGT
TCTATTTTCGGAATATACAGCCCAAATGATGAGTGCCTCGCAGATCATGGAGATGATTCCACGCTT
GACAATGTTGAACAGAGGTGTATTGTGAATGTATGCCAACCAGGGAGCAACCTTCTTGCAGCAGGC
TACTGCATGTACTCAAGCTCTGTGATTTTTGTGGTCACCTTGGGAAACGGAGTCTTTGCCTTCAAC
TTGGATCCGATGTATGGAGAATTCGTTCTGACCCAAGAAAACATCCAAATACCAAAATCTGGAAAG
ATCTATTCGTTCAACGAAGGAAACTACCAGCTTTGGGATGACAAACTGAAGAAATACATCGATGAC
TTGAAGGACCCTGGTCCTAGCGGCAAGCCTTACTCTGCTAGGTACATTGGTAGTTTGGTTGGTGAC
TTCCATAGAACTCTTCTATATGGTGGCATTTATGGCTATCCTAGAGATAAAAAGAGTAAGAATGGA
AAGTTGAGGCTTTTGTATGAGTGTGCCCCTATGAGCTTCCTCGTGGAACAAGCTGGTGGCAAAGGA
TCCGATGGCCACCAAAGAGTTCTTGATATCCAACCAACTGAGATACACCAGCGAGTCCCATTGTAC
ATTGGAAGCACAGAAGAAGTTGAAAAGTTGGAGAAGTATTTATCTTAA

**SEQ ID NO: 173, *Nicotiana tabacum* chloroplastic fructose-1,6-biphosphatase (Nicta_cpFBPase) deduced polypeptide sequence**

MAASSATATATTSFLCALDKKTPFLCTLDKKGTPFLCPKGSSTTKRRSFNGGVKCMAIETTAGATE
TKKRSGYELQTLTSWLLRQEQAGSIDAELTIVISSISMACKQIASLVQRAGISNLTGVQGAVNIQG
EDQKKLDVVSNEVFSNCLRSSGRTGIIASEEEDVPVAVEESYSGNYIVVFDPLDGSSNIDAAVSTG
SIFGIYSPNDECLADHGDDSTLDNVEQRCIVNVCQPGSNLLAAGYCMYSSSVIFVVTLGNGVFAFN
LDPMYGEFVLTQENIQIPKSGKIYSFNEGNYQLWDDKLKKYIDDLKDPGPSGKPYSARYIGSLVGD
FHRTLLYGGIYGYPRDKKSKNGKLRLLYECAPMSFLVEQAGGKGSDGHQRVLDIQPTEIHQRVPLY
IGSTEEVEKLEKYLS

**SEQ ID NO: 174, *Oryza sativa* chloroplastic fructose-1,6-biphosphatase (Orysa_cpFBPase) nucleic acid sequence AB007194**

ATGGCCGCCGCAGCCACGACATCCTCCCACCTGCTCCTGCTCTCCCGCCAGCAGGCGGCGGCCTCG
CTCCAATGCGGCCTCTCCTTCCGGAGGCAGCCCGGCAGGCTCGCCGGTGGGTCGTCGGCCCCGAGC
GTGCGGTGCATGGCGGCCGTCGACACGGCCTCGGCGCCCGCCGCGACGGAGGCTAGCAAGAAGAGC
AGCTACGAGATCACCACGCTGACGACGTGGCTGCTGAAGCAGGAGCAGGCCGGGACCATCGACGGC
GAGATGACCATCGTGCTGGCCAGCATCTCCACGGCGTGCAAGCAGATCGCCTCGCTGGTGCAGCGC
GCGCCCATCTCCAACCTCACCGGCGTCCAGGGCGCCGTCAACGTGCAGGGCGAGGACCAGAAAAAA
CTCGACGTCGTCTCCAACGAGGTGTTCTCCAACTGCCTCAAATCGAGCGGGCGCACCGGCGTGATC
GCGTCGGAGGAGGAGGACGTGCCGGTGGCCGTGGAGGAGAGCTACTCGGGCAACTACATCGTGGTG
TTCGACCCGCTCGACGGCTCCTCCAACATCGACGCCGCCGTCTCCACCGGCTCCATCTTCGGCATC
TACAGCCCCAACGACGAGTGCCTAGCCGACATCGCCGACGACCAAAATCTTGACCAGGTGGAGCAG
AGGTGCATCGTGAGCGTGTGCCAGCCGGGGAGCAACCTGCTCGCCGCCGGCTACTGCATGTACTCG
AGCTCGGTCATCTTCGTGCTCACCATCGGGACAGGGGTGTACGTGTTCACGCTGGACCCGATGTAC
GGCGAGTTCGTGCTGACGCAGGAGAAGGTGCAGATCCCCAAGGCAGGCAAGATCTATGCCTTCAAC
GAGGGCAACTACGCGCTCTGGGACGACAAGCTCAAGAGCTACATGGACAGCCTCAAGGAGCCCGGG
CCGTCCGGGAAGCCATACTCCGCGCGCTACATCGGCAGCCTCGTCGGCGACTTCCACCGCACGCTG
CTCTACGGCGGCATCTACGGCTACCCCAGGGACCAGAAGAGCAAGAACGGCAAGCTGCGGCTGCTG
TACGAGTGCGCGCCGATGAGCTTCATCGTCGAGCAGGCCGGCGGCAAGGGCTCCGATGGCCACCAG
AGGATACTTGACATCATGCCTACGGAGATCCATCAGAGAGTGCCGCTGTACATCGGGAGCGTGGAG
GAAGTGGAGAAGGTCGAGAAGTTCTTGGCTTGA

**FIGURE 25 (continued)**

**SEQ ID NO: 175, *Oryza sativa* chloroplastic fructose-1,6-biphosphatase (Orysa_cpFBPase) deduced polypeptide sequence**
MAAAATTSSHLLLLSRQQAAASLQCGLSFRRQPGRLAGGSSAPSVRCMAAVDTASAPAATEASKKS
SYEITTLTTWLLKQEQAGTIDGEMTIVLASISTACKQIASLVQRAPISNLTGVQGAVNVQGEDQKK
LDVVSNEVFSNCLKSSGRTGVIASEEEDVPVAVEESYSGNYIVVFDPLDGSSNIDAAVSTGSIFGI
YSPNDECLADIADDQNLDQVEQRCIVSVCQPGSNLLAAGYCMYSSSVIFVLTIGTGVYVFTLDPMY
GEFVLTQEKVQIPKAGKIYAFNEGNYALWDDKLKSYMDSLKEPGPSGKPYSARYIGSLVGDFHRTL
LYGGIYGYPRDQKSKNGKLRLLYECAPMSFIVEQAGGKGSDGHQRILDIMPTEIHQRVPLYIGSVE
EVEKVEKFLA

**SEQ ID NO: 176, *Ostreococcus lucimarinus* chloroplastic fructose-1,6-biphosphatase (Ostlu_cpFBPase) nucleic acid sequence jgi|Ost9901_3|92356|ost_03_002_042 *Ostreococcus lucimarinus* database at the DOE Joint Genome Institute, US Department of Energy**
ATGTCTGCCGCCACCTCCGCCTGCGCTCGCGCGATCGTCGCGACGAAGAAGACCACCGCGGCGCGC
CGCGCGGGCAAGTCGGCGACGCGGGCGACGCCGCGATCCGTCGCGGCGCGCGCGGGGGGCGCGGGC
ACGCCGTTCACGACGTGGATCTTGCAACAAGAGATGGAAGAAAAGATCGACGGCGAACTCGCGGTC
GTGTTGTCGAGCATCGGCTTGGCGTGCAAGCAAATCGCGAGCTTGGTGCAACGCGCCGGGCTTCAA
GGCATGACGGGTTTGGCGGGCGAGACGAACGTGCAAGGTGAAGACCAAAAGAAGCTCGACGTCATC
TCCAACGATGTGTTCTGCGATGTCTTGCGTCAAACCGGCCGCACGGGCGTGATTGCGTCCGAGGAA
GAAGACGTGCCGGTCGCCGTCGAAGAAACCTTCGGCGGTAACTACGTCGTCGTCTTCGATCCGCTC
GATGGCTCTTCCAACATCGACGCCGCCGTTTCCACGGGTTCCATCTGGGGCATCTACGAGTCCGAC
TCCACGTGCATCCCGGATTTTGGCACCGAAGATGCGGCCAAGATTGAAGAGAAGTGCGTCATGAAC
GTGTGCCAACCGGGGAACAACTTGTTGTGCGCGGGCTACTGCATGTACTCCTCTTCCACCATCCTC
GTCTTGACCCTCGGTGAGGGTGTGTACGGTTTCACCCTCGACCCGACCGTCGGCGAGTTCATCATG
TCCCACGACAACATCAAGGTTCCGGAATCTGGTAAGATTTACTCCTTCAACGAAGGCAACTACGAC
ATGTGGACTCCGGGCTTGAAGAAGTACATGGACTCCCTCAAGACTGGCGGCGCGGAACAAGGCACC
AAGCCGTACAGCGCCCGTTACATCGGTTCCCTCGTCGGTGACTTCCACAGAACCATCTTGTACGGA
GGCATCTACGGCTACCCGGGCGACTCCAAGAACCCGAACGGTAAGCTTCGCCTCTTGTACGAGTGC
GCGCCGATGTCCTTCATCGCCGAACAAGCCGGCGGTATGGGTTCCACCGGTAAGGAGCGCGTCCTT
GACGTTGTCCCGGAAAAGTTTCACCAACGTGTGCCGTTCTTCACCGGCTCCAAGAAGGAAGTGCAG
TACTTGGAATCCTTCATGTAG

**SEQ ID NO: 177, *Ostreococcus lucimarinus* chloroplastic fructose-1,6-biphosphatase (Ostlu_cpFBPase) deduced polypeptide sequence**
MSAATSACARAIVATKKTTAARRAGKSATRATPRSVAARAGGAGTPFTTWILQQEMEEKIDGELAV
VLSSIGLACKQIASLVQRAGLQGMTGLAGETNVQGEDQKKLDVISNDVFCDVLRQTGRTGVIASEE
EDVPVAVEETFGGNYVVVFDPLDGSSNIDAAVSTGSIWGIYESDSTCIPDFGTEDAAKIEEKCVMN
VCQPGNNLLCAGYCMYSSSTILVLTLGEGVYGFTLDPTVGEFIMSHDNIKVPESGKIYSFNEGNYD
MWTPGLKKYMDSLKTGGAEQGTKPYSARYIGSLVGDFHRTILYGGIYGYPGDSKNPNGKLRLLYEC
APMSFIAEQAGGMGSTGKERVLDVVPEKFHQRVPFFTGSKKEVQYLESFM

**SEQ ID NO: 178, *Ostreococcus tauri* chloroplastic fructose-1,6-biphosphatase (Ostta_cpFBPase) nucleic acid sequence CR954203**
ATGTCTGCGTGTATTTCTGCCTCTGTGACGCGCGCGCGAACGGCGCGCGCGCCCGCGGCTCGCCGC
GCGGCGAGTAAGGCGCGGGCGTCGCCGCGCGCCGTCGCGGCGCGCGCGGGGGGCGTTGGTACGCCG
TACACGACGTGGATTCTCCAGCAAGAGATGCAAGAGAACATTGATGGTGAATTGGCGGTGGTTTTG

**FIGURE 25 (continued)**

TCCTCCATCGGTCTCGCGTGCAAGCAAATCGCGAGCCTCGTCCAGCGCGCGGGTCTCGCGGGCATG
ACTGGTTTGGCGGGCGAGCAAAACGTGCAAGGCGAGGACCAGAAGAAGCTCGACGTCATCTCCAAC
GATGTTTTCTGCAACGTATTGCGCCAATCCGGCCGCACGGGCGTCATCGCCTCCGAAGAAGAAGAC
GTTCCGGTCGCCGTCGAGGAAACGTACGGCGGTAACTACGTCGTCGTCTTCGATCCGCTTGATGGT
TCCTCCAATATCGACGCCGCCGTCTCCACGGGATCCATCTGGGGTATCTACGAGTCCGACTCCTCG
TGTATTCCCGACTTCGGCTCCGATGACTCCGCCAAGGTTGAAGAGAAGTGCGTCATGAACGTTTGC
CAACCGGGCAGCAACTTGCTCTGCGCGGGTTACTGCATGTACTCCTCGTCCACCATCCTCGTCATC
ACCATCGGCCAAGGCGTGTTCGGTTTTACCCTCGACCCGACCGTCGGTGAGTTCATCATGTCCCAC
GAGAACATCAAGGTTCCGGACTCTGGCAAGATTTACTCTTTCAACGAAGGCAACTACGCCATGTGG
TCCGACGGTTTGAAGAAGTACATGGACTCCCTCAAGACGGGCGGCAAGGACGGTGGCAAGCCGTAC
AGCGCCCGCTACATCGGTTCGCTCGTCGGTGACTTCCACCGCACGATCCTTTACGGAGGCATCTAC
GGTTACCCGGGTGACGCGAAGAACCCGAACGGTAAGCTCCGCCTCCTGTACGAGTGCGCGCCGATG
TCCATGATCGCTGAACAAGCCGGTGGTAAGGGCTCCACCGGTGTCGCGCGTGTCCTGGACATCGTT
CCGGAAAAGGTTCACCAGCGCGTGCCGTTCTTCGTTGGCTCCAAGAACGAGGTTGCCTACTTGGAG
TCCTTCATGTGA

**SEQ ID NO: 179, *Ostreococcus tauri* chloroplastic fructose-1,6-biphosphatase (Ostta_cpFBPase) deduced polypeptide sequence**

MSACISASVTRARTARAPAARRAASKARASPRAVAARAGGVGTPYTTWILQQEMQENIDGELAVVL
SSIGLACKQIASLVQRAGLAGMTGLAGEQNVQGEDQKKLDVISNDVFCNVLRQSGRTGVIASEEED
VPVAVEETYGGNYVVVFDPLDGSSNIDAAVSTGSIWGIYESDSSCIPDFGSDDSAKVEEKCVMNVC
QPGSNLLCAGYCMYSSSTILVITIGQGVFGFTLDPTVGEFIMSHENIKVPDSGKIYSFNEGNYAMW
SDGLKKYMDSLKTGGKDGGKPYSARYIGSLVGDFHRTILYGGIYGYPGDAKNPNGKLRLLYECAPM
SMIAEQAGGKGSTGVARVLDIVPEKVHQRVPFFVGSKNEVAYLESFM

**SEQ ID NO: 180, *Phaeodactylum tricornutum* chloroplastic fructose-1,6-biphosphatase (Phatr_cpFBPase) nucleic acid sequence scaffold_27: 145102-145476 *Phaeodactylum tricornutum* databases at the DOE Joint Genome Institute, US Department of Energy**

ATGTTTATTTTGAAGTCCCCGGCGCTTTGGTTGCTTCTTTACCCAGTTGTTGCTTTTACGGCGGCG
AGGGCGAACTCGATTCGTCCAGCGGCCGCGTTATCTGTCTTCGATCTGTCGAGCGTGGAGGCAGTA
CCGTCTAGAAAGACCAAGGCCCCAATTTTCGATGAAGTTTGCGACACAACTGGAGTCACTCTCAAA
CGCTTCATGACCGAGGTTTCTCTACTGAATCCTGAAATCGAAGAGCTGACGACGCTGTTTGGTGCG
ATTGAAACCGCTTGCAAAGCCATTGCAAATCTTGTCAAGCGATCGCCGCTTCCCTCCAGTGACACG
TTGGGTCTTCAGGGAGAAATCAACGTTCAAGGCGAAGACCAAAAGAAATTAGATGTGATCGCCAAC
GATATTTTGAAGCGAGCACTCCGCTTCACGGGCCGCCTCGGAGTCTTAGCCTCGGAAGAAGAGGAT
ACTCCCGTCGATTTGATGCCAAGGGATCCTAGTACCAAAAAAGTTCTAATCGATGAGGGAGAAAAG
TATGTCGCTGTCTTCGATCCGCTCGATGGTAGCTCAAACGTTGATGCAGGCATACCGACAGGCACA
ATAATTGGGATATACGAGCACGACGAAACTTGCAAGATTGATCCTGATGCTTTGGAAGAGGATCGG
ACCAAACAAGAAAACCTATGCCTCGCAAATACTCTGCAGCCCGGCACCAACTTGGTAGCAGCGGCG
TACTGTTTATATTCTTCGTCAACATTTTTGGTGTTGACGCTGGGAGCTGGAACATATGGATTCACG
CTAGATGAGACTATCGGTGAATTTGTCTTGAGCCATCCAAACATTAAGATTCCTGAATGCTCATCC
ATTATGTCGTTCAACGAGGCAAATACTCCCAGCTGGGATCGTCCGCTTCAAGACACTTTCGCAAAG
TGGAGGACAGGGACAGGAAAGAGCGGCAAGAAATTTTCAAGTCGCTACATTGGTTCTATGGTAGGG
GATGTCCATCGGACGCTCCTGTACGGAGGAGTTTTTGGGTATCCTGGCGACAAAAAGAATCCCAAC
GGGAAGCTACGCCTGCTTTATGAAGGAGCTCCAATGTCATTCATCATGGAACAGGCGGGTGGATTG
TCGACCACTGGCACGCAGCGTGTCATGGAAATCTCCCCAGATACGGTCCATCAACGTGTGCCGATT
ATCATGGGATCCAGACAAGATGTCGAAGAGGTCATGGACGCCTATAAAAACTTTGGCATCGAATAA

FIGURE 25 (continued)

**SEQ ID NO: 181, *Phaeodactylum tricornutum* chloroplastic fructose-1,6-biphosphatase (Phatr_cpFBPase) deduced polypeptide sequence scaffold_27: 145102-145476 *Phaeodactylum tricornutum* databases at the DOE Joint Genome Institute, US Department of Energy**
MFILKSPALWLLLYPVVAFTAARANSIRPAAALSVFDLSSVEAVPSRKTKAPIFDEVCDTTGVTLK
RFMTEVSLLNPEIEELTTLFGAIETACKAIANLVKRSPLPSSDTLGLQGEINVQGEDQKKLDVIAN
DILKRALRFTGRLGVLASEEEDTPVDLMPRDPSTKKVLIDEGEKYVAVFDPLDGSSNVDAGIPTGT
IIGIYEHDETCKIDPDALEEDRTKQENLCLANTLQPGTNLVAAAYCLYSSSTFLVLTLGAGTYGFT
LDETIGEFVLSHPNIKIPECSSIMSFNEANTPSWDRPLQDTFAKWRTGTGKSGKKFSSRYIGSMVG
DVHRTLLYGGVFGYPGDKKNPNGKLRLLYEGAPMSFIMEQAGGLSTTGTQRVMEISPDTVHQRVPI
IMGSRQDVEEVMDAYKNFGIE

**SEQ ID NO: 182, *Pisum sativa* chloroplastic fructose-1,6-biphosphatase (Pissa_cpFBPase) nucleic acid sequence L34806**
ATGGTTGCAATGGCAGCAGCAACAGCCTCATCTCAGTTAATATTCTCAAAACCTTACTCTCCTTCA
CGTCTTTGCCCCTTCCAACTCTGTGTCTTTGATGCAAAATCAGTGTTATCAAGTTCAAGGAGAAAG
CATGTGAATGGCTCTGGTGTTAGATGTATGGCTGTGAAGGAAGCAACTAGTGAGACAAAGAAAAGA
AGTGGATATGAGATTATAACACTGACTAGTTGGTTGTTGCAGCAAGAACAAAAAGGGATTATTGAT
GCAGAACTTACTATTGTACTTTCTAGTATTTCTATGGCATGTAAACAAATTGCTTCTTTGGTTCAA
AGAGCCAATATTTCTAACCTCACTGGTACTCAAGGTGCTGTAAATATTCAAGGGGAAGACCAGAAA
AAACTTGATGTTATCTCAAATGAGGTATTCTCAAATTGCTTGAGGTCAAGTGGGAGGACAGGGATA
ATTGCGTCGGAGGAAGAGGATGTCGCGGTGGCAGTAGAAGAGAGTTATTCAGGAAACTACATTGTT
GTATTTGATCCACTTGATGGTTCATCCAATCTTGATGCTGCAGTCTCAACCGGTTCCATTTTCGGG
ATTTACAGCCCCAATGACGAGTGTCTTCCTGATTTTGGTGATGACTCTGATGACAACACACTTGGC
ACAGAAGAACAAAGGTGCATTGTGAATGTGTGTCAACCAGGAAGCAACCTTCTAGCAGCTGGCTAC
TGCATGTATTCTAGTTCAGTAGCTTTTGTTCTTACCATAGGCAAAGGAGTGTTTGTATTCACATTA
GATCCATTGTACGGAGAATTCGTTTTGACTCAAGAGAATCTCCAAATACCGAAATCAGGGGAAATC
TATTCTTTCAATGAAGGGAATTACAAGTTGTGGGATGAAAACTTGAAGAAATATATTGATGATCTT
AAGGAACCAGGTCCTAGTGGCAAGCCTTATTCAGCAAGGTATATTGGTAGTTTGGTTGGTGATTTT
CACAGGACACTGTTATATGGTGGCATTTATGGATACCCTAGGGACAAGAAAAGTAAGAATGGGAAG
CTTAGGCTTTTATATGAATGTGCTCCAATGAGCTTCATTGTTGAACAGGCTGGTGGAAAAGGTTCA
GATGGTCATCAAAGAGTACTTGACATTCAACCCACAGAAATTCATCAACGTGTTCCACTTTACATT
GGGAGCACAGAAGAGGTGGAGAAGGTTGAAAAGTACTTAGCTTAA

**SEQ ID NO: 183, *Pisum sativa* chloroplastic fructose-1,6-biphosphatase (Pissa_cpFBPase) deduced polypeptide sequence**
MVAMAAATASSQLIFSKPYSPSRLCPFQLCVFDAKSVLSSSRRKHVNGSGVRCMAVKEATSETKKR
SGYEIITLTSWLLQQEQKGIIDAELTIVLSSISMACKQIASLVQRANISNLTGTQGAVNIQGEDQK
KLDVISNEVFSNCLRSSGRTGIIASEEEDVAVAVEESYSGNYIVVFDPLDGSSNLDAAVSTGSIFG
IYSPNDECLPDFGDDSDDNTLGTEEQRCIVNVCQPGSNLLAAGYCMYSSSVAFVLTIGKGVFVFTL
DPLYGEFVLTQENLQIPKSGEIYSFNEGNYKLWDENLKKYIDDLKEPGPSGKPYSARYIGSLVGDF
HRTLLYGGIYGYPRDKKSKNGKLRLLYECAPMSFIVEQAGGKGSDGHQRVLDIQPTEIHQRVPLYI
GSTEEVEKVEKYLA

**SEQ ID NO: 184, *Poncirus trifoliata* chloroplastic fructose-1,6-biphosphatase (Pontr_cpFBPase) nucleic acid sequence contig of CV705787 and CV705786**
ATGGTTGCAGCAGCAGCGACAACATCATCACAGCTTCTCTTTTCAAGCTCTCACTCTTTCTCTCGG
CTCTCTCCTTACCAAATATGTGTCTTCGATTCCAAAGCACTCGTGTCGTCATGTCCCAGCAACGTT
ATGAAGAGAAGACATGTTGGTGTTGCTGCTGGGGTTCGGTGCATGGCTGTTGGGACAACATCGGAG

FIGURE 25 (continued)

GTTGCAACCAAGAAGAGAAGTTCGTATGAGATTGAAACGCTGACAAACTGGCTGTTGAAGCAAGAA
CAGTCTGGCGTTATTGATGCTGAGCTCACTATTGTGCTTTCCAGCATTTCAACGGCGTGCAAGCAG
ATTGCTTCTTTGGTGCAAAGAGCTGGCATTTCCAACTTGACTGGAATTCAGGGTGCTGTCAATGTT
CAAGGCGAGGACCAGAAGAAGCTCGACGTCGTTTCAAATGAGGTGTTCTCAAACTGTTTGAGATCA
AGTGGGCGAACTGGGATTATAGCATCAGAGGAAGAGGATGTACCAGTGGCGGTAGAAGAGAGCTAC
TCTGGAAACTATATTGTAGTTTTTGATCCACTTGATGGATCATCCAACATTGATGCTGCAGTGTCT
ACTGGATCCATCTTTGGCATATACAGCCCAAATGATGAGTGTCTTGCCGATATTGGTGATGATTCT
ACTCTGGGCAATACTGAACAAAGATGTGTAGTGAACGTGTGCCAGCCAGGAAGCAACCTTCTTGCT
GCAGGCTATTGCATGTATTCAAGCTCTGTAATCTTTGTGATAACTTTAGGCAACGGAGTCTTTGCA
TTCACCCTGGATCCCATGTATGGAGAATTTGTTTTGACACAAGAGAACATTCAGATACCCAAAACC
GGAAAGATCTATGCTTTTAATGAAGGCAACTACCAGCTTTGGGATGACAAGTTGAAGAAGTACATT
GACGATCTTAAGGATCCAGGACCCAGCGGCAAGCCCTATTCTGCTAGGTACATTGGCAGCTTGGTT
GGTGATTTCCATCGAACTCTGCTCTACGGCGGCATTTATGGCTATCCAAGAGACAAGAAGAGCAAG
AATGGAAAGCTGAGGCTCTTGTATGAATGTGCACCAATGAGCTTCATAGTGGAACAAGCAGGAGGC
AAAGGATCTGACGGCCATCAAAGAGTACTTGACATTCAACCTACTGAGATTCACCAGCGTATTCCT
TTAAAGATTGGAAGCCAGGAGGAAGTGGAGAAACTGGAGAAGTATTTGGCCTAA

**SEQ ID NO: 185, *Poncirus trifoliata* chloroplastic fructose-1,6-biphosphatase (Pontr_cpFBPase) deduced polypeptide sequence**

MVAAAATTSSQLLFSSSHSFSRLSPYQICVFDSKALVSSCPSNVMKRRHVGVAAGVRCMAVGTTSE
VATKKRSSYEIETLTNWLLKQEQSGVIDAELTIVLSSISTACKQIASLVQRAGISNLTGIQGAVNV
QGEDQKKLDVVSNEVFSNCLRSSGRTGIIASEEEDVPVAVEESYSGNYIVVFDPLDGSSNIDAAVS
TGSIFGIYSPNDECLADIGDDSTLGNTEQRCVVNVCQPGSNLLAAGYCMYSSSVIFVITLGNGVFA
FTLDPMYGEFVLTQENIQIPKTGKIYAFNEGNYQLWDDKLKKYIDDLKDPGPSGKPYSARYIGSLV
GDFHRTLLYGGIYGYPRDKKSKNGKLRLLYECAPMSFIVEQAGGKGSDGHQRVLDIQPTEIHQRIP
LKIGSQEEVEKLEKYLA

**SEQ ID NO: 186, *Populus tremuloides* chloroplastic fructose-1,6-biphosphatase (Poptr_cpFBPase) nucleic acid sequence contig of CV241715 and DT474034**

ATGGTTGCACAAGCAGCAGCAATAACAACTTCATCATCACATCTTCTCTTCTCAACCTCACGCTCA
CTGTCTCGCCCATCTCCTTCCCAGTTATGTGTCTTTGACTCAAAAACACTTGTGTCATACCCCAAC
AGCACTAGTACTTACAAGAAAAAACGTGGTGGTGATGGGCTCAAGTGCATGGCTGTGAGCACAGCC
TCGGATGCTAAAACAAAGAAGAGTACGTTTGAGATTCAAACACTGACTGGTTGGCTGTTGAAGCAA
GAACAAGCTGGTGTTATTGATGCTGAGCTCACTATTGTTATATCAAGCATTTCAATGGCATGTAAG
CAGATTGCTTCTTTGGTGCAAAGAGCTAGCATTTCTAACTTAACTGGAGTTCAAGGTTCTGTTAAC
GTTCAAGGAGAAGATCAGAAGAAGCTTGACGTGGTCTCTAATGAGGTGTTCTCTAGCTGCTTGAGA
TCAAGTGGGAGGACAGGAATCATAGCATCAGAGGAAGAGGACGTGCCAGTGGCAGTGGAGGAGAGT
TACTCTGGAAACTATATAGTGGTTTTTGACCCACTCGATGGATCATCCAACATTGATGCTGCAGTG
TCTACTGGTTCCATCTTTGGAATATACAGCCCCAATGACGAATGCCTGGCCGATATTGGAGATGAC
TCCACTCTTGATCAAACGGAACAGAGGTGTATTGTGAATGTGTGCCAGCCAGGAAATAACCTCCTT
GTTGCTGGCTACTGCATGTATTCAAGCTCAGTGATTTTTGTGCTAACTATTGGAAAAGGCGTGTTC
TCTTTCAGCTTGGATCCAATGTATGGAGAGTTTGTTTTAACTCAAGAAAACATCCAGATACCAAAG
GCTGGAAAGATTTATTCATTTAATGAAGGAAACTACCAGTTGTGGGATGACAAGCTGAAGAAGTAC
ATTGATGACCTTAAAGACCCTGGTCCGAGTGGCAAGCCCTACTCCGCTAGATACATTGGAAGCTTG
GTCGGTGACTTCCACCGGACGCTGCTGTACGGTGGCATTTATGGGTACCCCAGGGACAAGAAGAGC
AAGAATGGGAAGCTGAGGCTTCTGTATGAGTGTGCACCGATGAGCTTTATAGTGGAACAAGCTGGT
GGGAAAGGATCAGACGGGCATCAGAGAGTACTGGATATCACTCCTACTGAGATACACCAGCGTGTT
CCGCTTTACATAGGGAGCGTGGAGGAAGTGGAGAAATTGGAGAAGTATTTGGCTTGA

**FIGURE 25 (continued)**

**SEQ ID NO: 187, Populus tremuloides chloroplastic fructose-1,6-biphosphatase (Poptr_cpFBPase) deduced polypeptide sequence**
MVAQAAAITTSSSHLLFSTSRSLSRPSPSQLCVFDSKTLVSYPNSTSTYKKKRGGDGLKCMAVSTA
SDAKTKKSTFEIQTLTGWLLKQEQAGVIDAELTIVISSISMACKQIASLVQRASISNLTGVQGSVN
VQGEDQKKLDVVSNEVFSSCLRSSGRTGIIASEEEDVPVAVEESYSGNYIVVFDPLDGSSNIDAAV
STGSIFGIYSPNDECLADIGDDSTLDQTEQRCIVNVCQPGNNLLVAGYCMYSSSVIFVLTIGKGVF
SFSLDPMYGEFVLTQENIQIPKAGKIYSFNEGNYQLWDDKLKKYIDDLKDPGPSGKPYSARYIGSL
VGDFHRTLLYGGIYGYPRDKKSKNGKLRLLYECAPMSFIVEQAGGKGSDGHQRVLDITPTEIHQRV
PLYIGSVEEVEKLEKYLA

**SEQ ID NO: 188, Solanum tuberosum chloroplastic fructose-1,6-biphosphatase (Soltu_cpFBPase) nucleic acid sequence AF1340451**
ATGGCAGCATCAGCAGCCACAGCAACAGCAACAACTTCATATTTAAGTGCTCTAGACAAAAAGACT
CCATTTTTATTTGCCTTAGACAAAAAGACTCCATTTTTATGCCCAAAAAGCAGCACGAAGAGAAGG
TCATTTAATGGAGGAGTAAAGTGCATGGCAATAGAGACAACTTCAGGTTTTACAGCAACCAAGAAA
AGGAGTGGCTATGAGCTGCAAACTTTAACAAGTTGGCTATTAAGACAAGAACAAGCTGGAGTGATT
GATGCTGAACTTACCATAGTTATTTCAAGTATTTCAATGGCTTGTAAACAGATTGCTTCCTTAGTC
CAAAGAGCTGGAATTTCTAACCTTACTGGAGTTCAAGGTGCTGTCAATATTCAAGGAGAAGATCAG
AAGAAACTTGATGTTGTCTCTAATGAGGTTTTCTCAAATTGTCTAAGATCAAGTGGAAGGACTGGG
ATTATAGCATCAGAAGAAGAGGATGTACCTGTGGCAGTGGAAGAGAGTTACTCAGGCAACTACATT
GTGGTGTTTGATCCTCTTGATGGATCATCAAACATTGATGCTGCTGTGTCTACCGGTTCTATCTTT
GGAATATACAACCCGAATGATGAGTGCCTCGCTGATCATGGAGATGATTCCACGCTTGACAATATA
GAGCAGAAGTGCATTGTGAATGTATGTCAACCAGGGACAAACCTTCTTGCAGCAGGATACTGCATG
TACTCAAGCTCTGTGATATTCGTACTCACCTTGGGAAATGGCGTTTTTTCCTTTAACTTGGATCCG
ATGTACGGAGAATTTGTTCTGACTCAAGAAAATGTCCAAATACCAAAGTCTGGAAAGATCTATTCA
TTCAATGAAGGAAACTACCAGCTCTGGGATGACAAGTTGAAGAAATATATCGATGACTTGAAGGAC
CCTGGCCCTAGTGGCAAGCCTTACTCTGCAAGGTACATTGGTAGTTTGGTTGGTGACTTCCATAGA
ACTCTTCTATATGGTGGCATTTATGGTTATCCTAGAGACCAAAAGAGCAAGAATGGAAAGTTGAGG
CTTTTGTACGAGTGTGCTCCCATGAGCTTCATTGTGGAACAAGCTGGTGGTAAAGGATCCGATGGT
CACCAAAGAGTTCTCGATATCCAACCAACTGAGGTACATCAACGAGTTCCATTGTACATTGGAAGC
ACAGAAGAAGTTGAAAAATTGGAGAAGTACTTGTCTTAA

**SEQ ID NO: 189, Solanum tuberosum chloroplastic fructose-1,6-biphosphatase (Soltu_cpFBPase) deduced polypeptide sequence**
MAASAATATATTSYLSALDKKTPFLFALDKKTPFLCPKSSTKRRSFNGGVKCMAIETTSGFTATKK
RSGYELQTLTSWLLRQEQAGVIDAELTIVISSISMACKQIASLVQRAGISNLTGVQGAVNIQGEDQ
KKLDVVSNEVFSNCLRSSGRTGIIASEEEDVPVAVEESYSGNYIVVFDPLDGSSNIDAAVSTGSIF
GIYNPNDECLADHGDDSTLDNIEQKCIVNVCQPGTNLLAAGYCMYSSSVIFVLTLGNGVFSFNLDP
MYGEFVLTQENVQIPKSGKIYSFNEGNYQLWDDKLKKYIDDLKDPGPSGKPYSARYIGSLVGDFHR
TLLYGGIYGYPRDQKSKNGKLRLLYECAPMSFIVEQAGGKGSDGHQRVLDIQPTEVHQRVPLYIGS
TEEVEKLEKYLS

**SEQ ID NO: 190, Spinacia oleracea chloroplastic fructose-1,6-biphosphatase (Spiol_cpFBPase) nucleic acid sequence L76555**
ATGGCATCAATAGGACCAGCAACAACAACTGCAGTAAAACTGCGTAGCTCAATCTTCAATCCTCAG
TCATCTACTCTTTCCCCGTCTCAACAATGCATTACATTTACAAAGTCCCTTCACTCGTTTCCTACT
GCCACCCGACATAATGTGGCTTCCGGGGTTCGTTGTATGGCAGCCGTAGGAGAGGCGGCTACAGAA
ACAAAGGCAAGGACTAGAAGTAAGTACGAAATTGAAACACTAACAGGCTGGCTGCTTAAACAAGAA

## FIGURE 25 (continued)

ATGGCAGGTGTTATTGATGCTGAACTTACCATCGTTCTTTCTAGCATTTCATTGGCTTGTAAACAA
ATTGCTTCCTTGGTTCAACGAGCTGGTATTTCTAACTTGACTGGAATTCAAGGTGCTGTCAATATC
CAAGGAGAGGATCAGAAGAAACTTGATGTTGTCTCCAATGAGGTGTTTTCGAGCTGCTTGAGATCG
AGTGGAAGAACAGGAATAATAGCATCAGAAGAAGAGGATGTACCAGTGGCAGTGGAAGAGAGTTAC
TCTGGAAACTATATTGTTGTGTTTGATCCACTTGATGGTTCATCCAACATTGATGCAGCTGTCTCC
ACTGGTTCCATCTTTGGCATTTATAGCCCTAACGATGAGTGCATTGTTGACTCTGATCACGACGAT
GAGTCACAGCTAAGTGCAGAAGAACAGAGGTGTGTAGTGAATGTATGTCAACCAGGGGATAACCTA
TTAGCAGCAGGGTATTGTATGTACTCAAGCTCTGTTATCTTCGTACTTACAATTGGTAAAGGTGTG
TATGCATTCACATTAGATCCAATGTATGGTGAATTCGTACTCACTTCAGAGAAAATCCAAATCCCA
AAAGCTGGGAAGATCTATTCATTCAATGAAGGTAACTACAAAATGTGGGATGATAAATTGAAGAAG
TACATGGATGATCTTAAAGAGCCAGGAGAGTCACAGAAACCGTACTCGTCTCGTTACATAGGGAGT
TTAGTTGGGGACTTTCATAGAACACTTTTATATGGTGGGATTTATGGTTACCCAAGAGATGCAAAG
AGTAAGAATGGGAAATTGAGGCTTTTGTATGAATGTGCACCTATGAGTTTTATTGTTGAACAAGCT
GGTGGTAAAGGTTCTGATGGTCATCAAAGAATTCTTGACATTCAACCCACCGAGATACATCAACGT
GTGCCACTGTACATCGGGAGTGTGGAGGAAGTAGAGAAATTAGAGAAGTACTTAGCATAA

**SEQ ID NO: 191, *Spinacia oleracea* chloroplastic fructose-1,6-biphosphatase (Spiol_cpFBPase) deduced polypeptide sequence**
MASIGPATTTAVKLRSSIFNPQSSTLSPSQQCITFTKSLHSFPTATRHNVASGVRCMAAVGEAATE
TKARTRSKYEIETLTGWLLKQEMAGVIDAELTIVLSSISLACKQIASLVQRAGISNLTGIQGAVNI
QGEDQKKLDVVSNEVFSSCLRSSGRTGIIASEEEDVPVAVEESYSGNYIVVFDPLDGSSNIDAAVS
TGSIFGIYSPNDECIVDSDHDDESQLSAEEQRCVVNVCQPGDNLLAAGYCMYSSSVIFVLTIGKGV
YAFTLDPMYGEFVLTSEKIQIPKAGKIYSFNEGNYKMWDDKLKKYMDDLKEPGESQKPYSSRYIGS
LVGDFHRTLLYGGIYGYPRDAKSKNGKLRLLYECAPMSFIVEQAGGKGSDGHQRILDIQPTEIHQR
VPLYIGSVEEVEKLEKYLA

**SEQ ID NO: 192, *Triticum aestivum* chloroplastic fructose-1,6-biphosphatase (Triael_cpFBPase) nucleic acid sequence X07780**
ATGGCCGCCGCGACCACCACCACCTCCCGCCCGCTTCTGCTGTCCCGCCAGCAGGCGGCGGCTAGC
TCCCTCCAATGCCGCCTCCCCAGGAGGCCCGGAAGCAGCCTCTTTGCCGGCCAGGGCCAGGCGTCG
ACTCCGAATGTGCGGTGCATGGCAGTCGTGGACACGGCCTCGGCGCCGGCGCCGGCGGCGGCTAGG
AAGAGGAGCAGCTACGACATGATCACGCTGACGACGTGGCTGCTGAAGCAGGAGCAGGAGGGGGTC
ATCGACAACGAGATGACCATCGTGCTGTCCAGCATATCCACGGCGTGCAAGCAGATCGCCTCGTTG
GTGCAGCGCGCGCCCATCTCCAACCTCACCGGCGTCCAGGGCGCCACCAACGTGCAGGGCGAGGAC
CAGAAGAAGCTCGACGTCATCTCCAACGAGGTGTTCTCGAACTGCCTGAGGTGGAGTGGCCGCACC
GGCGTGATCGCATCGGAGGAGGAGGACGTGCCGGTGGCGGTGGAGGAGAGCTACTCGGGCAACTAC
ATCGTGGTGTTCGACCCGCTCGACGGCTCCTCCAACATCGACGCCGCCGTCTCCACCGGCTCCATC
TTCGGCATCTACAGCCCATCCGACGAGTGCCACATTGGCGACGACGCAACCCTTGACGAAGTGACG
CAGATGTGCATAGTGAACGTGTGCCAGCCAGGGAGCAACCTGCTCGCCGCCGGCTACTGCATGTAC
TCGAGCTCGGTCATCTTCGTGCTCACCATCGGCACCGGGGTGTACGTGTTCACGCTGGACCCGATG
TACGGCGAGTTCGTGCTGACGCAGGAGAAGGTGCAGATCCCAAAGTCGGGCAAGATCTACTCCTTC
AACGAGGGCAACTACGCGCTCTGGGACGACAAGCTCAAGAAGTACATGGACAGCCTCAAGGAGCCC
GGCACCTCCGGCAAGCCCTACTCCGCGCGCTACATCGGCAGCCTCGTCGGCGACTTCCACCGCACC
ATGCTCTACGGCGGCATCTACGGGTACCCCAGCGACCAGAAGAGCAAGAACGGCAAGCTGCGGCTG
CTCTACGAGTGCGCGCCCATGAGCTTCATCGCCGAGCAGGCCGGCGGCAAAGGCTCCGACGGCCAC
CAGAGGGTACTCGACATCATGCCCACAGCGGTCCATCAGAGAGTGCCTCTGTACGTCGGGAGCGTG
GAGGAAGTGGAGAAGGTGGAGAAATTCTTGTCTTCAGAGTAG

## FIGURE 25 (continued)

**SEQ ID NO: 193, *Triticum aestivum* chloroplastic fructose-1,6-biphosphatase (Triae_cpFBPase) deduced polypeptide sequence**
MAAATTTTSRPLLLSRQQAAASSLQCRLPRRPGSSLFAGQGQASTPNVRCMAVVDTASAPAPAAAR
KRSSYDMITLTTWLLKQEQEGVIDNEMTIVLSSISTACKQIASLVQRAPISNLTGVQGATNVQGED
QKKLDVISNEVFSNCLRWSGRTGVIASEEEDVPVAVEESYSGNYIVVFDPLDGSSNIDAAVSTGSI
FGIYSPSDECHIGDDATLDEVTQMCIVNVCQPGSNLLAAGYCMYSSSVIFVLTIGTGVYVFTLDPM
YGEFVLTQEKVQIPKSGKIYSFNEGNYALWDDKLKKYMDSLKEPGTSGKPYSARYIGSLVGDFHRT
MLYGGIYGYPSDQKSKNGKLRLLYECAPMSFIAEQAGGKGSDGHQRVLDIMPTAVHQRVPLYVGSV
EEVEKVEKFLSSE

**SEQ ID NO: 194, *Zea mays* chloroplastic fructose-1,6-biphosphatase (Zeama_cpFBPase) nucleic acid sequence contig of DR792524.1 and DT645374.1**
ATGGCCGCCGCCGCCACCACCTCCTCATCCTCCCACTTGCTCCTCCTCTCCCGCCAGCAGGCGGCC
TCCCTACGATGCCGCCTCTCCTTCCTCGGCCAGCCCGCCAGAAGGTCCGGCAGGGTCACGGCCCAG
GCGCCGGCCGCTAAGGACGTGCGGTGCATGGCGGCCGTGGACACTGCGGCGTCCGCGGCGGCGGCG
GAGACGAGCCCCAAGTCGAGCAGCTACGAGATCGTGACGCTCACGACGTGGCTGCTGCAGCAGGAG
CGGACCGGCGCGATCGACAACGAGATGACCATCGTGCTGGCCAGCATATCCACGGCGTGCAAGCAG
ATCGCCGCGCTGGTGCAGCGCGCGCCCATCTCCAACCTCACGGGCGTTCAGGGCGCCGTCAACGTG
CAGGGCGAGGACCAGAAGAAGCTCGATGTCGTCTCCAACGAGGTGTTCTCCAACTGCCTCAAGTCG
AGCGGGCGCACCGGCGTGATCGCCTCGGAGGAGGAGGACGTGCCCGTAGCGGTGGAGCAGAGCTAC
TCCGGCAACTACATCGTCGTGTTCGACCCTCTCGACGGCTCCTCCAACATCGACGCCGCCGTCTCC
ACTGGCTCCATCTTCGGCATCTACAACCCCAACGACGAGTGCCTCGCCGACGTCGACGACAACGAC
ACCCTTGATTCGGTGGAGCAGAGGTGCATCGTGAACGTGTGCCAGCCGGGGAGCAACCTGCTGGCC
GCCGGCTACTGCATGTACTCGAGCTCGGTGATCTTCGTGCTCACCGTCGGCACCGGGGTGTACGTG
TTCACGCTGGACCCCATGTACGGCGAGTTCGTGCTGACGCAGGAGAAGGTGCAGATCCCCAAGGCG
GGCAAGATCTACGCCTTCAACGAGGGCAACTACGCGCTCTGGGACGACAAGCTGAAGCTGTACATG
GACAGCCTCAAGGAGCCCGGCGACTCGGGGAAGCCCTACTCCGCGCGGTACATCGGCAGCCTCGTC
GGCGACTTCCACCGCACTCTGCTCTACGGAGGGATCTACGGGTACCCCAGGGACAAGAAGAGCAAG
AACGGCAAGCTGCGGCTTCTCTACGAGTGCGCCCCCATGAGCTTCATCGTCGAGCAGGCCGGTGGC
AAGGGCTCTGACGGCCACCAGAGAATTCTTGACATCACACCTACAGAGATCCACCAAAGAGTGCCT
CTGTACATTGGGAGCGTGGAGGAAGTGGACAAGGTGGAGAAATTCCTGGCTTGA

**SEQ ID NO: 195, *Zea mays* chloroplastic fructose-1,6-biphosphatase (Zeama_cpFBPase) deduced polypeptide sequence**
MAAAATTSSSSHLLLLSRQQAASLRCRLSFLGQPARRSGRVTAQAPAAKDVRCMAAVDTAASAAAA
ETSPKSSSYEIVTLTTWLLQQERTGAIDNEMTIVLASISTACKQIAALVQRAPISNLTGVQGAVNV
QGEDQKKLDVVSNEVFSNCLKSSGRTGVIASEEEDVPVAVEQSYSGNYIVVFDPLDGSSNIDAAVS
TGSIFGIYNPNDECLADVDDNDTLDSVEQRCIVNVCQPGSNLLAAGYCMYSSSVIFVLTVGTGVYV
FTLDPMYGEFVLTQEKVQIPKAGKIYAFNEGNYALWDDKLKLYMDSLKEPGDSGKPYSARYIGSLV
GDFHRTLLYGGIYGYPRDKKSKNGKLRLLYECAPMSFIVEQAGGKGSDGHQRILDITPTEIHQRVP
LYIGSVEEVDKVEKFLA

**SEQ ID NO: 196, *Physicomitrella patens* chloroplastic fructose-1,6-biphosphatase (Phypa_cpFBPase) nucleic acid sequence contig of BY991118, BJ168552 and proprietary**
ATGGCGACCACACAAGCGATTCTCTCTGCCACCCTTGCCATAGCCCCGGCTTCCAGCTGCGAGACT
TCGTCACGGAGCCCGGCGTCCACCAAAACTTGTCTCTCAGTGGCAGGATCGTCGCTGCATGGCTCA
GTGGCCGGACTCGGAGCTGGGAAACAGATTGTGAGCGTGCAGAGGAAGAGCGTTGCCGTGAGGGCC

FIGURE 25 (continued)

GCCGTTGCAGCTGAGACTGCCGCTCCCAAGCAGCAGGCGAAGAGCCAGTATGACATCACTACCCTG
ACGACGTGGTTGCTGAAGAAAGAGCAGGCGGGCGTCATCGATGGCGAGCTCACCATTGTGCTCTCC
AGCATCGCCCTGGCTTGCAAGCAAATTGCGTCTCTGGTGCAGAGGGCTGGCATCTCCAACATGACT
GGGTTGCAAGGAGCTGCTAACATTCAAGGGGAGGACCAGAAGAAGCTAGACGTTATTTCGAACGAG
GTGTTCTCAAGCTGTCTGCGCTCAAGCGGACGGACAGGCATCATCGCTTCTGAGGAAGAAGACACC
CCGGTTGCAGTGGAGGAGAGCTACTCTGGCAACTACATTGTGGTGTTCGACCCTCTTGACGGCTCT
TCCAACATCGATGCTGCTGTTTCCACGGGGTCAATCTGGGGAATCTACAAGCCCAACGAGGAATGC
CTGACCAATCTCGGAGAGGAGCCAACTATTGATGAGATCGCCGAAAACTGCGTCGTCAATGTCTGT
CAACCAGGGAGCAATTTGTTGTCCGCCGGGTACTGCATGTACTCGAGCTCCGTCATCCTCGTTCTC
TCAGTCGGTGATGGAGTCTACGGCTTCACTCTGGACCCTCTCTACGGAGAATTCGTCCTCTCGCAC
GACAACATCCAAATTCCAAAATCTGGTAAGATCTATTCCATGAATGAAGGCAACTACGCCCTCTGG
GATGACAACCTCAAGAAGTACGTCGACAGCTTGAAGGACCCCGGTCCCAGCGGCAAGCCCTACTCC
GCTCGTTACATCGGCAGTCTGGTGGGCGACTTCCACAGGACAATGCTGTATGGTGGCATCTATGGC
TACCCCAGGGATTCCAAGAGCAAGAACGGGAAGTTGAGGTTGCTTTACGAGTGTGCTCCCATGAGC
TACCTCGCTGAACAAGCAGGTGGGAAGGGCTCCGACGGTCACCAGAGGATTCTGGACATCCAACCT
GAGCAGGTTCACCAACGTGTGCCATTGTACGTTGGAAGCACGGAGGAGGTGGAGAAGTTGGAGAAG
TTCTTAGCTTAA

**SEQ ID NO: 197, *Physicomitrella patens* chloroplastic fructose-1,6-biphosphatase (Phypa_cpFBPase) deduced polypeptide sequence**
MATTQAILSATLAIAPASSCETSSRSPASTKTCLSVAGSSLHGSVAGLGAGKQIVSVQRKSVAVRA
AVAAETAAPKQQAKSQYDITTLTTWLLKKEQAGVIDGELTIVLSSIALACKQIASLVQRAGISNMT
GLQGAANIQGEDQKKLDVISNEVFSSCLRSSGRTGIIASEEEDTPVAVEESYSGNYIVVFDPLDGS
SNIDAAVSTGSIWGIYKPNEECLTNLGEEPTIDEIAENCVVNVCQPGSNLLSAGYCMYSSSVILVL
SVGDGVYGFTLDPLYGEFVLSHDNIQIPKSGKIYSMNEGNYALWDDNLKKYVDSLKDPGPSGKPYS
ARYIGSLVGDFHRTMLYGGIYGYPRDSKSKNGKLRLLYECAPMSYLAEQAGGKGSDGHQRILDIQP
EQVHQRVPLYVGSTEEVEKLEKFLA

**SEQ ID NO: 198, *Galderia sulphuraria* chloroplastic fructose-1,6-biphosphatase (Galsu_cpFBPase) partial nucleic acid sequence AJ302644**
GGCACGAGGAAAGATGTGCAAGTATACTTTATTCAAGTCCTACTATATCTTTACAAAGAAGAGTGT
CAAGAAATAACCACTGCAAAAGATATCTTCCTTATAAGATGGATATCCATCAAAATGATAGCTGTT
CAACCCCAACCCAAAAAGACTCCCGTAGCAGAATATTTACAAACCTCACAAGACACACCAACTTCA
CTCACTCGTTACTTGTTGGAAGTAGCCAAACAAAATAAGGATATGGGAGATATGGTGGCATTGATA
AACGGTATTCAATTTGCTTGCAAAAAGATAGCTTCATTGGTTGGTAAAGCAGGGGTCACTGACTTG
ATGGGAATCTATCAACAAGGCATAGTCAACGTTCACGGAGAAGAACAGAAAAAGTTGGATGTTCTT
TCTAATGAAGTATTGAAGAACGCTCTCAAATATTCGGGAAAAATGGCTGTCATAGCTTCGGAAGAA
GAAGACGTTCCTATCATGGTAGAAGAAAGTTATTCCGGTAACTATGTAGTCGTGTTTGATCCATTA
GATGGTTCTTCCAATTTGGATGCTGGATTGCCTACTGGAACTATATTTGGAGTGTTTCAACAACAA
TTCTCGTGTCTCATTCATGACTATGAGGAGTCCATCGATAACATGGAATTGGCTTGTTTACAAAAC
ACTTTACAACCAGGACGTAGATTGATTGCCGCTGGATATTGTATCTATTCTTCTTCTACCATGTTG
GTACTCTCATTGGGTAATGGTCTTCATTGTTTTACTTTGGATACGGAAGTTGGTGAATTTGTATTG
ACTCGTGCTAACATCCAAATTCCACAAAGAGGTAATATTTATTCTTTCAACGAGTCCAACTTTTAT
CAATGGGATAAAGGAGTTCAAGATTATATAGAGAGGTTAAAAAAAGGAAACAATCAAACAAATTGT
CGTTATTCCGCAAGATATGTTGGCTCCATGGTTGCTGATGTACATCGTACAATATTGTATGGCGGA
ATATTTGGTTATCCAGCAGATAAAAAGAATGTCTCTGGTAAATTGAGATTGGTATATGAATGTGCA

FIGURE 25 (continued)

CCGATGGCATATTTGGTTGAACAAGCAGGAGGTAAAGCAACCACGGGAATAGAAAATATTTTAGAT
TTGACACCAAAAGATATTCACGAAAGGAAGCCTCTTATATTAGGTTCTCCAGCAGATATCGAAGAA
TTTCTGCAAGTATATGGAATGTCACGAGTTGATAGAGAAGATATGCATATGTGGGATAACTTGAGT
AGTTTATAA

**SEQ ID NO: 199, *Galderia sulphuraria* chloroplastic fructose-1,6-biphosphatase (Galsu_cpFBPase) deduced partial polypeptide sequence**
GTRKDVQVYFIQVLLYLYKEECQEITTAKDIFLIRWISIKMIAVQPQPKKTPVAEYLQTSQDTPTS
LTRYLLEVAKQNKDMGDMVALINGIQFACKKIASLVGKAGVTDLMGIYQQGIVNVHGEEQKKLDVL
SNEVLKNALKYSGKMAVIASEEEDVPIMVEESYSGNYVVVFDPLDGSSNLDAGL
PTGTIFGVFQQQFSCLIHDYEESIDNMELACLQNTLQPGRRLIAAGYCIYSSSTMLVLSLGNGLHC
FTLDTEVGEFVLTRANIQIPQRGNIYSFNESNFYQWDKGVQDYIERLKKGNNQTNCRYSARYVGSM
VADVHRTILYGGIFGYPADKKNVSGKLRLVYECAPMAYLVEQAGGKATTGIENILDLTPKDIHERK
PLILGSPADIEEFLQVYGMSRVDREDMHMWDNLSSL

**SEQ ID NO: 200, *Marchantia polymorpha* chloroplastic fructose-1,6-biphosphatase (Marpo_cpFBPase) partial nucleic acid sequence BJ862191**
GAGGAGGATACCCCAGTCGCCGTCGAAGAGAGCTACTCAGGAAACTACGTCGTTGTCTTCGATCCT
CTCGATGGATCCTCCAACATCGACGCTGCGGTATCTACCGGATCCATCTTTGGAATCTACAGGCCC
ACTGAGGAGTGTCTTGCAGACATGGACGACGACTCACAGCTCGGTATGGTGGAACAAAACTGCATC
GTGAACGTATGCCAGCCCGGTAGCAACCTCCTATCCGCTGGGTACTGCATGTACTCCAGCTCCGTC
ATTTTGGTGTTGTCAGTCGGAGACGGTGTCTATGGATTCACACTGGATCCCCTGTACGGTGAATTC
GTCATGACCCACGACAACATCAAGATCCCAAAGAAGGGATCGATCTACTCGTTCAATGAAGGTAAC
TACGCACTCTGGGATGACAAGCTCAAGAAGTACATCGACTCACTGAAGGACCCCGAACCCACCGGC
AAGCCTTACTCTGCTCGTTACATCGGTAGTCTGGTCGGTGACTTCCACAGAACCATGCTCTATGGT
GGCATCTACGGATACCCTGCCGACAAGAAAAGCAAGAACGGAAAGTTGAGACTTCTGTACGAGTGT
GCTCCTATGAGCTACTTGGCAGAGCAGGCCGGAGGAAAGGGTTCTGATGGTTACCGCAGAGTTCTG
GAAATCGAGCCTGAGCAGGTACATCAGCGAGTGCCACTTTTCGTCGGAAG

**SEQ ID NO: 201, *Marchantia polymorpha* chloroplastic fructose-1,6-biphosphatase (Marpo_cpFBPase) deduced partial polypeptide sequence**
EEDTPVAVEESYSGNYVVVFDPLDGSSNIDAAVSTGSIFGIYRPTEECLADMDDDSQLGMVEQNCI
VNVCQPGSNLLSAGYCMYSSSVILVLSVGDGVYGFTLDPLYGEFVMTHDNIKIPKKGSIYSFNEGN
YALWDDKLKKYIDSLKDPEPTGKPYSARYIGSLVGDFHRTMLYGGIYGYPADKKSKNGKLRLLYEC
APMSYLAEQAGGKGSDGYRRVLEIEPEQVHQRVPLFVG

**SEQ ID NO: 202, *Tagetes patula* chloroplastic fructose-1,6-biphosphatase (Tagpa_cpFBPase) partial nucleic acid sequence Contig CON_01b-cs_scarletade-4-e3.b1 proprietary**
ATCATTGCATCGGAGGAAGAAGACGTGCCGGTGGCTGTGGAAGAGAGTTACTCCGGAAACTACATT
GTCGTGTTTGATCCCCTTGATGGGTCATCTAATATTGATGCTGCTGTTTCAACCGGTTCTATTTTC
GGAATCTATAGCCCCAATGATGAGTGTCTCGCTGATATTAGTGACGACTCCACGCTAGACAGTGTG
GAACAAAAATGTATTGTCAACGTATGCCAGCCCGGAAGCAATCTACTAGCAGCCGGTTACTGTATG
TACTCAAGCTCCGTAATCTTCGTGCTCTCGATCGGTACTGGTGTCTACGCGTTCACATTAGACCCA
ATGTACGGTGAGTTTGTACTCACTCAAGAAAAGATTCAAATCCCGAAATCGGGGAAGATTTACTCG

**FIGURE 25 (continued)**

TTTAACGAAGGAAACTATCAACTATGGGACGATAAATTGAAGAAGTACATGGATGATCTAAAGGAC
CCGGGCCCCACGGGCAAGCCGTATTCGGCTCGCTACATTGGTAGCTTGGTTGGTGATTTTCATAGG
ACATTATTGTACGGAGGGATTTACGGGTACCCACGTGACAAAAAGAGCAAGAACGGGAAGCTTCGG
TTGTTGTATGAGTGTGCACCGATGAGTTACTTGGTTGAACAAGCGGGTGGAAAGGGGTCGGATGGA
CATCAACGAGTGCTCGACATTCAACCAACCGAGATTCATCAGCGCGTTCCGCTATACATTGGGAGC
GTAGAGGAAGTGGAAAAATTGGAGAAGTATTTGGCTTGA

**SEQ ID NO: 203, *Tagetes patula* chloroplastic fructose-1,6-biphosphatase (Tagpa_cpFBPase) deduced partial polypeptide sequence**
IIASEEEDVPVAVEESYSGNYIVVFDPLDGSSNIDAAVSTGSIFGIYSPNDECLADISDDSTLDSV
EQKCIVNVCQPGSNLLAAGYCMYSSSVIFVLSIGTGVYAFTLDPMYGEFVLTQEKIQIPKSGKIYS
FNEGNYQLWDDKLKKYMDDLKDPGPTGKPYSARYIGSLVGDFHRTLLYGGIYGYPRDKKSKNGKLR
LLYECAPMSYLVEQAGGKGSDGHQRVLDIQPTEIHQRVPLYIGSVEEVEKLEKYLA

**SEQ ID NO: 204, *Linum usitatissimum* chloroplastic fructose-1,6-biphosphatase (Linus_cpFBPase) partial nucleic acid sequence contig6298 proprietary**
GAGGAGGAACAACGTCGCCGGGGGTTCTGGTTTCAAATGCTCTGCCGTCGGGACGCGCCGTCGGAG
GCGGCAACGACGACGAAGAAGAGGAGTAGCTACGAGATTCTGACGCTGACGACCTGGCTTCTGCAG
CAGGAACAGGCCGGAGTGATCGACGCCGAGCTCACGATTGTGCTCTCCAGCATCTCCACGGCGTGT
AAGCAGATCGCTTCTCTAGTTCAGCGATCCGGGATTTCTAACCTCACCGGCGTCCAGGGCGCCGTC
AATGTCCAGGGTGAGGATCAGAAGAAGCTCGACGTCGTTTCGAACGAGGTGTTTTCAAATTGCTTG
AGGTCGAGCGGCAGGACGGGGATCATAGCGTCGGAGGAAGAAGACGTGCCGGTCGCCGTGGAGGAA
AGCTACTCCGGTAACTATATCGTAGTGTTCGATCCACTTGATGGATCGTCAAACATCGATGCCGCA
GTCTCCACCGGCTCAATCTTCGGAATCTACAGTCCCAACGATGAGTGCCTGGCCGACATCGGAGAC
GGAGACGAGTCAAATCTAGATACTCAGGAGCAGAAGTGTGTGGTGAGCGTGTGCCAGCCGGGGAGC
AACCTACTCGCCGCCGGCTACTGCATGTACTCAAGCTCGGTGATCTTCGTCCTCACGATCGGAAAC
GGCGTTTTCGCCTTCAATCTGGACCCAATGTACGGCGAGTTGGTGTTGACTCAAGAGAACATTCAG
ATCCCGAAAGCNGGAAAGATCTACTCATTCAACGAAGGGGACTACCAAATGTGGGATGAGAAATTG
AAG

**SEQ ID NO: 205, Linum usitatissimum chloroplastic fructose-1,6-biphosphatase (Linus_cpFBPase) deduced partial polypeptide sequence**
EEEQRRRGFWFQMLCRRDAPSEAATTTKKRSSYEILTLTTWLLQQEQAGVIDAELTIVLSSISTAC
KQIASLVQRSGISNLTGVQGAVNVQGEDQKKLDVVSNEVFSNCLRSSGRTGIIASEEEDVPVAVEE
SYSGNYIVVFDPLDGSSNIDAAVSTGSIFGIYSPNDECLADIGDGDESNLDTQEQKCVVSVCQPGS
NLLAAGYCMYSSSVIFVLTIGNGVFAFNLDPMYGELVLTQENIQIPKAGKIYSFNEGDYQMWDEKL
K

**SEQ ID NO: 206, prm08448**
GGGGACAAGTTTGTACAAAAAAGCAGGCTTAAACAATGGCCGCCACCATG

**SEQ ID NO: 207, prm08449**
GGGGACCACTTTGTACAAGAAAGCTGGGTAGCTGCTTAGTGCTTCTTGGT

## FIGURE 25 (continued)

**SEQ ID NO: 208, GOS2 promoter**

ATCCAAGCCAAGAAGAGGGAGAGCACCAAGGACACGCGACTAGCAGAAGCCGAGCGACCGCCTTCT
TCGATCCATATCTTCCGGTCGAGTTCTTGGTCGATCTCTTCCCTCCTCCACCTCCTCCTCACAGGG
TATGTGCCCTTCGGTTGTTCTTGGATTTATTGTTCTAGGTTGTGTAGTACGGGCGTTGATGTTAGG
AAAGGGGATCTGTATCTGTGATGATTCCTGTTCTTGGATTTGGGATAGAGGGGTTCTTGATGTTGC
ATGTTATCGGTTCGGTTTGATTAGTAGTATGGTTTTCAATCGTCTGGAGAGCTCTATGGAAATGAA
ATGGTTTAGGGTACGGAATCTTGCGATTTTGTGAGTACCTTTTGTTTGAGGTAAAATCAGAGCACC
GGTGATTTTGCTTGGTGTAATAAAAGTACGGTTGTTTGGTCCTCGATTCTGGTAGTGATGCTTCTC
GATTTGACGAAGCTATCCTTTGTTTATTCCCTATTGAACAAAAATAATCCAACTTTGAAGACGGTC
CCGTTGATGAGATTGAATGATTGATTCTTAAGCCTGTCCAAAATTTCGCAGCTGGCTTGTTTAGAT
ACAGTAGTCCCCATCACGAAATTCATGGAAACAGTTATAATCCTCAGGAACAGGGGATTCCCTGTT
CTTCCGATTTGCTTTAGTCCCAGAATTTTTTTTCCCAAATATCTTAAAAAGTCACTTTCTGGTTCA
GTTCAATGAATTGATTGCTACAAATAATGCTTTTATAGCGTTATCCTAGCTGTAGTTCAGTTAATA
GGTAATACCCCTATAGTTTAGTCAGGAGAAGAACTTATCCGATTTCTGATCTCCATTTTTAATTAT
ATGAAATGAACTGTAGCATAAGCAGTATTCATTTGGATTATTTTTTTATTAGCTCTCACCCCTTC
ATTATTCTGAGCTGAAAGTCTGGCATGAACTGTCCTCAATTTTGTTTTCAAATTCACATCGATTAT
CTATGCATTATCCTCTTGTATCTACCTGTAGAAGTTTCTTTTTGGTTATTCCTTGACTGCTTGATT
ACAGAAAGAAATTTATGAAGCTGTAATCGGGATAGTTATACTGCTTGTTCTTATGATTCATTTCCT
TTGTGCAGTTCTTGGTGTAGCTTGCCACTTTCACCAGCAAAGTTC

**SEQ ID NO: 209, DNA - *Oryza sativa***

ATGATGGGTTGCTTCACTGTCCTGAGATCCAAGAAGAAGAAGCCTCTTGCTCTCACCAAGAAATCG
GTTGATGCAAGGGAAAGCACGTCCTCAAGACTCCCAGAGCCAGAAGCGCATGTGCCATCGTTACAA
TCTGCTCCTCCTAGTTTTAGGAACAAGGCTAAAATCCACCAATCGGAAAAGAAAGCTTCTTACAGC
AGAGCGCGCGTGCTGTCTGCTCCTTCCAGCCTAATTGTGGTTGATCAGGATGGTCTTCCATATGCC
GAATTCGATGATCAAGATGACTCCAGGGGCAAGGGAGGTTCTATAAAGGGCCACCGTTTCTCTAAT
CCACTGCCCCTTCCTCTCCCATCACCAGAAGGAAAATCATTGAGGAACTTTGGCAGCTTCAAAGCC
ATCAATGCAAGTGGACCACTCGATGCTTCAGGCCCTCTGCCACTTCCTCCAAAGAAGTGTGATGGG
CTTAAGAATTTCTCCTATGAGGAACTTTCATCAGCTTGCCAATGGTTTTCTGGTGACCAGTGTGTT
TCCGAAAGTTTGACATCAACATCATACAAGGCGTCCTTTAGGGATGATTTTACCGACCCAAAGACC
ATTGAAGCAATAGTATCTCGGTTGCTCTCCTCCACTCAGAGTTTGAAAGAGTTTAAAACACAAGTG
AATACCTTGGCATCACTTCAGCATCCCAACTTATGTAAACTAATCGGCTTTCACGCAAGAGAAGAA
TCTAATGAAAGGATGTTGGTCTATGAGCGACTCCATCATGGCAGCTTAGATAAACTACTCTTTGGA
AGATCGGATGGTCGTTTCATGGACTGGTCAGCACGTTTGAAGGTTGCTCTTGGTGCTGCTAGAGGC
CTGGCTTTCCTACATGATGAAGGGCCTTTTCAGGCCATGTACAATGACTTCTCAACCTCAAACATC
CAAATTGACAAAGATTTCACTGCAAAGCTATCAGGATATGGATGTGTTGGATTCAATACCGAGGAG
GAAATATCAAATGCATCTGTGGCTGCTGCAAACCTCTCAGTGGAAACCTTGGAGAAAGGTGTACTG
ACTCCCAAGAGCAACGTATGGTGCTTTGGAGTTGTCCTGCTGGAGCTAATAACAGGAAGGAAGAAC
CTTGATGTCCGTTCCTCAAAAGAAGAACGCAATATTGTCAAGTGGAGTAGGCCTTTCCTCACCGAT
GATAGTCGCCTATCGTTAATCATGGACTCCCGTATAAAAGGACGCTTCCCTACCAAGGCTGCTCGG
ATTGTAGCAGATATCATATTGAGATGCCTTAATAAAGATCCATCAGAGAGGCCTACCATGAGGGCC
GTTGTGGAGTCCCTAGCAAGCGTCCAGGACATAAAGGTTCCATGTCGATATCCTTTGCAAGAGCCA
TCTGCTGCCCCAAGAAAGGTGATGTTAAAATCTACAAGTCTCAATGGCATCATTCATCACCATCCT
GTCGTAACCTTCTCACCGTCACCTCCTTCGCGAAACCAACATTTGCTCTCGCCAAGGTCATCCACG
TCTGCACTGCTTCCTCCAAGGACCAGCTGTGCTCTGGATGACCCTAGAGTAAGCTCTATCAAGAAA
TCGCCTTCCCCTATTTTACGGAGATCTGGTGTTGAGGGTTTTTGA

## FIGURE 25 (continued)

**SEQ ID NO: 210, protein - *Oryza sativa***
MMGCFTVLRSKKKKPLALTKKSVDARESTSSRLPEPEAHVPSLQSAPPSFRNKAKIHQSEKKASYS
RARVLSAPSSLIVVDQDGLPYAEFDDQDDSRGKGGSIKGHRFSNPLPLPLPSPEGKSLRNFGSFKA
INASGPLDASGPLPLPPKKCDGLKNFSYEELSSACQWFSGDQCVSESLTSTSYKASFRDDFTDPKT
IEAIVSRLLSSTQSLKEFKTQVNTLASLQHPNLCKLIGFHAREESNERMLVYERLHHGSLDKLLFG
RSDGRFMDWSARLKVALGAARGLAFLHDEGPFQAMYNDFSTSNIQIDKDFTAKLSGYGCVGFNTEE
EISNASVAAANLSVETLEKGVLTPKSNVWCFGVVLLELITGRKNLDVRSSKEERNIVKWSRPFLTD
DSRLSLIMDSRIKGRFPTKAARIVADIILRCLNKDPSERPTMRAVVESLASVQDIKVPCRYPLQEP
SAAPRKVMLKSTSLNGIIHHHPVVTFSPSPPSRNQHLLSPRSSTSALLPPRTSCALDDPRVSSIKK
SPSPILRRSGVEGF

**SEQ ID NO: 211, DNA - *Arabidopsis thaliana***
ATGGTTTTGGGGTGTTTCCCTTTGAAAAGCAAGAAGAAACGTGGCTCTGTTTCTATGAAGCGGTTG
GATCTTGAAGAAAGCAAGCCAACTGCTTTACCTGAGCCACCAAAGATTCCAAGTCGTAATTTACAA
TCAGGTCCTCCGAGTTTCAGAAATCGTGTGAAGCCAATTCAATCAAACAACGGTGGAAACGGAGAG
ATGAGTAGCCGAGCAAGAGTCATGTTTGCTCCGTCAAGCATCCACGGTGCAGCGGAACGGGATTTG
CTTGCTGGTGTTTACCACGACGAGCAAGATGAACAACCAAGAGATCCACGTACTTCTACTAAAGAA
TCTAGCCCTCAACCACTTCCGTTACCGTCACCAAGAACTGGTTCTTCATTGAAGAATTGGGGAAGC
TTTAAGTCGTTTAACGGAAGCAGCGGTCGGTTATCATCATCCGCAGCTGTATCTGGACCTTTACCT
TTGCCACCTAGCGGGTCAGTTAGGAGCTTTTCATATGATGAAGTAATGGCTGCGTGTAACGCTTTT
TCTTCAGACCGATGTGTCATGGAAGGTCTTTCATCTGTTATGTACATGGCTTCCTTTGGTGATGAG
GCTTCGACCTCAGGTTTAAAGAAGGTTGACGCAACTGTTGTACGACTTCACGTAACTACTCAGAGT
ATTAGGGAGTTCATTAATGAAGTCAACACATTGGCGTCGCTGCAACACCAGAACCTTTGTAAGCTG
GTAGGCTATCATGCTCGTGACGGTTCTGACACAAGAATGTTGGTGTACGAGAGGCTTGCTCTGGGC
AGCTTGGACCGTTTACTGCATGGGAGATCAGATGGGCCTCCTCTTGATTGGAACACTAGAATGAAG
ATTGCACTATGTGCAGCTCAGGGTCTAACCTTCTTGCACGAAGAAGGCCCTTTTCAGGCAATGTAC
AATGAATTTTCGACGGCAAATATCCAAGTCGATAAAGATTTCAGCGCCAAGCTATCAGGATACGGT
TGTGCAGGCCATGCGCCTGAGACAGAGACATCTAATAGTTCGGCACTTGCTAATCTCTCTGTCGAG
ACTCTAGAGAGAGGGCTTTTGACCCCGAAGAGCAATGTGTGGAGCTATGGAATAGTTCTTCTTGAG
ATGTTAACGGGTCGGAAAAATATGGACGGGTCTTACCCGAAAGAAGAGAGGAACTTAGTGAAATGG
AGCAGAGCTTTTCTAGCAGATGATTGCAGGCTCTCGCTTATAATGGATCCTCAGCTTAAAGGTCGG
TTTCCGGCAAAAGCGGCGAGGAGCATAGCAGATATAGCACAGAAATGTCTGCAGGTGGAGCCATCA
GAGCGTCCAACCATGAGAAACATCGTGGATCAACTCAAGATCATACAGGACATGAAGTACTCGTGT
AGGTTCCCGTTAAGAGAACCCGCACCAGTCGCGGCAAGGAAACATATGGGAAGATCAAGCAGTCTC
AACACGATTATTTGGACCCCGGCATCAGTGCCACCAAGGTCAAGTTTTTCACCGTCACCTCCACCA
CGACGACCGTCTGTTTCACCCACAAGGGGACGGACGCTCGTGTTTCCCCCAGTGTTTCCGCCGCGA
GCGTGTTCATCTTTGGAGGAAATGGCTCGGGAAGAGGTTCGAAGATCGTCTTCAGCCAGTGGTAGG
AGAACTAGCCTCGAAGGGTTTTGA

**SEQ ID NO: 212, protein - *Arabidopsis thaliana***
MVLGCFPLKSKKKRGSVSMKRLDLEESKPTALPEPPKIPSRNLQSGPPSFRNRVKPIQSNNGGNGE
MSSRARVMFAPSSIHGAAERDLLAGVYHDEQDEQPRDPRTSTKESSPQPLPLPSPRTGSSLKNWGS
FKSFNGSSGRLSSSAAVSGPLPLPPSGSVRSFSYDEVMAACNAFSSDRCVMEGLSSVMYMASFGDE
ASTSGLKKVDATVVRLHVTTQSIREFINEVNTLASLQHQNLCKLVGYHARDGSDTRMLVYERLALG
SLDRLLHGRSDGPPLDWNTRMKIALCAAQGLTFLHEEGPFQAMYNEFSTANIQVDKDFSAKLSGYG
CAGHAPETETSNSSALANLSVETLERGLLTPKSNVWSYGIVLLEMLTGRKNMDGSYPKEERNLVKW
SRAFLADDCRLSLIMDPQLKGRFPAKAARSIADIAQKCLQVEPSERPTMRNIVDQLKIIQDMKYSC
RFPLREPAPVAARKHMGRSSSLNTIIWTPASVPPRSSFSPSPPPRRPSVSPTRGRTLVFPPVFPPR
ACSSLEEMAREEVRRSSSASGRRTSLEGF

**FIGURE 25 (continued)**

**SEQ ID NO: 213, DNA - Sorghum bicolor**
CTACTGGATTAACCCCCCGCGTGCGTTCCCTCCCTCTGTCAGTCTGTCTCTCTCTTGGCGTCGACT
GGGCGACCGTCGCAGCCGCCCTAAGCCAGGTACCCAGACCATCTTCGGGCCCTTCGCCGGCGACGA
GCACCACCAGGAATTTTCCCAGCTGTAGCAATGATGGGTTGCTTCACTGTTCTCAGATCCAAGAAG
AAGAAAATCCCTTTTGATAATCCTCTTCTTCCAAGCAAGAAATCGGTTGATGCAAGGGAAAGTACG
TCGTCTAGACTTCCGGAACCAGAAGTTCATGTGCCATCTTTGCAATCAGCTCTCTCCTAGTTTTAG
GAACAGGACTAAGATATCCCAGTCGTCAAATAAAGTTTCAAACAGCAGAGCTCGCGTGTTGTCTGC
TCCGTCAACCCTTATTGTGGTTGATCAGTTTGGCTTTCCATATGCTGAATACCGAGATCAAGACGA
CTCCAGAGATAAGGAAGGTTCAACAAAGGGGCATCGCTTTTCAAATCCTTTGCCCCTTCCTCTTCC
TTCACCGGAAGGACATTCTTTTAGGAACTCTGGTAGCTTCAAAGCTAGCAACGTAAGTGGGCCATT
GGAGATGTCCAGCCCTCTCCCATTGCCTCCAAAGAAATGTGATGGACTTAGAATCTTTTCTTACGA
GGAGGTTTTGTCTGCTTGCCAATGGTTTTCAAGTGATCAGTGTGTTTCAGAAGCACTTGGTTCAAC
ATCATACAAGGCAACATTTAGGGATGAATTTATTGATACAAAGACCACTGAAGCAACGGTAGCTCG
ATTACTCCCCTCCACTCAGAGTTTGAAGGAGTTTAAAACACAAGCAACTACATTGGCATTGCTTCA
GCATCCCAATTTATGTAAACTGATTGGCTATTATGCAAAAGAAGATTCTAATGAAAGGATGTTGGT
CTATGAACGGCTTCATCATGGGAGCTTAGATAAGCTACTCTTTGGAAGACCAGATGGTCGTTTCAT
GGACTGGTCTAAACGTTTGAAGGTTGCCCTTGGTGCGGCCAGAGGTCTTGCTTTCTTGCATGATGA
AGGACCCTTTCAGGCCATGTACAGCGAGTTCTCAACTTTGAACATCCAAATAGATAAAGATTTCAC
TGCTAAGCTTTCAGGATATGGTTGTGTTGGCTTCAATACTGAGGAGATATCTAATGCACCTGCGTC
TGCAGCAAACCTTTCGGTGGAGACCTTGGAGAAGGGTTTACTCACTCCCAAGAGCAATGTCTGGAG
CTTTGGAGTTGTGTTACTGGAGCTAATAACTGGAAGGAAGAACCTTGATGCCAATTCTTCTAAAGA
AGAACGCAATATTGTCAGGTGGAGTAGGCCTTTCCTTACTGATGATAGTCGCCTATCTCTGATCAT
GGACTCCCGTATAAAAGGGCGCTTTCCTACTAAGGCTGCTCGGATAGTAGCAGACATCATTCTGAA
ATGCTTGCATAATGATCCATCCGAGAGGCCGACCATGAGGGATGTTGTGGAGGCTCTAGCAAGAGT
CCAGGAGATAAAGGTTCCGTGCAGATATCCTTTGCAAGAACCTTCTGCTGCACCAAGAAAAGTAAT
GCTAAAATCTACATCCCTCAATGGAATTGTGCCTCATCATCCTGTCATAACCTTCTCTCCATCGCC
GCCTTCGCATAACCAGCACTTGATTTCACCAAGGTCATCAACATCTGCCTTGTTTCATCCAAGGAC
ATGCTCTTCTACTCTGGATGATCCCGGTGTAAGCTCTATAAAGAAAACACCTCCTATTATGCGTAG
GTCTAGCGTTGAAGGCTTTTGATTGTCCATATTGTTCTTTTATTTCTTTTTCTCGGATTTATTTGT
TTCTTTGTTAGCCTAGTGATTTTAGCTGTGTTCTGTATTGTAAGACAAGTGGCCTTATGTAGTGCC
CTTGAGTCTCGAGTAATAACTAAGCAGAGCAGGATGAATTGTAAATAGTATCAGGTTGTAGATACC
TTTTTGTGCTCTAATTGGGTGGAAGCAGCGTGCTGAGTCTGAGTAGGCCTTGTAACCTCATAAATA
AACTCGTTTCCCAGTTTCTGTCC

**SEQ ID NO: 214, DNA - *Saccharum officinarum***
AGAAACACTTGGTTCAACATCATACAAGGCAACATTTAGGGATGAATTTATTGATACAAAGACCAC
TGAAGCAACGGTAGCTCGATTACTCCCCTCCACTCAGAGTTTGAAGGAGTTTAAAACACAAGCGAC
CACATTGGCATCACTTCAGCATCCCAATTTGTGTAAACTGATTGGCTATTATGCAAAAGAAGATTC
TAATGAAAGGATGTTGGTCTATGAACGGCTTCATCATGGGAGCTTAGATAAGCTACTCTTTGGAAG
ACCAGATGGTCGTTTCATGGACTGGTCTAAACGTTTGAAGGTTTCCCTTGGTGCTGCCCGAGGTCT
AGCTTTCTTGCATGATGAAGGACCCTTTCAGGCCATGTACAGTGAGTTCTCAACTTTGAACATCCA
AATAGATAAAGATTTCACTGCTAAGCTTTCAGGATATGGTTGTGTTGGCTTCAATACTGAGGAGAT
ATCTAATGCACCTGCGTCTGCAGCAAACCTTTCGGTGGAGACCTTGGAGAAGGGTTTACTCACTCC
CAAGAGCAACGTCTGGAGCTTTGGAGTTGTGTTACTGGAGCTAATAACTGGAAGGAAGAACCTTGA
TGCCAATTCTTCCAAAGAAGAACGCAATATTGTCAGGTGGAGTAGGCCTTTCCTTACTGATGATAG
TCGCCTATCTCTGATCATGGACTCCCGTATAAAAGGGCGCTTTCCAACTAAGGCTGCTCGGATAGT
AGCAGACATCATTCTGAAATGCTTGCATAAAGATCCATCAGAGAGGCCGACCATGAGGGATGTTGT
GGAGGCCCTAGCAAGAGTCCAGGAAATAAAGGTTCCATGCAGATATCCTCTGCAAGAACCTTTTGC

## FIGURE 25 (continued)

TGCACCAAGAAAAATAATGTTAAAATCTACATCCCTCAATGGAATTGTGCCTCATCATCCTGTCAT
AACCTTCTCCCCTTCGCCGCCTTCACATAACCAACACTTGATTTCACCAAGGTCATCAACATCTGC
CTTGTTTCATCCAAGGACATGCTCTTCTACTCTGGATGATCCCGGTGTAAGCTCTATAAAGAAAAC
ACCTCCTATTATGCGTAGGTCTAGCGTCGAAGGCTTTTGATTGTCCATATTGTTCTTTTATTTCCT
TTTCTTGGATTTATTAGTTTCTTTGGTAGCCTAGTGATTCTAGCTATGTTCTGTATTGCAAGACAA
GCGGCCTTAATGTAGTGCCCTTGGGTCTAGAGTAATAACTAAGCAGAGCAGGATGAATTGTAAATA
GGATCAGGTTGTAGATACCTTTTTGTGCTCTAATTGGGTGGAAGCAGCGTGCTAAGTGAGCCTTGT
AACCTCCCAATGCAATAAACTCGTTTTTCAGTTTTTGTTC

**SEQ ID NO: 215, DNA - *Medicago truncatula***
ACGAGGCTATTCTCTCTCTTTCTCTCTCTACATTTCTCAACATTCTTCAAATTTCTCTCTCCAAAA
CCTTCACTTCGCAGCTTTTTCAATCTGCTTTCTTTGACTCTGTAACAGTTTTTTTCGTGGAAGAAT
CAGAACCACAAAAAAGTTTCGATTTTTACCCATTTCTTCAACCCGTGATCGCTTCACTGTAATTGG
CATGAGCTTCCATTTCTCGTTCTGACAAAAACAGGTATCTATAACATCCTAGTTCTGTACATAGAA
TGGGGTGTTTTACTATATTGAAGAGAAAGAAGAAAAAGCCTGATCAGATTGTGTATGTAAAACGCG
TAAGCCCTGGCGAAGATTCACCTACAGTACTGCCCGAACCCCAAACTCATACCCGCTCACTCCAGT
CTGCGCCTCCTAGTTTTAAGATCAGAGTGAAACCCATTCAACCTAGCAATAAAGCCACTAACAATA
GAATACGAGCATTGTCTGCTCCATCGAGTCTTGATGATGCAGAACAAGATGCATTGGCCACCATTG
AGTATGAAGAACAAGAAGGGTCAAAATACCGAACTGGATCATGGAAGGAGCAGCGTTCGCCTAGTC
CACAACCATTACCGCTTCCATCTCCTAAGGGTGGTGGTACATTGAAGACTGTTGGAAGCTTTAAGT
TGGGGATAGCTAGTAGTCCTTTATATGCTTCTGGACCTTTGCCGCTTCCACCAACTGGGTCACTGA
GAAACTTTTCTTATGATGAACTTGCTGCTGCTTGCCTCAATTTCTCTTCAGATCGATACATGTCAG
AATCTCTTTCATCCACCATGTATAAAGCTTCCTTTGGTGATGATACCTCAACTTCAAGTTCAAAGA
AGTTTGAAGCTACTGTCACACGCCTTCGCCCATCATCTCAGGGCCTGAAGGAATTCATAAATGAGG
TTAATACTCTTGCATCATTGCAGCATCCGAACCTCTGTAGATTGCTAGGATTTCACGCAGGTGATG
GTTCAGAGCATAGAATGTTGGTTTATGAGAGGCTATACCATGGAAGCTTGGACCGCTTATTGTATG
GGAGATCTGATGGGCCATCAATTGATTGGAATACAAGAATGAAAATTGCAATATGTGCTGCACTAG
GTCTTTCTTTCTTGCATGAAGAAGGGCCTTTTCAGGCAATGTATAATGAATTTTCAACAGCTAACA
TTCAGATTGACAAAGATTTCAGTGCAAAGCTTTCAGGATATGGTTGTGTTGGACATGTTCCGAAGG
AAGAGATTTCAAGCAGTTCATCTGCCGTTGGAAACCTATCGATGGAGACACTGGAGAAAGGAATGC
TTACTCCGAAAAGCAATGTATGGAGTTTCGGAATTTCCTTCTAGAGCTACTTACAGGAAGAAAGA
ATCTCGATAGCCCGTCACCCAAAGGAAGAGAGAAATTTGGTGAAGTGGAGCAGGCCTTTCCTGTCT
GATAATCATCGTTTGTCAATGATCATGGATCCTCAACTTAAAGGTCGCTTTCCTTCTAAAGCGGCG
AGTACAATAGCTAACATTGCACAAAGATGCCTTCAAATGGAGCCATCAGAACGACCAACCATGGGA
ACAGTTGTTGAGCAGCTGAAAAAGATACAAGATTTGAAGCATTCTAGCAGATTCCCGCTGCAAGAA
CCTGCACAAATGTCGAGATCACCAAGTCTTAACGGTATCAACCACCCTGCACCAAGGCCGAGTTTC
TCTCCATCACCATCATCCAGAGCCCTGGTATCTGTCTCACCTCCAAGATGGTCGGGAGTGTCAATT
CAACTTCCACCTCGCGCGTTTTCTTCAACCCTCTATTTGGAGGAGCTTGATAGGCAAGAAAGCCGC
AAGTCAGCTTCAGCCTCTAGGAAGGCTAGTGTTGAAGGATTTTGATTGTCGATAGTGTTCATTTTT
TATTTGTTATTCTTTTTTTGGTGTAGTTCAACAGAGATTTATAGGAGATAAAGATTTGTAATCATC
CCTCAGTGTGATGCAGAGAGGATGCTCTTTTGTACATAGTGCAAAGGTTGGTCCCCTTGGTTCAAT
TAGTTACTCCATTTTG

**SEQ ID NO: 216, DNA - *Zea mays***
CTCCCCCGGCGTGCGCTCCCTCCTGTCTCTCTTGGCGACGACTGGGCAACGGTCGCAGCTGTATGT
TCGAGCCCTTCGCCGACGACGTTCACCACCAGGAATTTTCTCAGCTGTGCCAATGATGGGTTGCTT
CACTGTTCTCAGATCCAAGAAGAAGAAAAGCCATTTTGATAATCCTCTTGTCCCAAGCAAGAAATC
AGTTGATGCAAGGGAAAGTACGTCCTCTAGACTTCCGGAGCCAGAAGTTCATGTGCCATCTTTGCA

FIGURE 25 (continued)

ATCAGCTCCTCCTAGTTTTAGGAACAGGGTCAAGATATCCGAGTCATCAAATGAAGTTTCAAACAG
AAACAGCAGGGCTCGCGTGCTGTCTGCTCCATCAGCCCTTATTGTGGTTGATCAGTTTGGCTTTCC
ATATTCGGAATACCGAGATCAAGATGACTCCAGGGATAAGGAAGGTTTGACAAAGGGGCATCGCTT
TTCAAATCCTTTGCCCCTTCCTCTTCCTTCACGCGAAGGACATTCTTTTAGGAACTCTGGTAGCTT
CAAAGCCAGCAACGTAAGCGGGCCATTGGAGATGTCTGGCCCTCTCCCATTGCCTCTAAAGAAATG
TGATGGACTTAGAATCTTCTCTTATGAGGAGATTTCATCTGCCTGCCAACGATTTTCTAGTGATCA
GTGTGTTTCAGAAACACTTGGTTCAACATCATACAAGGCAACATTTAGAGATGAATTTATTGATAC
GAGGACCACTGAAGCAACAGTAGCTCGATTACTCCCCTCCACTCAGAGTTTGAAGGAGTTTAAAAC
GCAAGCGACCACATTGGCATCGCTTCAGCATCCCAATTTATGTAAACTGATTGGCTATTATGCAAA
AGAAGATTCTAATGAAAGGATGTTGGTCTATGAACGGCTTTATCATGGAAGCTTAGATAAGCTACT
CTTTGGAAGATCAGATGGTCGTTTCATGGACTGGTCTAAACGTTTGAAGGTTGCCCTGGGTGCTGC
CAGAGGTCTAGCTTTCTTGCATGATGAAGGACCCTTTCAGGCCATGTACAGCGAGTTCTCAACTTT
GAACATCCAAATAGATAAAGGTTTCACTGCTAAGCTTTCAGGATATGGTTGTGTTGGCTTCAATAC
CGAGGAGATATCTAATGCACCTGTGTCTGCAGCAAACCTTTCGGTGGAGACCTTGGAGAAGGGTTT
ACTCACTCCCAAGAGCAACGTCTGGAGCTTTGGAGTCGTGTTACTGGAGCTAATAACTGGAAGGAA
GAACCTTGATCCCAATTATTCCAAAGAAGAACGCAATATTGTCAACTGGAGTAGGCCTTTCCTTAC
TGATGACAGTCGCTTGTCTCTTATCATGGACTCCCGTATAAAAGGGCGCTTTCCCACTAAGGCTGC
TCGGATAGTAGCAGACATCATTTTGAAATGCCTGCGTAAGGATCCATCGGAGAGGCCTACCATGAG
GGATGTTGTGGAGGCCCTAGCAAGAGTCCAGGAAATAAAGGTTCCGTGCAGATATCCTCTGCAAGA
ACCTTCTGCTGCACCAAGAAAAGTGATGTTGAAATCTACATCCCTCAATGGGATTGTGCCTCACCA
TCCTGTCATAACCTTCTCTCCATCGCCGCCTTCACATAACCAGCACTTGATTTCACCAAGGTCGTC
GACATCTGCCTTGTTTCATCCAAGGGCATGCTCTTCTACCCTGGACGATCCGAGTGTAAGTTCTAT
AAAGAAAACACCACCTATTATGCGAAGGTCTAGCGTCGAAGGCTTTTGATTGTCCATATTGTTCCT
TTATTTCCTTTTCTTGGATTTATTTGTTTCTTTGTTAGTAGTGTAGCGGTTTTTAGCTGTGTTCTG
TATTGTAAGACAAGTGGCCTCATATGTAGTGCCCTTGGGTCTGGAGTAAAGCAGAACAGGATGAAT
TGTAAATAGGACCAGGTTGTAGATACCTTTTTTTTGTGCTCGAATTGGGTGGAAGCAGCGTGCCGA
GTGGGCCTTGTAACCTCATAATGCAATAAACTCCGTTTCCCAGTTTCTGTAAAAAAAAA

**SEQ ID NO: 217, DNA - *Hordeum vulgare***
TGCCGAATTCGGCACGAGAAAGAGGTCCGAGGGGCTTAAGAACTTCTCGTACGATGAGATTTCCAC
TGCTTGCCAGTGGTTTTCTGGCGATCATCGTGTCTCAGAAACTCTGACTTCGACATCATACAAGGC
ATTGTTCAGGGATGATTTCGTTGAACCAAAGAAGATGGAAGCAATAGTAGCCAGGTTACTCCCTTC
CAATCAGAGTTTCAAAGAATTTAAGGCACAAGTAAATACGTTGGCATCACTTCAACATCCCAATTT
ATGTAAACTCATCGGCTATCACGCAAGAGAAGAATCTAATGAAAGGATGTTGGTCTATGAGCGGCT
CCATCATGGCAGTTTAGACAGGTTACTCTTTGGAAGACCGGAAGGTCGTTTCATGGACTGGTCTAC
ACGTTTGAAGGTTGCCCTTGGTGCTGCTAAAGGTCTAGCTTTCCTACACGATGAAGGACCCTTTCA
GGCAATGTATGATGACTTCTCAACGTCAAACATCCAAATAGAGAAAGATTTCACTGCAAAGCTGTC
GGGATATGGTTGTGTTGGCTTCAATTCTGACGAGGAAAGATCCAAGGCATCTGTGGCTGCAAACCT
CTCAGAGGAAACCTTGGAGAAAGGCGTACTGACTCCGAAGAGCAACGTATGGAGCTTTGGAGTTGT
CTTGCTCGAGCTAATAACAGGACGGAAGAACCTCGATGTACGTTCCACCAAAGAAGAACGTAATAT
TGTCAAGTGGGGTAGGCCTTTCCTCACCGACGATAGTCGTCTATCCCTCATCATGGATCCCCGTAT
AAAAGGACGCTTTCCTACCAAGGCTGCTCGAACTGTGGCAGACATAATTCTGAAGTGCCTTCAAAG
AGATCCATCAGAAAGGCCTACGATGAGGGCCGTCGTGGAGGCCCTAACAAGCGTTCAGGACATAAA
GGTCCCCTGCCGGTATCCTCTTCAAGTACCGTCCCGCCGCACCCGAGGAAAGTGATGCTAAAGTCT
ACGAGCCTCAATGGCATTGTTCCTCAGCATCCCGTCATGACCTTCTCGCCGTCGCCTCCTTCGCGC
AACCAGCACCTGGCGTCGCCAAGATCATCGACTTCCGCGCTTCTTCCCCCAAGGACCTGCTCTTCC
ATCATCACCCTGGATTACCCCAGGGTAAGCTCGGTCAAGAAGTCGCCTTCCGGTATCCTGCGGAGA
CCTGGCGTCGAGGGTTTTTGATTGTCCATACATGGTCGGATTTCTTCTCTTTGCCTTGGATTTATT

## FIGURE 25 (continued)

TGTTGTTTTGGTTAGCCTAGCGAGTGGTCTCAGTGCTATGTGATATGTATATGTTGGAAAGACATC
TGAAAAAAGGGCAGAGTGAAGTGTAGATAGTAGAACCAGCTTTGTAGATACTTGTCGTTCTCTGAT
TGGATGGGGGTCATGGAAGCAGTGTGTGTTAGAGTGGGGCTTGTAACCCCATTATTATGCAATAAA
CGTGGGTTGGTCGGTCGTATTTGCTCG

**SEQ ID NO: 218, DNA - *Glycine max***
GTAAATTGCTAGGATTTCATGCACGAGAGGGTTCAGAACATAGAATGTTGGTTTATGAAAGGCTAT
ACCATGGAAGCTTGGATCGCTTATTGTATGGGAGATCTGATGGGCCATCAATTGATTGGAATACAA
GAATGAAAATTGCTATATGTGCTGCACAAGGTCTAACCTTCTTGCACGAAGAGGGGCCTTTTCAGG
CTATGTATAATGAGTTCTCAACAGCCAACATACAGATTGACAAAGATTTCAGCGCAAAGCTTTCAG
GATATGGTTGTGTTGGACATATTCCCGAAGAAGAGATTTCAAGCAGCTCATCTGCTGTTGGAAACC
TATCAATGGAAACACTGGAGAAAGGAATGCTCACTCCAAAGAGCAACGTATGGAGTTTTGGAATTT
TTCTTCTGGAGCTACTTACTGGAAGAAAGAATCTTGATAGCCGTCACCCCAAGGAAGAGAGGAATT
TAGTCAAGTGGAGCCGGCCTTTCTTAGCCGATAACTACCGTCTGTCGTTGATCATGGATCCTCAAC
TCAAAGGCCGCTTTCCTTCTAAAGCAGCAAGAACAATAGCTGATATTGCACAAAGATGCCTTCAAA
AGGAGCCATCAGACAGACCTACCATGAGGACTGTTGTTGAGCATCTCAAGATAATACAGGATTTGA
AATATTCGTGCCGGTTCCCTCTGCAAGAACCAGCATCAAACTCTGGAAAACATATGTCAAGATCAC
CAAGTCTCAATGGCATCATCTGCCCTGCACCAAGGGTGAGTTT

**SEQ ID NO: 219, DNA - *Solanum tuberosum***
TTCTTAGCTCATCGAAACGTTAAACCTTACAATACTTCAAACATCGATCAATACAATTTGCTGAAG
CACGCAATTTTTCAAGGACTGTATAAACAACAAAGATGGGTTGTTTCACAGTTTTAAAAAGTAAGA
AGAAGAAGTCTGAACAGAGTATTCACATCAAACGTGTGAATCCTCAGGAACATTCTCCTACTGCAT
TGCCCGAGCCTCAAGTGCAGACACGGTCGTTGCAGTCGGCACCTCCAAGTTTTAGAACTAGAGTAA
AACCTGTACAGTCGAGTAATAGAGTTACAAGCAGTAGGGCGCGGGCACTCTCTGCCCCATCTAGCC
TGGACTCAGCAGAACAAGATGTAGCATCAAATGAATGTGAGGAACATGATGAGTTCAAGAGTCGTA
TTGGTTCAATCAAGGAGTACCAATCACCAAGTCCTCAGCCTCTTCCTCTTCCATCTCCACAGAGTG
CCGCTGCCACTCTCAAGACTATGGGAAGCTTTAAAGTTGGCAACGCCAGCGGTCCGTTAAATGCCT
CTGGACCCCTGCCACTGCCTCCTACACTGCCTTCAACATTGCCTTCTACTGGAGCACTCAGGAACT
TCTCATTTGAAGAACTTGCTGCTGCCTGCCACCGCTTCTCTCCTGAACGGTGTATGTCAGAAGGTC
TCTCTTCTGTTATTTACAGAGCTTCTTTTGGAGATGATGCAACTGGTACAAAGAAGCTTGAAGCCA
CTGTAACCCGGCTTCATCCTTCTTCGCAGGGGTTGAAGGAATTTGTGACTGAGGTGAACACACTAG
CTTCTTTGCAACATCCATCACTTTGTAAACTGATTGGTTTCCATGCCACGGGGAAGGTTCCGAGCA
CAGAATGTTGGTTTATGAAAGGCTTTTTCATGGAAGCTTAGACCGGCTTTTGTTTGGGAGGTCCGA
TGGTCCCCCGATAGACTGGAATGCTCGAACGAAAATTGCTTTATGTGCTGCTCAAGGTCTCACATT
CCTGCATGAGGAAGGACCTTTTCAGGCAATGTTCCATGAATTTTCCACTGCAAATATACAAATTGA
TAAGGATTGCAGTGCAAAGCTCTCGGGATATGGATGCATTACTCCTATACCAGAGACAGACATATC
ATGCAGTTCAGCTGCCCTGGCAATCTCT

**SEQ ID NO: 220, DNA - *Lycopersicon esculentum***
CATGGAAGCTTACACCGGCTTTTATTTGGGAGGTCGGATGGTCCCCCAATAGACTGGAATGCTCGA
ATAAAAATTGCTTTATGTGCTGCTCAAGGTCTCACATTCCTGCATGAGGAAGGACCTTTTCAGGCA
ATGTTCCATGAATTTTCCACTGCAAATATACAAATTGATAAGGATTGCAGTGCAAAGCTCTCGGGA
TATGGATGCATTACTCATATACAAGAGACAGACATATCATGCAGTTCAGCTGCCCTGGCAAATCTC
TCTGAGGAGACTCTGGAGCGAGGATTGGTAACTCCAAAGAGTAATGTTTGGAGTTTTGGGATTGTT
CTTCTTGAGCTGCTGACTGGCCGGAAGAATTTAAGCAGTCGGCATCCAAAGGAAGAGAGGAATTTA
GTGAAGTGGAGCAAGCTTTTTCTAGCTGATGACAGTAGATTATCGCTAATCATGGACCCTCAGTTA
AAAGGTCGGTTCCCTGCCAAAGCAGCAAGAACTGTGGCTGATATTGCTCAAAGATGTCTGCAAAAG

## FIGURE 25 (continued)

GATCCATCTGAAAGGCCCACCATGAGAACTGTAGTGGAGCAACTCAAGACTGTACAAGTTATGAAG
TACCCTTCTCGGTTTCCTCTTCAAGAGCCAAGGGCAGTTGGTGTAAAACACATGTCAAAGTGTCCG
AGCCTAAATGGAATTATTACCCCAACATCAAGATTGAGCTTCTCCCCTTCACCACCAACCCACCCT
ATATCTATTTCTCCGACAAGGACAGCTGCTCCACTGCTGTCTCTACCTTCATGTTCCTCCATCCTC
TCTATGGAGGACTTTGATCGATTGGAAAATCGAAGGCCATCATCTTCATCTGTTCGGAGGTCTAGT
GTCGAAGGATTTTGATCGATTGTAGAGCACTTTTTTCTTCAAATTGCTTTTC

**SEQ ID NO: 221, DNA - *Lycopersicon esculentum***
GAGAGGCTTTTTCATGGAAGCTTAGACAGACTTTTGTTCGGAAGATCAGATGGCCCCTCTATAGAT
TGGAATGCGAGAACCAAAATTGCCCTATGTGCTGCACAAGGTCTTACATTTCTACATGAGGAGGGA
CCTTTTCAGGCAATGTTCCAAGAATTTTCAACTGGAAATATACAAATCGACAAGGATTTTAGTGCA
AAGCTCTCGGGGTATGGATGCATTACGAATATACAAGAGACGGAGATATCTTGCAATTCAATCGCC
CTGGCGAATCTCTCACAGGAGACACTGGAGAGAGGGTTGATAACTCCTAAGAGCAATGTTTGGAGT
TTCGGGATTGTTCTTTTAGAACTGCTCACTGGCCGGAAGAATCTTGATGGTCGGTATTCAAAGGAA
GAGAGGAATCTAGTCAAGTGGAGTAGGCCTTTCCTTGCTGATGATGGTAGATTATCGCTTATCATG
GATCCTCAGCTTAAAGGACGGTTTCCCGCAAAAGCAGCCCGTACAGTGGCTGATATTGCCCAAAGA
TGCCTGCAAAAGGATCCATCTGAAAGGCCCACCATGAGAACTATCTTGGACCAACTCAAATCCGTA
CAAGTGATGAAGTGCCCTTCACGGTTTCCTCTGCAAGAACCAGCGGTTGTTGGTGGTAAACACATG
TCAAAGTCTCCAAGCATGAAT

**SEQ ID NO: 222, DNA - *Triticum aestivum***
TTTCCTCCCAAGGCTGCTAGGACTGTGGCAGACATCATTCTGAAGTGCCTTCATAGGGATCCATCC
GAAAGGCCTACGATGAGGGCCGTCGTGGAGTCTCTAGCAAGCGTCCAGGACATAAAGGTCCCCTGC
CGGTATCCTCTTCAAGTACCGTCCGCCGCACCGAGGAAAGTGATGCTAAAATCCACGAGTCTCAAC
GGCATTGTTCCTCAGCATCCTGTCATGACCTTCTCGCCGTCGCCTCCTTCGCGCAACCAGCACCTG
GTGTCACCGAGATCATCGACGTCTGCGCTGCTTCCCCCAAGGAACTGCTCTTCCACCATCACCCTG
GACTACCCCAGGGTAAGCTCGGTCAAGAAATCGCCCCCCAACATCATGCGGAGACCTGGCGTCGAG
GGTTTTTGATTGTCCATATAGTGTCGGATTTTCTTCTCTTTGCCTTGATTTATTTGTTGTTTTGGT
TAGCCTAGCGAGTGATCTCAGTGCTATGGGATATACTATGTTGCAAGGACATGTGAAAAAGGGCAG
AGTGAAGCGTAGATAGTAGAACCAGCTTGTAGATACTTGTCGTTCTCTGATTGGATGGGGGTCATG
GAAGAAGTGTGTGTTGAGTGGGGCTTGTAACCCCACTATGCAATAAACGTGGGTTGGTTGGTCGGT
CGGTATTTGCTCGCCGTCAAACATTTCAGCTGTTTCTTTCTTCTCTTGAATGCTAAGTTTTGTTGC
TGCTAGTCGTGGAAT

**SEQ ID NO: 223, DNA - *Zea mays***
CTCCCCCGGCGTGCGCTCCCTCCTGTCTCTCTTGGCGACGACTGGGCAACGGTCGCAGCTGTATGT
TCGAGCCCTTCGCCGACGACGTTCACCACCAGGAATTTTCTCAGCTGTGCCAATGATGGGTTGCTT
CACTGTTCTCAGATCCAAGAAGAAGAAAAGCCATTTTGATAATCCTCTTGTCCCAAGCAAGAAATC
AGTTGATGCAAGGGAAAGTACGTCCTCTAGACTTCCGGAGCCAGAAGTTCATGTGCCATCTTTGCA
ATCAGCTCCTCCTAGTTTTAGGAACAGGGTCAAGATATCCGAGTCATCAAATGAAGTTTCAAACAG
AAACAGCAGGGCTCGCGTGCTGTCTGCTCCATCAGCCCTTATTGTGGTTGATCAGTTTGGCTTTCC
ATATTCGGAATACCGAGATCAAGATGACTCCAGGGATAAGGAAGGTTTGACAAAGGGGCATCGCTT
TTCAAATCCTTTGCCCCTTCCTCTTCCTTCACGCGAAGGACATTCTTTTAGGAACTCTGGTAGCTT
CAAAGCCAGCAACGTAAGCGGGCCATTGGAGATGTCTGGCCCTCTCCCATTGCCTCTAAAGAAATG
TGATGGACTTAGAATCTTCTCTTATGAGGAGATTTCATCTGCCTGCCAACGATTTTCTAGTGATCA
GTGTGTTTCAGAAACACTTGGTTCAACATCATACAAGGCAACATTTAGAGATGAATTTATTGATAC
GAGGACCACTGAAGCAACAGTAGCTCGATTACTCCCCTCCACTCAGAGTTTGAAGGAGTTTAAAAC
GCAAGCGACCACATTGGCATCGCTTCAGCATCCCAATTTATGTAAACTGATTGGCTATTATGCAAA

## FIGURE 25 (continued)

AGAAGATTCTAATGAAAGGATGTTGGTCTATGAACGGCTTTATCATGGAAGCTTAGATAAGCTACT
CTTTGGAAGATCAGATGGTCGTTTCATGGACTGGTCTAAACGTTTGAAGGTTGCCCTGGGTGCTGC
CAGAGGTCTAGCTTTCTTGCATGATGAAGGACCCTTTCAGGCCATGTACAGCGAGTTCTCAACTTT
GAACATCCAAATAGATAAAGGTTTCACTGCTAAGCTTTCAGGATATGGTTGTGTTGGCTTCAATAC
CGAGGAGATATCTAATGCACCTGTGTCTGCAGCAAACCTTTCGGTGGAGACCTTGGAGAAGGGTTT
ACTCACTCCCAAGAGCAACGTCTGGAGCTTTGGAGTCGTGTTACTGGAGCTAATAACTGGAAGGAA
GAACCTTGATCCCAATTATTCCAAAGAAGAACGCAATATTGTCAACTGGAGTAGGCCTTTCCTTAC
TGATGACAGTCGCTTGTCTCTTATCATGGACTCCCGTATAAAAGGGCGCTTTCCCACTAAGGCTGC
TCGGATAGTAGCAGACATCATTTTGAAATGCCTGCGTAAGGATCCATCGGAGAGGCCTACCATGAG
GGATGTTGTGGAGGCCCTAGCAAGAGTCCAGGAAATAAAGGTTCCGTGCAGATATCCTCTGCAAGA
ACCTTCTGCTGCACCAAGAAAAGTGATGTTGAAATCTACATCCCTCAATGGGATTGTGCCTCACCA
TCCTGTCATAACCTTCTCTCCATCGCCGCCTTCACATAACCAGCACTTGATTTCACCAAGGTCGTC
GACATCTGCCTTGTTTCATCCAAGGGCATGCTCTTCTACCCTGGACGATCCGAGTGTAAGTTCTAT
AAAGAAAACACCACCTATTATGCGAAGGTCTAGCGTCGAAGGCTTTTGATTGTCCATATTGTTCCT
TTATTTCCTTTTCTTGGATTTATTTGTTTCTTTGTTAGTAGTGTAGCGGTTTTTAGCTGTGTTCTG
TATTGTAAGACAAGTGGCCTCATATGTAGTGCCCTTGGGTCTGGAGTAAAGCAGAACAGGATGAAT
TGTAAATAGGACCAGGTTGTAGATACCTTTTTTTGTGCTCGAATTGGGTGGAAGCAGCGTGCCGA
GTGGGCCTTGTAACCTCATAATGCAATAAACTCCGTTTCCCAGTTTCTGTAAAAAAAAA

**SEQ ID NO: 224, DNA - *Lactuca sativa***
ACCGACTGATGGCCGCTAGTCGGGGAAGAACACGAAGAATCCAAAACCCGTGGCAGCGGCGGAGGC
GGAGGCGGGTTGCCGCCTGTCCCGCAGCCGCTGCCGCTTCCTGCACCGCATTCACGCCAACTTCGA
AAACCATCGCGGAATGCTTTTAAAGATTGAGTGGCGGTGAGTTGGGCATGCTGCCAGCCTCTTAAT
ACTTCCGGACCCTCTGCCACTTCCGCCATCGGGAACCACCCATCTTCCGCCAATGATTCCGGCATC
TTTACCGACATCTGGAACACTTAAGAACTTCACATATTGAAGAAATCGCAGCTGCTTGCCACAACA
TTTTCACCCTGACAGATGCGTGTCTGAAGGTCTTTCTTCTGTTATGTATAGAGCTTCTTTTGGAGA
AGATACTTCTAATTTAAAGAATCTTCAAGCCACTGTTACCAGTCTCCATCCTTCAACTCAGGGTTT
GAAGGAATTTGTGAGTGAAGTGAACACGCTAGCATCATTGCAACACCCTTATCTCTGTAAATAGAT
TGGTTTCCATGCGCGTGAGGGATCTGATAGAAGAATGTTGGTTTACGAGAGGCTTTTTCATGGAAG
CTTAGATCGGCTTTTATATGGTACAACAGATGGCCCACCTATTGATTGCAATGCAAGAATGAAAGT
CGCACTCTGTGCTGCTCAAGGGCTTACTTTTTTGCATGAGGAAGGACCATTTCAGGCGATGTTTCA
TGAATTTTCCACTGCTAATATACAAATTGATAAAGATTTTAG

**SEQ ID NO: 225, DNA - *Gossypium* spp.**
CTTTTTCCTCTCAAACCTCTCTCACACTGATAAACTTTTCTCTCCATTACCTTTCTTTGACTTGCA
AAGTTGTTCTCCTCAAACTAACCAAACAAGGGATTCTGGGTTTTCAGCTTTCTCTGTTTTCCTACT
CTCTTTCGCCTTTGCATGACCTGCTTACATAGAAACATAAAAGTCCGAAATACAATTTAGGTGGTT
TGTATATAAGATGGGGTGTTTTACAGTTTTGAGAAGCAACAAGAAAAAGTCCAAACAGTCAGTTTT
TGTTAAACACATTGCTCATAAAGAGCATATTCCTACCATGCTGCCTGAGCCCCAAATTCAGACACG
ATCACTGCAATCTGCACCCCCAAGTTTTATAACCAGAGTAAAACCAATTCAATCTAATAACAAGGC
AAGCTGCAATAGGACACGTGCATTATCTGCTCCGTCAAGTCTTGATGCTGCTGAGCAAGATGACCT
TGCGTCAGTTGAATTTGAAGAACAAGAAGAGTTGAAGAGTCGTGTTGGTTTAGTCAAGGAACAGAA
GTCATCAAGTCCACAGCCTCTTCCACTTCCATCTCCCCACAGTACTGCGCTGAAGACAATGGGAAG
TTTTAAAGCAGGGAATGTTAGTGGCCCTCTTTTTGCTTCGGGACCATTACCCCTGCCTCCCTCTGG
AACACTACGTAACTTCGCCTACGAAGAAATTGCAGCTGCTTGCCATCATTTCTCTTCTGATCGATG
CACATCTGAGGGTCTATCTTCTGTTATGTACAAGGCATCCTTCGGAGATGACACATCAAGTTCAAA
GAAGTTTGAAGCTACTGTTACTCGCCTTCACCCATCCACTCAGGGTTTAAGGGAATTTATAAACGA
AGTAAACACTCTTGCATCATTGCAACATCCAAATCTCTGTAAATTGCTTGGGTACCATGCACGTGA

## FIGURE 25 (continued)

TAATTCAGAACAACGAATGTTGGTCTATGAGAGGTTATTTCATGGAAGCTTAGACCGGCTTTTATA
CGGGAGATCGGATGGACCGCCACTTGATTGGAATACTCGCATGAAAATTGCTTTATGTTCTGCACA
GGGTCTTACTTTCTTGCATGAGGAAGGACCATTTCAGGCAATGTACAATGAATTTTCGACTGCCAA
CATACAGATCGACAAGGATTTCAGTGCAAAGCTCTCAGGATATGGGTGCGTTGGTCATATCCCAGA
GACAGAAGAGATCTCCAGTAATTCAGTTGCTGTGGCAAATATATCCGTAGAGACTCTGGAGAGAGG
GCGACTAACTCCAAAGAGCAATGTTTGGAGTTTTGGGATCATTCTACTTGAATTACTCACTGGCCG
GAAGAACCTTGACAACCGTTATCCCAAGGAAGAGAGGAACCTAGTGAAGTGGAGCCGGCCATTCCT
AGCCGACAATTGTAGATTGTCACTCATCATGGATCCTCAGCTCAAAGGTCGCTTTCCTATGAAAGC
TGCCCGTACGGTGGCTGACATTGCACAAAGGTGTCTCCAGATGGACCCATCAGAGAGGCCAACCAT
GAGAACCATCGTTGAGCATCTCAAAATCATCCAAGACCTGAAATACTCTTGTCGGTTTCCTCTGCA
AGATCCAGCAGCAATTGCTGGAAAACAGATGTCAAGGTCACCGAGTCTCAACG

**SEQ ID NO: 226, DNA - *Oryza sativa***
AATCCGAAAAGTTTCTGCACCGTTTTCACCCCCTAACTAACAATATAGGGAACGTGTGCTAAATAT
AAAATGAGACCTTATATATGTAGCGCTGATAACTAGAACTATGCAAGAAAAACTCATCCACCTACT
TTAGTGGCAATCGGGCTAAATAAAAAAGAGTCGCTACACTAGTTTCGTTTTCCTTAGTAATTAAGT
GGGAAAATGAAATCATTATTGCTTAGAATATACGTTCACATCTCTGTCATGAAGTTAAATTATTCG
AGGTAGCCATAATTGTCATCAAACTCTTCTTGAATAAAAAAATCTTTCTAGCTGAACTCAATGGGT
AAAGAGAGAGATTTTTTTAAAAAAATAGAATGAAGATATTCTGAACGTATTGGCAAAGATTTAAA
CATATAATTATATAATTTTATAGTTTGTGCATTCGTCATATCGCACATCATTAAGGACATGTCTTA
CTCCATCCCAATTTTTATTTAGTAATTAAAGACAATTGACTTATTTTTATTATTTATCTTTTTTCG
ATTAGATGCAAGGTACTTACGCACACACTTTGTGCTCATGTGCATGTGTGAGTGCACCTCCTCAAT
ACACGTTCAACTAGCAACACATCTCTAATATCACTCGCCTATTTAATACATTTAGGTAGCAATATC
TGAATTCAAGCACTCCACCATCACCAGACCACTTTTAATAATATCTAAAATACAAAAAATAATTTT
ACAGAATAGCATGAAAAGTATGAAACGAACTATTTAGGTTTTTCACATACAAAAAAAAAAAGAATT
TTGCTCGTGCGCGAGCGCCAATCTCCCATATTGGGCACACAGGCAACAACAGAGTGGCTGCCCACA
GAACAACCCACAAAAAACGATGATCTAACGGAGGACAGCAAGTCCGCAACAACCTTTTAACAGCAG
GCTTTGCGGCCAGGAGAGAGGAGGAGAGGCAAAGAAAACCAAGCATCCTCCTCCTCCCATCTATAA
ATTCCTCCCCCCTTTTCCCCTCTCTATATAGGAGGCATCCAAGCCAAGAAGAGGGAGAGCACCAAG
GACACGCGACTAGCAGAAGCCGAGCGACCGCCTTCTTCGATCCATATCTTCCGGTCGAGTTCTTGG
TCGATCTCTTCCCTCCTCCACCTCCTCCTCACAGGGTATGTGCCCTTCGGTTGTTCTTGGATTTAT
TGTTCTAGGTTGTGTAGTACGGGCGTTGATGTTAGGAAAGGGGATCTGTATCTGTGATGATTCCTG
TTCTTGGATTTGGGATAGAGGGGTTCTTGATGTTGCATGTTATCGGTTCGGTTTGATTAGTAGTAT
GGTTTTCAATCGTCTGGAGAGCTCTATGGAAATGAAATGGTTTAGGGTACGGAATCTTGCGATTTT
GTGAGTACCTTTTGTTTGAGGTAAAATCAGAGCACCGGTGATTTTGCTTGGTGTAATAAAAGTACG
GTTGTTTGGTCCTCGATTCTGGTAGTGATGCTTCTCGATTTGACGAAGCTATCCTTTGTTTATTCC
CTATTGAACAAAAATAATCCAACTTTGAAGACGGTCCCGTTGATGAGATTGAATGATTGATTCTTA
AGCCTGTCCAAAATTTCGCAGCTGGCTTGTTTAGATACAGTAGTCCCCATCACGAAATTCATGGAA
ACAGTTATAATCCTCAGGAACAGGGGATTCCCTGTTCTTCCGATTTGCTTTAGTCCCAGAATTTTT
TTTCCCAAATATCTTAAAAAGTCACTTTCTGGTTCAGTTCAATGAATTGATTGCTACAAATAATGC
TTTTATAGCGTTATCCTAGCTGTAGTTCAGTTAATAGGTAATACCCCTATAGTTTAGTCAGGAGAA
GAACTTATCCGATTTCTGATCTCCATTTTTAATTATATGAAATGAACTGTAGCATAAGCAGTATTC
ATTTGGATTATTTTTTTTATTAGCTCTCACCCCTTCATTATTCTGAGCTGAAAGTCTGGCATGAAC
TGTCCTCAATTTTGTTTTCAAATTCACATCGATTATCTATGCATTATCCTCTTGTATCTACCTGTA
GAAGTTTCTTTTTGGTTATTCCTTGACTGCTTGATTACAGAAAGAAATTTATGAAGCTGTAATCGG
GATAGTTATACTGCTTGTTCTTATGATTCATTTCCTTTGTGCAGTTCTTGGTGTAGCTTGCCACTT
TCACCAGCAAAGTTC

## FIGURE 25 (continued)

**SEQ ID NO: 227, DNA - Artificial sequence**
GGGGACAAGTTTGTACAAAAAAGCAGGCTTAAACAATGATGGGTTGCTTCACTGTC

**SEQ ID NO: 228, DNA - Artificial sequence**
GGGGACCACTTTGTACAAGAAAGCTGGGTATGGACAATCAAAAACCCTCA

**SEQ ID NO: 229, *Arabidopsis thaliana* - HAT4 - DNA**
CTTCAACTGCTGCGTCTAGCTGTCCTTCCTTCTTCATTTGCTTCTTCTTCTCTAGCTCAGCGCGGA
TCGCTGCAGTAGTACCCTGACGTAGCCTTTCTTCTTCCTTTACTTTCTCATCTTCTATCTCTCAAA
AGAAAAGCAGACAACTTTATTTGCAAAAACAGAGTTTTTTTTTCTTATCTTGAGAAAGTTCAACAG
AAGATGATGTTCGAGAAAGACGATCTGGGTCTAAGCTTAGGCTTGAATTTTCCAAAGAAACAGATC
AATCTCAAATCAAATCCATCTGTTTCTGTTACTCCTTCTTCTTCTTCTTTTGGATTATTCAGAAGA
TCTTCATGGAACGAGAGTTTTACTTCTTCAGTTCCAAACTCAGATTCGTCACAAAAAGAAACAAGA
ACTTTCATCCGAGGAATCGACGTGAACAGACCACCGTCTACAGCGGAATACGGCGACGAAGACGCT
GGAGTATCTTCACCTAACAGTACAGTCTCAAGCTCTACAGGGAAAAGAAGCGAGAGAGAAGAAGAC
ACAGATCCACAAGGCTCAAGAGGAATCAGTGACGATGAAGATGGTGATAACTCCAGGAAAAAGCTT
AGACTTTCCAAAGATCAATCTGCTATTCTTGAAGAGACCTTCAAAGATCACAGTACTCTCAATCCG
AAGCAGAAGCAAGCATTGGCTAAACAATTAGGGTTACGAGCAAGACAAGTGGAAGTTTGGTTTCAG
AACAGACGAGCAAGAACAAAGCTGAAGCAAACGGAGGTAGACTGCGAGTTCTTACGGAGATGCTGC
GAGAATCTAACGGAAGAGAACCGTCGGCTACAAAAAGAAGTAACGGAATTGAGAGTACTTAAGCTC
TCTCCTCAGTTCTACATGCACATGAGCCCACCCACTACTTTGACCATGTGCCCTTCATGTGAACAC
GTGTCGGTCCCGCCACCACAACCTCAGGCTGCTACGTCAGCGCACCACCGGTCGTTGCCGGTCAAT
GCGTGGGCTCCTGCTACGAGGATATCTCACGGCTTGACTTTTGACGCTCTTCGTCCTAGGTCCTAA
GTCTTTTTACTTGCAACCAAAGGGCATTTTGGTCGTTTTTTAAGTTTCATGGACCAGATATGCATG
TAGTTGTTAACATGTATGTATTTCTTAGAAAGAAAGAAAAACAGATTAATATT

**SEQ ID NO: 230, *Arabidopsis thaliana* - HAT4 - protein**
MMFEKDDLGLSLGLNFPKKQINLKSNPSVSVTPSSSSFGLFRRSSWNESFTSSVPNSDSSQKETRT
FIRGIDVNRPPSTAEYGDEDAGVSSPNSTVSSSTGKRSEREEDTDPQGSRGISDDEDGDNSRKKLR
LSKDQSAILEETFKDHSTLNPKQKQALAKQLGLRARQVEVWFQNRRARTKLKQTEVDCEFLRRCCE
NLTEENRRLQKEVTELRALKLSPQFYMHMSPPTTLTMCPSCEHVSVPPPQPQAATSAHHRSLPVNA
WAPATRISHGLTFDALRPRS

**SEQ ID NO: 231, *Arabidopsis thaliana* - LUMINIDEPENDENS - DNA**
GACAATCCGAAATTAGGTAACCATGTTCATTGAATTTATCGTTTCAACACATTCGAATCACTGAGT
CCTACGTTTCAGTTTAATTCCCTGATGCACATTTTGAATTCGGTTTGCGTATTGGTCTTCGAGTTT
TCCGTCGGCGGCCATGGACGCGTTCAAGGAGGAGATAGAAATCGGGAGCTCGGTGGAGTCTTTAAT
GGAGCTATTGGATTCGCAGAAGGTGCTTTTTCATAGCCAGATCGATCAGCTCCAAGATGTCGTCGT
TGCGCAATGCAAACTTACCGGCGTTAATCCCCTTGCGCAAGAAATGGCTGCTGGTGCTTTGTCCAT
TAAAATTGGAAAGCGGCCAAGAGACTTGTTGAATCCTAAGGCTGTTAAGTATCTACAAGCAGTTTT
CGCAATTAAAGATGCTATTAGTAAGAGGGAATCTCGGGAGATAAGTGCTTTATTTGGCATCACAGT
CGCCCAGGTTCGAGAATTTTTTGTTACTCAAAAGACAAGAGTGAGGAAACAGGTGAGGCTTTCAAG
GGAGAAAGTAGTTATGTCCAATACGCATGCTTTACAAGATGATGGTGTTCCGGAAAATAACAATGC
CACAAATCATGTTGAACCCGTTCCCTTGAACTCTATACATCCGGAGGCATGTTCTATAAGCTGGGG
TGAAGGTGAAACAGTGGCACTTATTCCACCTGAAGATATTCCACCTGACATCAGCGATTCAGACAA
ATACTTTGTTGAGAATATATTTTCTCTGCTGCGTAAAGAGGAAACATTTTCAGGCCAGGTGAAACT
AATGGAGTGGATCATGCAGATACAAGATGCTTCTGTGCTGATCTGGTTTTTATCAAAAGGAGGGGT
TTTGATACTTACAACATGGTTAAGTCAAGCTGCTAGTGAAGAGCAAACAAGTGTCTTACTTCTTAT

**FIGURE 25 (continued)**

CCTGAAGGTTCTTTGTCATTTACCTCTCCACAAAGCATCTCCTGAAAATATGTCTGCCATATTACA
AAGCGTTAATGGACTTCGTTTCTATAGGATATCAGACATATCAAACAGGGCAAAAGGTTTGTTATC
AAGGTGGACCAAGTTATTTGCGAAAATCCAAGCTATGAAGAAACAAAATCGTAACAGTTCGCAAAT
TGATTCGCAGAGTCAATTGCTTCTGAAACAGAGTATTGCTGAAATCATGGGTGATAGTAGCAATCC
TGAAGATATTCTTAGTCTCTCAAATGGAAAGTCAGAGAATGTCAGGAGGATTGAATCGTCACAGGG
TCCAAAACTGTTGCTTACTTCTGCAGATGATTCCACCAAGAAACACATGCTTGGTTCAAATCCATC
GTATAACAAAGAACGCAGGAAAGTACAGATGGTGGAACAACCAGGCCAAAAAGCTGCTGGAAAGAG
TCCGCAGACAGTAAGAATAGGAACTTCAGGTCGAAGCCGCCCAATGTCTGCTGATGATATTCAGAA
AGCAAAGATGCGTGCCCTTTATATGCAGAGCAAGAACAGTAAAAAGGATCCTTTACCAAGTGCCAT
TGGTGATTCGAAAATCGTTGCTCCTGAGAAGCCCTTGGCTCTTCATTCAGCCAAGGATTCTCCACC
TATTCAGAACAATGAAGCTAAGACTGAAGACACACCTGTACTCTCGACTGTTCAGCCCGTCAATGG
ATTTTCAACTATTCAGCCCGTCAATGGACCTTCAGCTGTTCAGCCCGTCAATGGACCTTTGGCTGT
TCAGCCTGTCAATGGACCTTCGGCTCTTCAGCCCGTCAATGGACCTTCGGCCGTAATTGTCCCGGT
ACAAGCTGATGAAATTAAAAAACCTTCAACACCTCCTAAAAGCATTTCTAGTAAGGTGGGAGTTAT
GATGAAAATGAGTTCACAAACTATTCTCAAGAATTGCAAGAGAAAACAGATTGATTGGCATGTACC
ACCAGGAATGGAACTTGACGAACTCTGGAGAGTAGCCGCTGGTGGTAATAGCAAGGAGGCTGATGT
TCAGAGAAACAGAAACCGGCGAGAAAGAGAAACAACATATCAGTCTCTTCAAACTATACCGTTGAA
CCCTAAAGAACCATGGGATAGGGAAATGGACTATGATGACAGTTTGACCCCTGAAATTCCATCTCA
ACAGCCACCAGAAGAAAGTTTAACGGAACCACAGGATTCACTTGATGAACGAAGAATTGCTGCTGG
TGCTGCCACAACCTCTTCATCTCTAAGCAGTCCTGAACCTGATCTCGAGTTATTAGCTGCGTTACT
TAAGAACCCAGATCTTGTTTATGCACTAACTTCGGGAAAACCCAGTAATTTAGCCGGCCAAGATAT
GGTAAAACTGCTTGATGTGATTAAGACTGGTGCACCAAACTCAAGCAGTAGCTCAAATAAACAGGT
TGAAGAAAGGGTCGAAGTTTCCCTTCCATCTCCCACTCCATCAACTAATCCTGGAATGAGTGGATG
GGGACAAGAAGGGATTCGGAATCCATTTTCAAGGCAAAACCAAGTTGGTACTGCAGTTGCTAGATC
GGGTACACAGCTTCGTGTTGGTTCAATGCAATGGCATCAAACAAATGAACAATCAATCCCACGACA
TGCTCCATCAGCATACAGTAACTCGATCACATTGGCTCACACAGAAAGAGAACAGCAACAATATAT
GCAACCAAAACTTCATCACAATTTACATTTTCAACAACAACAACAACAACCAATCTCAACAACCTC
GTATGCAGTTAGGGAACCAGTAGGACAAATGGGAACAGGTACATCGAGTTCATGGAGGAGTCAGCA
GAGTCAGAACAGTTACTACTCACATCAAGAAAACGAGATTGCATCGGCTTCACAAGTTACTTCATA
CCAAGGGAATAGCCAGTACATGAGTAGCAATCCAGGATATGAATCATGGAGTCCTGATAATAGCCC
AAGTAGGAACCAGCTTAACATGAGGGGACAACAACAACAAGCATCAAGGAAACATGATTCTTCTAC
TCATCCATATTGGAACCAAAACAAAAGATGGCGTTAAACCATATATAAGACTATGGTTCTTTGGTC
CTCAATATTTGCTTGTCATGGTTGAATTAGCATTATACTCTGTTCAGACACAGATTTTCATTTTTA
ACTCACAACAGATTCATCAGAGATAATAAACTAAAAGAAAGAATGAATGGATAAAGTTATTACTGT
CTC

**SEQ ID NO: 232, *Arabidopsis thaliana* - LUMINIDEPENDENS - protein**
MDAFKEEIEIGSSVESLMELLDSQKVLFHSQIDQLQDVVVAQCKLTGVNPLAQEMAAGALSIKIGK
RPRDLLNPKAVKYLQAVFAIKDAISKRESREISALFGITVAQVREFFVTQKTRVRKQVRLSREKVV
MSNTHALQDDGVPENNNATNHVEPVPLNSIHPEACSISWGEGETVALIPPEDIPPDISDSDKYFVE
NIFSLLRKEETFSGQVKLMEWIMQIQDASVLIWFLSKGGVLILTTWLSQAASEEQTSVLLLILKVL
CHLPLHKASPENMSAILQSVNGLRFYRISDISNRAKGLLSRWTKLFAKIQAMKKQNRNSSQIDSQS
QLLLKQSIAEIMGDSSNPEDILSLSNGKSENVRRIESSQGPKLLLTSADDSTKKHMLGSNPSYNKE
RRKVQMVEQPGQKAAGKSPQTVRIGTSGRSRPMSADDIQKAKMRALYMQSKNSKKDPLPSAIGDSK
IVAPEKPLALHSAKDSPPIQNNEAKTEDTPVLSTVQPVNGFSTIQPVNGPSAVQPVNGPLAVQPVN
GPSALQPVNGPSAVIVPVQADEIKKPSTPPKSISSKVGVMMKMSSQTILKNCKRKQIDWHVPPGME
LDELWRVAAGGNSKEADVQRNRNRRERETTYQSLQTIPLNPKEPWDREMDYDDSLTPEIPSQQPPE
ESLTEPQDSLDERRIAAGAATTSSSLSSPEPDLELLAALLKNPDLVYALTSGKPSNLAGQDMVKLL

## FIGURE 25 (continued)

DVIKTGAPNSSSSSNKQVEERVEVSLPSPTPSTNPGMSGWGQEGIRNPFSRQNQVGTAVARSGTQL
RVGSMQWHQTNEQSIPRHAPSAYSNSITLAHTEREQQQYMQPKLHHNLHFQQQQQQPISTTSYAVR
EPVGQMGTGTSSSWRSQQSQNSYYSHQENEIASASQVTSYQGNSQYMSSNPGYESWSPDNSPSRNQ
LNMRGQQQQASRKHDSSTHPYWNQNKRWR

**SEQ ID NO: 233, *Oryza sativa* - DNA**
ATGGAGCAGCAGCTTCCTCTTCTTGCACCTGACTCTAAGGCTGCCACGTCGTCCCCCTTGTGCCTC
ACCTTGGACAACCCAACCTCCACATCCACCTCGCCGGCGGTGCCGTCGTCGGCGCCGCCGCCGGCA
GCCGCCTTGGAACCTTCTAGACAATCTTTCCATGAGAGGGAAACGGATGCAATCAAAGCCAAGATC
ATGTCGCACCCCCTCTACCCGGCTCTCCTCAGAGCCTTCATAGATTGCCAGAAGGTCGGAGCTCCG
CCGGAGGTCGTCGGCCGGCTTTCCGCCCTCGCCGGCGAGCTCGACTCGCGTGCAGAAGACAGGTAC
CTTCAAGGGCAGTCGTCAGACCCGGAGCTCGACGAGTTTATGGAAACCTACATTGATATGCTGGTG
AGCTACAGGCAGGAGCTGACAAGACCAATTCAAGAGGCCGACCAGTTCTTCAGAAACATGGAGGCA
CAGATCGACTCGTTTACACTAGAGATGTGCAGTTTCTGA

**SEQ ID NO: 234, *Oryza sativa* - protein**
MEQQLPLLAPDSKAATSSPLCLTLDNPTSTSTSPAVPSSAPPPAAALEPSRQSFHERETDAIKAKI
MSHPLYPALLRAFIDCQKVGAPPEVVGRLSALAGELDSRAEDRYLQGQSSDPELDEFMETYIDMLV
SYRQELTRPIQEADQFFRNMEAQIDSFTLEMCSF

**SEQ ID NO: 235, *Oryza sativa* - Homeobox associated leucine zipper family protein - DNA**
ATGGCTCAGGAGGACGTCGGTCACCTGAGCGACGCCGGCCTGGCGCTGGGCCTGTCCCTCGGCGGG
GGAGGAGGAGGGACGACCGACGCGGCGGCGGCGCACCGTGGCGGCTGCCGGCGGCCGTCGCCGTCG
TCGCAGTGCCCGCCGCTGGAGCCGTCGCTGACCCTGAGCTTGCCCGACGACGCGGCGGCCGGCGCG
GCCGCGACCGCGACCGCGACCGCGTCCGGCGGGGGCGGCCCTGCGCACAGCGTGTCGTCGCTGTCC
GTCGGCGCGGCGGCGGCGGCGGCCGTGAAGAGGGAGCGCGCGGAGGAGGCCGACGGCGAGAGGGTG
TCGTCGACGGCGGCCGGGCGTGACGACGACGACGACGGGAGCACCCGCAAGAAGCTCCGGCTGACC
AAGGAGCAGTCCGCGCTCCTGGAGGACCGCTTCCGGGAGCACAGCACGCTCAACCCGAAGCAGAAA
GTCGCTTTAGCGAAGCAACTGAACCTCAGGCCAAGGCAGGTGGAGGTCTGGTTCCAAAACAGAAGA
GCAAGGACAAAGCTGAAGCAGACGGAGGTGGACTGCGAGTTCCTGAAGCGCTGCTGCGAGACGCTC
ACCGAGGAGAACCGTCGGCTGCAGCGCGAGCTGCAGGAGCTCCGCGCGCTCAAGTTCGCCCCGCCG
CCGCCGTCCTCGGCGGCCCACCAGCCGTCGCCGGCGCCACCGGCGCCGTTCTACATGCAACTCCCG
GCCGCCACGCTCACCATCTGCCCGTCCTGCGAGCGCGTCGGCGGGCCCGCGTCCGCCGCCAAGGTC
GTCGCCGCCGACGGGACCAAGGCCGGCCCCGGCCGGACCACCACCCACCACTTCTTCAACCCCTTC
ACCCACTCCGCCGCCTGCTGA

**SEQ ID NO: 236, *Oryza sativa* - Homeobox associated leucine zipper family protein - protein**
MAQEDVGHLSDAGLALGLSLGGGGGGTTDAAAAHRGGCRRPSPSSQCPPLEPSLTLSLPDDAAAGA
AATATATASGGGGPAHSVSSLSVGAAAAAAVKRERAEEADGERVSSTAAGRDDDDDGSTRKKLRLT
KEQSALLEDRFREHSTLNPKQKVALAKQLNLRPRQVEVWFQNRRARTKLKQTEVDCEFLKRCCETL
TEENRRLQRELQELRALKFAPPPPSSAAHQPSPAPPAPFYMQLPAATLTICPSCERVGGPASAAKV
VAADGTKAGPGRTTTHHFFNPFTHSAAC

**SEQ ID NO: 237, *Oryza sativa* - HAT22 - DNA**
ATGGCGCAAGATGATGAGGACGTGGGTCTAGCTCTGGGCCTCTCCCTGGGCTCCGGCGGCCACAGG
CGGCAGAGAGAGAAAGTAGAGACGAAGCGCCCGTCGTCGGCGGCGGCGTCCCTGCTGACGCTGAGGCTC
CCGGCAGAGAGAGCGGGGGGCAGCCGCAGGTGGTGGTGAAGAGGGAGGTGGTGCGGGCGGAAGAGGAG

**FIGURE 25 (continued)**

GAGTACGAATACGAGTACGAGAGGGCGCTCTACTCGTCGTCGGCTGCGGCGGCGGACGACGACGAG
GGCTGCAACAGCCGGAAGAAGCTGAGGCTGAGCAAGGAGCAGTCGGCTCTGCTGGAGGATCGCTTC
AAGGAGCATAGCACTCTCAACCCTAAGCAAAAGGTTGCTTTGGCGAAGCAGCTAAACCTGAGGCCA
AGGCAAGTTGAGGTGTGGTTCCAAAACAGAAGAGCCAGGACGAAGCTGAAGCAGACGGAGGTGGAT
TGCGAGCTTCTGAAGCGCTGCTGCGAGACGCTGACGGAGGAGAACCGACGCCTCCATCGTGAGCTC
CAGCAGCTCAGGGCTCTGACCCACTCCACCGCCGCCGGCTTCTTCATGGCCACCACCCTCCCCGTC
CCCGCCGCCACGCTCTCCATCTGCCCCTCCTGCGAGCGCCTCGCCACCGCCGCCGCCGCCGGAGCT
TCCCCCACCGCCGCCGCCGACCGCACCAACAAGCCCACCGCCCCGCACTTGTTCAGCCCTTTCGCC
AAGTCCGCCGCCTGCTAG

**SEQ ID NO: 238, *Oryza sativa* - HAT22 - protein**
MAQDDEDVGLALGLSLGSGGHRRQRESRDEAPSSAAASLLTLRLPAESGGQPQVVVKREVVRAEEE
EYEYEYERALYSSSAAAADDDEGCNSRKKLRLSKEQSALLEDRFKEHSTLNPKQKVALAKQLNLRP
RQVEVWFQNRRARTKLKQTEVDCELLKRCCETLTEENRRLHRELQQLRALTHSTAAGFFMATTLPV
PAATLSICPSCERLATAAAAGASPTAAADRTNKPTAPHLFSPFAKSAAC

**SEQ ID NO: 239, *Oryza sativa* - HAT14 - DNA**
ATGGAGCTGGGGCTGAGCTTGGGGGATGCGGTGACCGTGGCGGATGGCGGGAGGCTGGAGCTGGTT
CTTGGGCTCGGGGTTGGGGTTGGGGCTGGGGTGAGGAGAGGAGAGGAGGAGGAGAGGGGGAGGAGG
GAGGATGTGGTGGGAGCTGGGAGGTGGGCGGCGATGGCGGCGGCCTCGCCGGAGCCGTCGGTGCGG
CTCAGCCTCGTGTCGAGCCTCGGGCTCCACTGGCCTTCCGAGACCGGGCGTTCGGAGGCGGCGGCG
CGTGGGTTCGACGTGAACCGGGCGCCGTCGGTGGCGGCGGGCGCGCCGGGGATGGAGGACGACGAG
GAGGGCCCGGGCGCCGCGCCGGCGTTGTCGTCGTCGCCCAACGACAGCGGGGGATCCTTCCCGCTG
GACCTCTCCGGGCAAGGCCTCCGTGGCCACGCCGAGGCGGCGGCGCAGGGTGGCGGCGGCGGCGGC
GGCGGCGAGCGGTCGTCCTCGCGCGCGAGCGACGACGACGAGGGCGCGTCCGCGCGCAAGAAGCTG
CGACTCTCCAAGGAGCAGTCGGCGTTCCTCGAGGAGAGCTTCAAGGAGCACAGCACGCTGAATCCC
AAGCAGAAGGTGGCGCTGGCGAAGCAGCTCAACCTCCGGCCGCGGCAAGTGGAGGTCTGGTTCCAG
AACCGCCGAGCCAGGACGAAGCTGAAGCAGACGGAGGTGGACTGCGAGTACCTGAAGCGCTGCTGC
GAGACGCTCACCGAGGAGAACCGGCGGCTGCACAAGGAGCTCGCCGAGCTGCGCGCGCTCAAGACG
GCGCGCCCCTTCTACATGCACCTCCCGGCCACGACCCTCTCCATGTGCCCCTCCTGCGAGCGTGTC
GCCTCCAACCCGGCCACCGCTTCGACCTCCGCCCCCGCCGCGGCCACGTCCCCGGCGGCGGCGCCG
ACCGCGGCCGCAAGAACCGCCGTCGCGTCGCCCGAGCCGCACCGGCCGTCGTCCTTCGCCGCGCTG
TTCGCGGCACCCCTCGGCTTCCCGCTGACCGCCGCCCAGCCGCGGCCGCCGCCGCCGGCGAGCAAC
TGCCTGTAA

**SEQ ID NO: 240, *Oryza sativa* - HAT14 - protein**
MELGLSLGDAVTVADGGRLELVLGLGVGVGAGVRRGEEEERGRREDVVGAGRWAAMAAASPEPSVR
LSLVSSLGLHWPSETGRSEAAARGFDVNRAPSVAAGAPGMEDDEEGPGAAPALSSSPNDSGGSFPL
DLSGQGLRGHAEAAAQGGGGGGGGERSSSRASDDDEGASARKKLRLSKEQSAFLEESFKEHSTLNP
KQKVALAKQLNLRPRQVEVWFQNRRARTKLKQTEVDCEYLKRCCETLTEENRRLHKELAELRALKT
ARPFYMHLPATTLSMCPSCERVASNPATASTSAPAAATSPAAAPTAAARTAVASPEPHRPSSFAAL
FAAPLGFPLTAAQPRPPPPASNCL

**SEQ ID NO: 241, *Oryza sativa* - Homeobox associated leucine zipper family protein - DNA**
ATGGAGCTGGGGTTGAGCTTGGGGGAGGCTATGGCGGATGCTGGGAGGGAGCTGGTTCTTGGGCTT
GGGATGGGGAGGAGGGAGGAGGCGGCGGAGGCGGGGAGGAGGGATCATGAGGTGAGGAGGGAGCTG
GAGTTCGGGTCGATGTCGAGCAGGTGCGGTGGTTCTTCGCCGGAGCCGACGGTGCGGCTCACGCTT

## FIGURE 25 (continued)

CTGCCCATGGTGCCCGGCCTTGGCCTCCCATGGCCGCCGCCGCCGCCGCCGTCGTCCGAGAGCAGG
CATTTGGAGGCGTCGACGCGGGGATTCGACGTGAACCGCCCGCCGTCGTCCGGCGGCGGCGGCGGC
GGCGGCGGCGCGGAGGAGGAGCAGGACGACGTGGCCGGGGCGGCACTCTCGTCGTCCCCCAACAAC
AGCGCCGGATCCTTCCCGATGGATGACTTCTCCGGGCACGGCCTCGGCGGCAACGACGCGGCCCCT
GGCGGTGGCGGCGGCGACCGCTCGTGCTCCCGCGCCAGCGACGAGGACGACGGCGGCTCCGCGCGC
AAGAAGCTCCGCCTCTCCAAGGAGCAGTCCGCGTTCCTCGAGGAGAGCTTCAAGGAGCACAGCACC
CTAAACCCCAAGCAGAAGCTGGCGCTGGCGAAGCAGCTCAACCTCCGGCCGCGCCAGGTGGAGGTG
TGGTTCCAGAACCGCCGCGCCAGGACGAAGCTGAAGCAGACGGAGGTGGACTGCGAGTACCTGAAG
CGGTGCTGCGAGACGCTGACGGAGGAGAACCGGCGGCTGCAGAAGGAGCTCGCCGAGCTCCGGGCG
CTCAAGACGGTGCACCCCTTCTACATGCACCTCCCGGCGACGACACTCTCCATGTGCCCCTCCTGC
GAGCGCGTCGCCTCCAACTCCGCCCCGGCCACCGCCTCCTCCGCCGCAACATCGTCGACGGCCGCG
CCGCCCGCGGCACCCTCATCCGGCGGCATTGCGGCCACCTCCTCCTCCGCCGCCGCCGCCGCGGCG
CCGGACCACAGGCCGTCGTCGTTCGCCGCGCTGTTCTCGTCGCCGCGTGGCTTCCCGCTATCCGTA
GCCCCGCAGGCGCAGCCGCCGACGAGCTCGTGA

**SEQ ID NO: 242, *Oryza sativa* - Homeobox associated leucine zipper family protein - protein**
MELGLSLGEAMADAGRELVLGLGMGRREEAAEAGRRDHEVRRELEFGSMSSRCGGSSPEPTVRLTL
LPMVPGLGLPWPPPPPPSSESRHLEASTRGFDVNRPPSSGGGGGGGGAEEEQDDVAGAALSSSPNN
SAGSFPMDDFSGHGLGGNDAAPGGGGGDRSCSRASDEDDGGSARKKLRLSKEQSAFLEESFKEHST
LNPKQKLALAKQLNLRPRQVEVWFQNRRARTKLKQTEVDCEYLKRCCETLTEENRRLQKELAELRA
LKTVHPFYMHLPATTLSMCPSCERVASNSAPATASSAATSSTAAPPAAPSSGGIAATSSSAAAAAA
PDHRPSSFAALFSSPRGFPLSVAPQAQPPTSS

**SEQ ID NO: 243, *Oryza sativa* - HAT3 - DNA**
ATGGAGAGGCAAGGCTTGGATCTTGGCCTGAGCCTCGGGCTAGGCTTGACGACGGCGGCGACATGG
CCGGCTGCTGGGTTCTGTCTGAACTCCGGCATGGCGGAGCAGGAAGTGATCAGGCGTGATGATGTG
GTTGCGGCGACGGCGGCGGAGGATGAGAGGTTCGCGTGCTCACCCGGCAGCCCGGTGTCGAGCGGC
AGCGGGAAGCGAGGCAGCGGCAGCGGCAGCGGCGACGAGGTCGACGACGCCGGCTGCGACGTCGGC
GGCGGCGGCGCGCGCAAGAAGCTGCGGTTGTCCAAGGACCAGGCCGCCGTCCTCGAGGAGTGCTTC
AAGACGCACCACACCCTCACTCCGAAGCAGAAGGTGGCGCTGGCGAAGAGCTTGAACCTGCGGCCG
CGGCAGGTGGAGGTGTGGTTCCAGAACCGCCGCGCGAGGACGAAGCTGAAGCAGACGGAGGTGGAC
TGCGAGCACCTCAAGCGGTGGTGCGACCAGCTCGCCGACGACAACCGCCGCCTCCACAAGGAGCTC
GCCGAGCTCAGGGCGCTCAAGGCCACGCCCACACCGCCCGCCGCCGCGCCGCCATTGACCACCCTC
ACAATGTGCCTCTCCTGCAAGCGCGTCGCCAATGCCGGCGTGCCCTCGCCGGCGGCGGCGATATTC
CCCGGCCACCCCCAGTTCTTGTGCGGATTCAGAGATCACGCCGGAGCAGCGTCGTCGTCGTACGGC
GGCGCATCATCTGGACTCGCGAAGGCGGTCAGGGCGGCGAGGTAG

**SEQ ID NO: 244, *Oryza sativa* - HAT3 - protein**
MERQGLDLGLSLGLGLTTAATWPAAGFCLNSGMAEQEVIRRDDVVAATAAEDERFACSPGSPVSSG
SGKRGSGSGSGDEVDDAGCDVGGGGARKKLRLSKDQAAVLEECFKTHHTLTPKQKVALAKSLNLRP
RQVEVWFQNRRARTKLKQTEVDCEHLKRWCDQLADDNRRLHKELAELRALKATPTPPAAAPPLTTL
TMCLSCKRVANAGVPSPAAAIFPGHPQFLCGFRDHAGAASSSYGGASSGLAKAVRAAR

**SEQ ID NO: 245, *Oryza sativa* - SlHDL2 - DNA**
ATGATGGATCTCGGCCTCAGCCTCGGCCTCGGCCTCGCCTCGCAGGGCAGCCTCACCTCCTCCACC
ACCACCACCTCCTCCCCCGGCGCCGGATCATCCTCCCCGTGGGCCGCCGCGCTCAACTCCATCGTC
GGCGACGTGAGGCGGGATCAGGCCGCGGCGCATGCTGCCGCGGCGGTGGGGGTTGGGGTTGGGGGC

## FIGURE 25 (continued)

GAGGAGATGTACCAGGGGAGGGCGTCCACGTCGCCGGACAGCGCGGCGGCGCTGTCGAGCGCGAGC
GGGAAGAGGGAGAGGGAGCTGGAGCGGTCGGGATCCGGGGTTGACGACGACGACGGCGCGGACGGC
GCCGGCGGGCGGAAGAAGCTCAGGCTGTCCAAGGACCAGGCCGCCGTGCTCGAGGAGTGCTTCAAG
ACGCACTCCACTCTCAACCCCAAGCAGAAGGTGGCGCTGGCGAACAGGCTGGGGCTGCGGCCGCGG
CAGGTGGAGGTGTGGTTCCAGAACCGGAGGGCGAGGACGAAGCTGAAGCAGACGGAGGTGGACTGC
GAGTACCTGAAGCGGTGGTGCGAGCGCCTCGCCGACGAGAACAAGCGCCTCGAGAAGGAGCTCGCC
GACCTCAGGGCGCTCAAGGCCGCGCCCTCGCCGGCGTCCGCGTCCGCGATGCAGCCCTCCTCCTCC
GCCGCCGCCACGCTCACCATGTGCCCCTCCTGCCGCCGCGTCGCCACCGCCGGCGCGCCGCACCAG
CCTAACCACCAACAATGCCATCCCAAATCTAACACCACCATCTCCTCCTCCTCCACCGCCGCCGCC
GCCGTCGCCGTCGCCGGCGGCAACGTGCTGCCCAGCCACTGCCAGTTCTTCCCGGCCGCCGCCGCC
GCCGCCGACCGGACAAGCCAGAGCACGTGGAACGCCGCCGCGCCGCTCGTCACCAGAGAGCTCTTC
TAG

**SEQ ID NO: 246, *Oryza sativa* - SlHDL2 - protein**
MMDLGLSLGLGLASQGSLTSSTTTTSSPGAGSSSPWAAALNSIVGDVRRDQAAAHAAAAVGVGVGG
EEMYQGRASTSPDSAAALSSASGKRERELERSGSGVDDDDGADGAGGRKKLRLSKDQAAVLEECFK
THSTLNPKQKVALANRLGLRPRQVEVWFQNRRARTKLKQTEVDCEYLKRWCERLADENKRLEKELA
DLRALKAAPSPASASAMQPSSSAAATLTMCPSCRRVATAGAPHQPNHQQCHPKSNTTISSSSTAAA
AVAVAGGNVLPSHCQFFPAAAAAADRTSQSTWNAAAPLVTRELF

**SEQ ID NO: 247, *Oryza sativa* - HD-ZIP protein - DNA**
ATGGAGATGATGGTTCATGGGAGGAGAGACGAGCAGTATGGCGGGCTCAGGCTCGGGCTTGGGCTT
GGGCTCAGCCTCGGCGTCGCCGGTGGTGCAGCCGACGACGAGCAGCCGCCGCCGCGCCGTGGTGCC
GCCCCGCCGCCGCAGCAGCAGCTGTGCGGCTGGAACGGCGGCGGTCTCTTCTCCTCGTCTTCCTCC
GATCATCGGGGGAGGTCGGCGATGATGGCGTGCCACGACGTCATCGAGATGCCGTTCCTGCGGGGG
ATCGACGTGAACCGTGCGCCGGCGGCAGAGACGACCACGACGACGGCGAGGGGGCCCAGCTGCAGC
GAGGAAGACGAGGAGCCCGGCGCGTCCTCCCCCAACAGCACGCTCTCCAGCCTCAGCGGCAAGCGC
GGCGCACCATCTGCCGCCACCGCCGCCGCCGCCGCCGCCAGCGACGACGAGGACTCCGGCGGCGGA
TCCCGCAAGAAGCTCCGCCTCTCCAAGGACCAAGCCGCCGTCCTCGAGGACACCTTCAAAGAGCAC
AACACCCTCAATCCCAAGCAGAAGGCGGCGCTGGCGAGGCAGCTGAATCTGAAGCCGCGGCAGGTG
GAGGTGTGGTTCCAGAACAGGAGGGCGAGGACGAAGCTGAAGCAGACGGAGGTGGACTGCGAGCTG
CTCAAGCGCTGCTGCGAGACGCTCACCGACGAGAACCGCCGCCTCCACCGCGAGCTCCAGGAGCTC
CGCGCCCTCAAGCTCGCCACCGCCGCCGCCGCGCCGCACCACCTCTACGGCGCCCGCGTCCCGCCG
CCCACCACCCTCACCATGTGCCCCTCCTGCGAGCGCGTCGCCTCCGCAGCCACCACCACCCGCAAC
AACTCCGGCGCCGCCCCCGCGCGGCCGGTGCCCACCCGCCCGTGGCCGCCGGCGGCGGCGCAGAGG
TCGTCGGCGTAG

**SEQ ID NO: 248, *Oryza sativa* - HD-ZIP protein - protein**
MEMMVHGRRDEQYGGLRLGLGLGLSLGVAGGAADDEQPPPRRGAAPPPQQQLCGWNGGGLFSSSSS
DHRGRSAMMACHDVIEMPFLRGIDVNRAPAAETTTTARGPSCSEEDEEPGASSPNSTLSSLSGKR
GAPSAATAAAAAASDDEDSGGGSRKKLRLSKDQAAVLEDTFKEHNTLNPKQKAALARQLNLKPRQV
EVWFQNRRARTKLKQTEVDCELLKRCCETLTDENRRLHRELQELRALKLATAAAAPHHLYGARVPP
PTTLTMCPSCERVASAATTTRNNSGAAPARPVPTRPWPPAAAQRSSA

**SEQ ID NO: 249, *Arabidopsis thaliana* - nucleic acid binding / transcription factor - DNA**
AAAATGGAAAACTCAGACACTGATTCAGAAGTTTTCTTTTGGTTTCAGAACCAAAACCAAAACCAT
AGCCATAAGTTTCCTTCTTCTTGTTTTCCTCCGAGCTCTCACTCTGCTTTTTATGGATCTAGCTCA
ATGATCAACACAGAGACTGCAACTATGGACGAAGAAGACGTATGTGAGAGCTACATGATGCGTGAG

## FIGURE 25 (continued)

ATAACCAAGAAACGGAAGCTAACACCGATCCAATTGCGTTTACTAGAGGAGAGTTTCGAAGAAGAG
AAAAGACTCGAACCAGACAGGAAACTCTGGCTAGCCGAGAAGCTAGGGCTGCAGCCAAGCCAAGTC
GCTGTCTGGTTCCAAAACAGAAGAGCTAGATACAAGACCAAACAGCTCGAACACGACTGTGACTCT
CTTAAAGCTAGCTATGCTAAGCTCAAGACTGACTGGGACATTCTCTTTGTACAAAACCAAACCCTC
AAGAGCAAGGTACAGTTTCTTAATAGATTAACAAGCCACTATTTTCAAGAGTCTGTTCAAAATTTT
GATGACACATTCAAACAGGTCGATCTTCTCAAAGAGAAGCTGAAAATGCAAGAGAATCTTGAAACT
CAGTCTATTGAGAGGAAAAGACTTGGGGAAGAAGGTAGTTCGGTGAAAAGCGATAACACGCAGTAC
AGTGAAGAAGAAGGTTTGGAGAATCAATACAGTTTCCCGGAGCTTGCAGTTCTCGGATTTTATTAT
GATCCAACCTTAACTGCTTCAAATCTAAGACAAGAACCTTTGAAAGTCACGTGTGCGGATCAGATG
ACTCAGATCCAAATATCTGACGTCACAGAACCTGCGAGCTCCGCACATAAAAAAATTGAAGTGACA
CAGCGAAGTTCGAGTATGAGCCGCAAGAGAGATAAACCCTACACAAACCGTCACACACCGGCGCGA
ATTTCGAAACGCCGGCGACCATGGGCGCCTTCGTCATCGGAGCACGATGAGATTATCGACAAACCG
ATCACCAAACCTCCGCCGCCACCGGCGTTGGTAGTTATGGGACTTCCCGCCAACTGTTCAGTTCTG
GAGCTAAAATCTCGATTCGAGATCTACGGTTCAATCTCCCGAATCCGAATCCATAAAGACGGAATT
GGCTCCGTTTCGTATCGAACCGCCGAGTCAGCAGAAGCCGCCATTGCTGGTAGTCACGAGCCTTCT
TTCGGTATCTCCATCGATTCCAAAAAGTTGGAGGTGGTTTGGGCGACGGATCCATTGGTGAAGTGG
AAGGAAGGTGTGACGGCGGGAGAAGGAAAGGAGAGAACGTCGTCGTTTCGTCGAAGCTTTTACGA
CCTGTGATGCCTTTGAGAAAACATGGGAGAAGCAGTAGGTTAGCGTCAGCTATTGTTAATCCGAGA
AGTGATAATACTAAAGGAATTAGTGGAGATGGAGGGATATCATCTCCGGCGACGACAAGTGAAGTT
AAACAGAGAAATATAGTAACCTACGACGATATCGTTTAACACCATTCTACTACAATTATAGTAACT
TTGATTCTTTTACATATTTTGGCAATTTTATGAGTTTAGCTAATCCTGTTGGTAATCTCAGTTGTC
GAATGTTGTGAAATGAGTTGAGTTTTGTTTATTTGTATGATTGTAACTTATAAGAAAGAGAAGGAT
TCCAAAATGGGTAGAAGATTCAAA

**SEQ ID NO: 250, *Arabidopsis thaliana* - nucleic acid binding / transcription factor - protein**

MENSDTDSEVFFWFQNQNQNHSHKFPSSCFPPSSHSAFYGSSSMINTETATMDEEDVCESYMMREI
TKKRKLTPIQLRLLEESFEEEKRLEPDRKLWLAEKLGLQPSQVAVWFQNRRARYKTKQLEHDCDSL
KASYAKLKTDWDILFVQNQTLKSKVQFLNRLTSHYFQESVQNFDDTFKQVDLLKEKLKMQENLETQ
SIERKRLGEEGSSVKSDNTQYSEEEGLENQYSFPELAVLGFYYDPTLTASNLRQEPLKVTCADQMT
QIQISDVTEPASSAHKKIEVTQRSSSMSRKRDKPYTNRHTPARISKRRRPWAPSSSEHDEIIDKPI
TKPPPPPALVVMGLPANCSVLELKSRFEIYGSISRIRIHKDGIGSVSYRTAESAEAAIAGSHEPSF
GISIDSKKLEVVWATDPLVKWKEGVTAGEGKERTSSFSSKLLRPVMPLRKHGRSSRLASAIVNPRS
DNTKGISGDGGISSPATTSEVKQRNIVTYDDIV

**SEQ ID NO: 251, *Arabidopsis thaliana* - HAT14 - DNA**

AAGGCCACAACAGATCTTCTTGTTCCTCTCTCTCAATCTCTCTCTCTAAAACTCTTATTTTCTTCG
TAAGAGATGGAACTAGCGTTGTCTCTAGGCGACAATACTAAGAAACAGTTCTCTTTTATGGAGAAG
AACTCGAAGATTAATAATCCCTCTGTCTCGTCGACATCTACTTCCGAGAAGGATCTTGGGTTCTGC
ATGGCTTTAGATGTTGCTTTTGGTGGTCACAGATCGTTGTCATCCTCTTCGTCTCCGTCGGTAGAG
GATGAGAAGAAGAAACCGGCGCCCAGAGCAAAAAAATCTGACGAATTTAGGGTTTCGTCTTCTGTA
GATCCACCATTACAGCTTCAGCTTCACTTCCCTAATTGGCTCCCTGAGAACAGTAAAGGTCGACAA
GGAGGAAGAATGCCCTTAGGAGCAGCTACGGTTGTGGAGGAGGAAGAGGAGGAGGAGGAAGCGGTG
CCTAGTATGTCAGTATCGCCGCCGGATAGTGTAACGTCGTCGTTTCAATTGGACTTTGGGATTAAA
AGTTATGGTTATGAGAGAAGAAGCAATAAGAGAGATATTGATGATGAAGTGGAGAGATCAGCTTCA
AGAGCCAGCAACGAAGACAACGATGACGAGAATGGATCCACTAGGAAGAAACTTAGACTCTCCAAA
GACCAATCTGCTTTTCTTGAAGACAGCTTCAAAGAACACAGTACCCTTAATCCTGTTCGTGTCCCA
TTCTTTACAGTTTTTATTTATTTAAAATTTGTCTTTCTTGAATTTATTCTATTTTTTTAGTACCAA

FIGURE 25 (continued)

ATTTTAGCCTTAATGCTTTTTTTTTTCTTTCTAAAACATTGCAGAAACAGAAGATTGCATTGGCGA
AGCAGTTGAATCTTCGTCCTCGTCAGGTTGAAGTCTGGTTTCAAAACAGACGAGCCAGGACAAAGC
TGAAGCAAACGGAAGTGGACTGTGAATACCTAAAGAGATGCTGTGAGTCACTAACCGAAGAAAACC
GGAGGCTTCAAAAAGAGGTTAAAGAATTGAGAACCTTGAAGACTTCCACACCCTTTTACATGCAAC
TTCCGGCCACTACTCTCACTATGTGCCCTTCTTGTGAACGTGTTGCCACTTCAGCAGCACAGCCCT
CCACGTCAGCTGCCCACAACCTCTGTTTGTCCACGTCATCATTGATTCCGGTTAAGCCTCGGCCGG
CCAAACAAGTTTCATGAAAGCACCTGCGAAATACAGTTTGAGCAAACGGTCGAGCTAGAGTGGTTT
TAAAAGTTGTCTTCTTGTGTATATATTTATTTTACTTTTCATATTTTATTAGAGACCGCTATTTTG
AAAGACGAATAGATTGATTATCCGGTTAGTGTTTTGTTTTTCTTAGATAGGACCGGATAAAAAACA
GATGGAGCAAAAGGGTTGACATGTTTTATTGAATGATAGAGG

**SEQ ID NO: 252, *Arabidopsis thaliana* - HAT14 - protein**
MELALSLGDNTKKQFSFMEKNSKINNPSVSSTSTSEKDLGFCMALDVAFGGHRSLSSSSSPSVEDE
KKKPAPRAKKSDEFRVSSSVDPPLQLQLHFPNWLPENSKGRQGGRMPLGAATVVEEEEEEEEAVPS
MSVSPPDSVTSSFQLDFGIKSYGYERRSNKRDIDDEVERSASRASNEDNDDENGSTRKKLRLSKDQ
SAFLEDSFKEHSTLNPVRVPFFTVFIYLKFVFLEFILFF

**SEQ ID NO: 253, *Arabidopsis thaliana* - HAT4 - DNA**
TGCCCCCAGCTAGTCACATACTCATGATTGCAAAATCTCTCTCTCTCTGCCTCTCTATATATTA
ACCTTTCTTCTTCCTTTACTTTCTCATCTTCTATCTCTCAAAAGAAAAGCAGACAACTTTATTTGC
AAAAACAGAGTTTTTTTTTCTTATCTTGAGAAAGTTCAACAGAAGATGATGTTCGAGAAAGACGAT
CTGGGTCTAAGCTTAGGCTTGAATTTTCCAAAGAAACAGATCAATCTCAAATCAAATCCATCTGTT
TCTGTTACTCCTTCTTCTTCTTCTTTTGGATTATTCAGAAGATCTTCATGGAACGAGAGTTTTACT
TCTTCAGTTCCAAACTCAGATTCGTCACAAAAAGAAACAAGAACTTTCATCCGAGGAATCGACGTG
AACAGACCACCGTCTACAGCGGAATACGGCGACGAAGACGCTGGAGTATCTTCACCTAACAGTACA
GTCTCAAGCTCTACAGGGAAAAGAAGCGAGAGAGAAGAAGACACAGATCCACAAGGCTCAAGAGGA
ATCAGTGACGATGAAGATGGTGATAACTCCAGGAAAAAGCTTAGACTTTCCAAAGATCAATCTGCT
ATTCTTGAAGAGACCTTCAAAGATCACAGTACTCTCAATCCGAAGCAGAAGCAAGCATTGGCTAAA
CAATTAGGGTTACGAGCAAGACAAGTGGAAGTTTGGTTTCAGAACAGACGAGCAAGAACAAAGCTG
AAGCAAACGGAGGTAGACTGCGAGTTCTTACGGAGATGCTGCGAGAATCTAACGGAAGAGAACCGT
CGGCTACAAAAAGAAGTAACGGAATTGAGAGCACTTAAGCTCTCTCCTCAGTTCTACATGCACATG
AGCCCACCCACTACTTTGACCATGTGCCCTTCATGTGAACACGTGTCGGTCCCGCCACCACAACCT
CAGGCTGCTACGTCAGCGCACCACCGGTCGTTGCCGGTCAATGCGTGGGCTCCTGCGACGAGGATA
TCTCACGGCTTGACTTTTGACGCTCTTCGTCCTAGGTCCTAAGTCTTTTTACTTGCAACCAAAGGG
CATTTTGGTCGTTTTTTAAGTTTCATGGACCAGATATGCATGTAGTTGTTAACATGTATGTATTTT
CTTAGAAAGAAAGAAAAACAGATTAATATTTTTCTAGCTTAAACC

**SEQ ID NO: 254, *Arabidopsis thaliana* - HAT4 - protein**
MMFEKDDLGLSLGLNFPKKQINLKSNPSVSVTPSSSSFGLFRRSSWNESFTSSVPNSDSSQKETRT
FIRGIDVNRPPSTAEYGDEDAGVSSPNSTVSSSTGKRSEREEDTDPQGSRGISDDEDGDNSRKKLR
LSKDQSAILEETFKDHSTLNPKQKQALAKQLGLRARQVEVWFQNRRARTKLKQTEVDCEFLRRCCE
NLTEENRRLQKEVTELRALKLSPQFYMHMSPPTTLTMCPSCEHVSVPPPQPQAATSAHHRSLPVNA
WAPATRISHGLTFDALRPRS

**SEQ ID NO: 255, *Arabidopsis thaliana* - HAT1 - DNA**
ATTTCACATCTCTCTCTCTCTATAAGAACCCTAGAAGAAGGTTTCTCTTGTCCTCCATACACTTAG
CACAACTGATAAATCTTTTGAGGTAAAATCAGCTTTAGATCAAGGTTTTTCTAGTCATCTCTACTC
ATAAAGATCAAAGCTTTTGCTATTCTCATTTTCTACCAAGAGACAATATCATGATGATGGGTAAAG

## FIGURE 25 (continued)

AGGATTTGGGTTTAAGTCTTAGCTTGGGATTTGCACAAAACCATCCTCTCCAGCTAAATCTTAAAC
CCACTTCTTCACCAATGTCCAATCTCCAGATGTTTCCATGGAACCAAACCCTTGTTTCTTCCTCAG
ATCAACAAAAGCAACAGTTTCTTAGGAAAATCGACGTGAACAGCTTGCCAACAACGGTGGATTTGG
AAGAGGAGACAGGAGTTTCGTCTCCAAACAGTACGATCTCGAGCACAGTGAGTGGAAAGAGGAGGA
GTACTGAAAGAGAAGGTACCTCCGGTGGTGGTTGCGGAGATGACCTTGACATCACTCTAGATAGAT
CTTCCTCACGTGGAACCTCCGATGAAGAGGAAGATTACGGAGGTGAGACTTGTAGGAAGAAGCTTA
GACTATCCAAAGATCAATCCGCAGTTCTCGAAGACACTTTCAAAGAGCACAATACTCTCAATCCCA
AACAGAAGCTGGCTTTGGCTAAGAAGCTAGGTTTAACAGCAAGACAAGTGGAAGTGTGGTTCCAAA
ACAGAAGAGCAAGGACAAAGTTAAAGCAGACCGAAGTGGATTGCGAGTATTTGAAAAGATGTGTTG
AGAAATTAACGGAAGAGAATCGGCGGCTCGAGAAAGAGGCAGCGGAACTAAGAGCATTAAAGCTTT
CACCGCGGTTGTATGGTCAGATGAGTCCACCGACCACACTTTTGATGTGTCCATCGTGTGAACGTG
TGGCCGGACCATCCTCATCTAACCACAACCAGCGATCTGTCTCATTGAGTCCATGGCTCCAAATGG
CCCATGGGTCAACCTTTGATGTGATGCGTCCTAGGTCTTAACTTTAATGCTGCTTCTATGGGTTGT
GTGTGGGTCATTGTACTTTTTAGATTATTGACTCTCAGCTAATGTATCCTTAAAAGCCTTTTTCTA
CTTTTAAATTTACTTTAATCTAATTAAATTAGTTATTCTTGTCTTCTTGATAACAAACAAATTTAT
AATAAT

**SEQ ID NO: 256, *Arabidopsis thaliana* - HAT1 - protein**
MMMGKEDLGLSLSLGFAQNHPLQLNLKPTSSPMSNLQMFPWNQTLVSSSDQQKQQFLRKIDVNSLP
TTVDLEEETGVSSPNSTISSTVSGKRRSTEREGTSGGGCGDDLDITLDRSSSRGTSDEEEDYGGET
CRKKLRLSKDQSAVLEDTFKEHNTLNPKQKLALAKKLGLTARQVEVWFQNRRARTKLKQTEVDCEY
LKRCVEKLTEENRRLEKEAAELRALKLSPRLYGQMSPPTTLLMCPSCERVAGPSSSNHNQRSVSLS
PWLQMAHGSTFDVMRPRS

**SEQ ID NO: 257, *Arabidopsis thaliana* - HAT2 - DNA**
AATAATGATGGATTGTAATTAGCCACCTGACAAAATCTCTCATCATTCAAAGTACTATATTAATCC
CCTCTCTTCTTCATTACCATCTCACATCTCTCTCTATTTCTCTTCCACAAAGAGTCCTAACTTCGA
GTTGAAACAAACACCATTTCTCATCTCTATCTCAGAAAGAACAAACCATTTCGTGTTCTTTCTTTC
TCTATTCTCATAAGGAAATATAATTCCTGAAACTGTTGAGTTCTTGTGAAAGGAAATAAAAAACAT
GATGATGGGCAAAGAAGATCTAGGTTTGAGCCTAAGCTTAGGGTTTTCACAAAATCACAATCCTCT
TCAGATGAATCTGAATCCTAACTCTTCATTATCAAACAATCTCCAGAGACTCCCATGGAACCAAAC
ATTCGATCCTACATCAGATCTTCGCAAGATAGACGTGAACAGTTTTCCATCAACGGTTAACTGCGA
GGAAGACACAGGAGTTTCGTCACCAAACAGTACGATCTCAAGCACCATTAGCGGGAAGAGAAGTGA
GAGAGAAGGAATCTCCGGAACCGGCGTTGGCTCCGGCGACGATCACGACGAGATCACTCCGGATCG
AGGGTACTCACGTGGAACCTCAGATGAAGAAGAAGACGGGGGCGAAACGTCGAGGAAGAAGCTCAG
GTTATCAAAAGATCAGTCTGCTTTTCTCGAAGAGACTTTCAAAGAACACAACACTCTCAATCCCAA
ACAGAAGCTAGCTTTGGCTAAGAAGCTGAACTTGACGGCAAGACAAGTGGAAGTGTGGTTCCAAAA
CAGAAGAGCTAGAACCAAGTTAAAGCAAACGGAGGTAGATTGCGAATACTTGAAACGGTGCGTAGA
GAAGCTAACGGAAGAGAACCGGAGACTTCAGAAAGAGGCTATGGAGCTTCGAACTCTCAAGCTGTC
TCCACAATTCTACGGTCAGATGACTCCACCAACTACACTCATCATGTGTCCTTCGTGCGAGCGTGT
GGGTGGCCCATCATCATCGAACCATCACCACAATCACAGGCCCGTTTCTATCAATCCGTGGGTTGC
TTGTGCTGGTCAGGTGGCTCATGGGCTGAATTTTGAAGCCTTGCGTCCACGATCGTGATTTTTTAT
TTTAGTGGTGGGAAAAGGGTGTTTTGGTATTTTTCGTTATCGTTATATAGTCTATCTGTGTGGGGT
CATTGTAATTTTGGATGATTGGCCTTCTCATGAACTAGTCCTATGTATGATGCAACCTTAAAAAGA
TTTAAATTAGCAAAAATTAGTTACAAACTT

**FIGURE 25 (continued)**

**SEQ ID NO: 258, *Arabidopsis thaliana* - HAT2 - protein**
MMMGKEDLGLSLSLGFSQNHNPLQMNLNPNSSLSNNLQRLPWNQTFDPTSDLRKIDVNSFPSTVNC
EEDTGVSSPNSTISSTISGKRSEREGISGTGVGSGDDHDEITPDRGYSRGTSDEEEDGGETSRKKL
RLSKDQSAFLEETFKEHNTLNPKQKLALAKKLNLTARQVEVWFQNRRARTKLKQTEVDCEYLKRCV
EKLTEENRRLQKEAMELRTLKLSPQFYGQMTPPTTLIMCPSCERVGGPSSSNHHHNHRPVSINPWV
ACAGQVAHGLNFEALRPRS

**SEQ ID NO: 259, *Arabidopsis thaliana* - HB4 - DNA**
ATGGGGGAAAGAGATGATGGGTTGGGTTTGAGTCTAAGCTTGGGAAATAGTCAACAAAAAGAACCA
TCTCTGAGGTTGAATCTTATGCCGTTGACAACTTCTTCTTCTTCTTCGTTTCAACACATGCAC
AATCAGAATAACAATAGCCATCCCCAGAAGATTCATAACATCTCTTGGACTCATCTGTTTCAATCT
TCTGGGATTAAACGTACAACTGCAGAGAGAAACTCCGACGCCGGGTCATTTCTAAGAGGTTTCAAC
GTGAACAGAGCTCAGTCTTCGGTGGCGGTAGTGGACTTGGAAGAAGAAGCCGCCGTCGTCTCGTCT
CCAAACAGCGCCGTTTCGAGTCTGAGTGGAAATAAAAGGGATCTTGCGGTGGCGAGAGGAGGAGAT
GAAAACGAGGCGGAGAGAGCTTCTTGCTCACGCGGAGGGGGAAGCGGTGGTAGCGACGATGAAGAC
GGCGGAAACGGCGACGGATCAAGGAAGAAACTACGGTTATCGAAGGATCAAGCTCTTGTTCTCGAG
GAGACTTTTAAAGAACATAGCACTCTTAATCCGAAGCAAAAGCTGGCTCTAGCAAAACAGTTGAAT
CTAAGGGCAAGACAAGTTGAAGTGTGGTTTCAGAACCGTAGGGCAAGGACGAAGCTGAAACAAACG
GAGGTTGATTGTGAGTATTTAAAGAGATGTTGCGATAATCTGACCGAGGAGAATCGACGGCTGCAG
AAAGAAGTGTCGGAGCTGAGGGCGTTGAAGTTGTCTCCACATCTCTACATGCACATGACTCCTCCT
ACTACTCTCACCATGTGCCCTTCTTGCGAACGTGTCTCCTCCTCTGCCGCCACTGTGACCGCTGCT
CCTTCCACTACTACTACTCCTACGGTGGTGGGGCGGCCAAGTCCACAGCGATTAACTCCTTGGACT
GCTATTTCTCTCCAGCAAAAATCAGGTCGCTAG

**SEQ ID NO: 260, *Arabidopsis thaliana* - HB4 - protein**
MGERDDGLGLSLSLGNSQQKEPSLRLNLMPLTTSSSSSSFQHMHNQNNNSHPQKIHNISWTHLFQS
SGIKRTTAERNSDAGSFLRGFNVNRAQSSVAVVDLEEEAAVVSSPNSAVSSLSGNKRDLAVARGGD
ENEAERASCSRGGGSGGSDDEDGGNGDGSRKKLRLSKDQALVLEETFKEHSTLNPKQKLALAKQLN
LRARQVEVWFQNRRARTKLKQTEVDCEYLKRCCDNLTEENRRLQKEVSELRALKLSPHLYMHMTPP
TTLTMCPSCERVSSSAATVTAAPSTTTTPTVVGRPSPQRLTPWTAISLQQKSGR

**SEQ ID NO: 261, *Arabidopsis thaliana* - HAT3 - DNA**
CCACAACCCCATATCTCTTTGTCTGAAAAAACTCTTTTGCATATATAAATAAATATAAGCCATATC
TTTTGAAACTGTACTGCTGCACAAGTGTAGAGGCAGTGGCACAACATCTTTAAGAAAGAGAGAGAG
AAAGAGTCATTTATTCATTTCCTCGCTTAAAAATCTTGAGCACCCAGACAGAATTCTTCTTTCTTC
TTTCTGGGGTTGAGAAAAATGAGTGAAAGAGATGATGGATTGGGGCTAAGTTTGAGCTTGAGTTTA
GGTTTTAATCAAAAGGACCCGTCTTCGAGGTTAAATCCAATGCCTCTGGCTTCTTATGCATCTTCA
TCACACATGCAGCATATGCAGCAGAGCAATTATAACCATCCTCAAAAGATTCAGAACACTTGGATT
AACATGTTTCAGTCATCAGAGAGAAACTCGGACATGAGATCGTTTCTCCGGGGAATAGACGTGAAC
AGAGCTCCATCGACGGTGGTGGTTGACGTGGAGGATGAAGGCGCCGGAGTTTCGTCTCCGAACAGC
ACCGTCTCAAGCGTGATGAGCGGGAAGAAGAGCGAGCGAGAGCTAATGGCTGCGGCAGGTGCAGTT
GGAGGAGGTAGAGTAGAAGATAATGAGATTGAGAGAGCTTCTTGCTCGCTCGGCGGTGGTAGCGAC
GATGAAGACGGTAGCGGGAACGGAGATGACAGTTCGAGGAAGAAACTCCGATTGTCTAAAGAACAA
GCTTTGGTTCTTGAAGAAACTTTTAAAGAACATAGTACACTCAATCCGAAGCAAAAGATGGCTTTG
GCTAAGCAATTGAATCTGAGGACGAGACAAGTTGAAGTGTGGTTCCAAAACCGAAGGGCAAGGACG
AAGCTGAAGCAAACGGAAGTAGACTGTGAATATCTTAAGAGATGTTGCGAGAATCTAACGGATGAG
AATCGGAGATTGCAAAAGGAAGTGAGTGAGCTTAGGGCTTTAAAGCTTTCTCCACACTTATACATG
CACATGAAACCTCCCACTACTCTCACAATGTGTCCTTCTTGCGAGCGAGTCGCTGTTACGTCATCT

**FIGURE 25 (continued)**

TCGTCATCGGTGGCTCCTCCTGTGATGAATTCATCGTCTCCGATGGGTCCGATGAGTCCGTGGGCT
GCCATGCCTCTACGGCAACGACCTGCTGCTGGTTCTCATTAGAGTTTAATTAGTCTAAAGATAATA
GGTTTGGTGATTTGTTTTTATTATATTGTTGAACGGACTTGGAAGTTCTTTTCAAGAGTTGGTCCC
ATGCTCTTCTTTCCTTTGTTTTATTCAGTTTCTATTTATAGCTGAATTCTGGATAATGGAAAATCT
ACTAATATTATTGTCCAATTATTAGTTTGGGGAAAGA

**SEQ ID NO: 262, *Arabidopsis thaliana* - HAT3 - protein**
MSERDDGLGLSLSLSLGFNQKDPSSRLNPMPLASYASSSHMQHMQQSNYNHPQKIQNTWINMFQSS
ERNSDMRSFLRGIDVNRAPSTVVVDVEDEGAGVSSPNSTVSSVMSGKKSERELMAAAGAVGGGRVE
DNEIERASCSLGGGSDDEDGSGNGDDSSRKKLRLSKEQALVLEETFKEHSTLNPKQKMALAKQLNL
RTRQVEVWFQNRRARTKLKQTEVDCEYLKRCCENLTDENRRLQKEVSELRALKLSPHLYMHMKPPT
TLTMCPSCERVAVTSSSSSVAPPVMNSSSPMGPMSPWAAMPLRQRPAAGSH

**SEQ ID NO: 263, *Oryza sativa* - Homeobox associated leucine zipper family protein - DNA**
ATGATGGAGAGGGCCGAGGACCTGCGCCTGAGCCTCAGTCTCAGCTCGCCGCTTATTGCTCCTCGT
ACTCACCATGTCGCCATGCTGTTCCACGCTCCTCCAGAGAAAAGATTCCTGGAGATGCCGCTGCTC
CCTGCTGCGAAGCGGAGCGAGGTCGTCGCGGCAGAAGAGGAGCGCGCGGGCCTGCGCGGCGGCGGC
GGCAGCGACGAGGAGGACGGTGGCTGCGGCATCGACGGCTCACGCAAGAAGCTCCGGCTTTCCAAG
GACCAGTCCGCCGTGCTCGAGGACAGCTTCCGGGAGCACCCCACCCTCAACCCCAGGCAGAAGGCA
ACTTTGGCGCAGCAGCTCGGGCTTCGGCCTCGGCAGGTCGAGGTGTGGTTTCAGAACAGACGCGCA
AGGACGAAGCTGAAGCAGACGGAGGTGGACTGCGAGTTCCTGAAGCGCTGCTGCGAGACGCTCACG
GAGGAGAACCGGAGGCTGCAGAAGGAGGTGCAGGAGCTGCGAGCGCTCAAGCTCGTCTCGCCGCAC
CTCTACATGAACATGTCCCCGCCCACCACGCTCACCATGTGCCCCTCCTGCGAGCGCGTCTCCAAC
ACCAATAACAACTCCAGCGCCGCCGCCGCCGCCGACCGCCGCGGCATCAGGACTACTACTGCCGCA
GGCGGCGGCAGCGTCGTCGACACCGCCGCCGACGGGGGCATCCTCTGCCACCGCCCGATCGCCGTC
CGGCCGCAGCAGTCATGA

**SEQ ID NO: 264, *Oryza sativa* - Homeobox associated leucine zipper family protein - protein**
MMERAEDLRLSLSLSSPLIAPRTHHVAMLFHAPPEKRFLEMPLLPAAKRSEVVAAEEERAGLRGGG
GSDEEDGGCGIDGSRKKLRLSKDQSAVLEDSFREHPTLNPRQKATLAQQLGLRPRQVEVWFQNRRA
RTKLKQTEVDCEFLKRCCETLTEENRRLQKEVQELRALKLVSPHLYMNMSPPTTLTMCPSCERVSN
TNNNSSAAAAADRRGIRTTTAAGGGSVVDTAADGGILCHRPIAVRPQQS

**SEQ ID NO: 265, *Oryza sativa* - Homeobox domain containing protein - DNA**
ATGAGGTCGTACATGGACGGCGGCGGCGCGGCGGCGTACGAGGAGGAGGAGGAGGAGGTTGAGGAC
GACGACGGCGGCGGCGGCGGCGGCGGCGGCGGCGGCGGTGGGGGGCTCGGGGAGAAGAAGCGGCGG
CTGGCGGCGGAGCAGGTGCGGGCGCTGGAGCGGAGCTTCGAGGCGGACAACAAGCTGGACCCGGAG
CGGAAGGCCCGGATCGCCCGCGACCTTCGCCTCCACCCTCGCCAGGTCGCCGTCTGGTTCCAGAAC
CGCCGCGCGAGGTGGAAGACCAAGCAGATCGAGCGCGACTTCGCCGCCCTCCGCTCCCGCCACGAC
GCCCTCCGCCTCGAGTGCGACGCCCTCCGCCGCGACAAGGACGCCCTCGCCGCCGAGATCGCCGAC
CTCCGGGACAGGGTGGACGGCCAGATGTCCGTCAAGCTGGAGGCCGTGGCCGCGGACGAACACCAG
CCGCCTCCGCCGCCGCCGCCGCCGCCACTGGCGTATAACAGCAAGGTGGTGGACGGCTCGACGGAC
AGCGACTCGAGCGCGGTGTTCAACGAGGAGGCGTCGCCGTACTCCGGCGCGGCCATCGACCACCAC
CACCACCAAACTCCGGCGAGCTACGACACGGCGGGGTTCACCTCCTTCTTCGCGCCATCCACCACG
CTCACCTCGTCCCTCTCCTTCCCTTCCATGTTCCACGCGTCATCGCATTTCGATGGCCACCAAGAA
CTCCTCGTCGGCGGCGGCGGCGCCGGCGCAGTGGCCGACGCCGACCTCGGAGGCGCCGGATTCTTC
GCCGGCGACGAGCACGCCGGCGGCCTCTCCTGGTACGGCGCCGAGGGTTGGTAG

**FIGURE 25 (continued)**

**SEQ ID NO: 266, *Oryza sativa* - Homeobox domain containing protein - protein**

MRSYMDGGGAAAYEEEEEEVEDDDGGGGGGGGGGGGGLGEKKRRLAAEQVRALERSFEADNKLDPE
RKARIARDLRLHPRQVAVWFQNRRARWKTKQIERDFAALRSRHDALRLECDALRRDKDALAAEIAD
LRDRVDGQMSVKLEAVAADEHQPPPPPPPPPLAYNSKVVDGSTDSDSSAVFNEEASPYSGAAIDHH
HHQTPASYDTAGFTSFFAPSTTLTSSLSFPSMFHASSHFDGHQELLVGGGGAGAVADADLGGAGFF
AGDEHAGGLSWYGAEGW

**SEQ ID NO: 267, *Oryza sativa* - Homeobox domain containing protein - DNA**

ATGAAGAGGCCCAGCTGCAGAGGCTCCTCCATGGCCATCATCCATGACACCTCTGATCAACAAGAG
GACAACATGAGGTCGTACATGGACGGCGGCGGCGCGGCGGCGTACGAGGAGGAGGAGGAGGAGGTT
GAGGACGACGACGGCGGCGGCGGCGGCGGCGGCGGCGGCGGCGGTGGGGGGCTCGGGGAGAAGAAG
CGGCGGCTGGCGGCGGAGCAGGTGCGGGCGCTGGAGCGGAGCTTCGAGGCGGACAACAAGCTGGAC
CCGGAGCGGAAGGCCCGGATCGCCCGCGACCTTCGCCTCCACCCTCGCCAGGTCGCCGTCTGGTTC
CAGAACCGCCGCGCGAGGTGGAAGACCAAGCAGATCGAGCGCGACTTCGCCGCCCTCCGCTCCCGC
CACGACGCCCTCCGCCTCGAGTGCGACGCCCTCCGCCGCGACAAGGACGCCCTCGCCGCCGAGATC
GCCGACCTCCGGGACAGGGTGGACGGCCAGATGTCCGTCAAGCTGGAGGCCGTGGCCGCGGACGAA
CACCAGCCGCCTCCGCCGCCGCCGCCGCCGCCACTGGCGTATAACAGCAAGGTGGTGGACGGCTCG
ACGGACAGCGACTCGAGCGCGGTGTTCAACGAGGAGGCGTCGCCGTACTCCGGCGCGGCCATCGAC
CACCACCACCACCAAACTCCGGCGAGCTACGACACGGCGGGGTTCACCTCCTTCTTCGCGCCATCC
ACCACGCTCACCTCGTCCCTCTCCTTCCCTTCCATGTTCCACGCGTCATCGCATTTCGATGGCCAC
CAAGAACTCCTCGTCGGCGGCGGCGGCGCCGGCGCAGTGGCCGACGCCGACCTCGGAGGCGCCGGA
TTCTTCGCCGGCGACGAGCACGCCGGCGGCCTCTCCTGGTACGGCGCCGAGGGTTGGTAG

**SEQ ID NO: 268, *Oryza sativa* - Homeobox domain containing protein - protein**

MKRPSCRGSSMAIIHDTSDQQEDNMRSYMDGGGAAAYEEEEEEVEDDDGGGGGGGGGGGGGLGEKK
RRLAAEQVRALERSFEADNKLDPERKARIARDLRLHPRQVAVWFQNRRARWKTKQIERDFAALRSR
HDALRLECDALRRDKDALAAEIADLRDRVDGQMSVKLEAVAADEHQPPPPPPPPPLAYNSKVVDGS
TDSDSSAVFNEEASPYSGAAIDHHHHQTPASYDTAGFTSFFAPSTTLTSSLSFPSMFHASSHFDGH
QELLVGGGGAGAVADADLGGAGFFAGDEHAGGLSWYGAEGW

**SEQ ID NO: 269, *Oryza sativa* - Homeobox domain containing protein - DNA**

ATGAAGAGGCCCACCAGCAGCAGCCGAAAATCCAAAAAACAAGGAGAGGACCTGGCGTTCTCTGAG
GAGGGCAGCTTGCCCGCGGTGACCATGGAGCAGAAAGATGAAGCCGAGATGGAGGAGGTGGACGAG
GAGGAGGAGGAGGAGGTCGACGAAGACATGGCCGGCGGGCACGCGGCGCAGTCGCCGTCGCCGTCG
TGCGGGCTGGGCGAGAAGAAGCGGCGGCTGGCGCTGGAGCAGGTGCGCGCTCTGGAGCGGAGCTTC
GACACGGACAACAAGCTGGACCCGGACCGCAAGGCCCGCATCGCGCGCGACCTCGGCCTGCAGCCG
CGCCAGGTCGCCGTCTGGTTCCAGAACCGCCGCGCCCGGTGGAAGACGAAGCAGCTCGAGCGCGAC
TTCGCCGCCCTCCGCGCCCGCCACGACGCCCTCCGCGCCGACTGCGACGCCCTGCGCCGCGACAAG
GACGCCCTCGCCGCCGAGATTCGGGAGCTGAGGGAGAAGCTGCCCACCAAGCCGGCGGACACGGCG
GCATCGGTGAAGGTAGAAGCCGGCAATGACGCGGCGGCCGGCGCCGCGGCCGCCACGGTGTGCAAG
GACGGCTCGTCGGACGACAGCGACTCCAGCGTGGTGTTCAACGACGAGGCGTCGCCGTACTCCGGC
GCGGCCTTCATTGGATTCGGCCCGTCGTTCTTGGTCGACGACGCGTCGGCGGCAACCGTGGGCTGC
TCGTCGTCGCTCCCCGCGCTCGAGTCCAAATGGCACGGTCCGTACTCCGACGACTCGTGCAAAGGC
GGCGTCTATGGCTTCACGGAGGAATGGCTCGCTGCCTGCTCCGGCGAGATGGCCGGCAACGACGCC
GCCGGCTTCTTCTCCGACGAGCACGCCTCCAACCTCAACTTCGGTTGGTGCGCGAGTGGTAACGAG
GGTTGGGAATGA

**FIGURE 25 (continued)**

**SEQ ID NO: 270, *Oryza sativa* - Homeobox domain containing protein - protein**

MKRPTSSSRKSKKQGEDLAFSEEGSLPAVTMEQKDEAEMEEVDEEEEEEVDEDMAGGHAAQSPSPS
CGLGEKKRRLALEQVRALERSFDTDNKLDPDRKARIARDLGLQPRQVAVWFQNRRARWKTKQLERD
FAALRARHDALRADCDALRRDKDALAAEIRELREKLPTKPADTAASVKVEAGNDAAAGAAAATVCK
DGSSDDSDSSVVFNDEASPYSGAAFIGFGPSFLVDDASAATVGCSSSLPALESKWHGPYSDDSCKG
GVYGFTEEWLAACSGEMAGNDAAGFFSDEHASNLNFGWCASGNEGWE

**SEQ ID NO: 271, *Oryza sativa* - Homeobox associated leucine zipper family protein - DNA**

ATGAGAAGTCCAGCAGCGCTTCTCCCGGTGGTTGCAGATGGTGGTGGTGGTGTTGGGGTGGAGGAG
GAGATGGATGTGGACGAGGACATGGCGATGTGCGGCGGCCGCGGCGGCGGCGGCGGGGAGAAGAAG
CGGCGGCTGAGCGTGGAGCAGGTGCGCGCGCTGGAGCGGAGCTTCGAGACGGAGAACAAGCTGGAG
CCGGAGCGGAAGGCGCGGCTGGCGCGCGACCTCGGGCTGCAGCCGCGCCAGGTCGCCGTCTGGTTC
CAGAACCGCCGCGCGCGGTGGAAGACCAAGCAGCTCGAGCGCGACTACGCCGCGCTCCGCCAATCC
TACGACGCGCTCCGCGCCGACCACGACGCGCTTCGCCGCGACAAGGACGCCCTCCTCGCCGAGATC
AAGGAGCTGAAGGGGAAGCTCGGCGACGAGGACGCCGCGGCGAGCTTCTCGTCGGTGAAGGAGGAG
GAGGACCCGGCGGCGTCCGACGCCGACCCCCCGGCCACCGGCGCGCCGCAGGGCTCGTCCGAGAGC
GACTCGAGCGCGGTGCTGAACGACGCGGAGATCCTTCCACACAAGCCAGCGCCGGCGGCCGCCGCC
GACGCCGCGGCCTCGGAGGAGACGGAGGCGGTGGTGACCGGCGCCGCGCTGCTCCACCACGCCGAG
GTGTTCTTCCACGGGCAGCTTCTCAAGGTGGACGACGACGAGGCGGCGTTCCTAGGCGACGACGGC
GCGGCGTGCGGCGGCTTCTTCGCCGACGAGCATCTCCCGTCGCTGCCGTGGTGGGCCGAGCCCACC
GAGCAATGGACGACCTAG

**SEQ ID NO: 272, *Oryza sativa* - Homeobox associated leucine zipper family protein - protein**

MRSPAALLPVVADGGGGVGVEEEMDVDEDMAMCGGRGGGGGEKKRRLSVEQVRALERSFETENKLE
PERKARLARDLGLQPRQVAVWFQNRRARWKTKQLERDYAALRQSYDALRADHDALRRDKDALLAEI
KELKGKLGDEDAAASFSSVKEEEDPAASDADPPATGAPQGSSESDSSAVLNDAEILPHKPAPAAAA
DAAASEETEAVVTGAALLHHAEVFFHGQLLKVDDDEAAFLGDDGAACGGFFADEHLPSLPWWAEPT
EQWTT

**SEQ ID NO: 273, *Oryza sativa* - Homeobox associated leucine zipper family protein - DNA**

ATGAAGCGACCCGGCGGTGCCGGCGGCGGCGGAGGCAGCCCATCGCTCGTCACGATGGCTAATTCT
AGTGATGATGGATATGGAGGGGTTGGGATGGAGGCGGAGGGGGACGTGGAGGAGGAGATGATGGCG
TGCGGCGGCGGCGGGGAGAAGAAGCGGCGGCTGAGCGTGGAGCAGGTTCGCGCGCTGGAGCGGAGC
TTCGAGGTGGAGAACAAGCTTGAGCCTGAGCGGAAGGCGCGGCTGGCGCGCGACCTCGGCCTGCAG
CCGCGCCAGGTCGCCGTCTGGTTCCAGAACCGCCGCGCGCGGTGGAAGACCAAGCAGCTCGAGCGC
GACTACGCCGCGCTCCGCCATTCCTACGACTCCCTGCGCCTCGATCACGACGCGCTCCGCCGCGAC
AAGGACGCCCTCCTCGCCGAGATCAAGGAGCTGAAGGCGAAGCTCGGGGACGAGGAGGCGGCGGCG
AGCTTCACGTCGGTGAAGGAGGAGCCGGCGGCCTCCGACGGGCCACCGGCGGCGGGATTTGGGTCG
TCCGACAGCGACTCAAGCGCGGTGCTGAACGACGTGGACGCGGCCGGCGCCGCGCCCGCGGCGACG
GACGCGCTGGCTCCGGAGGCGTGCACGTTTCTCGGCGCGCCGCCCGCCGCGGGCGCGGGCGCGGGC
GCAGCGGCGGCGGCGAGCCACGAGGAGGTGTTCTTCCACGGCAATTTCCTCAAGGTGGAGGAGGAC
GAGACGGGGTTCCTCGACGACGACGAGCCGTGCGGCGGGTTCTTCGCCGACGATCAGCCCCCGCCG
CTGTCGTCGTGGTGGGCCGAGCCCACGGAGCACTGGAACTGA

FIGURE 25 (continued)

**SEQ ID NO: 274, *Oryza sativa* - Homeobox associated leucine zipper family protein - protein**

MKRPGGAGGGGGSPSLVTMANSSDDGYGGVGMEAEGDVEEEMMACGGGGEKKRRLSVEQVRALERS
FEVENKLEPERKARLARDLGLQPRQVAVWFQNRRARWKTKQLERDYAALRHSYDSLRLDHDALRRD
KDALLAEIKELKAKLGDEEAAASFTSVKEEPAASDGPPAAGFGSSDSDSSAVLNDVDAAGAAPAAT
DALAPEACTFLGAPPAAGAGAGAAAAASHEEVFFHGNFLKVEEDETGFLDDDEPCGGFFADDQPPP
LSSWWAEPTEHWN

**SEQ ID NO: 275, *Oryza sativa* - Homeobox domain containing protein - DNA**

ATGTCATCCTCTCTCTTTCTTCTTCCTCCTCTCTTTCTTTCTCTCTTCTCTCCCCTTCGGCTAGCC
GCAGGGAGCACGGGCTGGGGCGGTAGTGGGCGGGGACAAGAGAGGCGGAGGGAGCTGAAGCGGCGC
CGGCGGCGGCACACGCTCTCCAGCCTCAGCGGCAAGCGTGGTGCGCCATCTGCCGCTGCCGCCGCC
GTCGGCGGCAGCGACGACGAGGACTCCGACGACGGATCCCGCAAGAAGCTCCGCCTCTCCAAGGAC
CAAGCCGCCGTCCTCGAGGACACCTTCAACAAGCACAACACCCTCAACCCCAAGCAGAAGGCGGCG
CTGGCGAGGCAGCTGAATCTGAAGCCGCGGCAGGTGGAGGTGTAG

**SEQ ID NO: 276, *Oryza sativa* - Homeobox domain containing protein - protein**

MSSSLFLLPPLFLSLFSPLRLAAGSTGWGGSGRGQERRRELKRRRRRHTLSSLSGKRGAPSAAAAA
VGGSDDEDSDDGSRKKLRLSKDQAAVLEDTFNKHNTLNPKQKAALARQLNLKPRQVEV

**SEQ ID NO: 277, *Oryza sativa* - protein - DNA**

ATGTTTTATTCAAGTCCCTTTTGTTCATCTCCTTTGCAGATTCCATTTCTGACCCAAATTTTGGCT
ATTCCTCATGATGAGTTTGTCTCAAACTGGTGCTCTGTTAATCTGCCAGTGATAGAAGAAGACGCT
AATCTTGATTATGATCCATTTGGTGCAGCTGAGCTGGCACTGGCAGCTGCTGGTAATAAGTTAACT
GAAGCTAAAGCAAACTATTCTTGCCCTTTTCGCCCTATCAGTATGCCTTCTATAGCATATGCGCAG
ACAAGAACATCATGTGTGGTGAAAATAATAGCAAATTTGCATGTTTTTGTCCCAAACATATGCGAA
GAGCAAGAAAGAGACCTTTTTCTTCAGAAATTTCAGAAGTACTTGGTATCAGGGAATCCTAGATCA
TCAGTTGATCATCCAGCATCAGCTGATCTCAAGGCCACTACAGTTTGCAGAAACTTGGGATCTTTG
TCTGAGTATGCTAGATCGTTAATTCCTAATAACTTGTTAAACGAGGAAGATGTGCAATTGTTAAGT
GAATTTGCTTATAAGTTACAAACTTGGTGTAAATCACATGTTGGACAGAGTACATCCCAGGCAGTA
AAGATTGATCCGTCATCAGAAAGCAAGGAAGACTTTAAGCCACTGCAGCATCCCTTGATACCAAGT
ACTGTTGTTCCAGATTCCAGTATAAATAACCTTCCGAAGAACATGGAAGAGCCTACACCAACAAAC
ATGGAAGAGCCTGCACCAACACCCTCAACAAAGCAAGAGGGAAATGCCAGGGATGAGACTCCTAGA
AGCACTGTCGCTTTAAATGGTGGGTTCCTGCAGAATTCAGTCGGCCAGGACTTAGTCCATCTTGGT
GTGGCGAGAACTAGTTCAGGCTTTCTAGGGGGAGGTACCAGTACAAGTACAGGATCCCTGCGCTGC
AAAATGGATCTTGATCCTGCATCCAGCAGTATGGACCATTTCAAAACACCAGATAGAAAAGAAAGT
GGTCTTCAGGATGATGAGAAAGGAGACACTCACATGTATGATGAGAGACAACCTAAGAGAAGGAAG
CGAACCATTATGAATGATAGGCAAATAAACGAAATCGAGAAGGCTCTTATTGATGAGCCTGAGATG
CATAAGAATGCTGCTTTACTGCAGGCATGGTCAGAGAAGTTAAGTGGGCAGGGCTCGGAGATTACG
TCATCTCAGCTAAAGAATTGGCTGAACAACAGAAAAGCTAAGCTTGCTCGTATTGCGAAAGAAAGA
GGAGTACTATCTGAGGGTGAGAACGCAGATAAGCCATCTACGCCAGCTACTCCCCACCACTGTGAC
TCCTCAGAAAGTGCTGGCGAGGAGAGCTACTTGCCACCTGCGAGGGTCATGAGTGCTCTAGGCATA
TCCAAGGGCAGCAGATTTGTGAGCCCAGATGGCAATGAGACAACATCACAGGCAGAATTCAATCAA
AATATCATGCTTAGCCGCCCTTTCACAAGATCATTCTCATTTGAACCTGGCCGTCTTGTTTCGCTC
ATTGATAATGATGGGAAGGAGGTTGGCAGGGGAAAGATCTTCCAAGTTGAGGGGAGACTGCAAGGG
AAGGCCCTGACAGATACTCGCGTTTGCATCGTTGATGTTATTGAGCTCAAGATTGAGAAATGGCGG
GAGCTACCTCATCCCTCGGAAGCATCAGGAAGAACATTCCAAGAGGCAGAATCAAGGAACGGTGGT
GTGATGAGGGTCGCGTGGGATGTCATCAGACTATCTCCAGTGGTTCAGTAA

FIGURE 25 (continued)

**SEQ ID NO: 278, *Oryza sativa* - protein - DNA**
MFYSSPFCSSPLQIPFLTQILAIPHDEFVSNWCSVNLPVIEEDANLDYDPFGAAELALAA
AGNKLTEAKANYSCPFRPISMPSIAYAQTRTSCVVKIIANLHVFVPNICEEQERDLFLQK
FQKYLVSGNPRSSVDHPASADLKATTVCRNLGSLSEYARSLIPNNLLNEEDVQLLSEFAY
KLQTWCKSHVGQSTSQAVKIDPSSESKEDFKPLQHPLIPSTVVPDSSINNLPKNMEEPTP
TNMEEPAPTPSTKQEGNARDETPRSTVALNGGFLQNSVGQDLVHLGVARTSSGFLGGGTS
TSTGSLRCKMDLDPASSSMDHFKTPDRKESGLQDDEKGDTHMYDERQPKRRKRTIMNDRQ
INEIEKALIDEPEMHKNAALLQAWSEKLSGQGSEITSSQLKNWLNNRKAKLARIAKERGV
LSEGENADKPSTPATPHHCDSSESAGEESYLPPARVMSALGISKGSRFVSPDGNETTSQA
EFNQNIMLSRPFTRSFSFEPGRLVSLIDNDGKEVGRGKIFQVEGRLQGKALTDTRVCIVD
VIELKIEKWRELPHPSEASGRTFQEAESRNGGVMRVAWDVIRLSPVVQ*

**SEQ ID NO: 279, motif I**
RKKLRL

**SEQ ID NO: 280, motif II**
TKLKQTEVDCEFLRRCCENLTEEN

**SEQ ID NO: 281, motif III**

TLTMCPSCER

**SEQ ID NO: 282, *Oryza sativa* - gos2 promoter**
AATCCGAAAAGTTTCTGCACCGTTTTCACCCCCTAACTAACAATATAGGGAACGTGTGCTAAATAT
AAAATGAGACCTTATATATGTAGCGCTGATAACTAGAACTATGCAAGAAAAACTCATCCACCTACT
TTAGTGGCAATCGGGCTAAATAAAAAAGAGTCGCTACACTAGTTTCGTTTTCCTTAGTAATTAAGT
GGGAAAATGAAATCATTATTGCTTAGAATATACGTTCACATCTCTGTCATGAAGTTAAATTATTCG
AGGTAGCCATAATTGTCATCAAACTCTTCTTGAATAAAAAAATCTTTCTAGCTGAACTCAATGGGT
AAAGAGAGAGATTTTTTTTAAAAAAATAGAATGAAGATATTCTGAACGTATTGGCAAAGATTTAAA
CATATAATTATATAATTTTATAGTTTGTGCATTCGTCATATCGCACATCATTAAGGACATGTCTTA
CTCCATCCCAATTTTTATTTAGTAATTAAAGACAATTGACTTATTTTTATTATTTATCTTTTTTCG
ATTAGATGCAAGGTACTTACGCACACACTTTGTGCTCATGTGCATGTGTGAGTGCACCTCCTCAAT
ACACGTTCAACTAGCAACACATCTCTAATATCACTCGCCTATTTAATACATTTAGGTAGCAATATC
TGAATTCAAGCACTCCACCATCACCAGACCACTTTTAATAATATCTAAAATACAAAAAATAATTTT
ACAGAATAGCATGAAAAGTATGAAACGAACTATTTAGGTTTTTCACATACAAAAAAAAAAAGAATT
TTGCTCGTGCGCGAGCGCCAATCTCCCATATTGGGCACACAGGCAACAACAGAGTGGCTGCCCACA
GAACAACCCACAAAAAACGATGATCTAACGGAGGACAGCAAGTCCGCAACAACCTTTTAACAGCAG
GCTTTGCGGCCAGGAGAGAGGAGGAGAGGCAAAGAAAACCAAGCATCCTCCTCCTCCCATCTATAA
ATTCCTCCCCCCTTTTCCCCTCTCTATATAGGAGGCATCCAAGCCAAGAAGAGGGAGAGCACCAAG
GACACGCGACTAGCAGAAGCCGAGCGACCGCCTTCTTCGATCCATATCTTCCGGTCGAGTTCTTGG
TCGATCTCTTCCCTCCTCCACCTCCTCCTCACAGGGTATGTGCCCTTCGGTTGTTCTTGGATTTAT
TGTTCTAGGTTGTGTAGTACGGGCGTTGATGTTAGGAAAGGGGATCTGTATCTGTGATGATTCCTG
TTCTTGGATTTGGGATAGAGGGGTTCTTGATGTTGCATGTTATCGGTTCGGTTTGATTAGTAGTAT
GGTTTTCAATCGTCTGGAGAGCTCTATGGAAATGAAATGGTTTAGGGTACGGAATCTTGCGATTTT
GTGAGTACCTTTTGTTTGAGGTAAAATCAGAGCACCGGTGATTTTGCTTGGTGTAATAAAAGTACG
GTTGTTTGGTCCTCGATTCTGGTAGTGATGCTTCTCGATTTGACGAAGCTATCCTTTGTTTATTCC
CTATTGAACAAAAATAATCCAACTTTGAAGACGGTCCCGTTGATGAGATTGAATGATTGATTCTTA

**FIGURE 25 (continued)**

AGCCTGTCCAAAATTTCGCAGCTGGCTTGTTTAGATACAGTAGTCCCCATCACGAAATTCATGGAA
ACAGTTATAATCCTCAGGAACAGGGGATTCCCTGTTCTTCCGATTTGCTTTAGTCCCAGAATTTTT
TTTCCCAAATATCTTAAAAAGTCACTTTCTGGTTCAGTTCAATGAATTGATTGCTACAAATAATGC
TTTTATAGCGTTATCCTAGCTGTAGTTCAGTTAATAGGTAATACCCCTATAGTTTAGTCAGGAGAA
GAACTTATCCGATTTCTGATCTCCATTTTTAATTATATGAAATGAACTGTAGCATAAGCAGTATTC
ATTTGGATTATTTTTTTATTAGCTCTCACCCCTTCATTATTCTGAGCTGAAAGTCTGGCATGAAC
TGTCCTCAATTTTGTTTTCAAATTCACATCGATTATCTATGCATTATCCTCTTGTATCTACCTGTA
GAAGTTTCTTTTTGGTTATTCCTTGACTGCTTGATTACAGAAAGAAATTTATGAAGCTGTAATCGG
GATAGTTATACTGCTTGTTCTTATGATTCATTTCCTTTGTGCAGTTCTTGGTGTAGCTTGCCACTT
TCACCAGCAAAGTTC

**SEQ ID NO: 283, primer - p01294**
GGGGACAAGTTTGTACAAAAAAGCAGGCTTCACAATGATGTTCGAGAAAGACGATCTG

**SEQ ID NO: 284, primer - p00402**
GGGGACCACTTTGTACAAGAAAGCTGGGTTTAGGACCTAGGACGAAGAGCGT

**SEQ ID NO: 285, SYB1 coding sequence, ORF 1-492, start and stop in bold**
**ATG**AGCAGACCCGGAGATTGGAACTGCAGGTCATGCAGCCATCTCAACTTCCAGCGCCGTGACTCT
TGCCAGCGATGCGGTGACTCTCGTTCCGGCCCCGGTGGAGTTGGTGGCTTAGACTTTGGTAATTTC
GGTGGCAGAGCCATGTCTGCTTTCGGATTCACCACCGGCTCCGACGTTCGTCCCGGTGATTGGTAC
TGCACCGTGGGAAACTGCGGGACACACAACTTCGCCAGTCGCTCCACCTGCTTCAAATGCGGCACT
TTCAAGGACGAGACCGGCGCTGGAGGCGGAGGTGGTGGCATCGGCGGTCCGGCCATGTTTGACGCC
GACATTATGCGGTCTAGAGTCCCCGGTAACGGTGGTCGCTCTAGCTGGAAATCCGGCGACTGGATT
TGCACTAGGATTGGTTGCAATGAGCATAACTTTGCAAGCAGAATGGAATGCTTCAGGTGCAATGCA
CCAAGGGACTTCAGCAACAGAACCTCTTTC**TAA**GTTATACAAACTGCTTTTGAAGTAGCCTTGTCT
ACCCAGCTTTCTTGTACAAAGTTGGCATTATAAGAAAGCATTGCTTATCAATTTGTTGCAACGAAC
AGGTCACTATCAGTCAAAATAAAATCAT

**SEQ ID NO: 286, SYB1**
MSRPGDWNCRSCSHLNFQRRDSCQRCGDSRSGPGGVGGLDFGNFGGRAMSAFGFTTGSDVRPGDWY
CTVGNCGTHNFASRSTCFKCGTFKDETGAGGGGGGIGGPAMFDADIMRSRVPGNGGRSSWKSGDWI
CTRIGCNEHNFASRMECFRCNAPRDFSNRTSF

**SEQ ID NO: 287, AAZ94630 gi|74027077|gb|DQ173926.1| *Gossypium hirsutum* cultivar Xiangmian 18 zinc finger protein-like protein mRNA, complete cds**
ATGAGCAGGCCAGGAGATTGGAACTGCAGGTCATGCCAACACCTCAACTTCCAAAGGAGGGACAAC
TGCCAACGTTGCGGTGAATCTCGATACGGTGTTAGAGTCGGCTCGACATTCGGGTTTACCGCTGGC
TCGGACGTTCGACCTGGTGACTGGTATTGCACGGCTGGAAACTGCGGCACCCACAATTTCGCCAGC
CGGTCTACTTGTTTCAATTGCGGCGCGTTCAAGGACGAGTCGGCTGGAGGTTTCGACTTGGACATG
TCTCGATCAAGAGGGTTCGGAGGTAACCGATCCGGCTGGAAATCAGGGGATTGGATATGTACCAGG
TTAGGGTGCAATGAACATAATTTTGCTAGCAGAATGGAATGTTTCAGATGCAGTGCTCCAAGAGAA
TTCAACAATAGAACTTCATATTAAATCACATGCATGCTACTATATCCATTTTTGGGTGTTTTGAGG
CAATTAATAGGAGATTATAATGAGAGAGTGTTACTGGCTTTGATGATGAACACCACAAGCATTTTG
TCATGTTTCTTTTATAAGATTCATGGCAATGTAGTACTTTTTCTTGTGATGATTATTTATGGTTTC
AATTCTATGGTTTTATTGTCTTTTATTTAGAGAGTTTTATTTATATTTTTGTAATGGTGCTTTGG
CTTTATTAAAAGAAGATGATTCTCTTCTGTCTTTTCAAAAAAAAAAAAAAAAAAAA

**FIGURE 25 (continued)**

**SEQ ID NO: 288, *Gossypium hirsutum*, AAZ94630.1**
MSRPGDWNCRSCQHLNFQRRDNCQRCGESRYGVRVGSTFGFTAGSDVRPGDWYCTAGNCGTHNFAS
RSTCFNCGAFKDESAGGFDLDMSRSRGFGGNRSGWKSGDWICTRLGCNEHNFASRMECFRCSAPRE
FNNRTSY

**SEQ ID NO: 289, Q53AV6 gi|48374867|gb|AY599410.1| *Zea mays* putative zinc finger protein ZF2 mRNA**
CGGGCACGACCAGCAACACAACAGCCGAGCTTTGCTTAGGCAAGATGAACAGGAAGCCAGGAGACT
GGGACTGCAGGGCGTGCCAGCACCTCAACTTCAGCCGCCGAGACATATGCCAGCGCTGCAGCGAGC
CACGTGGAGTTGCTGATCGTGGCAGTGGCGGCGGCGGAGGAGGCGACTACGCCAGCTTCGGTGGCC
GCGGTGGCTCCTCCTTCGGCGGCGGCTTTGGCGCTGCTGGCTCCGACGTCCGCCCTGGTGACTGGT
ACTGCTCCTGCGGCGCGCACAACTTCGCCAGCCGCTCCAGCTGCTTCAAGTGCTCCGCCTACAAGG
AGGAGGCCGCTGTGAACAGTGGCGCTGGCGGCTTTGATGGCGACATGTCACGCTCACGGGGCTACG
GCTTCGGCAGCGGTGCTGCTGCTGCTGCTGGTGCTGGCGCTGCCCGTACTACCAACCGCCCCGGTT
GGAAGTCCGGAGACTGGATCTGCACCAGATCCGGATGCAACGAGCACAACTTCGCCAGCAGGATGG
AGTGCTTCAGGTGCAACGCACCGCGGGACTCTGGCACTGAGGTGTAGGATCGAGCAAGTTAAAAAG
TCTGCAGCGCCGAAGAAAGCGACGACAAGAGGAGTCCTCATCACGTCGTAACGTAAGAGAGAGAGT
AGTGGATTTGCAACAAAAAAAAAAAAAAAGACGGCCGCGATGCTCTGTTACTAGCTAGCTAGTTTTG
TTCAACCACCCATGCCGTCTCTTCTCTTTTATTAGATTTGGTTTGGTTCTCATAGCCCTTTAATTA
CCATTTGGGACCTATGTTTGCTGTTCTGTTTCCCGTTCGTCTCCTCCGCATGTTTGCGCTTGGATC
GAGTCTTGTGATGTAACCCCCCAAAAAACGCTTGCTTAACTAGTACTGTTGCTTCTTAATAAAAAA
AAAAAAAAAAAAA

**SEQ ID NO: 290, Q53AV6|Q53AV6_MAIZE Putative zinc finger protein ZF2 [*Zea mays*]**
MNRKPGDWDCRACQHLNFSRRDICQRCSEPRGVADRGSGGGGGDYASFGGRGGSSFGGGFGAAGS
DVRPGDWYCSCGAHNFASRSSCFKCSAYKEEAAVNSGAGGFDGDMSRSRGYGFGSGAAAAAGAGAA
RTTNRPGWKSGDWICTRSGCNEHNFASRMECFRCNAPRDSGTEV

**SEQ ID NO: 291, Q6Z6E6 (gi|46390506:c30380-29965, c29249-29179, c29075-29062) *Oryza sativa* (japonica cultivar-group) genomic DNA, chromosome 2, PAC clone:P0544H11 AP005008**
ATGAACAGGAAGCCAGGAGACTGGGACTGCAGGGCGTGCCAGCACCTCAACTTCAGCCGCCGGGAC
CTATGCCAGCGCTGCGGCGAGCCGCGTGGCGCCGCTGATCGCGGCAGCGGTGGTGGCGGTGACTAC
GCCAACTTCGGCGGCCGTGGTGGTTCCTCCTTCGGTGGAGGCTTTGGCACTGGCTCTGATGTCCGC
CCAGGTGACTGGTACTGCAACTGCGGCGCGCACAACTTCGCCAGCCGCTCCAGCTGCTTCAAGTGC
GCTGCTTTCAAGGACGATGCTGCCGTCAACAGTGGCGGCGCTGGTGCCTTTGATGGTGGGGACATG
TCGCGCTCGCGGGGCTACGGCTTCGGCAGCGGCGCCGTCCGCGCCAGCCGCCCTGGCTGGAAGTCT
GGCGACTGGATTTGCACCAGGTCTGGATGCAATGAGCACAACTTCGCCAGCAGGATGGAGTGCTTC
AGGTGCAACGCACCGCGGGACTCCGGCACTGAGGTGTAA

**SEQ ID NO: 292, Q6Z6E6_ORYSA Zinc finger transcription factor ZFP30 [*Oryza sativa*]**
MNRKPGDWDCRACQHLNFSRRDLCQRCGEPRGAADRGSGGGDYANFGGRGGSSFGGGFGTGSDVR
PGDWYCNCGAHNFASRSSCFKCAAFKDDAAVNSGGAGAFDGGDMSRSRGYGFGSGAVRASRPGWKS
GDWICTRSGCNEHNFASRMECFRCNAPRDSGTEV

## FIGURE 25 (continued)

**SEQ ID NO: 293, Q8GWD1 gi|26452847|dbj|AK118921.1| *Arabidopsis thaliana* At5g25490 mRNA for unknown protein**
ATAAGCTCTTACACTCATTTCAACTCTCTTTTTCGTTTCCATTACCCTCAGAAGAAGATGAATAGG
CCGGGAGATTGGAACTGCAGATTGTGTAGCCACCTCAACTTCCAGAGGAGGGATTCATGCCAACGT
TGTAGAGAGCCTAGACCGGGCGGGATCAGTACCGATTTACTCAGCGGTTTTGGTGGCCGTCCGGTT
AGTAGCTCCTTCGGTTTCAACACCGGGCCCGATGTGCGACCCGGGGATTGGTATTGCAACCTTGGG
GATTGTGGGACACATAATTTTGCCAATAGGTCCAGTTGTTTCAAGTGTGGTGCCGCAAAAGATGAG
TTTTCATGCTCAAGTGCTGCTGCAACAACCGGGTTTATGGACATGAATGTTGGTCCGAGACGTGGC
CTTTTTGGTTTTGGCGGCAGCAGTAGTGGTGGTGGTGGTACGGGCCGTTCTCCTTGGAAATCTGGA
GATTGGATTTGCCCAAGGTCAGGCTGTAACGAACATAACTTCGCAAGCAGGTCAGAGTGTTTCAGG
TGTAACGCACCAAAGGAACTTGCCACCGAACCACCCTATTAGTCATTTAGTCCTCCTACCTTCTTC
ATCATTTCCAATACTCTGGAAGCATTAGAGGAGAGCAGAAAGGTGAAGAATCGAAGCAAGATACCG
ACCGCCCTTAATCTCTTGATTTGTTTAATTTCTTAGATTTGACTCGTTTTATATCTCGTAGTAGTC
AGACCTATGTTAGAATGTAAGCATGTCGAGAGTTTTCCGAAGCATTTTGTTCTGATATCGGTCTCC
TAGCTAGTATGTAAGTTTGTTTGAATGTATTCTTATTTCTGATAAAGTTGTTTCCTTCTGATTCCG
C

**SEQ ID NO: 294, Q8GWD1|Q8GWD1_ARATH Hypothetical protein At5g25490/T14C9_30 [*Arabidopsis thaliana*]**
MNRPGDWNCRLCSHLNFQRRDSCQRCREPRPGGISTDLLSGFGGRPVSSSFGFNTGPDVRPGDWYC
NLGDCGTHNFANRSSCFKCGAAKDEFSCSSAAATTGFMDMNVGPRRGLFGFGGSSSGGGGTGRSPW
KSGDWICPRSGCNEHNFASRSECFRCNAPKELATEPPY

**SEQ ID NO: 295, Q9SW92 gi|5679335|gb|AF171223.1| *Oryza sativa* putative zinc finger protein mRNA**
CCTAGTACTAAGAACAGCAACCACCTCTGAAAGCTTTACAAGTTCGCAGGGGCTTAACTGCGAATG
AACATCCAGAGGAAGCCAGGAGACTGGAACTGCAAATCGTGCCAGCATCTCAACTTCAGCCGCCGG
GACTACTGCCAGCGCTGCCATACCCCACGCCAGGACCTGCCGCTTGGCGATGGTTATGTCCCAGGT
GGTGTGCTGTCCTCCCTGGACATTCGCCCGGGCGACTGGTACTGCAACTGCGGCTATCACAACTTT
GCTAGCCGAGCAAGCTGCTTCAAATGTGGCGCCATTGTGAAGGACCTTCCAGCAGGCCAAGGTGGT
GGTGTTGCCAACGGTGACTTTGCCCGTGCCCTCGACAGCAGCGCAGTTCGTGCTGGGTGGAAGGCG
GGTGACTGGATTTGCACAAGGCCTGGTTGCAACGTCCACAACTTTGCAAGTAGGATTGAGTGCTAT
AGGTGCAATGCACCTAGGGAAGCAGGTAATGTGAAGTAAGAAAAGACTGACGCGACCAAAGATCGT
GAGACGTGACGAGCTGGCCGAGGATGAATAAAGTGACTGCTGTCAGTTGTCACATTCACAGGCTGC
GATCCAGAAACTATGGATGGGACTATGTAGTAACGTGCTGATATATTATTGCTAAGTGTTACTACT
GCATGGTTGCAAGGGTGGTAGAAGTACCTTAGCTTCCAAGATCGTTGTAATGTGTGAGTTTAATTT
TGGCGTTTTAAGTAATAAGTCTAGTGATTGCAGGATTGTACGGGGTAATGTACTTGCATTATCCAT
TATAATCTTAAGCATCCAATATAATTATGCAAAAAAAAAAAA

**SEQ ID NO: 296, Q9SW92_ORYSA Q9SW92 Putative zinc finger protein ZFP30 [*Oryza sativa*]**
MNIQRKPGDWNCKSCQHLNFSRRDYCQRCHTPRQDLPLGDGYVPGGVLSSLDIRPGDWYCNCGYHN
FASRASCFKCGAIVKDLPAGQGGGVANGDFARALDSSAVRAGWKAGDWICTRPGCNVHNFASRIEC
YRCNAPREAGNVK

**SEQ ID NO: 297, Q8RYZ5 (gi|20161685:150341-150912, 151518-151588, 151701-151997, 157408-157425) *Oryza sativa***
AAGCCTCCATCCATCCATCCATTCATCAGAGCTCAAGCTCAAGCAAGCAAGCTTGCTAGCTAGCTG
CTGAGCAGCATTCCAGTCTGCTCCAGCTAGCTCAGCTCTCTCTGCTCTGCTCTTTGCTCGATAACG
ACCATCATCTTCTTCTTCTTCTTCTTCTTCTTCTTCTTCTTCTTGTACATTCTATTTGCTCGA

FIGURE 25 (continued)

TAGATAGATAGATAGATAGATAGGTAGATAGAGATGGAGACGAAGGCGGCGGCGATGGCGATGAGG
AAGCCGGGGGACTGGAGCTGCAGGTCGTGCCAGTACGTGAACTTCTGCAAGAGGGAGGCGTGCCAG
CGGTGCGGGGAGGCGAAGCTCGGGGTGGAGCGGACGGACTACGCCGCCATGGGCGGCGGGTGGGAG
GTGAAGCCCGGCGACTGGTGCTGCCGCTGCTGCGCCGTCAACAACTACGCCAGCCGCGGCAGCTGC
TTCAAGTGCGGCGCCGCCAAGAACGACTCCGCCGCCGCCGTCGCCCAGGGCTGGGGCTTCTCCGTC
GCCTCCCAGGCCGGCTGGAAGAACGGCGACTGGATCTGCCCCAGAATGGAATGCAACGTGCAGAAC
TACGCTAACAGAACCGAGTGCTTCCGGTGCAATTTCCCCAGATACTACGTTGATTGATCTGACATA
TATGATATATGCCTTGCACTGAGAAGAAATGAAAGGGAAATAAAATTTACGAGATCGAAGATCGAC
GGACAAGATTGATCGGCCACCGGTCATGCCCTTCAGGGTTTTCTTTTTTCTATTTTTTTTTAGAAT
AACCCTCGATAACCTGAGGGTTTTTTTTCATTTGTAAGAGCGGTAACGGTACTAAAAAAACACAAA
AACGGCACAAAATTGTGGCTGATGACTGATCAGTCTTCCTCTTTTTTTTCCCAATAAAAGGCAGAT
AATTAAGAAAGCAAAATTATTAAAAAAAAAAAAA

**SEQ ID NO: 298, Q8RYZ5_ORYSA Q8RYZ5 Putative zinc finger transcription factor ZFP30 [*Oryza sativa*]**
METKAAAMAMRKPGDWSCRSCQYVNFCKREACQRCGEAKLGVERTDYAAMGGGWEVKPGDWCCRCC
AVNNYASRGSCFKCGAAKNDSAAAVAQGWGFSVASQAGWKNGDWICPRMECNVQNYANRTECFRCN
FPRYYVD

**SEQ ID NO: 299, ABD83289.1(gi|89953388:<880-1295, 1548-1618, 1895->1926) *Beta vulgaris***
ATGAGTAGGCCAGGTGATTGGAATTGTAGGTCATGCAGCCACTTGAACTTCCAAAGGAGGGACTCC
TGCCAGCGCTGTGGGGACGTACGTCCTGACGGCCGAGGCGGCGGAGGGGGAGGAGGAGACTTTGGC
AGTAGCTTTGGAGGGAGGTCAGGTGGGTCCCCCTTTGGTGGGGGTTTTGCAGGGCCCGATGTTAGG
CCCGGTGATTGGTATTGTAGCATTGGCAACTGTGGGGCCCACAACTTTGCCAGCAGGTCTAGCTGC
TTCAAGTGTGGGGCCTACAAAGAGGAAGCTGGCTGTGGTGATAGCATGGGCCGTTCACGTGGAGGA
TTTTCCTTTGGTGGCATCGGCGGTGGCGGTAGCGGTGCCGCTACCGGCCGCTCAGGCTGGAAATCC
GGTGACTGGATTTGCACTAGGTCGGGTTGCAACGAGCATAACTTCGCTAGTAGGACTGAGTGCTTC
AGATGCAGGGAACCAAGGGACTCCGGTAATGCAATGCTAAAGGAAGTACCATGCTGA

**SEQ ID NO: 300, ABD83289.1 *Beta vulgaris* GlimmerM protein 152**
MSRPGDWNCRSCSHLNFQRRDSCQRCGDVRPDGRGGGGGGGDFGSSFGGRSGGSPFGGGFAGPDVR
PGDWYCSIGNCGAHNFASRSSCFKCGAYKEEAGCGDSMGRSRGGFSFGGIGGGGSGAATGRSGWKS
GDWICTRSGCNEHNFASRTECFRCREPRDSGNAMLKEVPC

**SEQ ID NO: 301, Q8S8K1 gi|53828594|gb|BT015844.1| *Arabidopsis thaliana* At2g17975 gene**
ATGGGAGACGGAAGAGAAGGAGACTGGGAATGTTTAGGATGCAGAAACAGGAATTATGCGTTTAGA
TCATTCTGTAACAGATGCAAGCAGCCTCGTCTTATCATGGATAATAATACTTCTCCAAACTCTAAG
TGGCTTCCTCGTATCGGCGATTGGATCTGCACTGGTTGTACTAACAACAATTATGCATCACGAGAA
AAGTGCAAGAAATGTGGGCAATCTAAGGAAGTAGCAGCATTGTCAGCACTTGCTATCCCTGGAGCT
TCTCTTCAAACTCATCTCCATTACTTCACCCGTGGACCTGAGTCACATGATCAACCTGGTTCTTTA
CTCGCATTCTCTAACGCTACAAATCAAGCTTCGGTTCATAAAGAATGGAGGAGTGGTGACTGGATT
TGCAGATGTGGTTTTCACAATTATTCCTCTCGTATACAGTGCAAAAAGTGCAATGAAATAGCTCCA
CTAGCCCTTGGTACAAAGAGATTAGCATCAGAAGCTTTGGCTCATGAATGGGATAGCAAAAGACTG
AATCAAGGATATACAAGCATGCAAACACAGTCAGCGATATATGCATCTTTTCCTGGTATGAGCCTA
GGAAGGGTCTCAAATTGGCAACTTCCTCTCCCGTTTCTACAACAACACTCAACACCTGCTTTACTT
GGAATGGGAGTGAAACAATGGCGTGATGGCGACTGGATGTGTACAAATTGCAAGAATCACAATTAT
GCATCACGAGCAGAGTGCAATAGGTGCAAGACTACACGAGATATCCTTGATCAGGACATAACTCCA
ACAGAACAATCTTGA

**FIGURE 25 (continued)**

**SEQ ID NO: 302, Q8S8K1_ARATH Q8S8K1 Predicted protein [*Arabidopsis thaliana*]**
MGDGREGDWECLGCRNRNYAFRSFCNRCKQPRLIMDNNTSPNSKWLPRIGDWICTGCTNNNYASRE
KCKKCGQSKEVAALSALAIPGASLQTHLHYFTRGPESHDQPGSLLAFSNATNQASVHKEWRSGDWI
CRCGFHNYSSRIQCKKCNEIAPLALGTKRLASEALAHEWDSKRLNQGYTSMQTQSAIYASFPGMSL
GRVSNWQLPLPFLQQHSTPALLGMGVKQWRDGDWMCTNCKNHNYASRAECNRCKTTRDILDQDITP
TEQS

**SEQ ID NO: 303, Q8GZ43 gi|26449539|dbj|AK117220.1| *Arabidopsis thaliana* At1g67320 mRNA for unknown protein**
CTATTTCTCCTACCGTCGAACTAAATCCTAGGGCACAGGTGAAATCGCCGAGTGACGTCTAGCCAC
CGCACCGCCTCCTTCTCCGGTTATCTCGCTACTAATCAGACTATTCCACAGCTATGTCACAGGTAG
ACAACAGAAATTCATCAGCAGCCAAGCGTGCTAGAACTGACGGGGGGCGTAGAGAAGATGATTGGA
TCTGCCCAAGTTGTGGCAATGTCAACTTTTCATTCAGGACAACTTGCAATATGCGTAATTGCACTC
AGCCTAGACCTGCAGATCATAATGGAAAGTCTGCTCCCAAACCTATGCAACATCAACAAGGTTTCT
CATCACCCGGGGCATACTTAGGATCTGGGGGTCCCCCTCCAGTATATATGGGCGGGTCACCATATG
GATCTCCTCTCTTTAATGGATCATCTATGCCTCCTTATGACGTCCCATTTTCTGGGGGTTCGCCTT
ACCATTTTAACTATAATAGCCGAATGCCTGCCGGAGCTCATTACAGACCATTACATATGTCTGGAC
CACCACCATACCATGGCGGATCTATGATGGGAAGTGGTGGTATGTATGGAATGCCTCCACCAATAG
ACAGGTATGGCCTTGGTATGGCAATGGGTCCTGGTTCTGCCGCTGCCATGATGCCAAGACCAAGGT
TTTACCCAGATGAAAAATCACAAAAGAGAGATTCAACTCGCGATAATGATTGGACATGTCCGAATT
GTGGTAATGTAAACTTCTCATTCAGAACTGTATGTAACATGAGGAAGTGCAACACTCCAAAGCCTG
GTTCTCAGCAGGGTGGAAGCTCAGATAAAATATCCAAACAAAATGCACCGGAAGGGAGCTGGAAGT
GTGATAACTGTGGAAATATAAACTACCCATTCAGGAGCAAATGCAACAGGCAAAACTGTGGAGCTG
ATAAGCCTGGGGATCGGTCGAATGGATCTCCGTCCCGTGCACCAGAAGAGAACGATCAGTGAGTCG
TAGACATGTTTGGGGGTTGAGAAGGGTGAGTCTAATCCAGCGGGTGTCGTAATGTCAGTCAATGTC
TCGTCAGGTCAGGTCGTCCATGTTGCTGCGTCGTCAGTCAAGTTGCTTCTATGCATTTGTCTCTTT
ACTTCCCTTGCGTGGTTGTGGATTGTATTAATAATGCAAAAATTGTTTATTTACTTTGTCGTCCTC
GTGGATCTTGTCTTGCTATAGAATCCTCCTCCAGGTCCATACTGTTTTACATCCTAATCTTAAGTA
AGTTGGCACC

**SEQ ID NO: 304, YZR3_ARATH Q8GZ43 Hypothetical RanBP2-type zinc-finger protein At1g67325 [*Arabidopsis thaliana*]**
MSQVDNRNSSAAKRARTDGGRREDDWICPSCGNVNFSFRTTCNMRNCTQPRPADHNGKSAPKPMQH
QQGFSSPGAYLGSGGPPPVYMGGSPYGSPLFNGSSMPPYDVPFSGGSPYHFNYNSRMPAGAHYRPL
HMSGPPPYHGGSMMGSGGMYGMPPPIDRYGLGMAMGPGSAAAMMPRPRFYPDEKSQKRDSTRDNDW
TCPNCGNVNFSFRTVCNMRKCNTPKPGSQQGGSSDKISKQNAPEGSWKCDNCGNINYPFRSKCNRQ
NCGADKPGDRSNGSPSRAPEENDQ

**SEQ ID NO: 305, Q7F1K4 (gi|24460028:14730-14944, 15166-15211, 15311-15423, 15522-15807, 16234-16298, 16519-16570, 16651-16760, 17488-17518, 17730-17881, 19298-20080) *Oryza sativa***
CAGTTTCCACTTTCTTCCAAAGCCGAAAAATCGAGAGAGAAAGAGCAAAACCCTAACCGCGGCGCT
CCGCCCCCTTCCGCCGCCGGATTCCTCCTCCTCCTTCGTCCGCCTTCGCCGGCGCCGCCCTGCTCC
GAAGGAGCCCAGGCCTTGTCGCCGCGTCGCTTGCCCCGCCGCCGGTTTCAGCAGCAGCCACCCCCA
CCACCATGTCTTCTCAGGTCGACAACCGTAGCCAATCTGCTGGAAAGCGTGCCCGCACCGACGGTG
GGCGGCGTGAGGACGACTGGGTTTGCCCCAGCTGCCAGAACGTCAACTTCGCCTTCCGCACCACCT
GCAACATGCGCAATTGCAACCAATCCAGGCCCACCGACTATACGAAGGATATGCAGAAACCTATGC

**FIGURE 25 (continued)**

AGACACCGCCGCCCCATTTCCCCATGTCAGGGGGATACATGAGCCCAGGGACGCCGCCATCCATGT
ACCTCGGGGGTGGTGCTCCCCCTTATGGCACCTCCCTCTATGGCGGACCTGCTTTACCACGTTATG
GTATTGCTCAGTTCCCTGGGGGTTCTGGATATCCATATGGCTATGGTGGCCGCCTGCCAATGGGGA
GCCCCTATGGGCCGCCGATGCATATGGCAGGGCCTCCATATTCTGCTGGATCCATGATGGGACCAG
GTGGAATGTATGGGATGCCCATGGACAGATATAGCTTGGGCTTACCTGCTGGTCCTGGTCCAATGG
GCGCGAGGGCTGGTTCATATTCCGAGGAAGGATCCCAGAAGAAGCCTGCAGGAGCTGGGCGTGATA
ATGATTGGAAATGCCCTAATTGCAACAACATTAACTTTGCATTCCGGACAGTCTGTAACATGAGAA
AGTGCAATACACCAAGACCTGAAAACCAGGGGTCCAAACCTGATGGTGCAAGAGGTCCAAAACCAA
AGATGCCAGAAGGTAGTTGGAAGTGCGAGAAGTGCAATAACATAAACTATCCATTCCGCACAAAAT
GTAACCGTCCAAGTTGCGAGGCTGAAAAGCCATTTCAGACAAACAATGCTAATGAGTCATCTGCTG
ATCAGGACAATCAGTTGTTGTCATGTAATATTGTGAAGCTTTTATCAAAGCTGCAACTTTCACATG
ACTTTCAAGACAATAATGAGCAAACAAAGGTGGATCCTCCCGGTGGCCCTGCTGGAGTACCGACAA
GTTCGCACGCCCTGCGAATGGCAGCTCTCAGTGAGTTGCAGAACAGTGTTAAGAGCTGATACCCTT
TTCATTTTCCAAACAAGGTGAGAACTTTGATCCATGTGGATGTGCTTCTATCAGGAGCAGCTGACA
GTATTGCATGCAGTGATCTTGCTAAGGAAGTTGGAGATGGGAAACATCGTGTGATGGCCAGCCTTG
ATGTAAGAATGCAAATCAGTCTTACTCCGTACCATATCTGTGTGGAGTGTGCACACCGGAAGCATA
GTCATTTTTTGAATGGTCGATTGCTTCTTCCACAGTAAAGTCTATAATACCAGTGTGCAGGTGTTT
TTTTCCCTTTCTTTCAGTGTGAAGGTGTTTTTTTACCCCTTTCTTTCGTTCATTCATTCTTTTTCT
TTTTCCTTGTTGAGACGGTATCCTGGAGCACTTTGTAATACTCTAATGTGCTTCGGAAAGTAGAAT
ATAAGTTGTGCTTATGGGCGGGCCAAAATGCTTTGGCCGTTAGTTGCATGACAGCCTTTCCAACAT
GCTGCTGCCTAGCCGTAGTTCTCCCAAAAATGAAACCATCTTGAGATCCGTTTGCGGTTGAACTGA
AATTAGACGGTATCTCTGGTTTCAACATCATCCTTTAAACCCTGGATGAAAGATTTGCAATATTCC
TGCGG

**SEQ ID NO: 306, Q7F1K4_ORYSA Q7F1K4 P53 binding protein-like [Oryza sativa]**

MSSQVDNRSQSAGKRARTDGGRREDDWVCPSCQNVNFAFRTTCNMRNCNQSRPTDYTKDMQKPMQT
PPPHFPMSGGYMSPGTPPSMYLGGGAPPYGTSLYGGPALPRYGIAQFPGGSGYPYGYGGRLPMGSP
YGPPMHMAGPPYSAGSMMGPGGMYGMPMDRYSLGLPAGPGPMGARAGSYSEEGSQKKPAGAGRDND
WKCPNCNNINFAFRTVCNMRKCNTPRPENQGSKPDGARGPKPKMPEGSWKCEKCNNINYPFRTKCN
RPSCEAEKPFQTNNANESSADQDNQLLSCNIVKLLSKLQLSHDFQDNNEQTKVDPPGGPAGVPTSS
HALRMAALSELQNSVKS

**SEQ ID NO: 307, Q7XHQ8 (gi|33146980:c128631-128484, c128334-128283, c127038-126920, c125678-125438, c125352-125285, c125195-125123, c124539-124427, c123679-123416) *Oryza sativa***

CTGGTCTCTCGCTCTCTTCGCTCGCGGCGGCTTCTCCGCCTCCGCCTCCGCCGCCCTCTCCTCCTC
TCGCCTCGCCGCCGGCGCCGCCAGCCACCGCGCGGGTCGGGCGGGCCGTATCTTCCTTTCTGTTCC
GATGGCTTCCGCGAAGGTGGAGAACCGCGGCGGCGGTGGGTTCGGTTCGAAGAGGTCACGCAACGA
CGTGTCTGTAAGGGAGGGGGACTGGACTTGTCCTCAGTGTGGTAATGTCAACTTCAGTTTTAGAAA
TGTTTGCAACCGCGGAGCCTGTGGTGCACCCCGTCCATCACCGAGTCTAAGCCCAAGAGTGCCACC
TCCTCCTGCTGCTGGATATGATCGGCCACATCTTGGATATGATCGGCCACATCTGTTTTATGGTAG
TGCTGGCACCCCACCTCCTATTCCTCTTGGATCTGGTAGCTATGGTGCCCCCTATCCACATCTTGG
CTTGCGGTATGGATATGGTCCACCAGTAGGACCTCCTGCTTCATATGGCCTTTTTTCTTCTTATGG
TCAACCTGGACCAATGGGCAGTCCGATGGGAGGCATGGGCTATGGCCCTGGACCTGAGCTAGGCCG
ATATGGTTATGGTTTTAGAGGATCTCCAATGCCGGTTTCTAGCCCATGGTCTGGTGGAGCATTAGT
GGAAAATAATGACAGCTCTGCTTCACGCAAGCGTCGTGGAGGCCCAGATGGAATGGCTGAGAATGA
CTGGATCTGCCCAAAGTGTGAGAATGTCAACTTTTCCTTCAGAAACAGTTGCAATATGAAGAAATG

**FIGURE 25 (continued)**

TGGAGCTCCAAGGCCAAGCCCTGGATCTAATGCTACCCCATGTCGCAAAGACAAGGACGCTCCGGA
AGGGAGCTGGACCTGCCCGGAGTGCAACAACCTGAACTACCCTTTCCGCACGGCGTGCAATCGGAA
AGGCTGCGGAAGCAGCAGGCCGGCAGCGGCCACGGCGAACTAGGACCCTACTAACTTTGCGCCGGC
GATTGGGGGCAGAGAGAGTATTGTTGTATCCTAGCTATATACAAGTTAATTTAAACTGTAAACGGC
TCAAATATATTTTCTTGTGGCC

**SEQ ID NO: 308, Q7XHQ8_ORYSA Q7XHQ8 Putative p53 binding protein [*Oryza sativa*]**
MASAKVENRGGGGFGSKRSRNDVSVREGDWTCPQCGNVNFSFRNVCNRGACGAPRPSPSLSPRVPP
PPAAGYDRPHLGYDRPHLFYGSAGTPPPIPLGSGSYGAPYPHLGLRYGYGPPVGPPASYGLFSSYG
QPGPMGSPMGGMGYGPGPELGRYGYGFRGSPMPVSSPWSGGALVENNDSSASRKRRGGPDGMAEND
WICPKCENVNFSFRNSCNMKKCGAPRPSPGSNATPCRKDKDAPEGSWTCPECNNLNYPFRTACNRK
GCGSSRPAAATAN

**SEQ ID NO: 309, CX272690b 39RDBRT_UP_043_G09_21JUL2004_067 *Brassica rapa* 39RDBRT Brassica**
GGTCTTGTTTCTTTCAATAAACACATAATAAGATAGCTTCCAAGTTCATCAAGAAATAAACGTAA
GCGCACGCATACAATAACCTATCGAAGATGAGCAGACCCGGAGACTGGAACTGTAGATCATGCACC
CACCTCAACTTCCAGCGCCGTGATTCTTGCCAGCGATGCGGTGACTCCCGTTTGGGTGCAGGTGGA
GTCGGTGGCTTAGAGTTTGGTGATTTCGGCGGCAGAGGTATGTCTGCTTTTGGATTCACCACGGGC
TCCGACGTTCGTCCAGGTGACTGGTACTGCACAGTTGGAAACTGCGGGACACATAACTTTGCCAGC
CGCTCCACCTGCTTCAAATGCGGCACTTTCAAGGACGAATCCCTCGGTGGGGGCGGCGGCGGTGGC
GTAGGCGTAAGGCGGTCCGGTCATGTTGACGCTGACGTTATGCGGTCTAGAGTCTCCGGCAACGGT
GGCCGCTCCAGCTGGAAATCCGGTGATTGGATTTGCACCAGGCTTGGTTGCAATGAGCATAACTTT
GCAAGCAGAATGGAGTGCTTCAGATGCAATGCACCAAGGGACTTCAGCATGAGAACCTCTTTCTAA
GTTACACAATAATGCCTTTGAAGTAGCCTTGTTTCCAATCTCTTGGGCACCGTTTCAATCAATGGA
AA

**SEQ ID NO: 310, CX272690_1 [94 - 591] 39RDBRT_UP_043_G09 21JUL2004 067 *Brassica rapa* 39RDBRT Brassica**
MSRPGDWNCRSCTHLNFQRRDSCQRCGDSRLGAGGVGGLEFGDFGGRGMSAFGFTTGSDVRPGDWY
CTVGNCGTHNFASRSTCFKCGTFKDESLGGGGGGGVGVRRSGHVDADVMRSRVSGNGGRSSWKSGD
WICTRLGCNEHNFASRMECFRCNAPRDFSMRTSF

**SEQ ID NO: 311, DV143669 CV03134A2H09.f1 CV03-normalized library *Euphorbia esula* cDNA clone**
ATTTTCCCTTCTTCACCCAAATATCTCTCACCTTTTAACAAGAGAAAAGCAAAAAGAAGATGAGCA
GACCAGGAGATTGGAACTGCAGGTCCTGCCAGCATCTCAACTTCCAGAGAAGGGACTCGTGCCAGC
GCTGTGGGGACTCCAGGTCCGGGACTGGAGGTTCAGGAGCTGACTTTGGCGGGTTTGGAAGCCGGG
TTGGGTCCTCATTCGGGTTCAGCACCGGGTCTGATGTTCGACCCGGTGACTGGTACTGTACTGCTG
GCAACTGTGGGGCCCACAACTTTGCCAGCCGGGCAAGTTGCTTCAAATGTGGAGTTTATAAGGATG
ATTCTGGGGCCCCTGGCGGGTTTGATTCCGATATCCTCCGGGCCTCTAGAGGTTTTGGTAGTGGCA
GCAATCGCTCTTCTTGGAAATCTGGTGATTGGATCTGCACTCGGTGGGGATGTAATGAACACAACT
TTGCAAGCAGAATGGAGTGTTTCAAATGCAGTGCCCCTAGGGACCTTAGTAACAGAACTTCATACT
AGACATTTTGGATGGTGCAGCAGCCAAGAAAGAGAATTAAGATTACTACTTTTAAATTTTATTTTA
TTTTTACTATTTGTTTTGGGTGTTATTAGAGTGGAAAACAAACATGGCTTAAAAACCCATGTTTCA
TCCCTAATTTAAGCTAAGGAAGCAAACCCCTTTTGGCATTTTGTAAGGCAGATTTAGGATTGTAGT
ACTTAATTAATTAGTGGGTTGTT

## FIGURE 25 (continued)

**SEQ ID NO: 312, DV143669_1 [60 - 527] CV03134A2H09.f1 CV03-normalized library *Euphorbia esula* cDNA clone**
MSRPGDWNCRSCQHLNFQRRDSCQRCGDSRSGTGGSGADFGGFGSRVGSSFGFSTGSDVRPGDWYC
TAGNCGAHNFASRASCFKCGVYKDDSGAPGGFDSDILRASRGFGSGSNRSSWKSGDWICTRWGCNE
HNFASRMECFKCSAPRDLSNRTSY

**SEQ ID NO: 313, DR459119 CM072H11 Cotton Lambda Zap Express Library *Gossypium hirsutum* cDNA**
CTCCATTCTCTTATTTAACTCAAACTCACCACCTTTTCCATTTTTCTAATTAAGAAAAGAAAAGAT
GAGCAGGCCAGGAGATTGGAACTGCAGGTCGTGCCAACACCTAAACTTCCAAAGGAGGGACTCCTG
CCAACGCTGCGGGGAATTCCGGTCGGGTGATCACTTCGGTAGCTACGGTGGTGGCAGGGGTGGCTC
CTCCTTTGGATTCGCCACCGGCTCCGACGTCCGACCTGGTGATTGGTACTGCACTGCGGGAAACTG
CGGTACCCACAATTTCGCCAGTCGTTCCAGCTGCTTCAAATGTGGTGCATTCAAGGACGACCCTGC
CGGAGGTTTCGACAGCGACGTTCCGCGTTCTAGAGGATTTGGCGGCGGTAATCGATCCGGCTGGAA
ATCCGGCGACTGGATATGTACCAGGTCGGGATGCAATGAGCATAACTTTGCTAGCCGAATGGAATG
TTTCAGATGCAGTGCCCCAAGAGACTTCACCGCTAGAACTTCATACTAAATACAAGCTATCCAGTT
TTGGGTGTTGCAAACCAAGAGAGACTCAAATGAGAGAAAAAATTTTACGGGGTTAGGGTTACCCGG
GTAAATATATGGCTTGGGGGGGTGTGGCACCAGGGTTGAAAACACCAGGGTG

**SEQ ID NO: 314, DR459119_1 [65 - 508] CM072H11 Cotton Lambda Zap Express Library *Gossypium hirsutum* cDNA**
MSRPGDWNCRSCQHLNFQRRDSCQRCGEFRSGDHFGSYGGGRGGSSFGFATGSDVRPGDWYCTAGN
CGTHNFASRSSCFKCGAFKDDPAGGFDSDVPRSRGFGGGNRSGWKSGDWICTRSGCNEHNFASRME
CFRCSAPRDFTARTSY

**SEQ ID NO: 315, DV221228 VVI193G05_614118 CabSau Flower Stage 12 (FLOu0012) *Vitis vinifera***
GGTCTTCTTCCATCTCTGCACAGTTATCCTGATATTTCCTTGGAAAACATGAGCAGGCCAGGAGAT
TGGAACTGCAGGTCATGCCAGCACATGAACTTCCAAAGGCGCGATTCCTGCCAACGCTGCGGTGAC
CCAAAATCGGGTGGGGGTGACTTTGGAAGCTTTGGTGGGAGGGGTGGATCCTCCTTTGGGTTCACG
GGCTCGGATGTCCGCCCAGGGGACTGGTACTGCAATGCAGGCAACTGTGGAGCTCACAACTTTGCT
AGCCGCTCTAACTGCTTCAAGTGTGGTGCATTCAAAGATGAGTCTGCTGGGGGCTACGATTCCGAC
ATGTCACGCTCCCGAGGTTTCGGGTTCGGCGGTGGCAGCGGCCGGTCTGGGTGGAAATCCGGTGAT
TGGATATGCAGCAGGTCTGGATGCAATGAGCACAACTTTGCTAGCAGAATGGAATGTTTCAGATGC
AATGCCCCGAGGGACTTGAGTAACAAAACTTCATACTAGACATATATCTTCCATTTTTGGGTACTG
CAGCCAGCCAAGAGAGACCAAATCATAGAATATCTATTAATCCTTGTGTTGTTATTAATTTCTTTG
CTTTGGGTGCTAGGTTCTT

**SEQ ID NO: 316, DV221228_2 [49 - 498] VVI193G05_614118 CabSau Flower Stage 12 (FLOu0012) *Vitis vinifera***
MSRPGDWNCRSCQHMNFQRRDSCQRCGDPKSGGGDFGSFGGRGGSSFGFTGSDVRPGDWYCNAGNC
GAHNFASRSNCFKCGAFKDESAGGYDSDMSRSRGFGFGGGSGRSGWKSGDWICSRSGCNEHNFASR
MECFRCNAPRDLSNKTSY

**SEQ ID NO: 317, DT496999 WS01125.BR_D02 PT-P-FL-A-2 *Populus trichocarpa* cDNA clone**
TCAGACCATTTCCCTTTCATCTTCTAGCTAGCATAGCTTCTCTCAGAGGTTTTTGAAACCCCTTTT
TGCCATTCTTCTCTCACTTAGTATACTTAGTCTTCTCTAATCAATTCATAAGCAAATATGAACAGG
CCAGGAGACTGGAACTGCAGGTCATGCCAACACCTCAATTTCCAGAGGCGTGACTCTTGCCAACGT

**FIGURE 25 (continued)**

TGTGGGGACCCCAGGTCCGCAGGTGATTTTGGGGGTTTCGGTGGGCGGGGTGGCTCATCACTTGGG
TTCACCGGGTCGGATGTTCGTCCCGGTGATTGGTACTGCACTGCCGGAAACTGCGGGGCCCACAAC
TTTGCTAGCCGTTCTAGTTGCTTCAAATGTGGAGTGTACAAGGAAATGGACTCCGCCGGGGGCTTC
GATTCTGATTTTTCTCGAACTAGAGGGTTTGGTGGGAGCACTGGAGGTGGCAATCGATCTGGATGG
AAATCCGGAGACTGGATTTGCACTAGGTGGGGATGCAACGAACATAACTTTGCTAGCAGAATGGAG
TGCTTCAAGTGCAATGCCCCAAGAGATCTTAGCAACAGAACTTCATACTAGATACAATTTCTCCAT
TTCCTGGGACTGCACCAGCCAAGAACAAAGACAACATGATTAAAAAATATATATCACTACTTTTCA
TTTTCTTCTTGATTTCTTTTTGTATTAGCTAGCTAGGGTCTTGAGGCGAGACATGGTTTAAGAACC
ATGTTTACTGCTTTGAGCTATATATAACCCTTGGCATTTTGTCAGGCTTCCTGTAGGAAGTATATC
GTTGTCTTTGTGGGCTTTTGATCTATTATATTCATTATTGTAACCAAGGGAG

**SEQ ID NO: 318, DT496999_1 [124 - 576] WS01125.BR_D02 PT-P-FL-A-2 *Populus trichocarpa* cDNA clone**
MNRPGDWNCRSCQHLNFQRRDSCQRCGDPRSAGDFGGFGGRGGSSLGFTGSDVRPGDWYCTAGNCG
AHNFASRSSCFKCGVYKEMDSAGGFDSDFSRTRGFGGSTGGGNRSGWKSGDWICTRWGCNEHNFAS
RMECFKCNAPRDLSNRTSY

**SEQ ID NO: 319, DT457997 GH_ON35G17.r GH_ON *Gossypium hirsutum* cDNA clone GH_ON35G17 3'**
CCTTGCGTCCGCTTTCTTCTTCATTGTTCCTGTCTTTGCTTACTCACCCCTTATATATCTGCTTTC
TCAAGTAGGGGAGAAAATATGAGCAGGCCAGGAGACTGGAATTGCAGGTCATGCCAACACCTCAAC
TTCCAAAGGAGGGACTCATGCCAGCGCTGTGGAGAACCAAGACCTGGTGGTGGTGACAGAGGCGGC
GACTATGGAAGCTTTGGTGGCAGGGGTGGCTCATCTTTCGGGTTTACTGGACCCGATGTTAGGCCT
GGTGACTGGTATTGCACTGTGGGCAACTGCGGTGCTCACAACTTCGCCAGCAGGTCGAGCTGCTTC
AAATGTGGTGCGGCCAAAGATGAATCATCCGGAGGATTTGAAAGTGACATCCCACGTATGAGGGGT
TATGGTTTTAGCACTGGCAGCTCTAGTCGCTCTAACTGGAAATCTGGAGACTGGATTTGCACCAGG
TCGGGTTGCAATGAACACAACTTCGCCAGCAGGATGGAATGTTTCAGATGCAATGCACCAAGGGAC
TCCACCCACAAATCTTCATACTAATTAATAAAATTTTCATTTTTGGGTACTGCAGTCGCAAGAGAG
AGATCAGACAAGGCAATCCCAGATCTTGCTTTCTTTTTCTTTTGTTTGTTTCTTTATTTTAGATCT
TTAGATGAATCATGTTTGAAAGACTTCAGTTGAAGTACTTAAGCTAATATCAAAGTACATAGGGCA
TGCATGTAATTGATGTATGGTATCAGCAATGTTATATCAGTGTCTTTGTGCCAAAAAAA

**SEQ ID NO: 320, DT457997_1 [85 - 549] GH_ON35G17.r GH_ON *Gossypium hirsutum* cDNA clone GH_ON35G17 3'**
MSRPGDWNCRSCQHLNFQRRDSCQRCGEPRPGGGDRGGDYGSFGGRGGSSFGFTGPDVRPGDWYCT
VGNCGAHNFASRSSCFKCGAAKDESSGGFESDIPRMRGYGFSTGSSSRSNWKSGDWICTRSGCNEH
NFASRMECFRCNAPRDSTHKSSY

**SEQ ID NO: 321, DW105125 CLRX2940.b1_G16.ab1 CLR(XYZ) lettuce serriola *Lactuca serriola* cDNA**
GTATTCGGAATCCTCAAAGAAACACACACTTCCTATCCATCACAAGAAAGATGAGCAGGCCAGGAG
ATTGGAACTGCAGGTCATGCCAGCACTTGAACTTCCAGAGGAGGGACTCTTGCCAAAGATGTGGGG
AGACGAGGTATGGTGGTGGGGGTGGTGTGTTTGGTGGTAGAGGAAGTATCATCAGCCCTTCAGCAT
TTGGCTTCACAGGCCCAGATGTCCGACCGGGTGATTGGTACTGCAATGTTGGCAACTGCGGGGCTC
ACAACTTTGCTAGCCGCTCGAGCTGCTTCAAGTGTGGTGCGTTCAAGGATGACTTAGCTTGTAGTG
GTGGTGGTGGTGGTGGTGGTGGCGTTTTTGATGGTGATATGTCACGTGGCAGGGGTTTCGGGTTTG
GTGGAGGAAGTGGTGGTGGAGGTGGTGGCAGCAGCCGTTCAGGGTGGAAGTCCGGTGACTGGATAT
GCGGCAGGCCTGGTTGCAATGAGCACAACTTTGCAAGCAGAATGGAATGTTTTAGGTGCAACGCAC

**FIGURE 25 (continued)**

CTCGGGAATCGGGTAACAAGTCTCCTTATTAAGCAGGTTGCGGCATTTCCAGTTCCGGGTATCATG
GACTTGCTATCTACCAAACAGGAAGAGAGATAAGTGATCGATCAGACAGAATCATGAAGAGCATCC
AGTTGATAGGAAATTCTAAACTGACCCCCTTTTTGCCCATATCATAATATGCAATCCTATCTTTGA
TTTTTTTTTTCCTTTTGTTTTATTTTTTTTTTTT

**SEQ ID NO: 322, DW105125_1 [51 - 557] CLRX2940.b1_G16.ab1 CLR(XYZ) lettuce serriola Lactuca serriola cDNA**
MSRPGDWNCRSCQHLNFQRRDSCQRCGETRYGGGGGVFGGRGSIISPSAFGFTGPDVRPGDWYCNV
GNCGAHNFASRSSCFKCGAFKDDLACSGGGGGGGGVFDGDMSRGRGFGFGGGSGGGGGGSSRSGWK
SGDWICGRPGCNEHNFASRMECFRCNAPRESGNKSPY

**SEQ ID NO: 323, BG238374b sab50c06.y1 Gm-c1043 *Glycine max* cDNA clone GENOME SYSTEMS CLONE**
TTTCTGCATCTCTAATACTTCAACTCACTGTATTATCTTGAGTAATTTTGCAGAGAAGTAAAATAT
GAGCAGGCCAGGAGACTGGAATTGCAGGTCGTGCCAGCACCTGAACTTTCAGAGGAGAGACTCATG
CCAGCGATGTGGGGACTCAAAATATGGAGATAGAGTTGTTGATTTTGGTGGTTTTGGAGGAAGAGG
AGGGTCCTCATTTGGTTTAACTGGCTCAGATGTTCGCCCCGGCGACTGGTACTGTGCTGCTGCTAA
CTGTGGTGCACACAACTTTGCTAGCCGCTCAAGCTGCTTCAAGTGTGGTGCTTTCAAGGATGACTT
GGCTGGAGGAGGCTATAACAGTTCTGACATCTTGCGCTCCAGAGCTTTTGGTGGCAGTGGAAGACC
TGGATGGAAATCTGGTGATTGGATATGCAGCAGATCAGGATGCAATGAGCACAACTTTGCTAGCAG
AATGGAATGTTTTAAATGCAGTGCTCCCAGGGACACGTACTAGAAAAAAATGAGTT

**SEQ ID NO: 324, BG238374b [65 - 502] sab50c06.y1 Gm-c1043 *Glycine max* cDNA clone GENOME SYSTEMS CLONE**
MSRPGDWNCRSCQHLNFQRRDSCQRCGDSKYGDRVVDFGGFGGRGGSSFGLTGSDVRPGDWYCAAA
NCGAHNFASRSSCFKCGAFKDDLAGGGYNSSDILRSRAFGGSGRPGWKSGDWICSRSGCNEHNFAS
RMECFKCSAPRDTY

**SEQ ID NO: 325, DT494117 WS01117.BR_H13 PT-P-FL-A-2 *Populus trichocarpa* cDNA clone**
GACAAGTTCCTTAAACAATCTATTGCTCTTTCAACTACTAGCTAGGTTACACTTCATTTCTGTACG
TGTTAATTCTCCTATCTTTCTCCTTTCTGCTTTCTCGAGATGAGCAGACCAGGAGATTGGAATTGC
AGGTCATGCCAGCACTTGAACTTTCAAAGGAGGGACTCATGCCAGCGTTGCGGTGATCCAAGGCCC
GGAGAGAGAGATCACTATGGAAGTTTCGGTGGAAGATCATCAGGGGGCTCATTCGGATTTACGGGC
CCTGATGTTAGGCCTGGTGATTGGTATTGCACGGCTGGCAATTGTGGAGCTCACAACTTTGCTAGT
CGTTCAAGCTGCTTCAAGTGTGGTGTGTCCAAGGATGAATCCTCTGGTGGTGGACTTGATGCTGAT
ATGTCACGGATGAGAGGTTATGGCTTCGGCGGAGGCGGAGGCGGAGGCAGTGGCTCTAGCCGTAAT
TGGAAATCCGGAGACTGGATTTGCACCAGGTCCGGTTGCAACGAGCACAACTTTGCTAGCAGGACT
GAATGCTATAGATGCAATGCACCAAGAGAATCTAGCAGCAACAAGTCTTCGTATTAATCATCGATA
TCGATAGCATTTTTGTGTCCTGCAGTGCTGCAAGGAGGGAATTAACGAAGAAGGCTCATGAGAAAT
CTTGGATTCATCTTTTTGCTCTTAATTACTTCTATTTTTGTTCTTTTGGATAGCTGTACTCTTAAG
CTGAGAAGCTTTAGAAGGATCATGGGAGTGAAATTAAGTTCAAGGAGAGCTATATATATATCATTA
GCCAGTAATTTGTCAGGTTCCCTAAATATAGACATGTAGTTCTGTACTTGGTATCAATGTCTTTGT
GTTTTGAGACGGGTTTAAGCCCTTGTCGTTC

**SEQ ID NO: 326, DT494117_1 [106 - 582] WS01117.BR_H13 PT-P-FL-A-2 *Populus trichocarpa* cDNA clone**
MSRPGDWNCRSCQHLNFQRRDSCQRCGDPRPGERDHYGSFGGRSSGGSFGFTGPDVRPGDWYCTAG
NCGAHNFASRSSCFKCGVSKDESSGGGLDADMSRMRGYGFGGGGGGGSGSSRNWKSGDWICTRSGC
NEHNFASRTECYRCNAPRESSSNKSSY

**FIGURE 25 (continued)**

**SEQ ID NO: 327, DV465465b MTUNUL1.P4.A03 NUL *Populus fremontii* x *Populus angustifolia* cDNA**
AGGGAAAAAGAAAGCCTAACCCTCCTGCCTGCCTCAAGCTTACCCCATTATCGAGGTCTAAAACAA
GTATAAAAACAGCCTTAGCCCCAATACGTAAATCACAAGTTCCTTAAACAATCTATTGCTCTTTCA
ACTACTAGGTTTCCCACTTCATTTCTGTGCCTACTGATTCTCCTCTCTTTCAACTTTCTGCCTTCT
CAAGCTAGCTAGAGAAGGGAAGATGAGCAGACCAGGAGATTGGAATTGCAGGTCATGCCAACACTT
GAACTTCCAGAGGAGGGACTCGTGCCAGCGTTGTGGGGACCCAAGGCCCGGAGAGAGAGATCATTA
TGGAAGTTTTGGTGGAAGATCAGGGGGCTCGTTCGGATTTACGGGGCCTGATGTTAGGCCCGGTGA
CTGGTATTGCTCGGTTGGCAACTGTGGAGCTCACAACTTTGCTAGTCGTTCAAGCTGCTTCAAGTG
TGGTATGTCCAAGGATGAATCCTCTGGTGGTGGCTTGATGCTGACATTTCATGGATGAGAGGTTA
TGGCTTCGGCGGAGGCAGCGCCTCTAGCCGCTCTAATTGGAAATCCGGAGACTGGATTTGCACCAG
GTCAGGTTGCAACGAGCACAACTTTGCTAGCAGGACTGAGTGTTACAGATGCAATGCACCAAGAGA
ATCAGGCAGCAACAAGTCTTCGTATTAATCATCAACATCGATCGCATTTTTGTGTACTGCAGT

**SEQ ID NO: 328, DV465465b [221 - 685] MTUNUL1.P4.A03 NUL *Populus fremontii* x *Populus angustifolia* cDNA**
MSRPGDWNCRSCQHLNFQRRDSCQRCGDPRPGERDHYGSFGGRSGGSFGFTGPDVRPGDWYCSVGN
CGAHNFASRSSCFKCGMSKDESSGGGLDADISWMRGYGFGGGSASSRSNWKSGDWICTRSGCNEHN
FASRTECYRCNAPRESGSNKSSY

**SEQ ID NO: 329, AY219846 *Oryza sativa* (japonica cultivar-group) zinc finger transcription**
CACCTCACAGCATTTTCCACCTTAGAGCTCACCAACACAGCAGCTGATACTTGTTTAGGGTAAGAC
AAGATGAACAGGAAGCCAGGAGACTGGGACTGCAGGGCGTGCCAGCACCTCAACTTCAGCCGCCGG
GACCTATGCCAGCGCTGCGGCGGGCCGCGTGGCGCCGCTGATCGCGGCAGCGGTGGTGGCGGTGAC
TACGCCAACTTCGGCGGCCGTGGTGGTTCCTCCTTCGGTGGAGGCTTTGGCACTGGCTCTGATGTC
CGCCCAGGTGACTGGTACTGCAACTGCGGCGCGCACAACTTCGCCAGCCGCTCCAGCTGCTTCAAG
TGCGCTGCTTTCAAGGACGATGCTGCCGTCAACAGTGGCGGCGCTGGTGCCTTTGACGGTGGGGAC
ATGTCGCGCTCGCGGGGCTACGGCTTCGGCAGCGGCGCCGTCCGCGCCAGCCGCCCTGGCTGGAAG
TCTGGCGACTGGATTTGCACCAGGTCTGGATGCAATGAGCACAACTTCGCCAGCAGGATGGAGTGC
TTCAGGTGCAACGCACCGCGGGACTCCGGCACTGAGGTGTAATTTGCCGTACGTGTCCGATCGATC
TGGATCCGATGAGGCTTGCAGCAGTGACGACGAGCAGCAGAAGCAGCGTTAAGAGTTGTGATGTCT
ACATAAGAAGAAGAAGAAAGTAGAATGCAAAAGAAATCTCCCCATGGTTTTACTAGTTTTGTTTCT
TCCCGTTTTAGATTTGGTTCTGATTCCCATTTGGGAGGACCCGTCGACCCCTGATTATCTATGTTT
TACCCGTTTTATTTCCTGTTTCTTTCGGCATGTTTGCTCTTCGATCGAGTCGTGTAACCCGAAACG
CTTGCGCTTGAGAAGTATTATTATTATTAACTAGTATGTTGCTTCTTAAAAAAAAAAAAAAA

**SEQ ID NO: 330, AY219846_1 [70 - 567] *Oryza sativa* (japonica cultivar-group) zinc finger transcription**
MNRKPGDWDCRACQHLNFSRRDLCQRCGGPRGAADRGSGGGDYANFGGRGGSSFGGGFGTGSDVR
PGDWYCNCGAHNFASRSSCFKCAAFKDDAAVNSGGAGAFDGGDMSRSRGYGFGSGAVRASRPGWKS
GDWICTRSGCNEHNFASRMECFRCNAPRDSGTEV

**SEQ ID NO: 331, DN152082 5228_H11_P22 Switchgrass callus cDNA library *Panicum virgatum* cDNA**
CCACGCGTCCGCAGAACTCCTTGTCCTAGGCCCCAGAAACACAAACGACCGCTCTTTGTTTAGGCA
AGATGAACAGGAAGCCTGGAGACTGGGATTGCAGGGCTTGCCAACACCTCAACTTCAGTCGGCGGG
ACCTATGCCAGCGCTGTGGTGAGCCACGTGGAGCTGCTGACCGTGGCAGCGGCGGTGGAGGTGACT
ATGCCAACTTCGGTGGCCGTGGTGGCTCCTCCTTCGGCGGTGGCTTTGGTGCTGGCTCTGATGTCC

## FIGURE 25 (continued)

GCCCTGGTGACTGGTTATGTTCCTGCGGCGCGCACAACTTCGCCAGCCGCTCCAACTGCTTCAAGT
GCTCTGCCTTCAAGGAGGAGGCTGCTGTCAACAGTGGTGCTGGTGGCTTTGATGGTGACATGTCAC
GCTCGCGCTACGGCTTCGGTGGCGGTGCTGCCCGCACCAACCGCCCTGGTTGGAAGTCTGGAGACT
GGATCTGCACCAGGTCCGGATGCAACGAGCACAACTTTGCCAGCAGGATGGAGTGTTTCAGGTGCA
ACGCACCACGGGACTCCGGCACTGAGGTGTAGGATCGGAGCTGGTGCTTCGAACGGACCCCGACGA
GCAGAAGCAGCAAGAAACCTGATAAGAAGAGTAGTAGATTTGCAAAAGGCATCCCGGATGCTCTAT
TACTGTTTTGTTCCACCCCTACCGCTTCCTTTTAGAATTTGGT

**SEQ ID NO: 332, DN152082_1 [69 - 557] 5228_H11_P22 Switchgrass callus cDNA library Panicum virgatum cDNA**
MNRKPGDWDCRACQHLNFSRRDLCQRCGEPRGAADRGSGGGGDYANFGGRGGSSFGGGFGAGSDVR
PGDWLCSCGAHNFASRSNCFKCSAFKEEAAVNSGAGGFDGDMSRSRYGFGGGAARTNRPGWKSGDW
ICTRSGCNEHNFASRMECFRCNAPRDSGTEV

**SEQ ID NO: 333, *Medicago truncatula* (barrel medic) Zinc finger, RanBP2-type (Q1RWK5)**
ATGAACAGGAAAATGAGCTGGTCTGGAGGAGATTGGATGTGTGGTGCTTGCGAGCACATAAATTTC
AAGAAGAGAGAAGCATGCCAAAATTGTGGATACCCAAAGTATGGAGGCCCTGACCCATCGACCTAT
AGATATAACAGGACTGAAACGTTGGCAGGGGACTGGTTTTGCACTTCTATGAACTGTGGAGCTCAC
AACTATGCAAGCCGATCAAACTGCTATAGATGTGGTGCATTTAAAGATCCTTATTCTTCTGGATAT
GGGGGTAACATGGTGGGTTCTGGAGGATATGGATCAGATTGTAGTTCTCCCCCAGGATGGAAAAGT
GGAGACTGGATTTGCCCTAGAATTGGCTGTGGAATCCATAATTATGCAAGCAGGACAGAGTGCTAC
AAATGCAAAATGCCAAGGGATTATGGTGGTGCAGACTGA

**SEQ ID NO: 334, tr|Q1RWK5|Q1RWK5_MEDTR Zinc finger, RanBP2-type - *Medicago truncatula* (Barrel medic)**
MNRKMSWSGGDWMCGACEHINFKKREACQNCGYPKYGGPDPSTYRYNRTETLAGDWFCTSMNCGAH
NYASRSNCYRCGAFKDPYSSGYGGNMVGSGGYGSDCSSPPGWKSGDWICPRIGCGIHNYASRTECY
KCKMPRDYGGAD

**SEQ ID NO: 335, *Medicago truncatula* (barrel medic) Zinc finger, RanBP2-type (Q1S406)**
ATGAGCAGACCAGGAGATTGGAACTGCAGGACATGCAACCACCTCAACTTTCAAAGAAGAGAATCT
TGCCAACGATGTGGGGAGTCAAGAATGACTTCTGGCTGCGGCGCCGTTGATTTTGGTGGCTCCTTT
CTTGGTGGAAGAGGCTCTAGCTCCCCTTTTCCTTTCACCACCGGCCCTGATGTCCGTCCTGGTGAC
TGGTATTGCACTGTTGGAAACTGTGGAGCTCACAACTTTGCCAGCCGCTCCAGCTGCTTCAAATGT
GGTGCCCCTAAGGATATTGATACCTTCTCCTCTGACTCCTCAGACATGCCACGATTATTGAGATCA
CCATACGGTTTTGGAGCAGGCAGCGCTGGTGGCGGTGCCTCCACTCGCCCCGGCTGGAAATCCGGT
GACTGGATATGCACCAGGTCTGGGTGTAACGAGCATAACTTCGCCAATAGAATGGAATGCTACCGA
TGCAACGGTCCAAGGGACTCTAGTACTGGAAGATCTTCCTATTTATCGTGA

**SEQ ID NO: 336, tr|Q1S406|Q1S406_MEDTR Zinc finger, RanBP2-type - *Medicago truncatula* (Barrel medic)**
MSRPGDWNCRTCNHLNFQRRESCQRCGESRMTSGCGAVDFGGSFLGGRGSSSPFPFTTGPDVRPGD
WYCTVGNCGAHNFASRSSCFKCGAPKDIDTFSSDSSDMPRLLRSPYGFGAGSAGGGASTRPGWKSG
DWICTRSGCNEHNFANRMECYRCNGPRDSSTGRSSYLS

## FIGURE 25 (continued)

**SEQ ID NO: 337, DT581158 yfi01-4ms2-b02 Yfi01 Yucca filamentosa cDNA clone yfi01-4ms2-b02**
GGCACAAGCTCTCACGCAACCCCTTGTCTCCATTTACTCTTCCTTCCTCTTTCAAGGGACTTGAG
AAGATGAACAGGAAGCCAGGAGACTGGAACTGCAGGTCATGCCAGCACCTTAATTTCAGCCGCAGG
GACTCGTGCCAGCGCTGCGGCGACCCCCGGTCGATCGGCAGCGAGCGATCGGACTACCCGGGCTTC
GTCGGGGGCCGCGGGGGGTCCTCATTCGGGTTCAGCGGCTCGGACGTCAGGCCCGGGGACTGGTAC
TGCCAGTGCGGGGCCCACAACTTCGCCAGCCGCTCGAGCTGCTTCAAGTGCAATGCTTTCAAGGAT
GAGTCCGCCGCTAGCGGCGGCCTCGACGGCGGCGACATGCTGAGATCCAGGGGGTTTGGCTTCGGC
GGCGGCGGCGGCGCTCGCAGTGGCTGGAAGTCTGGTGACTGGATTTGCAACAGGTCTGGCTGCAAT
GAGCACAACTTCGCTAGCAGGATGGAATGCTTCCGATGCAATGCACCTCGAGATTCGGGCACTGAG
GTTTAAGGAGACAGCCAAGAGAGAAGTGACGAGATGAGATCAGTGATGATGATACCAGTAGTAATC
TCGAGTTATTACTAGTTTTGCCACCAGCCCCAACTTTATCTCCTTTTGATGTTTTTTAGAGCCCC
CTTCTAAACGGAGGTGTCTTCTCTTTTTCATCCGTTTGTTTGCTTTTGAAAGCTTGCACTTACCCT
TTACACCCCCCCGCCCCCCTCCACCTTGTAGTTTGTGTTGAGAGTGTCTTTGTAAGTCTTTGGAGA
AGGGCTCCTTTAGCTTCTTGGGGGAGTCCCTTCT

**SEQ ID NO: 338, DT581158_1 [70 - 531] yfi01-4ms2-b02 Yfi01 *Yucca filamentosa* cDNA clone yfi01-4ms2-b02**
MNRKPGDWNCRSCQHLNFSRRDSCQRCGDPRSIGSERSDYPGFVGGRGGSSFGFSGSDVRPGDWYC
QCGAHNFASRSSCFKCNAFKDESAASGGLDGGDMLRSRGFGFGGGGGARSGWKSGDWICNRSGCNE
HNFASRMECFRCNAPRDSGTEV

**SEQ ID NO: 339, BQ471337 HV02C06r HV *Hordeum vulgare* subsp. vulgare cDNA clone HV02C06**
CGGCACGAGGCACTTCTCATCTTGGAGCTCAGCAGCACAGCATCCAAGCTTTTTCTTCGGCAAGAT
GAACAGGAAGCCAGGAGACTGGGACTGCAGGTCGTGCCAGCACCTCAACTTCAGTCGCCGGGACCT
ATGCCAGCGCTGCGGTGAGCCACGTAGTGCCGCTGACCGTGGCAGCGTCGGTGGTGCTCTTGGTGG
TGACTACGCCAACTTTGGCGGCCGTGGCGGGGGTGGTTCCTCATTTGGTGCCGGCTTTGGTGCCGG
CTCTGACGTCCGCCCGGGTGACTGGTACTGCACCTGTGGAGCGCACAACTTCGCCAGCCGCTCCAG
CTGCTTCAAGTGTGCTGCTTTCAAGGAGGAAGCTGCCGTCAATGGTGGCGCTGGTGGCTTTGATGG
TGACATGTCACGCTCAAGGGGCTTTGGCTTTGGCGCTGTCGGTGGCATGGGTGGCGGCATGGGAGC
CGGCGCAGCCGGTGGTCGTGCCAGTCGCCCTGGCTGGAAGTCTCGCGACTGGATTTGCACCAGGTC
TGGATGCAACGAGCACAACTTCGCCAGCAGGCAGGAGTGCTTCAGGTGCAACGCGCCGAGGGACTC
CGGTAGCGCCACACCATACG

**SEQ ID NO: 340, BQ471337_1 [65 - 613] HV02C06r HV Hordeum vulgare subsp. vulgare cDNA clone HV02C06**
MNRKPGDWDCRSCQHLNFSRRDLCQRCGEPRSAADRGSVGGALGGDYANFGGRGGGGSSF
GAGFGAGSDVRPGDWYCTCGAHNFASRSSCFKCAAFKEEAAVNGGAGGFDGDMSRSRGFG
FGAVGGMGGGMGAGAAGGRASRPGWKSRDWICTRSGCNEHNFASRQECFRCNAPRDSGSA
TPY

**SEQ ID NO: 341, prm5539**
GGGGACAAGTTTGTACAAAAAAGCAGGCTTAAACAATGAGCAGACCCGGAGATT

**SEQ ID NO: 342, prm5540**
GGGGACCACTTTGTACAAGAAAGCTGGGTAGACAAGGCTACTTCAAAAGCA

## FIGURE 25 (continued)

**SEQ ID NO: 343, rice GOS2 promoter**
AATCCGAAAAGTTTCTGCACCGTTTTCACCCCCTAACTAACAATATAGGGAACGTGTGCTAAATAT
AAAATGAGACCTTATATATGTAGCGCTGATAACTAGAACTATGCAAGAAAAACTCATCCACCTACT
TTAGTGGCAATCGGGCTAAATAAAAAAGAGTCGCTACACTAGTTTCGTTTTCCTTAGTAATTAAGT
GGGAAAATGAAATCATTATTGCTTAGAATATACGTTCACATCTCTGTCATGAAGTTAAATTATTCG
AGGTAGCCATAATTGTCATCAAACTCTTCTTGAATAAAAAAATCTTTCTAGCTGAACTCAATGGGT
AAAGAGAGAGATTTTTTTTAAAAAAAATAGAATGAAGATATTCTGAACGTATTGGCAAAGATTTAAA
CATATAATTATATAATTTTATAGTTTGTGCATTCGTCATATCGCACATCATTAAGGACATGTCTTA
CTCCATCCCAATTTTTATTTAGTAATTAAAGACAATTGACTTATTTTTATTATTTATCTTTTTTCG
ATTAGATGCAAGGTACTTACGCACACACTTTGTGCTCATGTGCATGTGTGAGTGCACCTCCTCAAT
ACACGTTCAACTAGCAACACATCTCTAATATCACTCGCCTATTTAATACATTTAGGTAGCAATATC
TGAATTCAAGCACTCCACCATCACCAGACCACTTTTAATAATATCTAAAATACAAAAAAATAATTTT
ACAGAATAGCATGAAAAGTATGAAACGAACTATTTAGGTTTTTCACATACAAAAAAAAAAAGAATT
TTGCTCGTGCGCGAGCGCCAATCTCCCATATTGGGCACACAGGCAACAACAGAGTGGCTGCCCACA
GAACAACCCACAAAAAACGATGATCTAACGGAGGACAGCAAGTCCGCAACAACCTTTTAACAGCAG
GCTTTGCGGCCAGGAGAGAGGAGGAGAGGCAAAGAAAACCAAGCATCCTCCTCCTCCCATCTATAA
ATTCCTCCCCCCTTTTCCCCTCTCTATATAGGAGGCATCCAAGCCAAGAAGAGGGAGAGCACCAAG
GACACGCGACTAGCAGAAGCCGAGCGACCGCCTTCTTCGATCCATATCTTCCGGTCGAGTTCTTGG
TCGATCTCTTCCCTCCTCCACCTCCTCCTCACAGGGTATGTGCCCTTCGGTTGTTCTTGGATTTAT
TGTTCTAGGTTGTGTAGTACGGGCGTTGATGTTAGGAAAGGGGATCTGTATCTGTGATGATTCCTG
TTCTTGGATTTGGGATAGAGGGGTTCTTGATGTTGCATGTTATCGGTTCGGTTTGATTAGTAGTAT
GGTTTTCAATCGTCTGGAGAGCTCTATGGAAATGAAATGGTTTAGGGTACGGAATCTTGCGATTTT
GTGAGTACCTTTTGTTTGAGGTAAAATCAGAGCACCGGTGATTTTGCTTGGTGTAATAAAAGTACG
GTTGTTTGGTCCTCGATTCTGGTAGTGATGCTTCTCGATTTGACGAAGCTATCCTTTGTTTATTCC
CTATTGAACAAAAATAATCCAACTTTGAAGACGGTCCCGTTGATGAGATTGAATGATTGATTCTTA
AGCCTGTCCAAAATTTCGCAGCTGGCTTGTTTAGATACAGTAGTCCCCATCACGAAATTCATGGAA
ACAGTTATAATCCTCAGGAACAGGGGATTCCCTGTTCTTCCGATTTGCTTTAGTCCCAGAATTTTT
TTTCCCAAATATCTTAAAAAGTCACTTTCTGGTTCAGTTCAATGAATTGATTGCTACAAATAATGC
TTTTATAGCGTTATCCTAGCTGTAGTTCAGTTAATAGGTAATACCCCTATAGTTTAGTCAGGAGAA
GAACTTATCCGATTTCTGATCTCCATTTTTAATTATATGAAATGAACTGTAGCATAAGCAGTATTC
ATTTGGATTATTTTTTTTATTAGCTCTCACCCCTTCATTATTCTGAGCTGAAAGTCTGGCATGAAC
TGTCCTCAATTTTGTTTTCAAATTCACATCGATTATCTATGCATTATCCTCTTGTATCTACCTGTA
GAAGTTTCTTTTTGGTTATTCCTTGACTGCTTGATTACAGAAAGAAATTTATGAAGCTGTAATCGG
GATAGTTATACTGCTTGTTCTTATGATTCATTTCCTTTGTGCAGTTCTTGGTGTAGCTTGCCACTT
TCACCAGCAAAGTTC

**SEQ ID NO: 344, sequence motif 1**
(G/R/D/N)DW

**SEQ ID NO: 345, sequence motif 2**
GSW

**SEQ ID NO: 346, sequence motif 3**
NF(Q/C/S)(R/K)R

**SEQ ID NO: 347, sequence motif 4**
N(F/Y)(A/S/P)(N/S/F)R

## FIGURE 25 (continued)

Europäisches Patentamt
European Patent Office
Office européen des brevets

# EUROPEAN SEARCH REPORT

Application Number
EP 09 17 8586

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X | US 2004/034888 A1 (LIU JINGDONG [US] ET AL) 19 February 2004 (2004-02-19)<br>* sequence ID NO 62164 *<br>----- | 1 | INV.<br>C12N15/82<br>A01H5/00 |
| X,D | HORIGUCHI GOROU ET AL: "The transcription factor AtGRF5 and the transcription coactivator AN3 regulate cell proliferation in leaf primordia of Arabidopsis thaliana"<br>PLANT JOURNAL,<br>vol. 43, no. 1, July 2005 (2005-07), pages 68-78, XP002410132<br>ISSN: 0960-7412<br>* the whole document *<br>----- | 1-20 | |
| X | JEONG HOE KIM ET AL: "THE ATGRF FAMILY OF PUTATIVE TRANSCRIPTION FACTORS US INVOLVED IN LEAF AND COTYLEDON GROWTH IN ARABIDOPSIS"<br>PLANT JOURNAL, BLACKWELL SCIENTIFIC PUBLICATIONS, OXFORD, GB,<br>vol. 36, no. 1, October 2003 (2003-10), pages 94-104, XP008058318<br>ISSN: 0960-7412<br>* abstract; figure 4; table 1 *<br>-----<br>-/-- | 1-20 | TECHNICAL FIELDS SEARCHED (IPC)<br>C12N<br>A01H |

The present search report has been drawn up for all claims

| Place of search | Date of completion of the search | Examiner |
|---|---|---|
| Munich | 13 April 2010 | Bilang, Jürg |

2

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 09 17 8586

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| X,D | KIM JEONG HOE ET AL: "A transcriptional coactivator, AtGIF1, is involved in regulating leaf growth and morphology in Arabidopsis" PROCEEDINGS OF THE NATIONAL ACADEMY OF SCIENCES OF USA, NATIONAL ACADEMY OF SCIENCE, WASHINGTON, DC, US, vol. 101, no. 36, 7 September 2004 (2004-09-07), pages 13374-13379, XP002362467 ISSN: 0027-8424 * abstract; figure 2 * * page 13378 - page 13379 * ----- | 1-20 | |
| X | PATENT ABSTRACTS OF JAPAN vol. 2003, no. 12, 5 December 2003 (2003-12-05) & JP 2004 350553 A (JAPAN SCIENCE & TECHNOLOGY AGENCY), 16 December 2004 (2004-12-16) * abstract * ----- | 1-20 | TECHNICAL FIELDS SEARCHED (IPC) |
| X | KIM J-H ET AL: "STUDIES ON THE FUNCTION OF OSGRF1-LIKE (ATGRL) GENES IN ARABIDOPSIS" PLANT BIOLOGY, NEW YORK, NY, US, August 2002 (2002-08), page 56, XP008058260 * the whole document * ----- -/-- | 1-20 | |

The present search report has been drawn up for all claims

2

| Place of search | Date of completion of the search | Examiner |
|---|---|---|
| Munich | 13 April 2010 | Bilang, Jürg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
& : member of the same patent family, corresponding document

EPO FORM 1503 03.82 (P04C01)

Europäisches Patentamt
European Patent Office
Office européen des brevets

# EUROPEAN SEARCH REPORT

Application Number
EP 09 17 8586

## DOCUMENTS CONSIDERED TO BE RELEVANT

| Category | Citation of document with indication, where appropriate, of relevant passages | Relevant to claim | CLASSIFICATION OF THE APPLICATION (IPC) |
|---|---|---|---|
| A | GOROU HORIGUCHI ET AL: "Coordination of cell proliferation and cell expansion in the control of leaf size in Arabidopsis thaliana"<br>JOURNAL OF PLANT RESEARCH, SPRINGER-VERLAG, TO LNKD-DOI:10.1007/S10265-005-0232-4,<br>vol. 119, no. 1,<br>1 January 2006 (2006-01-01), pages 37-42, XP019375380<br>ISSN: 1618-0860<br>* the whole document *<br>----- | 1-20 | |
| X,P | WO 2006/079655 A2 (CROPDESIGN NV [BE]; FRANKARD VALERIE [BE])<br>3 August 2006 (2006-08-03)<br>* the whole document *<br>----- | 1-20 | |

TECHNICAL FIELDS SEARCHED (IPC)

The present search report has been drawn up for all claims

2

| Place of search | Date of completion of the search | Examiner |
|---|---|---|
| Munich | 13 April 2010 | Bilang, Jürg |

CATEGORY OF CITED DOCUMENTS

X : particularly relevant if taken alone
Y : particularly relevant if combined with another document of the same category
A : technological background
O : non-written disclosure
P : intermediate document

T : theory or principle underlying the invention
E : earlier patent document, but published on, or after the filing date
D : document cited in the application
L : document cited for other reasons
.......................................................................
& : member of the same patent family, corresponding document

EPO FORM 1503 03.82 (P04C01)

Europäisches Patentamt
European Patent Office
Office européen des brevets

Application Number

EP 09 17 8586

## CLAIMS INCURRING FEES

The present European patent application comprised at the time of filing claims for which payment was due.

- [ ] Only part of the claims have been paid within the prescribed time limit. The present European search report has been drawn up for those claims for which no payment was due and for those claims for which claims fees have been paid, namely claim(s):

- [ ] No claims fees have been paid within the prescribed time limit. The present European search report has been drawn up for those claims for which no payment was due.

## LACK OF UNITY OF INVENTION

The Search Division considers that the present European patent application does not comply with the requirements of unity of invention and relates to several inventions or groups of inventions, namely:

see sheet B

- [ ] All further search fees have been paid within the fixed time limit. The present European search report has been drawn up for all claims.

- [X] As all searchable claims could be searched without effort justifying an additional fee, the Search Division did not invite payment of any additional fee.

- [ ] Only part of the further search fees have been paid within the fixed time limit. The present European search report has been drawn up for those parts of the European patent application which relate to the inventions in respect of which search fees have been paid, namely claims:

- [ ] None of the further search fees have been paid within the fixed time limit. The present European search report has been drawn up for those parts of the European patent application which relate to the invention first mentioned in the claims, namely claims:

- [ ] The present supplementary European search report has been drawn up for those parts of the European patent application which relate to the invention first mentioned in the claims (Rule 164 (1) EPC).

Europäisches Patentamt
European Patent Office
Office européen des brevets

## LACK OF UNITY OF INVENTION SHEET B

Application Number
EP 09 17 8586

The Search Division considers that the present European patent application does not comply with the requirements of unity of invention and relates to several inventions or groups of inventions, namely:

1. claim: 1(partially)

Claim 1 (part) - Method for increasing plant yield comprising modulating expression in a plant of a nucleic acid sequence encoding an SYT polypeptide, which comprises an SNH domain having at least 20% identity to SEQ ID NO 58 and ii) a Met rich domain and iii) a QG rich domain .

---

2-46. claims: 2-20(completely); 1(partially)

Claims 1(part) and 2-34. The method of invention 1 wherein the nucleic acid sequence/SYT polypeptide corresponds to each of the sequence pairs listed in Table 6, respectively.

---

**ANNEX TO THE EUROPEAN SEARCH REPORT ON EUROPEAN PATENT APPLICATION NO.** EP 09 17 8586

This annex lists the patent family members relating to the patent documents cited in the above-mentioned European search report.
The members are as contained in the European Patent Office EDP file on
The European Patent Office is in no way liable for these particulars which are merely given for the purpose of information.

13-04-2010

| Patent document cited in search report | Publication date | Patent family member(s) | Publication date |
|---|---|---|---|
| US 2004034888 A1 | 19-02-2004 | US 2004031072 A1 | 12-02-2004 |
| | | US 2006236419 A1 | 19-10-2006 |
| JP 2004350553 A | 16-12-2004 | NONE | |
| WO 2006079655 A2 | 03-08-2006 | AR 052101 A1 | 28-02-2007 |
| | | AU 2006208779 A1 | 03-08-2006 |
| | | BR PI0607211 A2 | 22-12-2009 |
| | | CA 2595672 A1 | 03-08-2006 |
| | | EP 1844151 A2 | 17-10-2007 |
| | | JP 2008528014 T | 31-07-2008 |
| | | KR 20070111458 A | 21-11-2007 |
| | | NZ 556605 A | 26-03-2010 |

EPO FORM P0459

For more details about this annex : see Official Journal of the European Patent Office, No. 12/82

## REFERENCES CITED IN THE DESCRIPTION

*This list of references cited by the applicant is for the reader's convenience only. It does not form part of the European patent document. Even though great care has been taken in compiling the references, errors or omissions cannot be excluded and the EPO disclaims all liability in this regard.*

### Patent documents cited in the description


- WO 0244389 A
- JP 2004350553 A
- WO 02074801 A
- US 5955650 A
- US 6084164 A
- US 5811238 A
- US 6395547 A
- WO 2004070039 A
- WO 2004065596 A
- US 4962028 A
- WO 0114572 A
- WO 9514098 A
- WO 9412015 A
- EP 99106056 A
- US 5565350 A, Kmiec
- WO 9322443 A, Zarling
- WO 9853083 A, Grierson
- WO 9953050 A, Waterhouse
- US 4987071 A, Cech
- US 5116742 A, Cech
- WO 9400012 A, Atkins
- WO 9503404 A, Lenne
- WO 0000619 A, Lutziger
- WO 9713865 A, Prinsen
- WO 9738116 A, Scott
- WO 9836083 A
- WO 9915682 A
- WO 0015815 A
- EP 1198985 A1
- US 5352605 A
- WO 8402913 A
- EP 388186 A
- EP 335528 A
- WO 9706268 A
- WO 9519443 A
- WO 9321334 A
- US 5187267 A
- WO 9612814 A
- EP 0375091 A
- US 5608152 A
- WO 9845461 A
- US 5504200 A
- WO 9113980 A
- WO 0026388 A
- WO 9515389 A
- WO 9523230 A
- WO 9916890 A
- US 5677474 A
- US 5530149 A
- EP 571741 A
- JP 6062870 A
- WO 9808962 A
- US 5689040 A
- EP 781849 A
- DE 19644478 A
- EP 0249676 A
- EP O249676 A
- US 57673666 B
- US 6225105 B
- US 5164310 A


### Non-patent literature cited in the description


- **Wang et al.** *Planta,* 2003, vol. 218, 1-14
- **Tittonell et al.** *Agric Ecosys & Environ,* 2005, vol. 105, 213
- **Fasoula ; Tollenaar.** *Maydica,* 2005, vol. 50, 39
- **ter Steege et al.** *Plant Physiology,* 2005, vol. 139, 1078
- **Hittalmani et al.** *Theoretical Applied Genetics,* 2003, vol. 107, 679
- **Rebetzke et al.** *Crop Science,* 2002, vol. 42, 739
- **Gardener et al.** Physiology of Crop Plants. Iowa State University Press, 1985, 68-73
- **Stegmaier et al.** *Genome informatics,* 2004, vol. 15, 276-286
- **Li ; Thomas.** *Plant Cell,* 1998, vol. 10, 383-398
- **Boyer.** *Science,* 1982, vol. 218, 443-448
- **Frink et al.** *Proc Natl Acad Sci USA,* 1999, vol. 96 (4), 1175-1180
- **Kim HJ ; Kende H.** *Proc Nat Acad Sc,* 2004, vol. 101, 13374-9
- **Horiguchi et al.** *Plant J,* 2005, vol. 43, 68-78
- **van der Knaap E et al.** *Plant Phys,* 2000, vol. 122, 695-704

- **Näär AM et al.** *Annu Rev Biochem,* 2001, vol. 70, 475-501
- **Thaete et al.** *Hum Molec Genet,* 1999, vol. 8, 585-591
- **Chiadmi et al.** *EMBO,* 1999, vol. 18 (23), 6809-6815
- **Buchanan.** *Annu Rev Plant Physiol,* 1980, vol. 31, 341-374
- **Jacquot.** *Bot Acta,* 1984, vol. 103, 323-334
- **Huppe ; Buchanan.** *Z Naturforsch,* 1989, vol. 44 (5-6), 487-94
- **Thorbjornsen et al.** *Planta,* 2002, vol. 214, 616-624
- **Miyagawa et al.** *Nature Biotech,* 2001, vol. 19, 965-969
- **Tamoi et al.** *Plant Cell Physiol,* 2006, vol. 47 (3), 380-390
- **Töpfer et al.** *Science,* 1995, vol. 268, 681-686
- **Töpfer et al.** *Science,* 1995, vol. 268, 68 1-686
- **Cahoon et al.** *Proc. Natl. Acad. Sci. USA,* 1992, vol. 89, 11184-11188
- **Van de Loo F.J. et al.** Unusual Fatty Acids in Lipid Metabolism in Plants. CRC Press, 1993, 91-126
- **Millar et al.** *Trends Plant Sci.,* 2000, vol. 5, 95-101
- **Browse et al.** *Biochemical J.,* 1986, vol. 235, 25-31
- **Ohlrogge ; Browse.** *Plant Ce11,* May 1995, vol. 7, 957-970
- **Voelker.** Genetic Engineering. 1996, vol. 1 8, 111-113
- **Shanklin ; Cahoon.** *Annu. Rev. Plant Physiol. Plant Mol. Biol.,* 1998, vol. 49, 611-641
- **Frentzen.** *Lipids,* 1998, vol. 100, 161-166
- **Plaxton.** *Annu. Rev. Plant Physiol. Plant Mol. Biol.,* 1996, vol. 47, 185-214
- **Kang ; Rawsthorne.** *Plant J.,* 1994, vol. 6, 795-805
- **Ohlrogge ; Browse.** *Plant Ce11,* 1995, vol. 7, 957-970
- **Van de Loo et al.** *Proc. Natl. Acad. Sci USA,* 1995, vol. 92, 6743-6747
- **Cahoon et al.** *Proc. Natl. Acad. Sci USA,* 1992, vol. 89, 11 184-1 1 188
- **Brenner.** *Adv. Exp. Med. Biol.,* 1976, vol. 83, 85-101
- **Riechmann et al.** *Science,* 2000, vol. 290, 2105-2109
- **Sakakibara et al.** *Mol. Biol. Evol.,* 2001, vol. 18 (4), 491-502
- **Aso et al.** *Mol. Biol. Evol.,* 1999, vol. 16 (4), 544-552
- **Sakakibara et al.** *Mol Biol Evol,* 2001, vol. 18 (4), 491-502
- **Sessa et al.** *EMBO J,* 1993, vol. 12 (9), 3507-3517
- **Henriksson et al.** *Plant Phys,* 2005, vol. 139, 509-518
- **Meijer et al.** *Mol Gen Genet,* 2000, vol. 263, 12-21
- **Meijer et al.** *Plant J,* 1997, vol. 11, 263-276
- **Morelli ; Ruberti.** *TIPS,* September 2002, vol. 7 (9
- Creighton. Proteins. W.H. Freeman and Company, 1984
- **Terpe.** *Appl. Microbiol. Biotechnol.,* 2003, vol. 60, 523-533
- **Meinkoth ; Wahl.** *Anal. Biochem.,* 1984, vol. 138, 267-284
- **Sambrook et al.** Molecular Cloning: a laboratory manual. Cold Spring Harbor Laboratory Press, 2001
- Current Protocols in Molecular Biology. John Wiley & Sons, 1989
- **Foissac ; Schiex.** *BMC Bioinformatics,* 2005, vol. 6, 25
- **Castle et al.** *Science,* 2004, vol. 304 (5674), 1151-4
- **Heid et al.** *Genome Methods,* 1996, vol. 6, 986-994
- **McElroy et al.** *Plant Cell,* 1990, vol. 2, 163-171
- **Odell et al.** *Nature,* 1985, vol. 313, 810-812
- **Nilsson et al.** *Physiol. Plant.,* 1997, vol. 100, 456-462
- **de Pater et al.** *Plant J,* November 1992, vol. 2 (6), 837-44
- **Christensen et al.** *Plant Mol. Biol.,* 1992, vol. 18, 675-689
- **Buchholz et al.** *Plant Mol Biol.,* 1994, vol. 25 (5), 837-43
- **Lepetit et al.** *Mol. Gen. Genet.,* 1992, vol. 231, 276-285
- **Wu et al.** *Plant Mol. Biol.,* 1988, vol. 11, 641-649
- **An et al.** *Plant J.,* 1996, vol. 10 (1), 107-121
- **Sanger et al.** *Plant. Mol. Biol.,* 1990, vol. 14, 433-443
- **Leisner.** *Proc Natl Acad Sci USA,* 1988, vol. 85 (5), 2553
- **Jain et al.** *Crop Science,* 1999, vol. 39 (6), 1696
- **Shaw et al.** *Nucleic Acids Res.,* 1984, vol. 12 (20), 7831-7846
- **Gatz.** *Annu. Rev. Plant Physiol. Plant Mol. Biol.,* 1997, vol. 48, 89-108
- **Qing Qu ; Takaiwa.** *Plant Biotechnol. J.,* 2004, vol. 2, 113-125
- **Simon et al.** *Plant Mol. Biol.,* 1985, vol. 5, 191
- **Scofield et al.** *J. Biol. Chem.,* 1987, vol. 262, 12202

- **Baszczynski et al.** *Plant Mol. Biol.,* 1990, vol. 14, 633
- **Pearson et al.** *Plant Mol. Biol.,* 1992, vol. 18, 235-245
- **Ellis et al.** *Plant Mol. Biol.,* 1988, vol. 10, 203-214
- **Takaiwa et al.** *Mol. Gen. Genet.,* 1986, vol. 208, 15-22
- **Takaiwa et al.** *FEBS Letts,* 1987, vol. 221, 43-47
- **Matzke et al.** *Plant Mol Biol,* 1990, vol. 14 (3), 323-32
- **Stalberg et al.** *Planta,* 1996, vol. 199, 515-519
- *Mol Gen Genet,* 1989, vol. 216, 81-90
- *NAR,* 1989, vol. 17, 461-2
- **Albani et al.** *Plant Cell,* 1997, vol. 9, 171-184
- *EMBO J.,* 1984, vol. 3, 1409-15
- **Diaz et al.** *Mol Gen Genet,* 1995, vol. 248 (5), 592-8
- *Theor Appl Gen,* 1999, vol. 98, 1253-62
- *Plant J,* 1993, vol. 4, 343-55
- *Mol Gen Genet,* 1996, vol. 250, 750-60
- **Mena et al.** *The Plant Journal,* 1998, vol. 116 (1), 53-62
- **Vicente-Carbajosa et al.** *Plant J.,* 1998, vol. 13, 629-640
- **Wu et al.** *Plant Cell Physiology,* 1998, vol. 39 (8), 885-889
- **Sato et al.** *Proc. Natl. Acad. Sci. USA,* 1996, vol. 93, 8117-8122
- **Nakase et al.** *Plant Mol. Biol.,* 1997, vol. 33, 513-522
- *Trans Res,* 1997, vol. 6, 157-68
- *Plant J,* 1997, vol. 12, 235-46
- **DeRose et al.** *Plant Mol. Biol,* 1996, vol. 32, 1029-35
- **Postma-Haarsma et al.** *Plant Mol. Biol.,* 1999, vol. 39, 257-71
- **Wu et al.** *J. Biochem.,* 1998, vol. 123, 386
- **Cummins et al.** *Plant Mol. Biol.,* 1992, vol. 19, 873-876
- **Lanahan et al.** *Plant Cell,* 1992, vol. 4, 203-211
- **Skriver et al.** *Proc Natl Acad Sci USA,* 1991, vol. 88, 7266-7270
- **Cejudo et al.** *Plant Mol Biol,* 1992, vol. 20, 849-856
- **Kalla et al.** *Plant J.,* 1994, vol. 6, 849-60
- **Leah et al.** *Plant J.,* 1994, vol. 4, 579-89
- **Selinger et al.** *Genetics,* 1998, vol. 149, 1125-38
- **Takaiwa et al.** *Mol Gen Genet,* 1986, vol. 208, 15-22
- **Takaiwa et al.** *FEBS Letts.,* 1987, vol. 221, 43-47
- **Colot et al.** *Mol Gen Genet,* 1989, vol. 216, 81-90
- **Anderson et al.** *NAR,* 1989, vol. 17, 461-2
- **Rafalski et al.** *EMBO,* 1984, vol. 3, 1409-15
- **Cho et al.** *Theor Appl Genet,* 1999, vol. 98, 1253-62
- **Muller et al.** *Plant J,* 1993, vol. 4, 343-55
- **Sorenson et al.** *Mol Gen Genet,* 1996, vol. 250, 750-60
- **Mena et al.** *Plant J,* 1998, vol. 116 (1), 53-62
- **Onate et al.** *J Biol Chem,* 1999, vol. 274 (14), 9175-82
- **Vicente-Carbajosa et al.** *Plant J,* 1998, vol. 13, 629-640
- **Wu et al.** *Plant Cell Physiol,* 1998, vol. 39 (8), 885-889
- **Nakase et al.** *Plant Molec Biol,* 1997, vol. 33, 513-522
- **Russell et al.** *Trans Res,* 1997, vol. 6, 157-68
- **Opsahl-Ferstad et al.** *Plant J,* 1997, vol. 12, 235-46
- **DeRose et al.** *Plant Mol Biol,* 1996, vol. 32, 1029-35
- **Buchman ; Berg.** *Mol. Cell biol.,* 1988, vol. 8, 4395-4405
- **Callis et al.** *Genes Dev,* 1987, vol. 1, 1183-1200
- The Maize Handbook. Springer, 1994
- **Gaultier et al.** *Nucl Ac Res,* 1987, vol. 15, 6625-6641
- **Inoue et al.** *Nucl Ac Res,* 1987, vol. 15, 6131-6148
- **Inoue et al.** *FEBS Lett.,* 1987, vol. 215, 327-330
- **Haselhoff ; Gerlach.** *Nature,* 1988, vol. 334, 585-591
- **Bartel ; Szostak.** *Science,* 1993, vol. 261, 1411-1418
- **Angell ; Baulcombe.** *Plant J,* 1999, vol. 20 (3), 357-62
- **Helene, C.** *Anticancer Drug Res.,* 1991, vol. 6, 569-84
- **Helene et al.** *Ann. N.Y. Acad. Sci.,* 1992, vol. 660, 27-36
- **Maher, L.J.** *Bioassays,* 1992, vol. 14, 807-15
- **Schwab et al.** *Dev. Cell,* 2005, vol. 8, 517-527
- **Schwab et al.** *Plant Cell,* 2006, vol. 18, 1121-1133
- **Tribble et al.** *J. Biol. Chem.,* 2000, vol. 275, 22255-22267
- **Velmurugan et al.** *J. Cell Biol.,* 2000, vol. 149, 553-566
- **Krens, F.A. et al.** *Nature,* 1982, vol. 296, 72-74
- **Negrutiu I et al.** *Plant Mol Biol,* 1987, vol. 8, 363-373
- **Shillito R.D. et al.** *Bio/Technol,* 1985, vol. 3, 1099-1102
- **Crossway A et al.** *Mol. Gen Genet,* 1986, vol. 202, 179-185

- **Klein TM et al.** *Nature,* 1987, vol. 327, 70
- **Clough ; Bent.** *Plant J.,* 1998, vol. 16, 735-743
- **Aldemita ; Hodges.** *Planta,* 1996, vol. 199, 612-617
- **Chan et al.** *Plant Mol Biol,* 1993, vol. 22 (3), 491-506
- **Hiei et al.** *Plant J,* 1994, vol. 6 (2), 271-282
- **Ishida et al.** *Nat. Biotechnol,* 1996, vol. 14 (6), 745-50
- **Frame et al.** *Plant Physiol,* 2002, vol. 129 (1), 13-22
- Techniques for Gene Transfer. **B. Jenes et al.** Transgenic Plants, Vol. 1, Engineering and Utilization. Academic Press, 1993, vol. 1, 128-143
- *Potrykus Annu. Rev. Plant Physiol. Plant Molec. Biol.,* 1991, vol. 42, 205-225
- **Bevan et al.** *Nucl. Acids Res.,* 1984, vol. 12, 8711
- **Höfgen ; Willmitzer.** *Nucl. Acid Res.,* 1988, vol. 16, 9877
- Vectors for Gene Transfer in Higher Plants. **F.F. White.** Transgenic Plants, Vol. 1, Engineering and Utilization. Academic Press, 1993, vol. 1, 15-38
- **Feldman, KA ; Marks MD.** *Mol Gen Genet,* 1987, vol. 208, 274-289
- **Feldmann K.** Methods in Arabidopsis Research. Word Scientific, 1992, 274-289
- **Chang.** *Plant J.,* 1994, vol. 5, 551-558
- **Katavic.** *Mol Gen Genet,* 1994, vol. 245, 363-370
- **Bechthold, N.** *C R Acad Sci Paris Life Sci,* 1993, vol. 316, 1194-1199
- **Clough, SJ ; Bent AF.** *The Plant J.,* 1998, vol. 16, 735-743
- **Klaus et al.** *Nature Biotechnology,* 2004, vol. 22 (2), 225-229
- **Bock.** Transgenic plastids in basic research and plant biotechnology. *J Mol Biol.,* 21 September 2001, vol. 312 (3), 425-38
- **Maliga, P.** Progress towards commercialization of plastid transformation technology. *Trends Biotechnol.,* 2003, vol. 21, 20-28
- **Hayashi et al.** *Science,* 1992, 1350-1353
- **Redei GP ; Koncz C.** Methods in Arabidopsis Research. World Scientific Publishing Co, 1992, 16-82
- **Feldmann et al.** Arabidopsis. Cold Spring Harbor Laboratory Press, 1994, 137-172
- **Lightner J ; Caspar T.** Methods on Molecular Biology. Humana Press, 1998, vol. 82, 91-104
- **McCallum et al.** *Nat Biotechnol,* 2000, vol. 18, 455-457
- **Stemple.** *Nat Rev Genet,* 2004, vol. 5 (2), 145-50
- **Offringa et al.** *EMBO J,* 1990, vol. 9 (10), 3077-84
- **Terada et al.** *Nat Biotech,* 2002, vol. 20 (10), 1030-4
- **Iida ; Terada.** *Curr Opin Biotech,* 2004, vol. 15 (2), 132-8
- **Rabbani et al.** *Plant Physiol,* 2003, vol. 133, 1755-1767
- **Schultz et al.** *Proc. Natl. Acad. Sci. USA,* 1998, vol. 95, 5857-5864
- **Letunic et al.** *Nucleic Acids Res,* 2002, vol. 30, 242-244
- **Mulder et al.** *Nucl. Acids. Res.,* 2003, vol. 31, 315-318
- A generalized profile syntax for biomolecular sequences motifs and its function in automatic sequence interpretation. **Bucher ; Bairoch.** ISMB-94; Proceedings 2nd International Conference on Intelligent Systems for Molecular Biology. AAAIPress, 1994, 53-61
- **Hulo et al.** *Nucl. Acids. Res.,* 2004, vol. 32, D134-D137
- **Bateman et al.** *Nucleic Acids Research,* 2002, vol. 30 (1), 276-280
- **Gasteiger et al.** ExPASy: the proteomics server for in-depth protein knowledge and analysis. *Nucleic Acids Res.,* 2003, vol. 31, 3784-3788
- **Bateman et al.** *Nucl. Acids Res.,* 2004, vol. 32, D138-141
- **Mulder et al.** *Nucl. Acids. Res.,* 2005, vol. 33, D201-205
- **Needleman ; Wunsch.** *J Mol Biol,* 1970, vol. 48, 443-453
- **Altschul et al.** *J Mol Biol,* 1990, vol. 215, 403-10
- **Campanella et al.** MatGAT: an application that generates similarity/identity matrices using protein or DNA sequences. *BMC Bioinformatics,* 10 July 2003, vol. 4, 29
- **Fields ; Song.** *Nature,* 1989, vol. 340, 245-6
- Current Protocols in Molecular Biology. Wiley
- **Benfey et al.** *EMBO J.,* 1989, vol. 8, 2195-2202
- **Franck et al.** *Cell,* 1980, vol. 21, 285-294
- **Ward et al.** *Plant. Mol. Biol.,* 1993, vol. 22
- **Leisner.** *Proc Natl Acad Sci USA,* 1988, vol. 85 (5), 2553-2557
- **Comai.** *Plant Mol Biol,* 1990, vol. 15 (3), 373-381
- **Schenk.** *Plant Mol Biol,* 1999, vol. 39 (6), 1221-1230

- **Jain et al.** *Crop Science,* 1999, vol. 39 (6), 1696-1701
- **Gatz et al.** *Plant J.,* 1992, vol. 2, 397-404
- **Ward et al.** *Plant. Mol. Biol.,* 1993, vol. 22, 361-366
- **Baeumlein et al.** *Mol Gen Genet,* 1991, vol. 225 (3), 459-67
- **Baeumlein et al.** *Plant Journal,* 1992, vol. 2 (2), 233-9
- **Stockhaus et al.** *EMBO J.,* 1989, vol. 8, 2445
- **Sambrook J ; Fritsch EF ; Maniatis T.** Molecular Cloning, A Laboratory Manual. 1989
- **Lander et al.** *Genomics,* 1987, vol. 1, 174-181
- **Botstein et al.** *Am. J. Hum. Genet.,* 1980, vol. 32, 314-331
- **Bernatzky ; Tanksley.** *Plant Mol. Biol. Reporter,* 1986, vol. 4, 37-41
- **Hoheisel et al.** Non-mammalian Genomic Analysis: A Practical Guide. Academic press, 1996, 319-346
- **Trask.** *Trends Genet.,* 1991, vol. 7, 149-154
- **Laan et al.** *Genome Res.,* 1995, vol. 5, 13-20
- **Kazazian.** *J. Lab. Clin. Med,* 1989, vol. 11, 95-96
- **Sheffield et al.** *Genomics,* 1993, vol. 16, 325-332
- **Landegren et al.** *Science,* 1988, vol. 241, 1077-1080
- **Sokolov.** *Nucleic Acid Res.,* 1990, vol. 18, 3671
- **Walter et al.** *Nat. Genet.,* 1997, vol. 7, 22-28
- **Dear ; Cook.** *Nucleic Acid Res.,* 1989, vol. 17, 6795-6807
- A generalized profile syntax for biomolecular sequences motifs and its function in automatic sequence interpretation. **Bucher ; Bairoch.** ISMB-94; Proceedings 2nd International Conference on Intelligent Systems for Molecular Biology. AAAI Press, 1994, 53-61
- **Altschul et al.** Basic Local Alignment Search Tool. *J Mol Biol,* 1990, vol. 215, 403-10
- **Gasteiger E et al.** ExPASy: the proteomics server for in-depth protein knowledge and analysis. *Nucleic Acids Res,* 2003, vol. 31, 3784-3788
- **Field et al.** *Nature,* 1989, vol. 340 (6230), 245-246
- **Jian-Kang Zhu.** *TRENDS in Plant Science,* February 2001, vol. 6 (2
- **Rabbani et al.** *Plant Physiology,* December 2003, vol. 133, 1755-1767
- **Ward et al.** *Plant. Mol. Biol.,* 1993, 22
- GenBank Accession. U87999
- **Bematzky ; Tanksley.** *Plant Mol. Biol. Reporter,* 1986, vol. 4, 37-41
- **Chiadmi et al.** *EMBO J,* 1999, vol. 18 (23), 6809-6815
- **Huppe ; Buchanan.** *Naturforsch.,* 1989, vol. 44c, 487-494
- **Alscher-Herman.** *Plant Physiol,* 1982, vol. 70, 728-734
- **Ausubel et al.** Current Protocols. *Current Protocols in Molecular Biology,* 1994, vol. 1, 2, http://www.protocol-online.org
- *Biochimica et Biophysica Acta,* 1996, vol. 1314, 191-225
- **Altschul et al.** *J Mol Biol,* 1990, vol. 215, 403-410
- **Campanella et al.** *BMC Bioinformatics,* 2003, vol. 4, 29
- **Sessa et al.** *J Mol Biol,* 1997, vol. 274 (3), 303-309
- **Wootton ; Federhen.** *Methods Enzymol.,* 1996, vol. 266, 554-571
- **Henrikson et al.** *Plant Physiol,* 2005, vol. 139, 509-518
- **Sambrook.** Molecular Cloning: a laboratory manual. Cold Spring Harbor Laboratory Press, 2001
- **Ausubel et al.** Current Protocols in Molecular Biology, Current Protocols. 1994, vol. 1, 2
- **R.D.D. Croy.** Plant Molecular Biology Labfax. BIOS Scientific Publications Ltd (UK) and Blackwell Scientific Publications (UK), 1993
- **Altschul et al.** *J. Mol. Biol.,* 1990, vol. 215, 403-410
- **Altschul et al.** *Nucleic Acids Res.,* 1997, vol. 25, 3389-3402
- **An, G.** Agrobacterium Protocols. Methods in Molecular Biology. Humana Press, vol. 44, 47-62
- **Bevan.** *Nucleic Acid Research,* 1984, vol. 12, 8711-8721
- **Ishida et al.** *Nature Biotech,* 1996, vol. 14 (6), 745-50
- **Babic et al.** *Plant Cell Rep,* 1998, vol. 17, 183-188
- **McKersie et al.** *Plant Physiol,* 1999, vol. 119, 839-847
- **Brown DCW ; A Atanassov.** *Plant Cell Tissue Organ Culture,* 1985, vol. 4, 111-112
- **Walker et al.** *Am J Bot,* 1978, vol. 65, 654-659
- **Thompson et al.** *Nucleic Acids Res,* 1997, vol. 25, 4876-4882
- **Chenna et al.** *Nucleic Acids Res,* 2003, vol. 31, 3497-3500

- **Campanella JJ ; Bitincka L ; Smalley J.** MatGAT: an application that generates similarity/identity matrices using protein or DNA sequences. *BMC Bioinformatics,* 2003, vol. 4, 29
- **Ausubel et al.** *Current Protocols in Molecular Biology, Current Protocols,* 1994, vol. 1, 2
- **Ausubel et al.** Current Protocols. *Current Protocols in Molecular Biology,* 1994, vol. 1, 2

SEQUENCE LISTING

<110> CropDesign N.V.

<120> Plants having increased yield and method for making the same

<130> PF58401-prio

<160> 347

<170> PatentIn version 3.3

<210> 1
<211> 2151
<212> DNA
<213> Arabidopsis thaliana

<400> 1
atgtgctgtg gatcagaccg attaaaccag atcgtgtcat caagatcttc gttgccaatt 60
tctttcgagg aagataacaa tcttgttacc aacacagaca tgaatcactt aacagtcgaa 120
acagaggata cgtttgcgag cttgcttgag cttgcagcta acaacgatgt tgaaggtgta 180
aggctatcta tcgagagaga cccttcttgt gtagacgaag ctggtctctg gtacggtcgt 240
caaaaaggtt ctaaagctat ggtcaacgat tacaggactc cgttgatggt tgctgctact 300
tacggaagca ttgatgtgat caagcttatt gtttctttga ctgatgctga cgtgaaccgt 360
gcttgcggga atgatcagac cactgcgtta cactgcgctg cttctggagg agctgtgaat 420
gctatccaag ttgttaagct gcttcttgca gctggagctg atttgaatct gttggatgct 480
gaaggtcaac gagctggtga tgttattgtt gttcctccta agcttgaagg cgtgaagctg 540
atgcttcagg agcttctttc tgctgatgga tcatctactg cggagcggaa tctacgggtt 600
gtgacaaatg ttccgaatag aagctcatct ccgtgtcatt ctcctactgg agagaatggt 660
ggatcagggt ctggttcacc gctcggctct ccttttaagc tgaaatctac tgaattcaag 720
aaagagtatc cggttgatcc gtctttgcca gatatcaaga acagtatcta cgcgactgat 780
gagtttagaa tgtattcctt caaggtccgg ccttgctctc gtgcttattc acatgattgg 840
actgagtgtc cttttgttca cccgggtgaa aacgcgagga ggagagaccc gaggaagttc 900
cattacagct gcgttccttg cccggatttt aggaaaggag cttgtaggag aggagatatg 960
tgtgagtatg cgcacggtgt gtttgaatgc tggcttcatc cggctcagta caggacccgt 1020
ctttgcaaag atggaacagg ctgtgctcgg cgggtttgtt tctttgcgca tacacccgag 1080
gagcttcgac ctttgtacgc atcaactggt tcagcggttc cttcgcctag atcgaatgct 1140
gattatgcag ctgctttgag tctccttcct ggttctccat caggagtctc tgtcatgtcc 1200
ccgctttccc catcagcagc ggggaacgga atgtctcatt cgaatatggc ttggccacaa 1260
ccaaatgtcc ctgcgttgca cttaccagga agcaatctac agtcaagcag gctaaggtct 1320
tctctcaatg caagggatat cccgacggat gagttcaata tgttagcgga ttacgagcag 1380
cagcaactcc tcaacgagta ttccaatgct ctgagccgtt ctggtcggat gaaatcaatg 1440
cctccttcga atcttgaaga tcttttctca gcagaaggct cttcatctcc ccggttcact 1500
gattccgctt tagcttccgc ggtgttctcg cctacacaca agtcagctgt cttcaaccag 1560
ttccaacaac agcaacagca gcagcagagc atgttgtctc caatcaacac aagcttttct 1620
tcaccaaaga gcgttgacca ctcattgttt tcaggtggag gaagaatgtc tcctcggaat 1680
gttgttgaac caatatcacc catgagtgct cgggtttcca tgttggctca gtgcgtgaag 1740
caacaacaac agcaacagca gcagcagcag cagcaacatc agttccgtag ccttagctcc 1800
agagagctca gaacaaactc tagcccaatc gttggttcac cggtaaacaa caacacatgg 1860
tcatcaaaat ggggatcttc aaatggtcaa ccggattggg gaatgagctc agaagcactt 1920
ggtaagttga gatcttcgtc atcgtttgat ggtgatgagc ctgatgtgtc atgggtccag 1980
tcactggtga aggagactcc agcagaagcc aaagagaaag cagcaacatc ttcctcaggg 2040
gaacacgtga tgaagcagcc aaatccggtt gaaccggtaa tggatcatgc tgggctagaa 2100
gcttggattg agcaaatgca gctcgatcag cttgtggctc agcagaattg a 2151

<210> 2
<211> 716
<212> PRT
<213> Arabidopsis thaliana

<400>  2
Met Cys Cys Gly Ser Asp Arg Leu Asn Gln Ile Val Ser Ser Arg Ser
1               5                   10                  15
Ser Leu Pro Ile Ser Phe Glu Glu Asp Asn Asn Leu Val Thr Asn Thr
                20                  25                  30
Asp Met Asn His Leu Thr Val Glu Thr Glu Asp Thr Phe Ala Ser Leu
            35                  40                  45
Leu Glu Leu Ala Ala Asn Asn Asp Val Glu Gly Val Arg Leu Ser Ile
    50                  55                  60
Glu Arg Asp Pro Ser Cys Val Asp Glu Ala Gly Leu Trp Tyr Gly Arg
65                  70                  75                  80
Gln Lys Gly Ser Lys Ala Met Val Asn Asp Tyr Arg Thr Pro Leu Met
                85                  90                  95
Val Ala Ala Thr Tyr Gly Ser Ile Asp Val Ile Lys Leu Ile Val Ser
            100                 105                 110
Leu Thr Asp Ala Asp Val Asn Arg Ala Cys Gly Asn Asp Gln Thr Thr
        115                 120                 125
Ala Leu His Cys Ala Ala Ser Gly Gly Ala Val Asn Ala Ile Gln Val
    130                 135                 140
Val Lys Leu Leu Leu Ala Ala Gly Ala Asp Leu Asn Leu Leu Asp Ala
145                 150                 155                 160
Glu Gly Gln Arg Ala Gly Asp Val Ile Val Val Pro Pro Lys Leu Glu
                165                 170                 175
Gly Val Lys Leu Met Leu Gln Glu Leu Leu Ser Ala Asp Gly Ser Ser
            180                 185                 190
Thr Ala Glu Arg Asn Leu Arg Val Val Thr Asn Val Pro Asn Arg Ser
        195                 200                 205
Ser Ser Pro Cys His Ser Pro Thr Gly Glu Asn Gly Gly Ser Gly Ser
    210                 215                 220
Gly Ser Pro Leu Gly Ser Pro Phe Lys Leu Lys Ser Thr Glu Phe Lys
225                 230                 235                 240
Lys Glu Tyr Pro Val Asp Pro Ser Leu Pro Asp Ile Lys Asn Ser Ile
                245                 250                 255
Tyr Ala Thr Asp Glu Phe Arg Met Tyr Ser Phe Lys Val Arg Pro Cys
            260                 265                 270
Ser Arg Ala Tyr Ser His Asp Trp Thr Glu Cys Pro Phe Val His Pro
        275                 280                 285
Gly Glu Asn Ala Arg Arg Arg Asp Pro Arg Lys Phe His Tyr Ser Cys
    290                 295                 300
Val Pro Cys Pro Asp Phe Arg Lys Gly Ala Cys Arg Arg Gly Asp Met
305                 310                 315                 320
Cys Glu Tyr Ala His Gly Val Phe Glu Cys Trp Leu His Pro Ala Gln
                325                 330                 335
Tyr Arg Thr Arg Leu Cys Lys Asp Gly Thr Gly Cys Ala Arg Arg Val
            340                 345                 350
Cys Phe Phe Ala His Thr Pro Glu Glu Leu Arg Pro Leu Tyr Ala Ser
        355                 360                 365
Thr Gly Ser Ala Val Pro Ser Pro Arg Ser Asn Ala Asp Tyr Ala Ala
    370                 375                 380
Ala Leu Ser Leu Leu Pro Gly Ser Pro Ser Gly Val Ser Val Met Ser
385                 390                 395                 400
Pro Leu Ser Pro Ser Ala Ala Gly Asn Gly Met Ser His Ser Asn Met
                405                 410                 415
Ala Trp Pro Gln Pro Asn Val Pro Ala Leu His Leu Pro Gly Ser Asn
            420                 425                 430
Leu Gln Ser Ser Arg Leu Arg Ser Ser Leu Asn Ala Arg Asp Ile Pro
        435                 440                 445
Thr Asp Glu Phe Asn Met Leu Ala Asp Tyr Glu Gln Gln Gln Leu Leu

```
    450                 455                 460
Asn Glu Tyr Ser Asn Ala Leu Ser Arg Ser Gly Arg Met Lys Ser Met
465                 470                 475                 480
Pro Pro Ser Asn Leu Glu Asp Leu Phe Ser Ala Glu Gly Ser Ser Ser
                485                 490                 495
Pro Arg Phe Thr Asp Ser Ala Leu Ala Ser Ala Val Phe Ser Pro Thr
            500                 505                 510
His Lys Ser Ala Val Phe Asn Gln Phe Gln Gln Gln Gln Gln Gln Gln
        515                 520                 525
Gln Ser Met Leu Ser Pro Ile Asn Thr Ser Phe Ser Ser Pro Lys Ser
    530                 535                 540
Val Asp His Ser Leu Phe Ser Gly Gly Gly Arg Met Ser Pro Arg Asn
545                 550                 555                 560
Val Val Glu Pro Ile Ser Pro Met Ser Ala Arg Val Ser Met Leu Ala
                565                 570                 575
Gln Cys Val Lys Gln Gln Gln Gln Gln Gln Gln Gln Gln Gln Gln Gln
            580                 585                 590
His Gln Phe Arg Ser Leu Ser Ser Arg Glu Leu Arg Thr Asn Ser Ser
        595                 600                 605
Pro Ile Val Gly Ser Pro Val Asn Asn Asn Thr Trp Ser Ser Lys Trp
    610                 615                 620
Gly Ser Ser Asn Gly Gln Pro Asp Trp Gly Met Ser Ser Glu Ala Leu
625                 630                 635                 640
Gly Lys Leu Arg Ser Ser Ser Ser Phe Asp Gly Asp Glu Pro Asp Val
                645                 650                 655
Ser Trp Val Gln Ser Leu Val Lys Glu Thr Pro Ala Glu Ala Lys Glu
            660                 665                 670
Lys Ala Ala Thr Ser Ser Ser Gly Glu His Val Met Lys Gln Pro Asn
        675                 680                 685
Pro Val Glu Pro Val Met Asp His Ala Gly Leu Glu Ala Trp Ile Glu
    690                 695                 700
Gln Met Gln Leu Asp Gln Leu Val Ala Gln Gln Asn
705                 710                 715

<210>  3
<211>  13
<212>  PRT
<213>  Artificial sequence

<220>
<223>  motif 1

<220>
<221>  VARIANT
<222>  (1)..(1)
<223>  \replace = "Ala"

<220>
<221>  VARIANT
<222>  (7)..(7)
<223>  \replace = "Thr"

<400>  3
Pro Cys Ser Arg Ala Tyr Ser His Asp Trp Thr Glu Cys
1               5                   10

<210>  4
<211>  12
<212>  PRT
```

<213> Artificial sequence

<220>
<223> motif 2

<400> 4
His Pro Gly Glu Asn Ala Arg Arg Arg Asp Pro Arg
1 5 10

<210> 5
<211> 19
<212> PRT
<213> Artificial sequence

<220>
<223> motif 3

<220>
<221> VARIANT
<222> (3)..(3)
<223> \replace = "Ile"

<220>
<221> VARIANT
<222> (6)..(6)
<223> \replace = "Ser"

<220>
<221> VARIANT
<222> (11)..(11)
<223> \replace = "Ser"

<220>
<221> VARIANT
<222> (14)..(14)
<223> \replace = "Lys"

<400> 5
His Gly Val Phe Glu Cys Trp Leu His Pro Ala Gln Tyr Arg Thr Arg
1 5 10 15
Leu Cys Lys

<210> 6
<211> 5
<212> PRT
<213> Artificial sequence

<220>
<223> motif 4

<400> 6
Cys Phe Phe Ala His
1 5

<210> 7
<211> 54
<212> DNA
<213> Artificial sequence

<220>
<223> primer: prm06717

<400> 7
ggggacaagt ttgtacaaaa aagcaggctt aaacaatgtg ctgtggatca gacc 54

<210> 8
<211> 50
<212> DNA
<213> Artificial sequence

<220>
<223> primer: prm06718

<400> 8
ggggaccact ttgtacaaga aagctgggtg gttaggtctc tcaattctgc 50

<210> 9
<211> 981
<212> DNA
<213> Oryza sativa

<400> 9
aagaggcaag agcatccgta ttaaccagcc ttttgagact tgagagtgtg tgtgactcga 60
tccagcgtag tttcagttcg tgtgttggtg agtgattcca gccaagtttg cgatggcttc 120
tcagcaggaa cggctagct accacgccgg cgagaccaag gcccgcgccg aggagaagac 180
ggggcgcatg atgggcacgg cgcaggagaa ggcgcgggag gccaaggaca cggcgtccga 240
cgccgcgggg cgcgcgatgg gcaggggaca cggcgccaag gaggcgacca aggagaaggc 300
gtacgagacc aaggacgcga ccaaggagaa ggcgtacgag gcaaaggacg cggcctccga 360
cgccaccggc cgcgccatgg acaagggccg cggcgccgcg ggcgccacga gggacaaggc 420
gtacgatgcc aaggacaggg cggctgacac ggcgcagtcc gccgccgacc gcgcccgcga 480
cggcgccggg cagaccggga gctacattgg acagaccgcc gaggccgcca agcagaaagc 540
ggccggcgcc gcgcagtacg ccaaggagac cgcgatcgcc ggcaaggaca agaccggcgc 600
cgtgctccag caggcagggg agcaggtgaa gagcgtggcg gtgggggcga aggacgcggt 660
gatgtacacg ctcgggatgt caggcgataa caagaacaac gccgctgccg gcaaggacac 720
cagcacctac aagcctggaa ctgggagtga ctaccagtaa tacggtagaa gaagcatgtg 780
tcgtctttgg cactgatgcc aaagtgtacg tgttgtatcc tcttttttaa gtttcagctc 840
gacttcgacg tgttcggtgt cacactttgg tttttcagtt gtgctcaact gttcatgttt 900
ctggttccat ggagggccag tgtggaggtc aatgtttaag ctttcgtttt aaaatctgat 960
aataaagttg gttaagacct g 981

<210> 10
<211> 3372
<212> DNA
<213> Eucalyptus grandis

<400> 10
ggaagacgaa gagcaacaaa ataggtctca ctccctcctc tctcctctct cctctctctt 60
ctttctctct cttctgcttc taacaaagtc tcttccttga gagacagggc tgcgtcgtcg 120
tctttctctc tcctcgctgc gagcttctga gaaagttcaa ttctttttct cttgttctct 180
ctctctaccc ttctgggtac cactgtgaag ctccggtctt ttctattttt ttttttttg 240
ggctatctgg gtctgggcaa atccatcgcg cgctctgctc tggactgaga ggccgtcagt 300
ggctttagat ctgcgacgcc tcttgcttgc tcagtgagct gggctagttc aaatcgacga 360
agaaagcatg cgctagtgat tggtgtgggt aatacactgc attcgatctc tactaagtat 420
ccccaagtat actaagatcc cgttctcagc catgagtcaa ctgaccattc agactgagga 480
cacttttgcc agcttgcttg agcttgctgc taacaacgac acagaatctt tcggacggtg 540
tgtggaacgt gatccttcga gcatagatga aattggatat tggtatggtc gccaaaaggg 600
ttcgaagcag gtggtcaata tgcaaagaac tcctcttatg gtggctgcta catatggtag 660

```
tgttgatgta atgagactca ttctttgcct atctgatgct gatgtgaatc gaacctgcag     720
cacagacaag agcacagccc ttcactgtgc tgcctctggt ggtgctgtga atgctgtaga     780
tgctgtgagg ctactcctgt cagctggtgc tgacccaagt ttagcagatg ctaacggtca     840
gcggcctgtg gatgttattg ttgttcctcc aaagctcctt tcaataaagt ttgctcttga     900
agagctcttg tcgaccgaag gatctgtaaa tgaacacaat ctgagagtgt ccgtagccac     960
ttccaattca acctctcccc cactttcatc ttccccggat aatggttccc cagcatctgc    1020
taattgttct tcccccaaga actcaaagtt aagtgatgcc cctgttcttt atgcatcaga    1080
aaagaaggaa tacccggtgg atccatctct tccagatatc aagaatagca tttactcaac    1140
agatgaattc cgaatgtatt cttttaaagt gcggccttgt tcacgagcgt actcgcatga    1200
ttggacggag tgcccttttg ttcatccagg ggagaatgcc cgtagaaggg atccaaggaa    1260
gttccactac agctgtgtcc cttgccctga tttccggaag ggtgcttgta gacgtggaga    1320
tatgtgtgaa tatgctcatg gtgtttttga gtgctggctc catcctgctc agtatcggac    1380
tcgattatgc aaggatggta caagttgtgc tcggagagtg tgcttctttg cccacacgga    1440
gcaagagctg cgtccattgt acgtctccac tggttctgct gttccgtctc ctcgctcgag    1500
tacctctgga gctgctgcca tggattttgc tgcagccatg agcctcttac ctggttcccc    1560
atcatcagta tccatcatgt ccccttcacc cttcactcct cccatgtctc catctgctaa    1620
tggtatttct cacccatctg ttgcctggcc ccagcaaaat gtaccaactt tgcatcttcc    1680
cggaagcaat cttcagtcca gccgcttgag atcttctctt aatgcaagag atattcctca    1740
ggaggatttt gacttgctgt cagattatga tgtgcaacag cagcagctcc taaatgagtt    1800
ttccatcctt tcacaacaat cgatgggtgc taattccttg aaccgttctg gtcggctgaa    1860
aactttgacc ccctcaaacc ttgatgatct cttctctgct gagagctcat cccctcgcta    1920
cgctgatcaa gccctggctt ctgctgtttt ttcaccaacg cacaaatctg cagtaatcaa    1980
tcaatttcag cagcagcagc agagcatgtt atcacccatc aacacaacct tctctcctaa    2040
gagtgtcgac cacccttttgt tgcaagcgtc tttcggtgtt caatctgggc gaatgtcccc    2100
tcgtaacatg gatcccatct ctcctataag ttctcgtgtg tcgatgttgg cccaacgaga    2160
gaaacagcaa cagcaattac gcagcctaag ctctcgtgaa ctcggttcca attcagccgc    2220
cattgtgggt tcccccgtgg gttcttggtc gaaatgggga gctacaaatg ggaaaccaga    2280
ctgggctgtt agtgcagatg aactaggtaa gcttcgcagg tctaattcat ttgagcttgg    2340
gaacaatggt gaggagccag atctttcatg ggttcaatcc ctcgttaaag aatctcctac    2400
cgagatgaaa gaaaagcttt cgtcaactct ctctggtgtt ccagcccccg ctacatccag    2460
tgaggttccg agtatcagct cgcagatgga atcggttgat cacgaagtgc taggagcatg    2520
gctccagcag atgcagctcg atccgctcgt ggctcagcaa aactaggttg tttttttcc     2580
tacatggcct tgaggaagta gacagcggaa agttttttt ggtaaatact atgtttttc      2640
tggaaatttt tgatgctggg ggtggggtct ggaagaagat aacaaggcag gaaaggggtc    2700
agtgaagtca ctggagaaaa ggaattcatt tttaaccatt ttatcattct attacaacag    2760
aaagtaggga aaaaaaagga agaccctctg ggttatgaag agaaattaaa cccaggctag    2820
gcgttctcct ttctaatatt tccaatttta ggtccatatt actgtcattt cctttttgcc    2880
gtcttatcat atttcatcaa aatggaactg gggactaatg tttgttccat tctttcgctc    2940
ttctgattta tttgcaccct tggggtaaga tcaaaagaga aattatgatc attttctttt    3000
gaggatattt tttttcccca atatttgtga gaatgaaagt taagagggga tatgatgtgt    3060
ctggtgttgt agtatgaaaa accaataacc gagttcacct gttgctgctg gtggtagaag    3120
aagtggagaa gaagctatga tcctttgatg taacagtcaa tcaaacattt taataccttt    3180
atttttgtt tcctcatgta atccatcctt tgtgattgtc ctctctctct ctctctctct     3240
ctctctctcc ctccccgtgt tctttcttca taagcgtctt gcttgtcgat ctgtaaatta    3300
ttgaaaaggg tcatggaaag ccgtgccggt gtggattctc atttttgcaa aaaaaaaaaa    3360
aaaaaaaaaa aa                                                        3372
```

<210> 11
<211> 704
<212> PRT
<213> Eucalyptus grandis

<400> 11

```
Met Ser Gln Leu Thr Ile Gln Thr Glu Asp Thr Phe Ala Ser Leu Leu
1               5                   10                  15
Glu Leu Ala Ala Asn Asn Asp Thr Glu Ser Phe Gly Arg Cys Val Glu
            20                  25                  30
Arg Asp Pro Ser Ser Ile Asp Glu Ile Gly Tyr Trp Tyr Gly Arg Gln
        35                  40                  45
```

```
Lys Gly Ser Lys Gln Val Val Asn Met Gln Arg Thr Pro Leu Met Val
    50                  55                  60
Ala Ala Thr Tyr Gly Ser Val Asp Val Met Arg Leu Ile Leu Cys Leu
65                  70                  75                  80
Ser Asp Ala Asp Val Asn Arg Thr Cys Ser Thr Asp Lys Ser Thr Ala
                85                  90                  95
Leu His Cys Ala Ala Ser Gly Gly Ala Val Asn Ala Val Asp Ala Val
            100                 105                 110
Arg Leu Leu Leu Ser Ala Gly Ala Asp Pro Ser Leu Ala Asp Ala Asn
        115                 120                 125
Gly Gln Arg Pro Val Asp Val Ile Val Val Pro Pro Lys Leu Leu Ser
    130                 135                 140
Ile Lys Phe Ala Leu Glu Glu Leu Leu Ser Thr Glu Gly Ser Val Asn
145                 150                 155                 160
Glu His Asn Leu Arg Val Ser Val Ala Thr Ser Asn Ser Thr Ser Pro
                165                 170                 175
Pro Leu Ser Ser Ser Pro Asp Asn Gly Ser Pro Ala Ser Ala Asn Cys
            180                 185                 190
Ser Ser Pro Lys Asn Ser Lys Leu Ser Asp Ala Pro Val Leu Tyr Ala
        195                 200                 205
Ser Glu Lys Lys Glu Tyr Pro Val Asp Pro Ser Leu Pro Asp Ile Lys
    210                 215                 220
Asn Ser Ile Tyr Ser Thr Asp Glu Phe Arg Met Tyr Ser Phe Lys Val
225                 230                 235                 240
Arg Pro Cys Ser Arg Ala Tyr Ser His Asp Trp Thr Glu Cys Pro Phe
                245                 250                 255
Val His Pro Gly Glu Asn Ala Arg Arg Arg Asp Pro Arg Lys Phe His
            260                 265                 270
Tyr Ser Cys Val Pro Cys Pro Asp Phe Arg Lys Gly Ala Cys Arg Arg
        275                 280                 285
Gly Asp Met Cys Glu Tyr Ala His Gly Val Phe Glu Cys Trp Leu His
    290                 295                 300
Pro Ala Gln Tyr Arg Thr Arg Leu Cys Lys Asp Gly Thr Ser Cys Ala
305                 310                 315                 320
Arg Arg Val Cys Phe Phe Ala His Thr Glu Gln Glu Leu Arg Pro Leu
                325                 330                 335
Tyr Val Ser Thr Gly Ser Ala Val Pro Ser Pro Arg Ser Ser Thr Ser
            340                 345                 350
Gly Ala Ala Ala Met Asp Phe Ala Ala Ala Met Ser Leu Leu Pro Gly
        355                 360                 365
Ser Pro Ser Ser Val Ser Ile Met Ser Pro Ser Pro Phe Thr Pro Pro
    370                 375                 380
Met Ser Pro Ser Ala Asn Gly Ile Ser His Pro Ser Val Ala Trp Pro
385                 390                 395                 400
Gln Gln Asn Val Pro Thr Leu His Leu Pro Gly Ser Asn Leu Gln Ser
                405                 410                 415
Ser Arg Leu Arg Ser Ser Leu Asn Ala Arg Asp Ile Pro Gln Glu Asp
            420                 425                 430
Phe Asp Leu Leu Ser Asp Tyr Asp Val Gln Gln Gln Gln Leu Leu Asn
        435                 440                 445
Glu Phe Ser Ile Leu Ser Gln Gln Ser Met Gly Ala Asn Ser Leu Asn
    450                 455                 460
Arg Ser Gly Arg Leu Lys Thr Leu Thr Pro Ser Asn Leu Asp Asp Leu
465                 470                 475                 480
Phe Ser Ala Glu Ser Ser Ser Pro Arg Tyr Ala Asp Gln Ala Leu Ala
                485                 490                 495
Ser Ala Val Phe Ser Pro Thr His Lys Ser Ala Val Ile Asn Gln Phe
            500                 505                 510
Gln Gln Gln Gln Gln Ser Met Leu Ser Pro Ile Asn Thr Thr Phe Ser
```

```
        515                 520                 525
Pro Lys Ser Val Asp His Pro Leu Leu Gln Ala Ser Phe Gly Val Gln
    530                 535                 540
Ser Gly Arg Met Ser Pro Arg Asn Met Asp Pro Ile Ser Pro Ile Ser
545                 550                 555                 560
Ser Arg Val Ser Met Leu Ala Gln Arg Glu Lys Gln Gln Gln Gln Leu
                565                 570                 575
Arg Ser Leu Ser Ser Arg Glu Leu Gly Ser Asn Ser Ala Ala Ile Val
            580                 585                 590
Gly Ser Pro Val Gly Ser Trp Ser Lys Trp Gly Ala Thr Asn Gly Lys
        595                 600                 605
Pro Asp Trp Ala Val Ser Ala Asp Glu Leu Gly Lys Leu Arg Arg Ser
    610                 615                 620
Asn Ser Phe Glu Leu Gly Asn Asn Gly Glu Glu Pro Asp Leu Ser Trp
625                 630                 635                 640
Val Gln Ser Leu Val Lys Glu Ser Pro Thr Glu Met Lys Glu Lys Leu
                645                 650                 655
Ser Ser Thr Leu Ser Gly Val Pro Ala Pro Ala Thr Ser Ser Glu Val
            660                 665                 670
Pro Ser Ile Ser Ser Gln Met Glu Ser Val Asp His Glu Val Leu Gly
        675                 680                 685
Ala Trp Leu Gln Gln Met Gln Leu Asp Pro Leu Val Ala Gln Gln Asn
    690                 695                 700

<210>  12
<211>  1860
<212>  DNA
<213>  Oryza sativa

<400>  12
atgggggagc ctgggggcgc cgaggcggcc gtctccgcga ggctgctcga gctggcggcc      60
gacgacaacg cggcggggct cggggagctc ctcgcggcgt ggccctccct cgccgacgag     120
cccgcgccgt ggtacacccc ggcgcggggc gcggagccgc tgaccccgct catggtcgcc     180
gccgtgtacg gctcggtggg ctgcctcgac gcgctcctct cgccgcccta cctcgtggac     240
cccaaccgcg cctcggcgtc gtcgctctcc accccgctcc acctcgccgc cgcgggcggg     300
tccgcctccg cccccgcggc ggtctcccgc ctcctcgccg ccggcgccga cccggccctc     360
ctcgaccacc tccagcgccg ggcgtccgac ctcgtcgcgc tcccgcccaa ctcgctcccg     420
ctcaagaacc acctcctctc cctcctcggc gcccgcaagg agtggcctcc cgacccctcc     480
ctccccgaca tcaagaacgg cgcctacgcc tccgacgact tcaggatgta ctcgttcaag     540
gtgcgcgcgt gctcgcgggc ctactcccat gactggacgg agtgcccctt cgtccacccc     600
ggcgagaacg cgcggcggcg cgacccgagg aagtaccact acagctgcgt gccgtgcccg     660
gagttcaaga agggggccgg gtgcaggaga ggggacatgt gcgagtacgc gcacggggtg     720
ttcgagagct ggctccaccc ggcgcagtac cggacgcgcc tctgcaagga cggcgtcggc     780
tgcgcccgcc gcgtctgctt cttcgcccac acgcccgacg agctccgccc gctctacgtc     840
tccacgggct ccgccgtgcc gtcgccgcgc ggggcgttgg agatggcggc ggcggcggcg     900
gcgatgggga tggggctgtc gtcgccgggg tcgtcgtcgt tcacgccgcc gctatcgccg     960
tcggccggcg gggcggggg cgggggcggg ggcagcggcg gcggcggcgc gtggccgcag    1020
cagccgagcg tgccggcgct ctgcctgccc gggagcgccg ggaacctcca cctgagccgg    1080
ctgcgcacgt cgctgagcgc gcgcgacatg gccgtcgacg agctgctcgc cgcggcggcg    1140
gcggcggcgg actacgacgg cctcgtcgcc tcccccgcct ccatccggtc cgcgaggggg    1200
aaggcgcttg tgccgtcaaa tctcgacgag ctcttctccg ctgagctcgc cgccgccgcg    1260
gcgtcgcgct cgccgcgcta cgccgaccaa ggcggcgccg cgttctcccc gacccgcaag    1320
gccaccgtgc tcaaccaatt ccagctgcag cagcagcata gcttgctctc gccgcgggcg    1380
gccgcggtga caccagagcc ggtctcccca atgagctccc gcctcctcgc cgcgctggcg    1440
cagcgggaga agatgcagca gcagacgctg cggagcatga gctcacggga cctcggcaac    1500
gccgcgtcgc tgctggtcgg ctcgccggtg agctcgagca tgtccaaatg gggttcccc     1560
tccggcaacc cggactgggg cgccgacgac gaggagctcg gccgcctcaa gcgttgctcc    1620
tcgttcgagc tccggtccgg agccgccaat ggcaaccatg agcctgacct ctcatgggtc    1680
aacaccctag tgaaggagcc gacaccggag aagatgatga cgacgacatc ggcaatggat    1740
```

```
tccattggca tcttgggaca gaacacaagc cgtgatcaca tcgtcggagg cgaggatgac    1800
actgccggag tcatcagcag ctggcttgaa cagctccagc tcgatgagat ggttgtctag    1860

<210>  13
<211>  619
<212>  PRT
<213>  Oryza sativa

<400>  13
Met Gly Glu Pro Gly Gly Ala Glu Ala Ala Val Ser Ala Arg Leu Leu
1               5                   10                  15
Glu Leu Ala Ala Asp Asp Asn Ala Ala Gly Leu Gly Glu Leu Leu Ala
            20                  25                  30
Ala Trp Pro Ser Leu Ala Asp Glu Pro Ala Pro Trp Tyr Thr Pro Ala
        35                  40                  45
Arg Gly Ala Glu Pro Leu Thr Pro Leu Met Val Ala Ala Val Tyr Gly
    50                  55                  60
Ser Val Gly Cys Leu Asp Ala Leu Leu Ser Pro Pro Tyr Leu Val Asp
65                  70                  75                  80
Pro Asn Arg Ala Ser Ala Ser Ser Leu Ser Thr Pro Leu His Leu Ala
                85                  90                  95
Ala Ala Gly Gly Ser Ala Ser Ala Pro Ala Ala Val Ser Arg Leu Leu
            100                 105                 110
Ala Ala Gly Ala Asp Pro Ala Leu Leu Asp His Leu Gln Arg Arg Ala
        115                 120                 125
Ser Asp Leu Val Ala Leu Pro Pro Asn Ser Leu Pro Leu Lys Asn His
    130                 135                 140
Leu Leu Ser Leu Leu Gly Ala Arg Lys Glu Trp Pro Pro Asp Pro Ser
145                 150                 155                 160
Leu Pro Asp Ile Lys Asn Gly Ala Tyr Ala Ser Asp Asp Phe Arg Met
                165                 170                 175
Tyr Ser Phe Lys Val Arg Ala Cys Ser Arg Ala Tyr Ser His Asp Trp
            180                 185                 190
Thr Glu Cys Pro Phe Val His Pro Gly Glu Asn Ala Arg Arg Arg Asp
        195                 200                 205
Pro Arg Lys Tyr His Tyr Ser Cys Val Pro Cys Pro Glu Phe Lys Lys
    210                 215                 220
Gly Ala Gly Cys Arg Arg Gly Asp Met Cys Glu Tyr Ala His Gly Val
225                 230                 235                 240
Phe Glu Ser Trp Leu His Pro Ala Gln Tyr Arg Thr Arg Leu Cys Lys
                245                 250                 255
Asp Gly Val Gly Cys Ala Arg Arg Val Cys Phe Phe Ala His Thr Pro
            260                 265                 270
Asp Glu Leu Arg Pro Leu Tyr Val Ser Thr Gly Ser Ala Val Pro Ser
        275                 280                 285
Pro Arg Gly Ala Leu Glu Met Ala Ala Ala Ala Ala Ala Met Gly Met
    290                 295                 300
Gly Leu Ser Ser Pro Gly Ser Ser Ser Phe Thr Pro Pro Leu Ser Pro
305                 310                 315                 320
Ser Ala Gly Gly Gly Gly Gly Gly Gly Gly Gly Ser Gly Gly Gly Gly
                325                 330                 335
Ala Trp Pro Gln Gln Pro Ser Val Pro Ala Leu Cys Leu Pro Gly Ser
            340                 345                 350
Ala Gly Asn Leu His Leu Ser Arg Leu Arg Thr Ser Leu Ser Ala Arg
        355                 360                 365
Asp Met Ala Val Asp Glu Leu Leu Ala Ala Ala Ala Ala Ala Ala Asp
    370                 375                 380
Tyr Asp Gly Leu Val Ala Ser Pro Ala Ser Ile Arg Ser Ala Arg Gly
385                 390                 395                 400
```

```
Lys Ala Leu Val Pro Ser Asn Leu Asp Glu Leu Phe Ser Ala Glu Leu
                405                 410                 415
Ala Ala Ala Ala Ala Ser Arg Ser Pro Arg Tyr Ala Asp Gln Gly Gly
            420                 425                 430
Ala Ala Phe Ser Pro Thr Arg Lys Ala Thr Val Leu Asn Gln Phe Gln
        435                 440                 445
Leu Gln Gln Gln His Ser Leu Leu Ser Pro Arg Ala Ala Ala Val Thr
    450                 455                 460
Pro Glu Pro Val Ser Pro Met Ser Ser Arg Leu Leu Ala Ala Leu Ala
465                 470                 475                 480
Gln Arg Glu Lys Met Gln Gln Gln Thr Leu Arg Ser Met Ser Ser Arg
                485                 490                 495
Asp Leu Gly Asn Ala Ala Ser Leu Leu Val Gly Ser Pro Val Ser Ser
            500                 505                 510
Ser Met Ser Lys Trp Gly Phe Pro Ser Gly Asn Pro Asp Trp Gly Ala
        515                 520                 525
Asp Asp Glu Glu Leu Gly Arg Leu Lys Arg Cys Ser Ser Phe Glu Leu
    530                 535                 540
Arg Ser Gly Ala Ala Asn Gly Asn His Glu Pro Asp Leu Ser Trp Val
545                 550                 555                 560
Asn Thr Leu Val Lys Glu Pro Thr Pro Glu Lys Met Met Thr Thr Thr
                565                 570                 575
Ser Ala Met Asp Ser Ile Gly Ile Leu Gly Gln Asn Thr Ser Arg Asp
            580                 585                 590
His Ile Val Gly Gly Glu Asp Asp Thr Ala Gly Val Ile Ser Ser Trp
        595                 600                 605
Leu Glu Gln Leu Gln Leu Asp Glu Met Val Val
    610                 615
```

<210> 14
<211> 2106
<212> DNA
<213> Medicago truncatula

<400> 14

```
atgaaaaatc taactgttcg tactgatgat tctttttcca gcttacttga acatgcttct      60
aacaatgatt ttgaagattt caaggtagct ctagatagtg atgcttcact tattaatgaa     120
gttggcttct ggtatgtccg tcaaaaggga tctaaccaaa ttgttcttga gcaccgaacc     180
cctttaatgg tggctgcttc ctatgggagt attgatattc taaagcttat actctcatat     240
cccgaggctg atgttaattt ctcctgtgga actgataaaa gcactgctct tcactgtgct     300
gcctcaagtg gttcagttaa tgctgttgat gctataaaat tgcttttatc agctggtgct     360
gatatcaatt ctgtggatgc taatgggaaa cgccctgtgg atgttatcgt tgttcctatt     420
gttgttcctc ataagctcga aggtgttaaa acaattcttg aagaacttct ctcagacagt     480
gcttctgaag gatctgtgga tgattgctct cttcccctgt ctcttatttc atcgagtcct     540
ggttcatctg cccctttatc atctgctgaa aatggatctc catcctctcc tgtggctccc     600
aagtttacag atacagctgt taattctaca tcagaaaaga aagagtatcc agttgaccca     660
tctcttcctg acataaaaaa cagcatgtat gccacagatg aattccgcat gtattcattc     720
aaggttcgtc cttgttctcg tgcatactct catgattgga ctgagtgtcc ttttgtgcat     780
cctggagaga atgctcgaag gagagaccct agaaagtttc actacagctg tgtgccatgc     840
cctgatttta ggaaaggggc ttgccgacgt tcggatatgt gtgaatatgc tcatggagta     900
ttcgagtgct ggctacaccc agctcagtat cggacaaggc tgtgcaaaga cggtatgggt     960
tgtaaccgaa gggtgtgctt cttcgctcac tcacctgaag agctgcgtcc gctgtatgtg    1020
tccactggtt ctgctgttcc ttcaccccga tcagctgctt ctactgctaa tgtcatggac    1080
atggctgctg ctatgagcct ttccctggt  tcaccatcat caatctcttt gatgtctcaa    1140
tcaccctttg cacagcctcc tctatctcca tctgcaaatg gcaataatgc ttggccacag    1200
cccaatgtgc cagctcttca tttaccagga agcattaatc aaactagtcg tttgagatct    1260
tctcttagtg cccgtgatat gccacacgac gacttcaaca atatgttgca agactttgat    1320
gggcagcagc agatactaaa tgacttgagc tgtttctcac agccccgtcc tggtgctatt    1380
tcagttggtc gatctggccg ccctaaaaca ctaactccct caaatctgga tgatcttttt    1440
```

tgtgctgaga ttgcttcatc tcctaggtat tccgaccccg ctgcggcttc tgtattttcc 1500
ccaacacaca aatctgctgt cttcaaccag tttcaacagc ttcaaagctc cttatcaccc 1560
atcaacacaa atgtcatgtc tcctacaaac gtagagcatc ccctgttcca ccaggcttca 1620
tatggtctct cttctcctgg aaggatgtca ccaagaagta tggaagccct atctccaatg 1680
agttctcggc tgtcagcttt tgctcagcgt gagaaacaac agcagcagca gcaacagctg 1740
cgtagcctca gctcaagaga actcggtgct aacaatcctc tctcagctgt tgggtcccct 1800
gttaactcct ggtccaagtg ggatcatccc cctattggaa aagctgattg gtcggtaaat 1860
ccaaatgact tcggtcaaac acagagatca acttcttttg agcatggaaa caatggagaa 1920
gagcctgatg taggttgggt ccattccctt gtcaaggatc ccacacctga gaagaaagag 1980
aagcttgcag gttccggccc aattccatcc gttgaaaaga atcccaatcc tcaagcggac 2040
ggcattgatc actctgtttt gggagcttgg ctcgagcaac tgcagctgga tcaacttgta 2100
gtctag 2106

<210> 15
<211> 701
<212> PRT
<213> Medicago truncatula

<400> 15
Met Lys Asn Leu Thr Val Arg Thr Asp Asp Ser Phe Ser Ser Leu Leu
1 5 10 15
Glu His Ala Ser Asn Asn Asp Phe Glu Asp Phe Lys Val Ala Leu Asp
20 25 30
Ser Asp Ala Ser Leu Ile Asn Glu Val Gly Phe Trp Tyr Val Arg Gln
35 40 45
Lys Gly Ser Asn Gln Ile Val Leu Glu His Arg Thr Pro Leu Met Val
50 55 60
Ala Ala Ser Tyr Gly Ser Ile Asp Ile Leu Lys Leu Ile Leu Ser Tyr
65 70 75 80
Pro Glu Ala Asp Val Asn Phe Ser Cys Gly Thr Asp Lys Ser Thr Ala
85 90 95
Leu His Cys Ala Ala Ser Ser Gly Ser Val Asn Ala Val Asp Ala Ile
100 105 110
Lys Leu Leu Leu Ser Ala Gly Ala Asp Ile Asn Ser Val Asp Ala Asn
115 120 125
Gly Lys Arg Pro Val Asp Val Ile Val Val Pro Ile Val Val Pro His
130 135 140
Lys Leu Glu Gly Val Lys Thr Ile Leu Glu Glu Leu Leu Ser Asp Ser
145 150 155 160
Ala Ser Glu Gly Ser Val Asp Asp Cys Ser Leu Pro Leu Ser Leu Ile
165 170 175
Ser Ser Ser Pro Gly Ser Ser Ala Pro Leu Ser Ser Ala Glu Asn Gly
180 185 190
Ser Pro Ser Ser Pro Val Ala Pro Lys Phe Thr Asp Thr Ala Val Asn
195 200 205
Ser Thr Ser Glu Lys Lys Glu Tyr Pro Val Asp Pro Ser Leu Pro Asp
210 215 220
Ile Lys Asn Ser Met Tyr Ala Thr Asp Glu Phe Arg Met Tyr Ser Phe
225 230 235 240
Lys Val Arg Pro Cys Ser Arg Ala Tyr Ser His Asp Trp Thr Glu Cys
245 250 255
Pro Phe Val His Pro Gly Glu Asn Ala Arg Arg Arg Asp Pro Arg Lys
260 265 270
Phe His Tyr Ser Cys Val Pro Cys Pro Asp Phe Arg Lys Gly Ala Cys
275 280 285
Arg Arg Ser Asp Met Cys Glu Tyr Ala His Gly Val Phe Glu Cys Trp
290 295 300
Leu His Pro Ala Gln Tyr Arg Thr Arg Leu Cys Lys Asp Gly Met Gly
305 310 315 320

```
Cys Asn Arg Arg Val Cys Phe Phe Ala His Ser Pro Glu Glu Leu Arg
                325                 330                 335
Pro Leu Tyr Val Ser Thr Gly Ser Ala Val Pro Ser Pro Arg Ser Ala
            340                 345                 350
Ala Ser Thr Ala Asn Val Met Asp Met Ala Ala Ala Met Ser Leu Phe
        355                 360                 365
Pro Gly Ser Pro Ser Ser Ile Ser Leu Met Ser Gln Ser Pro Phe Ala
    370                 375                 380
Gln Pro Pro Leu Ser Pro Ser Ala Asn Gly Asn Asn Ala Trp Pro Gln
385                 390                 395                 400
Pro Asn Val Pro Ala Leu His Leu Pro Gly Ser Ile Asn Gln Thr Ser
                405                 410                 415
Arg Leu Arg Ser Ser Leu Ser Ala Arg Asp Met Pro His Asp Asp Phe
            420                 425                 430
Asn Asn Met Leu Gln Asp Phe Asp Gly Gln Gln Gln Ile Leu Asn Asp
        435                 440                 445
Leu Ser Cys Phe Ser Gln Pro Arg Pro Gly Ala Ile Ser Val Gly Arg
    450                 455                 460
Ser Gly Arg Pro Lys Thr Leu Thr Pro Ser Asn Leu Asp Asp Leu Phe
465                 470                 475                 480
Cys Ala Glu Ile Ala Ser Ser Pro Arg Tyr Ser Asp Pro Ala Ala Ala
                485                 490                 495
Ser Val Phe Ser Pro Thr His Lys Ser Ala Val Phe Asn Gln Phe Gln
            500                 505                 510
Gln Leu Gln Ser Ser Leu Ser Pro Ile Asn Thr Asn Val Met Ser Pro
        515                 520                 525
Thr Asn Val Glu His Pro Leu Phe His Gln Ala Ser Tyr Gly Leu Ser
    530                 535                 540
Ser Pro Gly Arg Met Ser Pro Arg Ser Met Glu Ala Leu Ser Pro Met
545                 550                 555                 560
Ser Ser Arg Leu Ser Ala Phe Ala Gln Arg Glu Lys Gln Gln Gln Gln
                565                 570                 575
Gln Gln Gln Leu Arg Ser Leu Ser Ser Arg Glu Leu Gly Ala Asn Asn
            580                 585                 590
Pro Leu Ser Ala Val Gly Ser Pro Val Asn Ser Trp Ser Lys Trp Gly
        595                 600                 605
Ser Ser Pro Ile Gly Lys Ala Asp Trp Ser Val Asn Pro Asn Asp Phe
    610                 615                 620
Gly Gln Thr Gln Arg Ser Thr Ser Phe Glu His Gly Asn Asn Gly Glu
625                 630                 635                 640
Glu Pro Asp Val Gly Trp Val His Ser Leu Val Lys Asp Pro Thr Pro
                645                 650                 655
Glu Lys Lys Glu Lys Leu Ala Gly Ser Gly Pro Ile Pro Ser Val Glu
            660                 665                 670
Lys Asn Pro Asn Pro Gln Ala Asp Gly Ile Asp His Ser Val Leu Gly
        675                 680                 685
Ala Trp Leu Glu Gln Leu Gln Leu Asp Gln Leu Val Val
    690                 695                 700

<210>  16
<211>  2841
<212>  DNA
<213>  Oryza sativa

<400>  16
atgaacggca cgccgatctc cgcgtccgcc gcggccggcg tcgacggagt cggcgcggcg      60
gtggcgctgg cggccgcgac caagaagagt gccgccgcgg cggccgccgt cgccgagatg     120
gcgaaaaccc tcaccgtcga cacggacgac gccttcgcgg ggctcctcga gctcgccgcg     180
gacgacgacg cggagggcct gcgccgcgcg ctggagcgcg ccccgcccgc cgccgcggac     240
```

```
gaggcgggcc tctggtacgg ccgccgcaag gtcctcgagc accgcacgcc gctgatggtc     300
gcggccacct atggcagcct cgcggtgctt cgcctgctgc tgtccctccc gtccgtcgat     360
gtcaatcgcc gctgtggctc cgacggcacc accgccctcc actgtgcggc gtctggtggc     420
tcgccgtctt gtgtggaggc cgtcaagctg ctgcttgctg ctggggctga tgctgatgcc     480
acggatgctt ccggatatcg tccagctgat gtgatctctg ttcctccaaa gatgtttgac     540
gccaagattg ccctccaaga tcttcttgga tgcccaaagg ctgggcatgg cgttctccgg     600
gtggtgacaa gggccgcaaa ctctatgttg tcacctgtat catcccctac agcagaagat     660
gcacgatctc catcagctgc tgtgatgatg acgacaaagt ttgcagatct tccaagggtt     720
gtgacatcgg aaaagaaaga atatccagtg gatccgtccc ttcccgatat caagaacagc     780
atctatgctt ccgatgagtt ccgcatgtac tcatttaaga tcaggccatg ctcgcgggcg     840
tactcacatg attggactga gtgcccgttt gttcacccag gggagaacgc acggcgtcgg     900
gaccctcgca agtatcacta cagctgtgtg ccatgccccg actttagaaa gggagtttgc     960
cggcgtggtg acatgtgtga atatgctcat ggcgtgttcg agtgttggct ccatccagca    1020
cagtaccgta ctcgcctttg caaggatggc acaagctgta atcgccgtgt ctgtttcttt    1080
gcgcatacaa ctgatgagct ccgaccacta tatgtttcca ctggatctgc agtaccatcc    1140
ccaagagcct cggcaacagc tacaatggag atggctgcag caatgggctt gatgcctggt    1200
tctccatcat cagtttcagc agtcatgtcc ccatttacac caccaatgtc cccttcaggc    1260
aatgggatgc ccccttcatt gggctggcag cagccaaatg ttccgacact acaccttcca    1320
ggcagcagcc ttcagtcgag ccggctccgt acctcactta gtgcaaggga tatgcctgct    1380
gatgattact ccctgatgca ggatattgat tcacagctta taaatgattt gtgctattca    1440
cgtattggtt catcaacagg aaaccacacg tctcggacca agtccctaaa tccgtcaaac    1500
ttggatgatc tcttctctgc tgagatggtc tcttccccga ggtatagtaa tgctgatcag    1560
ggtggtatgt tttcaccatc tcacaaggct gctttcctta atcagttcca gcaacagcag    1620
caggcacttc tttcaccaat caacacagtc ttctccccga agtctgtgga caaccagcag    1680
ttgccttcac actcatctct gttgcaagca tcacttggta tatcctcccc tggccgcatg    1740
tctcctcgat gtgttgaatc tgggtcccct atgaactctc atcttgctgc tgctcttgct    1800
cagcgtgaga agcaacagca gacaatgaga agtctcagtt ctcgtgatct tgggccgagt    1860
gctgcaagag catcaggtgt tgttggctcc cctctaagct catcatggtc aaagtgggga    1920
tcaccttcag ggacacctga ctggggtgtt aatggtgaag aattgggcaa gcttcgccgg    1980
tcatcatcgt ttgagctgag atctggtggt gatgatccag atctctcttg ggtacacaca    2040
ctggttaagg aatctccacc agagaagcaa gtcactactg ctgaatccat aaactctgtt    2100
ggaccttcac cactgatgcc tcccagtgta agcaacggtg aaggtcctag tctgaatgcc    2160
ccgctggatg ggcatgacca agctgctgtt attggagcat tgcttgaaca gatgcagctt    2220
gatcagcata ttggtagtct agcaacataa gcgctgaatg agcctggaaa gtgcaaggag    2280
ttattattct tagttaatga atttggagta attttttttcc tgttcattaa gatggtcagc    2340
aagcaaaagg atggatagct gatggtggtg attcagagat tggttttctt tactttattg    2400
aggtaaatca tatacattat tgaggttcca gtaggttgaa agattgaagt accttgattg    2460
gggtcgtttc aagaccgacc caggtagaat cgcacccccgg cagcttcaat tcatcggtca    2520
aaaatatttc cctgttttgt taattaaccc cgttaaaaaa gaagactcgt ttggtgtttc    2580
ggaattcttt tctttacctt agcggtgttt attttgttta ttatgatatt gatacttgat    2640
gtactgatgg gtataaggtt ggttaccagg catgctatag tggtatatca agtcccaaag    2700
tattcttttt ctccctttca ccatttgtcg aggatcatac tatggccttg ttttggtcag    2760
atcttgaggc ctgtataatc cttggatttg taataatgta atattgtcat tgaacttaca    2820
ttgctattgt tttgcaatcg c                                             2841
```

<210> 17
<211> 749
<212> PRT
<213> Oryza sativa

<400> 17

```
Met Asn Gly Thr Pro Ile Ser Ala Ser Ala Ala Ala Gly Val Asp Gly
1               5                   10                  15
Val Gly Ala Ala Val Ala Leu Ala Ala Ala Thr Lys Lys Ser Ala Ala
            20                  25                  30
Ala Ala Ala Ala Val Ala Glu Met Ala Lys Thr Leu Thr Val Asp Thr
        35                  40                  45
Asp Asp Ala Phe Ala Gly Leu Leu Glu Leu Ala Ala Asp Asp Asp Ala
    50                  55                  60
```

```
Glu Gly Leu Arg Arg Ala Leu Glu Arg Ala Pro Pro Ala Ala Ala Asp
65                  70                  75                  80
Glu Ala Gly Leu Trp Tyr Gly Arg Arg Lys Val Leu Glu His Arg Thr
                85                  90                  95
Pro Leu Met Val Ala Ala Thr Tyr Gly Ser Leu Ala Val Leu Arg Leu
            100                 105                 110
Leu Leu Ser Leu Pro Ser Val Asp Val Asn Arg Arg Cys Gly Ser Asp
        115                 120                 125
Gly Thr Thr Ala Leu His Cys Ala Ala Ser Gly Gly Ser Pro Ser Cys
    130                 135                 140
Val Glu Ala Val Lys Leu Leu Leu Ala Ala Gly Ala Asp Ala Asp Ala
145                 150                 155                 160
Thr Asp Ala Ser Gly Tyr Arg Pro Ala Asp Val Ile Ser Val Pro Pro
                165                 170                 175
Lys Met Phe Asp Ala Lys Ile Ala Leu Gln Asp Leu Leu Gly Cys Pro
            180                 185                 190
Lys Ala Gly His Gly Val Leu Arg Val Val Thr Arg Ala Ala Asn Ser
        195                 200                 205
Met Leu Ser Pro Val Ser Ser Pro Thr Ala Glu Asp Ala Arg Ser Pro
    210                 215                 220
Ser Ala Ala Val Met Met Thr Thr Lys Phe Ala Asp Leu Pro Arg Val
225                 230                 235                 240
Val Thr Ser Glu Lys Lys Glu Tyr Pro Val Asp Pro Ser Leu Pro Asp
                245                 250                 255
Ile Lys Asn Ser Ile Tyr Ala Ser Asp Glu Phe Arg Met Tyr Ser Phe
            260                 265                 270
Lys Ile Arg Pro Cys Ser Arg Ala Tyr Ser His Asp Trp Thr Glu Cys
        275                 280                 285
Pro Phe Val His Pro Gly Glu Asn Ala Arg Arg Arg Asp Pro Arg Lys
    290                 295                 300
Tyr His Tyr Ser Cys Val Pro Cys Pro Asp Phe Arg Lys Gly Val Cys
305                 310                 315                 320
Arg Arg Gly Asp Met Cys Glu Tyr Ala His Gly Val Phe Glu Cys Trp
                325                 330                 335
Leu His Pro Ala Gln Tyr Arg Thr Arg Leu Cys Lys Asp Gly Thr Ser
            340                 345                 350
Cys Asn Arg Arg Val Cys Phe Phe Ala His Thr Thr Asp Glu Leu Arg
        355                 360                 365
Pro Leu Tyr Val Ser Thr Gly Ser Ala Val Pro Ser Pro Arg Ala Ser
    370                 375                 380
Ala Thr Ala Thr Met Glu Met Ala Ala Ala Met Gly Leu Met Pro Gly
385                 390                 395                 400
Ser Pro Ser Ser Val Ser Ala Val Met Ser Pro Phe Thr Pro Pro Met
                405                 410                 415
Ser Pro Ser Gly Asn Gly Met Pro Pro Ser Leu Gly Trp Gln Gln Pro
            420                 425                 430
Asn Val Pro Thr Leu His Leu Pro Gly Ser Ser Leu Gln Ser Ser Arg
        435                 440                 445
Leu Arg Thr Ser Leu Ser Ala Arg Asp Met Pro Ala Asp Asp Tyr Ser
    450                 455                 460
Leu Met Gln Asp Ile Asp Ser Gln Leu Ile Asn Asp Leu Cys Tyr Ser
465                 470                 475                 480
Arg Ile Gly Ser Ser Thr Gly Asn His Thr Ser Arg Thr Lys Ser Leu
                485                 490                 495
Asn Pro Ser Asn Leu Asp Asp Leu Phe Ser Ala Glu Met Val Ser Ser
            500                 505                 510
Pro Arg Tyr Ser Asn Ala Asp Gln Gly Gly Met Phe Ser Pro Ser His
        515                 520                 525
Lys Ala Ala Phe Leu Asn Gln Phe Gln Gln Gln Gln Gln Ala Leu Leu
```

```
    530                 535                 540
Ser Pro Ile Asn Thr Val Phe Ser Pro Lys Ser Val Asp Asn Gln Gln
545                 550                 555                 560
Leu Pro Ser His Ser Ser Leu Leu Gln Ala Ser Leu Gly Ile Ser Ser
                565                 570                 575
Pro Gly Arg Met Ser Pro Arg Cys Val Glu Ser Gly Ser Pro Met Asn
            580                 585                 590
Ser His Leu Ala Ala Ala Leu Ala Gln Arg Glu Lys Gln Gln Gln Thr
        595                 600                 605
Met Arg Ser Leu Ser Ser Arg Asp Leu Gly Pro Ser Ala Ala Arg Ala
    610                 615                 620
Ser Gly Val Val Gly Ser Pro Leu Ser Ser Ser Trp Ser Lys Trp Gly
625                 630                 635                 640
Ser Pro Ser Gly Thr Pro Asp Trp Gly Val Asn Gly Glu Glu Leu Gly
                645                 650                 655
Lys Leu Arg Arg Ser Ser Ser Phe Glu Leu Arg Ser Gly Gly Asp Asp
            660                 665                 670
Pro Asp Leu Ser Trp Val His Thr Leu Val Lys Glu Ser Pro Pro Glu
        675                 680                 685
Lys Gln Val Thr Thr Ala Glu Ser Ile Asn Ser Val Gly Pro Ser Pro
    690                 695                 700
Leu Met Pro Pro Ser Val Ser Asn Gly Glu Gly Pro Ser Leu Asn Ala
705                 710                 715                 720
Pro Leu Asp Gly His Asp Gln Ala Ala Val Ile Gly Ala Leu Leu Glu
                725                 730                 735
Gln Met Gln Leu Asp Gln His Ile Gly Ser Leu Ala Thr
            740                 745

<210>  18
<211>  2769
<212>  DNA
<213>  Arabidopsis thaliana

<400>  18
acaccagtta ccctctcatc cgttttcgtt ttttttttct ctctttcaaa aatctctcag      60
ctgaggttga tcgatcttct tcttcttctt cctcactctt tagatttgtt ccttttgcat     120
tttacacttt tggatctgaa aatgtggttc tctgtttcgc ccgttaccgt ttagattcag     180
ttctgttttt ttcttacccg atcgcttgat tcggactgtg atctttgatc ttttttcttc     240
tccagtgccg tgaaggatgt gtggtcttgc taagaagctg gatatagagg atactttgac     300
atcactgtca gaccaagaga atgaatcttt ggccaaaccc atgaatgatg ctgctgaatg     360
ggaacattcg ttttctgcct tgcttgagtt tgctgcagac aacgatgtgg aggggtttag     420
gcggcaactc tctgatgtgt cttgtatcaa ccagatgggt ctttggtaca gacggcagag     480
gtttgttaga agaatggttc ttgagcaaag aaccccgctg atggttgctt cgttatatgg     540
gagtttagat gttgtgaagt ttattctttc tttcccggaa gcggagttga atctgtcttg     600
tggtcctgat aaaagtactg ctcttcattg cgctgcttct ggtgcttctg tgaattcctt     660
ggatgttgtc aagttgcttt tgagtgtagg agcagatcct aatatccctg atgctcatgg     720
aaatcgtcct gttgatgttc ttgttgtgtc tccacacgct cctggtttga gaaccatcct     780
tgaagagatc ttgaagaaag acgagattat atctgaagat ctgcatgcct cgtcatctag     840
cttgggatca agtttccggt ctctctcatc atcccctgat aatggttcct cgttactctc     900
cttagattca gtatcctctc cgactaagcc acacggtact gatgtaactt tcgcatcaga     960
gaagaaagag tacccaattg atccatcatt gcctgatatc aaaagcggga tttattcaac    1020
cgatgagttt cgtatgttct cgttcaagat ccgcccatgt tctcgagcat attcccatga    1080
ctggactgaa tgtccatttg cacacccagg tgagaatgca aggagaagag acccgaggaa    1140
gtttcactat acgtgtgttc catgcccgga ttttaagaaa ggatcctgta agcaaggtga    1200
tatgtgtgaa tatgctcatg ggtttttga atgctggcta caccctgctc agtacagaac     1260
acgattgtgc aaggacggaa tgggttgcaa ccgaagggtt tgcttctttg ctcacgcaaa    1320
tgaggagttg cgtcccttgt acccttccac aggatctgga ttgccatctc ctcgggcttc    1380
gtctgctgtt tccgcctcta ctatggacat ggcgtcagtt ttgaacatgt taccaggctc    1440
accatctgct gctcaacatt cgttcacccc accaatatct ccttctggaa atggtagtat    1500
```

gccccattca tcgatgggtt ggcctcagca gaacataccg gcgttgaatc ttcctggaag 1560
caatatccag ttgagtcgtc tgagatcttc tcttaacgct agagatattc cttctgagca 1620
gcttagcatg ctgcatgagt ttgaaatgca acgtcagctt gctggcgata tgcacagtcc 1680
acgctttatg aatcattccg ctcgtcctaa gacactgaac ccttcaaatc tggaggaact 1740
cttctcagct gaggttgcat ctcctcgttt ctctgatcaa cttgctgttt catctgttct 1800
atcgccttcc cacaagtccg cgcttcttaa tcagctgcag aataataagc agagcatgct 1860
ttctcctatc aagacaaatc taatgtcttc tccaaagaat gtggagcaac attctcttct 1920
gcagcaagcc tcgtcacccc gaggcggaga gcctatttcc ccaatgaatg ctcgaatgaa 1980
acagcagcta cattcacgca gcctaagctc ccgtgatttt ggatctagtc tgccccgtga 2040
tttaatgccg actgattctg gttcgccatt aagtccatgg tcaagttggg accagaccca 2100
tggaagcaag gtggattggt cagtccaatc agatgagtta ggtcggttga gaaaatctca 2160
ttccttggct aataacccaa acagggaagc agatgtttca tgggctcagc agatgttaaa 2220
agactcttca tcacctagga acggaaaccg tgttgtgaac atgaatggtg caaggccatt 2280
gactcaaggt ggttcgagtg tgaatcctca caacagtgac actcgtgaga gcgacattct 2340
tgatgcgtgg cttgaacagc tgcacctaga tcgctgagcc tcagctgcga gagagaggtt 2400
cacatttctg tgaagctgtg aaactgatga ttcgtttatt tattattcaa gaaagcaaac 2460
ggaaacaaaa gcaaactccg ggtaagcttt tttcgattct aataacccta aaaggctcag 2520
tttttttcagg cttctttctg aaatttcttt actttcttat ttttatcacc tcattaaatt 2580
aattattgta tcatctctgt tgtaacaatg gccaaagtgc gcctctatta cttcccggat 2640
ttctgattta catttttgt atcctctcag tttgtcaatt gtttctaata tctccttcat 2700
atttgtcaaa gaacactgta tgagaaataa taacatattg tttcagctaa taagattcat 2760
tcatttcct 2769

<210> 19
<211> 706
<212> PRT
<213> Arabidopsis thaliana

<400> 19
Met Cys Gly Leu Ala Lys Lys Leu Asp Ile Glu Asp Thr Leu Thr Ser
1 5 10 15
Leu Ser Asp Gln Glu Asn Glu Ser Leu Ala Lys Pro Met Asn Asp Ala
20 25 30
Ala Glu Trp Glu His Ser Phe Ser Ala Leu Leu Glu Phe Ala Ala Asp
35 40 45
Asn Asp Val Glu Gly Phe Arg Arg Gln Leu Ser Asp Val Ser Cys Ile
50 55 60
Asn Gln Met Gly Leu Trp Tyr Arg Arg Gln Arg Phe Val Arg Arg Met
65 70 75 80
Val Leu Glu Gln Arg Thr Pro Leu Met Val Ala Ser Leu Tyr Gly Ser
85 90 95
Leu Asp Val Val Lys Phe Ile Leu Ser Phe Pro Glu Ala Glu Leu Asn
100 105 110
Leu Ser Cys Gly Pro Asp Lys Ser Thr Ala Leu His Cys Ala Ala Ser
115 120 125
Gly Ala Ser Val Asn Ser Leu Asp Val Val Lys Leu Leu Leu Ser Val
130 135 140
Gly Ala Asp Pro Asn Ile Pro Asp Ala His Gly Asn Arg Pro Val Asp
145 150 155 160
Val Leu Val Val Ser Pro His Ala Pro Gly Leu Arg Thr Ile Leu Glu
165 170 175
Glu Ile Leu Lys Lys Asp Glu Ile Ile Ser Glu Asp Leu His Ala Ser
180 185 190
Ser Ser Ser Leu Gly Ser Ser Phe Arg Ser Leu Ser Ser Ser Pro Asp
195 200 205
Asn Gly Ser Ser Leu Leu Ser Leu Asp Ser Val Ser Ser Pro Thr Lys
210 215 220
Pro His Gly Thr Asp Val Thr Phe Ala Ser Glu Lys Lys Glu Tyr Pro
225 230 235 240

```
Ile Asp Pro Ser Leu Pro Asp Ile Lys Ser Gly Ile Tyr Ser Thr Asp
                245                 250                 255
Glu Phe Arg Met Phe Ser Phe Lys Ile Arg Pro Cys Ser Arg Ala Tyr
            260                 265                 270
Ser His Asp Trp Thr Glu Cys Pro Phe Ala His Pro Gly Glu Asn Ala
        275                 280                 285
Arg Arg Arg Asp Pro Arg Lys Phe His Tyr Thr Cys Val Pro Cys Pro
    290                 295                 300
Asp Phe Lys Lys Gly Ser Cys Lys Gln Gly Asp Met Cys Glu Tyr Ala
305                 310                 315                 320
His Gly Val Phe Glu Cys Trp Leu His Pro Ala Gln Tyr Arg Thr Arg
                325                 330                 335
Leu Cys Lys Asp Gly Met Gly Cys Asn Arg Arg Val Cys Phe Phe Ala
            340                 345                 350
His Ala Asn Glu Glu Leu Arg Pro Leu Tyr Pro Ser Thr Gly Ser Gly
        355                 360                 365
Leu Pro Ser Pro Arg Ala Ser Ser Ala Val Ser Ala Ser Thr Met Asp
    370                 375                 380
Met Ala Ser Val Leu Asn Met Leu Pro Gly Ser Pro Ser Ala Ala Gln
385                 390                 395                 400
His Ser Phe Thr Pro Pro Ile Ser Pro Ser Gly Asn Gly Ser Met Pro
                405                 410                 415
His Ser Ser Met Gly Trp Pro Gln Gln Asn Ile Pro Ala Leu Asn Leu
            420                 425                 430
Pro Gly Ser Asn Ile Gln Leu Ser Arg Leu Arg Ser Ser Leu Asn Ala
        435                 440                 445
Arg Asp Ile Pro Ser Glu Gln Leu Ser Met Leu His Glu Phe Glu Met
    450                 455                 460
Gln Arg Gln Leu Ala Gly Asp Met His Ser Pro Arg Phe Met Asn His
465                 470                 475                 480
Ser Ala Arg Pro Lys Thr Leu Asn Pro Ser Asn Leu Glu Glu Leu Phe
                485                 490                 495
Ser Ala Glu Val Ala Ser Pro Arg Phe Ser Asp Gln Leu Ala Val Ser
            500                 505                 510
Ser Val Leu Ser Pro Ser His Lys Ser Ala Leu Leu Asn Gln Leu Gln
        515                 520                 525
Asn Asn Lys Gln Ser Met Leu Ser Pro Ile Lys Thr Asn Leu Met Ser
    530                 535                 540
Ser Pro Lys Asn Val Glu Gln His Ser Leu Leu Gln Gln Ala Ser Ser
545                 550                 555                 560
Pro Arg Gly Gly Glu Pro Ile Ser Pro Met Asn Ala Arg Met Lys Gln
                565                 570                 575
Gln Leu His Ser Arg Ser Leu Ser Ser Arg Asp Phe Gly Ser Ser Leu
            580                 585                 590
Pro Arg Asp Leu Met Pro Thr Asp Ser Gly Ser Pro Leu Ser Pro Trp
        595                 600                 605
Ser Ser Trp Asp Gln Thr His Gly Ser Lys Val Asp Trp Ser Val Gln
    610                 615                 620
Ser Asp Glu Leu Gly Arg Leu Arg Lys Ser His Ser Leu Ala Asn Asn
625                 630                 635                 640
Pro Asn Arg Glu Ala Asp Val Ser Trp Ala Gln Gln Met Leu Lys Asp
                645                 650                 655
Ser Ser Ser Pro Arg Asn Gly Asn Arg Val Val Asn Met Asn Gly Ala
            660                 665                 670
Arg Pro Leu Thr Gln Gly Gly Ser Ser Val Asn Pro His Asn Ser Asp
        675                 680                 685
Thr Arg Glu Ser Asp Ile Leu Asp Ala Trp Leu Glu Gln Leu His Leu
    690                 695                 700
Asp Arg
```

705

<210> 20
<211> 2674
<212> DNA
<213> Oryza sativa

<400> 20
tgcgagtcct cctcctcttc tcgtcgccgt gctactctcg ctttctctct ctctctctct 60
cacttgttcc ccaaggcgag aagcagccgc cgccggcgag cgtcgcgggg gaggggaggg 120
aagggaggga ggagcggtgg atccgggctt gattggattg ggtcggattc gattttggat 180
caaccccgga gggcgggagc ggttgctaca gatgcgttga gctttggtta atctatccgg 240
cgagagataa tgggcgagct tgctgatctc gttgtcgtgc cgtcgcagcc gccgctcgcc 300
ggcggccggc gggacaggct ggcggcgctg ctggagctcg cggcggcgga tgatgttgat 360
gggctcaggg gggcgctcgc ggagggaggc gaggaggcgg cggagttggc tgatggggtc 420
gggctgtggt atggtcggag caaggcgtac gaggcgcgca cgccgctgat ggtggcggcg 480
acgtacggca gcgccggggt ggtctcgctg ctggtgggcc tcggcggttg cgtcgacgtc 540
aaccgtcgcc ctggagccga cggcgccacc gcgctccact gcgccgcctc cggtggctcg 600
cgcaacgccg tcgctgttgt caagctgctt ttggccgctg gcgccgatcc ggccaccccc 660
gattccgccg gccgcttccc cgccgacgtc atcctagctc ctccggcttc gccagatgcc 720
cttggcgatc tcgaggtgct cctcggccgc cgccgagcac tcgccgtggc gacctcggtg 780
gcttcaggtt cgtcatcccc tccgctctcg tcctcaccag atgagggcaa caggtcgccc 840
tcgtcgcgtt cgtcgtcgct gtctcccatc actgtggatc gtgggaagaa ggagtatccg 900
gtggatccaa ctctgccgga catcaagagc agcgtgtatg cttcggatga gttccgcatg 960
tttgcgttca aggtccggcc ctgctcccgt gcctactcac acgactggac tgagtgcccg 1020
tttgtgcacc ccggcgagaa cgcccgccgc cgtgatcccc gcaagcaccc atacactgct 1080
gtgccttgcc ccaactttcg ccggcctggt ggctgcccta gcggcgatag ctgtgagttc 1140
tcgcatggcg tgtttgagag ctggctacac ccatcacagt atcgcacaag gctctgcaag 1200
gagggagcag cttgcgcccg tcgcatttgc ttctttgccc atgatgagga tgagctccgc 1260
catgtgcctc acaacagtgg tgccggcctg ctgtctcccc gcgcttcttc atccattgat 1320
atgactgctg cagctgcgct cgggcttctt ccaggttctc ctaccagaca ctttgcaccg 1380
ccgcctgtgt caccatctgc tgggagcaat ggaggagctg ctgctgcgca ttggctccaa 1440
ggcagtaggc tgcgttcttc tttcaatgca agggatgctg ctgttgatga ccttggcatg 1500
ctcctcgaat gggaatcaca ataccttggg gcactctgcc tgccacccag cagccgcccc 1560
caaccacgcc tttcagctgg tctgagtatc aggccaacaa ttgctccatc caatcttgaa 1620
gacatgtatg cttcagacat ggcaatgtct ccgaggttcc ctaatgacca aggtcactca 1680
gtctactcac cagcccacaa atcagccctc ctcaacaagc ttcatcaaca gaagggcctc 1740
ttatcacctg ttaacaccaa cagaatgtac tccccaaggg ctcttgatcc gtcatctttg 1800
gcacattctc catttggtgg catgtctccc cggtcccccc gtaccatgga acctacatca 1860
cccctaagtg ctcgtgtagg agcccctgcc acacagcggc cttctgttgg ttcaccacgg 1920
aattccagtg cttggggcac cgtggggtcc ccgatgggta aggttgactg gggtgtcgat 1980
agcgaggagc tagtccgctt gagacgccct gcacaaccag ggtttggaga agatgagaca 2040
gatgtatcat gggtgcagtc actggtaagc aatgctgagc ttaatggcaa gaggggcgaa 2100
gtacaaggca tgcctggtac ttctgcattg atgaacaggc ctgacctgaa caatcagggt 2160
gacttgttgg accagacggt gatcggtgct tggcttgagc agatgcacct ggatcagaag 2220
tgatttccaa gggaagccat gaagtcccaa agtggatgaa gcctttattt tgccaaggtt 2280
atttaccaaa gaatagttgt tggtcctagt aaataataat ttattctttt taattcttga 2340
aatttttggt gggcaaagtc agagatggtg gtcaagttca acaaaacatt tggtcacaga 2400
ttggtagctg aaatcagttc cagagattgg taaacaacct cattacttgg ggtcctaact 2460
agtattcttt tgattagctc agatgagtct ttattttagt gggttaaaat tcatatgttc 2520
cccatggtta ttatgtccat gatctcttcc taacaaaaga gagattataa ttgtccattt 2580
ttcatttatc aatgaatgat tttgttaaaa caatgtaagt tacattctta attttttctc 2640
tgttcaatgg aattaccttc cttggttagt cctc 2674

<210> 21
<211> 657
<212> PRT
<213> Oryza sativa

<400> 21

```
Met Gly Glu Leu Ala Asp Leu Val Val Val Pro Ser Gln Pro Pro Leu
1               5                   10                  15
Ala Gly Gly Arg Arg Asp Arg Leu Ala Ala Leu Leu Glu Leu Ala Ala
            20                  25                  30
Ala Asp Asp Val Asp Gly Leu Arg Gly Ala Leu Ala Glu Gly Gly Glu
        35                  40                  45
Glu Ala Ala Glu Leu Ala Asp Gly Val Gly Leu Trp Tyr Gly Arg Ser
    50                  55                  60
Lys Ala Tyr Glu Ala Arg Thr Pro Leu Met Val Ala Ala Thr Tyr Gly
65                  70                  75                  80
Ser Ala Gly Val Val Ser Leu Leu Val Gly Leu Gly Gly Cys Val Asp
                85                  90                  95
Val Asn Arg Arg Pro Gly Ala Asp Gly Ala Thr Ala Leu His Cys Ala
            100                 105                 110
Ala Ser Gly Gly Ser Arg Asn Ala Val Ala Val Val Lys Leu Leu Leu
        115                 120                 125
Ala Ala Gly Ala Asp Pro Ala Thr Pro Asp Ser Ala Gly Arg Phe Pro
    130                 135                 140
Ala Asp Val Ile Leu Ala Pro Pro Ala Ser Pro Asp Ala Leu Gly Asp
145                 150                 155                 160
Leu Glu Val Leu Leu Gly Arg Arg Arg Ala Leu Ala Val Ala Thr Ser
                165                 170                 175
Val Ala Ser Gly Ser Ser Ser Pro Pro Leu Ser Ser Ser Pro Asp Glu
            180                 185                 190
Gly Asn Arg Ser Pro Ser Ser Arg Ser Ser Ser Leu Ser Pro Ile Thr
        195                 200                 205
Val Asp Arg Gly Lys Lys Glu Tyr Pro Val Asp Pro Thr Leu Pro Asp
    210                 215                 220
Ile Lys Ser Ser Val Tyr Ala Ser Asp Glu Phe Arg Met Phe Ala Phe
225                 230                 235                 240
Lys Val Arg Pro Cys Ser Arg Ala Tyr Ser His Asp Trp Thr Glu Cys
                245                 250                 255
Pro Phe Val His Pro Gly Glu Asn Ala Arg Arg Arg Asp Pro Arg Lys
            260                 265                 270
His Pro Tyr Thr Ala Val Pro Cys Pro Asn Phe Arg Arg Pro Gly Gly
        275                 280                 285
Cys Pro Ser Gly Asp Ser Cys Glu Phe Ser His Gly Val Phe Glu Ser
    290                 295                 300
Trp Leu His Pro Ser Gln Tyr Arg Thr Arg Leu Cys Lys Glu Gly Ala
305                 310                 315                 320
Ala Cys Ala Arg Arg Ile Cys Phe Phe Ala His Asp Glu Asp Glu Leu
                325                 330                 335
Arg His Val Pro His Asn Ser Gly Ala Gly Leu Leu Ser Pro Arg Ala
            340                 345                 350
Ser Ser Ser Ile Asp Met Thr Ala Ala Ala Ala Leu Gly Leu Leu Pro
        355                 360                 365
Gly Ser Pro Thr Arg His Phe Ala Pro Pro Pro Val Ser Pro Ser Ala
    370                 375                 380
Gly Ser Asn Gly Gly Ala Ala Ala Ala His Trp Leu Gln Gly Ser Arg
385                 390                 395                 400
Leu Arg Ser Ser Phe Asn Ala Arg Asp Ala Ala Val Asp Asp Leu Gly
                405                 410                 415
Met Leu Leu Glu Trp Glu Ser Gln Tyr Leu Gly Ala Leu Cys Leu Pro
            420                 425                 430
Pro Ser Ser Arg Pro Gln Pro Arg Leu Ser Ala Gly Leu Ser Ile Arg
        435                 440                 445
Pro Thr Ile Ala Pro Ser Asn Leu Glu Asp Met Tyr Ala Ser Asp Met
    450                 455                 460
```

```
Ala Met Ser Pro Arg Phe Pro Asn Asp Gln Gly His Ser Val Tyr Ser
465                 470                 475                 480
Pro Ala His Lys Ser Ala Leu Leu Asn Lys Leu His Gln Gln Lys Gly
                485                 490                 495
Leu Leu Ser Pro Val Asn Thr Asn Arg Met Tyr Ser Pro Arg Ala Leu
            500                 505                 510
Asp Pro Ser Ser Leu Ala His Ser Pro Phe Gly Gly Met Ser Pro Arg
        515                 520                 525
Ser Pro Arg Thr Met Glu Pro Thr Ser Pro Leu Ser Ala Arg Val Gly
    530                 535                 540
Ala Pro Ala Thr Gln Arg Pro Ser Val Gly Ser Pro Arg Asn Ser Ser
545                 550                 555                 560
Ala Trp Gly Thr Val Gly Ser Pro Met Gly Lys Val Asp Trp Gly Val
                565                 570                 575
Asp Ser Glu Glu Leu Val Arg Leu Arg Arg Pro Ala Gln Pro Gly Phe
            580                 585                 590
Gly Glu Asp Glu Thr Asp Val Ser Trp Val Gln Ser Leu Val Ser Asn
        595                 600                 605
Ala Glu Leu Asn Gly Lys Arg Gly Glu Val Gln Gly Met Pro Gly Thr
    610                 615                 620
Ser Ala Leu Met Asn Arg Pro Asp Leu Asn Asn Gln Gly Asp Leu Leu
625                 630                 635                 640
Asp Gln Thr Val Ile Gly Ala Trp Leu Glu Gln Met His Leu Asp Gln
                645                 650                 655
Lys
```

<210> 22
<211> 2223
<212> DNA
<213> Arabidopsis thaliana

<400> 22

```
ttcttcaaaa accccaacca cttcttctcc ccaaaaacct ccaaagtttc aatctttact     60
tctctctttt tctccaagtt atcttctttt ctaggaagag atatgtgcgg tgcaaagagc    120
aacctttgct catctaaaac cctaacagaa gtcgaattca tgaggcagaa atcagaagac    180
ggagcttccg ccacgtgtct cctcgaattc gccgcctgtg atgatctttc atcgtttaag    240
agagagatcg aagagaatcc atcggtggag attgatgagt cagggttttg gtattgcaga    300
cgggtcgggt ctaagaagat gggttttgaa gaaagaacac cacttatggt tgctgctatg    360
tatggaagca tggaagtgtt gaattacata attgccacag gaagatccga tgtgaacaga    420
gtttgcagtg acgagaaagt cactgctctt cactgtgcag tttctggctg ttctgtttct    480
atcgttgaga tcatcaagat cttgcttgat gcttctgctt cacctaattg tgttgacgct    540
aatgggaaca aaccggttga tttgttggct aaagattctc ggtttgttcc taaccagagt    600
agaaaggcgg ttgaggtttt actgaccggg attcatggtt cggttatgga agaagaggag    660
gaggaactga agagtgttgt gactaagtat ccagctgatg catcacttcc tgatattaac    720
gaaggtgttt atggaactga tgattttagg atgtttagct ttaaggttaa gccatgttct    780
agggcttatt cacatgattg gactgaatgt ccttttgttc atcctggtga gaatgcaagg    840
aggagagatc ctaggaagta tccttacact tgtgtgcctt gtcccgagtt tcgtaaaggg    900
tcttgtccta aaggagattc gtgtgagtac gcgcacggtg ttttcgagtc ttggcttcac    960
ccggcgcagt ataggacacg gctttgcaaa gatgagactg gttgtgctag gagagtttgt   1020
ttctttgctc atagacggga tgagttaaga ccggttaatg cttctactgg ttctgcaatg   1080
gtttcaccaa ggtcgtctaa tcagtctcct gagatgtctg ttatgtctcc tttgacgctg   1140
ggatcatcgc caatgaactc tcctatggct aatggtgttc ctttgtctcc aagaaatggt   1200
ggtttatggc agaacagagt taatagcctt acaccaccac cgttgcagct taatggtagc   1260
agattgaagt cgactttgag tgctagagat atggatatgg agatggaact taggtttcgc   1320
ggtttggata accggagact tggtgatctc aagccatcca acctcgaaga gactttcgga   1380
tcatatgact cagcttctgt gatgcaactt caatcaccaa gcaggcattc tcagatgaac   1440
cactatccgt cttcacctgt gaggcagcct cctcctcatg gattcgaatc ttcagcagcc   1500
atggcagctg cagtgatgaa tgcaagatcc tcagcgtttg cgaaacgcag cttgagtttc   1560
```

aaaccagctc cagtagcttc taatgtctcc gattggggat caccaaatgg gaagcttgag 1620
tggggaatgc aaagagatga gctgaacaag ttgaggagaa gtgcctcctt cggcattcat 1680
ggaaacaaca acaacagtgt gtcacgccct gctagagact acagtgacga gccagatgtg 1740
tcgtgggtga actcactggt gaaagagaat gcaccagaga gagtgaatga gagggttggg 1800
aatacggtga atggtgcagc gagtagagac aagtttaagc tgccgtcgtg ggcagagcaa 1860
atgtatatag accatgagca gcagattgtg gcataagaag cagaaagaaa gatgtgggat 1920
ttatattgct tttgtcttct gggcctctct acacagaatc taacaaatct ggcaataatt 1980
ctttgatttg tgtttgaccc atagtttggt tactagtata tgttttttta tgttcttttt 2040
ttctttgtca ttctcttgtc cttcgtgaca ctatgtaatg attaaaagca aataattgat 2100
gcatgagttc aaatgttctt tgaaggatcc atcttattag ctttgtaatt gttgtgatat 2160
cttaatctta ttggttacgt atttcaagtg ctttagaaaa aatgggccta agagattttg 2220
ggg 2223

<210> 23
<211> 597
<212> PRT
<213> Arabidopsis thaliana

<400> 23
Met Cys Gly Ala Lys Ser Asn Leu Cys Ser Ser Lys Thr Leu Thr Glu
1 5 10 15
Val Glu Phe Met Arg Gln Lys Ser Glu Asp Gly Ala Ser Ala Thr Cys
20 25 30
Leu Leu Glu Phe Ala Ala Cys Asp Asp Leu Ser Ser Phe Lys Arg Glu
35 40 45
Ile Glu Glu Asn Pro Ser Val Glu Ile Asp Glu Ser Gly Phe Trp Tyr
50 55 60
Cys Arg Arg Val Gly Ser Lys Lys Met Gly Phe Glu Glu Arg Thr Pro
65 70 75 80
Leu Met Val Ala Ala Met Tyr Gly Ser Met Glu Val Leu Asn Tyr Ile
85 90 95
Ile Ala Thr Gly Arg Ser Asp Val Asn Arg Val Cys Ser Asp Glu Lys
100 105 110
Val Thr Ala Leu His Cys Ala Val Ser Gly Cys Ser Val Ser Ile Val
115 120 125
Glu Ile Ile Lys Ile Leu Leu Asp Ala Ser Ala Ser Pro Asn Cys Val
130 135 140
Asp Ala Asn Gly Asn Lys Pro Val Asp Leu Leu Ala Lys Asp Ser Arg
145 150 155 160
Phe Val Pro Asn Gln Ser Arg Lys Ala Val Glu Val Leu Leu Thr Gly
165 170 175
Ile His Gly Ser Val Met Glu Glu Glu Glu Glu Glu Leu Lys Ser Val
180 185 190
Val Thr Lys Tyr Pro Ala Asp Ala Ser Leu Pro Asp Ile Asn Glu Gly
195 200 205
Val Tyr Gly Thr Asp Asp Phe Arg Met Phe Ser Phe Lys Val Lys Pro
210 215 220
Cys Ser Arg Ala Tyr Ser His Asp Trp Thr Glu Cys Pro Phe Val His
225 230 235 240
Pro Gly Glu Asn Ala Arg Arg Arg Asp Pro Arg Lys Tyr Pro Tyr Thr
245 250 255
Cys Val Pro Cys Pro Glu Phe Arg Lys Gly Ser Cys Pro Lys Gly Asp
260 265 270
Ser Cys Glu Tyr Ala His Gly Val Phe Glu Ser Trp Leu His Pro Ala
275 280 285
Gln Tyr Arg Thr Arg Leu Cys Lys Asp Glu Thr Gly Cys Ala Arg Arg
290 295 300
Val Cys Phe Phe Ala His Arg Arg Asp Glu Leu Arg Pro Val Asn Ala
305 310 315 320

```
Ser Thr Gly Ser Ala Met Val Ser Pro Arg Ser Ser Asn Gln Ser Pro
                325                 330                 335
Glu Met Ser Val Met Ser Pro Leu Thr Leu Gly Ser Ser Pro Met Asn
            340                 345                 350
Ser Pro Met Ala Asn Gly Val Pro Leu Ser Pro Arg Asn Gly Gly Leu
        355                 360                 365
Trp Gln Asn Arg Val Asn Ser Leu Thr Pro Pro Pro Leu Gln Leu Asn
    370                 375                 380
Gly Ser Arg Leu Lys Ser Thr Leu Ser Ala Arg Asp Met Asp Met Glu
385                 390                 395                 400
Met Glu Leu Arg Phe Arg Gly Leu Asp Asn Arg Arg Leu Gly Asp Leu
                405                 410                 415
Lys Pro Ser Asn Leu Glu Glu Thr Phe Gly Ser Tyr Asp Ser Ala Ser
            420                 425                 430
Val Met Gln Leu Gln Ser Pro Ser Arg His Ser Gln Met Asn His Tyr
        435                 440                 445
Pro Ser Ser Pro Val Arg Gln Pro Pro Pro His Gly Phe Glu Ser Ser
    450                 455                 460
Ala Ala Met Ala Ala Ala Val Met Asn Ala Arg Ser Ser Ala Phe Ala
465                 470                 475                 480
Lys Arg Ser Leu Ser Phe Lys Pro Ala Pro Val Ala Ser Asn Val Ser
                485                 490                 495
Asp Trp Gly Ser Pro Asn Gly Lys Leu Glu Trp Gly Met Gln Arg Asp
            500                 505                 510
Glu Leu Asn Lys Leu Arg Arg Ser Ala Ser Phe Gly Ile His Gly Asn
        515                 520                 525
Asn Asn Asn Ser Val Ser Arg Pro Ala Arg Asp Tyr Ser Asp Glu Pro
    530                 535                 540
Asp Val Ser Trp Val Asn Ser Leu Val Lys Glu Asn Ala Pro Glu Arg
545                 550                 555                 560
Val Asn Glu Arg Val Gly Asn Thr Val Asn Gly Ala Ala Ser Arg Asp
                565                 570                 575
Lys Phe Lys Leu Pro Ser Trp Ala Glu Gln Met Tyr Ile Asp His Glu
            580                 585                 590
Gln Gln Ile Val Ala
        595
```

<210> 24
<211> 1761
<212> DNA
<213> Arabidopsis thaliana

<400> 24

```
atggaaaaag atagtattat gtgcagtgga ccaaagagca atctctgctc ttcaagaacc     60
ttaacagaaa tcgaatcaag gcaaaaggaa gaagaaacaa tgcttctcct cgaattcgct    120
gcttgtgatg atcttgactc gttcaagaga gaggttgaag agaaagggct tgatttggat    180
gagtcagggt tatggtattg cagacgtgtc ggttctaaga agatgggtct tgaagaaaga    240
acacctttaa tggttgcagc tatgtatgga agcataaagg ttttgacttt catcgtttcc    300
actggaaaat ctgatgtgaa cagagcttgt ggtgaagaga gagttactcc gcttcactgt    360
gctgttgctg gctgttctgt gaatatgatt gaagtcatca atgtcttgct tgatgcttct    420
gctttggtta actctgttga tgctaatggg aatcaacctt tggatgtgtt tgttcgagtt    480
tcgaggtttg tggctagtcc gaggaggaaa gcggttgagt tgttgctgag aggaggaggt    540
gttggaggat tgatcgatga ggcggttgaa gaagagatca agattgtctc taagtatcca    600
gctgatgctt ctttaccgga tataaacgaa ggggtttatg gaagtgatga gtttaggatg    660
tatagcttta aggttaagcc atgttctagg gcttattctc atgattggac cgagtgtgct    720
tttgttcatc cgggagaaaa tgcgaggagg agagatccga ggaagtatcc ttacacttgt    780
gtcccctgtc ccgagttccg taaaggatca tgcccgaaag gagattcttg cgagtatgct    840
cacggggttt tcgagtcgtg gcttcacccc gcgcagtata aacccggct ttgtaaagat    900
gaaacgggtt gtgcaaggaa agtttgtttc tttgctcata aacgcgaaga gatgagacct    960
```

gttaatgctt caactggctc tgccgtggct cagtctccgt ttagcagctt ggagatgatg 1020
ccagggttgt ctcctcttgc ttattcttca ggagtttcga ctcctccggt ttctccaatg 1080
gctaatggtg ttccttcctc tccaagaaac ggcggatcat ggcagaacag agtcaatacc 1140
cttactccac cggctttgca gctcaatggt ggaagcagat tgaagtccac actgagcgct 1200
agagatatcg atatggagat ggagatggaa ttgagactcc gcggttttgg caacaatgtg 1260
gaagagacgt tcgggtctta tgtttcctct ccaagtagga attctcaaat gggtcaaaac 1320
atgaaccaac attatccatc ttccccggtg agacaaccgc catctcaaca cgggttcgaa 1380
tcttcagcag ctgcagcggt tgcagtgatg aaagcgagat caaccgcctt tgcgaaacgt 1440
agcttgagct tcaaaccagc tactcaagca gcaccacagt cgaatctctc ggattgggga 1500
tctccaaacg ggaagctgga atggggaatg aaaggagaag agctgaataa gatgagaaga 1560
agtgtttcct ttggaatcca tggaaacaac aacaataacg cagctagaga ctacagggac 1620
gagccagatg tgtcatgggt taactcttta gttaaagaca gtactgtggt gtctgagaga 1680
agctttggaa tgaatgagag ggttcggata atgtcgtggg ctgagcaaat gtacagagag 1740
aaggagcaga ctgtggtgta a 1761

<210> 25
<211> 586
<212> PRT
<213> Arabidopsis thaliana

<400> 25

```
Met Glu Lys Asp Ser Ile Met Cys Ser Gly Pro Lys Ser Asn Leu Cys
1               5                   10                  15
Ser Ser Arg Thr Leu Thr Glu Ile Glu Ser Arg Gln Lys Glu Glu Glu
            20                  25                  30
Thr Met Leu Leu Leu Glu Phe Ala Ala Cys Asp Asp Leu Asp Ser Phe
        35                  40                  45
Lys Arg Glu Val Glu Glu Lys Gly Leu Asp Leu Asp Glu Ser Gly Leu
    50                  55                  60
Trp Tyr Cys Arg Arg Val Gly Ser Lys Lys Met Gly Leu Glu Glu Arg
65                  70                  75                  80
Thr Pro Leu Met Val Ala Ala Met Tyr Gly Ser Ile Lys Val Leu Thr
                85                  90                  95
Phe Ile Val Ser Thr Gly Lys Ser Asp Val Asn Arg Ala Cys Gly Glu
            100                 105                 110
Glu Arg Val Thr Pro Leu His Cys Ala Val Ala Gly Cys Ser Val Asn
        115                 120                 125
Met Ile Glu Val Ile Asn Val Leu Leu Asp Ala Ser Ala Leu Val Asn
    130                 135                 140
Ser Val Asp Ala Asn Gly Asn Gln Pro Leu Asp Val Phe Val Arg Val
145                 150                 155                 160
Ser Arg Phe Val Ala Ser Pro Arg Arg Lys Ala Val Glu Leu Leu Leu
                165                 170                 175
Arg Gly Gly Gly Val Gly Gly Leu Ile Asp Glu Ala Val Glu Glu Glu
            180                 185                 190
Ile Lys Ile Val Ser Lys Tyr Pro Ala Asp Ala Ser Leu Pro Asp Ile
        195                 200                 205
Asn Glu Gly Val Tyr Gly Ser Asp Glu Phe Arg Met Tyr Ser Phe Lys
    210                 215                 220
Val Lys Pro Cys Ser Arg Ala Tyr Ser His Asp Trp Thr Glu Cys Ala
225                 230                 235                 240
Phe Val His Pro Gly Glu Asn Ala Arg Arg Arg Asp Pro Arg Lys Tyr
                245                 250                 255
Pro Tyr Thr Cys Val Pro Cys Pro Glu Phe Arg Lys Gly Ser Cys Pro
            260                 265                 270
Lys Gly Asp Ser Cys Glu Tyr Ala His Gly Val Phe Glu Ser Trp Leu
        275                 280                 285
His Pro Ala Gln Tyr Lys Thr Arg Leu Cys Lys Asp Glu Thr Gly Cys
    290                 295                 300
```

```
Ala Arg Lys Val Cys Phe Phe Ala His Lys Arg Glu Glu Met Arg Pro
305                 310                 315                 320
Val Asn Ala Ser Thr Gly Ser Ala Val Ala Gln Ser Pro Phe Ser Ser
                325                 330                 335
Leu Glu Met Met Pro Gly Leu Ser Pro Leu Ala Tyr Ser Ser Gly Val
            340                 345                 350
Ser Thr Pro Pro Val Ser Pro Met Ala Asn Gly Val Pro Ser Ser Pro
        355                 360                 365
Arg Asn Gly Gly Ser Trp Gln Asn Arg Val Asn Thr Leu Thr Pro Pro
    370                 375                 380
Ala Leu Gln Leu Asn Gly Gly Ser Arg Leu Lys Ser Thr Leu Ser Ala
385                 390                 395                 400
Arg Asp Ile Asp Met Glu Met Glu Met Glu Leu Arg Leu Arg Gly Phe
                405                 410                 415
Gly Asn Asn Val Glu Glu Thr Phe Gly Ser Tyr Val Ser Ser Pro Ser
            420                 425                 430
Arg Asn Ser Gln Met Gly Gln Asn Met Asn Gln His Tyr Pro Ser Ser
        435                 440                 445
Pro Val Arg Gln Pro Pro Ser Gln His Gly Phe Glu Ser Ser Ala Ala
    450                 455                 460
Ala Ala Val Ala Val Met Lys Ala Arg Ser Thr Ala Phe Ala Lys Arg
465                 470                 475                 480
Ser Leu Ser Phe Lys Pro Ala Thr Gln Ala Ala Pro Gln Ser Asn Leu
                485                 490                 495
Ser Asp Trp Gly Ser Pro Asn Gly Lys Leu Glu Trp Gly Met Lys Gly
            500                 505                 510
Glu Glu Leu Asn Lys Met Arg Arg Ser Val Ser Phe Gly Ile His Gly
        515                 520                 525
Asn Asn Asn Asn Asn Ala Ala Arg Asp Tyr Arg Asp Glu Pro Asp Val
    530                 535                 540
Ser Trp Val Asn Ser Leu Val Lys Asp Ser Thr Val Val Ser Glu Arg
545                 550                 555                 560
Ser Phe Gly Met Asn Glu Arg Val Arg Ile Met Ser Trp Ala Glu Gln
                565                 570                 575
Met Tyr Arg Glu Lys Glu Gln Thr Val Val
            580                 585


<210>  26
<211>  2709
<212>  DNA
<213>  Eucalyptus grandis

<400>  26
cttctgaaag cttttgact taagacgaga gagaaggaga gaaggtcccc ctcctcgtcc      60
tcgtcccccc gtggattttg aagaagaaaa gtcgcacctt ccttctcctt tcccactcct     120
ccctctgctc gaagcttttc tcttccgcag aattacataa aaacctcgac tttgcgcatc     180
attccgattc acctcacacc ttcactttcc cactcgaggt ctccccctc ttttcctagc     240
tctttccctt tccctccctc tctctcgaga atcgccgcat ttggaggagc tccaatctgc     300
tttgctttgc tttgctctct tcttgctcgg ttccccctca taaggagtcg attatgtgca     360
acggttcttc gaagggtaaa cttttcccct cgagtatggg catggagggc gaattccaca     420
acaaggatgg cgaagcaccc cgtaaatgct ctgccttgct tgaattggca gcctcggacg     480
atctctcgtc gttcaaaagt gaagtggaag agaagggctg cgacgttgat gaggccagct     540
tttggtatgg taggagaatc gggtcgaaga agatgggttt tgaagagagg actccattga     600
tgatctctgc tttgtttgga agcaccaagg tcttgaaata cataatcgag accgccagag     660
ctgatgtcaa caggtcttgt gggtccgaca aggtggccgc cctccattgc gcagccgcgg     720
gtgggtccag ttcttcactt gaaattgtga agctcttgat tgaggcctca gcggatatta     780
attctgtaga tggcaatgga aataggccca tcgacgtgct tgccccggca gggaagtctc     840
gctgcaattc cagaaataag tttgttagat cgttgctgaa aggtgaaaac tatgtcgtgg     900
aaggtgacca atcctttgac atagaaggag aggagaagct agtcgctctt ccaaaggagg     960
```

```
gaggcgagaa gaaagagtat cctgttgatg tctctctacc tgacataaac aatgggttct   1020
acagtaccga tgagttccgg atgtatgctt tcaaggtgaa gccttgctcg agggcttact   1080
cccacgactg gaccgagtgc ccgtttgtgc accctgggga gaacgcgagg aggagggacc   1140
cacgcaagta cccttacagc tgtgtccctt gtcctgagtt tcgcaagggt tcgtgcgtaa   1200
gggggatgc ttgtgagtat gctcatggag tctttgagtc gtggcttcac ccagcgcaat   1260
accgaacccg gctgtgcaag gatgaaactg gttgtactcg caaagtttgc ttctttgctc   1320
acaagtccga agaattgcgt cccgtgtatg cttccacagg ttctgctatg ccctcaccca   1380
agtccttttc agctaatgcc ctagacatga caaccctgag ccccttatcc cttaattcac   1440
catctctgcc tttgcctgct acttccacgc cccccatgtc acctttggct gcctcatctt   1500
cacccaaggg catgaacttg tggcataaca aaattaacct gaccccacca agcctgcagc   1560
ttcctggcag ccggctgaag acggctatga gtgcgcggga cttcgatttt gagttggaat   1620
ttcttgggct ggaaaagcaa gcttctcagc ggcagcaact gatagaagag atttctcgtc   1680
tctcatcgcc ctctcatatg tggaactcgg aatttggcag aaccgcagag ctgaagccca   1740
ctaaccttga tgatgcgttt ggatctcttg acacttctct tttgtctccg ttgcaggggt   1800
cgtcgatgaa aacatcgact cctacccagt tgcaatcccc cacagggctt aaaatttcga   1860
atttgaacca actccgtgcg agctacccgt ctagcagctt gtcgtcctct cctgtgagga   1920
agacctcttc ttttgggttc gactcatcca gtgcagttgc tgcagcagtc atgaactcac   1980
ggtctgctgc tatgacgaag cggagccaga gcttcattga ccgtggagca gtgggtcaac   2040
ggtctggact cattggacct gctaattctg ctcctaggat gtccaacctt tcggactggg   2100
gctcgcctga tgggaagttg gattggggtg ttcaagggga cgagctcaac aagcttagga   2160
agtccgcttc cttcggcttt agaaacaaca gtatggcgaa cccaaacaac gtggcgtctc   2220
ccagtgctga tgagccggac gtgtcgtggg ttggttcatt ggtgaaggat gtggctccgc   2280
ccgaagggta tccacagtat ctgtacatag aacaggagca gatggtggca taactaaagc   2340
gaagagcacc acacgaactc tctcctgatg gcttaagatg acttgtttga cattctttat   2400
attcttacaa acagcgcgtt cttaggagtt agctggagga aagaaggaaa cggtattgag   2460
tttgagattc aggctcttag ctggacagcg aaaatttggg gaaggaagag aatttggttt   2520
cttgcccaac ttagataatg atgcttttga aggcttaaaa gaaagatgaa ggcaaacatt   2580
cttttgttag tattgtatta ttgttttaat ttttcatccc ctctgtcggg gtgtggtggg   2640
tgtcgatgtt tctttcatca gtaaaatata taatgaggtt tactcatcta ttttctacta   2700
aaaaaaaaa                                                           2709
```

<210> 27
<211> 659
<212> PRT
<213> Eucalyptus grandis

<400> 27

```
Met Cys Asn Gly Ser Ser Lys Gly Lys Leu Phe Pro Ser Ser Met Gly
1               5                   10                  15
Met Glu Gly Glu Phe His Asn Lys Asp Gly Glu Ala Pro Arg Lys Cys
            20                  25                  30
Ser Ala Leu Leu Glu Leu Ala Ala Ser Asp Asp Leu Ser Ser Phe Lys
        35                  40                  45
Ser Glu Val Glu Glu Lys Gly Cys Asp Val Asp Glu Ala Ser Phe Trp
    50                  55                  60
Tyr Gly Arg Arg Ile Gly Ser Lys Lys Met Gly Phe Glu Glu Arg Thr
65                  70                  75                  80
Pro Leu Met Ile Ser Ala Leu Phe Gly Ser Thr Lys Val Leu Lys Tyr
                85                  90                  95
Ile Ile Glu Thr Ala Arg Ala Asp Val Asn Arg Ser Cys Gly Ser Asp
            100                 105                 110
Lys Val Ala Ala Leu His Cys Ala Ala Ala Gly Gly Ser Ser Ser Ser
        115                 120                 125
Leu Glu Ile Val Lys Leu Leu Ile Glu Ala Ser Ala Asp Ile Asn Ser
    130                 135                 140
Val Asp Gly Asn Gly Asn Arg Pro Ile Asp Val Leu Ala Pro Ala Gly
145                 150                 155                 160
Lys Ser Arg Cys Asn Ser Arg Asn Lys Phe Val Arg Ser Leu Leu Lys
                165                 170                 175
```

Gly Glu Asn Tyr Val Val Glu Gly Asp Gln Ser Phe Asp Ile Glu Gly
180 185 190
Glu Glu Lys Leu Val Ala Leu Pro Lys Glu Gly Gly Glu Lys Lys Glu
195 200 205
Tyr Pro Val Asp Val Ser Leu Pro Asp Ile Asn Asn Gly Phe Tyr Ser
210 215 220
Thr Asp Glu Phe Arg Met Tyr Ala Phe Lys Val Lys Pro Cys Ser Arg
225 230 235 240
Ala Tyr Ser His Asp Trp Thr Glu Cys Pro Phe Val His Pro Gly Glu
245 250 255
Asn Ala Arg Arg Arg Asp Pro Arg Lys Tyr Pro Tyr Ser Cys Val Pro
260 265 270
Cys Pro Glu Phe Arg Lys Gly Ser Cys Val Arg Gly Asp Ala Cys Glu
275 280 285
Tyr Ala His Gly Val Phe Glu Ser Trp Leu His Pro Ala Gln Tyr Arg
290 295 300
Thr Arg Leu Cys Lys Asp Glu Thr Gly Cys Thr Arg Lys Val Cys Phe
305 310 315 320
Phe Ala His Lys Ser Glu Glu Leu Arg Pro Val Tyr Ala Ser Thr Gly
325 330 335
Ser Ala Met Pro Ser Pro Lys Ser Phe Ser Ala Asn Ala Leu Asp Met
340 345 350
Thr Thr Leu Ser Pro Leu Ser Leu Asn Ser Pro Ser Leu Pro Leu Pro
355 360 365
Ala Thr Ser Thr Pro Pro Met Ser Pro Leu Ala Ala Ser Ser Ser Pro
370 375 380
Lys Gly Met Asn Leu Trp His Asn Lys Ile Asn Leu Thr Pro Pro Ser
385 390 395 400
Leu Gln Leu Pro Gly Ser Arg Leu Lys Thr Ala Met Ser Ala Arg Asp
405 410 415
Phe Asp Phe Glu Leu Glu Phe Leu Gly Leu Glu Lys Gln Ala Ser Gln
420 425 430
Arg Gln Gln Leu Ile Glu Glu Ile Ser Arg Leu Ser Ser Pro Ser His
435 440 445
Met Trp Asn Ser Glu Phe Gly Arg Thr Ala Glu Leu Lys Pro Thr Asn
450 455 460
Leu Asp Asp Ala Phe Gly Ser Leu Asp Thr Ser Leu Leu Ser Pro Leu
465 470 475 480
Gln Gly Ser Ser Met Lys Thr Ser Thr Pro Thr Gln Leu Gln Ser Pro
485 490 495
Thr Gly Leu Lys Ile Ser Asn Leu Asn Gln Leu Arg Ala Ser Tyr Pro
500 505 510
Ser Ser Ser Leu Ser Ser Ser Pro Val Arg Lys Thr Ser Ser Phe Gly
515 520 525
Phe Asp Ser Ser Ser Ala Val Ala Ala Ala Val Met Asn Ser Arg Ser
530 535 540
Ala Ala Met Thr Lys Arg Ser Gln Ser Phe Ile Asp Arg Gly Ala Val
545 550 555 560
Gly Gln Arg Ser Gly Leu Ile Gly Pro Ala Asn Ser Ala Pro Arg Met
565 570 575
Ser Asn Leu Ser Asp Trp Gly Ser Pro Asp Gly Lys Leu Asp Trp Gly
580 585 590
Val Gln Gly Asp Glu Leu Asn Lys Leu Arg Lys Ser Ala Ser Phe Gly
595 600 605
Phe Arg Asn Asn Ser Met Ala Asn Pro Asn Asn Val Ala Ser Pro Ser
610 615 620
Ala Asp Glu Pro Asp Val Ser Trp Val Gly Ser Leu Val Lys Asp Val
625 630 635 640
Ala Pro Pro Glu Gly Tyr Pro Gln Tyr Leu Tyr Ile Glu Gln Glu Gln

645 650 655
Met Val Ala

<210> 28
<211> 2518
<212> DNA
<213> Eucalyptus grandis

<400> 28
tctccttcga gtttctttct tcactagaat tcgctcccga gtctgttgtt gctcgtgtag 60
ttttgcttac tccgtccttc gtttagctcg ctgaccagcg cggagctagg agcggtcgct 120
aaaggattac tcgtacaaaa cgtaaactca gctctgccaa ttttcccatg gaggggaat 180
cttacttcga gaaagatgaa aaatattcta attgctcaat cttgctcgaa ttatctgctt 240
cggacgatct cccagctttt gaaaggaaag cgaaagagaa gggctgtaac attgatggtg 300
ctagcttctg gtacggtaga agaattggct caaggaagat gggtcttgaa gagaggactc 360
ctctcatggt ggcttccttg tttggaagct ctagggttgt gaagtacatt ctcgaatctg 420
gcaaagtcga tgtaaatagg gcttgtggtt cggacaaggt cactgccctt cactgtgctg 480
ttgccagtgg ctctgcttct gcggtggagg ttgtcaagct cttgcttcac gcatctgccg 540
atgctaattg cattgatggc aatggaaaga agccaattga tgtgatagcc cttccattaa 600
agtcacgcgg cgattcaagg aggaagctga tggagctgtt gctgaaaggc gataattctg 660
atggggaatt tgaatcccac gaggagaagc cgattgccgc accgcaagca tccaaagagg 720
gaagcgaaaa gaaagagtat caatttcctg ttgatatctc tctgcctgac ataaatgttg 780
ggatttacag tactgatgag ttcagaatgt atgctttcaa agtaaagcct tgctcgcggg 840
catactccca tgactggaca gagtgcccat ttgttcatcc tggcgagaat gcgaggaggc 900
gggaccctcg caagtacccc tacagctgcg tcccttgccc tgaatttcgg aagggatctt 960
gccaaaaggg tgactcctgt gagtacgcgc acggcgtatt tgagtcgtgg cttcatcctg 1020
cacagtatag aacaagactg tgcaaggatg agactggatg tgctcgcaaa gtttgtttct 1080
ttgctcacaa gcccgaagaa ttaaggcctg tctatgcttc gacgggatca gctatgcctt 1140
ccccaaaatc ctactcatca agtgggctgg acatgtccac attgagtcct ctctcaatca 1200
gttctccgtc agcatcgttg cctgttactt caacagcacc catgtctcct cttgcagcct 1260
cgtcatctcc gatgtctgtg aacatgtggc agagcaaggc taacaagctc tccccgccaa 1320
tgctgcagct ctcaggtagt aggctgaaga ctgctttgag tgctagggac ttggacctgg 1380
agatggaatt gcgtggtcta gagagtcaga tggccactca acagcatcag ttgatggaag 1440
agatatctcg tctctcctca ccatcatcct gctttagtag taggattggg gaagtgaaac 1500
ccactaacct cgatgacgtt tttgggtctc cggatcctgc tttgctgcct caattgcagg 1560
ggctgtcaag accttcaaca ccaagccagt tgcaatctcc aactgggctt cagatgcgcc 1620
agaatgcaac ccagtttcgt ggggcgtacc agagcaatgc aaatgcattg tcatctccag 1680
caatgaagca ggcaccttct tatgggtttg actcatctag tgcagttgca gcagcggtga 1740
tgaattcgag gtcagccgct tttgcgaagc ggagtcagag ttttatcgac aggggaatgg 1800
cgtgccctgg aattgccaat tcttccccta tgatgtcttc agctatgtcg agctggagct 1860
cacctcatgg gaaattggat tggggcgtcc aaggagatga gttgaatagg ctgaggaaag 1920
ctgcttcctt taagatgaga agcagcaccg gagcaggtgc taatactgtc tcggcagcag 1980
ccatggctga tgagccagat atttcttggg tcagttcatt ggttaaggac gtgccttctg 2040
cggaggacgc gatgttcgct gcagagaaag gacagcgcac ttatgggaaa gacatccgcg 2100
aaaggattac cccatgggtg gagcagctgt acagagaagt gccacggatg gcgatgtaag 2160
attgccactg caagtcggat gccttagtat gctgactaat tgatattctt tgcatttgtt 2220
ttgaggcatt tggtagccat tagatacgag aaaaggccaa gcagcaggtg gtgtcttggc 2280
aaggaatagg atgcacatag tctgttatcg agtagaatag acttgggaac aatggttata 2340
gccaaatgtt aaaagttatg atattctttt ccaattcttt ctcttcctca tagtaggttt 2400
ctcaccaagt cttttagtga gagcctgcgg gatgtactat atgtttccct tatgtaacgt 2460
ctcttcgttg aaagaaatgg ctttataata taaagcatca agtttttaa aaaaaaa 2518

<210> 29
<211> 663
<212> PRT
<213> Eucalyptus grandis

<400> 29

```
Met Glu Gly Glu Ser Tyr Phe Glu Lys Asp Glu Lys Tyr Ser Asn Cys
1               5                   10                  15
Ser Ile Leu Leu Glu Leu Ser Ala Ser Asp Asp Leu Pro Ala Phe Glu
            20                  25                  30
Arg Lys Ala Lys Glu Lys Gly Cys Asn Ile Asp Gly Ala Ser Phe Trp
        35                  40                  45
Tyr Gly Arg Arg Ile Gly Ser Arg Lys Met Gly Leu Glu Glu Arg Thr
    50                  55                  60
Pro Leu Met Val Ala Ser Leu Phe Gly Ser Ser Arg Val Val Lys Tyr
65                  70                  75                  80
Ile Leu Glu Ser Gly Lys Val Asp Val Asn Arg Ala Cys Gly Ser Asp
                85                  90                  95
Lys Val Thr Ala Leu His Cys Ala Val Ala Ser Gly Ser Ala Ser Ala
            100                 105                 110
Val Glu Val Val Lys Leu Leu Leu His Ala Ser Ala Asp Ala Asn Cys
        115                 120                 125
Ile Asp Gly Asn Gly Lys Lys Pro Ile Asp Val Ile Ala Leu Pro Leu
    130                 135                 140
Lys Ser Arg Gly Asp Ser Arg Arg Lys Leu Met Glu Leu Leu Leu Lys
145                 150                 155                 160
Gly Asp Asn Ser Asp Gly Glu Phe Glu Ser His Glu Glu Lys Pro Ile
                165                 170                 175
Ala Ala Pro Gln Ala Ser Lys Glu Gly Ser Glu Lys Lys Glu Tyr Gln
            180                 185                 190
Phe Pro Val Asp Ile Ser Leu Pro Asp Ile Asn Val Gly Ile Tyr Ser
        195                 200                 205
Thr Asp Glu Phe Arg Met Tyr Ala Phe Lys Val Lys Pro Cys Ser Arg
    210                 215                 220
Ala Tyr Ser His Asp Trp Thr Glu Cys Pro Phe Val His Pro Gly Glu
225                 230                 235                 240
Asn Ala Arg Arg Arg Asp Pro Arg Lys Tyr Pro Tyr Ser Cys Val Pro
                245                 250                 255
Cys Pro Glu Phe Arg Lys Gly Ser Cys Gln Lys Gly Asp Ser Cys Glu
            260                 265                 270
Tyr Ala His Gly Val Phe Glu Ser Trp Leu His Pro Ala Gln Tyr Arg
        275                 280                 285
Thr Arg Leu Cys Lys Asp Glu Thr Gly Cys Ala Arg Lys Val Cys Phe
    290                 295                 300
Phe Ala His Lys Pro Glu Glu Leu Arg Pro Val Tyr Ala Ser Thr Gly
305                 310                 315                 320
Ser Ala Met Pro Ser Pro Lys Ser Tyr Ser Ser Ser Gly Leu Asp Met
                325                 330                 335
Ser Thr Leu Ser Pro Leu Ser Ile Ser Ser Pro Ser Ala Ser Leu Pro
            340                 345                 350
Val Thr Ser Thr Ala Pro Met Ser Pro Leu Ala Ala Ser Ser Ser Pro
        355                 360                 365
Met Ser Val Asn Met Trp Gln Ser Lys Ala Asn Lys Leu Ser Pro Pro
    370                 375                 380
Met Leu Gln Leu Ser Gly Ser Arg Leu Lys Thr Ala Leu Ser Ala Arg
385                 390                 395                 400
Asp Leu Asp Leu Glu Met Glu Leu Arg Gly Leu Glu Ser Gln Met Ala
                405                 410                 415
Thr Gln Gln His Gln Leu Met Glu Glu Ile Ser Arg Leu Ser Ser Pro
            420                 425                 430
Ser Ser Cys Phe Ser Ser Arg Ile Gly Glu Val Lys Pro Thr Asn Leu
        435                 440                 445
Asp Asp Val Phe Gly Ser Pro Asp Pro Ala Leu Leu Pro Gln Leu Gln
    450                 455                 460
Gly Leu Ser Arg Pro Ser Thr Pro Ser Gln Leu Gln Ser Pro Thr Gly
```

```
465                 470                 475                 480
Leu Gln Met Arg Gln Asn Ala Thr Gln Phe Arg Gly Ala Tyr Gln Ser
                485                 490                 495
Asn Ala Asn Ala Leu Ser Ser Pro Ala Met Lys Gln Ala Pro Ser Tyr
            500                 505                 510
Gly Phe Asp Ser Ser Ser Ala Val Ala Ala Ala Val Met Asn Ser Arg
        515                 520                 525
Ser Ala Ala Phe Ala Lys Arg Ser Gln Ser Phe Ile Asp Arg Gly Met
    530                 535                 540
Ala Cys Pro Gly Ile Ala Asn Ser Ser Pro Met Met Ser Ser Ala Met
545                 550                 555                 560
Ser Ser Trp Ser Ser Pro His Gly Lys Leu Asp Trp Gly Val Gln Gly
                565                 570                 575
Asp Glu Leu Asn Arg Leu Arg Lys Ala Ala Ser Phe Lys Met Arg Ser
            580                 585                 590
Ser Thr Gly Ala Gly Ala Asn Thr Val Ser Ala Ala Ala Met Ala Asp
        595                 600                 605
Glu Pro Asp Ile Ser Trp Val Ser Ser Leu Val Lys Asp Val Pro Ser
    610                 615                 620
Ala Glu Asp Ala Met Phe Ala Ala Glu Lys Gly Gln Arg Thr Tyr Gly
625                 630                 635                 640
Lys Asp Ile Arg Glu Arg Ile Thr Pro Trp Val Glu Gln Leu Tyr Arg
                645                 650                 655
Glu Val Pro Arg Met Ala Met
            660
```

```
<210>  30
<211>  2001
<212>  DNA
<213>  Triticum aestivum

<220>
<221>  misc_feature
<222>  (481)..(530)
<223>  n is a, c, g, or t

<400>  30
cgaattccgg tcgacgattt ctcgatttcc ttctctataa cacaacgctc tcttctcttg      60
caaccaaagt acttgttcca gtgtctactc tactcaaaaa ggatttggga catcatgtgc     120
agtgattcga aaagtaaact ttcttcccca accctcgtcg tcatggagaa tagtaacatt     180
cagaagcaga atctggatgg tctctacaac tcggttttgc ttgaattgtc tgcatctgat     240
gattatgaag ctttcaaaag agaggtggag gaaaaaggct tagatgtgaa cgaggcaggc     300
ttttggtacg gtagaagaat tgggtcaaag aagatgggat ctgaaacgag gacccctctg     360
atgattgctt ctttgtttgg aagcgccaag gtgctcaatt atattcttct tcagaaagga     420
ggaggtgttg atgtgaacag ggtctgtggt tctgataggg ccactgctct ccattgtgct     480
nnnnnnnnnn nnnnnnnnnn nnnnnnnnnn nnnnnnnnnn nnnnnnnnnn tcccgggtcg     540
cgatttcgta ggagattcac agagagaaaa gaagcaatta gtgataataa gaaagaatac     600
cctgttgata tatcactgcc agacataaac aacggtgtat atggaacaga tgattttagg     660
atgtacaact tcaaggtgaa gccttgctca agggcttact cccatgactg gaccgagtgt     720
ccattcgttc acccagggga gaacgctagg aggagagacc cacggaaata cccttacagc     780
tgtgttcctt gccctgagtt ccgcaaaggg acctgccaga agggtgattc ctgtgagtat     840
gctcatggtg tttttgagtc ctggctgcat cctgcccaat accggacaag gctttgcaag     900
gatgagactg gctgcgctag aaaagtctgc ttctttgccc acaaacctga agagctacgc     960
cctgtgtatg cttccactgg gtcggctatg ccatcaccaa aatcatattc agctagtgga    1020
cttgacatga cagcgatgag tccattggct ctaagttcca catctttgcc taatgccccc    1080
ccgtttccag cctcacccta tcgtgcgccc tcgttcttct ctcagagtga agctgtgcag    1140
aacaaaataa accttactcc accatcgttg cagctccctg gtagccgact gaaggctgct    1200
ttgagtgcca gggatctgga gatggagatg gaactgctcg gtctagaaag ccctgctcgc    1260
caacaacagc agcagcagca acaattgatc gaagagattg ccaggatctc ttccccatct    1320
```

ttccggagca aggaattcaa taggattgtt gatttgaatc ctactaacct tgatgacctg 1380
ttagcatctg ctgacccttc tgtattttct caactacatg gactttctgt gcaaccttca 1440
acacccacac aaagtgggct tcagatgcgc caaaacatga accacctccg tgcgagttat 1500
ccatccaaca tcccttcctc tcctgtgagg aagccctcag cttttgggtt tgactcatca 1560
gctgctgtgg caactgcagt gatgaattct aggtctgctg ccttcgcaaa gcgaagccaa 1620
agtttcattg atcgtggagc tgcaacccac catcttgggc tgtcttcagc ttccaactct 1680
tcttgcaggg tatcctctac cctttcagat tggagttccc ctaccgggaa actggattgg 1740
ggtgtaaacg gagacaagct gaacaagctg aggaaatcta cttcctttgg attcagaaac 1800
agtggggtaa ctgcatcccc catagcacag cctgaatttg gtgctgagcc ggatgtctca 1860
tgggttcatt cattggttaa agatgttccc tccgagaggt ctgagatatt tggtgctgag 1920
aagcaacaat atgatctcag taaagagatg cttccaccat ggatggagca gctgtatata 1980
gagcaggagc agatggtagc a 2001

<210> 31
<211> 667
<212> PRT
<213> Triticum aestivum

<220>
<221> UNSURE
<222> (161)..(177)
<223> Xaa can be any naturally occurring amino acid

<400> 31
Arg Ile Pro Val Asp Asp Phe Ser Ile Ser Phe Ser Ile Thr Gln Arg
1 5 10 15
Ser Leu Leu Leu Gln Pro Lys Tyr Leu Phe Gln Cys Leu Leu Tyr Ser
20 25 30
Lys Arg Ile Trp Asp Ile Met Cys Ser Asp Ser Lys Ser Lys Leu Ser
35 40 45
Ser Pro Thr Leu Val Val Met Glu Asn Ser Asn Ile Gln Lys Gln Asn
50 55 60
Leu Asp Gly Leu Tyr Asn Ser Val Leu Leu Glu Leu Ser Ala Ser Asp
65 70 75 80
Asp Tyr Glu Ala Phe Lys Arg Glu Val Glu Glu Lys Gly Leu Asp Val
85 90 95
Asn Glu Ala Gly Phe Trp Tyr Gly Arg Arg Ile Gly Ser Lys Lys Met
100 105 110
Gly Ser Glu Thr Arg Thr Pro Leu Met Ile Ala Ser Leu Phe Gly Ser
115 120 125
Ala Lys Val Leu Asn Tyr Ile Leu Leu Gln Lys Gly Gly Gly Val Asp
130 135 140
Val Asn Arg Val Cys Gly Ser Asp Arg Ala Thr Ala Leu His Cys Ala
145 150 155 160
Xaa Xaa Xaa Xaa Xaa Xaa Xaa Xaa Xaa Xaa Xaa Xaa Xaa Xaa Xaa Xaa
165 170 175
Xaa Pro Gly Ser Arg Phe Arg Arg Arg Phe Thr Glu Arg Lys Glu Ala
180 185 190
Ile Ser Asp Asn Lys Lys Glu Tyr Pro Val Asp Ile Ser Leu Pro Asp
195 200 205
Ile Asn Asn Gly Val Tyr Gly Thr Asp Asp Phe Arg Met Tyr Asn Phe
210 215 220
Lys Val Lys Pro Cys Ser Arg Ala Tyr Ser His Asp Trp Thr Glu Cys
225 230 235 240
Pro Phe Val His Pro Gly Glu Asn Ala Arg Arg Arg Asp Pro Arg Lys
245 250 255
Tyr Pro Tyr Ser Cys Val Pro Cys Pro Glu Phe Arg Lys Gly Thr Cys
260 265 270
Gln Lys Gly Asp Ser Cys Glu Tyr Ala His Gly Val Phe Glu Ser Trp

```
        275                 280                 285
Leu His Pro Ala Gln Tyr Arg Thr Arg Leu Cys Lys Asp Glu Thr Gly
    290                 295                 300
Cys Ala Arg Lys Val Cys Phe Phe Ala His Lys Pro Glu Glu Leu Arg
305                 310                 315                 320
Pro Val Tyr Ala Ser Thr Gly Ser Ala Met Pro Ser Pro Lys Ser Tyr
                325                 330                 335
Ser Ala Ser Gly Leu Asp Met Thr Ala Met Ser Pro Leu Ala Leu Ser
            340                 345                 350
Ser Thr Ser Leu Pro Asn Ala Pro Pro Phe Pro Ala Ser Pro Tyr Arg
        355                 360                 365
Ala Pro Ser Phe Phe Ser Gln Ser Glu Ala Val Gln Asn Lys Ile Asn
    370                 375                 380
Leu Thr Pro Pro Ser Leu Gln Leu Pro Gly Ser Arg Leu Lys Ala Ala
385                 390                 395                 400
Leu Ser Ala Arg Asp Leu Glu Met Glu Met Glu Leu Leu Gly Leu Glu
                405                 410                 415
Ser Pro Ala Arg Gln Gln Gln Gln Gln Gln Gln Gln Leu Ile Glu Glu
            420                 425                 430
Ile Ala Arg Ile Ser Ser Pro Ser Phe Arg Ser Lys Glu Phe Asn Arg
        435                 440                 445
Ile Val Asp Leu Asn Pro Thr Asn Leu Asp Asp Leu Leu Ala Ser Ala
    450                 455                 460
Asp Pro Ser Val Phe Ser Gln Leu His Gly Leu Ser Val Gln Pro Ser
465                 470                 475                 480
Thr Pro Thr Gln Ser Gly Leu Gln Met Arg Gln Asn Met Asn His Leu
                485                 490                 495
Arg Ala Ser Tyr Pro Ser Asn Ile Pro Ser Ser Pro Val Arg Lys Pro
            500                 505                 510
Ser Ala Phe Gly Phe Asp Ser Ser Ala Ala Val Ala Thr Ala Val Met
        515                 520                 525
Asn Ser Arg Ser Ala Ala Phe Ala Lys Arg Ser Gln Ser Phe Ile Asp
    530                 535                 540
Arg Gly Ala Ala Thr His His Leu Gly Leu Ser Ser Ala Ser Asn Ser
545                 550                 555                 560
Ser Cys Arg Val Ser Ser Thr Leu Ser Asp Trp Ser Ser Pro Thr Gly
                565                 570                 575
Lys Leu Asp Trp Gly Val Asn Gly Asp Lys Leu Asn Lys Leu Arg Lys
            580                 585                 590
Ser Thr Ser Phe Gly Phe Arg Asn Ser Gly Val Thr Ala Ser Pro Ile
        595                 600                 605
Ala Gln Pro Glu Phe Gly Ala Glu Pro Asp Val Ser Trp Val His Ser
    610                 615                 620
Leu Val Lys Asp Val Pro Ser Glu Arg Ser Glu Ile Phe Gly Ala Glu
625                 630                 635                 640
Lys Gln Gln Tyr Asp Leu Ser Lys Glu Met Leu Pro Pro Trp Met Glu
                645                 650                 655
Gln Leu Tyr Ile Glu Gln Glu Gln Met Val Ala
            660                 665
```

<210> 32
<211> 2683
<212> DNA
<213> Eucalyptus grandis

<400> 32

```
gcaaaggtcg atcacttcct ccctagaaag cgagtgtgga gttgaagctt gataaccaga      60
ggccgcctct cgtctcgtct cgcccgcctg cgcttgctct gctctccgcg tgccaaggga     120
gtgttcctag gtgctgaatc tttccatgtg tagcggttca aaagggaagg gagagtgaat     180
```

```
tcgagaagca gaggatgtcg gcccgtcagt tctcgatcct gctcgagtta tctgctgcgg    240
atgatctgac gaactttaag aaagcagttg aggaagacgg ctacgatatt gatgagtcga    300
gcttgtggta tggtaggagg atcgggtcga agaagattgg gcttgaagag agaactcccc    360
tcatgattgc cgcgatgttc ggcagtatgt ccgtgctgga ttatattatc aagtctggcc    420
gggccaatgt aaacaaggcg tgtggttcag atggtgctac cgcgcttcac tgtgctgcgg    480
ctggtggctc ggtacaatct cctgaggtgg tcaagctgtt gcttgattct tcagcgaatg    540
ctaactccat tgatgcgaat gggaaacgag cgggagactt gatttctgag gtctctggtt    600
cgcccttcaa ttcgagaagg aagactttgg atgtcatgtt gactggaggt gggactgttg    660
agtttgttga ggaaacttac aatctgcctg agaatctggg tagtcaaatt gaaggaaacg    720
aacaaagaga gagtccaacg gcccgcgctt ccaaggatgg ttctgaaaag aaagagtatc    780
ctgtcgacct ttctcttccg gacatcaaca atggaatata tagcacagat gagtttagga    840
tgtattcttt caaagtgaag ccttgctcga gagcttactc tcatgactgg actgagtgtc    900
catttgttca ccctggggag aatgcaagac ggcgtgaccc acggaaatat cactacagct    960
gtgtgccttg ccctgagttc cgcaaggggt catgcaggca aggggatggc tgcgagtatg   1020
ctcatggtat atttgagtgc tggcttcacc cagctcaata tcgcacccgt ctctgtaagg   1080
atgagattgg atgcaccaga aaagtctgtt tctttgccca caaacatgaa gagcttcgtc   1140
cattgtatgc atcaactggt tcggcgcttc cttctccaag atcattttcg cccgttgctg   1200
cttctctaga catgggatca ctgagccctc tctctctcgg ttcttcttca gtccggatac   1260
cgccaacttc aacaccacct atgactccat caggggcctc ttctcccctt ggtgggtcga   1320
tgtggaaaag ccaaattaat agcactccgc ctggcttgca gcttccaggt agcaggttga   1380
gaagcgcatt gagtgctaga gacatggatt tagatgttga cttgatcgat ctagaaaata   1440
attatcgttt gcagaagcag ttgctcgaac actttcctga tctgtcctct cctcgtggtt   1500
ggaacaactc ttcatccacc acgtcggctt tccctgagta ttcaggtgac atgactggag   1560
aaataagtag gttaggagta aaaccaaata atctcgagga tagtttcagg tcattggacc   1620
tgaccctctt gtctcagtta caagggctgt cacttgatgg tgcaatatcc cagctgcaat   1680
ctcctactgg aatgaagatt cggcagaaca tgacccagca gctctactca aactatactg   1740
acaagctttc ctcgtcacct agggcaatgc catcatttgg aaccgatcct tccagagctt   1800
cagcagcagc cactctgagt tccaggtcat tggcatttgc aaaaaggagc cacagcttca   1860
ttgagcggag tacagtgaac agtcagtctg gatattcagc aggtgctgct tctccaactg   1920
caaggatgtc ttcccagaat gactggggct cgcccgatgg caaactagac tggggcattc   1980
aaggggagga gctgaacaag ctgaggaaat ctgcatcatt cgggctcagg agcagcagca   2040
accgcttcca tgcgtctgca gattctgcga cagcaactgt aggggaccca gacatgccct   2100
ggattcagtc cttggcaaag gaagccccgt cacaaaaccc tggcaatttt ggagcagagc   2160
atcagcagca gcagcagcag cagcagcagc agcagtatca tcttaattct ggaggtactg   2220
agctgcttcc agcttgggtg gagcagttgt acgcggatca ggagcagatg gtcgcctgag   2280
atcaacattg gcttcttatc taaccactat tagtcatttc gttattgctt taattttttt   2340
tcttctgagt ctagtattaa tgtctaggat tcgaacgaac tggaaaatta aatctagagg   2400
gaagatggga agaaaagagc aggatggaag gtttctgctc ggtccgagat ttctcatagt   2460
ctattataga ctatcgtatt tctcgttctt ttccgtccca atgttcttga tttggttctc   2520
agcatgtttt ctggatgagg cttacaaact atgtaatctt gtcttgctaa aagaatcaga   2580
gctgcacctg caccaaaggt tgtgatacta ccgcttattg atgatgatga taataataat   2640
aattcggaca tttagtacca agtccgatgt ctcaaaaaaa aaa                     2683
```

```
<210>  33
<211>  694
<212>  PRT
<213>  Eucalyptus grandis

<400>  33
Met Ser Ala Arg Gln Phe Ser Ile Leu Leu Glu Leu Ser Ala Ala Asp
1               5                   10                  15
Asp Leu Thr Asn Phe Lys Lys Ala Val Glu Glu Asp Gly Tyr Asp Ile
            20                  25                  30
Asp Glu Ser Ser Leu Trp Tyr Gly Arg Arg Ile Gly Ser Lys Lys Ile
        35                  40                  45
Gly Leu Glu Glu Arg Thr Pro Leu Met Ile Ala Ala Met Phe Gly Ser
    50                  55                  60
Met Ser Val Leu Asp Tyr Ile Ile Lys Ser Gly Arg Ala Asn Val Asn
65                  70                  75                  80
```

```
Lys Ala Cys Gly Ser Asp Gly Ala Thr Ala Leu His Cys Ala Ala Ala
                85                  90                  95
Gly Gly Ser Val Gln Ser Pro Glu Val Val Lys Leu Leu Leu Asp Ser
            100                 105                 110
Ser Ala Asn Ala Asn Ser Ile Asp Ala Asn Gly Lys Arg Ala Gly Asp
        115                 120                 125
Leu Ile Ser Glu Val Ser Gly Ser Pro Phe Asn Ser Arg Arg Lys Thr
    130                 135                 140
Leu Asp Val Met Leu Thr Gly Gly Gly Thr Val Glu Phe Val Glu Glu
145                 150                 155                 160
Thr Tyr Asn Leu Pro Glu Asn Leu Gly Ser Gln Ile Glu Gly Asn Glu
                165                 170                 175
Gln Arg Glu Ser Pro Thr Ala Arg Ala Ser Lys Asp Gly Ser Glu Lys
            180                 185                 190
Lys Glu Tyr Pro Val Asp Leu Ser Leu Pro Asp Ile Asn Asn Gly Ile
        195                 200                 205
Tyr Ser Thr Asp Glu Phe Arg Met Tyr Ser Phe Lys Val Lys Pro Cys
    210                 215                 220
Ser Arg Ala Tyr Ser His Asp Trp Thr Glu Cys Pro Phe Val His Pro
225                 230                 235                 240
Gly Glu Asn Ala Arg Arg Arg Asp Pro Arg Lys Tyr His Tyr Ser Cys
                245                 250                 255
Val Pro Cys Pro Glu Phe Arg Lys Gly Ser Cys Arg Gln Gly Asp Gly
            260                 265                 270
Cys Glu Tyr Ala His Gly Ile Phe Glu Cys Trp Leu His Pro Ala Gln
        275                 280                 285
Tyr Arg Thr Arg Leu Cys Lys Asp Glu Ile Gly Cys Thr Arg Lys Val
    290                 295                 300
Cys Phe Phe Ala His Lys His Glu Glu Leu Arg Pro Leu Tyr Ala Ser
305                 310                 315                 320
Thr Gly Ser Ala Leu Pro Ser Pro Arg Ser Phe Ser Pro Val Ala Ala
                325                 330                 335
Ser Leu Asp Met Gly Ser Leu Ser Pro Leu Ser Leu Gly Ser Ser Ser
            340                 345                 350
Val Arg Ile Pro Pro Thr Ser Thr Pro Pro Met Thr Pro Ser Gly Ala
        355                 360                 365
Ser Ser Pro Leu Gly Gly Ser Met Trp Lys Ser Gln Ile Asn Ser Thr
    370                 375                 380
Pro Pro Gly Leu Gln Leu Pro Gly Ser Arg Leu Arg Ser Ala Leu Ser
385                 390                 395                 400
Ala Arg Asp Met Asp Leu Asp Val Asp Leu Ile Asp Leu Glu Asn Asn
                405                 410                 415
Tyr Arg Leu Gln Lys Gln Leu Leu Glu His Phe Pro Asp Leu Ser Ser
            420                 425                 430
Pro Arg Gly Trp Asn Asn Ser Ser Ser Thr Thr Ser Ala Phe Pro Glu
        435                 440                 445
Tyr Ser Gly Asp Met Thr Gly Glu Ile Ser Arg Leu Gly Val Lys Pro
    450                 455                 460
Asn Asn Leu Glu Asp Ser Phe Arg Ser Leu Asp Leu Thr Leu Leu Ser
465                 470                 475                 480
Gln Leu Gln Gly Leu Ser Leu Asp Gly Ala Ile Ser Gln Leu Gln Ser
                485                 490                 495
Pro Thr Gly Met Lys Ile Arg Gln Asn Met Thr Gln Gln Leu Tyr Ser
            500                 505                 510
Asn Tyr Thr Asp Lys Leu Ser Ser Ser Pro Arg Ala Met Pro Ser Phe
        515                 520                 525
Gly Thr Asp Pro Ser Arg Ala Ser Ala Ala Ala Thr Leu Ser Ser Arg
    530                 535                 540
Ser Leu Ala Phe Ala Lys Arg Ser His Ser Phe Ile Glu Arg Ser Thr
```

```
545                 550                 555                 560
Val Asn Ser Gln Ser Gly Tyr Ser Ala Gly Ala Ala Ser Pro Thr Ala
                565                 570                 575
Arg Met Ser Ser Gln Asn Asp Trp Gly Ser Pro Asp Gly Lys Leu Asp
            580                 585                 590
Trp Gly Ile Gln Gly Glu Glu Leu Asn Lys Leu Arg Lys Ser Ala Ser
        595                 600                 605
Phe Gly Leu Arg Ser Ser Ser Asn Arg Phe His Ala Ser Ala Asp Ser
    610                 615                 620
Ala Thr Ala Thr Val Gly Asp Pro Asp Met Pro Trp Ile Gln Ser Leu
625                 630                 635                 640
Ala Lys Glu Ala Pro Ser Gln Asn Pro Gly Asn Phe Gly Ala Glu His
                645                 650                 655
Gln Gln Gln Gln Gln Gln Gln Gln Gln Gln Gln Tyr His Leu Asn Ser
            660                 665                 670
Gly Gly Thr Glu Leu Leu Pro Ala Trp Val Glu Gln Leu Tyr Ala Asp
        675                 680                 685
Gln Glu Gln Met Val Ala
    690
```

<210> 34
<211> 2499
<212> DNA
<213> Arabidopsis thaliana

<400> 34

```
atttgacct taagaagaaa gtgacaagga gaggaagaag aagaaaaaaa acataatttg      60
aggaagaaga aaaaaaattc ggatttgttt tttcaataaa ttgactaatt gagtactcgt     120
ttaaaggaag tgaagagcgg ttttttggta gtggtggtcg agaaaagaga gagtttgtct     180
ctgtgactca gagtgaaatc aatagagcgg gaaaagattg ttgctttttt ttgccatggg     240
agttgatgag ctgtctcacc tcaaattctc tcttctgcta gaatcatcag cctgcaatga     300
tttgtccggt tttaagtctc tagttgaaga agaaggtctt gagagcattg atggctctgg     360
tttgtggtat gggaggagat taggatcaaa gaagatgggt tttgaggaga ggacgcctct     420
tatgattgct gccttgtttg gaagcaaaga ggttgttgat tacatcatta gtactggtct     480
tgttgacgtg aaccgctctt gtggctctga tggtgccacg gctcttcact gtgcggtctc     540
tggcttgtct gccaatagcc ttgagattgt tactcttctg ctgaagggct ctgcgaatcc     600
ggattcttgt gatgcttatg gtaacaagcc tggagatgtg attttccctt gtttgagtcc     660
ggtttttagc gcgaggatga aggttttgga gcgtttgttg aaaggaaatg atgatttgaa     720
tgaagttaat gggcaagaag aaagcgagcc agaggttgag gttgaggttg aggtttcgcc     780
tcctcggggg tctgagagga aggagtatcc ggttgatcca acgcttcctg atatcaagaa     840
cggtgtatat gggacggatg agttccggat gtatgctttc aagatcaagc cgtgctctag     900
agcatactct cacgactgga cggaatgtcc ctttgttcat ccgggtgaga acgcaaggag     960
gcgtgatccg aggaagtacc attatagttg tgtcccttgt cctgaattcc ggaaggggtc    1020
ttgttccaga ggtgatactt gcgagtatgc tcatggtatc tttgagtgct ggcttcaccc    1080
ggctcagtac cggactcgtc tctgcaagga cgagacgaat tgctcgagaa gagtttgttt    1140
ctttgcccac aaacccgagg agctgcgtcc tttgtaccct tcaactggat caggtgttcc    1200
gtccccgcgg tcttccttct catcttgcaa ttcctcgacc gctttcgaca tgggaccgat    1260
tagtccgctt cctatcggag caacaaccac acctcctttg agtcctaacg gtgtatcctc    1320
tccaataggt ggaggaaaaa cgtggatgaa ctggcctaac ataacccctc ctgcattgca    1380
gcttccaggg agcagattga aatctgcatt gaatgcaaga gaaatcgatt tctctgaaga    1440
gatgcaaagt cttacttctc caactacatg gaacaacacg ccaatgtcat ctccattctc    1500
cggaaagggc atgaacaggc ttgcaggagg agcaatgagc ccggtgaata gtctcagtga    1560
tatgtttggg acagaggata atacatcggg tttgcagatc cgacgcagcg tcattaaccc    1620
gcagctgcat tccaacagtc tttcttcatc acctgtggga gccaattctc tgttttcgat    1680
ggattcctcc gcagtcttgg cttcaagagc ggctgaattt gctaaacagc gaagccaaag    1740
cttcatagaa cgcaacaacg gactgaatca ccatcccgca atctcttcca tgactacaac    1800
ttgtttaaac gattggggct cattggatgg gaagcttgac tggagcgtcc aaggagacga    1860
gctacagaag ctcagaaaat ccacttcttt ccgtctcaga gccggtggca tggaatcaag    1920
actgcctaac gaagggactg ggctcgaaga gccagatgtc tcatgggtgg agccgctggt    1980
```

```
gaaagagcca caggagacaa gactagctcc ggtttggatg gagcaatcat acatggagac   2040
agaacagacc gtggcttgaa tcaaaagttt tgaactttca ttaaccgttc cacaagaagc   2100
aaagtcagaa agattccgag aggtcgatgc taatctattt cattttattt gtttaatgct   2160
ttgttatttt tctttagaat aaaaagaaaa aattcttagg ggacaaaaga gagttcgttt   2220
gtctctctct ctgtctccaa agaaaaacag aggtgaaaaa aggtttcaaa acctaagaaa   2280
ccttgaatta cctcacctca cttccttgat tctttactat tcacaatgag taatcgattt   2340
tttttttct tggtaacact ctcacgctga atatatatgt tttttagtaa taatataatt   2400
ggaatacaga aatgtattta cacttgtgaa gttagggaaa gtgttgtaat tgtttcttct   2460
aagagttgat ctaagatgtt tgagactata tcttcgctt                          2499

<210>  35
<211>  607
<212>  PRT
<213>  Arabidopsis thaliana

<400>  35
Met Gly Val Asp Glu Leu Ser His Leu Lys Phe Ser Leu Leu Leu Glu
1               5                   10                  15
Ser Ser Ala Cys Asn Asp Leu Ser Gly Phe Lys Ser Leu Val Glu Glu
            20                  25                  30
Glu Gly Leu Glu Ser Ile Asp Gly Ser Gly Leu Trp Tyr Gly Arg Arg
        35                  40                  45
Leu Gly Ser Lys Lys Met Gly Phe Glu Glu Arg Thr Pro Leu Met Ile
    50                  55                  60
Ala Ala Leu Phe Gly Ser Lys Glu Val Val Asp Tyr Ile Ile Ser Thr
65                  70                  75                  80
Gly Leu Val Asp Val Asn Arg Ser Cys Gly Ser Asp Gly Ala Thr Ala
                85                  90                  95
Leu His Cys Ala Val Ser Gly Leu Ser Ala Asn Ser Leu Glu Ile Val
            100                 105                 110
Thr Leu Leu Leu Lys Gly Ser Ala Asn Pro Asp Ser Cys Asp Ala Tyr
        115                 120                 125
Gly Asn Lys Pro Gly Asp Val Ile Phe Pro Cys Leu Ser Pro Val Phe
    130                 135                 140
Ser Ala Arg Met Lys Val Leu Glu Arg Leu Leu Lys Gly Asn Asp Asp
145                 150                 155                 160
Leu Asn Glu Val Asn Gly Gln Glu Glu Ser Glu Pro Glu Val Glu Val
                165                 170                 175
Glu Val Glu Val Ser Pro Pro Arg Gly Ser Glu Arg Lys Glu Tyr Pro
            180                 185                 190
Val Asp Pro Thr Leu Pro Asp Ile Lys Asn Gly Val Tyr Gly Thr Asp
        195                 200                 205
Glu Phe Arg Met Tyr Ala Phe Lys Ile Lys Pro Cys Ser Arg Ala Tyr
    210                 215                 220
Ser His Asp Trp Thr Glu Cys Pro Phe Val His Pro Gly Glu Asn Ala
225                 230                 235                 240
Arg Arg Arg Asp Pro Arg Lys Tyr His Tyr Ser Cys Val Pro Cys Pro
                245                 250                 255
Glu Phe Arg Lys Gly Ser Cys Ser Arg Gly Asp Thr Cys Glu Tyr Ala
            260                 265                 270
His Gly Ile Phe Glu Cys Trp Leu His Pro Ala Gln Tyr Arg Thr Arg
        275                 280                 285
Leu Cys Lys Asp Glu Thr Asn Cys Ser Arg Arg Val Cys Phe Phe Ala
    290                 295                 300
His Lys Pro Glu Glu Leu Arg Pro Leu Tyr Pro Ser Thr Gly Ser Gly
305                 310                 315                 320
Val Pro Ser Pro Arg Ser Ser Phe Ser Ser Cys Asn Ser Ser Thr Ala
                325                 330                 335
Phe Asp Met Gly Pro Ile Ser Pro Leu Pro Ile Gly Ala Thr Thr Thr
```

```
                340                 345                 350
Pro Pro Leu Ser Pro Asn Gly Val Ser Ser Pro Ile Gly Gly Gly Lys
        355                 360                 365
Thr Trp Met Asn Trp Pro Asn Ile Thr Pro Pro Ala Leu Gln Leu Pro
    370                 375                 380
Gly Ser Arg Leu Lys Ser Ala Leu Asn Ala Arg Glu Ile Asp Phe Ser
385                 390                 395                 400
Glu Glu Met Gln Ser Leu Thr Ser Pro Thr Thr Trp Asn Asn Thr Pro
                405                 410                 415
Met Ser Ser Pro Phe Ser Gly Lys Gly Met Asn Arg Leu Ala Gly Gly
            420                 425                 430
Ala Met Ser Pro Val Asn Ser Leu Ser Asp Met Phe Gly Thr Glu Asp
        435                 440                 445
Asn Thr Ser Gly Leu Gln Ile Arg Arg Ser Val Ile Asn Pro Gln Leu
    450                 455                 460
His Ser Asn Ser Leu Ser Ser Ser Pro Val Gly Ala Asn Ser Leu Phe
465                 470                 475                 480
Ser Met Asp Ser Ser Ala Val Leu Ala Ser Arg Ala Ala Glu Phe Ala
                485                 490                 495
Lys Gln Arg Ser Gln Ser Phe Ile Glu Arg Asn Asn Gly Leu Asn His
            500                 505                 510
His Pro Ala Ile Ser Ser Met Thr Thr Thr Cys Leu Asn Asp Trp Gly
        515                 520                 525
Ser Leu Asp Gly Lys Leu Asp Trp Ser Val Gln Gly Asp Glu Leu Gln
    530                 535                 540
Lys Leu Arg Lys Ser Thr Ser Phe Arg Leu Arg Ala Gly Gly Met Glu
545                 550                 555                 560
Ser Arg Leu Pro Asn Glu Gly Thr Gly Leu Glu Glu Pro Asp Val Ser
                565                 570                 575
Trp Val Glu Pro Leu Val Lys Glu Pro Gln Glu Thr Arg Leu Ala Pro
            580                 585                 590
Val Trp Met Glu Gln Ser Tyr Met Glu Thr Glu Gln Thr Val Ala
        595                 600                 605
```

```
<210>  36
<211>  1806
<212>  DNA
<213>  Oryza sativa

<400>  36
atgtgctctg ggccgcgcaa gccgtccaca ccgccgctgc cgcagcagca gaaggaggcg      60
acggtgatgg cggcgtcctt gcttcttgag ctggcggcag cggacgacgt ggcggcggtg     120
aggagggtcg tggaggagga gaaggtgtct cttggcgtgg ctgggttgtg gtatgggcct     180
tcggcgagcg gcgtggcgag gctcgggatg gagcggagga cggcggcgat ggtggcggcg     240
ctgtacggga gcacgggggt gcttgggtat gtcgtggcgg cagcgccggc ggaggccgcg     300
cgcgcgtcgg agacggatgg ggccacgccg ctgcacatgg cggctgccgg tggcgcggcg     360
aacgcggtcg cggccacgcg cctgttgctc gccgcggggg cgtcggtcga cgcgctctcg     420
gcttcggggc tccgcgccgg tgacctcctc ccgcgcgcca ccgcggcgga gaaggccatc     480
cggctgctgc tcaagtcgcc ggccgtgtcg ccgtcgtcgt cgccgaagaa gtcggcctcg     540
ccgccgtcgc cgccgccgcc gcaggaggcg aagaaggagt acccgcctga cctgacgctg     600
cccgacctca agagcggact gttcagcacc gacgagttcc gcatgtacag cttcaaggtg     660
aagccgtgct cccgcgccta ctcccatgac tggaccgagt gccccttcgt ccaccccggc     720
gagaacgcgc gccgccgcga ccctcgccgc tactcctaca gctgcgtgcc ttgcccggag     780
ttccgcaagg gcggctcgtg ccgcaagggc gacgcgtgcg agtacgccca tggcgtgttc     840
gagtgctggc tccacccggc gcagtacagg acgcgcctct gcaaggacga ggtcggctgc     900
gcgcgccgca tctgcttctt cgcccacaag cccgacgagc tccgcgccgt caacccctcc     960
gccgtgtccg tcggcatgca gcccaccgta tcgtcgccgc gctcctcgcc gcccaacggg    1020
ctcgacatgg cggcggcggc ggcggcgatg atgagccccg cctggccgtc gtccccagcg    1080
agccgcctca agacggcgct cggcgcgcgg gagctcgact tcgacctcga gatgctcgcg    1140
```

```
ctggaccagt accagcagaa gctgttcgac aaggtgtccg gcgcgccgtc gccgagggcg    1200
agctggggcg ccgcggcgaa cggcctcgcc accgcgtcgc cggcgagggc cgtgccggac    1260
tacaccgacc tgctcggctc cgtcgacccg gccatgctgt cccagctcca cgcgctgtcc    1320
ctcaagcagg ccggcgacat gcccgcgtac agctccatgg cggacaccac gcagatgcac    1380
atgccgacct cgccgatggt gggcggcgcg aacaccgcgt tcgggctgga ccactccatg    1440
gcgaaggcga tcatgagctc ccgcgcctcg gcgttcgcca agcgcagcca gagcttcatc    1500
gaccgcggag gccgcgcccc ggcggcgcgt tcgctcatgt cgccggcgac gaccggcgcg    1560
ccgtccattc tctcggactg gggctcgccg gacggcaagc tggactgggg cgtccagggc    1620
gacgagctgc acaagctccg caagtcggcg tcgttcgcgt tccgcggcca atccgccatg    1680
ccggtggcga cgcacgccgc ggcggcggag ccggacgtgt catgggtgaa ctctcttgtc    1740
aaggacggcc acgccgccgg cgacatattc gcgcagtggc cggagcagga gcagatggtg    1800
gcatga                                                               1806
```

<210> 37
<211> 601
<212> PRT
<213> Oryza sativa

<400> 37

```
Met Cys Ser Gly Pro Arg Lys Pro Ser Thr Pro Pro Leu Pro Gln Gln
1               5                   10                  15
Gln Lys Glu Ala Thr Val Met Ala Ala Ser Leu Leu Leu Glu Leu Ala
            20                  25                  30
Ala Ala Asp Asp Val Ala Ala Val Arg Arg Val Val Glu Glu Glu Lys
        35                  40                  45
Val Ser Leu Gly Val Ala Gly Leu Trp Tyr Gly Pro Ser Ala Ser Gly
    50                  55                  60
Val Ala Arg Leu Gly Met Glu Arg Arg Thr Ala Ala Met Val Ala Ala
65                  70                  75                  80
Leu Tyr Gly Ser Thr Gly Val Leu Gly Tyr Val Val Ala Ala Ala Pro
                85                  90                  95
Ala Glu Ala Ala Arg Ala Ser Glu Thr Asp Gly Ala Thr Pro Leu His
            100                 105                 110
Met Ala Ala Ala Gly Gly Ala Ala Asn Ala Val Ala Ala Thr Arg Leu
        115                 120                 125
Leu Leu Ala Ala Gly Ala Ser Val Asp Ala Leu Ser Ala Ser Gly Leu
    130                 135                 140
Arg Ala Gly Asp Leu Leu Pro Arg Ala Thr Ala Ala Glu Lys Ala Ile
145                 150                 155                 160
Arg Leu Leu Leu Lys Ser Pro Ala Val Ser Pro Ser Ser Ser Pro Lys
                165                 170                 175
Lys Ser Ala Ser Pro Pro Ser Pro Pro Pro Pro Gln Glu Ala Lys Lys
            180                 185                 190
Glu Tyr Pro Pro Asp Leu Thr Leu Pro Asp Leu Lys Ser Gly Leu Phe
        195                 200                 205
Ser Thr Asp Glu Phe Arg Met Tyr Ser Phe Lys Val Lys Pro Cys Ser
    210                 215                 220
Arg Ala Tyr Ser His Asp Trp Thr Glu Cys Pro Phe Val His Pro Gly
225                 230                 235                 240
Glu Asn Ala Arg Arg Arg Asp Pro Arg Arg Tyr Ser Tyr Ser Cys Val
                245                 250                 255
Pro Cys Pro Glu Phe Arg Lys Gly Gly Ser Cys Arg Lys Gly Asp Ala
            260                 265                 270
Cys Glu Tyr Ala His Gly Val Phe Glu Cys Trp Leu His Pro Ala Gln
        275                 280                 285
Tyr Arg Thr Arg Leu Cys Lys Asp Glu Val Gly Cys Ala Arg Arg Ile
    290                 295                 300
Cys Phe Phe Ala His Lys Pro Asp Glu Leu Arg Ala Val Asn Pro Ser
305                 310                 315                 320
```

```
Ala Val Ser Val Gly Met Gln Pro Thr Val Ser Ser Pro Arg Ser Ser
                325                 330                 335
Pro Pro Asn Gly Leu Asp Met Ala Ala Ala Ala Ala Ala Met Met Ser
            340                 345                 350
Pro Ala Trp Pro Ser Ser Pro Ala Ser Arg Leu Lys Thr Ala Leu Gly
        355                 360                 365
Ala Arg Glu Leu Asp Phe Asp Leu Glu Met Leu Ala Leu Asp Gln Tyr
    370                 375                 380
Gln Gln Lys Leu Phe Asp Lys Val Ser Gly Ala Pro Ser Pro Arg Ala
385                 390                 395                 400
Ser Trp Gly Ala Ala Ala Asn Gly Leu Ala Thr Ala Ser Pro Ala Arg
                405                 410                 415
Ala Val Pro Asp Tyr Thr Asp Leu Leu Gly Ser Val Asp Pro Ala Met
            420                 425                 430
Leu Ser Gln Leu His Ala Leu Ser Leu Lys Gln Ala Gly Asp Met Pro
        435                 440                 445
Ala Tyr Ser Ser Met Ala Asp Thr Thr Gln Met His Met Pro Thr Ser
    450                 455                 460
Pro Met Val Gly Gly Ala Asn Thr Ala Phe Gly Leu Asp His Ser Met
465                 470                 475                 480
Ala Lys Ala Ile Met Ser Ser Arg Ala Ser Ala Phe Ala Lys Arg Ser
                485                 490                 495
Gln Ser Phe Ile Asp Arg Gly Gly Arg Ala Pro Ala Ala Arg Ser Leu
            500                 505                 510
Met Ser Pro Ala Thr Thr Gly Ala Pro Ser Ile Leu Ser Asp Trp Gly
        515                 520                 525
Ser Pro Asp Gly Lys Leu Asp Trp Gly Val Gln Gly Asp Glu Leu His
    530                 535                 540
Lys Leu Arg Lys Ser Ala Ser Phe Ala Phe Arg Gly Gln Ser Ala Met
545                 550                 555                 560
Pro Val Ala Thr His Ala Ala Ala Ala Glu Pro Asp Val Ser Trp Val
                565                 570                 575
Asn Ser Leu Val Lys Asp Gly His Ala Ala Gly Asp Ile Phe Ala Gln
            580                 585                 590
Trp Pro Glu Gln Glu Gln Met Val Ala
        595                 600
```

<210> 38
<211> 1692
<212> DNA
<213> Hordeum vulgare

<400> 38

```
cggcacgagg cacatccatc atctaacctc acctctcctc tcctcccctc tcctcctacc     60
aaacccaaaa ccaagcagag caagagcaag agcaagagca agagcaagca agcatgtgcc    120
ctggcctgcg caacctcgcc gccgccatgc caccctccgc ccacgaccac ccctcctcct    180
acctgctcga gctcgccgcc gacgacgacc tccccgcctt ccgccgcgcc gtccaggagg    240
acaacctctc cctcgacgcc gcatccccga ggtacgagcc atcccccaaa tcagaccaac    300
aacaacaaca cgccccagct cgcgctccac ctgcgcaccc ccgccatggt cgccgcgctc    360
tacggcagca ccaccgtcct ctcctacgtc ctctccatcg ccccctccga ggccgcccgc    420
gcctccgcat ccgacggcgc caccccgctc ctcctcgccc accagggccg cgcgccatcc    480
gcgccccacg ccgcacgcct cctcctcacc gacggcgcat catcgtcctc cctactcgcg    540
ccccaagctc accctctcaa ccaccaaaac caaaaccaaa acagccccac caagaaagac    600
tcgccgccgg actccaggag gaccaccacc aagaaggact actcctccgc ctccgactcc    660
cagacggagg acatcaacgc gggcgtcttc gccaccgacg acttccggat gtacagcttc    720
aaggtgaacc cgtgctcccg cgcctacacg cacgactgga ccgagtgccc cttcgcccac    780
cccggcgaga acgcgcgccg ccgcgacccg cgccgcgtgc catactcgtg cgtcccatgc    840
ccggacttcc gccgcgaccc ggccgcatgc cgcaagggcg acgcctgcga gtacgcgcac    900
ggcgtcttcg agtcatggct ccaccccgcg cagtaccgca ccaggctctg caaggacgag    960
```

```
gtcggatgcc cgcgccgcat ctgcttcttc gcgcacggcg cccgacagct acgcgccgtc   1020
aacccctccg ccgcatccat ggactcgcca tccccaactt cctcttcgcc gccgcgaacc   1080
tccaggccgg ccgcgctcac cgcgtcgctc agctcgcggg acctcgactt ggacgccgac   1140
aaccaggccc agtacgcgcg caggatgatg atggccaggg ccaactcccc gccggactac   1200
tcgcccgacc tcgtcgccgc ctacgtacag gcgctctcct ccctgcaaca gcagcagcat   1260
cagcagaacc agcaacagca gcatcagcag cagaaccagc accagcagca acatcagcag   1320
aaccagcacc agcagcatca gcagcaacat cagcagagca tggggatggg ggggctgagc   1380
gcccgcgccg ccgccttcac caaccgcagc cagaccttcg tgcaccgctc tccgtccccg   1440
gctccggcgc ggtcgttcaa gtctccggcg ccgtcgtcca tgctcgcgga ctgggggtcg   1500
ccggacggga agctggactg gggcgtgcag gccgcggagc tgcgcaagtc cacgtctttc   1560
ggagtcagaa gcagcagcag gccgcatcat gagacgacga gggcggagga caacatgtac   1620
ccgtcgtgga tgaaggacgg cagcgatatg ctgctggcgg cgcggtggtc ggacctggag   1680
cagatggtcg cc                                                       1692
```

```
<210>  39
<211>  564
<212>  PRT
<213>  Hordeum vulgare

<400>  39
Arg His Glu Ala His Pro Ser Ser Asn Leu Thr Ser Pro Leu Leu Pro
1               5                   10                  15
Ser Pro Pro Thr Lys Pro Lys Thr Lys Gln Ser Lys Ser Lys Ser Lys
            20                  25                  30
Ser Lys Ser Lys Gln Ala Cys Ala Leu Ala Cys Ala Thr Ser Pro Pro
        35                  40                  45
Pro Cys His Pro Pro Pro Thr Thr Thr Pro Pro Pro Thr Cys Ser Ser
    50                  55                  60
Ser Pro Pro Thr Thr Thr Ser Pro Pro Ser Ala Ala Pro Ser Arg Arg
65                  70                  75                  80
Thr Thr Ser Pro Ser Thr Pro His Pro Arg Gly Thr Ser His Pro Pro
                85                  90                  95
Asn Gln Thr Asn Asn Asn Asn Thr Pro Gln Leu Ala Leu His Leu Arg
            100                 105                 110
Thr Pro Ala Met Val Ala Ala Leu Tyr Gly Ser Thr Thr Val Leu Ser
        115                 120                 125
Tyr Val Leu Ser Ile Ala Pro Ser Glu Ala Ala Arg Ala Ser Ala Ser
    130                 135                 140
Asp Gly Ala Thr Pro Leu Leu Leu Ala His Gln Gly Arg Ala Pro Ser
145                 150                 155                 160
Ala Pro His Ala Ala Arg Leu Leu Leu Thr Asp Gly Ala Ser Ser Ser
                165                 170                 175
Ser Leu Leu Ala Pro Gln Ala His Pro Leu Asn His Gln Asn Gln Asn
            180                 185                 190
Gln Asn Ser Pro Thr Lys Lys Asp Ser Pro Pro Asp Ser Arg Arg Thr
        195                 200                 205
Thr Thr Lys Lys Asp Tyr Ser Ser Ala Ser Asp Ser Gln Thr Glu Asp
    210                 215                 220
Ile Asn Ala Gly Val Phe Ala Thr Asp Asp Phe Arg Met Tyr Ser Phe
225                 230                 235                 240
Lys Val Asn Pro Cys Ser Arg Ala Tyr Thr His Asp Trp Thr Glu Cys
                245                 250                 255
Pro Phe Ala His Pro Gly Glu Asn Ala Arg Arg Arg Asp Pro Arg Arg
            260                 265                 270
Val Pro Tyr Ser Cys Val Pro Cys Pro Asp Phe Arg Arg Asp Pro Ala
        275                 280                 285
Ala Cys Arg Lys Gly Asp Ala Cys Glu Tyr Ala His Gly Val Phe Glu
    290                 295                 300
Ser Trp Leu His Pro Ala Gln Tyr Arg Thr Arg Leu Cys Lys Asp Glu
```

305 310 315 320
Val Gly Cys Pro Arg Arg Ile Cys Phe Phe Ala His Gly Ala Arg Gln
325 330 335
Leu Arg Ala Val Asn Pro Ser Ala Ala Ser Met Asp Ser Pro Ser Pro
340 345 350
Thr Ser Ser Ser Pro Pro Arg Thr Ser Arg Pro Ala Ala Leu Thr Ala
355 360 365
Ser Leu Ser Ser Arg Asp Leu Asp Leu Asp Ala Asp Asn Gln Ala Gln
370 375 380
Tyr Ala Arg Arg Met Met Met Ala Arg Ala Asn Ser Pro Pro Asp Tyr
385 390 395 400
Ser Pro Asp Leu Val Ala Ala Tyr Val Gln Ala Leu Ser Ser Leu Gln
405 410 415
Gln Gln Gln His Gln Gln Asn Gln Gln Gln Gln His Gln Gln Gln Asn
420 425 430
Gln His Gln Gln Gln His Gln Gln Asn Gln His Gln Gln His Gln Gln
435 440 445
Gln His Gln Gln Ser Met Gly Met Gly Gly Leu Ser Ala Arg Ala Ala
450 455 460
Ala Phe Thr Asn Arg Ser Gln Thr Phe Val His Arg Ser Pro Ser Pro
465 470 475 480
Ala Pro Ala Arg Ser Phe Lys Ser Pro Ala Pro Ser Ser Met Leu Ala
485 490 495
Asp Trp Gly Ser Pro Asp Gly Lys Leu Asp Trp Gly Val Gln Ala Ala
500 505 510
Glu Leu Arg Lys Ser Thr Ser Phe Gly Val Arg Ser Ser Ser Arg Pro
515 520 525
His His Glu Thr Thr Arg Ala Glu Asp Asn Met Tyr Pro Ser Trp Met
530 535 540
Lys Asp Gly Ser Asp Met Leu Leu Ala Ala Arg Trp Ser Asp Leu Glu
545 550 555 560
Gln Met Val Ala

<210> 40
<211> 3610
<212> DNA
<213> Pinus radiata

<400> 40
tgtttccagg cgggcactaa agcaagggag ggggtaggct ttactttctg ctctgcgcaa 60
agaacgttga aatcaatcgc cctggctggt ctggcgtgac tactagattc aatttcttca 120
tggccgtctt cacataccca ttctttaccg gttcagagct gtgatcttta tttttaacag 180
ccacaatcat ggtttgtgtt tccagtgtta tgatctgagt gaagttcgtt ctttttctcg 240
tgacccaggc ttgatactag gccggacctt tctgaggtgg aagagatcta tacatttgag 300
gcctattttg tgtagccatg tgtggaggcc cagaacattt gaagcctgcc agcccacacg 360
aaggagaaga taaagtcaaa atggccgaga atcagtctat caaagtgaag gaattgtctg 420
aatcttgttc aagtctacat gaactagctg ctaataatga ccttattggc tttaagaaag 480
caatggagga agaagggtca aagatagatg aggttaactt ttggtacggg aggcagaatg 540
gttctaatca gatggtcctg gagcaaagga ctccattgat ggttgctgca ctttatggca 600
gtgtagatgc gctgagttac atcttatcca tttatgtaac ttgtggagca gatgttaacc 660
aagcctgtgg gtcagataac tccactgcct tgcattgtgc ggctgtggga gggtctgcct 720
gtgcagttga aactgtaaaa ttgttacttc atgcaggcag tgatgtgaat cgcttggatg 780
cttatggcag aagaccagca gatgtgatta tggtttctcc taagctaacc gaaatcaagg 840
ccaagctaga agaaatgtta aacgcagctg gttcatgtca aacttctccg gcaaagttgc 900
ctaacatagt ttcagggcca cctgggtttg agtcaaaggg gatggagtcc atgtccccat 960
tgccattgtt gcctctttca ttgtctttag aagcatccaa taatagatca ggttgtgtga 1020
attctccaac atcttcgcca aagtccatgg aagcattaaa gggtttcggt gatgttaatg 1080
agaagaagga atatcctgtg gacccttctt ttccagacat aaagaatagc atctatacta 1140

```
cagatgaatt tcggatgttt tccttcaagg tgcggccatg ttcacgggca tattctcatg   1200
attggactga atgcccattt gtgcatcctg gtgaaaatgc cagaaggcgg gatccaagaa   1260
ggtatcatta tagctgtgtt ccttgcccag attttcggaa agggacttgt aggcgcagtg   1320
atgtttgtga atatgcacac ggtgtttttg agtgctggtt acatcctgct caatatagga   1380
cacggttgtg caaagatggg actaattgtt cacgtagagt ttgcttcttt gctcacacat   1440
ctgaggaact acgccctctc attgtctcta ctgggtctgc tgttccatcc ccaagggcat   1500
catcatctct ggacatgaca tctgtcatga gtcctcttgc cctggttctt ccctcttcag   1560
tttcaatgat gtcacccttc ctatcaaatc ctcagcaagg cagtgtgctt actccgccta   1620
tgtctccatc agcgtcctct gtaaatggat atggaggctg gccacagcct aatgtaccaa   1680
ccttacacct tcctggtagc aatgttcaaa ccagccgtct tagagcggaa cttaatgcca   1740
gagacatgcc tgttgaggat tctcctcgaa tttcagacta tgaagggcag caactcctga   1800
atgatttttc tccactgtcc acacaagcca ggctgaatgc tgctgctgct gttatatctg   1860
gtggcgggaa caccacaaca aggtctggaa aatacaagag tcacgggatc aatactgttg   1920
ctccaacgaa tcttgaagac ttgtttgcct ccgaggtaac atctcctaga gtagcagttc   1980
ttgaaccttc catcttttct cagatgagtc cccaaatgca agctcataag actgcccagg   2040
catatatgca gattcaaaac cagatgctgc ctcctataaa tacacaggca ttttcgcagg   2100
gaattacaca gatgcagcag gctgcaatag agcctcagag ccctggacat tctttgatgc   2160
aatcaccttt ccaatcttcc tcgtatgggt tgggatcccc tggtagaatg tcacctcgtt   2220
gtgtggatgt ggaacgtcat aatacatgtg ggtctccctt atcaccggct atggctgcaa   2280
cgataaattc aagaatggct atggctgctt ttgttcagag ggaaaaacgg agccatagtt   2340
cccgtgactt gggagctaat gtgaatccca gttcatggtc tgattggggc tcgcctacag   2400
gtaaagttga ctggggggtt caaggagaag agttgagcaa attaagaaag tcggcttcat   2460
ttggtccccg cagttatgaa gaaccggatt tgtcttgggt tcaaacactg gtaaaggaaa   2520
ctacaccaga gggtaaagat ggaggaaatg taagctgttc tggggaaact ccacacaagg   2580
ggcaaataga aaatgttgat cattcagttt tgggtgcctg gattgaacag atgcagcttg   2640
atcagattgt agcttgagat taggattatt tatttggagt ggtggtaggg ataggctcat   2700
ttaaaattca atttctcatt ttttactatt tcttttataa aaattcccca ttatagttta   2760
ggaaatagtc tggttttcta cctattatca gaattacacc tgcaggaaat tttggaggaa   2820
agcatgcaaa aagtagatag ggatgttatt cctatcagca ggttgacaag ctgaaaatca   2880
cttgggtggt agaccagaga atgacactat tttttgttga catggcaact gaagatgctg   2940
ttttctttac ttatcattaa caaccctata tatatttgtt ttgaaagaac tgagcggaga   3000
aatgttgtca gttggttact ctgcgcaagg ccttggaaga aatccaagat gtggcatctt   3060
ggtgcatttt taatttatca agtgtgaaat ccataacagg tttcagtgag tgacttctga   3120
ggttgtatat ggaaaaacct atgatgttgg ctgtctactg ctatttttct gtgcctaaac   3180
tgtcaactaa agtttgcagg tggcaatttt gtggcagcat atttgcacat tgaagcggat   3240
ggtctgcacc tgctatagaa gttttcgagt ctgtagaatt tgatggtgca agatgatttt   3300
ctagttgata tatttggaag gctttgccaa agtagtggca tgtacatttt gcaaaaattt   3360
aaaggatggc aatccattgt tttgccatgt agcttcactt tattgattag gtggaaagga   3420
attttgagac acttcaattt gtgcatactt ttgttctgaa ctgcaaaatc agtctcttgt   3480
gatgtcctca aggctattat gctcagggat ttgcctaaaa ccataagtgg ccttagataa   3540
ggtaccattg tattaccttt tattgtttgg atattttatt tatgaaagtg aatttatttt   3600
aaaaaaaaaa                                                           3610
```

<210> 41
<211> 779
<212> PRT
<213> Pinus radiata

<400> 41

```
Met Cys Gly Gly Pro Glu His Leu Lys Pro Ala Ser Pro His Glu Gly
1               5                   10                  15
Glu Asp Lys Val Lys Met Ala Glu Asn Gln Ser Ile Lys Val Lys Glu
            20                  25                  30
Leu Ser Glu Ser Cys Ser Ser Leu His Glu Leu Ala Ala Asn Asn Asp
        35                  40                  45
Leu Ile Gly Phe Lys Lys Ala Met Glu Glu Glu Gly Ser Lys Ile Asp
    50                  55                  60
Glu Val Asn Phe Trp Tyr Gly Arg Gln Asn Gly Ser Asn Gln Met Val
65                  70                  75                  80
```

Leu Glu Gln Arg Thr Pro Leu Met Val Ala Ala Leu Tyr Gly Ser Val
85 90 95
Asp Ala Leu Ser Tyr Ile Leu Ser Ile Tyr Val Thr Cys Gly Ala Asp
100 105 110
Val Asn Gln Ala Cys Gly Ser Asp Asn Ser Thr Ala Leu His Cys Ala
115 120 125
Ala Val Gly Gly Ser Ala Cys Ala Val Glu Thr Val Lys Leu Leu Leu
130 135 140
His Ala Gly Ser Asp Val Asn Arg Leu Asp Ala Tyr Gly Arg Arg Pro
145 150 155 160
Ala Asp Val Ile Met Val Ser Pro Lys Leu Thr Glu Ile Lys Ala Lys
165 170 175
Leu Glu Glu Met Leu Asn Ala Ala Gly Ser Cys Gln Thr Ser Pro Ala
180 185 190
Lys Leu Pro Asn Ile Val Ser Gly Pro Pro Gly Phe Glu Ser Lys Gly
195 200 205
Met Glu Ser Met Ser Pro Leu Pro Leu Leu Pro Leu Ser Leu Ser Leu
210 215 220
Glu Ala Ser Asn Asn Arg Ser Gly Cys Val Asn Ser Pro Thr Ser Ser
225 230 235 240
Pro Lys Ser Met Glu Ala Leu Lys Gly Phe Gly Asp Val Asn Glu Lys
245 250 255
Lys Glu Tyr Pro Val Asp Pro Ser Phe Pro Asp Ile Lys Asn Ser Ile
260 265 270
Tyr Thr Thr Asp Glu Phe Arg Met Phe Ser Phe Lys Val Arg Pro Cys
275 280 285
Ser Arg Ala Tyr Ser His Asp Trp Thr Glu Cys Pro Phe Val His Pro
290 295 300
Gly Glu Asn Ala Arg Arg Arg Asp Pro Arg Arg Tyr His Tyr Ser Cys
305 310 315 320
Val Pro Cys Pro Asp Phe Arg Lys Gly Thr Cys Arg Arg Ser Asp Val
325 330 335
Cys Glu Tyr Ala His Gly Val Phe Glu Cys Trp Leu His Pro Ala Gln
340 345 350
Tyr Arg Thr Arg Leu Cys Lys Asp Gly Thr Asn Cys Ser Arg Arg Val
355 360 365
Cys Phe Phe Ala His Thr Ser Glu Glu Leu Arg Pro Leu Ile Val Ser
370 375 380
Thr Gly Ser Ala Val Pro Ser Pro Arg Ala Ser Ser Ser Leu Asp Met
385 390 395 400
Thr Ser Val Met Ser Pro Leu Ala Pro Gly Ser Pro Ser Ser Val Ser
405 410 415
Met Met Ser Pro Phe Leu Ser Asn Pro Gln Gln Gly Ser Val Leu Thr
420 425 430
Pro Pro Met Ser Pro Ser Ala Ser Ser Val Asn Gly Tyr Gly Gly Trp
435 440 445
Pro Gln Pro Asn Val Pro Thr Leu His Leu Pro Gly Ser Asn Val Gln
450 455 460
Thr Ser Arg Leu Arg Ala Glu Leu Asn Ala Arg Asp Met Pro Val Glu
465 470 475 480
Asp Ser Pro Arg Ile Ser Asp Tyr Glu Gly Gln Gln Leu Leu Asn Asp
485 490 495
Phe Ser Pro Leu Ser Thr Gln Ala Arg Leu Asn Ala Ala Ala Ala Val
500 505 510
Ile Ser Gly Gly Gly Asn Thr Thr Thr Arg Ser Gly Lys Tyr Lys Ser
515 520 525
His Gly Ile Asn Thr Val Ala Pro Thr Asn Leu Glu Asp Leu Phe Ala
530 535 540
Ser Glu Val Thr Ser Pro Arg Val Ala Val Leu Glu Pro Ser Ile Phe

```
545                 550                 555                 560
Ser Gln Met Ser Pro Gln Met Gln Ala His Lys Thr Ala Gln Ala Tyr
                565                 570                 575
Met Gln Ile Gln Asn Gln Met Leu Pro Pro Ile Asn Thr Gln Ala Phe
            580                 585                 590
Ser Gln Gly Ile Thr Gln Met Gln Gln Ala Ala Ile Glu Pro Gln Ser
        595                 600                 605
Pro Gly His Ser Leu Met Gln Ser Pro Phe Gln Ser Ser Ser Tyr Gly
    610                 615                 620
Leu Gly Ser Pro Gly Arg Met Ser Pro Arg Cys Val Asp Val Glu Arg
625                 630                 635                 640
His Asn Thr Cys Gly Ser Pro Leu Ser Pro Ala Met Ala Ala Thr Ile
                645                 650                 655
Asn Ser Arg Met Ala Met Ala Ala Phe Val Gln Arg Glu Lys Arg Ser
            660                 665                 670
His Ser Ser Arg Asp Leu Gly Ala Asn Val Asn Pro Ser Ser Trp Ser
        675                 680                 685
Asp Trp Gly Ser Pro Thr Gly Lys Val Asp Trp Gly Val Gln Gly Glu
    690                 695                 700
Glu Leu Ser Lys Leu Arg Lys Ser Ala Ser Phe Gly Pro Arg Ser Tyr
705                 710                 715                 720
Glu Glu Pro Asp Leu Ser Trp Val Gln Thr Leu Val Lys Glu Thr Thr
                725                 730                 735
Pro Glu Gly Lys Asp Gly Gly Asn Val Ser Cys Ser Gly Glu Thr Pro
            740                 745                 750
His Lys Gly Gln Ile Glu Asn Val Asp His Ser Val Leu Gly Ala Trp
        755                 760                 765
Ile Glu Gln Met Gln Leu Asp Gln Ile Val Ala
    770                 775
```

<210> 42
<211> 3610
<212> DNA
<213> Pinus radiata

<400> 42

```
tgtttccagg cgggcactaa agcaagggag ggggtaggct ttactttctg ctctgcgcaa      60
agaacgttga aatcaatcgc cctggctggt ctggcgtgac tactagattc aatttcttca     120
tggccgtctt cacataccca ttctttaccg gttcagagct gtgatcttta tttttaacag     180
ccacaatcat ggtttgtgtt tccagtgtta tgatctgagt gaagttcgtt ctttttctcg     240
tgacccaggc ttgatactag gccggacctt tctgaggtgg aagagatcta tacatttgag     300
gcctattttg tgtagccatg tgtggaggcc cagaacattt gaagcctgcc agcccacacg     360
aaggagaaga taaagtcaaa atggccgaga atcagtctat caaagtgaag gaattgtctg     420
aatcttgttc aagtctacat gaactagctg ctaataatga ccttattggc tttaagaaag     480
caatggagga agaaggtca aagatagatg aggttaactt ttggtacggg aggcagaatg      540
gttctaatca gatggtcctg gagcaaagga ctccattgat ggttgctgca ctttatggca     600
gtgtagatgc gctgagttac atcttatcca tttatgtaac ttgtggagca gatgttaacc     660
aagcctgtgg gtcagataac tccactgcct tgcattgtgc ggctgtggga gggtctgcct     720
gtgcagttga aactgtaaaa ttgttacttc atgcaggcag tgatgtgaat cgcttggatg     780
cttatggcag aagaccagca gatgtgatta tggtttctcc taagctaacc gaaatcaagg     840
ccaagctaga agaaatgtta aacgcagctg gttcatgtca aacttctccg gcaaagttgc     900
ctaacatagt ttcagggcca cctgggtttg agtcaaaggg gatggagtcc atgtccccat     960
tgccattgtt gcctctttca ttgtctttag aagcatccaa taatagatca ggttgtgtga    1020
attctccaac atcttcgcca aagtccatgg aagcattaaa gggtttcggt gatgttaatg    1080
agaagaagga atatcctgtg gacccttctt ttccagacat aaagaatagc atctatacta    1140
cagatgaatt tcggatgttt tccttcaagg tgcggccatg ttcacgggca tattctcatg    1200
attggactga atgcccattt gtgcatcctg gtgaaaatgc cagaaggcgg gatccaagaa    1260
ggtatcatta tagctgtgtt ccttgcccag attttcggaa agggacttgt aggcgcagtg    1320
atgtttgtga atatgcacac ggtgtttttg agtgctggtt acatcctgct caatatagga    1380
```

```
cacggttgtg caaagatggg actaattgtt cacgtagagt ttgcttcttt gctcacacat   1440
ctgaggaact acgccctctc attgtctcta ctgggtctgc tgttccatcc ccaagggcat   1500
catcatctct ggacatgaca tctgtcatga gtcctcttgc ccctggttct ccctcttcag   1560
tttcaatgat gtcacccttc ctatcaaatc ctcagcaagg cagtgtgctt actccgccta   1620
tgtctccatc agcgtcctct gtaaatggat atggaggctg gccacagcct aatgtaccaa   1680
ccttacacct tcctggtagc aatgttcaaa ccagccgtct tagagcggaa cttaatgcca   1740
gagacatgcc tgttgaggat tctcctcgaa tttcagacta tgaagggcag caactcctga   1800
atgatttttc tccactgtcc acacaagcca ggctgaatgc tgctgctgct gttatatctg   1860
gtggcgggaa caccacaaca aggtctggaa aatacaagag tcacgggatc aatactgttg   1920
ctccaacgaa tcttgaagac ttgtttgcct ccgaggtaac atctcctaga gtagcagttc   1980
ttgaaccttc catcttttct cagatgagtc cccaaatgca agctcataag actgcccagg   2040
catatatgca gattcaaaac cagatgctgc ctcctataaa tacacaggca ttttcgcagg   2100
gaattacaca gatgcagcag gctgcaatag agcctcagag ccctggacat tctttgatgc   2160
aatcaccttt ccaatcttcc tcgtatgggt tgggatcccc tggtagaatg tcacctcgtt   2220
gtgtggatgt ggaacgtcat aatacatgtg ggtctccctt atcaccggct atggctgcaa   2280
cgataaattc aagaatggct atggctgctt ttgttcagag ggaaaaacgg agccatagtt   2340
cccgtgactt gggagctaat gtgaatccca gttcatggtc tgattggggc tcgcctacag   2400
gtaaagttga ctggggggtt caaggagaag agttgagcaa attaagaaag tcggcttcat   2460
ttggtccccg cagttatgaa gaaccggatt tgtcttgggt tcaaacactg gtaaaggaaa   2520
ctacaccaga gggtaaagat ggaggaaatg taagctgttc tggggaaact ccacacaagg   2580
ggcaaataga aaatgttgat cattcagttt tgggtgcctg gattgaacag atgcagcttg   2640
atcagattgt agcttgagat taggattatt tatttggagt ggtggtaggg ataggctcat   2700
ttaaaattca atttctcatt ttttactatt tcttttataa aaattcccca ttatagttta   2760
ggaaatagtc tggttttcta cctattatca gaattacacc tgcaggaaat tttggaggaa   2820
agcatgcaaa aagtagatag ggatgttatt cctatcagca ggttgacaag ctgaaaatca   2880
cttgggtggt agaccagaga atgacactat tttttgttga catggcaact gaagatgctg   2940
ttttctttac ttatcattaa caaccctata tatatttgtt ttgaaagaac tgagcggaga   3000
aatgttgtca gttggttact ctgcgcaagg ccttggaaga aatccaagat gtggcatctt   3060
ggtgcatttt taatttatca agtgtgaaat ccataacagg tttcagtgag tgacttctga   3120
ggttgtatat ggaaaaacct atgatgttgg ctgtctactg ctatttttct gtgcctaaac   3180
tgtcaactaa agtttgcagg tggcaatttt gtggcagcat atttgcacat tgaagcggat   3240
ggtctgcacc tgctatagaa gttttcgagt ctgtagaatt tgatggtgca agatgatttt   3300
ctagttgata tatttggaag gctttgccaa agtagtggca tgtacatttt gcaaaaattt   3360
aaaggatggc aatccattgt tttgccatgt agcttcactt tattgattag gtggaaagga   3420
attttgagac acttcaattt gtgcatactt ttgttctgaa ctgcaaaatc agtctcttgt   3480
gatgtcctca aggctattat gctcagggat ttgcctaaaa ccataagtgg ccttagataa   3540
ggtaccattg tattaccttt tattgtttgg atattttatt tatgaaagtg aatttatttt   3600
aaaaaaaaaa                                                          3610
```

<210> 43
<211> 749
<212> PRT
<213> Pinus radiata

<400> 43

```
Met Lys Glu Met Ala Glu Tyr Cys Ser Pro Ala Leu Leu Glu Leu Ala
1               5                   10                  15
Ala Asn Asn Asp Leu Ser Gly Phe Lys Gln Ala Val Glu Glu Gly Gly
            20                  25                  30
Ser Ser Val Asn Glu Arg Gly Leu Trp Tyr Gly Arg Gln Ile Gly Ser
        35                  40                  45
Gly Gln Lys Met Val Leu Glu Gln Arg Thr Pro Leu Met Val Ala Ala
    50                  55                  60
Leu Tyr Gly Ser Leu Asp Val Leu Ser Tyr Met Leu Ser Gly Gly Arg
65                  70                  75                  80
Val Asp Val Asn Gln Ser Cys Gly Ser Asp Met Ser Thr Ala Leu His
                85                  90                  95
Cys Ala Ala Ala Gly Gly Ser Ile Leu Ala Ile Glu Thr Val Gly Met
            100                 105                 110
```

```
Leu Ile Lys Ala Gly Ala Asp Val Asn Phe Met Asn Ala Gly Gly Arg
        115                 120                 125
Lys Pro Ala Asp Val Ile Met Val Ser Pro Lys Leu Ala His Phe Lys
    130                 135                 140
Asn Val Leu Glu Asp Leu Leu Ile Met Gly Ser Asn Ser Pro Met Lys
145                 150                 155                 160
Ile Pro Cys Arg Val Ser Gly Ser Gly Phe Tyr Leu Pro Glu Gly Gly
                165                 170                 175
Gly Cys Phe Phe Asp Glu His Gly Cys Val Val Ser Val Pro Thr Ser
            180                 185                 190
Ser Pro Leu Phe Ser Ser Pro Asp Ala Thr Ser Pro Ala Thr Val Asn
        195                 200                 205
Ser Pro Leu Ser Ser Pro Pro Thr Ser Leu Asp Thr Pro Lys Asn Leu
    210                 215                 220
Cys Asp Cys Gly Gln Lys Lys Glu Phe Ala Val Asp Ser Ser Leu Pro
225                 230                 235                 240
Asp Ile Lys Asn Ser Ile Tyr Ser Thr Asp Glu Phe Arg Met Tyr Ser
                245                 250                 255
Phe Lys Val Arg Pro Cys Ser Arg Ala Tyr Ser His Asp Trp Thr Glu
            260                 265                 270
Cys Pro Phe Val His Pro Gly Glu Asn Ala Arg Arg Arg Asp Pro Arg
        275                 280                 285
Lys Tyr His Tyr Ser Cys Val Pro Cys Pro Asp Phe Arg Lys Gly Ala
    290                 295                 300
Cys Arg Arg Gly Asp Val Cys Glu Tyr Ala His Gly Val Phe Glu Cys
305                 310                 315                 320
Trp Leu His Pro Ala Gln Tyr Arg Thr Arg Leu Cys Lys Asp Gly Thr
                325                 330                 335
Asn Cys Ser Arg Arg Val Cys Phe Phe Ala His Thr Pro Glu Glu Leu
            340                 345                 350
Arg Pro Leu Tyr Pro Pro Ala Cys Ser Ser Met Leu Ser Gln Arg Thr
        355                 360                 365
Thr Met Thr Ser Ser Asp Lys Met Ala Val Met His Pro Leu Ala Pro
    370                 375                 380
Gly Ser Ala Ser Ser Val Leu Met Met Ser Ser Ser Asn Ser Ser Gln
385                 390                 395                 400
Ser Ser Phe Pro Asn Ser Pro Val Ser Pro Leu Ser Ser Ala Asn Thr
                405                 410                 415
Ser Ser His Ser Ser Phe Gly Gly Gly Ser Trp Ala His Pro Asn Leu
            420                 425                 430
Pro Thr Leu His Leu Ser Asn Gly Ala Leu Gln Ala Ser Arg Leu Arg
        435                 440                 445
Thr Ala Val Asn Ala Arg Asp Met His Pro Asp Cys Ser Ile Glu Ser
    450                 455                 460
Gly Asp Tyr Glu Gly Gln Leu Leu Asn Glu Phe Ala Tyr Leu Ser Thr
465                 470                 475                 480
Gln Ala Arg Gly Asn Gly Pro Met Ala Thr Val Ser Ser Ser Gly Asn
                485                 490                 495
Thr Pro Cys Arg Pro Arg Lys Phe Arg Ala His Asn Val Ala Pro Thr
            500                 505                 510
Asn Leu Glu Asp Leu Phe Ala Ser Glu Val Phe Ser Pro Lys Met Thr
        515                 520                 525
Ala Ser Glu Ser Ala Phe Leu Ser Glu Ile Gln Ser His Lys Ser Ala
    530                 535                 540
Gln Leu Ser Pro Gln Leu Gln Ser Gln Met Leu Ser Ser Phe Asn Thr
545                 550                 555                 560
Gln Val Tyr Pro Gln Gly Ser Thr Gln Gly Gln Met His Met Gln His
                565                 570                 575
Gly Gly Val Asp Cys Gln Ser Pro Ser Val Phe Leu Ser Pro Pro Pro
```

```
                    580                 585                 590
Val Gln Leu Ala Ser Tyr Ser Leu Ser Ser Leu Gly Pro Leu Ser Ser
            595                 600                 605
Leu Thr Gly Glu Leu Glu Arg Gln Asn Ser Asn Gly Ser Pro Leu Ser
    610                 615                 620
Pro Ile Met Ser Thr Ala Ala Asp Ser Arg Ala Val Ala Phe Ser Gln
625                 630                 635                 640
Arg Asp Lys Gly Ser Ser Arg Ser Gly Asp Leu Gly Gly Ala Thr Thr
                645                 650                 655
Trp Ser Glu Trp Gly Ser Pro Thr Gly Lys Val Asn Trp Gly Ile Arg
            660                 665                 670
Gly Glu Glu Leu Gln Lys Phe Arg Lys Ser Ala Ser Phe Gly Ile Arg
        675                 680                 685
Ser Ser Asp Glu Pro Asp Leu Ser Trp Val Gln Lys Leu Phe Lys Glu
    690                 695                 700
Ala Pro Met Glu Ser Met Asp Arg Gly Thr Met Gly Arg Ser Met Asp
705                 710                 715                 720
Ile Ala Asn Ser Val Gln Met Glu Ala Thr Asp Leu Gly Gly Trp Ile
                725                 730                 735
Ser Gln Ile Asn Pro Asp Gln Val Ala Pro Leu Thr Leu
            740                 745
```

<210> 44
<211> 711
<212> PRT
<213> Glycine max

<400> 44

```
Lys Ser Ala Asn Asp Lys Glu Met Lys Ser Leu Thr Val Asn Thr Glu
1               5                   10                  15
Asp Ser Phe Ser Ser Leu Leu Glu Leu Ala Ser Asn Asn Asp Ile Glu
            20                  25                  30
Gly Phe Lys Val Leu Leu Glu Lys Asp Ser Ser Ser Ile Asn Glu Val
        35                  40                  45
Gly Leu Trp Tyr Gly Arg Gln Asn Gly Ser Lys Gln Phe Val Leu Glu
    50                  55                  60
His Arg Thr Pro Leu Met Val Ala Ala Thr Tyr Gly Ser Ile Asp Val
65                  70                  75                  80
Met Lys Ile Ile Leu Leu Cys Pro Glu Ala Asp Val Asn Phe Ala Cys
                85                  90                  95
Gly Ala Asn Lys Thr Thr Ala Leu His Cys Ala Ala Ser Gly Gly Ser
            100                 105                 110
Ala Asn Ala Val Asp Ala Val Lys Ile Leu Leu Ser Ala Gly Ala Asp
        115                 120                 125
Val Asn Gly Val Asp Ala Asn Gly Asn Arg Pro Ile Asp Val Ile Ala
    130                 135                 140
Val Pro Pro Lys Leu Gln Gly Ala Lys Ala Val Leu Glu Glu Leu Leu
145                 150                 155                 160
Ser Asp Ser Ala Ser Glu Gly Ser Ile Gly Glu Phe Ser Val Pro Val
                165                 170                 175
Ser Val Asn Thr Ser Ser Leu Gly Ser Pro Gly His Ser Ser Asn Gly
            180                 185                 190
Met Pro Tyr Thr Pro Ser Ser Ser Pro Pro Ser Pro Val Val Ala Lys
        195                 200                 205
Phe Thr Asp Ala Ala Val Cys Ser Leu Ser Glu Lys Lys Glu Tyr Pro
    210                 215                 220
Ile Asp Pro Ser Leu Pro Asp Ile Lys Asn Ser Ile Tyr Ala Thr Asp
225                 230                 235                 240
Glu Phe Arg Met Phe Ser Phe Lys Val Arg Pro Cys Ser Arg Ala Tyr
```

```
                    245                 250                 255
Ser His Asp Trp Thr Glu Cys Pro Phe Val His Pro Gly Glu Asn Ala
                260                 265                 270
Arg Arg Arg Asp Pro Arg Lys Phe His Tyr Ser Cys Val Pro Cys Pro
            275                 280                 285
Asp Phe Arg Lys Gly Ala Cys Arg Arg Gly Asp Met Cys Glu Tyr Ala
    290                 295                 300
His Gly Val Phe Glu Cys Trp Leu His Pro Ala Gln Tyr Arg Thr Arg
305                 310                 315                 320
Leu Cys Lys Asp Gly Thr Ser Cys Asn Arg Arg Val Cys Phe Phe Ala
                325                 330                 335
His Thr Ala Glu Glu Leu Arg Pro Leu Tyr Val Ser Thr Gly Ser Ala
            340                 345                 350
Val Pro Ser Pro Arg Ser Ser Ala Ser Ala Pro Asn Val Met Asp Met
        355                 360                 365
Ala Ala Ala Met Ser Leu Leu Pro Gly Ser Pro Ser Ser Val Ser Ser
    370                 375                 380
Met Ser Pro Ser His Phe Gly Gln Pro Met Ser Pro Ser Ala Asn Gly
385                 390                 395                 400
Met Ser Leu Ser Ser Ala Trp Ala Gln Pro Asn Val Ser Ala Leu His
                405                 410                 415
Leu Pro Gly Ser Asn Leu Gln Ser Ser Arg Leu Arg Ser Ser Leu Ser
            420                 425                 430
Ala Arg Asp Met Pro Pro Asp Asp Leu Asn Met Met Ser Asp Leu Asp
        435                 440                 445
Gly Gln Gln Gln His Pro Leu Asn Asp Leu Ser Cys Tyr Leu Gln Pro
    450                 455                 460
Arg Pro Gly Ala Gly Ser Val Ser Arg Ser Gly Arg Ser Lys Ile Leu
465                 470                 475                 480
Thr Pro Ser Asn Leu Glu Asp Leu Phe Ser Ala Glu Ile Ser Ser Ser
                485                 490                 495
Pro Arg Tyr Ser Asp Pro Ala Ala Gly Ser Val Phe Ser Pro Thr His
            500                 505                 510
Lys Ser Ala Val Leu Asn Gln Phe Gln Gln Leu Gln Ser Met Leu Ser
        515                 520                 525
Pro Ile Asn Thr Asn Leu Leu Ser Pro Lys Asn Val Glu His Pro Leu
    530                 535                 540
Leu Gln Ala Ser Phe Gly Val Ser Pro Ser Gly Arg Met Ser Pro Arg
545                 550                 555                 560
Ser Val Glu Pro Ile Ser Pro Met Ser Ser Arg Ile Ser Ala Phe Ala
                565                 570                 575
Gln Arg Glu Lys Gln Gln Gln Gln Gln Gln Gln Leu Arg Ser Leu Ser
            580                 585                 590
Ser Arg Asp Leu Gly Ala Asn Ser Pro Ala Ser Leu Val Gly Ser Pro
        595                 600                 605
Ala Asn Pro Trp Ser Lys Trp Gly Ser Pro Asn Gly Lys Ala Asp Trp
    610                 615                 620
Ser Val Asn Gly Asp Thr Leu Gly Arg Gln Met Arg Arg Ser Ser Ser
625                 630                 635                 640
Phe Glu Leu Lys Asn Asn Gly Glu Glu Pro Asp Leu Ser Trp Val Gln
                645                 650                 655
Ser Leu Val Lys Glu Ser Pro Pro Glu Met Ile Lys Glu Lys Phe Ala
            660                 665                 670
Ser Pro Met Pro Thr Ala Ser Ala Asp Gly Pro Asn Ser Asn Ser Gln
        675                 680                 685
Ile Glu Ser Ile Asp His Ser Val Leu Gly Ala Trp Leu Glu Gln Met
    690                 695                 700
Gln Leu Asp Gln Leu Val Val
705                 710
```

<210> 45
<211> 643
<212> PRT
<213> Glycine max

<400> 45

```
Arg Gly Ser Gly Phe Pro Gly Arg Pro Thr Arg Pro Arg Ser Gly Arg
1               5                   10                  15
Thr Arg Gly Arg Thr Arg Gly Val Asn Phe Ala Cys Gly Ala Asn Lys
            20                  25                  30
Thr Thr Ala Leu His Cys Ala Ala Ser Gly Ala Ser Thr Lys Ala Val
        35                  40                  45
Asp Ala Val Lys Leu Leu Leu Ser Ala Gly Ala Asp Val Asn Cys Val
    50                  55                  60
Asp Ala Asn Gly Asn Arg Pro Ile Asp Val Ile Ala Val Pro Pro Lys
65                  70                  75                  80
Leu Gln Gly Ala Lys Ala Val Leu Glu Glu Leu Leu Ser Asp Asn Ala
                85                  90                  95
Ser Asp Val Ser Val Gly Glu Phe Ser Val Pro Val Ser Val Asn Ser
            100                 105                 110
Ser Ser Pro Gly Ser Pro Ala His Ser Ser Asn Gly Met Pro Tyr Thr
        115                 120                 125
Pro Ser Val Ser Pro Pro Ser Pro Val Ala Ala Lys Phe Thr Asp Ala
    130                 135                 140
Ala Ile Cys Ser Leu Ser Glu Lys Ala Arg Glu Tyr Pro Ile Asp Pro
145                 150                 155                 160
Ser Leu Pro Asp Ile Lys Asn Ser Ile Tyr Ala Thr Asp Glu Phe Arg
                165                 170                 175
Met Phe Ser Phe Lys Val Arg Pro Cys Ser Arg Ala Tyr Ser His Asp
            180                 185                 190
Trp Thr Glu Cys Pro Phe Val His Pro Gly Glu Asn Ala Arg Arg Arg
        195                 200                 205
Asp Pro Arg Lys Phe His Tyr Ser Cys Val Pro Cys Pro Asp Phe Arg
    210                 215                 220
Lys Gly Ala Cys Arg Arg Gly Asp Met Cys Glu Tyr Ala His Gly Val
225                 230                 235                 240
Phe Glu Cys Trp Leu His Pro Ala Gln Tyr Arg Thr Arg Leu Cys Lys
                245                 250                 255
Asp Gly Thr Ser Cys Asn Arg Arg Val Cys Phe Phe Ala His Thr Ala
            260                 265                 270
Glu Glu Leu Arg Pro Leu Tyr Val Ser Thr Gly Ser Ala Ala Pro Ser
        275                 280                 285
Pro Arg Ser Ser Ala Ser Gly Pro Asn Val Met Asp Met Ala Ala Ala
    290                 295                 300
Met Ser Leu Phe Pro Gly Ser Pro Ser Ser Gly Ser Ser Ile Ser Leu
305                 310                 315                 320
Ser Ile Ser Phe Ser Leu Asp Pro Met Ser Pro Ser Ala Asn Gly Met
                325                 330                 335
Pro Leu Ser Ser Ala Trp Ala Gln Pro Asn Val Pro Ala Leu His Leu
            340                 345                 350
Pro Gly Ser Asn Leu Gln Ser Ser Arg Leu Arg Ser Ser Leu Ser Ala
        355                 360                 365
Arg Asp Ile Pro Pro Glu Asp Leu Asn Met Met Ser Asp Leu Asp Gly
    370                 375                 380
Gln Gln Gln His His Leu Asn Asp Leu Ser Cys Tyr Ile Gln Pro Arg
385                 390                 395                 400
Pro Gly Ala Ser Ser Val Ser Arg Ser Gly Arg Ser Lys Thr Leu Thr
                405                 410                 415
```

```
Pro Ser Asn Leu Glu Glu Leu Phe Ser Ala Glu Ile Ser Leu Ser Pro
            420                 425                 430
Arg Tyr Ser Asp Pro Ala Ala Gly Ser Val Phe Ser Pro Thr His Lys
        435                 440                 445
Ser Ala Val Leu Asn Gln Phe Gln Gln Leu Gln Ser Met Leu Ser Pro
    450                 455                 460
Ile Asn Thr Asn Leu Leu Ser Pro Lys Asn Val Glu His Pro Leu Phe
465                 470                 475                 480
Gln Ala Ser Phe Gly Val Ser Pro Ser Gly Arg Met Ser Pro Arg Ser
                485                 490                 495
Val Glu Pro Ile Ser Pro Met Ser Ala Arg Leu Ser Ala Phe Ala Gln
            500                 505                 510
Arg Glu Lys Gln Gln Gln Gln Leu Arg Ser Val Ser Ser Arg Asp Leu
        515                 520                 525
Gly Ala Asn Ser Pro Ala Ser Leu Val Gly Ser Pro Ala Asn Pro Trp
    530                 535                 540
Ser Lys Trp Gly Ser Pro Ile Gly Lys Ala Asp Trp Ser Val Asn Gly
545                 550                 555                 560
Asp Ser Leu Gly Arg Gln Met Arg Arg Ser Ser Ser Phe Glu Arg Lys
                565                 570                 575
Asn Asn Gly Glu Glu Pro Asp Leu Ser Trp Val Gln Ser Leu Val Lys
            580                 585                 590
Glu Ser Pro Pro Glu Met Ile Lys Glu Lys Phe Ala Ser Pro Met Pro
        595                 600                 605
Thr Ala Ser Ala Asp Gly Pro Asn Ser Asn Ser Gln Ile Glu Ser Ile
    610                 615                 620
Asp His Ser Val Leu Gly Ala Trp Leu Glu Gln Met Gln Leu Asp Gln
625                 630                 635                 640
Leu Val Val


<210>  46
<211>  669
<212>  PRT
<213>  Glycine max

<400>  46
Ser His Glu Met Asn His Leu Ser Leu Asp Thr Glu Asp Ser Leu Ala
1               5                   10                  15
Ser Leu Leu Leu Glu Leu Ala Ala Asn Asn Asp Val Ser Gly Phe Lys
            20                  25                  30
Arg Leu Ile Glu Cys Glu Pro Ser Ser Ile Asp Glu Val Gly Leu Trp
        35                  40                  45
Tyr Gly Arg His Lys Glu Ser Lys Lys Met Val Asn Glu Gln Arg Thr
    50                  55                  60
Pro Leu Met Val Ala Ala Thr Tyr Gly Ser Ile Asp Val Met Thr Leu
65                  70                  75                  80
Ile Leu Ser Leu Ser Glu Ala Asp Val Asn Arg Ser Ser Gly Leu Asp
                85                  90                  95
Lys Ser Thr Ala Leu His Cys Ala Ala Ser Gly Gly Ser Glu Asn Ala
            100                 105                 110
Val Asp Ala Val Lys Leu Leu Leu Glu Ala Gly Ala Asp Arg Asn Ser
        115                 120                 125
Val Asp Ala Asn Gly Arg Arg Pro Gly Asp Val Ile Val Ser Pro Pro
    130                 135                 140
Lys Leu Asp Tyr Val Lys Lys Ser Leu Glu Glu Leu Leu Gly Ser Asp
145                 150                 155                 160
Asp Trp Ser Leu Leu Arg Val Met Arg Ser Thr Cys Asn Gly Cys Ser
                165                 170                 175
```

```
Ala Glu Asp Leu Lys Met Lys Thr Asn Glu Val Ser Glu Lys Lys Glu
            180                 185                 190
Tyr Pro Val Asp Leu Ser Leu Pro Asp Ile Lys Asn Ser Ile Tyr Ser
        195                 200                 205
Ser Asp Glu Phe Arg Met Tyr Ser Phe Lys Val Arg Pro Cys Ser Arg
    210                 215                 220
Ala Tyr Ser His Asp Trp Thr Glu Cys Pro Phe Val His Pro Gly Glu
225                 230                 235                 240
Asn Ala Arg Arg Arg Asp Pro Arg Lys Phe His Tyr Ser Cys Val Pro
                245                 250                 255
Cys Pro Glu Phe Arg Lys Gly Ala Cys Arg Arg Gly Asp Met Cys Glu
            260                 265                 270
Tyr Ala His Gly Val Phe Glu Cys Trp Leu His Pro Ala Gln Tyr Arg
        275                 280                 285
Thr Arg Leu Cys Lys Asp Gly Thr Asn Cys Ala Arg Arg Val Cys Phe
    290                 295                 300
Phe Ala His Thr Asn Glu Glu Leu Arg Pro Leu Tyr Val Ser Thr Gly
305                 310                 315                 320
Ser Ala Val Pro Ser Pro Arg Ser Ser Ala Ser Ser Ala Met Asp Phe
                325                 330                 335
Val Ala Ala Ile Ser Pro Ser Ser Met Ser Val Met Ser Pro Ser Pro
            340                 345                 350
Phe Thr Pro Pro Met Ser Pro Ser Ser Ala Ser Ile Ala Trp Pro Gln
        355                 360                 365
Pro Asn Ile Pro Ala Leu His Leu Pro Gly Ser Asn Phe His Ser Ser
    370                 375                 380
Arg Leu Arg Ser Ser Leu Asn Ala Arg Asp Phe Ser Val Asp Asp Phe
385                 390                 395                 400
Asp Leu Leu Leu Pro Asp Tyr Asp His His His His Gln Gln Gln Gln
                405                 410                 415
Gln Gln Phe Leu Asn Glu Leu Ser Cys Leu Ser Pro His Ala Met Asn
            420                 425                 430
Cys Asn Thr Met Asn Arg Ser Gly Arg Met Lys Pro Leu Thr Pro Ser
        435                 440                 445
Asn Leu Asp Asp Leu Phe Ser Ala Glu Ser Ser Ser Pro Arg Tyr Ala
    450                 455                 460
Asp Pro Ala Leu Ala Ser Ala Val Phe Ser Pro Thr His Lys Ser Ala
465                 470                 475                 480
Val Phe Asn Gln Phe Gln His Gln Gln Ser Met Leu Ala Pro Leu Asn
                485                 490                 495
Thr Asn Phe Ala Ser Lys Asn Phe Glu His Pro Leu Leu Gln Ala Ser
            500                 505                 510
Leu Gly Met Ser Pro Arg Asn Val Glu Pro Ile Ser Pro Met Gly Ser
        515                 520                 525
Arg Ile Ser Met Leu Ala Gln Arg Glu Lys Gln Gln Phe Arg Ser Leu
    530                 535                 540
Ser Phe Arg Glu Leu Gly Ser Asn Ser Ala Ala Ala Ser Ala Asp Ser
545                 550                 555                 560
Trp Ser Lys Trp Gly Ser Pro Asn Val Lys Leu Asp Trp Pro Val Gly
                565                 570                 575
Ala Gly Glu Val Gly Lys Leu Arg Arg Ser Ser Ser Phe Glu Leu Gly
            580                 585                 590
Asn Asn Gly Glu Glu Pro Asp Leu Ser Trp Val Gln Ser Leu Val Lys
        595                 600                 605
Glu Ser Pro Ala Glu Val Lys Asp Lys Leu Ala Thr Thr Val Ser Tyr
    610                 615                 620
Val Ala Ala Ala Ala Ala Gly Ser Ser Ser Glu Gly Ser Asn Ile Ser
625                 630                 635                 640
Thr Gln Met Glu Ser Val Val Asp His Ala Val Leu Gly Ala Trp Leu
```

```
                645                 650                 655
Glu Gln Met Gln Leu Asp His Leu Val Ala Gln Gln Asn
            660                 665

<210>  47
<211>  580
<212>  PRT
<213>  Arabidopsis thaliana

<400>  47
Met Cys Ser Gly Pro Lys Ser Asn Leu Cys Ser Ser Arg Thr Leu Thr
1               5                   10                  15
Glu Ile Glu Ser Arg Gln Lys Glu Glu Glu Thr Met Leu Leu Leu Glu
            20                  25                  30
Phe Ala Ala Cys Asp Asp Leu Asp Ser Phe Lys Arg Glu Val Glu Glu
        35                  40                  45
Lys Gly Leu Asp Leu Asp Glu Ser Gly Leu Trp Tyr Cys Arg Arg Val
    50                  55                  60
Gly Ser Lys Lys Met Gly Leu Glu Glu Arg Thr Pro Leu Met Val Ala
65                  70                  75                  80
Ala Met Tyr Gly Ser Ile Lys Val Leu Thr Phe Ile Val Ser Thr Gly
                85                  90                  95
Lys Ser Asp Val Asn Arg Ala Cys Gly Glu Glu Arg Val Thr Pro Leu
            100                 105                 110
His Cys Ala Val Ala Gly Cys Ser Val Asn Met Ile Glu Val Ile Asn
        115                 120                 125
Val Leu Leu Asp Ala Ser Ala Leu Val Asn Ser Val Asp Ala Asn Gly
    130                 135                 140
Asn Gln Pro Leu Asp Val Phe Val Arg Val Ser Arg Phe Val Ala Ser
145                 150                 155                 160
Pro Arg Arg Lys Ala Val Glu Leu Leu Leu Arg Gly Gly Gly Val Gly
                165                 170                 175
Gly Leu Ile Asp Glu Ala Val Glu Glu Glu Ile Lys Ile Val Ser Lys
            180                 185                 190
Tyr Pro Ala Asp Ala Ser Leu Pro Asp Ile Asn Glu Gly Val Tyr Gly
        195                 200                 205
Ser Asp Glu Phe Arg Met Tyr Ser Phe Lys Val Lys Pro Cys Ser Arg
    210                 215                 220
Ala Tyr Ser His Asp Trp Thr Glu Cys Ala Phe Val His Pro Gly Glu
225                 230                 235                 240
Asn Ala Arg Arg Arg Asp Pro Arg Lys Tyr Pro Tyr Thr Cys Val Pro
                245                 250                 255
Cys Pro Glu Phe Arg Lys Gly Ser Cys Pro Lys Gly Asp Ser Cys Glu
            260                 265                 270
Tyr Ala His Gly Val Phe Glu Ser Trp Leu His Pro Ala Gln Tyr Lys
        275                 280                 285
Thr Arg Leu Cys Lys Asp Glu Thr Gly Cys Ala Arg Lys Val Cys Phe
    290                 295                 300
Phe Ala His Lys Arg Glu Glu Met Arg Pro Val Asn Ala Ser Thr Gly
305                 310                 315                 320
Ser Ala Val Ala Gln Ser Pro Phe Ser Ser Leu Glu Met Met Pro Gly
                325                 330                 335
Leu Ser Pro Leu Ala Tyr Ser Ser Gly Val Ser Thr Pro Pro Val Ser
            340                 345                 350
Pro Met Ala Asn Gly Val Pro Ser Ser Pro Arg Asn Gly Gly Ser Trp
        355                 360                 365
Gln Asn Arg Val Asn Thr Leu Thr Pro Pro Ala Leu Gln Leu Asn Gly
    370                 375                 380
Gly Ser Arg Leu Lys Ser Thr Leu Ser Ala Arg Asp Ile Asp Met Glu
```

```
385                 390                 395                 400
Met Glu Met Glu Leu Arg Leu Arg Gly Phe Gly Asn Asn Val Glu Glu
                405                 410                 415
Thr Phe Gly Ser Tyr Val Ser Ser Pro Ser Arg Asn Ser Gln Met Gly
            420                 425                 430
Gln Asn Met Asn Gln His Tyr Pro Ser Ser Pro Val Arg Gln Pro Pro
        435                 440                 445
Ser Gln His Gly Phe Glu Ser Ser Ala Ala Ala Ala Val Ala Val Met
    450                 455                 460
Lys Ala Arg Ser Thr Ala Phe Ala Lys Arg Ser Leu Ser Phe Lys Pro
465                 470                 475                 480
Ala Thr Gln Ala Ala Pro Gln Ser Asn Leu Ser Asp Trp Gly Ser Pro
                485                 490                 495
Asn Gly Lys Leu Glu Trp Gly Met Lys Gly Glu Glu Leu Asn Lys Met
            500                 505                 510
Arg Arg Ser Val Ser Phe Gly Ile His Gly Asn Asn Asn Asn Asn Ala
        515                 520                 525
Ala Arg Asp Tyr Arg Asp Glu Pro Asp Val Ser Trp Val Asn Ser Leu
    530                 535                 540
Val Lys Asp Ser Thr Val Val Ser Glu Arg Ser Phe Gly Met Asn Glu
545                 550                 555                 560
Arg Val Arg Ile Met Ser Trp Ala Glu Gln Met Tyr Arg Glu Lys Glu
                565                 570                 575
Gln Thr Val Val
            580
```

<210> 48
<211> 719
<212> PRT
<213> Arabidopsis thaliana

<400> 48

```
Met Cys Cys Gly Ser Asp Arg Leu Asn Gln Ile Val Ser Ser Arg Ser
1               5                   10                  15
Ser Leu Pro Ile Ser Phe Glu Glu Asp Asn Asn Leu Val Thr Asn Thr
            20                  25                  30
Asp Met Asn His Ile Thr Val Glu Thr Glu Asp Thr Phe Ala Ser Leu
        35                  40                  45
Leu Glu Leu Ala Ala Asn Asn Asp Val Glu Gly Val Arg Leu Ser Ile
    50                  55                  60
Glu Arg Asp Pro Ser Cys Val Asp Glu Ala Gly Leu Trp Tyr Gly Arg
65                  70                  75                  80
Gln Lys Gly Ser Lys Ala Met Val Asn Asp Tyr Arg Thr Pro Leu Met
                85                  90                  95
Val Ala Ala Thr Tyr Gly Ser Ile Asp Val Ile Lys Leu Ile Val Ser
            100                 105                 110
Leu Thr Asp Ala Asp Val Asn Arg Ala Cys Gly Asn Asp Gln Thr Thr
        115                 120                 125
Ala Leu His Cys Ala Ala Ser Gly Gly Ala Val Asn Ala Ile Gln Val
    130                 135                 140
Val Lys Leu Leu Leu Ala Ala Gly Ala Asp Leu Asn Leu Leu Asp Ala
145                 150                 155                 160
Glu Gly Gln Arg Ala Gly Asp Val Ile Val Val Pro Pro Lys Leu Glu
                165                 170                 175
Gly Val Lys Leu Met Leu Gln Glu Leu Leu Ser Ala Asp Gly Ser Ser
            180                 185                 190
Thr Ala Glu Arg Asn Leu Arg Val Val Thr Asn Val Pro Asn Arg Ser
        195                 200                 205
Ser Ser Pro Cys His Ser Pro Thr Gly Glu Asn Gly Gly Ser Gly Ser
```

```
    210                 215                 220
Gly Ser Pro Leu Gly Ser Pro Phe Lys Leu Lys Ser Thr Glu Phe Lys
225                 230                 235                 240
Lys Glu Tyr Pro Val Asp Pro Ser Leu Pro Asp Ile Lys Asn Ser Ile
                245                 250                 255
Tyr Ala Thr Asp Glu Phe Arg Met Tyr Ser Phe Lys Val Arg Pro Cys
            260                 265                 270
Ser Arg Ala Tyr Ser His Asp Trp Thr Glu Cys Pro Phe Val His Pro
        275                 280                 285
Gly Glu Asn Ala Arg Arg Arg Asp Pro Arg Lys Phe His Tyr Ser Cys
    290                 295                 300
Val Pro Cys Pro Asp Phe Arg Lys Gly Ala Cys Arg Arg Gly Asp Met
305                 310                 315                 320
Cys Glu Tyr Ala His Gly Val Phe Glu Cys Trp Leu His Pro Ala Gln
                325                 330                 335
Tyr Arg Thr Arg Leu Cys Lys Asp Gly Thr Gly Cys Ala Arg Arg Val
            340                 345                 350
Cys Phe Phe Ala His Thr Pro Glu Glu Leu Arg Pro Leu Tyr Ala Ser
        355                 360                 365
Thr Gly Ser Ala Val Pro Ser Pro Arg Ser Asn Ala Asp Tyr Ala Ala
    370                 375                 380
Ala Leu Ser Leu Leu Pro Gly Ser Pro Ser Gly Val Ser Val Met Ser
385                 390                 395                 400
Pro Leu Ser Pro Ser Ala Ala Gly Asn Gly Met Ser His Ser Asn Met
                405                 410                 415
Ala Trp Pro Gln Pro Asn Val Pro Ala Leu His Leu Pro Gly Ser Asn
            420                 425                 430
Leu Gln Ser Ser Arg Leu Arg Ser Ser Leu Asn Ala Arg Asp Ile Pro
        435                 440                 445
Thr Asp Glu Phe Asn Met Leu Ala Asp Tyr Glu Gln Gln Gln Leu Leu
    450                 455                 460
Asn Glu Tyr Ser Asn Ala Leu Ser Arg Ser Gly Arg Met Lys Ser Met
465                 470                 475                 480
Pro Pro Ser Asn Leu Glu Asp Leu Phe Ser Ala Glu Gly Ser Ser Ser
                485                 490                 495
Pro Arg Phe Thr Asp Ser Ala Leu Ala Ser Ala Val Phe Ser Pro Thr
            500                 505                 510
His Lys Ser Ala Val Phe Asn Gln Phe Gln Gln Gln Gln Gln Gln Gln
        515                 520                 525
Gln Gln Gln Ser Met Leu Ser Pro Ile Asn Thr Ser Phe Ser Ser Pro
    530                 535                 540
Lys Ser Val Asp His Ser Leu Phe Ser Gly Gly Gly Arg Met Ser Pro
545                 550                 555                 560
Arg Asn Val Val Glu Pro Ile Ser Pro Met Ser Ala Arg Val Ser Met
                565                 570                 575
Leu Ala Gln Cys Val Lys Gln Gln Gln Gln Gln Gln Gln Gln Gln Gln
            580                 585                 590
Gln Gln Gln His Gln Phe Arg Ser Leu Ser Ser Arg Glu Leu Arg Thr
        595                 600                 605
Asn Ser Ser Pro Ile Val Gly Ser Pro Val Asn Asn Asn Thr Trp Ser
    610                 615                 620
Ser Lys Trp Gly Ser Ser Asn Gly Gln Pro Asp Trp Gly Met Ser Ser
625                 630                 635                 640
Glu Ala Leu Gly Lys Leu Arg Ser Ser Ser Ser Phe Asp Gly Asp Glu
                645                 650                 655
Pro Asp Val Ser Trp Val Gln Ser Leu Val Lys Glu Thr Pro Ala Glu
            660                 665                 670
Ala Lys Glu Lys Ala Ala Thr Ser Ser Ser Gly Glu His Val Met Lys
        675                 680                 685
```

Gln Pro Asn Pro Val Glu Pro Val Met Asp His Ala Gly Leu Glu Ala
    690                 695                 700
Trp Ile Glu Gln Met Gln Leu Asp Gln Leu Val Ala Gln Gln Asn
705                 710                 715

<210> 49
<211> 686
<212> PRT
<213> Arabidopsis thaliana

<400> 49
Met Asn His Ile Thr Val Glu Thr Glu Asp Thr Phe Ala Ser Leu Leu
1               5                   10                  15
Glu Leu Ala Ala Asn Asn Asp Val Glu Gly Val Arg Leu Ser Ile Glu
            20                  25                  30
Arg Asp Pro Ser Cys Val Asp Glu Ala Gly Leu Trp Tyr Gly Arg Gln
        35                  40                  45
Lys Gly Ser Lys Ala Met Val Asn Asp Tyr Arg Thr Pro Leu Met Val
    50                  55                  60
Ala Ala Thr Tyr Gly Ser Ile Asp Val Ile Lys Leu Ile Val Ser Leu
65                  70                  75                  80
Thr Asp Ala Asp Val Asn Arg Ala Cys Gly Asn Asp Gln Thr Thr Ala
                85                  90                  95
Leu His Cys Ala Ala Ser Gly Gly Ala Val Asn Ala Ile Gln Val Val
            100                 105                 110
Lys Leu Leu Leu Ala Ala Gly Ala Asp Leu Asn Leu Leu Asp Ala Glu
        115                 120                 125
Gly Gln Arg Ala Gly Asp Val Ile Val Val Pro Pro Lys Leu Glu Gly
    130                 135                 140
Val Lys Leu Met Leu Gln Glu Leu Leu Ser Ala Asp Gly Ser Ser Thr
145                 150                 155                 160
Ala Glu Arg Asn Leu Arg Val Val Thr Asn Val Pro Asn Arg Ser Ser
                165                 170                 175
Ser Pro Cys His Ser Pro Thr Gly Glu Asn Gly Gly Ser Gly Ser Gly
            180                 185                 190
Ser Pro Leu Gly Ser Pro Phe Lys Leu Lys Ser Thr Glu Phe Lys Lys
        195                 200                 205
Glu Tyr Pro Val Asp Pro Ser Leu Pro Asp Ile Lys Asn Ser Ile Tyr
    210                 215                 220
Ala Thr Asp Glu Phe Arg Met Tyr Ser Phe Lys Val Arg Pro Cys Ser
225                 230                 235                 240
Arg Ala Tyr Ser His Asp Trp Thr Glu Cys Pro Phe Val His Pro Gly
                245                 250                 255
Glu Asn Ala Arg Arg Arg Asp Pro Arg Lys Phe His Tyr Ser Cys Val
            260                 265                 270
Pro Cys Pro Asp Phe Arg Lys Gly Ala Cys Arg Arg Gly Asp Met Cys
        275                 280                 285
Glu Tyr Ala His Gly Val Phe Glu Cys Trp Leu His Pro Ala Gln Tyr
    290                 295                 300
Arg Thr Arg Leu Cys Lys Asp Gly Thr Gly Cys Ala Arg Arg Val Cys
305                 310                 315                 320
Phe Phe Ala His Thr Pro Glu Glu Leu Arg Pro Leu Tyr Ala Ser Thr
                325                 330                 335
Gly Ser Ala Val Pro Ser Pro Arg Ser Asn Ala Asp Tyr Ala Ala Ala
            340                 345                 350
Leu Ser Leu Leu Pro Gly Ser Pro Ser Gly Val Ser Val Met Ser Pro
        355                 360                 365
Leu Ser Pro Ser Ala Ala Gly Asn Gly Met Ser His Ser Asn Met Ala
    370                 375                 380

Trp Pro Gln Pro Asn Val Pro Ala Leu His Leu Pro Gly Ser Asn Leu
385 390 395 400
Gln Ser Ser Arg Leu Arg Ser Ser Leu Asn Ala Arg Asp Ile Pro Thr
405 410 415
Asp Glu Phe Asn Met Leu Ala Asp Tyr Glu Gln Gln Gln Leu Leu Asn
420 425 430
Glu Tyr Ser Asn Ala Leu Ser Arg Ser Gly Arg Met Lys Ser Met Pro
435 440 445
Pro Ser Asn Leu Glu Asp Leu Phe Ser Ala Glu Gly Ser Ser Ser Pro
450 455 460
Arg Phe Thr Asp Ser Ala Leu Ala Ser Ala Val Phe Ser Pro Thr His
465 470 475 480
Lys Ser Ala Val Phe Asn Gln Phe Gln Gln Gln Gln Gln Gln Gln Gln
485 490 495
Gln Gln Ser Met Leu Ser Pro Ile Asn Thr Ser Phe Ser Ser Pro Lys
500 505 510
Ser Val Asp His Ser Leu Phe Ser Gly Gly Gly Arg Met Ser Pro Arg
515 520 525
Asn Val Val Glu Pro Ile Ser Pro Met Ser Ala Arg Val Ser Met Leu
530 535 540
Ala Gln Cys Val Lys Gln Gln Gln Gln Gln Gln Gln Gln Gln Gln Gln
545 550 555 560
Gln Gln His Gln Phe Arg Ser Leu Ser Ser Arg Glu Leu Arg Thr Asn
565 570 575
Ser Ser Pro Ile Val Gly Ser Pro Val Asn Asn Asn Thr Trp Ser Ser
580 585 590
Lys Trp Gly Ser Ser Asn Gly Gln Pro Asp Trp Gly Met Ser Ser Glu
595 600 605
Ala Leu Gly Lys Leu Arg Ser Ser Ser Ser Phe Asp Gly Asp Glu Pro
610 615 620
Asp Val Ser Trp Val Gln Ser Leu Val Lys Glu Thr Pro Ala Glu Ala
625 630 635 640
Lys Glu Lys Ala Ala Thr Ser Ser Ser Gly Glu His Val Met Lys Gln
645 650 655
Pro Asn Pro Val Glu Pro Val Met Asp His Ala Gly Leu Glu Ala Trp
660 665 670
Ile Glu Gln Met Gln Leu Asp Gln Leu Val Ala Gln Gln Asn
675 680 685

<210> 50
<211> 633
<212> PRT
<213> Arabidopsis thaliana

<400> 50
Met Val Asn Asp Tyr Arg Thr Pro Leu Met Val Ala Ala Thr Tyr Gly
1 5 10 15
Ser Ile Asp Val Ile Lys Leu Ile Val Ser Leu Thr Asp Ala Asp Val
20 25 30
Asn Arg Ala Cys Gly Asn Asp Gln Thr Thr Ala Leu His Cys Ala Ala
35 40 45
Ser Gly Gly Ala Val Asn Ala Ile Gln Val Val Lys Leu Leu Leu Ala
50 55 60
Ala Gly Ala Asp Leu Asn Leu Leu Asp Ala Glu Gly Gln Arg Ala Gly
65 70 75 80
Asp Val Ile Val Val Pro Pro Lys Leu Glu Gly Val Lys Leu Met Leu
85 90 95
Gln Glu Leu Leu Ser Ala Asp Gly Ser Ser Thr Ala Glu Arg Asn Leu
100 105 110

```
Arg Val Val Thr Asn Val Pro Asn Arg Ser Ser Ser Pro Cys His Ser
        115                 120                 125
Pro Thr Gly Glu Asn Gly Gly Ser Gly Ser Gly Ser Pro Leu Gly Ser
    130                 135                 140
Pro Phe Lys Leu Lys Ser Thr Glu Phe Lys Lys Glu Tyr Pro Val Asp
145                 150                 155                 160
Pro Ser Leu Pro Asp Ile Lys Asn Ser Ile Tyr Ala Thr Asp Glu Phe
                165                 170                 175
Arg Met Tyr Ser Phe Lys Val Arg Pro Cys Ser Arg Ala Tyr Ser His
            180                 185                 190
Asp Trp Thr Glu Cys Pro Phe Val His Pro Gly Glu Asn Ala Arg Arg
        195                 200                 205
Arg Asp Pro Arg Lys Phe His Tyr Ser Cys Val Pro Cys Pro Asp Phe
    210                 215                 220
Arg Lys Gly Ala Cys Arg Arg Gly Asp Met Cys Glu Tyr Ala His Gly
225                 230                 235                 240
Val Phe Glu Cys Trp Leu His Pro Ala Gln Tyr Arg Thr Arg Leu Cys
                245                 250                 255
Lys Asp Gly Thr Gly Cys Ala Arg Arg Val Cys Phe Phe Ala His Thr
            260                 265                 270
Pro Glu Glu Leu Arg Pro Leu Tyr Ala Ser Thr Gly Ser Ala Val Pro
        275                 280                 285
Ser Pro Arg Ser Asn Ala Asp Tyr Ala Ala Ala Leu Ser Leu Leu Pro
    290                 295                 300
Gly Ser Pro Ser Gly Val Ser Val Met Ser Pro Leu Ser Pro Ser Ala
305                 310                 315                 320
Ala Gly Asn Gly Met Ser His Ser Asn Met Ala Trp Pro Gln Pro Asn
                325                 330                 335
Val Pro Ala Leu His Leu Pro Gly Ser Asn Leu Gln Ser Ser Arg Leu
            340                 345                 350
Arg Ser Ser Leu Asn Ala Arg Asp Ile Pro Thr Asp Glu Phe Asn Met
        355                 360                 365
Leu Ala Asp Tyr Glu Gln Gln Gln Leu Leu Asn Glu Tyr Ser Asn Ala
    370                 375                 380
Leu Ser Arg Ser Gly Arg Met Lys Ser Met Pro Pro Ser Asn Leu Glu
385                 390                 395                 400
Asp Leu Phe Ser Ala Glu Gly Ser Ser Ser Pro Arg Phe Thr Asp Ser
                405                 410                 415
Ala Leu Ala Ser Ala Val Phe Ser Pro Thr His Lys Ser Ala Val Phe
            420                 425                 430
Asn Gln Phe Gln Gln Gln Gln Gln Gln Gln Gln Gln Gln Ser Met Leu
        435                 440                 445
Ser Pro Ile Asn Thr Ser Phe Ser Ser Pro Lys Ser Val Asp His Ser
    450                 455                 460
Leu Phe Ser Gly Gly Gly Arg Met Ser Pro Arg Asn Val Val Glu Pro
465                 470                 475                 480
Ile Ser Pro Met Ser Ala Arg Val Ser Met Leu Ala Gln Cys Val Lys
                485                 490                 495
Gln Gln Gln Gln Gln Gln Gln Gln Gln Gln Gln Gln Gln His Gln Phe
            500                 505                 510
Arg Ser Leu Ser Ser Arg Glu Leu Arg Thr Asn Ser Ser Pro Ile Val
        515                 520                 525
Gly Ser Pro Val Asn Asn Asn Thr Trp Ser Ser Lys Trp Gly Ser Ser
    530                 535                 540
Asn Gly Gln Pro Asp Trp Gly Met Ser Ser Glu Ala Leu Gly Lys Leu
545                 550                 555                 560
Arg Ser Ser Ser Ser Phe Asp Gly Asp Glu Pro Asp Val Ser Trp Val
                565                 570                 575
Gln Ser Leu Val Lys Glu Thr Pro Ala Glu Ala Lys Glu Lys Ala Ala
```

```
                580                 585                 590
Thr Ser Ser Ser Gly Glu His Val Met Lys Gln Pro Asn Pro Val Glu
        595                 600                 605
Pro Val Met Asp His Ala Gly Leu Glu Ala Trp Ile Glu Gln Met Gln
    610                 615                 620
Leu Asp Gln Leu Val Ala Gln Gln Asn
625                 630

<210>  51
<211>  678
<212>  PRT
<213>  Arabidopsis thaliana

<400>  51
Met Asn Asp Ala Ala Glu Trp Glu His Ser Phe Ser Ala Leu Leu Glu
1               5                   10                  15
Phe Ala Ala Asp Asn Asp Val Glu Gly Phe Arg Arg Gln Leu Ser Asp
            20                  25                  30
Val Ser Cys Ile Asn Gln Met Gly Leu Trp Tyr Arg Arg Gln Arg Phe
        35                  40                  45
Val Arg Arg Met Val Leu Glu Gln Arg Thr Pro Leu Met Val Ala Ser
    50                  55                  60
Leu Tyr Gly Ser Leu Asp Val Val Lys Phe Ile Leu Ser Phe Pro Glu
65                  70                  75                  80
Ala Glu Leu Asn Leu Ser Cys Gly Pro Asp Lys Ser Thr Ala Leu His
                85                  90                  95
Cys Ala Ala Ser Gly Ala Ser Val Asn Ser Leu Asp Val Val Lys Leu
            100                 105                 110
Leu Leu Ser Val Gly Ala Asp Pro Asn Ile Pro Asp Ala His Gly Asn
        115                 120                 125
Arg Pro Val Asp Val Leu Val Val Ser Pro His Ala Pro Gly Leu Arg
    130                 135                 140
Thr Ile Leu Glu Glu Ile Leu Lys Lys Asp Glu Ile Ile Ser Glu Asp
145                 150                 155                 160
Leu His Ala Ser Ser Ser Ser Leu Gly Ser Ser Phe Arg Ser Leu Ser
                165                 170                 175
Ser Ser Pro Asp Asn Gly Ser Ser Leu Leu Ser Leu Asp Ser Val Ser
            180                 185                 190
Ser Pro Thr Lys Pro His Gly Thr Asp Val Thr Phe Ala Ser Glu Lys
        195                 200                 205
Lys Glu Tyr Pro Ile Asp Pro Ser Leu Pro Asp Ile Lys Ser Gly Ile
    210                 215                 220
Tyr Ser Thr Asp Glu Phe Arg Met Phe Ser Phe Lys Ile Arg Pro Cys
225                 230                 235                 240
Ser Arg Ala Tyr Ser His Asp Trp Thr Glu Cys Pro Phe Ala His Pro
                245                 250                 255
Gly Glu Asn Ala Arg Arg Arg Asp Pro Arg Lys Phe His Tyr Thr Cys
            260                 265                 270
Val Pro Cys Pro Asp Phe Lys Lys Gly Ser Cys Lys Gln Gly Asp Met
        275                 280                 285
Cys Glu Tyr Ala His Gly Val Phe Glu Cys Trp Leu His Pro Ala Gln
    290                 295                 300
Tyr Arg Thr Arg Leu Cys Lys Asp Gly Met Gly Cys Asn Arg Arg Val
305                 310                 315                 320
Cys Phe Phe Ala His Ala Asn Glu Glu Leu Arg Pro Leu Tyr Pro Ser
                325                 330                 335
Thr Gly Ser Gly Leu Pro Ser Pro Arg Ala Ser Ser Ala Val Ser Ala
            340                 345                 350
Ser Thr Met Asp Met Ala Ser Val Leu Asn Met Leu Pro Gly Ser Pro
```

```
            355                 360                 365
Ser Ala Ala Gln His Ser Phe Thr Pro Pro Ile Ser Pro Ser Gly Asn
        370                 375                 380
Gly Ser Met Pro His Ser Ser Met Gly Trp Pro Gln Gln Asn Ile Pro
385                 390                 395                 400
Ala Leu Asn Leu Pro Gly Ser Asn Ile Gln Leu Ser Arg Leu Arg Ser
                405                 410                 415
Ser Leu Asn Ala Arg Asp Ile Pro Ser Glu Gln Leu Ser Met Leu His
            420                 425                 430
Glu Phe Glu Met Gln Arg Gln Leu Ala Gly Asp Met His Ser Pro Arg
        435                 440                 445
Phe Met Asn His Ser Ala Arg Pro Lys Thr Leu Asn Pro Ser Asn Leu
    450                 455                 460
Glu Glu Leu Phe Ser Ala Glu Val Ala Ser Pro Arg Phe Ser Asp Gln
465                 470                 475                 480
Leu Ala Val Ser Ser Val Leu Ser Pro Ser His Lys Ser Ala Leu Leu
                485                 490                 495
Asn Gln Leu Gln Asn Asn Lys Gln Ser Met Leu Ser Pro Ile Lys Thr
            500                 505                 510
Asn Leu Met Ser Ser Pro Lys Asn Val Glu Gln His Ser Leu Leu Gln
        515                 520                 525
Gln Ala Ser Ser Pro Arg Gly Gly Glu Pro Ile Ser Pro Met Asn Ala
    530                 535                 540
Arg Met Lys Gln Gln Leu His Ser Arg Ser Leu Ser Ser Arg Asp Phe
545                 550                 555                 560
Gly Ser Ser Leu Pro Arg Asp Leu Met Pro Thr Asp Ser Gly Ser Pro
                565                 570                 575
Leu Ser Pro Trp Ser Ser Trp Asp Gln Thr His Gly Ser Lys Val Asp
            580                 585                 590
Trp Ser Val Gln Ser Asp Glu Leu Gly Arg Leu Arg Lys Ser His Ser
        595                 600                 605
Leu Ala Asn Asn Pro Asn Arg Glu Ala Asp Val Ser Trp Ala Gln Gln
    610                 615                 620
Met Leu Lys Asp Ser Ser Ser Pro Arg Asn Gly Asn Arg Val Val Asn
625                 630                 635                 640
Met Asn Gly Ala Arg Pro Leu Thr Gln Gly Gly Ser Ser Val Asn Pro
                645                 650                 655
His Asn Ser Asp Thr Arg Glu Ser Asp Ile Leu Asp Ala Trp Leu Glu
            660                 665                 670
Gln Leu His Leu Asp Arg
        675

<210>  52
<211>  640
<212>  PRT
<213>  Arabidopsis thaliana

<400>  52
Met Gly Leu Trp Tyr Arg Arg Gln Arg Phe Val Arg Arg Met Val Leu
1               5                   10                  15
Glu Gln Arg Thr Pro Leu Met Val Ala Ser Leu Tyr Gly Ser Leu Asp
            20                  25                  30
Val Val Lys Phe Ile Leu Ser Phe Pro Glu Ala Glu Leu Asn Leu Ser
        35                  40                  45
Cys Gly Pro Asp Lys Ser Thr Ala Leu His Cys Ala Ala Ser Gly Ala
    50                  55                  60
Ser Val Asn Ser Leu Asp Val Val Lys Leu Leu Leu Ser Val Gly Ala
65                  70                  75                  80
Asp Pro Asn Ile Pro Asp Ala His Gly Asn Arg Pro Val Asp Val Leu
```

```
                    85                      90                      95
Val Val Ser Pro His Ala Pro Gly Leu Arg Thr Ile Leu Glu Glu Ile
                100                     105                     110
Leu Lys Lys Asp Glu Ile Ile Ser Glu Asp Leu His Ala Ser Ser Ser
            115                     120                     125
Ser Leu Gly Ser Ser Phe Arg Ser Leu Ser Ser Ser Pro Asp Asn Gly
    130                     135                     140
Ser Ser Leu Leu Ser Leu Asp Ser Val Ser Ser Pro Thr Lys Pro His
145                     150                     155                     160
Gly Thr Asp Val Thr Phe Ala Ser Glu Lys Lys Glu Tyr Pro Ile Asp
                165                     170                     175
Pro Ser Leu Pro Asp Ile Lys Ser Gly Ile Tyr Ser Thr Asp Glu Phe
                180                     185                     190
Arg Met Phe Ser Phe Lys Ile Arg Pro Cys Ser Arg Ala Tyr Ser His
            195                     200                     205
Asp Trp Thr Glu Cys Pro Phe Ala His Pro Gly Glu Asn Ala Arg Arg
    210                     215                     220
Arg Asp Pro Arg Lys Phe His Tyr Thr Cys Val Pro Cys Pro Asp Phe
225                     230                     235                     240
Lys Lys Gly Ser Cys Lys Gln Gly Asp Met Cys Glu Tyr Ala His Gly
                245                     250                     255
Val Phe Glu Cys Trp Leu His Pro Ala Gln Tyr Arg Thr Arg Leu Cys
                260                     265                     270
Lys Asp Gly Met Gly Cys Asn Arg Arg Val Cys Phe Phe Ala His Ala
            275                     280                     285
Asn Glu Glu Leu Arg Pro Leu Tyr Pro Ser Thr Gly Ser Gly Leu Pro
    290                     295                     300
Ser Pro Arg Ala Ser Ser Ala Val Ser Ala Ser Thr Met Asp Met Ala
305                     310                     315                     320
Ser Val Leu Asn Met Leu Pro Gly Ser Pro Ser Ala Ala Gln His Ser
                325                     330                     335
Phe Thr Pro Pro Ile Ser Pro Ser Gly Asn Gly Ser Met Pro His Ser
                340                     345                     350
Ser Met Gly Trp Pro Gln Gln Asn Ile Pro Ala Leu Asn Leu Pro Gly
            355                     360                     365
Ser Asn Ile Gln Leu Ser Arg Leu Arg Ser Ser Leu Asn Ala Arg Asp
    370                     375                     380
Ile Pro Ser Glu Gln Leu Ser Met Leu His Glu Phe Glu Met Gln Arg
385                     390                     395                     400
Gln Leu Ala Gly Asp Met His Ser Pro Arg Phe Met Asn His Ser Ala
                405                     410                     415
Arg Pro Lys Thr Leu Asn Pro Ser Asn Leu Glu Glu Leu Phe Ser Ala
                420                     425                     430
Glu Val Ala Ser Pro Arg Phe Ser Asp Gln Leu Ala Val Ser Ser Val
            435                     440                     445
Leu Ser Pro Ser His Lys Ser Ala Leu Leu Asn Gln Leu Gln Asn Asn
    450                     455                     460
Lys Gln Ser Met Leu Ser Pro Ile Lys Thr Asn Leu Met Ser Ser Pro
465                     470                     475                     480
Lys Asn Val Glu Gln His Ser Leu Leu Gln Gln Ala Ser Ser Pro Arg
                485                     490                     495
Gly Gly Glu Pro Ile Ser Pro Met Asn Ala Arg Met Lys Gln Gln Leu
                500                     505                     510
His Ser Arg Ser Leu Ser Ser Arg Asp Phe Gly Ser Ser Leu Pro Arg
            515                     520                     525
Asp Leu Met Pro Thr Asp Ser Gly Ser Pro Leu Ser Pro Trp Ser Ser
    530                     535                     540
Trp Asp Gln Thr His Gly Ser Lys Val Asp Trp Ser Val Gln Ser Asp
545                     550                     555                     560
```

```
Glu Leu Gly Arg Leu Arg Lys Ser His Ser Leu Ala Asn Asn Pro Asn
                565                 570                 575
Arg Glu Ala Asp Val Ser Trp Ala Gln Gln Met Leu Lys Asp Ser Ser
            580                 585                 590
Ser Pro Arg Asn Gly Asn Arg Val Val Asn Met Asn Gly Ala Arg Pro
        595                 600                 605
Leu Thr Gln Gly Gly Ser Ser Val Asn Pro His Asn Ser Asp Thr Arg
    610                 615                 620
Glu Ser Asp Ile Leu Asp Ala Trp Leu Glu Gln Leu His Leu Asp Arg
625                 630                 635                 640
```

```
<210>  53
<211>  2158
<212>  DNA
<213>  Oryza sativa

<400>  53
tgctccttct tcattgcaaa gaaagcacca ccttttaaag aatcctcctc acactccatt      60
cttcttaaaa aacccaccac aacacaattc ccacttgttt cttcatcatc acctacttca     120
atcaaaaaac attccaactt tcttctcaat ttcattccag gatagtatta tgtgcagtgg     180
accaaagagc aatctctgct cttcaagaac cttaacagaa atcgaatcaa ggcaaaagga     240
agaagaaaca atgcttctcc tcgaattcgc tgcttgtgat gatcttgact cgttcaagag     300
agaggttgaa gagaaagggc ttgatttgga tgagtcaggg ttatggtatt gcagacgtgt     360
cggttctaag aagatgggtc ttgaagaaag aacacccttta atggttgcag ctatgtatgg     420
aagcataaag gttttgactt tcatcgtttc cactggaaaa tctgatgtga acagagcttg     480
tggtgaagag agagttactc cgcttcactg tgctgttgct ggctgttctg tgaatatgat     540
tgaagtcatc aatgtcttgc ttgatgcttc tgctttggtt aactctgttg atgctaatgg     600
gaatcaacct ttggatgtgt ttgttcgagt ttcgaggttt gtggctagtc cgaggaggaa     660
agcggttgag ttgttgctga gaggaggagg tgttggagga ttgatcgatg aggcggttga     720
agaagagatc aagattgtct ctaagtatcc agctgatgct tctttaccgg atataaacga     780
aggggtttat ggaagtgatg agtttaggat gtatagcttt aaggttaagc catgttctag     840
ggcttattct catgattgga ccgagtgtgc ttttgttcat ccgggagaaa atgcgaggag     900
gagagatccg aggaagtatc cttacacttg tgtcccctgt cccgagttcc gtaaaggatc     960
atgcccgaaa ggagattctt gcgagtatgc tcacggggtt ttcgagtcgt ggcttcaccc    1020
cgcgcagtat aaaacccggc tttgtaaaga tgaaacgggt tgtgcaagga aagtttgttt    1080
ctttgctcat aaacgcgaag agatgagacc tgttaatgct tcaactggct ctgccgtggc    1140
tcagtctccg tttagcagct tggagatgat gccagggttg tctcctcttg cttattcttc    1200
aggagtttcg actcctccgg tttctccaat ggctaatggt gttccttcct ctccaagaaa    1260
cggcggatca tggcagaaca gagtcaatac ccttactcca ccggctttgc agctcaatgg    1320
tggaagcaga ttgaagtcca cactgagcgc tagagatatc gatatggaga tggagatgga    1380
attgagactc cgcggttttg gcaacaatgt ggaagagacg ttcgggtctt atgtttcctc    1440
tccaagtagg aattctcaaa tgggtcaaaa catgaaccaa cattatccat cttccccggt    1500
gagacaaccg ccatctcaac acgggttcga atcttcagca gctgcagcgg ttgcagtgat    1560
gaaagcgaga tcaaccgcct ttgcgaaacg tagcttgagc ttcaaaccag ctactcaagc    1620
agcaccacag tcgaatctct cggattgggg atctccaaac gggaagctgg aatggggaat    1680
gaaaggagaa gagctgaata agatgagaag aagtgtttcc tttggaatcc atggaaacaa    1740
caacaataac gcagctagag actacaggga cgagccagat gtgtcatggg ttaactcttt    1800
agttaaagac agtactgtgg tgtctgagag aagctttgga atgaatgaga gggttcggat    1860
aatgtcgtgg gctgagcaaa tgtacagaga gaaggagcag actgtggtgt aaacacacac    1920
aaagatggtt tcttatatat attgcttttg ggccatctct gcaaatttga ttctttaatt    1980
tttgtgactt tctttagttg ttactgttat tagtagtata tggtttgttg tcactacgag    2040
tctacgtgat gaaaagatag aagttaattg cattagtttc tatattcgtt tctcatcctc    2100
ttgtaattta tcaaaccatg aaatggctaa gcaatccaaa ccgaaaaaaa aaaaaaa       2158

<210>  54
<211>  2193
<212>  DNA
<213>  Oryza sativa
```

<400> 54
aatccgaaaa gtttctgcac cgttttcacc ccctaactaa caatataggg aacgtgtgct 60
aaatataaaa tgagacctta tatatgtagc gctgataact agaactatgc aagaaaaact 120
catccaccta ctttagtggc aatcgggcta aataaaaaag agtcgctaca ctagtttcgt 180
tttccttagt aattaagtgg gaaaatgaaa tcattattgc ttagaatata cgttcacatc 240
tctgtcatga agttaaatta ttcgaggtag ccataattgt catcaaactc ttcttgaata 300
aaaaaatctt tctagctgaa ctcaatgggt aaagagagag attttttta aaaaaataga 360
atgaagatat tctgaacgta ttggcaaaga tttaaacata taattatata attttatagt 420
ttgtgcattc gtcatatcgc acatcattaa ggacatgtct tactccatcc caatttttat 480
ttagtaatta aagacaattg acttattttt attatttatc tttttcgat tagatgcaag 540
gtacttacgc acacactttg tgctcatgtg catgtgtgag tgcacctcct caatacacgt 600
tcaactagca acacatctct aatatcactc gcctatttaa tacatttagg tagcaatatc 660
tgaattcaag cactccacca tcaccagacc acttttaata atatctaaaa tacaaaaaat 720
aattttacag aatagcatga aaagtatgaa acgaactatt taggtttttc acatacaaaa 780
aaaaaaagaa ttttgctcgt gcgcgagcgc caatctccca tattgggcac acaggcaaca 840
acagagtggc tgcccacaga acaacccaca aaaaacgatg atctaacgga ggacagcaag 900
tccgcaacaa ccttttaaca gcaggctttg cggccaggag agaggaggag aggcaaagaa 960
aaccaagcat cctcctcctc ccatctataa attcctcccc ccttttcccc tctctatata 1020
ggaggcatcc aagccaagaa gagggagagc accaaggaca cgcgactagc agaagccgag 1080
cgaccgcctt cttcgatcca tatcttccgg tcgagttctt ggtcgatctc ttccctcctc 1140
cacctcctcc tcacagggta tgtgcccttc ggttgttctt ggatttattg ttctaggttg 1200
tgtagtacgg gcgttgatgt taggaaaggg gatctgtatc tgtgatgatt cctgttcttg 1260
gatttgggat agaggggttc ttgatgttgc atgttatcgg ttcggtttga ttagtagtat 1320
ggttttcaat cgtctggaga gctctatgga aatgaaatgg tttagggtac ggaatcttgc 1380
gattttgtga gtaccttttg tttgaggtaa aatcagagca ccggtgattt tgcttggtgt 1440
aataaaagta cggttgtttg gtcctcgatt ctggtagtga tgcttctcga tttgacgaag 1500
ctatcctttg tttattccct attgaacaaa aataatccaa ctttgaagac ggtcccgttg 1560
atgagattga atgattgatt cttaagcctg tccaaaattt cgcagctggc ttgtttagat 1620
acagtagtcc ccatcacgaa attcatggaa acagttataa tcctcaggaa caggggattc 1680
cctgttcttc cgatttgctt tagtcccaga attttttttc ccaaatatct taaaaagtca 1740
ctttctggtt cagttcaatg aattgattgc tacaaataat gcttttatag cgttatccta 1800
gctgtagttc agttaatagg taataccccct atagtttagt caggagaaga acttatccga 1860
tttctgatct ccatttttaa ttatatgaaa tgaactgtag cataagcagt attcatttgg 1920
attattttt ttattagctc tcacccttc attattctga gctgaaagtc tggcatgaac 1980
tgtcctcaat tttgttttca aattcacatc gattatctat gcattatcct cttgtatcta 2040
cctgtagaag tttcttttg gttattcctt gactgcttga ttacagaaag aaatttatga 2100
agctgtaatc gggatagtta tactgcttgt tcttatgatt catttccttt gtgcagttct 2160
tggtgtagct tgccactttc accagcaaag ttc 2193

<210> 55
<211> 1827
<212> DNA
<213> Oryza sativa

<400> 55
gcttgagtca tagggagaaa acaaatcgat catatttgac tcttttccct ccatctctct 60
taccggcaaa aaaagtagta ctggtttata tgtaaagtaa gattctttaa ttatgtgaga 120
tccggcttaa tgcttttctt ttgtcacata tactgcattg caacaattgc catatattca 180
cttctgccat cccattatat agcaactcaa gaatggattg atatatcccc tattactaat 240
ctagacatgt taaggctgag ttgggcagtc catcttccca acccaccacc ttcgtttttc 300
gcgcacatac ttttcaaact actaaatggt gtgtttttta aaaatatttt caatacaaaa 360
gttgctttaa aaaattatat tgatccattt ttttaaaaaa aatagctaat acttaattaa 420
tcacgtgtta aaagaccgct ccgttttgcg tgcaggaggg ataggttcac atcctgcatt 480
accgaacaca gcctaaatct tgttgtctag attcgtagta ctggatatat taaatcatgt 540
tctaagttac tatatactga gatgaataga ataagtaaaa ttagacccac cttaagtctt 600
gatgaagtta ctactagctg cgtttgggag gacttcccaa aaaaaaaagt attagccatt 660
agcacgtgat taattaagta ctagtttaaa aaacttaaaa aataaattaa tatgattctc 720
ttaagtaact ctcctataga aaacttttac aaaattacac cgtttaatag tttggaaaat 780
atgtcagtaa aaaataagag agtagaagtt atgaaagtta gaaaaagaat tgttttagta 840

```
gtatacagtt ataaactatt ccctctgttc taaaacataa gggattatgg atggattcga     900
catgtaccag taccatgaat cgaatccaga caagtttttt atgcatattt attctactat     960
aatatatcac atctgctcta aatatcttat atttcgaggt ggagactgtc gctatgtttt    1020
tctgcccgtt gctaagcaca cgccaccccc gatgcgggga cgcctctggc cttcttgcca    1080
cgataattga atggaacttc cacattcaga ttcgataggt gaccgtcgac tccaagtgct    1140
ttgcacaaaa caactccggc ctcccggcca ccagtcacac gactcacggc actaccaccc    1200
ctgactccct gaggcggacc tgccactgtt ctgcatgcga agctatctaa aattctgaag    1260
caaagaaagc acagcacatg ctccgggaca cgcgccaccc ggcggaaaag ggctcggtgt    1320
ggcgatctca cagccgcata tcgcatttca caagccgccc atctccaccg gcttcacgag    1380
gctcatcgcg gcacgaccgc gcacggaacg cacgcggccg acccgcgcgc ctcgatgcgc    1440
gagcccatcc gccgcgtcct ccctttgcct ttgccgctat cctctcggtc gtatcccgtt    1500
tctctgtctt ttgctccccg gcgcgcgcca gttcggagta ccagcgaaac ccggacacct    1560
ggtacacctc cgccggccac aacgcgtgtc cccctacgtg gccgcgcagc acatgcccat    1620
gcgcgacacg tgcacctcct catccaaact ctcaagtctc aacggtccta taaatgcacg    1680
gatagcctca agctgctcgt cacaaggcaa gaggcaagag gcaagagcat ccgtattaac    1740
cagccttttg agacttgaga gtgtgtgtga ctcgatccag cgtagtttca gttcgtgtgt    1800
tggtgagtga ttccagccaa gtttgcg                                        1827
```

<210> 56
<211> 2194
<212> DNA
<213> Oryza sativa

<400> 56

```
aatccgaaaa gtttctgcac cgttttcacc ccctaactaa caatataggg aacgtgtgct      60
aaatataaaa tgagacctta tatatgtagc gctgataact agaactatgc aagaaaaact     120
catccaccta ctttagtggc aatcgggcta aataaaaaag agtcgctaca ctagtttcgt     180
tttccttagt aattaagtgg gaaaatgaaa tcattattgc ttagaatata cgttcacatc     240
tctgtcatga agttaaatta ttcgaggtag ccataattgt catcaaactc ttcttgaata     300
aaaaaatctt tctagctgaa ctcaatgggt aaagagagag atttttttta aaaaaataga     360
atgaagatat tctgaacgta ttggcaaaga tttaaacata taattatata atttatagt      420
ttgtgcattc gtcatatcgc acatcattaa ggacatgtct tactccatcc caatttttat     480
ttagtaatta aagacaattg acttattttt attatttatc tttttcgat tagatgcaag      540
gtacttacgc acacactttg tgctcatgtg catgtgtgag tgcacctcct caatacacgt     600
tcaactagca acacatctct aatatcactc gcctatttaa tacatttagg tagcaatatc     660
tgaattcaag cactccacca tcaccagacc acttttaata atatctaaaa tacaaaaaat     720
aattttacag aatagcatga aaagtatgaa acgaactatt taggtttttc acatacaaaa     780
aaaaaaagaa ttttgctcgt gcgcgagcgc caatctccca tattgggcac acaggcaaca     840
acagagtggc tgcccacaga acaacccaca aaaaacgatg atctaacgga ggacagcaag     900
tccgcaacaa ccttttaaca gcaggctttg cggccaggag agaggaggag aggcaaagaa     960
aaccaagcat cctccttctc ccatctataa attcctcccc ccttttcccc tctctatata    1020
ggaggcatcc aagccaagaa gagggagagc accaaggaca cgcgactagc agaagccgag    1080
cgaccgcctt ctcgatccat atcttccggt cgagttcttg gtcgatctct tccctcctcc    1140
acctcctcct cacagggtat gtgcctccct tcggttgttc ttggatttat tgttctaggt    1200
tgtgtagtac gggcgttgat gttaggaaag gggatctgta tctgtgatga ttcctgttct    1260
tggatttggg atagaggggt tcttgatgtt gcatgttatc ggttcggttt gattagtagt    1320
atggttttca atcgtctgga gagctctatg gaaatgaaat ggtttaggga tcggaatctt    1380
gcgattttgt gagtaccttt tgtttgaggt aaaatcagag caccggtgat tttgcttggt    1440
gtaataaagt acggttgttt ggtcctcgat tctggtagtg atgcttctcg atttgacgaa    1500
gctatccttt gtttattccc tattgaacaa aaataatcca actttgaaga cggtcccgtt    1560
gatgagattg aatgattgat tcttaagcct gtccaaaatt tcgcagctgg cttgtttaga    1620
tacagtagtc cccatcacga aattcatgga aacagttata atcctcagga acaggggatt    1680
ccctgttctt ccgatttgct ttagtcccag aatttttttt cccaaatatc ttaaaaagtc    1740
actttctggt tcagttcaat gaattgattg ctacaaataa tgcttttata gcgttatcct    1800
agctgtagtt cagttaatag gtaatacccc tatagtttag tcaggagaag aacttatccg    1860
atttctgatc tccattttta attatatgaa atgaactgta gcataagcag tattcatttg    1920
gattattttt tttattagct ctcacccctt cattattctg agctgaaagt ctggcatgaa    1980
ctgtcctcaa ttttgttttc aaattcacat cgattatcta tgcattatcc tcttgtatct    2040
acctgtagaa gtttcttttt ggttattcct tgactgcttg attacagaaa gaaatttatg    2100
```

aagctgtaat cgggatagtt atactgcttg ttcttatgat tcatttcctt tgtgcagttc 2160
ttggtgtagc ttgccacttt caccagcaaa gttc 2194

<210> 57
<211> 46
<212> PRT
<213> Artificial sequence

<220>
<223> SNH domain

<220>
<221> VARIANT
<222> (3)..(3)
<223> /replace = "Lys"

<220>
<221> UNSURE
<222> (4)..(4)
<223> Xaa can be any naturally occurring amino acid

<220>
<221> VARIANT
<222> (6)..(6)
<223> /replace = "Glu"

<220>
<221> VARIANT
<222> (7)..(7)
<223> /replace = "Asp"

<220>
<221> VARIANT
<222> (9)..(9)
<223> /replace = "Asn"

<220>
<221> UNSURE
<222> (10)..(10)
<223> Xaa can be any naturally occurring amino acid

<220>
<221> UNSURE
<222> (13)..(13)
<223> Xaa can be any naturally occurring amino acid

<220>
<221> VARIANT
<222> (14)..(14)
<223> /replace = "Ala" /replace = "Lys"

<220>
<221> VARIANT
<222> (16)..(16)
<223> /replace = "Val" /replace = "Met"

<220>
<221> VARIANT
<222> (17)..(17)

<223> /replace = "Asp" /replace = "Ser"

<220>
<221> VARIANT
<222> (18)..(18)
<223> /replace = "Asn"

<220>
<221> VARIANT
<222> (19)..(19)
<223> /replace = "Leu"

<220>
<221> UNSURE
<222> (21)..(21)
<223> Xaa can be any naturally occurring amino acid

<220>
<221> VARIANT
<222> (23)..(23)
<223> /replace = "Arg"

<220>
<221> UNSURE
<222> (24)..(25)
<223> Xaa can be any naturally occurring amino acid

<220>
<221> VARIANT
<222> (28)..(28)
<223> /replace = "Asp" /replace = "Ser"

<220>
<221> UNSURE
<222> (29)..(30)
<223> Xaa can be any naturally occurring amino acid

<220>
<221> VARIANT
<222> (32)..(32)
<223> /replace = "Ser" /replace = "Gln"

<220>
<221> UNSURE
<222> (33)..(33)
<223> Xaa can be any naturally occurring amino acid

<220>
<221> VARIANT
<222> (35)..(35)
<223> /replace = "His"

<220>
<221> UNSURE
<222> (36)..(36)
<223> Xaa can be any naturally occurring amino acid

<220>
<221> VARIANT

<222> (39)..(39)
<223> /replace = "Leu" /replace = "Val"

<220>
<221> VARIANT
<222> (43)..(43)
<223> /replace = "Thr"

<400> 57
Ile Gln Gln Xaa Leu Asp Glu Asn Lys Xaa Leu Ile Xaa Cys Ile Leu
1 5 10 15
Glu Ser Gln Asn Xaa Gly Lys Xaa Xaa Glu Cys Ala Xaa Xaa Gln Ala
20 25 30
Xaa Leu Gln Xaa Asn Leu Met Tyr Leu Ala Ala Ile Ala Asp
35 40 45

<210> 58
<211> 46
<212> PRT
<213> Arabidopsis thaliana

<400> 58
Ile Gln Gln Tyr Leu Asp Glu Asn Lys Ser Leu Ile Leu Lys Ile Val
1 5 10 15
Glu Ser Gln Asn Ser Gly Lys Leu Ser Glu Cys Ala Glu Asn Gln Ala
20 25 30
Arg Leu Gln Arg Asn Leu Met Tyr Leu Ala Ala Ile Ala Asp
35 40 45

<210> 59
<211> 633
<212> DNA
<213> Arabidopsis thaliana

<400> 59
atgcaacagc acctgatgca gatgcagccc atgatggctg gttactaccc cagcaatgtt 60
acctctgatc atatccaaca gtacttggac gaaaacaaat cgttgattct gaagattgtt 120
gagtctcaaa actctggaaa gcttagcgaa tgcgccgaga atcaagcaag gcttcaacgc 180
aacctaatgt acctagctgc aatagcagat tctcagcctc agccaccaag tgtgcatagc 240
cagtatggat ctgctggtgg tgggatgatt cagggagaag gagggtcaca ctatttgcag 300
cagcaacaag cgactcaaca gcaacagatg actcagcagt ctctaatggc ggctcgatct 360
tcaatgttgt atgctcagca acagcagcag cagcagcctt acgcgacgct tcagcatcag 420
caattgcacc atagccagct tggaatgagc tcgagcagcg gaggaggagg aagcagtggt 480
ctccatatcc ttcagggaga ggctggtggg tttcatgatt ttggccgtgg gaagccggaa 540
atgggaagtg gtggtggcgg tgaaggcaga ggaggaagtt caggggatgg tggagaaacc 600
ctttacttga aatcatcaga tgatgggaat tga 633

<210> 60
<211> 210
<212> PRT
<213> Arabidopsis thaliana

<400> 60
Met Gln Gln His Leu Met Gln Met Gln Pro Met Met Ala Gly Tyr Tyr
1 5 10 15
Pro Ser Asn Val Thr Ser Asp His Ile Gln Gln Tyr Leu Asp Glu Asn
20 25 30
Lys Ser Leu Ile Leu Lys Ile Val Glu Ser Gln Asn Ser Gly Lys Leu
35 40 45

Ser Glu Cys Ala Glu Asn Gln Ala Arg Leu Gln Arg Asn Leu Met Tyr
    50                  55                  60
Leu Ala Ala Ile Ala Asp Ser Gln Pro Gln Pro Pro Ser Val His Ser
65                  70                  75                  80
Gln Tyr Gly Ser Ala Gly Gly Gly Met Ile Gln Gly Glu Gly Gly Ser
                85                  90                  95
His Tyr Leu Gln Gln Gln Gln Ala Thr Gln Gln Gln Gln Met Thr Gln
            100                 105                 110
Gln Ser Leu Met Ala Ala Arg Ser Ser Met Leu Tyr Ala Gln Gln Gln
        115                 120                 125
Gln Gln Gln Gln Pro Tyr Ala Thr Leu Gln His Gln Gln Leu His His
    130                 135                 140
Ser Gln Leu Gly Met Ser Ser Ser Ser Gly Gly Gly Gly Ser Ser Gly
145                 150                 155                 160
Leu His Ile Leu Gln Gly Glu Ala Gly Gly Phe His Asp Phe Gly Arg
                165                 170                 175
Gly Lys Pro Glu Met Gly Ser Gly Gly Gly Gly Glu Gly Arg Gly Gly
            180                 185                 190
Ser Ser Gly Asp Gly Gly Glu Thr Leu Tyr Leu Lys Ser Ser Asp Asp
        195                 200                 205
Gly Asn
    210

<210> 61
<211> 588
<212> DNA
<213> Arabidopsis thaliana

<400> 61
atgcagcagc agcagtctcc gcaaatgttt ccgatggttc cgtcgattcc ccctgctaac 60
aacatcacta ccgaacagat ccaaaagtac cttgatgaga acaagaagct gattatggcc 120
atcatggaaa accagaatct cggtaaactt gctgagtgcg cccagtacca agctcttctc 180
cagaagaact tgatgtatct tgctgcaatt gctgatgctc aaccccccacc acctacgcca 240
ggaccttcac catctacagc tgtcgctgcc cagatggcaa caccgcattc tgggatgcaa 300
ccacctagct acttcatgca acacccacaa gcatcccctg cagggatttt cgctccaagg 360
ggtcctttac agtttggtag cccactccag tttcaggatc cgcaacagca gcagcagata 420
catcagcaag ctatgcaagg acacatgggg attagaccaa tgggtatgac caacaacggg 480
atgcagcatg cgatgcaaca accagaaacc ggtcttggag gaaacgtggg gcttagagga 540
ggaaagcaag atggagcaga tggacaagga aaagatgatg gcaagtga 588

<210> 62
<211> 195
<212> PRT
<213> Arabidopsis thaliana

<400> 62
Met Gln Gln Gln Gln Ser Pro Gln Met Phe Pro Met Val Pro Ser Ile
1               5                   10                  15
Pro Pro Ala Asn Asn Ile Thr Thr Glu Gln Ile Gln Lys Tyr Leu Asp
            20                  25                  30
Glu Asn Lys Lys Leu Ile Met Ala Ile Met Glu Asn Gln Asn Leu Gly
        35                  40                  45
Lys Leu Ala Glu Cys Ala Gln Tyr Gln Ala Leu Leu Gln Lys Asn Leu
    50                  55                  60
Met Tyr Leu Ala Ala Ile Ala Asp Ala Gln Pro Pro Pro Pro Thr Pro
65                  70                  75                  80
Gly Pro Ser Pro Ser Thr Ala Val Ala Ala Gln Met Ala Thr Pro His
                85                  90                  95
Ser Gly Met Gln Pro Pro Ser Tyr Phe Met Gln His Pro Gln Ala Ser

```
        100                 105                 110
Pro Ala Gly Ile Phe Ala Pro Arg Gly Pro Leu Gln Phe Gly Ser Pro
    115                 120                 125
Leu Gln Phe Gln Asp Pro Gln Gln Gln Gln Gln Ile His Gln Gln Ala
    130                 135                 140
Met Gln Gly His Met Gly Ile Arg Pro Met Gly Met Thr Asn Asn Gly
145                 150                 155                 160
Met Gln His Ala Met Gln Gln Pro Glu Thr Gly Leu Gly Gly Asn Val
                165                 170                 175
Gly Leu Arg Gly Gly Lys Gln Asp Gly Ala Asp Gly Gln Gly Lys Asp
            180                 185                 190
Asp Gly Lys
        195
```

<210> 63
<211> 672
<212> DNA
<213> Arabidopsis thaliana

<400> 63

```
atgcagcaat ctccacagat gattccgatg gttcttcctt catttccgcc caccaataat      60
atcaccaccg aacagatcca aaagtatctt gatgagaaca agaagctgat aatggcgatc     120
ttggaaaatc agaacctcgg taaacttgca gaatgtgctc agtatcaagc tcttctccag     180
aagaatttga tgtatctcgc tgcaattgcg gatgctcaac ctcagccacc agcagctaca     240
ctaacatcag gagccatgac tccccaagca atggctccta atccgtcatc aatgcagcca     300
ccaccaagct acttcatgca gcaacatcaa gctgtgggaa tggctcaaca aatacctcct     360
gggattttcc ctcctagagg tccattgcaa tttggtagcc cgcatcagtt tctggatccg     420
cagcaacagt tacatcaaca agctatgcaa gggcacatgg ggattagacc aatgggtttg     480
aataataaca acggactgca acatcaaatg caccaccatg aaactgctct tgccgcaaac     540
aatgcgggtc ctaacgatgc tagtggagga ggtaaaccgg atgggaccaa tatgagccag     600
agtggagctg atgggcaagg tggctcagcc gctagacatg gcggtggtga tgcaaaaact     660
gaaggaaaat ga                                                          672
```

<210> 64
<211> 223
<212> PRT
<213> Arabidopsis thaliana

<400> 64

```
Met Gln Gln Ser Pro Gln Met Ile Pro Met Val Leu Pro Ser Phe Pro
1               5                   10                  15
Pro Thr Asn Asn Ile Thr Thr Glu Gln Ile Gln Lys Tyr Leu Asp Glu
            20                  25                  30
Asn Lys Lys Leu Ile Met Ala Ile Leu Glu Asn Gln Asn Leu Gly Lys
        35                  40                  45
Leu Ala Glu Cys Ala Gln Tyr Gln Ala Leu Leu Gln Lys Asn Leu Met
    50                  55                  60
Tyr Leu Ala Ala Ile Ala Asp Ala Gln Pro Gln Pro Pro Ala Ala Thr
65                  70                  75                  80
Leu Thr Ser Gly Ala Met Thr Pro Gln Ala Met Ala Pro Asn Pro Ser
                85                  90                  95
Ser Met Gln Pro Pro Pro Ser Tyr Phe Met Gln Gln His Gln Ala Val
            100                 105                 110
Gly Met Ala Gln Gln Ile Pro Pro Gly Ile Phe Pro Pro Arg Gly Pro
        115                 120                 125
Leu Gln Phe Gly Ser Pro His Gln Phe Leu Asp Pro Gln Gln Gln Leu
    130                 135                 140
His Gln Gln Ala Met Gln Gly His Met Gly Ile Arg Pro Met Gly Leu
145                 150                 155                 160
```

```
Asn Asn Asn Asn Gly Leu Gln His Gln Met His His His Glu Thr Ala
                165                 170                 175
Leu Ala Ala Asn Asn Ala Gly Pro Asn Asp Ala Ser Gly Gly Gly Lys
            180                 185                 190
Pro Asp Gly Thr Asn Met Ser Gln Ser Gly Ala Asp Gly Gln Gly Gly
        195                 200                 205
Ser Ala Ala Arg His Gly Gly Gly Asp Ala Lys Thr Glu Gly Lys
    210                 215                 220

<210>  65
<211>  633
<212>  DNA
<213>  Aspergillus officinalis

<400>  65
atgcagcagc acctgatgca gatgcagccc atgatggcaa cctacggttc accgaatcag      60
gtcaccaccg atatcattca gcagtatctg gacgagaaca agcagttgat tctggctatt     120
cttgaaaacc aaaattcagg aaaagctgat gaatgtgctg agaatcaggc taagcttcag     180
aggaatctga tgtatcttgc agccattgcg gatagccagc cccaagttcc taccattgct     240
cagtatcctc ccaacgctgt tgctgctatg caatcgagtg ctcgctacat gcaacaacac     300
caagcagctc aacagatgac ccctcaatct ctcatggctg ctcgctcctc aatgctctac     360
tcacagtccc caatgtctgc actccagcag caacagcagc aagcagcaat gcatagccag     420
ctcgccatga gctccggagg caacaacagc agcaccggag gattcaccat tcttcatggt     480
gaagctagca taggaggcaa tggctcaatg aattctggtg gagtctttgg agattttgga     540
cggagcagcg gtgggaagca agagactggg agcgaagggc acgggacaga gactcctatg     600
tacctgaaag gctctgaaga agaaggaaac tga                                  633

<210>  66
<211>  210
<212>  PRT
<213>  Aspergillus officinalis

<400>  66
Met Gln Gln His Leu Met Gln Met Gln Pro Met Met Ala Thr Tyr Gly
1               5                   10                  15
Ser Pro Asn Gln Val Thr Thr Asp Ile Ile Gln Gln Tyr Leu Asp Glu
            20                  25                  30
Asn Lys Gln Leu Ile Leu Ala Ile Leu Glu Asn Gln Asn Ser Gly Lys
        35                  40                  45
Ala Asp Glu Cys Ala Glu Asn Gln Ala Lys Leu Gln Arg Asn Leu Met
    50                  55                  60
Tyr Leu Ala Ala Ile Ala Asp Ser Gln Pro Gln Val Pro Thr Ile Ala
65                  70                  75                  80
Gln Tyr Pro Pro Asn Ala Val Ala Ala Met Gln Ser Ser Ala Arg Tyr
                85                  90                  95
Met Gln Gln His Gln Ala Ala Gln Gln Met Thr Pro Gln Ser Leu Met
            100                 105                 110
Ala Ala Arg Ser Ser Met Leu Tyr Ser Gln Ser Pro Met Ser Ala Leu
        115                 120                 125
Gln Gln Gln Gln Gln Gln Ala Ala Met His Ser Gln Leu Ala Met Ser
    130                 135                 140
Ser Gly Gly Asn Asn Ser Ser Thr Gly Gly Phe Thr Ile Leu His Gly
145                 150                 155                 160
Glu Ala Ser Ile Gly Gly Asn Gly Ser Met Asn Ser Gly Gly Val Phe
                165                 170                 175
Gly Asp Phe Gly Arg Ser Ser Gly Gly Lys Gln Glu Thr Gly Ser Glu
            180                 185                 190
Gly His Gly Thr Glu Thr Pro Met Tyr Leu Lys Gly Ser Glu Glu Glu
        195                 200                 205
```

```
Gly Asn
    210

<210>  67
<211>  591
<212>  DNA
<213>  Brassica napus

<400>  67
atgcagccca tgatggctgg ttactacccc agcaatgtca cctctgatca tatccagcag      60
tacttggatg agaacaagtc tttgattctg aagatagttg agtctcaaaa ctcaggaaag     120
ctcagcgagt gtgccgagaa tcaggcaagg cttcaacgca acctcatgta cttggctgca     180
atagcagatt ctcagcctca acctccaagc gtgcatagcc agtatggatc tgctggtggt     240
gggttgattc agggagaagg agcgtcacac tatttgcagc agcaacaggc gactcaacag     300
cagcagatga ctcagcagtc tcttatggca gctcgttctt caatgatgta tcagcagcag     360
caacagcctt atgcaacgct tcagcatcag cagttgcacc atagccagct tgggatgagc     420
tctagcagcg gaggaggaag cagtggtctc catatccttc agggagaggc tggtgggttt     480
catgaatttg gccgtgggaa gccggagatg ggaagtggtg aaggcagggg tggaagctca     540
ggggatggtg gagaaacact ctacttgaag tcatcagatg atgggaactg a              591

<210>  68
<211>  203
<212>  PRT
<213>  Brassica napus

<400>  68
Met Gln Gln His Leu Met Gln Met Gln Pro Met Met Ala Gly Tyr Tyr
1               5                   10                  15
Pro Ser Asn Val Thr Ser Asp His Ile Gln Gln Tyr Leu Asp Glu Asn
            20                  25                  30
Lys Ser Leu Ile Leu Lys Ile Val Glu Ser Gln Asn Ser Gly Lys Leu
        35                  40                  45
Ser Glu Cys Ala Glu Asn Gln Ala Arg Leu Gln Arg Asn Leu Met Tyr
    50                  55                  60
Leu Ala Ala Ile Ala Asp Ser Gln Pro Gln Pro Pro Ser Val His Ser
65                  70                  75                  80
Gln Tyr Gly Ser Ala Gly Gly Gly Leu Ile Gln Gly Glu Gly Ala Ser
                85                  90                  95
His Tyr Leu Gln Gln Gln Gln Ala Thr Gln Gln Gln Gln Met Thr Gln
            100                 105                 110
Gln Ser Leu Met Ala Ala Arg Ser Ser Met Met Tyr Gln Gln Gln Gln
        115                 120                 125
Gln Pro Tyr Ala Thr Leu Gln His Gln Gln Leu His His Ser Gln Leu
    130                 135                 140
Gly Met Ser Ser Ser Ser Gly Gly Gly Ser Ser Gly Leu His Ile Leu
145                 150                 155                 160
Gln Gly Glu Ala Gly Gly Phe His Glu Phe Gly Arg Gly Lys Pro Glu
                165                 170                 175
Met Gly Ser Gly Glu Gly Arg Gly Gly Ser Ser Gly Asp Gly Gly Glu
            180                 185                 190
Thr Leu Tyr Leu Lys Ser Ser Asp Asp Gly Asn
        195                 200

<210>  69
<211>  663
<212>  DNA
<213>  Citrus sinensis

<400>  69
```

atgcaacagc acctgatgca gatgcagccc atgatggcag cttattatcc caacaacgtc 60
actactgacc acattcaaca gtatctagat gagaacaaat cattgatttt gaagattgtt 120
gagagccaga attcagggaa actgagcgag tgtgcagaga accaggcaag attgcagcgg 180
aatctcatgt acctggctgc tattgctgat gctcaacccc aaccacctag cgttcatgcc 240
cagttctctt ctggtggcat tatgcagcca ggagctcact atatgcaaca ccagcaatct 300
cagccaatga caccacagtc acttatggct gcacgctcat ccatggtgta ctctcaacag 360
caattttcag tgcttcagca acagcaagcc ttgcatggtc agcttggcat gagctctggt 420
ggtagctcag gacttcacat gctgcaaagt gagggtagta ctgcaggagg tagtggttca 480
cttggggggtg gggattccc tgattttggc cgtggctcat ctggtgaagg cttgcactca 540
aggggaatgg ggagcaagca tgatataggc agttctggat ctgctgaagg acgaggaggg 600
agctcaggaa gccaagatgg aggcgaaact ctctacttga aggggctga tgatggaaat 660
taa 663

<210> 70
<211> 219
<212> PRT
<213> Citrus sinensis

<400> 70
Met Gln Gln His Leu Met Gln Met Gln Pro Met Met Ala Ala Tyr Tyr
1 5 10 15
Pro Asn Asn Val Thr Thr Asp His Ile Gln Gln Tyr Leu Asp Glu Asn
20 25 30
Lys Ser Leu Ile Leu Lys Ile Val Glu Ser Gln Asn Ser Gly Lys Leu
35 40 45
Ser Glu Cys Ala Glu Asn Gln Ala Arg Leu Gln Arg Asn Leu Met Tyr
50 55 60
Leu Ala Ala Ile Ala Asp Ala Gln Pro Gln Pro Pro Ser Val His Ala
65 70 75 80
Gln Phe Ser Ser Gly Gly Ile Met Gln Pro Gly Ala His Tyr Met Gln
85 90 95
His Gln Gln Ser Gln Pro Met Thr Pro Gln Ser Leu Met Ala Ala Arg
100 105 110
Ser Ser Met Val Tyr Ser Gln Gln Gln Phe Ser Val Leu Gln Gln Gln
115 120 125
Gln Ala Leu His Gly Gln Leu Gly Met Ser Ser Gly Gly Ser Ser Gly
130 135 140
Leu His Met Leu Gln Ser Glu Gly Ser Thr Ala Gly Gly Ser Gly Ser
145 150 155 160
Leu Gly Gly Gly Gly Phe Pro Asp Phe Gly Arg Gly Ser Ser Gly Glu
165 170 175
Gly Leu His Ser Arg Gly Met Gly Ser Lys His Asp Ile Gly Ser Ser
180 185 190
Gly Ser Ala Glu Gly Arg Gly Gly Ser Ser Gly Ser Gln Asp Gly Gly
195 200 205
Glu Thr Leu Tyr Leu Lys Gly Ala Asp Asp Gly
210 215

<210> 71
<211> 660
<212> DNA
<213> Gossypium arboreum

<220>
<221> misc_feature
<222> (309)..(309)
<223> n is a, c, g, or t

<400> 71

```
atgcagcagc acctgatgca gatgcagccc atgatggcag cttattatcc caacaacgtc     60
actactgatc atattcaaca gtatctcgat gagaacaagt cattgatctt aaagattgtt    120
gagagccaga attctgggaa attgagtgaa tgtgctgaga accaagcaag gctgcagcga    180
aacctcatgt acctggctgc cattgcggat tctcaacccc aaccacccac cgtgcatgca    240
cagtttccat ctggtggtat catgcagcaa ggagctgggc actacatgca gcaccaacaa    300
gctcaacana tgacacaaca gtcgcttatg gctgctcggt cctcaatgtt gtattctcag    360
caaccatttt ctgcactgca acaacaacaa caacaaggct ttgcacagtc agcttggcat    420
gagctctggc gggagcacag gcctttcata tgctgcaaac tgaatctagt actgcagggg    480
gcagtgagac accttgggcc cgagggttgt cctgatttgg acggggtct tttggagagg    540
catccctggt ggcaggccaa tggccggggg aacaaccaaa aatccgggga ggccggctca    600
cctaagggcc gggaggagcc cttggggcag gggggggtga tgggggaac ctcttcttaa     660
```

```
<210>  72
<211>  219
<212>  PRT
<213>  Gossypium arboreum

<220>
<221>  UNSURE
<222>  (103)..(103)
<223>  Xaa can be any naturally occurring amino acid

<400>  72
Met Gln Gln His Leu Met Gln Met Gln Pro Met Met Ala Ala Tyr Tyr
1               5                   10                  15
Pro Asn Asn Val Thr Thr Asp His Ile Gln Gln Tyr Leu Asp Glu Asn
            20                  25                  30
Lys Ser Leu Ile Leu Lys Ile Val Glu Ser Gln Asn Ser Gly Lys Leu
        35                  40                  45
Ser Glu Cys Ala Glu Asn Gln Ala Arg Leu Gln Arg Asn Leu Met Tyr
    50                  55                  60
Leu Ala Ala Ile Ala Asp Ser Gln Pro Gln Pro Pro Thr Val His Ala
65                  70                  75                  80
Gln Phe Pro Ser Gly Gly Ile Met Gln Gln Gly Ala Gly His Tyr Met
                85                  90                  95
Gln His Gln Gln Ala Gln Xaa Met Thr Gln Gln Ser Leu Met Ala Ala
            100                 105                 110
Arg Ser Ser Met Leu Tyr Ser Gln Gln Pro Phe Ser Ala Leu Gln Gln
        115                 120                 125
Gln Gln Gln Gln Gly Phe Ala Gln Ser Ala Trp His Glu Leu Trp Arg
    130                 135                 140
Glu His Arg Pro Phe Ile Cys Cys Lys Leu Asn Leu Val Leu Gln Gly
145                 150                 155                 160
Ala Val Arg His Leu Gly Pro Glu Gly Cys Pro Asp Leu Asp Gly Gly
                165                 170                 175
Leu Leu Glu Arg His Pro Trp Trp Gln Ala Asn Gly Arg Gly Asn Asn
            180                 185                 190
Gln Lys Ser Gly Glu Ala Gly Ser Pro Lys Gly Arg Glu Glu Pro Leu
        195                 200                 205
Gly Gln Gly Gly Val Met Gly Gly Thr Ser Ser
    210                 215

<210>  73
<211>  636
<212>  DNA
<213>  Medicago truncatula

<400>  73
atgcagcagc acctgatgca gatgcagccc atgatggcag cttactatcc taacaacgtc     60
```

```
actactgatc atattcaaca gtatcttgat gagaacaagt ccttgattct caagattgtt    120
gaaagccaga acactggcaa gctcaccgag tgtgctgaga accaatcaag gcttcagaga    180
aatctcatgt acctagctgc aatagctgat tctcaacccc aaccacctac tatgcctggc    240
cagtaccctt caagtggaat gatgcagcag ggaggacact acatgcaggc tcaacaagct    300
cagcagatga cacaacaaca attaatggct gcacgttcct ctcttatgta tgctcaacag    360
cttcaacagc agcaagcctt gcaaagccaa cttggtatga attccagtgg aagtcaaggc    420
cttcacatgt tgcatagtga aggggctaat gttggaggca attcatctct aggggctggt    480
tttcctgatt ttggccgtag ctcagccggt gatggtttgc acggcagtgg taagcaagac    540
attggaagca ctgatggccg cggtggaagc tctagtggtc actctggtga tggcggcgaa    600
acactttacc tgaaatcttc tggtgatggg aattag                              636
```

```
<210>  74
<211>  211
<212>  PRT
<213>  Medicago truncatula

<400>  74
Met Gln Gln His Leu Met Gln Met Gln Pro Met Met Ala Ala Tyr Tyr
1               5                   10                  15
Pro Asn Asn Val Thr Thr Asp His Ile Gln Gln Tyr Leu Asp Glu Asn
            20                  25                  30
Lys Ser Leu Ile Leu Lys Ile Val Glu Ser Gln Asn Thr Gly Lys Leu
        35                  40                  45
Thr Glu Cys Ala Glu Asn Gln Ser Arg Leu Gln Arg Asn Leu Met Tyr
    50                  55                  60
Leu Ala Ala Ile Ala Asp Ser Gln Pro Gln Pro Pro Thr Met Pro Gly
65                  70                  75                  80
Gln Tyr Pro Ser Ser Gly Met Met Gln Gln Gly Gly His Tyr Met Gln
                85                  90                  95
Ala Gln Gln Ala Gln Gln Met Thr Gln Gln Gln Leu Met Ala Ala Arg
            100                 105                 110
Ser Ser Leu Met Tyr Ala Gln Gln Leu Gln Gln Gln Gln Ala Leu Gln
        115                 120                 125
Ser Gln Leu Gly Met Asn Ser Ser Gly Ser Gln Gly Leu His Met Leu
    130                 135                 140
His Ser Glu Gly Ala Asn Val Gly Gly Asn Ser Ser Leu Gly Ala Gly
145                 150                 155                 160
Phe Pro Asp Phe Gly Arg Ser Ser Ala Gly Asp Gly Leu His Gly Ser
                165                 170                 175
Gly Lys Gln Asp Ile Gly Ser Thr Asp Gly Arg Gly Gly Ser Ser Ser
            180                 185                 190
Gly His Ser Gly Asp Gly Gly Glu Thr Leu Tyr Leu Lys Ser Ser Gly
        195                 200                 205
Asp Gly Asn
    210

<210>  75
<211>  684
<212>  DNA
<213>  Oryza sativa

<400>  75
atgcagcagc aacacctgat gcagatgaac cagggcatga tgggggata tgcttcccct      60
accaccgtca ccactgatct cattcagcag tatctggatg agaacaagca gctgatcctg    120
gccatccttg acaaccagaa caatgggaag gtggaagagt gcgctcggaa ccaagctaag    180
ctccagcaca atctcatgta cctcgccgcc atcgccgaca gccagccgcc gcagacggcc    240
gccatgtccc agtatccgtc gaacctgatg atgcagtccg ggcgaggta catgccgcag     300
cagtcggcgc agatgatggc gccgcagtcg ctgatggcgg cgaggtcttc gatgatgtac    360
gcgcagccgg cgctgtcgcc gctccagcag cagcagcagc agcaggcggc ggcggcgcac    420
```

gggcagctgg gcatgggctc gggggcacc accagcgggt tcagcatcct ccacggcgag 480
gccagcatgg gcggcggcgg cggcggcggt ggcgccggta acagcatgat gaacgccggc 540
gtgttctccg acttcggacg cggcggcggc ggcggcggca aggaggggtc cacctcgctg 600
tccgtcgacg tccggggcgc caactccggc gcccagagcg gcgacgggga gtacctcaag 660
ggcaccgagg aggaaggcag ctag 684

<210> 76
<211> 227
<212> PRT
<213> Oryza sativa

<400> 76
Met Gln Gln Gln His Leu Met Gln Met Asn Gln Gly Met Met Gly Gly
1 5 10 15
Tyr Ala Ser Pro Thr Thr Val Thr Thr Asp Leu Ile Gln Gln Tyr Leu
20 25 30
Asp Glu Asn Lys Gln Leu Ile Leu Ala Ile Leu Asp Asn Gln Asn Asn
35 40 45
Gly Lys Val Glu Glu Cys Ala Arg Asn Gln Ala Lys Leu Gln His Asn
50 55 60
Leu Met Tyr Leu Ala Ala Ile Ala Asp Ser Gln Pro Pro Gln Thr Ala
65 70 75 80
Ala Met Ser Gln Tyr Pro Ser Asn Leu Met Met Gln Ser Gly Ala Arg
85 90 95
Tyr Met Pro Gln Gln Ser Ala Gln Met Met Ala Pro Gln Ser Leu Met
100 105 110
Ala Ala Arg Ser Ser Met Met Tyr Ala Gln Pro Ala Leu Ser Pro Leu
115 120 125
Gln Gln Gln Gln Gln Gln Gln Ala Ala Ala Ala His Gly Gln Leu Gly
130 135 140
Met Gly Ser Gly Gly Thr Thr Ser Gly Phe Ser Ile Leu His Gly Glu
145 150 155 160
Ala Ser Met Gly Gly Gly Gly Gly Gly Gly Gly Ala Gly Asn Ser Met
165 170 175
Met Asn Ala Gly Val Phe Ser Asp Phe Gly Arg Gly Gly Gly Gly Gly
180 185 190
Gly Lys Glu Gly Ser Thr Ser Leu Ser Val Asp Val Arg Gly Ala Asn
195 200 205
Ser Gly Ala Gln Ser Gly Asp Gly Glu Tyr Leu Lys Gly Thr Glu Glu
210 215 220
Glu Gly Ser
225

<210> 77
<211> 558
<212> DNA
<213> Oryza sativa

<400> 77
atgcagcagc agccgatgcc gatgcccgcg caggcgccgc cgacggccgg aatcaccacc 60
gagcagatcc aaaagtatct ggatgaaaac aagcagctta ttttggctat tttggaaaat 120
cagaatctgg gaaagttggc agaatgtgct cagtatcaag cgcagcttca gaagaatctc 180
ttgtacttgg ctgcaattgc tgatactcaa ccgcagacca ctataagccg tccccagatg 240
gtgccgcatg gtgcatcgcc ggggttaggg ggcaataca tgtcgcaggt gccaatgttc 300
ccccccagga cccctctaac gccccagcag atgcaggagc agcagctgca gcaacagcaa 360
gcccagctgc tctcgttcgg cggtcagatg gttatgaggc ctggcgttgt gaatggcatt 420
cctcagcttc tgcaaggcga aatgcaccgc ggagcagatc accagaacgc tggcggggcc 480
acctcggagc cttccgagag ccacaggagc accggcaccg aaaatgacgg tggaagcgac 540
ttcggcgatc aatcctaa 558

<210> 78
<211> 185
<212> PRT
<213> Oryza sativa

<400> 78
Met Gln Gln Gln Pro Met Pro Met Pro Ala Gln Ala Pro Pro Thr Ala
1               5                   10                  15
Gly Ile Thr Thr Glu Gln Ile Gln Lys Tyr Leu Asp Glu Asn Lys Gln
            20                  25                  30
Leu Ile Leu Ala Ile Leu Glu Asn Gln Asn Leu Gly Lys Leu Ala Glu
        35                  40                  45
Cys Ala Gln Tyr Gln Ala Gln Leu Gln Lys Asn Leu Leu Tyr Leu Ala
    50                  55                  60
Ala Ile Ala Asp Thr Gln Pro Gln Thr Thr Ile Ser Arg Pro Gln Met
65                  70                  75                  80
Val Pro His Gly Ala Ser Pro Gly Leu Gly Gly Gln Tyr Met Ser Gln
                85                  90                  95
Val Pro Met Phe Pro Pro Arg Thr Pro Leu Thr Pro Gln Gln Met Gln
            100                 105                 110
Glu Gln Gln Leu Gln Gln Gln Gln Ala Gln Leu Leu Ser Phe Gly Gly
        115                 120                 125
Gln Met Val Met Arg Pro Gly Val Val Asn Gly Ile Pro Gln Leu Leu
    130                 135                 140
Gln Gly Glu Met His Arg Gly Ala Asp His Gln Asn Ala Gly Gly Ala
145                 150                 155                 160
Thr Ser Glu Pro Ser Glu Ser His Arg Ser Thr Gly Thr Glu Asn Asp
                165                 170                 175
Gly Gly Ser Asp Phe Gly Asp Gln Ser
            180                 185

<210> 79
<211> 618
<212> DNA
<213> Oryza sativa

<400> 79
atgcagcagc agatggccat gccggcgggg gccgccgccg ccgcggtgcc gccggcggcc 60
ggcatcacca ccgagcagat ccaaaagtat ttggatgaaa ataaacagct aattttggcc 120
atcctggaaa atcaaaacct agggaagttg gctgaatgtg ctcagtacca agctcagctt 180
caaaagaatc tcttgtatct ggctgccatt gcagatgccc aaccacctca gaatccagga 240
agtcgccctc agatgatgca gcctggtgct accccaggtg ctgggcatta catgtcccaa 300
gtaccgatgt tccctccaag aactccctta accccacaac agatgcaaga gcagcagcag 360
cagcaactcc agcaacagca agctcaggct ctagccttcc ccggccagat gctaatgaga 420
ccaggtactg tcaatggcat gcaatctatc ccagttgctg accctgctcg cgcagccgat 480
cttcagacgg cagcaccggg ctcggtagat ggccgaggaa acaagcagga tgcaacctcg 540
gagccttccg ggaccgagag ccacaagagt gcgggagcag ataacgacgc aggcggtgac 600
atagcggaga agtcctga 618

<210> 80
<211> 205
<212> PRT
<213> Oryza sativa

<400> 80
Met Gln Gln Gln Met Ala Met Pro Ala Gly Ala Ala Ala Ala Ala Val
1               5                   10                  15
Pro Pro Ala Ala Gly Ile Thr Thr Glu Gln Ile Gln Lys Tyr Leu Asp

```
                20                  25                  30
Glu Asn Lys Gln Leu Ile Leu Ala Ile Leu Glu Asn Gln Asn Leu Gly
        35                  40                  45
Lys Leu Ala Glu Cys Ala Gln Tyr Gln Ala Gln Leu Gln Lys Asn Leu
    50                  55                  60
Leu Tyr Leu Ala Ala Ile Ala Asp Ala Gln Pro Pro Gln Asn Pro Gly
65                  70                  75                  80
Ser Arg Pro Gln Met Met Gln Pro Gly Ala Thr Pro Gly Ala Gly His
                85                  90                  95
Tyr Met Ser Gln Val Pro Met Phe Pro Pro Arg Thr Pro Leu Thr Pro
            100                 105                 110
Gln Gln Met Gln Glu Gln Gln Gln Gln Gln Leu Gln Gln Gln Gln Ala
        115                 120                 125
Gln Ala Leu Ala Phe Pro Gly Gln Met Leu Met Arg Pro Gly Thr Val
    130                 135                 140
Asn Gly Met Gln Ser Ile Pro Val Ala Asp Pro Ala Arg Ala Ala Asp
145                 150                 155                 160
Leu Gln Thr Ala Ala Pro Gly Ser Val Asp Gly Arg Gly Asn Lys Gln
                165                 170                 175
Asp Ala Thr Ser Glu Pro Ser Gly Thr Glu Ser His Lys Ser Ala Gly
            180                 185                 190
Ala Asp Asn Asp Ala Gly Gly Asp Ile Ala Glu Lys Ser
        195                 200                 205
```

<210> 81
<211> 540
<212> DNA
<213> Solanum tuberosum

<400> 81

```
atgcagcagc agcacctgat gcagatgcag cccatgatgg cagcctatta tcccaacaat     60
gtcactactg atcatattca acagttcctg gatgagaaca aatcacttat tctgaagatt    120
gttgagagcc agaactctgg gaaaataagt gaatgtgcag agtcccaagc taaacttcag    180
agaaatctta tgtaccttgc agctattgct gattcacagc cccagcctcc tagtatgcat    240
tcacagttag cttctggtgg gatgatgcag ggaggggcac attatatgca gcaacaacaa    300
gctcaacaac tcacaacgca atcgcttatg gctgcagcaa gatcctcctc ctcaatgctc    360
tatggacaac aacaacaaca acaacaacaa caactatcat cattgcaaca acagcaagca    420
gcctttcata gccagcaact cggaatgagc agctctggtg gaggaagcag tagtggactt    480
cacatgctac aaagcgaaaa cactcatagt gctagcactg gtggtgggtg gtttccctga    540
```

<210> 82
<211> 179
<212> PRT
<213> Solanum tuberosum

<400> 82

```
Met Gln Gln Gln His Leu Met Gln Met Gln Pro Met Met Ala Ala Tyr
1               5                   10                  15
Tyr Pro Asn Asn Val Thr Thr Asp His Ile Gln Gln Phe Leu Asp Glu
            20                  25                  30
Asn Lys Ser Leu Ile Leu Lys Ile Val Glu Ser Gln Asn Ser Gly Lys
        35                  40                  45
Ile Ser Glu Cys Ala Glu Ser Gln Ala Lys Leu Gln Arg Asn Leu Met
    50                  55                  60
Tyr Leu Ala Ala Ile Ala Asp Ser Gln Pro Gln Pro Pro Ser Met His
65                  70                  75                  80
Ser Gln Leu Ala Ser Gly Gly Met Met Gln Gly Gly Ala His Tyr Met
                85                  90                  95
Gln Gln Gln Gln Ala Gln Gln Leu Thr Thr Gln Ser Leu Met Ala Ala
```

```
                100                 105                 110
Ala Arg Ser Ser Ser Ser Met Leu Tyr Gly Gln Gln Gln Gln Gln Gln
            115                 120                 125
Gln Gln Gln Leu Ser Ser Leu Gln Gln Gln Gln Ala Ala Phe His Ser
    130                 135                 140
Gln Gln Leu Gly Met Ser Ser Ser Gly Gly Gly Ser Ser Ser Gly Leu
145                 150                 155                 160
His Met Leu Gln Ser Glu Asn Thr His Ser Ala Ser Thr Gly Gly Gly
                165                 170                 175
Trp Phe Pro
```

<210> 83
<211> 684
<212> DNA
<213> Zea mays

<400> 83

```
atgcagcagc aacacctgat gcagatgaac cagaacatga tgggggggcta cacctctcct    60
gccgccgtga ccaccgatct catccagcag cacctggacg agaacaagca gctgatcctg   120
gccatcctcg acaaccagaa caatggcaag gcggaggagt gcgaacggca ccaagctaag   180
ctccagcaca acctcatgta cctggccgcc atcgctgaca gccagccgcc acagaccgcg   240
ccactatcac agtacccgtc caacctgatg atgcagccgg gccctcggta catgccaccg   300
cagtccgggc agatgatgaa cccgcagtcg ctgatggcgg cgcggtcctc catgatgtac   360
gcgcacccgt ccctgtcgcc actccagcag cagcaggcgg cgcacggaca gctgggtatg   420
gctccagggg gcggcggtgg cggcacgacc agcgggttca gcatcctcca cggcgaggcc   480
agcatgggcg gtggtggtgc tggcgcaggc gccggcaaca acatgatgaa cgccggcatg   540
ttctcgggct ttggccgcag cggcagtggc gccaaggaag ggtcgacctc tctgtcggtt   600
gacgtccggg gtggaaccag ctccggcgcg cagagcgggg acggcgagta cctcaaagtc   660
ggcaccgagg aagaaggcag ttag                                          684
```

<210> 84
<211> 227
<212> PRT
<213> Zea mays

<400> 84

```
Met Gln Gln Gln His Leu Met Gln Met Asn Gln Asn Met Met Gly Gly
1               5                   10                  15
Tyr Thr Ser Pro Ala Ala Val Thr Thr Asp Leu Ile Gln Gln His Leu
            20                  25                  30
Asp Glu Asn Lys Gln Leu Ile Leu Ala Ile Leu Asp Asn Gln Asn Asn
        35                  40                  45
Gly Lys Ala Glu Glu Cys Glu Arg His Gln Ala Lys Leu Gln His Asn
    50                  55                  60
Leu Met Tyr Leu Ala Ala Ile Ala Asp Ser Gln Pro Pro Gln Thr Ala
65                  70                  75                  80
Pro Leu Ser Gln Tyr Pro Ser Asn Leu Met Met Gln Pro Gly Pro Arg
                85                  90                  95
Tyr Met Pro Pro Gln Ser Gly Gln Met Met Asn Pro Gln Ser Leu Met
            100                 105                 110
Ala Ala Arg Ser Ser Met Met Tyr Ala His Pro Ser Leu Ser Pro Leu
        115                 120                 125
Gln Gln Gln Gln Ala Ala His Gly Gln Leu Gly Met Ala Pro Gly Gly
    130                 135                 140
Gly Gly Gly Gly Thr Thr Ser Gly Phe Ser Ile Leu His Gly Glu Ala
145                 150                 155                 160
Ser Met Gly Gly Gly Gly Ala Gly Ala Gly Ala Gly Asn Asn Met Met
                165                 170                 175
```

```
Asn Ala Gly Met Phe Ser Gly Phe Gly Arg Ser Gly Ser Gly Ala Lys
            180                 185                 190
Glu Gly Ser Thr Ser Leu Ser Val Asp Val Arg Gly Gly Thr Ser Ser
        195                 200                 205
Gly Ala Gln Ser Gly Asp Gly Glu Tyr Leu Lys Val Gly Thr Glu Glu
    210                 215                 220
Glu Gly Ser
225
```

<210> 85
<211> 549
<212> DNA
<213> Zea mays

<400> 85

```
atgcagcagc cgatgcacat gcagccacag gcgccggcga taaccccagc tgccggaatc      60
agcacggagc agatccaaaa gtatctggat gagaataagc agcttatttt ggctattttg     120
gaaaatcaga acctaggaaa attggcagaa tgtgctcagt atcaatcaca acttcagaag     180
aacctcttgt atctcgctgc aatcgcagat gctcaaccgc agactgctgt aagccgccct     240
cagatggcgc cgcctggtgg atcgcctgga gtagggcagt acatgtcaca ggtgcctatg     300
ttcccaccga ggacacctct tacaccccag cagatgcagg agcagcagct tcagcagcag     360
caggctcagt tgctaaactt cagtggccaa atggttgcta gaccaggcat ggtcaacggc     420
atggctcagt ccatgcaagc tcagctacca ccgggtgtga acaagcagga tgctggtggg     480
gtcgcctctg agccctcggg caccgagagc cacaggagca ctggtggtga cgatggtgga     540
agcgactag                                                             549
```

<210> 86
<211> 182
<212> PRT
<213> Zea mays

<400> 86

```
Met Gln Gln Pro Met His Met Gln Pro Gln Ala Pro Ala Ile Thr Pro
1               5                   10                  15
Ala Ala Gly Ile Ser Thr Glu Gln Ile Gln Lys Tyr Leu Asp Glu Asn
            20                  25                  30
Lys Gln Leu Ile Leu Ala Ile Leu Glu Asn Gln Asn Leu Gly Lys Leu
        35                  40                  45
Ala Glu Cys Ala Gln Tyr Gln Ser Gln Leu Gln Lys Asn Leu Leu Tyr
    50                  55                  60
Leu Ala Ala Ile Ala Asp Ala Gln Pro Gln Thr Ala Val Ser Arg Pro
65                  70                  75                  80
Gln Met Ala Pro Pro Gly Gly Ser Pro Gly Val Gly Gln Tyr Met Ser
                85                  90                  95
Gln Val Pro Met Phe Pro Pro Arg Thr Pro Leu Thr Pro Gln Gln Met
            100                 105                 110
Gln Glu Gln Gln Leu Gln Gln Gln Gln Ala Gln Leu Leu Asn Phe Ser
        115                 120                 125
Gly Gln Met Val Ala Arg Pro Gly Met Val Asn Gly Met Ala Gln Ser
    130                 135                 140
Met Gln Ala Gln Leu Pro Pro Gly Val Asn Lys Gln Asp Ala Gly Gly
145                 150                 155                 160
Val Ala Ser Glu Pro Ser Gly Thr Glu Ser His Arg Ser Thr Gly Gly
                165                 170                 175
Asp Asp Gly Gly Ser Asp
            180
```

<210> 87
<211> 1173

<212> DNA
<213> Homo sapiens

<400> 87
atgggcggca acatgtctgt ggctttcgcg gccccgaggc agcgaggcaa gggggagatc 60
actcccgctg cgattcagaa gatgttggat gacaataacc atcttattca gtgtataatg 120
gactctcaga ataaaggaaa gacctcagag tgttctcagt atcagcagat gttgcacaca 180
aacttggtat accttgctac aatagcagat tctaatcaaa atatgcagtc tcttttacca 240
gcaccaccca cacagaatat gcctatgggt cctggaggga tgaatcagag cggccctccc 300
ccacctccac gctctcacaa catgccttca gatggaatgg taggtggggg tcctcctgca 360
ccgcacatgc agaaccagat gaacggccag atgcctgggc ctaaccatat gcctatgcag 420
ggacctggac ccaatcaact caatatgaca aacagttcca tgaatatgcc ttcaagtagc 480
catggatcca tgggaggtta caaccattct gtgccatcat cacagagcat gccagtacag 540
aatcagatga caatgagtca gggacaacca atgggaaact atggtcccag accaaatatg 600
agtatgcagc caaaccaagg tccaatgatg catcagcagc ctccttctca gcaatacaat 660
atgccacagg gaggcggaca gcattaccaa ggacagcagc cacctatggg aatgatgggt 720
caagttaacc aaggcaatca tatgatgggt cagagacaga ttcctcccta tagacctcct 780
caacagggcc caccacagca gtactcaggc caggaagact attacgggga ccaatacagt 840
catggtggac aaggtcctcc agaaggcatg aaccagcaat attaccctga tggaaattca 900
cagtatggcc aacagcaaga tgcataccag ggaccacctc cacaacaggg atatccaccc 960
cagcagcagc agtacccagg gcagcaaggt tacccaggac agcagcaggg ctacggtcct 1020
tcacagggtg gtccaggtcc tcagtatcct aactacccac aggacaagg tcagcagtat 1080
ggaggatata gaccaacaca gcctggacca ccacagccac cccagcagag gccttatgga 1140
tatgaccagg gacagtatgg aaattaccag cag 1173

<210> 88
<211> 391
<212> PRT
<213> Homo sapiens

<400> 88
Met Gly Gly Asn Met Ser Val Ala Phe Ala Ala Pro Arg Gln Arg Gly
1 5 10 15
Lys Gly Glu Ile Thr Pro Ala Ala Ile Gln Lys Met Leu Asp Asp Asn
20 25 30
Asn His Leu Ile Gln Cys Ile Met Asp Ser Gln Asn Lys Gly Lys Thr
35 40 45
Ser Glu Cys Ser Gln Tyr Gln Gln Met Leu His Thr Asn Leu Val Tyr
50 55 60
Leu Ala Thr Ile Ala Asp Ser Asn Gln Asn Met Gln Ser Leu Leu Pro
65 70 75 80
Ala Pro Pro Thr Gln Asn Met Pro Met Gly Pro Gly Gly Met Asn Gln
85 90 95
Ser Gly Pro Pro Pro Pro Pro Arg Ser His Asn Met Pro Ser Asp Gly
100 105 110
Met Val Gly Gly Gly Pro Pro Ala Pro His Met Gln Asn Gln Met Asn
115 120 125
Gly Gln Met Pro Gly Pro Asn His Met Pro Met Gln Gly Pro Gly Pro
130 135 140
Asn Gln Leu Asn Met Thr Asn Ser Ser Met Asn Met Pro Ser Ser Ser
145 150 155 160
His Gly Ser Met Gly Gly Tyr Asn His Ser Val Pro Ser Ser Gln Ser
165 170 175
Met Pro Val Gln Asn Gln Met Thr Met Ser Gln Gly Gln Pro Met Gly
180 185 190
Asn Tyr Gly Pro Arg Pro Asn Met Ser Met Gln Pro Asn Gln Gly Pro
195 200 205
Met Met His Gln Gln Pro Pro Ser Gln Gln Tyr Asn Met Pro Gln Gly
210 215 220

Gly Gly Gln His Tyr Gln Gly Gln Gln Pro Pro Met Gly Met Met Gly
225 230 235 240
Gln Val Asn Gln Gly Asn His Met Met Gly Gln Arg Gln Ile Pro Pro
245 250 255
Tyr Arg Pro Pro Gln Gln Gly Pro Pro Gln Gln Tyr Ser Gly Gln Glu
260 265 270
Asp Tyr Tyr Gly Asp Gln Tyr Ser His Gly Gly Gln Gly Pro Pro Glu
275 280 285
Gly Met Asn Gln Gln Tyr Tyr Pro Asp Gly Asn Ser Gln Tyr Gly Gln
290 295 300
Gln Gln Asp Ala Tyr Gln Gly Pro Pro Pro Gln Gln Gly Tyr Pro Pro
305 310 315 320
Gln Gln Gln Gln Tyr Pro Gly Gln Gln Gly Tyr Pro Gly Gln Gln Gln
325 330 335
Gly Tyr Gly Pro Ser Gln Gly Gly Pro Gly Pro Gln Tyr Pro Asn Tyr
340 345 350
Pro Gln Gly Gln Gly Gln Gln Tyr Gly Gly Tyr Arg Pro Thr Gln Pro
355 360 365
Gly Pro Pro Gln Pro Pro Gln Gln Arg Pro Tyr Gly Tyr Asp Gln Gly
370 375 380
Gln Tyr Gly Asn Tyr Gln Gln
385 390

<210> 89
<211> 627
<212> DNA
<213> Allium cepa

<400> 89
atgcagcagc cgcagccagc gatgggaacc atgggctcgg tgccacctac tagcatcacc 60
accgaacaga ttcaaaggta cttggatgag aacaaacagt taatattggc aattttggat 120
aatcaaaatt taggaagact gaatgagtgt gctcaatatc aagctcagct tcaaaagaat 180
ctgctttacc tggcagcaat agctgatgct cagcctcagt ctcctgcggt gcgtctgcag 240
atgatgcctc aaggtgcagc tgccacgcct caagctggaa accaatttat gcagcagcag 300
agccctaatt tccctcccaa aacaggaatg caatttactc ctcaacaagt acaagaattg 360
cagcagcaac agctacaaca tcagccacat atgatgcctc catttcaagg tcaaatgggt 420
atgagaccta tgaatggaat gcaggcagca atgcatgcag attcatctct tgcttataac 480
actaacaata agcaagatgc aggaaacgca gcttatgaaa atactgctgc caacacagat 540
ggttccattc aaaagaaaac agcaaatgat gatttagacc cttctgcagc aaaccctaga 600
aggtctgaag atgccaaatc atcatga 627

<210> 90
<211> 208
<212> PRT
<213> Allium cepa

<400> 90
Met Gln Gln Pro Gln Pro Ala Met Gly Thr Met Gly Ser Val Pro Pro
1 5 10 15
Thr Ser Ile Thr Thr Glu Gln Ile Gln Arg Tyr Leu Asp Glu Asn Lys
20 25 30
Gln Leu Ile Leu Ala Ile Leu Asp Asn Gln Asn Leu Gly Arg Leu Asn
35 40 45
Glu Cys Ala Gln Tyr Gln Ala Gln Leu Gln Lys Asn Leu Leu Tyr Leu
50 55 60
Ala Ala Ile Ala Asp Ala Gln Pro Gln Ser Pro Ala Val Arg Leu Gln
65 70 75 80
Met Met Pro Gln Gly Ala Ala Ala Thr Pro Gln Ala Gly Asn Gln Phe
85 90 95

```
Met Gln Gln Gln Ser Pro Asn Phe Pro Pro Lys Thr Gly Met Gln Phe
                100                 105                 110
Thr Pro Gln Gln Val Gln Glu Leu Gln Gln Gln Gln Leu Gln His Gln
        115                 120                 125
Pro His Met Met Pro Pro Phe Gln Gly Gln Met Gly Met Arg Pro Met
    130                 135                 140
Asn Gly Met Gln Ala Ala Met His Ala Asp Ser Ser Leu Ala Tyr Asn
145                 150                 155                 160
Thr Asn Asn Lys Gln Asp Ala Gly Asn Ala Ala Tyr Glu Asn Thr Ala
                165                 170                 175
Ala Asn Thr Asp Gly Ser Ile Gln Lys Lys Thr Ala Asn Asp Asp Leu
            180                 185                 190
Asp Pro Ser Ala Ala Asn Pro Arg Arg Ser Glu Asp Ala Lys Ser Ser
        195                 200                 205
```

<210> 91
<211> 633
<212> DNA
<213> Aquilegia formosa x Aquilegia pubescens

<400> 91

```
atgcaacaca tgcagatgca gcccatgatg ccaccttata gtgccaacag cgtcactact      60
gatcatatcc aacagtactt ggatgaaaat aaggcgttga ttctgaagat acttgagaac     120
caaaattcgg gaaaagttag tgaatgtgca gagaaccaag caagacttca acgaaatctt     180
atgtatctgg ctgcaattgc tgattctcaa ccacagcctc ccaatatgca tgctcagtac     240
tctaatgcgg gtataccacc tggtgcacat tacctacaac accaacaggc ccaacagatg     300
acacaacagt cgctcatggc tgctcgatca aatatgctgt atgctcagcc aatcacagga     360
atgcagcaac agcaagcaat gcatagccag cttggcatga gctctggtgg taacagtgga     420
ctccacatga tgcacaatga gggcagcatg ggaggtagtg ggcacttgg aagctattct     480
gattatggcc gtggcagtgg tggtggagta actatcgcta gcaaacaaga tggtggaagt     540
ggttctggtg aaggacgagg tggaaactct ggaggccaaa gtgcagatgg aggtgaatct     600
ctttacctga aaaacagtga cgaagggaac taa                                  633
```

<210> 92
<211> 210
<212> PRT
<213> Aquilegia formosa x Aquilegia pubescens

<400> 92

```
Met Gln His Met Gln Met Gln Pro Met Met Pro Pro Tyr Ser Ala Asn
1               5                   10                  15
Ser Val Thr Thr Asp His Ile Gln Gln Tyr Leu Asp Glu Asn Lys Ala
            20                  25                  30
Leu Ile Leu Lys Ile Leu Glu Asn Gln Asn Ser Gly Lys Val Ser Glu
        35                  40                  45
Cys Ala Glu Asn Gln Ala Arg Leu Gln Arg Asn Leu Met Tyr Leu Ala
    50                  55                  60
Ala Ile Ala Asp Ser Gln Pro Gln Pro Pro Asn Met His Ala Gln Tyr
65                  70                  75                  80
Ser Asn Ala Gly Ile Pro Pro Gly Ala His Tyr Leu Gln His Gln Gln
                85                  90                  95
Ala Gln Gln Met Thr Gln Gln Ser Leu Met Ala Ala Arg Ser Asn Met
            100                 105                 110
Leu Tyr Ala Gln Pro Ile Thr Gly Met Gln Gln Gln Gln Ala Met His
        115                 120                 125
Ser Gln Leu Gly Met Ser Ser Gly Gly Asn Ser Gly Leu His Met Met
    130                 135                 140
His Asn Glu Gly Ser Met Gly Gly Ser Gly Ala Leu Gly Ser Tyr Ser
145                 150                 155                 160
```

```
Asp Tyr Gly Arg Gly Ser Gly Gly Gly Val Thr Ile Ala Ser Lys Gln
                165                 170                 175
Asp Gly Gly Ser Gly Ser Gly Glu Gly Arg Gly Gly Asn Ser Gly Gly
            180                 185                 190
Gln Ser Ala Asp Gly Gly Glu Ser Leu Tyr Leu Lys Asn Ser Asp Glu
        195                 200                 205
Gly Asn
    210
```

<210> 93
<211> 615
<212> DNA
<213> Brachypodium distachyon

<400> 93

```
atgcagcagg cgatgtccat gtccccgggg tcggccggcg cggtgccgcc tccggccggc      60
atcaccacag agcagatcca aaagtatttg gatgaaaata agcaacttat tttggccatc     120
ctggaaaatc agaacctagg aaagttgact gaatgtgctc agtatcaagc tcaacttcag     180
aagaatctct tgtatctggc tgccattgcg gatgcccaac caccacagaa ccctggaagt     240
cgcccccaga tggtgcagcc tggtggtatg ccaggtgcag ggcattacat gtcgcaagta     300
ccaatgttcc ctccaagaac ccctttaacc ccacaacaga tgcaagagca acagcaccag     360
cagcttcagc agcagcaagc acaggctctt gctttcccca gccagatggt catgagacca     420
ggtactgtga acggcatgca gcctatgcaa gctgatctcc aagcagcagc agcagcacct     480
ggcctggcag acagccgagg aagtaagcag gacgcagcgg tagctggggc catctcggaa     540
ccttctggca ccgagagtca caagagtaca ggagcggatc atgaggcagg tggcgatgta     600
gctgagcaat cctaa                                                      615
```

<210> 94
<211> 204
<212> PRT
<213> Brachypodium distachyon

<400> 94

```
Met Gln Gln Ala Met Ser Met Ser Pro Gly Ser Ala Gly Ala Val Pro
1               5                   10                  15
Pro Pro Ala Gly Ile Thr Thr Glu Gln Ile Gln Lys Tyr Leu Asp Glu
            20                  25                  30
Asn Lys Gln Leu Ile Leu Ala Ile Leu Glu Asn Gln Asn Leu Gly Lys
        35                  40                  45
Leu Thr Glu Cys Ala Gln Tyr Gln Ala Gln Leu Gln Lys Asn Leu Leu
    50                  55                  60
Tyr Leu Ala Ala Ile Ala Asp Ala Gln Pro Pro Gln Asn Pro Gly Ser
65                  70                  75                  80
Arg Pro Gln Met Val Gln Pro Gly Gly Met Pro Gly Ala Gly His Tyr
                85                  90                  95
Met Ser Gln Val Pro Met Phe Pro Pro Arg Thr Pro Leu Thr Pro Gln
            100                 105                 110
Gln Met Gln Glu Gln Gln His Gln Gln Leu Gln Gln Gln Gln Ala Gln
        115                 120                 125
Ala Leu Ala Phe Pro Ser Gln Met Val Met Arg Pro Gly Thr Val Asn
    130                 135                 140
Gly Met Gln Pro Met Gln Ala Asp Leu Gln Ala Ala Ala Ala Ala Pro
145                 150                 155                 160
Gly Leu Ala Asp Ser Arg Gly Ser Lys Gln Asp Ala Ala Val Ala Gly
                165                 170                 175
Ala Ile Ser Glu Pro Ser Gly Thr Glu Ser His Lys Ser Thr Gly Ala
            180                 185                 190
Asp His Glu Ala Gly Gly Asp Val Ala Glu Gln Ser
        195                 200
```

<210> 95
<211> 636
<212> DNA
<213> Brassica napus

<400> 95
atgcagcagc agcagcagca gcagcagcag cctccgcaaa tgtttccgat ggctccttcg 60
atgccgccaa ctaacatcac caccgaacag atccaaaagt accttgagga gaacaagaag 120
ctgataatgg caatcatgga aaatcagaat cttggcaagc ttgcagagtg tgcacagtac 180
caagctcttc tccagaagaa cttaatgtac ctcgctgcta ttgctgatgc tcaacctcct 240
ccatctaccg ctggagctac accaccacca gctatggctt cccagatggg ggcaccgcat 300
cctgggatgc aaccgccgag ctactttatg caacacccac aagcttcagg gatggctcaa 360
caagcaccac ccgctggtat cttccctccg agaggtcctt tgcagtttgg tagcccacac 420
cagcttcagg atccgcaaca gcagcatatg catcaacagg ctatgcaagg acacatgggg 480
atgcgaccaa tgggtatcaa caacaacaat gggatgcagc atcagatgca gcaacaacaa 540
ccagaaacct ctcttggagg aagcgctgca aacgtggggc ttagaggtgg aaagcaagat 600
ggagcagatg gacaaggaaa agatgatggc aaatga 636

<210> 96
<211> 203
<212> PRT
<213> Brassica napus

<400> 96
Met Gln Gln His Leu Met Gln Met Gln Pro Met Met Ala Gly Tyr Tyr
1 5 10 15
Pro Ser Asn Val Thr Ser Asp His Ile Gln Gln Tyr Leu Asp Glu Asn
20 25 30
Lys Ser Leu Ile Leu Lys Ile Val Glu Ser Gln Asn Ser Gly Lys Leu
35 40 45
Ser Glu Cys Ala Glu Asn Gln Ala Arg Leu Gln Arg Asn Leu Met Tyr
50 55 60
Leu Ala Ala Ile Ala Asp Ser Gln Pro Gln Pro Pro Ser Val His Ser
65 70 75 80
Gln Tyr Gly Ser Ala Gly Gly Gly Leu Ile Gln Gly Glu Gly Ala Ser
85 90 95
His Tyr Leu Gln Gln Gln Gln Ala Thr Gln Gln Gln Gln Met Thr Gln
100 105 110
Gln Ser Leu Met Ala Ala Arg Ser Ser Met Met Tyr Gln Gln Gln Gln
115 120 125
Gln Pro Tyr Ala Thr Leu Gln His Gln Gln Leu His His Ser Gln Leu
130 135 140
Gly Met Ser Ser Ser Ser Gly Gly Gly Ser Ser Gly Leu His Ile Leu
145 150 155 160
Gln Gly Glu Ala Gly Gly Phe His Glu Phe Gly Arg Gly Lys Pro Glu
165 170 175
Met Gly Ser Gly Glu Gly Arg Gly Gly Ser Ser Gly Asp Gly Gly Glu
180 185 190
Thr Leu Tyr Leu Lys Ser Ser Asp Asp Gly Asn
195 200

<210> 97
<211> 636
<212> DNA
<213> Citrus sinensis

<400> 97
atgcagcagc caccgcaaat gatccctgtt atgccttcat ttccacccac caacatcacc 60

acagagcaga ttcaaaagta ccttgatgag aacaaaaagt tgattttggc aattttggac 120
aatcaaaatc ttggaaagct tacagaatgt gcccactatc aagctcagct tcaaaagaat 180
ttaatgtatt tagctgcaat tgctgatgca caaccacaag caccaacaat gcctcctcag 240
atggctccac atcctgcaat gcaagctagt gggtattaca tgcaacatcc tcaggcggca 300
gcaatggctc agcaacaagg aatctttccc caaaagatgc cattacaatt caataaccct 360
catcaactac aggatcctca acagcagcta caccaacatc aagccatgca agcacaaatg 420
ggaatgagac cgggtgccac taacaatggt atgcatccca tgcatgctga aagctctctt 480
ggaggtggca gcagtggagg accccccttca gcatcaggcc caggtgacat acgtggtgga 540
aataagcaag atgcctcgga ggctgggact actggtgctg atggccaggg cagttcggct 600
ggtgggcatg gtggggatgg agaggaggca aagtga 636

<210> 98
<211> 211
<212> PRT
<213> Citrus sinensis

<400> 98
Met Gln Gln Pro Pro Gln Met Ile Pro Val Met Pro Ser Phe Pro Pro
1 5 10 15
Thr Asn Ile Thr Thr Glu Gln Ile Gln Lys Tyr Leu Asp Glu Asn Lys
20 25 30
Lys Leu Ile Leu Ala Ile Leu Asp Asn Gln Asn Leu Gly Lys Leu Thr
35 40 45
Glu Cys Ala His Tyr Gln Ala Gln Leu Gln Lys Asn Leu Met Tyr Leu
50 55 60
Ala Ala Ile Ala Asp Ala Gln Pro Gln Ala Pro Thr Met Pro Pro Gln
65 70 75 80
Met Ala Pro His Pro Ala Met Gln Ala Ser Gly Tyr Tyr Met Gln His
85 90 95
Pro Gln Ala Ala Ala Met Ala Gln Gln Gln Gly Ile Phe Pro Gln Lys
100 105 110
Met Pro Leu Gln Phe Asn Asn Pro His Gln Leu Gln Asp Pro Gln Gln
115 120 125
Gln Leu His Gln His Gln Ala Met Gln Ala Gln Met Gly Met Arg Pro
130 135 140
Gly Ala Thr Asn Asn Gly Met His Pro Met His Ala Glu Ser Ser Leu
145 150 155 160
Gly Gly Gly Ser Ser Gly Gly Pro Pro Ser Ala Ser Gly Pro Gly Asp
165 170 175
Ile Arg Gly Gly Asn Lys Gln Asp Ala Ser Glu Ala Gly Thr Thr Gly
180 185 190
Ala Asp Gly Gln Gly Ser Ser Ala Gly Gly His Gly Gly Asp Gly Glu
195 200 205
Glu Ala Lys
210

<210> 99
<211> 597
<212> DNA
<213> Euphorbia esula

<400> 99
atgcagcagc aaccgcagat gatgcctatg atgccttcat atccaccagc aaacattacc 60
acggagcaaa tccaaaagta tcttgatgaa aataaaaaat tgattttggc gatcttggat 120
aatcaaaatc ttggaaaact cgctgagtgt gcacagtatc aagccctgct gcaaaaaaat 180
ctgatgtatt tagccgcaat tgctgatgca caaccccaga ccccacccat gccacctcag 240
atgtccccac atccggctat gcaacaagga gcatattaca tgcaacatcc tcaggctgca 300
gcagcagcaa tggctcatca gtcgggtatt ttcccaccaa agatgtctcc gttacaattc 360
aataatcctc atcaaataca ggacccccag cagttacatc aagcagccct ccaagggcaa 420

atgggaatga ggcccatggg gcccaataac gggatgcatc cgatgcaccc cgaggcaaat 480
cttggaggat ctaatgatgg tcgtggagga aacaaacagg atgctccgga gacgggagca 540
tcgggaggtg atgggcaagg caattctggt ggtgatgggg ctgaagatgg gaaatga 597

<210> 100
<211> 198
<212> PRT
<213> Euphorbia esula

<400> 100
Met Gln Gln Gln Pro Gln Met Met Pro Met Met Pro Ser Tyr Pro Pro
1 5 10 15
Ala Asn Ile Thr Thr Glu Gln Ile Gln Lys Tyr Leu Asp Glu Asn Lys
20 25 30
Lys Leu Ile Leu Ala Ile Leu Asp Asn Gln Asn Leu Gly Lys Leu Ala
35 40 45
Glu Cys Ala Gln Tyr Gln Ala Leu Leu Gln Lys Asn Leu Met Tyr Leu
50 55 60
Ala Ala Ile Ala Asp Ala Gln Pro Gln Thr Pro Pro Met Pro Pro Gln
65 70 75 80
Met Ser Pro His Pro Ala Met Gln Gln Gly Ala Tyr Tyr Met Gln His
85 90 95
Pro Gln Ala Ala Ala Ala Ala Met Ala His Gln Ser Gly Ile Phe Pro
100 105 110
Pro Lys Met Ser Pro Leu Gln Phe Asn Asn Pro His Gln Ile Gln Asp
115 120 125
Pro Gln Gln Leu His Gln Ala Ala Leu Gln Gly Gln Met Gly Met Arg
130 135 140
Pro Met Gly Pro Asn Asn Gly Met His Pro Met His Pro Glu Ala Asn
145 150 155 160
Leu Gly Gly Ser Asn Asp Gly Arg Gly Gly Asn Lys Gln Asp Ala Pro
165 170 175
Glu Thr Gly Ala Ser Gly Gly Asp Gly Gln Gly Asn Ser Gly Gly Asp
180 185 190
Gly Ala Glu Asp Gly Lys
195

<210> 101
<211> 642
<212> DNA
<213> Glycine max

<400> 101
atgcagcaga caccgccaat gattcctatg atgccttctt tcccacctac gaacataacc 60
accgagcaga ttcaaaaata ccttgatgag aacaagaagc tgattctggc aatattggac 120
aatcaaaatc ttggaaaact tgcagaatgt gcccagtacc aagctcagct tcaaaagaat 180
ttgatgtatt tagctgcaat tgctgatgcc cagcctcaaa ccccggccat gcctccgcag 240
atggcaccgc accctgccat gcaaccagga ttctatatgc aacatcctca ggctgctgca 300
gcagcaatgg ctcagcagca gcaaggaatg ttcccccaga aaatgccatt gcaatttggc 360
aatccacatc aaatgcagga acaacaacag cagctacacc agcaggccat ccaaggtcaa 420
atgggactta gacctggaga tataaataat ggcatgcatc caatgcacag tgaggctgct 480
cttggaggtg gaaacagcgg tggtccacct tcggctactg gtccaaacga tgcacgtggt 540
ggaagcaagc aagatgcctc tgaggctgga acagctggtg gagacggcca aggcagctcc 600
gcggctgctc ataacagtgg agatggtgaa gaggcaaagt ga 642

<210> 102
<211> 213
<212> PRT
<213> Glycine max

<400> 102
Met Gln Gln Thr Pro Pro Met Ile Pro Met Met Pro Ser Phe Pro Pro
1 5 10 15
Thr Asn Ile Thr Thr Glu Gln Ile Gln Lys Tyr Leu Asp Glu Asn Lys
20 25 30
Lys Leu Ile Leu Ala Ile Leu Asp Asn Gln Asn Leu Gly Lys Leu Ala
35 40 45
Glu Cys Ala Gln Tyr Gln Ala Gln Leu Gln Lys Asn Leu Met Tyr Leu
50 55 60
Ala Ala Ile Ala Asp Ala Gln Pro Gln Thr Pro Ala Met Pro Pro Gln
65 70 75 80
Met Ala Pro His Pro Ala Met Gln Pro Gly Phe Tyr Met Gln His Pro
85 90 95
Gln Ala Ala Ala Ala Ala Met Ala Gln Gln Gln Gln Gly Met Phe Pro
100 105 110
Gln Lys Met Pro Leu Gln Phe Gly Asn Pro His Gln Met Gln Glu Gln
115 120 125
Gln Gln Gln Leu His Gln Gln Ala Ile Gln Gly Gln Met Gly Leu Arg
130 135 140
Pro Gly Asp Ile Asn Asn Gly Met His Pro Met His Ser Glu Ala Ala
145 150 155 160
Leu Gly Gly Gly Asn Ser Gly Gly Pro Pro Ser Ala Thr Gly Pro Asn
165 170 175
Asp Ala Arg Gly Gly Ser Lys Gln Asp Ala Ser Glu Ala Gly Thr Ala
180 185 190
Gly Gly Asp Gly Gln Gly Ser Ser Ala Ala Ala His Asn Ser Gly Asp
195 200 205
Gly Glu Glu Ala Lys
210

<210> 103
<211> 633
<212> DNA
<213> Glycine soya

<400> 103
atgcagcaga caccgcctat gattcctatg atgccttcgt tcccacctac gaacataacc 60
accgagcaga ttcaaaaata ccttgatgag aacaagaagc tgattctggc aatattggac 120
aatcaaaatc ttggaaaact tgcagaatgt gcccagtacc aagctcagct tcaaaagaat 180
ttgatgtatt tagctgcaat tgctgatgcc cagcctcaaa caccagccat gcctccacag 240
atggcaccac accctgccat gcaaccagga ttctatatgc aacatcctca ggctgcagca 300
gcagcaatgg ctcagcagca gcagcaagga atgttccccc agaaaatgcc attgcaattt 360
ggcaatccac atcaaatgca ggaacaacag cagcagctac accagcaagc catccaaggt 420
caaatgggac tgagacctgg aggaataaat aatggcatgc atccaatgca caatgagggc 480
ggcaacagcg gtggtccacc ctcggctacc ggtccgaacg acgcacgtgg tggaagcaag 540
caagatgctt ctgaggctgg aacagctggt ggagatggcc aaggcagctc tgcagctgct 600
cataacagtg gagatggtga agaggcaaag tga 633

<210> 104
<211> 210
<212> PRT
<213> Glycine soya

<400> 104
Met Gln Gln Thr Pro Pro Met Ile Pro Met Met Pro Ser Phe Pro Pro
1 5 10 15
Thr Asn Ile Thr Thr Glu Gln Ile Gln Lys Tyr Leu Asp Glu Asn Lys
20 25 30

```
Lys Leu Ile Leu Ala Ile Leu Asp Asn Gln Asn Leu Gly Lys Leu Ala
        35                  40                  45
Glu Cys Ala Gln Tyr Gln Ala Gln Leu Gln Lys Asn Leu Met Tyr Leu
    50                  55                  60
Ala Ala Ile Ala Asp Ala Gln Pro Gln Thr Pro Ala Met Pro Pro Gln
65                  70                  75                  80
Met Ala Pro His Pro Ala Met Gln Pro Gly Phe Tyr Met Gln His Pro
                85                  90                  95
Gln Ala Ala Ala Ala Ala Met Ala Gln Gln Gln Gln Gln Gly Met Phe
            100                 105                 110
Pro Gln Lys Met Pro Leu Gln Phe Gly Asn Pro His Gln Met Gln Glu
        115                 120                 125
Gln Gln Gln Gln Leu His Gln Gln Ala Ile Gln Gly Gln Met Gly Leu
    130                 135                 140
Arg Pro Gly Gly Ile Asn Asn Gly Met His Pro Met His Asn Glu Gly
145                 150                 155                 160
Gly Asn Ser Gly Gly Pro Pro Ser Ala Thr Gly Pro Asn Asp Ala Arg
                165                 170                 175
Gly Gly Ser Lys Gln Asp Ala Ser Glu Ala Gly Thr Ala Gly Gly Asp
            180                 185                 190
Gly Gln Gly Ser Ser Ala Ala Ala His Asn Ser Gly Asp Gly Glu Glu
        195                 200                 205
Ala Lys
    210

<210>  105
<211>  690
<212>  DNA
<213>  Gossypium hirsutum

<400>  105
atgcagcagc acctgatgca gatgcagccc atgatggcag cttattatcc caacaacgtc      60
actactgatc atattcaaca gtatctcgat gagaacaagt cattgatctt aaagattgtt     120
gagagccaga attctgggaa attgagtgaa tgtgctgaga accaagcaag gctgcagcga     180
aacctcatgt acctggctgc cattgcggat tctcaacccc aaccacccac cgtgcatgca     240
cagtttccat ctggtggtat catgcagcca ggagctgggc actacatgca gcaccaacaa     300
gctcaacaaa tgacacaaca gtcgcttatg gctgctcggt cctcaatgtt gtattctcag     360
caaccatttt ctgcactgca acaacaacag cagcaagctt tgcacagtca gcttggcatg     420
agctctggcg gaagcacagg ccttcatatg ctgcaaactg aatctagtac tgcaggtggc     480
agtggagcac ttgggggccgg agggtttcct gattttggac gtggttcttc tggagaaggc     540
atccatggtg gcaggccaat ggcaggtgga agcaagcaag atatcgggag tgccggctca     600
gctgaaggtc gtggaggaag ctctggtggt cagggtggtg gtgatggggg tgaaaccctt     660
tacttaaaag cagccgatga tgggaactga                                      690

<210>  106
<211>  229
<212>  PRT
<213>  Gossypium hirsutum

<400>  106
Met Gln Gln His Leu Met Gln Met Gln Pro Met Met Ala Ala Tyr Tyr
1               5                   10                  15
Pro Asn Asn Val Thr Thr Asp His Ile Gln Gln Tyr Leu Asp Glu Asn
            20                  25                  30
Lys Ser Leu Ile Leu Lys Ile Val Glu Ser Gln Asn Ser Gly Lys Leu
        35                  40                  45
Ser Glu Cys Ala Glu Asn Gln Ala Arg Leu Gln Arg Asn Leu Met Tyr
    50                  55                  60
Leu Ala Ala Ile Ala Asp Ser Gln Pro Gln Pro Pro Thr Val His Ala
```

```
65                  70                  75                  80
Gln Phe Pro Ser Gly Gly Ile Met Gln Pro Gly Ala Gly His Tyr Met
                85                  90                  95
Gln His Gln Gln Ala Gln Gln Met Thr Gln Gln Ser Leu Met Ala Ala
            100                 105                 110
Arg Ser Ser Met Leu Tyr Ser Gln Gln Pro Phe Ser Ala Leu Gln Gln
        115                 120                 125
Gln Gln Gln Gln Ala Leu His Ser Gln Leu Gly Met Ser Ser Gly Gly
    130                 135                 140
Ser Thr Gly Leu His Met Leu Gln Thr Glu Ser Ser Thr Ala Gly Gly
145                 150                 155                 160
Ser Gly Ala Leu Gly Ala Gly Gly Phe Pro Asp Phe Gly Arg Gly Ser
                165                 170                 175
Ser Gly Glu Gly Ile His Gly Gly Arg Pro Met Ala Gly Gly Ser Lys
            180                 185                 190
Gln Asp Ile Gly Ser Ala Gly Ser Ala Glu Gly Arg Gly Gly Ser Ser
        195                 200                 205
Gly Gly Gln Gly Gly Gly Asp Gly Gly Glu Thr Leu Tyr Leu Lys Ala
    210                 215                 220
Ala Asp Asp Gly Asn
225
```

<210> 107
<211> 642
<212> DNA
<213> Gossypium hirsutum

<400> 107

```
atgccgcagc caccgcaaat gattcctgtg atgccttcat atccacctac taatatcact     60
actgaacaga ttcagaagta ccttgatgag aataagaagt tgattttggc aattttggac    120
aatcagaatc ttggaaaact cgctgaatgc gcccagtatc aagctcagct gcaaaagaat    180
ttgatgtatt tagctgcaat tgcggatgct caacctcaat caacgccagc aatgtcgcct    240
cagatggcac cgcatccagc aatgcaaccc ggaggatatt ttatgcaaca tcctcaagct    300
gctgcaatgt cacagcaacc tggcatgtac cctcaaaagg tgccattgca attcaatagt    360
ccgcatcaaa tgcaggaccc tcagcacctc ctatatcagc agcatcaaca agcaatgcaa    420
ggtcaaatgg gaatcaggcc tggggggaccc aataatagca tgcatcccat gcattcagag    480
gctagccttg gaggcggcag cagtggtggt ccccctcaac cttcaggccc aagtgatgga    540
cgtgctggaa acaagcaaga gggctccgaa gctggtggta atgggcaggg cagcacaact    600
ggtgggcatg gtggcggtga tggagcggat gaggcaaagt ga                       642
```

<210> 108
<211> 213
<212> PRT
<213> Gossypium hirsutum

<400> 108

```
Met Pro Gln Pro Pro Gln Met Ile Pro Val Met Pro Ser Tyr Pro Pro
1               5                   10                  15
Thr Asn Ile Thr Thr Glu Gln Ile Gln Lys Tyr Leu Asp Glu Asn Lys
            20                  25                  30
Lys Leu Ile Leu Ala Ile Leu Asp Asn Gln Asn Leu Gly Lys Leu Ala
        35                  40                  45
Glu Cys Ala Gln Tyr Gln Ala Gln Leu Gln Lys Asn Leu Met Tyr Leu
    50                  55                  60
Ala Ala Ile Ala Asp Ala Gln Pro Gln Ser Thr Pro Ala Met Ser Pro
65                  70                  75                  80
Gln Met Ala Pro His Pro Ala Met Gln Pro Gly Gly Tyr Phe Met Gln
                85                  90                  95
His Pro Gln Ala Ala Ala Met Ser Gln Gln Pro Gly Met Tyr Pro Gln
```

```
                100                 105                 110
Lys Val Pro Leu Gln Phe Asn Ser Pro His Gln Met Gln Asp Pro Gln
        115                 120                 125
His Leu Leu Tyr Gln Gln His Gln Gln Ala Met Gln Gly Gln Met Gly
    130                 135                 140
Ile Arg Pro Gly Gly Pro Asn Asn Ser Met His Pro Met His Ser Glu
145                 150                 155                 160
Ala Ser Leu Gly Gly Gly Ser Ser Gly Gly Pro Pro Gln Pro Ser Gly
                165                 170                 175
Pro Ser Asp Gly Arg Ala Gly Asn Lys Gln Glu Gly Ser Glu Ala Gly
            180                 185                 190
Gly Asn Gly Gln Gly Ser Thr Thr Gly Gly His Gly Gly Gly Asp Gly
        195                 200                 205
Ala Asp Glu Ala Lys
    210
```

<210> 109
<211> 561
<212> DNA
<213> Hordeum vulgare

<400> 109

```
atgcagcaag cgatgcccat gccgccggcg gcggcggcgc ctgggatgcc tccttctgcc     60
ggcctcagca ccgagcagat ccaaaagtac ctggatgaaa ataaacaact aattttggct    120
atcttggaaa atcagaacct gggaaagttg gcggaatgtg ctcagtatca agctcagctt    180
cagaagaatc ttttgtattt ggctgcgatt gctgatactc agccacagac ctctgtaagc    240
cgtcctcaga tggcaccacc tgctgcatcc ccaggggcag ggcattacat gtcacaggtg    300
ccaatgttcc ctccgaggac ccctctaacg cctcagcaga tgcaggagca gcaactacag    360
caacaacagg ctcagatgct tccgtttgct ggtcaaatgg ttgcgagacc cggggctgtc    420
aatggcattc cccaggcccc tcaagttgaa caaccagcct atgcagcagg tggggccagt    480
tccgagcctt ctggcaccga gagccacagg agcactggcg ccgataacga tggtgggagc    540
ggcttggctg accagtccta a                                              561
```

<210> 110
<211> 186
<212> PRT
<213> Hordeum vulgare

<400> 110

```
Met Gln Gln Ala Met Pro Met Pro Pro Ala Ala Ala Ala Pro Gly Met
1               5                   10                  15
Pro Pro Ser Ala Gly Leu Ser Thr Glu Gln Ile Gln Lys Tyr Leu Asp
            20                  25                  30
Glu Asn Lys Gln Leu Ile Leu Ala Ile Leu Glu Asn Gln Asn Leu Gly
        35                  40                  45
Lys Leu Ala Glu Cys Ala Gln Tyr Gln Ala Gln Leu Gln Lys Asn Leu
    50                  55                  60
Leu Tyr Leu Ala Ala Ile Ala Asp Thr Gln Pro Gln Thr Ser Val Ser
65                  70                  75                  80
Arg Pro Gln Met Ala Pro Pro Ala Ala Ser Pro Gly Ala Gly His Tyr
                85                  90                  95
Met Ser Gln Val Pro Met Phe Pro Pro Arg Thr Pro Leu Thr Pro Gln
            100                 105                 110
Gln Met Gln Glu Gln Gln Leu Gln Gln Gln Gln Ala Gln Met Leu Pro
        115                 120                 125
Phe Ala Gly Gln Met Val Ala Arg Pro Gly Ala Val Asn Gly Ile Pro
    130                 135                 140
Gln Ala Pro Gln Val Glu Gln Pro Ala Tyr Ala Ala Gly Gly Ala Ser
145                 150                 155                 160
```

Ser Glu Pro Ser Gly Thr Glu Ser His Arg Ser Thr Gly Ala Asp Asn
165 170 175
Asp Gly Gly Ser Gly Leu Ala Asp Gln Ser
180 185

<210> 111
<211> 555
<212> DNA
<213> Lactuca serriola

<220>
<221> misc_feature
<222> (253)..(253)
<223> n is a, c, g, or t

<400> 111
atgaagcagc cgatgatgcc gaatccaatg atgtcttctt cgtttcctcc tacaaacatc 60
accaccgatc agatccaaaa gttccttgat gaaaacaagc aactaattat agcaataatg 120
agcaacctaa atcttggaaa gcttgctgaa tgtgcccagt accaagctct actccaaaaa 180
aatttgatgt atctagcagc cattgcagat gctcaaccac ctacacctac accaacacta 240
aatatctctt atnagatggg cccggttcca catccaggga tgccacagca aggtggattt 300
tacatggcgc agcagcaccc tcaggcggct gtaatgacgg ctcagccacc ttctggtttt 360
ccacaaccga tgcctggtat gcaatttaac agcccacagg ctattcaagg gcagatgggc 420
gggaggtccg gtgggccgcc aagctcagcc gctagtgatg tctggagagg aagcatgcaa 480
gatggtggtg gtggtgctgc tgctgatggt ggtaaggatg gtcatgctgg cggtggacct 540
gaggaagcaa agtaa 555

<210> 112
<211> 184
<212> PRT
<213> Lactuca serriola

<220>
<221> UNSURE
<222> (85)..(85)
<223> Xaa can be any naturally occurring amino acid

<400> 112
Met Lys Gln Pro Met Met Pro Asn Pro Met Met Ser Ser Ser Phe Pro
1 5 10 15
Pro Thr Asn Ile Thr Thr Asp Gln Ile Gln Lys Phe Leu Asp Glu Asn
20 25 30
Lys Gln Leu Ile Ile Ala Ile Met Ser Asn Leu Asn Leu Gly Lys Leu
35 40 45
Ala Glu Cys Ala Gln Tyr Gln Ala Leu Leu Gln Lys Asn Leu Met Tyr
50 55 60
Leu Ala Ala Ile Ala Asp Ala Gln Pro Pro Thr Pro Thr Pro Thr Leu
65 70 75 80
Asn Ile Ser Tyr Xaa Met Gly Pro Val Pro His Pro Gly Met Pro Gln
85 90 95
Gln Gly Gly Phe Tyr Met Ala Gln Gln His Pro Gln Ala Ala Val Met
100 105 110
Thr Ala Gln Pro Pro Ser Gly Phe Pro Gln Pro Met Pro Gly Met Gln
115 120 125
Phe Asn Ser Pro Gln Ala Ile Gln Gly Gln Met Gly Gly Arg Ser Gly
130 135 140
Gly Pro Pro Ser Ser Ala Ala Ser Asp Val Trp Arg Gly Ser Met Gln
145 150 155 160
Asp Gly Gly Gly Gly Ala Ala Ala Asp Gly Gly Lys Asp Gly His Ala

```
                165                 170                 175
Gly Gly Gly Pro Glu Glu Ala Lys
            180

<210>  113
<211>  627
<212>  DNA
<213>  Lycopersicon esculentum

<400>  113
atgcagcagc acctgatgca gatgcagccc atgatggcag cttactatcc aacgaacgtc      60
actactgacc atattcaaca gtatttggat gaaaacaaat cactcattct gaagattgtt     120
gagagccaga actctgggaa actcagtgaa tgtgcggaga accaagctag gcttcagagg     180
aatctgatgt accttgctgc gattgctgat tcacaacctc aaccttctag catgcattct     240
cagttctctt ctggtgggat gatgcagcca gggacacaca gttacttgca gcagcagcag     300
cagcaacaac aagcgcaaca aatggcaaca caacaactca tggctgcaag atcctcgtcg     360
atgctctatg gacaacagca gcagcaatct cagttatcgc aatatcaaca aggcttgcat     420
agtagccaac tcggcatgag ttctggcagt ggcggaagca ctggacttca tcacatgctt     480
caaagtgaat catcacctca tggtggtggt ttctctcatg acttcggccg cgcaaataag     540
caagacattg ggagtagtat gtctgctgaa gggcgcggcg gaagttcagg tggtgagaat     600
ctttatctga aagcttctga ggattga                                         627

<210>  114
<211>  208
<212>  PRT
<213>  Lycopersicon esculentum

<400>  114
Met Gln Gln His Leu Met Gln Met Gln Pro Met Met Ala Ala Tyr Tyr
1               5                   10                  15
Pro Thr Asn Val Thr Thr Asp His Ile Gln Gln Tyr Leu Asp Glu Asn
            20                  25                  30
Lys Ser Leu Ile Leu Lys Ile Val Glu Ser Gln Asn Ser Gly Lys Leu
        35                  40                  45
Ser Glu Cys Ala Glu Asn Gln Ala Arg Leu Gln Arg Asn Leu Met Tyr
    50                  55                  60
Leu Ala Ala Ile Ala Asp Ser Gln Pro Gln Pro Ser Ser Met His Ser
65                  70                  75                  80
Gln Phe Ser Ser Gly Gly Met Met Gln Pro Gly Thr His Ser Tyr Leu
                85                  90                  95
Gln Gln Gln Gln Gln Gln Gln Gln Ala Gln Gln Met Ala Thr Gln Gln
            100                 105                 110
Leu Met Ala Ala Arg Ser Ser Ser Met Leu Tyr Gly Gln Gln Gln Gln
        115                 120                 125
Gln Ser Gln Leu Ser Gln Tyr Gln Gln Gly Leu His Ser Ser Gln Leu
    130                 135                 140
Gly Met Ser Ser Gly Ser Gly Gly Ser Thr Gly Leu His His Met Leu
145                 150                 155                 160
Gln Ser Glu Ser Ser Pro His Gly Gly Gly Phe Ser His Asp Phe Gly
                165                 170                 175
Arg Ala Asn Lys Gln Asp Ile Gly Ser Ser Met Ser Ala Glu Gly Arg
            180                 185                 190
Gly Gly Ser Ser Gly Gly Glu Asn Leu Tyr Leu Lys Ala Ser Glu Asp
        195                 200                 205

<210>  115
<211>  624
<212>  DNA
<213>  Malus domestica
```

<400> 115
atgcagcagc caccacaaat gatccccgtc atgccttcat ttcctcccac caacatcacc 60
accgaacaaa ttcagaagta ccttgatgac aacaaaaagt tgattctggc aatattggat 120
aatcaaaatc ttggaaaact tgctgagtgt gctcagtacc aggctctgct tcaaaagaat 180
ctgatgtatt tagcagcaat tgccgatgcg caaccacagg caccagctgc ccctccccag 240
atggccccac atcctgctat gcaacaggca ggatattaca tgcaacatcc tcaggcagca 300
gcaatggctc agcaacaggg tattttctcc ccaaagatgc cgatgcaatt caataacatg 360
catcaaatgc acgatccaca gcagcaccaa caagccatgc aagggcaaat gggaatgaga 420
cctggagggc ctaacggcat gccttccatg cttcatactg aggccacaca tggtggtggt 480
agtggcggcc caaattcagc tggagaccca aatgatgggc gtggaggaag caagcaagac 540
gcctctgagt ctggggcagg tggtgatggc caggggacct cagccggcgg gcgtggaact 600
ggtgatggag aggacggcaa gtga 624

<210> 116
<211> 207
<212> PRT
<213> Malus domestica

<400> 116
Met Gln Gln Pro Pro Gln Met Ile Pro Val Met Pro Ser Phe Pro Pro
1 5 10 15
Thr Asn Ile Thr Thr Glu Gln Ile Gln Lys Tyr Leu Asp Asp Asn Lys
20 25 30
Lys Leu Ile Leu Ala Ile Leu Asp Asn Gln Asn Leu Gly Lys Leu Ala
35 40 45
Glu Cys Ala Gln Tyr Gln Ala Leu Leu Gln Lys Asn Leu Met Tyr Leu
50 55 60
Ala Ala Ile Ala Asp Ala Gln Pro Gln Ala Pro Ala Ala Pro Pro Gln
65 70 75 80
Met Ala Pro His Pro Ala Met Gln Gln Ala Gly Tyr Tyr Met Gln His
85 90 95
Pro Gln Ala Ala Ala Met Ala Gln Gln Gln Gly Ile Phe Ser Pro Lys
100 105 110
Met Pro Met Gln Phe Asn Asn Met His Gln Met His Asp Pro Gln Gln
115 120 125
His Gln Gln Ala Met Gln Gly Gln Met Gly Met Arg Pro Gly Gly Pro
130 135 140
Asn Gly Met Pro Ser Met Leu His Thr Glu Ala Thr His Gly Gly Gly
145 150 155 160
Ser Gly Gly Pro Asn Ser Ala Gly Asp Pro Asn Asp Gly Arg Gly Gly
165 170 175
Ser Lys Gln Asp Ala Ser Glu Ser Gly Ala Gly Gly Asp Gly Gln Gly
180 185 190
Thr Ser Ala Gly Gly Arg Gly Thr Gly Asp Gly Glu Asp Gly Lys
195 200 205

<210> 117
<211> 639
<212> DNA
<213> Medicago trunculata

<400> 117
atgcagcaga cacctcaaat gattcctatg atgccttcat tcccacaaca aacaaacata 60
accactgagc agattcaaaa atatcttgat gagaacaaga agctgatcct ggcaatattg 120
gacaatcaaa atcttggaaa acttgcagaa tgtgcccagt accaagctca gcttcagaag 180
aatttgatgt atttagctgc aattgctgac gcgcagccac aaacaccggc cttgcctcca 240
cagatggccc cgcaccctgc gatgcaacaa ggattctata tgcaacatcc tcaggctgca 300
gcaatggctc agcaacaagg aatgttcccc caaaaaatgc caatgcagtt cggtaatccg 360

catcaaatgc aggatcagca gcatcagcag caacaacagc agctacatca gcaagctatg 420
caaggtcaaa tgggacttag acctggaggg ataaataacg gcatgcatcc aatgcacaac 480
gaggctgctc tcggaggtag cggcagtggt ggtcaaatga cgggcgtggt ggtggagcaa 540
gcaagatgct tcggagctgg gacagccggc ggtgatggtc aaggaacctc tgccgcagct 600
gcgcacaaca gtggagatgc ttcagaagaa ggaaagtaa 639

<210> 118
<211> 213
<212> PRT
<213> Medicago trunculata

<400> 118
Met Gln Gln Thr Pro Gln Met Ile Pro Met Met Pro Ser Phe Pro Gln
1 5 10 15
Gln Thr Asn Ile Thr Thr Glu Gln Ile Gln Lys Tyr Leu Asp Glu Asn
20 25 30
Lys Lys Leu Ile Leu Ala Ile Leu Asp Asn Gln Asn Leu Gly Lys Leu
35 40 45
Ala Glu Cys Ala Gln Tyr Gln Ala Gln Leu Gln Lys Asn Leu Met Tyr
50 55 60
Leu Ala Ala Ile Ala Asp Ala Gln Pro Gln Thr Pro Ala Leu Pro Pro
65 70 75 80
Gln Met Ala Pro His Pro Ala Met Gln Gln Gly Phe Tyr Met Gln His
85 90 95
Pro Gln Ala Ala Ala Met Ala Gln Gln Gln Gly Met Phe Pro Gln Lys
100 105 110
Met Pro Met Gln Phe Gly Asn Pro His Gln Met Gln Asp Gln Gln His
115 120 125
Gln Gln Gln Gln Gln Gln Leu His Gln Gln Ala Met Gln Gly Gln Met
130 135 140
Gly Leu Arg Pro Gly Gly Ile Asn Asn Gly Met His Pro Met His Asn
145 150 155 160
Glu Ala Ala Leu Gly Gly Ser Gly Ser Gly Gly Pro Asn Asp Gly Arg
165 170 175
Gly Gly Gly Ser Lys Gln Asp Ala Ser Glu Ala Gly Thr Ala Gly Gly
180 185 190
Asp Gly Gln Gly Thr Ser Ala Ala Ala Ala His Asn Ser Gly Asp Ala
195 200 205
Ser Glu Glu Gly Lys
210

<210> 119
<211> 624
<212> DNA
<213> Panicum virgatum

<400> 119
atgcagcagc agatgcccat gcagtcggcg cccccggcga ccggcatcac caccgagcag 60
atccaaaagt atttggatga aaataagcag cttattttgg ccatcctgga aaatcagaac 120
ttaggaaagt tggctgaatg tgctcagtat caagctcagc ttcaaaagaa tctcttgtac 180
ctggctgcga ttgcagatgc ccaacccaa ccaccacaga accctgcaag tcgcccacag 240
atgatgcaac ctggcatggt accaggtgca gggcattaca tgtcccaagt accaatgttc 300
ccgccaagaa caccattaac cccgcaacag atgcaagaac agcagcagca gcagcagcag 360
cttcaacagc agcaagcaca ggctcttgct ttcccgggac agatggtcat gagacctacc 420
attaatggca tgcagcctat gcaagccgac cctgctgccg ccgccgccag cctacagcag 480
tcagcacctg gccctactga tgggcgagga ggcaagcaag atgcaactgc tggggtgagc 540
acagagcctt ctggcaccga gagccacaag agcacaaccg cagcagatca cgatgtgggc 600
actgatgtcg cggagaaatc ctaa 624

<210> 120
<211> 207
<212> PRT
<213> Panicum virgatum

<400> 120
Met Gln Gln Gln Met Pro Met Gln Ser Ala Pro Pro Ala Thr Gly Ile
1 5 10 15
Thr Thr Glu Gln Ile Gln Lys Tyr Leu Asp Glu Asn Lys Gln Leu Ile
20 25 30
Leu Ala Ile Leu Glu Asn Gln Asn Leu Gly Lys Leu Ala Glu Cys Ala
35 40 45
Gln Tyr Gln Ala Gln Leu Gln Lys Asn Leu Leu Tyr Leu Ala Ala Ile
50 55 60
Ala Asp Ala Gln Pro Gln Pro Pro Gln Asn Pro Ala Ser Arg Pro Gln
65 70 75 80
Met Met Gln Pro Gly Met Val Pro Gly Ala Gly His Tyr Met Ser Gln
85 90 95
Val Pro Met Phe Pro Pro Arg Thr Pro Leu Thr Pro Gln Gln Met Gln
100 105 110
Glu Gln Gln Gln Gln Gln Gln Gln Leu Gln Gln Gln Gln Ala Gln Ala
115 120 125
Leu Ala Phe Pro Gly Gln Met Val Met Arg Pro Thr Ile Asn Gly Met
130 135 140
Gln Pro Met Gln Ala Asp Pro Ala Ala Ala Ala Ala Ser Leu Gln Gln
145 150 155 160
Ser Ala Pro Gly Pro Thr Asp Gly Arg Gly Gly Lys Gln Asp Ala Thr
165 170 175
Ala Gly Val Ser Thr Glu Pro Ser Gly Thr Glu Ser His Lys Ser Thr
180 185 190
Thr Ala Ala Asp His Asp Val Gly Thr Asp Val Ala Glu Lys Ser
195 200 205

<210> 121
<211> 747
<212> DNA
<213> Picea sitchensis

<400> 121
atgcagcagc atctcatgca aatgcagccc atgatggcgg catacgcctc caacaacatc 60
accactgatc acatccagaa gtacctggat gagaacaagc agttgattct ggcaattctg 120
gacaaccaaa atcttggaaa gctcaatgag tgtgctcagt accaagcaaa acttcagcag 180
aatttgatgt atctggctgc gattgctgat tctcaaccac aagcacaaac tgcacatgct 240
cagattcctc ctaatgcagt gatgcagtct ggtgggcatt acatgcagca ccagcaggca 300
cagcaacaag tgactcctca gtctctgatg gcagctagat cttccatgct gtattctcag 360
cagccgatgg ctgctttgca tcaagctcag caacaacagc agcagcagca tcagcagcaa 420
caacaatctc ttcacagcca gcttggcata aattctggag gaagcagtgg attgcatatg 480
ttgcatggtg agacaaacat gggatgtaat gggcctctct catctggggg cttccctgaa 540
tttgggcgtg ggtctgctac ctctgctgaa ggtatgcagg ccaacagggg cttcactata 600
gatcgtggtt caaataagca ggatggagta ggatcagaga atgcccatcc aggtgctggt 660
gatggaagag ggagttcaac tggagggcag aatgcagatg agtcagaacc atcatacctg 720
aaagcctccg aagaagaagg aaactag 747

<210> 122
<211> 248
<212> PRT
<213> Picea sitchensis

<400> 122

```
Met Gln Gln His Leu Met Gln Met Gln Pro Met Met Ala Ala Tyr Ala
1               5                   10                  15
Ser Asn Asn Ile Thr Thr Asp His Ile Gln Lys Tyr Leu Asp Glu Asn
            20                  25                  30
Lys Gln Leu Ile Leu Ala Ile Leu Asp Asn Gln Asn Leu Gly Lys Leu
        35                  40                  45
Asn Glu Cys Ala Gln Tyr Gln Ala Lys Leu Gln Gln Asn Leu Met Tyr
    50                  55                  60
Leu Ala Ala Ile Ala Asp Ser Gln Pro Gln Ala Gln Thr Ala His Ala
65                  70                  75                  80
Gln Ile Pro Pro Asn Ala Val Met Gln Ser Gly Gly His Tyr Met Gln
                85                  90                  95
His Gln Gln Ala Gln Gln Gln Val Thr Pro Gln Ser Leu Met Ala Ala
            100                 105                 110
Arg Ser Ser Met Leu Tyr Ser Gln Gln Pro Met Ala Ala Leu His Gln
        115                 120                 125
Ala Gln Gln Gln Gln Gln Gln Gln His Gln Gln Gln Gln Gln Ser Leu
    130                 135                 140
His Ser Gln Leu Gly Ile Asn Ser Gly Gly Ser Ser Gly Leu His Met
145                 150                 155                 160
Leu His Gly Glu Thr Asn Met Gly Cys Asn Gly Pro Leu Ser Ser Gly
                165                 170                 175
Gly Phe Pro Glu Phe Gly Arg Gly Ser Ala Thr Ser Ala Glu Gly Met
            180                 185                 190
Gln Ala Asn Arg Gly Phe Thr Ile Asp Arg Gly Ser Asn Lys Gln Asp
        195                 200                 205
Gly Val Gly Ser Glu Asn Ala His Pro Gly Ala Gly Asp Gly Arg Gly
    210                 215                 220
Ser Ser Thr Gly Gly Gln Asn Ala Asp Glu Ser Glu Pro Ser Tyr Leu
225                 230                 235                 240
Lys Ala Ser Glu Glu Glu Gly Asn
                245
```

<210> 123
<211> 735
<212> DNA
<213> Pinus taeda

<400> 123

```
atgcagcagc acctcatgca aatgcagccc atgatggcgg cctacgcctc caacaatatc     60
accactgatc acatccagaa gtacctggat gagaacaagc agttgattct ggcaattttg    120
gacaaccaaa atctcggaaa gctcaatgag tgtgctcaat accaagcaaa acttcagcag    180
aatttgatgt atctggctgc tattgctgat tctcaacctc aagcacaaac tgcacatgct    240
cagattcctc caaatgcggt gatgcagtct ggtgggcatt acatgcagca tcaacaggca    300
cagcaacaag ttactcctca gtctctgatg gcagctagat cttccatact gtatgctcag    360
caacaacagc agcagcagca tcagcagcat cagcagcaac agcagcaaca acagtctctt    420
cacagccagc ttggcataaa ttctggagga agcagcggtt tgcatatgtt gcatggtgag    480
acaaacatgg gatgtaatgg gcctctgtca tctgggggat tccctgaatt tgggcgtggg    540
tctgctacct ctgctgatgg tatgcaggtg aacaggggct ttgctataga tcgtggttca    600
aacaagcagg atggagttgg atcagagaat gcccatgctg gtgctggtga tggaagaggg    660
agttcaactg gagggcagaa tgcagatgag tcagaaccat catacctgaa ggcctccgag    720
gaagaaggaa actag                                                      735
```

<210> 124
<211> 244
<212> PRT
<213> Pinus taeda

<400> 124

```
Met Gln Gln His Leu Met Gln Met Gln Pro Met Met Ala Ala Tyr Ala
1               5                   10                  15
Ser Asn Asn Ile Thr Thr Asp His Ile Gln Lys Tyr Leu Asp Glu Asn
            20                  25                  30
Lys Gln Leu Ile Leu Ala Ile Leu Asp Asn Gln Asn Leu Gly Lys Leu
        35                  40                  45
Asn Glu Cys Ala Gln Tyr Gln Ala Lys Leu Gln Gln Asn Leu Met Tyr
    50                  55                  60
Leu Ala Ala Ile Ala Asp Ser Gln Pro Gln Ala Gln Thr Ala His Ala
65                  70                  75                  80
Gln Ile Pro Pro Asn Ala Val Met Gln Ser Gly Gly His Tyr Met Gln
                85                  90                  95
His Gln Gln Ala Gln Gln Gln Val Thr Pro Gln Ser Leu Met Ala Ala
            100                 105                 110
Arg Ser Ser Ile Leu Tyr Ala Gln Gln Gln Gln Gln Gln Gln His Gln
        115                 120                 125
Gln His Gln Gln Gln Gln Gln Gln Gln Gln Ser Leu His Ser Gln Leu
    130                 135                 140
Gly Ile Asn Ser Gly Gly Ser Ser Gly Leu His Met Leu His Gly Glu
145                 150                 155                 160
Thr Asn Met Gly Cys Asn Gly Pro Leu Ser Ser Gly Gly Phe Pro Glu
                165                 170                 175
Phe Gly Arg Gly Ser Ala Thr Ser Ala Asp Gly Met Gln Val Asn Arg
            180                 185                 190
Gly Phe Ala Ile Asp Arg Gly Ser Asn Lys Gln Asp Gly Val Gly Ser
        195                 200                 205
Glu Asn Ala His Ala Gly Ala Gly Asp Gly Arg Gly Ser Ser Thr Gly
    210                 215                 220
Gly Gln Asn Ala Asp Glu Ser Glu Pro Ser Tyr Leu Lys Ala Ser Glu
225                 230                 235                 240
Glu Glu Gly Asn
```

<210> 125
<211> 663
<212> DNA
<213> Populus tremula

<400> 125

```
atgcaacagc acctgatgca gatgcagccc atgatggcag cctattaccc cagcaacgtc     60
actactgatc atattcaaca gtatctggac gaaaacaagt cattgatttt gaagattgtt    120
gagagccaga attcagggaa actcagtgag tgtgcagaga accaagcaag actgcaacaa    180
aatctcatgt acttggctgc aattgctgat tgtcagcccc aaccacctac catgcatgcc    240
cagttcccct ccagcggcat tatgcagcca ggagcacatt acatgcagca tcaacaagct    300
caacagatga caccacaagc ccttatggct gcacgctctt ctatgctgca gtatgctcaa    360
cagccattct cagcgcttca acaacagcaa gccttacaca gccagctcgg catgagctct    420
ggtggaagcg caggacttca tatgatgcaa agcgaggcta acactgcagg aggcagtgga    480
gctcttggtg ctggacgatt tcctgatttt ggcatggatg cctccagtag aggaatcgca    540
agtgggagca agcaagatat tcggagtgca gggtctagtg aagggcgagg aggaagctct    600
ggaggccagg gtggtgatgg aggtgaaacc ctttacttga aatctgctga tgatgggaac    660
tga                                                                  663
```

<210> 126
<211> 220
<212> PRT
<213> Populus tremula

<400> 126

```
Met Gln Gln His Leu Met Gln Met Gln Pro Met Met Ala Ala Tyr Tyr
```

```
1               5                   10                  15
Pro Ser Asn Val Thr Thr Asp His Ile Gln Gln Tyr Leu Asp Glu Asn
            20                  25                  30
Lys Ser Leu Ile Leu Lys Ile Val Glu Ser Gln Asn Ser Gly Lys Leu
        35                  40                  45
Ser Glu Cys Ala Glu Asn Gln Ala Arg Leu Gln Gln Asn Leu Met Tyr
    50                  55                  60
Leu Ala Ala Ile Ala Asp Cys Gln Pro Gln Pro Pro Thr Met His Ala
65                  70                  75                  80
Gln Phe Pro Ser Ser Gly Ile Met Gln Pro Gly Ala His Tyr Met Gln
                85                  90                  95
His Gln Gln Ala Gln Gln Met Thr Pro Gln Ala Leu Met Ala Ala Arg
            100                 105                 110
Ser Ser Met Leu Gln Tyr Ala Gln Gln Pro Phe Ser Ala Leu Gln Gln
        115                 120                 125
Gln Gln Ala Leu His Ser Gln Leu Gly Met Ser Ser Gly Gly Ser Ala
    130                 135                 140
Gly Leu His Met Met Gln Ser Glu Ala Asn Thr Ala Gly Gly Ser Gly
145                 150                 155                 160
Ala Leu Gly Ala Gly Arg Phe Pro Asp Phe Gly Met Asp Ala Ser Ser
                165                 170                 175
Arg Gly Ile Ala Ser Gly Ser Lys Gln Asp Ile Arg Ser Ala Gly Ser
            180                 185                 190
Ser Glu Gly Arg Gly Gly Ser Ser Gly Gly Gln Gly Gly Asp Gly Gly
        195                 200                 205
Glu Thr Leu Tyr Leu Lys Ser Ala Asp Asp Gly Asn
    210                 215                 220
```

<210> 127
<211> 678
<212> DNA
<213> Saccharum officinarum

<400> 127

```
atgcagcagc aacacctgat gcagatgaac cagaacatga ttgggggcta cacctctcct      60
gccgctgtga caaccgatct catccagcag tacctggatg agaacaagca gctgatcctg     120
gccatcctcg acaaccagaa caatggcaag gtggaggagt gcgaacggca ccaagctaag     180
ctccagcaca acctcatgta cctggccgcc atcgccgaca gccagccacc acagactgca     240
ccactatcac aatacccgtc caacctgatg atgcagccgg gccctcggta catgccaccg     300
cagtccgggc agatgatgag cccgcagtcg ctaatggcgg cgcggtcctc catgatgtac     360
gcgcacccgt ccatgtcacc actccagcag cagcaggcag cgcacgggca gctgggcatg     420
gcttcagggg gcggcggtgg cacgaccagt gggttcaaca tcctccatgg cgaggccagt     480
atgggcggtg ctggtggcgc ttgtgccggc aacaacatga tgaacgccgg catgttctca     540
ggctttggcc gcagcggcag tggcgccaag gagggatcga cctcgctgtc ggttgacgtc     600
cgtggtggca ccagctccgg cgcgcaaagc ggggacggcg agtacctgaa agcaggcacc     660
gaggaagaag gcagttaa                                                   678
```

<210> 128
<211> 225
<212> PRT
<213> Saccharum officinarum

<400> 128

```
Met Gln Gln Gln His Leu Met Gln Met Asn Gln Asn Met Ile Gly Gly
1               5                   10                  15
Tyr Thr Ser Pro Ala Ala Val Thr Thr Asp Leu Ile Gln Gln Tyr Leu
            20                  25                  30
Asp Glu Asn Lys Gln Leu Ile Leu Ala Ile Leu Asp Asn Gln Asn Asn
        35                  40                  45
```

```
Gly Lys Val Glu Glu Cys Glu Arg His Gln Ala Lys Leu Gln His Asn
    50                  55                  60
Leu Met Tyr Leu Ala Ala Ile Ala Asp Ser Gln Pro Pro Gln Thr Ala
65                  70                  75                  80
Pro Leu Ser Gln Tyr Pro Ser Asn Leu Met Met Gln Pro Gly Pro Arg
                85                  90                  95
Tyr Met Pro Pro Gln Ser Gly Gln Met Met Ser Pro Gln Ser Leu Met
            100                 105                 110
Ala Ala Arg Ser Ser Met Met Tyr Ala His Pro Ser Met Ser Pro Leu
        115                 120                 125
Gln Gln Gln Gln Ala Ala His Gly Gln Leu Gly Met Ala Ser Gly Gly
    130                 135                 140
Gly Gly Gly Thr Thr Ser Gly Phe Asn Ile Leu His Gly Glu Ala Ser
145                 150                 155                 160
Met Gly Gly Ala Gly Gly Ala Cys Ala Gly Asn Asn Met Met Asn Ala
                165                 170                 175
Gly Met Phe Ser Gly Phe Gly Arg Ser Gly Ser Gly Ala Lys Glu Gly
            180                 185                 190
Ser Thr Ser Leu Ser Val Asp Val Arg Gly Gly Thr Ser Ser Gly Ala
        195                 200                 205
Gln Ser Gly Asp Gly Glu Tyr Leu Lys Ala Gly Thr Glu Glu Glu Gly
    210                 215                 220
Ser
225
```

<210> 129
<211> 561
<212> DNA
<213> Saccharum officinarum

<400> 129

```
atgcagcagc cgatgcccat gcagccgcag gcgccggaga tgaccccggc cgccggaatc      60
accacggagc agatccaaaa gtatctggat gagaataagc agcttatttt ggctattttg     120
gaaaatcaga acctaggaaa attggcagaa tgtgctcagt atcaatcaca acttcagaag     180
aacctcttgt atctcgctgc aatcgcagat gcccaaccac agactgctgt aagccgccct     240
cagatggcgc cgcctggtgc attgcctgga gtagggcagt acatgtcaca ggtgcctatg     300
ttcccaccga ggacacctct aacaccccag cagatgcagg agcagcaact tcagcagcag     360
caggctcagc tgctaaattt cagtggccta atggttgcta gacctggcat ggtcaacggc     420
atgcctcagt ccattcaagt tcagcaagct cagccaccac cagcagggaa caaacaggat     480
gctggtgggg tcgcctcgga gccctcgggc attgagaacc acaggagcac tggtggtgat     540
aatgatggtg gaagcgacta g                                               561
```

<210> 130
<211> 186
<212> PRT
<213> Saccharum officinarum

<400> 130

```
Met Gln Gln Pro Met Pro Met Gln Pro Gln Ala Pro Glu Met Thr Pro
1               5                   10                  15
Ala Ala Gly Ile Thr Thr Glu Gln Ile Gln Lys Tyr Leu Asp Glu Asn
            20                  25                  30
Lys Gln Leu Ile Leu Ala Ile Leu Glu Asn Gln Asn Leu Gly Lys Leu
        35                  40                  45
Ala Glu Cys Ala Gln Tyr Gln Ser Gln Leu Gln Lys Asn Leu Leu Tyr
    50                  55                  60
Leu Ala Ala Ile Ala Asp Ala Gln Pro Gln Thr Ala Val Ser Arg Pro
65                  70                  75                  80
Gln Met Ala Pro Pro Gly Ala Leu Pro Gly Val Gly Gln Tyr Met Ser
```

```
                85                  90                  95
Gln Val Pro Met Phe Pro Pro Arg Thr Pro Leu Thr Pro Gln Gln Met
            100                 105                 110
Gln Glu Gln Gln Leu Gln Gln Gln Gln Ala Gln Leu Leu Asn Phe Ser
        115                 120                 125
Gly Leu Met Val Ala Arg Pro Gly Met Val Asn Gly Met Pro Gln Ser
    130                 135                 140
Ile Gln Val Gln Gln Ala Gln Pro Pro Pro Ala Gly Asn Lys Gln Asp
145                 150                 155                 160
Ala Gly Gly Val Ala Ser Glu Pro Ser Gly Ile Glu Asn His Arg Ser
                165                 170                 175
Thr Gly Gly Asp Asn Asp Gly Gly Ser Asp
            180                 185
```

<210> 131
<211> 642
<212> DNA
<213> Saccharum officinarum

<400> 131

```
atgcagcagc agatgcccat gccgccggcg cccgctgcgg cggcggcgcc cccggcggcc     60
ggcatcacca ccgagcagat ccaaaagtat ttggacgaaa ataagcaact tattttggcc    120
atcctggaaa atcagaactt aggaaagttg gctgaatgtg ctcagtatca agctcaactt    180
caaaagaacc tcttgtacct ggctgcgatt gctgatgccc aaccccagcc accacaaaac    240
cctgcaggtc gccctcagat gatgcaacct ggtatagtgc caggtgcggg gcattacatg    300
tcacaagtac caatgttccc tccaagaact ccattaaccc cacagcagat gcaagagcag    360
cagcagcaac agcttcagca gcagcaagcg caggctctta cattccctgg acagatggtc    420
atgagaccag ctaccatcaa cggcatacag cagcctatgc aagctgaccc tgcccgggca    480
gcggagctgc aacaaccacc acctatccca gctgacgggc gagtaagcaa gcagcaggac    540
acaacggctg gcgtgagctc agagccttct gccaatgaga gccacaagac cacaactgga    600
gcagatagtg aggcaggtgg tgacgtggcg gagaaatcct aa                        642
```

<210> 132
<211> 213
<212> PRT
<213> Saccharum officinarum

<400> 132

```
Met Gln Gln Gln Met Pro Met Pro Pro Ala Pro Ala Ala Ala Ala Ala
1               5                   10                  15
Pro Pro Ala Ala Gly Ile Thr Thr Glu Gln Ile Gln Lys Tyr Leu Asp
            20                  25                  30
Glu Asn Lys Gln Leu Ile Leu Ala Ile Leu Glu Asn Gln Asn Leu Gly
        35                  40                  45
Lys Leu Ala Glu Cys Ala Gln Tyr Gln Ala Gln Leu Gln Lys Asn Leu
    50                  55                  60
Leu Tyr Leu Ala Ala Ile Ala Asp Ala Gln Pro Gln Pro Pro Gln Asn
65                  70                  75                  80
Pro Ala Gly Arg Pro Gln Met Met Gln Pro Gly Ile Val Pro Gly Ala
                85                  90                  95
Gly His Tyr Met Ser Gln Val Pro Met Phe Pro Pro Arg Thr Pro Leu
            100                 105                 110
Thr Pro Gln Gln Met Gln Glu Gln Gln Gln Gln Gln Leu Gln Gln Gln
        115                 120                 125
Gln Ala Gln Ala Leu Thr Phe Pro Gly Gln Met Val Met Arg Pro Ala
    130                 135                 140
Thr Ile Asn Gly Ile Gln Gln Pro Met Gln Ala Asp Pro Ala Arg Ala
145                 150                 155                 160
Ala Glu Leu Gln Gln Pro Pro Pro Ile Pro Ala Asp Gly Arg Val Ser
```

```
                165                 170                 175
Lys Gln Gln Asp Thr Thr Ala Gly Val Ser Ser Glu Pro Ser Ala Asn
            180                 185                 190
Glu Ser His Lys Thr Thr Thr Gly Ala Asp Ser Glu Ala Gly Gly Asp
        195                 200                 205
Val Ala Glu Lys Ser
    210

<210>  133
<211>  645
<212>  DNA
<213>  Solanum tuberosum

<400>  133
atgcagcagc acctgatgca gatgcagccc atgatggcag cttactatcc aacgaacgtc      60
actactgacc atattcaaca gtatttggat gagaacaaat cactcattct gaaaattgtt     120
gagagccaaa actcgggaaa actcagtgaa tgtgcagaga accaagctag gcttcagagg     180
aatctgatgt accttgctgc tattgctgat tcacaacctc agccttctag catgcattct     240
cagttctctt ctggtgggat gatgcagcca gggacacaca gttacctgca gcagcagcag     300
cagcaacaac aagcgcaaca aatggcaaca caacaactca tggctgcaag atcctcatca     360
atgctctatg gacaacaaca gcagcagcag cagcagtctc agttatcaca atttcaacaa     420
ggcttgcata gtagccaact tggcatgagt tctggcagtg gtggaagcac tggacttcat     480
cacatgcttc aaagtgaatc atcacctcat ggtggtggtt tctctcatga cttcggccgt     540
gcaaataagc aagacattgg gagtagtatg tctgctgaag ggcgcggcgg aagctcaggt     600
ggtgatggtg gtgagaatct ttatctgaaa gcttctgagg attga                     645

<210>  134
<211>  214
<212>  PRT
<213>  Solanum tuberosum

<400>  134
Met Gln Gln His Leu Met Gln Met Gln Pro Met Met Ala Ala Tyr Tyr
1               5                   10                  15
Pro Thr Asn Val Thr Thr Asp His Ile Gln Gln Tyr Leu Asp Glu Asn
            20                  25                  30
Lys Ser Leu Ile Leu Lys Ile Val Glu Ser Gln Asn Ser Gly Lys Leu
        35                  40                  45
Ser Glu Cys Ala Glu Asn Gln Ala Arg Leu Gln Arg Asn Leu Met Tyr
    50                  55                  60
Leu Ala Ala Ile Ala Asp Ser Gln Pro Gln Pro Ser Ser Met His Ser
65                  70                  75                  80
Gln Phe Ser Ser Gly Gly Met Met Gln Pro Gly Thr His Ser Tyr Leu
                85                  90                  95
Gln Gln Gln Gln Gln Gln Gln Gln Ala Gln Gln Met Ala Thr Gln Gln
            100                 105                 110
Leu Met Ala Ala Arg Ser Ser Ser Met Leu Tyr Gly Gln Gln Gln Gln
        115                 120                 125
Gln Gln Gln Gln Ser Gln Leu Ser Gln Phe Gln Gln Gly Leu His Ser
    130                 135                 140
Ser Gln Leu Gly Met Ser Ser Gly Ser Gly Gly Ser Thr Gly Leu His
145                 150                 155                 160
His Met Leu Gln Ser Glu Ser Ser Pro His Gly Gly Gly Phe Ser His
                165                 170                 175
Asp Phe Gly Arg Ala Asn Lys Gln Asp Ile Gly Ser Ser Met Ser Ala
            180                 185                 190
Glu Gly Arg Gly Gly Ser Ser Gly Gly Asp Gly Gly Glu Asn Leu Tyr
        195                 200                 205
Leu Lys Ala Ser Glu Asp
```

210

<210> 135
<211> 645
<212> DNA
<213> Sorghum bicolor

<400> 135
atgcagcagc agatgcccat gccgccggcg cccgctgcgg cggcggcgac ggcgcccccg 60
gcggccggca tcaccaccga gcagatccag aagtatttgg acgaaaataa gcaacttatt 120
ttggccatcc tagaaaatca gaacttagga aagttggctg aatgtgctca gtatcaagct 180
caacttcaaa agaacctctt gtacctggct gcgattgctg atgcccaacc ccgaccaccg 240
caaaaccctg caggtcgccc tcagatgatg caacctggta tagtgccagg tgcagggcat 300
tacatgtcac aagtaccaat gttccctcca agaactccat taaccccaca gcaaatgcaa 360
gagcagcagc agcaacagct tcagcagcag caagcgcagg ctcttgcatt ccctgggcag 420
atggtcatga gaccagctac catcaacggc atgcagcagc ctatgcaggc tgaccctgcc 480
cgggcagcgg agctgcaaca gccagcatct gtcccagccg acgggcgagt aagcaagcag 540
gacacagcgg ctggggtgag ctcagagcct tctgccaatg agagccacaa gaccacaacc 600
ggagcagata gtgaggcagg tggagacgtg gcggagaaat cctaa 645

<210> 136
<211> 214
<212> PRT
<213> Sorghum bicolor

<400> 136
Met Gln Gln Gln Met Pro Met Pro Pro Ala Pro Ala Ala Ala Ala Ala
1 5 10 15
Thr Ala Pro Pro Ala Ala Gly Ile Thr Thr Glu Gln Ile Gln Lys Tyr
20 25 30
Leu Asp Glu Asn Lys Gln Leu Ile Leu Ala Ile Leu Glu Asn Gln Asn
35 40 45
Leu Gly Lys Leu Ala Glu Cys Ala Gln Tyr Gln Ala Gln Leu Gln Lys
50 55 60
Asn Leu Leu Tyr Leu Ala Ala Ile Ala Asp Ala Gln Pro Arg Pro Pro
65 70 75 80
Gln Asn Pro Ala Gly Arg Pro Gln Met Met Gln Pro Gly Ile Val Pro
85 90 95
Gly Ala Gly His Tyr Met Ser Gln Val Pro Met Phe Pro Pro Arg Thr
100 105 110
Pro Leu Thr Pro Gln Gln Met Gln Glu Gln Gln Gln Gln Gln Leu Gln
115 120 125
Gln Gln Gln Ala Gln Ala Leu Ala Phe Pro Gly Gln Met Val Met Arg
130 135 140
Pro Ala Thr Ile Asn Gly Met Gln Gln Pro Met Gln Ala Asp Pro Ala
145 150 155 160
Arg Ala Ala Glu Leu Gln Gln Pro Ala Ser Val Pro Ala Asp Gly Arg
165 170 175
Val Ser Lys Gln Asp Thr Ala Ala Gly Val Ser Ser Glu Pro Ser Ala
180 185 190
Asn Glu Ser His Lys Thr Thr Thr Gly Ala Asp Ser Glu Ala Gly Gly
195 200 205
Asp Val Ala Glu Lys Ser
210

<210> 137
<211> 558
<212> DNA
<213> Triticum aestivum

<400> 137
atgcagcaag cgatgcccat gccgccggcg gcggcggcgc cggggatgcc tccgtctgct 60
ggcctcagca ccgagcagat ccaaaagtac ctggatgaaa ataagcaact aattttggct 120
atcttggaaa atcagaacct gggaaagttg gcggaatgtg ctcagtatca agctcagctt 180
cagaagaatc ttttgtattt ggctgcaatc gctgatactc agccacagac cactgtaagc 240
cgtcctcaga tggcaccacc tagtgcatcc ccaggggcag ggcattacat gtcacaggtg 300
ccaatgttcc ctccgaggac ccctctaacg cctcagcaga tgcaggagca gcaactacag 360
cagcaacagg ctcagatgct tccgtttgct ggtcaaatgg ttgcgagacc tggggctgtc 420
aatggcatgc ctcaggcccc tcaagttgaa ccagcctatg cagcaggtgg ggccagttct 480
gagccttctg gcactgagag ccacaggagc actggtgccg ataatgacgg ggggagcggc 540
tgggctgatc agtcctaa 558

<210> 138
<211> 185
<212> PRT
<213> Triticum aestivum

<400> 138
Met Gln Gln Ala Met Pro Met Pro Pro Ala Ala Ala Ala Pro Gly Met
1 5 10 15
Pro Pro Ser Ala Gly Leu Ser Thr Glu Gln Ile Gln Lys Tyr Leu Asp
20 25 30
Glu Asn Lys Gln Leu Ile Leu Ala Ile Leu Glu Asn Gln Asn Leu Gly
35 40 45
Lys Leu Ala Glu Cys Ala Gln Tyr Gln Ala Gln Leu Gln Lys Asn Leu
50 55 60
Leu Tyr Leu Ala Ala Ile Ala Asp Thr Gln Pro Gln Thr Thr Val Ser
65 70 75 80
Arg Pro Gln Met Ala Pro Pro Ser Ala Ser Pro Gly Ala Gly His Tyr
85 90 95
Met Ser Gln Val Pro Met Phe Pro Pro Arg Thr Pro Leu Thr Pro Gln
100 105 110
Gln Met Gln Glu Gln Gln Leu Gln Gln Gln Gln Ala Gln Met Leu Pro
115 120 125
Phe Ala Gly Gln Met Val Ala Arg Pro Gly Ala Val Asn Gly Met Pro
130 135 140
Gln Ala Pro Gln Val Glu Pro Ala Tyr Ala Ala Gly Gly Ala Ser Ser
145 150 155 160
Glu Pro Ser Gly Thr Glu Ser His Arg Ser Thr Gly Ala Asp Asn Asp
165 170 175
Gly Gly Ser Gly Trp Ala Asp Gln Ser
180 185

<210> 139
<211> 603
<212> DNA
<213> Triticum aestivum

<400> 139
atgcagcagg cgatgtcctt gcccccggga gcggtcggcg cggtgtcctc gccggccggc 60
atcaccaccg agcagatcca aaagtatttg gatgaaaata agcaacttat tttggccatc 120
cttgaaaatc agaacctagg aaagttggct gaatgtgctc agtatcaagc tcaactccaa 180
aagaatctct tgtatctagc tgctatcgcg gatgcccaac caccacagaa ccctacaagt 240
caccctcaga tggtgcagcc tggtagtatg caaggtgcag ggcattacat gtcacaagta 300
ccaatgttcc ctccaagaac gcctttaacc ccacagcaga tgcaagagca gcagcaccag 360
cagcttcagc agcagcaagc ccaggccctt tctttccccg cccaggtggt catgagacca 420
ggcaccgtca acggcatgca gcagcctatg caagcagccg gcgacctcca gccagcagca 480
gcacctggag ggagcaagca ggacgccgca gtggctgggg ccagctcgga accatctggc 540

accaagagcc acaagaacgc gggagcagag gaggtgggcg ctgatgtagc agaacaatcc 600
taa 603

<210> 140
<211> 200
<212> PRT
<213> Triticum aestivum

<400> 140
Met Gln Gln Ala Met Ser Leu Pro Pro Gly Ala Val Gly Ala Val Ser
1 5 10 15
Ser Pro Ala Gly Ile Thr Thr Glu Gln Ile Gln Lys Tyr Leu Asp Glu
20 25 30
Asn Lys Gln Leu Ile Leu Ala Ile Leu Glu Asn Gln Asn Leu Gly Lys
35 40 45
Leu Ala Glu Cys Ala Gln Tyr Gln Ala Gln Leu Gln Lys Asn Leu Leu
50 55 60
Tyr Leu Ala Ala Ile Ala Asp Ala Gln Pro Pro Gln Asn Pro Thr Ser
65 70 75 80
His Pro Gln Met Val Gln Pro Gly Ser Met Gln Gly Ala Gly His Tyr
85 90 95
Met Ser Gln Val Pro Met Phe Pro Pro Arg Thr Pro Leu Thr Pro Gln
100 105 110
Gln Met Gln Glu Gln Gln His Gln Gln Leu Gln Gln Gln Gln Ala Gln
115 120 125
Ala Leu Ser Phe Pro Ala Gln Val Val Met Arg Pro Gly Thr Val Asn
130 135 140
Gly Met Gln Gln Pro Met Gln Ala Ala Gly Asp Leu Gln Pro Ala Ala
145 150 155 160
Ala Pro Gly Gly Ser Lys Gln Asp Ala Ala Val Ala Gly Ala Ser Ser
165 170 175
Glu Pro Ser Gly Thr Lys Ser His Lys Asn Ala Gly Ala Glu Glu Val
180 185 190
Gly Ala Asp Val Ala Glu Gln Ser
195 200

<210> 141
<211> 672
<212> DNA
<213> Vitis vinifera

<400> 141
atgcagcagc acctgatgca gatgcagccc atgatggcag cctattaccc cagcaacgtc 60
accactgatc acattcagca gtatcttgat gaaaacaagt cattgattct gaagattgtt 120
gagagccaga attcaggaaa attgactgaa tgtgcagaga accaggcaag actacagaga 180
aacctcatgt acctggctgc aattgctgat tctcaacccc aaccacccac catgcatgct 240
cagttccctc ctagtggcat tgttcagcca ggagctcact acatgcaaca ccaacaagct 300
caacaaatga caccacagtc gctcctggct gcacgctcct ccatgctgta cacccaacaa 360
ccattttcgg ccctgcaaca acaacaagcc atccatagcc agcttggcat gggctctggt 420
ggaagtgcag gacttcacat gctgcaaagc gaggggagta atccaggagg caatggaaca 480
ctggggactg gtgggtttcc tgatttcagc cgtggaactt ctggagaagg cctgcaggct 540
gcaggcaggg gaatggctgg tgggagcaag caagatatgg gaaatgcaga agggcgagga 600
gggaactcag gaggtcaggg tggggatgga ggtgagactc tttacttgaa agctgctgaa 660
gatgggaatt ga 672

<210> 142
<211> 223
<212> PRT
<213> Vitis vinifera

<400> 142

Met Gln Gln His Leu Met Gln Met Gln Pro Met Met Ala Ala Tyr Tyr
1 5 10 15
Pro Ser Asn Val Thr Thr Asp His Ile Gln Gln Tyr Leu Asp Glu Asn
20 25 30
Lys Ser Leu Ile Leu Lys Ile Val Glu Ser Gln Asn Ser Gly Lys Leu
35 40 45
Thr Glu Cys Ala Glu Asn Gln Ala Arg Leu Gln Arg Asn Leu Met Tyr
50 55 60
Leu Ala Ala Ile Ala Asp Ser Gln Pro Gln Pro Pro Thr Met His Ala
65 70 75 80
Gln Phe Pro Pro Ser Gly Ile Val Gln Pro Gly Ala His Tyr Met Gln
85 90 95
His Gln Gln Ala Gln Gln Met Thr Pro Gln Ser Leu Leu Ala Ala Arg
100 105 110
Ser Ser Met Leu Tyr Thr Gln Gln Pro Phe Ser Ala Leu Gln Gln Gln
115 120 125
Gln Ala Ile His Ser Gln Leu Gly Met Gly Ser Gly Gly Ser Ala Gly
130 135 140
Leu His Met Leu Gln Ser Glu Gly Ser Asn Pro Gly Gly Asn Gly Thr
145 150 155 160
Leu Gly Thr Gly Gly Phe Pro Asp Phe Ser Arg Gly Thr Ser Gly Glu
165 170 175
Gly Leu Gln Ala Ala Gly Arg Gly Met Ala Gly Gly Ser Lys Gln Asp
180 185 190
Met Gly Asn Ala Glu Gly Arg Gly Gly Asn Ser Gly Gly Gln Gly Gly
195 200 205
Asp Gly Gly Glu Thr Leu Tyr Leu Lys Ala Ala Glu Asp Gly Asn
210 215 220

<210> 143
<211> 663
<212> DNA
<213> Zea mays

<400> 143

atgcagcagc agatgcccat gccgccggcg cccgctgccg ccgcggcggc ggcgcccccg 60
gcggcaggca tcactaccga gcagatccag aagtatttgg acgaaaataa gcaacttatt 120
ttggccatcc tggaaaatca gaacttaggg aagttggctg aatgtgctca gtatcaagct 180
caacttcaaa agaacctctt gtacctggct gcgattgctg atgcccaacc ccagcctccg 240
caaaaccctg caggtcgccc tcagatgatg cagcctggta tagtgccagg tgcggggcat 300
tacatgtcac aagtaccaat gttccctcca agaaccccat taaccccaca gcagatgcag 360
gagcagcagc aacaacaaca gtttcagcag cagcagcagc aagtgcaggc tcttacattt 420
cctggacaga tggtcatgag accaggcacc atcaacggca tgcagcagca gcagcctatg 480
caggctgacc ctgcccgggc agcagcggag ctgcagcagg cagcacctat cccagctgac 540
gggcgaggaa gcaagcagga caccgcgggt ggggcgagct cagagccttc tgccaatgag 600
agccacaaga gcgccaccgg agcagatacc gaggcaggtg gcgacgtggc cgagaaatcc 660
taa 663

<210> 144
<211> 220
<212> PRT
<213> Zea mays

<400> 144

Met Gln Gln Gln Met Pro Met Pro Pro Ala Pro Ala Ala Ala Ala Ala
1 5 10 15
Ala Ala Pro Pro Ala Ala Gly Ile Thr Thr Glu Gln Ile Gln Lys Tyr

```
            20                  25                  30
Leu Asp Glu Asn Lys Gln Leu Ile Leu Ala Ile Leu Glu Asn Gln Asn
        35                  40                  45
Leu Gly Lys Leu Ala Glu Cys Ala Gln Tyr Gln Ala Gln Leu Gln Lys
    50                  55                  60
Asn Leu Leu Tyr Leu Ala Ala Ile Ala Asp Ala Gln Pro Gln Pro Pro
65                  70                  75                  80
Gln Asn Pro Ala Gly Arg Pro Gln Met Met Gln Pro Gly Ile Val Pro
                85                  90                  95
Gly Ala Gly His Tyr Met Ser Gln Val Pro Met Phe Pro Pro Arg Thr
            100                 105                 110
Pro Leu Thr Pro Gln Gln Met Gln Glu Gln Gln Gln Gln Gln Gln Phe
        115                 120                 125
Gln Gln Gln Gln Gln Gln Val Gln Ala Leu Thr Phe Pro Gly Gln Met
    130                 135                 140
Val Met Arg Pro Gly Thr Ile Asn Gly Met Gln Gln Gln Gln Pro Met
145                 150                 155                 160
Gln Ala Asp Pro Ala Arg Ala Ala Ala Glu Leu Gln Gln Ala Ala Pro
                165                 170                 175
Ile Pro Ala Asp Gly Arg Gly Ser Lys Gln Asp Thr Ala Gly Gly Ala
            180                 185                 190
Ser Ser Glu Pro Ser Ala Asn Glu Ser His Lys Ser Ala Thr Gly Ala
        195                 200                 205
Asp Thr Glu Ala Gly Gly Asp Val Ala Glu Lys Ser
    210                 215                 220
```

<210> 145
<211> 2193
<212> DNA
<213> Oryza sativa

<400> 145

```
aatccgaaaa gtttctgcac cgttttcacc ccctaactaa caatataggg aacgtgtgct      60
aaatataaaa tgagacctta tatatgtagc gctgataact agaactatgc aagaaaaact     120
catccaccta ctttagtggc aatcgggcta aataaaaaag agtcgctaca ctagtttcgt     180
tttccttagt aattaagtgg gaaaatgaaa tcattattgc ttagaatata cgttcacatc     240
tctgtcatga agttaaatta ttcgaggtag ccataattgt catcaaactc ttcttgaata     300
aaaaaatctt tctagctgaa ctcaatgggt aaagagagag atttttttta aaaaaataga     360
atgaagatat tctgaacgta ttggcaaaga tttaaacata taattatata attttatagt     420
ttgtgcattc gtcatatcgc acatcattaa ggacatgtct tactccatcc caatttttat     480
ttagtaatta aagacaattg acttattttt attatttatc ttttttcgat tagatgcaag     540
gtacttacgc acacactttg tgctcatgtg catgtgtgag tgcacctcct caatacacgt     600
tcaactagca acacatctct aatatcactc gcctatttaa tacatttagg tagcaatatc     660
tgaattcaag cactccacca tcaccagacc acttttaata atatctaaaa tacaaaaaat     720
aattttacag aatagcatga aaagtatgaa acgaactatt taggtttttc acatacaaaa     780
aaaaaaagaa ttttgctcgt gcgcgagcgc caatctccca tattgggcac acaggcaaca     840
acagagtggc tgcccacaga acaacccaca aaaaacgatg atctaacgga ggacagcaag     900
tccgcaacaa ccttttaaca gcaggctttg cggccaggag agaggaggag aggcaaagaa     960
aaccaagcat cctcctcctc ccatctataa attcctcccc cctttcccc tctctatata     1020
ggaggcatcc aagccaagaa gagggagagc accaaggaca cgcgactagc agaagccgag    1080
cgaccgcctt cttcgatcca tatcttccgg tcgagttctt ggtcgatctc ttccctcctc    1140
cacctcctcc tcacagggta tgtgcccttc ggttgttctt ggatttattg ttctaggttg    1200
tgtagtacgg gcgttgatgt taggaaaggg gatctgtatc tgtgatgatt cctgttcttg    1260
gatttgggat agaggggttc ttgatgttgc atgttatcgg ttcggtttga ttagtagtat    1320
ggttttcaat cgtctggaga gctctatgga aatgaaatgg tttagggtac ggaatcttgc    1380
gattttgtga gtaccttttg tttgaggtaa aatcagagca ccggtgattt tgcttggtgt    1440
aataaaagta cggttgtttg gtcctcgatt ctggtagtga tgcttctcga tttgacgaag    1500
ctatcctttg tttattccct attgaacaaa aataatccaa ctttgaagac ggtcccgttg    1560
atgagattga atgattgatt cttaagcctg tccaaaattt cgcagctggc ttgtttagat    1620
```

```
acagtagtcc ccatcacgaa attcatggaa acagttataa tcctcaggaa caggggattc   1680
cctgttcttc cgatttgctt tagtcccaga attttttttc ccaaatatct taaaaagtca   1740
ctttctggtt cagttcaatg aattgattgc tacaaataat gcttttatag cgttatccta   1800
gctgtagttc agttaatagg taatacccct atagtttagt caggagaaga acttatccga   1860
tttctgatct ccatttttaa ttatatgaaa tgaactgtag cataagcagt attcatttgg   1920
attatttttt ttattagctc tcaccccttc attattctga gctgaaagtc tggcatgaac   1980
tgtcctcaat tttgttttca aattcacatc gattatctat gcattatcct cttgtatcta   2040
cctgtagaag tttctttttg gttattcctt gactgcttga ttacagaaag aaatttatga   2100
agctgtaatc gggatagtta tactgcttgt tcttatgatt catttccttt gtgcagttct   2160
tggtgtagct tgccactttc accagcaaag ttc                                2193
```

<210> 146
<211> 12
<212> PRT
<213> Artificial sequence

<220>
<223> Box I

<220>
<221> VARIANT
<222> (3)..(3)
<223> /replace = "Lys"

<220>
<221> VARIANT
<222> (4)..(4)
<223> /replace = "Met" /replace = "Phe" /replace = "His"

<220>
<221> VARIANT
<222> (6)..(6)
<223> /replace = "Glu"

<220>
<221> VARIANT
<222> (7)..(7)
<223> /replace = "Asp"

<220>
<221> VARIANT
<222> (9)..(9)
<223> /replace = "Asn"

<220>
<221> VARIANT
<222> (10)..(10)
<223> Xaa can be any naturally occurring amino acid

<400> 146

```
Ile Gln Gln Tyr Leu Asp Glu Asn Lys Xaa Leu Ile
1               5                   10
```

<210> 147
<211> 10
<212> PRT
<213> Artificial sequence

<220>

<223> Box II

<220>
<221> VARIANT
<222> (3)..(3)
<223> /replace= "Leu" /replace= "Val"

<220>
<221> VARIANT
<222> (7)..(7)
<223> /replace= "Thr"

<400> 147
Asn Leu Met Tyr Leu Ala Ala Ile Ala Asp
1 5 10

<210> 148
<211> 53
<212> DNA
<213> Artificial sequence

<220>
<223> primer: prm06681

<400> 148
ggggacaagt ttgtacaaaa aagcaggctt aaacaatgca acagcacctg atg 53

<210> 149
<211> 50
<212> DNA
<213> Artificial sequence

<220>
<223> primer: prm06682

<400> 149
ggggaccact ttgtacaaga aagctgggtc atcattaaga ttccttgtgc 50

<210> 150
<211> 615
<212> DNA
<213> Brassica napus

<400> 150
atgcaacagc acctgatgca gatgcagccc atgatggctg gttactaccc cagcaatgtc 60
acctctgatc atattcagca gtacttggac gagaacaaat cgttgattct gaagatagtt 120
gaatctcaaa actcgggaaa gctcagcgag tgtgccgaga accaggcaag gcttcaacgc 180
aacttaatgt acttagctgc aattgcagat tctcagcctc aacctccaag catgcatagc 240
cagtatggaa ctgctggtgg tggtgggttg atgcagggag aaggagggtc acactatttg 300
caacagcaac aggcaattca acagcagcag agtcagcagt ctctaatggc ggctcgatct 360
tcaatgttgt atgctcagca gcagcaacag ccttatgcaa cgcttcagca gcagcaattg 420
caccatagcc agcttgggat gagctcaagc agcggaggag gaagcagcgg tctccatatg 480
ctacagggag aggctggtgg gtttcatgat tttggccgtg agaagttgga aatgggaagt 540
ggtgaaggca gaggaggaag ctcaggggat ggtggagaaa ccctttactt gaagtcatca 600
gatgatggga actga 615

<210> 151
<211> 204
<212> PRT

<213> Brassica napus

<400> 151

```
Met Gln Gln His Leu Met Gln Met Gln Pro Met Met Ala Gly Tyr Tyr
1               5                   10                  15
Pro Ser Asn Val Thr Ser Asp His Ile Gln Gln Tyr Leu Asp Glu Asn
            20                  25                  30
Lys Ser Leu Ile Leu Lys Ile Val Glu Ser Gln Asn Ser Gly Lys Leu
        35                  40                  45
Ser Glu Cys Ala Glu Asn Gln Ala Arg Leu Gln Arg Asn Leu Met Tyr
    50                  55                  60
Leu Ala Ala Ile Ala Asp Ser Gln Pro Gln Pro Pro Ser Met His Ser
65                  70                  75                  80
Gln Tyr Gly Thr Ala Gly Gly Gly Gly Leu Met Gln Gly Glu Gly Gly
                85                  90                  95
Ser His Tyr Leu Gln Gln Gln Gln Ala Ile Gln Gln Gln Gln Ser Gln
            100                 105                 110
Gln Ser Leu Met Ala Ala Arg Ser Ser Met Leu Tyr Ala Gln Gln Gln
        115                 120                 125
Gln Gln Pro Tyr Ala Thr Leu Gln Gln Gln Gln Leu His His Ser Gln
    130                 135                 140
Leu Gly Met Ser Ser Ser Ser Gly Gly Gly Ser Ser Gly Leu His Met
145                 150                 155                 160
Leu Gln Gly Glu Ala Gly Gly Phe His Asp Phe Gly Arg Glu Lys Leu
                165                 170                 175
Glu Met Gly Ser Gly Glu Gly Arg Gly Gly Ser Ser Gly Asp Gly Gly
            180                 185                 190
Glu Thr Leu Tyr Leu Lys Ser Ser Asp Asp Gly Asn
        195                 200
```

<210> 152
<211> 639
<212> DNA
<213> Glycine max

<400> 152

```
atgcagcagc acctgatgca gatgcagccc atgatggctg cctactaccc caacaacgtc      60
accactgatc acattcaaca gtacctggat gagaacaagt ccttgattct gaagattgtt     120
gaaagccaga attctggcaa gctgagcgag tgtgccgaga accaatcaag gctgcagaga     180
aatctcatgt acctagctgc aatagctgat tctcaaccac aaccatctcc attggctggt     240
cagtatcctt ctagtggact tgtgcagcag ggagcacact acatgcaggc tcaacaggct     300
cagcagatgt cacaacaaca gctaatggct tcgcgctcct cgctcctgta ctcccaacag     360
cctttctcag tgcttcaaca gcagcaaggc atgcacagcc aacttggcat gagctccagt     420
ggaagtcaag gcctccacat gctgcaaagt gaagccacta atgttggagg caatgcaacc     480
ataggaaccg gaggagggtt tccggacttt gtacgcattg gtagtggcaa gcaagatatt     540
ggaatctctg gtgaaggcag aggaggaaac tctagtggcc actctggtga tggtggtgag     600
acacttaatt acctgaaagc tgctggtgat ggaaactga                            639
```

<210> 153
<211> 212
<212> PRT
<213> Glycine max

<400> 153

```
Met Gln Gln His Leu Met Gln Met Gln Pro Met Met Ala Ala Tyr Tyr
1               5                   10                  15
Pro Asn Asn Val Thr Thr Asp His Ile Gln Gln Tyr Leu Asp Glu Asn
            20                  25                  30
Lys Ser Leu Ile Leu Lys Ile Val Glu Ser Gln Asn Ser Gly Lys Leu
```

```
        35                  40                  45
Ser Glu Cys Ala Glu Asn Gln Ser Arg Leu Gln Arg Asn Leu Met Tyr
    50                  55                  60
Leu Ala Ala Ile Ala Asp Ser Gln Pro Gln Pro Ser Pro Leu Ala Gly
65                  70                  75                  80
Gln Tyr Pro Ser Ser Gly Leu Val Gln Gln Gly Ala His Tyr Met Gln
                85                  90                  95
Ala Gln Gln Ala Gln Gln Met Ser Gln Gln Gln Leu Met Ala Ser Arg
            100                 105                 110
Ser Ser Leu Leu Tyr Ser Gln Gln Pro Phe Ser Val Leu Gln Gln Gln
        115                 120                 125
Gln Gly Met His Ser Gln Leu Gly Met Ser Ser Ser Gly Ser Gln Gly
    130                 135                 140
Leu His Met Leu Gln Ser Glu Ala Thr Asn Val Gly Gly Asn Ala Thr
145                 150                 155                 160
Ile Gly Thr Gly Gly Gly Phe Pro Asp Phe Val Arg Ile Gly Ser Gly
                165                 170                 175
Lys Gln Asp Ile Gly Ile Ser Gly Glu Gly Arg Gly Gly Asn Ser Ser
            180                 185                 190
Gly His Ser Gly Asp Gly Gly Glu Thr Leu Asn Tyr Leu Lys Ala Ala
        195                 200                 205
Gly Asp Gly Asn
    210
```

<210> 154
<211> 1248
<212> DNA
<213> Chlamydomonas reinhardtii

<400> 154

```
atggccgcca ccatgctccg ctccagcacc cagtcgggca ttgccgctaa ggccggccgc     60
aaggaggctg tcagcgtccg cgcggtcgcc cagccccagc gccaggctgg tgctgccagc    120
gtcttctcct cgtcgtcgtc gggcgctgct gctcgccgcg gggtcgtcgc tcaggccacc    180
gctgttgcca cccccgcggc caagcctgcg gccaagacca gccagtatga gctgttcacg    240
ctcaccacct ggctgctgaa ggaggagatg aagggcacaa tcgatggcga gcttgtgacc    300
gtcatctcgt cggtgtcgct ggcctgcaag caaatcgcgt cgctggtgaa ccgcgctggt    360
atctccaacc tgaccggtgt ggctggcaac cagaacgtgc agggtgagga ccagaagaag    420
ctggacgtgg tgtccaacga ggtcttcaag aactgcctgg cctcctgcgg ccgcacgggt    480
gtgatcgcct ccgaggagga ggaccagccc gtggccgtgg aggagaccta ctcgggcaac    540
tacatcgtgg tgttcgaccc cctggacggc tcgtccaaca tcgacgccgg catctccgtc    600
ggctccatct tcggcatcta cgagcccagc gaggagtgcc ccattgacgc catggacgac    660
ccccagaaga tgatggagca gtgcgtcatg aacgtgtgcc agcccggctc gcgcctcaag    720
tgcgccggct actgcctgta cagcagcagc accatcatgg tgctgaccat cggcaacggt    780
gtgttcggct tcacgctgga cccccctggtc ggcgagttcg tgctgaccca ccccaacgtg    840
cagatccccg aggtgggcaa gatctacccg ttcaacgagg gcaactacgg cctgtgggac    900
gacagcgtta aggcttacat ggacagcctg aaggacccca agaagtggga cggcaagccc    960
tactcggccc gctacatcgg ctccctggtc ggtgacttcc accgcaccct gctgtacgga   1020
ggcatctacg gctaccccgg cgacgccaag aacaagaacg gcaagctccg cctgctgtac   1080
gagtgcgcgc ccatgtcgtt cattgccgag caggccggcg gcctcggctc caccggccag   1140
gagcgcgtgc tggacgtgaa ccccgagaag gtgcaccagc gcgtgccgct gttcatcggc   1200
tccaagaagg aggtcgagta cctggagtcc ttcaccaaga agcactaa                 1248
```

<210> 155
<211> 415
<212> PRT
<213> Chlamydomonas reinhardtii

<400> 155

```
Met Ala Ala Thr Met Leu Arg Ser Ser Thr Gln Ser Gly Ile Ala Ala
```

```
1               5                   10                  15
Lys Ala Gly Arg Lys Glu Ala Val Ser Val Arg Ala Val Ala Gln Pro
            20                  25                  30
Gln Arg Gln Ala Gly Ala Ala Ser Val Phe Ser Ser Ser Ser Ser Gly
        35                  40                  45
Ala Ala Ala Arg Arg Gly Val Val Ala Gln Ala Thr Ala Val Ala Thr
    50                  55                  60
Pro Ala Ala Lys Pro Ala Ala Lys Thr Ser Gln Tyr Glu Leu Phe Thr
65                  70                  75                  80
Leu Thr Thr Trp Leu Leu Lys Glu Glu Met Lys Gly Thr Ile Asp Gly
                85                  90                  95
Glu Leu Val Thr Val Ile Ser Ser Val Ser Leu Ala Cys Lys Gln Ile
            100                 105                 110
Ala Ser Leu Val Asn Arg Ala Gly Ile Ser Asn Leu Thr Gly Val Ala
        115                 120                 125
Gly Asn Gln Asn Val Gln Gly Glu Asp Gln Lys Lys Leu Asp Val Val
    130                 135                 140
Ser Asn Glu Val Phe Lys Asn Cys Leu Ala Ser Cys Gly Arg Thr Gly
145                 150                 155                 160
Val Ile Ala Ser Glu Glu Glu Asp Gln Pro Val Ala Val Glu Glu Thr
                165                 170                 175
Tyr Ser Gly Asn Tyr Ile Val Val Phe Asp Pro Leu Asp Gly Ser Ser
            180                 185                 190
Asn Ile Asp Ala Gly Ile Ser Val Gly Ser Ile Phe Gly Ile Tyr Glu
        195                 200                 205
Pro Ser Glu Glu Cys Pro Ile Asp Ala Met Asp Asp Pro Gln Lys Met
    210                 215                 220
Met Glu Gln Cys Val Met Asn Val Cys Gln Pro Gly Ser Arg Leu Lys
225                 230                 235                 240
Cys Ala Gly Tyr Cys Leu Tyr Ser Ser Ser Thr Ile Met Val Leu Thr
                245                 250                 255
Ile Gly Asn Gly Val Phe Gly Phe Thr Leu Asp Pro Leu Val Gly Glu
            260                 265                 270
Phe Val Leu Thr His Pro Asn Val Gln Ile Pro Glu Val Gly Lys Ile
        275                 280                 285
Tyr Pro Phe Asn Glu Gly Asn Tyr Gly Leu Trp Asp Asp Ser Val Lys
    290                 295                 300
Ala Tyr Met Asp Ser Leu Lys Asp Pro Lys Lys Trp Asp Gly Lys Pro
305                 310                 315                 320
Tyr Ser Ala Arg Tyr Ile Gly Ser Leu Val Gly Asp Phe His Arg Thr
                325                 330                 335
Leu Leu Tyr Gly Gly Ile Tyr Gly Tyr Pro Gly Asp Ala Lys Asn Lys
            340                 345                 350
Asn Gly Lys Leu Arg Leu Leu Tyr Glu Cys Ala Pro Met Ser Phe Ile
        355                 360                 365
Ala Glu Gln Ala Gly Gly Leu Gly Ser Thr Gly Gln Glu Arg Val Leu
    370                 375                 380
Asp Val Asn Pro Glu Lys Val His Gln Arg Val Pro Leu Phe Ile Gly
385                 390                 395                 400
Ser Lys Lys Glu Val Glu Tyr Leu Glu Ser Phe Thr Lys Lys His
                405                 410                 415
```

<210> 156
<211> 1239
<212> DNA
<213> Bigelowiella natans

<400> 156
atggctgccg tgctctttcg cggaggtgtg ctgtcttcca ccatgactgc ggctcgcaca 60

ttcgcagcgc catctgtgca cacccgtggc atgcacatgt cagtcaagag cagtaaccct 120
ttctctcagg ctggtcgtcg tgctgctgtt agatccagtg tcgcaccctc ccctgtccaa 180
gactctgctg gtactatcgt tgatgacgga actgtcacat taacaagatt catgattgag 240
gaggcaatga agagcaagac tcctgggcag gaggacatgg ttcgtttgat ttcttccatc 300
tcagttgcat gcaaacgcat tgcatccatg gtgcaaactg caggaatctc cggatctaca 360
ggtctggctg aaggtggtgg atcagtcaac gttcaaggcg aggaacagaa gaaacttgat 420
gtcatttcta acgatgtact aaagtctgcc cttcgtcctt ctggaaaact tggagtaatt 480
gcctctgagg aggaagataa tccagttgtc gttgatgaac tttactccgg cgaatatgtt 540
gctactttcg atccgctcga tggatcttcc aatattgatg ctgcaatttc cactggaact 600
atcttcggag tgttcaaagc tccagaagag tgcttgatcg gggattctga taatctcagt 660
attgcagagc agcaatgttt ggaggcaaca cttcaacctg gaactaacct tgttgctgct 720
ggatactgca tgtattcgtc ctccaccatc cttgttctca ccaccggaga cgggctcaat 780
ggatttactc ttgacccttc cattggagag ttcatcctca cccatcctaa tatccagatt 840
cctagccgtg gaaagatcta ttctatgaac gaagcaaact acttcgattg ggaccctaag 900
cttcagacct acgttgataa ccttaagaag gcagaaggtc aaactggaga aaagtacagc 960
tctcgctaca tcggatccat ggtcggagat gtgcaccgta cccttctcta tggaggcatc 1020
ttcgcttacc ctggagataa gaagaacgtc aacggaaaac ttcgccttct gtacgaagct 1080
gctccaatgt cccttatctt cgaacaagcg ggtggaaaat ctattactgg acctggtgga 1140
cgtgtgttgg acttagttcc tgataaggtt caccagcgtt gtcctgtgtt cattggatcc 1200
cctgatgatg tggatgaagt tgaaaaggct ctcgcataa 1239

<210> 157
<211> 412
<212> PRT
<213> Bigelowiella natans

<400> 157
Met Ala Ala Val Leu Phe Arg Gly Gly Val Leu Ser Ser Thr Met Thr
1 5 10 15
Ala Ala Arg Thr Phe Ala Ala Pro Ser Val His Thr Arg Gly Met His
20 25 30
Met Ser Val Lys Ser Ser Asn Pro Phe Ser Gln Ala Gly Arg Arg Ala
35 40 45
Ala Val Arg Ser Ser Val Ala Pro Ser Pro Val Gln Asp Ser Ala Gly
50 55 60
Thr Ile Val Asp Asp Gly Thr Val Thr Leu Thr Arg Phe Met Ile Glu
65 70 75 80
Glu Ala Met Lys Ser Lys Thr Pro Gly Gln Glu Asp Met Val Arg Leu
85 90 95
Ile Ser Ser Ile Ser Val Ala Cys Lys Arg Ile Ala Ser Met Val Gln
100 105 110
Thr Ala Gly Ile Ser Gly Ser Thr Gly Leu Ala Glu Gly Gly Gly Ser
115 120 125
Val Asn Val Gln Gly Glu Glu Gln Lys Lys Leu Asp Val Ile Ser Asn
130 135 140
Asp Val Leu Lys Ser Ala Leu Arg Pro Ser Gly Lys Leu Gly Val Ile
145 150 155 160
Ala Ser Glu Glu Glu Asp Asn Pro Val Val Val Asp Glu Leu Tyr Ser
165 170 175
Gly Glu Tyr Val Ala Thr Phe Asp Pro Leu Asp Gly Ser Ser Asn Ile
180 185 190
Asp Ala Ala Ile Ser Thr Gly Thr Ile Phe Gly Val Phe Lys Ala Pro
195 200 205
Glu Glu Cys Leu Ile Gly Asp Ser Asp Asn Leu Ser Ile Ala Glu Gln
210 215 220
Gln Cys Leu Glu Ala Thr Leu Gln Pro Gly Thr Asn Leu Val Ala Ala
225 230 235 240
Gly Tyr Cys Met Tyr Ser Ser Ser Thr Ile Leu Val Leu Thr Thr Gly
245 250 255

```
Asp Gly Leu Asn Gly Phe Thr Leu Asp Pro Ser Ile Gly Glu Phe Ile
            260                 265                 270
Leu Thr His Pro Asn Ile Gln Ile Pro Ser Arg Gly Lys Ile Tyr Ser
        275                 280                 285
Met Asn Glu Ala Asn Tyr Phe Asp Trp Asp Pro Lys Leu Gln Thr Tyr
    290                 295                 300
Val Asp Asn Leu Lys Lys Ala Glu Gly Gln Thr Gly Glu Lys Tyr Ser
305                 310                 315                 320
Ser Arg Tyr Ile Gly Ser Met Val Gly Asp Val His Arg Thr Leu Leu
                325                 330                 335
Tyr Gly Gly Ile Phe Ala Tyr Pro Gly Asp Lys Lys Asn Val Asn Gly
            340                 345                 350
Lys Leu Arg Leu Leu Tyr Glu Ala Ala Pro Met Ser Leu Ile Phe Glu
        355                 360                 365
Gln Ala Gly Gly Lys Ser Ile Thr Gly Pro Gly Gly Arg Val Leu Asp
    370                 375                 380
Leu Val Pro Asp Lys Val His Gln Arg Cys Pro Val Phe Ile Gly Ser
385                 390                 395                 400
Pro Asp Asp Val Asp Glu Val Glu Lys Ala Leu Ala
                405                 410
```

<210> 158
<211> 1230
<212> DNA
<213> Aquilegia formosa x Aquilegia pubescens

<400> 158

```
atggttgcag cagctatccc tacttcttgt caactgctct tctccacctc ttcttcgacc      60
acttctcgtc tatatcctcc ttatcttgat gccaaaactc tcttctcatt tcctacaaac     120
aagagacatg taagcattgt tagaactccg gcaggtgtac ggtgtcaagc attaggagca     180
gaggtagtag tgaccaagag gagtgcattt gagatacaaa ctctaacagg atggttattg     240
aaacaagaac aaacaggggt tatagatgca gagttaacta tagttttgtc tagcatttca     300
atggcctgta agcagattgc ttcattggtg cagagggcaa gtatttccaa cttaactgga     360
gttcaaggag ctgttaatat tcaaggtgaa gatcagaaga agcttgatgt catctctaac     420
gaggtgttct ctaattgcct tagatcaagt ggaagaacag ggattatagc atcagaagaa     480
gaggatgtac cagttgcagt ggaggaaagc tactctggca actatattgt cgtttttgat     540
cctcttgacg ggtcatcaaa cattgatgcc gctgtgtcaa ctggatccat atttggaatt     600
tatagtccga acgatgagtg tcttactgaa gtcgatgata atgccacagt gcttcagcaa     660
gtggaacaga agtgcatcat caatgtgtgt caacctggca acaacttgtt ggcagctggc     720
tactgcatgt actcaagctc tgtaatcttt gtgctttcca ttggacaagg ggtttctca     780
tttaccttag accctatgta cggagaattc gtcttgacac aggaaaacat tcaaatacct     840
aaatcaggta aaatctactc attcaacgaa ggcaactacc aattatggga tgataagttg     900
aagaagtata tcgatgacct taaggaccct ggtcctagtg gcaaaccata ctcttccaga     960
tacattggaa gcttggttgg cgatttccac cggacccttc tttatggagg gatatatgga    1020
taccctaggg ataagaatag aaaaaatggg aagctaaggc tactgtatga gtgtgcacct    1080
atgagttatt tagttgaaca agcaggagga aaaggatcag atggacacca aagagtactc    1140
gatattgaac cggtggagat tcatcagcgt gttccacttt tcattggcag cgttgaagaa    1200
gttgagaaac tagagaagtt cttggcttga                                     1230
```

<210> 159
<211> 409
<212> PRT
<213> Aquilegia formosa x Aquilegia pubescens

<400> 159

```
Met Val Ala Ala Ala Ile Pro Thr Ser Cys Gln Leu Leu Phe Ser Thr
1               5                   10                  15
Ser Ser Ser Thr Thr Ser Arg Leu Tyr Pro Pro Tyr Leu Asp Ala Lys
            20                  25                  30
```

```
Thr Leu Phe Ser Phe Pro Thr Asn Lys Arg His Val Ser Ile Val Arg
        35                  40                  45
Thr Pro Ala Gly Val Arg Cys Gln Ala Leu Gly Ala Glu Val Val Val
    50                  55                  60
Thr Lys Arg Ser Ala Phe Glu Ile Gln Thr Leu Thr Gly Trp Leu Leu
65                  70                  75                  80
Lys Gln Glu Gln Thr Gly Val Ile Asp Ala Glu Leu Thr Ile Val Leu
                85                  90                  95
Ser Ser Ile Ser Met Ala Cys Lys Gln Ile Ala Ser Leu Val Gln Arg
            100                 105                 110
Ala Ser Ile Ser Asn Leu Thr Gly Val Gln Gly Ala Val Asn Ile Gln
        115                 120                 125
Gly Glu Asp Gln Lys Lys Leu Asp Val Ile Ser Asn Glu Val Phe Ser
    130                 135                 140
Asn Cys Leu Arg Ser Ser Gly Arg Thr Gly Ile Ile Ala Ser Glu Glu
145                 150                 155                 160
Glu Asp Val Pro Val Ala Val Glu Glu Ser Tyr Ser Gly Asn Tyr Ile
                165                 170                 175
Val Val Phe Asp Pro Leu Asp Gly Ser Ser Asn Ile Asp Ala Ala Val
            180                 185                 190
Ser Thr Gly Ser Ile Phe Gly Ile Tyr Ser Pro Asn Asp Glu Cys Leu
        195                 200                 205
Thr Glu Val Asp Asp Asn Ala Thr Val Leu Gln Gln Val Glu Gln Lys
    210                 215                 220
Cys Ile Ile Asn Val Cys Gln Pro Gly Asn Asn Leu Leu Ala Ala Gly
225                 230                 235                 240
Tyr Cys Met Tyr Ser Ser Ser Val Ile Phe Val Leu Ser Ile Gly Gln
                245                 250                 255
Gly Val Phe Ser Phe Thr Leu Asp Pro Met Tyr Gly Glu Phe Val Leu
            260                 265                 270
Thr Gln Glu Asn Ile Gln Ile Pro Lys Ser Gly Lys Ile Tyr Ser Phe
        275                 280                 285
Asn Glu Gly Asn Tyr Gln Leu Trp Asp Asp Lys Leu Lys Lys Tyr Ile
    290                 295                 300
Asp Asp Leu Lys Asp Pro Gly Pro Ser Gly Lys Pro Tyr Ser Ser Arg
305                 310                 315                 320
Tyr Ile Gly Ser Leu Val Gly Asp Phe His Arg Thr Leu Leu Tyr Gly
                325                 330                 335
Gly Ile Tyr Gly Tyr Pro Arg Asp Lys Asn Arg Lys Asn Gly Lys Leu
            340                 345                 350
Arg Leu Leu Tyr Glu Cys Ala Pro Met Ser Tyr Leu Val Glu Gln Ala
        355                 360                 365
Gly Gly Lys Gly Ser Asp Gly His Gln Arg Val Leu Asp Ile Glu Pro
    370                 375                 380
Val Glu Ile His Gln Arg Val Pro Leu Phe Ile Gly Ser Val Glu Glu
385                 390                 395                 400
Val Glu Lys Leu Glu Lys Phe Leu Ala
                405
```

<210> 160
<211> 1254
<212> DNA
<213> Arabidopsis thaliana

<400> 160

```
atggcagcat cggccgcaac aacgacgtcg tctcaccttc ttctctcaag ctcacgtcac      60
gtggcttcat catcccagcc ttcaatcctt tcaccgagat ctctcttctc caacaacggg     120
aaacgagcac caactggagt gagaaaccat cagtatgcga gtggagtgag gtgtatggcc     180
gtagcggcgg atgcttctga gacgaaaacg gcggcgagga agaagagtgg atacgaactt     240
```

caaacgttga cgggctggtt gctgagacaa gagatgaaag gagagataga tgcagagctg 300
acgatagtga tgtcgagtat atcattggct tgtaagcaga tcgcttcact tgttcaacgt 360
gccggaatat ctaacctgac cggagttcaa ggcgccatta atattcaggg agaggatcag 420
aagaagcttg acgtcatctc taatgaggtg ttttccaact gtttgagatc aagtggaaga 480
acgggaatca tagcctcgga ggaagaggac gtgccagttg cggtggagga gagttactcc 540
ggcaactacg tcgtcgtgtt tgaccctctt gatggttcct ccaacattga cgctgccgtc 600
tctactggtt ctatcttcgg tatctatagc cccaatgacg aatgcattgt cgacgactcc 660
gacgatatct cagctcttgg gtcagaagaa caaaggtgta tagtaaacgt gtgccagcca 720
gggaacaact tgttagcagc cggctactgt atgtactcga gctcggtcat cttcgttctt 780
actctaggca aaggcgtttt ctccttcacg cttgatccaa tgtacggtga gtttgtcctc 840
acgcaagaaa acattgagat ccccaaagcc gggagaatct actctttcaa cgaagggaat 900
taccagatgt gggacgataa actaaagaag tacattgatg accttaagga ccctggtcca 960
actgggaagc cttactcggc aaggtacatt ggaagtttgg ttggagattt tcacaggact 1020
ttgttgtacg gtgggattta cgggtaccct cgtgacgcaa agagcaaaaa tggaaagctt 1080
aggcttttgt atgagtgtgc accaatgagt ttcattgttg aacaagctgg agggaaaggt 1140
tctgatggac attcgagagt actagatatc caaccgactg agatacatca gagggttcct 1200
ctatacattg gaagcacaga ggaagtagag aagttggaga agtacttggc ttga 1254

<210> 161
<211> 417
<212> PRT
<213> Arabidopsis thaliana

<400> 161
Met Ala Ala Ser Ala Ala Thr Thr Thr Ser Ser His Leu Leu Leu Ser
1 5 10 15
Ser Ser Arg His Val Ala Ser Ser Ser Gln Pro Ser Ile Leu Ser Pro
20 25 30
Arg Ser Leu Phe Ser Asn Asn Gly Lys Arg Ala Pro Thr Gly Val Arg
35 40 45
Asn His Gln Tyr Ala Ser Gly Val Arg Cys Met Ala Val Ala Ala Asp
50 55 60
Ala Ser Glu Thr Lys Thr Ala Ala Arg Lys Lys Ser Gly Tyr Glu Leu
65 70 75 80
Gln Thr Leu Thr Gly Trp Leu Leu Arg Gln Glu Met Lys Gly Glu Ile
85 90 95
Asp Ala Glu Leu Thr Ile Val Met Ser Ser Ile Ser Leu Ala Cys Lys
100 105 110
Gln Ile Ala Ser Leu Val Gln Arg Ala Gly Ile Ser Asn Leu Thr Gly
115 120 125
Val Gln Gly Ala Ile Asn Ile Gln Gly Glu Asp Gln Lys Lys Leu Asp
130 135 140
Val Ile Ser Asn Glu Val Phe Ser Asn Cys Leu Arg Ser Ser Gly Arg
145 150 155 160
Thr Gly Ile Ile Ala Ser Glu Glu Glu Asp Val Pro Val Ala Val Glu
165 170 175
Glu Ser Tyr Ser Gly Asn Tyr Val Val Val Phe Asp Pro Leu Asp Gly
180 185 190
Ser Ser Asn Ile Asp Ala Ala Val Ser Thr Gly Ser Ile Phe Gly Ile
195 200 205
Tyr Ser Pro Asn Asp Glu Cys Ile Val Asp Asp Ser Asp Asp Ile Ser
210 215 220
Ala Leu Gly Ser Glu Glu Gln Arg Cys Ile Val Asn Val Cys Gln Pro
225 230 235 240
Gly Asn Asn Leu Leu Ala Ala Gly Tyr Cys Met Tyr Ser Ser Ser Val
245 250 255
Ile Phe Val Leu Thr Leu Gly Lys Gly Val Phe Ser Phe Thr Leu Asp
260 265 270
Pro Met Tyr Gly Glu Phe Val Leu Thr Gln Glu Asn Ile Glu Ile Pro

```
        275                 280                 285
Lys Ala Gly Arg Ile Tyr Ser Phe Asn Glu Gly Asn Tyr Gln Met Trp
    290                 295                 300
Asp Asp Lys Leu Lys Lys Tyr Ile Asp Asp Leu Lys Asp Pro Gly Pro
305                 310                 315                 320
Thr Gly Lys Pro Tyr Ser Ala Arg Tyr Ile Gly Ser Leu Val Gly Asp
                325                 330                 335
Phe His Arg Thr Leu Leu Tyr Gly Gly Ile Tyr Gly Tyr Pro Arg Asp
            340                 345                 350
Ala Lys Ser Lys Asn Gly Lys Leu Arg Leu Leu Tyr Glu Cys Ala Pro
        355                 360                 365
Met Ser Phe Ile Val Glu Gln Ala Gly Gly Lys Gly Ser Asp Gly His
    370                 375                 380
Ser Arg Val Leu Asp Ile Gln Pro Thr Glu Ile His Gln Arg Val Pro
385                 390                 395                 400
Leu Tyr Ile Gly Ser Thr Glu Glu Val Glu Lys Leu Glu Lys Tyr Leu
                405                 410                 415
Ala
```

<210> 162
<211> 1254
<212> DNA
<213> Brassica napus

<400> 162

```
atggcagcaa ccgccgcgac aacgtcatcg tctcatcttc ttctctcaag ctctcgccac      60
gtggcagcct catctcagcc acaaatcctt ttccagagat ctctgttttc cggcgggaaa     120
cgatcggcag ctggaaaaaa ccatcatgct agtggtggag tgaggtgtat ggcggttgca     180
gcggatgctt cggcggaggc taagccggcg gcagcgagga agaagagtgg gtacgagctt     240
cagacgctga cgggttggtt gctgagacaa gagcagaaag gagagataga tacagagctg     300
acgatagtga tgtcgagcat agcgatggct tgtaagcaga tcgcttcgct tgtacagcgc     360
gccggaatct ctaatctgac cggcgttcaa ggagccgtta acattcaggg agaggatcag     420
aaaaagctcg acgtcgtctc taatgaggta ttttcaaact gtttgagatc aagtggaagg     480
acagggatca ttgcgtcaga ggaagaggac gtccccgtag cagttgaaga gagttactcc     540
ggaaactaca ttgtcgtctt tgatcctctc gacggttcct ccaacatcga cgctgcagtc     600
tccactggtt caatcttcgg tatctacagc cccaatgacg agtgtatcgt tgacgactcc     660
gacgatatct cctctcttgg ttcagaagaa caaaggtgta tagtaaacgt gtgtcaacca     720
gggaacaact tgctcgcagc tggctactgt atgtactcga gctcagtcat cttcgttctc     780
accttaggca agggcgtttt ctccttcaca ctcgacccaa tgtacggcga gttcgtcctc     840
actcaagaga acattgagat ccccaaagca gggaaaatct actctttcaa cgaagggaac     900
taccagatgt gggacgagaa actgaagaag tacattgatg atcttaagga ccctggtcca     960
agtgggaagc cttactctgc aaggtacatt ggtagtttgg tcggagactt tcacagaact    1020
ttgttgtacg gtgggattta cgggtacccct cgtgacgcca agagcaaaaa cggtaagctt  1080
aggcttttgt atgagtgtgc accgatgagt ttcatcgttg aacaagctgg aggaaaagga    1140
tcagatggcc accaaagagt actagatatc caacccaccg agatacatca gagggttcca    1200
ctttacattg gaagcaaaga agaagtagag aagctggaga agtacttggc ttga          1254
```

<210> 163
<211> 417
<212> PRT
<213> Brassica napus

<400> 163

```
Met Ala Ala Thr Ala Ala Thr Thr Ser Ser Ser His Leu Leu Leu Ser
1               5                   10                  15
Ser Ser Arg His Val Ala Ala Ser Ser Gln Pro Gln Ile Leu Phe Gln
            20                  25                  30
Arg Ser Leu Phe Ser Gly Gly Lys Arg Ser Ala Ala Gly Lys Asn His
```

```
        35                  40                  45
His Ala Ser Gly Gly Val Arg Cys Met Ala Val Ala Ala Asp Ala Ser
    50                  55                  60
Ala Glu Ala Lys Pro Ala Ala Ala Arg Lys Lys Ser Gly Tyr Glu Leu
65                  70                  75                  80
Gln Thr Leu Thr Gly Trp Leu Leu Arg Gln Glu Gln Lys Gly Glu Ile
                85                  90                  95
Asp Thr Glu Leu Thr Ile Val Met Ser Ser Ile Ala Met Ala Cys Lys
            100                 105                 110
Gln Ile Ala Ser Leu Val Gln Arg Ala Gly Ile Ser Asn Leu Thr Gly
        115                 120                 125
Val Gln Gly Ala Val Asn Ile Gln Gly Glu Asp Gln Lys Lys Leu Asp
    130                 135                 140
Val Val Ser Asn Glu Val Phe Ser Asn Cys Leu Arg Ser Ser Gly Arg
145                 150                 155                 160
Thr Gly Ile Ile Ala Ser Glu Glu Glu Asp Val Pro Val Ala Val Glu
                165                 170                 175
Glu Ser Tyr Ser Gly Asn Tyr Ile Val Val Phe Asp Pro Leu Asp Gly
            180                 185                 190
Ser Ser Asn Ile Asp Ala Ala Val Ser Thr Gly Ser Ile Phe Gly Ile
        195                 200                 205
Tyr Ser Pro Asn Asp Glu Cys Ile Val Asp Asp Ser Asp Asp Ile Ser
    210                 215                 220
Ser Leu Gly Ser Glu Glu Gln Arg Cys Ile Val Asn Val Cys Gln Pro
225                 230                 235                 240
Gly Asn Asn Leu Leu Ala Ala Gly Tyr Cys Met Tyr Ser Ser Ser Val
                245                 250                 255
Ile Phe Val Leu Thr Leu Gly Lys Gly Val Phe Ser Phe Thr Leu Asp
            260                 265                 270
Pro Met Tyr Gly Glu Phe Val Leu Thr Gln Glu Asn Ile Glu Ile Pro
        275                 280                 285
Lys Ala Gly Lys Ile Tyr Ser Phe Asn Glu Gly Asn Tyr Gln Met Trp
    290                 295                 300
Asp Glu Lys Leu Lys Lys Tyr Ile Asp Asp Leu Lys Asp Pro Gly Pro
305                 310                 315                 320
Ser Gly Lys Pro Tyr Ser Ala Arg Tyr Ile Gly Ser Leu Val Gly Asp
                325                 330                 335
Phe His Arg Thr Leu Leu Tyr Gly Gly Ile Tyr Gly Tyr Pro Arg Asp
            340                 345                 350
Ala Lys Ser Lys Asn Gly Lys Leu Arg Leu Leu Tyr Glu Cys Ala Pro
        355                 360                 365
Met Ser Phe Ile Val Glu Gln Ala Gly Gly Lys Gly Ser Asp Gly His
    370                 375                 380
Gln Arg Val Leu Asp Ile Gln Pro Thr Glu Ile His Gln Arg Val Pro
385                 390                 395                 400
Leu Tyr Ile Gly Ser Lys Glu Glu Val Glu Lys Leu Glu Lys Tyr Leu
                405                 410                 415
Ala
```

<210> 164
<211> 1130
<212> DNA
<213> Cyanidioschyzon merolae

<400> 164

```
atggcgaagc aagcaccgca cgctttcgtt agccttgggg ctcctaagct gcgctcaaca     60
aacatatggg gaagggtatc ggtgtcggtc ttgccgctgg aaacaaggca tcaacgtcgc    120
tttggtgcag tgagactgcg cgcagcttta gacctaccca tgacactcgg acagtgggtt    180
```

```
ctccaacagg agaggcagca tccagagacc gctgatttgg ctcctttgat aacagccaca     240
gcgacagctg gcaagcagat tgcctcactg gtcgcccgag cgggcgtgag caacttgact     300
ggccttcagg gctcggtcaa cgtgcagggc gaggagcaga aaaagctgga cgtgttgacg     360
aacgaggtgc tcaagaacgc gctacgctca acagggaaac taggtgtctt ggcttcggaa     420
gaggaaaacg agccagtgtc gctcatggaa gaggcgtata caggtgactt tattgctgcg     480
tttgacccgt tggacggatc ctcgaatttg gatgcagcga tcgccacggg tacgattttt     540
ggtgtaatgc gcagcggcga gcactgtctg acgggacccg aggacgcgga tataagtcag     600
cgacaaatgc agtgcctcgt gcagacgctg caaccgggtg cgaatctggt ggcagcgggc     660
tacattctct actcctcatc ggtgattttc atgctgtcta ttggtcacgg agtgcagggt     720
ttttctctgg acccagcgat tggcgagttt gttttgacgc acccagatgt tcgtgttccg     780
gagcgtggca agatctactc cttcaacgag gcaaactcgg ataactggga ccctgttctt     840
caagaatata ttcgatcgat gaagaaaaag ggctattctt cgcgctacat aggatctctc     900
gttggcgatg tccatcgcac gctgatttac ggcggcgttt ttggataccc tggtgataag     960
aagaacccga acggcaagtt gcgtcttctg tacgagtgcg cacctatggc gtatctcatg    1020
gagcaggccg gcggtatagc gaccacggga aagcaaagaa ttctggatat tgttccgcga    1080
gatgtccatc agcgagagcc gttcatttgc ggaagcccca gggatgttga               1130
```

<210> 165
<211> 391
<212> PRT
<213> Cyanidioschyzon merolae

<400> 165

```
Met Ala Lys Gln Ala Pro His Ala Phe Val Ser Leu Gly Ala Pro Lys
1               5                   10                  15
Leu Arg Ser Thr Asn Ile Trp Gly Arg Val Ser Val Ser Val Leu Pro
            20                  25                  30
Leu Glu Thr Arg His Gln Arg Arg Phe Gly Ala Val Arg Leu Arg Ala
        35                  40                  45
Ala Leu Asp Leu Pro Met Thr Leu Gly Gln Trp Val Leu Gln Gln Glu
    50                  55                  60
Arg Gln His Pro Glu Thr Ala Asp Leu Ala Pro Leu Ile Thr Ala Thr
65                  70                  75                  80
Ala Thr Ala Gly Lys Gln Ile Ala Ser Leu Val Ala Arg Ala Gly Val
                85                  90                  95
Ser Asn Leu Thr Gly Leu Gln Gly Ser Val Asn Val Gln Gly Glu Glu
            100                 105                 110
Gln Lys Lys Leu Asp Val Leu Thr Asn Glu Val Leu Lys Asn Ala Leu
        115                 120                 125
Arg Ser Thr Gly Lys Leu Gly Val Leu Ala Ser Glu Glu Glu Asn Glu
    130                 135                 140
Pro Val Ser Leu Met Glu Glu Ala Tyr Thr Gly Asp Phe Ile Ala Ala
145                 150                 155                 160
Phe Asp Pro Leu Asp Gly Ser Ser Asn Leu Asp Ala Ala Ile Ala Thr
                165                 170                 175
Gly Thr Ile Phe Gly Val Met Arg Ser Gly Glu His Cys Leu Thr Gly
            180                 185                 190
Pro Glu Asp Ala Asp Ile Ser Gln Arg Gln Met Gln Cys Leu Val Gln
        195                 200                 205
Thr Leu Gln Pro Gly Ala Asn Leu Val Ala Ala Gly Tyr Ile Leu Tyr
    210                 215                 220
Ser Ser Ser Val Ile Phe Met Leu Ser Ile Gly His Gly Val Gln Gly
225                 230                 235                 240
Phe Ser Leu Asp Pro Ala Ile Gly Glu Phe Val Leu Thr His Pro Asp
                245                 250                 255
Val Arg Val Pro Glu Arg Gly Lys Ile Tyr Ser Phe Asn Glu Ala Asn
            260                 265                 270
Ser Asp Asn Trp Asp Pro Val Leu Gln Glu Tyr Ile Arg Ser Met Lys
        275                 280                 285
```

Lys Lys Gly Tyr Ser Ser Arg Tyr Ile Gly Ser Leu Val Gly Asp Val
290 295 300
His Arg Thr Leu Ile Tyr Gly Gly Val Phe Gly Tyr Pro Gly Asp Lys
305 310 315 320
Lys Asn Pro Asn Gly Lys Leu Arg Leu Leu Tyr Glu Cys Ala Pro Met
325 330 335
Ala Tyr Leu Met Glu Gln Ala Gly Gly Ile Ala Thr Thr Gly Lys Gln
340 345 350
Arg Ile Leu Asp Ile Val Pro Arg Asp Val His Gln Arg Glu Pro Phe
355 360 365
Ile Cys Gly Ser Pro Arg Asp Val Glu Asp Leu Leu Lys Ile Tyr Gln
370 375 380
Gly Ser Met Ala Met Arg Gly
385 390

<210> 166
<211> 1218
<212> DNA
<213> Glycine max

<400> 166
atggttgcaa tggcagcagc aacagcatcc acccagttga ttttctcaaa gccttgttcc 60
ccttcacgtc tatgcccctt ccaactatgt gtctttgaca ctaaacaagt gctatcaagt 120
ggcaggagaa ggcatgtggg gggttctgga gttaggtgca tggctgtggg ggaagcagca 180
accactggga caaagaagag aagtggatat gagcttcaaa cactcactag ctggttgctg 240
aagcaggagc aagctggggt gattgatgca gaactcacta ttgtgctgtc tagcatttcc 300
atggcatgca aacagattgc ttctttggtg caaagagcta acatttccaa cctcactggg 360
gttcaaggtg ctgtcaatgt tcaaggggaa gaccagaaaa agcttgatgt tgtttcaaat 420
gaggttttct caaactgctt gaggtcaagt gggaggacag ggataatagc atcagaggag 480
gaagatgtgc cagtggcagt agaagagagt tattctggaa actacattgt ggtgtttgac 540
ccacttgatg ggtcatccaa tattgatgct gcagcgtcaa ctgggtccaa tttttggata 600
tacagcccca atgatgagtg tcttgctgac attgatgatg accccaccct tgacacaaca 660
gaacaaagat gtattgtgaa cgtgtgccaa cctggaagca accttcttgc agctggttac 720
tgcatgtatt ctagctcaat aatctttgtt ctcacacttg gaaatggagt gtttgtgttt 780
acattggacc cgatgtatgg cgaattcgtt ttgactcagg aaaacctcca gatacctaga 840
gcaggcaaaa tctatgcatt caatgaaggt aattatcagt tgtgggatga gaagctaaag 900
aaatatattg atgatctcaa ggacccaggt caaagcggca agccttattc tgcaaggtac 960
attggtagct tggtaggaga tttccacagg acactgctat atggtggcat ttacgggtac 1020
cccagggaca agaaaagcaa gaatgggaaa ctaaggctcc tgtatgaatg tgctcctatt 1080
aacttcattg tagaacaagc tggtggaaaa ggtacagatg gccttcaagt actccggctt 1140
caagggacag agattcatca acgtgtgcca ctgtacattg ggaagaggt agagaaggtg 1200
gaaaagtact tggcttaa 1218

<210> 167
<211> 405
<212> PRT
<213> Glycine max

<400> 167
Met Val Ala Met Ala Ala Ala Thr Ala Ser Thr Gln Leu Ile Phe Ser
1 5 10 15
Lys Pro Cys Ser Pro Ser Arg Leu Cys Pro Phe Gln Leu Cys Val Phe
20 25 30
Asp Thr Lys Gln Val Leu Ser Ser Gly Arg Arg Arg His Val Gly Gly
35 40 45
Ser Gly Val Arg Cys Met Ala Val Gly Glu Ala Ala Thr Thr Gly Thr
50 55 60
Lys Lys Arg Ser Gly Tyr Glu Leu Gln Thr Leu Thr Ser Trp Leu Leu
65 70 75 80

```
Lys Gln Glu Gln Ala Gly Val Ile Asp Ala Glu Leu Thr Ile Val Leu
                85                  90                  95
Ser Ser Ile Ser Met Ala Cys Lys Gln Ile Ala Ser Leu Val Gln Arg
            100                 105                 110
Ala Asn Ile Ser Asn Leu Thr Gly Val Gln Gly Ala Val Asn Val Gln
        115                 120                 125
Gly Glu Asp Gln Lys Lys Leu Asp Val Val Ser Asn Glu Val Phe Ser
    130                 135                 140
Asn Cys Leu Arg Ser Ser Gly Arg Thr Gly Ile Ile Ala Ser Glu Glu
145                 150                 155                 160
Glu Asp Val Pro Val Ala Val Glu Glu Ser Tyr Ser Gly Asn Tyr Ile
                165                 170                 175
Val Val Phe Asp Pro Leu Asp Gly Ser Ser Asn Ile Asp Ala Ala Ala
            180                 185                 190
Ser Thr Gly Ser Asn Phe Trp Ile Tyr Ser Pro Asn Asp Glu Cys Leu
        195                 200                 205
Ala Asp Ile Asp Asp Asp Pro Thr Leu Asp Thr Thr Glu Gln Arg Cys
    210                 215                 220
Ile Val Asn Val Cys Gln Pro Gly Ser Asn Leu Leu Ala Ala Gly Tyr
225                 230                 235                 240
Cys Met Tyr Ser Ser Ser Ile Ile Phe Val Leu Thr Leu Gly Asn Gly
                245                 250                 255
Val Phe Val Phe Thr Leu Asp Pro Met Tyr Gly Glu Phe Val Leu Thr
            260                 265                 270
Gln Glu Asn Leu Gln Ile Pro Arg Ala Gly Lys Ile Tyr Ala Phe Asn
        275                 280                 285
Glu Gly Asn Tyr Gln Leu Trp Asp Glu Lys Leu Lys Lys Tyr Ile Asp
    290                 295                 300
Asp Leu Lys Asp Pro Gly Gln Ser Gly Lys Pro Tyr Ser Ala Arg Tyr
305                 310                 315                 320
Ile Gly Ser Leu Val Gly Asp Phe His Arg Thr Leu Leu Tyr Gly Gly
                325                 330                 335
Ile Tyr Gly Tyr Pro Arg Asp Lys Lys Ser Lys Asn Gly Lys Leu Arg
            340                 345                 350
Leu Leu Tyr Glu Cys Ala Pro Ile Asn Phe Ile Val Glu Gln Ala Gly
        355                 360                 365
Gly Lys Gly Thr Asp Gly Leu Gln Val Leu Arg Leu Gln Gly Thr Glu
    370                 375                 380
Ile His Gln Arg Val Pro Leu Tyr Ile Gly Glu Glu Val Glu Lys Val
385                 390                 395                 400
Glu Lys Tyr Leu Ala
                405
```

<210> 168
<211> 1212
<212> DNA
<213> Lycopersicon esculentum

<400> 168

```
atggcagcaa cagcaacaac ttcatattta agtgctctag acaaaaagac tccattttta     60
tttgccttgg acaaaaagac tccattttta tgcccaaaaa acagcacaaa gagaaggtca    120
tttaatggag gagttaagtg catggcaata gagacagctt caggtgttac acaaaccaag    180
aaaaagagtg gctatgagtt acaaacttta acaagttggc tattgagaca agaacaagct    240
ggagttattg atgctgaact taccatagta atttcaagta tttcaatggc ttgtaaacag    300
attgcttctt tggtccaaag agctggaatt tctaacctta ctggagttca aggtgctgtc    360
aatattcaag gagaagatca gaagaaactt gatgttgtct ctaatgaggt tttctcgaat    420
tgtctaagat caagtggaag gactgggatt atagcatcag aagaagagga tgtacctgtg    480
gcagtggaag agagttactc aggcaactac attgtggtgt ttgatcctct tgatggatca    540
tcaaacattg atgctgctgt atctaccggt tctatctttg gaatatacag cccgaatgat    600
```

```
gagtgcctag ctgatcttgg agatgattcc acgcttgaca atatagagca aaagtgcatc     660
gtgaatgtat gtcaaccagg gacaaacctt cttgcagcag gatactgcat gtactcaagc     720
tctgtgattt tcgtactcac cttgggaaat ggcgtttttt cctttaactt ggatccaatg     780
tatggagaat ttgttctgac tcaagaaaat gtccaaatac caaagtctgg aaagatctat     840
tcattcaatg aaggaaacta ccagctctgg gatgacaagt tgaaaaaata tatcgatgac     900
ttgaaagacc ctggtcctag tggcaagcct tactctgcaa ggtacattgg tagtttggta     960
ggtgacttcc atagaactct tctatatggt ggcatttatg gttatcctag agacagaaag    1020
agcaagaatg gaaagttgag gcttttgtac gaatgtgctc ccatgagctt cattgtggaa    1080
caagctggtg gcaaaggatc cgatggtcac caaagagttc tcgatatcca accaactgag    1140
atacatcaac gagttccatt gtacattgga agcacagaag aagttgaaaa attggagaag    1200
tacttgtctt aa                                                        1212
```

<210> 169
<211> 403
<212> PRT
<213> Lycopersicon esculentum

<400> 169

```
Met Ala Ala Thr Ala Thr Thr Ser Tyr Leu Ser Ala Leu Asp Lys Lys
1               5                   10                  15
Thr Pro Phe Leu Phe Ala Leu Asp Lys Lys Thr Pro Phe Leu Cys Pro
            20                  25                  30
Lys Asn Ser Thr Lys Arg Arg Ser Phe Asn Gly Gly Val Lys Cys Met
        35                  40                  45
Ala Ile Glu Thr Ala Ser Gly Val Thr Gln Thr Lys Lys Lys Ser Gly
    50                  55                  60
Tyr Glu Leu Gln Thr Leu Thr Ser Trp Leu Leu Arg Gln Glu Gln Ala
65                  70                  75                  80
Gly Val Ile Asp Ala Glu Leu Thr Ile Val Ile Ser Ser Ile Ser Met
                85                  90                  95
Ala Cys Lys Gln Ile Ala Ser Leu Val Gln Arg Ala Gly Ile Ser Asn
            100                 105                 110
Leu Thr Gly Val Gln Gly Ala Val Asn Ile Gln Gly Glu Asp Gln Lys
        115                 120                 125
Lys Leu Asp Val Val Ser Asn Glu Val Phe Ser Asn Cys Leu Arg Ser
    130                 135                 140
Ser Gly Arg Thr Gly Ile Ile Ala Ser Glu Glu Glu Asp Val Pro Val
145                 150                 155                 160
Ala Val Glu Glu Ser Tyr Ser Gly Asn Tyr Ile Val Val Phe Asp Pro
                165                 170                 175
Leu Asp Gly Ser Ser Asn Ile Asp Ala Ala Val Ser Thr Gly Ser Ile
            180                 185                 190
Phe Gly Ile Tyr Ser Pro Asn Asp Glu Cys Leu Ala Asp Leu Gly Asp
        195                 200                 205
Asp Ser Thr Leu Asp Asn Ile Glu Gln Lys Cys Ile Val Asn Val Cys
    210                 215                 220
Gln Pro Gly Thr Asn Leu Leu Ala Ala Gly Tyr Cys Met Tyr Ser Ser
225                 230                 235                 240
Ser Val Ile Phe Val Leu Thr Leu Gly Asn Gly Val Phe Ser Phe Asn
                245                 250                 255
Leu Asp Pro Met Tyr Gly Glu Phe Val Leu Thr Gln Glu Asn Val Gln
            260                 265                 270
Ile Pro Lys Ser Gly Lys Ile Tyr Ser Phe Asn Glu Gly Asn Tyr Gln
        275                 280                 285
Leu Trp Asp Asp Lys Leu Lys Lys Tyr Ile Asp Asp Leu Lys Asp Pro
    290                 295                 300
Gly Pro Ser Gly Lys Pro Tyr Ser Ala Arg Tyr Ile Gly Ser Leu Val
305                 310                 315                 320
Gly Asp Phe His Arg Thr Leu Leu Tyr Gly Gly Ile Tyr Gly Tyr Pro
```

```
                325                 330                 335
Arg Asp Arg Lys Ser Lys Asn Gly Lys Leu Arg Leu Leu Tyr Glu Cys
            340                 345                 350
Ala Pro Met Ser Phe Ile Val Glu Gln Ala Gly Gly Lys Gly Ser Asp
        355                 360                 365
Gly His Gln Arg Val Leu Asp Ile Gln Pro Thr Glu Ile His Gln Arg
    370                 375                 380
Val Pro Leu Tyr Ile Gly Ser Thr Glu Glu Val Glu Lys Leu Glu Lys
385                 390                 395                 400
Tyr Leu Ser
```

<210> 170
<211> 1236
<212> DNA
<213> Medicago truncatula

<400> 170

```
atggttgcaa tggcagcagc aacagcatca tcacaattaa tattctcaaa accttgttct      60
ccctcacgtc tatgtccctt ccaactttgt gttttcgaca cgaaatcagt gttatcaagt     120
tcaagaagaa agcatgtgag tggctctgga ggagtgagat gcatggctgt tggtgaagta     180
gctgctgaga caaagaaaag aagtagttat gagcttataa cattgactag ttggttgttg     240
aagcaagaac aaacaggggt tattgatgct gaacttacta ttgttttgaa tagtatttcg     300
ttggcatgta aacagattgc ttctttggtt caaagagcta atatttctaa ccttactggt     360
gttcaaggtg ctgtaaatat tcaaggggag gatcagaaaa aacttgatgt tgtctcaaat     420
gaggtattct caaattgttt gaggtcaagt gggaggacag ggattatagc atcagaggaa     480
gaggatgtgc cagtggcagt agaagagagt tattcaggaa actacattgt ggtctttgat     540
ccacttgatg gttcatcgaa tattgatgct gcagtttcaa ctggttctat ttttgggatt     600
tacagcccca atgatgagtg tcttgctgac gtaggcaatg agtccgatga ccccacactt     660
ggcacagaag aacaacgatg cattgtgaat gtgtgccaac caggaagcaa ccttctagca     720
gccggttact gcatgtattc tagctcagta atcttcgttc taacaatcgg caaaggagtt     780
ttcgtattca cattagatcc aatgtatggg gaatttgttt tgactcaaga aaatctccaa     840
ataccaaaat cagggaaaat ttattctttc aatgaaggaa attacaagtt atgggatgac     900
aacttgaaga aatacatcga tgatctcaag gaaccgggtg ctaatggcaa accttattca     960
gcaaggtata ttggtagttt ggtaggtgat ttccatagga cactgctata tggtggcatt    1020
tatggttacc ctagggacaa gaaaagtaag aatggaaggc ttaggctttt atatgagtgt    1080
gctccaatga gtttcattgt agaacaggct ggtggaaaag gttcagatgg tcatcaaaga    1140
gtacttgaca ttcaacccac cgaaattcat caacgtgttc cactgtacat tgggagcaca    1200
gaggaagtgg agaaggttga aaagtacttg gcttaa                              1236
```

<210> 171
<211> 411
<212> PRT
<213> Medicago truncatula

<400> 171

```
Met Val Ala Met Ala Ala Ala Thr Ala Ser Ser Gln Leu Ile Phe Ser
1               5                   10                  15
Lys Pro Cys Ser Pro Ser Arg Leu Cys Pro Phe Gln Leu Cys Val Phe
            20                  25                  30
Asp Thr Lys Ser Val Leu Ser Ser Ser Arg Arg Lys His Val Ser Gly
        35                  40                  45
Ser Gly Gly Val Arg Cys Met Ala Val Gly Glu Val Ala Ala Glu Thr
    50                  55                  60
Lys Lys Arg Ser Ser Tyr Glu Leu Ile Thr Leu Thr Ser Trp Leu Leu
65                  70                  75                  80
Lys Gln Glu Gln Thr Gly Val Ile Asp Ala Glu Leu Thr Ile Val Leu
                85                  90                  95
Asn Ser Ile Ser Leu Ala Cys Lys Gln Ile Ala Ser Leu Val Gln Arg
```

```
                100                 105                 110
Ala Asn Ile Ser Asn Leu Thr Gly Val Gln Gly Ala Val Asn Ile Gln
        115                 120                 125
Gly Glu Asp Gln Lys Lys Leu Asp Val Val Ser Asn Glu Val Phe Ser
    130                 135                 140
Asn Cys Leu Arg Ser Ser Gly Arg Thr Gly Ile Ile Ala Ser Glu Glu
145                 150                 155                 160
Glu Asp Val Pro Val Ala Val Glu Glu Ser Tyr Ser Gly Asn Tyr Ile
                165                 170                 175
Val Val Phe Asp Pro Leu Asp Gly Ser Ser Asn Ile Asp Ala Ala Val
            180                 185                 190
Ser Thr Gly Ser Ile Phe Gly Ile Tyr Ser Pro Asn Asp Glu Cys Leu
        195                 200                 205
Ala Asp Val Gly Asn Glu Ser Asp Asp Pro Thr Leu Gly Thr Glu Glu
    210                 215                 220
Gln Arg Cys Ile Val Asn Val Cys Gln Pro Gly Ser Asn Leu Leu Ala
225                 230                 235                 240
Ala Gly Tyr Cys Met Tyr Ser Ser Ser Val Ile Phe Val Leu Thr Ile
                245                 250                 255
Gly Lys Gly Val Phe Val Phe Thr Leu Asp Pro Met Tyr Gly Glu Phe
            260                 265                 270
Val Leu Thr Gln Glu Asn Leu Gln Ile Pro Lys Ser Gly Lys Ile Tyr
        275                 280                 285
Ser Phe Asn Glu Gly Asn Tyr Lys Leu Trp Asp Asp Asn Leu Lys Lys
    290                 295                 300
Tyr Ile Asp Asp Leu Lys Glu Pro Gly Ala Asn Gly Lys Pro Tyr Ser
305                 310                 315                 320
Ala Arg Tyr Ile Gly Ser Leu Val Gly Asp Phe His Arg Thr Leu Leu
                325                 330                 335
Tyr Gly Gly Ile Tyr Gly Tyr Pro Arg Asp Lys Lys Ser Lys Asn Gly
            340                 345                 350
Arg Leu Arg Leu Leu Tyr Glu Cys Ala Pro Met Ser Phe Ile Val Glu
        355                 360                 365
Gln Ala Gly Gly Lys Gly Ser Asp Gly His Gln Arg Val Leu Asp Ile
    370                 375                 380
Gln Pro Thr Glu Ile His Gln Arg Val Pro Leu Tyr Ile Gly Ser Thr
385                 390                 395                 400
Glu Glu Val Glu Lys Val Glu Lys Tyr Leu Ala
                405                 410
```

<210> 172
<211> 1236
<212> DNA
<213> Nicotiana tabacum

<400> 172

```
atggcagcat catcagcaac agcaacagca acaacttcat ttttatgtgc tttagacaaa      60
aagactccat ttttatgtac tctagacaaa aaaggtactc catttttatg cccaaaaggc     120
agcagcacaa caaagagaag gtcatttaat ggaggagtga agtgcatggc aatagagaca     180
acagcaggag ctacagagac caagaaaaga agtggctatg agttacaaac tttaacaagc     240
tggctattaa ggcaagaaca agctgggagt attgatgctg aacttaccat agtgatttca     300
agtatttcta tggcttgtaa gcagattgct tctttggttc agagagctgg aatttctaac     360
cttactggag ttcaaggtgc tgtcaatatt caaggagaag accagaagaa gcttgatgtt     420
gtctctaacg aggttttctc gaattgtcta aggtcgagtg gaaggactgg gattatagca     480
tcagaggaag aggatgtacc agtggcagtg gaagagagtt actcaggaaa ctacattgtg     540
gtgtttgacc ctcttgatgg atcatcaaac attgatgctg ctgtttctac tggttctatt     600
ttcggaatat acagcccaaa tgatgagtgc ctcgcagatc atggagatga ttccacgctt     660
gacaatgttg aacagaggtg tattgtgaat gtatgccaac cagggagcaa ccttcttgca     720
gcaggctact gcatgtactc aagctctgtg atttttgtgg tcaccttggg aaacggagtc     780
```

```
tttgccttca acttggatcc gatgtatgga gaattcgttc tgacccaaga aaacatccaa    840
ataccaaaat ctggaaagat ctattcgttc aacgaaggaa actaccagct ttgggatgac    900
aaactgaaga aatacatcga tgacttgaag gaccctggtc ctagcggcaa gccttactct    960
gctaggtaca ttggtagttt ggttggtgac ttccatagaa ctcttctata tggtggcatt   1020
tatggctatc ctagagataa aaagagtaag aatggaaagt tgaggctttt gtatgagtgt   1080
gcccctatga gcttcctcgt ggaacaagct ggtggcaaag gatccgatgg ccaccaaaga   1140
gttcttgata tccaaccaac tgagatacac cagcgagtcc cattgtacat tggaagcaca   1200
gaagaagttg aaaagttgga gaagtattta tcttaa                             1236
```

```
<210>  173
<211>  411
<212>  PRT
<213>  Nicotiana tabacum

<400>  173
Met Ala Ala Ser Ser Ala Thr Ala Thr Ala Thr Thr Ser Phe Leu Cys
1               5                   10                  15
Ala Leu Asp Lys Lys Thr Pro Phe Leu Cys Thr Leu Asp Lys Lys Gly
            20                  25                  30
Thr Pro Phe Leu Cys Pro Lys Gly Ser Ser Thr Thr Lys Arg Arg Ser
        35                  40                  45
Phe Asn Gly Gly Val Lys Cys Met Ala Ile Glu Thr Thr Ala Gly Ala
    50                  55                  60
Thr Glu Thr Lys Lys Arg Ser Gly Tyr Glu Leu Gln Thr Leu Thr Ser
65                  70                  75                  80
Trp Leu Leu Arg Gln Glu Gln Ala Gly Ser Ile Asp Ala Glu Leu Thr
                85                  90                  95
Ile Val Ile Ser Ser Ile Ser Met Ala Cys Lys Gln Ile Ala Ser Leu
            100                 105                 110
Val Gln Arg Ala Gly Ile Ser Asn Leu Thr Gly Val Gln Gly Ala Val
        115                 120                 125
Asn Ile Gln Gly Glu Asp Gln Lys Lys Leu Asp Val Val Ser Asn Glu
    130                 135                 140
Val Phe Ser Asn Cys Leu Arg Ser Ser Gly Arg Thr Gly Ile Ile Ala
145                 150                 155                 160
Ser Glu Glu Glu Asp Val Pro Val Ala Val Glu Glu Ser Tyr Ser Gly
                165                 170                 175
Asn Tyr Ile Val Val Phe Asp Pro Leu Asp Gly Ser Ser Asn Ile Asp
            180                 185                 190
Ala Ala Val Ser Thr Gly Ser Ile Phe Gly Ile Tyr Ser Pro Asn Asp
        195                 200                 205
Glu Cys Leu Ala Asp His Gly Asp Asp Ser Thr Leu Asp Asn Val Glu
    210                 215                 220
Gln Arg Cys Ile Val Asn Val Cys Gln Pro Gly Ser Asn Leu Leu Ala
225                 230                 235                 240
Ala Gly Tyr Cys Met Tyr Ser Ser Ser Val Ile Phe Val Val Thr Leu
                245                 250                 255
Gly Asn Gly Val Phe Ala Phe Asn Leu Asp Pro Met Tyr Gly Glu Phe
            260                 265                 270
Val Leu Thr Gln Glu Asn Ile Gln Ile Pro Lys Ser Gly Lys Ile Tyr
        275                 280                 285
Ser Phe Asn Glu Gly Asn Tyr Gln Leu Trp Asp Asp Lys Leu Lys Lys
    290                 295                 300
Tyr Ile Asp Asp Leu Lys Asp Pro Gly Pro Ser Gly Lys Pro Tyr Ser
305                 310                 315                 320
Ala Arg Tyr Ile Gly Ser Leu Val Gly Asp Phe His Arg Thr Leu Leu
                325                 330                 335
Tyr Gly Gly Ile Tyr Gly Tyr Pro Arg Asp Lys Lys Ser Lys Asn Gly
            340                 345                 350
```

Lys Leu Arg Leu Leu Tyr Glu Cys Ala Pro Met Ser Phe Leu Val Glu
355 360 365
Gln Ala Gly Gly Lys Gly Ser Asp Gly His Gln Arg Val Leu Asp Ile
370 375 380
Gln Pro Thr Glu Ile His Gln Arg Val Pro Leu Tyr Ile Gly Ser Thr
385 390 395 400
Glu Glu Val Glu Lys Leu Glu Lys Tyr Leu Ser
405 410

<210> 174
<211> 1221
<212> DNA
<213> Oryza sativa

<400> 174
atggccgccg cagccacgac atcctcccac ctgctcctgc tctcccgcca gcaggcggcg 60
gcctcgctcc aatgcggcct ctccttccgg aggcagcccg gcaggctcgc cggtgggtcg 120
tcggccccga gcgtgcggtg catggcggcc gtcgacacgg cctcggcgcc cgccgcgacg 180
gaggctagca agaagagcag ctacgagatc accacgctga cgacgtggct gctgaagcag 240
gagcaggccg ggaccatcga cggcgagatg accatcgtgc tggccagcat ctccacggcg 300
tgcaagcaga tcgcctcgct ggtgcagcgc gcgcccatct ccaacctcac cggcgtccag 360
ggcgccgtca acgtgcaggg cgaggaccag aaaaaactcg acgtcgtctc caacgaggtg 420
ttctccaact gcctcaaatc gagcgggcgc accggcgtga tcgcgtcgga ggaggaggac 480
gtgccggtgg ccgtggagga gagctactcg ggcaactaca tcgtggtgtt cgacccgctc 540
gacggctcct ccaacatcga cgccgccgtc tccaccggct ccatcttcgg catctacagc 600
cccaacgacg agtgcctagc cgacatcgcc gacgaccaaa atcttgacca ggtggagcag 660
aggtgcatcg tgagcgtgtg ccagccgggg agcaacctgc tcgccgccgg ctactgcatg 720
tactcgagct cggtcatctt cgtgctcacc atcgggacag ggtgtacgt gttcacgctg 780
gacccgatgt acggcgagtt cgtgctgacg caggagaagg tgcagatccc caaggcaggc 840
aagatctatg ccttcaacga gggcaactac gcgctctggg acgacaagct caagagctac 900
atggacagcc tcaaggagcc cggccgtcc gggaagccat actccgcgcg ctacatcggc 960
agcctcgtcg gcgacttcca ccgcacgctg ctctacggcg gcatctacgg ctaccccagg 1020
gaccagaaga gcaagaacgg caagctgcgg ctgctgtacg agtgcgcgcc gatgagcttc 1080
atcgtcgagc aggccggcgg caagggctcc gatggccacc agaggatact tgacatcatg 1140
cctacggaga tccatcagag agtgccgctg tacatcggga gcgtggagga agtggagaag 1200
gtcgagaagt tcttggcttg a 1221

<210> 175
<211> 406
<212> PRT
<213> Oryza sativa

<400> 175
Met Ala Ala Ala Ala Thr Thr Ser Ser His Leu Leu Leu Leu Ser Arg
1 5 10 15
Gln Gln Ala Ala Ala Ser Leu Gln Cys Gly Leu Ser Phe Arg Arg Gln
20 25 30
Pro Gly Arg Leu Ala Gly Gly Ser Ser Ala Pro Ser Val Arg Cys Met
35 40 45
Ala Ala Val Asp Thr Ala Ser Ala Pro Ala Ala Thr Glu Ala Ser Lys
50 55 60
Lys Ser Ser Tyr Glu Ile Thr Thr Leu Thr Thr Trp Leu Leu Lys Gln
65 70 75 80
Glu Gln Ala Gly Thr Ile Asp Gly Glu Met Thr Ile Val Leu Ala Ser
85 90 95
Ile Ser Thr Ala Cys Lys Gln Ile Ala Ser Leu Val Gln Arg Ala Pro
100 105 110
Ile Ser Asn Leu Thr Gly Val Gln Gly Ala Val Asn Val Gln Gly Glu
115 120 125

```
Asp Gln Lys Lys Leu Asp Val Val Ser Asn Glu Val Phe Ser Asn Cys
    130                 135                 140
Leu Lys Ser Ser Gly Arg Thr Gly Val Ile Ala Ser Glu Glu Glu Asp
145                 150                 155                 160
Val Pro Val Ala Val Glu Glu Ser Tyr Ser Gly Asn Tyr Ile Val Val
                165                 170                 175
Phe Asp Pro Leu Asp Gly Ser Ser Asn Ile Asp Ala Ala Val Ser Thr
            180                 185                 190
Gly Ser Ile Phe Gly Ile Tyr Ser Pro Asn Asp Glu Cys Leu Ala Asp
        195                 200                 205
Ile Ala Asp Asp Gln Asn Leu Asp Gln Val Glu Gln Arg Cys Ile Val
    210                 215                 220
Ser Val Cys Gln Pro Gly Ser Asn Leu Leu Ala Ala Gly Tyr Cys Met
225                 230                 235                 240
Tyr Ser Ser Ser Val Ile Phe Val Leu Thr Ile Gly Thr Gly Val Tyr
                245                 250                 255
Val Phe Thr Leu Asp Pro Met Tyr Gly Glu Phe Val Leu Thr Gln Glu
            260                 265                 270
Lys Val Gln Ile Pro Lys Ala Gly Lys Ile Tyr Ala Phe Asn Glu Gly
        275                 280                 285
Asn Tyr Ala Leu Trp Asp Asp Lys Leu Lys Ser Tyr Met Asp Ser Leu
    290                 295                 300
Lys Glu Pro Gly Pro Ser Gly Lys Pro Tyr Ser Ala Arg Tyr Ile Gly
305                 310                 315                 320
Ser Leu Val Gly Asp Phe His Arg Thr Leu Leu Tyr Gly Gly Ile Tyr
                325                 330                 335
Gly Tyr Pro Arg Asp Gln Lys Ser Lys Asn Gly Lys Leu Arg Leu Leu
            340                 345                 350
Tyr Glu Cys Ala Pro Met Ser Phe Ile Val Glu Gln Ala Gly Gly Lys
        355                 360                 365
Gly Ser Asp Gly His Gln Arg Ile Leu Asp Ile Met Pro Thr Glu Ile
    370                 375                 380
His Gln Arg Val Pro Leu Tyr Ile Gly Ser Val Glu Glu Val Glu Lys
385                 390                 395                 400
Val Glu Lys Phe Leu Ala
                405
```

<210> 176
<211> 1143
<212> DNA
<213> Ostreococcus lucimarinus

<400> 176

```
atgtctgccg ccacctccgc ctgcgctcgc gcgatcgtcg cgacgaagaa gaccaccgcg     60
gcgcgccgcg cgggcaagtc ggcgacgcgg gcgacgccgc gatccgtcgc ggcgcgcgcg    120
gggggcgcgg gcacgccgtt cacgacgtgg atcttgcaac aagagatgga agaaaagatc    180
gacggcgaac tcgcggtcgt gttgtcgagc atcggcttgg cgtgcaagca aatcgcgagc    240
ttggtgcaac gcgccgggct tcaaggcatg acgggtttgg cgggcgagac gaacgtgcaa    300
ggtgaagacc aaaagaagct cgacgtcatc tccaacgatg tgttctgcga tgtcttgcgt    360
caaaccggcc gcacgggcgt gattgcgtcc gaggaagaag acgtgccggt cgccgtcgaa    420
gaaaccttcg gcggtaacta cgtcgtcgtc ttcgatccgc tcgatggctc ttccaacatc    480
gacgccgccg tttccacggg ttccatctgg ggcatctacg agtccgactc cacgtgcatc    540
ccggattttg gcaccgaaga tgcggccaag attgaagaga agtgcgtcat gaacgtgtgc    600
caaccgggga acaacttgtt gtgcgcgggc tactgcatgt actcctcttc caccatcctc    660
gtcttgaccc tcggtgaggg tgtgtacggt ttcaccctcg acccgaccgt cggcgagttc    720
atcatgtccc acgacaacat caaggttccg gaatctggta agatttactc cttcaacgaa    780
ggcaactacg acatgtggac tccgggcttg aagaagtaca tggactccct caagactggc    840
ggcgcggaac aaggcaccaa gccgtacagc gcccgttaca tcggttccct cgtcggtgac    900
ttccacagaa ccatcttgta cggaggcatc tacggctacc cgggcgactc caagaacccg    960
```

aacggtaagc ttcgcctctt gtacgagtgc gcgccgatgt ccttcatcgc cgaacaagcc 1020
ggcggtatgg gttccaccgg taaggagcgc gtccttgacg ttgtcccgga aaagtttcac 1080
caacgtgtgc cgttcttcac cggctccaag aaggaagtgc agtacttgga atccttcatg 1140
tag 1143

<210> 177
<211> 380
<212> PRT
<213> Ostreococcus lucimarinus

<400> 177
Met Ser Ala Ala Thr Ser Ala Cys Ala Arg Ala Ile Val Ala Thr Lys
1 5 10 15
Lys Thr Thr Ala Ala Arg Arg Ala Gly Lys Ser Ala Thr Arg Ala Thr
20 25 30
Pro Arg Ser Val Ala Ala Arg Ala Gly Gly Ala Gly Thr Pro Phe Thr
35 40 45
Thr Trp Ile Leu Gln Gln Glu Met Glu Glu Lys Ile Asp Gly Glu Leu
50 55 60
Ala Val Val Leu Ser Ser Ile Gly Leu Ala Cys Lys Gln Ile Ala Ser
65 70 75 80
Leu Val Gln Arg Ala Gly Leu Gln Gly Met Thr Gly Leu Ala Gly Glu
85 90 95
Thr Asn Val Gln Gly Glu Asp Gln Lys Lys Leu Asp Val Ile Ser Asn
100 105 110
Asp Val Phe Cys Asp Val Leu Arg Gln Thr Gly Arg Thr Gly Val Ile
115 120 125
Ala Ser Glu Glu Glu Asp Val Pro Val Ala Val Glu Glu Thr Phe Gly
130 135 140
Gly Asn Tyr Val Val Val Phe Asp Pro Leu Asp Gly Ser Ser Asn Ile
145 150 155 160
Asp Ala Ala Val Ser Thr Gly Ser Ile Trp Gly Ile Tyr Glu Ser Asp
165 170 175
Ser Thr Cys Ile Pro Asp Phe Gly Thr Glu Asp Ala Ala Lys Ile Glu
180 185 190
Glu Lys Cys Val Met Asn Val Cys Gln Pro Gly Asn Asn Leu Leu Cys
195 200 205
Ala Gly Tyr Cys Met Tyr Ser Ser Ser Thr Ile Leu Val Leu Thr Leu
210 215 220
Gly Glu Gly Val Tyr Gly Phe Thr Leu Asp Pro Thr Val Gly Glu Phe
225 230 235 240
Ile Met Ser His Asp Asn Ile Lys Val Pro Glu Ser Gly Lys Ile Tyr
245 250 255
Ser Phe Asn Glu Gly Asn Tyr Asp Met Trp Thr Pro Gly Leu Lys Lys
260 265 270
Tyr Met Asp Ser Leu Lys Thr Gly Gly Ala Glu Gln Gly Thr Lys Pro
275 280 285
Tyr Ser Ala Arg Tyr Ile Gly Ser Leu Val Gly Asp Phe His Arg Thr
290 295 300
Ile Leu Tyr Gly Gly Ile Tyr Gly Tyr Pro Gly Asp Ser Lys Asn Pro
305 310 315 320
Asn Gly Lys Leu Arg Leu Leu Tyr Glu Cys Ala Pro Met Ser Phe Ile
325 330 335
Ala Glu Gln Ala Gly Gly Met Gly Ser Thr Gly Lys Glu Arg Val Leu
340 345 350
Asp Val Val Pro Glu Lys Phe His Gln Arg Val Pro Phe Phe Thr Gly
355 360 365
Ser Lys Lys Glu Val Gln Tyr Leu Glu Ser Phe Met
370 375 380

<210> 178
<211> 1134
<212> DNA
<213> Ostreococcus tauri

<400> 178
atgtctgcgt gtatttctgc ctctgtgacg cgcgcgcgaa cggcgcgcgc gcccgcggct 60
cgccgcgcgg cgagtaaggc gcgggcgtcg ccgcgcgccg tcgcggcgcg cgcggggggc 120
gttggtacgc cgtacacgac gtggattctc cagcaagaga tgcaagagaa cattgatggt 180
gaattggcgg tggttttgtc ctccatcggt ctcgcgtgca agcaaatcgc gagcctcgtc 240
cagcgcgcgg gtctcgcggg catgactggt ttggcgggcg agcaaaacgt gcaaggcgag 300
gaccagaaga agctcgacgt catctccaac gatgttttct gcaacgtatt gcgccaatcc 360
ggccgcacgg gcgtcatcgc ctccgaagaa gaagacgttc cggtcgccgt cgaggaaacg 420
tacggcggta actacgtcgt cgtcttcgat ccgcttgatg gttcctccaa tatcgacgcc 480
gccgtctcca cgggatccat ctggggtatc tacgagtccg actcctcgtg tattcccgac 540
ttcggctccg atgactccgc caaggttgaa gagaagtgcg tcatgaacgt ttgccaaccg 600
ggcagcaact tgctctgcgc gggttactgc atgtactcct cgtccaccat cctcgtcatc 660
accatcggcc aaggcgtgtt cggttttacc ctcgacccga ccgtcggtga gttcatcatg 720
tcccacgaga acatcaaggt tccggactct ggcaagattt actctttcaa cgaaggcaac 780
tacgccatgt ggtccgacgg tttgaagaag tacatggact ccctcaagac gggcggcaag 840
gacggtggca agccgtacag cgcccgctac atcggttcgc tcgtcggtga cttccaccgc 900
acgatccttt acggaggcat ctacggttac ccgggtgacg cgaagaaccc gaacggtaag 960
ctccgcctcc tgtacgagtg cgcgcccgatg tccatgatcg ctgaacaagc cggtggtaag 1020
ggctccaccg gtgtcgcgcg tgtcctggac atcgttccgg aaaaggttca ccagcgcgtg 1080
ccgttcttcg ttggctccaa gaacgaggtt gcctacttgg agtccttcat gtga 1134

<210> 179
<211> 377
<212> PRT
<213> Ostreococcus tauri

<400> 179
Met Ser Ala Cys Ile Ser Ala Ser Val Thr Arg Ala Arg Thr Ala Arg
1 5 10 15
Ala Pro Ala Ala Arg Arg Ala Ala Ser Lys Ala Arg Ala Ser Pro Arg
20 25 30
Ala Val Ala Ala Arg Ala Gly Gly Val Gly Thr Pro Tyr Thr Thr Trp
35 40 45
Ile Leu Gln Gln Glu Met Gln Glu Asn Ile Asp Gly Glu Leu Ala Val
50 55 60
Val Leu Ser Ser Ile Gly Leu Ala Cys Lys Gln Ile Ala Ser Leu Val
65 70 75 80
Gln Arg Ala Gly Leu Ala Gly Met Thr Gly Leu Ala Gly Glu Gln Asn
85 90 95
Val Gln Gly Glu Asp Gln Lys Lys Leu Asp Val Ile Ser Asn Asp Val
100 105 110
Phe Cys Asn Val Leu Arg Gln Ser Gly Arg Thr Gly Val Ile Ala Ser
115 120 125
Glu Glu Glu Asp Val Pro Val Ala Val Glu Glu Thr Tyr Gly Gly Asn
130 135 140
Tyr Val Val Val Phe Asp Pro Leu Asp Gly Ser Ser Asn Ile Asp Ala
145 150 155 160
Ala Val Ser Thr Gly Ser Ile Trp Gly Ile Tyr Glu Ser Asp Ser Ser
165 170 175
Cys Ile Pro Asp Phe Gly Ser Asp Asp Ser Ala Lys Val Glu Glu Lys
180 185 190
Cys Val Met Asn Val Cys Gln Pro Gly Ser Asn Leu Leu Cys Ala Gly
195 200 205

Tyr Cys Met Tyr Ser Ser Ser Thr Ile Leu Val Ile Thr Ile Gly Gln
    210                 215                 220
Gly Val Phe Gly Phe Thr Leu Asp Pro Thr Val Gly Glu Phe Ile Met
225                 230                 235                 240
Ser His Glu Asn Ile Lys Val Pro Asp Ser Gly Lys Ile Tyr Ser Phe
                245                 250                 255
Asn Glu Gly Asn Tyr Ala Met Trp Ser Asp Gly Leu Lys Lys Tyr Met
            260                 265                 270
Asp Ser Leu Lys Thr Gly Gly Lys Asp Gly Gly Lys Pro Tyr Ser Ala
        275                 280                 285
Arg Tyr Ile Gly Ser Leu Val Gly Asp Phe His Arg Thr Ile Leu Tyr
    290                 295                 300
Gly Gly Ile Tyr Gly Tyr Pro Gly Asp Ala Lys Asn Pro Asn Gly Lys
305                 310                 315                 320
Leu Arg Leu Leu Tyr Glu Cys Ala Pro Met Ser Met Ile Ala Glu Gln
                325                 330                 335
Ala Gly Gly Lys Gly Ser Thr Gly Val Ala Arg Val Leu Asp Ile Val
            340                 345                 350
Pro Glu Lys Val His Gln Arg Val Pro Phe Phe Val Gly Ser Lys Asn
        355                 360                 365
Glu Val Ala Tyr Leu Glu Ser Phe Met
    370                 375

<210> 180
<211> 1254
<212> DNA
<213> Phaeodactylum tricornutum

<400> 180
atgtttattt tgaagtcccc ggcgctttgg ttgcttcttt acccagttgt tgcttttacg 60
gcggcgaggg cgaactcgat tcgtccagcg gccgcgttat ctgtcttcga tctgtcgagc 120
gtggaggcag taccgtctag aaagaccaag gccccaattt tcgatgaagt ttgcgacaca 180
actggagtca ctctcaaacg cttcatgacc gaggtttctc tactgaatcc tgaaatcgaa 240
gagctgacga cgctgtttgg tgcgattgaa accgcttgca aagccattgc aaatcttgtc 300
aagcgatcgc cgcttccctc cagtgacacg ttgggtcttc agggagaaat caacgttcaa 360
ggcgaagacc aaaagaaatt agatgtgatc gccaacgata ttttgaagcg agcactccgc 420
ttcacgggcc gcctcggagt cttagcctcg gaagaagagg atactcccgt cgatttgatg 480
ccaagggatc ctagtaccaa aaaagttcta atcgatgagg gagaaaagta tgtcgctgtc 540
ttcgatccgc tcgatggtag ctcaaacgtt gatgcaggca taccgacagg cacaataatt 600
gggatatacg agcacgacga aacttgcaag attgatcctg atgctttgga agaggatcgg 660
accaaacaag aaaacctatg cctcgcaaat actctgcagc ccggcaccaa cttggtagca 720
gcggcgtact gtttatattc ttcgtcaaca tttttggtgt tgacgctggg agctggaaca 780
tatggattca cgctagatga gactatcggt gaatttgtct tgagccatcc aaacattaag 840
attcctgaat gctcatccat tatgtcgttc aacgaggcaa atactcccag ctgggatcgt 900
ccgcttcaag acactttcgc aaagtggagg acagggacag gaaagagcgg caagaaattt 960
tcaagtcgct acattggttc tatggtaggg gatgtccatc ggacgctcct gtacggagga 1020
gtttttgggt atcctggcga caaaaagaat cccaacggga agctacgcct gctttatgaa 1080
ggagctccaa tgtcattcat catggaacag gcgggtggat tgtcgaccac tggcacgcag 1140
cgtgtcatgg aaatctcccc agatacggtc catcaacgtg tgccgattat catgggatcc 1200
agacaagatg tcgaagaggt catggacgcc tataaaaact ttggcatcga ataa 1254

<210> 181
<211> 417
<212> PRT
<213> Phaeodactylum tricornutum

<400> 181
Met Phe Ile Leu Lys Ser Pro Ala Leu Trp Leu Leu Leu Tyr Pro Val
1               5                   10                  15

```
Val Ala Phe Thr Ala Ala Arg Ala Asn Ser Ile Arg Pro Ala Ala Ala
            20                  25                  30
Leu Ser Val Phe Asp Leu Ser Ser Val Glu Ala Val Pro Ser Arg Lys
        35                  40                  45
Thr Lys Ala Pro Ile Phe Asp Glu Val Cys Asp Thr Thr Gly Val Thr
    50                  55                  60
Leu Lys Arg Phe Met Thr Glu Val Ser Leu Leu Asn Pro Glu Ile Glu
65                  70                  75                  80
Glu Leu Thr Thr Leu Phe Gly Ala Ile Glu Thr Ala Cys Lys Ala Ile
                85                  90                  95
Ala Asn Leu Val Lys Arg Ser Pro Leu Pro Ser Ser Asp Thr Leu Gly
            100                 105                 110
Leu Gln Gly Glu Ile Asn Val Gln Gly Glu Asp Gln Lys Lys Leu Asp
        115                 120                 125
Val Ile Ala Asn Asp Ile Leu Lys Arg Ala Leu Arg Phe Thr Gly Arg
    130                 135                 140
Leu Gly Val Leu Ala Ser Glu Glu Glu Asp Thr Pro Val Asp Leu Met
145                 150                 155                 160
Pro Arg Asp Pro Ser Thr Lys Lys Val Leu Ile Asp Glu Gly Glu Lys
                165                 170                 175
Tyr Val Ala Val Phe Asp Pro Leu Asp Gly Ser Ser Asn Val Asp Ala
            180                 185                 190
Gly Ile Pro Thr Gly Thr Ile Ile Gly Ile Tyr Glu His Asp Glu Thr
        195                 200                 205
Cys Lys Ile Asp Pro Asp Ala Leu Glu Glu Asp Arg Thr Lys Gln Glu
    210                 215                 220
Asn Leu Cys Leu Ala Asn Thr Leu Gln Pro Gly Thr Asn Leu Val Ala
225                 230                 235                 240
Ala Ala Tyr Cys Leu Tyr Ser Ser Ser Thr Phe Leu Val Leu Thr Leu
                245                 250                 255
Gly Ala Gly Thr Tyr Gly Phe Thr Leu Asp Glu Thr Ile Gly Glu Phe
            260                 265                 270
Val Leu Ser His Pro Asn Ile Lys Ile Pro Glu Cys Ser Ser Ile Met
        275                 280                 285
Ser Phe Asn Glu Ala Asn Thr Pro Ser Trp Asp Arg Pro Leu Gln Asp
    290                 295                 300
Thr Phe Ala Lys Trp Arg Thr Gly Thr Gly Lys Ser Gly Lys Lys Phe
305                 310                 315                 320
Ser Ser Arg Tyr Ile Gly Ser Met Val Gly Asp Val His Arg Thr Leu
                325                 330                 335
Leu Tyr Gly Gly Val Phe Gly Tyr Pro Gly Asp Lys Lys Asn Pro Asn
            340                 345                 350
Gly Lys Leu Arg Leu Leu Tyr Glu Gly Ala Pro Met Ser Phe Ile Met
        355                 360                 365
Glu Gln Ala Gly Gly Leu Ser Thr Thr Gly Thr Gln Arg Val Met Glu
    370                 375                 380
Ile Ser Pro Asp Thr Val His Gln Arg Val Pro Ile Ile Met Gly Ser
385                 390                 395                 400
Arg Gln Asp Val Glu Glu Val Met Asp Ala Tyr Lys Asn Phe Gly Ile
                405                 410                 415
Glu
```

<210> 182
<211> 1233
<212> DNA
<213> Pisum sativa

<400> 182

```
atggttgcaa tggcagcagc aacagcctca tctcagttaa tattctcaaa accttactct      60
ccttcacgtc tttgcccctt ccaactctgt gtctttgatg caaaatcagt gttatcaagt     120
tcaaggagaa agcatgtgaa tggctctggt gttagatgta tggctgtgaa ggaagcaact     180
agtgagacaa agaaaagaag tggatatgag attataacac tgactagttg gttgttgcag     240
caagaacaaa aagggattat tgatgcagaa cttactattg tactttctag tatttctatg     300
gcatgtaaac aaattgcttc tttggttcaa agagccaata tttctaacct cactggtact     360
caaggtgctg taaatattca aggggaagac cagaaaaaac ttgatgttat ctcaaatgag     420
gtattctcaa attgcttgag gtcaagtggg aggacaggga taattgcgtc ggaggaagag     480
gatgtcgcgg tggcagtaga agagagttat tcaggaaact acattgttgt atttgatcca     540
cttgatggtt catccaatct tgatgctgca gtctcaaccg gttccatttt cgggatttac     600
agccccaatg acgagtgtct tcctgatttt ggtgatgact ctgatgacaa cacacttggc     660
acagaagaac aaaggtgcat tgtgaatgtg tgtcaaccag gaagcaacct tctagcagct     720
ggctactgca tgtattctag ttcagtagct tttgttctta ccataggcaa aggagtgttt     780
gtattcacat tagatccatt gtacggagaa ttcgttttga ctcaagagaa tctccaaata     840
ccgaaatcag ggaaatcta ttctttcaat gaaggaatt acaagttgtg ggatgaaaac      900
ttgaagaaat atattgatga tcttaaggaa ccaggtccta gtggcaagcc ttattcagca     960
aggtatattg gtagtttggt tggtgatttt cacaggacac tgttatatgg tggcatttat    1020
ggatacccta gggacaagaa aagtaagaat gggaagctta ggcttttata tgaatgtgct    1080
ccaatgagct tcattgttga acaggctggt ggaaaaggtt cagatggtca tcaaagagta    1140
cttgacattc aacccacaga aattcatcaa cgtgttccac tttacattgg gagcacagaa    1200
gaggtggaga aggttgaaaa gtacttagct taa                                 1233
```

```
<210>  183
<211>  410
<212>  PRT
<213>  Pisum sativa

<400>  183
Met Val Ala Met Ala Ala Ala Thr Ala Ser Ser Gln Leu Ile Phe Ser
1               5                   10                  15
Lys Pro Tyr Ser Pro Ser Arg Leu Cys Pro Phe Gln Leu Cys Val Phe
            20                  25                  30
Asp Ala Lys Ser Val Leu Ser Ser Ser Arg Arg Lys His Val Asn Gly
        35                  40                  45
Ser Gly Val Arg Cys Met Ala Val Lys Glu Ala Thr Ser Glu Thr Lys
    50                  55                  60
Lys Arg Ser Gly Tyr Glu Ile Ile Thr Leu Thr Ser Trp Leu Leu Gln
65                  70                  75                  80
Gln Glu Gln Lys Gly Ile Ile Asp Ala Glu Leu Thr Ile Val Leu Ser
                85                  90                  95
Ser Ile Ser Met Ala Cys Lys Gln Ile Ala Ser Leu Val Gln Arg Ala
            100                 105                 110
Asn Ile Ser Asn Leu Thr Gly Thr Gln Gly Ala Val Asn Ile Gln Gly
        115                 120                 125
Glu Asp Gln Lys Lys Leu Asp Val Ile Ser Asn Glu Val Phe Ser Asn
    130                 135                 140
Cys Leu Arg Ser Ser Gly Arg Thr Gly Ile Ile Ala Ser Glu Glu Glu
145                 150                 155                 160
Asp Val Ala Val Ala Val Glu Glu Ser Tyr Ser Gly Asn Tyr Ile Val
                165                 170                 175
Val Phe Asp Pro Leu Asp Gly Ser Ser Asn Leu Asp Ala Ala Val Ser
            180                 185                 190
Thr Gly Ser Ile Phe Gly Ile Tyr Ser Pro Asn Asp Glu Cys Leu Pro
        195                 200                 205
Asp Phe Gly Asp Asp Ser Asp Asp Asn Thr Leu Gly Thr Glu Glu Gln
    210                 215                 220
Arg Cys Ile Val Asn Val Cys Gln Pro Gly Ser Asn Leu Leu Ala Ala
225                 230                 235                 240
Gly Tyr Cys Met Tyr Ser Ser Ser Val Ala Phe Val Leu Thr Ile Gly
```

```
                245                 250                 255
Lys Gly Val Phe Val Phe Thr Leu Asp Pro Leu Tyr Gly Glu Phe Val
            260                 265                 270
Leu Thr Gln Glu Asn Leu Gln Ile Pro Lys Ser Gly Glu Ile Tyr Ser
        275                 280                 285
Phe Asn Glu Gly Asn Tyr Lys Leu Trp Asp Glu Asn Leu Lys Lys Tyr
    290                 295                 300
Ile Asp Asp Leu Lys Glu Pro Gly Pro Ser Gly Lys Pro Tyr Ser Ala
305                 310                 315                 320
Arg Tyr Ile Gly Ser Leu Val Gly Asp Phe His Arg Thr Leu Leu Tyr
                325                 330                 335
Gly Gly Ile Tyr Gly Tyr Pro Arg Asp Lys Lys Ser Lys Asn Gly Lys
            340                 345                 350
Leu Arg Leu Leu Tyr Glu Cys Ala Pro Met Ser Phe Ile Val Glu Gln
        355                 360                 365
Ala Gly Gly Lys Gly Ser Asp Gly His Gln Arg Val Leu Asp Ile Gln
    370                 375                 380
Pro Thr Glu Ile His Gln Arg Val Pro Leu Tyr Ile Gly Ser Thr Glu
385                 390                 395                 400
Glu Val Glu Lys Val Glu Lys Tyr Leu Ala
                405                 410
```

<210> 184
<211> 1242
<212> DNA
<213> Poncirus trifoliata

<400> 184

```
atggttgcag cagcagcgac aacatcatca cagcttctct tttcaagctc tcactctttc      60
tctcggctct ctccttacca aatatgtgtc ttcgattcca aagcactcgt gtcgtcatgt     120
cccagcaacg ttatgaagag aagacatgtt ggtgttgctg ctggggttcg gtgcatggct     180
gttgggacaa catcggaggt tgcaaccaag aagagaagtt cgtatgagat tgaaacgctg     240
acaaactggc tgttgaagca agaacagtct ggcgttattg atgctgagct cactattgtg     300
ctttccagca tttcaacggc gtgcaagcag attgcttctt tggtgcaaag agctggcatt     360
tccaacttga ctggaattca gggtgctgtc aatgttcaag gcgaggacca gaagaagctc     420
gacgtcgttt caaatgaggt gttctcaaac tgtttgagat caagtgggcg aactgggatt     480
atagcatcag aggaagagga tgtaccagtg gcggtagaag agagctactc tggaaactat     540
attgtagttt ttgatccact tgatggatca tccaacattg atgctgcagt gtctactgga     600
tccatctttg gcatatacag cccaaatgat gagtgtcttg ccgatattgg tgatgattct     660
actctgggca atactgaaca aagatgtgta gtgaacgtgt gccagccagg aagcaacctt     720
cttgctgcag gctattgcat gtattcaagc tctgtaatct ttgtgataac tttaggcaac     780
ggagtctttg cattcaccct ggatcccatg tatggagaat ttgttttgac acaagagaac     840
attcagatac ccaaaaccgg aaagatctat gcttttaatg aaggcaacta ccagctttgg     900
gatgacaagt tgaagaagta cattgacgat cttaaggatc caggacccag cggcaagccc     960
tattctgcta ggtacattgg cagcttggtt ggtgatttcc atcgaactct gctctacggc    1020
ggcatttatg gctatccaag agacaagaag agcaagaatg gaaagctgag gctcttgtat    1080
gaatgtgcac caatgagctt catagtggaa caagcaggag gcaaaggatc tgacggccat    1140
caaagagtac ttgacattca acctactgag attcaccagc gtattccttt aaagattgga    1200
agccaggagg aagtggagaa actggagaag tatttggcct aa                       1242
```

<210> 185
<211> 413
<212> PRT
<213> Poncirus trifoliata

<400> 185

```
Met Val Ala Ala Ala Ala Thr Thr Ser Ser Gln Leu Leu Phe Ser Ser
1               5                   10                  15
Ser His Ser Phe Ser Arg Leu Ser Pro Tyr Gln Ile Cys Val Phe Asp
```

```
                20                  25                  30
Ser Lys Ala Leu Val Ser Ser Cys Pro Ser Asn Val Met Lys Arg Arg
            35                  40                  45
His Val Gly Val Ala Ala Gly Val Arg Cys Met Ala Val Gly Thr Thr
    50                  55                  60
Ser Glu Val Ala Thr Lys Lys Arg Ser Ser Tyr Glu Ile Glu Thr Leu
65                  70                  75                  80
Thr Asn Trp Leu Leu Lys Gln Glu Gln Ser Gly Val Ile Asp Ala Glu
                85                  90                  95
Leu Thr Ile Val Leu Ser Ser Ile Ser Thr Ala Cys Lys Gln Ile Ala
            100                 105                 110
Ser Leu Val Gln Arg Ala Gly Ile Ser Asn Leu Thr Gly Ile Gln Gly
        115                 120                 125
Ala Val Asn Val Gln Gly Glu Asp Gln Lys Lys Leu Asp Val Val Ser
    130                 135                 140
Asn Glu Val Phe Ser Asn Cys Leu Arg Ser Ser Gly Arg Thr Gly Ile
145                 150                 155                 160
Ile Ala Ser Glu Glu Glu Asp Val Pro Val Ala Val Glu Glu Ser Tyr
                165                 170                 175
Ser Gly Asn Tyr Ile Val Val Phe Asp Pro Leu Asp Gly Ser Ser Asn
            180                 185                 190
Ile Asp Ala Ala Val Ser Thr Gly Ser Ile Phe Gly Ile Tyr Ser Pro
        195                 200                 205
Asn Asp Glu Cys Leu Ala Asp Ile Gly Asp Asp Ser Thr Leu Gly Asn
    210                 215                 220
Thr Glu Gln Arg Cys Val Val Asn Val Cys Gln Pro Gly Ser Asn Leu
225                 230                 235                 240
Leu Ala Ala Gly Tyr Cys Met Tyr Ser Ser Ser Val Ile Phe Val Ile
                245                 250                 255
Thr Leu Gly Asn Gly Val Phe Ala Phe Thr Leu Asp Pro Met Tyr Gly
            260                 265                 270
Glu Phe Val Leu Thr Gln Glu Asn Ile Gln Ile Pro Lys Thr Gly Lys
        275                 280                 285
Ile Tyr Ala Phe Asn Glu Gly Asn Tyr Gln Leu Trp Asp Asp Lys Leu
    290                 295                 300
Lys Lys Tyr Ile Asp Asp Leu Lys Asp Pro Gly Pro Ser Gly Lys Pro
305                 310                 315                 320
Tyr Ser Ala Arg Tyr Ile Gly Ser Leu Val Gly Asp Phe His Arg Thr
                325                 330                 335
Leu Leu Tyr Gly Gly Ile Tyr Gly Tyr Pro Arg Asp Lys Lys Ser Lys
            340                 345                 350
Asn Gly Lys Leu Arg Leu Leu Tyr Glu Cys Ala Pro Met Ser Phe Ile
        355                 360                 365
Val Glu Gln Ala Gly Gly Lys Gly Ser Asp Gly His Gln Arg Val Leu
    370                 375                 380
Asp Ile Gln Pro Thr Glu Ile His Gln Arg Ile Pro Leu Lys Ile Gly
385                 390                 395                 400
Ser Gln Glu Glu Val Glu Lys Leu Glu Lys Tyr Leu Ala
                405                 410

<210>  186
<211>  1245
<212>  DNA
<213>  Populus tremuloides

<400>  186
atggttgcac aagcagcagc aataacaact tcatcatcac atcttctctt ctcaacctca      60
cgctcactgt ctcgcccatc tccttcccag ttatgtgtct ttgactcaaa aacacttgtg     120
tcatacccca acagcactag tacttacaag aaaaaacgtg gtggtgatgg gctcaagtgc     180
```

```
atggctgtga gcacagcctc ggatgctaaa acaaagaaga gtacgtttga gattcaaaca     240
ctgactggtt ggctgttgaa gcaagaacaa gctggtgtta ttgatgctga gctcactatt     300
gttatatcaa gcatttcaat ggcatgtaag cagattgctt ctttggtgca aagagctagc     360
atttctaact taactggagt tcaaggttct gttaacgttc aaggagaaga tcagaagaag     420
cttgacgtgg tctctaatga ggtgttctct agctgcttga gatcaagtgg gaggacagga     480
atcatagcat cagaggaaga ggacgtgcca gtggcagtgg aggagagtta ctctggaaac     540
tatatagtgg tttttgaccc actcgatgga tcatccaaca ttgatgctgc agtgtctact     600
ggttccatct ttggaatata cagccccaat gacgaatgcc tggccgatat tggagatgac     660
tccactcttg atcaaacgga acagaggtgt attgtgaatg tgtgccagcc aggaaataac     720
ctccttgttg ctggctactg catgtattca agctcagtga tttttgtgct aactattgga     780
aaaggcgtgt tctctttcag cttggatcca atgtatggag agtttgtttt aactcaagaa     840
aacatccaga taccaaaggc tggaaagatt tattcattta atgaaggaaa ctaccagttg     900
tgggatgaca agctgaagaa gtacattgat gaccttaaag accctggtcc gagtggcaag     960
ccctactccg ctagatacat tggaagcttg gtcggtgact tccaccggac gctgctgtac    1020
ggtggcattt atgggtaccc cagggacaag aagagcaaga atgggaagct gaggcttctg    1080
tatgagtgtg caccgatgag ctttatagtg gaacaagctg gtgggaaagg atcagacggg    1140
catcagagag tactggatat cactcctact gagatacacc agcgtgttcc gctttacata    1200
gggagcgtgg aggaagtgga gaaattggag aagtatttgg cttga                   1245
```

```
<210>  187
<211>  414
<212>  PRT
<213>  Populus tremuloides

<400>  187
Met Val Ala Gln Ala Ala Ala Ile Thr Thr Ser Ser Ser His Leu Leu
1               5                   10                  15
Phe Ser Thr Ser Arg Ser Leu Ser Arg Pro Ser Pro Ser Gln Leu Cys
            20                  25                  30
Val Phe Asp Ser Lys Thr Leu Val Ser Tyr Pro Asn Ser Thr Ser Thr
        35                  40                  45
Tyr Lys Lys Lys Arg Gly Gly Asp Gly Leu Lys Cys Met Ala Val Ser
    50                  55                  60
Thr Ala Ser Asp Ala Lys Thr Lys Lys Ser Thr Phe Glu Ile Gln Thr
65                  70                  75                  80
Leu Thr Gly Trp Leu Leu Lys Gln Glu Gln Ala Gly Val Ile Asp Ala
                85                  90                  95
Glu Leu Thr Ile Val Ile Ser Ser Ile Ser Met Ala Cys Lys Gln Ile
            100                 105                 110
Ala Ser Leu Val Gln Arg Ala Ser Ile Ser Asn Leu Thr Gly Val Gln
        115                 120                 125
Gly Ser Val Asn Val Gln Gly Glu Asp Gln Lys Lys Leu Asp Val Val
    130                 135                 140
Ser Asn Glu Val Phe Ser Ser Cys Leu Arg Ser Ser Gly Arg Thr Gly
145                 150                 155                 160
Ile Ile Ala Ser Glu Glu Glu Asp Val Pro Val Ala Val Glu Glu Ser
                165                 170                 175
Tyr Ser Gly Asn Tyr Ile Val Val Phe Asp Pro Leu Asp Gly Ser Ser
            180                 185                 190
Asn Ile Asp Ala Ala Val Ser Thr Gly Ser Ile Phe Gly Ile Tyr Ser
        195                 200                 205
Pro Asn Asp Glu Cys Leu Ala Asp Ile Gly Asp Asp Ser Thr Leu Asp
    210                 215                 220
Gln Thr Glu Gln Arg Cys Ile Val Asn Val Cys Gln Pro Gly Asn Asn
225                 230                 235                 240
Leu Leu Val Ala Gly Tyr Cys Met Tyr Ser Ser Ser Val Ile Phe Val
                245                 250                 255
Leu Thr Ile Gly Lys Gly Val Phe Ser Phe Ser Leu Asp Pro Met Tyr
            260                 265                 270
```

Gly Glu Phe Val Leu Thr Gln Glu Asn Ile Gln Ile Pro Lys Ala Gly
275 280 285
Lys Ile Tyr Ser Phe Asn Glu Gly Asn Tyr Gln Leu Trp Asp Asp Lys
290 295 300
Leu Lys Lys Tyr Ile Asp Asp Leu Lys Asp Pro Gly Pro Ser Gly Lys
305 310 315 320
Pro Tyr Ser Ala Arg Tyr Ile Gly Ser Leu Val Gly Asp Phe His Arg
325 330 335
Thr Leu Leu Tyr Gly Gly Ile Tyr Gly Tyr Pro Arg Asp Lys Lys Ser
340 345 350
Lys Asn Gly Lys Leu Arg Leu Leu Tyr Glu Cys Ala Pro Met Ser Phe
355 360 365
Ile Val Glu Gln Ala Gly Gly Lys Gly Ser Asp Gly His Gln Arg Val
370 375 380
Leu Asp Ile Thr Pro Thr Glu Ile His Gln Arg Val Pro Leu Tyr Ile
385 390 395 400
Gly Ser Val Glu Glu Val Glu Lys Leu Glu Lys Tyr Leu Ala
405 410

<210> 188
<211> 1227
<212> DNA
<213> Solanum tuberosum

<400> 188
atggcagcat cagcagccac agcaacagca acaacttcat atttaagtgc tctagacaaa 60
aagactccat ttttatttgc cttagacaaa aagactccat ttttatgccc aaaaagcagc 120
acgaagagaa ggtcatttaa tggaggagta aagtgcatgg caatagagac aacttcaggt 180
tttacagcaa ccaagaaaag gagtggctat gagctgcaaa ctttaacaag ttggctatta 240
agacaagaac aagctggagt gattgatgct gaacttacca tagttatttc aagtatttca 300
atggcttgta aacagattgc ttccttagtc caaagagctg gaatttctaa ccttactgga 360
gttcaaggtg ctgtcaatat tcaaggagaa gatcagaaga aacttgatgt tgtctctaat 420
gaggttttct caaattgtct aagatcaagt ggaaggactg ggattatagc atcagaagaa 480
gaggatgtac ctgtggcagt ggaagagagt tactcaggca actacattgt ggtgtttgat 540
cctcttgatg gatcatcaaa cattgatgct gctgtgtcta ccggttctat ctttggaata 600
tacaacccga atgatgagtg cctcgctgat catggagatg attccacgct tgacaatata 660
gagcagaagt gcattgtgaa tgtatgtcaa ccagggacaa accttcttgc agcaggatac 720
tgcatgtact caagctctgt gatattcgta ctcaccttgg gaaatggcgt tttttccttt 780
aacttggatc cgatgtacgg agaatttgtt ctgactcaag aaaatgtcca aataccaaag 840
tctggaaaga tctattcatt caatgaagga aactaccagc tctgggatga caagttgaag 900
aaatatatcg atgacttgaa ggaccctggc cctagtggca agccttactc tgcaaggtac 960
attggtagtt tggttggtga cttccataga actcttctat atggtggcat ttatggttat 1020
cctagagacc aaaagagcaa gaatggaaag ttgaggcttt tgtacgagtg tgctcccatg 1080
agcttcattg tggaacaagc tggtggtaaa ggatccgatg gtcaccaaag agttctcgat 1140
atccaaccaa ctgaggtaca tcaacgagtt ccattgtaca ttggaagcac agaagaagtt 1200
gaaaaattgg agaagtactt gtcttaa 1227

<210> 189
<211> 408
<212> PRT
<213> Solanum tuberosum

<400> 189
Met Ala Ala Ser Ala Ala Thr Ala Thr Ala Thr Thr Ser Tyr Leu Ser
1 5 10 15
Ala Leu Asp Lys Lys Thr Pro Phe Leu Phe Ala Leu Asp Lys Lys Thr
20 25 30
Pro Phe Leu Cys Pro Lys Ser Ser Thr Lys Arg Arg Ser Phe Asn Gly
35 40 45

```
Gly Val Lys Cys Met Ala Ile Glu Thr Thr Ser Gly Phe Thr Ala Thr
    50                  55                  60
Lys Lys Arg Ser Gly Tyr Glu Leu Gln Thr Leu Thr Ser Trp Leu Leu
65                  70                  75                  80
Arg Gln Glu Gln Ala Gly Val Ile Asp Ala Glu Leu Thr Ile Val Ile
                85                  90                  95
Ser Ser Ile Ser Met Ala Cys Lys Gln Ile Ala Ser Leu Val Gln Arg
            100                 105                 110
Ala Gly Ile Ser Asn Leu Thr Gly Val Gln Gly Ala Val Asn Ile Gln
        115                 120                 125
Gly Glu Asp Gln Lys Lys Leu Asp Val Val Ser Asn Glu Val Phe Ser
    130                 135                 140
Asn Cys Leu Arg Ser Ser Gly Arg Thr Gly Ile Ile Ala Ser Glu Glu
145                 150                 155                 160
Glu Asp Val Pro Val Ala Val Glu Glu Ser Tyr Ser Gly Asn Tyr Ile
                165                 170                 175
Val Val Phe Asp Pro Leu Asp Gly Ser Ser Asn Ile Asp Ala Ala Val
            180                 185                 190
Ser Thr Gly Ser Ile Phe Gly Ile Tyr Asn Pro Asn Asp Glu Cys Leu
        195                 200                 205
Ala Asp His Gly Asp Asp Ser Thr Leu Asp Asn Ile Glu Gln Lys Cys
    210                 215                 220
Ile Val Asn Val Cys Gln Pro Gly Thr Asn Leu Leu Ala Ala Gly Tyr
225                 230                 235                 240
Cys Met Tyr Ser Ser Ser Val Ile Phe Val Leu Thr Leu Gly Asn Gly
                245                 250                 255
Val Phe Ser Phe Asn Leu Asp Pro Met Tyr Gly Glu Phe Val Leu Thr
            260                 265                 270
Gln Glu Asn Val Gln Ile Pro Lys Ser Gly Lys Ile Tyr Ser Phe Asn
        275                 280                 285
Glu Gly Asn Tyr Gln Leu Trp Asp Asp Lys Leu Lys Lys Tyr Ile Asp
    290                 295                 300
Asp Leu Lys Asp Pro Gly Pro Ser Gly Lys Pro Tyr Ser Ala Arg Tyr
305                 310                 315                 320
Ile Gly Ser Leu Val Gly Asp Phe His Arg Thr Leu Leu Tyr Gly Gly
                325                 330                 335
Ile Tyr Gly Tyr Pro Arg Asp Gln Lys Ser Lys Asn Gly Lys Leu Arg
            340                 345                 350
Leu Leu Tyr Glu Cys Ala Pro Met Ser Phe Ile Val Glu Gln Ala Gly
        355                 360                 365
Gly Lys Gly Ser Asp Gly His Gln Arg Val Leu Asp Ile Gln Pro Thr
    370                 375                 380
Glu Val His Gln Arg Val Pro Leu Tyr Ile Gly Ser Thr Glu Glu Val
385                 390                 395                 400
Glu Lys Leu Glu Lys Tyr Leu Ser
                405
```

<210> 190
<211> 1248
<212> DNA
<213> Spinacia oleracea

<400> 190

```
atggcatcaa taggaccagc aacaacaact gcagtaaaac tgcgtagctc aatcttcaat      60
cctcagtcat ctactctttc cccgtctcaa caatgcatta catttacaaa gtcccttcac     120
tcgtttccta ctgccacccg acataatgtg gcttccgggg ttcgttgtat ggcagccgta     180
ggagaggcgg ctacagaaac aaaggcaagg actagaagta agtacgaaat tgaaacacta     240
acaggctggc tgcttaaaca agaaatggca ggtgttattg atgctgaact taccatcgtt     300
ctttctagca tttcattggc ttgtaaacaa attgcttcct tggttcaacg agctggtatt     360
```

```
tctaacttga ctggaattca aggtgctgtc aatatccaag gagaggatca gaagaaactt     420
gatgttgtct ccaatgaggt gttttcgagc tgcttgagat cgagtggaag aacaggaata     480
atagcatcag aagaagagga tgtaccagtg gcagtggaag agagttactc tggaaactat     540
attgttgtgt ttgatccact tgatggttca tccaacattg atgcagctgt ctccactggt     600
tccatctttg gcatttatag ccctaacgat gagtgcattg ttgactctga tcacgacgat     660
gagtcacagc taagtgcaga agaacagagg tgtgtagtga atgtatgtca accaggggat     720
aacctattag cagcagggta ttgtatgtac tcaagctctg ttatcttcgt acttacaatt     780
ggtaaaggtg tgtatgcatt cacattagat ccaatgtatg gtgaattcgt actcacttca     840
gagaaaatcc aaatcccaaa agctgggaag atctattcat tcaatgaagg taactacaaa     900
atgtgggatg ataaattgaa gaagtacatg gatgatctta aagagccagg agagtcacag     960
aaaccgtact cgtctcgtta catagggagt ttagttgggg actttcatag aacactttta    1020
tatggtggga tttatggtta cccaagagat gcaaagagta agaatgggaa attgaggctt    1080
ttgtatgaat gtgcacctat gagttttatt gttgaacaag ctggtggtaa aggttctgat    1140
ggtcatcaaa gaattcttga cattcaaccc accgagatac atcaacgtgt gccactgtac    1200
atcgggagtg tggaggaagt agagaaatta gagaagtact tagcataa                 1248
```

<210> 191
<211> 415
<212> PRT
<213> Spinacia oleracea

<400> 191

```
Met Ala Ser Ile Gly Pro Ala Thr Thr Thr Ala Val Lys Leu Arg Ser
1               5                   10                  15
Ser Ile Phe Asn Pro Gln Ser Ser Thr Leu Ser Pro Ser Gln Gln Cys
            20                  25                  30
Ile Thr Phe Thr Lys Ser Leu His Ser Phe Pro Thr Ala Thr Arg His
        35                  40                  45
Asn Val Ala Ser Gly Val Arg Cys Met Ala Ala Val Gly Glu Ala Ala
    50                  55                  60
Thr Glu Thr Lys Ala Arg Thr Arg Ser Lys Tyr Glu Ile Glu Thr Leu
65                  70                  75                  80
Thr Gly Trp Leu Leu Lys Gln Glu Met Ala Gly Val Ile Asp Ala Glu
                85                  90                  95
Leu Thr Ile Val Leu Ser Ser Ile Ser Leu Ala Cys Lys Gln Ile Ala
            100                 105                 110
Ser Leu Val Gln Arg Ala Gly Ile Ser Asn Leu Thr Gly Ile Gln Gly
        115                 120                 125
Ala Val Asn Ile Gln Gly Glu Asp Gln Lys Lys Leu Asp Val Val Ser
    130                 135                 140
Asn Glu Val Phe Ser Ser Cys Leu Arg Ser Ser Gly Arg Thr Gly Ile
145                 150                 155                 160
Ile Ala Ser Glu Glu Glu Asp Val Pro Val Ala Val Glu Glu Ser Tyr
                165                 170                 175
Ser Gly Asn Tyr Ile Val Val Phe Asp Pro Leu Asp Gly Ser Ser Asn
            180                 185                 190
Ile Asp Ala Ala Val Ser Thr Gly Ser Ile Phe Gly Ile Tyr Ser Pro
        195                 200                 205
Asn Asp Glu Cys Ile Val Asp Ser Asp His Asp Asp Glu Ser Gln Leu
    210                 215                 220
Ser Ala Glu Glu Gln Arg Cys Val Val Asn Val Cys Gln Pro Gly Asp
225                 230                 235                 240
Asn Leu Leu Ala Ala Gly Tyr Cys Met Tyr Ser Ser Ser Val Ile Phe
                245                 250                 255
Val Leu Thr Ile Gly Lys Gly Val Tyr Ala Phe Thr Leu Asp Pro Met
            260                 265                 270
Tyr Gly Glu Phe Val Leu Thr Ser Glu Lys Ile Gln Ile Pro Lys Ala
        275                 280                 285
Gly Lys Ile Tyr Ser Phe Asn Glu Gly Asn Tyr Lys Met Trp Asp Asp
```

```
    290                 295                 300
Lys Leu Lys Lys Tyr Met Asp Asp Leu Lys Glu Pro Gly Glu Ser Gln
305                 310                 315                 320
Lys Pro Tyr Ser Ser Arg Tyr Ile Gly Ser Leu Val Gly Asp Phe His
                325                 330                 335
Arg Thr Leu Leu Tyr Gly Gly Ile Tyr Gly Tyr Pro Arg Asp Ala Lys
            340                 345                 350
Ser Lys Asn Gly Lys Leu Arg Leu Leu Tyr Glu Cys Ala Pro Met Ser
        355                 360                 365
Phe Ile Val Glu Gln Ala Gly Gly Lys Gly Ser Asp Gly His Gln Arg
    370                 375                 380
Ile Leu Asp Ile Gln Pro Thr Glu Ile His Gln Arg Val Pro Leu Tyr
385                 390                 395                 400
Ile Gly Ser Val Glu Glu Val Glu Lys Leu Glu Lys Tyr Leu Ala
                405                 410                 415
```

<210> 192
<211> 1230
<212> DNA
<213> Triticum aestivum

<400> 192

```
atggccgccg cgaccaccac cacctcccgc ccgcttctgc tgtcccgcca gcaggcggcg      60
gctagctccc tccaatgccg cctccccagg aggcccggaa gcagcctctt tgccggccag     120
ggccaggcgt cgactccgaa tgtgcggtgc atggcagtcg tggacacggc ctcggcgccg     180
gcgccggcgg cggctaggaa gaggagcagc tacgacatga tcacgctgac gacgtggctg     240
ctgaagcagg agcaggaggg ggtcatcgac aacgagatga ccatcgtgct gtccagcata     300
tccacggcgt gcaagcagat cgcctcgttg gtgcagcgcg cgcccatctc caacctcacc     360
ggcgtccagg gcgccaccaa cgtgcagggc gaggaccaga agaagctcga cgtcatctcc     420
aacgaggtgt tctcgaactg cctgaggtgg agtggccgca ccggcgtgat cgcatcggag     480
gaggaggacg tgccggtggc ggtggaggag agctactcgg gcaactacat cgtggtgttc     540
gacccgctcg acggctcctc caacatcgac gccgccgtct ccaccggctc catcttcggc     600
atctacagcc catccgacga gtgccacatt ggcgacgacg caacccttga cgaagtgacg     660
cagatgtgca tagtgaacgt gtgccagcca gggagcaacc tgctcgccgc cggctactgc     720
atgtactcga gctcggtcat cttcgtgctc accatcggca ccggggtgta cgtgttcacg     780
ctggacccga tgtacggcga gttcgtgctg acgcaggaga aggtgcagat cccaaagtcg     840
ggcaagatct actccttcaa cgagggcaac tacgcgctct gggacgacaa gctcaagaag     900
tacatggaca gcctcaagga gcccggcacc tccggcaagc cctactccgc gcgctacatc     960
ggcagcctcg tcggcgactt ccaccgcacc atgctctacg gcggcatcta cggttacccc    1020
agcgaccaga agagcaagaa cggcaagctg cggctgctct acgagtgcgc gcccatgagc    1080
ttcatcgccg agcaggccgg cggcaaaggc tccgacggcc accagagggt actcgacatc    1140
atgcccacag cggtccatca gagagtgcct ctgtacgtcg ggagcgtgga ggaagtggag    1200
aaggtggaga aattcttgtc ttcagagtag                                     1230
```

<210> 193
<211> 409
<212> PRT
<213> Triticum aestivum

<400> 193

```
Met Ala Ala Ala Thr Thr Thr Thr Ser Arg Pro Leu Leu Leu Ser Arg
1               5                   10                  15
Gln Gln Ala Ala Ala Ser Ser Leu Gln Cys Arg Leu Pro Arg Arg Pro
            20                  25                  30
Gly Ser Ser Leu Phe Ala Gly Gln Gly Gln Ala Ser Thr Pro Asn Val
        35                  40                  45
Arg Cys Met Ala Val Val Asp Thr Ala Ser Ala Pro Ala Pro Ala Ala
    50                  55                  60
Ala Arg Lys Arg Ser Ser Tyr Asp Met Ile Thr Leu Thr Thr Trp Leu
```

```
65                  70                  75                  80
Leu Lys Gln Glu Gln Glu Gly Val Ile Asp Asn Glu Met Thr Ile Val
                85                  90                  95
Leu Ser Ser Ile Ser Thr Ala Cys Lys Gln Ile Ala Ser Leu Val Gln
            100                 105                 110
Arg Ala Pro Ile Ser Asn Leu Thr Gly Val Gln Gly Ala Thr Asn Val
        115                 120                 125
Gln Gly Glu Asp Gln Lys Lys Leu Asp Val Ile Ser Asn Glu Val Phe
    130                 135                 140
Ser Asn Cys Leu Arg Trp Ser Gly Arg Thr Gly Val Ile Ala Ser Glu
145                 150                 155                 160
Glu Glu Asp Val Pro Val Ala Val Glu Glu Ser Tyr Ser Gly Asn Tyr
                165                 170                 175
Ile Val Val Phe Asp Pro Leu Asp Gly Ser Ser Asn Ile Asp Ala Ala
            180                 185                 190
Val Ser Thr Gly Ser Ile Phe Gly Ile Tyr Ser Pro Ser Asp Glu Cys
        195                 200                 205
His Ile Gly Asp Asp Ala Thr Leu Asp Glu Val Thr Gln Met Cys Ile
    210                 215                 220
Val Asn Val Cys Gln Pro Gly Ser Asn Leu Leu Ala Ala Gly Tyr Cys
225                 230                 235                 240
Met Tyr Ser Ser Ser Val Ile Phe Val Leu Thr Ile Gly Thr Gly Val
                245                 250                 255
Tyr Val Phe Thr Leu Asp Pro Met Tyr Gly Glu Phe Val Leu Thr Gln
            260                 265                 270
Glu Lys Val Gln Ile Pro Lys Ser Gly Lys Ile Tyr Ser Phe Asn Glu
        275                 280                 285
Gly Asn Tyr Ala Leu Trp Asp Asp Lys Leu Lys Lys Tyr Met Asp Ser
    290                 295                 300
Leu Lys Glu Pro Gly Thr Ser Gly Lys Pro Tyr Ser Ala Arg Tyr Ile
305                 310                 315                 320
Gly Ser Leu Val Gly Asp Phe His Arg Thr Met Leu Tyr Gly Gly Ile
                325                 330                 335
Tyr Gly Tyr Pro Ser Asp Gln Lys Ser Lys Asn Gly Lys Leu Arg Leu
            340                 345                 350
Leu Tyr Glu Cys Ala Pro Met Ser Phe Ile Ala Glu Gln Ala Gly Gly
        355                 360                 365
Lys Gly Ser Asp Gly His Gln Arg Val Leu Asp Ile Met Pro Thr Ala
    370                 375                 380
Val His Gln Arg Val Pro Leu Tyr Val Gly Ser Val Glu Glu Val Glu
385                 390                 395                 400
Lys Val Glu Lys Phe Leu Ser Ser Glu
                405
```

<210> 194
<211> 1242
<212> DNA
<213> Zea mays

<400> 194

```
atggccgccg ccgccaccac ctcctcatcc tcccacttgc tcctcctctc ccgccagcag      60
gcggcctccc tacgatgccg cctctccttc ctcggccagc ccgccagaag gtccggcagg     120
gtcacggccc aggcgccggc cgctaaggac gtgcggtgca tggcggccgt ggacactgcg     180
gcgtccgcgg cggcggcgga gacgagcccc aagtcgagca gctacgagat cgtgacgctc     240
acgacgtggc tgctgcagca ggagcggacc ggcgcgatcg acaacgagat gaccatcgtg     300
ctggccagca tatccacggc gtgcaagcag atcgccgcgc tggtgcagcg cgcgcccatc     360
tccaacctca cgggcgttca gggcgccgtc aacgtgcagg gcgaggacca gaagaagctc     420
gatgtcgtct ccaacgaggt gttctccaac tgcctcaagt cgagcgggcg caccggcgtg     480
atcgcctcgg aggaggagga cgtgcccgta gcggtggagc agagctactc cggcaactac     540
```

```
atcgtcgtgt tcgaccctct cgacggctcc tccaacatcg acgccgccgt ctccactggc    600
tccatcttcg gcatctacaa ccccaacgac gagtgcctcg ccgacgtcga cgacaacgac    660
acccttgatt cggtggagca gaggtgcatc gtgaacgtgt gccagccggg gagcaacctg    720
ctggccgccg gctactgcat gtactcgagc tcggtgatct tcgtgctcac cgtcggcacc    780
ggggtgtacg tgttcacgct ggaccccatg tacggcgagt tcgtgctgac gcaggagaag    840
gtgcagatcc ccaaggcggg caagatctac gccttcaacg agggcaacta cgcgctctgg    900
gacgacaagc tgaagctgta catggacagc ctcaaggagc ccggcgactc ggggaagccc    960
tactccgcgc ggtacatcgg cagcctcgtc ggcgacttcc accgcactct gctctacgga   1020
gggatctacg ggtaccccag ggacaagaag agcaagaacg gcaagctgcg gcttctctac   1080
gagtgcgccc ccatgagctt catcgtcgag caggccggtg gcaagggctc tgacggccac   1140
cagagaattc ttgacatcac acctacagag atccaccaaa gagtgcctct gtacattggg   1200
agcgtggagg aagtggacaa ggtggagaaa ttcctggctt ga                       1242
```

<210> 195
<211> 413
<212> PRT
<213> Zea mays

<400> 195

```
Met Ala Ala Ala Ala Thr Thr Ser Ser Ser Ser His Leu Leu Leu Leu
1               5                   10                  15
Ser Arg Gln Gln Ala Ala Ser Leu Arg Cys Arg Leu Ser Phe Leu Gly
            20                  25                  30
Gln Pro Ala Arg Arg Ser Gly Arg Val Thr Ala Gln Ala Pro Ala Ala
        35                  40                  45
Lys Asp Val Arg Cys Met Ala Ala Val Asp Thr Ala Ala Ser Ala Ala
    50                  55                  60
Ala Ala Glu Thr Ser Pro Lys Ser Ser Ser Tyr Glu Ile Val Thr Leu
65                  70                  75                  80
Thr Thr Trp Leu Leu Gln Gln Glu Arg Thr Gly Ala Ile Asp Asn Glu
                85                  90                  95
Met Thr Ile Val Leu Ala Ser Ile Ser Thr Ala Cys Lys Gln Ile Ala
            100                 105                 110
Ala Leu Val Gln Arg Ala Pro Ile Ser Asn Leu Thr Gly Val Gln Gly
        115                 120                 125
Ala Val Asn Val Gln Gly Glu Asp Gln Lys Lys Leu Asp Val Val Ser
    130                 135                 140
Asn Glu Val Phe Ser Asn Cys Leu Lys Ser Ser Gly Arg Thr Gly Val
145                 150                 155                 160
Ile Ala Ser Glu Glu Glu Asp Val Pro Val Ala Val Glu Gln Ser Tyr
                165                 170                 175
Ser Gly Asn Tyr Ile Val Val Phe Asp Pro Leu Asp Gly Ser Ser Asn
            180                 185                 190
Ile Asp Ala Ala Val Ser Thr Gly Ser Ile Phe Gly Ile Tyr Asn Pro
        195                 200                 205
Asn Asp Glu Cys Leu Ala Asp Val Asp Asp Asn Asp Thr Leu Asp Ser
    210                 215                 220
Val Glu Gln Arg Cys Ile Val Asn Val Cys Gln Pro Gly Ser Asn Leu
225                 230                 235                 240
Leu Ala Ala Gly Tyr Cys Met Tyr Ser Ser Ser Val Ile Phe Val Leu
                245                 250                 255
Thr Val Gly Thr Gly Val Tyr Val Phe Thr Leu Asp Pro Met Tyr Gly
            260                 265                 270
Glu Phe Val Leu Thr Gln Glu Lys Val Gln Ile Pro Lys Ala Gly Lys
        275                 280                 285
Ile Tyr Ala Phe Asn Glu Gly Asn Tyr Ala Leu Trp Asp Asp Lys Leu
    290                 295                 300
Lys Leu Tyr Met Asp Ser Leu Lys Glu Pro Gly Asp Ser Gly Lys Pro
305                 310                 315                 320
```

Tyr Ser Ala Arg Tyr Ile Gly Ser Leu Val Gly Asp Phe His Arg Thr
325 330 335
Leu Leu Tyr Gly Gly Ile Tyr Gly Tyr Pro Arg Asp Lys Lys Ser Lys
340 345 350
Asn Gly Lys Leu Arg Leu Leu Tyr Glu Cys Ala Pro Met Ser Phe Ile
355 360 365
Val Glu Gln Ala Gly Gly Lys Gly Ser Asp Gly His Gln Arg Ile Leu
370 375 380
Asp Ile Thr Pro Thr Glu Ile His Gln Arg Val Pro Leu Tyr Ile Gly
385 390 395 400
Ser Val Glu Glu Val Asp Lys Val Glu Lys Phe Leu Ala
405 410

<210> 196
<211> 1266
<212> DNA
<213> Physicomitrella patens

<400> 196
atggcgacca cacaagcgat tctctctgcc acccttgcca tagccccggc ttccagctgc 60
gagacttcgt cacggagccc ggcgtccacc aaaacttgtc tctcagtggc aggatcgtcg 120
ctgcatggct cagtggccgg actcggagct gggaaacaga ttgtgagcgt gcagaggaag 180
agcgttgccg tgagggccgc cgttgcagct gagactgccg ctcccaagca gcaggcgaag 240
agccagtatg acatcactac cctgacgacg tggttgctga agaaagagca ggcgggcgtc 300
atcgatggcg agctcaccat tgtgctctcc agcatcgccc tggcttgcaa gcaaattgcg 360
tctctggtgc agagggctgg catctccaac atgactgggt tgcaaggagc tgctaacatt 420
caaggggagg accagaagaa gctagacgtt atttcgaacg aggtgttctc aagctgtctg 480
cgctcaagcg gacggacagg catcatcgct tctgaggaag aagacacccc ggttgcagtg 540
gaggagagct actctggcaa ctacattgtg gtgttcgacc ctcttgacgg ctcttccaac 600
atcgatgctg ctgtttccac ggggtcaatc tggggaatct acaagcccaa cgaggaatgc 660
ctgaccaatc tcggagagga gccaactatt gatgagatcg ccgaaaactg cgtcgtcaat 720
gtctgtcaac cagggagcaa tttgttgtcc gccgggtact gcatgtactc gagctccgtc 780
atcctcgttc tctcagtcgg tgatggagtc tacggcttca ctctggaccc tctctacgga 840
gaattcgtcc tctcgcacga caacatccaa attccaaaat ctggtaagat ctattccatg 900
aatgaaggca actacgccct ctgggatgac aacctcaaga agtacgtcga cagcttgaag 960
gaccccggtc ccagcggcaa gccctactcc gctcgttaca tcggcagtct ggtgggcgac 1020
ttccacagga caatgctgta tggtggcatc tatggctacc ccagggattc caagagcaag 1080
aacgggaagt tgaggttgct ttacgagtgt gctcccatga gctacctcgc tgaacaagca 1140
ggtgggaagg gctccgacgg tcaccagagg attctggaca tccaacctga gcaggttcac 1200
caacgtgtgc cattgtacgt tggaagcacg gaggaggtgg agaagttgga gaagttctta 1260
gcttaa 1266

<210> 197
<211> 421
<212> PRT
<213> Physicomitrella patens

<400> 197
Met Ala Thr Thr Gln Ala Ile Leu Ser Ala Thr Leu Ala Ile Ala Pro
1 5 10 15
Ala Ser Ser Cys Glu Thr Ser Ser Arg Ser Pro Ala Ser Thr Lys Thr
20 25 30
Cys Leu Ser Val Ala Gly Ser Ser Leu His Gly Ser Val Ala Gly Leu
35 40 45
Gly Ala Gly Lys Gln Ile Val Ser Val Gln Arg Lys Ser Val Ala Val
50 55 60
Arg Ala Ala Val Ala Ala Glu Thr Ala Ala Pro Lys Gln Gln Ala Lys
65 70 75 80
Ser Gln Tyr Asp Ile Thr Thr Leu Thr Thr Trp Leu Leu Lys Lys Glu

```
                85                  90                  95
Gln Ala Gly Val Ile Asp Gly Glu Leu Thr Ile Val Leu Ser Ser Ile
            100                 105                 110
Ala Leu Ala Cys Lys Gln Ile Ala Ser Leu Val Gln Arg Ala Gly Ile
        115                 120                 125
Ser Asn Met Thr Gly Leu Gln Gly Ala Ala Asn Ile Gln Gly Glu Asp
    130                 135                 140
Gln Lys Lys Leu Asp Val Ile Ser Asn Glu Val Phe Ser Ser Cys Leu
145                 150                 155                 160
Arg Ser Ser Gly Arg Thr Gly Ile Ile Ala Ser Glu Glu Glu Asp Thr
                165                 170                 175
Pro Val Ala Val Glu Glu Ser Tyr Ser Gly Asn Tyr Ile Val Val Phe
            180                 185                 190
Asp Pro Leu Asp Gly Ser Ser Asn Ile Asp Ala Ala Val Ser Thr Gly
        195                 200                 205
Ser Ile Trp Gly Ile Tyr Lys Pro Asn Glu Glu Cys Leu Thr Asn Leu
    210                 215                 220
Gly Glu Glu Pro Thr Ile Asp Glu Ile Ala Glu Asn Cys Val Val Asn
225                 230                 235                 240
Val Cys Gln Pro Gly Ser Asn Leu Leu Ser Ala Gly Tyr Cys Met Tyr
                245                 250                 255
Ser Ser Ser Val Ile Leu Val Leu Ser Val Gly Asp Gly Val Tyr Gly
            260                 265                 270
Phe Thr Leu Asp Pro Leu Tyr Gly Glu Phe Val Leu Ser His Asp Asn
        275                 280                 285
Ile Gln Ile Pro Lys Ser Gly Lys Ile Tyr Ser Met Asn Glu Gly Asn
    290                 295                 300
Tyr Ala Leu Trp Asp Asp Asn Leu Lys Lys Tyr Val Asp Ser Leu Lys
305                 310                 315                 320
Asp Pro Gly Pro Ser Gly Lys Pro Tyr Ser Ala Arg Tyr Ile Gly Ser
                325                 330                 335
Leu Val Gly Asp Phe His Arg Thr Met Leu Tyr Gly Gly Ile Tyr Gly
            340                 345                 350
Tyr Pro Arg Asp Ser Lys Ser Lys Asn Gly Lys Leu Arg Leu Leu Tyr
        355                 360                 365
Glu Cys Ala Pro Met Ser Tyr Leu Ala Glu Gln Ala Gly Gly Lys Gly
    370                 375                 380
Ser Asp Gly His Gln Arg Ile Leu Asp Ile Gln Pro Glu Gln Val His
385                 390                 395                 400
Gln Arg Val Pro Leu Tyr Val Gly Ser Thr Glu Glu Val Glu Lys Leu
                405                 410                 415
Glu Lys Phe Leu Ala
            420
```

<210> 198
<211> 1263
<212> DNA
<213> Galderia sulphuraria

<400> 198

```
ggcacgagga aagatgtgca agtatacttt attcaagtcc tactatatct ttacaaagaa     60
gagtgtcaag aaataaccac tgcaaaagat atcttcctta taagatggat atccatcaaa    120
atgatagctg ttcaacccca acccaaaaag actcccgtag cagaatattt acaaacctca    180
caagacacac caacttcact cactcgttac ttgttggaag tagccaaaca aaataaggat    240
atgggagata tggtggcatt gataaacggt attcaatttg cttgcaaaaa gatagcttca    300
ttggttggta aagcaggggt cactgacttg atgggaatct atcaacaagg catagtcaac    360
gttcacggag aagaacagaa aaagttggat gttctttcta atgaagtatt gaagaacgct    420
ctcaaatatt cgggaaaaat ggctgtcata gcttcggaag aagaagacgt tcctatcatg    480
gtagaagaaa gttattccgg taactatgta gtcgtgtttg atccattaga tggttcttcc    540
```

```
aatttggatg ctggattgcc tactggaact atatttggag tgtttcaaca acaattctcg     600
tgtctcattc atgactatga ggagtccatc gataacatgg aattggcttg tttacaaaac     660
actttacaac caggacgtag attgattgcc gctggatatt gtatctattc ttcttctacc     720
atgttggtac tctcattggg taatggtctt cattgtttta ctttggatac ggaagttggt     780
gaatttgtat tgactcgtgc taacatccaa attccacaaa gaggtaatat ttattctttc     840
aacgagtcca acttttatca atgggataaa ggagttcaag attatataga gaggttaaaa     900
aaaggaaaca atcaaacaaa ttgtcgttat tccgcaagat atgttggctc catggttgct     960
gatgtacatc gtacaatatt gtatggcgga atatttggtt atccagcaga taaaaagaat    1020
gtctctggta aattgagatt ggtatatgaa tgtgcaccga tggcatattt ggttgaacaa    1080
gcaggaggta aagcaaccac gggaatagaa aatattttag atttgacacc aaaagatatt    1140
cacgaaagga agcctcttat attaggttct ccagcagata tcgaagaatt tctgcaagta    1200
tatggaatgt cacgagttga tagagaagat atgcatatgt gggataactt gagtagttta    1260
taa                                                                  1263
```

```
<210>  199
<211>  420
<212>  PRT
<213>  Galderia sulphuraria

<400>  199
Gly Thr Arg Lys Asp Val Gln Val Tyr Phe Ile Gln Val Leu Leu Tyr
1               5                   10                  15
Leu Tyr Lys Glu Glu Cys Gln Glu Ile Thr Thr Ala Lys Asp Ile Phe
            20                  25                  30
Leu Ile Arg Trp Ile Ser Ile Lys Met Ile Ala Val Gln Pro Gln Pro
        35                  40                  45
Lys Lys Thr Pro Val Ala Glu Tyr Leu Gln Thr Ser Gln Asp Thr Pro
    50                  55                  60
Thr Ser Leu Thr Arg Tyr Leu Leu Glu Val Ala Lys Gln Asn Lys Asp
65                  70                  75                  80
Met Gly Asp Met Val Ala Leu Ile Asn Gly Ile Gln Phe Ala Cys Lys
                85                  90                  95
Lys Ile Ala Ser Leu Val Gly Lys Ala Gly Val Thr Asp Leu Met Gly
            100                 105                 110
Ile Tyr Gln Gln Gly Ile Val Asn Val His Gly Glu Glu Gln Lys Lys
        115                 120                 125
Leu Asp Val Leu Ser Asn Glu Val Leu Lys Asn Ala Leu Lys Tyr Ser
    130                 135                 140
Gly Lys Met Ala Val Ile Ala Ser Glu Glu Glu Asp Val Pro Ile Met
145                 150                 155                 160
Val Glu Glu Ser Tyr Ser Gly Asn Tyr Val Val Val Phe Asp Pro Leu
                165                 170                 175
Asp Gly Ser Ser Asn Leu Asp Ala Gly Leu Pro Thr Gly Thr Ile Phe
            180                 185                 190
Gly Val Phe Gln Gln Gln Phe Ser Cys Leu Ile His Asp Tyr Glu Glu
        195                 200                 205
Ser Ile Asp Asn Met Glu Leu Ala Cys Leu Gln Asn Thr Leu Gln Pro
    210                 215                 220
Gly Arg Arg Leu Ile Ala Ala Gly Tyr Cys Ile Tyr Ser Ser Ser Thr
225                 230                 235                 240
Met Leu Val Leu Ser Leu Gly Asn Gly Leu His Cys Phe Thr Leu Asp
                245                 250                 255
Thr Glu Val Gly Glu Phe Val Leu Thr Arg Ala Asn Ile Gln Ile Pro
            260                 265                 270
Gln Arg Gly Asn Ile Tyr Ser Phe Asn Glu Ser Asn Phe Tyr Gln Trp
        275                 280                 285
Asp Lys Gly Val Gln Asp Tyr Ile Glu Arg Leu Lys Lys Gly Asn Asn
    290                 295                 300
Gln Thr Asn Cys Arg Tyr Ser Ala Arg Tyr Val Gly Ser Met Val Ala
```

```
305                 310                 315                 320
Asp Val His Arg Thr Ile Leu Tyr Gly Gly Ile Phe Gly Tyr Pro Ala
                325                 330                 335
Asp Lys Lys Asn Val Ser Gly Lys Leu Arg Leu Val Tyr Glu Cys Ala
            340                 345                 350
Pro Met Ala Tyr Leu Val Glu Gln Ala Gly Gly Lys Ala Thr Thr Gly
        355                 360                 365
Ile Glu Asn Ile Leu Asp Leu Thr Pro Lys Asp Ile His Glu Arg Lys
    370                 375                 380
Pro Leu Ile Leu Gly Ser Pro Ala Asp Ile Glu Glu Phe Leu Gln Val
385                 390                 395                 400
Tyr Gly Met Ser Arg Val Asp Arg Glu Asp Met His Met Trp Asp Asn
                405                 410                 415
Leu Ser Ser Leu
            420
```

<210> 200
<211> 710
<212> DNA
<213> Marchantia polymorpha

<400> 200

```
gaggaggata ccccagtcgc cgtcgaagag agctactcag gaaactacgt cgttgtcttc      60
gatcctctcg atggatcctc caacatcgac gctgcggtat ctaccggatc catctttgga     120
atctacaggc ccactgagga gtgtcttgca gacatggacg acgactcaca gctcggtatg     180
gtggaacaaa actgcatcgt gaacgtatgc cagcccggta gcaacctcct atccgctggg     240
tactgcatgt actccagctc cgtcattttg gtgttgtcag tcggagacgg tgtctatgga     300
ttcacactgg atcccctgta cggtgaattc gtcatgaccc acgacaacat caagatccca     360
aagaagggat cgatctactc gttcaatgaa ggtaactacg cactctggga tgacaagctc     420
aagaagtaca tcgactcact gaaggacccc gaacccaccg gcaagcctta ctctgctcgt     480
tacatcggta gtctggtcgg tgacttccac agaaccatgc tctatggtgg catctacgga     540
taccctgccg acaagaaaag caagaacgga aagttgagac ttctgtacga gtgtgctcct     600
atgagctact tggcagagca ggccggagga aagggttctg atggttaccg cagagttctg     660
gaaatcgagc ctgagcaggt acatcagcga gtgccacttt tcgtcggaag                710
```

<210> 201
<211> 236
<212> PRT
<213> Marchantia polymorpha

<400> 201

```
Glu Glu Asp Thr Pro Val Ala Val Glu Glu Ser Tyr Ser Gly Asn Tyr
1               5                   10                  15
Val Val Val Phe Asp Pro Leu Asp Gly Ser Ser Asn Ile Asp Ala Ala
            20                  25                  30
Val Ser Thr Gly Ser Ile Phe Gly Ile Tyr Arg Pro Thr Glu Glu Cys
        35                  40                  45
Leu Ala Asp Met Asp Asp Asp Ser Gln Leu Gly Met Val Glu Gln Asn
    50                  55                  60
Cys Ile Val Asn Val Cys Gln Pro Gly Ser Asn Leu Leu Ser Ala Gly
65                  70                  75                  80
Tyr Cys Met Tyr Ser Ser Ser Val Ile Leu Val Leu Ser Val Gly Asp
                85                  90                  95
Gly Val Tyr Gly Phe Thr Leu Asp Pro Leu Tyr Gly Glu Phe Val Met
            100                 105                 110
Thr His Asp Asn Ile Lys Ile Pro Lys Lys Gly Ser Ile Tyr Ser Phe
        115                 120                 125
Asn Glu Gly Asn Tyr Ala Leu Trp Asp Asp Lys Leu Lys Lys Tyr Ile
    130                 135                 140
```

```
Asp Ser Leu Lys Asp Pro Glu Pro Thr Gly Lys Pro Tyr Ser Ala Arg
145                 150                 155                 160
Tyr Ile Gly Ser Leu Val Gly Asp Phe His Arg Thr Met Leu Tyr Gly
                165                 170                 175
Gly Ile Tyr Gly Tyr Pro Ala Asp Lys Lys Ser Lys Asn Gly Lys Leu
            180                 185                 190
Arg Leu Leu Tyr Glu Cys Ala Pro Met Ser Tyr Leu Ala Glu Gln Ala
        195                 200                 205
Gly Gly Lys Gly Ser Asp Gly Tyr Arg Arg Val Leu Glu Ile Glu Pro
    210                 215                 220
Glu Gln Val His Gln Arg Val Pro Leu Phe Val Gly
225                 230                 235

<210>  202
<211>  765
<212>  DNA
<213>  Tagetes patula

<400>  202
atcattgcat cggaggaaga agacgtgccg gtggctgtgg aagagagtta ctccggaaac     60
tacattgtcg tgtttgatcc ccttgatggg tcatctaata ttgatgctgc tgtttcaacc    120
ggttctattt tcggaatcta tagccccaat gatgagtgtc tcgctgatat tagtgacgac    180
tccacgctag acagtgtgga acaaaaatgt attgtcaacg tatgccagcc cggaagcaat    240
ctactagcag ccggttactg tatgtactca agctccgtaa tcttcgtgct ctcgatcggt    300
actggtgtct acgcgttcac attagaccca atgtacggtg agtttgtact cactcaagaa    360
aagattcaaa tcccgaaatc ggggaagatt tactcgttta acgaaggaaa ctatcaacta    420
tgggacgata aattgaagaa gtacatggat gatctaaagg acccgggccc cacgggcaag    480
ccgtattcgg ctcgctacat tggtagcttg gttggtgatt ttcataggac attattgtac    540
ggagggattt acgggtaccc acgtgacaaa aagagcaaga acgggaagct tcggttgttg    600
tatgagtgtg caccgatgag ttacttggtt gaacaagcgg gtggaaaggg gtcggatgga    660
catcaacgag tgctcgacat tcaaccaacc gagattcatc agcgcgttcc gctatacatt    720
gggagcgtag aggaagtgga aaaattggag aagtatttgg cttga                    765

<210>  203
<211>  254
<212>  PRT
<213>  Tagetes patula

<400>  203
Ile Ile Ala Ser Glu Glu Glu Asp Val Pro Val Ala Val Glu Glu Ser
1               5                   10                  15
Tyr Ser Gly Asn Tyr Ile Val Val Phe Asp Pro Leu Asp Gly Ser Ser
            20                  25                  30
Asn Ile Asp Ala Ala Val Ser Thr Gly Ser Ile Phe Gly Ile Tyr Ser
        35                  40                  45
Pro Asn Asp Glu Cys Leu Ala Asp Ile Ser Asp Asp Ser Thr Leu Asp
    50                  55                  60
Ser Val Glu Gln Lys Cys Ile Val Asn Val Cys Gln Pro Gly Ser Asn
65                  70                  75                  80
Leu Leu Ala Ala Gly Tyr Cys Met Tyr Ser Ser Ser Val Ile Phe Val
                85                  90                  95
Leu Ser Ile Gly Thr Gly Val Tyr Ala Phe Thr Leu Asp Pro Met Tyr
            100                 105                 110
Gly Glu Phe Val Leu Thr Gln Glu Lys Ile Gln Ile Pro Lys Ser Gly
        115                 120                 125
Lys Ile Tyr Ser Phe Asn Glu Gly Asn Tyr Gln Leu Trp Asp Asp Lys
    130                 135                 140
Leu Lys Lys Tyr Met Asp Asp Leu Lys Asp Pro Gly Pro Thr Gly Lys
145                 150                 155                 160
```

```
Pro Tyr Ser Ala Arg Tyr Ile Gly Ser Leu Val Gly Asp Phe His Arg
                165                 170                 175
Thr Leu Leu Tyr Gly Gly Ile Tyr Gly Tyr Pro Arg Asp Lys Lys Ser
            180                 185                 190
Lys Asn Gly Lys Leu Arg Leu Leu Tyr Glu Cys Ala Pro Met Ser Tyr
        195                 200                 205
Leu Val Glu Gln Ala Gly Gly Lys Gly Ser Asp Gly His Gln Arg Val
    210                 215                 220
Leu Asp Ile Gln Pro Thr Glu Ile His Gln Arg Val Pro Leu Tyr Ile
225                 230                 235                 240
Gly Ser Val Glu Glu Val Glu Lys Leu Glu Lys Tyr Leu Ala
                245                 250
```

<210> 204
<211> 795
<212> DNA
<213> Linum usitatissimum

<220>
<221> misc_feature
<222> (738)..(738)
<223> n is a, c, g, or t

<400> 204

```
gaggaggaac aacgtcgccg gggttctgg tttcaaatgc tctgccgtcg ggacgcgccg      60
tcggaggcgg caacgacgac gaagaagagg agtagctacg agattctgac gctgacgacc    120
tggcttctgc agcaggaaca ggccggagtg atcgacgccg agctcacgat tgtgctctcc    180
agcatctcca cggcgtgtaa gcagatcgct tctctagttc agcgatccgg gatttctaac    240
ctcaccggcg tccagggcgc cgtcaatgtc cagggtgagg atcagaagaa gctcgacgtc    300
gtttcgaacg aggtgttttc aaattgcttg aggtcgagcg gcaggacggg gatcatagcg    360
tcggaggaag aagacgtgcc ggtcgccgtg gaggaaagct actccggtaa ctatatcgta    420
gtgttcgatc cacttgatgg atcgtcaaac atcgatgccg cagtctccac cggctcaatc    480
ttcggaatct acagtcccaa cgatgagtgc ctggccgaca tcggagacgg agacgagtca    540
aatctagata ctcaggagca gaagtgtgtg gtgagcgtgt gccagccggg gagcaaccta    600
ctcgccgccg gctactgcat gtactcaagc tcggtgatct tcgtcctcac gatcggaaac    660
ggcgttttcg ccttcaatct ggacccaatg tacggcgagt tggtgttgac tcaagagaac    720
attcagatcc cgaaagcngg aaagatctac tcattcaacg aaggggacta ccaaatgtgg    780
gatgagaaat tgaag                                                      795
```

<210> 205
<211> 265
<212> PRT
<213> Linum usitatissimum

<400> 205

```
Glu Glu Glu Gln Arg Arg Arg Gly Phe Trp Phe Gln Met Leu Cys Arg
1               5                   10                  15
Arg Asp Ala Pro Ser Glu Ala Ala Thr Thr Thr Lys Lys Arg Ser Ser
            20                  25                  30
Tyr Glu Ile Leu Thr Leu Thr Thr Trp Leu Leu Gln Gln Glu Gln Ala
        35                  40                  45
Gly Val Ile Asp Ala Glu Leu Thr Ile Val Leu Ser Ser Ile Ser Thr
    50                  55                  60
Ala Cys Lys Gln Ile Ala Ser Leu Val Gln Arg Ser Gly Ile Ser Asn
65                  70                  75                  80
Leu Thr Gly Val Gln Gly Ala Val Asn Val Gln Gly Glu Asp Gln Lys
                85                  90                  95
Lys Leu Asp Val Val Ser Asn Glu Val Phe Ser Asn Cys Leu Arg Ser
            100                 105                 110
```

```
Ser Gly Arg Thr Gly Ile Ile Ala Ser Glu Glu Glu Asp Val Pro Val
        115                 120                 125
Ala Val Glu Glu Ser Tyr Ser Gly Asn Tyr Ile Val Val Phe Asp Pro
    130                 135                 140
Leu Asp Gly Ser Ser Asn Ile Asp Ala Ala Val Ser Thr Gly Ser Ile
145                 150                 155                 160
Phe Gly Ile Tyr Ser Pro Asn Asp Glu Cys Leu Ala Asp Ile Gly Asp
                165                 170                 175
Gly Asp Glu Ser Asn Leu Asp Thr Gln Glu Gln Lys Cys Val Val Ser
            180                 185                 190
Val Cys Gln Pro Gly Ser Asn Leu Leu Ala Ala Gly Tyr Cys Met Tyr
        195                 200                 205
Ser Ser Ser Val Ile Phe Val Leu Thr Ile Gly Asn Gly Val Phe Ala
    210                 215                 220
Phe Asn Leu Asp Pro Met Tyr Gly Glu Leu Val Leu Thr Gln Glu Asn
225                 230                 235                 240
Ile Gln Ile Pro Lys Ala Gly Lys Ile Tyr Ser Phe Asn Glu Gly Asp
                245                 250                 255
Tyr Gln Met Trp Asp Glu Lys Leu Lys
            260                 265
```

<210> 206
<211> 50
<212> DNA
<213> Artificial sequence

<220>
<223> primer: prm08448

<400> 206

```
ggggacaagt ttgtacaaaa aagcaggctt aaacaatggc cgccaccatg              50
```

<210> 207
<211> 50
<212> DNA
<213> Artificial sequence

<220>
<223> primer: prm08449

<400> 207

```
ggggaccact ttgtacaaga aagctgggta gctgcttagt gcttcttggt              .50
```

<210> 208
<211> 1167
<212> DNA
<213> Oryza sativa

<400> 208

```
atccaagcca agaagaggga gagcaccaag gacacgcgac tagcagaagc cgagcgaccg    60
ccttcttcga tccatatctt ccggtcgagt tcttggtcga tctcttccct cctccacctc   120
ctcctcacag ggtatgtgcc cttcggttgt tcttggattt attgttctag gttgtgtagt   180
acgggcgttg atgttaggaa aggggatctg tatctgtgat gattcctgtt cttggatttg   240
ggatagaggg gttcttgatg ttgcatgtta tcggttcggt ttgattagta gtatggtttt   300
caatcgtctg gagagctcta tggaaatgaa atggtttagg gtacggaatc ttgcgatttt   360
gtgagtacct tttgtttgag gtaaaatcag agcaccggtg attttgcttg gtgtaataaa   420
agtacggttg tttggtcctc gattctggta gtgatgcttc tcgatttgac gaagctatcc   480
tttgtttatt ccctattgaa caaaaataat ccaactttga agacggtccc gttgatgaga   540
ttgaatgatt gattcttaag cctgtccaaa atttcgcagc tggcttgttt agatacagta   600
```

```
gtccccatca cgaaattcat ggaaacagtt ataatcctca ggaacagggg attccctgtt    660
cttccgattt gctttagtcc cagaattttt tttcccaaat atcttaaaaa gtcactttct    720
ggttcagttc aatgaattga ttgctacaaa taatgctttt atagcgttat cctagctgta    780
gttcagttaa taggtaatac ccctatagtt tagtcaggag aagaacttat ccgatttctg    840
atctccattt ttaattatat gaaatgaact gtagcataag cagtattcat ttggattatt    900
tttttatta gctctcaccc cttcattatt ctgagctgaa agtctggcat gaactgtcct    960
caattttgtt ttcaaattca catcgattat ctatgcatta tcctcttgta tctacctgta   1020
gaagtttctt tttggttatt ccttgactgc ttgattacag aaagaaattt atgaagctgt   1080
aatcgggata gttatactgc ttgttcttat gattcatttc ctttgtgcag ttcttggtgt   1140
agcttgccac tttcaccagc aaagttc                                       1167
```

<210> 209
<211> 1629
<212> DNA
<213> Oryza sativa

<400> 209

```
atgatgggtt gcttcactgt cctgagatcc aagaagaaga agcctcttgc tctcaccaag     60
aaatcggttg atgcaaggga aagcacgtcc tcaagactcc cagagccaga agcgcatgtg    120
ccatcgttac aatctgctcc tcctagtttt aggaacaagg ctaaaatcca ccaatcggaa    180
aagaaagctt cttacagcag agcgcgcgtg ctgtctgctc cttccagcct aattgtggtt    240
gatcaggatg gtcttccata tgccgaattc gatgatcaag atgactccag gggcaaggga    300
ggttctataa aggggccaccg tttctctaat ccactgcccc ttcctctccc atcaccagaa    360
ggaaaatcat tgaggaactt tggcagcttc aaagccatca atgcaagtgg accactcgat    420
gcttcaggcc ctctgccact tcctccaaag aagtgtgatg ggcttaagaa tttctcctat    480
gaggaacttt catcagcttg ccaatggttt tctggtgacc agtgtgtttc cgaaagtttg    540
acatcaacat catacaaggc gtcctttagg gatgatttta ccgacccaaa gaccattgaa    600
gcaatagtat ctcggttgct ctcctccact cagagtttga aagagtttaa aacacaagtg    660
aataccttgg catcacttca gcatcccaac ttatgtaaac taatcggctt tcacgcaaga    720
gaagaatcta atgaaaggat gttggtctat gagcgactcc atcatggcag cttagataaa    780
ctactctttg gaagatcgga tggtcgtttc atggactggt cagcacgttt gaaggttgct    840
cttggtgctg ctagaggcct ggctttccta catgatgaag ggcctttttca ggccatgtac    900
aatgacttct caacctcaaa catccaaatt gacaaagatt tcactgcaaa gctatcagga    960
tatggatgtg ttggattcaa taccgaggag gaaatatcaa atgcatctgt ggctgctgca   1020
aacctctcag tggaaacctt ggagaaaggt gtactgactc ccaagagcaa cgtatggtgc   1080
tttggagttg tcctgctgga gctaataaca ggaaggaaga accttgatgt ccgttcctca   1140
aaagaagaac gcaatattgt caagtggagt aggcctttcc tcaccgatga tagtcgccta   1200
tcgttaatca tggactcccg tataaaagga cgcttcccta ccaaggctgc tcggattgta   1260
gcagatatca tattgagatg ccttaataaa gatccatcag agaggcctac catgagggcc   1320
gttgtggagt ccctagcaag cgtccaggac ataaaggttc catgtcgata tcctttgcaa   1380
gagccatctg ctgccccaag aaaggtgatg ttaaaatcta caagtctcaa tggcatcatt   1440
catcaccatc ctgtcgtaac cttctcaccg tcacctcctt cgcgaaacca acatttgctc   1500
tcgccaaggt catccacgtc tgcactgctt cctccaagga ccagctgtgc tctggatgac   1560
cctagagtaa gctctatcaa gaaatcgcct tcccctattt tacggagatc tggtgttgag   1620
ggtttttga                                                           1629
```

<210> 210
<211> 542
<212> PRT
<213> Oryza sativa

<400> 210

```
Met Met Gly Cys Phe Thr Val Leu Arg Ser Lys Lys Lys Lys Pro Leu
1               5                   10                  15
Ala Leu Thr Lys Lys Ser Val Asp Ala Arg Glu Ser Thr Ser Ser Arg
            20                  25                  30
Leu Pro Glu Pro Glu Ala His Val Pro Ser Leu Gln Ser Ala Pro Pro
        35                  40                  45
Ser Phe Arg Asn Lys Ala Lys Ile His Gln Ser Glu Lys Lys Ala Ser
```

50 55 60
Tyr Ser Arg Ala Arg Val Leu Ser Ala Pro Ser Ser Leu Ile Val Val
65 70 75 80
Asp Gln Asp Gly Leu Pro Tyr Ala Glu Phe Asp Asp Gln Asp Asp Ser
85 90 95
Arg Gly Lys Gly Gly Ser Ile Lys Gly His Arg Phe Ser Asn Pro Leu
100 105 110
Pro Leu Pro Leu Pro Ser Pro Glu Gly Lys Ser Leu Arg Asn Phe Gly
115 120 125
Ser Phe Lys Ala Ile Asn Ala Ser Gly Pro Leu Asp Ala Ser Gly Pro
130 135 140
Leu Pro Leu Pro Pro Lys Lys Cys Asp Gly Leu Lys Asn Phe Ser Tyr
145 150 155 160
Glu Glu Leu Ser Ser Ala Cys Gln Trp Phe Ser Gly Asp Gln Cys Val
165 170 175
Ser Glu Ser Leu Thr Ser Thr Ser Tyr Lys Ala Ser Phe Arg Asp Asp
180 185 190
Phe Thr Asp Pro Lys Thr Ile Glu Ala Ile Val Ser Arg Leu Leu Ser
195 200 205
Ser Thr Gln Ser Leu Lys Glu Phe Lys Thr Gln Val Asn Thr Leu Ala
210 215 220
Ser Leu Gln His Pro Asn Leu Cys Lys Leu Ile Gly Phe His Ala Arg
225 230 235 240
Glu Glu Ser Asn Glu Arg Met Leu Val Tyr Glu Arg Leu His His Gly
245 250 255
Ser Leu Asp Lys Leu Leu Phe Gly Arg Ser Asp Gly Arg Phe Met Asp
260 265 270
Trp Ser Ala Arg Leu Lys Val Ala Leu Gly Ala Ala Arg Gly Leu Ala
275 280 285
Phe Leu His Asp Glu Gly Pro Phe Gln Ala Met Tyr Asn Asp Phe Ser
290 295 300
Thr Ser Asn Ile Gln Ile Asp Lys Asp Phe Thr Ala Lys Leu Ser Gly
305 310 315 320
Tyr Gly Cys Val Gly Phe Asn Thr Glu Glu Glu Ile Ser Asn Ala Ser
325 330 335
Val Ala Ala Ala Asn Leu Ser Val Glu Thr Leu Glu Lys Gly Val Leu
340 345 350
Thr Pro Lys Ser Asn Val Trp Cys Phe Gly Val Val Leu Leu Glu Leu
355 360 365
Ile Thr Gly Arg Lys Asn Leu Asp Val Arg Ser Ser Lys Glu Glu Arg
370 375 380
Asn Ile Val Lys Trp Ser Arg Pro Phe Leu Thr Asp Asp Ser Arg Leu
385 390 395 400
Ser Leu Ile Met Asp Ser Arg Ile Lys Gly Arg Phe Pro Thr Lys Ala
405 410 415
Ala Arg Ile Val Ala Asp Ile Ile Leu Arg Cys Leu Asn Lys Asp Pro
420 425 430
Ser Glu Arg Pro Thr Met Arg Ala Val Val Glu Ser Leu Ala Ser Val
435 440 445
Gln Asp Ile Lys Val Pro Cys Arg Tyr Pro Leu Gln Glu Pro Ser Ala
450 455 460
Ala Pro Arg Lys Val Met Leu Lys Ser Thr Ser Leu Asn Gly Ile Ile
465 470 475 480
His His His Pro Val Val Thr Phe Ser Pro Ser Pro Pro Ser Arg Asn
485 490 495
Gln His Leu Leu Ser Pro Arg Ser Ser Thr Ser Ala Leu Leu Pro Pro
500 505 510
Arg Thr Ser Cys Ala Leu Asp Asp Pro Arg Val Ser Ser Ile Lys Lys
515 520 525

```
Ser Pro Ser Pro Ile Leu Arg Arg Ser Gly Val Glu Gly Phe
    530                 535                 540

<210>  211
<211>  1674
<212>  DNA
<213>  Arabidopsis thaliana

<400>  211
atggttttgg ggtgtttccc tttgaaaagc aagaagaaac gtggctctgt ttctatgaag      60
cggttggatc ttgaagaaag caagccaact gctttacctg agccaccaaa gattccaagt     120
cgtaatttac aatcaggtcc tccgagtttc agaaatcgtg tgaagccaat tcaatcaaac     180
aacggtggaa acggagagat gagtagccga gcaagagtca tgtttgctcc gtcaagcatc     240
cacggtgcag cggaacggga tttgcttgct ggtgtttacc acgacgagca agatgaacaa     300
ccaagagatc cacgtacttc tactaaagaa tctagccctc aaccacttcc gttaccgtca     360
ccaagaactg gttcttcatt gaagaattgg ggaagcttta agtcgtttaa cggaagcagc     420
ggtcggttat catcatccgc agctgtatct ggacctttac ctttgccacc tagcgggtca     480
gttaggagct tttcatatga tgaagtaatg gctgcgtgta acgctttttc ttcagaccga     540
tgtgtcatgg aaggtctttc atctgttatg tacatggctt cctttggtga tgaggcttcg     600
acctcaggtt taaagaaggt tgacgcaact gttgtacgac ttcacgtaac tactcagagt     660
attagggagt tcattaatga agtcaacaca ttggcgtcgc tgcaacacca gaacctttgt     720
aagctggtag gctatcatgc tcgtgacggt tctgacacaa gaatgttggt gtacgagagg     780
cttgctctgg gcagcttgga ccgtttactg catgggagat cagatgggcc tcctcttgat     840
tggaacacta gaatgaagat tgcactatgt gcagctcagg gtctaacctt cttgcacgaa     900
gaaggccctt ttcaggcaat gtacaatgaa ttttcgacgg caaatatcca agtcgataaa     960
gatttcagcg ccaagctatc aggatacggt tgtgcaggcc atgcgcctga gacagagaca    1020
tctaatagtt cggcacttgc taatctctct gtcgagactc tagagagagg gcttttgacc    1080
ccgaagagca atgtgtggag ctatggaata gttcttcttg agatgttaac gggtcggaaa    1140
aatatggacg ggtcttaccc gaaagaagag aggaacttag tgaaatggag cagagctttt    1200
ctagcagatg attgcaggct ctcgcttata atggatcctc agcttaaagg tcggtttccg    1260
gcaaaagcgg cgaggagcat agcagatata gcacagaaat gtctgcaggt ggagccatca    1320
gagcgtccaa ccatgagaaa catcgtggat caactcaaga tcatacagga catgaagtac    1380
tcgtgtaggt tcccgttaag agaacccgca ccagtcgcgg caaggaaaca tatgggaaga    1440
tcaagcagtc tcaacacgat tatttggacc ccggcatcag tgccaccaag gtcaagtttt    1500
tcaccgtcac ctccaccacg acgaccgtct gtttcaccca caaggggacg gacgctcgtg    1560
tttcccccag tgtttccgcc gcgagcgtgt tcatctttgg aggaaatggc tcgggaagag    1620
gttcgaagat cgtcttcagc cagtggtagg agaactagcc tcgaagggtt ttga          1674

<210>  212
<211>  557
<212>  PRT
<213>  Arabidopsis thaliana

<400>  212
Met Val Leu Gly Cys Phe Pro Leu Lys Ser Lys Lys Lys Arg Gly Ser
1               5                   10                  15
Val Ser Met Lys Arg Leu Asp Leu Glu Glu Ser Lys Pro Thr Ala Leu
            20                  25                  30
Pro Glu Pro Pro Lys Ile Pro Ser Arg Asn Leu Gln Ser Gly Pro Pro
        35                  40                  45
Ser Phe Arg Asn Arg Val Lys Pro Ile Gln Ser Asn Asn Gly Gly Asn
    50                  55                  60
Gly Glu Met Ser Ser Arg Ala Arg Val Met Phe Ala Pro Ser Ser Ile
65                  70                  75                  80
His Gly Ala Ala Glu Arg Asp Leu Leu Ala Gly Val Tyr His Asp Glu
                85                  90                  95
Gln Asp Glu Gln Pro Arg Asp Pro Arg Thr Ser Thr Lys Glu Ser Ser
            100                 105                 110
Pro Gln Pro Leu Pro Leu Pro Ser Pro Arg Thr Gly Ser Ser Leu Lys
```

```
        115                 120                 125
Asn Trp Gly Ser Phe Lys Ser Phe Asn Gly Ser Ser Gly Arg Leu Ser
    130                 135                 140
Ser Ser Ala Ala Val Ser Gly Pro Leu Pro Leu Pro Pro Ser Gly Ser
145                 150                 155                 160
Val Arg Ser Phe Ser Tyr Asp Glu Val Met Ala Ala Cys Asn Ala Phe
                165                 170                 175
Ser Ser Asp Arg Cys Val Met Glu Gly Leu Ser Ser Val Met Tyr Met
            180                 185                 190
Ala Ser Phe Gly Asp Glu Ala Ser Thr Ser Gly Leu Lys Lys Val Asp
        195                 200                 205
Ala Thr Val Val Arg Leu His Val Thr Thr Gln Ser Ile Arg Glu Phe
    210                 215                 220
Ile Asn Glu Val Asn Thr Leu Ala Ser Leu Gln His Gln Asn Leu Cys
225                 230                 235                 240
Lys Leu Val Gly Tyr His Ala Arg Asp Gly Ser Asp Thr Arg Met Leu
                245                 250                 255
Val Tyr Glu Arg Leu Ala Leu Gly Ser Leu Asp Arg Leu Leu His Gly
            260                 265                 270
Arg Ser Asp Gly Pro Pro Leu Asp Trp Asn Thr Arg Met Lys Ile Ala
        275                 280                 285
Leu Cys Ala Ala Gln Gly Leu Thr Phe Leu His Glu Glu Gly Pro Phe
    290                 295                 300
Gln Ala Met Tyr Asn Glu Phe Ser Thr Ala Asn Ile Gln Val Asp Lys
305                 310                 315                 320
Asp Phe Ser Ala Lys Leu Ser Gly Tyr Gly Cys Ala Gly His Ala Pro
                325                 330                 335
Glu Thr Glu Thr Ser Asn Ser Ser Ala Leu Ala Asn Leu Ser Val Glu
            340                 345                 350
Thr Leu Glu Arg Gly Leu Leu Thr Pro Lys Ser Asn Val Trp Ser Tyr
        355                 360                 365
Gly Ile Val Leu Leu Glu Met Leu Thr Gly Arg Lys Asn Met Asp Gly
    370                 375                 380
Ser Tyr Pro Lys Glu Glu Arg Asn Leu Val Lys Trp Ser Arg Ala Phe
385                 390                 395                 400
Leu Ala Asp Asp Cys Arg Leu Ser Leu Ile Met Asp Pro Gln Leu Lys
                405                 410                 415
Gly Arg Phe Pro Ala Lys Ala Ala Arg Ser Ile Ala Asp Ile Ala Gln
            420                 425                 430
Lys Cys Leu Gln Val Glu Pro Ser Glu Arg Pro Thr Met Arg Asn Ile
        435                 440                 445
Val Asp Gln Leu Lys Ile Ile Gln Asp Met Lys Tyr Ser Cys Arg Phe
    450                 455                 460
Pro Leu Arg Glu Pro Ala Pro Val Ala Ala Arg Lys His Met Gly Arg
465                 470                 475                 480
Ser Ser Ser Leu Asn Thr Ile Ile Trp Thr Pro Ala Ser Val Pro Pro
                485                 490                 495
Arg Ser Ser Phe Ser Pro Ser Pro Pro Pro Arg Arg Pro Ser Val Ser
            500                 505                 510
Pro Thr Arg Gly Arg Thr Leu Val Phe Pro Pro Val Phe Pro Pro Arg
        515                 520                 525
Ala Cys Ser Ser Leu Glu Glu Met Ala Arg Glu Glu Val Arg Arg Ser
    530                 535                 540
Ser Ser Ala Ser Gly Arg Arg Thr Ser Leu Glu Gly Phe
545                 550                 555
```

<210> 213
<211> 2069
<212> DNA

<213> Sorghum bicolor

<400> 213
ctactggatt aaccccccgc gtgcgttccc tccctctgtc agtctgtctc tctcttggcg 60
tcgactgggc gaccgtcgca gccgccctaa gccaggtacc cagaccatct tcgggccctt 120
cgccggcgac gagcaccacc aggaattttc ccagctgtag caatgatggg ttgcttcact 180
gttctcagat ccaagaagaa gaaaatccct tttgataatc ctcttcttcc aagcaagaaa 240
tcggttgatg caagggaaag tacgtcgtct agacttccgg aaccagaagt tcatgtgcca 300
tctttgcaat cagctctctc ctagttttag gaacaggact aagatatccc agtcgtcaaa 360
taaagtttca aacagcagag ctcgcgtgtt gtctgctccg tcaaccctta ttgtggttga 420
tcagtttggc tttccatatg ctgaataccg agatcaagac gactccagag ataaggaagg 480
ttcaacaaag gggcatcgct tttcaaatcc tttgcccctt cctcttcctt caccggaagg 540
acattctttt aggaactctg gtagcttcaa agctagcaac gtaagtgggc cattggagat 600
gtccagccct ctcccattgc ctccaaagaa atgtgatgga cttagaatct ttcttacga 660
ggaggttttg tctgcttgcc aatggttttc aagtgatcag tgtgtttcag aagcacttgg 720
ttcaacatca tacaaggcaa catttaggga tgaatttatt gatacaaaga ccactgaagc 780
aacggtagct cgattactcc cctccactca gagtttgaag gagtttaaaa cacaagcaac 840
tacattggca ttgcttcagc atcccaattt atgtaaactg attggctatt atgcaaaaga 900
agattctaat gaaaggatgt tggtctatga acggcttcat catgggagct tagataagct 960
actctttgga agaccagatg gtcgtttcat ggactggtct aaacgtttga aggttgccct 1020
tggtgcggcc agaggtcttg ctttcttgca tgatgaagga ccctttcagg ccatgtacag 1080
cgagttctca actttgaaca tccaaataga taaagatttc actgctaagc tttcaggata 1140
tggttgtgtt ggcttcaata ctgaggagat atctaatgca cctgcgtctg cagcaaacct 1200
ttcggtggag accttggaga agggtttact cactcccaag agcaatgtct ggagctttgg 1260
agttgtgtta ctggagctaa taactggaag gaagaacctt gatgccaatt cttctaaaga 1320
agaacgcaat attgtcaggt ggagtaggcc tttccttact gatgatagtc gcctatctct 1380
gatcatggac tcccgtataa aagggcgctt tcctactaag gctgctcgga tagtagcaga 1440
catcattctg aaatgcttgc ataatgatcc atccgagagg ccgaccatga gggatgttgt 1500
ggaggctcta gcaagagtcc aggagataaa ggttccgtgc agatatcctt tgcaagaacc 1560
ttctgctgca ccaagaaaag taatgctaaa atctacatcc ctcaatggaa ttgtgcctca 1620
tcatcctgtc ataaccttct ctccatcgcc gccttcgcat aaccagcact tgatttcacc 1680
aaggtcatca acatctgcct tgtttcatcc aaggacatgc tcttctactc tggatgatcc 1740
cggtgtaagc tctataaaga aaacacctcc tattatgcgt aggtctagcg ttgaaggctt 1800
ttgattgtcc atattgttct tttatttctt tttctcggat ttatttgttt ctttgttagc 1860
ctagtgattt tagctgtgtt ctgtattgta agacaagtgg ccttatgtag tgcccttgag 1920
tctcgagtaa taactaagca gagcaggatg aattgtaaat agtatcaggt tgtagatacc 1980
tttttgtgct ctaattgggt ggaagcagcg tgctgagtct gagtaggcct tgtaacctca 2040
taaataaact cgtttcccag tttctgtcc 2069

<210> 214
<211> 1360
<212> DNA
<213> Saccharum officinarum

<400> 214
agaaacactt ggttcaacat catacaaggc aacatttagg gatgaattta ttgatacaaa 60
gaccactgaa gcaacggtag ctcgattact cccctccact cagagtttga aggagtttaa 120
aacacaagcg accacattgg catcacttca gcatcccaat ttgtgtaaac tgattggcta 180
ttatgcaaaa gaagattcta atgaaaggat gttggtctat gaacggcttc atcatgggag 240
cttagataag ctactctttg gaagaccaga tggtcgtttc atggactggt ctaaacgttt 300
gaaggtttcc cttggtgctg cccgaggtct agctttcttg catgatgaag gaccctttca 360
ggccatgtac agtgagttct caactttgaa catccaaata gataaagatt tcactgctaa 420
gctttcagga tatggttgtg ttggcttcaa tactgaggag atatctaatg cacctgcgtc 480
tgcagcaaac ctttcggtgg agaccttgga gaagggttta ctcactccca agagcaacgt 540
ctggagcttt ggagttgtgt tactggagct aataactgga aggaagaacc ttgatgccaa 600
ttcttccaaa gaagaacgca atattgtcag gtggagtagg cctttcctta ctgatgatag 660
tcgcctatct ctgatcatgg actcccgtat aaaagggcgc tttccaacta aggctgctcg 720
gatagtagca gacatcattc tgaaatgctt gcataaagat ccatcagaga ggccgaccat 780
gagggatgtt gtggaggccc tagcaagagt ccaggaaata aaggttccat gcagatatcc 840

```
tctgcaagaa ccttttgctg caccaagaaa aataatgtta aaatctacat ccctcaatgg     900
aattgtgcct catcatcctg tcataacctt ctccccttcg ccgccttcac ataaccaaca     960
cttgatttca ccaaggtcat caacatctgc cttgtttcat ccaaggacat gctcttctac    1020
tctggatgat cccggtgtaa gctctataaa gaaaacacct cctattatgc gtaggtctag    1080
cgtcgaaggc ttttgattgt ccatattgtt cttttatttc cttttcttgg atttattagt    1140
ttctttggta gcctagtgat tctagctatg ttctgtattg caagacaagc ggccttaatg    1200
tagtgccctt gggtctagag taataactaa gcagagcagg atgaattgta aataggatca    1260
ggttgtagat accttttgt gctctaattg ggtggaagca gcgtgctaag tgagccttgt    1320
aacctcccaa tgcaataaac tcgtttttca gtttttgttc                          1360
```

<210> 215
<211> 2062
<212> DNA
<213> Medicago truncatula

<400> 215

```
acgaggctat tctctctctt tctctctcta catttctcaa cattcttcaa atttctctct      60
ccaaaacctt cacttcgcag ctttttcaat ctgctttctt tgactctgta acagtttttt     120
tcgtggaaga atcagaacca caaaaaagtt tcgattttta cccatttctt caacccgtga     180
tcgcttcact gtaattggca tgagcttcca tttctcgttc tgacaaaaac aggtatctat     240
aacatcctag ttctgtacat agaatggggt gttttactat attgaagaga aagaagaaaa     300
agcctgatca gattgtgtat gtaaaacgcg taagccctgg cgaagattca cctacagtac     360
tgcccgaacc ccaaactcat acccgctcac tccagtctgc gcctcctagt tttaagatca     420
gagtgaaacc cattcaacct agcaataaag ccactaacaa tagaatacga gcattgtctg     480
ctccatcgag tcttgatgat gcagaacaag atgcattggc caccattgag tatgaagaac     540
aagaagggtc aaaataccga actggatcat ggaaggagca gcgttcgcct agtccacaac     600
cattaccgct tccatctcct aagggtggtg gtacattgaa gactgttgga agctttaagt     660
tggggatagc tagtagtcct ttatatgctt ctggaccttt gccgcttcca ccaactgggt     720
cactgagaaa cttttcttat gatgaacttg ctgctgcttg cctcaatttc tcttcagatc     780
gatacatgtc agaatctctt tcatccacca tgtataaagc ttcctttggt gatgatacct     840
caacttcaag ttcaaagaag tttgaagcta ctgtcacacg ccttcgccca tcatctcagg     900
gcctgaagga attcataaat gaggttaata ctcttgcatc attgcagcat ccgaacctct     960
gtagattgct aggatttcac gcaggtgatg gttcagagca tagaatgttg gtttatgaga    1020
ggctatacca tggaagcttg gaccgcttat tgtatgggag atctgatggg ccatcaattg    1080
attggaatac aagaatgaaa attgcaatat gtgctgcact aggtctttct ttcttgcatg    1140
aagaagggcc ttttcaggca atgtataatg aattttcaac agctaacatt cagattgaca    1200
aagatttcag tgcaaagctt tcaggatatg gttgtgttgg acatgttccg aaggaagaga    1260
tttcaagcag ttcatctgcc gttggaaacc tatcgatgga gacactggag aaaggaatgc    1320
ttactccgaa aagcaatgta tggagtttcg gaatttttcct tctagagcta cttacaggaa    1380
gaaagaatct cgatagcccg tcacccaaag gaagagagaa atttggtgaa gtggagcagg    1440
cctttcctgt ctgataatca tcgtttgtca atgatcatgg atcctcaact taaaggtcgc    1500
tttccttcta aagcggcgag tacaatagct aacattgcac aaagatgcct tcaaatggag    1560
ccatcagaac gaccaaccat gggaacagtt gttgagcagc tgaaaaagat acaagatttg    1620
aagcattcta gcagattccc gctgcaagaa cctgcacaaa tgtcgagatc accaagtctt    1680
aacggtatca accaccctgc accaaggccg agtttctctc catcaccatc atccagagcc    1740
ctggtatctg tctcacctcc aagatggtcg ggagtgtcaa ttcaacttcc acctcgcgcg    1800
ttttcttcaa ccctctattt ggaggagctt gataggcaag aaagccgcaa gtcagcttca    1860
gcctctagga aggctagtgt tgaaggattt tgattgtcga tagtgttcat tttttatttg    1920
ttattctttt tttggtgtag ttcaacagag atttatagga gataaagatt tgtaatcatc    1980
cctcagtgtg atgcagagag gatgctcttt tgtacatagt gcaaaggttg gtccccttgg    2040
ttcaattagt tactccattt tg                                             2062
```

<210> 216
<211> 2039
<212> DNA
<213> Zea mays

<400> 216

```
ctcccccggc gtgcgctccc tcctgtctct cttggcgacg actgggcaac ggtcgcagct      60
```

gtatgttcga gcccttcgcc gacgacgttc accaccagga attttctcag ctgtgccaat 120
gatgggttgc ttcactgttc tcagatccaa gaagaagaaa agccattttg ataatcctct 180
tgtcccaagc aagaaatcag ttgatgcaag ggaaagtacg tcctctagac ttccggagcc 240
agaagttcat gtgccatctt tgcaatcagc tcctcctagt tttaggaaca gggtcaagat 300
atccgagtca tcaaatgaag tttcaaacag aaacagcagg gctcgcgtgc tgtctgctcc 360
atcagccctt attgtggttg atcagtttgg ctttccatat tcggaatacc gagatcaaga 420
tgactccagg gataaggaag gtttgacaaa ggggcatcgc ttttcaaatc ctttgcccct 480
tcctcttcct tcacgcgaag gacattcttt taggaactct ggtagcttca aagccagcaa 540
cgtaagcggg ccattggaga tgtctggccc tctcccattg cctctaaaga aatgtgatgg 600
acttagaatc ttctcttatg aggagatttc atctgcctgc caacgatttt ctagtgatca 660
gtgtgtttca gaaacacttg gttcaacatc atacaaggca acatttagag atgaatttat 720
tgatacgagg accactgaag caacagtagc tcgattactc ccctccactc agagtttgaa 780
ggagtttaaa acgcaagcga ccacattggc atcgcttcag catcccaatt tatgtaaact 840
gattggctat tatgcaaaag aagattctaa tgaaaggatg ttggtctatg aacggcttta 900
tcatggaagc ttagataagc tactctttgg aagatcagat ggtcgtttca tggactggtc 960
taaacgtttg aaggttgccc tgggtgctgc cagaggtcta gctttcttgc atgatgaagg 1020
accctttcag gccatgtaca gcgagttctc aactttgaac atccaaatag ataaaggttt 1080
cactgctaag ctttcaggat atggttgtgt tggcttcaat accgaggaga tatctaatgc 1140
acctgtgtct gcagcaaacc tttcggtgga gaccttggag aagggtttac tcactcccaa 1200
gagcaacgtc tggagctttg gagtcgtgtt actggagcta ataactggaa ggaagaacct 1260
tgatcccaat tattccaaag aagaacgcaa tattgtcaac tggagtaggc ctttccttac 1320
tgatgacagt cgcttgtctc ttatcatgga ctcccgtata aaagggcgct ttcccactaa 1380
ggctgctcgg atagtagcag acatcatttt gaaatgcctg cgtaaggatc catcggagag 1440
gcctaccatg agggatgttg tggaggccct agcaagagtc caggaaataa aggttccgtg 1500
cagatatcct ctgcaagaac cttctgctgc accaagaaaa gtgatgttga aatctacatc 1560
cctcaatggg attgtgcctc accatcctgt cataaccttc tctccatcgc cgccttcaca 1620
taaccagcac ttgatttcac caaggtcgtc gacatctgcc ttgtttcatc caagggcatg 1680
ctcttctacc ctggacgatc cgagtgtaag ttctataaag aaaacaccac ctattatgcg 1740
aaggtctagc gtcgaaggct tttgattgtc catattgttc ctttatttcc ttttcttgga 1800
tttatttgtt tctttgttag tagtgtagcg gtttttagct gtgttctgta ttgtaagaca 1860
agtggcctca tatgtagtgc ccttgggtct ggagtaaagc agaacaggat gaattgtaaa 1920
taggaccagg ttgtagatac cttttttttg tgctcgaatt gggtggaagc agcgtgccga 1980
gtgggccttg taacctcata atgcaataaa ctccgtttcc cagtttctgt aaaaaaaaa 2039

<210> 217
<211> 1479
<212> DNA
<213> Hordeum vulgare

<400> 217
tgccgaattc ggcacgagaa agaggtccga ggggcttaag aacttctcgt acgatgagat 60
ttccactgct tgccagtggt tttctggcga tcatcgtgtc tcagaaactc tgacttcgac 120
atcatacaag gcattgttca gggatgattt cgttgaacca aagaagatgg aagcaatagt 180
agccaggtta ctcccttcca atcagagttt caaagaattt aaggcacaag taaatacgtt 240
ggcatcactt caacatccca atttatgtaa actcatcggc tatcacgcaa gagaagaatc 300
taatgaaagg atgttggtct atgagcggct ccatcatggc agtttagaca ggttactctt 360
tggaagaccg gaaggtcgtt tcatggactg gtctacacgt ttgaaggttg cccttggtgc 420
tgctaaaggt ctagctttcc tacacgatga aggacccttt caggcaatgt atgatgactt 480
ctcaacgtca aacatccaaa tagagaaaga tttcactgca aagctgtcgg gatatggttg 540
tgttggcttc aattctgacg aggaaagatc caaggcatct gtggctgcaa acctctcaga 600
ggaaaccttg gagaaaggcg tactgactcc gaagagcaac gtatggagct ttggagttgt 660
cttgctcgag ctaataacag gacggaagaa cctcgatgta cgttccacca aagaagaacg 720
taatattgtc aagtggggta ggcctttcct caccgacgat agtcgtctat ccctcatcat 780
ggatccccgt ataaaaggac gctttcctac caaggctgct cgaactgtgg cagacataat 840
tctgaagtgc cttcaaagag atccatcaga aaggcctacg atgagggccg tcgtggaggc 900
cctaacaagc gttcaggaca taaaggtccc ctgccggtat cctcttcaag taccgtcccg 960
ccgcacccga ggaaagtgat gctaaagtct acgagcctca atggcattgt tcctcagcat 1020
cccgtcatga ccttctcgcc gtcgcctcct tcgcgcaacc agcacctggc gtcgccaaga 1080
tcatcgactt ccgcgcttct tcccccaagg acctgctctt ccatcatcac cctggattac 1140

```
cccagggtaa gctcggtcaa gaagtcgcct tccggtatcc tgcggagacc tggcgtcgag   1200
ggtttttgat tgtccataca tggtcggatt tcttctcttt gccttggatt tatttgttgt   1260
tttggttagc ctagcgagtg gtctcagtgc tatgtgatat gtatatgttg gaaagacatc   1320
tgaaaaaagg gcagagtgaa gtgtagatag tagaaccagc tttgtagata cttgtcgttc   1380
tctgattgga tgggggtcat ggaagcagtg tgtgttagag tggggcttgt aaccccatta   1440
ttatgcaata aacgtgggtt ggtcggtcgt atttgctcg                          1479

<210>  218
<211>  769
<212>  DNA
<213>  Glycine max

<400>  218
gtaaattgct aggatttcat gcacgagagg gttcagaaca tagaatgttg gtttatgaaa     60
ggctatacca tggaagcttg gatcgcttat tgtatgggag atctgatggg ccatcaattg    120
attggaatac aagaatgaaa attgctatat gtgctgcaca aggtctaacc ttcttgcacg    180
aagaggggcc ttttcaggct atgtataatg agttctcaac agccaacata cagattgaca    240
aagatttcag cgcaaagctt tcaggatatg gttgtgttgg acatattccc gaagaagaga    300
tttcaagcag ctcatctgct gttggaaacc tatcaatgga aacactggag aaaggaatgc    360
tcactccaaa gagcaacgta tggagttttg gaatttttct tctggagcta cttactggaa    420
gaaagaatct tgatagccgt caccccaagg aagagaggaa tttagtcaag tggagccggc    480
ctttcttagc cgataactac cgtctgtcgt tgatcatgga tcctcaactc aaaggccgct    540
ttccttctaa agcagcaaga acaatagctg atattgcaca aagatgcctt caaaaggagc    600
catcagacag acctaccatg aggactgttg ttgagcatct caagataata caggatttga    660
aatattcgtg ccggttccct ctgcaagaac cagcatcaaa ctctggaaaa catatgtcaa    720
gatcaccaag tctcaatggc atcatctgcc ctgcaccaag ggtgagttt                769

<210>  219
<211>  1150
<212>  DNA
<213>  Solanum tuberosum

<400>  219
ttcttagctc atcgaaacgt taaaccttac aatacttcaa acatcgatca atacaatttg     60
ctgaagcacg caatttttca aggactgtat aaacaacaaa gatgggttgt ttcacagttt    120
taaaaagtaa gaagaagaag tctgaacaga gtattcacat caaacgtgtg aatcctcagg    180
aacattctcc tactgcattg cccgagcctc aagtgcagac acggtcgttg cagtcggcac    240
ctccaagttt tagaactaga gtaaaacctg tacagtcgag taatagagtt acaagcagta    300
gggcgcgggc actctctgcc ccatctagcc tggactcagc agaacaagat gtagcatcaa    360
atgaatgtga ggaacatgat gagttcaaga gtcgtattgg ttcaatcaag gagtaccaat    420
caccaagtcc tcagcctctt cctcttccat ctccacagag tgccgctgcc actctcaaga    480
ctatgggaag ctttaaagtt ggcaacgcca gcggtccgtt aaatgcctct ggacccctgc    540
cactgcctcc tacactgcct tcaacattgc cttctactgg agcactcagg aacttctcat    600
ttgaagaact tgctgctgcc tgccaccgct tctctcctga acggtgtatg tcagaaggtc    660
tctcttctgt tatttacaga gcttcttttg gagatgatgc aactggtaca aagaagcttg    720
aagccactgt aacccggctt catccttctt cgcaggggtt gaaggaattt gtgactgagg    780
tgaacacact agcttctttg caacatccat cactttgtaa actgattggt ttccatgcca    840
cggggaaggt tccgagcaca gaatgttggt ttatgaaagg ctttttcatg gaagcttaga    900
ccggcttttg tttgggaggt ccgatggtcc cccgatagac tggaatgctc gaacgaaaat    960
tgctttatgt gctgctcaag gtctcacatt cctgcatgag gaaggacctt ttcaggcaat   1020
gttccatgaa ttttccactg caaatataca aattgataag gattgcagtg caaagctctc   1080
gggatatgga tgcattactc ctataccaga gacagacata tcatgcagtt cagctgccct   1140
ggcaatctct                                                          1150

<210>  220
<211>  910
<212>  DNA
<213>  Lycopersicon esculentum
```

<400> 220
catggaagct tacaccggct tttatttggg aggtcggatg gtccccccaat agactggaat 60
gctcgaataa aaattgcttt atgtgctgct caaggtctca cattcctgca tgaggaagga 120
ccttttcagg caatgttcca tgaattttcc actgcaaata tacaaattga taaggattgc 180
agtgcaaagc tctcgggata tggatgcatt actcatatac aagagacaga catatcatgc 240
agttcagctg ccctggcaaa tctctctgag gagactctgg agcgaggatt ggtaactcca 300
aagagtaatg tttggagttt tgggattgtt cttcttgagc tgctgactgg ccggaagaat 360
ttaagcagtc ggcatccaaa ggaagagagg aatttagtga agtggagcaa gctttttcta 420
gctgatgaca gtagattatc gctaatcatg gaccctcagt taaaaggtcg gttccctgcc 480
aaagcagcaa gaactgtggc tgatattgct caaagatgtc tgcaaaagga tccatctgaa 540
aggcccacca tgagaactgt agtggagcaa ctcaagactg tacaagttat gaagtaccct 600
tctcggtttc ctcttcaaga gccaagggca gttggtgtaa aacacatgtc aaagtgtccg 660
agcctaaatg gaattattac cccaacatca agattgagct tctccccttc accaccaacc 720
caccctatat ctatttctcc gacaaggaca gctgctccac tgctgtctct accttcatgt 780
tcctccatcc tctctatgga ggactttgat cgattggaaa atcgaaggcc atcatcttca 840
tctgttcgga ggtctagtgt cgaaggattt tgatcgattg tagagcactt ttttcttcaa 900
attgcttttc 910

<210> 221
<211> 681
<212> DNA
<213> Lycopersicon esculentum

<400> 221
gagaggcttt ttcatggaag cttagacaga cttttgttcg gaagatcaga tggcccctct 60
atagattgga atgcgagaac caaaattgcc ctatgtgctg cacaaggtct tacatttcta 120
catgaggagg gaccttttca ggcaatgttc caagaatttt caactggaaa tatacaaatc 180
gacaaggatt ttagtgcaaa gctctcgggg tatggatgca ttacgaatat acaagagacg 240
gagatatctt gcaattcaat cgccctggcg aatctctcac aggagacact ggagagaggg 300
ttgataactc ctaagagcaa tgtttggagt ttcgggattg ttcttttaga actgctcact 360
ggccggaaga atcttgatgg tcggtattca aaggaagaga ggaatctagt caagtggagt 420
aggcctttcc ttgctgatga tggtagatta tcgcttatca tggatcctca gcttaaagga 480
cggtttcccg caaaagcagc ccgtacagtg gctgatattg cccaaagatg cctgcaaaag 540
gatccatctg aaaggcccac catgagaact atcttggacc aactcaaatc cgtacaagtg 600
atgaagtgcc cttcacggtt tcctctgcaa gaaccagcgg ttgttggtgg taaacacatg 660
tcaaagtctc caagcatgaa t 681

<210> 222
<211> 741
<212> DNA
<213> Triticum aestivum

<400> 222
tttcctccca aggctgctag gactgtggca gacatcattc tgaagtgcct tcatagggat 60
ccatccgaaa ggcctacgat gagggccgtc gtggagtctc tagcaagcgt ccaggacata 120
aaggtcccct gccggtatcc tcttcaagta ccgtccgccg caccgaggaa agtgatgcta 180
aaatccacga gtctcaacgg cattgttcct cagcatcctg tcatgacctt ctcgccgtcg 240
cctccttcgc gcaaccagca cctggtgtca ccgagatcat cgacgtctgc gctgcttccc 300
ccaaggaact gctcttccac catcaccctg gactacccca gggtaagctc ggtcaagaaa 360
tcgcccccca acatcatgcg gagacctggc gtcgagggtt tttgattgtc catatagtgt 420
cggattttct tctctttgcc ttgatttatt tgttgttttg gttagcctag cgagtgatct 480
cagtgctatg ggatatacta tgttgcaagg acatgtgaaa aagggcagag tgaagcgtag 540
atagtagaac cagcttgtag atacttgtcg ttctctgatt ggatgggggt catggaagaa 600
gtgtgtgttg agtggggctt gtaaccccac tatgcaataa acgtgggttg gttggtcggt 660
cggtatttgc tcgccgtcaa acatttcagc tgtttctttc ttctcttgaa tgctaagttt 720
tgttgctgct agtcgtggaa t 741

<210> 223
<211> 2039

<212> DNA
<213> Zea mays

<400> 223
```
ctcccccggc gtgcgctccc tcctgtctct cttggcgacg actgggcaac ggtcgcagct      60
gtatgttcga gcccttcgcc gacgacgttc accaccagga attttctcag ctgtgccaat     120
gatgggttgc ttcactgttc tcagatccaa gaagaagaaa agccattttg ataatcctct     180
tgtcccaagc aagaaatcag ttgatgcaag ggaaagtacg tcctctagac ttccggagcc     240
agaagttcat gtgccatctt tgcaatcagc tcctcctagt tttaggaaca gggtcaagat     300
atccgagtca tcaaatgaag tttcaaacag aaacagcagg gctcgcgtgc tgtctgctcc     360
atcagccctt attgtggttg atcagtttgg ctttccatat tcggaatacc gagatcaaga     420
tgactccagg gataaggaag gtttgacaaa ggggcatcgc ttttcaaatc ctttgcccct     480
tcctcttcct tcacgcgaag gacattcttt taggaactct ggtagcttca aagccagcaa     540
cgtaagcggg ccattggaga tgtctggccc tctcccattg cctctaaaga aatgtgatgg     600
acttagaatc ttctcttatg aggagatttc atctgcctgc caacgatttt ctagtgatca     660
gtgtgtttca gaaacacttg gttcaacatc atacaaggca acatttagag atgaatttat     720
tgatacgagg accactgaag caacagtagc tcgattactc ccctccactc agagtttgaa     780
ggagtttaaa acgcaagcga ccacattggc atcgcttcag catcccaatt tatgtaaact     840
gattggctat tatgcaaaag aagattctaa tgaaaggatg ttggtctatg aacggcttta     900
tcatggaagc ttagataagc tactctttgg aagatcagat ggtcgtttca tggactggtc     960
taaacgtttg aaggttgccc tgggtgctgc cagaggtcta gctttcttgc atgatgaagg    1020
acccttttcag gccatgtaca gcgagttctc aactttgaac atccaaatag ataaaggttt    1080
cactgctaag ctttcaggat atggttgtgt tggcttcaat accgaggaga tatctaatgc    1140
acctgtgtct gcagcaaacc tttcggtgga gaccttggag aagggtttac tcactcccaa    1200
gagcaacgtc tggagctttg gagtcgtgtt actggagcta ataactggaa ggaagaacct    1260
tgatcccaat tattccaaag aagaacgcaa tattgtcaac tggagtaggc ctttccttac    1320
tgatgacagt cgcttgtctc ttatcatgga ctcccgtata aaagggcgct ttcccactaa    1380
ggctgctcgg atagtagcag acatcatttt gaaatgcctg cgtaaggatc catcggagag    1440
gcctaccatg agggatgttg tggaggccct agcaagagtc caggaaataa aggttccgtg    1500
cagatatcct ctgcaagaac cttctgctgc accaagaaaa gtgatgttga aatctacatc    1560
cctcaatggg attgtgcctc accatcctgt cataaccttc tctccatcgc cgccttcaca    1620
taaccagcac ttgatttcac caaggtcgtc gacatctgcc ttgtttcatc caagggcatg    1680
ctcttctacc ctggacgatc cgagtgtaag ttctataaag aaaacaccac ctattatgcg    1740
aaggtctagc gtcgaaggct tttgattgtc catattgttc ctttatttcc ttttcttgga    1800
tttatttgtt tctttgttag tagtgtagcg gtttttagct gtgttctgta ttgtaagaca    1860
agtggcctca tatgtagtgc ccttgggtct ggagtaaagc agaacaggat gaattgtaaa    1920
taggaccagg ttgtagatac cttttttttg tgctcgaatt gggtggaagc agcgtgccga    1980
gtgggccttg taacctcata atgcaataaa ctccgtttcc cagtttctgt aaaaaaaaa     2039
```

<210> 224
<211> 768
<212> DNA
<213> Lactuca sativa

<400> 224
```
accgactgat ggccgctagt cggggaagaa cacgaagaat ccaaaacccg tggcagcggc      60
ggaggcggag gcgggttgcc gcctgtcccg cagccgctgc cgcttcctgc accgcattca     120
cgccaacttc gaaaaccatc gcggaatgct tttaaagatt gagtggcggt gagttgggca     180
tgctgccagc ctcttaatac ttccggaccc tctgccactt ccgccatcgg gaaccaccca     240
tcttccgcca atgattccgg catctttacc gacatctgga acacttaaga acttcacata     300
ttgaagaaat cgcagctgct tgccacaaca ttttcaccct gacagatgcg tgtctgaagg     360
tctttcttct gttatgtata gagcttcttt tggagaagat acttctaatt taaagaatct     420
tcaagccact gttaccagtc tccatccttc aactcagggt ttgaaggaat ttgtgagtga     480
agtgaacacg ctagcatcat tgcaacaccc ttatctctgt aaatagattg gtttccatgc     540
gcgtgaggga tctgatagaa gaatgttggt ttacgagagg ctttttcatg gaagcttaga     600
tcggctttta tatggtacaa cagatggccc acctattgat tgcaatgcaa gaatgaaagt     660
cgcactctgt gctgctcaag ggcttacttt tttgcatgag gaaggaccat ttcaggcgat     720
gtttcatgaa ttttccactg ctaatataca aattgataaa gattttag                 768
```

<210> 225
<211> 1637
<212> DNA
<213> Gossypium spp.

<400> 225
cttttttcctc tcaaacctct ctcacactga taaacttttc tctccattac ctttctttga 60
cttgcaaagt tgttctcctc aaactaacca aacaagggat tctgggtttt cagctttctc 120
tgttttccta ctctctttcg cctttgcatg acctgcttac atagaaacat aaaagtccga 180
aatacaattt aggtggtttg tatataagat ggggtgtttt acagttttga gaagcaacaa 240
gaaaaagtcc aaacagtcag tttttgttaa acacattgct cataaagagc atattcctac 300
catgctgcct gagccccaaa ttcagacacg atcactgcaa tctgcacccc caagttttat 360
aaccagagta aaaccaattc aatctaataa caaggcaagc tgcaatagga cacgtgcatt 420
atctgctccg tcaagtcttg atgctgctga gcaagatgac cttgcgtcag ttgaatttga 480
agaacaagaa gagttgaaga gtcgtgttgg tttagtcaag gaacagaagt catcaagtcc 540
acagcctctt ccacttccat ctccccacag tactgcgctg aagacaatgg gaagttttaa 600
agcagggaat gttagtggcc ctctttttgc ttcgggacca ttacccctgc ctccctctgg 660
aacactacgt aacttcgcct acgaagaaat tgcagctgct tgccatcatt tctcttctga 720
tcgatgcaca tctgagggtc tatcttctgt tatgtacaag gcatccttcg gagatgacac 780
atcaagttca aagaagtttg aagctactgt tactcgcctt cacccatcca ctcagggttt 840
aagggaattt ataaacgaag taaacactct tgcatcattg caacatccaa atctctgtaa 900
attgcttggg taccatgcac gtgataattc agaacaacga atgttggtct atgagaggtt 960
atttcatgga agcttagacc ggcttttata cgggagatcg gatggaccgc cacttgattg 1020
gaatactcgc atgaaaattg ctttatgttc tgcacagggt cttactttct tgcatgagga 1080
aggaccattt caggcaatgt acaatgaatt ttcgactgcc aacatacaga tcgacaagga 1140
tttcagtgca aagctctcag gatatgggtg cgttggtcat atcccagaga cagaagagat 1200
ctccagtaat tcagttgctg tggcaaatat atccgtagag actctggaga gagggcgact 1260
aactccaaag agcaatgttt ggagttttgg gatcattcta cttgaattac tcactggccg 1320
gaagaacctt gacaaccgtt atcccaagga agagaggaac ctagtgaagt ggagccggcc 1380
attcctagcc gacaattgta gattgtcact catcatggat cctcagctca aaggtcgctt 1440
tcctatgaaa gctgcccgta cggtggctga cattgcacaa aggtgtctcc agatggaccc 1500
atcagagagg ccaaccatga gaaccatcgt tgagcatctc aaaatcatcc aagacctgaa 1560
atactcttgt cggtttcctc tgcaagatcc agcagcaatt gctggaaaac agatgtcaag 1620
gtcaccgagt ctcaacg 1637

<210> 226
<211> 2193
<212> DNA
<213> Oryza sativa

<400> 226
aatccgaaaa gtttctgcac cgttttcacc ccctaactaa caatataggg aacgtgtgct 60
aaatataaaa tgagacctta tatatgtagc gctgataact agaactatgc aagaaaaact 120
catccaccta ctttagtggc aatcgggcta aataaaaaag agtcgctaca ctagtttcgt 180
tttccttagt aattaagtgg gaaaatgaaa tcattattgc ttagaatata cgttcacatc 240
tctgtcatga agttaaatta ttcgaggtag ccataattgt catcaaactc ttcttgaata 300
aaaaaatctt tctagctgaa ctcaatgggt aaagagagag attttttttta aaaaaataga 360
atgaagatat tctgaacgta ttggcaaaga tttaaacata taattatata attttatagt 420
ttgtgcattc gtcatatcgc acatcattaa ggacatgtct tactccatcc caatttttat 480
ttagtaatta aagacaattg acttattttt attatttatc ttttttcgat tagatgcaag 540
gtacttacgc acacactttg tgctcatgtg catgtgtgag tgcacctcct caatacacgt 600
tcaactagca acacatctct aatatcactc gcctatttaa tacatttagg tagcaatatc 660
tgaattcaag cactccacca tcaccagacc acttttaata atatctaaaa tacaaaaaat 720
aattttacag aatagcatga aaagtatgaa acgaactatt taggtttttc acatacaaaa 780
aaaaaaagaa ttttgctcgt gcgcgagcgc caatctccca tattgggcac acaggcaaca 840
acagagtggc tgcccacaga acaacccaca aaaaacgatg atctaacgga ggacagcaag 900
tccgcaacaa ccttttaaca gcaggctttg cggccaggag agaggaggag aggcaaagaa 960
aaccaagcat cctcctcctc ccatctataa attcctcccc ccttttcccc tctctatata 1020
ggaggcatcc aagccaagaa gagggagagc accaaggaca cgcgactagc agaagccgag 1080

cgaccgcctt cttcgatcca tatcttccgg tcgagttctt ggtcgatctc ttccctcctc 1140
cacctcctcc tcacagggta tgtgcccttc ggttgttctt ggatttattg ttctaggttg 1200
tgtagtacgg gcgttgatgt taggaaaggg gatctgtatc tgtgatgatt cctgttcttg 1260
gatttgggat agaggggttc ttgatgttgc atgttatcgg ttcggtttga ttagtagtat 1320
ggttttcaat cgtctggaga gctctatgga aatgaaatgg tttagggtac ggaatcttgc 1380
gattttgtga gtaccttttg tttgaggtaa aatcagagca ccggtgattt tgcttggtgt 1440
aataaaagta cggttgtttg gtcctcgatt ctggtagtga tgcttctcga tttgacgaag 1500
ctatcctttg tttattccct attgaacaaa aataatccaa ctttgaagac ggtcccgttg 1560
atgagattga atgattgatt cttaagcctg tccaaaattt cgcagctggc ttgtttagat 1620
acagtagtcc ccatcacgaa attcatggaa acagttataa tcctcaggaa caggggattc 1680
cctgttcttc cgatttgctt tagtcccaga atttttttc ccaaatatct taaaaagtca 1740
ctttctggtt cagttcaatg aattgattgc tacaaataat gcttttatag cgttatccta 1800
gctgtagttc agttaatagg taatacccct atagtttagt caggagaaga acttatccga 1860
tttctgatct ccatttttaa ttatatgaaa tgaactgtag cataagcagt attcatttgg 1920
attatttttt ttattagctc tcaccccttc attattctga gctgaaagtc tggcatgaac 1980
tgtcctcaat tttgttttca aattcacatc gattatctat gcattatcct cttgtatcta 2040
cctgtagaag tttctttttg gttattcctt gactgcttga ttacagaaag aaatttatga 2100
agctgtaatc gggatagtta tactgcttgt tcttatgatt catttccttt gtgcagttct 2160
tggtgtagct tgccactttc accagcaaag ttc 2193

<210> 227
<211> 56
<212> DNA
<213> Artificial sequence

<220>
<223> primer: prm06729

<400> 227
ggggacaagt ttgtacaaaa aagcaggctt aaacaatgat gggttgcttc actgtc 56

<210> 228
<211> 50
<212> DNA
<213> Artificial sequence

<220>
<223> primer: prm06730

<400> 228
ggggaccact ttgtacaaga aagctgggta tggacaatca aaaaccctca 50

<210> 229
<211> 1176
<212> DNA
<213> Arabidopsis thaliana

<400> 229
cttcaactgc tgcgtctagc tgtccttcct tcttcatttg cttcttcttc tctagctcag 60
cgcggatcgc tgcagtagta ccctgacgta gcctttcttc ttcctttact ttctcatctt 120
ctatctctca aaagaaaagc agacaacttt atttgcaaaa acagagtttt tttttcttat 180
cttgagaaag ttcaacagaa gatgatgttc gagaaagacg atctgggtct aagcttaggc 240
ttgaattttc caaagaaaca gatcaatctc aaatcaaatc catctgtttc tgttactcct 300
tcttcttctt cttttggatt attcagaaga tcttcatgga acgagagttt tacttcttca 360
gttccaaact cagattcgtc acaaaaagaa acaagaactt tcatccgagg aatcgacgtg 420
aacagaccac cgtctacagc ggaatacggc gacgaagacg ctggagtatc ttcacctaac 480
agtacagtct caagctctac agggaaaaga agcgagagag aagaagacac agatccacaa 540
ggctcaagag gaatcagtga cgatgaagat ggtgataact ccaggaaaaa gcttagactt 600
tccaaagatc aatctgctat tcttgaagag accttcaaag atcacagtac tctcaatccg 660

```
aagcagaagc aagcattggc taaacaatta gggttacgag caagacaagt ggaagtttgg    720
tttcagaaca gacgagcaag aacaaagctg aagcaaacgg aggtagactg cgagttctta    780
cggagatgct gcgagaatct aacggaagag aaccgtcggc tacaaaaaga agtaacggaa    840
ttgagagtac ttaagctctc tcctcagttc tacatgcaca tgagcccacc cactactttg    900
accatgtgcc cttcatgtga acacgtgtcg gtcccgccac cacaacctca ggctgctacg    960
tcagcgcacc accggtcgtt gccggtcaat gcgtgggctc ctgctacgag gatatctcac   1020
ggcttgactt ttgacgctct tcgtcctagg tcctaagtct ttttacttgc aaccaaaggg   1080
cattttggtc gttttttaag tttcatggac cagatatgca tgtagttgtt aacatgtatg   1140
tattttctta gaaagaaaga aaaacagatt aatatt                              1176
```

```
<210>  230
<211>  284
<212>  PRT
<213>  Arabidopsis thaliana

<400>  230
Met Met Phe Glu Lys Asp Asp Leu Gly Leu Ser Leu Gly Leu Asn Phe
1               5                   10                  15
Pro Lys Lys Gln Ile Asn Leu Lys Ser Asn Pro Ser Val Ser Val Thr
            20                  25                  30
Pro Ser Ser Ser Ser Phe Gly Leu Phe Arg Arg Ser Ser Trp Asn Glu
        35                  40                  45
Ser Phe Thr Ser Ser Val Pro Asn Ser Asp Ser Ser Gln Lys Glu Thr
    50                  55                  60
Arg Thr Phe Ile Arg Gly Ile Asp Val Asn Arg Pro Pro Ser Thr Ala
65                  70                  75                  80
Glu Tyr Gly Asp Glu Asp Ala Gly Val Ser Ser Pro Asn Ser Thr Val
                85                  90                  95
Ser Ser Ser Thr Gly Lys Arg Ser Glu Arg Glu Glu Asp Thr Asp Pro
            100                 105                 110
Gln Gly Ser Arg Gly Ile Ser Asp Asp Glu Asp Gly Asp Asn Ser Arg
        115                 120                 125
Lys Lys Leu Arg Leu Ser Lys Asp Gln Ser Ala Ile Leu Glu Glu Thr
    130                 135                 140
Phe Lys Asp His Ser Thr Leu Asn Pro Lys Gln Lys Gln Ala Leu Ala
145                 150                 155                 160
Lys Gln Leu Gly Leu Arg Ala Arg Gln Val Glu Val Trp Phe Gln Asn
                165                 170                 175
Arg Arg Ala Arg Thr Lys Leu Lys Gln Thr Glu Val Asp Cys Glu Phe
            180                 185                 190
Leu Arg Arg Cys Cys Glu Asn Leu Thr Glu Glu Asn Arg Arg Leu Gln
        195                 200                 205
Lys Glu Val Thr Glu Leu Arg Ala Leu Lys Leu Ser Pro Gln Phe Tyr
    210                 215                 220
Met His Met Ser Pro Pro Thr Thr Leu Thr Met Cys Pro Ser Cys Glu
225                 230                 235                 240
His Val Ser Val Pro Pro Pro Gln Pro Gln Ala Ala Thr Ser Ala His
                245                 250                 255
His Arg Ser Leu Pro Val Asn Ala Trp Ala Pro Ala Thr Arg Ile Ser
            260                 265                 270
His Gly Leu Thr Phe Asp Ala Leu Arg Pro Arg Ser
        275                 280

<210>  231
<211>  3171
<212>  DNA
<213>  Arabidopsis thaliana

<400>  231
```

```
gacaatccga aattaggtaa ccatgttcat tgaatttatc gtttcaacac attcgaatca      60
ctgagtccta cgtttcagtt taattccctg atgcacattt tgaattcggt ttgcgtattg     120
gtcttcgagt tttccgtcgg cggccatgga cgcgttcaag gaggagatag aaatcgggag     180
ctcggtggag tctttaatgg agctattgga ttcgcagaag gtgctttttc atagccagat     240
cgatcagctc caagatgtcg tcgttgcgca atgcaaactt accggcgtta atccccttgc     300
gcaagaaatg gctgctggtg ctttgtccat taaaattgga aagcggccaa gagacttgtt     360
gaatcctaag gctgttaagt atctacaagc agttttcgca attaaagatg ctattagtaa     420
gagggaatct cgggagataa gtgctttatt tggcatcaca gtcgcccagg ttcgagaatt     480
ttttgttact caaaagacaa gagtgaggaa acaggtgagg ctttcaaggg agaaagtagt     540
tatgtccaat acgcatgctt tacaagatga tggtgttccg gaaaataaca atgccacaaa     600
tcatgttgaa cccgttccct tgaactctat acatccggag gcatgttcta taagctgggg     660
tgaaggtgaa acagtggcac ttattccacc tgaagatatt ccacctgaca tcagcgattc     720
agacaaatac tttgttgaga atatattttc tctgctgcgt aaagaggaaa cattttcagg     780
ccaggtgaaa ctaatggagt ggatcatgca gatacaagat gcttctgtgc tgatctggtt     840
tttatcaaaa ggaggggttt tgatacttac aacatggtta agtcaagctg ctagtgaaga     900
gcaaacaagt gtcttacttc ttatcctgaa ggttctttgt catttacctc tccacaaagc     960
atctcctgaa aatatgtctg ccatattaca aagcgttaat ggacttcgtt tctataggat    1020
atcagacata tcaaacaggg caaaaggttt gttatcaagg tggaccaagt tatttgcgaa    1080
aatccaagct atgaagaaac aaaatcgtaa cagttcgcaa attgattcgc agagtcaatt    1140
gcttctgaaa cagagtattg ctgaaatcat gggtgatagt agcaatcctg aagatattct    1200
tagtctctca aatggaaagt cagagaatgt caggaggatt gaatcgtcac agggtccaaa    1260
actgttgctt acttctgcag atgattccac caagaaacac atgcttggtt caaatccatc    1320
gtataacaaa gaacgcagga aagtacagat ggtggaacaa ccaggccaaa aagctgctgg    1380
aaagagtccg cagacagtaa gaataggaac ttcaggtcga agccgcccaa tgtctgctga    1440
tgatattcag aaagcaaaga tgcgtgccct ttatatgcag agcaagaaca gtaaaaagga    1500
tcctttacca agtgccattg gtgattcgaa aatcgttgct cctgagaagc ccttggctct    1560
tcattcagcc aaggattctc cacctattca gaacaatgaa gctaagactg aagacacacc    1620
tgtactctcg actgttcagc ccgtcaatgg attttcaact attcagcccg tcaatggacc    1680
ttcagctgtt cagcccgtca atggaccttt ggctgttcag cctgtcaatg gaccttcggc    1740
tcttcagccc gtcaatggac cttcggccgt aattgtcccg gtacaagctg atgaaattaa    1800
aaaaccttca acacctccta aaagcatttc tagtaaggtg ggagttatga tgaaaatgag    1860
ttcacaaact attctcaaga attgcaagag aaaacagatt gattggcatg taccaccagg    1920
aatggaactt gacgaactct ggagagtagc cgctggtggt aatagcaagg aggctgatgt    1980
tcagagaaac agaaaccggc gagaaagaga aacaacatat cagtctcttc aaactatacc    2040
gttgaaccct aaagaaccat gggataggga aatggactat gatgacagtt tgacccctga    2100
aattccatct caacagccac cagaagaaag tttaacggaa ccacaggatt cacttgatga    2160
acgaagaatt gctgctggtg ctgccacaac ctcttcatct ctaagcagtc ctgaacctga    2220
tctcgagtta ttagctgcgt tacttaagaa cccagatctt gtttatgcac taacttcggg    2280
aaaacccagt aatttagccg gccaagatat ggtaaaactg cttgatgtga ttaagactgg    2340
tgcaccaaac tcaagcagta gctcaaataa acaggttgaa gaaagggtcg aagtttccct    2400
tccatctccc actccatcaa ctaatcctgg aatgagtgga tggggacaag aagggattcg    2460
gaatccattt tcaaggcaaa accaagttgg tactgcagtt gctagatcgg gtacacagct    2520
tcgtgttggt tcaatgcaat ggcatcaaac aaatgaacaa tcaatcccac gacatgctcc    2580
atcagcatac agtaactcga tcacattggc tcacacagaa agagaacagc aacaatatat    2640
gcaaccaaaa cttcatcaca atttacattt tcaacaacaa caacaacaac caatctcaac    2700
aacctcgtat gcagttaggg aaccagtagg acaaatggga acaggtacat cgagttcatg    2760
gaggagtcag cagagtcaga acagttacta ctcacatcaa gaaaacgaga ttgcatcggc    2820
ttcacaagtt acttcatacc aagggaatag ccagtacatg agtagcaatc caggatatga    2880
atcatggagt cctgataata gcccaagtag gaaccagctt aacatgaggg gacaacaaca    2940
acaagcatca aggaaacatg attcttctac tcatccatat tggaaccaaa acaaaagatg    3000
gcgttaaacc atatataaga ctatggttct ttggtcctca atatttgctt gtcatggttg    3060
aattagcatt atactctgtt cagacacaga ttttcatttt taactcacaa cagattcatc    3120
agagataata aactaaaaga aagaatgaat ggataaagtt attactgtct c             3171
```

<210> 232
<211> 953
<212> PRT
<213> Arabidopsis thaliana

<400> 232

```
Met Asp Ala Phe Lys Glu Glu Ile Glu Ile Gly Ser Ser Val Glu Ser
1               5                   10                  15
Leu Met Glu Leu Leu Asp Ser Gln Lys Val Leu Phe His Ser Gln Ile
            20                  25                  30
Asp Gln Leu Gln Asp Val Val Val Ala Gln Cys Lys Leu Thr Gly Val
        35                  40                  45
Asn Pro Leu Ala Gln Glu Met Ala Ala Gly Ala Leu Ser Ile Lys Ile
    50                  55                  60
Gly Lys Arg Pro Arg Asp Leu Leu Asn Pro Lys Ala Val Lys Tyr Leu
65                  70                  75                  80
Gln Ala Val Phe Ala Ile Lys Asp Ala Ile Ser Lys Arg Glu Ser Arg
                85                  90                  95
Glu Ile Ser Ala Leu Phe Gly Ile Thr Val Ala Gln Val Arg Glu Phe
            100                 105                 110
Phe Val Thr Gln Lys Thr Arg Val Arg Lys Gln Val Arg Leu Ser Arg
        115                 120                 125
Glu Lys Val Val Met Ser Asn Thr His Ala Leu Gln Asp Asp Gly Val
    130                 135                 140
Pro Glu Asn Asn Asn Ala Thr Asn His Val Glu Pro Val Pro Leu Asn
145                 150                 155                 160
Ser Ile His Pro Glu Ala Cys Ser Ile Ser Trp Gly Glu Gly Glu Thr
                165                 170                 175
Val Ala Leu Ile Pro Pro Glu Asp Ile Pro Pro Asp Ile Ser Asp Ser
            180                 185                 190
Asp Lys Tyr Phe Val Glu Asn Ile Phe Ser Leu Leu Arg Lys Glu Glu
        195                 200                 205
Thr Phe Ser Gly Gln Val Lys Leu Met Glu Trp Ile Met Gln Ile Gln
    210                 215                 220
Asp Ala Ser Val Leu Ile Trp Phe Leu Ser Lys Gly Gly Val Leu Ile
225                 230                 235                 240
Leu Thr Thr Trp Leu Ser Gln Ala Ala Ser Glu Glu Gln Thr Ser Val
                245                 250                 255
Leu Leu Leu Ile Leu Lys Val Leu Cys His Leu Pro Leu His Lys Ala
            260                 265                 270
Ser Pro Glu Asn Met Ser Ala Ile Leu Gln Ser Val Asn Gly Leu Arg
        275                 280                 285
Phe Tyr Arg Ile Ser Asp Ile Ser Asn Arg Ala Lys Gly Leu Leu Ser
    290                 295                 300
Arg Trp Thr Lys Leu Phe Ala Lys Ile Gln Ala Met Lys Lys Gln Asn
305                 310                 315                 320
Arg Asn Ser Ser Gln Ile Asp Ser Gln Ser Gln Leu Leu Leu Lys Gln
                325                 330                 335
Ser Ile Ala Glu Ile Met Gly Asp Ser Ser Asn Pro Glu Asp Ile Leu
            340                 345                 350
Ser Leu Ser Asn Gly Lys Ser Glu Asn Val Arg Arg Ile Glu Ser Ser
        355                 360                 365
Gln Gly Pro Lys Leu Leu Leu Thr Ser Ala Asp Asp Ser Thr Lys Lys
    370                 375                 380
His Met Leu Gly Ser Asn Pro Ser Tyr Asn Lys Glu Arg Arg Lys Val
385                 390                 395                 400
Gln Met Val Glu Gln Pro Gly Gln Lys Ala Ala Gly Lys Ser Pro Gln
                405                 410                 415
Thr Val Arg Ile Gly Thr Ser Gly Arg Ser Arg Pro Met Ser Ala Asp
            420                 425                 430
Asp Ile Gln Lys Ala Lys Met Arg Ala Leu Tyr Met Gln Ser Lys Asn
        435                 440                 445
Ser Lys Lys Asp Pro Leu Pro Ser Ala Ile Gly Asp Ser Lys Ile Val
    450                 455                 460
```

Ala Pro Glu Lys Pro Leu Ala Leu His Ser Ala Lys Asp Ser Pro Pro
465 470 475 480
Ile Gln Asn Asn Glu Ala Lys Thr Glu Asp Thr Pro Val Leu Ser Thr
485 490 495
Val Gln Pro Val Asn Gly Phe Ser Thr Ile Gln Pro Val Asn Gly Pro
500 505 510
Ser Ala Val Gln Pro Val Asn Gly Pro Leu Ala Val Gln Pro Val Asn
515 520 525
Gly Pro Ser Ala Leu Gln Pro Val Asn Gly Pro Ser Ala Val Ile Val
530 535 540
Pro Val Gln Ala Asp Glu Ile Lys Lys Pro Ser Thr Pro Pro Lys Ser
545 550 555 560
Ile Ser Ser Lys Val Gly Val Met Met Lys Met Ser Ser Gln Thr Ile
565 570 575
Leu Lys Asn Cys Lys Arg Lys Gln Ile Asp Trp His Val Pro Pro Gly
580 585 590
Met Glu Leu Asp Glu Leu Trp Arg Val Ala Ala Gly Gly Asn Ser Lys
595 600 605
Glu Ala Asp Val Gln Arg Asn Arg Asn Arg Arg Glu Arg Glu Thr Thr
610 615 620
Tyr Gln Ser Leu Gln Thr Ile Pro Leu Asn Pro Lys Glu Pro Trp Asp
625 630 635 640
Arg Glu Met Asp Tyr Asp Asp Ser Leu Thr Pro Glu Ile Pro Ser Gln
645 650 655
Gln Pro Pro Glu Glu Ser Leu Thr Glu Pro Gln Asp Ser Leu Asp Glu
660 665 670
Arg Arg Ile Ala Ala Gly Ala Ala Thr Thr Ser Ser Ser Leu Ser Ser
675 680 685
Pro Glu Pro Asp Leu Glu Leu Leu Ala Ala Leu Leu Lys Asn Pro Asp
690 695 700
Leu Val Tyr Ala Leu Thr Ser Gly Lys Pro Ser Asn Leu Ala Gly Gln
705 710 715 720
Asp Met Val Lys Leu Leu Asp Val Ile Lys Thr Gly Ala Pro Asn Ser
725 730 735
Ser Ser Ser Ser Asn Lys Gln Val Glu Glu Arg Val Glu Val Ser Leu
740 745 750
Pro Ser Pro Thr Pro Ser Thr Asn Pro Gly Met Ser Gly Trp Gly Gln
755 760 765
Glu Gly Ile Arg Asn Pro Phe Ser Arg Gln Asn Gln Val Gly Thr Ala
770 775 780
Val Ala Arg Ser Gly Thr Gln Leu Arg Val Gly Ser Met Gln Trp His
785 790 795 800
Gln Thr Asn Glu Gln Ser Ile Pro Arg His Ala Pro Ser Ala Tyr Ser
805 810 815
Asn Ser Ile Thr Leu Ala His Thr Glu Arg Glu Gln Gln Gln Tyr Met
820 825 830
Gln Pro Lys Leu His His Asn Leu His Phe Gln Gln Gln Gln Gln Gln
835 840 845
Pro Ile Ser Thr Thr Ser Tyr Ala Val Arg Glu Pro Val Gly Gln Met
850 855 860
Gly Thr Gly Thr Ser Ser Ser Trp Arg Ser Gln Gln Ser Gln Asn Ser
865 870 875 880
Tyr Tyr Ser His Gln Glu Asn Glu Ile Ala Ser Ala Ser Gln Val Thr
885 890 895
Ser Tyr Gln Gly Asn Ser Gln Tyr Met Ser Ser Asn Pro Gly Tyr Glu
900 905 910
Ser Trp Ser Pro Asp Asn Ser Pro Ser Arg Asn Gln Leu Asn Met Arg
915 920 925
Gly Gln Gln Gln Gln Ala Ser Arg Lys His Asp Ser Ser Thr His Pro

```
    930                 935                 940
Tyr Trp Asn Gln Asn Lys Arg Trp Arg
945                 950

<210>  233
<211>  501
<212>  DNA
<213>  Oryza sativa

<400>  233
atggagcagc agcttcctct tcttgcacct gactctaagg ctgccacgtc gtccccttg      60
tgcctcacct tggacaaccc aacctccaca tccacctcgc cggcggtgcc gtcgtcggcg    120
ccgccgccgg cagccgcctt ggaaccttct agacaatctt tccatgagag ggaaacggat    180
gcaatcaaag ccaagatcat gtcgcacccc ctctacccgg ctctcctcag agccttcata    240
gattgccaga aggtcggagc tccgccggag gtcgtcggcc ggctttccgc cctcgccggc    300
gagctcgact cgcgtgcaga agacaggtac cttcaagggc agtcgtcaga cccggagctc    360
gacgagttta tggaaaccta cattgatatg ctggtgagct acaggcagga gctgacaaga    420
ccaattcaag aggccgacca gttcttcaga aacatggagg cacagatcga ctcgtttaca    480
ctagagatgt gcagtttctg a                                              501

<210>  234
<211>  166
<212>  PRT
<213>  Oryza sativa

<400>  234
Met Glu Gln Gln Leu Pro Leu Leu Ala Pro Asp Ser Lys Ala Ala Thr
1               5                   10                  15
Ser Ser Pro Leu Cys Leu Thr Leu Asp Asn Pro Thr Ser Thr Ser Thr
            20                  25                  30
Ser Pro Ala Val Pro Ser Ser Ala Pro Pro Pro Ala Ala Ala Leu Glu
        35                  40                  45
Pro Ser Arg Gln Ser Phe His Glu Arg Glu Thr Asp Ala Ile Lys Ala
    50                  55                  60
Lys Ile Met Ser His Pro Leu Tyr Pro Ala Leu Leu Arg Ala Phe Ile
65                  70                  75                  80
Asp Cys Gln Lys Val Gly Ala Pro Pro Glu Val Val Gly Arg Leu Ser
                85                  90                  95
Ala Leu Ala Gly Glu Leu Asp Ser Arg Ala Glu Asp Arg Tyr Leu Gln
            100                 105                 110
Gly Gln Ser Ser Asp Pro Glu Leu Asp Glu Phe Met Glu Thr Tyr Ile
        115                 120                 125
Asp Met Leu Val Ser Tyr Arg Gln Glu Leu Thr Arg Pro Ile Gln Glu
    130                 135                 140
Ala Asp Gln Phe Phe Arg Asn Met Glu Ala Gln Ile Asp Ser Phe Thr
145                 150                 155                 160
Leu Glu Met Cys Ser Phe
                165

<210>  235
<211>  879
<212>  DNA
<213>  Oryza sativa

<400>  235
atggctcagg aggacgtcgg tcacctgagc gacgccggcc tggcgctggg cctgtccctc     60
ggcgggggag gaggagggac gaccgacgcg gcggcggcgc accgtggcgg ctgccggcgg    120
ccgtcgccgt cgtcgcagtg cccgccgctg gagccgtcgc tgaccctgag cttgcccgac    180
gacgcggcgg ccggcgcggc cgcgaccgcg accgcgaccg cgtccggcgg gggcggccct    240
```

gcgcacagcg tgtcgtcgct gtccgtcggc gcggcggcgg cggcggccgt gaagagggag 300
cgcgcggagg aggccgacgg cgagagggtg tcgtcgacgg cggccgggcg tgacgacgac 360
gacgacggga gcacccgcaa gaagctccgg ctgaccaagg agcagtccgc gctcctggag 420
gaccgcttcc gggagcacag cacgctcaac ccgaagcaga aagtcgcttt agcgaagcaa 480
ctgaacctca ggccaaggca ggtggaggtc tggttccaaa acagaagagc aaggacaaag 540
ctgaagcaga cggaggtgga ctgcgagttc ctgaagcgct gctgcgagac gctcaccgag 600
gagaaccgtc ggctgcagcg cgagctgcag gagctccgcg cgctcaagtt cgccccgccg 660
ccgccgtcct cggcggccca ccagccgtcg ccggcgccac cggcgccgtt ctacatgcaa 720
ctcccggccg ccacgctcac catctgcccg tcctgcgagc gcgtcggcgg gcccgcgtcc 780
gccgccaagg tcgtcgccgc cgacgggacc aaggccggcc ccggccggac caccacccac 840
cacttcttca accccttcac ccactccgcc gcctgctga 879

<210> 236
<211> 292
<212> PRT
<213> Oryza sativa

<400> 236
Met Ala Gln Glu Asp Val Gly His Leu Ser Asp Ala Gly Leu Ala Leu
1 5 10 15
Gly Leu Ser Leu Gly Gly Gly Gly Gly Gly Thr Thr Asp Ala Ala Ala
20 25 30
Ala His Arg Gly Gly Cys Arg Arg Pro Ser Pro Ser Ser Gln Cys Pro
35 40 45
Pro Leu Glu Pro Ser Leu Thr Leu Ser Leu Pro Asp Asp Ala Ala Ala
50 55 60
Gly Ala Ala Ala Thr Ala Thr Ala Thr Ala Ser Gly Gly Gly Gly Pro
65 70 75 80
Ala His Ser Val Ser Ser Leu Ser Val Gly Ala Ala Ala Ala Ala Ala
85 90 95
Val Lys Arg Glu Arg Ala Glu Glu Ala Asp Gly Glu Arg Val Ser Ser
100 105 110
Thr Ala Ala Gly Arg Asp Asp Asp Asp Asp Gly Ser Thr Arg Lys Lys
115 120 125
Leu Arg Leu Thr Lys Glu Gln Ser Ala Leu Leu Glu Asp Arg Phe Arg
130 135 140
Glu His Ser Thr Leu Asn Pro Lys Gln Lys Val Ala Leu Ala Lys Gln
145 150 155 160
Leu Asn Leu Arg Pro Arg Gln Val Glu Val Trp Phe Gln Asn Arg Arg
165 170 175
Ala Arg Thr Lys Leu Lys Gln Thr Glu Val Asp Cys Glu Phe Leu Lys
180 185 190
Arg Cys Cys Glu Thr Leu Thr Glu Glu Asn Arg Arg Leu Gln Arg Glu
195 200 205
Leu Gln Glu Leu Arg Ala Leu Lys Phe Ala Pro Pro Pro Pro Ser Ser
210 215 220
Ala Ala His Gln Pro Ser Pro Ala Pro Pro Ala Pro Phe Tyr Met Gln
225 230 235 240
Leu Pro Ala Ala Thr Leu Thr Ile Cys Pro Ser Cys Glu Arg Val Gly
245 250 255
Gly Pro Ala Ser Ala Ala Lys Val Val Ala Ala Asp Gly Thr Lys Ala
260 265 270
Gly Pro Gly Arg Thr Thr Thr His His Phe Phe Asn Pro Phe Thr His
275 280 285
Ser Ala Ala Cys
290

<210> 237
<211> 744

<212> DNA
<213> Oryza sativa

<400> 237
atggcgcaag atgatgagga cgtgggtcta gctctgggcc tctccctggg ctccggcggc 60
cacaggcggc agagagaaag tagagacgaa gcgccgtcgt cggcggcggc gtccctgctg 120
acgctgaggc tcccggcaga gagcgggggg cagccgcagg tggtggtgaa gagggaggtg 180
gtgcgggcgg aagaggagga gtacgaatac gagtacgaga gggcgctcta ctcgtcgtcg 240
gctgcggcgg cggacgacga cgagggctgc aacagccgga agaagctgag gctgagcaag 300
gagcagtcgg ctctgctgga ggatcgcttc aaggagcata gcactctcaa ccctaagcaa 360
aaggttgctt tggcgaagca gctaaacctg aggccaaggc aagttgaggt gtggttccaa 420
aacagaagag ccaggacgaa gctgaagcag acggaggtgg attgcgagct tctgaagcgc 480
tgctgcgaga cgctgacgga ggagaaccga cgcctccatc gtgagctcca gcagctcagg 540
gctctgaccc actccaccgc cgccggcttc ttcatggcca ccaccctccc cgtccccgcc 600
gccacgctct ccatctgccc ctcctgcgag cgcctcgcca ccgccgccgc cgccggagct 660
tcccccaccg ccgccgccga ccgcaccaac aagcccaccg ccccgcactt gttcagccct 720
ttcgccaagt ccgccgcctg ctag 744

<210> 238
<211> 247
<212> PRT
<213> Oryza sativa

<400> 238
Met Ala Gln Asp Asp Glu Asp Val Gly Leu Ala Leu Gly Leu Ser Leu
1 5 10 15
Gly Ser Gly Gly His Arg Arg Gln Arg Glu Ser Arg Asp Glu Ala Pro
20 25 30
Ser Ser Ala Ala Ala Ser Leu Leu Thr Leu Arg Leu Pro Ala Glu Ser
35 40 45
Gly Gly Gln Pro Gln Val Val Val Lys Arg Glu Val Val Arg Ala Glu
50 55 60
Glu Glu Glu Tyr Glu Tyr Glu Tyr Glu Arg Ala Leu Tyr Ser Ser Ser
65 70 75 80
Ala Ala Ala Ala Asp Asp Asp Glu Gly Cys Asn Ser Arg Lys Lys Leu
85 90 95
Arg Leu Ser Lys Glu Gln Ser Ala Leu Leu Glu Asp Arg Phe Lys Glu
100 105 110
His Ser Thr Leu Asn Pro Lys Gln Lys Val Ala Leu Ala Lys Gln Leu
115 120 125
Asn Leu Arg Pro Arg Gln Val Glu Val Trp Phe Gln Asn Arg Arg Ala
130 135 140
Arg Thr Lys Leu Lys Gln Thr Glu Val Asp Cys Glu Leu Leu Lys Arg
145 150 155 160
Cys Cys Glu Thr Leu Thr Glu Glu Asn Arg Arg Leu His Arg Glu Leu
165 170 175
Gln Gln Leu Arg Ala Leu Thr His Ser Thr Ala Ala Gly Phe Phe Met
180 185 190
Ala Thr Thr Leu Pro Val Pro Ala Ala Thr Leu Ser Ile Cys Pro Ser
195 200 205
Cys Glu Arg Leu Ala Thr Ala Ala Ala Ala Gly Ala Ser Pro Thr Ala
210 215 220
Ala Ala Asp Arg Thr Asn Lys Pro Thr Ala Pro His Leu Phe Ser Pro
225 230 235 240
Phe Ala Lys Ser Ala Ala Cys
245

<210> 239
<211> 1065

<212> DNA
<213> Oryza sativa

<400> 239
```
atggagctgg ggctgagctt gggggatgcg gtgaccgtgg cggatggcgg gaggctggag     60
ctggttcttg ggctcggggt tggggttggg gctggggtga ggagaggaga ggaggaggag    120
agggggagga gggaggatgt ggtgggagct gggaggtggg cggcgatggc ggcggcctcg    180
ccggagccgt cggtgcggct cagcctcgtg tcgagcctcg ggctccactg gccttccgag    240
accgggcgtt cggaggcggc ggcgcgtggg ttcgacgtga accgggcgcc gtcggtggcg    300
gcgggcgcgc cggggatgga ggacgacgag gagggcccgg gcgccgcgcc ggcgttgtcg    360
tcgtcgccca acgacagcgg gggatccttc ccgctggacc tctccgggca aggcctccgt    420
ggccacgccg aggcggcggc gcagggtggc ggcggcggcg gcggcggcga gcggtcgtcc    480
tcgcgcgcga gcgacgacga cgagggcgcg tccgcgcgca agaagctgcg actctccaag    540
gagcagtcgg cgttcctcga ggagagcttc aaggagcaca gcacgctgaa tcccaagcag    600
aaggtggcgc tggcgaagca gctcaacctc cggccgcggc aagtggaggt ctggttccag    660
aaccgccgag ccaggacgaa gctgaagcag acggaggtgg actgcgagta cctgaagcgc    720
tgctgcgaga cgctcaccga ggagaaccgg cggctgcaca aggagctcgc cgagctgcgc    780
gcgctcaaga cggcgcgccc cttctacatg cacctcccgg ccacgaccct ctccatgtgc    840
ccctcctgcg agcgtgtcgc ctccaacccg gccaccgctt cgacctccgc cccgccgcg     900
gccacgtccc cggcggcggc gccgaccgcg gccgcaagaa ccgccgtcgc gtcgcccgag    960
ccgcaccggc cgtcgtcctt cgccgcgctg ttcgcggcac cctcggcttt cccgctgacc   1020
gccgcccagc cgcggccgcc gccgccggcg agcaactgcc tgtaa                   1065
```

<210> 240
<211> 354
<212> PRT
<213> Oryza sativa

<400> 240
```
Met Glu Leu Gly Leu Ser Leu Gly Asp Ala Val Thr Val Ala Asp Gly
1               5                   10                  15
Gly Arg Leu Glu Leu Val Leu Gly Leu Gly Val Gly Val Gly Ala Gly
            20                  25                  30
Val Arg Arg Gly Glu Glu Glu Glu Arg Gly Arg Arg Glu Asp Val Val
        35                  40                  45
Gly Ala Gly Arg Trp Ala Ala Met Ala Ala Ala Ser Pro Glu Pro Ser
    50                  55                  60
Val Arg Leu Ser Leu Val Ser Ser Leu Gly Leu His Trp Pro Ser Glu
65                  70                  75                  80
Thr Gly Arg Ser Glu Ala Ala Ala Arg Gly Phe Asp Val Asn Arg Ala
                85                  90                  95
Pro Ser Val Ala Ala Gly Ala Pro Gly Met Glu Asp Asp Glu Glu Gly
            100                 105                 110
Pro Gly Ala Ala Pro Ala Leu Ser Ser Ser Pro Asn Asp Ser Gly Gly
        115                 120                 125
Ser Phe Pro Leu Asp Leu Ser Gly Gln Gly Leu Arg Gly His Ala Glu
    130                 135                 140
Ala Ala Ala Gln Gly Gly Gly Gly Gly Gly Gly Gly Glu Arg Ser Ser
145                 150                 155                 160
Ser Arg Ala Ser Asp Asp Asp Glu Gly Ala Ser Ala Arg Lys Lys Leu
                165                 170                 175
Arg Leu Ser Lys Glu Gln Ser Ala Phe Leu Glu Glu Ser Phe Lys Glu
            180                 185                 190
His Ser Thr Leu Asn Pro Lys Gln Lys Val Ala Leu Ala Lys Gln Leu
        195                 200                 205
Asn Leu Arg Pro Arg Gln Val Glu Val Trp Phe Gln Asn Arg Arg Ala
    210                 215                 220
Arg Thr Lys Leu Lys Gln Thr Glu Val Asp Cys Glu Tyr Leu Lys Arg
225                 230                 235                 240
```

Cys Cys Glu Thr Leu Thr Glu Glu Asn Arg Arg Leu His Lys Glu Leu
245 250 255
Ala Glu Leu Arg Ala Leu Lys Thr Ala Arg Pro Phe Tyr Met His Leu
260 265 270
Pro Ala Thr Thr Leu Ser Met Cys Pro Ser Cys Glu Arg Val Ala Ser
275 280 285
Asn Pro Ala Thr Ala Ser Thr Ser Ala Pro Ala Ala Ala Thr Ser Pro
290 295 300
Ala Ala Ala Pro Thr Ala Ala Ala Arg Thr Ala Val Ala Ser Pro Glu
305 310 315 320
Pro His Arg Pro Ser Ser Phe Ala Ala Leu Phe Ala Ala Pro Leu Gly
325 330 335
Phe Pro Leu Thr Ala Ala Gln Pro Arg Pro Pro Pro Pro Ala Ser Asn
340 345 350
Cys Leu

<210> 241
<211> 1089
<212> DNA
<213> Oryza sativa

<400> 241
atggagctgg ggttgagctt gggggaggct atggcggatg ctgggaggga gctggttctt 60
gggcttggga tggggaggag ggaggaggcg gcggaggcgg ggaggaggga tcatgaggtg 120
aggagggagc tggagttcgg gtcgatgtcg agcaggtgcg gtggttcttc gccggagccg 180
acggtgcggc tcacgcttct gcccatggtg cccggccttg gcctcccatg gccgccgccg 240
ccgccgccgt cgtccgagag caggcatttg gaggcgtcga cgcggggatt cgacgtgaac 300
cgcccgccgt cgtccggcgg cggcggcggc ggcggcggcg cggaggagga gcaggacgac 360
gtggccgggg cggcactctc gtcgtccccc aacaacagcg ccggatcctt cccgatggat 420
gacttctccg ggcacggcct cggcggcaac gacgcggccc ctggcggtgg cggcggcgac 480
cgctcgtgct cccgcgccag cgacgaggac gacggcggct ccgcgcgcaa gaagctccgc 540
ctctccaagg agcagtccgc gttcctcgag gagagcttca aggagcacag caccctaaac 600
cccaagcaga agctggcgct ggcgaagcag ctcaacctcc ggccgcgcca ggtggaggtg 660
tggttccaga accgccgcgc caggacgaag ctgaagcaga cggaggtgga ctgcgagtac 720
ctgaagcggt gctgcgagac gctgacggag gagaaccggc ggctgcagaa ggagctcgcc 780
gagctccggg cgctcaagac ggtgcacccc ttctacatgc acctcccggc gacgacactc 840
tccatgtgcc cctcctgcga gcgcgtcgcc tccaactccg ccccggccac cgcctcctcc 900
gccgcaacat cgtcgacggc cgcgccgccc gcggcaccct catccggcgg cattgcggcc 960
acctcctcct ccgccgccgc cgccgcggcg ccggaccaca ggccgtcgtc gttcgccgcg 1020
ctgttctcgt cgccgcgtgg cttcccgcta tccgtagccc cgcaggcgca gccgccgacg 1080
agctcgtga 1089

<210> 242
<211> 362
<212> PRT
<213> Oryza sativa

<400> 242
Met Glu Leu Gly Leu Ser Leu Gly Glu Ala Met Ala Asp Ala Gly Arg
1 5 10 15
Glu Leu Val Leu Gly Leu Gly Met Gly Arg Arg Glu Glu Ala Ala Glu
20 25 30
Ala Gly Arg Arg Asp His Glu Val Arg Arg Glu Leu Glu Phe Gly Ser
35 40 45
Met Ser Ser Arg Cys Gly Gly Ser Ser Pro Glu Pro Thr Val Arg Leu
50 55 60
Thr Leu Leu Pro Met Val Pro Gly Leu Gly Leu Pro Trp Pro Pro Pro
65 70 75 80

```
Pro Pro Pro Ser Ser Glu Ser Arg His Leu Glu Ala Ser Thr Arg Gly
                85                  90                  95
Phe Asp Val Asn Arg Pro Pro Ser Ser Gly Gly Gly Gly Gly Gly Gly
            100                 105                 110
Gly Ala Glu Glu Glu Gln Asp Asp Val Ala Gly Ala Ala Leu Ser Ser
        115                 120                 125
Ser Pro Asn Asn Ser Ala Gly Ser Phe Pro Met Asp Asp Phe Ser Gly
    130                 135                 140
His Gly Leu Gly Gly Asn Asp Ala Ala Pro Gly Gly Gly Gly Gly Asp
145                 150                 155                 160
Arg Ser Cys Ser Arg Ala Ser Asp Glu Asp Asp Gly Gly Ser Ala Arg
                165                 170                 175
Lys Lys Leu Arg Leu Ser Lys Glu Gln Ser Ala Phe Leu Glu Glu Ser
            180                 185                 190
Phe Lys Glu His Ser Thr Leu Asn Pro Lys Gln Lys Leu Ala Leu Ala
        195                 200                 205
Lys Gln Leu Asn Leu Arg Pro Arg Gln Val Glu Val Trp Phe Gln Asn
    210                 215                 220
Arg Arg Ala Arg Thr Lys Leu Lys Gln Thr Glu Val Asp Cys Glu Tyr
225                 230                 235                 240
Leu Lys Arg Cys Cys Glu Thr Leu Thr Glu Glu Asn Arg Arg Leu Gln
                245                 250                 255
Lys Glu Leu Ala Glu Leu Arg Ala Leu Lys Thr Val His Pro Phe Tyr
            260                 265                 270
Met His Leu Pro Ala Thr Thr Leu Ser Met Cys Pro Ser Cys Glu Arg
        275                 280                 285
Val Ala Ser Asn Ser Ala Pro Ala Thr Ala Ser Ser Ala Ala Thr Ser
    290                 295                 300
Ser Thr Ala Ala Pro Pro Ala Ala Pro Ser Ser Gly Gly Ile Ala Ala
305                 310                 315                 320
Thr Ser Ser Ser Ala Ala Ala Ala Ala Ala Pro Asp His Arg Pro Ser
                325                 330                 335
Ser Phe Ala Ala Leu Phe Ser Ser Pro Arg Gly Phe Pro Leu Ser Val
            340                 345                 350
Ala Pro Gln Ala Gln Pro Pro Thr Ser Ser
        355                 360
```

<210> 243
<211> 771
<212> DNA
<213> Oryza sativa

<400> 243

```
atggagaggc aaggcttgga tcttggcctg agcctcgggc taggcttgac gacggcggcg      60
acatggccgg ctgctgggtt ctgtctgaac tccggcatgg cggagcagga agtgatcagg     120
cgtgatgatg tggttgcggc gacggcggcg gaggatgaga ggttcgcgtg ctcacccggc     180
agcccggtgt cgagcggcag cgggaagcga ggcagcggca gcggcagcgg cgacgaggtc     240
gacgacgccg gctgcgacgt cggcggcggc ggcgcgcgca agaagctgcg gttgtccaag     300
gaccaggccg ccgtcctcga ggagtgcttc aagacgcacc acaccctcac tccgaagcag     360
aaggtggcgc tggcgaagag cttgaacctg cggccgcggc aggtggaggt gtggttccag     420
aaccgccgcg cgaggacgaa gctgaagcag acggaggtgg actgcgagca cctcaagcgg     480
tggtgcgacc agctcgccga cgacaaccgc cgcctccaca aggagctcgc cgagctcagg     540
gcgctcaagg ccacgcccac accgcccgcc gccgcgccgc cattgaccac cctcacaatg     600
tgcctctcct gcaagcgcgt cgccaatgcc ggcgtgccct cgccggcggc ggcgatattc     660
cccggccacc cccagttctt gtgcggattc agagatcacg ccggagcagc gtcgtcgtcg     720
tacggcggcg catcatctgg actcgcgaag gcggtcaggg cggcgaggta g              771
```

<210> 244
<211> 256

```
<212>  PRT
<213>  Oryza sativa

<400>  244
Met Glu Arg Gln Gly Leu Asp Leu Gly Leu Ser Leu Gly Leu Gly Leu
1               5                   10                  15
Thr Thr Ala Ala Thr Trp Pro Ala Ala Gly Phe Cys Leu Asn Ser Gly
            20                  25                  30
Met Ala Glu Gln Glu Val Ile Arg Arg Asp Asp Val Val Ala Ala Thr
        35                  40                  45
Ala Ala Glu Asp Glu Arg Phe Ala Cys Ser Pro Gly Ser Pro Val Ser
    50                  55                  60
Ser Gly Ser Gly Lys Arg Gly Ser Gly Ser Gly Ser Gly Asp Glu Val
65                  70                  75                  80
Asp Asp Ala Gly Cys Asp Val Gly Gly Gly Gly Ala Arg Lys Lys Leu
                85                  90                  95
Arg Leu Ser Lys Asp Gln Ala Ala Val Leu Glu Glu Cys Phe Lys Thr
            100                 105                 110
His His Thr Leu Thr Pro Lys Gln Lys Val Ala Leu Ala Lys Ser Leu
        115                 120                 125
Asn Leu Arg Pro Arg Gln Val Glu Val Trp Phe Gln Asn Arg Arg Ala
    130                 135                 140
Arg Thr Lys Leu Lys Gln Thr Glu Val Asp Cys Glu His Leu Lys Arg
145                 150                 155                 160
Trp Cys Asp Gln Leu Ala Asp Asp Asn Arg Arg Leu His Lys Glu Leu
                165                 170                 175
Ala Glu Leu Arg Ala Leu Lys Ala Thr Pro Thr Pro Pro Ala Ala Ala
            180                 185                 190
Pro Pro Leu Thr Thr Leu Thr Met Cys Leu Ser Cys Lys Arg Val Ala
        195                 200                 205
Asn Ala Gly Val Pro Ser Pro Ala Ala Ala Ile Phe Pro Gly His Pro
    210                 215                 220
Gln Phe Leu Cys Gly Phe Arg Asp His Ala Gly Ala Ala Ser Ser Ser
225                 230                 235                 240
Tyr Gly Gly Ala Ser Ser Gly Leu Ala Lys Ala Val Arg Ala Ala Arg
                245                 250                 255


<210>  245
<211>  927
<212>  DNA
<213>  Oryza sativa

<400>  245
atgatggatc tcggcctcag cctcggcctc ggcctcgcct cgcagggcag cctcacctcc      60
tccaccacca ccacctcctc ccccggcgcc ggatcatcct ccccgtgggc cgccgcgctc     120
aactccatcg tcggcgacgt gaggcgggat caggccgcgg cgcatgctgc cgcggcggtg     180
ggggttgggg ttgggggcga ggagatgtac caggggaggg cgtccacgtc gccggacagc     240
gcggcggcgc tgtcgagcgc gagcgggaag agggagaggg agctggagcg gtcgggatcc     300
ggggttgacg acgacgacgg cgcggacggc gccggcgggc ggaagaagct caggctgtcc     360
aaggaccagg ccgccgtgct cgaggagtgc ttcaagacgc actccactct caaccccaag     420
cagaaggtgg cgctggcgaa caggctgggg ctgcggccgc ggcaggtgga ggtgtggttc     480
cagaaccgga gggcgaggac gaagctgaag cagacggagg tggactgcga gtacctgaag     540
cggtggtgcg agcgcctcgc cgacgagaac aagcgcctcg agaaggagct cgccgacctc     600
agggcgctca aggccgcgcc ctcgccggcg tccgcgtccg cgatgcagcc ctcctcctcc     660
gccgccgcca cgctcaccat gtgcccctcc tgccgccgcg tcgccaccgc cggcgcgccg     720
caccagccta accaccaaca atgccatccc aaatctaaca ccaccatctc ctcctcctcc     780
accgccgccg ccgccgtcgc cgtcgccggc ggcaacgtgc tgcccagcca ctgccagttc     840
ttcccggccg ccgccgccgc cgccgaccgg acaagccaga gcacgtggaa cgccgccgcg     900
ccgctcgtca ccagagagct cttctag                                        927
```

<210> 246
<211> 308
<212> PRT
<213> Oryza sativa

<400> 246
Met Met Asp Leu Gly Leu Ser Leu Gly Leu Gly Leu Ala Ser Gln Gly
1 5 10 15
Ser Leu Thr Ser Ser Thr Thr Thr Thr Ser Ser Pro Gly Ala Gly Ser
20 25 30
Ser Ser Pro Trp Ala Ala Ala Leu Asn Ser Ile Val Gly Asp Val Arg
35 40 45
Arg Asp Gln Ala Ala Ala His Ala Ala Ala Ala Val Gly Val Gly Val
50 55 60
Gly Gly Glu Glu Met Tyr Gln Gly Arg Ala Ser Thr Ser Pro Asp Ser
65 70 75 80
Ala Ala Ala Leu Ser Ser Ala Ser Gly Lys Arg Glu Arg Glu Leu Glu
85 90 95
Arg Ser Gly Ser Gly Val Asp Asp Asp Asp Gly Ala Asp Gly Ala Gly
100 105 110
Gly Arg Lys Lys Leu Arg Leu Ser Lys Asp Gln Ala Ala Val Leu Glu
115 120 125
Glu Cys Phe Lys Thr His Ser Thr Leu Asn Pro Lys Gln Lys Val Ala
130 135 140
Leu Ala Asn Arg Leu Gly Leu Arg Pro Arg Gln Val Glu Val Trp Phe
145 150 155 160
Gln Asn Arg Arg Ala Arg Thr Lys Leu Lys Gln Thr Glu Val Asp Cys
165 170 175
Glu Tyr Leu Lys Arg Trp Cys Glu Arg Leu Ala Asp Glu Asn Lys Arg
180 185 190
Leu Glu Lys Glu Leu Ala Asp Leu Arg Ala Leu Lys Ala Ala Pro Ser
195 200 205
Pro Ala Ser Ala Ser Ala Met Gln Pro Ser Ser Ser Ala Ala Ala Thr
210 215 220
Leu Thr Met Cys Pro Ser Cys Arg Arg Val Ala Thr Ala Gly Ala Pro
225 230 235 240
His Gln Pro Asn His Gln Gln Cys His Pro Lys Ser Asn Thr Thr Ile
245 250 255
Ser Ser Ser Ser Thr Ala Ala Ala Ala Val Ala Val Ala Gly Gly Asn
260 265 270
Val Leu Pro Ser His Cys Gln Phe Phe Pro Ala Ala Ala Ala Ala Ala
275 280 285
Asp Arg Thr Ser Gln Ser Thr Trp Asn Ala Ala Ala Pro Leu Val Thr
290 295 300
Arg Glu Leu Phe
305

<210> 247
<211> 936
<212> DNA
<213> Oryza sativa

<400> 247
atggagatga tggttcatgg gaggagagac gagcagtatg gcgggctcag gctcgggctt 60
gggcttgggc tcagcctcgg cgtcgccggt ggtgcagccg acgacgagca gccgccgccg 120
cgccgtggtg ccgccccgcc gccgcagcag cagctgtgcg gctggaacgg cggcggtctc 180
ttctcctcgt cttcctccga tcatcggggg aggtcggcga tgatggcgtg ccacgacgtc 240
atcgagatgc cgttcctgcg ggggatcgac gtgaaccgtg cgccggcggc agagacgacc 300

```
acgacgacgg cgagggggcc cagctgcagc gaggaagacg aggagcccgg cgcgtcctcc     360
cccaacagca cgctctccag cctcagcggc aagcgcggcg caccatctgc cgccaccgcc     420
gccgccgccg ccgccagcga cgacgaggac tccggcggcg gatcccgcaa gaagctccgc     480
ctctccaagg accaagccgc cgtcctcgag gacaccttca aagagcacaa caccctcaat     540
cccaagcaga aggcggcgct ggcgaggcag ctgaatctga agccgcggca ggtggaggtg     600
tggttccaga acaggagggc gaggacgaag ctgaagcaga cggaggtgga ctgcgagctg     660
ctcaagcgct gctgcgagac gctcaccgac gagaaccgcc gcctccaccg cgagctccag     720
gagctccgcg ccctcaagct cgccaccgcc gccgccgcgc cgcaccacct ctacggcgcc     780
cgcgtcccgc cgcccaccac cctcaccatg tgcccctcct gcgagcgcgt cgcctccgca     840
gccaccacca cccgcaacaa ctccggcgcc gcccccgcgc ggccggtgcc cacccgcccg     900
tggccgccgg cggcggcgca gaggtcgtcg gcgtag                               936
```

```
<210>  248
<211>  311
<212>  PRT
<213>  Oryza sativa

<400>  248
Met Glu Met Met Val His Gly Arg Arg Asp Glu Gln Tyr Gly Gly Leu
1               5                   10                  15
Arg Leu Gly Leu Gly Leu Gly Leu Ser Leu Gly Val Ala Gly Gly Ala
            20                  25                  30
Ala Asp Asp Glu Gln Pro Pro Pro Arg Arg Gly Ala Ala Pro Pro Pro
        35                  40                  45
Gln Gln Gln Leu Cys Gly Trp Asn Gly Gly Gly Leu Phe Ser Ser Ser
    50                  55                  60
Ser Ser Asp His Arg Gly Arg Ser Ala Met Met Ala Cys His Asp Val
65                  70                  75                  80
Ile Glu Met Pro Phe Leu Arg Gly Ile Asp Val Asn Arg Ala Pro Ala
                85                  90                  95
Ala Glu Thr Thr Thr Thr Thr Ala Arg Gly Pro Ser Cys Ser Glu Glu
            100                 105                 110
Asp Glu Glu Pro Gly Ala Ser Ser Pro Asn Ser Thr Leu Ser Ser Leu
        115                 120                 125
Ser Gly Lys Arg Gly Ala Pro Ser Ala Ala Thr Ala Ala Ala Ala Ala
    130                 135                 140
Ala Ser Asp Asp Glu Asp Ser Gly Gly Gly Ser Arg Lys Lys Leu Arg
145                 150                 155                 160
Leu Ser Lys Asp Gln Ala Ala Val Leu Glu Asp Thr Phe Lys Glu His
                165                 170                 175
Asn Thr Leu Asn Pro Lys Gln Lys Ala Ala Leu Ala Arg Gln Leu Asn
            180                 185                 190
Leu Lys Pro Arg Gln Val Glu Val Trp Phe Gln Asn Arg Arg Ala Arg
        195                 200                 205
Thr Lys Leu Lys Gln Thr Glu Val Asp Cys Glu Leu Leu Lys Arg Cys
    210                 215                 220
Cys Glu Thr Leu Thr Asp Glu Asn Arg Arg Leu His Arg Glu Leu Gln
225                 230                 235                 240
Glu Leu Arg Ala Leu Lys Leu Ala Thr Ala Ala Ala Ala Pro His His
                245                 250                 255
Leu Tyr Gly Ala Arg Val Pro Pro Pro Thr Thr Leu Thr Met Cys Pro
            260                 265                 270
Ser Cys Glu Arg Val Ala Ser Ala Ala Thr Thr Thr Arg Asn Asn Ser
        275                 280                 285
Gly Ala Ala Pro Ala Arg Pro Val Pro Thr Arg Pro Trp Pro Pro Ala
    290                 295                 300
Ala Ala Gln Arg Ser Ser Ala
305                 310
```

<210> 249
<211> 1674
<212> DNA
<213> Arabidopsis thaliana

<400> 249
```
aaaatggaaa actcagacac tgattcagaa gttttctttt ggtttcagaa ccaaaaccaa      60
aaccatagcc ataagtttcc ttcttcttgt ttcctccgga gctctcactc tgctttttat     120
ggatctagct caatgatcaa cacagagact gcaactatgg acgaagaaga cgtatgtgag     180
agctacatga tgcgtgagat aaccaagaaa cggaagctaa caccgatcca attgcgttta     240
ctagaggaga gtttcgaaga agagaaaaga ctcgaaccag acaggaaact ctggctagcc     300
gagaagctag ggctgcagcc aagccaagtc gctgtctggt tccaaaacag aagagctaga     360
tacaagacca aacagctcga acacgactgt gactctctta aagctagcta tgctaagctc     420
aagactgact gggacattct ctttgtacaa aaccaaaccc tcaagagcaa ggtacagttt     480
cttaatagat taacaagcca ctattttcaa gagtctgttc aaaattttga tgacacattc     540
aaacaggtcg atcttctcaa agagaagctg aaaatgcaag agaatcttga aactcagtct     600
attgagagga aaagacttgg ggaagaaggt agttcggtga aaagcgataa cacgcagtac     660
agtgaagaag aaggtttgga gaatcaatac agtttcccgg agcttgcagt tctcggattt     720
tattatgatc caaccttaac tgcttcaaat ctaagacaag aacctttgaa agtcacgtgt     780
gcggatcaga tgactcagat ccaaatatct gacgtcacag aacctgcgag ctccgcacat     840
aaaaaaattg aagtgacaca gcgaagttcg agtatgagcc gcaagagaga taaaccctac     900
acaaaccgtc acacaccggc gcgaatttcg aaacgccggc gaccatgggc gccttcgtca     960
tcggagcacg atgagattat cgacaaaccg atcaccaaac ctccgccgcc accggcgttg    1020
gtagttatgg gacttcccgc caactgttca gttctggagc taaaatctcg attcgagatc    1080
tacggttcaa tctcccgaat ccgaatccat aaagacggaa ttggctccgt ttcgtatcga    1140
accgccgagt cagcagaagc cgccattgct ggtagtcacg agccttcttt cggtatctcc    1200
atcgattcca aaaagttgga ggtggtttgg gcgacggatc cattggtgaa gtggaaggaa    1260
ggtgtgacgg cgggagaagg aaaggagaga acgtcgtcgt tttcgtcgaa gcttttacga    1320
cctgtgatgc ctttgagaaa acatgggaga agcagtaggt tagcgtcagc tattgttaat    1380
ccgagaagtg ataatactaa aggaattagt ggagatggag ggatatcatc tccggcgacg    1440
acaagtgaag ttaaacagag aaatatagta acctacgacg atatcgttta acaccattct    1500
actacaatta tagtaacttt gattctttta catattttgg caattttatg agtttagcta    1560
atcctgttgg taatctcagt tgtcgaatgt tgtgaaatga gttgagtttt gtttatttgt    1620
atgattgtaa cttataagaa agagaaggat tccaaaatgg gtagaagatt caaa          1674
```

<210> 250
<211> 495
<212> PRT
<213> Arabidopsis thaliana

<400> 250
```
Met Glu Asn Ser Asp Thr Asp Ser Glu Val Phe Phe Trp Phe Gln Asn
1               5                   10                  15
Gln Asn Gln Asn His Ser His Lys Phe Pro Ser Ser Cys Phe Pro Pro
            20                  25                  30
Ser Ser His Ser Ala Phe Tyr Gly Ser Ser Ser Met Ile Asn Thr Glu
        35                  40                  45
Thr Ala Thr Met Asp Glu Glu Asp Val Cys Glu Ser Tyr Met Met Arg
    50                  55                  60
Glu Ile Thr Lys Lys Arg Lys Leu Thr Pro Ile Gln Leu Arg Leu Leu
65                  70                  75                  80
Glu Glu Ser Phe Glu Glu Glu Lys Arg Leu Glu Pro Asp Arg Lys Leu
                85                  90                  95
Trp Leu Ala Glu Lys Leu Gly Leu Gln Pro Ser Gln Val Ala Val Trp
            100                 105                 110
Phe Gln Asn Arg Arg Ala Arg Tyr Lys Thr Lys Gln Leu Glu His Asp
        115                 120                 125
Cys Asp Ser Leu Lys Ala Ser Tyr Ala Lys Leu Lys Thr Asp Trp Asp
    130                 135                 140
```

```
Ile Leu Phe Val Gln Asn Gln Thr Leu Lys Ser Lys Val Gln Phe Leu
145                 150                 155                 160
Asn Arg Leu Thr Ser His Tyr Phe Gln Glu Ser Val Gln Asn Phe Asp
                165                 170                 175
Asp Thr Phe Lys Gln Val Asp Leu Leu Lys Glu Lys Leu Lys Met Gln
            180                 185                 190
Glu Asn Leu Glu Thr Gln Ser Ile Glu Arg Lys Arg Leu Gly Glu Glu
        195                 200                 205
Gly Ser Ser Val Lys Ser Asp Asn Thr Gln Tyr Ser Glu Glu Glu Gly
    210                 215                 220
Leu Glu Asn Gln Tyr Ser Phe Pro Glu Leu Ala Val Leu Gly Phe Tyr
225                 230                 235                 240
Tyr Asp Pro Thr Leu Thr Ala Ser Asn Leu Arg Gln Glu Pro Leu Lys
                245                 250                 255
Val Thr Cys Ala Asp Gln Met Thr Gln Ile Gln Ile Ser Asp Val Thr
            260                 265                 270
Glu Pro Ala Ser Ser Ala His Lys Lys Ile Glu Val Thr Gln Arg Ser
        275                 280                 285
Ser Ser Met Ser Arg Lys Arg Asp Lys Pro Tyr Thr Asn Arg His Thr
    290                 295                 300
Pro Ala Arg Ile Ser Lys Arg Arg Arg Pro Trp Ala Pro Ser Ser Ser
305                 310                 315                 320
Glu His Asp Glu Ile Ile Asp Lys Pro Ile Thr Lys Pro Pro Pro Pro
                325                 330                 335
Pro Ala Leu Val Val Met Gly Leu Pro Ala Asn Cys Ser Val Leu Glu
            340                 345                 350
Leu Lys Ser Arg Phe Glu Ile Tyr Gly Ser Ile Ser Arg Ile Arg Ile
        355                 360                 365
His Lys Asp Gly Ile Gly Ser Val Ser Tyr Arg Thr Ala Glu Ser Ala
    370                 375                 380
Glu Ala Ala Ile Ala Gly Ser His Glu Pro Ser Phe Gly Ile Ser Ile
385                 390                 395                 400
Asp Ser Lys Lys Leu Glu Val Val Trp Ala Thr Asp Pro Leu Val Lys
                405                 410                 415
Trp Lys Glu Gly Val Thr Ala Gly Glu Gly Lys Glu Arg Thr Ser Ser
            420                 425                 430
Phe Ser Ser Lys Leu Leu Arg Pro Val Met Pro Leu Arg Lys His Gly
        435                 440                 445
Arg Ser Ser Arg Leu Ala Ser Ala Ile Val Asn Pro Arg Ser Asp Asn
    450                 455                 460
Thr Lys Gly Ile Ser Gly Asp Gly Gly Ile Ser Ser Pro Ala Thr Thr
465                 470                 475                 480
Ser Glu Val Lys Gln Arg Asn Ile Val Thr Tyr Asp Asp Ile Val
                485                 490                 495
```

```
<210>  251
<211>  1428
<212>  DNA
<213>  Arabidopsis thaliana

<400>  251
aaggccacaa cagatcttct tgttcctctc tctcaatctc tctctctaaa actcttattt      60
tcttcgtaag agatggaact agcgttgtct ctaggcgaca atactaagaa acagttctct     120
tttatggaga agaactcgaa gattaataat ccctctgtct cgtcgacatc tacttccgag     180
aaggatcttg ggttctgcat ggctttagat gttgcttttg gtggtcacag atcgttgtca     240
tcctcttcgt ctccgtcggt agaggatgag aagaagaaac cggcgcccag agcaaaaaaa     300
tctgacgaat ttagggtttc gtcttctgta gatccaccat tacagcttca gcttcacttc     360
cctaattggc tccctgagaa cagtaaaggt cgacaaggag gaagaatgcc cttaggagca     420
gctacggttg tggaggagga agaggaggag gaggaagcgg tgcctagtat gtcagtatcg     480
```

```
ccgccggata gtgtaacgtc gtcgtttcaa ttggactttg ggattaaaag ttatggttat    540
gagagaagaa gcaataagag agatattgat gatgaagtgg agagatcagc ttcaagagcc    600
agcaacgaag acaacgatga cgagaatgga tccactagga agaaacttag actctccaaa    660
gaccaatctg cttttcttga agacagcttc aaagaacaca gtacccttaa tcctgttcgt    720
gtcccattct ttacagtttt tatttattta aaatttgtct ttcttgaatt tattctattt    780
ttttagtacc aaattttagc cttaatgctt ttttttttct ttctaaaaca ttgcagaaac    840
agaagattgc attggcgaag cagttgaatc ttcgtcctcg tcaggttgaa gtctggtttc    900
aaaacagacg agccaggaca aagctgaagc aaacggaagt ggactgtgaa tacctaaaga    960
gatgctgtga gtcactaacc gaagaaaacc ggaggcttca aaaagaggtt aaagaattga   1020
gaaccttgaa gacttccaca cccttttaca tgcaacttcc ggccactact ctcactatgt   1080
gcccttcttg tgaacgtgtt gccacttcag cagcacagcc ctccacgtca gctgcccaca   1140
acctctgttt gtccacgtca tcattgattc cggttaagcc tcggccggcc aaacaagttt   1200
catgaaagca cctgcgaaat acagtttgag caaacggtcg agctagagtg gttttaaaag   1260
ttgtcttctt gtgtatatat ttattttact tttcatattt tattagagac cgctattttg   1320
aaagacgaat agattgatta tccggttagt gttttgtttt tcttagatag gaccggataa   1380
aaaacagatg gagcaaaagg gttgacatgt tttattgaat gatagagg                1428
```

<210> 252
<211> 237
<212> PRT
<213> Arabidopsis thaliana

<400> 252

```
Met Glu Leu Ala Leu Ser Leu Gly Asp Asn Thr Lys Lys Gln Phe Ser
1               5                   10                  15
Phe Met Glu Lys Asn Ser Lys Ile Asn Asn Pro Ser Val Ser Ser Thr
            20                  25                  30
Ser Thr Ser Glu Lys Asp Leu Gly Phe Cys Met Ala Leu Asp Val Ala
        35                  40                  45
Phe Gly Gly His Arg Ser Leu Ser Ser Ser Ser Ser Pro Ser Val Glu
    50                  55                  60
Asp Glu Lys Lys Lys Pro Ala Pro Arg Ala Lys Lys Ser Asp Glu Phe
65                  70                  75                  80
Arg Val Ser Ser Ser Val Asp Pro Pro Leu Gln Leu Gln Leu His Phe
                85                  90                  95
Pro Asn Trp Leu Pro Glu Asn Ser Lys Gly Arg Gln Gly Gly Arg Met
            100                 105                 110
Pro Leu Gly Ala Ala Thr Val Val Glu Glu Glu Glu Glu Glu Glu Glu
        115                 120                 125
Ala Val Pro Ser Met Ser Val Ser Pro Pro Asp Ser Val Thr Ser Ser
    130                 135                 140
Phe Gln Leu Asp Phe Gly Ile Lys Ser Tyr Gly Tyr Glu Arg Arg Ser
145                 150                 155                 160
Asn Lys Arg Asp Ile Asp Asp Glu Val Glu Arg Ser Ala Ser Arg Ala
                165                 170                 175
Ser Asn Glu Asp Asn Asp Asp Glu Asn Gly Ser Thr Arg Lys Lys Leu
            180                 185                 190
Arg Leu Ser Lys Asp Gln Ser Ala Phe Leu Glu Asp Ser Phe Lys Glu
        195                 200                 205
His Ser Thr Leu Asn Pro Val Arg Val Pro Phe Phe Thr Val Phe Ile
    210                 215                 220
Tyr Leu Lys Phe Val Phe Leu Glu Phe Ile Leu Phe Phe
225                 230                 235
```

<210> 253
<211> 1167
<212> DNA
<213> Arabidopsis thaliana

<400> 253
tgcccccagc tagtcacata ctcatgattg caaaatctct ctctctctct gcctctctat 60
atattaacct ttcttcttcc tttactttct catcttctat ctctcaaaag aaaagcagac 120
aactttattt gcaaaaacag agtttttttt tcttatcttg agaaagttca acagaagatg 180
atgttcgaga aagacgatct gggtctaagc ttaggcttga attttccaaa gaaacagatc 240
aatctcaaat caaatccatc tgtttctgtt actccttctt cttcttcttt tggattattc 300
agaagatctt catggaacga gagttttact tcttcagttc caaactcaga ttcgtcacaa 360
aaagaaacaa gaactttcat ccgaggaatc gacgtgaaca gaccaccgtc tacagcggaa 420
tacggcgacg aagacgctgg agtatcttca cctaacagta cagtctcaag ctctacaggg 480
aaaagaagcg agagagaaga agacacagat ccacaaggct caagaggaat cagtgacgat 540
gaagatggtg ataactccag gaaaaagctt agactttcca aagatcaatc tgctattctt 600
gaagagacct tcaaagatca cagtactctc aatccgaagc agaagcaagc attggctaaa 660
caattagggt tacgagcaag acaagtggaa gtttggtttc agaacagacg agcaagaaca 720
aagctgaagc aaacggaggt agactgcgag ttcttacgga gatgctgcga gaatctaacg 780
gaagagaacc gtcggctaca aaaagaagta acggaattga gagcacttaa gctctctcct 840
cagttctaca tgcacatgag cccacccact actttgacca tgtgcccttc atgtgaacac 900
gtgtcggtcc cgccaccaca acctcaggct gctacgtcag cgcaccaccg gtcgttgccg 960
gtcaatgcgt gggctcctgc gacgaggata tctcacggct tgactttgga cgctcttcgt 1020
cctaggtcct aagtcttttt acttgcaacc aaagggcatt ttggtcgttt tttaagtttc 1080
atggaccaga tatgcatgta gttgttaaca tgtatgtatt ttcttagaaa gaaagaaaaa 1140
cagattaata tttttctagc ttaaacc 1167

<210> 254
<211> 284
<212> PRT
<213> Arabidopsis thaliana

<400> 254
Met Met Phe Glu Lys Asp Asp Leu Gly Leu Ser Leu Gly Leu Asn Phe
1 5 10 15
Pro Lys Lys Gln Ile Asn Leu Lys Ser Asn Pro Ser Val Ser Val Thr
20 25 30
Pro Ser Ser Ser Ser Phe Gly Leu Phe Arg Arg Ser Ser Trp Asn Glu
35 40 45
Ser Phe Thr Ser Ser Val Pro Asn Ser Asp Ser Ser Gln Lys Glu Thr
50 55 60
Arg Thr Phe Ile Arg Gly Ile Asp Val Asn Arg Pro Pro Ser Thr Ala
65 70 75 80
Glu Tyr Gly Asp Glu Asp Ala Gly Val Ser Ser Pro Asn Ser Thr Val
85 90 95
Ser Ser Ser Thr Gly Lys Arg Ser Glu Arg Glu Glu Asp Thr Asp Pro
100 105 110
Gln Gly Ser Arg Gly Ile Ser Asp Asp Glu Asp Gly Asp Asn Ser Arg
115 120 125
Lys Lys Leu Arg Leu Ser Lys Asp Gln Ser Ala Ile Leu Glu Glu Thr
130 135 140
Phe Lys Asp His Ser Thr Leu Asn Pro Lys Gln Lys Gln Ala Leu Ala
145 150 155 160
Lys Gln Leu Gly Leu Arg Ala Arg Gln Val Glu Val Trp Phe Gln Asn
165 170 175
Arg Arg Ala Arg Thr Lys Leu Lys Gln Thr Glu Val Asp Cys Glu Phe
180 185 190
Leu Arg Arg Cys Cys Glu Asn Leu Thr Glu Glu Asn Arg Arg Leu Gln
195 200 205
Lys Glu Val Thr Glu Leu Arg Ala Leu Lys Leu Ser Pro Gln Phe Tyr
210 215 220
Met His Met Ser Pro Pro Thr Thr Leu Thr Met Cys Pro Ser Cys Glu
225 230 235 240
His Val Ser Val Pro Pro Pro Gln Pro Gln Ala Ala Thr Ser Ala His

```
            245                 250                 255
His Arg Ser Leu Pro Val Asn Ala Trp Ala Pro Ala Thr Arg Ile Ser
        260                 265                 270
His Gly Leu Thr Phe Asp Ala Leu Arg Pro Arg Ser
    275                 280

<210>  255
<211>  1194
<212>  DNA
<213>  Arabidopsis thaliana

<400>  255
atttcacatc tctctctctc tataagaacc ctagaagaag gtttctcttg tcctccatac      60
acttagcaca actgataaat cttttgaggt aaaatcagct ttagatcaag gtttttctag     120
tcatctctac tcataaagat caaagctttt gctattctca ttttctacca agagacaata     180
tcatgatgat gggtaaagag gatttgggtt taagtcttag cttgggattt gcacaaaacc     240
atcctctcca gctaaatctt aaacccactt cttcaccaat gtccaatctc cagatgtttc     300
catggaacca aacccttgtt tcttcctcag atcaacaaaa gcaacagttt cttaggaaaa     360
tcgacgtgaa cagcttgcca acaacggtgg atttggaaga ggagacagga gtttcgtctc     420
caaacagtac gatctcgagc acagtgagtg gaaagaggag gagtactgaa agagaaggta     480
cctccggtgg tggttgcgga gatgaccttg acatcactct agatagatct tcctcacgtg     540
gaacctccga tgaagaggaa gattacggag gtgagacttg taggaagaag cttagactat     600
ccaaagatca atccgcagtt ctcgaagaca ctttcaaaga gcacaatact ctcaatccca     660
aacagaagct ggctttggct aagaagctag gtttaacagc aagacaagtg gaagtgtggt     720
tccaaaacag aagagcaagg acaaagttaa agcagaccga agtggattgc gagtatttga     780
aaagatgtgt tgagaaatta acggaagaga atcggcggct cgagaaagag gcagcggaac     840
taagagcatt aaagctttca ccgcggttgt atggtcagat gagtccaccg accacacttt     900
tgatgtgtcc atcgtgtgaa cgtgtggccg gaccatcctc atctaaccac aaccagcgat     960
ctgtctcatt gagtccatgg ctccaaatgg cccatgggtc aacctttgat gtgatgcgtc    1020
ctaggtctta actttaatgc tgcttctatg ggttgtgtgt gggtcattgt actttttaga    1080
ttattgactc tcagctaatg tatccttaaa agccttttc tactttaaa tttactttaa    1140
tctaattaaa ttagttattc ttgtcttctt gataacaaac aaatttataa taat          1194

<210>  256
<211>  282
<212>  PRT
<213>  Arabidopsis thaliana

<400>  256
Met Met Met Gly Lys Glu Asp Leu Gly Leu Ser Leu Ser Leu Gly Phe
1               5                   10                  15
Ala Gln Asn His Pro Leu Gln Leu Asn Leu Lys Pro Thr Ser Ser Pro
            20                  25                  30
Met Ser Asn Leu Gln Met Phe Pro Trp Asn Gln Thr Leu Val Ser Ser
        35                  40                  45
Ser Asp Gln Gln Lys Gln Gln Phe Leu Arg Lys Ile Asp Val Asn Ser
    50                  55                  60
Leu Pro Thr Thr Val Asp Leu Glu Glu Glu Thr Gly Val Ser Ser Pro
65                  70                  75                  80
Asn Ser Thr Ile Ser Ser Thr Val Ser Gly Lys Arg Arg Ser Thr Glu
                85                  90                  95
Arg Glu Gly Thr Ser Gly Gly Gly Cys Gly Asp Asp Leu Asp Ile Thr
            100                 105                 110
Leu Asp Arg Ser Ser Ser Arg Gly Thr Ser Asp Glu Glu Glu Asp Tyr
        115                 120                 125
Gly Gly Glu Thr Cys Arg Lys Lys Leu Arg Leu Ser Lys Asp Gln Ser
    130                 135                 140
Ala Val Leu Glu Asp Thr Phe Lys Glu His Asn Thr Leu Asn Pro Lys
145                 150                 155                 160
```

```
Gln Lys Leu Ala Leu Ala Lys Lys Leu Gly Leu Thr Ala Arg Gln Val
                165                 170                 175
Glu Val Trp Phe Gln Asn Arg Arg Ala Arg Thr Lys Leu Lys Gln Thr
            180                 185                 190
Glu Val Asp Cys Glu Tyr Leu Lys Arg Cys Val Glu Lys Leu Thr Glu
        195                 200                 205
Glu Asn Arg Arg Leu Glu Lys Glu Ala Ala Glu Leu Arg Ala Leu Lys
    210                 215                 220
Leu Ser Pro Arg Leu Tyr Gly Gln Met Ser Pro Pro Thr Thr Leu Leu
225                 230                 235                 240
Met Cys Pro Ser Cys Glu Arg Val Ala Gly Pro Ser Ser Ser Asn His
                245                 250                 255
Asn Gln Arg Ser Val Ser Leu Ser Pro Trp Leu Gln Met Ala His Gly
            260                 265                 270
Ser Thr Phe Asp Val Met Arg Pro Arg Ser
        275                 280
```

<210> 257
<211> 1284
<212> DNA
<213> Arabidopsis thaliana

<400> 257

```
aataatgatg gattgtaatt agccacctga caaaatctct catcattcaa agtactatat      60
taatcccctc tcttcttcat taccatctca catctctctc tatttctctt ccacaaagag     120
tcctaacttc gagttgaaac aaacaccatt tctcatctct atctcagaaa gaacaaacca     180
tttcgtgttc tttctttctc tattctcata aggaaatata attcctgaaa ctgttgagtt     240
cttgtgaaag gaaataaaaa acatgatgat gggcaaagaa gatctaggtt tgagcctaag     300
cttagggttt tcacaaaatc acaatcctct tcagatgaat ctgaatccta actcttcatt     360
atcaaacaat ctccagagac tcccatggaa ccaaacattc gatcctacat cagatcttcg     420
caagatagac gtgaacagtt ttccatcaac ggttaactgc gaggaagaca caggagtttc     480
gtcaccaaac agtacgatct caagcaccat tagcgggaag agaagtgaga gagaaggaat     540
ctccggaacc ggcgttggct ccggcgacga tcacgacgag atcactccgg atcgagggta     600
ctcacgtgga acctcagatg aagaagaaga cgggggcgaa acgtcgagga agaagctcag     660
gttatcaaaa gatcagtctg cttttctcga agagactttc aaagaacaca acactctcaa     720
tcccaaacag aagctagctt tggctaagaa gctgaacttg acggcaagac aagtggaagt     780
gtggttccaa aacagaagag ctagaaccaa gttaaagcaa acggaggtag attgcgaata     840
cttgaaacgg tgcgtagaga agctaacgga agagaaccgg agacttcaga aagaggctat     900
ggagcttcga actctcaagc tgtctccaca attctacggt cagatgactc caccaactac     960
actcatcatg tgtccttcgt gcgagcgtgt gggtggccca tcatcatcga accatcacca    1020
caatcacagg cccgtttcta tcaatccgtg ggttgcttgt gctggtcagg tggctcatgg    1080
gctgaatttt gaagccttgc gtccacgatc gtgatttttt attttagtgg tgggaaaagg    1140
gtgttttggt atttttcgtt atcgttatat agtctatctg tgtggggtca ttgtaatttt    1200
ggatgattgg ccttctcatg aactagtcct atgtatgatg caaccttaaa aagatttaaa    1260
ttagcaaaaa ttagttacaa actt                                           1284
```

<210> 258
<211> 283
<212> PRT
<213> Arabidopsis thaliana

<400> 258

```
Met Met Met Gly Lys Glu Asp Leu Gly Leu Ser Leu Ser Leu Gly Phe
1               5                   10                  15
Ser Gln Asn His Asn Pro Leu Gln Met Asn Leu Asn Pro Asn Ser Ser
            20                  25                  30
Leu Ser Asn Asn Leu Gln Arg Leu Pro Trp Asn Gln Thr Phe Asp Pro
        35                  40                  45
Thr Ser Asp Leu Arg Lys Ile Asp Val Asn Ser Phe Pro Ser Thr Val
```

```
    50                  55                  60
Asn Cys Glu Glu Asp Thr Gly Val Ser Ser Pro Asn Ser Thr Ile Ser
65                  70                  75                  80
Ser Thr Ile Ser Gly Lys Arg Ser Glu Arg Glu Gly Ile Ser Gly Thr
                85                  90                  95
Gly Val Gly Ser Gly Asp Asp His Asp Glu Ile Thr Pro Asp Arg Gly
            100                 105                 110
Tyr Ser Arg Gly Thr Ser Asp Glu Glu Glu Asp Gly Gly Glu Thr Ser
        115                 120                 125
Arg Lys Lys Leu Arg Leu Ser Lys Asp Gln Ser Ala Phe Leu Glu Glu
    130                 135                 140
Thr Phe Lys Glu His Asn Thr Leu Asn Pro Lys Gln Lys Leu Ala Leu
145                 150                 155                 160
Ala Lys Lys Leu Asn Leu Thr Ala Arg Gln Val Glu Val Trp Phe Gln
                165                 170                 175
Asn Arg Arg Ala Arg Thr Lys Leu Lys Gln Thr Glu Val Asp Cys Glu
            180                 185                 190
Tyr Leu Lys Arg Cys Val Glu Lys Leu Thr Glu Glu Asn Arg Arg Leu
        195                 200                 205
Gln Lys Glu Ala Met Glu Leu Arg Thr Leu Lys Leu Ser Pro Gln Phe
    210                 215                 220
Tyr Gly Gln Met Thr Pro Pro Thr Thr Leu Ile Met Cys Pro Ser Cys
225                 230                 235                 240
Glu Arg Val Gly Gly Pro Ser Ser Ser Asn His His His Asn His Arg
                245                 250                 255
Pro Val Ser Ile Asn Pro Trp Val Ala Cys Ala Gly Gln Val Ala His
            260                 265                 270
Gly Leu Asn Phe Glu Ala Leu Arg Pro Arg Ser
        275                 280
```

<210> 259
<211> 957
<212> DNA
<213> Arabidopsis thaliana

<400> 259

```
atgggggaaa gagatgatgg gttgggtttg agtctaagct tgggaaatag tcaacaaaaa      60
gaaccatctc tgaggttgaa tcttatgccg ttgacaactt cttcttcttc ttcttcgttt     120
caacacatgc acaatcagaa taacaatagc catccccaga agattcataa catctcttgg     180
actcatctgt ttcaatcttc tgggattaaa cgtacaactg cagagagaaa ctccgacgcc     240
gggtcatttc taagaggttt caacgtgaac agagctcagt cttcggtggc ggtagtggac     300
ttggaagaag aagccgccgt cgtctcgtct ccaaacagcg ccgtttcgag tctgagtgga     360
aataaaaggg atcttgcggt ggcgagagga ggagatgaaa acgaggcgga gagagcttct     420
tgctcacgcg gaggggggaag cggtggtagc gacgatgaag acggcggaaa cggcgacgga     480
tcaaggaaga aactacggtt atcgaaggat caagctcttg ttctcgagga gacttttaaa     540
gaacatagca ctcttaatcc gaagcaaaag ctggctctag caaaacagtt gaatctaagg     600
gcaagacaag ttgaagtgtg gtttcagaac cgtagggcaa ggacgaagct gaaacaaacg     660
gaggttgatt gtgagtattt aaagagatgt tgcgataatc tgaccgagga gaatcgacgg     720
ctgcagaaag aagtgtcgga gctgagggcg ttgaagttgt ctccacatct ctacatgcac     780
atgactcctc ctactactct caccatgtgc ccttcttgcg aacgtgtctc ctcctctgcc     840
gccactgtga ccgctgctcc ttccactact actactccta cggtggtggg gcggccaagt     900
ccacagcgat taactccttg gactgctatt tctctccagc aaaaatcagg tcgctag        957
```

<210> 260
<211> 318
<212> PRT
<213> Arabidopsis thaliana

<400> 260

```
Met Gly Glu Arg Asp Asp Gly Leu Gly Leu Ser Leu Ser Leu Gly Asn
1               5                   10                  15
Ser Gln Gln Lys Glu Pro Ser Leu Arg Leu Asn Leu Met Pro Leu Thr
            20                  25                  30
Thr Ser Ser Ser Ser Ser Ser Phe Gln His Met His Asn Gln Asn Asn
        35                  40                  45
Asn Ser His Pro Gln Lys Ile His Asn Ile Ser Trp Thr His Leu Phe
    50                  55                  60
Gln Ser Ser Gly Ile Lys Arg Thr Thr Ala Glu Arg Asn Ser Asp Ala
65                  70                  75                  80
Gly Ser Phe Leu Arg Gly Phe Asn Val Asn Arg Ala Gln Ser Ser Val
                85                  90                  95
Ala Val Val Asp Leu Glu Glu Glu Ala Ala Val Val Ser Ser Pro Asn
            100                 105                 110
Ser Ala Val Ser Ser Leu Ser Gly Asn Lys Arg Asp Leu Ala Val Ala
        115                 120                 125
Arg Gly Gly Asp Glu Asn Glu Ala Glu Arg Ala Ser Cys Ser Arg Gly
    130                 135                 140
Gly Gly Ser Gly Gly Ser Asp Asp Glu Asp Gly Gly Asn Gly Asp Gly
145                 150                 155                 160
Ser Arg Lys Lys Leu Arg Leu Ser Lys Asp Gln Ala Leu Val Leu Glu
                165                 170                 175
Glu Thr Phe Lys Glu His Ser Thr Leu Asn Pro Lys Gln Lys Leu Ala
            180                 185                 190
Leu Ala Lys Gln Leu Asn Leu Arg Ala Arg Gln Val Glu Val Trp Phe
        195                 200                 205
Gln Asn Arg Arg Ala Arg Thr Lys Leu Lys Gln Thr Glu Val Asp Cys
    210                 215                 220
Glu Tyr Leu Lys Arg Cys Cys Asp Asn Leu Thr Glu Glu Asn Arg Arg
225                 230                 235                 240
Leu Gln Lys Glu Val Ser Glu Leu Arg Ala Leu Lys Leu Ser Pro His
                245                 250                 255
Leu Tyr Met His Met Thr Pro Pro Thr Thr Leu Thr Met Cys Pro Ser
            260                 265                 270
Cys Glu Arg Val Ser Ser Ser Ala Ala Thr Val Thr Ala Ala Pro Ser
        275                 280                 285
Thr Thr Thr Thr Pro Thr Val Val Gly Arg Pro Ser Pro Gln Arg Leu
    290                 295                 300
Thr Pro Trp Thr Ala Ile Ser Leu Gln Gln Lys Ser Gly Arg
305                 310                 315
```

```
<210>  261
<211>  1357
<212>  DNA
<213>  Arabidopsis thaliana

<400>  261
ccacaacccc atatctcttt gtctgaaaaa actcttttgc atatataaat aaatataagc    60
catatctttt gaaactgtac tgctgcacaa gtgtagaggc agtggcacaa catctttaag   120
aaagagagag agaaagagtc atttattcat ttcctcgctt aaaaatcttg agcacccaga   180
cagaattctt ctttcttctt tctggggttg agaaaaatga gtgaaagaga tgatggattg   240
gggctaagtt tgagcttgag tttaggtttt aatcaaaagg acccgtcttc gaggttaaat   300
ccaatgcctc tggcttctta tgcatcttca tcacacatgc agcatatgca gcagagcaat   360
tataaccatc ctcaaaagat tcagaacact tggattaaca tgtttcagtc atcagagaga   420
aactcggaca tgagatcgtt tctccgggga atagacgtga acagagctcc atcgacggtg   480
gtggttgacg tggaggatga aggcgccgga gtttcgtctc cgaacagcac cgtctcaagc   540
gtgatgagcg ggaagaagag cgagcgagag ctaatggctg cggcaggtgc agttggagga   600
ggtagagtag aagataatga gattgagaga gcttcttgct cgctcggcgg tggtagcgac   660
gatgaagacg gtagcgggaa cggagatgac agttcgagga agaaactccg attgtctaaa   720
```

gaacaagctt tggttcttga agaaactttt aaagaacata gtacactcaa tccgaagcaa 780
aagatggctt tggctaagca attgaatctg aggacgagac aagttgaagt gtggttccaa 840
aaccgaaggg caaggacgaa gctgaagcaa acggaagtag actgtgaata tcttaagaga 900
tgttgcgaga atctaacgga tgagaatcgg agattgcaaa aggaagtgag tgagcttagg 960
gctttaaagc tttctccaca cttatacatg cacatgaaac ctcccactac tctcacaatg 1020
tgtccttctt gcgagcgagt cgctgttacg tcatcttcgt catcggtggc tcctcctgtg 1080
atgaattcat cgtctccgat gggtccgatg agtccgtggg ctgccatgcc tctacggcaa 1140
cgacctgctg ctggttctca ttagagttta attagtctaa agataatagg tttggtgatt 1200
tgtttttatt atattgttga acggacttgg aagttctttt caagagttgg tcccatgctc 1260
ttctttcctt tgttttattc agtttctatt tatagctgaa ttctggataa tggaaaatct 1320
actaatatta ttgtccaatt attagtttgg ggaaaga 1357

<210> 262
<211> 315
<212> PRT
<213> Arabidopsis thaliana

<400> 262
Met Ser Glu Arg Asp Asp Gly Leu Gly Leu Ser Leu Ser Leu Ser Leu
1 5 10 15
Gly Phe Asn Gln Lys Asp Pro Ser Ser Arg Leu Asn Pro Met Pro Leu
20 25 30
Ala Ser Tyr Ala Ser Ser Ser His Met Gln His Met Gln Gln Ser Asn
35 40 45
Tyr Asn His Pro Gln Lys Ile Gln Asn Thr Trp Ile Asn Met Phe Gln
50 55 60
Ser Ser Glu Arg Asn Ser Asp Met Arg Ser Phe Leu Arg Gly Ile Asp
65 70 75 80
Val Asn Arg Ala Pro Ser Thr Val Val Val Asp Val Glu Asp Glu Gly
85 90 95
Ala Gly Val Ser Ser Pro Asn Ser Thr Val Ser Ser Val Met Ser Gly
100 105 110
Lys Lys Ser Glu Arg Glu Leu Met Ala Ala Ala Gly Ala Val Gly Gly
115 120 125
Gly Arg Val Glu Asp Asn Glu Ile Glu Arg Ala Ser Cys Ser Leu Gly
130 135 140
Gly Gly Ser Asp Asp Glu Asp Gly Ser Gly Asn Gly Asp Asp Ser Ser
145 150 155 160
Arg Lys Lys Leu Arg Leu Ser Lys Glu Gln Ala Leu Val Leu Glu Glu
165 170 175
Thr Phe Lys Glu His Ser Thr Leu Asn Pro Lys Gln Lys Met Ala Leu
180 185 190
Ala Lys Gln Leu Asn Leu Arg Thr Arg Gln Val Glu Val Trp Phe Gln
195 200 205
Asn Arg Arg Ala Arg Thr Lys Leu Lys Gln Thr Glu Val Asp Cys Glu
210 215 220
Tyr Leu Lys Arg Cys Cys Glu Asn Leu Thr Asp Glu Asn Arg Arg Leu
225 230 235 240
Gln Lys Glu Val Ser Glu Leu Arg Ala Leu Lys Leu Ser Pro His Leu
245 250 255
Tyr Met His Met Lys Pro Pro Thr Thr Leu Thr Met Cys Pro Ser Cys
260 265 270
Glu Arg Val Ala Val Thr Ser Ser Ser Ser Ser Val Ala Pro Pro Val
275 280 285
Met Asn Ser Ser Ser Pro Met Gly Pro Met Ser Pro Trp Ala Ala Met
290 295 300
Pro Leu Arg Gln Arg Pro Ala Ala Gly Ser His
305 310 315

<210> 263
<211> 744
<212> DNA
<213> Oryza sativa

<400> 263
atgatggaga gggccgagga cctgcgcctg agcctcagtc tcagctcgcc gcttattgct 60
cctcgtactc accatgtcgc catgctgttc cacgctcctc cagagaaaag attcctggag 120
atgccgctgc tccctgctgc gaagcggagc gaggtcgtcg cggcagaaga ggagcgcgcg 180
ggcctgcgcg gcggcggcgg cagcgacgag gaggacggtg gctgcggcat cgacggctca 240
cgcaagaagc tccggctttc caaggaccag tccgccgtgc tcgaggacag cttccgggag 300
cacccccaccc tcaaccccag gcagaaggca actttggcgc agcagctcgg gcttcggcct 360
cggcaggtcg aggtgtggtt tcagaacaga cgcgcaagga cgaagctgaa gcagacggag 420
gtggactgcg agttcctgaa gcgctgctgc gagacgctca cggaggagaa ccggaggctg 480
cagaaggagg tgcaggagct gcgagcgctc aagctcgtct cgccgcacct ctacatgaac 540
atgtccccgc ccaccacgct caccatgtgc ccctcctgcg agcgcgtctc caacaccaat 600
aacaactcca gcgccgccgc cgccgccgac cgccgcggca tcaggactac tactgccgca 660
ggcggcggca gcgtcgtcga caccgccgcc gacgggggca tcctctgcca ccgcccgatc 720
gccgtccggc cgcagcagtc atga 744

<210> 264
<211> 247
<212> PRT
<213> Oryza sativa

<400> 264
Met Met Glu Arg Ala Glu Asp Leu Arg Leu Ser Leu Ser Leu Ser Ser
1 5 10 15
Pro Leu Ile Ala Pro Arg Thr His His Val Ala Met Leu Phe His Ala
20 25 30
Pro Pro Glu Lys Arg Phe Leu Glu Met Pro Leu Leu Pro Ala Ala Lys
35 40 45
Arg Ser Glu Val Val Ala Ala Glu Glu Glu Arg Ala Gly Leu Arg Gly
50 55 60
Gly Gly Gly Ser Asp Glu Glu Asp Gly Gly Cys Gly Ile Asp Gly Ser
65 70 75 80
Arg Lys Lys Leu Arg Leu Ser Lys Asp Gln Ser Ala Val Leu Glu Asp
85 90 95
Ser Phe Arg Glu His Pro Thr Leu Asn Pro Arg Gln Lys Ala Thr Leu
100 105 110
Ala Gln Gln Leu Gly Leu Arg Pro Arg Gln Val Glu Val Trp Phe Gln
115 120 125
Asn Arg Arg Ala Arg Thr Lys Leu Lys Gln Thr Glu Val Asp Cys Glu
130 135 140
Phe Leu Lys Arg Cys Cys Glu Thr Leu Thr Glu Glu Asn Arg Arg Leu
145 150 155 160
Gln Lys Glu Val Gln Glu Leu Arg Ala Leu Lys Leu Val Ser Pro His
165 170 175
Leu Tyr Met Asn Met Ser Pro Pro Thr Thr Leu Thr Met Cys Pro Ser
180 185 190
Cys Glu Arg Val Ser Asn Thr Asn Asn Asn Ser Ser Ala Ala Ala Ala
195 200 205
Ala Asp Arg Arg Gly Ile Arg Thr Thr Thr Ala Ala Gly Gly Gly Ser
210 215 220
Val Val Asp Thr Ala Ala Asp Gly Gly Ile Leu Cys His Arg Pro Ile
225 230 235 240
Ala Val Arg Pro Gln Gln Ser
245

<210> 265
<211> 846
<212> DNA
<213> Oryza sativa

<400> 265
atgaggtcgt acatggacgg cggcggcgcg gcggcgtacg aggaggagga ggaggaggtt 60
gaggacgacg acggcggcgg cggcggcggc ggcggcggcg gcggtggggg gctcggggag 120
aagaagcggc ggctggcggc ggagcaggtg cgggcgctgg agcggagctt cgaggcggac 180
aacaagctgg acccggagcg gaaggcccgg atcgcccgcg accttcgcct ccaccctcgc 240
caggtcgccg tctggttcca gaaccgccgc gcgaggtgga agaccaagca gatcgagcgc 300
gacttcgccg ccctccgctc ccgccacgac gccctccgcc tcgagtgcga cgccctccgc 360
cgcgacaagg acgccctcgc cgccgagatc gccgacctcc gggacagggt ggacggccag 420
atgtccgtca agctggaggc cgtggccgcg gacgaacacc agccgcctcc gccgccgccg 480
ccgccgccac tggcgtataa cagcaaggtg gtggacggct cgacggacag cgactcgagc 540
gcggtgttca acgaggaggc gtcgccgtac tccggcgcgg ccatcgacca ccaccaccac 600
caaactccgg cgagctacga cacggcgggg ttcacctcct tcttcgcgcc atccaccacg 660
ctcacctcgt ccctctcctt cccttccatg ttccacgcgt catcgcattt cgatggccac 720
caagaactcc tcgtcggcgg cggcggcgcc ggcgcagtgg ccgacgccga cctcggaggc 780
gccggattct tcgccggcga cgagcacgcc ggcggcctct cctggtacgg cgccgagggt 840
tggtag 846

<210> 266
<211> 281
<212> PRT
<213> Oryza sativa

<400> 266
Met Arg Ser Tyr Met Asp Gly Gly Gly Ala Ala Ala Tyr Glu Glu Glu
1 5 10 15
Glu Glu Glu Val Glu Asp Asp Asp Gly Gly Gly Gly Gly Gly Gly Gly
20 25 30
Gly Gly Gly Gly Gly Leu Gly Glu Lys Lys Arg Arg Leu Ala Ala Glu
35 40 45
Gln Val Arg Ala Leu Glu Arg Ser Phe Glu Ala Asp Asn Lys Leu Asp
50 55 60
Pro Glu Arg Lys Ala Arg Ile Ala Arg Asp Leu Arg Leu His Pro Arg
65 70 75 80
Gln Val Ala Val Trp Phe Gln Asn Arg Arg Ala Arg Trp Lys Thr Lys
85 90 95
Gln Ile Glu Arg Asp Phe Ala Ala Leu Arg Ser Arg His Asp Ala Leu
100 105 110
Arg Leu Glu Cys Asp Ala Leu Arg Arg Asp Lys Asp Ala Leu Ala Ala
115 120 125
Glu Ile Ala Asp Leu Arg Asp Arg Val Asp Gly Gln Met Ser Val Lys
130 135 140
Leu Glu Ala Val Ala Ala Asp Glu His Gln Pro Pro Pro Pro Pro Pro
145 150 155 160
Pro Pro Pro Leu Ala Tyr Asn Ser Lys Val Val Asp Gly Ser Thr Asp
165 170 175
Ser Asp Ser Ser Ala Val Phe Asn Glu Glu Ala Ser Pro Tyr Ser Gly
180 185 190
Ala Ala Ile Asp His His His His Gln Thr Pro Ala Ser Tyr Asp Thr
195 200 205
Ala Gly Phe Thr Ser Phe Phe Ala Pro Ser Thr Thr Leu Thr Ser Ser
210 215 220
Leu Ser Phe Pro Ser Met Phe His Ala Ser Ser His Phe Asp Gly His
225 230 235 240
Gln Glu Leu Leu Val Gly Gly Gly Gly Ala Gly Ala Val Ala Asp Ala

```
                245                 250                 255
Asp Leu Gly Gly Ala Gly Phe Phe Ala Gly Asp Glu His Ala Gly Gly
            260                 265                 270
Leu Ser Trp Tyr Gly Ala Glu Gly Trp
        275                 280
```

<210> 267
<211> 918
<212> DNA
<213> Oryza sativa

<400> 267

```
atgaagaggc ccagctgcag aggctcctcc atggccatca tccatgacac ctctgatcaa      60
caagaggaca acatgaggtc gtacatggac ggcggcggcg cggcggcgta cgaggaggag     120
gaggaggagg ttgaggacga cgacggcggc ggcggcggcg gcggcggcgg cggcggtggg     180
gggctcgggg agaagaagcg gcggctggcg gcggagcagg tgcgggcgct ggagcggagc     240
ttcgaggcgg acaacaagct ggacccggag cggaaggccc ggatcgcccg cgaccttcgc     300
ctccaccctc gccaggtcgc cgtctggttc cagaaccgcc gcgcgaggtg gaagaccaag     360
cagatcgagc gcgacttcgc cgccctccgc tcccgccacg acgccctccg cctcgagtgc     420
gacgccctcc gccgcgacaa ggacgccctc gccgccgaga tcgccgacct ccgggacagg     480
gtggacggcc agatgtccgt caagctggag gccgtggccg cggacgaaca ccagccgcct     540
ccgccgccgc cgccgccgcc actggcgtat aacagcaagg tggtggacgg ctcgacggac     600
agcgactcga gcgcggtgtt caacgaggag gcgtcgccgt actccggcgc ggccatcgac     660
caccaccacc accaaactcc ggcgagctac gacacggcgg ggttcacctc cttcttcgcg     720
ccatccacca cgctcacctc gtccctctcc ttcccttcca tgttccacgc gtcatcgcat     780
ttcgatggcc accaagaact cctcgtcggc ggcggcggcg ccggcgcagt ggccgacgcc     840
gacctcggag gcgccggatt cttcgccggc gacgagcacg ccggcggcct ctcctggtac     900
ggcgccgagg gttggtag                                                   918
```

<210> 268
<211> 305
<212> PRT
<213> Oryza sativa

<400> 268

```
Met Lys Arg Pro Ser Cys Arg Gly Ser Ser Met Ala Ile Ile His Asp
1               5                   10                  15
Thr Ser Asp Gln Gln Glu Asp Asn Met Arg Ser Tyr Met Asp Gly Gly
            20                  25                  30
Gly Ala Ala Ala Tyr Glu Glu Glu Glu Glu Glu Val Glu Asp Asp Asp
        35                  40                  45
Gly Gly Gly Gly Gly Gly Gly Gly Gly Gly Gly Gly Gly Leu Gly Glu
    50                  55                  60
Lys Lys Arg Arg Leu Ala Ala Glu Gln Val Arg Ala Leu Glu Arg Ser
65                  70                  75                  80
Phe Glu Ala Asp Asn Lys Leu Asp Pro Glu Arg Lys Ala Arg Ile Ala
                85                  90                  95
Arg Asp Leu Arg Leu His Pro Arg Gln Val Ala Val Trp Phe Gln Asn
            100                 105                 110
Arg Arg Ala Arg Trp Lys Thr Lys Gln Ile Glu Arg Asp Phe Ala Ala
        115                 120                 125
Leu Arg Ser Arg His Asp Ala Leu Arg Leu Glu Cys Asp Ala Leu Arg
    130                 135                 140
Arg Asp Lys Asp Ala Leu Ala Ala Glu Ile Ala Asp Leu Arg Asp Arg
145                 150                 155                 160
Val Asp Gly Gln Met Ser Val Lys Leu Glu Ala Val Ala Ala Asp Glu
                165                 170                 175
His Gln Pro Pro Pro Pro Pro Pro Pro Pro Pro Leu Ala Tyr Asn Ser
            180                 185                 190
```

Lys Val Val Asp Gly Ser Thr Asp Ser Asp Ser Ser Ala Val Phe Asn
        195                 200                 205
Glu Glu Ala Ser Pro Tyr Ser Gly Ala Ala Ile Asp His His His His
    210                 215                 220
Gln Thr Pro Ala Ser Tyr Asp Thr Ala Gly Phe Thr Ser Phe Phe Ala
225                 230                 235                 240
Pro Ser Thr Thr Leu Thr Ser Ser Leu Ser Phe Pro Ser Met Phe His
                245                 250                 255
Ala Ser Ser His Phe Asp Gly His Gln Glu Leu Leu Val Gly Gly Gly
            260                 265                 270
Gly Ala Gly Ala Val Ala Asp Ala Asp Leu Gly Gly Ala Gly Phe Phe
        275                 280                 285
Ala Gly Asp Glu His Ala Gly Gly Leu Ser Trp Tyr Gly Ala Glu Gly
    290                 295                 300
Trp
305

<210> 269
<211> 936
<212> DNA
<213> Oryza sativa

<400> 269
atgaagaggc ccaccagcag cagccgaaaa tccaaaaaac aaggagagga cctggcgttc 60
tctgaggagg gcagcttgcc cgcggtgacc atggagcaga aagatgaagc cgagatggag 120
gaggtggacg aggaggagga ggaggaggtc gacgaagaca tggccggcgg gcacgcggcg 180
cagtcgccgt cgccgtcgtg cgggctgggc gagaagaagc ggcggctggc gctggagcag 240
gtgcgcgctc tggagcggag cttcgacacg gacaacaagc tggacccgga ccgcaaggcc 300
cgcatcgcgc gcgacctcgg cctgcagccg cgccaggtcg ccgtctggtt ccagaaccgc 360
cgcgcccggt ggaagacgaa gcagctcgag cgcgacttcg ccgccctccg cgcccgccac 420
gacgccctcc gcgccgactg cgacgccctg cgccgcgaca aggacgccct cgccgccgag 480
attcgggagc tgagggagaa gctgcccacc aagccggcgg acacggcggc atcggtgaag 540
gtagaagccg gcaatgacgc ggcggccggc gccgcggccg ccacggtgtg caaggacggc 600
tcgtcggacg acagcgactc cagcgtggtg ttcaacgacg aggcgtcgcc gtactccggc 660
gcggccttca ttggattcgg cccgtcgttc ttggtcgacg acgcgtcggc ggcaaccgtg 720
ggctgctcgt cgtcgctccc cgcgctcgag tccaaatggc acggtccgta ctccgacgac 780
tcgtgcaaag gcggcgtcta tggcttcacg gaggaatggc tcgctgcctg ctccggcgag 840
atggccggca acgacgccgc cggcttcttc tccgacgagc acgcctccaa cctcaacttc 900
ggttggtgcg cgagtggtaa cgagggttgg gaatga 936

<210> 270
<211> 311
<212> PRT
<213> Oryza sativa

<400> 270
Met Lys Arg Pro Thr Ser Ser Ser Arg Lys Ser Lys Lys Gln Gly Glu
1               5                   10                  15
Asp Leu Ala Phe Ser Glu Glu Gly Ser Leu Pro Ala Val Thr Met Glu
            20                  25                  30
Gln Lys Asp Glu Ala Glu Met Glu Glu Val Asp Glu Glu Glu Glu Glu
        35                  40                  45
Glu Val Asp Glu Asp Met Ala Gly Gly His Ala Ala Gln Ser Pro Ser
    50                  55                  60
Pro Ser Cys Gly Leu Gly Glu Lys Lys Arg Arg Leu Ala Leu Glu Gln
65                  70                  75                  80
Val Arg Ala Leu Glu Arg Ser Phe Asp Thr Asp Asn Lys Leu Asp Pro
                85                  90                  95
Asp Arg Lys Ala Arg Ile Ala Arg Asp Leu Gly Leu Gln Pro Arg Gln

```
                100                 105                 110
Val Ala Val Trp Phe Gln Asn Arg Arg Ala Arg Trp Lys Thr Lys Gln
            115                 120                 125
Leu Glu Arg Asp Phe Ala Ala Leu Arg Ala Arg His Asp Ala Leu Arg
        130                 135                 140
Ala Asp Cys Asp Ala Leu Arg Arg Asp Lys Asp Ala Leu Ala Ala Glu
145                 150                 155                 160
Ile Arg Glu Leu Arg Glu Lys Leu Pro Thr Lys Pro Ala Asp Thr Ala
                165                 170                 175
Ala Ser Val Lys Val Glu Ala Gly Asn Asp Ala Ala Ala Gly Ala Ala
            180                 185                 190
Ala Ala Thr Val Cys Lys Asp Gly Ser Ser Asp Asp Ser Asp Ser Ser
        195                 200                 205
Val Val Phe Asn Asp Glu Ala Ser Pro Tyr Ser Gly Ala Ala Phe Ile
    210                 215                 220
Gly Phe Gly Pro Ser Phe Leu Val Asp Asp Ala Ser Ala Ala Thr Val
225                 230                 235                 240
Gly Cys Ser Ser Ser Leu Pro Ala Leu Glu Ser Lys Trp His Gly Pro
                245                 250                 255
Tyr Ser Asp Asp Ser Cys Lys Gly Gly Val Tyr Gly Phe Thr Glu Glu
            260                 265                 270
Trp Leu Ala Ala Cys Ser Gly Glu Met Ala Gly Asn Asp Ala Ala Gly
        275                 280                 285
Phe Phe Ser Asp Glu His Ala Ser Asn Leu Asn Phe Gly Trp Cys Ala
    290                 295                 300
Ser Gly Asn Glu Gly Trp Glu
305                 310
```

<210> 271
<211> 810
<212> DNA
<213> Oryza sativa

<400> 271

```
atgagaagtc cagcagcgct tctcccggtg gttgcagatg gtggtggtgg tgttggggtg     60
gaggaggaga tggatgtgga cgaggacatg gcgatgtgcg gcggccgcgg cggcggcggc    120
ggggagaaga agcggcggct gagcgtggag caggtgcgcg cgctggagcg gagcttcgag    180
acggagaaca agctggagcc ggagcggaag gcgcggctgg cgcgcgacct cggcctgcag    240
ccgcgccagg tcgccgtctg gttccagaac cgccgcgcgc ggtggaagac caagcagctc    300
gagcgcgact acgccgcgct ccgccaatcc tacgacgcgc tccgcgccga ccacgacgcg    360
cttcgccgcg acaaggacgc cctcctcgcc gagatcaagg agctgaaggg gaagctcggc    420
gacgaggacg ccgcggcgag cttctcgtcg gtgaaggagg aggaggaccc ggcggcgtcc    480
gacgccgacc ccccggccac cggcgcgccg cagggctcgt ccgagagcga ctcgagcgcg    540
gtgctgaacg acgcggagat ccttccacac aagccagcgc cggcggccgc cgccgacgcc    600
gcggcctcgg aggagacgga ggcggtggtg accggcgccg cgctgctcca ccacgccgag    660
gtgttcttcc acgggcagct tctcaaggtg gacgacgacg aggcggcgtt cctaggcgac    720
gacggcgcgg cgtgcggcgg cttcttcgcc gacgagcatc tcccgtcgct gccgtggtgg    780
gccgagccca ccgagcaatg gacgacctag                                     810
```

<210> 272
<211> 269
<212> PRT
<213> Oryza sativa

<400> 272

```
Met Arg Ser Pro Ala Ala Leu Leu Pro Val Val Ala Asp Gly Gly Gly
1               5                   10                  15
Gly Val Gly Val Glu Glu Glu Met Asp Val Asp Glu Asp Met Ala Met
            20                  25                  30
```

Cys Gly Gly Arg Gly Gly Gly Gly Gly Glu Lys Lys Arg Arg Leu Ser
        35                  40                  45
Val Glu Gln Val Arg Ala Leu Glu Arg Ser Phe Glu Thr Glu Asn Lys
    50                  55                  60
Leu Glu Pro Glu Arg Lys Ala Arg Leu Ala Arg Asp Leu Gly Leu Gln
65                  70                  75                  80
Pro Arg Gln Val Ala Val Trp Phe Gln Asn Arg Arg Ala Arg Trp Lys
                85                  90                  95
Thr Lys Gln Leu Glu Arg Asp Tyr Ala Ala Leu Arg Gln Ser Tyr Asp
            100                 105                 110
Ala Leu Arg Ala Asp His Asp Ala Leu Arg Arg Asp Lys Asp Ala Leu
        115                 120                 125
Leu Ala Glu Ile Lys Glu Leu Lys Gly Lys Leu Gly Asp Glu Asp Ala
    130                 135                 140
Ala Ala Ser Phe Ser Ser Val Lys Glu Glu Glu Asp Pro Ala Ala Ser
145                 150                 155                 160
Asp Ala Asp Pro Pro Ala Thr Gly Ala Pro Gln Gly Ser Ser Glu Ser
                165                 170                 175
Asp Ser Ser Ala Val Leu Asn Asp Ala Glu Ile Leu Pro His Lys Pro
            180                 185                 190
Ala Pro Ala Ala Ala Ala Asp Ala Ala Ala Ser Glu Glu Thr Glu Ala
        195                 200                 205
Val Val Thr Gly Ala Ala Leu Leu His His Ala Glu Val Phe Phe His
    210                 215                 220
Gly Gln Leu Leu Lys Val Asp Asp Asp Glu Ala Ala Phe Leu Gly Asp
225                 230                 235                 240
Asp Gly Ala Ala Cys Gly Gly Phe Phe Ala Asp Glu His Leu Pro Ser
                245                 250                 255
Leu Pro Trp Trp Ala Glu Pro Thr Glu Gln Trp Thr Thr
            260                 265

<210> 273
<211> 834
<212> DNA
<213> Oryza sativa

<400> 273
atgaagcgac ccggcggtgc cggcggcggc ggaggcagcc catcgctcgt cacgatggct 60
aattctagtg atgatggata tggaggggtt gggatggagg cggaggggga cgtggaggag 120
gagatgatgg cgtgcggcgg cggcggggag aagaagcggc ggctgagcgt ggagcaggtt 180
cgcgcgctgg agcggagctt cgaggtggag aacaagcttg agcctgagcg gaaggcgcgg 240
ctggcgcgcg acctcggcct gcagccgcgc caggtcgccg tctggttcca gaaccgccgc 300
gcgcggtgga agaccaagca gctcgagcgc gactacgccg cgctccgcca ttcctacgac 360
tccctgcgcc tcgatcacga cgcgctccgc cgcgacaagg acgccctcct cgccgagatc 420
aaggagctga aggcgaagct cggggacgag gaggcggcgg cgagcttcac gtcggtgaag 480
gaggagccgg cggcctccga cgggccaccg gcggcgggat ttgggtcgtc cgacagcgac 540
tcaagcgcgg tgctgaacga cgtggacgcg gccggcgccg cgcccgcggc gacggacgcg 600
ctggctccgg aggcgtgcac gtttctcggc gcgccgcccg ccgcgggcgc gggcgcgggc 660
gcagcggcgg cggcgagcca cgaggaggtg ttcttccacg gcaatttcct caaggtggag 720
gaggacgaga cggggttcct cgacgacgac gagccgtgcg gcgggttctt cgccgacgat 780
cagccccccgc cgctgtcgtc gtggtgggcc gagcccacgg agcactggaa ctga 834

<210> 274
<211> 277
<212> PRT
<213> Oryza sativa

<400> 274
Met Lys Arg Pro Gly Gly Ala Gly Gly Gly Gly Gly Ser Pro Ser Leu

```
1               5                   10                  15
Val Thr Met Ala Asn Ser Ser Asp Asp Gly Tyr Gly Gly Val Gly Met
            20                  25                  30
Glu Ala Glu Gly Asp Val Glu Glu Glu Met Met Ala Cys Gly Gly Gly
        35                  40                  45
Gly Glu Lys Lys Arg Arg Leu Ser Val Glu Gln Val Arg Ala Leu Glu
    50                  55                  60
Arg Ser Phe Glu Val Glu Asn Lys Leu Glu Pro Glu Arg Lys Ala Arg
65                  70                  75                  80
Leu Ala Arg Asp Leu Gly Leu Gln Pro Arg Gln Val Ala Val Trp Phe
                85                  90                  95
Gln Asn Arg Arg Ala Arg Trp Lys Thr Lys Gln Leu Glu Arg Asp Tyr
            100                 105                 110
Ala Ala Leu Arg His Ser Tyr Asp Ser Leu Arg Leu Asp His Asp Ala
        115                 120                 125
Leu Arg Arg Asp Lys Asp Ala Leu Leu Ala Glu Ile Lys Glu Leu Lys
    130                 135                 140
Ala Lys Leu Gly Asp Glu Glu Ala Ala Ala Ser Phe Thr Ser Val Lys
145                 150                 155                 160
Glu Glu Pro Ala Ala Ser Asp Gly Pro Pro Ala Ala Gly Phe Gly Ser
                165                 170                 175
Ser Asp Ser Asp Ser Ser Ala Val Leu Asn Asp Val Asp Ala Ala Gly
            180                 185                 190
Ala Ala Pro Ala Ala Thr Asp Ala Leu Ala Pro Glu Ala Cys Thr Phe
        195                 200                 205
Leu Gly Ala Pro Pro Ala Ala Gly Ala Gly Ala Gly Ala Ala Ala Ala
    210                 215                 220
Ala Ser His Glu Glu Val Phe Phe His Gly Asn Phe Leu Lys Val Glu
225                 230                 235                 240
Glu Asp Glu Thr Gly Phe Leu Asp Asp Asp Glu Pro Cys Gly Gly Phe
                245                 250                 255
Phe Ala Asp Asp Gln Pro Pro Pro Leu Ser Ser Trp Trp Ala Glu Pro
            260                 265                 270
Thr Glu His Trp Asn
        275
```

<210> 275
<211> 375
<212> DNA
<213> Oryza sativa

<400> 275

```
atgtcatcct ctctctttct tcttcctcct ctctttcttt ctctcttctc tcccttcgg      60
ctagccgcag ggagcacggg ctggggcggt agtgggcggg gacaagagag gcggagggag    120
ctgaagcggc gccggcggcg gcacacgctc tccagcctca gcggcaagcg tggtgcgcca    180
tctgccgctg ccgccgccgt cggcggcagc gacgacgagg actccgacga cggatcccgc    240
aagaagctcc gcctctccaa ggaccaagcc gccgtcctcg aggacacctt caacaagcac    300
aacaccctca accccaagca gaaggcggcg ctggcgaggc agctgaatct gaagccgcgg    360
caggtggagg tgtag                                                     375
```

<210> 276
<211> 124
<212> PRT
<213> Oryza sativa

<400> 276

```
Met Ser Ser Ser Leu Phe Leu Leu Pro Pro Leu Phe Leu Ser Leu Phe
1               5                   10                  15
Ser Pro Leu Arg Leu Ala Ala Gly Ser Thr Gly Trp Gly Gly Ser Gly
```

```
            20                  25                  30
Arg Gly Gln Glu Arg Arg Arg Glu Leu Lys Arg Arg Arg Arg Arg His
        35                  40                  45
Thr Leu Ser Ser Leu Ser Gly Lys Arg Gly Ala Pro Ser Ala Ala Ala
    50                  55                  60
Ala Ala Val Gly Gly Ser Asp Asp Glu Asp Ser Asp Asp Gly Ser Arg
65                  70                  75                  80
Lys Lys Leu Arg Leu Ser Lys Asp Gln Ala Ala Val Leu Glu Asp Thr
                85                  90                  95
Phe Asn Lys His Asn Thr Leu Asn Pro Lys Gln Lys Ala Ala Leu Ala
            100                 105                 110
Arg Gln Leu Asn Leu Lys Pro Arg Gln Val Glu Val
        115                 120
```

<210> 277
<211> 1767
<212> DNA
<213> Oryza sativa

<400> 277

```
atgttttatt caagtccctt ttgttcatct cctttgcaga ttccatttct gacccaaatt      60
ttggctattc ctcatgatga gtttgtctca aactggtgct ctgttaatct gccagtgata     120
gaagaagacg ctaatcttga ttatgatcca tttggtgcag ctgagctggc actggcagct     180
gctggtaata agttaactga agctaaagca aactattctt gcccttttcg ccctatcagt     240
atgccttcta tagcatatgc gcagacaaga acatcatgtg tggtgaaaat aatagcaaat     300
ttgcatgttt ttgtcccaaa catatgcgaa gagcaagaaa gagacctttt tcttcagaaa     360
tttcagaagt acttggtatc agggaatcct agatcatcag ttgatcatcc agcatcagct     420
gatctcaagg ccactacagt ttgcagaaac ttgggatctt tgtctgagta tgctagatcg     480
ttaattccta ataacttgtt aaacgaggaa gatgtgcaat tgttaagtga atttgcttat     540
aagttacaaa cttggtgtaa atcacatgtt ggacagagta catcccaggc agtaaagatt     600
gatccgtcat cagaaagcaa ggaagacttt aagccactgc agcatccctt gataccaagt     660
actgttgttc cagattccag tataaataac cttccgaaga acatggaaga gcctacacca     720
acaaacatgg aagagcctgc accaacaccc tcaacaaagc aagagggaaa tgccagggat     780
gagactccta gaagcactgt cgctttaaat ggtgggttcc tgcagaattc agtcggccag     840
gacttagtcc atcttggtgt ggcgagaact agttcaggct ttctaggggg aggtaccagt     900
acaagtacag gatccctgcg ctgcaaaatg gatcttgatc ctgcatccag cagtatggac     960
catttcaaaa caccagatag aaaagaaagt ggtcttcagg atgatgagaa aggagacact    1020
cacatgtatg atgagagaca acctaagaga aggaagcgaa ccattatgaa tgataggcaa    1080
ataaacgaaa tcgagaaggc tcttattgat gagcctgaga tgcataagaa tgctgcttta    1140
ctgcaggcat ggtcagagaa gttaagtggg caggctcgg agattacgtc atctcagcta    1200
aagaattggc tgaacaacag aaaagctaag cttgctcgta ttgcgaaaga aagaggagta    1260
ctatctgagg gtgagaacgc agataagcca tctacgccag ctactcccca ccactgtgac    1320
tcctcagaaa gtgctggcga ggagagctac ttgccacctg cgagggtcat gagtgctcta    1380
ggcatatcca agggcagcag atttgtgagc ccagatggca atgagacaac atcacaggca    1440
gaattcaatc aaaatatcat gcttagccgc cctttcacaa gatcattctc atttgaacct    1500
ggccgtcttg tttcgctcat tgataatgat gggaaggagg ttggcagggg aaagatcttc    1560
caagttgagg ggagactgca agggaaggcc ctgacagata ctcgcgtttg catcgttgat    1620
gttattgagc tcaagattga gaaatggcgg gagctacctc atccctcgga agcatcagga    1680
agaacattcc aagaggcaga atcaaggaac ggtggtgtga tgagggtcgc gtgggatgtc    1740
atcagactat ctccagtggt tcagtaa                                        1767
```

<210> 278
<211> 588
<212> PRT
<213> Oryza sativa

<400> 278

```
Met Phe Tyr Ser Ser Pro Phe Cys Ser Ser Pro Leu Gln Ile Pro Phe
1               5                   10                  15
```

Leu Thr Gln Ile Leu Ala Ile Pro His Asp Glu Phe Val Ser Asn Trp
20 25 30
Cys Ser Val Asn Leu Pro Val Ile Glu Glu Asp Ala Asn Leu Asp Tyr
35 40 45
Asp Pro Phe Gly Ala Ala Glu Leu Ala Leu Ala Ala Ala Gly Asn Lys
50 55 60
Leu Thr Glu Ala Lys Ala Asn Tyr Ser Cys Pro Phe Arg Pro Ile Ser
65 70 75 80
Met Pro Ser Ile Ala Tyr Ala Gln Thr Arg Thr Ser Cys Val Val Lys
85 90 95
Ile Ile Ala Asn Leu His Val Phe Val Pro Asn Ile Cys Glu Glu Gln
100 105 110
Glu Arg Asp Leu Phe Leu Gln Lys Phe Gln Lys Tyr Leu Val Ser Gly
115 120 125
Asn Pro Arg Ser Ser Val Asp His Pro Ala Ser Ala Asp Leu Lys Ala
130 135 140
Thr Thr Val Cys Arg Asn Leu Gly Ser Leu Ser Glu Tyr Ala Arg Ser
145 150 155 160
Leu Ile Pro Asn Asn Leu Leu Asn Glu Glu Asp Val Gln Leu Leu Ser
165 170 175
Glu Phe Ala Tyr Lys Leu Gln Thr Trp Cys Lys Ser His Val Gly Gln
180 185 190
Ser Thr Ser Gln Ala Val Lys Ile Asp Pro Ser Ser Glu Ser Lys Glu
195 200 205
Asp Phe Lys Pro Leu Gln His Pro Leu Ile Pro Ser Thr Val Val Pro
210 215 220
Asp Ser Ser Ile Asn Asn Leu Pro Lys Asn Met Glu Glu Pro Thr Pro
225 230 235 240
Thr Asn Met Glu Glu Pro Ala Pro Thr Pro Ser Thr Lys Gln Glu Gly
245 250 255
Asn Ala Arg Asp Glu Thr Pro Arg Ser Thr Val Ala Leu Asn Gly Gly
260 265 270
Phe Leu Gln Asn Ser Val Gly Gln Asp Leu Val His Leu Gly Val Ala
275 280 285
Arg Thr Ser Ser Gly Phe Leu Gly Gly Gly Thr Ser Thr Ser Thr Gly
290 295 300
Ser Leu Arg Cys Lys Met Asp Leu Asp Pro Ala Ser Ser Ser Met Asp
305 310 315 320
His Phe Lys Thr Pro Asp Arg Lys Glu Ser Gly Leu Gln Asp Asp Glu
325 330 335
Lys Gly Asp Thr His Met Tyr Asp Glu Arg Gln Pro Lys Arg Arg Lys
340 345 350
Arg Thr Ile Met Asn Asp Arg Gln Ile Asn Glu Ile Glu Lys Ala Leu
355 360 365
Ile Asp Glu Pro Glu Met His Lys Asn Ala Ala Leu Leu Gln Ala Trp
370 375 380
Ser Glu Lys Leu Ser Gly Gln Gly Ser Glu Ile Thr Ser Ser Gln Leu
385 390 395 400
Lys Asn Trp Leu Asn Asn Arg Lys Ala Lys Leu Ala Arg Ile Ala Lys
405 410 415
Glu Arg Gly Val Leu Ser Glu Gly Glu Asn Ala Asp Lys Pro Ser Thr
420 425 430
Pro Ala Thr Pro His His Cys Asp Ser Ser Glu Ser Ala Gly Glu Glu
435 440 445
Ser Tyr Leu Pro Pro Ala Arg Val Met Ser Ala Leu Gly Ile Ser Lys
450 455 460
Gly Ser Arg Phe Val Ser Pro Asp Gly Asn Glu Thr Thr Ser Gln Ala
465 470 475 480
Glu Phe Asn Gln Asn Ile Met Leu Ser Arg Pro Phe Thr Arg Ser Phe

```
                485                 490                 495
Ser Phe Glu Pro Gly Arg Leu Val Ser Leu Ile Asp Asn Asp Gly Lys
            500                 505                 510
Glu Val Gly Arg Gly Lys Ile Phe Gln Val Glu Gly Arg Leu Gln Gly
        515                 520                 525
Lys Ala Leu Thr Asp Thr Arg Val Cys Ile Val Asp Val Ile Glu Leu
    530                 535                 540
Lys Ile Glu Lys Trp Arg Glu Leu Pro His Pro Ser Glu Ala Ser Gly
545                 550                 555                 560
Arg Thr Phe Gln Glu Ala Glu Ser Arg Asn Gly Gly Val Met Arg Val
                565                 570                 575
Ala Trp Asp Val Ile Arg Leu Ser Pro Val Val Gln
            580                 585


<210>  279
<211>  6
<212>  PRT
<213>  Artificial sequence

<220>
<223>  motif I

<400>  279
Arg Lys Lys Leu Arg Leu
1               5


<210>  280
<211>  24
<212>  PRT
<213>  Artificial sequence

<220>
<223>  motif II

<400>  280
Thr Lys Leu Lys Gln Thr Glu Val Asp Cys Glu Phe Leu Arg Arg Cys
1               5                   10                  15
Cys Glu Asn Leu Thr Glu Glu Asn
            20

<210>  281
<211>  10
<212>  PRT
<213>  Artificial sequence

<220>
<223>  motif III

<400>  281
Thr Leu Thr Met Cys Pro Ser Cys Glu Arg
1               5                   10


<210>  282
<211>  2193
<212>  DNA
<213>  Oryza sativa

<400>  282
aatccgaaaa gtttctgcac cgttttcacc ccctaactaa caatataggg aacgtgtgct      60
```

```
aaatataaaa tgagacctta tatatgtagc gctgataact agaactatgc aagaaaaact    120
catccaccta ctttagtggc aatcgggcta aataaaaaag agtcgctaca ctagtttcgt    180
tttccttagt aattaagtgg gaaaatgaaa tcattattgc ttagaatata cgttcacatc    240
tctgtcatga agttaaatta ttcgaggtag ccataattgt catcaaactc ttcttgaata    300
aaaaaatctt tctagctgaa ctcaatgggt aaagagagag atttttttta aaaaaataga    360
atgaagatat tctgaacgta ttggcaaaga tttaaacata taattatata attttatagt    420
ttgtgcattc gtcatatcgc acatcattaa ggacatgtct tactccatcc caatttttat    480
ttagtaatta aagacaattg acttattttt attatttatc tttttcgat tagatgcaag    540
gtacttacgc acacactttg tgctcatgtg catgtgtgag tgcacctcct caatacacgt    600
tcaactagca acacatctct aatatcactc gcctatttaa tacatttagg tagcaatatc    660
tgaattcaag cactccacca tcaccagacc acttttaata atatctaaaa tacaaaaaat    720
aattttacag aatagcatga aaagtatgaa acgaactatt taggtttttc acatacaaaa    780
aaaaaaagaa ttttgctcgt gcgcgagcgc caatctccca tattgggcac acaggcaaca    840
acagagtggc tgcccacaga acaacccaca aaaaacgatg atctaacgga ggacagcaag    900
tccgcaacaa ccttttaaca gcaggctttg cggccaggag agaggaggag aggcaaagaa    960
aaccaagcat cctcctcctc ccatctataa attcctcccc ccttttcccc tctctatata   1020
ggaggcatcc aagccaagaa gagggagagc accaaggaca cgcgactagc agaagccgag   1080
cgaccgcctt cttcgatcca tatcttccgg tcgagttctt ggtcgatctc ttccctcctc   1140
cacctcctcc tcacagggta tgtgcccttc ggttgttctt ggatttattg ttctaggttg   1200
tgtagtacgg gcgttgatgt taggaaaggg gatctgtatc tgtgatgatt cctgttcttg   1260
gatttgggat agaggggttc ttgatgttgc atgttatcgg ttcggtttga ttagtagtat   1320
ggttttcaat cgtctggaga gctctatgga aatgaaatgg tttagggtac ggaatcttgc   1380
gattttgtga gtaccttttg tttgaggtaa aatcagagca ccggtgattt tgcttggtgt   1440
aataaaagta cggttgtttg gtcctcgatt ctggtagtga tgcttctcga tttgacgaag   1500
ctatcctttg tttattccct attgaacaaa aataatccaa ctttgaagac ggtcccgttg   1560
atgagattga atgattgatt cttaagcctg tccaaaattt cgcagctggc ttgtttagat   1620
acagtagtcc ccatcacgaa attcatggaa acagttataa tcctcaggaa caggggattc   1680
cctgttcttc cgatttgctt tagtcccaga atttttttc ccaaatatct taaaaagtca    1740
ctttctggtt cagttcaatg aattgattgc tacaaataat gcttttatag cgttatccta   1800
gctgtagttc agttaatagg taatacccct atagtttagt caggagaaga acttatccga   1860
tttctgatct ccatttttaa ttatatgaaa tgaactgtag cataagcagt attcatttgg   1920
attattttt ttattagctc tcacccottc attattctga gctgaaagtc tggcatgaac    1980
tgtcctcaat tttgttttca aattcacatc gattatctat gcattatcct cttgtatcta   2040
cctgtagaag tttctttttg gttattcctt gactgcttga ttacagaaag aaatttatga   2100
agctgtaatc gggatagtta tactgcttgt tcttatgatt catttccttt gtgcagttct   2160
tggtgtagct tgccactttc accagcaaag ttc                                2193
```

<210> 283
<211> 58
<212> DNA
<213> Artificial sequence

<220>
<223> primer: p01294

<400> 283

```
ggggacaagt ttgtacaaaa aagcaggctt cacaatgatg ttcgagaaag acgatctg      58
```

<210> 284
<211> 52
<212> DNA
<213> Artificial sequence

<220>
<223> primer: p00402

<400> 284

```
ggggaccact ttgtacaaga aagctgggtt taggacctag gacgaagagc gt            52
```

<210> 285
<211> 622
<212> DNA
<213> Arabidopsis thaliana

<400> 285
atgagcagac ccggagattg gaactgcagg tcatgcagcc atctcaactt ccagcgccgt 60
gactcttgcc agcgatgcgg tgactctcgt tccggccccg gtggagttgg tggcttagac 120
tttggtaatt tcggtggcag agccatgtct gctttcggat tcaccaccgg ctccgacgtt 180
cgtcccggtg attggtactg caccgtggga aactgcggga cacacaactt cgccagtcgc 240
tccacctgct tcaaatgcgg cactttcaag gacgagaccg gcgctggagg cggaggtggt 300
ggcatcggcg gtccggccat gtttgacgcc gacattatgc ggtctagagt ccccggtaac 360
ggtggtcgct ctagctggaa atccggcgac tggatttgca ctaggattgg ttgcaatgag 420
cataactttg caagcagaat ggaatgcttc aggtgcaatg caccaaggga cttcagcaac 480
agaacctctt tctaagttat acaaactgct tttgaagtag ccttgtctac ccagctttct 540
tgtacaaagt tggcattata agaaagcatt gcttatcaat ttgttgcaac gaacaggtca 600
ctatcagtca aaataaaatc at 622

<210> 286
<211> 164
<212> PRT
<213> Arabidopsis thaliana

<400> 286
Met Ser Arg Pro Gly Asp Trp Asn Cys Arg Ser Cys Ser His Leu Asn
1 5 10 15
Phe Gln Arg Arg Asp Ser Cys Gln Arg Cys Gly Asp Ser Arg Ser Gly
20 25 30
Pro Gly Gly Val Gly Gly Leu Asp Phe Gly Asn Phe Gly Gly Arg Ala
35 40 45
Met Ser Ala Phe Gly Phe Thr Thr Gly Ser Asp Val Arg Pro Gly Asp
50 55 60
Trp Tyr Cys Thr Val Gly Asn Cys Gly Thr His Asn Phe Ala Ser Arg
65 70 75 80
Ser Thr Cys Phe Lys Cys Gly Thr Phe Lys Asp Glu Thr Gly Ala Gly
85 90 95
Gly Gly Gly Gly Gly Ile Gly Gly Pro Ala Met Phe Asp Ala Asp Ile
100 105 110
Met Arg Ser Arg Val Pro Gly Asn Gly Gly Arg Ser Ser Trp Lys Ser
115 120 125
Gly Asp Trp Ile Cys Thr Arg Ile Gly Cys Asn Glu His Asn Phe Ala
130 135 140
Ser Arg Met Glu Cys Phe Arg Cys Asn Ala Pro Arg Asp Phe Ser Asn
145 150 155 160
Arg Thr Ser Phe

<210> 287
<211> 717
<212> DNA
<213> Gossypium hirsutum

<400> 287
atgagcaggc caggagattg gaactgcagg tcatgccaac acctcaactt ccaaaggagg 60
gacaactgcc aacgttgcgg tgaatctcga tacggtgtta gagtcggctc gacattcggg 120
tttaccgctg gctcggacgt tcgacctggt gactggtatt gcacggctgg aaactgcggc 180
acccacaatt tcgccagccg gtctacttgt ttcaattgcg gcgcgttcaa ggacgagtcg 240
gctggaggtt tcgacttgga catgtctcga tcaagagggt tcggaggtaa ccgatccggc 300
tggaaatcag ggattggat atgtaccagg ttagggtgca atgaacataa ttttgctagc 360

agaatggaat gtttcagatg cagtgctcca agagaattca acaatagaac ttcatattaa 420
atcacatgca tgctactata tccatttttg ggtgttttga ggcaattaat aggagattat 480
aatgagagag tgttactggc tttgatgatg aacaccacaa gcattttgtc atgtttcttt 540
tataagattc atggcaatgt agtacttttt cttgtgatga ttatttatgg tttcaattct 600
atggttttat tgtcttttat tttagagagt tttatttata tttttgtaat ggtgctttgg 660
ctttattaaa agaagatgat tctcttctgt cttttcaaaa aaaaaaaaaa aaaaaaa 717

<210> 288
<211> 139
<212> PRT
<213> Gossypium hirsutum

<400> 288
Met Ser Arg Pro Gly Asp Trp Asn Cys Arg Ser Cys Gln His Leu Asn
1 5 10 15
Phe Gln Arg Arg Asp Asn Cys Gln Arg Cys Gly Glu Ser Arg Tyr Gly
20 25 30
Val Arg Val Gly Ser Thr Phe Gly Phe Thr Ala Gly Ser Asp Val Arg
35 40 45
Pro Gly Asp Trp Tyr Cys Thr Ala Gly Asn Cys Gly Thr His Asn Phe
50 55 60
Ala Ser Arg Ser Thr Cys Phe Asn Cys Gly Ala Phe Lys Asp Glu Ser
65 70 75 80
Ala Gly Gly Phe Asp Leu Asp Met Ser Arg Ser Arg Gly Phe Gly Gly
85 90 95
Asn Arg Ser Gly Trp Lys Ser Gly Asp Trp Ile Cys Thr Arg Leu Gly
100 105 110
Cys Asn Glu His Asn Phe Ala Ser Arg Met Glu Cys Phe Arg Cys Ser
115 120 125
Ala Pro Arg Glu Phe Asn Asn Arg Thr Ser Tyr
130 135

<210> 289
<211> 937
<212> DNA
<213> Zea mays

<400> 289
cgggcacgac cagcaacaca acagccgagc tttgcttagg caagatgaac aggaagccag 60
gagactggga ctgcagggcg tgccagcacc tcaacttcag ccgccgagac atatgccagc 120
gctgcagcga gccacgtgga gttgctgatc gtggcagtgg cggcggcgga ggaggcgact 180
acgccagctt cggtggccgc ggtggctcct ccttcggcgg cggctttggc gctgctggct 240
ccgacgtccg ccctggtgac tggtactgct cctgcggcgc gcacaacttc gccagccgct 300
ccagctgctt caagtgctcc gcctacaagg aggaggccgc tgtgaacagt ggcgctggcg 360
gctttgatgg cgacatgtca cgctcacggg gctacggctt cggcagcggt gctgctgctg 420
ctgctggtgc tggcgctgcc cgtactacca accgccccgg ttggaagtcc ggagactgga 480
tctgcaccag atccggatgc aacgagcaca acttcgccag caggatggag tgcttcaggt 540
gcaacgcacc gcgggactct ggcactgagg tgtaggatcg agcaagttaa aaagtctgca 600
gcgccgaaga aagcgacgac aagaggagtc ctcatcacgt cgtaacgtaa gagagagagt 660
agtggatttg caacaaaaaa aaaaaaaaga cggccgcgat gctctgttac tagctagcta 720
gttttgttca accacccatg ccgtctcttc tcttttatta gatttggttt ggttctcata 780
gccctttaat taccatttgg gacctatgtt tgctgttctg tttcccgttc gtctcctccg 840
catgtttgcg cttggatcga gtcttgtgat gtaacccccc aaaaaacgct tgcttaacta 900
gtactgttgc ttcttaataa aaaaaaaaaa aaaaaaa 937

<210> 290
<211> 176
<212> PRT
<213> Zea mays

<400> 290

Met Asn Arg Lys Pro Gly Asp Trp Asp Cys Arg Ala Cys Gln His Leu
1 5 10 15
Asn Phe Ser Arg Arg Asp Ile Cys Gln Arg Cys Ser Glu Pro Arg Gly
20 25 30
Val Ala Asp Arg Gly Ser Gly Gly Gly Gly Gly Gly Asp Tyr Ala Ser
35 40 45
Phe Gly Gly Arg Gly Gly Ser Ser Phe Gly Gly Gly Phe Gly Ala Ala
50 55 60
Gly Ser Asp Val Arg Pro Gly Asp Trp Tyr Cys Ser Cys Gly Ala His
65 70 75 80
Asn Phe Ala Ser Arg Ser Ser Cys Phe Lys Cys Ser Ala Tyr Lys Glu
85 90 95
Glu Ala Ala Val Asn Ser Gly Ala Gly Gly Phe Asp Gly Asp Met Ser
100 105 110
Arg Ser Arg Gly Tyr Gly Phe Gly Ser Gly Ala Ala Ala Ala Ala Gly
115 120 125
Ala Gly Ala Ala Arg Thr Thr Asn Arg Pro Gly Trp Lys Ser Gly Asp
130 135 140
Trp Ile Cys Thr Arg Ser Gly Cys Asn Glu His Asn Phe Ala Ser Arg
145 150 155 160
Met Glu Cys Phe Arg Cys Asn Ala Pro Arg Asp Ser Gly Thr Glu Val
165 170 175

<210> 291
<211> 501
<212> DNA
<213> Oryza sativa

<400> 291

```
atgaacagga agccaggaga ctgggactgc agggcgtgcc agcacctcaa cttcagccgc      60
cgggacctat gccagcgctg cggcgagccg cgtggcgccg ctgatcgcgg cagcggtggt     120
ggcggtgact acgccaactt cggcggccgt ggtggttcct ccttcggtgg aggctttggc     180
actggctctg atgtccgccc aggtgactgg tactgcaact gcggcgcgca caacttcgcc     240
agccgctcca gctgcttcaa gtgcgctgct ttcaaggacg atgctgccgt caacagtggc     300
ggcgctggtg cctttgatgg tggggacatg tcgcgctcgc ggggctacgg cttcggcagc     360
ggcgccgtcc gcgccagccg ccctggctgg aagtctggcg actggatttg caccaggtct     420
ggatgcaatg agcacaactt cgccagcagg atggagtgct tcaggtgcaa cgcaccgcgg     480
gactccggca ctgaggtgta a                                               501
```

<210> 292
<211> 166
<212> PRT
<213> Oryza sativa

<400> 292

Met Asn Arg Lys Pro Gly Asp Trp Asp Cys Arg Ala Cys Gln His Leu
1 5 10 15
Asn Phe Ser Arg Arg Asp Leu Cys Gln Arg Cys Gly Glu Pro Arg Gly
20 25 30
Ala Ala Asp Arg Gly Ser Gly Gly Gly Gly Asp Tyr Ala Asn Phe Gly
35 40 45
Gly Arg Gly Gly Ser Ser Phe Gly Gly Gly Phe Gly Thr Gly Ser Asp
50 55 60
Val Arg Pro Gly Asp Trp Tyr Cys Asn Cys Gly Ala His Asn Phe Ala
65 70 75 80
Ser Arg Ser Ser Cys Phe Lys Cys Ala Ala Phe Lys Asp Asp Ala Ala
85 90 95

```
Val Asn Ser Gly Gly Ala Gly Ala Phe Asp Gly Gly Asp Met Ser Arg
            100                 105                 110
Ser Arg Gly Tyr Gly Phe Gly Ser Gly Ala Val Arg Ala Ser Arg Pro
        115                 120                 125
Gly Trp Lys Ser Gly Asp Trp Ile Cys Thr Arg Ser Gly Cys Asn Glu
    130                 135                 140
His Asn Phe Ala Ser Arg Met Glu Cys Phe Arg Cys Asn Ala Pro Arg
145                 150                 155                 160
Asp Ser Gly Thr Glu Val
                165
```

<210> 293
<211> 859
<212> DNA
<213> Arabidopsis thaliana

<400> 293

```
ataagctctt acactcattt caactctctt tttcgtttcc attaccctca gaagaagatg      60
aataggccgg gagattggaa ctgcagattg tgtagccacc tcaacttcca gaggagggat     120
tcatgccaac gttgtagaga gcctagaccg ggcgggatca gtaccgattt actcagcggt     180
tttggtggcc gtccggttag tagctccttc ggtttcaaca ccgggcccga tgtgcgaccc     240
ggggattggt attgcaacct tggggattgt gggacacata attttgccaa taggtccagt     300
tgtttcaagt gtggtgccgc aaaagatgag ttttcatgct caagtgctgc tgcaacaacc     360
gggtttatgg acatgaatgt tggtccgaga cgtggccttt ttggttttgg cggcagcagt     420
agtggtggtg gtggtacggg ccgttctcct tggaaatctg gagattggat ttgcccaagg     480
tcaggctgta acgaacataa cttcgcaagc aggtcagagt gtttcaggtg taacgcacca     540
aaggaacttg ccaccgaacc accctattag tcatttagtc ctcctacctt cttcatcatt     600
tccaatactc tggaagcatt agaggagagc agaaaggtga agaatcgaag caagataccg     660
accgccctta atctcttgat ttgtttaatt tcttagattt gactcgtttt atatctcgta     720
gtagtcagac ctatgttaga atgtaagcat gtcgagagtt ttccgaagca ttttgttctg     780
atatcggtct cctagctagt atgtaagttt gtttgaatgt attcttattt ctgataaagt     840
tgtttccttc tgattccgc                                                  859
```

<210> 294
<211> 170
<212> PRT
<213> Arabidopsis thaliana

<400> 294

```
Met Asn Arg Pro Gly Asp Trp Asn Cys Arg Leu Cys Ser His Leu Asn
1               5                   10                  15
Phe Gln Arg Arg Asp Ser Cys Gln Arg Cys Arg Glu Pro Arg Pro Gly
            20                  25                  30
Gly Ile Ser Thr Asp Leu Leu Ser Gly Phe Gly Gly Arg Pro Val Ser
        35                  40                  45
Ser Ser Phe Gly Phe Asn Thr Gly Pro Asp Val Arg Pro Gly Asp Trp
    50                  55                  60
Tyr Cys Asn Leu Gly Asp Cys Gly Thr His Asn Phe Ala Asn Arg Ser
65                  70                  75                  80
Ser Cys Phe Lys Cys Gly Ala Ala Lys Asp Glu Phe Ser Cys Ser Ser
                85                  90                  95
Ala Ala Ala Thr Thr Gly Phe Met Asp Met Asn Val Gly Pro Arg Arg
            100                 105                 110
Gly Leu Phe Gly Phe Gly Gly Ser Ser Ser Gly Gly Gly Gly Thr Gly
        115                 120                 125
Arg Ser Pro Trp Lys Ser Gly Asp Trp Ile Cys Pro Arg Ser Gly Cys
    130                 135                 140
Asn Glu His Asn Phe Ala Ser Arg Ser Glu Cys Phe Arg Cys Asn Ala
145                 150                 155                 160
```

Pro Lys Glu Leu Ala Thr Glu Pro Pro Tyr
165 170

<210> 295
<211> 834
<212> DNA
<213> Oryza sativa

<400> 295
cctagtacta agaacagcaa ccacctctga aagctttaca agttcgcagg ggcttaactg 60
cgaatgaaca tccagaggaa gccaggagac tggaactgca aatcgtgcca gcatctcaac 120
ttcagccgcc gggactactg ccagcgctgc catacccccac gccaggacct gccgcttggc 180
gatggttatg tcccaggtgg tgtgctgtcc tccctggaca ttcgcccggg cgactggtac 240
tgcaactgcg gctatcacaa ctttgctagc cgagcaagct gcttcaaatg tggcgccatt 300
gtgaaggacc ttccagcagg ccaaggtggt ggtgttgcca acggtgactt tgcccgtgcc 360
ctcgacagca gcgcagttcg tgctgggtgg aaggcgggtg actggatttg cacaaggcct 420
ggttgcaacg tccacaactt tgcaagtagg attgagtgct ataggtgcaa tgcacctagg 480
gaagcaggta atgtgaagta agaaaagact gacgcgacca aagatcgtga gacgtgacga 540
gctggccgag gatgaataaa gtgactgctg tcagttgtca cattcacagg ctgcgatcca 600
gaaactatgg atgggactat gtagtaacgt gctgatatat tattgctaag tgttactact 660
gcatggttgc aagggtggta gaagtacctt agcttccaag atcgttgtaa tgtgtgagtt 720
taattttggc gttttaagta ataagtctag tgattgcagg attgtacggg gtaatgtact 780
tgcattatcc attataatct taagcatcca atataattat gcaaaaaaaa aaaa 834

<210> 296
<211> 145
<212> PRT
<213> Oryza sativa

<400> 296
Met Asn Ile Gln Arg Lys Pro Gly Asp Trp Asn Cys Lys Ser Cys Gln
1 5 10 15
His Leu Asn Phe Ser Arg Arg Asp Tyr Cys Gln Arg Cys His Thr Pro
20 25 30
Arg Gln Asp Leu Pro Leu Gly Asp Gly Tyr Val Pro Gly Gly Val Leu
35 40 45
Ser Ser Leu Asp Ile Arg Pro Gly Asp Trp Tyr Cys Asn Cys Gly Tyr
50 55 60
His Asn Phe Ala Ser Arg Ala Ser Cys Phe Lys Cys Gly Ala Ile Val
65 70 75 80
Lys Asp Leu Pro Ala Gly Gln Gly Gly Gly Val Ala Asn Gly Asp Phe
85 90 95
Ala Arg Ala Leu Asp Ser Ser Ala Val Arg Ala Gly Trp Lys Ala Gly
100 105 110
Asp Trp Ile Cys Thr Arg Pro Gly Cys Asn Val His Asn Phe Ala Ser
115 120 125
Arg Ile Glu Cys Tyr Arg Cys Asn Ala Pro Arg Glu Ala Gly Asn Val
130 135 140
Lys
145

<210> 297
<211> 958
<212> DNA
<213> Oryza sativa

<400> 297
aagcctccat ccatccatcc attcatcaga gctcaagctc aagcaagcaa gcttgctagc 60
tagctgctga gcagcattcc agtctgctcc agctagctca gctctctctg ctctgctctt 120

tgctcgataa cgaccatcat cttcttcttc ttcttcttct tcttcttctt cttcttcttg 180
tacattctat ttgctcgata gatagataga tagatagata ggtagataga gatggagacg 240
aaggcggcgg cgatggcgat gaggaagccg ggggactgga gctgcaggtc gtgccagtac 300
gtgaacttct gcaagaggga ggcgtgccag cggtgcgggg aggcgaagct cggggtggag 360
cggacggact acgccgccat gggcggcggg tgggaggtga agcccggcga ctggtgctgc 420
cgctgctgcg ccgtcaacaa ctacgccagc cgcggcagct gcttcaagtg cggcgccgcc 480
aagaacgact ccgccgccgc cgtcgcccag ggctggggct tctccgtcgc ctcccaggcc 540
ggctggaaga acggcgactg gatctgcccc agaatggaat gcaacgtgca gaactacgct 600
aacagaaccg agtgcttccg gtgcaatttc cccagatact acgttgattg atctgacata 660
tatgatatat gccttgcact gagaagaaat gaaagggaaa taaaatttac gagatcgaag 720
atcgacggac aagattgatc ggccaccggt catgcccttc agggtttct tttttctatt 780
ttttttaga ataaccctcg ataacctgag ggtttttttt catttgtaag agcggtaacg 840
gtactaaaaa aacacaaaaa cggcacaaaa ttgtggctga tgactgatca gtcttcctct 900
tttttttccc aataaaaggc agataattaa gaaagcaaaa ttattaaaaa aaaaaaaa 958

<210> 298
<211> 139
<212> PRT
<213> Oryza sativa

<400> 298
Met Glu Thr Lys Ala Ala Ala Met Ala Met Arg Lys Pro Gly Asp Trp
1 5 10 15
Ser Cys Arg Ser Cys Gln Tyr Val Asn Phe Cys Lys Arg Glu Ala Cys
20 25 30
Gln Arg Cys Gly Glu Ala Lys Leu Gly Val Glu Arg Thr Asp Tyr Ala
35 40 45
Ala Met Gly Gly Gly Trp Glu Val Lys Pro Gly Asp Trp Cys Cys Arg
50 55 60
Cys Cys Ala Val Asn Asn Tyr Ala Ser Arg Gly Ser Cys Phe Lys Cys
65 70 75 80
Gly Ala Ala Lys Asn Asp Ser Ala Ala Ala Val Ala Gln Gly Trp Gly
85 90 95
Phe Ser Val Ala Ser Gln Ala Gly Trp Lys Asn Gly Asp Trp Ile Cys
100 105 110
Pro Arg Met Glu Cys Asn Val Gln Asn Tyr Ala Asn Arg Thr Glu Cys
115 120 125
Phe Arg Cys Asn Phe Pro Arg Tyr Tyr Val Asp
130 135

<210> 299
<211> 519
<212> DNA
<213> Beta vulgaris

<400> 299
atgagtaggc caggtgattg gaattgtagg tcatgcagcc acttgaactt ccaaaggagg 60
gactcctgcc agcgctgtgg ggacgtacgt cctgacggcc gaggcggcgg aggggggagga 120
ggagactttg gcagtagctt tggagggagg tcaggtgggt ccccctttgg tgggggtttt 180
gcagggcccg atgttaggcc cggtgattgg tattgtagca ttggcaactg tgggggcccac 240
aactttgcca gcaggtctag ctgcttcaag tgtggggcct acaaagagga agctggctgt 300
ggtgatagca tgggccgttc acgtggagga ttttcctttg gtggcatcgg cggtggcggt 360
agcggtgccg ctaccggccg ctcaggctgg aaatccggtg actggatttg cactaggtcg 420
ggttgcaacg agcataactt cgctagtagg actgagtgct tcagatgcag ggaaccaagg 480
gactccggta atgcaatgct aaaggaagta ccatgctga 519

<210> 300
<211> 172
<212> PRT

<213> Beta vulgaris

<400> 300

```
Met Ser Arg Pro Gly Asp Trp Asn Cys Arg Ser Cys Ser His Leu Asn
1               5                   10                  15
Phe Gln Arg Arg Asp Ser Cys Gln Arg Cys Gly Asp Val Arg Pro Asp
            20                  25                  30
Gly Arg Gly Gly Gly Gly Gly Gly Gly Asp Phe Gly Ser Ser Phe Gly
        35                  40                  45
Gly Arg Ser Gly Gly Ser Pro Phe Gly Gly Gly Phe Ala Gly Pro Asp
    50                  55                  60
Val Arg Pro Gly Asp Trp Tyr Cys Ser Ile Gly Asn Cys Gly Ala His
65                  70                  75                  80
Asn Phe Ala Ser Arg Ser Ser Cys Phe Lys Cys Gly Ala Tyr Lys Glu
                85                  90                  95
Glu Ala Gly Cys Gly Asp Ser Met Gly Arg Ser Arg Gly Gly Phe Ser
            100                 105                 110
Phe Gly Gly Ile Gly Gly Gly Gly Ser Gly Ala Ala Thr Gly Arg Ser
        115                 120                 125
Gly Trp Lys Ser Gly Asp Trp Ile Cys Thr Arg Ser Gly Cys Asn Glu
    130                 135                 140
His Asn Phe Ala Ser Arg Thr Glu Cys Phe Arg Cys Arg Glu Pro Arg
145                 150                 155                 160
Asp Ser Gly Asn Ala Met Leu Lys Glu Val Pro Cys
                165                 170
```

<210> 301
<211> 807
<212> DNA
<213> Arabidopsis thaliana

<400> 301

```
atgggagacg gaagagaagg agactgggaa tgtttaggat gcagaaacag gaattatgcg      60
tttagatcat tctgtaacag atgcaagcag cctcgtctta tcatggataa taatacttct     120
ccaaactcta agtggcttcc tcgtatcggc gattggatct gcactggttg tactaacaac     180
aattatgcat cacgagaaaa gtgcaagaaa tgtgggcaat ctaaggaagt agcagcattg     240
tcagcacttg ctatccctgg agcttctctt caaactcatc tccattactt cacccgtgga     300
cctgagtcac atgatcaacc tggttcttta ctcgcattct ctaacgctac aaatcaagct     360
tcggttcata aagaatggag gagtggtgac tggatttgca gatgtggttt tcacaattat     420
tcctctcgta tacagtgcaa aaagtgcaat gaaatagctc cactagccct tggtacaaag     480
agattagcat cagaagcttt ggctcatgaa tgggatagca aaagactgaa tcaaggatat     540
acaagcatgc aaacacagtc agcgatatat gcatcttttc ctggtatgag cctaggaagg     600
gtctcaaatt ggcaacttcc tctcccgttt ctacaacaac actcaacacc tgctttactt     660
ggaatgggag tgaaacaatg gcgtgatggc gactggatgt gtacaaattg caagaatcac     720
aattatgcat cacgagcaga gtgcaatagg tgcaagacta cacgagatat ccttgatcag     780
gacataactc caacagaaca atcttga                                         807
```

<210> 302
<211> 268
<212> PRT
<213> Arabidopsis thaliana

<400> 302

```
Met Gly Asp Gly Arg Glu Gly Asp Trp Glu Cys Leu Gly Cys Arg Asn
1               5                   10                  15
Arg Asn Tyr Ala Phe Arg Ser Phe Cys Asn Arg Cys Lys Gln Pro Arg
            20                  25                  30
Leu Ile Met Asp Asn Asn Thr Ser Pro Asn Ser Lys Trp Leu Pro Arg
        35                  40                  45
```

```
Ile Gly Asp Trp Ile Cys Thr Gly Cys Thr Asn Asn Asn Tyr Ala Ser
    50                  55                  60
Arg Glu Lys Cys Lys Lys Cys Gly Gln Ser Lys Glu Val Ala Ala Leu
65                  70                  75                  80
Ser Ala Leu Ala Ile Pro Gly Ala Ser Leu Gln Thr His Leu His Tyr
                85                  90                  95
Phe Thr Arg Gly Pro Glu Ser His Asp Gln Pro Gly Ser Leu Leu Ala
            100                 105                 110
Phe Ser Asn Ala Thr Asn Gln Ala Ser Val His Lys Glu Trp Arg Ser
        115                 120                 125
Gly Asp Trp Ile Cys Arg Cys Gly Phe His Asn Tyr Ser Ser Arg Ile
    130                 135                 140
Gln Cys Lys Lys Cys Asn Glu Ile Ala Pro Leu Ala Leu Gly Thr Lys
145                 150                 155                 160
Arg Leu Ala Ser Glu Ala Leu Ala His Glu Trp Asp Ser Lys Arg Leu
                165                 170                 175
Asn Gln Gly Tyr Thr Ser Met Gln Thr Gln Ser Ala Ile Tyr Ala Ser
            180                 185                 190
Phe Pro Gly Met Ser Leu Gly Arg Val Ser Asn Trp Gln Leu Pro Leu
        195                 200                 205
Pro Phe Leu Gln Gln His Ser Thr Pro Ala Leu Leu Gly Met Gly Val
    210                 215                 220
Lys Gln Trp Arg Asp Gly Asp Trp Met Cys Thr Asn Cys Lys Asn His
225                 230                 235                 240
Asn Tyr Ala Ser Arg Ala Glu Cys Asn Arg Cys Lys Thr Thr Arg Asp
                245                 250                 255
Ile Leu Asp Gln Asp Ile Thr Pro Thr Glu Gln Ser
            260                 265
```

<210> 303
<211> 1264
<212> DNA
<213> Arabidopsis thaliana

<400> 303

```
ctatttctcc taccgtcgaa ctaaatccta gggcacaggt gaaatcgccg agtgacgtct      60
agccaccgca ccgcctcctt ctccggttat ctcgctacta atcagactat tccacagcta     120
tgtcacaggt agacaacaga aattcatcag cagccaagcg tgctagaact gacgggggc      180
gtagagaaga tgattggatc tgcccaagtt gtggcaatgt caacttttca ttcaggacaa     240
cttgcaatat gcgtaattgc actcagccta gacctgcaga tcataatgga aagtctgctc     300
ccaaacctat gcaacatcaa caaggtttct catcacccgg ggcatactta ggatctgggg     360
gtccccctcc agtatatatg ggcgggtcac catatggatc tcctctcttt aatggatcat     420
ctatgcctcc ttatgacgtc ccattttctg gggttcgcc ttaccatttt aactataata      480
gccgaatgcc tgccggagct cattacagac cattacatat gtctggacca ccaccatacc     540
atggcggatc tatgatggga agtggtggta tgtatggaat gcctccacca atagacaggt     600
atggccttgg tatggcaatg ggtcctggtt ctgccgctgc catgatgcca agaccaaggt     660
tttacccaga tgaaaaatca caaaagagag attcaactcg cgataatgat tggacatgtc     720
cgaattgtgg taatgtaaac ttctcattca gaactgtatg taacatgagg aagtgcaaca     780
ctccaaagcc tggttctcag cagggtggaa gctcagataa aatatccaaa caaaatgcac     840
cggaagggag ctggaagtgt gataactgtg gaaatataaa ctacccattc aggagcaaat     900
gcaacaggca aaactgtgga gctgataagc ctggggatcg gtcgaatgga tctccgtccc     960
gtgcaccaga agagaacgat cagtgagtcg tagacatgtt tgggggttga gaagggtgag    1020
tctaatccag cgggtgtcgt aatgtcagtc aatgtctcgt caggtcaggt cgtccatgtt    1080
gctgcgtcgt cagtcaagtt gcttctatgc atttgtctct ttacttccct tgcgtggttg    1140
tggattgtat taataatgca aaaattgttt atttactttg tcgtcctcgt ggatcttgtc    1200
ttgctataga atcctcctcc aggtccatac tgttttacat cctaatctta agtaagttgg    1260
cacc                                                                  1264
```

<210> 304

<211> 288
<212> PRT
<213> Arabidopsis thaliana

<400> 304
Met Ser Gln Val Asp Asn Arg Asn Ser Ser Ala Ala Lys Arg Ala Arg
1 5 10 15
Thr Asp Gly Gly Arg Arg Glu Asp Asp Trp Ile Cys Pro Ser Cys Gly
20 25 30
Asn Val Asn Phe Ser Phe Arg Thr Thr Cys Asn Met Arg Asn Cys Thr
35 40 45
Gln Pro Arg Pro Ala Asp His Asn Gly Lys Ser Ala Pro Lys Pro Met
50 55 60
Gln His Gln Gln Gly Phe Ser Ser Pro Gly Ala Tyr Leu Gly Ser Gly
65 70 75 80
Gly Pro Pro Pro Val Tyr Met Gly Gly Ser Pro Tyr Gly Ser Pro Leu
85 90 95
Phe Asn Gly Ser Ser Met Pro Pro Tyr Asp Val Pro Phe Ser Gly Gly
100 105 110
Ser Pro Tyr His Phe Asn Tyr Asn Ser Arg Met Pro Ala Gly Ala His
115 120 125
Tyr Arg Pro Leu His Met Ser Gly Pro Pro Pro Tyr His Gly Gly Ser
130 135 140
Met Met Gly Ser Gly Gly Met Tyr Gly Met Pro Pro Pro Ile Asp Arg
145 150 155 160
Tyr Gly Leu Gly Met Ala Met Gly Pro Gly Ser Ala Ala Ala Met Met
165 170 175
Pro Arg Pro Arg Phe Tyr Pro Asp Glu Lys Ser Gln Lys Arg Asp Ser
180 185 190
Thr Arg Asp Asn Asp Trp Thr Cys Pro Asn Cys Gly Asn Val Asn Phe
195 200 205
Ser Phe Arg Thr Val Cys Asn Met Arg Lys Cys Asn Thr Pro Lys Pro
210 215 220
Gly Ser Gln Gln Gly Gly Ser Ser Asp Lys Ile Ser Lys Gln Asn Ala
225 230 235 240
Pro Glu Gly Ser Trp Lys Cys Asp Asn Cys Gly Asn Ile Asn Tyr Pro
245 250 255
Phe Arg Ser Lys Cys Asn Arg Gln Asn Cys Gly Ala Asp Lys Pro Gly
260 265 270
Asp Arg Ser Asn Gly Ser Pro Ser Arg Ala Pro Glu Glu Asn Asp Gln
275 280 285

<210> 305
<211> 1853
<212> DNA
<213> Oryza sativa

<400> 305
cagtttccac tttcttccaa agccgaaaaa tcgagagaga aagagcaaaa ccctaaccgc 60
ggcgctccgc ccccttccgc cgccggattc ctcctcctcc ttcgtccgcc ttcgccggcg 120
ccgccctgct ccgaaggagc ccaggccttg tcgccgcgtc gcttgccccg ccgccggttt 180
cagcagcagc cacccccacc accatgtctt ctcaggtcga caaccgtagc caatctgctg 240
gaaagcgtgc ccgcaccgac ggtgggcggc gtgaggacga ctgggtttgc cccagctgcc 300
agaacgtcaa cttcgccttc cgcaccacct gcaacatgcg caattgcaac caatccaggc 360
ccaccgacta tacgaaggat atgcagaaac ctatgcagac accgccgccc catttcccca 420
tgtcaggggg atacatgagc ccagggacgc cgccatccat gtacctcggg ggtggtgctc 480
cccctatgg cacctccctc tatggcggac ctgctttacc acgttatggt attgctcagt 540
tccctggggg ttctggatat ccatatggct atggtggccg cctgccaatg gggagcccct 600
atgggccgcc gatgcatatg gcagggcctc catattctgc tggatccatg atgggaccag 660

```
gtggaatgta tgggatgccc atggacagat atagcttggg cttacctgct ggtcctggtc     720
caatgggcgc gagggctggt tcatattccg aggaaggatc ccagaagaag cctgcaggag     780
ctgggcgtga taatgattgg aaatgcccta attgcaacaa cattaacttt gcattccgga     840
cagtctgtaa catgagaaag tgcaatacac caagacctga aaaccagggg tccaaacctg     900
atggtgcaag aggtccaaaa ccaaagatgc cagaaggtag ttggaagtgc gagaagtgca     960
ataacataaa ctatccattc cgcacaaaat gtaaccgtcc aagttgcgag gctgaaaagc    1020
catttcagac aaacaatgct aatgagtcat ctgctgatca ggacaatcag ttgttgtcat    1080
gtaatattgt gaagctttta tcaaagctgc aactttcaca tgactttcaa gacaataatg    1140
agcaaacaaa ggtggatcct cccggtggcc ctgctggagt accgacaagt tcgcacgccc    1200
tgcgaatggc agctctcagt gagttgcaga acagtgttaa gagctgatac ccttttcatt    1260
ttccaaacaa ggtgagaact ttgatccatg tggatgtgct tctatcagga gcagctgaca    1320
gtattgcatg cagtgatctt gctaaggaag ttggagatgg gaaacatcgt gtgatggcca    1380
gccttgatgt aagaatgcaa atcagtctta ctccgtacca tatctgtgtg gagtgtgcac    1440
accggaagca tagtcatttt ttgaatggtc gattgcttct tccacagtaa agtctataat    1500
accagtgtgc aggtgttttt ttccctttct ttcagtgtga aggtgttttt ttaccccttt    1560
ctttcgttca ttcattcttt ttctttttcc ttgttgagac ggtatcctgg agcactttgt    1620
aatactctaa tgtgcttcgg aaagtagaat ataagttgtg cttatgggcg ggccaaaatg    1680
ctttggccgt tagttgcatg acagcctttc caacatgctg ctgcctagcc gtagttctcc    1740
caaaaatgaa accatcttga gatccgtttg cggttgaact gaaattagac ggtatctctg    1800
gtttcaacat catcctttaa accctggatg aaagatttgc aatattcctg cgg           1853
```

<210> 306
<211> 347
<212> PRT
<213> Oryza sativa

<400> 306

```
Met Ser Ser Gln Val Asp Asn Arg Ser Gln Ser Ala Gly Lys Arg Ala
1               5                   10                  15
Arg Thr Asp Gly Gly Arg Arg Glu Asp Asp Trp Val Cys Pro Ser Cys
            20                  25                  30
Gln Asn Val Asn Phe Ala Phe Arg Thr Thr Cys Asn Met Arg Asn Cys
        35                  40                  45
Asn Gln Ser Arg Pro Thr Asp Tyr Thr Lys Asp Met Gln Lys Pro Met
    50                  55                  60
Gln Thr Pro Pro Pro His Phe Pro Met Ser Gly Gly Tyr Met Ser Pro
65                  70                  75                  80
Gly Thr Pro Pro Ser Met Tyr Leu Gly Gly Gly Ala Pro Pro Tyr Gly
                85                  90                  95
Thr Ser Leu Tyr Gly Gly Pro Ala Leu Pro Arg Tyr Gly Ile Ala Gln
            100                 105                 110
Phe Pro Gly Gly Ser Gly Tyr Pro Tyr Gly Tyr Gly Gly Arg Leu Pro
        115                 120                 125
Met Gly Ser Pro Tyr Gly Pro Pro Met His Met Ala Gly Pro Pro Tyr
    130                 135                 140
Ser Ala Gly Ser Met Met Gly Pro Gly Gly Met Tyr Gly Met Pro Met
145                 150                 155                 160
Asp Arg Tyr Ser Leu Gly Leu Pro Ala Gly Pro Gly Pro Met Gly Ala
                165                 170                 175
Arg Ala Gly Ser Tyr Ser Glu Glu Gly Ser Gln Lys Lys Pro Ala Gly
            180                 185                 190
Ala Gly Arg Asp Asn Asp Trp Lys Cys Pro Asn Cys Asn Asn Ile Asn
        195                 200                 205
Phe Ala Phe Arg Thr Val Cys Asn Met Arg Lys Cys Asn Thr Pro Arg
    210                 215                 220
Pro Glu Asn Gln Gly Ser Lys Pro Asp Gly Ala Arg Gly Pro Lys Pro
225                 230                 235                 240
Lys Met Pro Glu Gly Ser Trp Lys Cys Glu Lys Cys Asn Asn Ile Asn
                245                 250                 255
```

```
Tyr Pro Phe Arg Thr Lys Cys Asn Arg Pro Ser Cys Glu Ala Glu Lys
            260                 265                 270
Pro Phe Gln Thr Asn Asn Ala Asn Glu Ser Ser Ala Asp Gln Asp Asn
        275                 280                 285
Gln Leu Leu Ser Cys Asn Ile Val Lys Leu Leu Ser Lys Leu Gln Leu
    290                 295                 300
Ser His Asp Phe Gln Asp Asn Asn Glu Gln Thr Lys Val Asp Pro Pro
305                 310                 315                 320
Gly Gly Pro Ala Gly Val Pro Thr Ser Ser His Ala Leu Arg Met Ala
                325                 330                 335
Ala Leu Ser Glu Leu Gln Asn Ser Val Lys Ser
            340                 345

<210>  307
<211>  1078
<212>  DNA
<213>  Oryza sativa

<400>  307
ctggtctctc gctctcttcg ctcgcggcgg cttctccgcc tccgcctccg ccgccctctc      60
ctcctctcgc ctcgccgccg gcgccgccag ccaccgcgcg ggtcgggcgg gccgtatctt     120
cctttctgtt ccgatggctt ccgcgaaggt ggagaaccgc ggcggcggtg ggttcggttc     180
gaagaggtca cgcaacgacg tgtctgtaag ggagggggac tggacttgtc ctcagtgtgg     240
taatgtcaac ttcagtttta gaaatgtttg caaccgcgga gcctgtggtg caccccgtcc     300
atcaccgagt ctaagcccaa gagtgccacc tcctcctgct gctggatatg atcggccaca     360
tcttggatat gatcggccac atctgtttta tggtagtgct ggcaccccac ctcctattcc     420
tcttggatct ggtagctatg gtgcccccta tccacatctt ggcttgcggt atggatatgg     480
tccaccagta ggacctcctg cttcatatgg cctttttct tcttatggtc aacctggacc      540
aatgggcagt ccgatgggag gcatgggcta tggccctgga cctgagctag gccgatatgg     600
ttatggtttt agaggatctc caatgccggt ttctagccca tggtctggtg gagcattagt     660
ggaaaataat gacagctctg cttcacgcaa gcgtcgtgga ggcccagatg gaatggctga     720
gaatgactgg atctgcccaa agtgtgagaa tgtcaacttt tccttcagaa acagttgcaa     780
tatgaagaaa tgtggagctc caaggccaag ccctggatct aatgctaccc catgtcgcaa     840
agacaaggac gctccggaag ggagctggac ctgcccggag tgcaacaacc tgaactaccc     900
tttccgcacg gcgtgcaatc ggaaaggctg cggaagcagc aggccggcag cggccacggc     960
gaactaggac cctactaact ttgcgccggc gattgggggc agagagagta ttgttgtatc    1020
ctagctatat acaagttaat ttaaactgta aacggctcaa atatattttc ttgtggcc      1078

<210>  308
<211>  277
<212>  PRT
<213>  Oryza sativa

<400>  308
Met Ala Ser Ala Lys Val Glu Asn Arg Gly Gly Gly Gly Phe Gly Ser
1               5                   10                  15
Lys Arg Ser Arg Asn Asp Val Ser Val Arg Glu Gly Asp Trp Thr Cys
            20                  25                  30
Pro Gln Cys Gly Asn Val Asn Phe Ser Phe Arg Asn Val Cys Asn Arg
        35                  40                  45
Gly Ala Cys Gly Ala Pro Arg Pro Ser Pro Ser Leu Ser Pro Arg Val
    50                  55                  60
Pro Pro Pro Pro Ala Ala Gly Tyr Asp Arg Pro His Leu Gly Tyr Asp
65                  70                  75                  80
Arg Pro His Leu Phe Tyr Gly Ser Ala Gly Thr Pro Pro Pro Ile Pro
                85                  90                  95
Leu Gly Ser Gly Ser Tyr Gly Ala Pro Tyr Pro His Leu Gly Leu Arg
            100                 105                 110
Tyr Gly Tyr Gly Pro Pro Val Gly Pro Pro Ala Ser Tyr Gly Leu Phe
```

```
        115                 120                 125
Ser Ser Tyr Gly Gln Pro Gly Pro Met Gly Ser Pro Met Gly Gly Met
    130                 135                 140
Gly Tyr Gly Pro Gly Pro Glu Leu Gly Arg Tyr Gly Tyr Gly Phe Arg
145                 150                 155                 160
Gly Ser Pro Met Pro Val Ser Ser Pro Trp Ser Gly Gly Ala Leu Val
                165                 170                 175
Glu Asn Asn Asp Ser Ser Ala Ser Arg Lys Arg Arg Gly Gly Pro Asp
            180                 185                 190
Gly Met Ala Glu Asn Asp Trp Ile Cys Pro Lys Cys Glu Asn Val Asn
        195                 200                 205
Phe Ser Phe Arg Asn Ser Cys Asn Met Lys Lys Cys Gly Ala Pro Arg
    210                 215                 220
Pro Ser Pro Gly Ser Asn Ala Thr Pro Cys Arg Lys Asp Lys Asp Ala
225                 230                 235                 240
Pro Glu Gly Ser Trp Thr Cys Pro Glu Cys Asn Asn Leu Asn Tyr Pro
                245                 250                 255
Phe Arg Thr Ala Cys Asn Arg Lys Gly Cys Gly Ser Ser Arg Pro Ala
            260                 265                 270
Ala Ala Thr Ala Asn
        275
```

<210> 309
<211> 662
<212> DNA
<213> Brassica rapa

<400> 309

```
ggtcttgttt tctttcaata aacacataat aagatagctt ccaagttcat caagaaataa      60
acgtaagcgc acgcatacaa taacctatcg aagatgagca gacccggaga ctggaactgt     120
agatcatgca cccacctcaa cttccagcgc cgtgattctt gccagcgatg cggtgactcc     180
cgtttgggtg caggtggagt cggtggctta gagtttggtg atttcggcgg cagaggtatg     240
tctgcttttg gattcaccac gggctccgac gttcgtccag gtgactggta ctgcacagtt     300
ggaaactgcg ggacacataa ctttgccagc cgctccacct gcttcaaatg cggcactttc     360
aaggacgaat ccctcggtgg gggcggcggc ggtggcgtag gcgtaaggcg gtccggtcat     420
gttgacgctg acgttatgcg gtctagagtc tccggcaacg gtggccgctc cagctggaaa     480
tccggtgatt ggatttgcac caggcttggt tgcaatgagc ataactttgc aagcagaatg     540
gagtgcttca gatgcaatgc accaagggac ttcagcatga gaacctcttt ctaagttaca     600
caataatgcc tttgaagtag ccttgtttcc aatctcttgg gcaccgtttc aatcaatgga     660
aa                                                                    662
```

<210> 310
<211> 166
<212> PRT
<213> Brassica rapa

<400> 310

```
Met Ser Arg Pro Gly Asp Trp Asn Cys Arg Ser Cys Thr His Leu Asn
1               5                   10                  15
Phe Gln Arg Arg Asp Ser Cys Gln Arg Cys Gly Asp Ser Arg Leu Gly
            20                  25                  30
Ala Gly Gly Val Gly Gly Leu Glu Phe Gly Asp Phe Gly Gly Arg Gly
        35                  40                  45
Met Ser Ala Phe Gly Phe Thr Thr Gly Ser Asp Val Arg Pro Gly Asp
    50                  55                  60
Trp Tyr Cys Thr Val Gly Asn Cys Gly Thr His Asn Phe Ala Ser Arg
65                  70                  75                  80
Ser Thr Cys Phe Lys Cys Gly Thr Phe Lys Asp Glu Ser Leu Gly Gly
                85                  90                  95
```

```
Gly Gly Gly Gly Gly Val Gly Val Arg Arg Ser Gly His Val Asp Ala
            100                 105                 110
Asp Val Met Arg Ser Arg Val Ser Gly Asn Gly Gly Arg Ser Ser Trp
        115                 120                 125
Lys Ser Gly Asp Trp Ile Cys Thr Arg Leu Gly Cys Asn Glu His Asn
    130                 135                 140
Phe Ala Ser Arg Met Glu Cys Phe Arg Cys Asn Ala Pro Arg Asp Phe
145                 150                 155                 160
Ser Met Arg Thr Ser Phe
                165
```

<210> 311
<211> 749
<212> DNA
<213> Euphorbia esula

<400> 311

```
atttccctt cttcacccaa atatctctca ccttttaaca agagaaaagc aaaaagaaga      60
tgagcagacc aggagattgg aactgcaggt cctgccagca tctcaacttc cagagaaggg    120
actcgtgcca gcgctgtggg gactccaggt ccgggactgg aggttcagga gctgactttg    180
gcgggtttgg aagccgggtt gggtcctcat tcgggttcag caccgggtct gatgttcgac    240
ccggtgactg gtactgtact gctggcaact gtggggccca caactttgcc agccgggcaa    300
gttgcttcaa atgtggagtt tataaggatg attctggggc ccctggcggg tttgattccg    360
atatcctccg ggcctctaga ggttttggta gtggcagcaa tcgctcttct tggaaatctg    420
gtgattggat ctgcactcgg tggggatgta atgaacacaa ctttgcaagc agaatggagt    480
gtttcaaatg cagtgcccct agggacctta gtaacagaac ttcatactag acattttgga    540
tggtgcagca gccaagaaag agaattaaga ttactacttt taaattttat tttattttta    600
ctatttgttt tgggtgttat tagagtggaa aacaaacatg gcttaaaaac ccatgtttca    660
tccctaattt aagctaagga agcaaacccc ttttggcatt ttgtaaggca gatttaggat    720
tgtagtactt aattaattag tgggttgtt                                      749
```

<210> 312
<211> 156
<212> PRT
<213> Euphorbia esula

<400> 312

```
Met Ser Arg Pro Gly Asp Trp Asn Cys Arg Ser Cys Gln His Leu Asn
1               5                   10                  15
Phe Gln Arg Arg Asp Ser Cys Gln Arg Cys Gly Asp Ser Arg Ser Gly
            20                  25                  30
Thr Gly Gly Ser Gly Ala Asp Phe Gly Gly Phe Gly Ser Arg Val Gly
        35                  40                  45
Ser Ser Phe Gly Phe Ser Thr Gly Ser Asp Val Arg Pro Gly Asp Trp
    50                  55                  60
Tyr Cys Thr Ala Gly Asn Cys Gly Ala His Asn Phe Ala Ser Arg Ala
65                  70                  75                  80
Ser Cys Phe Lys Cys Gly Val Tyr Lys Asp Asp Ser Gly Ala Pro Gly
                85                  90                  95
Gly Phe Asp Ser Asp Ile Leu Arg Ala Ser Arg Gly Phe Gly Ser Gly
            100                 105                 110
Ser Asn Arg Ser Ser Trp Lys Ser Gly Asp Trp Ile Cys Thr Arg Trp
        115                 120                 125
Gly Cys Asn Glu His Asn Phe Ala Ser Arg Met Glu Cys Phe Lys Cys
    130                 135                 140
Ser Ala Pro Arg Asp Leu Ser Asn Arg Thr Ser Tyr
145                 150                 155
```

<210> 313

<211> 646
<212> DNA
<213> Gossypium hirsutum

<400> 313
ctccattctc ttatttaact caaactcacc acctttttcca tttttctaat taagaaaaga 60
aaagatgagc aggccaggag attggaactg caggtcgtgc caacacctaa acttccaaag 120
gagggactcc tgccaacgct gcggggaatt ccggtcgggt gatcacttcg gtagctacgg 180
tggtggcagg ggtggctcct cctttggatt cgccaccggc tccgacgtcc gacctggtga 240
ttggtactgc actgcgggaa actgcggtac ccacaatttc gccagtcgtt ccagctgctt 300
caaatgtggt gcattcaagg acgaccctgc cggaggtttc gacagcgacg ttccgcgttc 360
tagaggattt ggcggcggta atcgatccgg ctggaaatcc ggcgactgga tatgtaccag 420
gtcgggatgc aatgagcata actttgctag ccgaatggaa tgtttcagat gcagtgcccc 480
aagagacttc accgctagaa cttcatacta aatacaagct atccagtttt gggtgttgca 540
aaccaagaga gactcaaatg agagaaaaaa ttttacgggg ttagggttac ccgggtaaat 600
atatggcttg ggggggtgtg gcaccagggt tgaaaacacc agggtg 646

<210> 314
<211> 148
<212> PRT
<213> Gossypium hirsutum

<400> 314
Met Ser Arg Pro Gly Asp Trp Asn Cys Arg Ser Cys Gln His Leu Asn
1               5                   10                  15
Phe Gln Arg Arg Asp Ser Cys Gln Arg Cys Gly Glu Phe Arg Ser Gly
            20                  25                  30
Asp His Phe Gly Ser Tyr Gly Gly Gly Arg Gly Gly Ser Ser Phe Gly
        35                  40                  45
Phe Ala Thr Gly Ser Asp Val Arg Pro Gly Asp Trp Tyr Cys Thr Ala
    50                  55                  60
Gly Asn Cys Gly Thr His Asn Phe Ala Ser Arg Ser Ser Cys Phe Lys
65                  70                  75                  80
Cys Gly Ala Phe Lys Asp Asp Pro Ala Gly Gly Phe Asp Ser Asp Val
                85                  90                  95
Pro Arg Ser Arg Gly Phe Gly Gly Gly Asn Arg Ser Gly Trp Lys Ser
            100                 105                 110
Gly Asp Trp Ile Cys Thr Arg Ser Gly Cys Asn Glu His Asn Phe Ala
        115                 120                 125
Ser Arg Met Glu Cys Phe Arg Cys Ser Ala Pro Arg Asp Phe Thr Ala
    130                 135                 140
Arg Thr Ser Tyr
145

<210> 315
<211> 613
<212> DNA
<213> Vitis vinifera

<400> 315
ggtcttcttc catctctgca cagttatcct gatatttcct tggaaaacat gagcaggcca 60
ggagattgga actgcaggtc atgccagcac atgaacttcc aaaggcgcga ttcctgccaa 120
cgctgcggtg acccaaaatc gggtgggggt gactttggaa gctttggtgg gaggggtgga 180
tcctcctttg ggttcacggg ctcggatgtc cgcccagggg actggtactg caatgcaggc 240
aactgtggag ctcacaactt tgctagccgc tctaactgct tcaagtgtgg tgcattcaaa 300
gatgagtctg ctgggggcta cgattccgac atgtcacgct cccgaggttt cggggttcggc 360
ggtggcagcg gccggtctgg gtggaaatcc ggtgattgga tatgcagcag gtctggatgc 420
aatgagcaca actttgctag cagaatggaa tgtttcagat gcaatgcccc gagggacttg 480
agtaacaaaa cttcatacta gacatatatc ttccattttt gggtactgca gccagccaag 540

agagaccaaa tcatagaata tctattaatc cttgtgttgt tattaatttc tttgctttgg 600
gtgctaggtt ctt 613

<210> 316
<211> 150
<212> PRT
<213> Vitis vinifera

<400> 316
Met Ser Arg Pro Gly Asp Trp Asn Cys Arg Ser Cys Gln His Met Asn
1 5 10 15
Phe Gln Arg Arg Asp Ser Cys Gln Arg Cys Gly Asp Pro Lys Ser Gly
20 25 30
Gly Gly Asp Phe Gly Ser Phe Gly Gly Arg Gly Gly Ser Ser Phe Gly
35 40 45
Phe Thr Gly Ser Asp Val Arg Pro Gly Asp Trp Tyr Cys Asn Ala Gly
50 55 60
Asn Cys Gly Ala His Asn Phe Ala Ser Arg Ser Asn Cys Phe Lys Cys
65 70 75 80
Gly Ala Phe Lys Asp Glu Ser Ala Gly Gly Tyr Asp Ser Asp Met Ser
85 90 95
Arg Ser Arg Gly Phe Gly Phe Gly Gly Gly Ser Gly Arg Ser Gly Trp
100 105 110
Lys Ser Gly Asp Trp Ile Cys Ser Arg Ser Gly Cys Asn Glu His Asn
115 120 125
Phe Ala Ser Arg Met Glu Cys Phe Arg Cys Asn Ala Pro Arg Asp Leu
130 135 140
Ser Asn Lys Thr Ser Tyr
145 150

<210> 317
<211> 844
<212> DNA
<213> Populus trichocarpa

<400> 317
tcagaccatt tccctttcat cttctagcta gcatagcttc tctcagaggt ttttgaaacc 60
ccttttttgcc attcttctct cacttagtat acttagtctt ctctaatcaa ttcataagca 120
aatatgaaca ggccaggaga ctggaactgc aggtcatgcc aacacctcaa tttccagagg 180
cgtgactctt gccaacgttg tggggacccc aggtccgcag gtgattttgg gggtttcggt 240
gggcggggtg gctcatcact tgggttcacc gggtcggatg ttcgtcccgg tgattggtac 300
tgcactgccg gaaactgcgg ggcccacaac tttgctagcc gttctagttg cttcaaatgt 360
ggagtgtaca aggaaatgga ctccgccggg ggcttcgatt ctgatttttc tcgaactaga 420
gggtttggtg ggagcactgg aggtggcaat cgatctggat ggaaatccgg agactggatt 480
tgcactaggt ggggatgcaa cgaacataac tttgctagca gaatggagtg cttcaagtgc 540
aatgccccaa gagatcttag caacagaact tcatactaga tacaatttct ccatttcctg 600
ggactgcacc agccaagaac aaagacaaca tgattaaaaa atatatatca ctacttttca 660
ttttcttctt gatttctttt tgtattagct agctagggtc ttgaggcgag acatggttta 720
agaaccatgt ttactgcttt gagctatata taacccttgg cattttgtca ggcttcctgt 780
aggaagtata tcgttgtctt tgtgggcttt tgatctatta tattcattat tgtaaccaag 840
ggag 844

<210> 318
<211> 151
<212> PRT
<213> Populus trichocarpa

<400> 318
Met Asn Arg Pro Gly Asp Trp Asn Cys Arg Ser Cys Gln His Leu Asn

```
1               5                   10                  15
Phe Gln Arg Arg Asp Ser Cys Gln Arg Cys Gly Asp Pro Arg Ser Ala
            20                  25                  30
Gly Asp Phe Gly Gly Phe Gly Gly Arg Gly Gly Ser Ser Leu Gly Phe
        35                  40                  45
Thr Gly Ser Asp Val Arg Pro Gly Asp Trp Tyr Cys Thr Ala Gly Asn
    50                  55                  60
Cys Gly Ala His Asn Phe Ala Ser Arg Ser Ser Cys Phe Lys Cys Gly
65                  70                  75                  80
Val Tyr Lys Glu Met Asp Ser Ala Gly Gly Phe Asp Ser Asp Phe Ser
                85                  90                  95
Arg Thr Arg Gly Phe Gly Gly Ser Thr Gly Gly Gly Asn Arg Ser Gly
            100                 105                 110
Trp Lys Ser Gly Asp Trp Ile Cys Thr Arg Trp Gly Cys Asn Glu His
        115                 120                 125
Asn Phe Ala Ser Arg Met Glu Cys Phe Lys Cys Asn Ala Pro Arg Asp
    130                 135                 140
Leu Ser Asn Arg Thr Ser Tyr
145                 150
```

<210> 319
<211> 785
<212> DNA
<213> Gossypium hirsutum

<400> 319

```
ccttgcgtcc gctttcttct tcattgttcc tgtctttgct tactcacccc ttatatatct     60
gctttctcaa gtaggggaga aaatatgagc aggccaggag actggaattg caggtcatgc    120
caacacctca acttccaaag gagggactca tgccagcgct gtggagaacc aagacctggt    180
ggtggtgaca gaggcggcga ctatggaagc tttggtggca ggggtggctc atctttcggg    240
tttactggac ccgatgttag gcctggtgac tggtattgca ctgtgggcaa ctgcggtgct    300
cacaacttcg ccagcaggtc gagctgcttc aaatgtggtg cggccaaaga tgaatcatcc    360
ggaggatttg aaagtgacat cccacgtatg aggggttatg gttttagcac tggcagctct    420
agtcgctcta actggaaatc tggagactgg atttgcacca ggtcgggttg caatgaacac    480
aacttcgcca gcaggatgga atgtttcaga tgcaatgcac caagggactc cacccacaaa    540
tcttcatact aattaataaa attttcattt ttgggtactg cagtcgcaag agagagatca    600
gacaaggcaa tcccagatct tgctttcttt ttcttttgtt tgtttcttta ttttagatct    660
ttagatgaat catgtttgaa agacttcagt tgaagtactt aagctaatat caaagtacat    720
agggcatgca tgtaattgat gtatggtatc agcaatgtta tatcagtgtc tttgtgccaa    780
aaaaa                                                                785
```

<210> 320
<211> 155
<212> PRT
<213> Gossypium hirsutum

<400> 320

```
Met Ser Arg Pro Gly Asp Trp Asn Cys Arg Ser Cys Gln His Leu Asn
1               5                   10                  15
Phe Gln Arg Arg Asp Ser Cys Gln Arg Cys Gly Glu Pro Arg Pro Gly
            20                  25                  30
Gly Gly Asp Arg Gly Gly Asp Tyr Gly Ser Phe Gly Gly Arg Gly Gly
        35                  40                  45
Ser Ser Phe Gly Phe Thr Gly Pro Asp Val Arg Pro Gly Asp Trp Tyr
    50                  55                  60
Cys Thr Val Gly Asn Cys Gly Ala His Asn Phe Ala Ser Arg Ser Ser
65                  70                  75                  80
Cys Phe Lys Cys Gly Ala Ala Lys Asp Glu Ser Ser Gly Gly Phe Glu
                85                  90                  95
```

```
Ser Asp Ile Pro Arg Met Arg Gly Tyr Gly Phe Ser Thr Gly Ser Ser
            100                 105                 110
Ser Arg Ser Asn Trp Lys Ser Gly Asp Trp Ile Cys Thr Arg Ser Gly
        115                 120                 125
Cys Asn Glu His Asn Phe Ala Ser Arg Met Glu Cys Phe Arg Cys Asn
    130                 135                 140
Ala Pro Arg Asp Ser Thr His Lys Ser Ser Tyr
145                 150                 155
```

<210> 321
<211> 760
<212> DNA
<213> Lactuca serriola

<400> 321

```
gtattcggaa tcctcaaaga aacacacact tcctatccat cacaagaaag atgagcaggc      60
caggagattg gaactgcagg tcatgccagc acttgaactt ccagaggagg gactcttgcc     120
aaagatgtgg ggagacgagg tatggtggtg ggggtggtgt gtttggtggt agaggaagta     180
tcatcagccc ttcagcattt ggcttcacag gcccagatgt ccgaccgggt gattggtact     240
gcaatgttgg caactgcggg gctcacaact ttgctagccg ctcgagctgc ttcaagtgtg     300
gtgcgttcaa ggatgactta gcttgtagtg gtggtggtgg tggtggtggt ggcgtttttg     360
atggtgatat gtcacgtggc aggggtttcg ggtttggtgg aggaagtggt ggtggaggtg     420
gtggcagcag ccgttcaggg tggaagtccg gtgactggat atgcggcagg cctggttgca     480
atgagcacaa ctttgcaagc agaatggaat gttttaggtg caacgcacct cgggaatcgg     540
gtaacaagtc tccttattaa gcaggttgcg gcatttccag ttccgggtat catggacttg     600
ctatctacca aacaggaaga gagataagtg atcgatcaga cagaatcatg aagagcatcc     660
agttgatagg aaattctaaa ctgacccccct ttttgcccat atcataatat gcaatcctat     720
ctttgatttt tttttccttt ttgttttatt ttttttttt                            760
```

<210> 322
<211> 169
<212> PRT
<213> Lactuca serriola

<400> 322

```
Met Ser Arg Pro Gly Asp Trp Asn Cys Arg Ser Cys Gln His Leu Asn
1               5                   10                  15
Phe Gln Arg Arg Asp Ser Cys Gln Arg Cys Gly Glu Thr Arg Tyr Gly
            20                  25                  30
Gly Gly Gly Gly Val Phe Gly Gly Arg Gly Ser Ile Ile Ser Pro Ser
        35                  40                  45
Ala Phe Gly Phe Thr Gly Pro Asp Val Arg Pro Gly Asp Trp Tyr Cys
    50                  55                  60
Asn Val Gly Asn Cys Gly Ala His Asn Phe Ala Ser Arg Ser Ser Cys
65                  70                  75                  80
Phe Lys Cys Gly Ala Phe Lys Asp Asp Leu Ala Cys Ser Gly Gly Gly
                85                  90                  95
Gly Gly Gly Gly Gly Val Phe Asp Gly Asp Met Ser Arg Gly Arg Gly
            100                 105                 110
Phe Gly Phe Gly Gly Gly Ser Gly Gly Gly Gly Gly Gly Ser Ser Arg
        115                 120                 125
Ser Gly Trp Lys Ser Gly Asp Trp Ile Cys Gly Arg Pro Gly Cys Asn
    130                 135                 140
Glu His Asn Phe Ala Ser Arg Met Glu Cys Phe Arg Cys Asn Ala Pro
145                 150                 155                 160
Arg Glu Ser Gly Asn Lys Ser Pro Tyr
                165
```

<210> 323

<211> 517
<212> DNA
<213> Glycine max

<400> 323
tttctgcatc tctaatactt caactcactg tattatcttg agtaattttg cagagaagta 60
aaatatgagc aggccaggag actggaattg caggtcgtgc cagcacctga actttcagag 120
gagagactca tgccagcgat gtggggactc aaaatatgga gatagagttg ttgattttgg 180
tggttttgga ggaagaggag ggtcctcatt tggtttaact ggctcagatg ttcgccccgg 240
cgactggtac tgtgctgctg ctaactgtgg tgcacacaac tttgctagcc gctcaagctg 300
cttcaagtgt ggtgctttca aggatgactt ggctggagga ggctataaca gttctgacat 360
cttgcgctcc agagcttttg gtggcagtgg aagacctgga tggaaatctg gtgattggat 420
atgcagcaga tcaggatgca atgagcacaa ctttgctagc agaatggaat gttttaaatg 480
cagtgctccc agggacacgt actagaaaaa atgagtt 517

<210> 324
<211> 146
<212> PRT
<213> Glycine max

<400> 324
Met Ser Arg Pro Gly Asp Trp Asn Cys Arg Ser Cys Gln His Leu Asn
1 5 10 15
Phe Gln Arg Arg Asp Ser Cys Gln Arg Cys Gly Asp Ser Lys Tyr Gly
20 25 30
Asp Arg Val Val Asp Phe Gly Gly Phe Gly Gly Arg Gly Gly Ser Ser
35 40 45
Phe Gly Leu Thr Gly Ser Asp Val Arg Pro Gly Asp Trp Tyr Cys Ala
50 55 60
Ala Ala Asn Cys Gly Ala His Asn Phe Ala Ser Arg Ser Ser Cys Phe
65 70 75 80
Lys Cys Gly Ala Phe Lys Asp Asp Leu Ala Gly Gly Gly Tyr Asn Ser
85 90 95
Ser Asp Ile Leu Arg Ser Arg Ala Phe Gly Gly Ser Gly Arg Pro Gly
100 105 110
Trp Lys Ser Gly Asp Trp Ile Cys Ser Arg Ser Gly Cys Asn Glu His
115 120 125
Asn Phe Ala Ser Arg Met Glu Cys Phe Lys Cys Ser Ala Pro Arg Asp
130 135 140
Thr Tyr
145

<210> 325
<211> 889
<212> DNA
<213> Populus trichocarpa

<400> 325
gacaagttcc ttaaacaatc tattgctctt tcaactacta gctaggttac acttcatttc 60
tgtacgtgtt aattctccta tctttctcct ttctgctttc tcgagatgag cagaccagga 120
gattggaatt gcaggtcatg ccagcacttg aactttcaaa ggagggactc atgccagcgt 180
tgcggtgatc caaggcccgg agagagagat cactatggaa gtttcggtgg aagatcatca 240
gggggctcat tcggatttac gggccctgat gttaggcctg gtgattggta ttgcacggct 300
ggcaattgtg gagctcacaa ctttgctagt cgttcaagct gcttcaagtg tggtgtgtcc 360
aaggatgaat cctctggtgg tggacttgat gctgatatgt cacggatgag aggttatggc 420
ttcggcggag gcggaggcgg aggcagtggc tctagccgta attggaaatc cggagactgg 480
atttgcacca ggtccggttg caacgagcac aactttgcta gcaggactga atgctataga 540
tgcaatgcac caagagaatc tagcagcaac aagtcttcgt attaatcatc gatatcgata 600
gcatttttgt gtcctgcagt gctgcaagga gggaattaac gaagaaggct catgagaaat 660

cttggattca tctttttgct cttaattact tctatttttg ttcttttgga tagctgtact 720
cttaagctga gaagctttag aaggatcatg ggagtgaaat taagttcaag gagagctata 780
tatatatcat tagccagtaa tttgtcaggt tccctaaata tagacatgta gttctgtact 840
tggtatcaat gtctttgtgt tttgagacgg gtttaagccc ttgtcgttc 889

<210> 326
<211> 159
<212> PRT
<213> Populus trichocarpa

<400> 326
Met Ser Arg Pro Gly Asp Trp Asn Cys Arg Ser Cys Gln His Leu Asn
1 5 10 15
Phe Gln Arg Arg Asp Ser Cys Gln Arg Cys Gly Asp Pro Arg Pro Gly
20 25 30
Glu Arg Asp His Tyr Gly Ser Phe Gly Gly Arg Ser Ser Gly Gly Ser
35 40 45
Phe Gly Phe Thr Gly Pro Asp Val Arg Pro Gly Asp Trp Tyr Cys Thr
50 55 60
Ala Gly Asn Cys Gly Ala His Asn Phe Ala Ser Arg Ser Ser Cys Phe
65 70 75 80
Lys Cys Gly Val Ser Lys Asp Glu Ser Ser Gly Gly Gly Leu Asp Ala
85 90 95
Asp Met Ser Arg Met Arg Gly Tyr Gly Phe Gly Gly Gly Gly Gly Gly
100 105 110
Gly Ser Gly Ser Ser Arg Asn Trp Lys Ser Gly Asp Trp Ile Cys Thr
115 120 125
Arg Ser Gly Cys Asn Glu His Asn Phe Ala Ser Arg Thr Glu Cys Tyr
130 135 140
Arg Cys Asn Ala Pro Arg Glu Ser Ser Ser Asn Lys Ser Ser Tyr
145 150 155

<210> 327
<211> 723
<212> DNA
<213> Populus fremontii x Populus angustifolia

<400> 327
agggaaaaag aaagcctaac cctcctgcct gcctcaagct taccccatta tcgaggtcta 60
aaacaagtat aaaaacagcc ttagccccaa tacgtaaatc acaagttcct taaacaatct 120
attgctcttt caactactag gtttcccact tcatttctgt gcctactgat tctcctctct 180
ttcaactttc tgccttctca agctagctag agaagggaag atgagcagac caggagattg 240
gaattgcagg tcatgccaac acttgaactt ccagaggagg gactcgtgcc agcgttgtgg 300
ggacccaagg cccggagaga gagatcatta tggaagtttt ggtggaagat caggggggctc 360
gttcggattt acggggcctg atgttaggcc cggtgactgg tattgctcgg ttggcaactg 420
tggagctcac aactttgcta gtcgttcaag ctgcttcaag tgtggtatgt ccaaggatga 480
atcctctggt ggtggcttg atgctgacat ttcatggatg agaggttatg gcttcggcgg 540
aggcagcgcc tctagccgct ctaattggaa atccggagac tggatttgca ccaggtcagg 600
ttgcaacgag cacaactttg ctagcaggac tgagtgttac agatgcaatg caccaagaga 660
atcaggcagc aacaagtctt cgtattaatc atcaacatcg atcgcatttt tgtgtactgc 720
agt 723

<210> 328
<211> 155
<212> PRT
<213> Populus fremontii x Populus angustifolia

<400> 328
Met Ser Arg Pro Gly Asp Trp Asn Cys Arg Ser Cys Gln His Leu Asn

```
1               5                   10                  15
Phe Gln Arg Arg Asp Ser Cys Gln Arg Cys Gly Asp Pro Arg Pro Gly
            20                  25                  30
Glu Arg Asp His Tyr Gly Ser Phe Gly Gly Arg Ser Gly Gly Ser Phe
        35                  40                  45
Gly Phe Thr Gly Pro Asp Val Arg Pro Gly Asp Trp Tyr Cys Ser Val
    50                  55                  60
Gly Asn Cys Gly Ala His Asn Phe Ala Ser Arg Ser Ser Cys Phe Lys
65                  70                  75                  80
Cys Gly Met Ser Lys Asp Glu Ser Ser Gly Gly Gly Leu Asp Ala Asp
                85                  90                  95
Ile Ser Trp Met Arg Gly Tyr Gly Phe Gly Gly Gly Ser Ala Ser Ser
            100                 105                 110
Arg Ser Asn Trp Lys Ser Gly Asp Trp Ile Cys Thr Arg Ser Gly Cys
        115                 120                 125
Asn Glu His Asn Phe Ala Ser Arg Thr Glu Cys Tyr Arg Cys Asn Ala
    130                 135                 140
Pro Arg Glu Ser Gly Ser Asn Lys Ser Ser Tyr
145                 150                 155
```

<210> 329
<211> 920
<212> DNA
<213> Oryza sativa

<400> 329

```
cacctcacag catttccac cttagagctc accaacacag cagctgatac ttgtttaggg      60
taagacaaga tgaacaggaa gccaggagac tgggactgca gggcgtgcca gcacctcaac    120
ttcagccgcc gggacctatg ccagcgctgc ggcgggccgc gtggcgccgc tgatcgcggc    180
agcggtggtg gcggtgacta cgccaacttc ggcggccgtg gtggttcctc cttcggtgga    240
ggctttggca ctggctctga tgtccgccca ggtgactggt actgcaactg cggcgcgcac    300
aacttcgcca gccgctccag ctgcttcaag tgcgctgctt tcaaggacga tgctgccgtc    360
aacagtggcg gcgctggtgc ctttgacggt ggggacatgt cgcgctcgcg gggctacggc    420
ttcggcagcg gcgccgtccg cgccagccgc cctggctgga agtctggcga ctggatttgc    480
accaggtctg gatgcaatga gcacaacttc gccagcagga tggagtgctt caggtgcaac    540
gcaccgcggg actccggcac tgaggtgtaa tttgccgtac gtgtccgatc gatctggatc    600
cgatgaggct tgcagcagtg acgacgagca gcagaagcag cgttaagagt tgtgatgtct    660
acataagaag aagaagaaag tagaatgcaa aagaaatctc cccatggttt tactagtttt    720
gtttcttccc gttttagatt tggttctgat tcccatttgg gaggacccgt cgacccctga    780
ttatctatgt tttacccgtt ttatttcctg tttctttcgg catgtttgct cttcgatcga    840
gtcgtgtaac ccgaaacgct tgcgcttgag aagtattatt attattaact agtatgttgc    900
ttcttaaaaa aaaaaaaaaa                                                920
```

<210> 330
<211> 166
<212> PRT
<213> Oryza sativa

<400> 330

```
Met Asn Arg Lys Pro Gly Asp Trp Asp Cys Arg Ala Cys Gln His Leu
1               5                   10                  15
Asn Phe Ser Arg Arg Asp Leu Cys Gln Arg Cys Gly Gly Pro Arg Gly
            20                  25                  30
Ala Ala Asp Arg Gly Ser Gly Gly Gly Gly Asp Tyr Ala Asn Phe Gly
        35                  40                  45
Gly Arg Gly Gly Ser Ser Phe Gly Gly Gly Phe Gly Thr Gly Ser Asp
    50                  55                  60
Val Arg Pro Gly Asp Trp Tyr Cys Asn Cys Gly Ala His Asn Phe Ala
65                  70                  75                  80
```

```
Ser Arg Ser Ser Cys Phe Lys Cys Ala Ala Phe Lys Asp Asp Ala Ala
                85                  90                  95
Val Asn Ser Gly Gly Ala Gly Ala Phe Asp Gly Gly Asp Met Ser Arg
            100                 105                 110
Ser Arg Gly Tyr Gly Phe Gly Ser Gly Ala Val Arg Ala Ser Arg Pro
        115                 120                 125
Gly Trp Lys Ser Gly Asp Trp Ile Cys Thr Arg Ser Gly Cys Asn Glu
    130                 135                 140
His Asn Phe Ala Ser Arg Met Glu Cys Phe Arg Cys Asn Ala Pro Arg
145                 150                 155                 160
Asp Ser Gly Thr Glu Val
                165
```

```
<210>  331
<211>  703
<212>  DNA
<213>  Panicum virgatum

<400>  331
ccacgcgtcc gcagaactcc ttgtcctagg ccccagaaac acaaacgacc gctctttgtt      60
taggcaagat gaacaggaag cctggagact gggattgcag ggcttgccaa cacctcaact     120
tcagtcggcg ggacctatgc cagcgctgtg gtgagccacg tggagctgct gaccgtggca     180
gcggcggtgg aggtgactat gccaacttcg gtggccgtgg tggctcctcc ttcggcggtg     240
gctttggtgc tggctctgat gtccgccctg gtgactggtt atgttcctgc ggcgcgcaca     300
acttcgccag ccgctccaac tgcttcaagt gctctgcctt caaggaggag gctgctgtca     360
acagtggtgc tggtggcttt gatggtgaca tgtcacgctc gcgctacggc ttcggtggcg     420
gtgctgcccg caccaaccgc cctggttgga agtctggaga ctggatctgc accaggtccg     480
gatgcaacga gcacaacttt gccagcagga tggagtgttt caggtgcaac gcaccacggg     540
actccggcac tgaggtgtag gatcggagct ggtgcttcga acggaccccg acgagcagaa     600
gcagcaagaa acctgataag aagagtagta gatttgcaaa aggcatcccg gatgctctat     660
tactgttttg ttccacccct accgcttcct tttagaattt ggt                       703

<210>  332
<211>  163
<212>  PRT
<213>  Panicum virgatum

<400>  332
Met Asn Arg Lys Pro Gly Asp Trp Asp Cys Arg Ala Cys Gln His Leu
1               5                   10                  15
Asn Phe Ser Arg Arg Asp Leu Cys Gln Arg Cys Gly Glu Pro Arg Gly
            20                  25                  30
Ala Ala Asp Arg Gly Ser Gly Gly Gly Gly Asp Tyr Ala Asn Phe Gly
        35                  40                  45
Gly Arg Gly Gly Ser Ser Phe Gly Gly Gly Phe Gly Ala Gly Ser Asp
    50                  55                  60
Val Arg Pro Gly Asp Trp Leu Cys Ser Cys Gly Ala His Asn Phe Ala
65                  70                  75                  80
Ser Arg Ser Asn Cys Phe Lys Cys Ser Ala Phe Lys Glu Glu Ala Ala
                85                  90                  95
Val Asn Ser Gly Ala Gly Gly Phe Asp Gly Asp Met Ser Arg Ser Arg
            100                 105                 110
Tyr Gly Phe Gly Gly Gly Ala Ala Arg Thr Asn Arg Pro Gly Trp Lys
        115                 120                 125
Ser Gly Asp Trp Ile Cys Thr Arg Ser Gly Cys Asn Glu His Asn Phe
    130                 135                 140
Ala Ser Arg Met Glu Cys Phe Arg Cys Asn Ala Pro Arg Asp Ser Gly
145                 150                 155                 160
Thr Glu Val
```

<210> 333
<211> 435
<212> DNA
<213> Medicago truncatula

<400> 333
atgaacagga aaatgagctg gtctggagga gattggatgt gtggtgcttg cgagcacata 60
aatttcaaga agagagaagc atgccaaaat tgtggatacc caaagtatgg aggccctgac 120
ccatcgacct atagatataa caggactgaa acgttggcag gggactggtt ttgcacttct 180
atgaactgtg gagctcacaa ctatgcaagc cgatcaaact gctatagatg tggtgcattt 240
aaagatcctt attcttctgg atatgggggt aacatggtgg gttctggagg atatggatca 300
gattgtagtt ctcccccagg atggaaaagt ggagactgga tttgccctag aattggctgt 360
ggaatccata attatgcaag caggacagag tgctacaaat gcaaaatgcc aagggattat 420
ggtggtgcag actga 435

<210> 334
<211> 144
<212> PRT
<213> Medicago truncatula

<400> 334
Met Asn Arg Lys Met Ser Trp Ser Gly Gly Asp Trp Met Cys Gly Ala
1 5 10 15
Cys Glu His Ile Asn Phe Lys Lys Arg Glu Ala Cys Gln Asn Cys Gly
20 25 30
Tyr Pro Lys Tyr Gly Gly Pro Asp Pro Ser Thr Tyr Arg Tyr Asn Arg
35 40 45
Thr Glu Thr Leu Ala Gly Asp Trp Phe Cys Thr Ser Met Asn Cys Gly
50 55 60
Ala His Asn Tyr Ala Ser Arg Ser Asn Cys Tyr Arg Cys Gly Ala Phe
65 70 75 80
Lys Asp Pro Tyr Ser Ser Gly Tyr Gly Gly Asn Met Val Gly Ser Gly
85 90 95
Gly Tyr Gly Ser Asp Cys Ser Ser Pro Pro Gly Trp Lys Ser Gly Asp
100 105 110
Trp Ile Cys Pro Arg Ile Gly Cys Gly Ile His Asn Tyr Ala Ser Arg
115 120 125
Thr Glu Cys Tyr Lys Cys Lys Met Pro Arg Asp Tyr Gly Gly Ala Asp
130 135 140

<210> 335
<211> 513
<212> DNA
<213> Medicago truncatula

<400> 335
atgagcagac caggagattg gaactgcagg acatgcaacc acctcaactt tcaaagaaga 60
gaatcttgcc aacgatgtgg ggagtcaaga atgacttctg gctgcggcgc cgttgatttt 120
ggtggctcct ttcttggtgg aagaggctct agctcccctt ttcctttcac caccggccct 180
gatgtccgtc ctggtgactg gtattgcact gttggaaact gtggagctca caactttgcc 240
agccgctcca gctgcttcaa atgtggtgcc cctaaggata ttgatacctt ctcctctgac 300
tcctcagaca tgccacgatt attgagatca ccatacggtt ttggagcagg cagcgctggt 360
ggcggtgcct ccactcgccc cggctggaaa tccggtgact ggatatgcac caggtctggg 420
tgtaacgagc ataacttcgc caatagaatg gaatgctacc gatgcaacgg tccaagggac 480
tctagtactg gaagatcttc ctatttatcg tga 513

<210> 336

<211> 170
<212> PRT
<213> Medicago truncatula

<400> 336

```
Met Ser Arg Pro Gly Asp Trp Asn Cys Arg Thr Cys Asn His Leu Asn
1               5                   10                  15
Phe Gln Arg Arg Glu Ser Cys Gln Arg Cys Gly Glu Ser Arg Met Thr
            20                  25                  30
Ser Gly Cys Gly Ala Val Asp Phe Gly Gly Ser Phe Leu Gly Gly Arg
        35                  40                  45
Gly Ser Ser Ser Pro Phe Pro Phe Thr Thr Gly Pro Asp Val Arg Pro
    50                  55                  60
Gly Asp Trp Tyr Cys Thr Val Gly Asn Cys Gly Ala His Asn Phe Ala
65                  70                  75                  80
Ser Arg Ser Ser Cys Phe Lys Cys Gly Ala Pro Lys Asp Ile Asp Thr
                85                  90                  95
Phe Ser Ser Asp Ser Ser Asp Met Pro Arg Leu Leu Arg Ser Pro Tyr
            100                 105                 110
Gly Phe Gly Ala Gly Ser Ala Gly Gly Gly Ala Ser Thr Arg Pro Gly
        115                 120                 125
Trp Lys Ser Gly Asp Trp Ile Cys Thr Arg Ser Gly Cys Asn Glu His
    130                 135                 140
Asn Phe Ala Asn Arg Met Glu Cys Tyr Arg Cys Asn Gly Pro Arg Asp
145                 150                 155                 160
Ser Ser Thr Gly Arg Ser Ser Tyr Leu Ser
                165                 170
```

<210> 337
<211> 826
<212> DNA
<213> Yucca filamentosa

<400> 337

```
ggcacaagct ctcacgcaac cccttgtctc cattttactc ttccttcctc tttcaaggga     60
cttgagaaga tgaacaggaa gccaggagac tggaactgca ggtcatgcca gcaccttaat    120
ttcagccgca gggactcgtg ccagcgctgc ggcgaccccc ggtcgatcgg cagcgagcga    180
tcggactacc cggcttcgt cgggggccgc gggggtcct cattcgggtt cagcggctcg    240
gacgtcaggc ccggggactg gtactgccag tgcggggccc acaacttcgc cagccgctcg    300
agctgcttca agtgcaatgc tttcaaggat gagtccgccg ctagcggcgg cctcgacggc    360
ggcgacatgc tgagatccag gggtttggc ttcggcggcg gcggcggcgc tcgcagtggc    420
tggaagtctg gtgactggat ttgcaacagg tctggctgca atgagcacaa cttcgctagc    480
aggatggaat gcttccgatg caatgcacct cgagattcgg gcactgaggt ttaaggagac    540
agccaagaga gaagtgacga gatgagatca gtgatgatga taccagtagt aatctcgagt    600
tattactagt tttgccacca gccccaactt tatctccttt tgatgttttt ttagagcccc    660
cttctaaacg gaggtgtctt ctctttttca tccgtttgtt tgcttttgaa agcttgcact    720
taccctttac accccccgc ccccctccac cttgtagttt gtgttgagag tgtctttgta    780
agtctttgga gaagggctcc tttagcttct tgggggagtc ccttct                  826
```

<210> 338
<211> 154
<212> PRT
<213> Yucca filamentosa

<400> 338

```
Met Asn Arg Lys Pro Gly Asp Trp Asn Cys Arg Ser Cys Gln His Leu
1               5                   10                  15
Asn Phe Ser Arg Arg Asp Ser Cys Gln Arg Cys Gly Asp Pro Arg Ser
            20                  25                  30
```

Ile Gly Ser Glu Arg Ser Asp Tyr Pro Gly Phe Val Gly Gly Arg Gly
35 40 45
Gly Ser Ser Phe Gly Phe Ser Gly Ser Asp Val Arg Pro Gly Asp Trp
50 55 60
Tyr Cys Gln Cys Gly Ala His Asn Phe Ala Ser Arg Ser Ser Cys Phe
65 70 75 80
Lys Cys Asn Ala Phe Lys Asp Glu Ser Ala Ala Ser Gly Gly Leu Asp
85 90 95
Gly Gly Asp Met Leu Arg Ser Arg Gly Phe Gly Phe Gly Gly Gly Gly
100 105 110
Gly Ala Arg Ser Gly Trp Lys Ser Gly Asp Trp Ile Cys Asn Arg Ser
115 120 125
Gly Cys Asn Glu His Asn Phe Ala Ser Arg Met Glu Cys Phe Arg Cys
130 135 140
Asn Ala Pro Arg Asp Ser Gly Thr Glu Val
145 150

<210> 339
<211> 614
<212> DNA
<213> Hordeum vulgare

<400> 339
cggcacgagg cacttctcat cttggagctc agcagcacag catccaagct ttttcttcgg 60
caagatgaac aggaagccag gagactggga ctgcaggtcg tgccagcacc tcaacttcag 120
tcgccgggac ctatgccagc gctgcggtga gccacgtagt gccgctgacc gtggcagcgt 180
cggtggtgct cttggtggtg actacgccaa ctttggcggc cgtggcgggg gtggttcctc 240
atttggtgcc ggctttggtg ccggctctga cgtccgcccg ggtgactggt actgcacctg 300
tggagcgcac aacttcgcca gccgctccag ctgcttcaag tgtgctgctt tcaaggagga 360
agctgccgtc aatggtggcg ctggtggctt tgatggtgac atgtcacgct caaggggctt 420
tggctttggc gctgtcggtg gcatgggtgg cggcatggga gccggcgcag ccggtggtcg 480
tgccagtcgc cctggctgga agtctcgcga ctggatttgc accaggtctg gatgcaacga 540
gcacaacttc gccagcaggc aggagtgctt caggtgcaac gcgccgaggg actccggtag 600
cgccacacca tacg 614

<210> 340
<211> 183
<212> PRT
<213> Hordeum vulgare

<400> 340
Met Asn Arg Lys Pro Gly Asp Trp Asp Cys Arg Ser Cys Gln His Leu
1 5 10 15
Asn Phe Ser Arg Arg Asp Leu Cys Gln Arg Cys Gly Glu Pro Arg Ser
20 25 30
Ala Ala Asp Arg Gly Ser Val Gly Gly Ala Leu Gly Gly Asp Tyr Ala
35 40 45
Asn Phe Gly Gly Arg Gly Gly Gly Gly Ser Ser Phe Gly Ala Gly Phe
50 55 60
Gly Ala Gly Ser Asp Val Arg Pro Gly Asp Trp Tyr Cys Thr Cys Gly
65 70 75 80
Ala His Asn Phe Ala Ser Arg Ser Ser Cys Phe Lys Cys Ala Ala Phe
85 90 95
Lys Glu Glu Ala Ala Val Asn Gly Gly Ala Gly Gly Phe Asp Gly Asp
100 105 110
Met Ser Arg Ser Arg Gly Phe Gly Phe Gly Ala Val Gly Gly Met Gly
115 120 125
Gly Gly Met Gly Ala Gly Ala Ala Gly Gly Arg Ala Ser Arg Pro Gly
130 135 140

Trp Lys Ser Arg Asp Trp Ile Cys Thr Arg Ser Gly Cys Asn Glu His
145 150 155 160
Asn Phe Ala Ser Arg Gln Glu Cys Phe Arg Cys Asn Ala Pro Arg Asp
165 170 175
Ser Gly Ser Ala Thr Pro Tyr
180

<210> 341
<211> 54
<212> DNA
<213> Artificial sequence

<220>
<223> primer: prm5539

<400> 341
ggggacaagt ttgtacaaaa aagcaggctt aaacaatgag cagacccgga gatt 54

<210> 342
<211> 51
<212> DNA
<213> Artificial sequence

<220>
<223> primer: prm5540

<400> 342
ggggaccact ttgtacaaga aagctgggta gacaaggcta cttcaaaagc a 51

<210> 343
<211> 2193
<212> DNA
<213> Oryza sativa

<400> 343
aatccgaaaa gtttctgcac cgttttcacc ccctaactaa caatataggg aacgtgtgct 60
aaatataaaa tgagacctta tatatgtagc gctgataact agaactatgc aagaaaaact 120
catccaccta ctttagtggc aatcgggcta aataaaaaag agtcgctaca ctagtttcgt 180
tttccttagt aattaagtgg gaaaatgaaa tcattattgc ttagaatata cgttcacatc 240
tctgtcatga agttaaatta ttcgaggtag ccataattgt catcaaactc ttcttgaata 300
aaaaaatctt tctagctgaa ctcaatgggt aaagagagag atttttttta aaaaaataga 360
atgaagatat tctgaacgta ttggcaaaga tttaaacata taattatata attttatagt 420
ttgtgcattc gtcatatcgc acatcattaa ggacatgtct tactccatcc caatttttat 480
ttagtaatta aagacaattg acttattttt attatttatc ttttttcgat tagatgcaag 540
gtacttacgc acacactttg tgctcatgtg catgtgtgag tgcacctcct caatacacgt 600
tcaactagca acacatctct aatatcactc gcctatttaa tacatttagg tagcaatatc 660
tgaattcaag cactccacca tcaccagacc acttttaata atatctaaaa tacaaaaaat 720
aattttacag aatagcatga aaagtatgaa acgaactatt taggtttttc acatacaaaa 780
aaaaaaagaa ttttgctcgt gcgcgagcgc caatctccca tattgggcac acaggcaaca 840
acagagtggc tgcccacaga acaacccaca aaaaacgatg atctaacgga ggacagcaag 900
tccgcaacaa ccttttaaca gcaggctttg cggccaggag agaggaggag aggcaaagaa 960
aaccaagcat cctcctcctc ccatctataa attcctcccc ccttttcccc tctctatata 1020
ggaggcatcc aagccaagaa gagggagagc accaaggaca cgcgactagc agaagccgag 1080
cgaccgcctt cttcgatcca tatcttccgg tcgagttctt ggtcgatctc ttccctcctc 1140
cacctcctcc tcacagggta tgtgcccttc ggttgttctt ggatttattg ttctaggttg 1200
tgtagtacgg gcgttgatgt taggaaaggg gatctgtatc tgtgatgatt cctgttcttg 1260
gatttgggat agaggggttc ttgatgttgc atgttatcgg ttcggtttga ttagtagtat 1320
ggttttcaat cgtctggaga gctctatgga aatgaaatgg tttagggtac ggaatcttgc 1380
gattttgtga gtacctttttg tttgaggtaa aatcagagca ccggtgattt tgcttggtgt 1440

```
aataaaagta cggttgtttg gtcctcgatt ctggtagtga tgcttctcga tttgacgaag   1500
ctatcctttg tttattccct attgaacaaa aataatccaa ctttgaagac ggtcccgttg   1560
atgagattga atgattgatt cttaagcctg tccaaaattt cgcagctggc ttgtttagat   1620
acagtagtcc ccatcacgaa attcatggaa acagttataa tcctcaggaa caggggattc   1680
cctgttcttc cgatttgctt tagtcccaga attttttttc ccaaatatct taaaaagtca   1740
ctttctggtt cagttcaatg aattgattgc tacaaataat gcttttatag cgttatccta   1800
gctgtagttc agttaatagg taatacccct atagtttagt caggagaaga acttatccga   1860
tttctgatct ccatttttaa ttatatgaaa tgaactgtag cataagcagt attcatttgg   1920
attatttttt ttattagctc tcaccccttc attattctga gctgaaagtc tggcatgaac   1980
tgtcctcaat tttgttttca aattcacatc gattatctat gcattatcct cttgtatcta   2040
cctgtagaag tttcttttg gttattcctt gactgcttga ttacagaaag aaatttatga    2100
agctgtaatc gggatagtta tactgcttgt tcttatgatt catttccttt gtgcagttct   2160
tggtgtagct tgccactttc accagcaaag ttc                                 2193
```

<210> 344
<211> 3
<212> PRT
<213> Artificial sequence

<220>
<223> sequence motif 1

<220>
<221> VARIANT
<222> (1)..(1)
<223> /replace="Arg" /replace="Asp" /replace="Asn"

<400> 344
Gly Asp Trp
1

<210> 345
<211> 3
<212> PRT
<213> Artificial sequence

<220>
<223> sequence motif 2

<400> 345
Gly Ser Trp
1

<210> 346
<211> 5
<212> PRT
<213> Artificial sequence

<220>
<223> sequence motif 3

<220>
<221> VARIANT
<222> (3)..(3)
<223> /replace="Cys" /replace="Ser"

<220>
<221> VARIANT
<222> (4)..(4)

```
<223>  /replace="Lys"

<400>  346
Asn Phe Gln Arg Arg
1               5

<210>  347
<211>  5
<212>  PRT
<213>  Artificial sequence

<220>
<223>  sequence motif 4

<220>
<221>  VARIANT
<222>  (2)..(2)
<223>  /replace="Tyr"

<220>
<221>  VARIANT
<222>  (3)..(3)
<223>  /replace="Ser" /replace="Pro"

<220>
<221>  VARIANT
<222>  (4)..(4)
<223>  /replace="Ser" /replace="Phe"

<400>  347
Asn Phe Ala Asn Arg
1               5
```